(19) Europäisches Patentamt / European Patent Office / Office européen des brevets

(11) EP 3 880 659 B1

(12) # EUROPEAN PATENT SPECIFICATION

(45) Date of publication and mention of the grant of the patent:
**03.01.2024 Bulletin 2024/01**

(21) Application number: **19817076.3**

(22) Date of filing: **14.11.2019**

(51) International Patent Classification (IPC):
***C07D 221/16*** *(2006.01)* ***C07D 409/12*** *(2006.01)*
***C07D 417/12*** *(2006.01)* ***C07D 213/71*** *(2006.01)*
***C07C 311/57*** *(2006.01)* ***C07D 263/46*** *(2006.01)*
***C07D 277/80*** *(2006.01)* ***C07D 307/64*** *(2006.01)*
***C07D 333/34*** *(2006.01)* ***C07D 405/12*** *(2006.01)*
***C07D 487/04*** *(2006.01)* ***C07D 491/04*** *(2006.01)*
***C07D 498/04*** *(2006.01)* ***A61K 31/64*** *(2006.01)*
***A61P 35/00*** *(2006.01)*

(52) Cooperative Patent Classification (CPC):
**C07C 381/10; C07D 213/71; C07D 263/46; C07D 277/80; C07D 307/64; C07D 333/34; C07D 405/12; C07D 487/04; C07D 491/04; C07D 498/04;** C07B 2200/05; C07C 2601/02; C07C 2603/10; C07C 2603/12

(86) International application number:
**PCT/US2019/061533**

(87) International publication number:
**WO 2020/102576 (22.05.2020 Gazette 2020/21)**

(54) **COMPOUNDS AND COMPOSITIONS FOR TREATING CONDITIONS ASSOCIATED WITH NLRP ACTIVITY**

VERBINDUNGEN UND ZUSAMMENSETZUNGEN ZUR BEHANDLUNG VON ERKRANKUNGEN IN ZUSAMMENHANG MIT NLRP-AKTIVITÄT

COMPOSÉS ET COMPOSITIONS DESTINÉS AU TRAITEMENT D’ÉTATS PATHOLOGIQUES ASSOCIÉS À UNE ACTIVITÉ DE NLRP

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**16.11.2018 US 201862768804 P**
**19.11.2018 US 201862769165 P**
**19.11.2018 US 201862769151 P**
**23.01.2019 US 201962795913 P**
**24.01.2019 US 201962796356 P**
**24.01.2019 US 201962796361 P**
**07.02.2019 US 201962802621 P**
**04.09.2019 US 201962895954 P**

(43) Date of publication of application:
**22.09.2021 Bulletin 2021/38**

(73) Proprietor: **Novartis AG**
**4056 Basel (CH)**

(72) Inventors:
- **FRANCHI, Luigi**
  **Boston, MA 02116 (US)**
- **GHOSH, Shomir**
  **Boston, MA 02116 (US)**
- **GLICK, Gary**
  **Boston, MA 02116 (US)**
- **KATZ, Jason**
  **Boston, MA 02116 (US)**
- **OPIPARI, Anthony, William, Jr.**
  **Boston, MA 02116 (US)**
- **ROUSH, William**
  **Boston, MA 02116 (US)**
- **SEIDEL, Hans, Martin**
  **Boston, MA 02116 (US)**
- **SHEN, Dong-Ming**
  **Boston, MA 02116 (US)**
- **VENKATRAMAN, Shankar**
  **Boston, MA 02116 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 880 659 B1

• **WINKLER, David, Guenther**
**Boston, MA 02116 (US)**

(74) Representative: **Strang, Andrea Josephine**
**Novartis Pharma AG**
**Patent Department**
**Postfach**
**4002 Basel (CH)**

(56) References cited:
**WO-A1-2016/131098** **WO-A1-2017/129897**
**WO-A1-2018/225018** **WO-A1-2019/023147**
**WO-A1-2019/068772**

- **TOTH J E ET AL: "SULFONIMIDAMIDE ANALOGS OF ONCOLYTIC SULFONYLUREAS", JOURNAL OF MEDICINAL CHEMISTRY, AMERICAN CHEMICAL SOCIETY, US, vol. 40, no. 6, 1 January 1997 (1997-01-01), pages 1018-1025, XP000926714, ISSN: 0022-2623, DOI: 10.1021/JM960673L**
- **SCOZZAFAVA A ET AL: "Arylsulfonyl-N,N-diethyl-dithiocarbamates : a novel class of antitumor agents", BIOORGANIC & MEDICINAL CHEMISTRY LETTERS, PERGAMON, AMSTERDAM, NL, vol. 10, no. 16, 21 August 2000 (2000-08-21), pages 1887-1891, XP004216023, ISSN: 0960-894X, DOI: 10.1016/S0960-894X(00)00375-9**
- **FLAVIA IZZO ET AL: "Exploration of Novel Chemical Space: Synthesis and in vitro Evaluation of N-Functionalized Tertiary Sulfonimidamides", CHEMISTRY - A EUROPEAN JOURNAL, vol. 24, no. 37, 19 June 2018 (2018-06-19) , pages 9295-9304, XP055626913, DE ISSN: 0947-6539, DOI: 10.1002/chem.201801557**

## Description

### TECHNICAL FIELD

The present invention relates to the compounds mentioned in claim 1, claim 2 and claim 3, as well as a pharmaceutical composition as claimed in claim 4.

The compounds as mentioned in claim 1, 2 or 3 and the composition of claim 4 are useful, e.g., for treating a condition, disease or disorder in which a decrease or increase in NLRP3 activity (e.g., an increase, e.g., a condition, disease or disorder associated with NLRP3 signaling) contributes to the pathology and/or symptoms and/or progression of the condition, disease or disorder in a subject (e.g., a human).

### BACKGROUND

The NLRP3 inflammasome is a component of the inflammatory process and its aberrant activation is pathogenic in inherited disorders such as the cryopyrin associated periodic syndromes (CAPS). The inherited CAPS Muckle-Wells syndrome (MWS), familial cold autoinflammatory syndrome (FCAS) and neonatal onset multi-system inflammatory disease (NOMID) are examples of indications that have been reported to be associated with gain of function mutations in NLRP3.

NLRP3 can form a complex and has been implicated in the pathogenesis of a number of complex diseases, including but not limited to metabolic disorders such as type 2 diabetes, atherosclerosis, obesity and gout, as well as diseases of the central nervous system, such as Alzheimer's disease and multiple sclerosis and Amyotrophic Lateral Sclerosis and Parkinson disease, lung disease, such as asthma and COPD and pulmonary idiopathic fibrosis, liver disease, such as NASH syndrome, viral hepatitis and cirrhosis, pancreatic disease, such as acute and chronic pancreatitis, kidney disease, such as acute and chronic kidney injury, intestinal disease such as Crohn's disease and Ulcerative Colitis, skin disease such as psoriasis, musculoskeletal disease such as scleroderma, vessel disorders, such as giant cell arteritis, disorders of the bones, such as Osteoarthritis , osteoporosis and osteopetrosis disorders eye disease, such as glaucoma and macular degeneration, diseased caused by viral infection such as HIV and AIDS, autoimmune disease such as Rheumatoid Arthritis, Systemic Lupus Erythematosus, Autoimmune Thyroiditis, Addison's disease, pernicious anemia, cancer and aging.

In light of the above, it would be desirable to provide compounds that modulate (e.g., antagonize) NLRP3.

Several patients having inflammatory or autoimmune diseases are treated with anti-TNF$\alpha$ agents. A subpopulation of such patients develop resistance to treatment with the anti-TNF$\alpha$ agents. It is desirable to develop methods for reducing a patient's resistance to anti-TNF$\alpha$ agents. In light of the this, it would also be desirable to provide alternative therapies for treating inflammatory or autoimmune diseases (for example NLRP3 inflammasome inhibitors) to avoid or minimise the use of anti-TNF$\alpha$ agents.

Intestinal bowel disease (IBD), encompassing Ulcerative Colitis (UC) and Crohn's disease (CD), are chronic diseases characterized by barrier dysfunction and uncontrolled inflammation and mucosal immune reactions in the gut. A number of inflammatory pathways have been implicated in the progression of IBD, and anti-inflammatory therapy such as tumor necrosis factor-alpha (TNF-$\alpha$) blockade has shown efficacy in the clinic (Rutgeerts P et al N Engl J Med 2005; 353:2462-76*)*. Anti-TNFa therapies, however, do not show complete efficacy, however, other cytokines such as IL-1$\beta$, IL-6, IL-12, IL-18, IL-21, and IL-23 have been shown to drive inflammatory disease pathology in IBD (Neurath MF Nat Rev Immunol 2014;14;329-42). IL-1$\beta$ and IL-18 are produced by the NLRP3 inflammasome in response to pathogenic danger signals, and have been shown to play a role in IBD. Anti-IL-1$\beta$ therapy is efficacious in patients with IBD driven by genetic mutations in CARD8 or IL-10R (Mao L et al, J Clin Invest 2018;238:1793-1806*,* Shouval DS et al, Gastroenterology 2016;151:1100-1104), IL-18 genetic polymorphisms have been linked to UC (Kanai T et al, Curr Drug Targets 2013;14:1392-9)*,* and NLRP3 inflammasome inhibitors have been shown to be efficacious in murine models of IBD *(*Perera AP et al, Sci Rep 2018;8:8618). Resident gut immune cells isolated from the lamina propria of IBD patients can produce IL-1$\beta$, either spontaneously or when stimulated by LPS, and this IL-1$\beta$ production can be blocked by the ex vivo addition of a NLRP3 antagonist. Based on strong clinical and preclinical evidence showing that inflammasome-driven IL-1$\beta$ and IL-18 play a role in IBD pathology, it is clear that NLRP3 inflammasome inhibitors could be an efficacious treatment option for UC, Crohn's disease, or subsets of IBD patients. These subsets of patients could be defined by their peripheral or gut levels of inflammasome related cytokines including IL-1$\beta$, IL-6, and IL-18, by genetic factors that pre-dispose IBD patients to having NLRP3 inflammasome activation such as mutations in genes including ATG16L1, CARD8, IL-10R, or PTPN2 (Saitoh T et al, Nature 2008;456:264*,* Spalinger MR, Cell Rep 2018;22:1835)*,* or by other clinical rationale such as non-response to TNF therapy.

Though anti-TNF therapy is an effective treatment option for Crohn's disease, 40% of patients fail to respond. One-third of non-responsive CD patients fail to respond to anti-TNF therapy at the onset of treatment, while another third lose response to treatment over time (secondary non-response). Secondary non-response can be due to the

generation of anti-drug antibodies, or a change in the immune compartment that desensitizes the patient to anti-TNF (Ben-Horin S et al, Autoimmun Rev 2014;13:24-30, Steenholdt C et al Gut 2014;63:919-27). Anti-TNF reduces inflammation in IBD by causing pathogenic T cell apoptosis in the intestine, therefore eliminating the T cell mediated inflammatory response (Van den Brande et al Gut 2007:56:509-17). There is increased NLRP3 expression and increased production of IL-1β in the gut of TNF-non-responsive CD patients (Leal RF et al Gut 2015;64:233-42) compared to TNF-responsive patients, suggesting NLRP3 inflammasome pathway activation. Furthermore, there is increased expression of TNF-receptor 2 (TNF-R2), which allows for TNF-mediated proliferation of T cells (Schmitt H et al Gut 2018;0:1-15). IL-1β signaling in the gut promotes T cell differentiation toward Th1/17 cells which can escape anti-TNF-a mediated apoptosis. It is therefore likely that NLRP3 inflammasome activation can cause non-responsiveness in CD patients to anti-TNF-a therapy by sensitizing pathogenic T cells in the gut to anti-TNF-a mediated apoptosis. Experimental data from immune cells isolated from the gut of TNF-resistant Crohn's patients show that these cells spontaneously release IL-1β, which can be inhibited by the addition of an NLRP3 antagonist. NLRP3 inflammasome antagonists - in part by blocking IL-1β secretion - would be expected to inhibit the mechanism leading to anti-TNF non-responsiveness, re-sensitizing the patient to anti-TNF therapy. In IBD patients who are naive to anti-TNF therapy, treatment with an NLRP3 antagonist would be expected to prevent primary- and secondary-non responsiveness by blocking the mechanism leading to non-response.

NLRP3 antagonists that are efficacious locally in the gut can be efficacious drugs to treat IBD; in particular in the treatment of TNF-resistant CD alone or in combination with anti-TNF therapy. Systemic inhibition of both IL-1β and TNF-α has been shown to increase the risk of opportunistic infections (Genovese MC et al, Arthritis Rheum 2004;50:1412), therefore, only blocking the NLRP3 inflammasome at the site of inflammation would reduce the infection risk inherent in neutralizing both IL-1β and TNF-α. NLRP3 antagonists that are potent in NLRP3-inflammasome driven cytokine secretion assays in cells, but have low permeability in vitro in a permeability assay such as an MDCK assay, have poor systemic bioavailability in a rat or mouse pharmacokinetic experiment, but high levels of compound in the colon and/or small intestine could be a useful therapeutic option for gut restricted purposes.

WO 2016/131098 and WO 2017/129897 provide compounds useful in modulating the activity of NLRP3.

In light of the above, the present invention also provides alternative therapies for the treatment of inflammatory or autoimmune diseases, including IBD, that solves the above problems associated with anti-TNFa agents.

## SUMMARY

This disclosure features chemical entities as defined in the claims (e.g., a compound that modulates (e.g., antagonizes) NLRP3, or a pharmaceutically acceptable salt, and/or hydrate, and/or cocrystal, and/or drug combination of the compound) that are useful, e.g., for treating a condition, disease or disorder in which a decrease or increase in NLRP3 activity (e.g., an increase, e.g., a condition, disease or disorder associated with NLRP3 signaling).

In one aspect, pharmaceutical compositions as claimed in claim 4 are featured that include a chemical entity described herein (e.g., a compound described generically or specifically herein or a pharmaceutically acceptable salt thereof or compositions containing the same) and one or more pharmaceutically acceptable excipients.

Embodiments of the disclosure (where not falling under the claims, only for disclosure and reference purposes and not as part of the invention) can include one or more of the following features:

- The chemical entity be administered in combination with one or more additional therapies with one or more agents suitable for the treatment of the condition, disease or disorder.
- Examples of the indications that may be treated by the compounds disclosed herein include but are not limited to metabolic disorders such as type 2 diabetes, atherosclerosis, obesity and gout, as well as diseases of the central nervous system, such as Alzheimer's disease and multiple sclerosis and Amyotrophic Lateral Sclerosis and Parkinson disease, lung disease, such as asthma and COPD and pulmonary idiopathic fibrosis, liver disease, such as NASH syndrome, viral hepatitis and cirrhosis, pancreatic disease, such as acute and chronic pancreatitis, kidney disease, such as acute and chronic kidney injury, intestinal disease such as Crohn's disease and Ulcerative Colitis, skin disease such as psoriasis, musculoskeletal disease such as scleroderma, vessel disorders, such as giant cell arteritis, disorders of the bones, such as osteoarthritis , osteoporosis and osteopetrosis disorders, eye disease, such as glaucoma and macular degeneration, diseases caused by viral infection such as HIV and AIDS, autoimmune disease such as rheumatoid arthritis, systemic Lupus erythematosus, autoimmune thyroiditis; Addison's disease, pernicious anemia, cancer and aging.
- The methods can further include identifying the subject.
- The present invention as defined in the claims is also relates to the Applicant's discovery that inhibition of NLRP3 inflammasomes can increase a subject's sensitivity to an anti-TNFα agent or can overcome resistance to an anti-TNFα agent in a subject, or indeed provide an alternative therapy to anti-TNFα agents.
- Provided herein are methods of treating a subject that include: (a) identifying a subject having a cell that has an

elevated level of NLRP3 inflammasome activity and/or expression as compared to a reference level; and (b) administering to the identified subject a therapeutically effective amount of an compound of Formula I or a pharmaceutically acceptable salt, solvate, or co-crystal thereof.

- Provided herein are methods for the treatment of inflammatory or autoimmune disease including IBD, such as UC and CD in a subject in need thereof, comprising administering to said subject a therapeutically effective amount a compound for Formula I or a pharmaceutically acceptable salt, solvate, or co-crystal thereof, wherein the NLRP3 antagonist is a gut-targeted NLRP3 antagonist.
- Provided herein are methods of treating a subject in need thereof, that include: (a) identifying a subject having resistance to an anti-TNFα agent; and (b) administering a treatment comprising a therapeutically effective amount of a compound for Formula I, or a pharmaceutically acceptable salt, solvate, or co-crystal thereof to the identified subj ect.
- Provided herein are methods of treating a subject in need thereof, that include: administering a treatment comprising a therapeutically effective amount of a compound for Formula I or a pharmaceutically acceptable salt, solvate, or co-crystal thereof to a subject identified as having resistance to an anti-TNFα agent.
- Provided herein are methods of selecting a treatment for a subject in need thereof, that include: (a) identifying a subject having resistance to an anti-TNFα agent; and (b) selecting for the identified subject a treatment comprising a therapeutically effective amount of a compound for Formula I or a pharmaceutically acceptable salt, solvate, or co-crystal thereof.
- Provided herein are methods of selecting a treatment for a subject in need thereof, that include selecting a treatment comprising a therapeutically effective amount of a compound for Formula I or a pharmaceutically acceptable salt, solvate, or co-crystal thereof for a subject identified as having resistance to an anti-TNFα agent.
- In some embodiments of any of the methods described herein, the treatment further includes a therapeutically effective amount of an anti-TNFα agent, in addition to the NLRP3 antagonist.

## Additional Definitions

To facilitate understanding of the disclosure set forth herein, a number of additional terms are defined below. Generally, the nomenclature used herein and the laboratory procedures in organic chemistry, medicinal chemistry, and pharmacology described herein are those well-known and commonly employed in the art. Unless defined otherwise, all technical and scientific terms used herein generally have the same meaning as commonly understood by one of ordinary skill in the art to which this disclosure belongs. Each of the patents, applications, published applications, and other publications that are mentioned throughout the specification and the attached appendices are incorporated herein by reference in their entireties.

As used herein, the term "NLRP3" is meant to include, without limitation, nucleic acids, polynucleotides, oligonucleotides, sense and antisense polynucleotide strands, complementary sequences, peptides, polypeptides, proteins, homologous and/or orthologous NLRP3 molecules, isoforms, precursors, mutants, variants, derivatives, splice variants, alleles, different species, and active fragments thereof.

The term "acceptable" with respect to a formulation, composition or ingredient, as used herein, means having no persistent detrimental effect on the general health of the subject being treated.

"API" refers to an active pharmaceutical ingredient.

The terms "effective amount" or "therapeutically effective amount," as used herein, refer to a sufficient amount of a chemical entity (e.g., a compound exhibiting activity as a modulator of NLRP3, or a pharmaceutically acceptable salt and/or hydrate and/or cocrystal thereof;) being administered which will relieve to some extent one or more of the symptoms of the disease or condition being treated. The result includes reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case is determined using any suitable technique, such as a dose escalation study.

The term "excipient" or "pharmaceutically acceptable excipient" means a pharmaceutically-acceptable material, composition, or vehicle, such as a liquid or solid filler, diluent, carrier, solvent, or encapsulating material. In one embodiment, each component is " pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. *See, e.g.,* Remington: The Science and Practice of Pharmacy, 21st ed.; Lippincott Williams & Wilkins: Philadelphia, PA, 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "pharmaceutically acceptable salt" may refer to pharmaceutically acceptable addition salts prepared from pharmaceutically acceptable non-toxic acids including inorganic and organic acids. In certain instances, pharmaceutically acceptable salts are obtained by reacting a compound described herein, with acids such as hydrochloric acid, hydrobromic acid, sulfuric acid, nitric acid, phosphoric acid, methanesulfonic acid, ethanesulfonic acid, p-toluenesulfonic acid, salicylic acid and the like. The term "pharmaceutically acceptable salt" may also refer to pharmaceutically acceptable addition salts prepared by reacting a compound having an acidic group with a base to form a salt such as an ammonium salt, an alkali metal salt, such as a sodium or a potassium salt, an alkaline earth metal salt, such as a calcium or a magnesium salt, a salt of organic bases such as dicyclohexylamine, *N*-methyl-D-glucamine, tris(hydroxymethyl)methylamine, and salts with amino acids such as arginine, lysine, and the like, or by other methods previously determined. The pharmacologically acceptable salt is not specifically limited as far as it can be used in medicaments. Examples of a salt that the compounds described hereinform with a base include the following: salts thereof with inorganic bases such as sodium, potassium, magnesium, calcium, and aluminum; salts thereof with organic bases such as methylamine, ethylamine and ethanolamine; salts thereof with basic amino acids such as lysine and ornithine; and ammonium salt. The salts may be acid addition salts, which are specifically exemplified by acid addition salts with the following: mineral acids such as hydrochloric acid, hydrobromic acid, hydroiodic acid, sulfuric acid, nitric acid, and phosphoric acid:organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethanesulfonic acid; acidic amino acids such as aspartic acid and glutamic acid.

The term "pharmaceutical composition" refers to a mixture of a compound described herein with other chemical components (referred to collectively herein as "excipients"), such as carriers, stabilizers, diluents, dispersing agents, suspending agents, and/or thickening agents. The pharmaceutical composition facilitates administration of the compound to an organism. Multiple techniques of administering a compound exist in the art including, but not limited to: rectal, oral, intravenous, aerosol, parenteral, ophthalmic, pulmonary, and topical administration.

The term "subject" refers to an animal, including, but not limited to, a primate (*e.g.*, human), monkey, cow, pig, sheep, goat, horse, dog, cat, rabbit, rat, or mouse. The terms "subject" and "patient" are used interchangeably herein in reference, for example, to a mammalian subject, such as a human.

As used herein, the term "treat", "treating" or "treatment" of any disease or disorder refers in one embodiment to ameliorating the disease or disorder (i.e. slowing or arresting or reducing the development of the disease or at least one of the clinical symptoms or pathological features thereof). In another embodiment "treat", "treating" or "treatment" refers to alleviating or ameliorating at least one physical parameter or pathological features of the disease, e.g. including those, which may not be discernible by the subject. In yet another embodiment, "treat", "treating" or "treatment" refers to modulating the disease or disorder, either physically, (e.g. stabilization of at least one discernible or non-discernible symptom), physiologically (e.g. stabilization of a physical parameter) or both. In yet another embodiment, "treat", "treating" or "treatment" refers to preventing or delaying the onset or development or progression of the disease or disorder, or of at least one symptoms or pathological features associated thereof. In yet another embodiment, "treat", "treating" or "treatment" refers to preventing or delaying progression of the disease to a more advanced stage or a more serious condition.

As used herein, the term "prevent", "preventing" or "prevention" in connection to a disease or disorder refers to the prophylactic treatment of a subject who is at risk of developing a condition (e.g., specific disease or disorder or clinical symptom thereof) resulting in a decrease in the probability that the subject will develop the condition.

## DETAILED DESCRIPTION

The present invention relates to a compound selected from the group consisting of the compounds below:

| | | | |
|---|---|---|---|
| 501 | 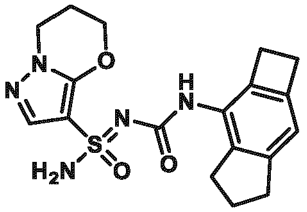 | 618 | 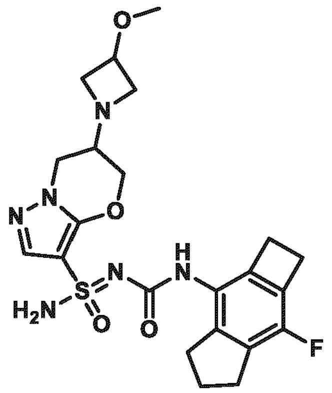 |
| 502 | 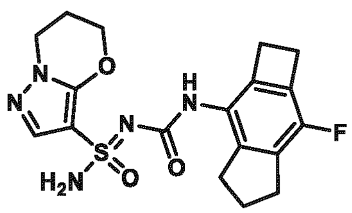 | 619 | 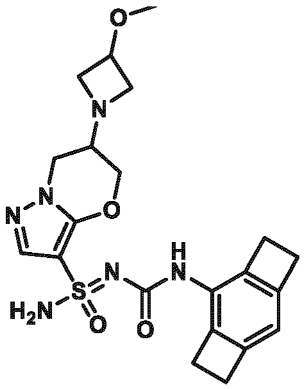 |
| 503 | 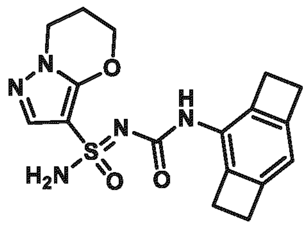 | 620 | 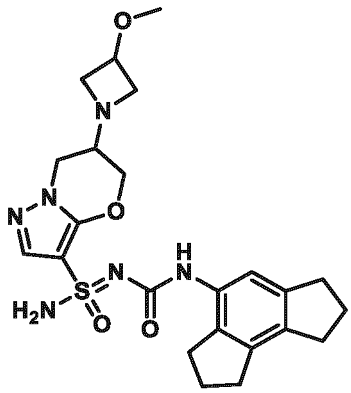 |
| 504 | 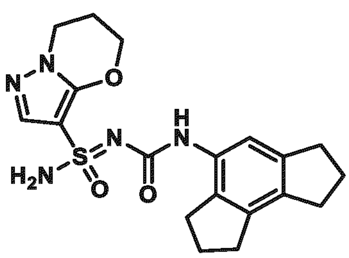 | 621 | 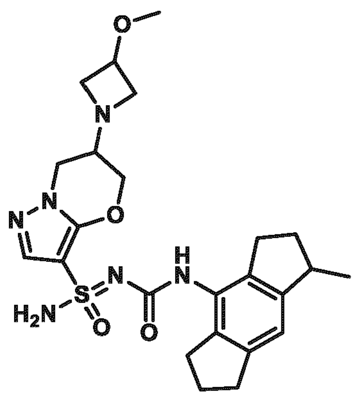 |

(continued)

| | | | |
|---|---|---|---|
| 505 | 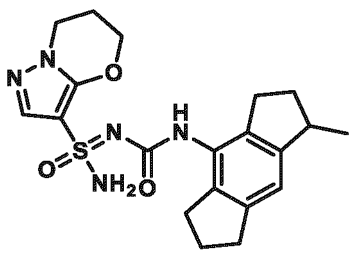 | 622 | 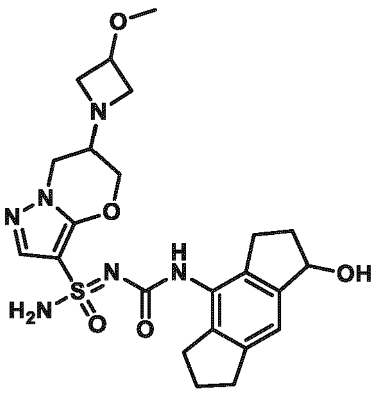 |
| 506 | 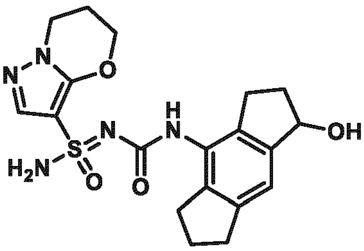 | 623 | 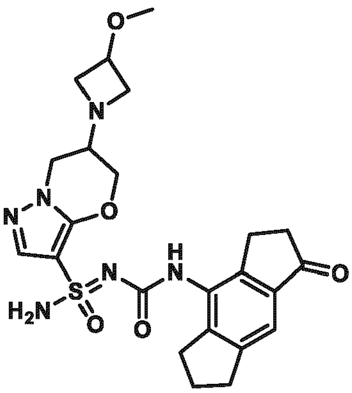 |
| 507 | 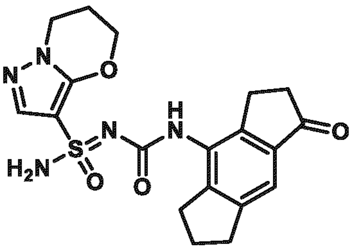 | 624 | 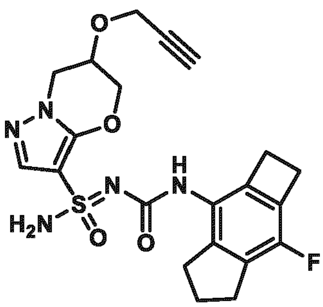 |
| 508 | 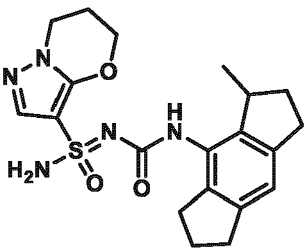 | 625 | 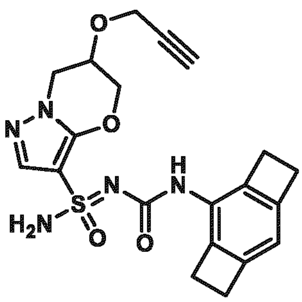 |

(continued)

| | | | |
|---|---|---|---|
| 509 | 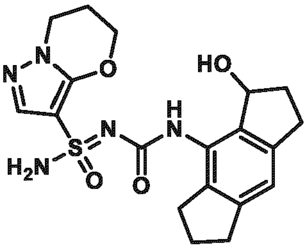 | 626 | 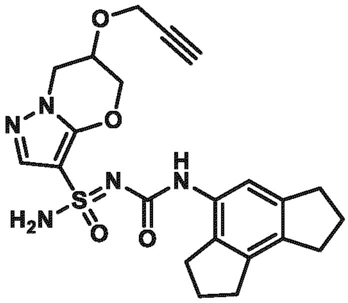 |
| 510 | 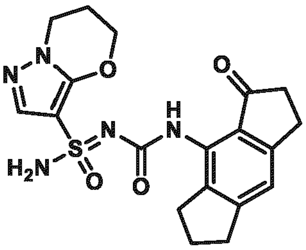 | 627 | 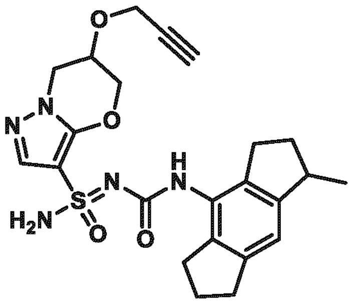 |
| 511 | 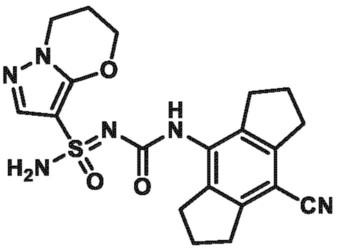 | 628 | 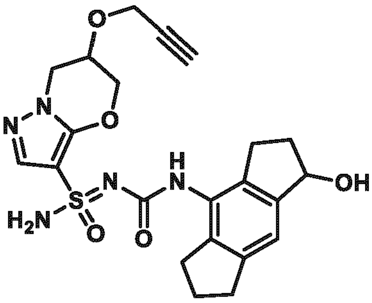 |
| 512 | 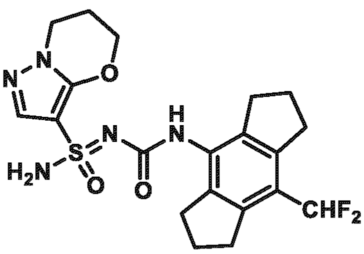 | 629 | 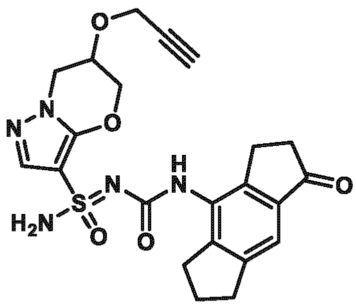 |
| 513 | 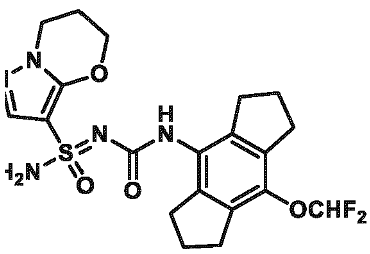 | 630 | 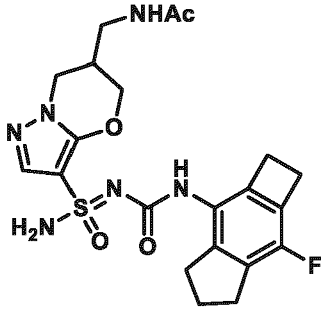 |

(continued)

| | | | |
|---|---|---|---|
| 514 | 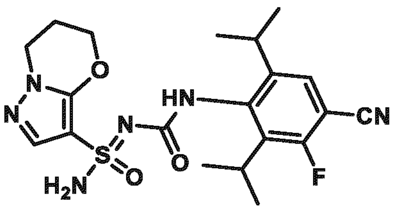 | 631 | 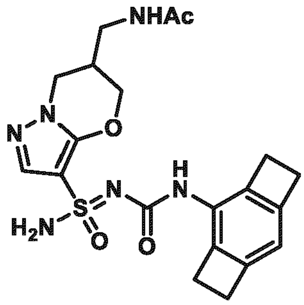 |
| 515 | 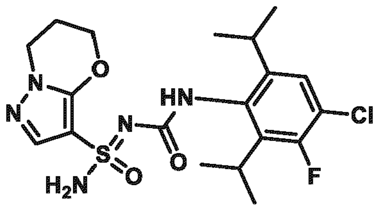 | 632 | 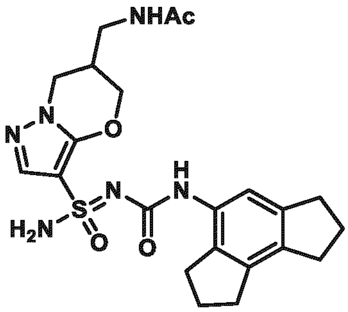 |
| 516 | 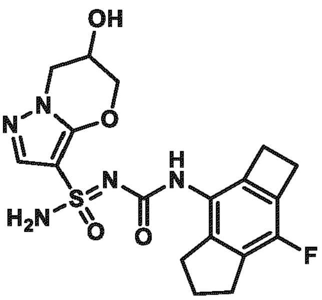 | 633 | 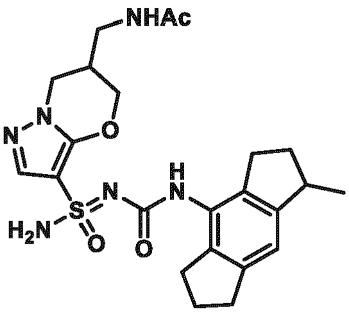 |
| 517 | 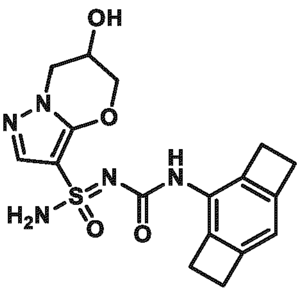 | 634 | 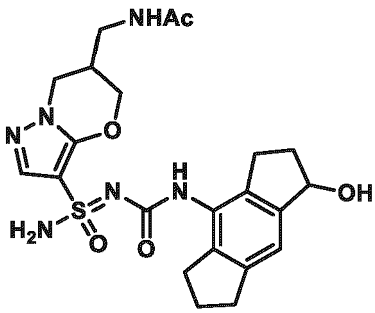 |
| 518 | 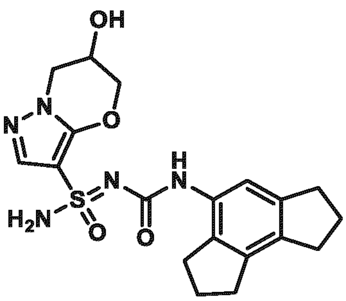 | 635 | 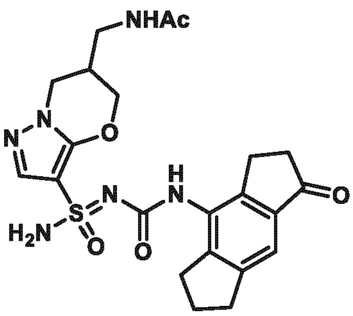 |

(continued)

| | | | |
|---|---|---|---|
| 519 | 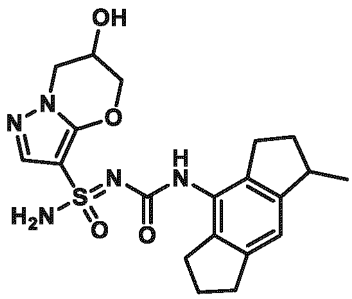 | 636 | 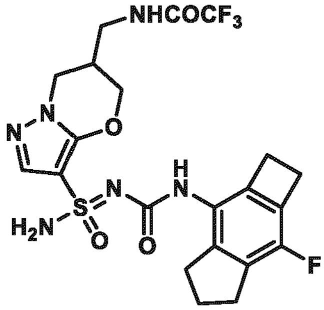 |
| 520 | 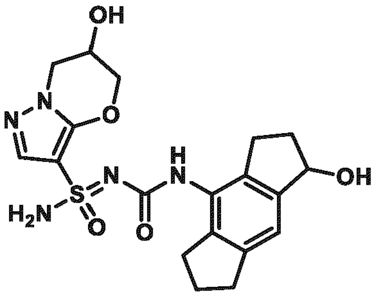 | 637 | 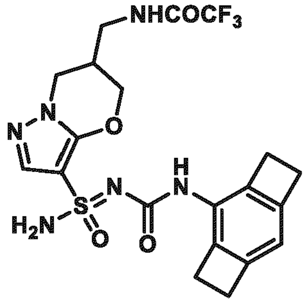 |
| 521 | 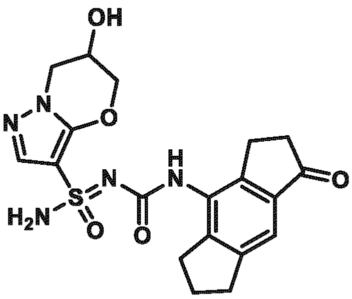 | 638 | 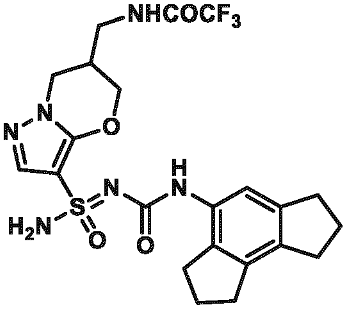 |
| 522 | 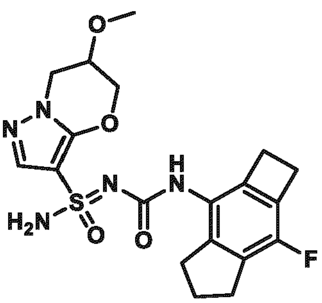 | 639 | 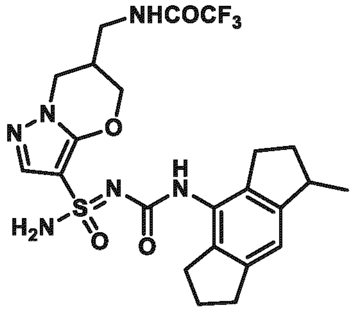 |
| 523 | 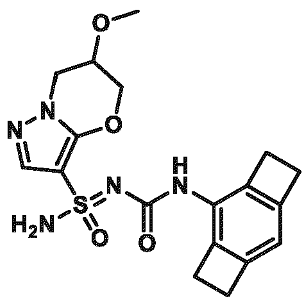 | 640 | 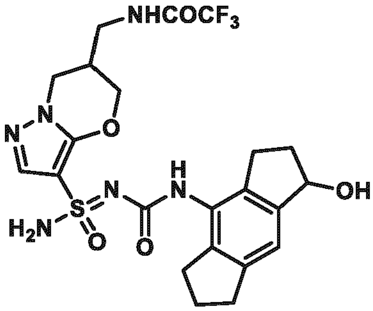 |

(continued)

| | | | |
|---|---|---|---|
| 524 | 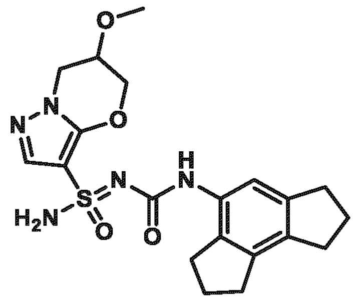 | 641 | 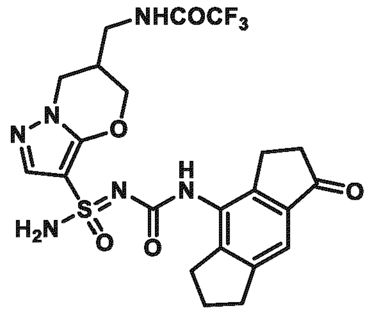 |
| 525 | 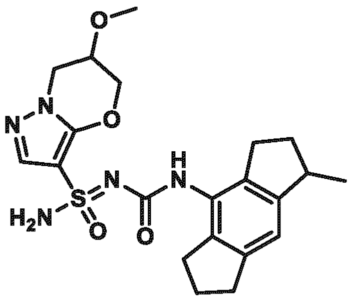 | 642 | 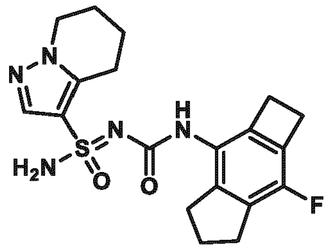 |
| 526 | 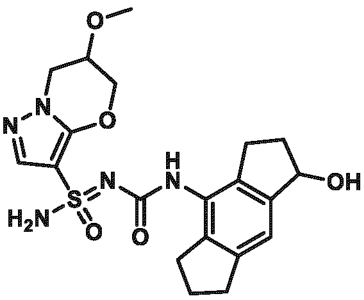 | 643 | 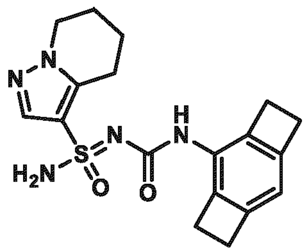 |
| 527 | 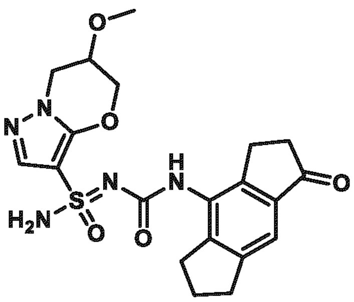 | 644 | 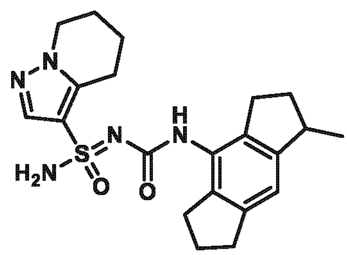 |
| 528 | 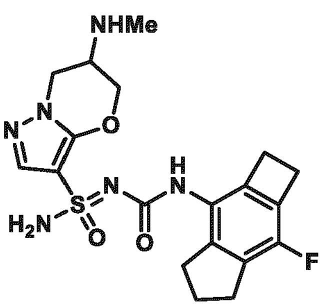 | 645 | 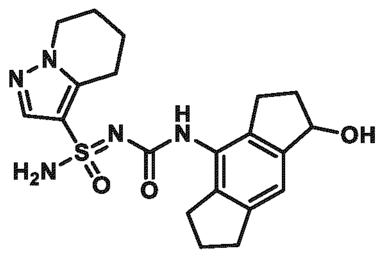 |

(continued)

| | | | |
|---|---|---|---|
| 529 | 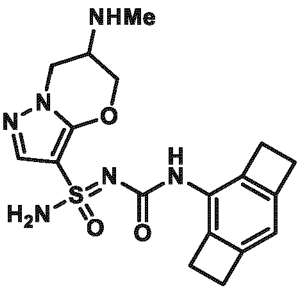 | 646 | 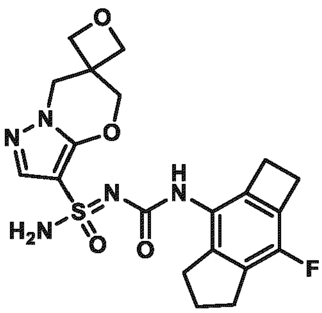 |
| 530 | 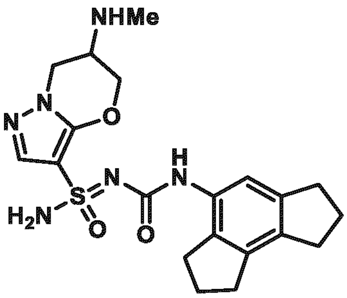 | 647 | 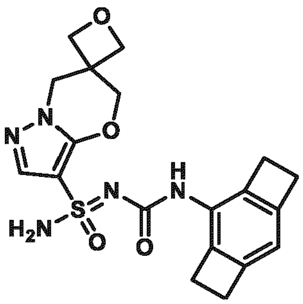 |
| 531 | 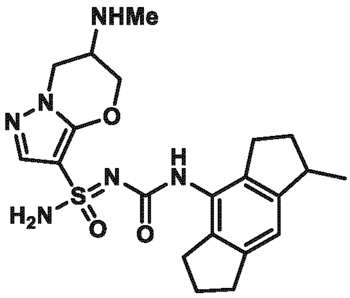 | 648 | 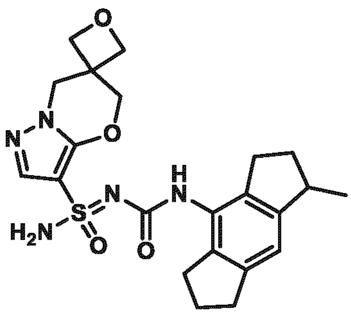 |
| 532 | 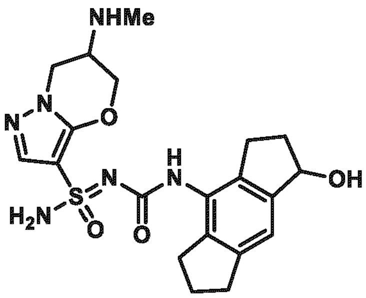 | 649 | 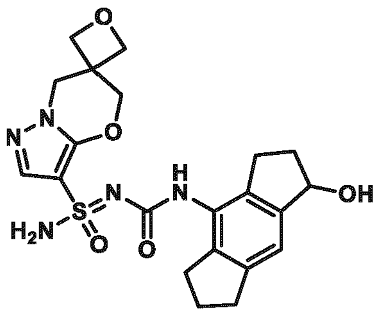 |
| 533 | 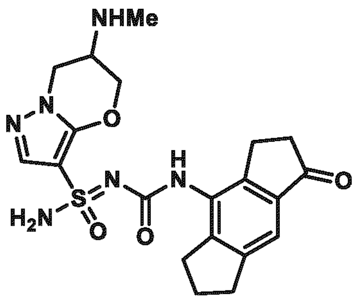 | 650 | 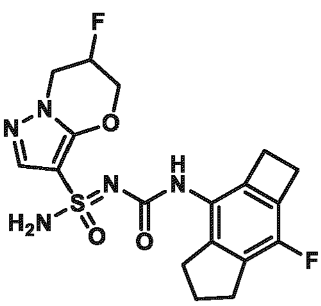 |

(continued)

| | | | |
|---|---|---|---|
| 534 | 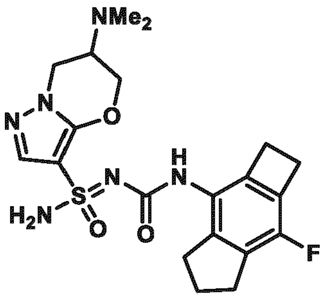 | 651 | 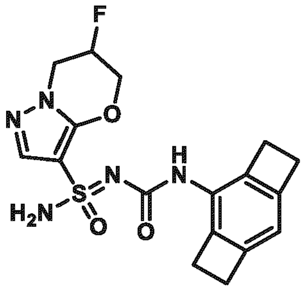 |
| 535 | 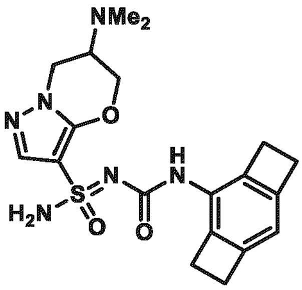 | 652 | 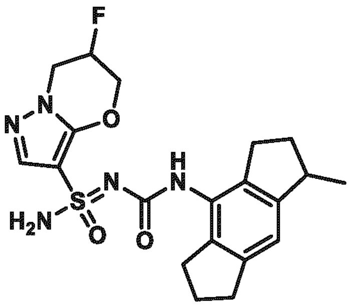 |
| 536 | 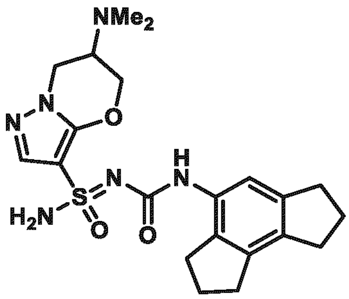 | 653 | 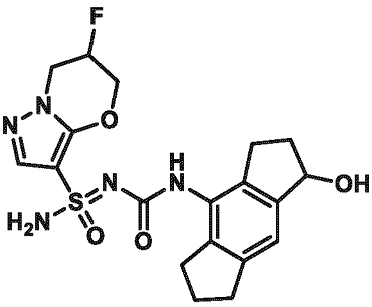 |
| 537 | 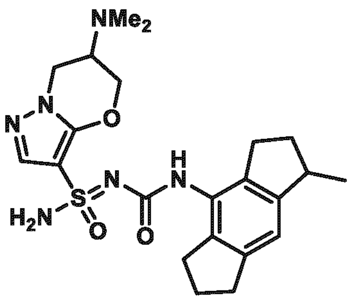 | 654 | 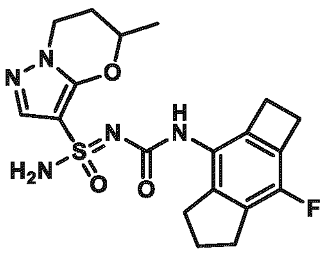 |
| 538 | 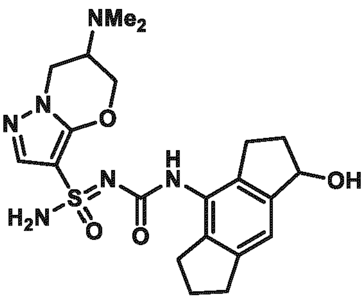 | 655 | 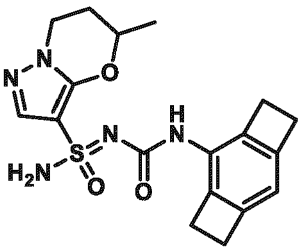 |

(continued)

| | | | |
|---|---|---|---|
| 539 | 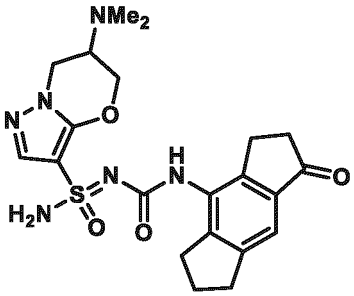 | 656 | 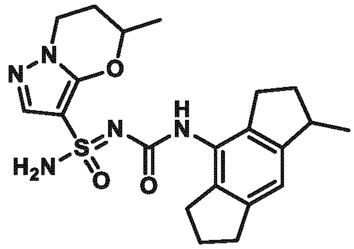 |
| 540 | 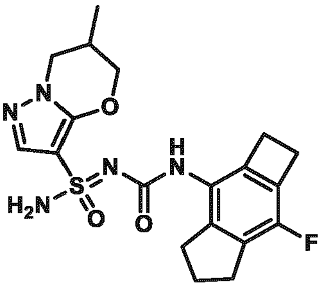 | 657 | 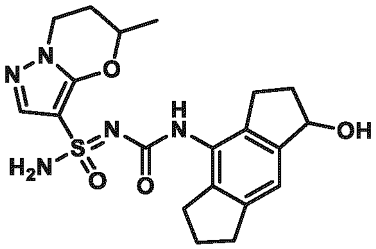 |
| 541 | 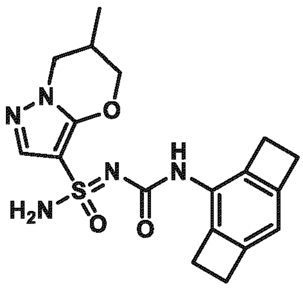 | 658 | 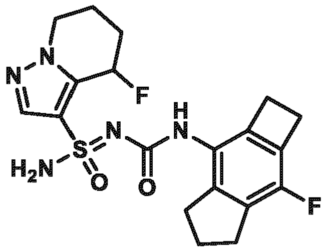 |
| 542 | 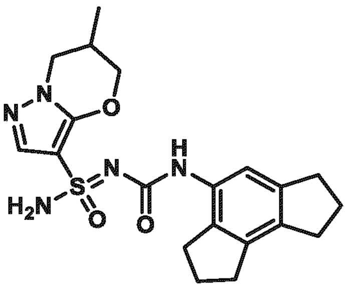 | 659 | 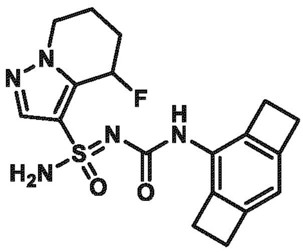 |
| 543 | 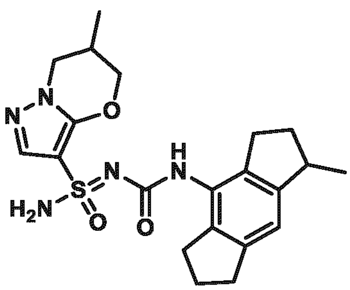 | 660 | 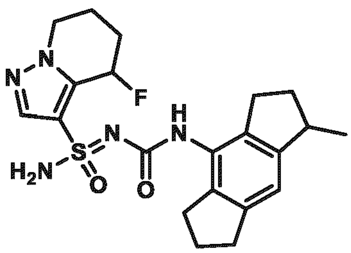 |

(continued)

| | | | |
|---|---|---|---|
| 544 | 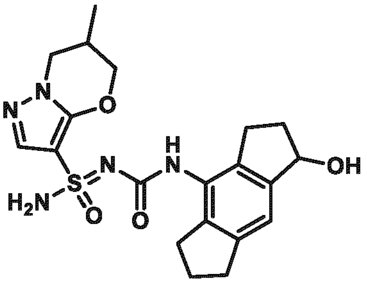 | 661 | 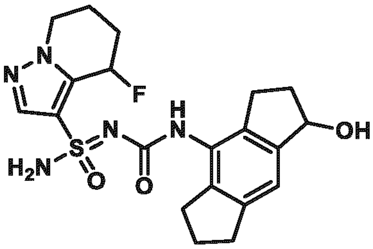 |
| 545 | 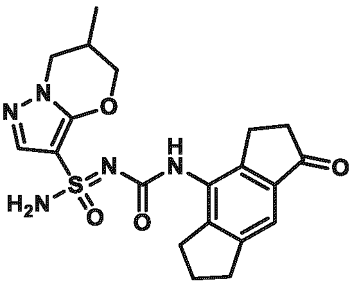 | 662 | 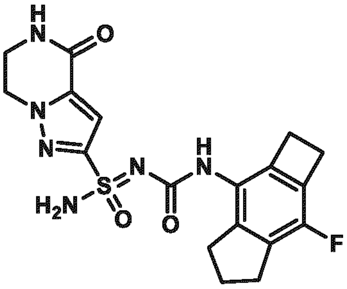 |
| 546 | 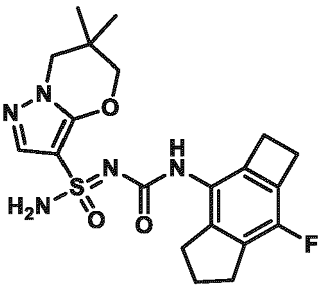 | 663 | 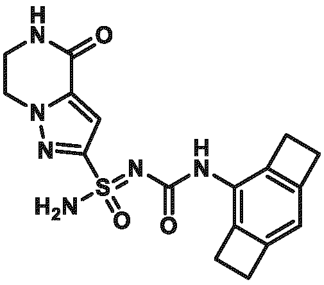 |
| 547 | 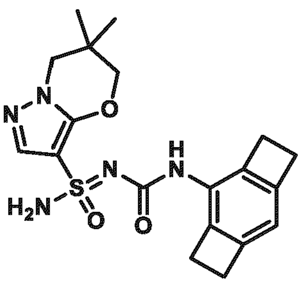 | 664 | 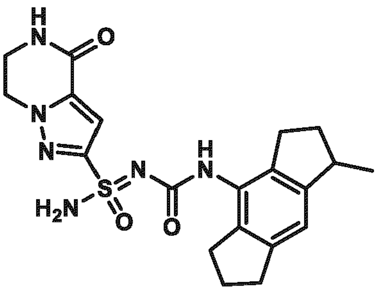 |
| 548 | 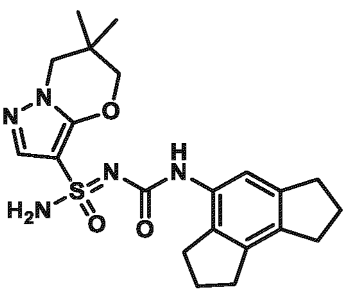 | 665 | 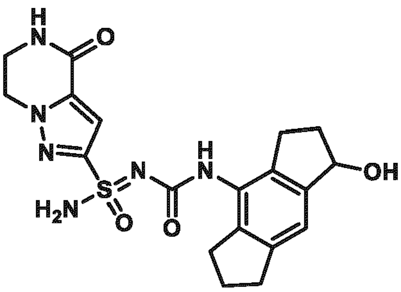 |

(continued)

| | | | |
|---|---|---|---|
| 549 | 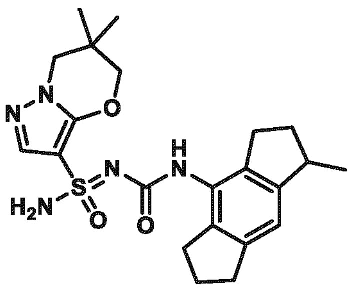 | 666 | 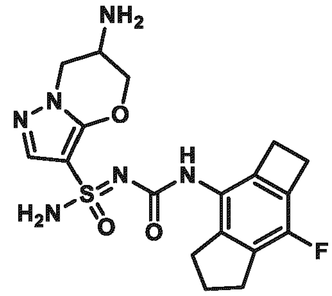 |
| 550 | 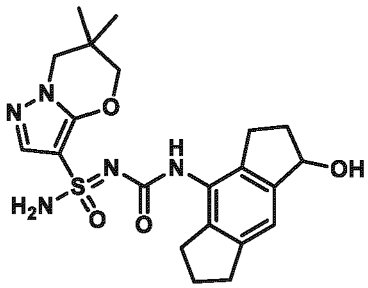 | 667 | 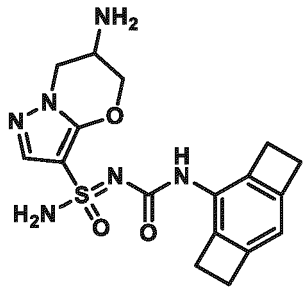 |
| 551 | 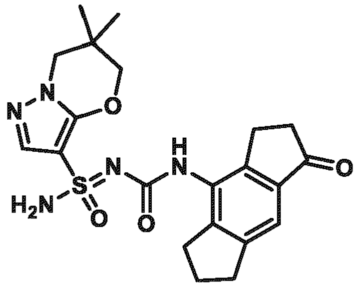 | 668 | 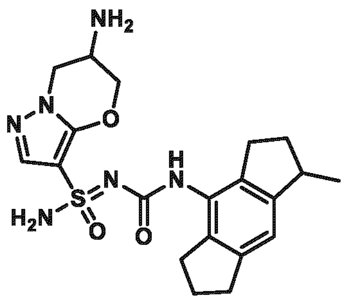 |
| 552 | 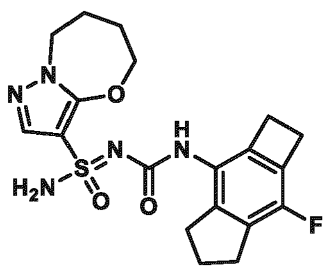 | 669 | 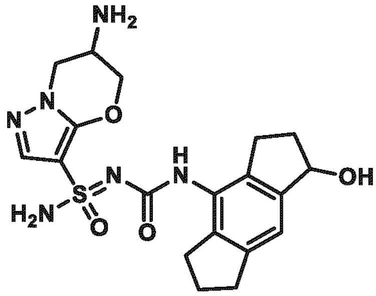 |
| 553 | 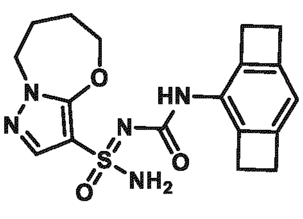 | 670 | 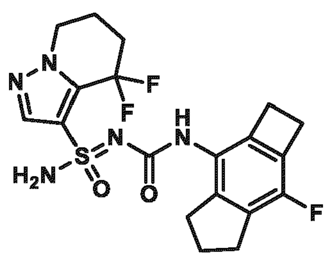 |

(continued)

| | | | |
|---|---|---|---|
| 554 | 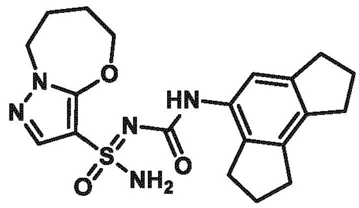 | 671 | 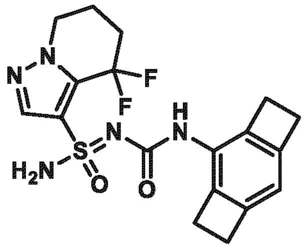 |
| 555 | 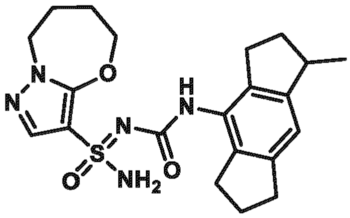 | 672 | 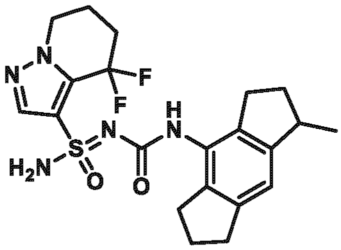 |
| 556 | 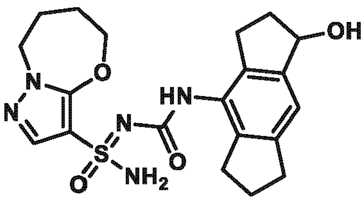 | 673 | 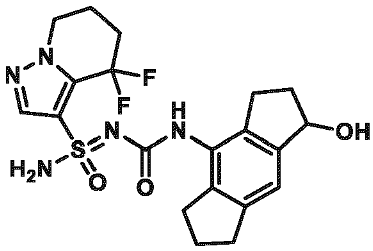 |
| 557 | 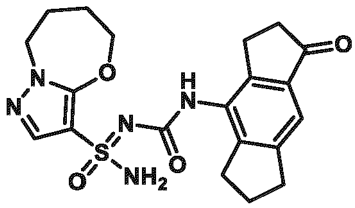 | 674 | 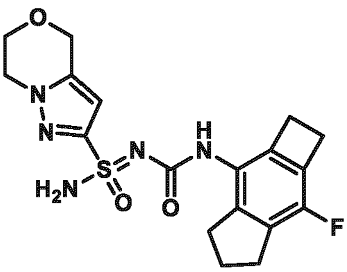 |
| 558 | 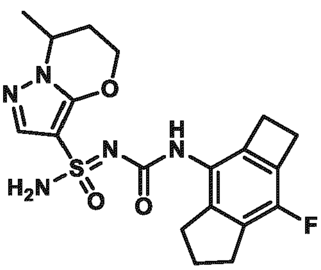 | 675 | 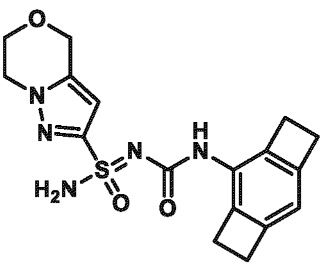 |
| 559 | 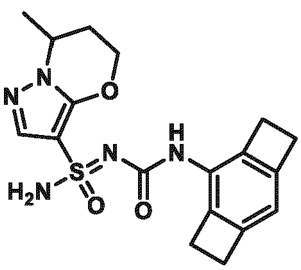 | 676 | 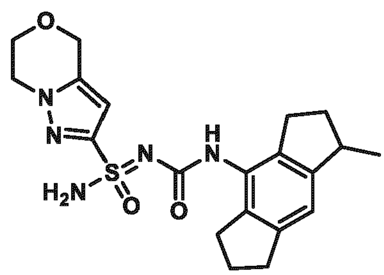 |

(continued)

| | | | |
|---|---|---|---|
| 560 | 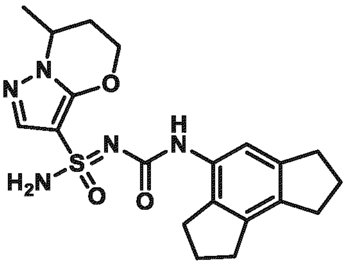 | 677 | 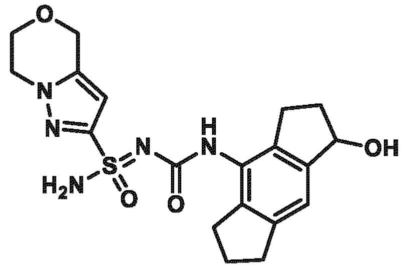 |
| 561 | 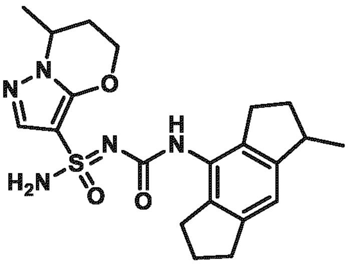 | 678 | 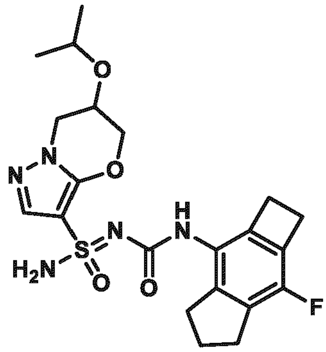 |
| 562 | 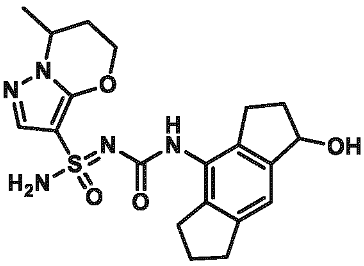 | 679 | 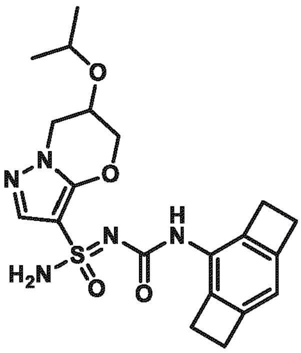 |
| 563 | 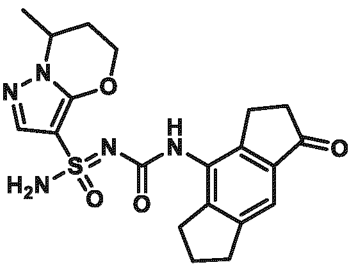 | 680 | 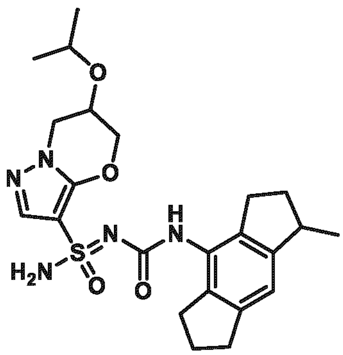 |

(continued)

| | | | |
|---|---|---|---|
| 564 | 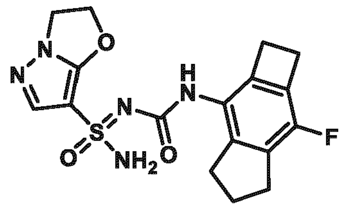 | 681 | 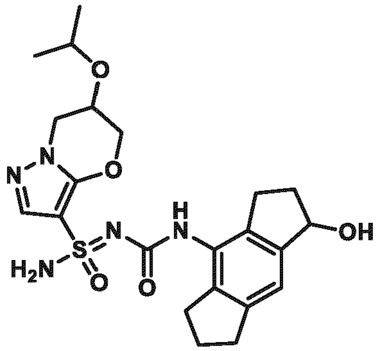 |
| 565 | 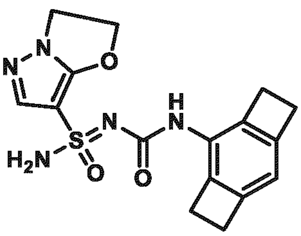 | 682 | 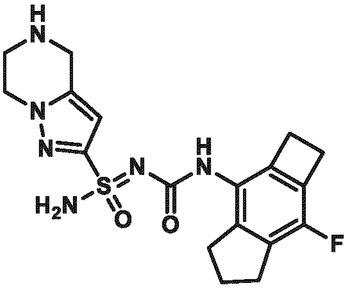 |
| 566 | 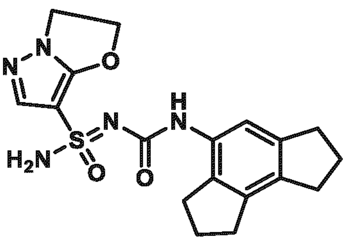 | 683 | 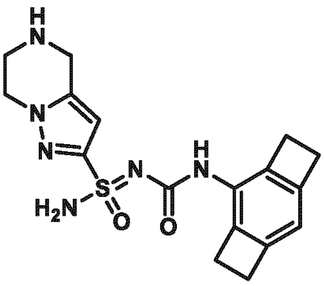 |
| 567 | 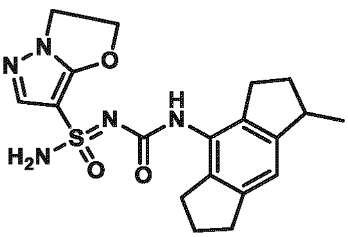 | 684 | 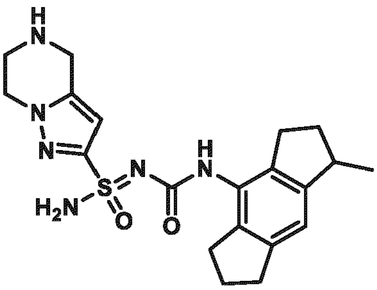 |
| 568 | 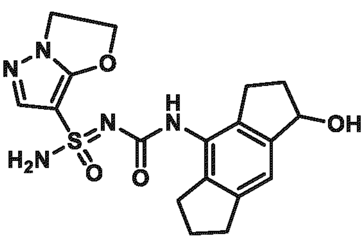 | 685 | 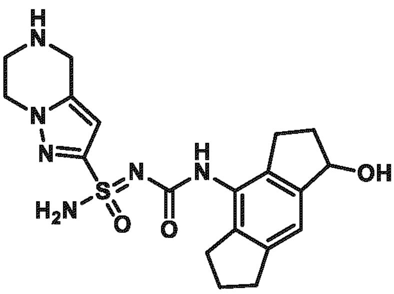 |

(continued)

| | | | |
|---|---|---|---|
| 569 | 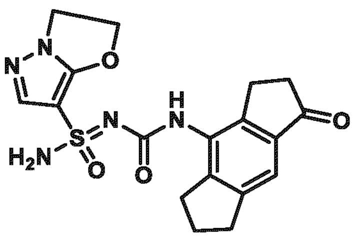 | 686 | 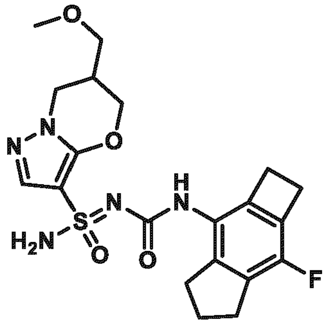 |
| 570 | 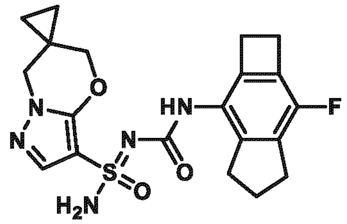 | 687 | 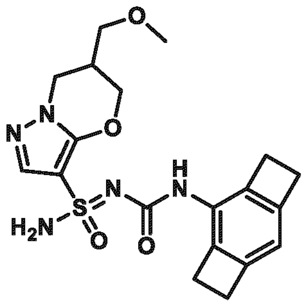 |
| 571 | 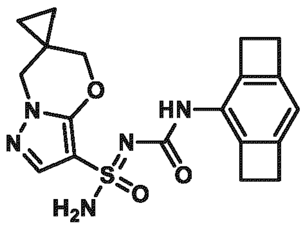 | 688 | 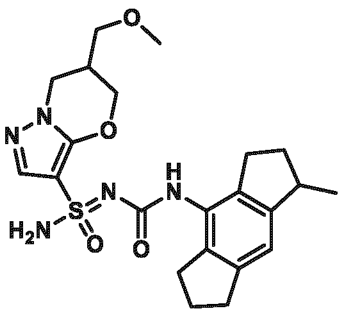 |
| 572 | 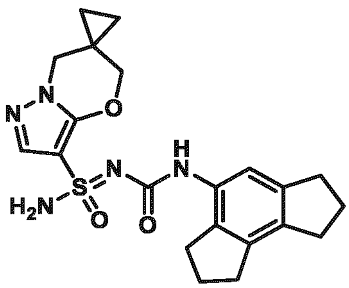 | 689 | 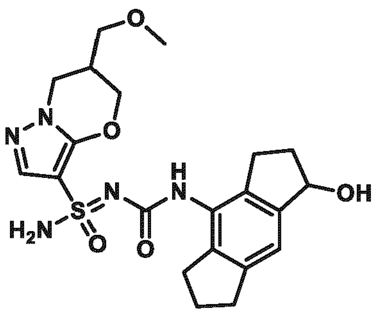 |
| 573 | 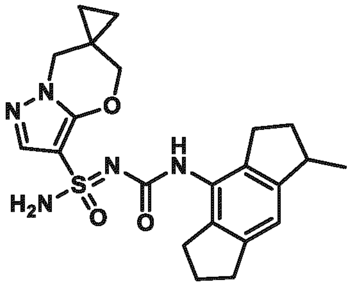 | 690 | 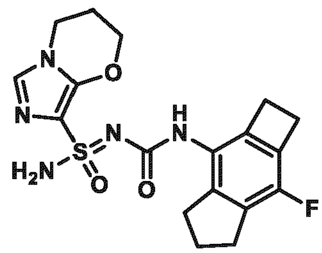 |

(continued)

| | | | |
|---|---|---|---|
| 574 | 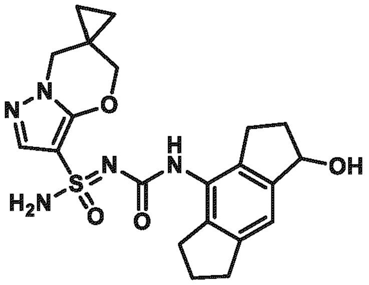 | 691 | 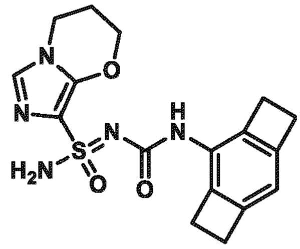 |
| 575 | 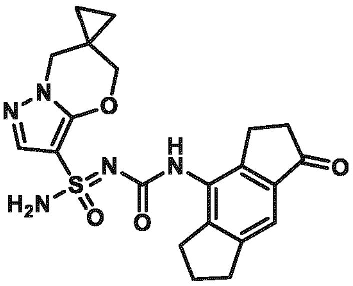 | 692 | 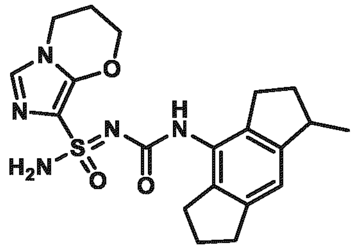 |
| 576 | 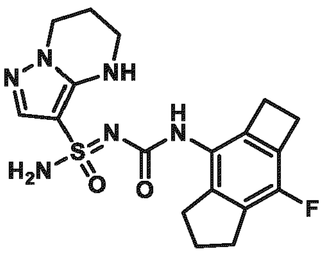 | 693 | 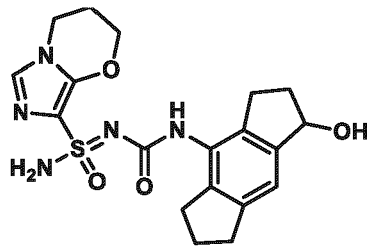 |
| 577 | 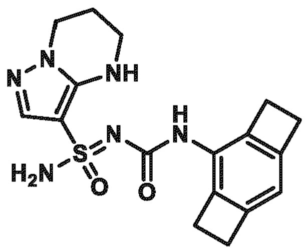 | 694 | 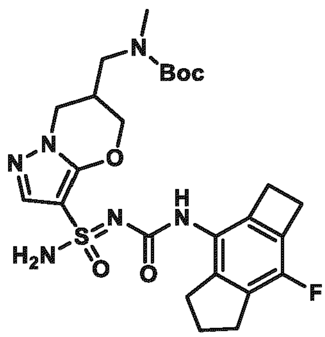 |
| 578 | 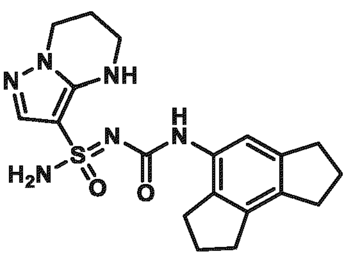 | 695 | 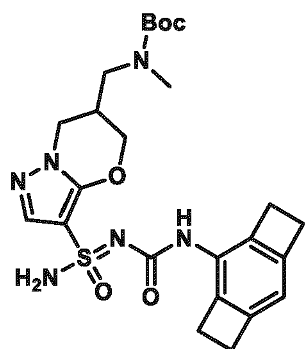 |

(continued)

| | | | |
|---|---|---|---|
| 579 | 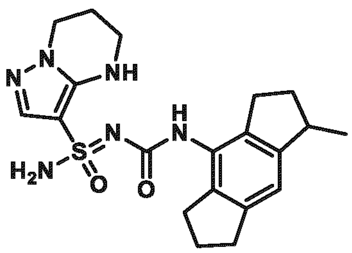 | 696 | 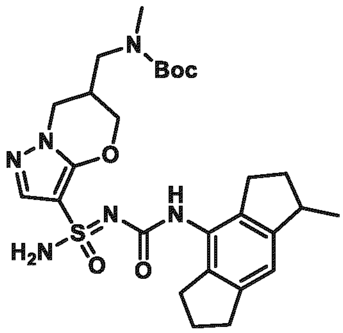 |
| 580 | 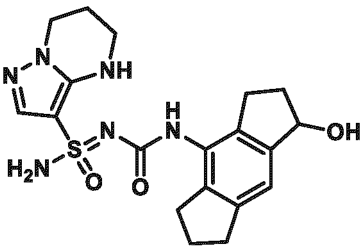 | 697 | 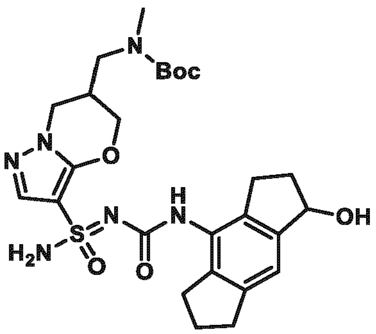 |
| 581 | 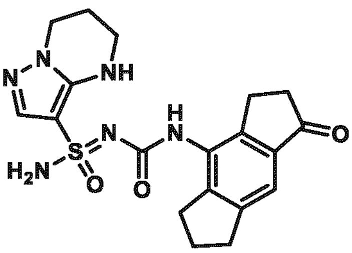 | 698 | 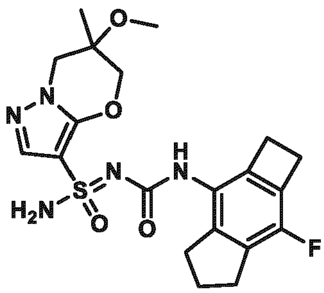 |
| 582 | 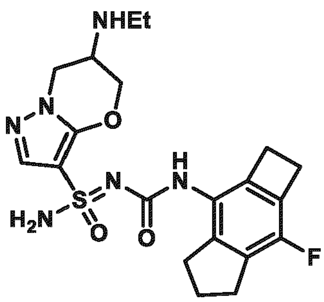 | 699 | 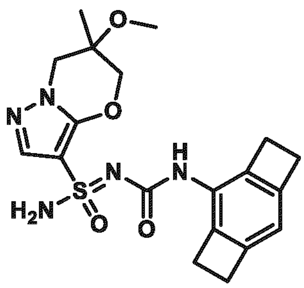 |
| 583 | 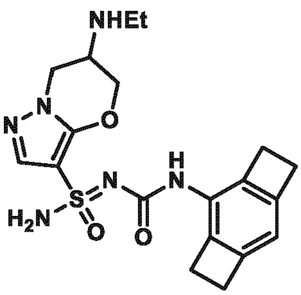 | 700 | 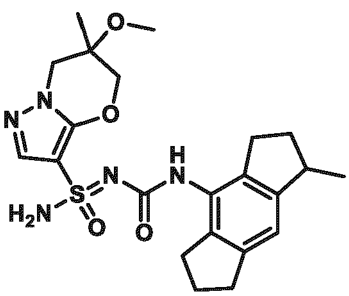 |

(continued)

| | | | |
|---|---|---|---|
| 584 | 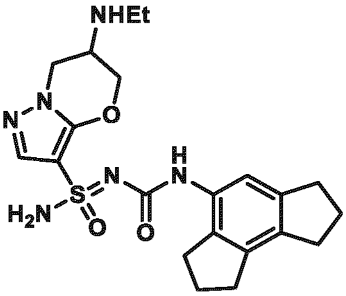 | 701 | 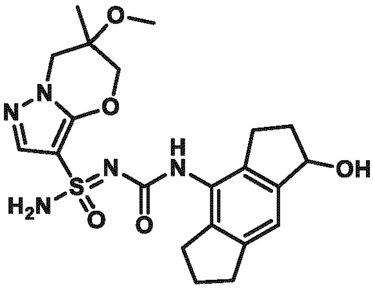 |
| 585 | 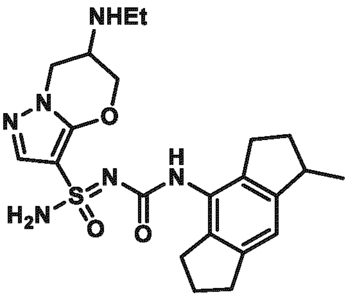 | 702 | 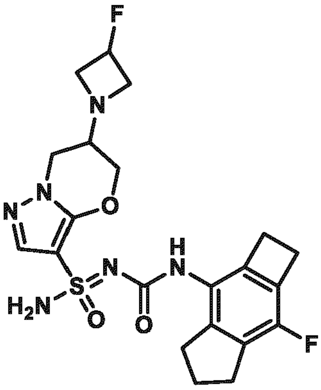 |
| 586 | 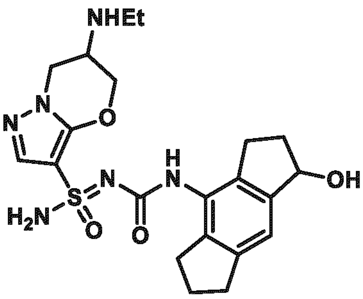 | 703 | 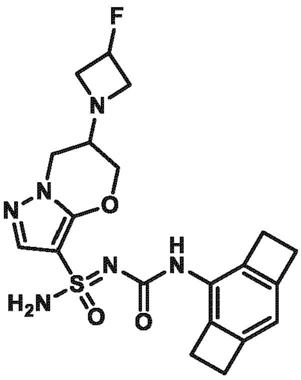 |
| 587 | 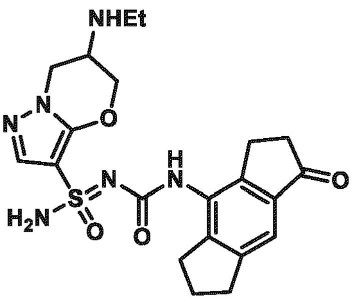 | 704 | 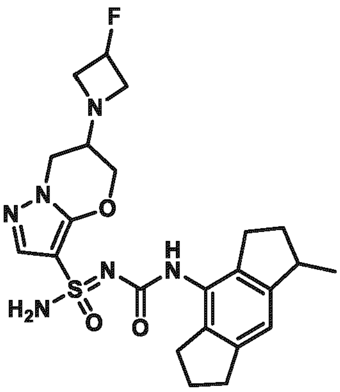 |

(continued)

| | | | |
|---|---|---|---|
| 588 | 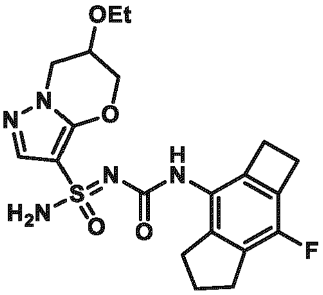 | 705 | 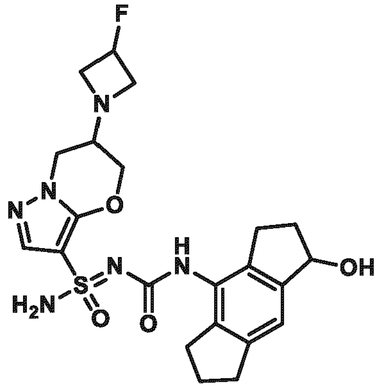 |
| 589 | 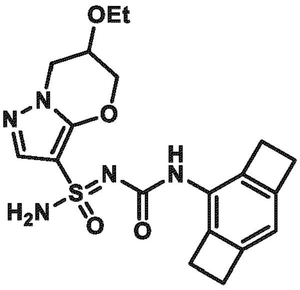 | 706 | 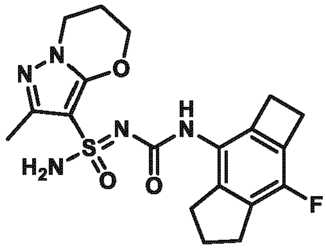 |
| 590 | 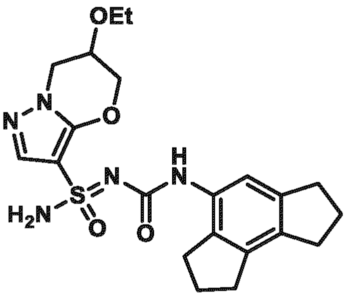 | 707 | 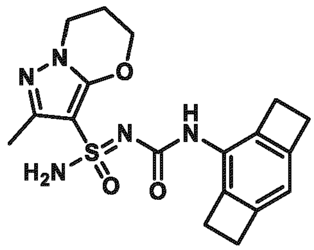 |
| 591 | 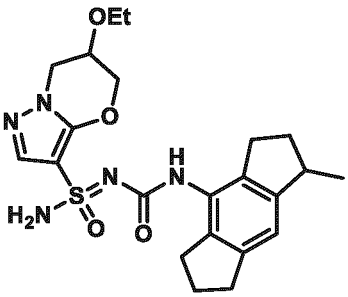 | 708 | 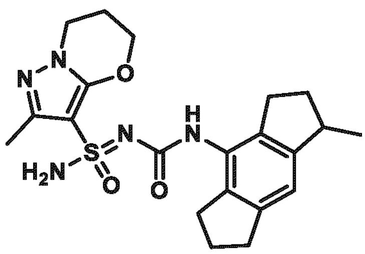 |
| 592 | 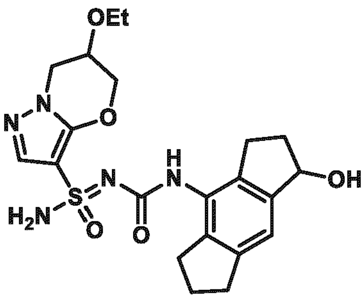 | 709 | 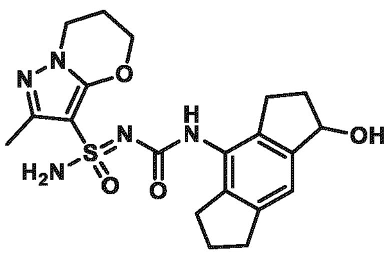 |

(continued)

| | | | |
|---|---|---|---|
| 593 | 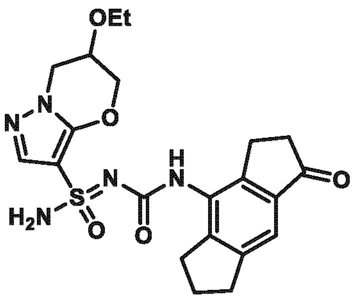 | 710 | 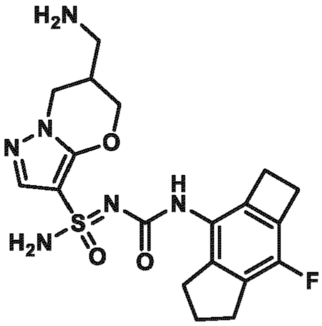 |
| 594 | 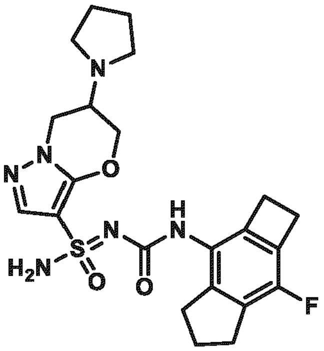 | 711 | 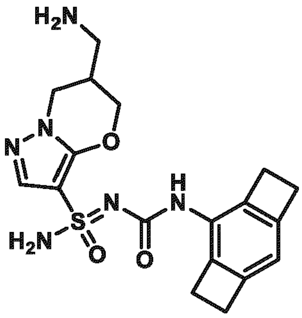 |
| 595 | 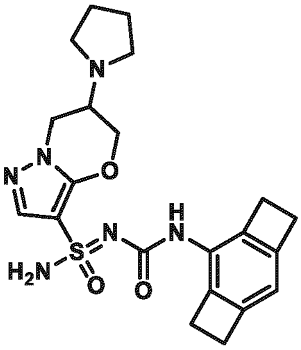 | 712 | 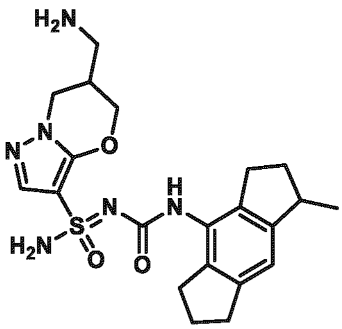 |
| 596 | 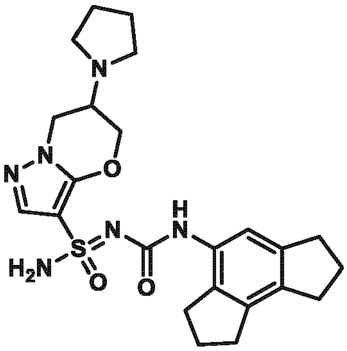 | 713 | 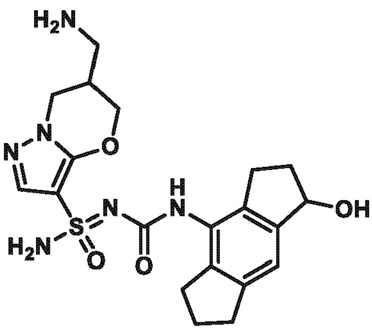 |

(continued)

| | | | |
|---|---|---|---|
| 597 | 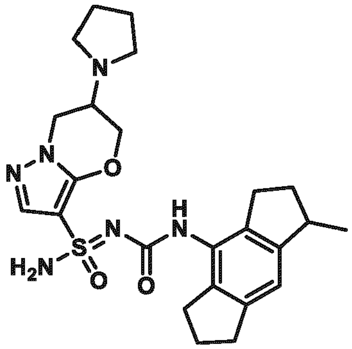 | 714 | 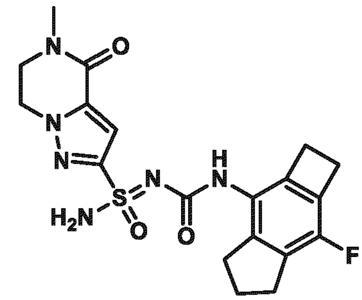 |
| 598 | 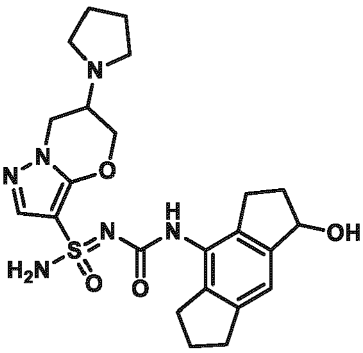 | 715 | 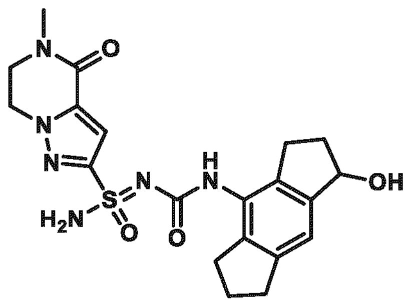 |
| 599 | 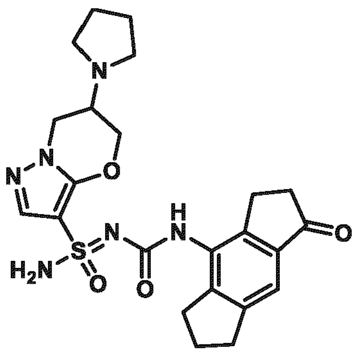 | 716 | 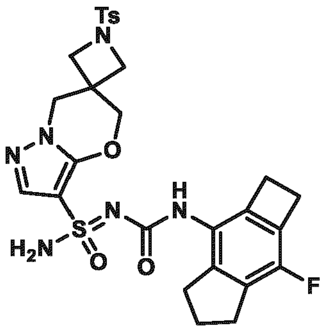 |
| 600 | 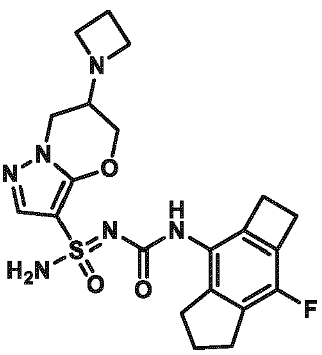 | 717 | 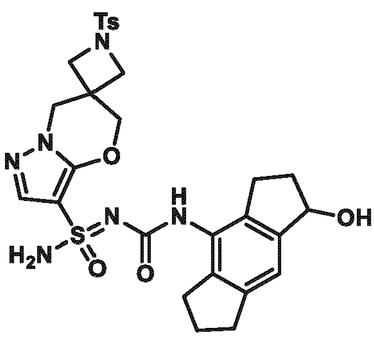 |

(continued)

| | | | |
|---|---|---|---|
| 601 | 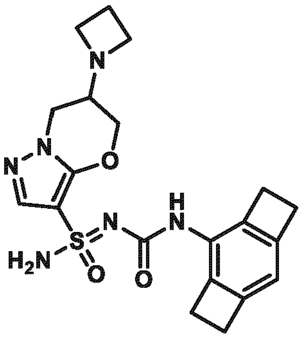 | 718 | 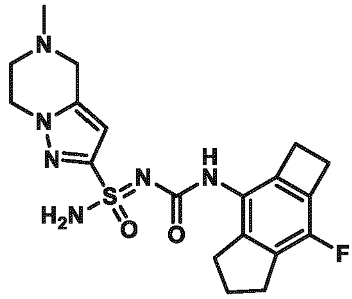 |
| 602 | 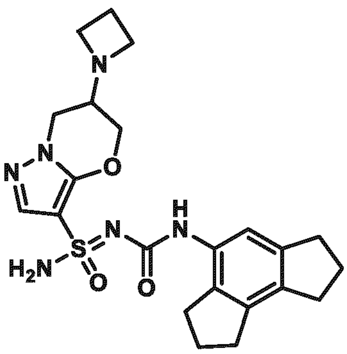 | 719 | 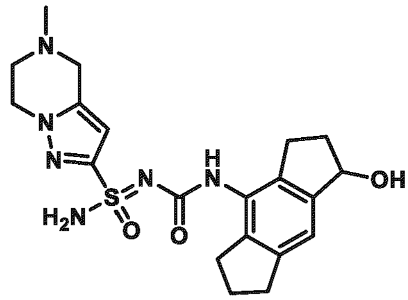 |
| 603 | 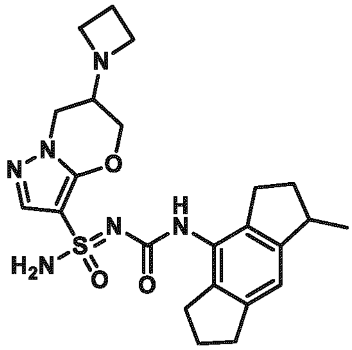 | 720 | 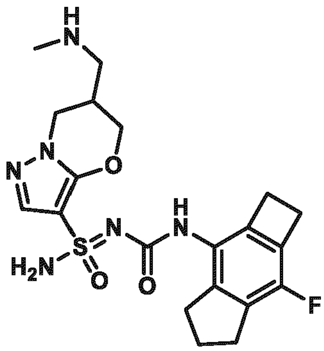 |
| 604 | 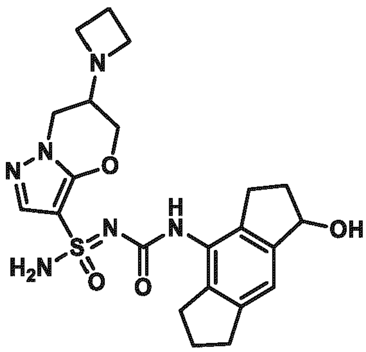 | 721 | 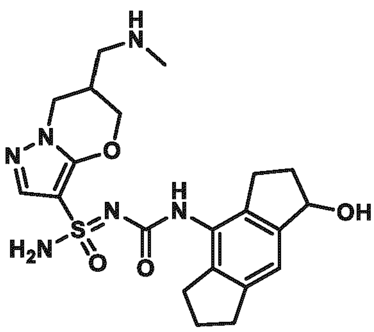 |

(continued)

| | | | |
|---|---|---|---|
| 605 | 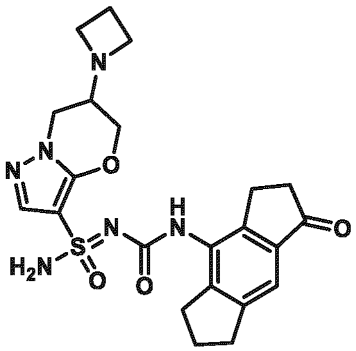 | 722 | 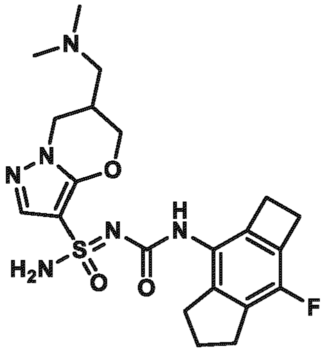 |
| 606 | 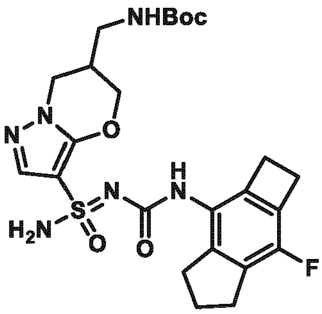 | 723 | 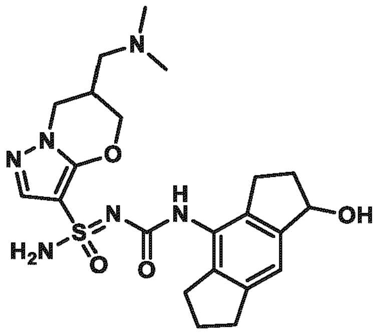 |
| 607 | 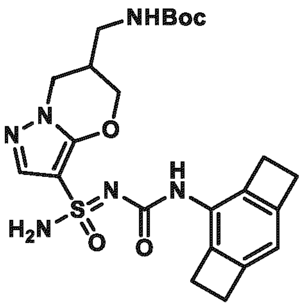 | 724 | 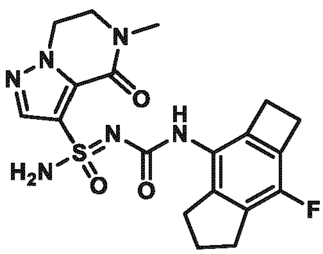 |
| 608 | 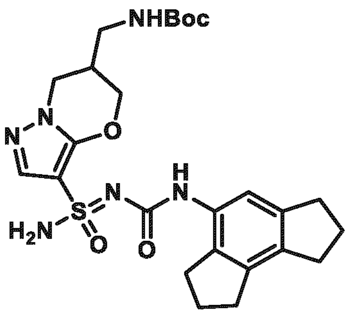 | 725 | 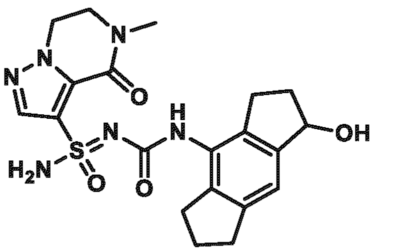 |

(continued)

| | | | |
|---|---|---|---|
| 609 | 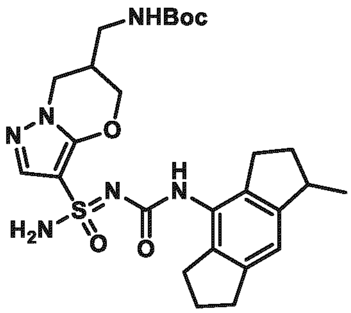 | 726 | 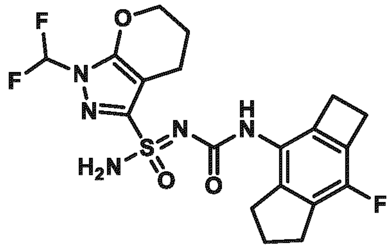 |
| 610 | 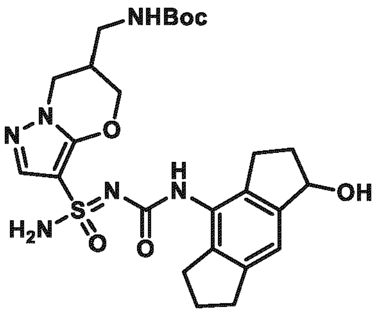 | 727 | 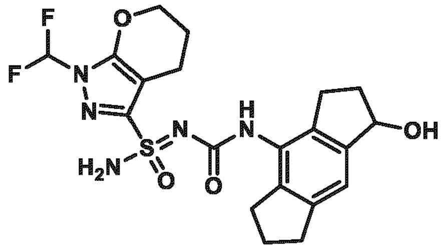 |
| 611 | 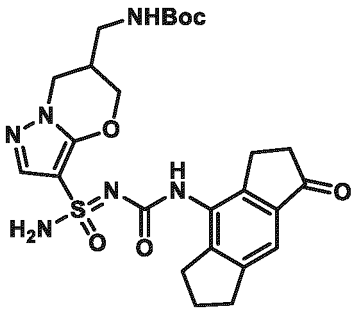 | 728 | 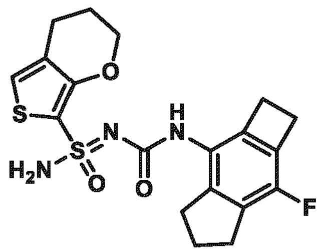 |
| 612 | 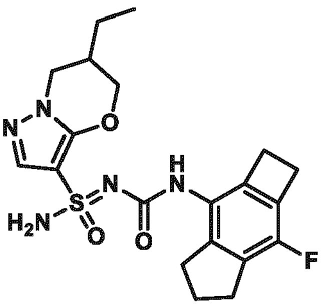 | 729 | 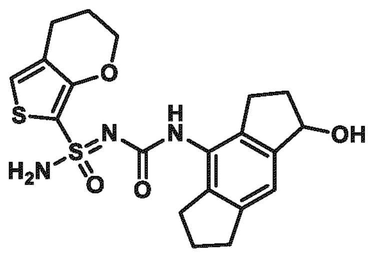 |
| 613 | 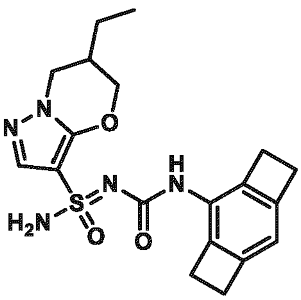 | 730 | 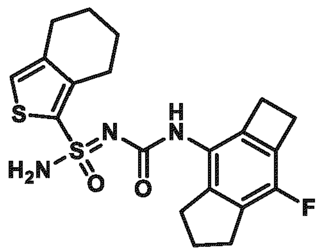 |

(continued)

| | | | |
|---|---|---|---|
| 614 | 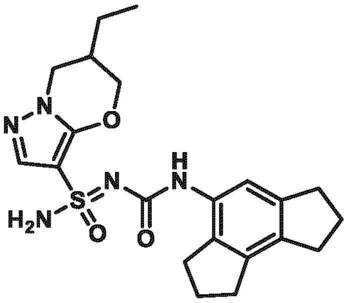 | 731 | 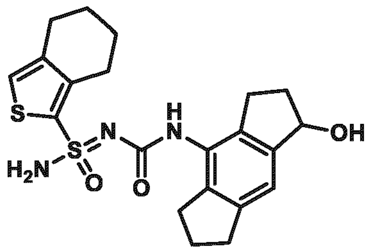 |
| 615 | 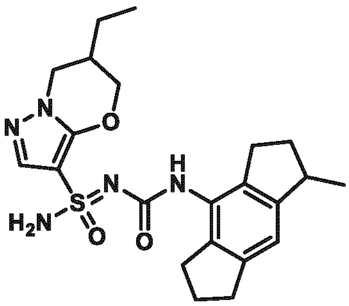 | 732 | 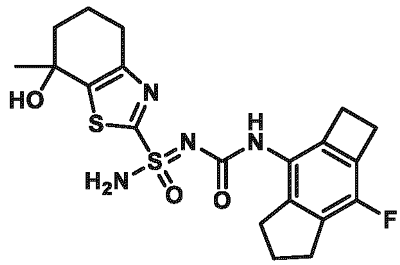 |
| 616 | 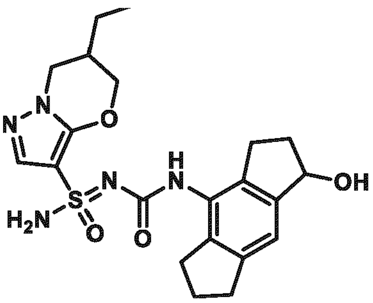 | 733 | 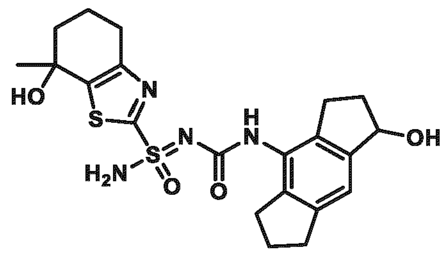 |
| 617 | 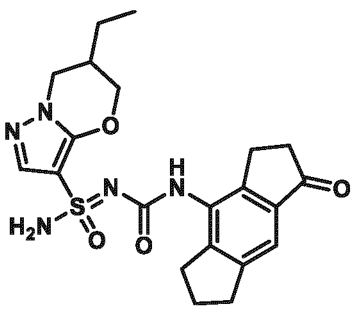 | 734 | 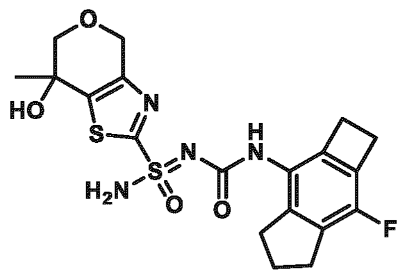 |
| | | 735 | 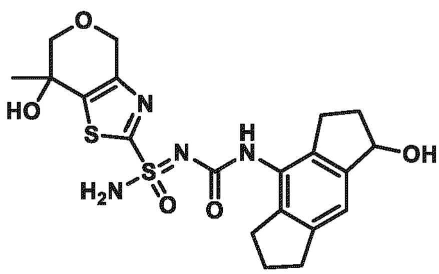 |

or a pharmaceutically acceptable salt thereof.

The invention especially related to a compound mentioned in the preceding paragraph, wherein the sulfur in

the moiety S(=O)($NHR^3$)=N- has (S) stereochemistry.

The invention relates especially to a compound in the second to last paragraph, wherein the sulfur in the moiety S(=O)($NHR^3$)=N- has (R) stereochemistry.

The invention also relates to a pharmaceutical composition comprising a compound or salt as claimed in the preceding three paragraphs and one or more pharmaceutically acceptable excipients.

In some embodiments, provided herein is a compound that is selected from the group consisting of the compounds in the following table:

| | | | | | |
|---|---|---|---|---|---|
| 501 | 1 | 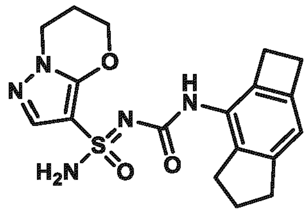 | | | |
| 502 | 2 | 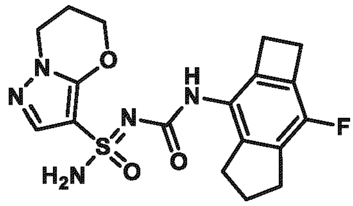 | | | |
| 503 | 3 | 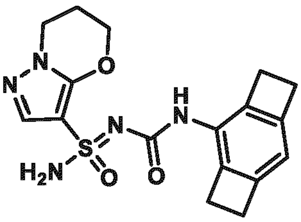 | | | |
| 504 | 4 | 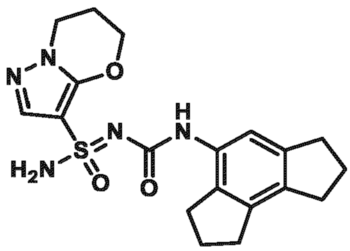 | | | |
| 505 | 5 | 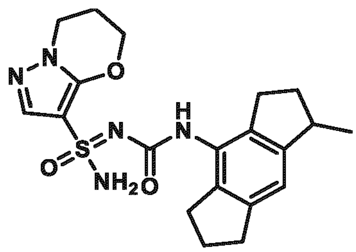 | | | |

(continued)

| | | | | | |
|---|---|---|---|---|---|
| 506 | 6 | 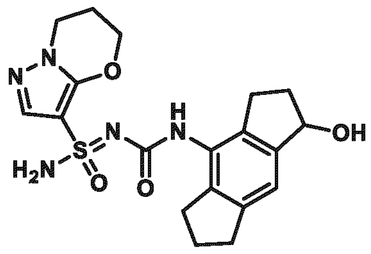 | | | |
| 507 | 7 | 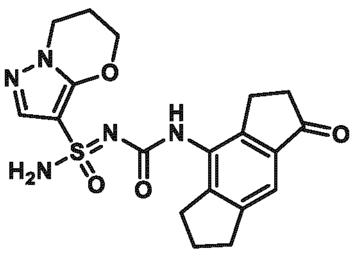 | | | |
| 508 | 8 | 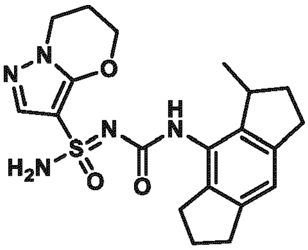 | | | |
| 509 | 9 | 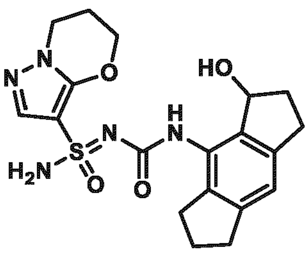 | | | |
| 510 | 10 | 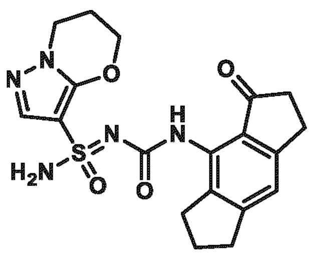 | | | |
| 511 | 11 | 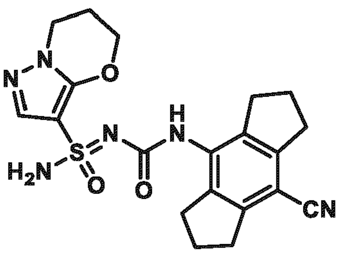 | | | |

(continued)

| | | | | | |
|---|---|---|---|---|---|
| 512 | 12 | 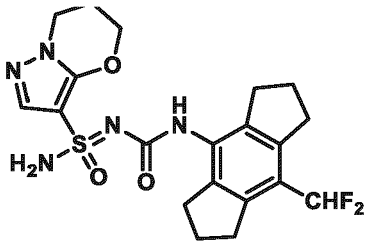 | | | |
| 513 | 13 | 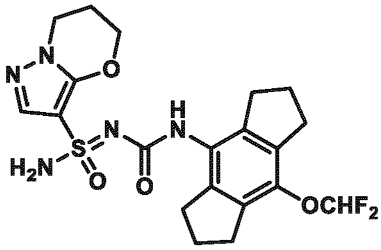 | | | |
| 514 | 14 | 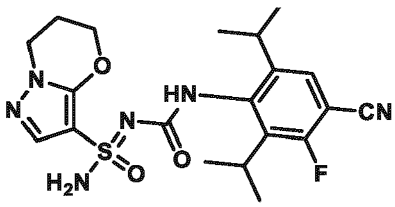 | | | |
| 515 | 15 | 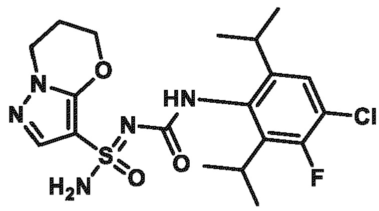 | | | |
| 516 | A1-2 | 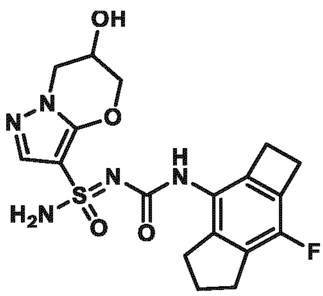 | | | |

(continued)

| | | | | | |
|---|---|---|---|---|---|
| 517 | A1-3 | 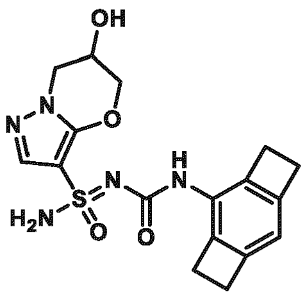 | | | |
| 518 | A1-4 | 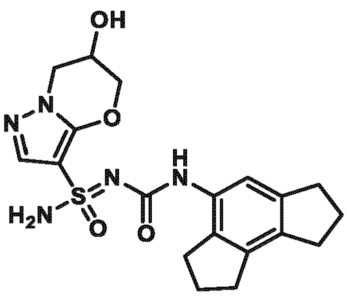 | | | |
| 519 | A1-5 | 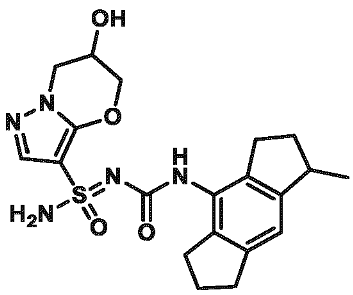 | | | |
| 520 | A1-6 | 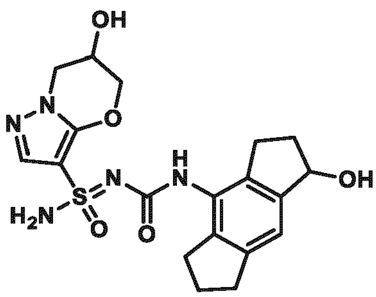 | | | |
| 521 | A1-7 | 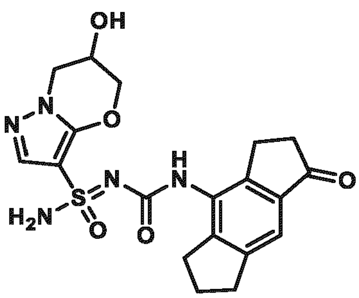 | | | |

(continued)

| | | | | | |
|---|---|---|---|---|---|
| 522 | A2-2 | 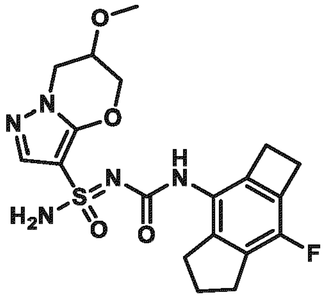 | | | |
| 523 | A2-3 | 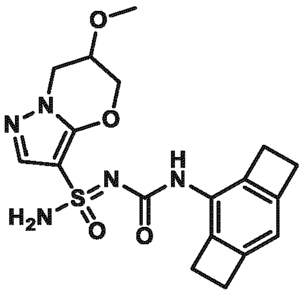 | | | |
| 524 | A2-4 | 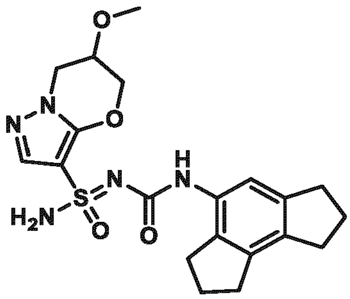 | | | |
| 525 | A2-5 | 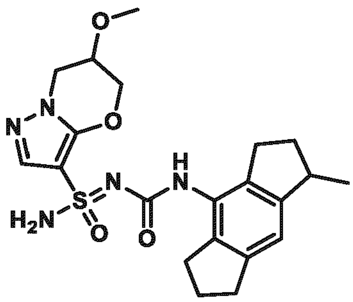 | | | |
| 526 | A2-6 | 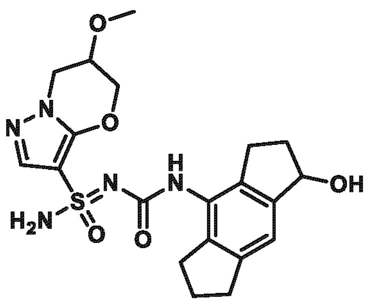 | | | |

(continued)

| | | | | | |
|---|---|---|---|---|---|
| 527 | A2-7 | 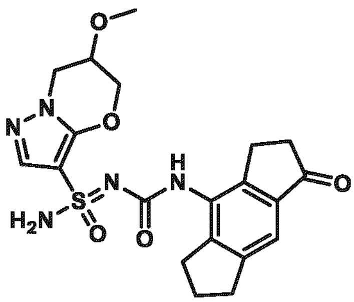 | | | |
| 528 | A3-2 | 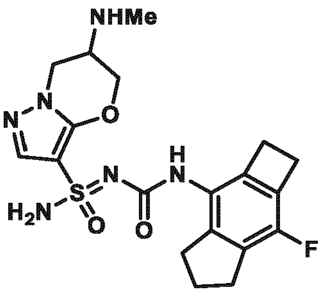 | | | |
| 529 | A3-3 | 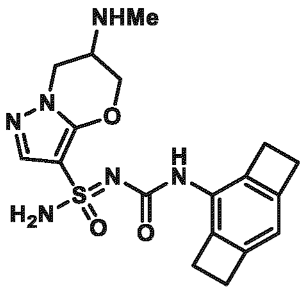 | | | |
| 530 | A3-4 | 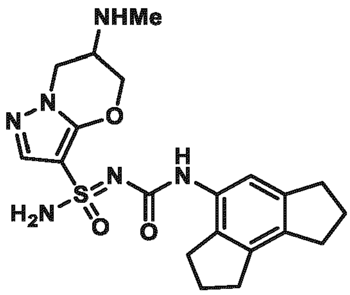 | | | |
| 531 | A3-5 | 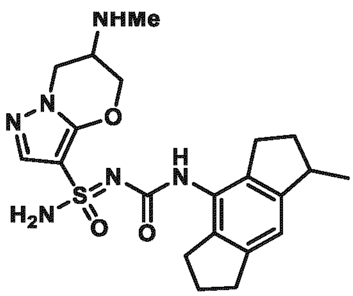 | | | |

(continued)

| | | | | | |
|---|---|---|---|---|---|
| 532 | A3-6 | 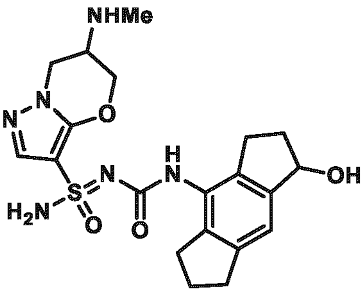 | | | |
| 533 | A3-7 | 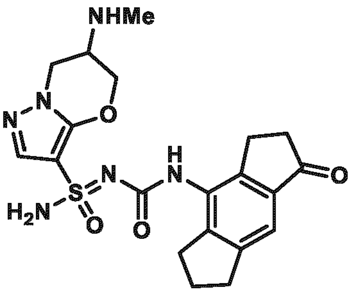 | | | |
| 534 | A4-2 | 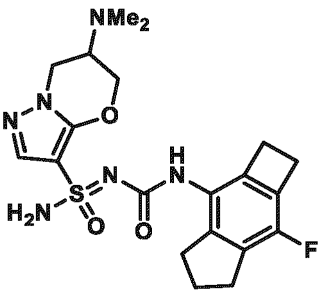 | | | |
| 535 | A4-3 | 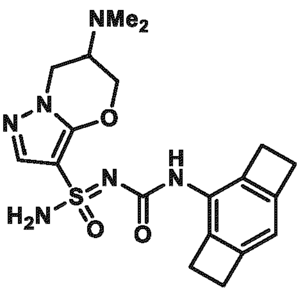 | | | |
| 536 | A4-4 | 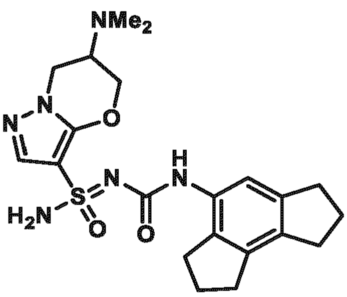 | | | |

(continued)

| | | | | | |
|---|---|---|---|---|---|
| 537 | A4-5 | 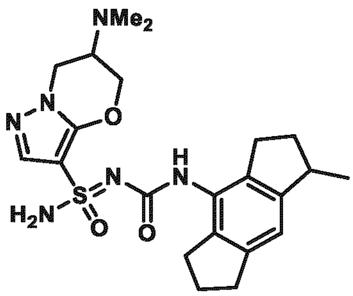 | | | |
| 538 | A4-6 | 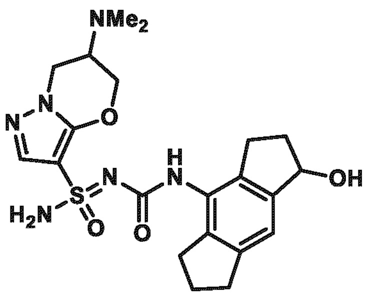 | | | |
| 539 | A4-7 | 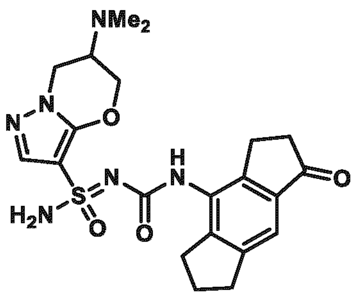 | | | |
| 540 | A5-2 | 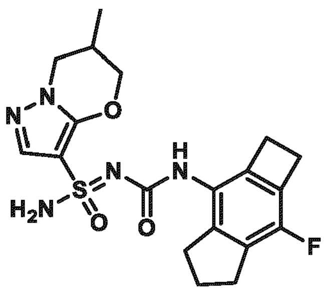 | | | |
| 541 | A5-3 | 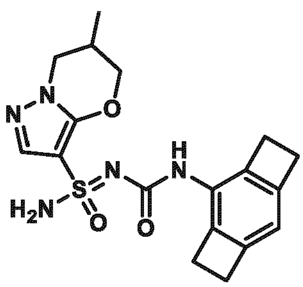 | | | |

(continued)

| | | | | | |
|---|---|---|---|---|---|
| 542 | A5-4 | 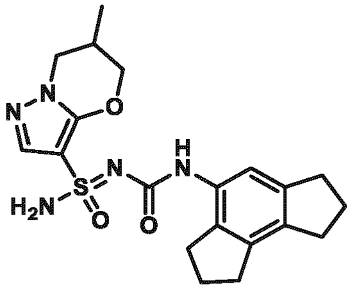 | | | |
| 543 | A5-5 | 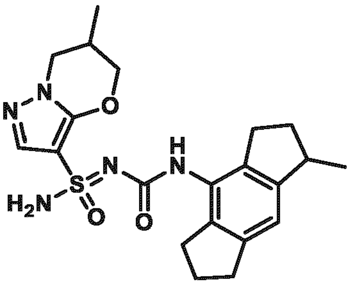 | | | |
| 544 | A5-6 | 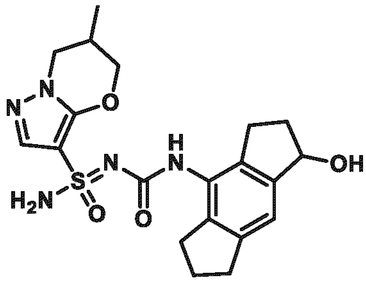 | | | |
| 545 | A5-7 | 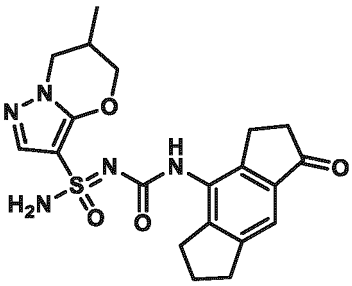 | | | |
| 546 | A6-2 | 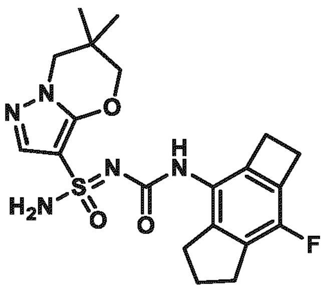 | | | |

(continued)

| | | | | | |
|---|---|---|---|---|---|
| 547 | A6-3 | 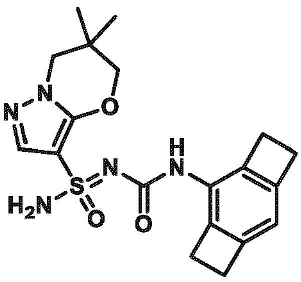 | | | |
| 548 | A6-4 | 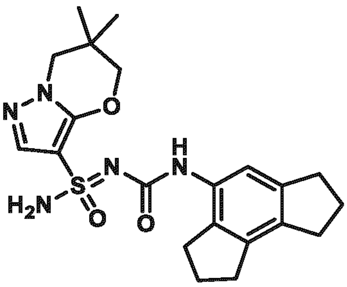 | | | |
| 549 | A6-5 | 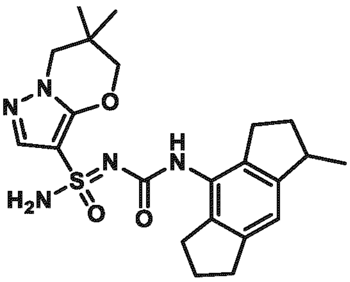 | | | |
| 550 | A6-6 | 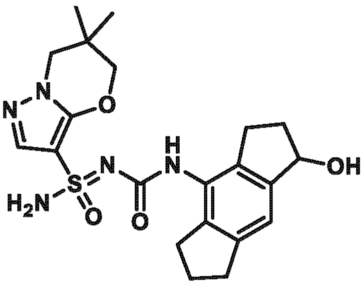 | | | |
| 551 | A6-7 | 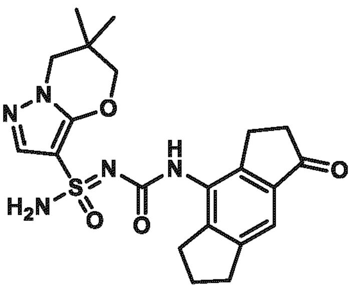 | | | |

(continued)

| | | | | | |
|---|---|---|---|---|---|
| 552 | A7-2 | 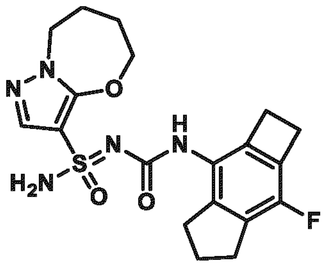 | | | |
| 553 | A7-3 | 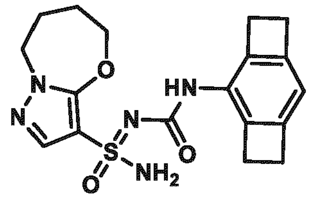 | | | |
| 554 | A7-4 | 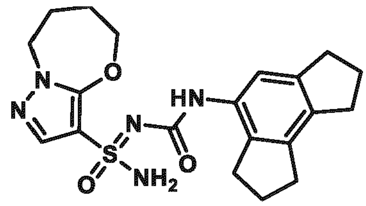 | | | |
| 555 | A7-5 | 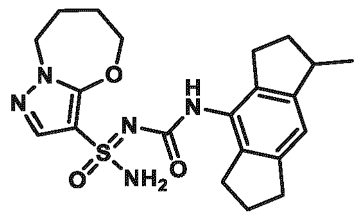 | | | |
| 556 | A7-6 | 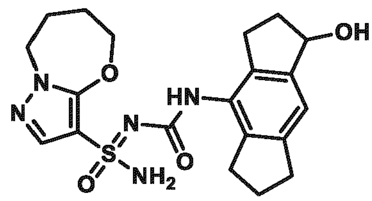 | | | |
| 557 | A7-7 | 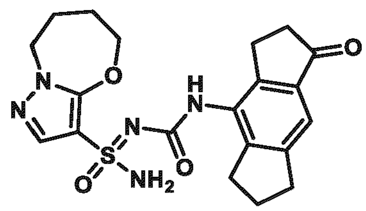 | | | |

(continued)

| | | | | | |
|---|---|---|---|---|---|
| 558 | A8-2 | 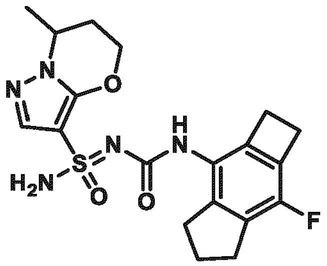 | | | |
| 559 | A8-3 | 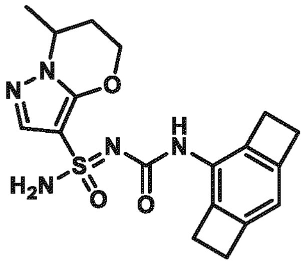 | | | |
| 560 | A8-4 | 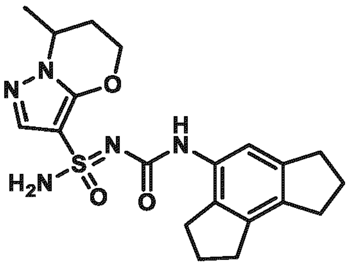 | | | |
| 561 | A8-5 | 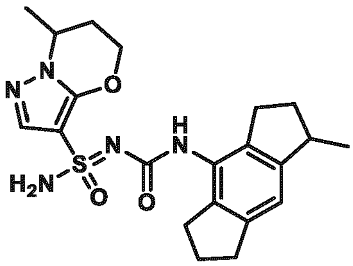 | | | |
| 562 | A8-6 | 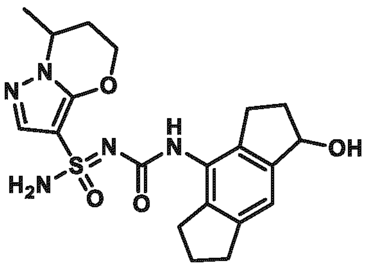 | | | |

(continued)

| | | | | | |
|---|---|---|---|---|---|
| 563 | A8-7 | 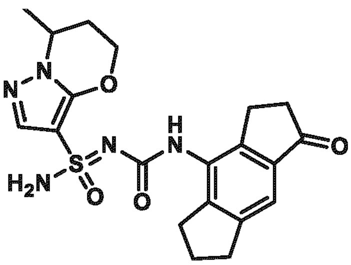 | | | |
| 564 | A9-2 | 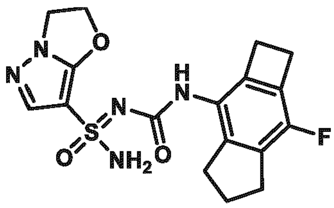 | | | |
| 565 | A9-3 | 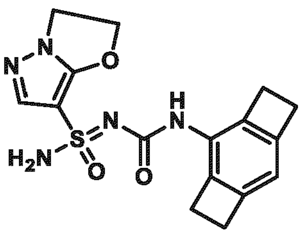 | | | |
| 566 | A9-4 | 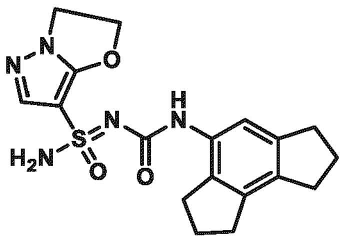 | | | |
| 567 | A9-5 | 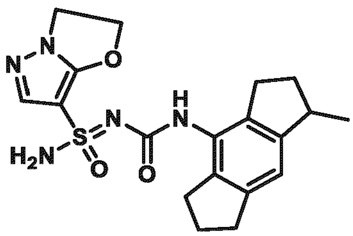 | | | |
| 568 | A9-6 | 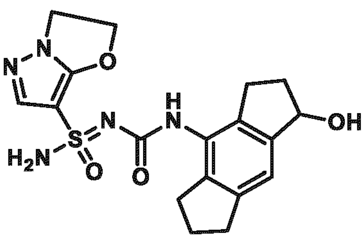 | | | |

(continued)

| | | | | | |
|---|---|---|---|---|---|
| 569 | A9-7 | 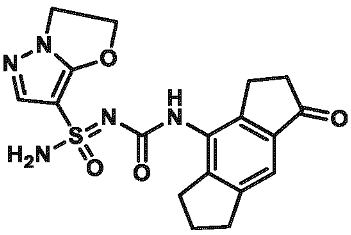 | | | |
| 570 | A10-2 | 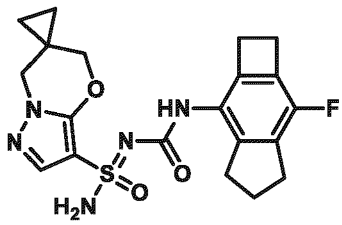 | | | |
| 571 | A10-3 | 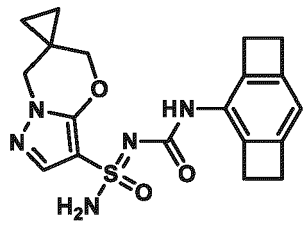 | | | |
| 572 | A10-4 | 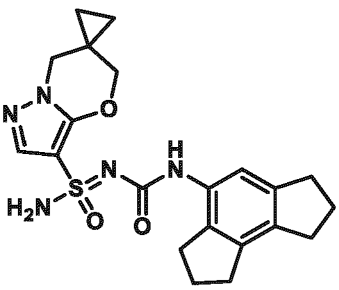 | | | |
| 573 | A10-5 | 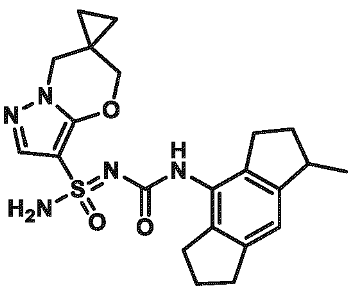 | | | |

(continued)

| | | | | | |
|---|---|---|---|---|---|
| 574 | A10-6 | 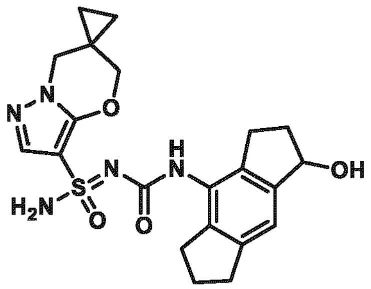 | | | |
| 575 | A10-7 | 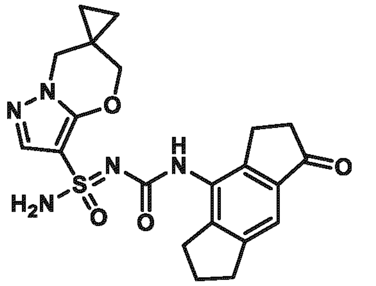 | | | |
| 576 | A11-2 | 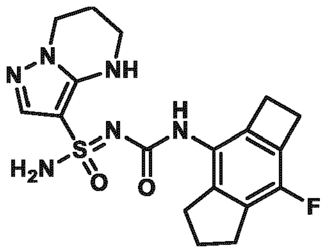 | | | |
| 577 | A11-3 | 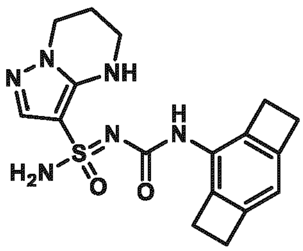 | | | |
| 578 | A11-4 | 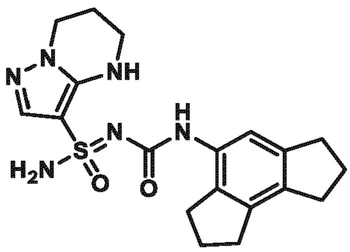 | | | |

(continued)

| | | | | | |
|---|---|---|---|---|---|
| 579 | A11-5 | 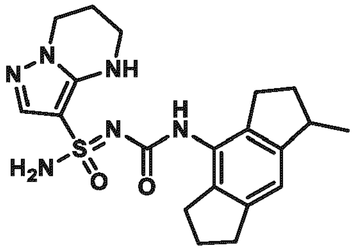 | | | |
| 580 | A11-6 | 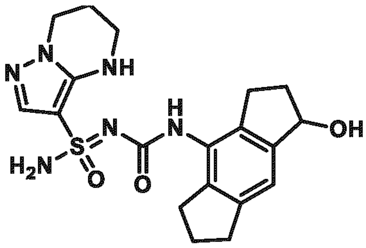 | | | |
| 581 | A11-7 | 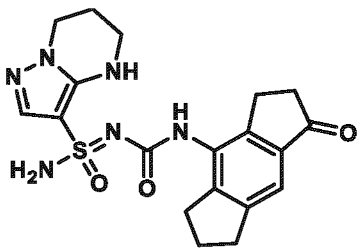 | | | |
| 582 | A12-2 | 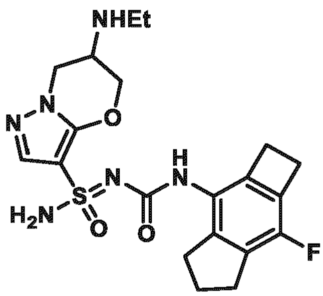 | | | |
| 583 | A12-3 | 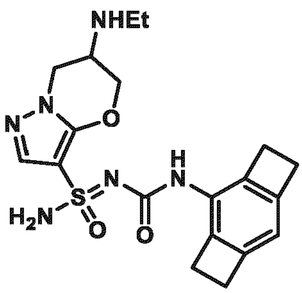 | | | |

(continued)

| | | | | | |
|---|---|---|---|---|---|
| 584 | A12-4 | 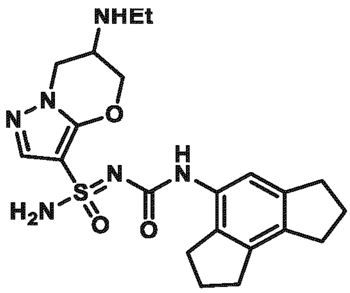 | | | |
| 585 | A12-5 | 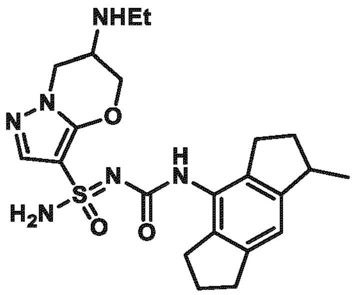 | | | |
| 586 | A12-6 | 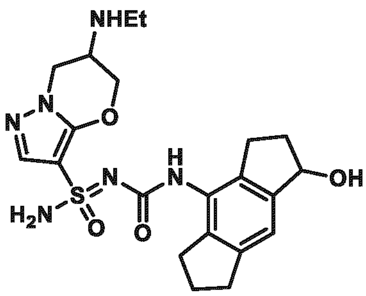 | | | |
| 587 | A12-7 | 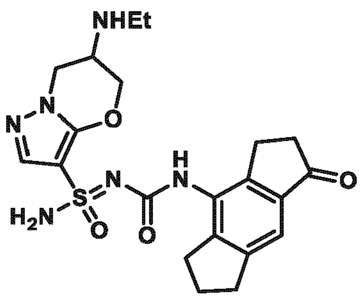 | | | |
| 588 | A13-2 | 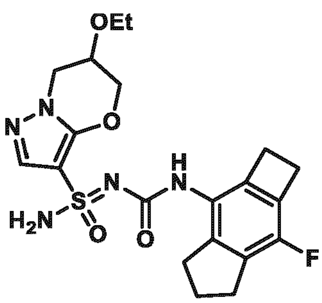 | | | |

(continued)

| | | | | | |
|---|---|---|---|---|---|
| 589 | A13-3 | 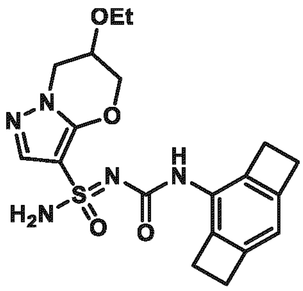 | | | |
| 590 | A13-4 | 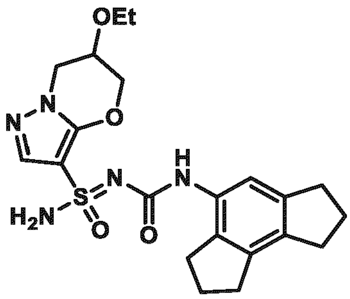 | | | |
| 591 | A13-5 | 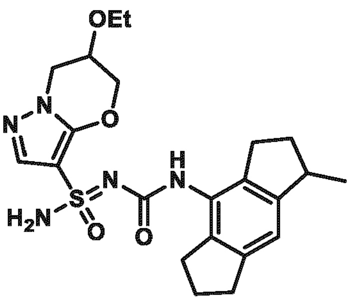 | | | |
| 592 | A13-6 | 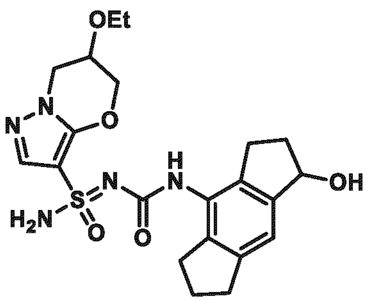 | | | |
| 593 | A13-7 | 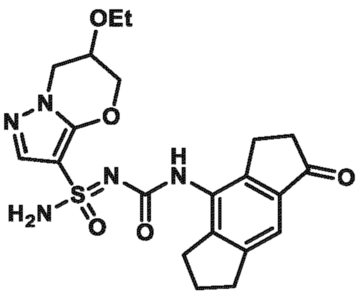 | | | |

(continued)

| | | | | | |
|---|---|---|---|---|---|
| 594 | A14-2 | 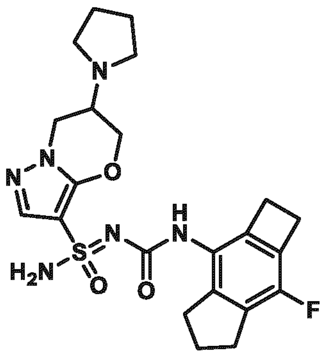 | | | |
| 595 | A14-3 | 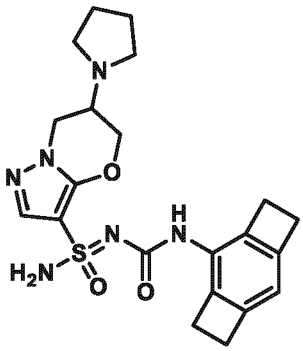 | | | |
| 596 | A14-4 | 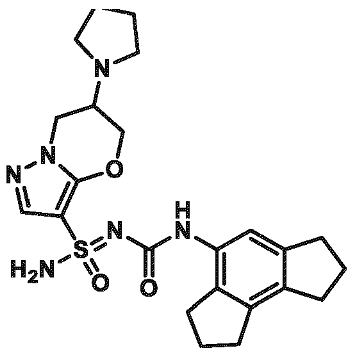 | | | |
| 597 | A14-5 | 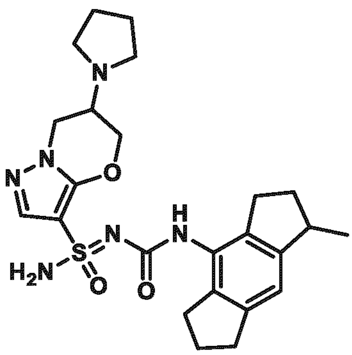 | | | |

(continued)

| | | | | | |
|---|---|---|---|---|---|
| 598 | A14-6 | 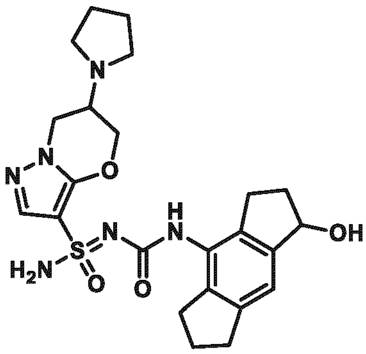 | | | |
| 599 | A14-7 | 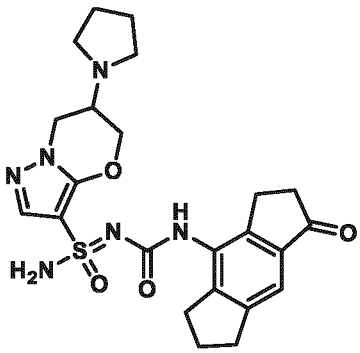 | | | |
| 600 | A15-2 | 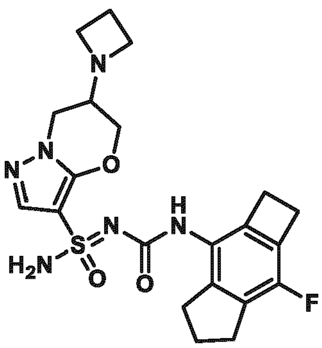 | | | |
| 601 | A15-3 | 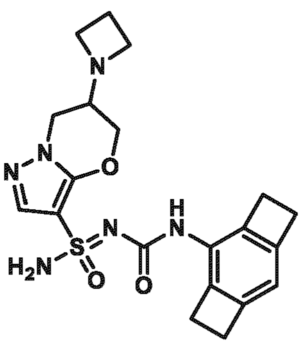 | | | |

(continued)

| | | | | | |
|---|---|---|---|---|---|
| 602 | A15-4 | 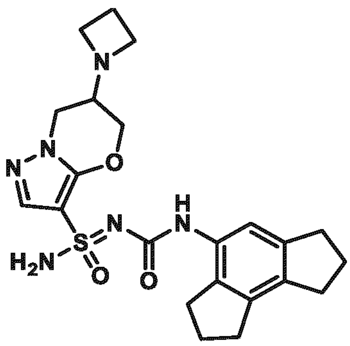 | | | |
| 603 | A15-5 | 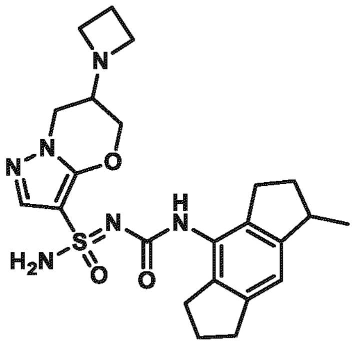 | | | |
| 604 | A15-6 | 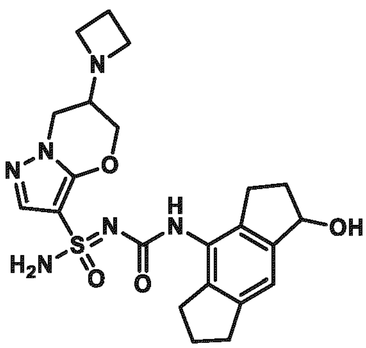 | | | |
| 605 | A15-7 | 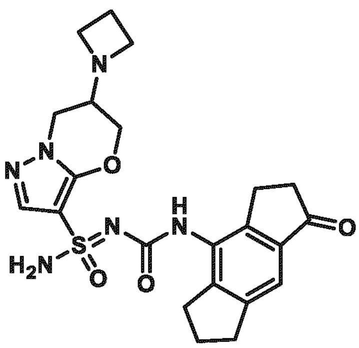 | | | |

(continued)

| | | | | | |
|---|---|---|---|---|---|
| 606 | A16-2 | 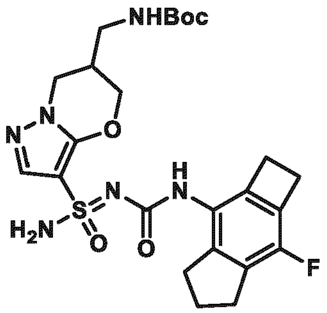 | | | |
| 607 | A16-3 | 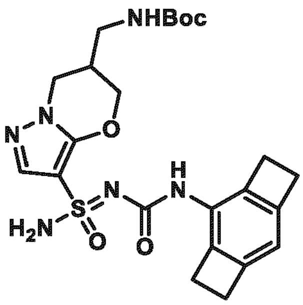 | | | |
| 608 | A16-4 | 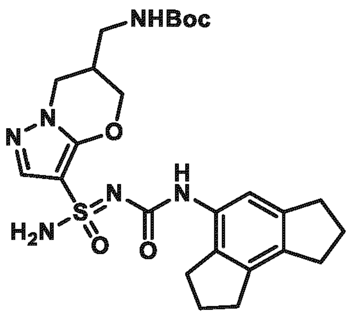 | | | |
| 609 | A16-5 | 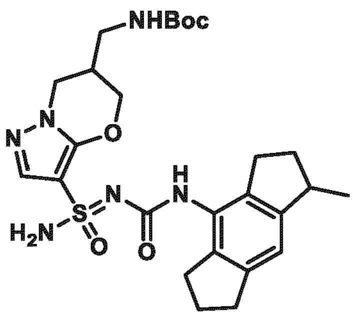 | | | |
| 610 | A16-6 | 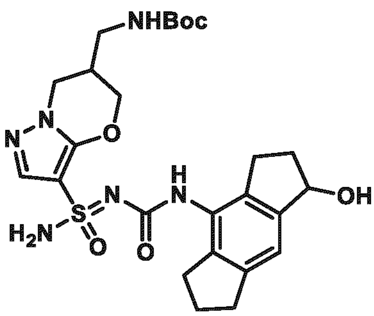 | | | |

(continued)

| | | | | | |
|---|---|---|---|---|---|
| 611 | A16-7 | 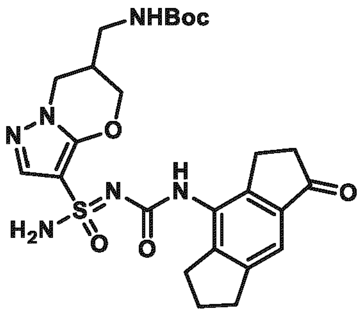 | | | |
| 612 | A17-2 | 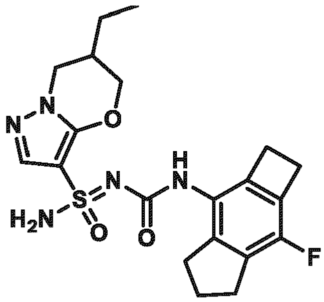 | | | |
| 613 | A17-3 | 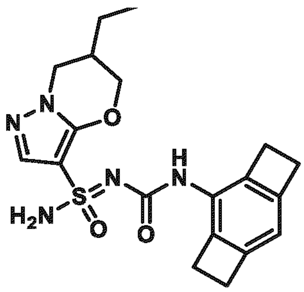 | | | |
| 614 | A17-4 | 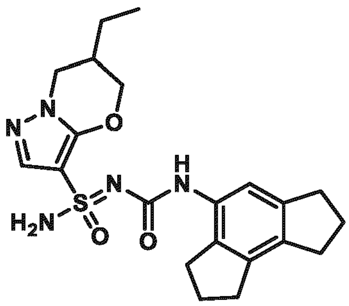 | | | |
| 615 | A17-5 | 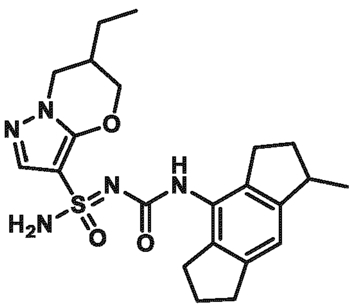 | | | |

(continued)

| | | | | | |
|---|---|---|---|---|---|
| 616 | A17-6 | 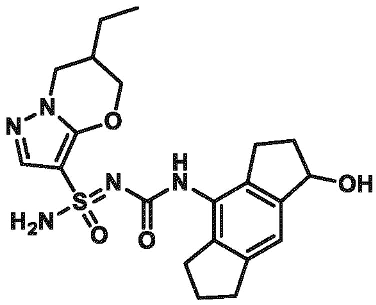 | | | |
| 617 | A17-7 | 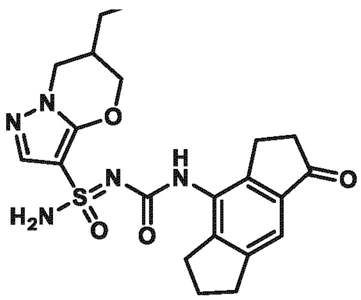 | | | |
| 618 | A18-2 | 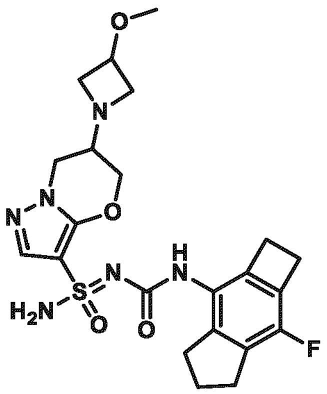 | | | |

## Pharmaceutical Compositions and Administration

*General*

In some embodiments, a chemical entity, i.e.., a compound that modulates (e.g., antagonizes) NLRP3 as defined in claim 1, claim 2 or claim 3, or a pharmaceutically acceptable salt, and thereof is formulated for administration as a pharmaceutical composition that includes the chemical entity and one or more pharmaceutically acceptable excipients, and optionally one or more additional therapeutic agents as described herein.

In some embodiments, the chemical entities as claimed are formulated for administration in combination with one or more conventional pharmaceutical excipients. Pharmaceutically acceptable excipients include, but are not limited to, ion exchangers, alumina, aluminum stearate, lecithin, self-emulsifying drug delivery systems (SEDDS) such as d-α-tocopherol polyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such

as hydroxyalkylcyclodextrins, including 2- and 3-hydroxypropyl-β-cyclodextrins, or other solubilized derivatives can also be used to enhance delivery of compounds described herein. Dosage forms or compositions containing a chemical entity as described herein in the range of 0.005% to 100% with the balance made up from nontoxic excipient may be prepared. The contemplated compositions may contain 0.001%-100% of a chemical entity provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

In some embodiments, an NLRP3 antagonist as claimed is formulated in a pharmaceutical composition that includes the NLRP3 antagonist and/or anti-TNFα agent and one or more pharmaceutically acceptable excipients, and optionally one or more additional therapeutic agents as described herein. Preferably the pharmaceutical composition that includes an NLRP3 antagonist and an anti-TNFα agent.

*Routes of Administration and Composition Components*

In some embodiments not forming part of the invention,, the chemical entities described herein or a pharmaceutical composition thereof can be administered to subject in need thereof by any accepted route of administration. Acceptable routes of administration include, but are not limited to, buccal, cutaneous, endocervical, endosinusial, endotracheal, enteral, epidural, interstitial, intra-abdominal, intra-arterial, intrabronchial, intrabursal, intracerebral, intracisternal, intracoronary, intradermal, intraductal, intraduodenal, intradural, intraepidermal, intraesophageal, intragastric, intragingival, intraileal, intralymphatic, intramedullary, intrameningeal, intramuscular, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal, nasogastric, oral, parenteral, percutaneous, peridural, rectal, respiratory (inhalation), subcutaneous, sublingual, submucosal, topical, transdermal, transmucosal, transtracheal, ureteral, urethral and vaginal. In certain embodiments, a preferred route of administration is parenteral (e.g., intratumoral).

Compositions as claimed and thus forming part of the invention can be formulated for parenteral administration, e.g., formulated for injection via the intravenous, intramuscular, subcutaneous, or even intraperitoneal routes. Typically, such compositions can be prepared as injectables, either as liquid solutions or suspensions; solid forms suitable for use to prepare solutions or suspensions upon the addition of a liquid prior to injection can also be prepared; and the preparations can also be emulsified. The preparation of such formulations will be known to those of skill in the art in light of the present disclosure.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof.

Intratumoral injections are discussed, e.g., in Lammers, et al., "Effect of Intratumoral Injection on the Biodistribution and the Therapeutic Potential of HPMA Copolymer-Based Drug Delivery Systems" Neoplasia. 2006, 10, 788-795.

In certain embodiments, the chemical entities described herein or a pharmaceutical composition thereof are suitable for local, topical administration to the digestive or GI tract, e.g., rectal administration. Rectal compositions include, without limitation, enemas, rectal gels, rectal foams, rectal aerosols, suppositories, jelly suppositories, and enemas (e.g., retention enemas).

Pharmacologically acceptable excipients usable in the rectal composition as a gel, cream, enema, or rectal suppository, include, without limitation, any one or more of cocoa butter glycerides, synthetic polymers such as polyvinylpyrrolidone, PEG (like PEG ointments), glycerine, glycerinated gelatin, hydrogenated vegetable oils, poloxamers, mixtures of polyethylene glycols of various molecular weights and fatty acid esters of polyethylene glycol Vaseline, anhydrous lanolin, shark liver oil, sodium saccharinate, menthol, sweet almond oil, sorbitol, sodium benzoate, anoxid SBN, vanilla essential oil, aerosol, parabens in phenoxyethanol, sodium methyl p-oxybenzoate, sodium propyl p-oxybenzoate, diethylamine, carbomers, carbopol, methyloxybenzoate, macrogol cetostearyl ether, cocoyl caprylocaprate, isopropyl alcohol, propylene glycol, liquid paraffin, xanthan gum, carboxy-metabisulfite, sodium edetate, sodium benzoate, potassium metabisulfite, grapefruit seed extract, methyl sulfonyl methane (MSM), lactic acid, glycine, vitamins, such as vitamin A and E and potassium acetate.

In certain embodiments, suppositories can be prepared by mixing the chemical entities claimed with suitable non-irritating excipients or carriers such as cocoa butter, polyethylene glycol or a suppository wax which are solid at ambient temperature but liquid at body temperature and therefore melt in the rectum and release the active compound. In other embodiments, compositions for rectal administration are in the form of an enema.

In other embodiments, the compounds claimed or a pharmaceutical composition thereof as claimed are suitable for local delivery to the digestive or GI tract by way of oral administration (e.g., solid or liquid dosage forms.).

Solid dosage forms for oral administration include capsules, tablets, pills, powders, and granules. In such solid dosage forms, the chemical entity is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In one embodiment, the compositions as claimed will take the form of a unit dosage form such as a pill or tablet and thus the composition may contain, along with a chemical entity provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In another solid dosage form, a powder, marume, solution or suspension (e.g., in propylene carbonate, vegetable oils, PEG's, poloxamer 124 or triglycerides) is encapsulated in a capsule (gelatin or cellulose base capsule). Unit dosage forms in which one or more compounds as claimed or additional active agents are physically separated are also contemplated; e.g., capsules with granules (or tablets in a capsule) of each drug; two-layer tablets; two-compartment gel caps, etc. Enteric coated or delayed release oral dosage forms are also contemplated.

Other physiologically acceptable compounds include wetting agents, emulsifying agents, dispersing agents or preservatives that are particularly useful for preventing the growth or action of microorganisms. Various preservatives are well known and include, for example, phenol and ascorbic acid.

In certain embodiments the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

In certain embodiments, solid oral dosage forms can further include one or more components that chemically and/or structurally predispose the composition for delivery of the chemical entity to the stomach or the lower GI; e.g., the ascending colon and/or transverse colon and/or distal colon and/or small bowel. Exemplary formulation techniques are described in, e.g., Filipski, K.J., et al., Current Topics in Medicinal Chemistry, 2013, 13, 776-802, which is incorporated herein by reference in its entirety.

Examples include upper-GI targeting techniques, e.g., Accordion Pill (Intec Pharma), floating capsules, and materials capable of adhering to mucosal walls.

Other examples include lower-GI targeting techniques. For targeting various regions in the intestinal tract, several enteric/pH-responsive coatings and excipients are available. These materials are typically polymers that are designed to dissolve or erode at specific pH ranges, selected based upon the GI region of desired drug release. These materials also function to protect acid labile drugs from gastric fluid or limit exposure in cases where the active ingredient may be irritating to the upper GI (e.g., hydroxypropyl methylcellulose phthalate series, Coateric (polyvinyl acetate phthalate), cellulose acetate phthalate, hydroxypropyl methylcellulose acetate succinate, Eudragit series (methacrylic acid-methyl methacrylate copolymers), and Marcoat). Other techniques include dosage forms that respond to local flora in the GI tract, Pressure-controlled colon delivery capsule, and Pulsincap.

Ocular compositions can include, without limitation, one or more of any of the following: viscogens (e.g., Carboxymethylcellulose, Glycerin, Polyvinylpyrrolidone, Polyethylene glycol); Stabilizers (e.g., Pluronic (triblock copolymers), Cyclodextrins); Preservatives (e.g., Benzalkonium chloride, ETDA, SofZia (boric acid, propylene glycol, sorbitol, and zinc chloride; Alcon Laboratories, Inc.), Purite (stabilized oxychloro complex; Allergan, Inc.)).

Topical compositions can include ointments and creams. Ointments are semisolid preparations that are typically based on petrolatum or other petroleum derivatives. Creams containing the selected active agent are typically viscous liquid or semisolid emulsions, often either oil-in-water or water-in-oil. Cream bases are typically water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. As with other carriers or vehicles, an ointment base should be inert, stable, nonirritating and non-sensitizing.

In any of the foregoing embodiments, pharmaceutical compositions described herein can include one or more one or more of the following: lipids, interbilayer crosslinked multilamellar vesicles, biodegradeable poly(D,L-lactic-co-glycolic acid) [PLGA]-based or poly anhydride-based nanoparticles or microparticles, and nanoporous particle-supported lipid bilayers.

Enema Formulations

In some embodiments, enema formulations containing the chemical entities as defined in the claims are provided in "ready-to-use" form.

In some embodiments, enema formulations containing the chemical entities as defined in the claims are provided in one or more kits or packs. In certain embodiments, the kit or pack includes two or more separately contained/packaged components, e.g. two components, which when mixed together, provide the desired formulation (e.g., as a suspension). In certain of these embodiments, the two component system includes a first component and a second component, in which: (i) the first component (e.g., contained in a sachet) includes the chemical entity as defined in the claims and optionally one or more pharmaceutically acceptable excipients (e.g., together formulated as a solid preparation, e.g., together formulated as a wet granulated solid preparation); and (ii) the second component (e.g., contained in a vial or bottle) includes one or more liquids and optionally one or more other pharmaceutically acceptable excipients together forming a liquid carrier. Prior to use (e.g., immediately prior to use), the contents of (i) and (ii) are combined to form the desired enema formulation, e.g., as a suspension. In other embodiments, each of component (i) and (ii) is provided in its own separate kit or pack.

In some embodiments, each of the one or more liquids is water, or a physiologically acceptable solvent, or a mixture of water and one or more physiologically acceptable solvents. Typical such solvents include, without limitation, glycerol, ethylene glycol, propylene glycol, polyethylene glycol and polypropylene glycol. In certain embodiments, each of the one or more liquids is water. In other embodiments, each of the one or more liquids is an oil, e.g. natural and/or synthetic oils that are commonly used in pharmaceutical preparations.

Further pharmaceutical excipients and carriers that may be used in the pharmaceutical products herein described are listed in various handbooks (e.g. D. E. Bugay and W. P. Findlay (Eds) Pharmaceutical excipients (Marcel Dekker, New York, 1999), E-M Hoepfner, A. Reng and P. C. Schmidt (Eds) Fiedler Encyclopedia of Excipients for Pharmaceuticals, Cosmetics and Related Areas (Edition Cantor, Munich, 2002) and H. P. Fielder (Ed) Lexikon der Hilfsstoffe für Pharmazie, Kosmetik and angrenzende Gebiete (Edition Cantor Aulendorf, 1989)).

In some embodiments, each of the one or more pharmaceutically acceptable excipients can be independently selelcted from thickeners, viscosity enhancing agents, bulking agents, mucoadhesive agents, penetration enhanceers, buffers, preservatives, diluents, binders, lubricants, glidants, disintegrants, fillers, solubilizing agents, pH modifying agents, preservatives, stabilizing agents, anti-oxidants, wetting or emulsifying agents, suspending agents, pigments, colorants, isotonic agents, chelating agents, emulsifiers, and diagnostic agents.

In certain embodiments, each of the one or more pharmaceutically acceptable excipients can be independently selelcted from thickeners, viscosity enhancing agents, mucoadhesive agents, buffers, preservatives, diluents, binders, lubricants, glidants, disintegrants, and fillers.

In certain embodiments, each of the one or more pharmaceutically acceptable excipients can be independently selelcted from thickeners, viscosity enhancing agents, bulking agents, mucoadhesive agents, buffers, preservatives, and fillers.

In certain embodiments, each of the one or more pharmaceutically acceptable excipients can be independently selelcted from diluents, binders, lubricants, glidants, and disintegrants.

Examples of thickeners, viscosity enhancing agents, and mucoadhesive agents include without limitation: gums, e.g. xanthan gum, guar gum, locust bean gum, tragacanth gums, karaya gum, ghatti gum, cholla gum, psyllium seed gum and gum arabic; poly(carboxylic acid-containing) based polymers, such as poly (acrylic, maleic, itaconic, citraconic, hydroxyethyl methacrylic or methacrylic) acid which have strong hydrogen-bonding groups, or derivatives thereof such as salts and esters; cellulose derivatives, such as methyl cellulose, ethyl cellulose, methylethyl cellulose, hydroxymethyl

cellulose, hydroxyethyl cellulose, hydroxypropyl cellulose, hydroxyethyl ethyl cellulose, carboxymethyl cellulose, hydroxypropylmethyl cellulose or cellulose esters or ethers or derivatives or salts thereof; clays such as manomorillonite clays, e.g. Veegun, attapulgite clay; polysaccharides such as dextran, pectin, amylopectin, agar, mannan or polygalactonic acid or starches such as hydroxypropyl starch or carboxymethyl starch; polypeptides such as casein, gluten, gelatin, fibrin glue; chitosan, e.g. lactate or glutamate or carboxymethyl chitin; glycosaminoglycans such as hyaluronic acid; metals or water soluble salts of alginic acid such as sodium alginate or magnesium alginate; schleroglucan; adhesives containing bismuth oxide or aluminium oxide; atherocollagen; polyvinyl polymers such as carboxyvinyl polymers; polyvinylpyrrolidone (povidone); polyvinyl alcohol; polyvinyl acetates, polyvinylmethyl ethers, polyvinyl chlorides, polyvinylidenes, and/or the like; polycarboxylated vinyl polymers such as polyacrylic acid as mentioned above; polysiloxanes; polyethers; polyethylene oxides and glycols; polyalkoxys and polyacrylamides and derivatives and salts thereof. Preferred examples can include cellulose derivatives, such as methyl cellulose, ethyl cellulose, methylethyl cellulose, hydroxymethyl cellulose, hydroxyethyl cellulose, hydroxypropyl cellulose, hydroxyethyl ethyl cellulose, carboxymethyl cellulose, hydroxypropylmethyl cellulose or cellulose esters or ethers or derivatives or salts thereof (e.g., methyl cellulose); and polyvinyl polymers such as polyvinylpyrrolidone (povidone).

Examples of preservatives include without limitation: benzalkonium chloride, benzoxonium chloride, benzethonium chloride, cetrimide, sepazonium chloride, cetylpyridinium chloride, domiphen bromide (Bradosol®), thiomersal, phenylmercuric nitrate, phenylmercuric acetate, phenylmercuric borate, methylparaben, propylparaben, chlorobutanol, benzyl alcohol, phenyl ethyl alcohol, chlorohexidine, polyhexamethylene biguanide, sodium perborate, imidazolidinyl urea, sorbic acid, Purite®), Polyquart®), and sodium perborate tetrahydrate and the like.

In certain embodiments, the preservative is a paraben, or a pharmaceutically acceptable salt thereof. In some embodiments, the paraben is an alkyl substituted 4-hydroxybenzoate, or a pharmaceutically acceptable salt or ester thereof. In certain embodiments, the alkyl is a C1-C4 alkyl. In certain embodiments, the preservative is methyl 4-hydroxybenzoate (methylparaben), or a pharmaceutically acceptable salt or ester thereof, propyl 4-hydroxybenzoate (propylparaben), or a pharmaceutically acceptable salt or ester thereof, or a combination thereof.

Examples of buffers include without limitation: phosphate buffer system (sodium dihydrogen phospahate dehydrate, disodium phosphate dodecahydrate, bibasic sodium phosphate, anhydrous monobasic sodium phosphate), bicarbonate buffer system, and bisulfate buffer system.

Examples of disintegrants include, without limitation: carmellose calcium, low substituted hydroxypropyl cellulose (L-HPC), carmellose, croscarmellose sodium, partially pregelatinized starch, dry starch, carboxymethyl starch sodium, crospovidone, polysorbate 80 (polyoxyethylenesorbitan oleate), starch, sodium starch glycolate, hydroxypropyl cellulose pregelatinized starch, clays, cellulose, alginine, gums or cross linked polymers, such as crosslinked PVP (Polyplasdone XL from GAF Chemical Corp). In certain embodiments, the disintegrant is crospovidone.

Examples of glidants and lubricants (aggregation inhibitors) include without limitation: talc, magnesium stearate, calcium stearate, colloidal silica, stearic acid, aqueous silicon dioxide, synthetic magnesium silicate, fine granulated silicon oxide, starch, sodium laurylsulfate, boric acid, magnesium oxide, waxes, hydrogenated oil, polyethylene glycol, sodium benzoate, stearic acid glycerol behenate, polyethylene glycol, and mineral oil. In certain embodiments, the glidant/lubricant is magnesium stearate, talc, and/or colloidal silica; e.g., magnesium stearate and/or talc.

Examples of diluents, also referred to as "fillers" or "bulking agents" include without limitation: dicalcium phosphate dihydrate, calcium sulfate, lactose (e.g., lactose monohydrate), sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powdered sugar. In certain embodiments, the diluent is lactose (e.g., lactose monohydrate).

Examples of binders include without limitation: starch, pregelatinized starch, gelatin, sugars (including sucrose, glucose, dxtrose, lactose and sorbitol), polyethylene glycol, waxes, natural and synthetic gums such as acacia tragacanth, sodium alginate cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone (povidone). In certain embodiments, the binder is polyvinylpyrrolidone (povidone).

In some embodiments, enema formulations containing the chemical entities as defined in the claims described herein include water and one or more (e.g., all) of the following excipients:

- One or more (e.g., one, two, or three) thickeners, viscosity enhancing agents, binders, and/or mucoadhesive agents (e.g., cellulose or cellulose esters or ethers or derivatives or salts thereof (e.g., methyl cellulose); and polyvinyl polymers such as polyvinylpyrrolidone (povidone);
- One or more (e.g., one or two; e.g., two) preservatives, such as a paraben, e.g., methyl 4-hydroxybenzoate (methylparaben), or a pharmaceutically acceptable salt or ester thereof, propyl 4-hydroxybenzoate (propylparaben), or a pharmaceutically acceptable salt or ester thereof, or a combination thereof;
- One or more (e.g., one or two; e.g., two) buffers, such as phosphate buffer system (e.g., sodium dihydrogen phospahate dehydrate, disodium phosphate dodecahydrate);

- One or more (e.g., one or two, e.g., two) glidants and/or lubricants, such as magnesium stearate and/or talc;
- One or more (e.g., one or two; e.g., one) disintegrants, such as crospovidone; and
- One or more (e.g., one or two; e.g., one) diluents, such as lactose (e.g., lactose monohydrate).

In certain embodiments, enema formulations containing the chemical entities as defined in the claims include water, methyl cellulose, povidone, methylparaben, propylparaben, sodium dihydrogen phospahate dehydrate, disodium phosphate dodecahydrate, crospovidone, lactose monohydrate, magnesium stearate, and talc.

In certain embodiments, enema formulations containing the chemical entities as defined in the claims are provided in one or more kits or packs. In certain embodiments, the kit or pack includes two separately contained/packaged components, which when mixed together, provide the desired formulation (e.g., as a suspension). In certain of these embodiments, the two component system includes a first component and a second component, in which: (**i**) the first component (e.g., contained in a sachet) includes the chemical entity as defined in the claims and one or more pharmaceutically acceptable excipients (e.g., together formulated as a solid preparation, e.g., together formulated as a wet granulated solid preparation); and (**ii**) the second component (e.g., contained in a vial or bottle) includes one or more liquids and one or more one or more other pharmaceutically acceptable excipients together forming a liquid carrier. In other embodiments, each of component (**i**) and (**ii**) is provided in its own separate kit or pack.

In certain of these embodiments, component (**i**) includes the chemical entitiy as defined in the claims and one or more (e.g., all) of the following excipients:

(**a**) One or more (e.g., one) binders (e.g., a polyvinyl polymer, such as polyvinylpyrrolidone (povidone);
(**b**) One or more (e.g., one or two, e.g., two) glidants and/or lubricants, such as magnesium stearate and/or talc;
(**c**) One or more (e.g., one or two; e.g., one) disintegrants, such as crospovidone; and
(**d**) One or more (e.g., one or two; e.g., one) diluents, such as lactose (e.g., lactose monohydrate).

In certain embodiments, component (**i**) includes from about 40 weight percent to about 80 weight percent (e.g., from about 50 weight percent to about 70 weight percent, from about 55 weight percent to about 70 weight percent; from about 60 weight percent to about 65 weight percent; e.g., about 62.1 weight percent) of the chemical entity as defined in the claims.

In certain embodiments, component (**i**) includes from about 0.5 weight percent to about 5 weight percent (e.g., from about 1.5 weight percent to about 4.5 weight percent, from about 2 weight percent to about 3.5 weight percent; e.g., about 2.76 weight percent) of the binder (e.g., povidone).

In certain embodiments, component (**i**) includes from about 0.5 weight percent to about 5 weight percent (e.g., from about 0.5 weight percent to about 3 weight percent, from about 1 weight percent to about 3 weight percent; about 2 weight percent e.g., about 1.9 weight percent) of the disintegrant (e.g., crospovidone).

In certain embodiments, component (**i**) includes from about 10 weight percent to about 50 weight percent (e.g., from about 20 weight percent to about 40 weight percent, from about 25 weight percent to about 35 weight percent; e.g., about 31.03 weight percent) of the diluent (e.g., lactose, e.g., lactose monohydrate).

In certain embodiments, component (**i**) includes from about 0.05 weight percent to about 5 weight percent (e.g., from about 0.05 weight percent to about 3 weight percent) of the glidants and/or lubricants.

In certain embodiments (e.g., when component (**i**) includes one or more lubricants, such as magnesium stearate), component (**i**) includes from about 0.05 weight percent to about 1 weight percent (e.g., from about 0.05 weight percent to about 1 weight percent; from about 0.1 weight percent to about 1 weight percent; from about 0.1 weight percent to about 0.5 weight percent; e.g., about 0.27 weight percent) of the lubricant (e.g., magnesium stearate).

In certain embodiments (when component (i) includes one or more lubricants, such as talc), component (**i**) includesfrom about 0.5 weight percent to about 5 weight percent (e.g., from about 0.5 weight percent to about 3 weight percent, from about 1 weight percent to about 3 weight percent; from about 1.5 weight percent to about 2.5 weight percent; from about 1.8 weight percent to about 2.2 weight percent; about 1.93 weight percent) of the lubricant (e.g., talc).

In certain of these embodiments, each of (**a**), (**b**), (**c**), and (**d**) above is present.

In certain embodiments, component (**i**) is formulated as a wet granulated solid preparation. In certain of these embodiments an internal phase of ingredients (the chemical entity, disintegrant, and diluent) are combined and mixed in a high-shear granulator. A binder (e.g., povidone) is dissolved in water to form a granulating solution. This solution is added to the Inner Phase mixture resulting in the development of granules. While not wishing to be bound by theory, granule development is believed to be facilitated by the interaction of the polymeric binder with the materials of the internal phase. Once the granulation is formed and dried, an external phase (e.g., one or more lubricants - not an intrinsic component of the dried granulation), is added to the dry granulation. It is believed that lubrication of the granulation is important to the flowability of the granulation, in particular for packaging.

In certain of the foregoing embodiments, component (**ii**) includes water and one or more (e.g., all) of the following

excipients:

(**a'**) One or more (e.g., one, two; e.g., two) thickeners, viscosity enhancing agents, binders, and/or mucoadhesive agents (e.g., cellulose or cellulose esters or ethers or derivatives or salts thereof (e.g., methyl cellulose); and polyvinyl polymers such as polyvinylpyrrolidone (povidone);
(**b'**) One or more (e.g., one or two; e.g., two) preservatives, such as a paraben, e.g., methyl 4-hydroxybenzoate (methylparaben), or a pharmaceutically acceptable salt or ester thereof, propyl 4-hydroxybenzoate (propylparaben), or a pharmaceutically acceptable salt or ester thereof, or a combination thereof; and
(**c'**) One or more (e.g., one or two; e.g., two) buffers, such as phosphate buffer system (e.g., sodium dihydrogen phospahate dihydrate, disodium phosphate dodecahydrate);

In certain of the foregoing embodiments, component (**ii**) includes water and one or more (e.g., all) of the following excipients:

(**a"**) a first thickener, viscosity enhancing agent, binder, and/or mucoadhesive agent (e.g., a cellulose or cellulose ester or ether or derivative or salt thereof (e.g., methyl cellulose));
(**a'''**) a second thickener, viscosity enhancing agent, binder, and/or mucoadhesive agent (e.g., a polyvinyl polymer, such as polyvinylpyrrolidone (povidone));
(**b"**) a first preservative, such as a paraben, e.g., propyl 4-hydroxybenzoate (propylparaben), or a pharmaceutically acceptable salt or ester thereof;
(**b"**) a second preservative, such as a paraben, e.g., methyl 4-hydroxybenzoate (methylparaben), or a pharmaceutically acceptable salt or ester thereof,
(**c"**) a first buffer, such as phosphate buffer system (e.g., disodium phosphate dodecahydrate);
(**c'''**) a second buffer, such as phosphate buffer system (e.g., sodium dihydrogen phospahate dehydrate),

In certain embodiments, component (**ii**) includes from about 0.05 weight percent to about 5 weight percent (e.g., from about 0.05 weight percent to about 3 weight percent, from about 0.1 weight percent to about 3 weight percent; e.g., about 1.4 weight percent) of (**a"**).
In certain embodiments, component (**ii**) includes from about 0.05 weight percent to about 5 weight percent (e.g., from about 0.05 weight percent to about 3 weight percent, from about 0.1 weight percent to about 2 weight percent; e.g., about 1.0 weight percent) of (**a'''**).
In certain embodiments, component (**ii**) includes from about 0.005 weight percent to about 0.1 weight percent (e.g., from about 0.005 weight percent to about 0.05 weight percent; e.g., about 0.02 weight percent) of (**b"**).
In certain embodiments, component (**ii**) includes from about 0.05 weight percent to about 1 weight percent (e.g., from about 0.05 weight percent to about 0.5 weight percent; e.g., about 0.20 weight percent) of (**b'''**).
In certain embodiments, component (**ii**) includes from about 0.05 weight percent to about 1 weight percent (e.g., from about 0.05 weight percent to about 0.5 weight percent; e.g., about 0.15 weight percent) of (**c"**).
In certain embodiments, component (**ii**) includes from about 0.005 weight percent to about 0.5 weight percent (e.g., from about 0.005 weight percent to about 0.3 weight percent; e.g., about 0.15 weight percent) of (**c'''**).
In certain of these embodiments, each of (**a"**) - (**c'''**) is present.
Ready-to-use" enemas are generally be provided in a "single-use" sealed disposable container of plastic or glass. Those formed of a polymeric material preferably have sufficient flexibility for ease of use by an unassisted patient. Typical plastic containers can be made of polyethylene. These containers may comprise a tip for direct introduction into the rectum. Such containers may also comprise a tube between the container and the tip. The tip is preferably provided with a protective shield which is removed before use. Optionally the tip has a lubricant to improve patient compliance.
In some embodiments, the enema formulation (e.g., suspension) is poured into a bottle for delivery after it has been prepared in a separate container. In certain embodiments, the bottle is a plastic bottle (e.g., flexible to allow for delivery by squeezing the bottle), which can be a polyethylene bottle (e.g., white in color). In some embodiments, the bottle is a single chamber bottle, which contains the suspension or solution. In other embodiments, the bottle is a multichamber bottle, where each chamber contains a separate mixture or solution. In still other embodiments, the bottle can further include a tip or rectal cannula for direct introduction into the rectum.

*Dosages*

In some embodiments, enema formulations include from about 0.5 mg to about 2500 mg (e.g., from about 0.5 mg to about 2000 mg, from about 0.5 mg to about 1000 mg, from about 0.5 mg to about 750 mg, from about 0.5 mg to about 600 mg, from about 0.5 mg to about 500 mg, from about 0.5 mg to about 400 mg, from about 0.5 mg to about 300 mg, from about 0.5 mg to about 200 mg; e.g., from about 5 mg to about 2500 mg, from about 5 mg to about 2000 mg,

from about 5 mg to about 1000 mg; from about 5 mg to about 750 mg; from about 5 mg to about 600 mg; from about 5 mg to about 500 mg; from about 5 mg to about 400 mg; from about 5 mg to about 300 mg; from about 5 mg to about 200 mg; e.g., from about 50 mg to about 2000 mg, from about 50 mg to about 1000 mg, from about 50 mg to about 750 mg, from about 50 mg to about 600 mg, from about 50 mg to about 500 mg, from about 50 mg to about 400 mg, from about 50 mg to about 300 mg, from about 50 mg to about 200 mg; e.g., from about 100 mg to about 2500 mg, from about 100 mg to about 2000 mg, from about 100 mg to about 1000 mg, from about 100 mg to about 750 mg, from about 100 mg to about 700 mg, from about 100 mg to about 600 mg, from about 100 mg to about 500 mg, from about 100 mg to about 400 mg, from about 100 mg to about 300 mg, from about 100 mg to about 200 mg; e.g., from about 150 mg to about 2500 mg, from about 150 mg to about 2000 mg, from about 150 mg to about 1000 mg, from about 150 mg to about 750 mg, from about 150 mg to about 700 mg, from about 150 mg to about 600 mg, from about 150 mg to about 500 mg, from about 150 mg to about 400 mg, from about 150 mg to about 300 mg, from about 150 mg to about 200 mg; e.g., from about 150 mg to about 500 mg; e.g., from about 300 mg to about 2500 mg, from about 300 mg to about 2000 mg, from about 300 mg to about 1000 mg, from about 300 mg to about 750 mg, from about 300 mg to about 700 mg, from about 300 mg to about 600 mg; e.g., from about 400 mg to about 2500 mg, from about 400 mg to about 2000 mg, from about 400 mg to about 1000 mg, from about 400 mg to about 750 mg, from about 400 mg to about 700 mg, from about 400 mg to about 600 from about 400 mg to about 500 mg; e.g., 150 mg or 450 mg) of the chemical entity as defined in the claims in from about 1 mL to about 3000 mL (e.g., from about 1 mL to about 2000 mL, from about 1 mL to about 1000 mL, from about 1 mL to about 500 mL, from about 1 mL to about 250 mL, from about 1 mL to about 100 mL, from about 10 mL to about 1000 mL, from about 10 mL to about 500 mL, from about 10 mL to about 250 mL, from about 10 mL to about 100 mL, from about 30 mL to about 90 mL, from about 40 mL to about 80 mL; from about 50 mL to about 70 mL; e.g., about 1 mL, about 5 mL, about 10 mL, about 15 mL, about 20 mL, about 25 mL, about 30 mL, about 35 mL, about 40 mL, about 45 mL,about 50 mL, about 55 mL, about 60 mL, about 65 mL, about 70 mL, about 75 mL, about 100 mL, about 250 mL, or about 500 mL, or about 1000 mL, or about 2000mL, or about 3000 mL; e.g., 60 mL) of liquid carrier.

In certain embodiments, enema formulations include from about 50 mg to about 250 mg (e.g., from about 100 mg to about 200; e.g., about 150 mg) of the chemical entity as defined in the claims in from about 10 mL to about 100 mL (e.g., from about 20 mL to about 100 mL, from about 30 mL to about 90 mL, from about 40 mL to about 80 mL; from about 50 mL to about 70 mL) of liquid carrier. In certain embodiments, enema formulations include about 150 mg of the chemical entity in about 60 mL of the liquid carrier.

In certain embodiments, enema formulations include from about 350 mg to about 550 mg (e.g., from about 400 mg to about 500; e.g., about 450 mg) of the chemical entity as defined in the claims in from about 10 mL to about 100 mL (e.g., from about 20 mL to about 100 mL, from about 30 mL to about 90 mL, from about 40 mL to about 80 mL; from about 50 mL to about 70 mL) of liquid carrier. In certain embodiments, enema formulations include about 450 mg of the chemical entity as defined in the claims in about 60 mL of the liquid carrier.

In some embodiments, enema formulations include from about from about 0.01 mg/mL to about 50 mg/mL (e.g., from about 0.01 mg/mL to about 25 mg/mL; from about 0.01 mg/mL to about 10 mg/mL; from about 0.01 mg/mL to about 5 mg/mL; from about 0.1 mg/mL to about 50 mg/mL; from about 0.01 mg/mL to about 25 mg/mL; from about 0.1 mg/mL to about 10 mg/mL; from about 0.1 mg/mL to about 5 mg/mL; from about 1 mg/mL to about 10 mg/mL; from about 1 mg/mL to about 5 mg/mL; from about 5 mg/mL to about 10 mg/mL; e.g., about 2.5 mg/mL or about 7.5 mg/mL) of the chemical entity as defined in the claims in liquid carrier.

*Regimens (only disclosed for reference purposes and not as part of the invention)*

The foregoing dosages can be administered on a daily basis (e.g., as a single dose or as two or more divided doses) or non-daily basis (e.g., every other day, every two days, every three days, once weekly, twice weeks, once every two weeks, once a month).

In some embodiments, the period of administration of a compound described herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In a further embodiment, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 1 1 months, 12 months, or more. In an embodiment, a therapeutic compound is administered to an individual for a period of time followed by a separate period of time. In another embodiment, a therapeutic compound is administered for a first period and a second period following the first period, with administration stopped during the second period, followed by a third period where administration of the therapeutic compound is started and then a fourth period following the third period where administration is stopped. In an aspect of this embodiment, the period of administration of a therapeutic compound followed by a period

where administration is stopped is repeated for a determined or undetermined period of time. In a further embodiment, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

**Methods of Treatment** *(only disclosed for reference purposes and not as part of the invention)*

In some embodiments, methods for treating a subject having condition, disease or disorder in which a decrease or increase in NLRP3 activity (e.g., an increase, e.g., NLRP3 signaling) contributes to the pathology and/or symptoms and/or progression of the condition, disease or disorder are provided, comprising administering to a subject an effective amount of a chemical entity described herein (e.g., a compound described generically or specifically herein or a pharmaceutically acceptable salt thereof or compositions containing the same).

*Indications*

In some embodiments, the condition, disease or disorder is selected from: inappropriate host responses to infectious diseases where active infection exists at any body site, such as septic shock, disseminated intravascular coagulation, and/or adult respiratory distress syndrome; acute or chronic inflammation due to antigen, antibody and/or complement deposition; inflammatory conditions including arthritis, cholangitis, colitis, encephalitis, endocarditis, glomerulonephritis, hepatitis, myocarditis, pancreatitis, pericarditis, reperfusion injury and vasculitis, immune-based diseases such as acute and delayed hypersensitivity, graft rejection, and graft-versus-host disease; auto-immune diseases including Type 1 diabetes mellitus and multiple sclerosis. For example, the condition, disease or disorder may be an inflammatory disorder such as rheumatoid arthritis, osteoarthritis, septic shock, COPD and periodontal disease.

In some embodiments, the condition, disease or disorder is an autoimmune diseases. Non-limiting examples include rheumatoid arthritis, systemic lupus erythematosus, multiple sclerosis, inflammatory bowel diseases (IBDs) comprising Crohn disease (CD) and ulcerative colitis (UC), which are chronic inflammatory conditions with polygenic susceptibility. In certain embodiments, the condition is an inflammatory bowel disease. In certain embodiments, the condition is Crohn's disease, autoimmune colitis, iatrogenic autoimmune colitis, ulcerative colitis, colitis induced by one or more chemotherapeutic agents, colitis induced by treatment with adoptive cell therapy, colitis associated by one or more alloimmune diseases (such as graft-vs-host disease, e.g., acute graft vs. host disease and chronic graft vs. host disease), radiation enteritis, collagenous colitis, lymphocytic colitis, microscopic colitis, and radiation enteritis. In certain of these embodiments, the condition is alloimmune disease (such as graft-vs-host disease, e.g., acute graft vs. host disease and chronic graft vs. host disease), celiac disease, irritable bowel syndrome, rheumatoid arthritis, lupus, scleroderma, psoriasis, cutaneous T-cell lymphoma, uveitis, and mucositis (e.g., oral mucositis, esophageal mucositis or intestinal mucositis).

In some embodiments, the condition, disease or disorder is selected from major adverse cardiovascular events such as carbiovascular death, non-fatal myocardial infarction and non-fatal stroke in patients with a prior hear attack and inflammatory atherosclerosis (see for example, NCT01327846).

In some embodiments, the condition, disease or disorder is selected from metabolic disorders such as type 2 diabetes, atherosclerosis, obesity and gout, as well as diseases of the central nervous system, such as Alzheimer's disease and multiple sclerosis and Amyotrophic Lateral Sclerosis and Parkinson disease, lung disease, such as asthma and COPD and pulmonary idiopathic fibrosis, liver disease, such as NASH syndrome, viral hepatitis and cirrhosis, pancreatic disease, such as acute and chronic pancreatitis, kidney disease, such as acute and chronic kidney injury, intestinal disease such as Crohn's disease and Ulcerative Colitis, skin disease such as psoriasis, musculoskeletal disease such as scleroderma, vessel disorders, such as giant cell arteritis, disorders of the bones, such as Osteoarthritis , osteoporosis and osteopetrosis disorders eye disease, such as glaucoma and macular degeneration, diseased caused by viral infection such as HIV and AIDS, autoimmune disease such as Rheumatoid Arthritis, Systemic Lupus Erythematosus, Autoimmune Thyroiditis, Addison's disease, pernicious anemia, cancer and aging.

In some embodiments, the condition, disease or disorder is a cardiovascular indication. In some embodiments, the condition, disease or disorder is myocardial infraction. In some embodiments, the condition, disease or disorder is stroke.

In some embodiments, the condition, disease or disorder is obesity.

In some embodiments, the condition, disease or disorder is Type 2 Diabetes.

In some embodiments, the condition, disease or disorder is NASH.

In some embodiments, the condition, disease or disorder is Alzheimer's disease.

In some embodiments, the condition, disease or disorder is gout.

In some embodiments, the condition, disease or disorder is SLE.

In some embodiments, the condition, disease or disorder is rheumatoid arthritis.

In some embodiments, the condition, disease or disorder is IBD.

In some embodiments, the condition, disease or disorder is multiple sclerosis.

In some embodiments, the condition, disease or disorder is COPD.

In some embodiments, the condition, disease or disorder is asthma.

In some embodiments, the condition, disease or disorder is scleroderma.

In some embodiments, the condition, disease or disorder is pulmonary fibrosis.

In some embodiments, the condition, disease or disorder is age related macular degeneration (AMD).

In some embodiments, the condition, disease or disorder is cystic fibrosis.

In some embodiments, the condition, disease or disorder is Muckle Wells syndrome.

In some embodiments, the condition, disease or disorder is familial cold autoinflammatory syndrome (FCAS).

In some embodiments, the condition, disease or disorder is chronic neurologic cutaneous and articular syndrome.

In some embodiments, the condition, disease or disorder is selected from: myelodysplastic syndromes (MDS); non-small cell lung cancer, such as non-small cell lung cancer in patients carrying mutation or overexpression of NLRP3; acute lymphoblastic leukemia (ALL), such as ALL in patients resistant to glucocorticoids treatment; Langerhan's cell histiocytosis (LCH); multiple myeloma; promyelocytic leukemia; acute myeloid leukemia (AML) chronic myeloid leukemia (CML); gastric cancer; and lung cancer metastasis.

In some embodiments, the condition, disease or disorder is selected from: myelodysplastic syndromes (MDS); non-small cell lung cancer, such as non-small cell lung cancer in patients carrying mutation or overexpression of NLRP3; acute lymphoblastic leukemia (ALL), such as ALL in patients resistant to glucocorticoids treatment; Langerhan's cell histiocytosis (LCH); multiple myeloma; promyelocytic leukemia; gastric cancer; and lung cancer metastasis.

In some embodiments, the indication is MDS.

In some embodiments, the indication is non-small lung cancer in patients carrying mutation or overexpression of NLRP3.

In some embodiments, the indication is ALL in patients resistant to glucocorticoids treatment.

In some embodiments, the indication is LCH.

In some embodiments, the indication is multiple myeloma.

In some embodiments, the indication is promyelocytic leukemia.

In some embodiments, the indication is gastric cancer.

In some embodiments, the indication is lung cancer metastasis.

*Combination therapy*

This disclosure contemplates both monotherapy regimens as well as combination therapy regimens.

In some embodiments, the methods described herein can further include administering one or more additional therapies (e.g., one or more additional therapeutic agents and/or one or more therapeutic regimens) in combination with administration of the compounds described herein.

In certain embodiments, the second therapeutic agent or regimen is administered to the subject prior to contacting with or administering the chemical entity (e.g., about one hour prior, or about 6 hours prior, or about 12 hours prior, or about 24 hours prior, or about 48 hours prior, or about 1 week prior, or about 1 month prior).

In other embodiments, the second therapeutic agent or regimen is administered to the subject at about the same time as contacting with or administering the chemical entity. By way of example, the second therapeutic agent or regimen and the chemical entity are provided to the subject simultaneously in the same dosage form. As another example, the second therapeutic agent or regimen and the chemical entity are provided to the subject concurrently in separate dosage forms.

In still other embodiments, the second therapeutic agent or regimen is administered to the subject after contacting with or administering the chemical entity (e.g., about one hour after, or about 6 hours after, or about 12 hours after, or about 24 hours after, or about 48 hours after, or about 1 week after, or about 1 month after).

*Patient Selection (only disclosed for reference purposes and not as part of the invention)*

In some embodiments, the methods described herein further include the step of identifying a subject (e.g., a patient) in need of treatment for an indication related to NLRP3 activity, such as an indication related to NLRP3 polymorphism.

In some embodiments, the methods described herein further include the step of identifying a subject (e.g., a patient) in need of treatment for an indication related to NLRP3 activity, such as an indication related to NLRP3 where polymorphism is a gain of function

In some embodiments, the methods described herein further include the step of identifying a subject (e.g., a patient) in need of treatment for an indication related to NLRP3 activity, such as an indication related to NLRP3 polymorphism found in CAPS syndromes.

In some embodiments, the methods described herein further include the step of identifying a subject (e.g., a patient) in need of treatment for an indication related to NLRP3 activity, such as an indication related NLRP3 polymorphism where the polymorphism is VAR_014104 (R262W)

In some embodiments, the methods described herein further include the step of identifying a subject (e.g., a patient) in need of treatment for an indication related to NLRP3 activity, such as an indication related NLRP3 polymorphism where the polymorphism is a natural variant reported in http://www.uniprot.org/uniprot/Q96P20.

In some embodiments, the methods described herein further include the step of identifying a subject (e.g., a patient) in need of treatment for an indication related to NLRP3 activity, such as an indication related to point mutation of NLRP3 signaling.

**Anti-TNFα Agents** *(only disclosed for reference purposes and not as part of the invention)*

The term "anti-TNFα agent" refers to an agent which directly or indirectly blocks, down-regulates, impairs, inhibits, impairs, or reduces TNFα activity and/or expression. In some embodiments, an anti-TNFα agent is an antibody or an antigen-binding fragment thereof, a fusion protein, a soluble TNFα receptor (a soluble tumor necrosis factor receptor superfamily member 1A (TNFR1) or a soluble tumor necrosis factor receptor superfamily 1B (TNFR2)), an inhibitory nucleic acid, or a small molecule TNFα antagonist. In some embodiments, the inhibitory nucleic acid is a ribozyme, small hairpin RNA, a small interfering RNA, an antisense nucleic acid, or an aptamer.

Exemplary anti-TNFα agents that directly block, down-regulate, impair, inhibit, or reduce TNFα activity and/or expression can, e.g., inhibit or decrease the expression level of TNFα or a receptor of TNFα (TNFR1 or TNFR2) in a cell (e.g., a cell obtained from a subject, a mammalian cell), or inhibit or reduce binding of TNFα to its receptor (TNFR1 and/or TNFR2) and/or. Non-limiting examples of anti-TNFα agents that directly block, down-regulate, impair, inhibit, or reduce TNFα activity and/or expression include an antibody or fragment thereof, a fusion protein, a soluble TNFα receptor (e.g., a soluble TNFR1 or soluble TNFR2), inhibitory nucleic acids (e.g., any of the examples of inhibitory nucleic acids described herein), and a small molecule TNFα antagonist.

Exemplary anti-TNFα agents that can indirectly block, down-regulate, impair, inhibitreduce TNFα activity and/or expression can, e.g., inhibit or decrease the level of downstream signaling of a TNFα receptor (e.g., TNFR1 or TNFR2) in a mammalian cell (e.g., decrease the level and/or activity of one or more of the following signaling proteins: AP-1, mitogen-activated protein kinase kinase kinase 5 (ASK1), inhibitor of nuclear factor kappa B (IKK), mitogen-activated protein kinase 8 (JNK), mitogen-activated protein kinase (MAPK), MEKK 1/4, MEKK 4/7, MEKK 3/6, nuclear factor kappa B (NF-κB), mitogen-activated protein kinase kinase kinase 14 (NIK), receptor interacting serine/threonine kinase 1 (RIP), TNFRSF1A associated via death domain (TRADD), and TNF receptor associated factor 2 (TRAF2), in a cell), and/or decrease the level of TNFα-induced gene expression in a mammalian cell (e.g., decrease the transcription of genes regulated by, e.g., one or more transcription factors selected from the group of activating transcription factor 2 (ATF2), c-Jun, and NF-κB). A description of downstream signaling of a TNFα receptor is provided in Wajant et al., Cell Death Differentiation 10:45-65, 2003 (incorporated herein by reference). For example, such indirect anti-TNFα agents can be an inhibitory nucleic acid that targets (decreases the expression) a signaling component downstream of a TNFα-induced gene (e.g., any TNFα-induced gene known in the art), a TNFα receptor (e.g., any one or more of the signaling components downstream of a TNFα receptor described herein or known in the art), or a transcription factor selected from the group of NF-κB, c-Jun, and ATF2.

In other examples, such indirect anti-TNFα agents can be a small molecule inhibitor of a protein encoded by a TNFα-induced gene (e.g., any protein encoded by a TNFα-induced gene known in the art), a small molecule inhibitor of a signaling component downstream of a TNFα receptor (e.g., any of the signaling components downstream of a TNFα receptor described herein or known in the art), and a small molecule inhibitor of a transcription factor selected from the group of ATF2, c-Jun, and NF-κB.

In other embodiments, anti-TNFα agents that can indirectly block, down-regulate, impair, or reduce one or more components in a cell (e.g., acell obtained from a subject, a mammalian cell) that are involved in the signaling pathway that results in TNFα mRNA transcription, TNFα mRNA stabilization, and TNFα mRNA translation (e.g., one or more components selected from the group of CD14, c-Jun, ERK1/2, IKK, IκB, interleukin 1 receptor associated kinase 1 (IRAK), JNK, lipopolysaccharide binding protein (LBP), MEK1/2, MEK3/6, MEK4/7, MK2, MyD88, NF-κB, NIK, PKR, p38, AKT serine/threonine kinase 1 (rac), raf kinase (raf), ras, TRAF6, TTP). For example, such indirect anti-TNFα agents can be an inhibitory nucleic acid that targets (decreases the expression) of a component in a mammalian cell

that is involved in the signaling pathway that results in TNFα mRNA transcription, TNFα mRNA stabilization, and TNFα mRNA translation (e.g., a component selected from the group of CD14, c-Jun, ERK1/2, IKK, IκB, IRAK, JNK, LBP, MEK1/2, MEK3/6, MEK4/7, MK2, MyD88, NF-κB, NIK, IRAK, lipopolysaccharide binding protein (LBP), PKR, p38, rac, raf, ras, TRAF6, TTP). In other examples, an indirect anti-TNFα agents is a small molecule inhibitor of a component in a mammalian cell that is involved in the signaling pathway that results in TNFα mRNA transcription, TNFα mRNA stabilization, and TNFα mRNA translation (e.g., a component selected from the group of CD14, c-Jun, ERK1/2, IKK, IκB, IRAK, JNK, lipopolysaccharide binding protein (LBP), MEK1/2, MEK3/6, MEK4/7, MK2, MyD88, NF-κB, NIK, IRAK, lipopolysaccharide binding protein (LBP), PKR, p38, rac, raf, ras, TRAF6, TTP).

***Antibodies** (only disclosed for reference purposes and not as part of the invention)*

In some embodiments, the anti-TNFα agent is an antibody or an antigen-binding fragment thereof (e.g., a Fab or a scFv). In some embodiments, an antibody or antigen-binding fragment of an antibody described herein can bind specifically to TNFα. In some embodiments, an antibody or antigen-binding fragment described herein binds specifically to any one of TNFα, TNFR1, or TNFR2. In some embodiments, an antibody or antigen-binding fragment of an antibody described herein can bind specifically to a TNFα receptor (TNFR1 or TNFR2).

In some embodiments, the antibody can be a humanized antibody, a chimeric antibody, a multivalent antibody, or a fragment thereof. In some embodiments, an antibody can be a scFv-Fc, a VHH domain, a VNAR domain, a (scFv)2, a minibody, or a BiTE.

In some embodiments, an antibody can be a crossmab, a diabody, a scDiabody, a scDiabody-CH3, a Diabody-CH3, a DutaMab, a DT-IgG, a diabody-Fc, a scDiabody-HAS, a charge pair antibody, a Fab-arm exchange antibody, a SEEDbody, a Triomab, a LUZ-Y, a Fcab, a kλ-body, an orthogonal Fab, a DVD-IgG, an IgG(H)-scFv, a scFv-(H)IgG, an IgG(L)-scFv, a scFv-(L)-IgG, an IgG (L,H)-Fc, an IgG(H)-V, a V(H)-IgG, an IgG(L)-V, a V(L)-IgG, an KIH IgG-scFab, a 2scFv-IgG, an IgG-2scFv, a scFv4-Ig, a Zybody, a DVI-IgG, a nanobody, a nanobody-HSA, a DVD-Ig, a dual-affinity re-targeting antibody (DART), a triomab, a kih IgG with a common LC, an ortho-Fab IgG, a 2-in-1-IgG, IgG-ScFv, scFv2-Fc, a bi-nanobody, tanden antibody, a DART-Fc, a scFv-HAS-scFv, a DAF (two-in-one or four-in-one), a DNL-Fab3, knobs-in-holes common LC, knobs-in-holes assembly, a TandAb, a Triple Body, a miniantibody, a minibody, a TriBi minibody, a scFv-CH3 KIH, a Fab-scFv, a scFv-CH-CL-scFv, a F(ab')2-scFV2, a scFv-KIH, a Fab-scFv-Fc, a tetravalent HCAb, a scDiabody-Fc, a tandem scFv-Fc, an intrabody, a dock and lock bispecific antibody, an ImmTAC, a HSAbody, a tandem scFv, an IgG-IgG, a Cov-X-Body, and a scFv1-PEG-scFv2.

Non-limiting examples of an antigen-binding fragment of an antibody include an Fv fragment, a Fab fragment, a F(ab')2 fragment, and a Fab' fragment. Additional examples of an antigen-binding fragment of an antibody is an antigen-binding fragment of an antigen-binding fragment of an IgA (e.g., an antigen-binding fragment of IgA1 or IgA2) (e.g., an antigen-binding fragment of a human or humanized IgA, e.g., a human or humanized IgA1 or IgA2); an antigen-binding fragment of an IgD (e.g., an antigen-binding fragment of a human or humanized IgD); an antigen-binding fragment of an IgE (e.g., an antigen-binding fragment of a human or humanized IgE); an IgG (e.g., an antigen-binding fragment of IgG1, IgG2, IgG3, or IgG4) (e.g., an antigen-binding fragment of a human or humanized IgG, e.g., human or humanized IgG1, IgG2, IgG3, or IgG4); or an antigen-binding fragment of an IgM (e.g., an antigen-binding fragment of a human or humanized IgM).

Non-limiting examples of anti-TNFα agents that are antibodies that specifically bind to TNFα are described in Ben-Horin et al., Autoimmunity Rev. 13(1):24-30, 2014; Bongartz et al., JAMA 295(19):2275-2285, 2006; Butler et al., Eur. Cytokine Network 6(4):225-230, 1994; Cohen et al., Canadian J. Gastroenterol. Hepatol. 15(6):376-384, 2001; Elliott et al., Lancet 1994; 344: 1125-1127, 1994; Feldmann et al., Ann. Rev. Immunol. 19(1):163-196, 2001; Rankin et al., Br. J. Rheumatol. 2:334-342, 1995; Knight et al., Molecular Immunol. 30(16):1443-1453, 1993; Lorenz et al., J. Immunol. 156(4):1646-1653, 1996; Hinshaw et al., Circulatory Shock 30(3):279-292, 1990; Ordas et al., Clin. Pharmacol. Therapeutics 91(4):635-646, 2012; Feldman, Nature Reviews Immunol. 2(5):364-371, 2002; Taylor et al., Nature Reviews Rheumatol. 5(10):578-582, 2009; Garces et al., Annals Rheumatic Dis. 72(12):1947-1955, 2013; Palladino et al., Nature Rev. Drug Discovery 2(9):736-746, 2003; Sandborn et al., Inflammatory Bowel Diseases 5(2):119-133, 1999; Atzeni et al., Autoimmunity Reviews 12(7):703-708, 2013; Maini et al., Immunol. Rev. 144(1):195-223, 1995; Wanner et al., Shock 11(6):391-395, 1999; and U.S. Patent Nos. 6,090,382; 6,258,562; and 6,509,015).

In certain embodiments, the anti-TNFα agent can include or is golimumab (golimumabTM), adalimumab (Humira™), infliximab (Remicade™), CDP571, CDP 870, or certolizumab pegol (Cimzia™). In certain embodiments, the anti-TNFα agent can be a TNFα inhibitor biosimilar. Examples of approved and late-phase TNFα inhibitor biosimilars include, but are not limited to, infliximab biosimilars such as Flixabi™ (SB2) from Samsung Bioepis, Inflectra® (CT-P13) from Celltrion/Pfizer, GS071 from Aprogen, Remsima™, PF-06438179 from Pfizer/Sandoz, NI-071 from Nichi-Iko Pharmaceutical Co., and ABP 710 from Amgen; adalimumab biosimilars such as Amgevita® (ABP 501) from Amgen and Exemptia™ from Zydus Cadila, BMO-2 or MYL-1401-A from Biocon/Mylan, CHS-1420 from Coherus, FKB327 from Kyowa Kirin, and BI 695501 from Boehringer Ingelheim;Solymbic®, SB5 from Samsung Bioepis, GP-2017 from Sandoz, ONS-3010 from Oncobiologics, M923 from Momenta, PF-06410293 from Pfizer, and etanercept biosimilars such as Erelzi™ from Sandoz/Novartis, Brenzys™ (SB4) from Samsung Bioepis, GP2015 from Sandoz, TuNEX® from Mycenax, LBEC0101 from LG Life, and CHS-0214 from Coherus.

In some embodiments of any of the methods described herein, the anti-TNF$\alpha$ agent is selected from the group consisting of: adalimumab, certolizumab, etanercept, golimumab, infliximabm, CDP571, and CDP 870.

In some embodiments, any of the antibodies or antigen-binding fragments described herein has a dissociation constant ($K_D$) of less than $1 \times 10^{-5}$ M (e.g., less than $0.5 \times 10^{-5}$ M, less than $1 \times 10^{-6}$ M, less than $0.5 \times 10^{-6}$ M, less than $1 \times 10^{-7}$ M, less than $0.5 \times 10^{-7}$ M, less than $1 \times 10^{-8}$ M, less than $0.5 \times 10^{-8}$ M, less than $1 \times 10^{-9}$ M, less than $0.5 \times 10^{-9}$ M, less than $1 \times 10^{-10}$ M, less than $0.5 \times 10^{-10}$ M, less than $1 \times 10^{-11}$ M, less than $0.5 \times 10^{-11}$ M, or less than $1 \times 10^{-12}$ M), e.g., as measured in phosphate buffered saline using surface plasmon resonance (SPR).

In some embodiments, any of the antibodies or antigen-binding fragments described herein has a $K_D$ of about $1 \times 10^{-12}$ M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, about $0.5 \times 10^{-7}$ M, about $1 \times 10^{-8}$ M, about $0.5 \times 10^{-8}$ M, about $1 \times 10^{-9}$ M, about $0.5 \times 10^{-9}$ M, about $1 \times 10^{-10}$ M, about $0.5 \times 10^{-10}$ M, about $1 \times 10^{-11}$ M, or about $0.5 \times 10^{-11}$ M (inclusive); about $0.5 \times 10^{-11}$ M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, about $0.5 \times 10^{-7}$ M, about $1 \times 10^{-8}$ M, about $0.5 \times 10^{-8}$ M, about $1 \times 10^{-9}$ M, about $0.5 \times 10^{-9}$ M, about $1 \times 10^{-10}$ M, about $0.5 \times 10^{-10}$ M, or about $1 \times 10^{-11}$ M (inclusive); about $1 \times 10^{-11}$ M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, about $0.5 \times 10^{-7}$ M, about $1 \times 10^{-8}$ M, about $0.5 \times 10^{-8}$ M, about $1 \times 10^{-9}$M, about $0.5 \times 10^{-9}$ M, about $1 \times 10^{-10}$ M, or about $0.5 \times 10^{-10}$ M (inclusive); about $0.5 \times 10^{-10}$ M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, about $0.5 \times 10^{-7}$ M, about $1 \times 10^{-8}$ M, about $0.5 \times 10^{-8}$ M, about $1 \times 10^{-9}$ M, about $0.5 \times 10^{-9}$ M, or about $1 \times 10^{-10}$ M (inclusive); about $1 \times 10^{-10}$ M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, about $0.5 \times 10^{-7}$ M, about $1 \times 10^{-8}$ M, about $0.5 \times 10^{-8}$ M, about $1 \times 10^{-9}$M, or about $0.5 \times 10^{-9}$ M (inclusive); about $0.5 \times 10^{-9}$M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, about $0.5 \times 10^{-7}$ M, about $1 \times 10^{-8}$ M, about $0.5 \times 10^{-8}$ M, or about $1 \times 10^{-9}$ M (inclusive); about $1 \times 10^{-9}$ M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, about $0.5 \times 10^{-7}$ M, about $1 \times 10^{-8}$ M, or about $0.5 \times 10^{-8}$ M (inclusive); about $0.5 \times 10^{-8}$ M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, about $0.5 \times 10^{-7}$ M, or about $1 \times 10^{-8}$ M (inclusive); about $1 \times 10^{-8}$ M to about $1 \times 10^{-5}$ M, about $0.5 \times 10^{-5}$ M, about $1 \times 10^{-6}$ M, about $0.5 \times 10^{-6}$ M, about $1 \times 10^{-7}$ M, or about $0.5 \times 10^{-7}$ M (inclusive); about $0.5 \times 10^{-7}$ M to about $1 \times 10^{-5}$ M, about $0.5 \times 10^{-5}$ M, about $1 \times 10^{-6}$ M, about $0.5 \times 10^{-6}$ M, or about $1 \times 10^{-7}$ M (inclusive); about $1 \times 10^{-7}$ M to about $1 \times 10^{-5}$ M, about $0.5 \times 10^{-5}$ M, about $1 \times 10^{-6}$ M, or about $0.5 \times 10^{-6}$ M (inclusive); about $0.5 \times 10^{-6}$ M to about $1 \times 10^{-5}$ M, about $0.5 \times 10^{-5}$ M, or about $1 \times 10^{-6}$ M (inclusive); about $1 \times 10^{-6}$ M to about $1 \times 10^{-5}$ M or about $0.5 \times 10^{-5}$ M (inclusive); or about $0.5 \times 10^{-5}$ M to about $1 \times 10^{-5}$ M (inclusive), e.g., as measured in phosphate buffered saline using surface plasmon resonance (SPR).

In some embodiments, any of the antibodies or antigen-binding fragments described herein has a $K_{off}$ of about $1 \times 10^{-6}$ $s^{-1}$ to about $1 \times 10^{-3}$ $s^{-1}$, about $0.5 \times 10^{-3}$ $s^{-1}$, about $1 \times 10^{-4}$ $s^{-1}$, about $0.5 \times 10^{-4}$ $s^{-1}$, about $1 \times 10^{-5}$ $s^{-1}$, or about $0.5 \times 10^{-5}$ $s^{-1}$ (inclusive); about $0.5 \times 10^{-5}$ $s^{-1}$ to about $1 \times 10^{-3}$ $s^{-1}$, about $0.5 \times 10^{-3}$ $s^{-1}$, about $1 \times 10^{-4}$ $s^{-1}$, about $0.5 \times 10^{-4}$ $s^{-1}$, or about $1 \times 10^{-5}$ $s^{-1}$ (inclusive); about $1 \times 10^{-5}$ $s^{-1}$ to about $1 \times 10^{-3}$ $s^{-1}$, about $0.5 \times 10^{-3}$ $s^{-1}$, about $1 \times 10^{-4}$ $s^{-1}$, or about $0.5 \times 10^{-4}$ $s^{-1}$ (inclusive); about $0.5 \times 10^{-4}$ $s^{-1}$ to about $1 \times 10^{-3}$ $s^{-1}$, about $0.5 \times 10^{-3}$ $s^{-1}$, or about $1 \times 10^{-4}$ $s^{-1}$ (inclusive); about $1 \times 10^{-4}$ $s^{-1}$ to about $1 \times 10^{-3}$ $s^{-1}$, or about $0.5 \times 10^{-3}$ $s^{-1}$ (inclusive); or about $0.5 \times 10^{-5}$ $s^{-1}$ to about $1 \times 10^{-3}$ $s^{-1}$ (inclusive), e.g., as measured in phosphate buffered saline using surface plasmon resonance (SPR).

In some embodiments, any of the antibodies or antigen-binding fragments described herein has a $K_{on}$ of about $1 \times 10^{2}$ $M^{-1}s^{-1}$ to about $1 \times 10^{6}$ $M^{-1}s^{-1}$, about $0.5 \times 10^{6}$ $M^{-1}s^{-1}$, about $1 \times 10^{5}$ $M^{-1}s^{-1}$, about $0.5 \times 10^{5}$ $M^{-1}s^{-1}$, about $1 \times 10^{4}$ $M^{-1}s^{-1}$, about $0.5 \times 10^{4}$ $M^{-1}s^{-1}$, about $1 \times 10^{3}$ $M^{-1}s^{-1}$, or about $0.5 \times 10^{3}$ $M^{-1}s^{-1}$ (inclusive); about $0.5 \times 10^{3}$ $M^{-1}s^{-1}$ to about $1 \times 10^{6}$ $M^{-1}s^{-1}$, about $0.5 \times 10^{6}$ $M^{-1}s^{-1}$, about $1 \times 10^{5}$ $M^{-1}s^{-1}$, about $0.5 \times 10^{5}$ $M^{-1}s^{-1}$, about $1 \times 10^{4}$ $M^{-1}s^{-1}$, about $0.5 \times 10^{4}$ $M^{-1}s^{-1}$, or about $1 \times 10^{3}$ $M^{-1}s^{-1}$ (inclusive); about $1 \times 10^{3}$ $M^{-1}s^{-1}$ to about $1 \times 10^{6}$ $M^{-1}s^{-1}$, about $0.5 \times 10^{6}$ $M^{-1}s^{-1}$, about $1 \times 10^{5}$ $M^{-1}s^{-1}$ , about $0.5 \times 10^{5}$ $M^{-1}s^{-1}$, about $1 \times 10^{4}$ $M^{-1}s^{-1}$, or about $0.5 \times 10^{4}$ $M^{-1}s^{-1}$ (inclusive); about $0.5 \times 10^{4}$ $M^{-1}s^{-1}$ to about $1 \times 10^{6}$ $M^{-1}s^{-1}$, about $0.5 \times 10^{6}$ $M^{-1}s^{-1}$, about $1 \times 10^{5}$ $M^{-1}s^{-1}$, about $0.5 \times 10^{5}$ $M^{-1}s^{-1}$, or about $1 \times 10^{4}$ $M^{-1}s^{-1}$ (inclusive); about $1 \times 10^{4}$ $M^{-1}s^{-1}$ to about $1 \times 10^{6}$ $M^{-1}s^{-1}$, about $0.5 \times 10^{6}$ $M^{-1}s^{-1}$, about $1 \times 10^{5}$ $M^{-1}s^{-1}$, or about $0.5 \times 10^{5}$ $M^{-1}s^{-1}$ (inclusive); about $0.5 \times 10^{5}$ $M^{-1}s^{-1}$ to about $1 \times 10^{6}$ $M^{-1}s^{-1}$, about $0.5 \times 10^{6}$ $M^{-1}s^{-1}$, or about $1 \times 10^{5}$ $M^{-1}s^{-1}$ (inclusive); about $1 \times 10^{5}$ $M^{-1}s^{-1}$ to about $1 \times 10^{6}$ $M^{-1}s^{-1}$, or about $0.5 \times 10^{6}$ $M^{-1}s^{-1}$ (inclusive); or about $0.5 \times 10^{6}$ $M^{-1}s^{-1}$ to about $1 \times 10^{6}$ $M^{-1}s^{-1}$ (inclusive), e.g., as measured in phosphate buffered saline using surface plasmon resonance (SPR).

***Fusion Proteins** (only disclosed for reference purposes and not as part of the invention)*

In some embodiments, the anti-TNF$\alpha$ agent is a fusion protein (e.g., an extracellular domain of a TNFR fused

to a partner peptide, e.g., an Fc region of an immunoglobulin, e.g., human IgG) (see, e.g., Deeg et al., Leukemia 16(2):162, 2002; Peppel et al., J. Exp. Med. 174(6):1483-1489, 1991) or a soluble TNFR (e.g., TNFR1 or TNFR2) that binds specifically to TNFα. In some embodiments, the anti-TNFα agent includes or is a soluble TNFα receptor (e.g., Bjornberg et al., Lymphokine Cytokine Res. 13(3):203-211, 1994; Kozak et al., Am. J. Physiol. Reg. Integrative Comparative Physiol. 269(1):R23-R29, 1995; Tsao et al., Eur Respir J. 14(3):490-495, 1999; Watt et al., J Leukoc Biol. 66(6):1005-1013, 1999; Mohler et al., J. Immunol. 151(3): 1548-1561, 1993; Nophar et al., EMBO J. 9(10):3269, 1990; Piguet et al., Eur. Respiratory J. 7(3):515-518, 1994; and Gray et al., Proc. Natl. Acad. Sci. U.S.A. 87(19):7380-7384, 1990). In some embodiments, the anti-TNFα agent includes or is etanercept (Enbrel™) (see, e.g., WO 91/03553 and WO 09/406,476, incorporated by reference herein). In some embodiments, the anti-TNFα agent inhibitor includes or is r-TBP-I (e.g., Gradstein et al., J. Acquir. Immune Defic. Syndr. 26(2): 111-117, 2001).

***Inhibitory Nucleic Acids*** *(only disclosed for reference purposes and not as part of the invention)*

An antisense nucleic acid molecule can be fully or partially complementary to all or part of a non-coding region of the coding strand of a nucleotide sequence encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein. Non-coding regions (5' and 3' untranslated regions) are the 5' and 3' sequences that flank the coding region in a gene and are not translated into amino acids.

Based upon the sequences disclosed herein, one of skill in the art can easily choose and synthesize any of a number of appropriate antisense nucleic acids to target a nucleic acid encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-xB, NIK, p38, PKR, rac, ras, raf, RIP, TNFα, TNFR1, TNFR2, TRADD, TRAF2, TRAF6, or TTP protein described herein. Antisense nucleic acids targeting a nucleic acid encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein can be designed using the software available at the Integrated DNA Technologies website.

An antisense nucleic acid can be, for example, about 5, 10, 15, 18, 20, 22, 24, 25, 26, 28, 30, 32, 35, 36, 38, 40, 42, 44, 45, 46, 48, or 50 nucleotides or more in length. An antisense oligonucleotide can be constructed using enzymatic ligation reactions and chemical synthesis using procedures known in the art. For example, an antisense nucleic acid can be chemically synthesized using variously modified nucleotides or naturally occurring nucleotides designed to increase the physical stability of the duplex formed between the antisense and sense nucleic acids, e.g., phosphorothioate derivatives and acridine substituted nucleotides or to increase the biological stability of the molecules.

Examples of modified nucleotides which can be used to generate an antisense nucleic acid include 1-methylguanine, 1-methylinosine, 2,2-dimethylguanine, 2-methyladenine, 2-methylguanine, 3-methylcytosine, 2-methylthio-N6-isopentenyladenine, uracil-5-oxyacetic acid (v), wybutoxosine, pseudouracil, queosine, 2-thiocytosine, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest).

The antisense nucleic acid molecules described herein can be prepared in vitro and administered to a subject, e.g., a human subject. Alternatively, they can be generated in situ such that they hybridize with or bind to cellular mRNA and/or genomic DNA encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein to thereby inhibit expression, e.g., by inhibiting transcription and/or translation. The hybridization can be by conventional nucleotide complementarities to form a stable duplex, or, for example, in the case of an antisense nucleic acid molecule that binds to DNA duplexes, through specific interactions in the major groove of the double helix. The antisense nucleic acid molecules can be delivered to a mammalian cell using a vector (e.g., an adenovirus vector, a lentivirus, or a retrovirus).

An antisense nucleic acid can be an α-anomeric nucleic acid molecule. An α-anomeric nucleic acid molecule forms specific double-stranded hybrids with complementary RNA in which, contrary to the usual, β-units, the strands run parallel to each other (Gaultier et al., Nucleic Acids Res. 15:6625-6641, 1987). The antisense nucleic acid can also comprise a chimeric RNA-DNA analog (Inoue et al., FEBS Lett. 215:327-330, 1987) or a 2'-O-methylribonucleotide (Inoue et al., Nucleic Acids Res. 15:6131-6148, 1987).

Another example of an inhibitory nucleic acid is a ribozyme that has specificity for a nucleic acid encoding an

AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA, e.g., specificity for any one of the sequences presented in Table E). Ribozymes are catalytic RNA molecules with ribonuclease activity that are capable of cleaving a single-stranded nucleic acid, such as an mRNA, to which they have a complementary region. Thus, ribozymes (e.g., hammerhead ribozymes (described in Haselhoff and Gerlach, Nature 334:585-591, 1988)) can be used to catalytically cleave mRNA transcripts to thereby inhibit translation of the protein encoded by the mRNA. An AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA can be used to select a catalytic RNA having a specific ribonuclease activity from a pool of RNA molecules. See, e.g., Bartel et al., Science 261:1411-1418, 1993.

Alternatively, a ribozyme having specificity for an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA can be designed based upon the nucleotide sequence of any of the AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA sequences disclosed herein (e.g., in Table E). For example, a derivative of a Tetrahymena L-19 IVS RNA can be constructed in which the nucleotide sequence of the active site is complementary to the nucleotide sequence to be cleaved in an AP-1, ASK1, CD14, c-jun, ERK1/2, IxB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA (see, e.g., U.S. Patent. Nos. 4,987,071 and 5,116,742).

An inhibitory nucleic acid can also be a nucleic acid molecule that forms triple helical structures. For example, expression of an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP polypeptide can be inhibited by targeting nucleotide sequences complementary to the regulatory region of the gene encoding the AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP polypeptide (e.g., the promoter and/or enhancer, e.g., a sequence that is at least 1 kb, 2 kb, 3 kb, 4 kb, or 5 kb upstream of the transcription initiation start state) to form triple helical structures that prevent transcription of the gene in target cells. See generally Maher, Bioassays 14(12):807-15, 1992; Helene, Anticancer Drug Des. 6(6):569-84, 1991; and Helene, Ann. N.Y. Acad. Sci. 660:27-36, 1992.

In various embodiments, inhibitory nucleic acids can be modified at the sugar moiety, the base moiety, or phosphate backbone to improve, e.g., the solubility, stability, or hybridization, of the molecule. For example, the deoxyribose phosphate backbone of the nucleic acids can be modified to generate peptide nucleic acids (see, e.g., Hyrup et al., Bioorganic Medicinal Chem. 4(1):5-23, 1996). Peptide nucleic acids (PNAs) are nucleic acid mimics, e.g., DNA mimics, in which the deoxyribose phosphate backbone is replaced by a pseudopeptide backbone and only the four natural nucleobases are retained. The neutral backbone of PNAs allows for specific hybridization to RNA and DNA under conditions of low ionic strength. PNA oligomers can be synthesized using standard solid phase peptide synthesis protocols (see, e.g., Perry-O'Keefe et al., Proc. Natl. Acad. Sci. U.S.A. 93:14670-675, 1996). PNAs can be used as antisense or antigene agents for sequence-specific modulation of gene expression by, e.g., inducing transcription or translation arrest or inhibiting replication.

An antisense nucleic acid molecule can be fully or partially complementary to all or part of a non-coding region of the coding strand of a nucleotide sequence encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein. Non-coding regions (5' and 3' untranslated regions) are the 5' and 3' sequences that flank the coding region in a gene and are not translated into amino acids.

Based upon the sequences disclosed herein, one of skill in the art can easily choose and synthesize any of a number of appropriate antisense nucleic acids to target a nucleic acid encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-xB, NIK, p38, PKR, rac, ras, raf, RIP, TNFα, TNFR1, TNFR2, TRADD, TRAF2, TRAF6, or TTP protein described herein. Antisense nucleic acids targeting a nucleic acid encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein can be designed using the software available at the Integrated DNA Technologies web site.

An antisense nucleic acid can be, for example, about 5, 10, 15, 18, 20, 22, 24, 25, 26, 28, 30, 32, 35, 36, 38, 40, 42, 44, 45, 46, 48, or 50 nucleotides or more in length. An antisense oligonucleotide can be constructed using enzymatic ligation reactions and chemical synthesis using procedures known in the art. For example, an antisense nucleic acid can be chemically synthesized using variously modified nucleotides or naturally occurring nucleotides designed to increase the physical stability of the duplex formed between the antisense and sense nucleic acids, e.g., phosphorothioate derivatives and acridine substituted nucleotides or to increase the biological stability of the molecules.

Examples of modified nucleotides which can be used to generate an antisense nucleic acid include 1-methylguanine, 1-methylinosine, 2,2-dimethylguanine, 2-methyladenine, 2-methylguanine, 3-methylcytosine, 2-methylthio-N6-isopentenyladenine, uracil-5-oxyacetic acid (v), wybutoxosine, pseudouracil, queosine, 2-thiocytosine, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest).

The antisense nucleic acid molecules described herein can be prepared in vitro and administered to a subject, e.g., a human subject. Alternatively, they can be generated in situ such that they hybridize with or bind to cellular mRNA and/or genomic DNA encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein to thereby inhibit expression, e.g., by inhibiting transcription and/or translation. The hybridization can be by conventional nucleotide complementarities to form a stable duplex, or, for example, in the case of an antisense nucleic acid molecule that binds to DNA duplexes, through specific interactions in the major groove of the double helix. The antisense nucleic acid molecules can be delivered to a mammalian cell using a vector (e.g., an adenovirus vector, a lentivirus, or a retrovirus).

An antisense nucleic acid can be an α-anomeric nucleic acid molecule. An α-anomeric nucleic acid molecule forms specific double-stranded hybrids with complementary RNA in which, contrary to the usual, β-units, the strands run parallel to each other (Gaultier et al., Nucleic Acids Res. 15:6625-6641, 1987). The antisense nucleic acid can also comprise a chimeric RNA-DNA analog (Inoue et al., FEBS Lett. 215:327-330, 1987) or a 2'-O-methylribonucleotide (Inoue et al., Nucleic Acids Res. 15:6131-6148, 1987).

Another example of an inhibitory nucleic acid is a ribozyme that has specificity for a nucleic acid encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA, e.g., specificity for any one of the sequences presented in Table E). Ribozymes are catalytic RNA molecules with ribonuclease activity that are capable of cleaving a single-stranded nucleic acid, such as an mRNA, to which they have a complementary region. Thus, ribozymes (e.g., hammerhead ribozymes (described in Haselhoff and Gerlach, Nature 334:585-591, 1988)) can be used to catalytically cleave mRNA transcripts to thereby inhibit translation of the protein encoded by the mRNA. An AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA can be used to select a catalytic RNA having a specific ribonuclease activity from a pool of RNA molecules. See, e.g., Bartel et al., Science 261:1411-1418, 1993.

Alternatively, a ribozyme having specificity for an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA can be designed based upon the nucleotide sequence of any of the AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA sequences disclosed herein (e.g., in Table E). For example, a derivative of a Tetrahymena L-19 IVS RNA can be constructed in which the nucleotide sequence of the active site is complementary to the nucleotide sequence to be cleaved in an AP-1, ASK1, CD14, c-jun, ERK1/2, IxB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA (see, e.g., U.S. Patent. Nos. 4,987,071 and 5,116,742).

An inhibitory nucleic acid can also be a nucleic acid molecule that forms triple helical structures. For example, expression of an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP polypeptide can be inhibited by targeting nucleotide sequences complementary to the regulatory region of the gene encoding the AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP polypeptide (e.g., the promoter and/or enhancer, e.g., a sequence that is at least 1 kb, 2 kb, 3 kb, 4 kb, or 5 kb upstream of the transcription initiation start state) to form triple helical structures that prevent transcription of the gene in target cells. See generally Maher, Bioassays 14(12):807-15, 1992; Helene, Anticancer Drug Des. 6(6):569-84, 1991; and Helene, Ann. N.Y. Acad. Sci. 660:27-36, 1992.

In various embodiments, inhibitory nucleic acids can be modified at the sugar moiety, the base moiety, or

phosphate backbone to improve, e.g., the solubility, stability, or hybridization, of the molecule. For example, the deoxyribose phosphate backbone of the nucleic acids can be modified to generate peptide nucleic acids (see, e.g., Hyrup et al., Bioorganic Medicinal Chem. 4(1):5-23, 1996). Peptide nucleic acids (PNAs) are nucleic acid mimics, e.g., DNA mimics, in which the deoxyribose phosphate backbone is replaced by a pseudopeptide backbone and only the four natural nucleobases are retained. The neutral backbone of PNAs allows for specific hybridization to RNA and DNA under conditions of low ionic strength. PNA oligomers can be synthesized using standard solid phase peptide synthesis protocols (see, e.g., Perry-O'Keefe et al., Proc. Natl. Acad. Sci. U.S.A. 93:14670-675, 1996). PNAs can be used as antisense or antigene agents for sequence-specific modulation of gene expression by, e.g., inducing transcription or translation arrest or inhibiting replication.

Inhibitory nucleic acids that can decrease the expression of AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA expression in a mammalian cell include antisense nucleic acid molecules, i.e., nucleic acid molecules whose nucleotide sequence is fully or partially complementary to all or part of a AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA (e.g., fully or partially complementary to all or a part of any one of the sequences presented in Table E).

Table E.

| **Human gene** | **mRNA GenBank accession number (s)** |
|---|---|
| Tumor necrosis factor (TNF, a.k.a. TNF-alpha) | NM_000594 |
| TNF receptor superfamily member 1A (TNFRSF1A) (a.k.a. TNFR1) | NM_001065<br>NM_001346091<br>NM_001346092 |
| TNF receptor superfamily member 1B (TNFRSF1B) (a.k.a. TNFR2) | NM_001066<br>XM_011542060<br>XM_011542063<br>XM_017002214<br>XM_017002215<br>XM_017002211 |
| TNFRSF1A associated via death domain (TRADD) | NM_003789<br>NM_001323552<br>XM_005256213<br>XM_017023815 |
| TNF receptor associated factor 2 (TRAF2) | NM_021138<br>XM_011518976<br>XM_011518977<br>XM_011518974 |
| JunD proto-oncogene, AP-1 transcription factor subunit (JUND) | NM_001286968<br>NM_005354 |
| Mitogen-activated protein kinase kinase kinase 5 (MAP3K5) (a.k.a. ASK1) | NM_005923<br>XM_017010875<br>XM_017010872<br>XM_017010873<br>XM_017010877<br>XM_017010874<br>XM_017010871<br>XM_017010870<br>XM_017010876<br>XM_011535839 |

(continued)

| Human gene | mRNA GenBank accession number (s) |
|---|---|
| CD14 | NM_000591<br>NM_001040021<br>NM_001174104<br>NM_001174105 |
| Mitogen-activated protein kinase 3 (MAPK3) (a.k.a. ERK1) | NM_001040056<br>NM_001109891<br>NM_002746 |
| Mitogen-activated protein kinase 1 (MAPK1) (a.k.a. ERK2) | NM_002745<br>NM_138957 |
| Inhibitor of nuclear factor kappa B kinase subunit beta (IKBKB) | NM_001190720<br>NM_001242778<br>NM_001556<br>XM_005273491<br>XM_005273496<br>XM_005273493<br>XM_005273498<br>XM_011544518<br>XM_005273492<br>XM_005273490<br>XM_005273494<br>12XM_017013396<br>XM_011544521<br>XM_011544522<br>XM_005273495<br>XM_011544517<br>XM_011544520<br>XM_011544519 |
| NFKB inhibitor alpha (NFKBIA) | NM_020529 |
| Interleukin 1 receptor associated kinase 1 (IRAK 1) | NM_001025242<br>NM_001025243<br>NM_001569<br>XM_005274668 |

(continued)

| Human gene | mRNA GenBank accession number (s) |
|---|---|
| Mitogen-activated protein kinase 8 (MAPK8) (a.k.a. JNK) | NM_001278547<br>NM_001278548<br>NM_001323302<br>NM_001323320<br>NM_001323321<br>NM_001323322<br>NM_001323323<br>NM_001323324<br>NM_001323325<br>NM_001323326<br>NM_001323327<br>NM_001323328<br>NM_001323329<br>NM_001323330<br>NM_001323331<br>NM_139046<br>NM_139049<br>XM_024448079<br>XM_024448080 |
| Lipopolysaccharide binding protein (LBP) | NM_004139 |
| Mitogen-activated protein kinase kinase 1 (MAP2K1) (a.k.a. MEK1) | NM_002755<br>XM_017022411<br>XM_011521783 |
| | XM_017022412<br>XM_017022413 |
| Mitogen-activated protein kinase kinase 2 (MAP2K2) (a.k.a. MEK2) | NM_030662<br>XM_006722799<br>XM_017026990<br>XM_017026989<br>XM_017026991 |
| Mitogen-activated protein kinase kinase 3 (MAP2K3) (a.k.a. MEK3) | NM_001316332<br>NM_002756<br>NM_145109<br>XM_017024859<br>XM_005256723<br>XM_017024857<br>XM_011523959<br>XM_017024858<br>XM_011523958 |
| Mitogen-activated protein kinase kinase 6 (MAP2K6) (a.k.a. MEK6) | NM_001330450<br>NM_002758<br>XM_005257516<br>XM_011525027<br>XM_011525026<br>XM_006721975 |

(continued)

| Human gene | mRNA GenBank accession number (s) |
|---|---|
| Mitogen-activated protein kinase kinase kinase 1 (MAP3K1) (a.k.a. MEKK1) | NM_005921<br>XM_017009485<br>XM_017009484 |
| Mitogen-activated protein kinase kinase kinase 3 (MAP3K3) (a.k.a. MEKK3) | NM_001330431<br>NM_001363768<br>NM_002401<br>NM_203351<br>XM_005257378 |
| Mitogen-activated protein kinase kinase kinase 4 (MAP3K4) (a.k.a. MEKK4) | NM_001291958<br>NM_001301072<br>NM_001363582<br>NM_005922<br>NM_006724<br>XM_017010869 |
| Mitogen-activated protein kinase kinase kinase 6 (MAP3K6) (a.k.a. MEKK6) | NM_001297609<br>NM_004672<br>XM_017002771<br>XM_017002772 |
| Mitogen-activated protein kinase kinase kinase 7 (MAP3K7) (a.k.a. MEKK7) | NM_003188<br>NM_145331<br>NM_145332<br>NM_145333<br>XM_006715553<br>XM_017011226 |
| MAPK activated protein kinase 2 (MAPKAPK2) (a.k.a. MK2) | NM_004759<br>NM_032960<br>XM_005273353<br>XM_017002810 |
| MYD88, innate immune signal transduction adaptor (MYD88) | NM_001172566<br>NM_001172567<br>NM_001172568<br>NM_001172569<br>NM_001365876<br>NM_001365877<br>NM_002468 |
| Nuclear factor kappa B subunit 1 (NFKB 1) | NM_001165412<br>NM_001319226<br>NM_003998<br>XM_024454069<br>XM_024454067<br>XM_011532006<br>XM_024454068 |
| Mitogen-activated protein kinase kinase kinase 14 (MAP3K14) (a.k.a. NIK) | NM_003954<br>XM_011525441 |

(continued)

| Human gene | mRNA GenBank accession number (s) |
|---|---|
| Mitogen-activated protein kinase 14 (MAPK 14) (a.k.a. p38) | NM_001315<br>NM_139012<br>NM_139013<br>NM_139014<br>XM_011514310<br>XM_017010300<br>XM_017010299<br>XM_017010301<br>XM_017010304<br>XM_017010303<br>XM_017010302<br>XM_006714998 |
| Eukaryotic translation initiation factor 2 alpha kinase 2 (EIF2AK2) (a.k.a. PKR) | NM_001135651<br>NM_001135652<br>NM_002759<br>XM_011532987<br>XM_017004503 |
| AKT serine/threonine kinase 1 (AKT1) (a.k.a. RAC) | NM_001014431<br>NM_001014432<br>NM_005163 |
| Zinc fingers and homeoboxes 2 (ZHX2) (a.k.a. RAF) | NM_001362797<br>NM_014943<br>XM_011516932<br>XM_005250836 |
| KRAS proto-oncogene, GTPase (KRAS) | NM_001369786<br>NM_001369787 |
| | NM_004985<br>NM_033360 |
| NRAS proto-oncogene, GTPase (NRAS) | NM_002524 |
| Receptor interacting serine/threonine kinase 1 (RIPK1) (a.k.a. RIP) | NM_001317061<br>NM_001354930<br>NM_001354931<br>NM_001354932<br>NM_001354933<br>NM_001354934<br>NM_003804<br>XM_017011405<br>XM_006715237<br>XM_017011403<br>XM_017011404 |
| TNF receptor associated factor 6 (TRAF6) | NM_004620<br>NM_145803<br>XM_017018220 |
| ZFP36 ring finger protein (ZFP36) (a.k.a. TTP) | NM_003407 |

***Small Molecules*** *(only disclosed for reference purposes and not as part of the invention)*

In some embodiments, the anti-TNFα agent is a small molecule. In some embodiments, the small molecule is a tumor necrosis factor-converting enzyme (TACE) inhibitor (e.g., Moss et al., Nature Clinical Practice Rheumatology 4: 300-309, 2008). In some embodiments, the anti-TNFα agent is C87 (Ma et al., J. Biol. Chem. 289(18):12457-66, 2014). In some embodiments, the small molecule is LMP-420 (e.g., Haraguchi et al., AIDS Res. Ther. 3:8, 2006). In some embodiments, the TACE inhibitor is TMI-005 and BMS-561392. Additional examples of small molecule inhibitors are described in, e.g., He et al., Science 310(5750):1022-1025, 2005.

In some examples, the anti-TNFα agent is a small molecule that inhibits the activity of one of AP-1, ASK1, IKK, JNK, MAPK, MEKK 1/4, MEKK4/7, MEKK 3/6, NIK, TRADD, RIP, NF-κB, and TRADD in a cell (e.g., in a cell obtained from a subject, a mammalian cell).

In some examples, the anti-TNFα agent is a small molecule that inhibits the activity of one of CD14, MyD88 (see, e.g., Olson et al., Scientific Reports 5:14246, 2015), ras (e.g., Baker et al., Nature 497:577-578, 2013), raf (e.g., vemurafenib (PLX4032, RG7204), sorafenib tosylate, PLX-4720, dabrafenib (GSK2118436), GDC-0879, RAF265 (CHIR-265), AZ 628, NVP-BHG712, SB590885, ZM 336372, sorafenib, GW5074, TAK-632, CEP-32496, encorafenib (LGX818), CCT196969, LY3009120, RO5126766 (CH5126766), PLX7904, and MLN2480).

In some examples, the anti-TNFα agent TNFα inhibitor is a small molecule that inhibits the activity of one of MK2 (PF 3644022 and PHA 767491), JNK (e.g., AEG 3482, BI 78D3, CEP 1347, c-JUN peptide, IQ 1S, JIP-1 (153-163), SP600125, SU 3327, and TCS JNK6o), c-jun (e.g., AEG 3482, BI 78D3, CEP 1347, c-JUN peptide, IQ 1S, JIP-1 (153-163), SP600125, SU 3327, and TCS JNK6o), MEK3/6 (e.g., Akinleye et al., J. Hematol. Oncol. 6:27, 2013), p38 (e.g., AL 8697, AMG 548, BIRB 796, CMPD-1, DBM 1285 dihydrochloride, EO 1428, JX 401, ML 3403, Org 48762-0, PH 797804, RWJ 67657, SB 202190, SB 203580, SB 239063, SB 706504, SCIO 469, SKF 86002, SX 011, TA 01, TA 02, TAK 715, VX 702, and VX 745), PKR (e.g., 2-aminopurine or CAS 608512-97-6), TTP (e.g., CAS 329907-28-0), MEK1/2 (e.g., Facciorusso et al., Expert Review Gastroentrol. Hepatol. 9:993-1003, 2015), ERK1/2 (e.g., Mandal et al., Oncogene 35:2547-2561, 2016), NIK (e.g., Mortier et al., Bioorg. Med. Chem. Lett. 20:4515-4520, 2010), IKK (e.g., Reilly et al., Nature Med. 19:313-321, 2013), IκB (e.g., Suzuki et al., Expert. Opin. Invest. Drugs 20:395-405, 2011), NF-κB (e.g., Gupta et al., Biochim. Biophys. Acta 1799(10-12):775-787, 2010), rac (e.g., U.S. Patent No. 9,278,956), MEK4/7, IRAK (Chaudhary et al., J. Med. Chem. 58(1):96-110, 2015), LBP (see, e.g., U.S. Patent No. 5,705,398), and TRAF6 (e.g., 3-[(2,5-Dimethylphenyl)amino]-1-phenyl-2-propen-1-one).

In some embodiments of any of the methods described herein, the inhibitory nucleic acid can be about 10 nucleotides to about 50 nucleotides (e.g., about 10 nucleotides to about 45 nucleotides, about 10 nucleotides to about 40 nucleotides, about 10 nucleotides to about 35 nucleotides, about 10 nucleotides to about 30 nucleotides, about 10 nucleotides to about 28 nucleotides, about 10 nucleotides to about 26 nucleotides, about 10 nucleotides to about 25 nucleotides, about 10 nucleotides to about 24 nucleotides, about 10 nucleotides to about 22 nucleotides, about 10 nucleotides to about 20 nucleotides, about 10 nucleotides to about 18 nucleotides, about 10 nucleotides to about 16 nucleotides, about 10 nucleotides to about 14 nucleotides, about 10 nucleotides to about 12 nucleotides, about 12 nucleotides to about 50 nucleotides, about 12 nucleotides to about 45 nucleotides, about 12 nucleotides to about 40 nucleotides, about 12 nucleotides to about 35 nucleotides, about 12 nucleotides to about 30 nucleotides, about 12 nucleotides to about 28 nucleotides, about 12 nucleotides to about 26 nucleotides, about 12 nucleotides to about 25 nucleotides, about 12 nucleotides to about 24 nucleotides, about 12 nucleotides to about 22 nucleotides, about 12 nucleotides to about 20 nucleotides, about 12 nucleotides to about 18 nucleotides, about 12 nucleotides to about 16 nucleotides, about 12 nucleotides to about 14 nucleotides, about 15 nucleotides to about 50 nucleotides, about 15nucleotides to about 45 nucleotides, about 15nucleotides to about 40 nucleotides, about 15nucleotides to about 35 nucleotides, about 15 nucleotides to about 30 nucleotides, about 15nucleotides to about 28 nucleotides, about 15nucleotides to about 26 nucleotides, about 15nucleotides to about 25 nucleotides, about 15nucleotides to about 24 nucleotides, about 15nucleotides to about 22 nucleotides, about 15nucleotides to about 20 nucleotides, about 15nucleotides to about 18 nucleotides, about 15nucleotides to about 16 nucleotides, about 16 nucleotides to about 50 nucleotides, about 16 nucleotides to about 45 nucleotides, about 16 nucleotides to about 40 nucleotides, about 16 nucleotides to about 35 nucleotides, about 16 nucleotides to about 30 nucleotides, about 16 nucleotides to about 28 nucleotides, about 16 nucleotides to about 26 nucleotides, about 16 nucleotides to about 25 nucleotides, about 16 nucleotides to about 24 nucleotides, about 16 nucleotides to about 22 nucleotides, about 16 nucleotides to about 20 nucleotides, about 16 nucleotides to about 18 nucleotides, about 18 nucleotides to about 20 nucleotides, about 20 nucleotides to about 50 nucleotides, about 20 nucleotides to about 45 nucleotides, about 20 nucleotides to about 40 nucleotides, about 20 nucleotides to about 35 nucleotides, about 20 nucleotides to about 30 nucleotides, about 20 nucleotides to about 28 nucleotides, about 20 nucleotides to about 26 nucleotides, about 20 nucleotides to about 25 nucleotides, about 20 nucleotides to about 24 nucleotides, about 20 nucleotides to about 22 nucleotides, about 24 nucleotides to about 50 nucleotides, about 24 nucleotides to about 45 nucleotides, about 24 nucleotides to about 40 nucleotides, about 24 nucleotides to about 35 nucleotides, about 24 nucleotides to about 30 nucleotides, about 24

nucleotides to about 28 nucleotides, about 24 nucleotides to about 26 nucleotides, about 24 nucleotides to about 25 nucleotides, about 26 nucleotides to about 50 nucleotides, about 26 nucleotides to about 45 nucleotides, about 26 nucleotides to about 40 nucleotides, about 26 nucleotides to about 35 nucleotides, about 26 nucleotides to about 30 nucleotides, about 26 nucleotides to about 28 nucleotides, about 28 nucleotides to about 50 nucleotides, about 28 nucleotides to about 45 nucleotides, about 28 nucleotides to about 40 nucleotides, about 28 nucleotides to about 35 nucleotides, about 28 nucleotides to about 30 nucleotides, about 30 nucleotides to about 50 nucleotides, about 30 nucleotides to about 45 nucleotides, about 30 nucleotides to about 40 nucleotides, about 30 nucleotides to about 38 nucleotides, about 30 nucleotides to about 36 nucleotides, about 30 nucleotides to about 34 nucleotides, about 30 nucleotides to about 32 nucleotides, about 32 nucleotides to about 50 nucleotides, about 32 nucleotides to about 45 nucleotides, about 32 nucleotides to about 40 nucleotides, about 32 nucleotides to about 35 nucleotides, about 35 nucleotides to about 50 nucleotides, about 35 nucleotides to about 45 nucleotides, about 35 nucleotides to about 40 nucleotides, about 40 nucleotides to about 50 nucleotides, about 40 nucleotides to about 45 nucleotides, about 42 nucleotides to about 50 nucleotides, about 42 nucleotides to about 45 nucleotides, or about 45 nucleotides to about 50 nucleotides) in length. One skilled in the art will appreciate that inhibitory nucleic acids may comprises at least one modified nucleic acid at either the 5' or 3' end of DNA or RNA.

In some embodiments, the inhibitory nucleic acid can be formulated in a liposome, a micelle (e.g., a mixed micelle), a nanoemulsion, or a microemulsion, a solid nanoparticle, or a nanoparticle (e.g., a nanoparticle including one or more synthetic polymers). Additional exemplary structural features of inhibitory nucleic acids and formulations of inhibitory nucleic acids are described in US 2016/0090598.

In some embodiments, the inhibitory nucleic acid (e.g., any of the inhibitory nucleic acid described herein) can include a sterile saline solution (e.g., phosphate-buffered saline (PBS)). In some embodiments, the inhibitory nucleic acid (e.g., any of the inhibitory nucleic acid described herein) can include a tissue-specific delivery molecule (e.g., a tissue-specific antibody).

In one embodiment, provided herein is a combination of a compound of any preceding embodiment, for use in the treatment or the prevention of a condition mediated by TNF-$\alpha$, in a patient in need thereof, wherein the compound is administered to said patient at a therapeutically effective amount. Preferably, the subject is resistant to treatment with an anti-TNF$\alpha$ agent. Preferably, the condition is a gut disease or disorder.

In one embodiment, provided herein is a pharmaceutical composition of comprising a compound of any preceding embodiment, and an anti-TNFa agent disclosed herein. Preferably wherein the anti-TNFa agent is Infliximab, Etanercept, Certolizumab pegol, Golimumab or Adalimumab, more preferably wherein the anti-TNFa agent is Adalimumab.

In one embodiment, provided herein is a pharmaceutical combination of a compound of any preceding embodiment, and an anti-TNFa agent Preferably wherein the anti-TNFa agent is Infliximab, Etanercept, Certolizumab pegol, Golimumab or Adalimumab, more preferably wherein the anti-TNF$\alpha$ agent is Adalimumab.

In one embodiment, the present invention relates to an NLRP3 antagonist for use in the treatment or the prevention of a condition mediated by TNF-$\alpha$, in particular a gut disease or disorder, in a patient in need thereof, wherein the NLRP3 antagonist is administered to said patient at a therapeutically effective amount.

In one embodiment, the present invention relates to an NLRP3 antagonist for use in the treatment or the prevention of a condition, in particular a gut disease or disorder, in a patient in need thereof wherein the NLRP3 antagonist is administered to said patient at a therapeutically effective amount.

In one embodiment, the present invention relates to an NLRP3 antagonist for use in the treatment, stabilization or lessening the severity or progression of gut disease or disorder, in a patient in need thereof wherein the NLRP3 antagonist is administered to said patient at a therapeutically effective amount.

In one embodiment, the present invention relates to an NLRP3 antagonist for use in the slowing, arresting, or reducing the development of a gut disease or disorder, in a patient in need thereof wherein the NLRP3 antagonist is administered to said patient at a therapeutically effective amount.

In one embodiment, the present invention relates to an NLRP3 antagonist for use according to above listed embodiments wherein the NLRP3 antagonist is a gut-targeted NLRP3 antagonist.

In one embodiment, the present invention relates ton NLRP3 antagonist for use according to any of the above embodiments, wherein the gut disease is IBD.

In one embodiment, the present invention relates to an NLRP3 antagonist for use according to any of the above embodiments, wherein the gut disease is US or CD.

In one embodiment, the present invention relates to a method for the treatment or the prevention of a condition mediated by TNF-$\alpha$, in particular a gut disease or disorder, in a patient in need thereof, comprising administering to said patient a therapeutically effective amount of a gut-targeted NLRP3 antagonist.

In one embodiment, the present invention relates to a method for the treatment or the prevention of a condition, in particular a gut disease or disorder, in a patient in need thereof, comprising administering to said patient a therapeutically effective amount of a gut-targeted NLRP3 antagonist.

In one embodiment, the present invention relates to a method for the treatment, stabilization or lessening the severity or progression of gut disease or disorder, in a patient in need thereof comprising administering to said patient a therapeutically effective amount of a gut-targeted NLRP3 antagonist.

In one embodiment, the present invention relates to a method for slowing, arresting, or reducing the development of a gut disease or disorder, in a patient in need thereof comprising administering to said patient a therapeutically effective amount of a gut-targeted NLRP3 antagonist.

In one embodiment, the present invention relates to a method according to any of the above embodiments, wherein the gut disease is IBD.

In one embodiment, the present invention relates to a method according to any of the above embodiments x to xx, wherein the gut disease is UC or CD.

In one embodiment, the present invention relates to a method for the treatment or the prevention of a condition mediated by TNF-$\alpha$, in particular a gut disease or disorder, in a patient in need thereof, comprising administering to said patient a therapeutically effective amount of a gut-targeted NLRP3 antagonist.

## Compound Preparation and Biological Assays

As can be appreciated by the skilled artisan, methods of synthesizing the compounds of the formulae herein will be evident to those of ordinary skill in the art. Synthetic chemistry transformations and protecting group methodologies (protection and deprotection) useful in synthesizing the compounds described herein are known in the art and include, for example, those such as described in R. Larock, Comprehensive Organic Transformations, VCH Publishers (1989); T. W. Greene and RGM. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

Preparative Examples (where

The following abbreviations have the indicated meanings:

ACN = acetonitrile

BTC = trichloromethyl chloroformate

Boc = *t*-butyloxy carbonyl

Davephos = 2-Dicyclohexylphosphino-2'-(N,N-dimethylamino)biphenyl

DCM = dichloromethane

DEA = diethylamine

DMF = N,N-dimethylformamide

DMSO = dimethyl sulfoxide

DIEA = N,N-diisopropylethylamine

DPPA = diphenylphosphoryl azide

dppf = 1,1 '-Bi s(diphenylphosphino)ferrocene

EtOH = ethanol

HATU = 1-[Bis(dimethylamino)methylene]-1H-1,2,3-triazolo[4,5-b]pyridinium 3-oxid hexafluorophosphate

Hex = hexane

HPLC = high performance liquid chromatography

LC-MS = liquid chromatography - mass spectrometry

LiHMDS = lithium bis(trimethylsilyl)amide

LDA = lithium diisopropylamide

M = mol/L

Me = methyl

MeOH = methanol

MSA = methanesulfonic acid

NBS = N-bromosuccinimide

NCS = N-chlorosuccinimide

NMR = nuclear magnetic resonance

Pd(dppf)$Cl_2$ = dichloro[1, 1'-bis(diphenylphosphino)ferrocene]palladium

Ph = phenyl

$PPh_3Cl_2$ = dichlorotriphenylphosphorane

Py = pyridine

RT = room temperature

Rt = Retention time

$R_f$ = Retardation factor

Sat. = saturated

TBAF = tetrabutylammonium fluoride

TBS = tert-butyldimethylsilyl

TBSCl = tert-butyldimethylsilyl chloride

TBDPSCl = tert-butyldiphenylsilyl chloride

TEA = triethylamine

TFA = trifluoroacetic acid

THF = tetrahydrofuran

TLC = thin layer chromatography

TsOH = 4-methylbenzenesulfonic acid

UV = ultraviolet

Burgess reagent = (Methoxycarbonylsulfamoyl)triethylammonium hydroxide inner salt

DIAD = Diisopropylazodicarboxylate

DIBAL-H = Diisobutylaluminum hydride

DPPA = Diphenylphosphoryl azide

LAH = Lithium aluminum hydride

NFSI = N-fluoro benzene sulfonamide

NMO = 4-Methylmorpholine N-oxide

$Pd(dtbpf)Cl_2$= [1,1'-Bis(di-tert-butylphosphino)ferrocene]dichloropalladium(II)

Ruphos = 2-Dicyclohexylphosphino-2,6-diisopropoxy-1,1-biphenyl

SEM-Cl = 2-(Trimethylsilyl)ethoxymethyl chloride

TsCl = 4-Methylbenzenesulfonyl chloride

TPAP = Tetrapropylammonium perruthenate

**General**

The progress of reactions was often monitored by TLC or LC-MS. The identity of the products was often confirmed by LC-MS. The LC-MS was recorded using one of the following methods.

Method A: Shim-pack XR-ODS, C18, 3x50 mm, 2.5 um column, 1.0 uL injection, 1.5 mL/min flow rate, 90-900 amu scan range, 190-400 nm UV range, 5-100% (1.1 min), 100% (0.6 min) gradient with ACN (0.05% TFA) and water (0.05% TFA), 2 minute total run time.

Method B: Kinetex EVO, C18, 3×50 mm, 2.2 um column, 1.0 uL injection, 1.5 mL/min flow rate, 90-900 amu scan range, 190-400 nm UV range, 10-95% (1.1 min), 95% (0.6 min) gradient with ACN and water (0.5% $NH_4HCO_3$ ), 2 minute total run time.

Method C: Shim-pack XR-ODS, C18, 3x50 mm, 2.5 um column, 1.0 uL injection, 1.5 mL/min flow rate, 90-900 amu scan range, 190-400 nm UV range, 5-100% (2.1 min), 100% (0.6 min) gradient with ACN (0.05% TFA) and water (0.05% TFA), 3 minute total run time.

Method D: Kinetex EVO, C18, 3x50 mm, 2.2 um column, 1.0 uL injection, 1.5 mL/min flow rate, 90-900 amu scan range, 190-400 nm UV range, 10-95% (2.1 min), 95% (0.6 min) gradient with ACN and water (0.5% $NH_4HCO_3$ ), 3 minute total run time.

Method F: Phenomenex, CH0-7644, Onyx Monolithic C18, 50 x 4.6 mm, 10.0 uL injection, 1.5 mL/min flow rate, 100-1500 amu scan range, 220 and 254 nm UV detection, 5% with ACN (0.1% TFA) to 100% water (0.1% TFA) over 9.5 min, with a stay at 100% (ACN, 0.1% TFA) for 1 min, then equilibration to 5% (ACN, 0.1% TFA) over 1.5 min.

The final targets were purified by Prep-HPLC. The Prep-HPLC was carried out using the following method.

Method E: Prep-HPLC: Column, XBridge Shield RP18 OBD (19x250 mm, 10 um); mobile phase, Water (10mmol/L $NH_4HCO_3$) and ACN, UV detection 254/210 nm.

Method G: Prep-HPLC: Higgins Analytical Proto 200, C18 Column, 250 x 20 mm, 10 um; mobile phase, Water (0.1% TFA) and ACN (0.1% TFA), UV detection 254/210 nm.

NMR was recorded on BRUKER NMR 300.03 MHz, DUL-C-H, ULTRASHIELD™300, AVANCE II 300 B-ACS™120 or BRUKER NMR 400.13 MHz, BBFO, ULTRASHIELD™400, AVANCE III 400, B-ACS™120 or BRUKER AC 250 NMR instrument with TMS as reference measured in ppm (part per million).

Racemic compounds of this invention as falling under the claims can be resolved to give individual enantiomers using a variety of known methods. For example, chiral stationary phases can used and the elution conditions can include normal phase or super-critical fluid with or without acidic or basic additives. Enantiomerically pure acids or bases can be used to form diatereomeric salts with the racemic compounds whereby pure enantiomers can be obtained by fractional crystallization. The racemates can also be derivatized with enantiomerically pure auxiliary reagents to form diastereomeric mixtures that can be separated. The auxiliary is then removed to give pure enantiomers.

**Schemes for the preparation of final targets:**

The preparation methods and intermediates presented below do not form part of the invention but are merely disclosed to provide information for the manufacture of the claimed compounds. As far as the Schemes and intermediates do not allow for the manufacture for the compounds defined in the claims, they are merely presented for reference purposes.

**Schemes 1-3** below illustrate several conditions used for coupling of sulfonimidamide **1** or **5** and isocyanate **2** to afford aminocarbonyl sulfonimidamide **4** via **3** or **6** after deprotection. As used in the schemes, rings "A" and "B" may be substituted as disclosed herein.

**Scheme 1:**

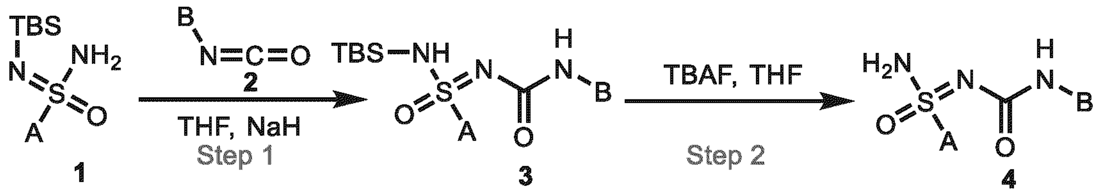


**Scheme 2:**

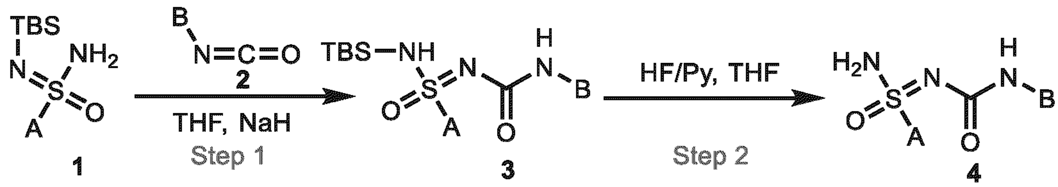


**Scheme 3:**

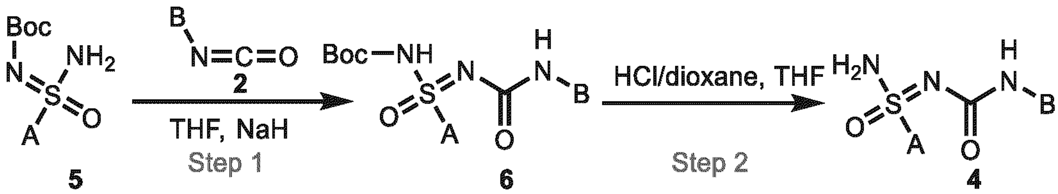


**Scheme 3A:**

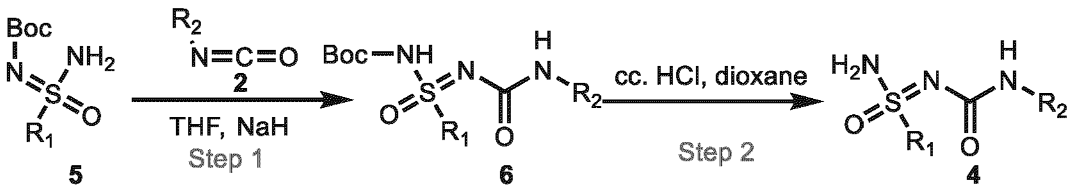


**Scheme 3B:**

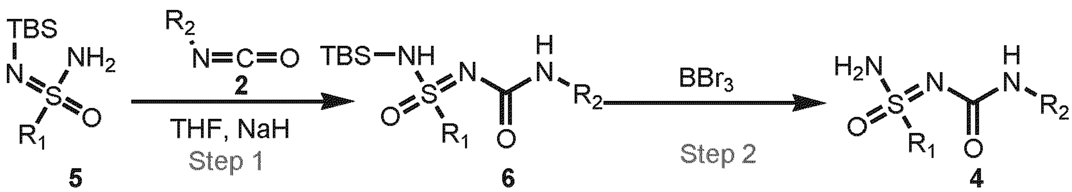


**Scheme 4** below illustrates the coupling between sulfonimidamide **7** and isocyanate **2** to provide sulfonimida-

mide 8.

**Scheme 4:**

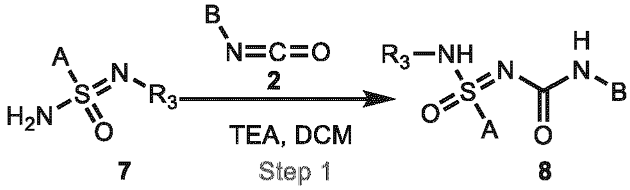


7 8

**Scheme 4A:**

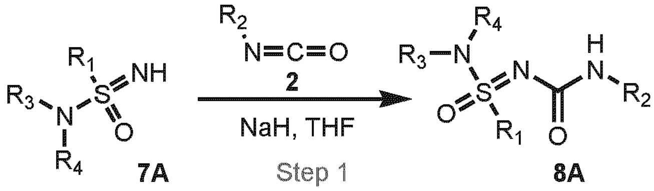


7A 8A

**Scheme 5** below illustrates the conversion of carboxylic acid **9** through Curtius rearrangement to isocyanate **2** via acyl azide **10,** whereupon coupling between **2** and sulfonimidamide **5** affords aminocarbonyl sulfonimidamide **4.**

**Scheme 5:**

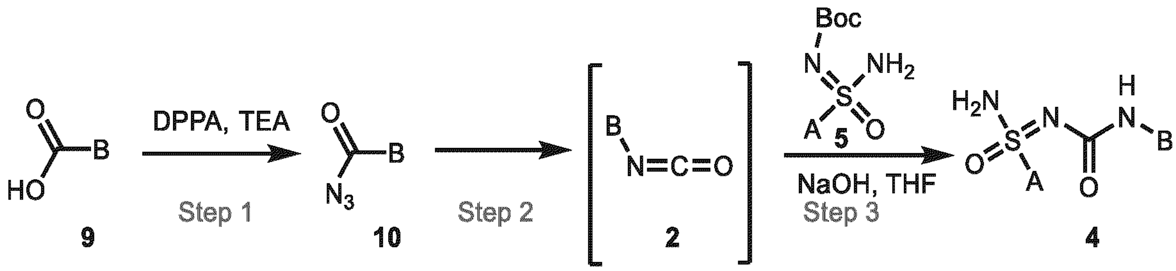


9 10 2 4

**Scheme of final targets:** Schemes below illustrate several conditions used for coupling of sulfonimidamide and isocyanate to afford aminocarbonyl sulfonimidamide.

**Scheme I:**

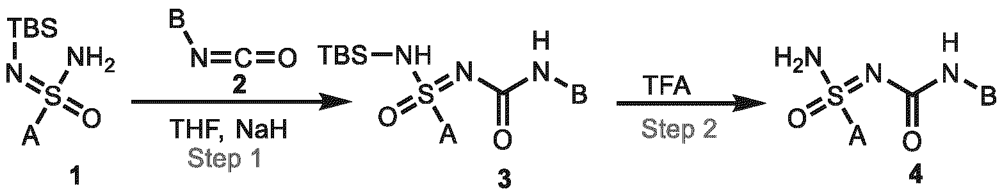


1 3 4

**Scheme II:**

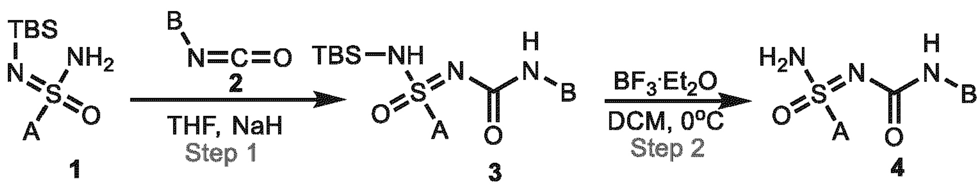


1 3 4

## Scheme III:

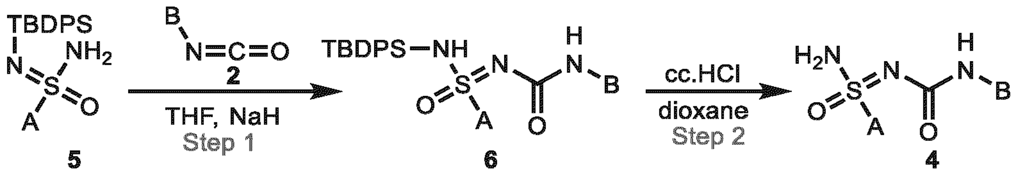


## Scheme IV:

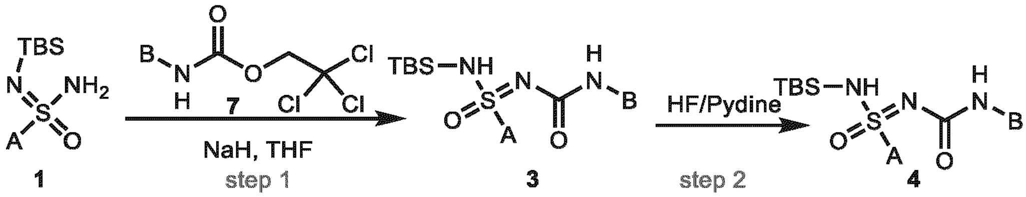


## Scheme V:

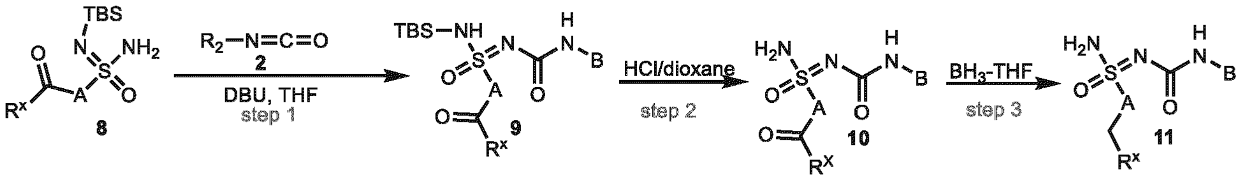


## Scheme VI:

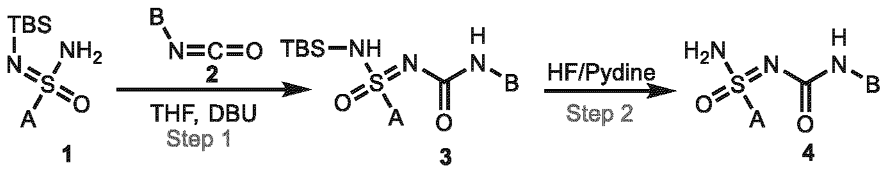


## Scheme VII:

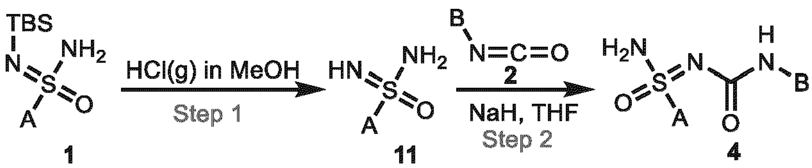


## Scheme VIII:

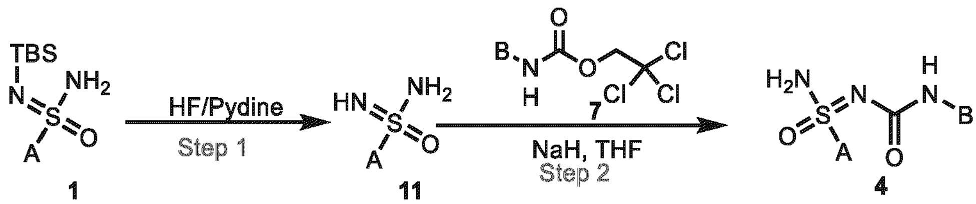


**Scheme IX:**

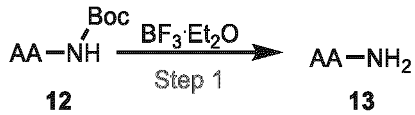


**Scheme X:**

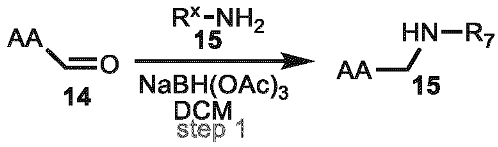


**Scheme XI:**

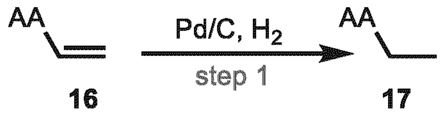


**Scheme XII:**

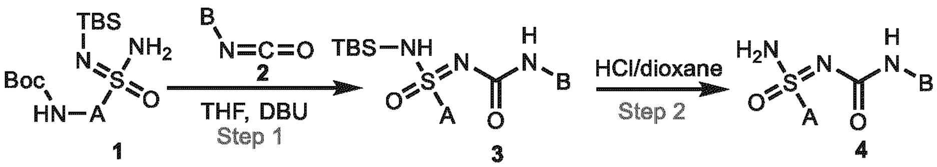


**Scheme XIII:**

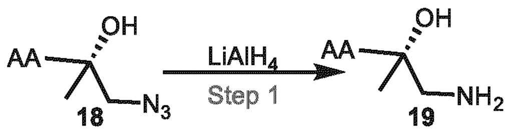


**Scheme XIV:**

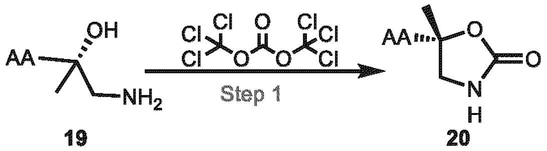


**Scheme XV:**

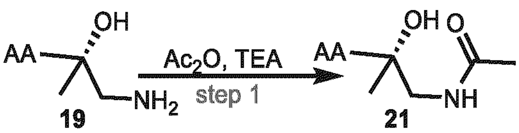

**Scheme XVI:**

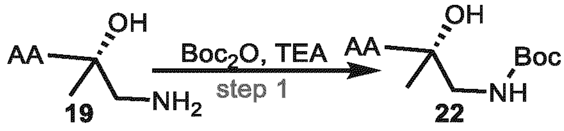


19 22

**Scheme XVII:**

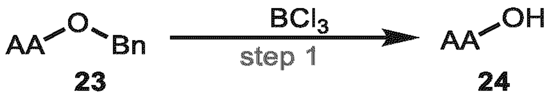


23 24

**Scheme of final targets:** Schemes below illustrate several conditions used for coupling of sulfonimidamide and isocyanate or isocyanate equivalents to afford aminocarbonyl sulfonimidamide.

**Scheme XVIII:**

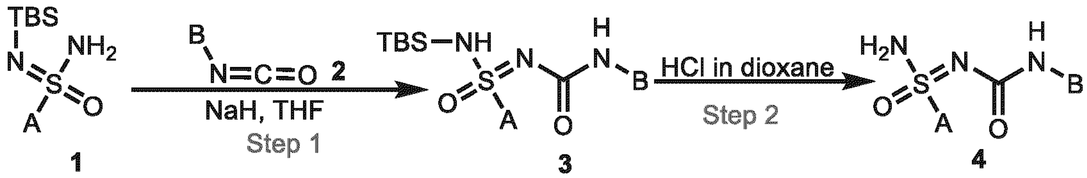


1 2 3 4

**Scheme XIX:**

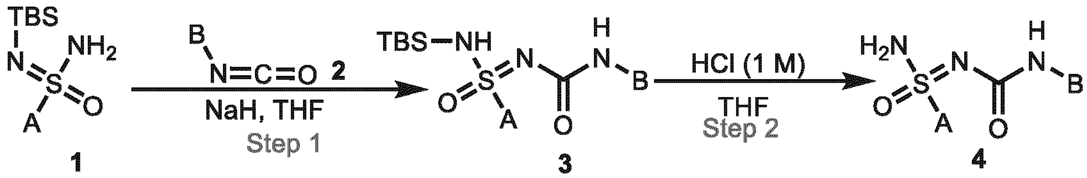


1 2 3 4

**Scheme XX:**

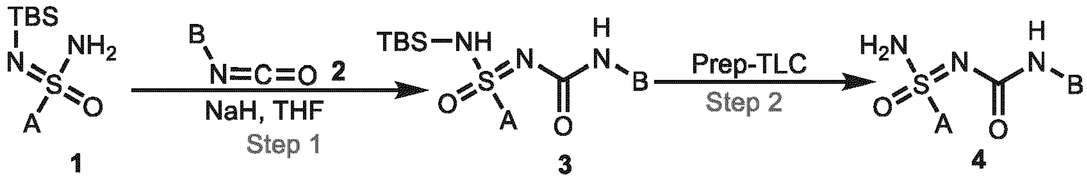


1 2 3 4

**Scheme XXI:**

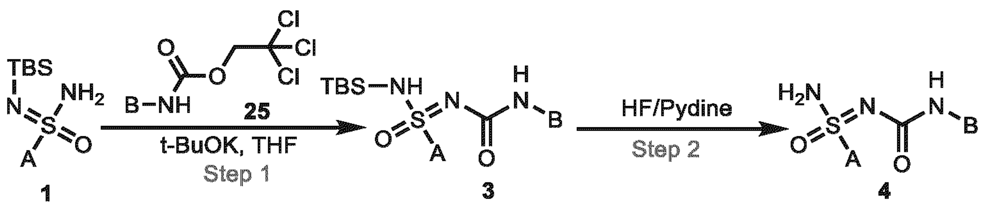


1 25 3 4

**Scheme XXII:**

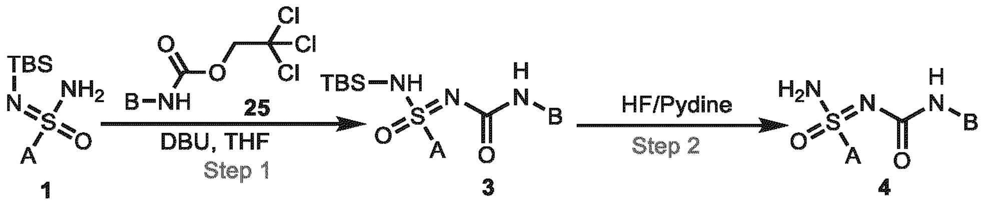


**Scheme XXIII:**

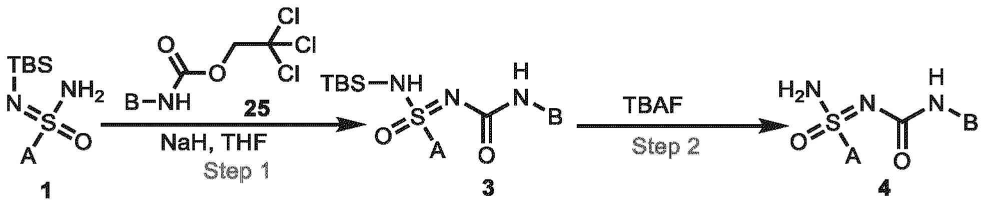


**Scheme XXIV:**

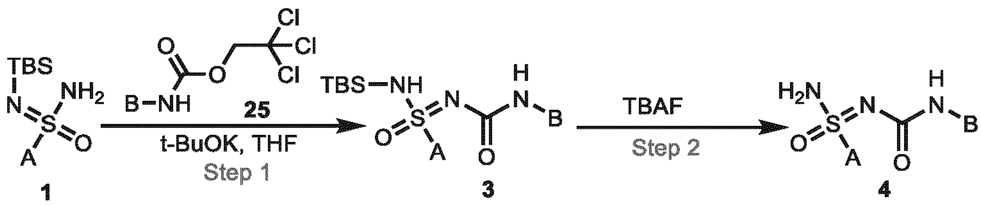


**Scheme XXV:**

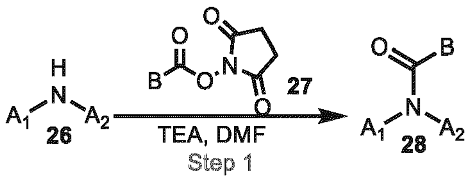


**Scheme XXVI:**

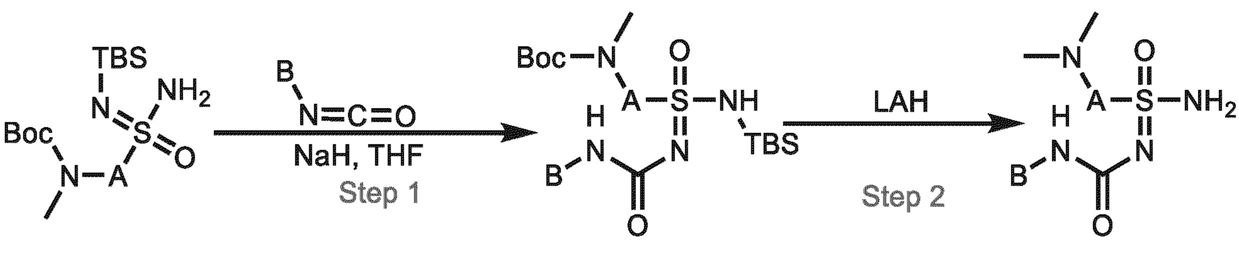


**Scheme XXVII:**

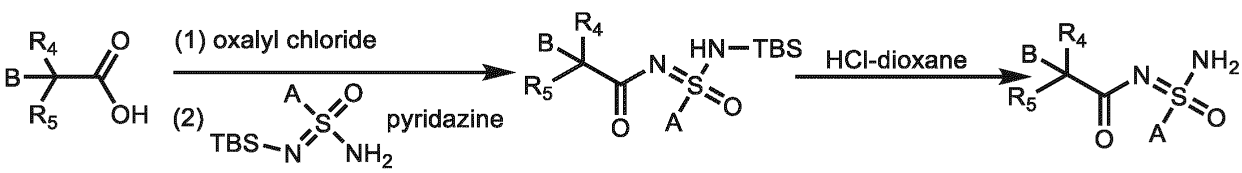

**Schemes for the preparation of Sulfonimidamide Intermediates 1-29:**

Schemes below illustrate the preparation of sulfonamide intermediates.

## Scheme 6:

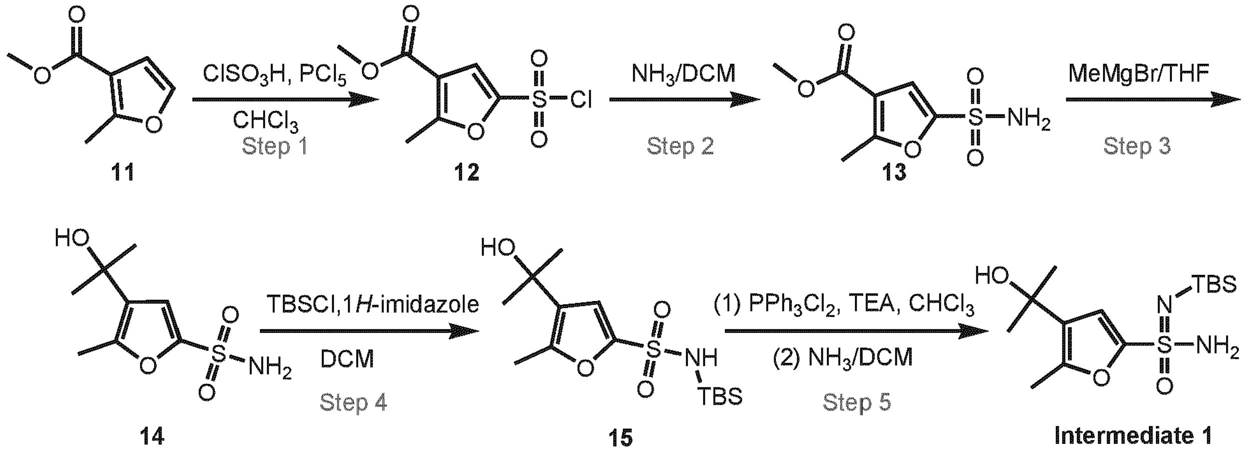


11 12 13

14 15 Intermediate 1

**Intermediate 1**

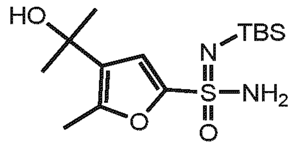

N'-(*tert*-bulyldimethylsilyl)-4-(2-hydroxypropan-2-yl)-5-methylfuran-2-sulfonimidamide

**Step 1: Methyl 5-(chlorosulfonyl)-2-methylfuran-3-carboxylate**

Into a 500-mL 3-necked round-bottom flask was placed methyl 2-methylfuran-3-carboxylate (7 g, 50 mmol) in $CHCl_3$ (200 mL). This was followed by the addition of chlorosulfonic acid (11.6 g, 100 mmol) dropwise with stirring at -10°C. The reaction mixture was stirred for 48 h at RT, after which the system was cooled to -10°C. Then to the above was added phosphorus pentachloride (22.9 g, 110 mmol). The resulting solution was stirred for 0.5 h at 50°C and then was quenched by pouring onto 200 mL of water/ice. The resulting mixture was extracted with 3x200 mL of DCM. The organic layers were combined and dried over anhydrous $Na_2SO_4$, and then concentrated under vacuum. This resulted in 7.5 g (crude, 63%) of the title compound as light brown oil. The crude product was used in the next step.

**Step 2: Methyl 2-methyl-5-sulfamoylfuran-3-carboxylate**

Into a 250-mL round-bottom flask was placed a solution of methyl 5-(chlorosulfonyl)-2-methylfuran-3-carboxylate (7.5 g, crude) in DCM (75 mL). To the above was added a saturated solution of ammonia in DCM (50 mL). The resulting solution was stirred for 3 h at RT and then was concentrated under vacuum. The residue was applied onto a silica gel column and eluted with a gradient of ethyl acetate/petroleum ether (1:4 to 1:2). This resulted in 5.0 g (46% over two steps) of the title compound as a light yellow solid. MS-ESI: 218.0 (M-1).

**Step 3: 4-(2-Hydroxypropan-2-yl)-5-methylfuran-2-sulfonamide**

Into a 250-mL 3-necked round-bottom flask purged with and maintained under nitrogen was placed a solution of methyl 2-methyl-5-sulfamoylfuran-3-carboxylate (3.7 g, 16.9 mmol) in THF (100 mL). This was followed by the addition of MeMgBr (3 M in THF, 25 mL) dropwise with stirring at -10°C. The resulting mixture was stirred for 10 h at RT and then was quenched by the addition of 50 mL of $NH_4Cl$ (sat.). The resulting solution was extracted with 3x50 mL of ethyl acetate. The organic layers were combined and dried over anhydrous $Na_2SO_4$, then concentrated under vacuum. The residue was applied onto a silica gel column and eluted with a gradient of ethyl acetate/petroleum ether (1:3 to 1:1). This resulted in 2.6 g (75%) of the title compound as a light yellow solid. MS-ESI: 218.0 (M-1).

**Step 4: N-(*tert*-butyldimethylsilyl)-4-(2-hydroxypropan-2-yl)-5-methylfuran-2-sulfonamide**

Into a 250-mL round-bottom flask purged with and maintained under nitrogen was placed 4-(2-hydroxypropan-2-yl)-5-methylfuran-2-sulfonamide (1.0 g, 4.56 mmol), DCM (100 mL), 1*H*-imidazole (612 mg, 9.12 mmol), and TBSCl (3.4 g, 22.6 mmol). The resulting solution was stirred for 14 h at RT and then was diluted with 100 mL of water. The resulting mixture was extracted with 3x50 mL of DCM and the organic layers were combined and concentrated under vacuum. The residue was applied onto a silica gel column and eluted with a gradient of ethyl acetate/petroleum ether (1:10 to 1:3). This resulted in 1.4 g (92%) of the title compound as a white solid. MS-ESI: 332.0 (M-1).

**Step 5: N'-(*tert*-butyldimethylsilyl)-4-(2-hydroxypropan-2-yl)-5-methylfuran-2-sulfonimidamide**

Into a 250-mL 3-necked round-bottom flask purged with and maintained under nitrogen was placed $PPh_3Cl_2$ (3.0 g, 10.2 mmol) in $CHCl_3$ (100 mL). This was followed by the addition of TEA (2.06 g, 20.4 mmol) dropwise with stirring at RT. After stirred at 0°C for 10 min, to the above was added a solution of N-(*tert*-butyldimethylsilyl)-4-(2-hydroxypropan-2-yl)-5-methylfuran-2-sulfonamide (2.3 g, 6.8 mmol) in $CHCl_3$ (10 mL) dropwise with stirring at 0°C. The resulting solution was allowed to react for 30 min at 0°C. To the mixture was added a saturated solution of ammonia in DCM (10 mL) at 0°C. The resulting solution was stirred for 2 h at RT. The reaction was then quenched by the addition of 100 mL of water. The resulting solution was extracted with 3x50 mL of DCM and the organic layers combined and concentrated under vacuum. The residue was applied onto a silica gel column and eluted with a gradient of ethyl acetate/petroleum ether (1:10 to 1:3). This resulted in 0.80 g (52.8%) of the title compound as a light yellow solid. MS-ESI: 333.0 (M+1).

Scheme 7A:

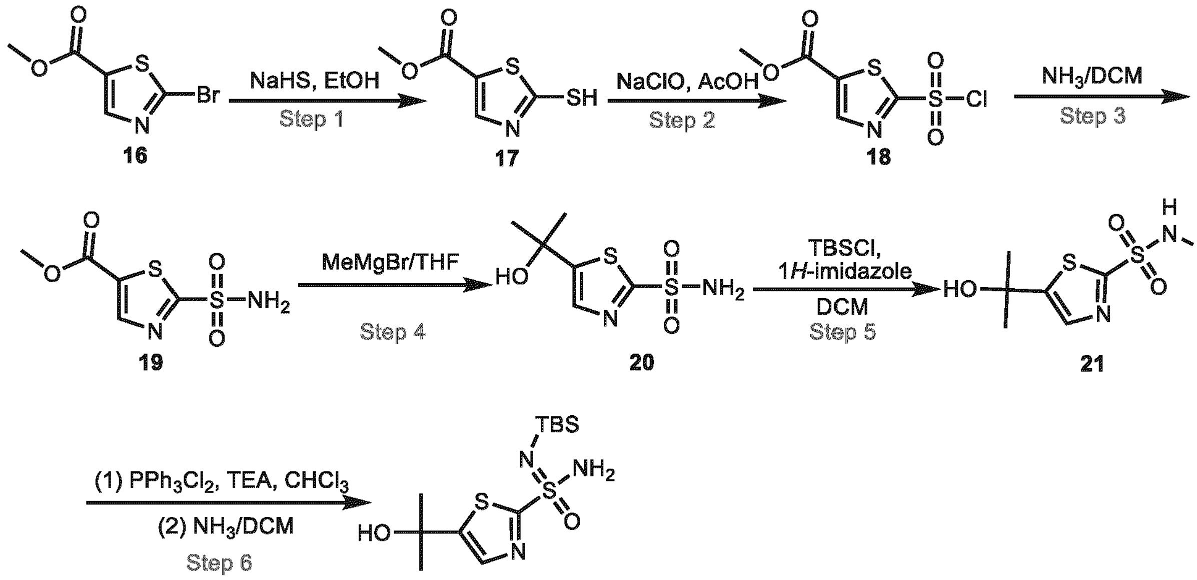

16 17 18 19 20 21

Intermediate 2

## Intermediate 2

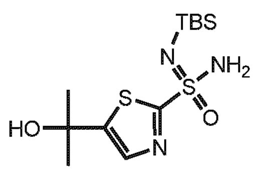

N'-(*tert*-butyldimethylsilyl)-5-(2-hydroxypropan-2-yl)thiazole-2-sulfonimidamide

**Step 1: Methyl 2-mercaptothiazole-5-carboxylate**

Into a 250-mL round-bottom flask was placed methyl 2-bromothiazole-5-carboxylate (10 g, 45 mmol), EtOH (100 mL), and sodium hydrogensulfide (5 g, 89 mmol). The resulting solution was stirred for 2 h at 80°C and then was cooled to 0°C with a water/ice bath. The pH value of the solution was adjusted to 3 with aq. HCl (1 N). The solids were collected by filtration. This resulted in 6 g (76%) of the title compound as a light yellow solid. MS-ESI: 176.0 (M+1).

**Step 2: Methyl 2-(chlorosulfonyl)thiazole-5-carboxylate**

Into a 250-mL round-bottom flask was placed methyl 2-mercaptothiazole-5-carboxylate (6 g, 34 mmol) and acetic acid (60 mL). This was followed by the addition of sodium hypochlorite (60 mL, 8%-10% wt.) in portions at 0°C. The resulting solution was stirred for 1 h at RT and then was diluted with 100 mL of water. The solution was extracted with 3x50 mL of DCM. The organic layers were combined and dried over anhydrous $Na_2SO_4$, then concentrated under vacuum. This resulted in 5 g (crude, 60%) of the title compound as yellow oil. The crude product was used in the next step.

**Step 3-6** used similar procedure for converting **compound 12** to **Intermediate 1** shown in **Scheme 6** to afford Intermediate 2. MS-ESI: 336.1 (M+1).

**Scheme 7B:**

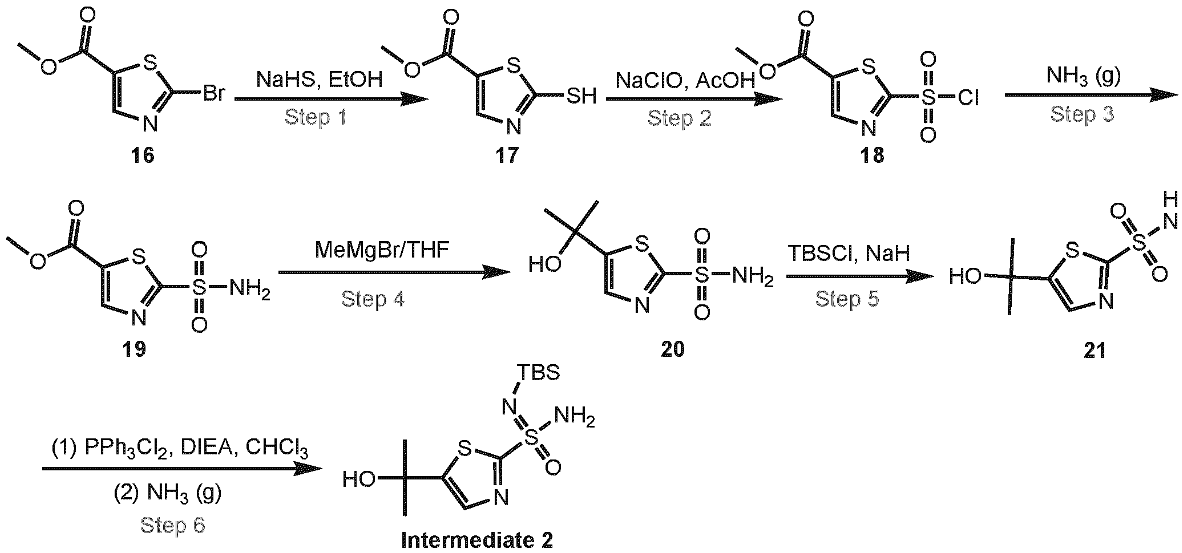


16 17 18 19 20 21 Intermediate 2

**Intermediate 2**

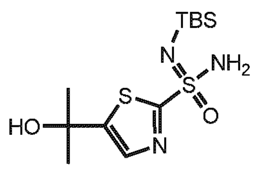

N'-(*tert*-butyldimethylsilyl)-5-(2-hydroxypropan-2-yl)thiazole-2-sulfonimidamide

**Step 1: Methyl 2-mercaptothiazole-5-carboxylate**

Into a 2-L round-bottom flask was placed methyl 2-bromothiazole-5-carboxylate (100 g, 450 mmol), EtOH (1000 mL), sodium hydrogensulfide (50 g, 890 mmol). The resulting solution was stirred for 2 h at 80°C and then was cooled to 0°C with a water/ice bath. The pH value of the solution was adjusted to 3 with hydrogen chloride (1 N). The solids were collected by filtration. This resulted in 63.2 g (80%) of the title compound as a light yellow solid. MS-ESI: 176.0 (M+1).

**Step 2: Methyl 2-(chlorosulfonyl)thiazole-5-carboxylate**

Into a 1-L round-bottom flask was placed methyl 2-mercaptothiazole-5-carboxylate (30 g, 170 mmol) and acetic acid (300 mL). This was followed by the addition of sodium hypochlorite (300 mL, 8%-10% wt.) in portions at 0°C. The resulting solution was stirred for 2 h at RT and then was diluted with 500 mL of water. The solution was extracted with 3x300 mL of DCM and the combined organic layers were washed with 2x300 mL of brine and dried over anhydrous $Na_2SO_4$. The crude product as a yellow solution in DCM was used in the next step.

**Step 3: Methyl 2-sulfamoylthiazole-5-carboxylate**

Into a 2-L round-bottom flask was placed methyl 2-(chlorosulfonyl)thiazole-5-carboxylate as a crude solution in DCM (900 mL). To the solution was introduced $NH_3$ (g) below 0°C for 20 minutes. The resulting solution was stirred for 1 h at RT and then concentrated under vacuum. The residue was applied onto a silica gel column and eluted with ethyl acetate/petroleum ether (1:5 to 1:3). This resulted in 23 g (75%, 2 steps) of the title compound as a white solid. MS-ESI: 223.0 (M+1).

**Step 4: 5-(2-Hydroxypropan-2-yl)thiazole-2-sulfonamide**

Into a 500-mL round-bottom flask purged with and maintained under nitrogen was placed a solution of methyl 2-sulfamoylthiazole-5-carboxylate (15 g, 67.5 mmol) in THF (150 mL). This was followed by the addition of MeMgBr/THF (3 M, 90 mL) dropwise with stirring at 0°C. The resulting solution was stirred for 14 h at RT and then was quenched by the addition of 100 mL of $NH_4Cl$ (sat.). The resulting solution was extracted with 3x150 mL of DCM. The organic layers were combined and dried over anhydrous $Na_2SO_4$, then concentrated under vacuum. The residue was applied onto a silica gel column and eluted with ethyl acetate/petroleum ether (1:5 to 1:3). This resulted in 11.5 g (78%) of the title compound as a white solid. MS-ESI: 223.0 (M+1), 221.0 (M-1) in positive and negative ion mode, respectively.

**Step 5: N-(*tert*-butyldimethylsilyl)-5-(2-hydroxypropan-2-yl)thiazole-2-sulfonamide**

Into a 250-mL 3-necked round-bottom flask purged with and maintained under nitrogen was placed a solution of 5-(2-hydroxypropan-2-yl)thiazole-2-sulfonamide (5 g, 22.5 mmol) in THF (100 mL). Then to the above was added NaH (60% wt, 1.8 g, 45.0 mmol) in portions in an ice/water bath. After stirring for 20 minutes in a water/ice bath, this was followed by the addition of a solution of TBSCl (4.1 g, 27.2 mmol) in THF (10 mL) dropwise with stirring at 0°C. The resulting solution was stirred for 4 h at RT. The reaction was quenched with sat. $NH_4Cl$ (100 mL). The resulting solution was extracted with 3 x 100 mL of ethyl acetate and the combined organic layers were dried over $Na_2SO_4$ and concentrated under vacuum. The crude solid was washed with ethyl acetate/hexane (1:5) (2x100 mL). This resulted in 6.81 g (90%) of the title compound as a yellow solid. MS-ESI: 337.1 (M+1), 335.1 (M-1) in positive and negative ion mode, respectively.

**Step 6: N'-(*tert*-butyldimethylsilyl)-5-(2-hydroxypropan-2-yl)thiazole-2-sulfonimidamide**

Into a 100-mL 3-necked round-bottom flask purged with and maintained under nitrogen was placed a solution of $PPh_3Cl_2$ (3 g, 9.0 mmol) in $CHCl_3$ (100 mL). This was followed by the addition of DIEA (1.54 g, 11.9 mmol) dropwise with stirring at RT. The resulting solution was stirred for 10 min at RT. This was followed by the addition of a solution of N-(tert-butyldimethylsilyl)-5-(2-hydroxypropan-2-yl)thiazole-2-sulfonamide (2.0 g, 5.9 mmol) in $CHCl_3$ (30 mL) dropwise with stirring in an ice/water bath. The resulting solution was stirred for 30 min in an ice/water bath. To the above was introduced $NH_3$ (g) below 0°C for 15 minutes. The resulting solution was stirred for 20 minutes at RT. The solids were filtered out and the filtrate was concentrated and the residue was dissolved in 300 mL of ethyl acetate. The solution was washed with brine (2x100 mL), dried over $Na_2SO_4$ and concentrated under vacuum. The crude solid was washed with $CHCl_3$ (100 mL). Then the filtrate was concentrated under vacuum and the residue was further purified by a silica gel column with ethyl acetate/petroleum ether (1:10 to 1:3). The original washed solid and solid from silica gel purification were combined. This resulted in 1.2 g (60%) of the title compound as a white solid. MS-ESI: 336.1 (M+1). $^1$H-NMR (300 MHz, DMSO-$d_6$) δ 7.66 (s, 1H), 7.12 (s, 2H), 5.78 (s, 1H), 1.51 (s, 6H), 0.86 (s, 9H), 0.02 (s, 3H), 0.01 (s, 3H).

**Table 2.** The Intermediate in the following Table was prepared using the similar procedures for converting **compound 16** to **Intermediate 2** shown in **Scheme 7B** starting from ethyl 5-bromo-4-methylthiazole-2-carboxylate.

| Intermediate # | Structure | IUPAC Name | Exact Mass $[M+H]^+$ |
|---|---|---|---|
| Intermediate 3 | 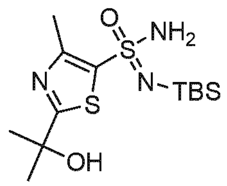 | N'-(tert-butyldimethylsilyl)-2-(2-hydroxypropan-2-yl)-4-methylthiazole-5-sulfonimidamide | 350.2 |

## Scheme 8:

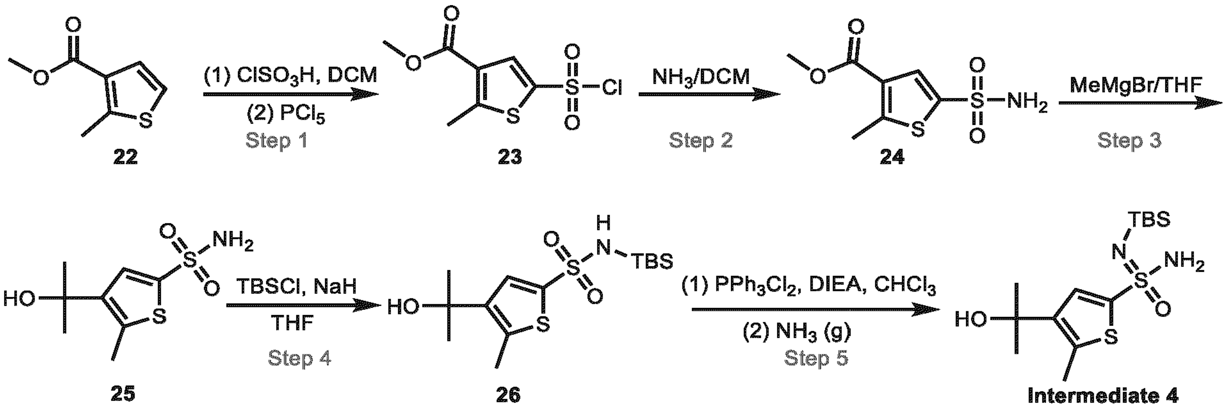


22 23 24 25 26 **Intermediate 4**

**Intermediate 4**

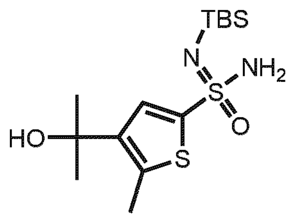

N'-(*tert*-butyldimethylsilyl)-4-(2-hydroxypropan-2-yl)-5-methylthiophene-2-sulfonimidamide

**Steps 1-3** used similar procedures for converting compound **11** to compound **14** shown in **Scheme 6** to afford compound **25** from compound **22.** MS-ESI: 234.0 (M-1).

**Steps 4-5** used similar procedure for converting compound **20** to Intermediate **2** shown in **Scheme 7B** to afford Intermediate **4** from compound **25.** MS-ESI: 349.1 (M+1).

**Table 3.** The Intermediate in the following Table was prepared using similar procedure as shown in **Scheme 8** above for converting compound **22** to Intermediate **4** starting from the appropriate materials.

| Intermediate # | Structure | IUPAC Name | Exact Mass $[M+H]^+$ |
|---|---|---|---|
| Intermediate 5 | 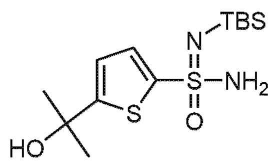 | N'-(*tert*-butyldimethylsilyl)-5-(2-hydroxypropan-2-yl)thiophene-2-sulfonimidamide | 335.1 |

(continued)

| Intermediate # | Structure | IUPAC Name | Exact Mass $[M+H]^+$ |
|---|---|---|---|
| Intermediate 6 | 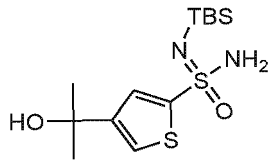 | N'-(tert-butyldimethylsilyl)-4-(2-hydroxypropan-2-yl)thiophene-2-sulfonimidamide | 335.1 |
| Intermediate 7 | 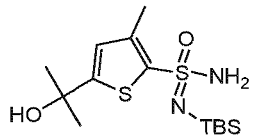 | N'-(tert-butyldimethylsilyl)-5-(2-hydroxypropan-2-yl)-3-methylthiophene-2-sulfonimidamide | 349.1 |
| Intermediate 8 | 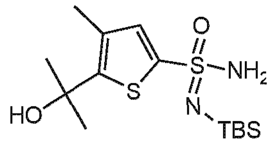 | N'-(tert-butyldimethylsilyl)-5-(2-hydroxypropan-2-yl)-4-methylthiophene-2-sulfonimidamide | 349.1 |
| Intermediate 9 | 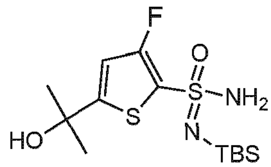 | N'-(tert-butyldimethylsilyl)-3-fluoro-5-(2-hydroxypropan-2-yl)thiophene-2-sulfonimidamide | 353.1 |
| Intermediate 10 | 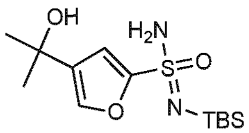 | N'-(*tert*-butyldimethylsilyl)-4-(2-hydroxypropan-2-yl)furan-2-sulfonimidamide | 319.1 |

## Scheme 9:

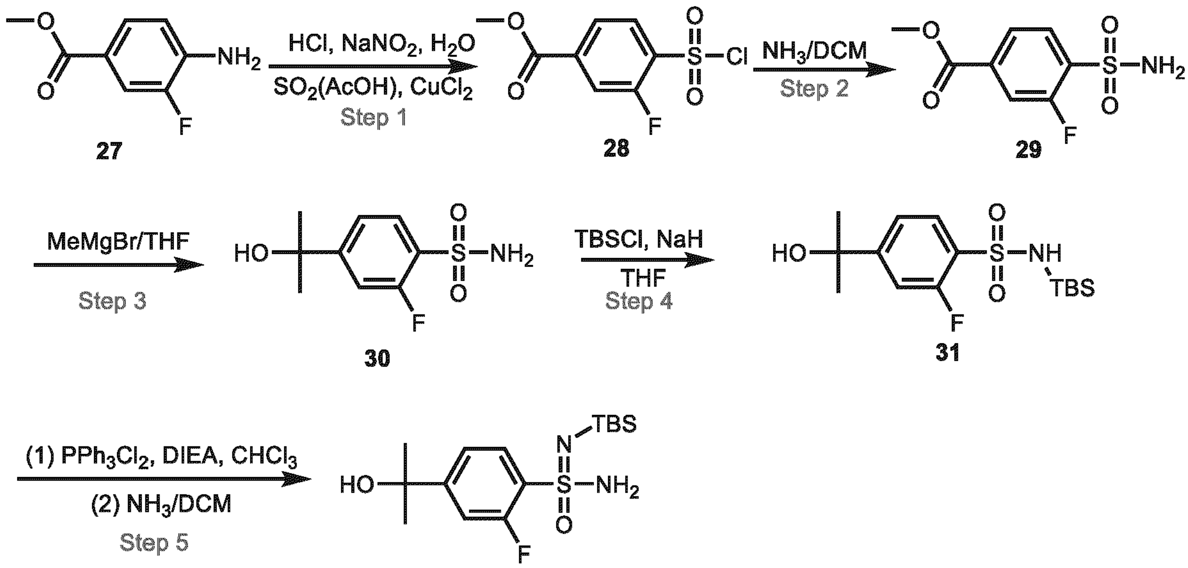


27

28

29

30

31

Intermediate 11

## Intermediate 11

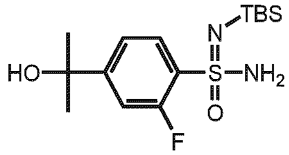

N'-(*tert*-butyldimethylsilyl)-2-fluoro-4-(2-hydroxypropan-2-yl)benzenesulfonimidamide

**Step 1: Methyl 4-(chlorosulfonyl)-3-fluorobenzoate**

Into a 1 L round-bottom flask was placed a solution of methyl 4-amino-3-fluorobenzoate (10 g, 59.1 mmol) in aq. HCl (6 N, 200 mL). This was followed by the addition of a solution of $NaNO_2$ (6.1 g, 88.8 mmol) in water (20 mL) dropwise with stirring at 0°C. The resulting solution was stirred for 30 min at 0°C. The above mixture was added to a saturated solution of $SO_2$ in AcOH (200 mL) dropwise with stirring at 0°C. Then to the above was added $CuCl_2$ (8.0 g, 59.6 mmol). The resulting solution was stirred for 1 h at RT and was then quenched by the addition of 200 mL of water. The resulting solution was extracted with 3x200 mL of DCM. The organic layers were combined, dried over anhydrous $Na_2SO_4$ and concentrated under vacuum. This resulted in 10 g (67%) of the title compound as yellow oil. The product was used in the next step without further purification.

**Step 2: Methyl 3-fluoro-4-sulfamoylbenzoate**

Into a 1000 mL round bottom flask was placed a solution of methyl 4-(chlorosulfonyl)-3-fluorobenzoate solution (10 g, 39.5 mmol) in DCM (50 mL). This was followed by the addition of a saturated solution of ammonia in DCM (500 mL) in portions with stirring at 0°C. The resulting solution was stirred for 1 h at 0°C. The resulting solution was concentrated and the residue was purified with $SiO_2$-gel column and diluted with ethyl acetate/ petroleum ether (1:2 to 1:1). This resulted in 8.28 g (90%) of the title compound as yellow solid. MS-ESI: 232.1 (M-1).

**Step 3: 2-Fluoro-4-(2-hydroxypropan-2-yl)benzenesulfonamide**

Into a 1 L 3-necked round-bottom flask was placed a solution of methyl 3-fluoro-4-sulfamoylbenzoate (8.28 g 35.5 mmol) in THF (500 mL). This was followed by the addition of MeMgBr/THF (3 M, 60 mL) dropwise with stirring at 0°C. The resulting solution was stirred overnight at RT and then was quenched by the addition of 100 mL of sat. $NH_4Cl$. The resulting solution was extracted with 3x200 mL of ethyl acetate and the combined organic layers were concentrated under vacuum. The residue was applied onto a silica gel column and eluted with ethyl acetate/petroleum ether (1:2 to 1:1). This resulted 7.45 g (89.9%) of the title compound as a white solid. MS-ESI: 233.1 (M+1).

**Step 4: N-(tert-butyldimethylsilyl)-2-fluoro-4-(2-hydroxypropan-2-yl)benzenesulfonamide**

Into a 500 mL round bottom flask was placed a solution of 2-fluoro-4-(2-hydroxypropan-2-yl)benzenesulfonamide (7.45 g 31.9 mmol) in THF (200 mL). This was followed by the addition of NaH (60% wt, 1.91 g, 79.6 mmol). The mixture was stirred at 0°C for 0.5 h. This was followed by the addition of the solution of TBSCl (7.19 g, 47.9 mmol) in THF (50 mL) dropwise. The resulting solution was stirred at RT overnight. The reaction was quenched with ice-water (100 mL); the resulting solution was extracted with EtOAc (3x200 mL). The combined organic layers were dried over anhydrous $Na_2SO_4$ and concentrated under vacuum. The residue was purified with $SiO_2$-gel column and eluted with ethyl acetate/petroleum ether (1:5 to 1:2). This resulted 10 g (90%)of the title compound as a white solid. MS-ESI: 348.1 (M+1).

**Step 5: N'-(tert-butyldimethylsilyl)-2-fluoro-4-(2-hydroxypropan-2-yl)benzenesulfonimidamide**

Into a 1 L 3-necked round-bottom flask purged with and maintained under nitrogen was placed a solution of $PPh_3Cl_2$ (19.2 g, 57.6 mmol) in $CHCl_3$ (100 mL). This was followed by the addition of DIEA (7.4 g, 57.6 mmol) dropwise with stirring at 0°C. After stirred at 0°C for 10 min, to the above was added a solution of N'-(tert-butyldimethylsilyl)-2-fluoro-4-(2-hydroxypropan-2-yl)benzenesulfonimidamide (10 g, 28.8 mmol) in $CHCl_3$ (100 mL) dropwise with stirring at 0°C. The resulting solution was allowed to react for 30 min at 0°C. To the mixture was added a saturated solution of ammonia in DCM (500 mL) at 0°C. The resulting solution was stirred for 2 h at RT. The solids were filtered out, and the filtrate was dilute with 100 mL of water. The resulting solution was extracted with 3x200 mL of DCM and the combined

organic layers were dried over anhydrous $Na_2SO_4$ concentrated under vacuum. The residue was applied onto a silica gel column and eluted with a gradient of ethyl acetate/petroleum ether (1:10 to 1:3). This resulted in 5 g (50%) of the title compound as a light yellow solid. MS-ESI: 347.2 (M+1).

**Table 4.** The Intermediates in the following Table were prepared using similar procedure as shown in **Scheme 9** above for converting compound **27** to Intermediate **11** starting from the appropriate materials.

| Intermediate # | Structure | IUPAC Name | Exact Mass $[M+H]^+$ |
|---|---|---|---|
| Intermediate 12 | 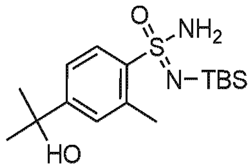 | N'-(*tert*-butyldimethylsilyl)-4-(2-hydroxypropan-2-yl)-2-methylbenzenesulfonimidamide | 343.2 |
| Intermediate 13 | 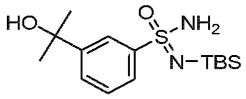 | N'-(*tert*-butyldimethylsilyl)-3-(2-hydroxypropan-2-yl) benzenesulfonimidamide | 329.1 |
| Intermediate 14 | 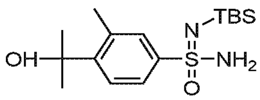 | N'-(*tert*-butyldimethylsilyl)-4-(2-hydroxypropan-2-yl)-3-methylbenzenesulfonimidamide | 343.2 |
| Intermediate 15 | 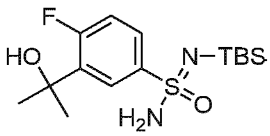 | N'-(*tert*-butyldimethylsilyl)-4-fluoro-3-(2-hydroxypropan-2-yl) benzenesulfonimidamide | 347.2 |
| Intermediate 16 | 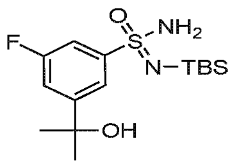 | N'-(*tert*-butyldimethylsilyl)-3-fluoro-5-(2-hydroxypropan-2-yl) benzenesulfonimidamide | 347.2 |
| Intermediate 17 | 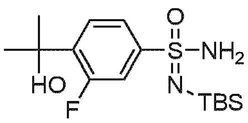 | N'-(*tert*-butyldimethylsilyl)-3-fluoro-4-(2-hydroxypropan-2-yl) benzenesulfonimidamide | 347.2 |
| Intermediate 18 | 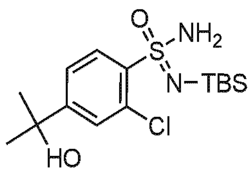 | N'-(*tert*-butyldimethylsilyl)-2-chloro-4-(2-hydroxypropan-2-yl) benzenesulfonimidamide | 363.1 |

**Table 5.** The Intermediate in the following Table was prepared using similar procedure as shown in **Scheme 9** above for converting compound **28** to Intermediate **11** starting from methyl 4-(chlorosulfonyl)benzoate.

| Intermediate # | Structure | IUPAC Name | Exact Mass $[M+H]^+$ |
|---|---|---|---|
| Intermediate 19 | 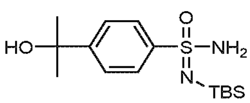 | N'-(*tert*-butyldimethylsilyl)-4-(2-hydroxypropan-2-yl) benzenesulfonimidamide | 329.2 |

**Scheme 10:**

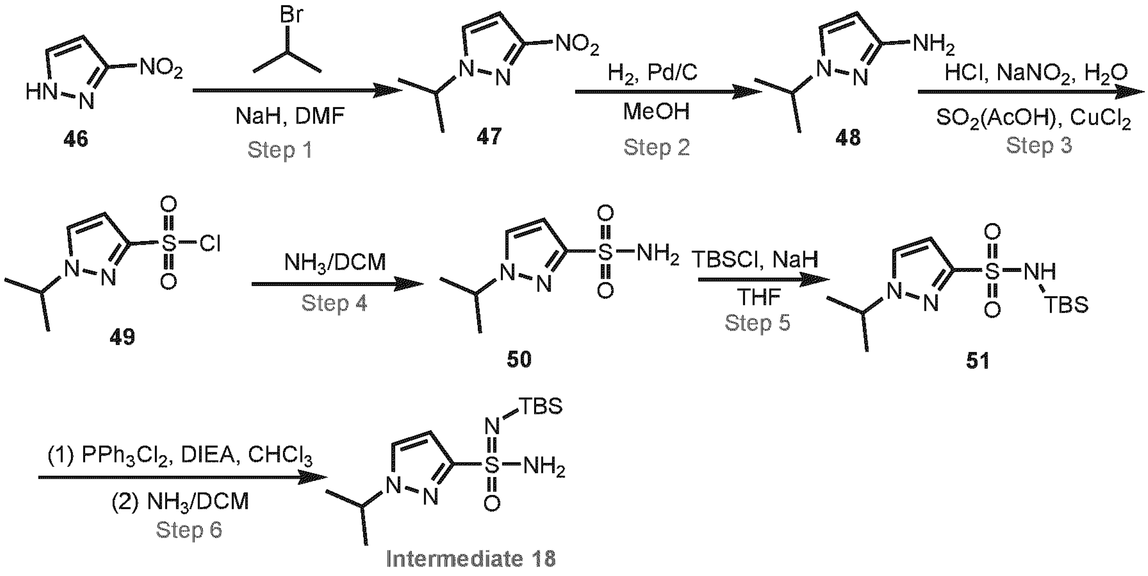


**Intermediate 18**

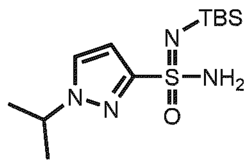

N'-(*tert*-butyldimethylsilyl)-1-isopropyl-1*H*-pyrazole-3-sulfonimidamide

**Step 1: 1-Isopropyl-3-nitro-1*H*-pyrazole**

Into a 250-mL round-bottom flask was placed a solution of 3-nitro-1*H*-pyrazole (10 g, 88.4 mmol) in DMF (100 mL). This was followed by the addition of NaH (60% wt., 3.9 g, 97.5 mmol) in portions at 0°C. The resulting solution was stirred for 0.5 h at 0°C. This was followed by the addition of 2-bromopropane (14.1 g, 114.6 mmol) dropwise with stirring at 0°C in 10 min. The resulting solution was stirred for 16 h at RT and then was quenched by the addition of 100 mL of water. The resulting solution was extracted with 3x100 mL of ethyl acetate. The organic layers were combined and dried over anhydrous $Na_2SO_4$, then concentrated under vacuum. The residue was applied onto a silica gel column and eluted with a gradient of ethyl acetate/petroleum ether (1:5 to 1:3). This resulted in 11.8 g (86%) of the title compound as yellow oil. MS-ESI: 156.1 (M+1).

**Step 2: 3-Amino-1-(propan-2-yl)-1*H*-pyrazole**

Into a 250-mL round-bottom flask was placed a solution of 1-isopropyl-3-nitro-1*H*-pyrazole (10.8 g, 69.6 mmol) in MeOH (100 mL). Then Pd/C (10% wt., 1.5 g) was added. The flask was evacuated and flushed three times with hydrogen. The mixture was stirred for 24 h at RT under an atmosphere of hydrogen. The solids were filtered out. The resulting filtrate was concentrated under vacuum. This resulted in 7.27 g (83%) of the title compound as yellow oil. MS-ESI: 126.1 (M+1).

**Steps 3-4** used similar procedures for converting **compound 27** to **compound 29** shown in **Scheme** 9 to afford compound 50 from compound 48. MS-ESI: 188.0 (M-1).

**Steps 5-6** were using the similar procedures for converting compound **30** to Intermediate **11** shown in **Scheme 9** to afford Intermediate 18 from compound 50. MS-ESI: 303.2 (M+1).

**Table** 6. The Intermediate in the following Table was prepared using similar procedure as shown in **Scheme 10** above for converting **compound 48** to **Intermediate 18** starting from the appropriate materials.

| Intermediate # | Structure | IUPAC Name | Exact Mass $[M+H]^+$ |
|---|---|---|---|
| Intermediate 21 | 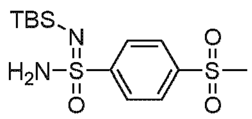 | N'-(*tert*-butyldimethylsilyl)-4-(methylsulfonyl) benzenesulfonimidamide | 349.1 |
| Intermediate 22 | 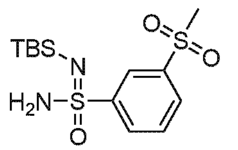 | N'-(*tert*-butyldimethylsilyl)-3-(methylsulfonyl) benzenesulfonimidamide | 349.1 |

**Scheme 11:**

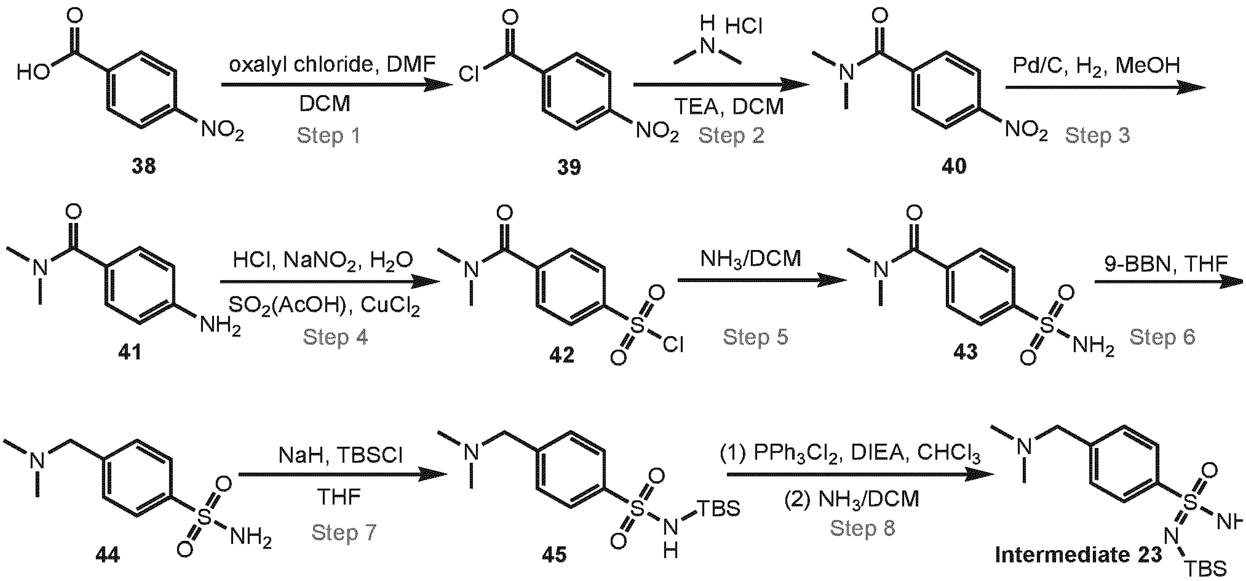


**Intermediate 23**

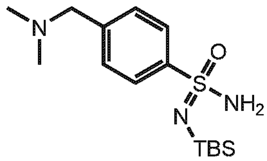

N'-(*tert*-butyldimethylsilyl)-4-((dimethylamino)methyl)benzenesulfonimidamide

**Step 1: 4-Nitrobenzoyl chloride**

Into a 500-mL round-bottom flask was placed 4-nitrobenzoic acid (20 g, 120 mmol), DCM (200 mL), and DMF (0.2 mL). This was followed by the addition of oxalyl chloride (15 mL, 177.1 mmol) dropwise with stirring at 0°C. The resulting solution was stirred for 4 h at RT and then was concentrated under vacuum. This resulted in 22 g (crude) of the title compound as yellow oil. The crude product was used in the next step.

**Step 2: *N*,*N*-dimethyl-4-nitrobenzamide**

Into a 500-mL round-bottom flask was placed dimethylamine hydrochloride (6.5 g, 79.7 mmol), DCM (200 mL), and TEA (50 mL). This was followed by the addition of 4-nitrobenzoyl chloride (22 g, 119 mmol) dropwise with stirring at 0°C. The resulting solution was stirred for 6 h at RT and then was concentrated under vacuum. The resulting mixture

was washed with 2x50 mL of water. The solids were collected by filtration. This resulted in 16 g (69% over two steps) of the title compound as a white solid. MS-ESI: 195.1 (M+1).

**Step 3: 4-Amino-*N,N*-dimethylbenzamide**

Into a 250-mL round-bottom flask was placed *N,N*-dimethyl-4-nitrobenzamide (16 g, 82.4 mmol), MeOH (100 mL). Then Pd/C (10% wt., 1 g) was added. The flask was evacuated and flushed three times with hydrogen. The resulting solution was stirred for 12 h at RT under an atmosphere of hydrogen. The solids were filtered out. The resulting filtrate was concentrated under vacuum. This resulted in 13 g (96%) of the title compound as a white solid. MS-ESI: 165.1 (M+1).

Steps 4-5 used similar procedures for converting compound 27 to compound 29 shown in Scheme 9 to afford compound 43 from compound 41. MS-ESI: 229.1 (M+1).

**Step 6: 4-((Dimethylamino)methyl)benzenesulfonamide**

Into a 100-mL round-bottom flask purged with and maintained under nitrogen was placed a solution of *N,N*-dimethyl-4-sulfamoylbenzamide (1.8 g, 7.9 mmol) in THF (50 mL). This was followed by the addition of 9-BBN (5.8 g) in portions at 0°C. The resulting solution was stirred for 12 h at 70°C and then was quenched by the addition of 20 mL of water/ice. The resulting solution was extracted with 3x100 mL of ethyl acetate and the organic layers were combined. The resulting mixture was washed with 200 mL of water and then the organic layer was concentrated under vacuum. The residue was applied onto a silica gel column and eluted with a gradient of DCM/MeOH (20:1 to 15:1). This resulted in 1 g (59%) of the title compound as a white solid. MS-ESI: 215.1 (M+1).

**Steps 7-8** were using the similar procedures for converting compound **30** to Intermediate **11** shown in **Scheme 9** to afford Intermediate **23** from compound **44.** MS-ESI: 328.2 (M+1).

## Scheme 12:

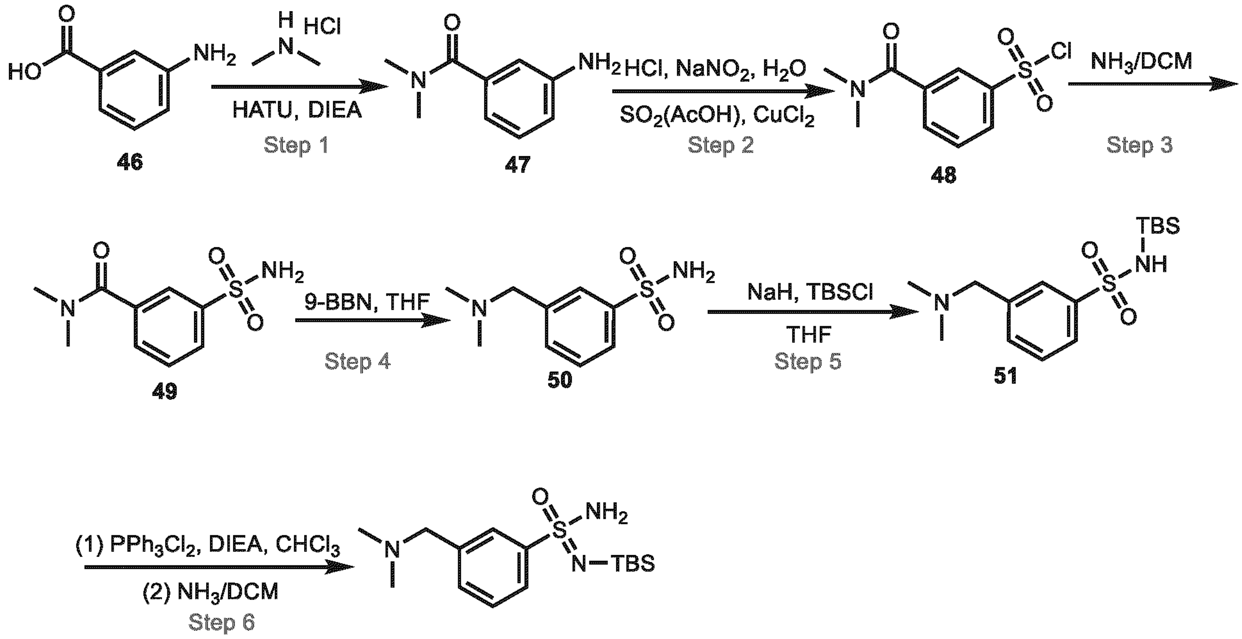


46, 47, 48, 49, 50, 51

**Intermediate 24**

## Intermediate 24

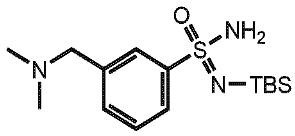

N'-(tert-butyldimethylsilyl)-3-((dimethylamino)methyl)benzenesulfonimidamide

**Step 1: 3-amino-N,N-dimethylbenzamide**

Into a 1000-mL round-bottom flask was placed dimethylamine as a hydrochloride salt (16.3 g, 200 mmol) in DCM (500 mL), DIEA (25.83 mg, 200 mmol). To the above was added 3-aminobenzoic acid (13.7 g, 100 mmol), HATU (57 g, 150 mmol). The resulting solution was stirred for 1 h at RT. The reaction was then quenched by the addition of 500 mL of $NH_4Cl$ (aq.). The resulting solution was extracted with 3x500 ml of ethyl acetate and the organic layers combined and dried over anhydrous sodium sulfate and concentrated. The residue was applied onto a silica gel column and eluted with a gradient of DCM/methanol (50:1 to 20:1). This resulted in 13.14 g (80%) of the title compound as a yellow solid. MS-ESI: 165.1 (M+1).

**Steps 2-6** used the similar procedures for converting compound **41** to Intermediate **23** shown in **Scheme 11** to afford Intermediate **24** from compound **47.** MS-ESI: 328.2 (M+1).

Scheme 13:

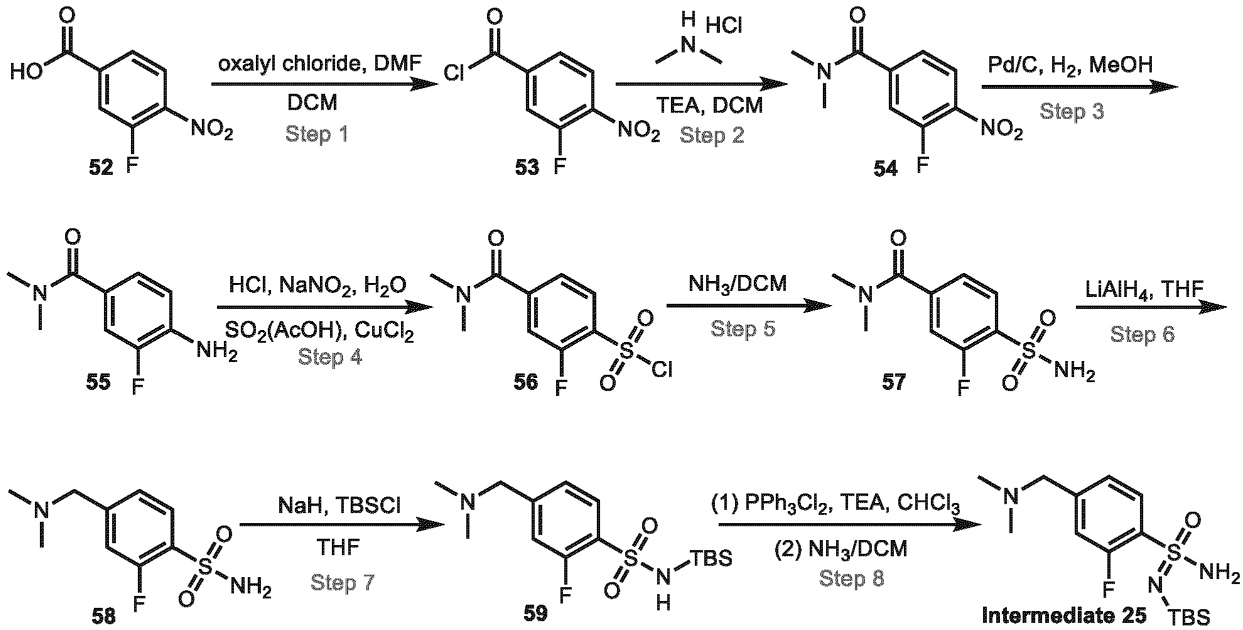


Intermediate 25

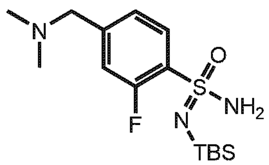

N'-(*tert*-butyldimethylsilyl)-4-((dimethylamino)methyl)-2-fluorobenzenesulfonimidamide

**Steps 1-5** used similar procedures for converting compound **38** to compound **43** shown in **Scheme 11** to afford compound 57. MS-ESI: 247.0 (M+1).

**Step 6: 4-((Dimethylamino)methyl)-2-fluorobenzenesulfonamide**

Into a 1-L round-bottom flask was placed a solution of 3-fluoro-N,N-dimethyl-4-sulfamoylbenzamide (19.3 g, 78.4 mmol) in THF (200 mL). This was followed by the addition of $LiAlH_4$ (8.8 g, 231.9 mmol) in portions at 0°C. The resulting solution was stirred for 12 h at RT and then was quenched by the addition of 10 mL of water. The resulting mixture was concentrated under vacuum. The residue was applied onto a silica gel column and eluted with a gradient of ethyl acetate/petroleum ether (6:1 to 8:1). This resulted in 7.0 g (38%) of the title compound as a white solid. MS-ESI: 233.1 (M+1).

**Steps 7-8** used similar procedures for converting compound **44** to Intermediate **23** shown in **Scheme 11** to afford Intermediate 25. MS-ESI: 346.2 (M+1).

**Scheme 14:**

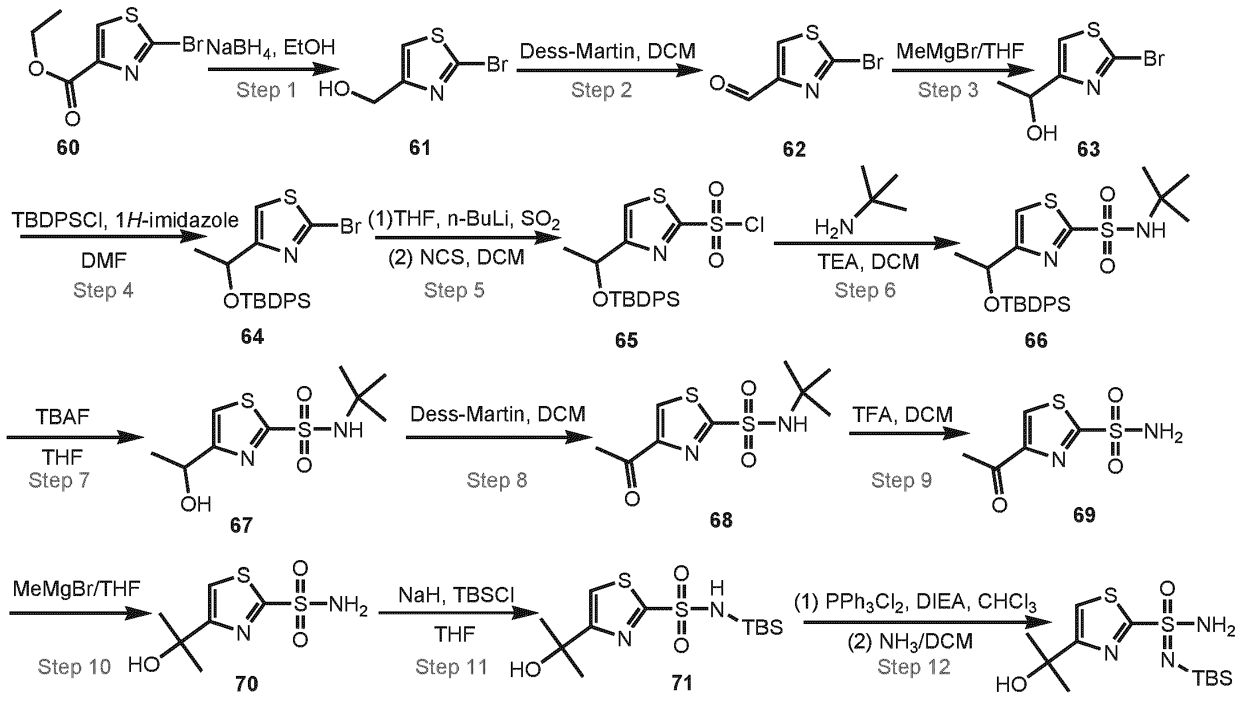


**Intermediate 26**

## Intermediate 26

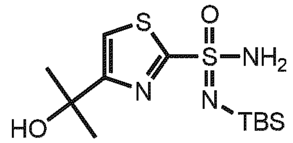

<u>N'-(*tert*-bulyldimethylsilyl)-4-(2-hydroxypropan-2-yl)thiazole-2-sulfonimidamide</u>

**Step 1: (2-Bromothiazol-4-yl)methanol**

Into a 500-mL round-bottom flask was placed a solution of ethyl 2-bromothiazole-4-carboxylate (14 g, 59.3 mmol), EtOH (200 mL). This was followed by the addition of $NaBH_4$ (2.3 g, 60.5 mmol) in portions at 0°C. The resulting solution was stirred for 3 h at RT and then was quenched by the addition of 100 mL of water. The resulting solution was extracted with 2x200 mL of DCM. The organic layers were combined, dried over anhydrous $Na_2SO_4$ and then concentrated under vacuum. This resulted in 10.0 g (87%) of the title compound as colorless oil. MS-ESI: 195.9, 193.9 (M+1).

**Step 2: 2-Bromothiazole-4-carbaldehyde**

Into a 250-mL round-bottom flask was placed a solution of (2-bromothiazol-4-yl)methanol (10.0 g, 51.5 mmol) in DCM (100 mL). To the solution was added Dess-Martin reagent (24.0 g, 56.6 mmol). The resulting solution was stirred for 2 h at RT and then was concentrated under vacuum. The residue was applied onto a silica gel column and eluted with a gradient of ethyl acetate/petroleum ether (1:50 to 1 :20). This resulted in 8.0 g (81%) of the title compound as yellow oil. MS-ESI: 193.9, 191.9 (M+1).

**Step 3: 1-(2-Bromothiazol-4-yl)ethanol**

Into a 250-mL 3-necked round-bottom flask purged with and maintained under nitrogen was placed a solution of 2-bromothiazole-4-carbaldehyde (8 g, 41.7 mmol) in THF (100 mL). This was followed by the addition of MeMgBr (3

M in THF, 15 mL) dropwise with stirring at 0°C. The resulting solution was stirred for 2 h at RT and then was quenched by the addition of 100 mL of $NH_4Cl$ (sat.). The resulting solution was extracted with 3x100 mL of DCM and the combined organic layers were concentrated under vacuum. The residue was applied onto a silica gel column and eluted with a gradient of ethyl acetate/petroleum ether (1:10 to 1:5). This resulted in 6.0 g (69%) of the title compound as brown oil. MS-ESI: 209.9, 207.9 (M+1).

**Step 4: 2-Bromo-4-(1-(*tert*-butyldiphenylsilyloxy)ethyl)thiazole**

Into a 250-mL round-bottom flask was placed a solution of 1-(2-bromothiazol-4-yl)ethanol (6.0 g, 28.8 mmol) and 1*H*-imidazole (4.0 g, 58.8 mmol) in DMF (50 mL). To the solution was added TBDPSCl (8.7 g, 31.6 mmol). The resulting solution was stirred for 12 h at RT and then was diluted with 100 mL of water. The resulting solution was extracted with 3x100 mL of DCM and the combined organic layers were concentrated under vacuum. The residue was applied onto a silica gel column and eluted with a gradient of ethyl acetate/petroleum ether (1:100 to 1:50). This resulted in 10.0 g (78%) of the title compound as light yellow oil. MS-ESI: 448.1, 446.1 (M+1).

**Step 5: 4-(1-(*Tert*-butyldiphenylsilyloxy)ethyl)thiazole-2-sulfonyl chloride**

Into a 250-mL 3-necked round-bottom flask purged with and maintained under nitrogen was placed a solution of 2-bromo-4-(1-(*tert*-butyldiphenylsilyloxy)ethyl)thiazole (10.0 g, 22.4 mmol) in THF (100 mL). This was followed by the addition of n-BuLi (2.5 M in THF, 11 mL) dropwise with stirring at -78°C. The resulting solution was stirred for 30 min at -78°C. To the above $SO_2$ gas was introduced. The reaction was warmed to RT and stirred for 30 min and then was concentrated under vacuum. The residue was dissolved in DCM (100 mL) and then NCS (3.6 g, 26.9 mmol) was added. The resulting solution was stirred for 30 min at RT and then was concentrated under vacuum. This resulted in 8.0 g (crude, 77%) of the title compound as a white solid. The crude product was used in the next step.

**Step 6: N-*tert*-butyl-4-(1-(*tert*-butyldiphenylsilyloxy)ethyl)thiazole-2-sulfonamide**

Into a 100-mL round-bottom flask purged with and maintained under nitrogen was placed a solution of 4-(1-(*tert*-butyldiphenylsilyloxy)ethyl)thiazole-2-sulfonyl chloride (8.0 g, 17.2 mmol) in DCM (50 mL). To the solution were added TEA (3.5 g, 34.6 mmol) and 2-methylpropan-2-amine (1.9 g, 26.0 mmol). The resulting solution was stirred for 2 h at RT and then was concentrated under vacuum. The residue was applied onto a silica gel column and eluted with a gradient of ethyl acetate/petroleum ether (1:15 to 1:5). This resulted in 8.0 g (71%, 2 steps) of the title compound as brown oil. MS-ESI: 503.2 (M+1).

**Step 7: N-*tert*-butyl-4-(1-hydroxyethyl)thiazole-2-sulfonamide**

Into a 250-mL round-bottom flask was placed a solution of N-*tert*-butyl-4-(1-(*tert*-butyldiphenylsilyloxy)ethyl)thiazole-2-sulfonamide (8.0 g, 15.9 mmol) in THF (100 mL). To the solution was added TBAF (9.6 g, 292.5 mmol). The resulting solution was stirred for 2 h at RT and then was diluted with 100 mL of water. The resulting solution was extracted with 3x100 mL of DCM and the combined organic layers were concentrated under vacuum. The residue was applied onto a silica gel column and eluted with a gradient of ethyl acetate/petroleum ether (1:10 to 1:3). This resulted in 4.0 g (95%) of the title compound as light yellow oil. MS-ESI: 265.1 (M+1).

**Step 8: 4-Acetyl-N-*tert*-butylthiazole-2-sulfonamide**

Into a 100-mL round-bottom flask was placed a solution of N-*tert*-butyl-4-(1-hydroxyethyl)thiazole-2-sulfonamide (4.0 g, 15.1 mmol) in DCM (50 mL). To the solution was added Dess-Martin reagent (7.1 g, 16.6 mmol). The resulting solution was stirred for 2 h at RT and then was concentrated under vacuum. The residue was applied onto a silica gel column and eluted with a gradient of ethyl acetate/petroleum ether (1:10 to 1:3). This resulted in 3.5 g (88%) of the title compound as light yellow oil. MS-ESI: 363.0 (M+1).

**Step 9: 4-Acetylthiazole-2-sulfonamide**

Into a 100-mL round-bottom flask was placed a solution of 4-acetyl-N-*tert*-butylthiazole-2-sulfonamide (3.5 g, 13.3 mmol) in DCM (5 mL). To the solution was added TFA (20 mL). The resulting solution was stirred for 14 h at 40°C and then was concentrated under vacuum. The residue was applied onto a silica gel column and eluted with a gradient of ethyl acetate/petroleum ether (1:10 to 1:3). This resulted in 2.5 g (91%) of the title compound as a gray solid. MS-ESI: 207.0 (M+1).

**Steps 10-12** used similar procedures for converting compound **29** to Intermediate **11** shown in **Scheme 9** to afford Intermediate **26** from compound **69.** MS-ESI: 336.1 (M+1).

**Scheme 15A:**

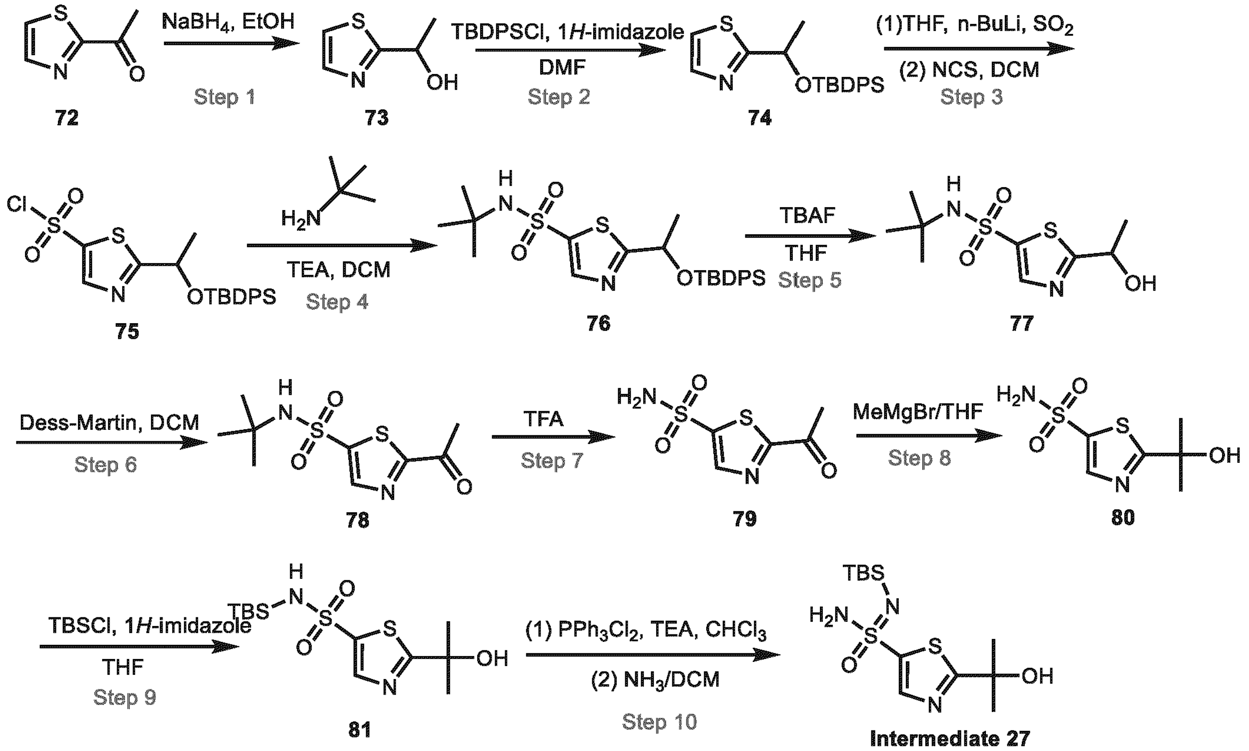


**Intermediate 27**

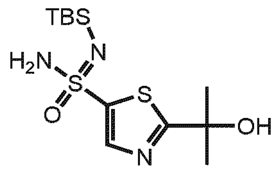

N'-(*tert*-butyldimethylsilyl)-2-(2-hydroxypropan-2-yl)thiazole-5-sulfonimidamide

**Step 1: 1-(Thiazol-2-yl)ethanol**

Into a 500-mL round-bottom flask was placed 1-(thiazol-2-yl)ethanone (20 g, 157 mmol), EtOH (200 mL). This was followed by the addition of $NaBH_4$ (3 g, 81.3 mmol) in portions at 0°C. The resulting solution was stirred for 2 h at RT and then was quenched by the addition of 10 mL of $NH_4Cl$ (sat.). The resulting solution was diluted with 200 mL of water and extracted with 2x200 mL of DCM. The organic layers were combined and dried over anhydrous $Na_2SO_4$, then concentrated under vacuum. This resulted in 20 g (98%) of the title compound as light yellow oil. MS-ESI: 130.0 (M+1).

**Step 2: 2-(1-(*Tert*-butyldiphenylsilyloxy)ethyl)thiazole**

Into a 500-mL round-bottom flask was placed 1-(thiazol-2-yl)ethanol (20 g, 154.8 mmol), DMF (150 mL), 177-imidazole (20.5 g, 301 mmol). This was followed by the addition of TBDPSCl (46 g, 167 mmol) dropwise with stirring at 0°C. The resulting solution was stirred for 2 h at RT and then was diluted with 300 mL of water. The resulting solution was extracted with 3x200 mL of DCM. The organic layers were combined and concentrated under vacuum. The residue was applied onto a silica gel column and eluted with a gradient of ethyl acetate/petroleum ether (1:100 to 1:80). This resulted in 55 g (97%) of the title compound as colorless oil. MS-ESI: 368.1 (M+1).

**Step 3: 2-(1-(*Tert*-butyldiphenylsilyloxy)ethyl)thiazole-5-sulfonyl chloride**

Into a 500-mL 3-necked round-bottom flask purged with and maintained under nitrogen was placed a solution of 2-(1-(*tert*-butyldiphenylsilyloxy)ethyl)thiazole (30 g, 81.6 mmol) in THF (200 mL). This was followed by the addition of n-BuLi (2.5 M in THF, 35.2 mL) dropwise with stirring at -78°C. The resulting solution was stirred for 0.5 h at -78°C and then $SO_2$ was introduced into the above reaction mixture. The reaction was slowly warmed to RT and then NCS (12.8 g, 95.86 mmol) was added. The resulting solution was stirred for 1 h at RT. The solids were filtered out. The resulting filtrate was concentrated under vacuum. This resulted in 30 g (crude, 79%) of the title compound as brown oil. The crude product was used in the next step.

**Step 4: N-*tert*-butyl-2-(1-(tert-butyldiphenylsilyloxy)ethyl)thiazole-5-sulfonamide**

Into a 500-mL round-bottom flask was placed 2-(1-(*tert*-butyldiphenylsilyloxy)ethyl)thiazole-5-sulfonyl chloride (crude, 30 g, 64.37 mmol), DCM (200 mL), TEA (13 g, 128.47 mmol). This was followed by the addition of 2-methylpropan-2-amine (5.6 g, 76.6 mmol) dropwise with stirring at 0°C. The resulting solution was stirred for 2 h at RT and then was concentrated under vacuum. The residue was applied onto a silica gel column and eluted with a gradient of ethyl acetate/petroleum ether (1:30 to 1:20). This resulted in 25 g (61% over two steps) of the title compound as brown oil. MS-ESI: 503.2 (M+1).

**Step 5: N-*tert*-butyl-2-(1-hydroxyethyl)thiazole-5-sulfonamide**

Into a 500-mL round-bottom flask was placed N-*tert*-butyl-2-(1-(*tert*-butyldiphenylsilyloxy)ethyl)thiazole- 5-sulfonamide (25 g, 49.7 mmol), THF (200 mL), TBAF (30 g, 99.67 mmol). The resulting solution was stirred for 2 h at RT and then was diluted with 200 mL of water. The resulting solution was extracted with 3x200 mL of DCM. The organic layers were combined and concentrated under vacuum. The residue was applied onto a silica gel column and eluted with a gradient of ethyl acetate/petroleum ether (1:20 to 1:10). This resulted in 12 g (91%) of the title compound as light yellow oil. MS-ESI: 265.1 (M+1).

**Step 6: 2-Acetyl-N-*tert*-butylthiazole-5-sulfonamide**

Into a 500-mL round-bottom flask was placed N-*tert*-butyl-2-(1-hydroxyethyl)thiazole-5-sulfonamide (12 g, 45.4 mmol), DCM (200 mL). To this solution was added Dess-Martin reagent (20 g, 47.2 mmol) in portions at RT. The resulting solution was stirred for 2 h at RT and then was concentrated under vacuum. The residue was applied onto a silica gel column and eluted with a gradient of ethyl acetate/petroleum ether (1:20 to 1:10). This resulted in 9 g (76%) of the title compound as a light yellow solid. MS-ESI: 263.0 (M+1).

**Step 7: 2-Acetylthiazole-5-sulfonamide**

Into a 100-mL round-bottom flask was placed 2-acetyl-N-*tert*-butylthiazole-5-sulfonamide (7 g, 26.7 mmol), TFA (20 mL). The resulting solution was stirred for 14 h at 70°C and then was concentrated under vacuum. The residue was applied onto a silica gel column and eluted with a gradient of ethyl acetate/petroleum ether (1:5 to 1:3). This resulted in 5 g (91%) of the title compound as a yellow solid. MS-ESI: 207.0 (M+1).

**Step 8: 2-(2-Hydroxypropan-2-yl)thiazole-5-sulfonamide**

Into a 500-mL 3-necked round-bottom flask purged with and maintained under nitrogen was placed 2-acetylthiazole-5-sulfonamide (5 g, 24.3 mmol), THF (100 mL). This was followed by the addition of MeMgBr (3 M in THF, 8.1 mL, 24.3 mmol) dropwise with stirring at 0°C. The resulting solution was stirred for 14 h at RT and then was quenched by the addition of 100 mL of $NH_4Cl$ (sat.). The resulting solution was extracted with 2x150 mL of DCM. The organic layers were combined and concentrated under vacuum. The residue was applied onto a silica gel column and eluted with a gradient of ethyl acetate/petroleum ether (1:5 to 1:3). This resulted in 2.9 g (54%) of the title compound as a light yellow solid. MS-ESI: 223.0 (M+1).

**Steps 9-10** used similar procedures for converting compound **14** to Intermediate **1** shown in **Scheme 6** to afford Intermediate **27** from compound 80. MS-ESI: 336.1 (M+1).

**Scheme 15B:**

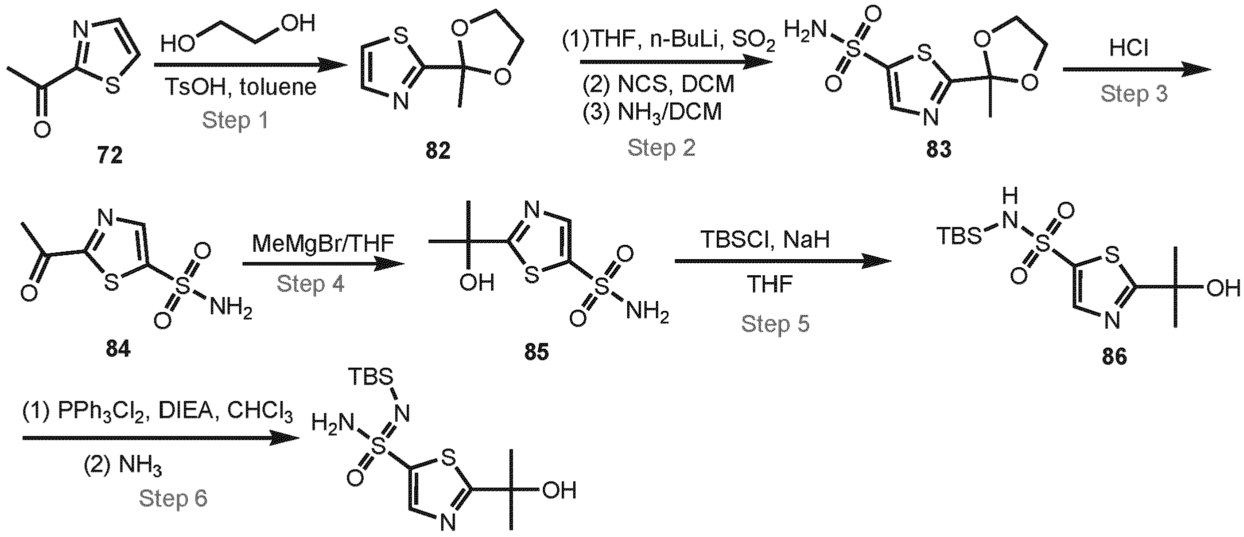


72 82 83 84 85 86

**Intermediate 27**

## Intermediate 27

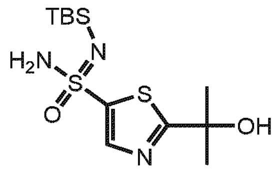

N'-(*tert*-butyldimethylsilyl)-2-(2-hydroxypropan-2-yl)thiazole-5-sulfonimidamide

**Step 1: 2-(2-Methyl-1,3-dioxolan-2-yl)thiazole**

Into a 500-mL round-bottom flask was placed a solution of 1-(thiazol-2-yl)ethanone (20 g, 157.0 mmol) in toluene (300 mL) and ethane-1,2-diol (19.5 g, 314 mmol). To the solution was added TsOH (2.7 g, 15.7 mmol). The resulting solution was refluxed overnight and water was separated from the solution during the reflux. The resulting solution was diluted with 200 mL of water and extracted with 2x100 mL of ethyl acetate. The organic layers were combined, dried over anhydrous $Na_2SO_4$, and then concentrated under vacuum. This resulted in 26.6 g (99%) of the title compound as light yellow oil. MS-ESI: 172.0 (M+1).

**Step 2: 2-(2-Methyl-1,3-dioxolan-2-yl)thiazole-5-sulfonamide**

Into a 500-mL 3-necked round-bottom flask purged with and maintained under nitrogen was placed a solution of 2-(2-methyl-1,3-dioxolan-2-yl)thiazole (14 g, 81.6 mmol) in THF (200 mL). This was followed by the addition of n-BuLi (2.5 M in THF, 35.2 mL, 88.0 mmol) dropwise with stirring at -78°C. The resulting solution was stirred for 0.5 h at -78°C and then $SO_2$ was introduced into the above reaction mixture. The reaction was slowly warmed to RT and then NCS (12.8 g, 95.86 mmol) was added. The resulting solution was stirred for 1 h at RT. The solids were filtered out. The resulting filtrate was concentrated under vacuum and then was diluted in DCM (160 mL). To the above was added a saturated solution of ammonia in DCM (300 mL). The resulting solution was stirred for 3 h at RT and then was concentrated under vacuum. The residue was applied onto a silica gel column and eluted with a gradient of ethyl acetate/petroleum ether (1:20 to 1:5). This resulted in 12.5 g (61%) of the title compound as a yellow solid. MS-ESI: 251.0 (M+1).

**Step 3: 2-Acetylthiazole-5-sulfonamide**

Into a 250-mL round-bottom flask was placed a solution of 2-(2-methyl-1,3-dioxolan-2-yl)thiazole-5-sulfonamide (12.5 g, 50.0 mmol) in THF (125 mL). To the above was added aq. HCl (4 N, 50.0 mL). The resulting solution was stirred for 6 h at 70°C. The resulting solution was diluted with 100 mL of water and extracted with 2x200 mL of ethyl acetate. The organic layers were combined, dried over anhydrous $Na_2SO_4$, then concentrated under vacuum. The residue was applied onto a silica gel column and eluted with a gradient of ethyl acetate/petroleum ether (1:2 to 1:1). This resulted in

9.3 g (90%) of the title compound as a yellow solid. MS-ESI: 207.0 (M+1). **Steps 4-6** used the same procedures for converting compound **19** to Intermediate **2** shown in **Scheme 7B** to afford Intermediate **27** from compound **84.** MS-ESI: 336.1 (M+1).

**Scheme 16:**

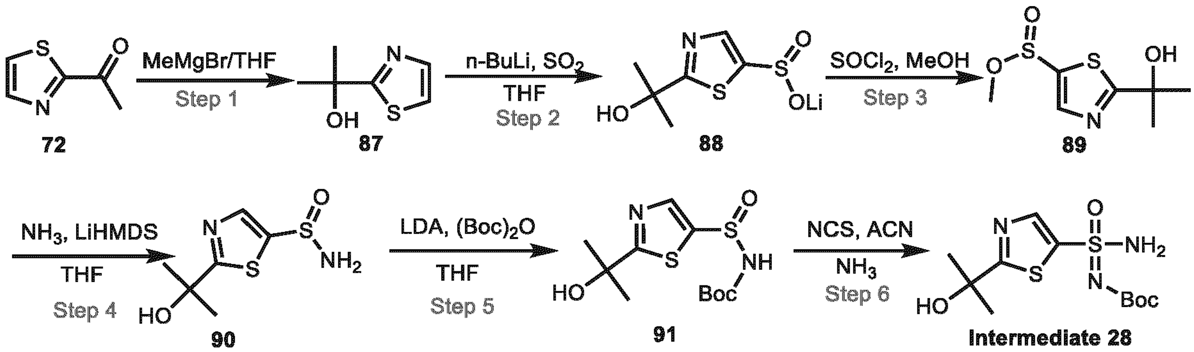


**Intermediate 28**

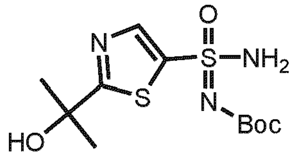

N'-(*tert*-butoxycarbonyl)-2-(2-hydroxypropan-2-yl)thiazole-5-sulfonimidamide

**Step 1: 2-(Thiazol-2-yl)propan-2-ol**

Into a 10-L 4-necked round-bottom flask purged with and maintained under nitrogen was placed a solution of 1-(thiazol-2-yl)ethanone (200 g, 1.6 mol) in THF (4 L). This was followed by the addition of MeMgBr (3 M in THF, 942 mL) dropwise with stirring at 0°C. The mixture was stirred at 0°C for 2 h. After warmed the mixture to RT, the solution was stirred for an additional 16 h. Then the reaction was quenched by the addition of 3 L of $NH_4Cl$ (sat.). The resulting solution was extracted with 3×1 L of ethyl acetate. The organic layers were combined, dried over anhydrous $Na_2SO_4$, then concentrated under vacuum. The residue was applied onto a silica gel column and eluted with a gradient of ethyl acetate/petroleum ether (1:3 to 1:1). This resulted in 210 g (93%) of the title compound as a brown oil. MS-ESI: 144.0 (M+1).

**Step 2: Lithium 2-(2-hydroxypropan-2-yl)thiazole-5-sulfinate**

Into a 10-L 4-necked round-bottom flask purged with and maintained under nitrogen was placed a solution of 2-(thiazol-2-yl)propan-2-ol (50 g, 349.0 mmol) in THF (1.5 L). This was followed by the addition of n-BuLi (2.5 M in hexane, 350 mL) dropwise with stirring at -78°C. The mixture was stirred at -78°C for 1 h. Then $SO_2$ was bubbled into the mixture for 15 min below -30°C. The mixture was stirred for an additional 1 h at RT and then was concentrated under vacuum. This resulted in 87 g (crude) of the title compound as a light yellow solid. The crude product was used directly in the next step.

**Step 3: Methyl 2-(2-hydroxypropan-2-yl)thiazole-5-sulfinate**

Into a 2-L 3-necked round-bottom flask, lithium 2-(2-hydroxypropan-2-yl)thiazole-5-sulfinate (87 g, crude) was dissolved in anhydrous MeOH (500 mL). Then $SOCl_2$ (43 g, 360 mmol) was added to the mixture dropwise with stirring at 0°C. The mixture was stirred overnight at RT and then was concentrated under vacuum. The residue was diluted with 500 mL of ethyl acetate. The resulting solution was washed with 2x200 mL of water and 2x200 mL of brine. The solution was dried over anhydrous $Na_2SO_4$, then concentrated under vacuum. This resulted in 72 g (crude) of the title compound as light yellow oil. The crude product was used directly in the next step.

**Step 4: 2-(2-Hydroxypropan-2-yl)thiazole-5-sulfinamide**

Into a 10-L 4-necked round-bottom flask purged with and maintained under nitrogen was placed a solution of methyl 2-(2-hydroxypropan-2-yl)thiazole-5-sulfinate (72 g, 326 mmol) in THF (500 mL). Then to the above $NH_3$ (0.5 M in THF, 2.0 L) was added. After cooling to -78°C, LiHMDS (1 M in THF, 2.0 L) was added to the mixture dropwise with stirring. Then the mixture was stirred at -78°C for 2 h. The reaction was quenched by the addition of 500 mL of $NH_4Cl$ (sat.). The resulting solution was extracted with 3x300 mL of ethyl acetate. The organic layers were combined, dried over anhydrous $Na_2SO_4$, then concentrated under vacuum. This resulted in 32 g (crude) of the title compound as brown oil. The crude product was used directly in the next step.

**Step 5: *Tert-butyl* 2-(2-hydroxypropan-2-yl)thiazol-5-ylsulfinylcarbamate**

Into a 1-L 3-necked round-bottom flask purged with and maintained under nitrogen was placed a solution of 2-(2-hydroxypropan-2-yl)thiazole-5-sulfinamide (32 g, crude) in THF (300 mL). This was followed by the addition of LDA (2 M in THF, 116 mL) dropwise with string at 0°C. The mixture was stirred at 0°C for 1 h, then $(Boc)_2O$ (33.8 g, 155 mmol) was added in portions at 0°C. The mixture was warmed to RT and stirred for an additional 2 h. The reaction was quenched with 200 mL of ice-water (200 mL), and the pH value of the solution was adjusted to 6 with HCOOH. The resulting solution was extracted with 3x200 mL of ethyl acetate. The organic layers were combined, dried over anhydrous $Na_2SO_4$, then concentrated under vacuum. The residue was applied onto a silica gel column and eluted with a gradient of ethyl acetate/petroleum ether (1:2 to 1:1). This resulted in 19 g (18%, 4 steps) of the title compound as a white solid.

**Step 6: N-(*tert*-butyldimethylsilyl)-2-(2-hydroxypropan-2-yl)thiazole-5-sulfonimidamide**

Into a 1-L 3-necked round-bottom flask purged with and maintained under nitrogen, *tert*-butyl 2-(2-hydroxypropan-2-yl)thiazol-5-ylsulfinylcarbamate (19 g, 62 mmol) was dissolved in fresh distilled ACN (200 mL). Then to the above solution was added NCS (9.8 g, 74 mmol) in portions. The mixture was stirred for 1 h at RT and then $NH_3$ was bubbled in the mixture for 15 min. The mixture was stirred at RT for 2 h and then was concentrated under vacuum. The residue was applied onto a silica gel column and eluted with a gradient of ethyl acetate/petroleum ether (1:2 to 1:1). This resulted in 13 g (65%) of the title compound as a white solid.

**Scheme 17:**

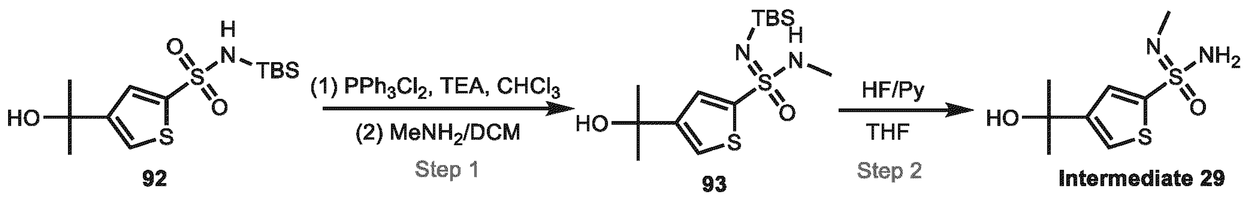


**92** **93** **Intermediate 29**

**Intermediate 29**

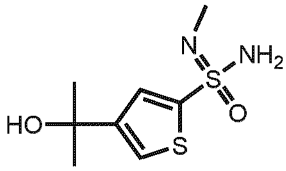

4-(2-Hydroxypropan-2-yl)-N'-methylthiophene-2-sulfonimidamide

**Step 1** used the procedures for converting compound **15** to Intermediate **1** shown in **Scheme 6** to afford compound **93** by substituting ammonia with methylamine. MS-ESI: 349.1 (M+1).

**Step 2: 4-(2-Hydroxypropan-2-yl)-N'-methylthiophene-2-sulfonimidamide**

Into a 25-mL round-bottom flask purged with under nitrogen was placed a solution of *N'-(tert*-butyldimethylsilyl)-4-(2-hydroxypropan-2-yl)-N-methylthiophene-2-sulfonimidamide (500 mg, 1.43 mmol) in DCM (10 mL). To the solution was added HF/Py (70% wt., 200 mg). The resulting solution was stirred for 2 h at RT. The pH value of the solution was adjusted to 8 with aq. $Na_2CO_3$ (5 % wt.). The resulting solution was extracted with 3x10 mL of ethyl acetate. The organic

layers were combined, dried over anhydrous $Na_2SO_4$, then concentrated under vacuum. This resulted in 300 mg (89%) of the title compound as brown oil. MS-ESI: 235.0 (M+1).

**Schemes for the preparation of isocyanate intermediates 30-58:**

Schemes below illustrate the synthesis of isocyanates.

## Scheme 18:

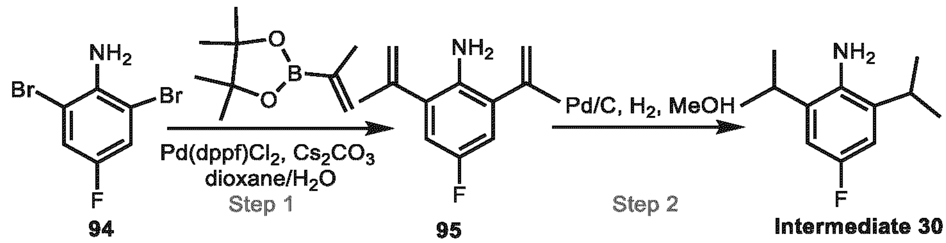


94 95 **Intermediate 30**

## Intermediate 30

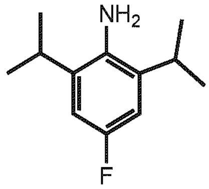

4-Fluoro-2,6-diisopropylbenzenamine

**Step 1: 4-Fluoro-2,6-bis(prop-1-en-2-yl)aniline**

Into a 500-mL round-bottom flask purged with and maintained under nitrogen was placed 2,6-dibromo-4-fluoroaniline (15 g, 55.8 mmol), dioxane (150 mL), water (15 mL), $Cs_2CO_3$ (55 g, 169 mmol), 4,4,5,5-tetramethyl-2-(prop-1-en-2-yl)-1,3,2-dioxaborolane (25 g, 149 mmol), and $Pd(dppf)Cl_2$ (4 g, 5.47 mmol). The resulting solution was stirred for 15 h at 100°C and then was concentrated under vacuum. The residue was applied onto a silica gel column and eluted with a gradient of ethyl acetate/petroleum ether (1: 10 to 1:8). This resulted in 9.2 g (86%) of the title compound as brown oil. MS-ESI: 192.1 (M+1).

**Step 2: 4-Fluoro-2,6-bis(propan-2-yl)aniline**

Into a 500-mL round-bottom flask was placed 4-fluoro-2,6-bis(prop-1-en-2-yl)aniline (9.2 g, 48.1 mmol), and MeOH (200 mL). Then Pd/C (10% wt., 900 mg) was added. The flask was evacuated and flushed three times with hydrogen. The resulting solution was stirred for 12 h at RT under an atmosphere of hydrogen. The solids were filtered out. The resulting filtrate was concentrated under vacuum. The residue was applied onto a silica gel column and eluted with a gradient of ethyl acetate/petroleum ether (1:10 to 1:8). This resulted in 7.2 g (77%) of the title compound as brown oil. MS-ESI: 196.1 (M+1).

## Scheme 19:

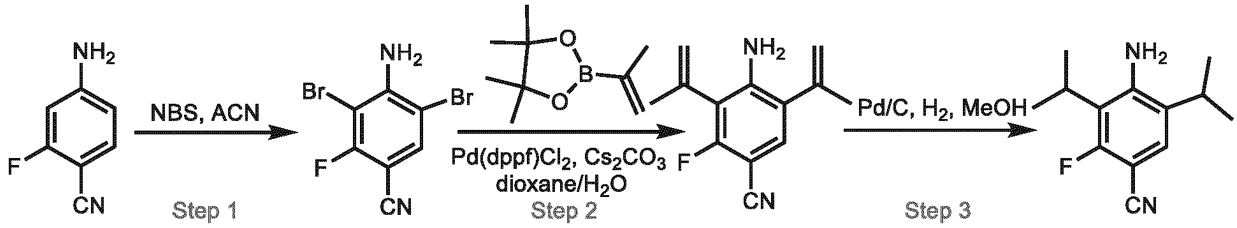


96 97 98 **Intermediate 31**

## Intermediate 31

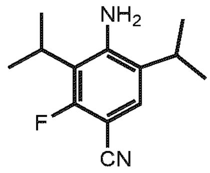

4-Amino-2-fluoro-3,5-diisopropylbenzonitrile

**Step 1: 4-Amino-3,5-dibromo-2-fluorobenzonitrile**

Into a 1-L round-bottom flask was placed 4-amino-2-fluorobenzonitrile (25 g, 184 mmol), ACN (500 mL), and NBS (81.7 g, 459 mmol). The resulting solution was stirred overnight at 75°C and then was concentrated under vacuum. The residue was applied onto a silica gel column and eluted with a gradient of ethyl acetate/petroleum ether (1:100 to 1 :98). This resulted in 50 g (93%) of the title compound as brown oil. MS-ESI: 294.9/292.9/296.9 (M+1).

**Steps 2-3** used similar procedures for converting compound **94** to Intermediate **30** shown in **Scheme 18** to afford Intermediate **31** from compound **97.** MS-ESI: 221.1 (M+1).

## Scheme 20:

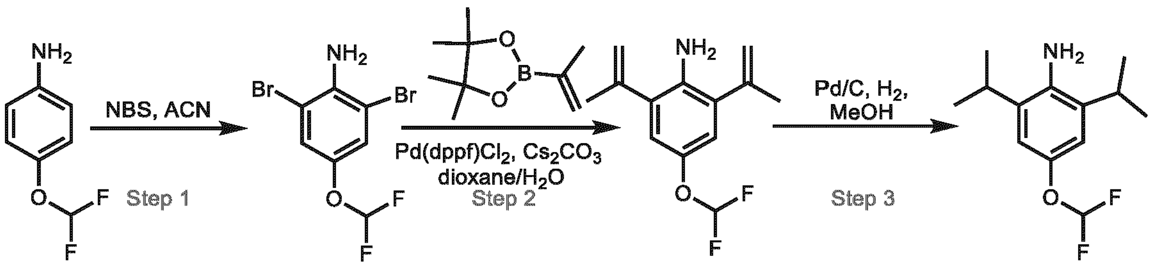


**99** **100"** **101"** **Intermediate 32**

## Intermediate 32

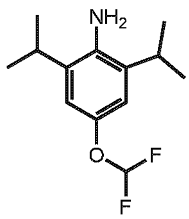

4-(Difluoromethoxv)-2,6-diisopropylbenzenamine

**Step 1: 2,6-Dibromo-4-(difluoromethoxy)benzenamine**

Into a 100-mL round-bottom flask was placed 4-(difluoromethoxy)benzenamine (3 g, 18.9 mmol), ACN (30 mL), and NBS (7.7 g, 43.3 mmol). The resulting solution was stirred overnight at RT and then was concentrated under vacuum. The residue was applied onto a silica gel column and eluted with a gradient of ethyl acetate/petroleum ether (1:30 to 1:20). This resulted in 2.9 g (48%) of the title compound as brown oil. MS-ESI: 317.9/315.9/319.9 (M+1).

**Steps 2-3** used similar procedures for converting compound **94** to Intermediate **30** shown in **Scheme 18** to afford Intermediate **32** from compound **100".** MS-ESI: 244.1 (M+1).

**Scheme 21:**

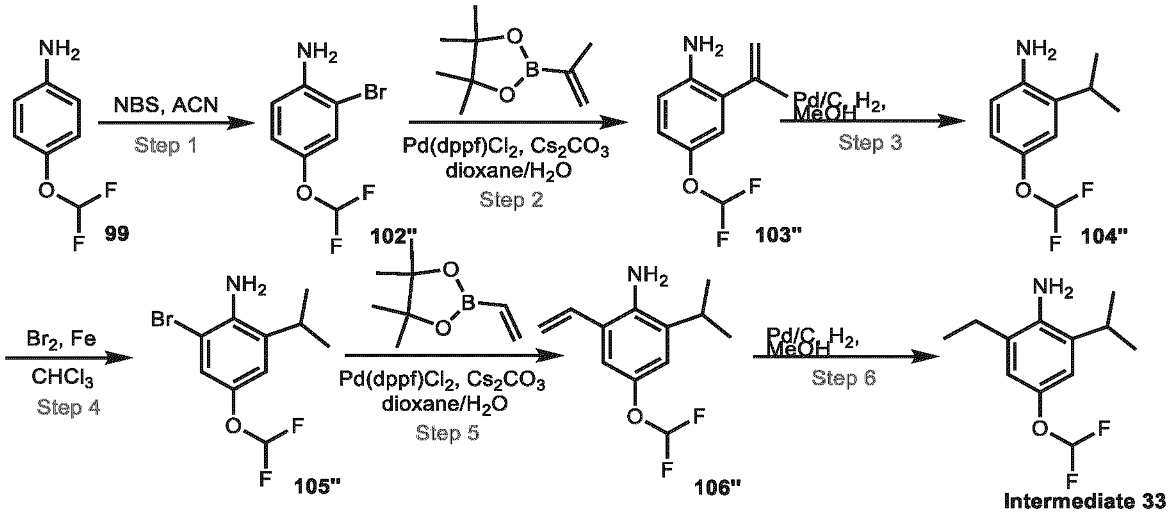

99 102" 103" 104" 105" 106" Intermediate 33

**Intermediate 33**

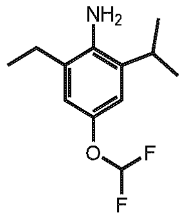

4-(Difluoromethoxy)-2-ethyl-6-isopropylbenzenamine

**Step 1: 2-Bromo-4-(difluoromethoxy)benzenamine**

Into a 250-mL round-bottom flask purged with and maintained under nitrogen was placed 4-(difluoromethoxy)benzenamine (10 g, 62.8 mmol), ACN (100 mL), andNBS (5.59 g, 31.4 mmol). The resulting solution was stirred for 1 h RT and then was concentrated under vacuum. The residue was applied onto a silica gel column and eluted with a gradient of ethyl acetate/petroleum ether (1:20 to 1:10). This resulted in 7.9 g (53%) of the title compound as red oil. MS-ESI: 238.0/240.0 (M+1).

**Step 2: 4-(Difluoromethoxy)-2-(prop-1-en-2-yl)benzenamine**

Into a 250-mL round-bottom flask purged with and maintained under nitrogen was placed 2-bromo-4-(difluoromethoxy)benzenamine (7.9 g, 33.2 mmol), dioxane (100 mL), water (10 mL), $Cs_2CO_3$ (32.46 g, 99.63 mmol), 4,4,5,5-tetramethyl-2-(prop-1-en-2-yl)-1,3,2-dioxaborolane (8.36 g, 49.8 mmol), and Pd(dppf)$Cl_2$ (1.21 g, 1.65 mmol). The resulting solution was stirred overnight at 90°C. The solids were filtered out. The filtrate was concentrated under vacuum. The residue was applied onto a silica gel column and eluted with a gradient of ethyl acetate/petroleum ether (1:30 to 1:20). This resulted in 5.3 g (80%) of the title compound as a yellow solid. MS-ESI: 200.1 (M+1).

**Step 3: 4-(Difluoromethoxy)-2-isopropylbenzenamine**

Into a 250-mL round-bottom flask was placed 4-(difluoromethoxy)-2-(prop-1-en-2-yl)benzenamine (5.3 g, 26.6 mmol) in MeOH (100 mL). Then Pd/C (10% wt., 500 mg) was added. The flask was evacuated and filled three times with hydrogen. The resulting solution was stirred for 3 h at RT under hydrogen. The solids were filtered out. The resulting filtrate was concentrated under vacuum. This resulted in 5.15 g (96%) of the title compound as red oil. MS-ESI: 202.1 (M+1).

**Step 4: 2-Bromo-4-(difluoromethoxy)-6-isopropylbenzenamine**

Into a 500-mL round-bottom flask was placed 4-(difluoromethoxy)-2-isopropylbenzenamine (5.15 g, 25.6 mmol),

$CHCl_3$ (200 mL), Fe turnings (500 mg), and $Br_2$ (4.45 g, 27.9 mmol). The resulting mixture was stirred overnight at 70°C and then was quenched by the addition of 200 mL of water. The resulting solution was extracted with 3x100 mL of DCM and the organic layers combined and concentrated under vacuum. The residue was applied onto a silica gel column and eluted with a gradient of ethyl acetate/petroleum ether (1:30 to 1:20). This resulted in 6.98 g (97%) of the title compound as dark red oil. MS-ESI: 280.0/282.0 (M+1).

**Step 5: 4-(Difluoromethoxy)-2-isopropyl-6-vinylbenzenamine**

Into a 250-mL round-bottom flask purged with and maintained under nitrogen was placed 2-bromo-4-(difluoromethoxy)-6-isopropylbenzenamine (3 g, 10.7 mmol), dioxane (100 mL), water (10 mL), $Cs_2CO_3$ (10.47 g, 32.13 mmol), 4,4,5,5-tetramethyl-2-vinyl-1,3,2-dioxaborolane (2.47 g, 16.0 mmol), and Pd(dppf)$Cl_2$ (784 mg, 1.07 mmol). The resulting solution was stirred overnight at 90°C and then was concentrated under vacuum. The residue was applied onto a silica gel column and eluted with a gradient of ethyl acetate/petroleum ether (1:30 to 1:20). This resulted in 2.3 g (94%) of the title compound as dark green oil. MS-ESI: 228.1 (M+1).

**Step 6: 4-(Difluoromethoxy)-2-ethyl-6-isopropylbenzenamine**

Into a 250-mL round-bottom flask was placed 4-(difluoromethoxy)-2-isopropyl-6-vinylbenzenamine (2.3 g, 10.1 mmol), MeOH (100 mL). Then Pd/C (10% wt., 200 mg) was added. The flask was evacuated and filled three times with hydrogen. The resulting solution was stirred overnight at RT under hydrogen. The solids were filtered out. The filtrate was concentrated under vacuum. This resulted in 2.2 g (95%) of the title compound as red oil. MS-ESI: 230.1 (M+1).

**Table 7.** The Intermediate **34** in the following Table was prepared from compound **105"** using similar procedure as shown in **Scheme 21** above for converting compound **105"** to **106".**

| Intermediate # | Structure | IUPAC Name | Exact Mass $[M+H]^+$ |
|---|---|---|---|
| Intermediate 34 | 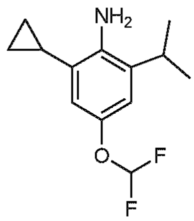 | 2-Cyclopropyl-4-(difluoromethoxy)-6-isopropylbenzenamine | 242.1 |

**Scheme 22:**

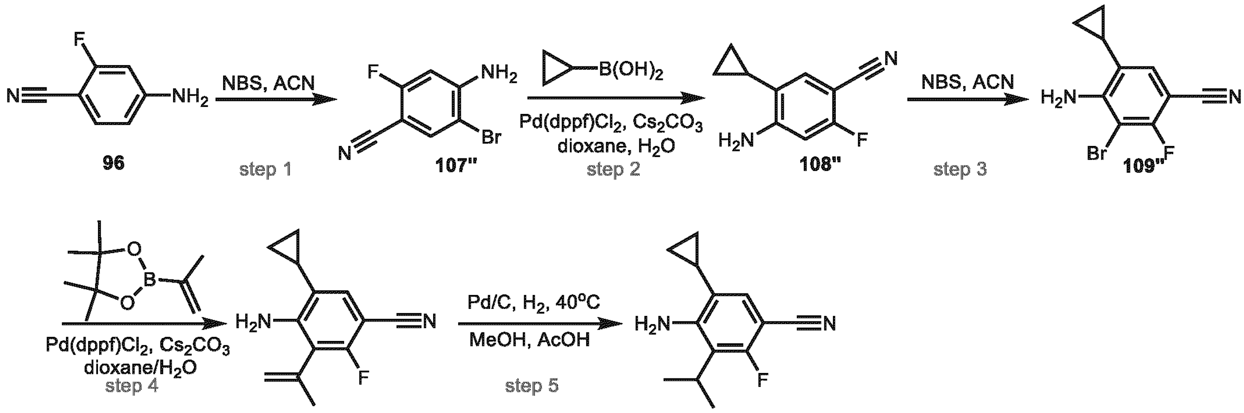


96 107" 108" 109" 110"

Intermediate 35

**Intermediate 35**

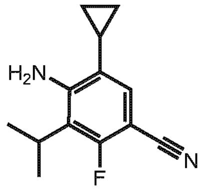

4-Amino-5-cyclopropyl-2-fluoro-3-isopropylbenzonitrile

**Step 1: 4-Amino-5-bromo-2-fluorobenzonitrile**

Into a 250-mL round-bottom flask was placed a solution of 4-amino-2-fluorobenzonitrile (9 g, 66.1 mmol) in ACN (120 mL). Then NBS (12.4 g, 69.7 mmol) was added. The resulting solution was stirred overnight at 80°C and then was concentrated under vacuum. The residue was applied onto a silica gel column and eluted with a gradient of ethyl acetate/petroleum ether (1:20 to 1:10). This resulted in 10.9 g (77%) of the title compound as a yellow solid. MS-ESI: 215.0/217.0 (M+1). $^1$H NMR (300 MHz, DMSO-$d_6$) $\delta$ 7.89 (d, $J$ = 6.0 Hz, 1H), 6.69 (br s, 2H), 6.63 (d, $J$ = 12.0 Hz, 1H).

**Step 2: 4-Amino-5-cyclopropyl-2-fluorobenzonitrile**

Into a 250-mL round-bottom flask purged with and maintained under nitrogen was placed a solution of 4-amino-5-bromo-2-fluorobenzonitrile (6.37 g, 29.6 mmol) in dioxane (70 mL) and water (10 mL). To the solution were added $Cs_2CO_3$ (9.7 g, 29.8 mmol), cyclopropylboronic acid (3.8 g, 44.2 mmol) and Pd(dppf)$Cl_2$ (1.08 g, 1.48 mmol). The resulting solution was stirred overnight at 90°C and then was concentrated under vacuum. The residue was applied onto a silica gel column and eluted with a gradient of ethyl acetate/petroleum ether (1:10 to 1:5). This resulted in 5.03 g (96%) of the title compound as a yellow solid. MS-ESI: 177.1 (M+1).

**Step 3: 4-Amino-3-bromo-5-cyclopropyl-2-fluorobenzonitrile**

Into a 250-mL round-bottom flask was placed a solution of 4-amino-5-cyclopropyl-2-fluorobenzonitrile (5.03 g, 28.7 mmol) in ACN (50 mL). To the solution was added NBS (5.6 g, 31.5 mmol). The resulting solution was stirred overnight at 80°C and then was concentrated under vacuum. The residue was applied onto a silica gel column and eluted with a gradient of ethyl acetate/petroleum ether (1:10 to 1:5). This resulted in 6.972 g (96%) of the title compound as a yellow solid. MS-ESI: 255.0/257.0 (M+1).

**Step 4: 4-Amino-5-cyclopropyl-2-fluoro-3-(prop-1-en-2-yl)benzonitrile**

Into a 250-mL round-bottom flask purged with and maintained under nitrogen was placed a solution of 4-amino-3-bromo-5-cyclopropyl-2-fluorobenzonitrile (6.972 g, 27.33 mmol) in dioxane (120 mL) and water (20 mL). To the solution were added 4,4,5,5-tetramethyl-2-(prop-1-en-2-yl)-1,3,2-dioxaborolane (6.9 g, 41.00 mmol), $Cs_2CO_3$ (13.4 g, 41.00 mmol) and Pd(dppf)$Cl_2$ (0.4 g, 0.55 mmol). The resulting solution was stirred overnight at 80°C and then was concentrated under vacuum. The residue was applied onto a silica gel column and eluted with a gradient of ethyl acetate/petroleum ether (1:10 to 1:5). This resulted in 4.73 g (80%) of the title compound as a yellow solid. MS-ESI: 217.1 (M+1).

**Step 5: 4-Amino-5-cyclopropyl-2-fluoro-3-isopropylbenzonitrile**

Into a 250-mL round-bottom flask was placed a solution of 4-amino-5-cyclopropyl-2-fluoro-3-(prop-1-en-2-yl)benzonitrile (4.73 g, 21.97 mmol), MeOH (100 mL). To the solution was added AcOH (0.5 mL). Then Pd/C (10% wt., 500 mg) was added. The flask was evacuated and filled three times with hydrogen. The resulting solution was stirred for 4 h at 40°C under an atmosphere of hydrogen. The solids were filtered out. The filtrate was concentrated under vacuum. This resulted in 4.71 g (99%) of the title compound as a light yellow solid. MS-ESI: 219.1

**Scheme 23:**

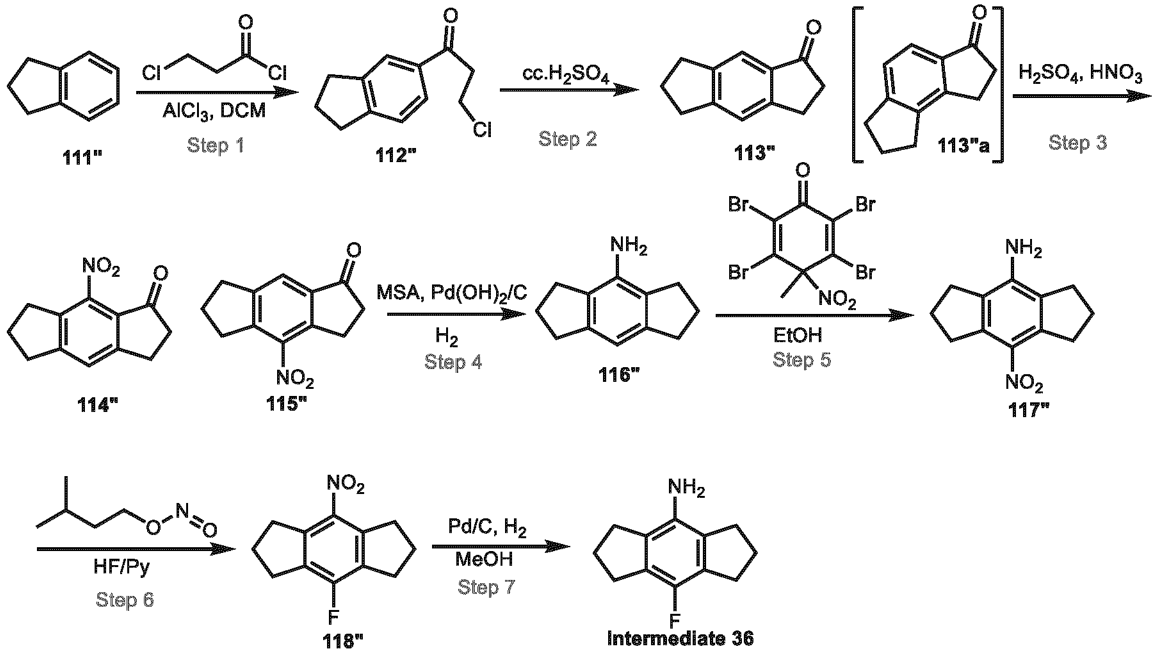


**Intermediate 36**

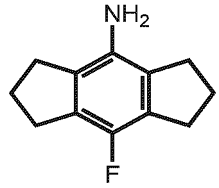

8-Fluoro-1,2,3,5,6,7-hexahydro-*s*-indacen-4-amine

**Step 1: 3-Chloro-1-(2,3-dihydro-1H-inden-5-yl)propan-1-one**

Into a 3-L round-bottom flask was placed a solution of $AlCl_3$ (111 g, 834 mmol) in DCM (1200 mL). This was followed by the addition of a solution of 2,3-dihydro-1H-indene (90 g, 762 mmol) and 3-chloropropanoyl chloride (96.3 g, 759 mmol) in DCM (300 mL) dropwise with stirring at - 10°C in 30 min. The resulting solution was stirred for 16 h at RT. Then the reaction mixture was added dropwise to cold HCl (3 N, 1200 mL) over 45 min at -10°C. The resulting solution was extracted with 3x600 mL of DCM and the organic layers were combined, dried over anhydrous $Na_2SO_4$, then concentrated under vacuum. This resulted in 160.5 g (crude) of the title compound as a yellow solid. The crude product was used in the next step.

**Step 2: 1,2,3,5,6,7-Hexahydro-*s*-indacen-1-one**

Into a 1-L round-bottom flask was placed a solution of 3-chloro-1-(2,3-dihydro-1H-inden-5-yl)propan-1-one (160.5 g, 759 mmol) in conc. $H_2SO_4$ (900 mL). The resulting solution was stirred for 16 h at 55°C and then was quenched by adding the reaction mixture carefully to 4500 mL of water/ice. The solids were collected by filtration and dried over infrared lamp for 24 h. The crude mixture was purified by chromatography and eluted with ethyl acetate/petroleum ether (1:100). This resulted in 10 g (7.6%) of 1,6,7,8-tetrahydro-as-indacen-3(2H)-one (compound **113"a**) and 112.2 g (85%) of the title compound (compound **113"**) as a yellow solid. $^1$H NMR (400 MHz, DMSO-$d_6$) δ 7.44 (s, 1H), 7.39 (s, 1H), 3.13 - 2.79 (m, 8H), 2.70 - 2.55 (m, 2H), 2.20 - 1.90 (m, 2H). $^1$H NMR (400 MHz, DMSO-$d_6$) δ 7.49 (d, *J* = 7.7 Hz, 1H), 7.31 (d, *J* = 7.7 Hz, 1H), 3.19 - 2.98 (m, 4H), 2.93 - 2.80 (m, 3H), 2.68 - 2.54 (m, 2H), 2.15 - 1.95 (m, 2H).

**Step 3: 4-nitro-2,3,6,7-tetrahydro-*s*-indacen-1(5H)-one (114) (Major) and 8-nitro-2,3,6,7-tetrahydro-*s*-indacen-1(5H)-one (115) (Minor)**

Into a 1-L round-bottom flask was placed a solution of 1,2,3,5,6,7-hexahydro-s-indacen-1-one (80 g, 464.5 mmol) in $H_2SO_4$ (500 mL). Then $HNO_3$ (58.5 g, 929 mmol) was added dropwise over 1h at 0°C. The resulting solution was stirred for 1 hr at 0°C. The reaction mixture was slowly added to a mixture of water/ice (1000 mL) and DCM (500 mL) with ice bath cooling. The organic layer was collected, dried over $Na_2SO_4$ and concentrated under vacuum. This resulted in 90 g (90%) of the mixture of 4-nitro-2,3,6,7-hexahydro-s-indacen-1-one and 8-nitro-2,3,6,7-tetrahydro-s-indacen-1(5H)-one as a yellow solid.

**Step 4: 1,2,3,5,6,7-hexahydro-s-indacen-4-amine**

Into a 1-L round-bottom flask was placed a solution of the mixture of 4-nitro-1,2,3,5,6,7-hexahydro-s-indacen-1-one and 8-nitro-2,3,6,7-tetrahydro-s-indacen-1(5H)-one (21.7 g, 100 mmol) in MeOH (300 mL). To the solution was added MSA (11.5 g, 120 mmol). Then $Pd(OH)_2/C$ (20% wt, 5.5 g) was added. The flask was evacuated and filled three times with hydrogen. The resulting mixture was stirred for 16 h at RT under hydrogen (50 psi). The solids were filtered out and washed with methanol. The methanol filtrate and wash was diluted with water (500 mL) and the pH was adjusted to 10.6 with 2N NaOH. The resulting slurry was filtered and the crude solids were recrystallized from methanol/water (9:1) with heating. This resulted in 13.7 g (79%) of the title compound as an off-white solid.

**Step 5: 8-Nitro-1,2,3,5,6,7-hexahydro-s-indacen-4-amine**

Into a 500-mL round-bottom flask was placed 1,2,3,5,6,7-hexahydro-s-indacen-4-amine (8 g, 46.2 mmol), EtOH (200 mL), and 2,3,5,6-tetrabromo-4-methyl-4-nitrocyclohexa-2,5-dienone (21.6 g, 46.1 mmol). The resulting solution was stirred for 12 h at RT and then was concentrated under vacuum. The residue was applied onto a silica gel column and eluted with a gradient of ethyl acetate/petroleum ether (1:50 to 1 :30). This resulted in 5 g (50%) of the title compound as a yellow solid. MS-ESI: 219.1

**Step 6: 4-Fluoro-8-nitro-1,2,3,5,6,7-hexahydro-*s*-indacene**

Into a 100-mL round-bottom flask was placed 8-nitro-1,2,3,5,6,7-hexahydro-s-indacen-4-amine (5 g, 22.9 mmol) and HF/Py (70% wt., 20 mL). This was followed by the addition of 3-methylbutyl nitrite (3 g, 25.6 mmol) dropwise with stirring at 0°C. The resulting solution was stirred for 2 h at RT and then was diluted with 50 mL of water. The resulting solution was extracted with 3x50 mL of DCM. The organic layers were combined and dried over anhydrous $Na_2SO_4$, then concentrated under vacuum. This resulted in 4 g (crude, 79%) of the title compound as brown oil.

**Step 7: 8-Fluoro-1,2,3,5,6,7-hexahydro-s-indacen-4-amine**

Into a 100-mL round-bottom flask was placed 4-fluoro-8-nitro-1,2,3,5,6,7-hexahydro-s-indacene (4 g, 18.1 mmol) in MeOH (50 mL). Then Pd/C (10% wt., 0.5 g) was added. The flask was evacuated and filled three times with hydrogen. The resulting mixture was stirred for 12 h at RT under an atmosphere of hydrogen. The solids were filtered out and the filtrate was concentrated under vacuum. The residue was applied onto a silica gel column and eluted with a gradient of ethyl acetate/petroleum ether (1:10 to 1:5). This resulted in 2 g (46%, 2 steps) of the title compound as a white solid. MS-ESI: 192.1

**Scheme 24:**

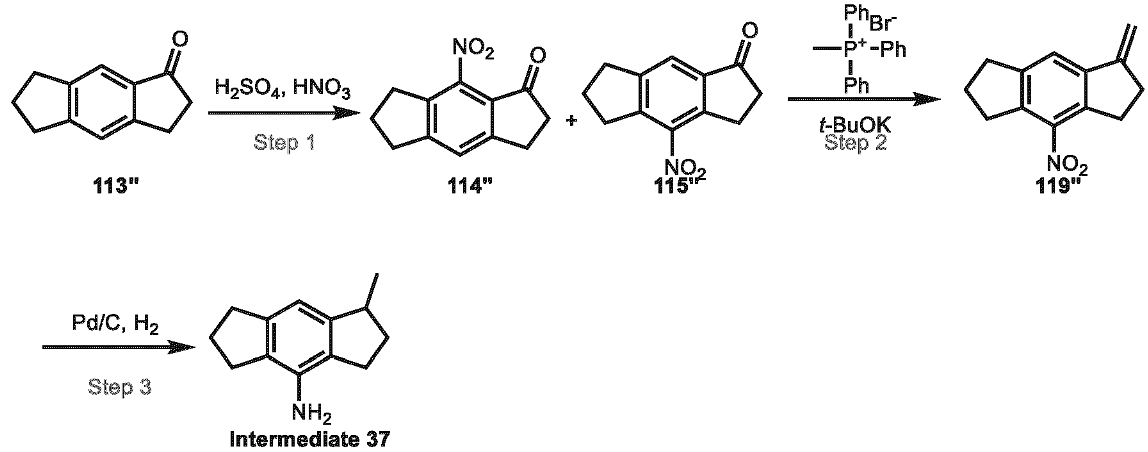


## Intermediate 37

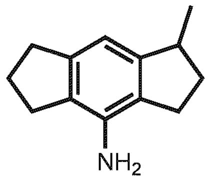

1-methyl-1,2,3, 5,6,7-hexahydro-s-indacen-4-amine

**Step 1: 4-nitro-2,3,6,7-tetrahydro-*s*-indacen-1(5H)-one**

Into a 1-L round-bottom flask was placed a solution of 1,2,3,5,6,7-hexahydro-s-indacen-1-one (40 g, 232 mmol) in $H_2SO_4$ (250 mL). Then $HNO_3$ (29 g, 464 mmol) was added dropwise over 1 h at 0°C. The resulting solution was stirred for 1 hr at 0°C. The reaction mixture was slowly added to a mixture of water/ice (500 mL) and DCM (250 mL) with ice bath cooling. The organic layer was collected, dried over anhydrous $Na_2SO_4$ and concentrated under vacuum. The crude product was purified by silica gel column with a gradient of ethyl acetate and petroleum ether (1:50 to 1:1). This resulted in minor product 5 g (10%) of the title compound and major product 30 g (60%) of 8-nitro-2,3,6,7-tetrahydro-*s*-indacen-1(5H)-one both as a yellow solid.

**Step 2: 1-methylene-4-nitro-1,2,3,5,6,7-hexahydro-*s*-indacene**

Into a 250-mL round-bottom flask was placed a solution of methyltriphenylphosphanium bromide (16.4 g, 46.04 mmol) and t-BuOK (5.2 g, 46.0 mmol) in THF (150 mL) at 0°C. The resulting solution was stirred for 30 min at 0°C. Then the solution of 4-nitro-1,2,3,5,6,7-hexahydro-s-indacen-1-one (5 g, 23.0 mmol) in THF (10 mL) was added dropwise to the reaction mixture at 0°C. The resulting solution was stirred overnight at RT. The resulting mixture was concentrated. The residue was applied onto a silica gel column with ethyl acetate/petroleum ether (1:10). This resulted in 2.6 g (52%) of the title compound as a green solid.

**Step 3: 1-methyl-1,2,3,5,6,7-hexahydro-*s*-indacen-4-amine**

Into a 100-mL round-bottom flask was placed a solution of 1-methylidene-4-nitro-1,2,3,5,6,7-hexahydro-s-indacene (2.6 g, 12.1 mmol) in MeOH (20 mL), Pd/C (10% wt, 300 mg) was added. The flask was evacuated and filled three times with hydrogen. then $H_2$ (g) was introduced in with a balloon. The resulting solution was stirred for 2 h at RT. The Pd/C catalyst was filtered out. The filtrate was concentrated. This resulted in 2 g of the title compound as red oil.

**Table 8.** Intermediate **38** in the following Table was prepared from Compound **114"** using similar procedure as shown in **Scheme 24** above for converting compound **115"** to intermediate **37.**

| Intermediate # | Structure | IUPAC Name | Exact Mass $[M+H]^+$ |
|---|---|---|---|
| Intermediate 38 | 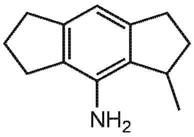 | 3-methyl-1,2,3,5,6,7-hexahydro-s-indacen-4-amine | 188.1 |

## Scheme 25:

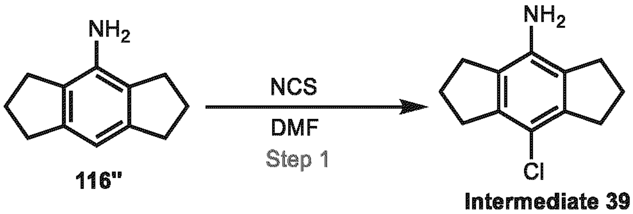


## Intermediate 39

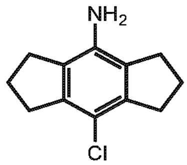

8-Chloro-1,2,3,5,6,7-hexahydro-*s*-indacen-4-amine

**Step 1: 8-Chloro-1,2,3,5,6,7-hexahydro-s-indacen-4-amine**

Into a 50-mL round-bottom flask was placed a solution of 1,2,3,5,6,7-hexahydro-s-indacen-4-amine (1.73 g, 9.99 mmol) in DMF (10 mL). To the solution was added NCS (1.47 g, 11.0 mmol). The resulting solution was stirred overnight at RT and then was diluted with 30 mL of DCM. The resulting mixture was washed with 3x10 mL of water and the organic layer was concentrated under vacuum. The residue was applied onto a silica gel column and eluted with a gradient of ethyl acetate/petroleum ether (1:20 to 1:10). This resulted in 1.88 g (91%) of the title compound as a yellow solid. MS-ESI: 208.1/210.1

## Scheme 26:

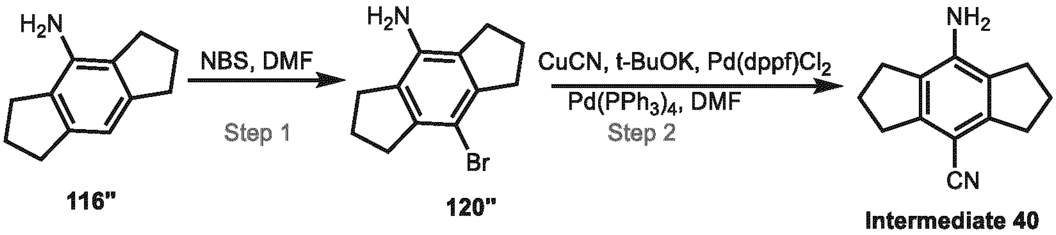


## Intermediate 40

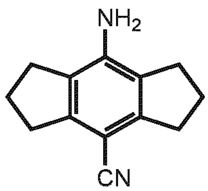

8-Amino-1,2,3,5,6,7-hexahydro-*s*-indacene-4-carbonitrile

**Step 1: 8-Bromo-1,2,3,5,6,7-hexahydro-*s*-indacen-4-amine**

Into a 100-mL round-bottom flask was placed a solution of 1,2,3,5,6,7-hexahydro-s-indacen-4-amine (2.6 g, 15.0 mmol) in DMF (30 mL). To the solution was added NBS (2.9 g, 16.3 mmol). The resulting solution was stirred for 12 h at RT and then was diluted with 80 mL of ethyl acetate. The resulting mixture was washed with 3x20 mL of water and concentrated under vacuum. The residue was applied onto a silica gel column and eluted with a gradient of ethyl acetate/petroleum ether (1:30 to 1 :20). This resulted in 3.0 g (79%) of the title compound as a brown solid. MS-ESI: 252.0, 254.0

**Step 2: 8-Amino-1,2,3,5,6,7-hexahydro-s-indacene-4-carbonitrile**

Into a 50-mL round-bottom flask purged with and maintained under nitrogen was placed a solution of 8-bromo-1,2,3,5,6,7-hexahydro-s-indacen-4-amine (725 mg, 2.88 mmol) in DMF (10 mL). To the solution were added t-BuOK (330 mg, 2.90 mmol), CuCN (386 mg, 4.32 mmol), and Pd(dppf)$Cl_2$ (424 mg, 0.58 mmol). The resulting solution was stirred for 12 h at 120°C and then was diluted with 20 mL of water. The resulting solution was extracted with 3x20 mL ethyl acetate. The organic layers were combined, dried over anhydrous $Na_2SO_4$, then concentrated under vacuum. The residue was applied onto a silica gel column and eluted with a gradient of ethyl acetate/petroleum ether (1:60 to 1:40). This resulted in 192 mg (34%) of the title compound as a yellow solid. MS-ESI: 199.1

**Scheme 27:**

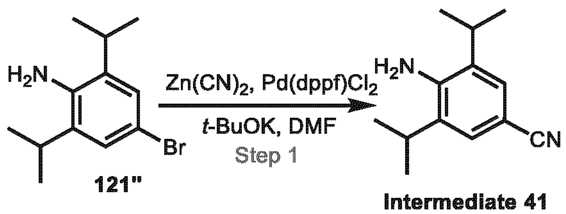


121"

Intermediate 41

**Intermediate 41**

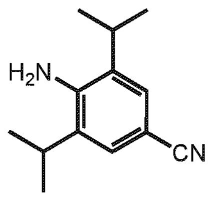

4-Amino-3,5-diisopropylbenzonitrile

**Step 1: 4-Amino-3,5-diisopropylbenzonitrile**

Into a 100-mL round-bottom flask purged with and maintained under nitrogen was placed a solution of 4-bromo-2,6-diisopropylbenzenamine (5.1 g, 19.9 mmol) in DMF (30 mL). To the solution were added $Zn(CN)_2$ (2.80 g, 23.9 mmol), Pd(dppf)$Cl_2$ (732 mg, 1.00 mmol) and t-BuOK (3.36 g, 29.9 mmol). The resulting mixture was stirred for 16 h at 120°C and then was diluted with 30 mL of water. The solution was extracted with 3x30 mL of ethyl acetate and the combined organic layers were concentrated under vacuum. The residue was applied onto a silica gel column and eluted with a gradiente of ethyl acetate/petroleum ether (1:30 to 1:20). This resulted in 3.2 g (80%) of the title compound as a yellow solid. MS-ESI: 203.1 (M+1).

**Scheme 28:**

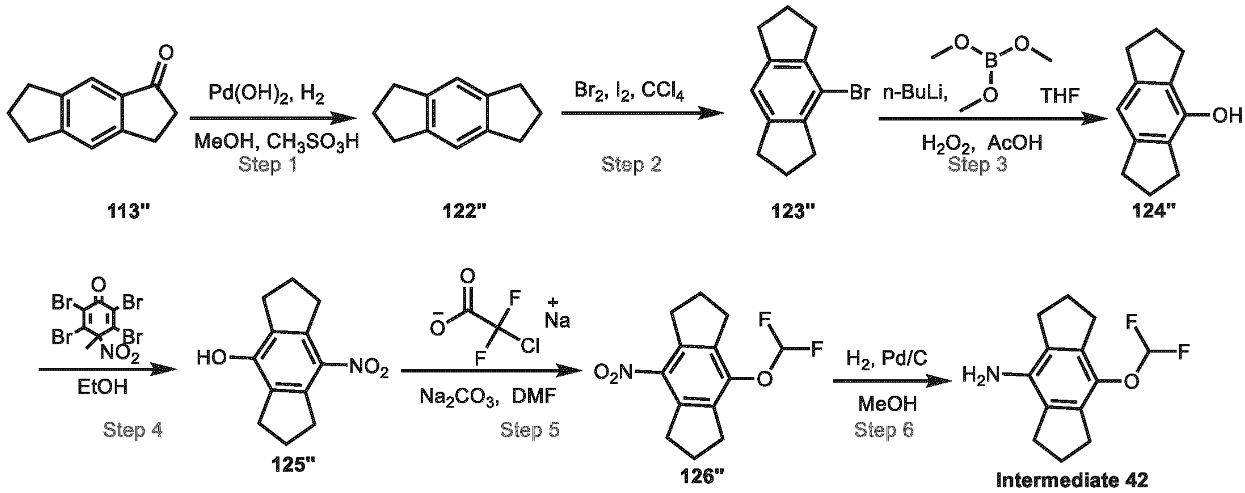


113" 122" 123" 124" 125" 126" Intermediate 42

## Intermediate 42

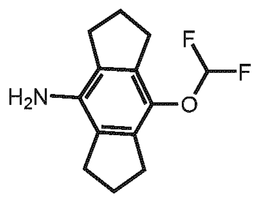

8-(Difluoromethoxy)-1,2,3,5,6,7-hexahydro-*s*-indacen-4-amine

**Step 1: 1,2,3,5,6,7-Hexahydro-*s*-indacene**

Into a 1-L round-bottom flask was placed a solution of 1,2,3,5,6,7-hexahydro-s-indacen-1-one (37.2 g, 216 mmol) and MSA (42 g, 437.5 mmol) in MeOH (300 mL). Then $Pd(OH)_2/C$ (20% wt, 8 g) was added. The flask was evacuated and filled three times with hydrogen. The resulting solution was stirred for 16 h at RT under an atmosphere of hydrogen. The solids were filtered out. The resulting mixture was concentrated under vacuum. The residue was applied onto a silica gel column and eluted with a gradient of ethyl acetate/petroleum ether (1:150 to 1: 100). This resulted in 27.1 g (79%) of the title compound as a white solid.

**Step 2: 4-Bromo-1,2,3,5,6,7-hexahydro-*s*-indacene**

Into a 500-mL 3-necked round-bottom flask purged with and maintained under nitrogen was placed a solution of 1,2,3,5,6,7-hexahydro-s-indacene (15 g, 94.8 mmol) in $CCl_4$ (200 mL). Then $I_2$ (1.2 g, 4.72 mmol) was added. This was followed by the addition of a solution of $Br_2$ (16 g, 100 mmol) in $CCl_4$ (50 mL) dropwise with stirring at 0°C in 10 min. The resulting solution was stirred for 2 h at 0°C. The reaction was then quenched by the addition of 150 mL of $NH_4Cl$ (sat.). The resulting solution was extracted with 3x150 mL of DCM and the combined organic layers were dried over anhydrous $Na_2SO_4$ and concentrated under vacuum. The crude product was purified by silica gel column with a gradient of ethyl acetate/hexane (1:500 to 1:100). This resulted in 19 g (85%) of the title compound as yellow oil. $^1H$ NMR (300 MHz, DMSO-$d_6$) δ 7.02 (s, 1H), 2.95-2.75 (m, 8H), 2.03-2.01 (m, 4H)

**Step 3: 1,2,3,5,6,7-Hexahydro-*s*-indacen-4-ol**

Into a 500-mL 3-necked round-bottom flask purged with and maintained under nitrogen was placed a solution of 4-bromo-1,2,3,5,6,7-hexahydro-s-indacene (5 g, 21.08 mmol) in THF (150 mL). This was followed by the addition of n-BuLi (2.5 M in hexane, 10 mL) dropwise with stirring at -78°C. The resulting solution was stirred for 30 min at -78°C. Then to the above was added trimethyl borate (2.6 g, 25.30 mmol) dropwise with stirring at -78°C. The reaction was warmed to RT slowly and then was stirred for 1 h at RT. Then to the mixture was added AcOH (2.0 mL, 33.20 mmol)

and $H_2O_2$ (1.0 mL, 28.88 mmol) dropwise with stirring at RT. The resulting solution was stirred for 2 h at RT and then was quenched by the addition of 200 mL of $NH_4Cl$ (sat.). The resulting solution was extracted with 3x200 mL of DCM. The organic layers were combined and dried over anhydrous $Na_2SO_4$, then concentrated under vacuum. The residue was applied onto a silica gel column and eluted with a gradient of ethyl acetate/petroleum ether (1:7 to 1:5). This resulted in 1.9 g (52%) of the title compound as an off-white solid. MS-ESI: 175.1 (M+1).

**Step 4: 8-Nitro-1,2,3,5,6,7-hexahydro-*s*-indacen-4-ol**

Into a 250-mL round-bottom flask purged with and maintained under nitrogen was placed a solution of 1,2,3,5,6,7-hexahydro-s-indacen-4-ol (1.9 g, 10.9 mmol) in EtOH (100 mL). To the solution was added 2,3,5,6-tetrabromo-4-methyl-4-nitrocyclohexa-2,5-dienone (6.1 g, 13.1 mmol). The resulting solution was stirred overnight at RT and then was concentrated under vacuum. The residue was applied onto a silica gel column and eluted with a gradient of ethyl acetate/petroleum ether (1:5 to 1:3). This resulted in 1.1 g (46%) of the title compound as a light yellow solid. MS-ESI: 218.1 (M-1).

**Step 5: 4-(Difluoromethoxy)-8-nitro-1,2,3,5,6,7-hexahydro-*s*-indacene**

Into a 100-mL round-bottom flask purged with and maintained under nitrogen was placed a solution of 8-nitro-1,2,3,5,6,7-hexahydro-*s*-indacen-4-ol (1.1 g, 5.0 mmol) in DMF (20 mL) and water (2 mL). To the solution were added $K_2CO_3$ (1.4 g, 10.0 mmol) and sodium 2-chloro-2,2-difluoroacetate (1.5 g, 10.0 mmol). The resulting solution was stirred for 1 h at 120°C and then was diluted with 20 mL of water. The pH value of the solution was adjusted to 7 with aq. HCl (1 N). The resulting solution was extracted with 3x20 mL of DCM. The organic layers were combined and dried over anhydrous $Na_2SO_4$, then concentrated under vacuum. The residue was applied onto a silica gel column and eluted with a gradient of ethyl acetate/petroleum ether (1:2 to 1:3). This resulted in 0.55 g (41%) of the title compound as a light yellow solid. MS-ESI: 270.1 (M+1).

**Step 6: 8-(Difluoromethoxy)-1,2,3,5,6,7-hexahydro-*s*-indacen-4-amine**

Into a 100-mL round-bottom flask was placed a solution of 4-(difluoromethoxy)-8-nitro-1,2,3,5,6,7-hexahydro-s-indacene (550 mg, 2.0 mmol) in MeOH (10 mL). Then Pd/C (10% wt., 100 mg) was added. The flask was evacuated and filled three times with hydrogen. The resulting solution was stirred for 12 h at RT under an atmosphere of hydrogen. The solids were filtered out. The resulting filtrate was concentrated under vacuum. This resulted in 460 mg (94%) of the title compound as a light yellow solid. MS-ESI: 240.1 (M+1).

**Scheme 29:**

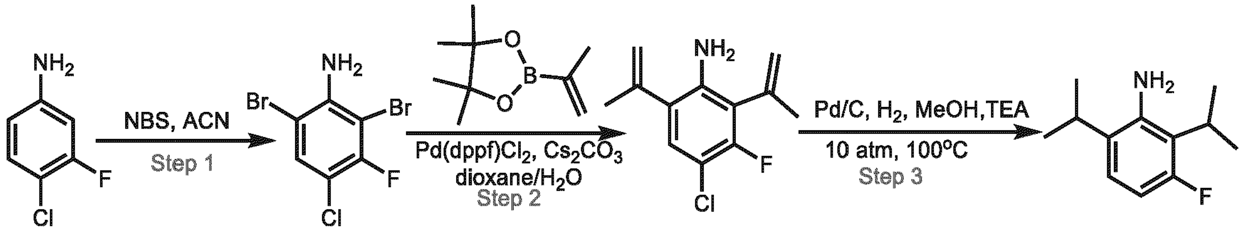


127" 128" 129" **Intermediate 43**

## Intermediate 43

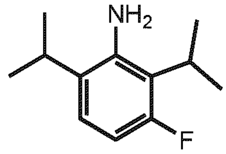

3-Fluoro-2,6-diisopropylbenzenamine

**Step 1: 2,6-Dibromo-4-chloro-3-fluoroaniline**

Into a 500-mL round-bottom flask was placed 4-chloro-3-fluoroaniline (5.08 g, 34.9 mmol), ACN (200 mL), and NBS (18.69 g, 105.0 mmol). The resulting solution was stirred for 12 h at RT and then was concentrated under vacuum. The residue was applied onto a silica gel column and eluted with a gradient of ethyl acetate/petroleum ether (1:200 to 1:100). This resulted in 9.7 g (92%) of the title compound as a light yellow solid. MS-ESI: 303.8/305.8/301.8 (M+1).

**Step 2: 4-Chloro-3-fluoro-2,6-bis(prop-1-en-2-yl)aniline**

Into a 500-mL round-bottom flask purged with and maintained under nitrogen was placed a solution of 2,6-dibromo-4-chloro-3-fluoroaniline (9.03 g, 29.8 mmol) in 1,4-dioxane (200 mL) and water (20 mL). To the solution were added 4,4,5,5-tetramethyl-2-(prop-1-en-2-yl)-1,3,2-dioxaborolane (15.12 g, 89.98 mmol), $Cs_2CO_3$ (29.34 g, 90.1 mmol) and $Pd(dppf)Cl_2$ (2.20 g, 3.0 mmol). The resulting solution was stirred for 12 h at 90°C and then was concentrated under vacuum. The residue was applied onto a silica gel column and eluted with a gradient of ethyl acetate/petroleum ether (1:30 to 1 :20). This resulted in 4.3 g (64%) of the title compound as yellow oil. MS-ESI: 226.1, 228.1 (M+1).

**Step 3: 3-Fluoro-2,6-bis(propan-2-yl)aniline**

Into a 100-mL round-bottom flask was placed a solution of 4-chloro-3-fluoro-2,6-bis(prop-1-en-2-yl)aniline (1 g, 4.4 mmol) in MeOH (15 mL). Then Pd/C(10% wt., 100 mg) was added. The flask was evacuated and filled three times with hydrogen. The resulting solution was stirred for 3 h at RT under an atmosphere of hydrogen. The solids were filtered out. The resulting filtrate was concentrated under vacuum. The residue was applied onto a silica gel column and eluted with a gradient of ethyl acetate/petroleum ether (1:5 to 1:3). This resulted in 700 mg (81%) of the title compound as light yellow oil. MS-ESI: 196.1 (M+1).

**Scheme 30:**

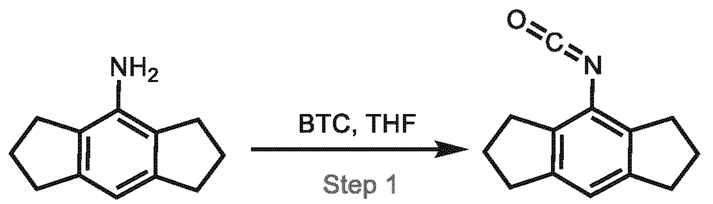


130"

Intermediate 44

**Intermediate 44**

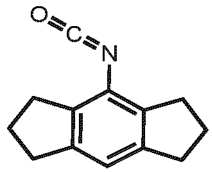

4-Isocyanato-1,2,3,5,6,7-hexahydro-*s*-indacene

**Step 1: 4-Isocyanato-1,2,3,5,6,7-hexahydro-*s*-indacene**

Into a 50-mL round-bottom flask purged with and maintained under nitrogen was placed 1,2,3,5,6,7-hexahydro-*s*-indacen-4-amine (64 mg, 0.4 mmol), THF (5 mL) and BTC (37 mg, 0.1 mmol). The resulting solution was stirred for 2 h at 65°C and then was concentrated under vacuum. This resulted in 75 mg (crude) of the title compound as light brown oil. The crude product was used directly in the next step.

**Table 9.** The Intermediates in the following Table were prepared using similar procedure as shown in **Scheme 30** above for converting **compound 130"** to **Intermediate 44.**

| Intermediate # | Structure | IUPAC Name |
|---|---|---|
| Intermediate 45 | 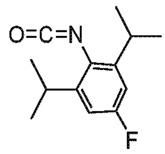 | 5-Fluoro-2-isocyanato-1,3 -diisopropylbenzene |
| Intermediate 46 | 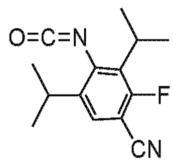 | 2-Fluoro-4-isocyanato-3,5-diisopropylbenzonitrile |
| Intermediate 47 | 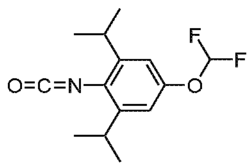 | 5-(Difluoromethoxy)-2-isocyanato-1,3-diisopropylbenzene |
| Intermediate 48 | 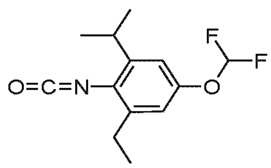 | 5-(Difluoromethoxy)-1-ethyl-2-isocyanato-3 -isopropylbenzene |
| Intermediate 49 | 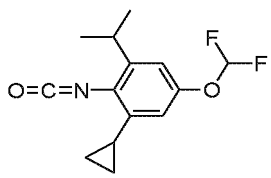 | 1-Cyclopropyl-5-(difluoromethoxy)-2-isocyanato-3-isopropylbenzene |
| Intermediate 50 | 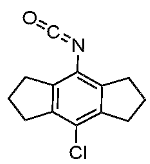 | 4-Chloro-8-isocyanato-1,2,3,5,6,7-hexahydro-s-indacene |
| Intermediate 51 | 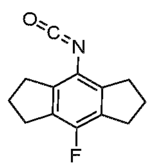 | 4-Fluoro-8-isocyanato-1,2,3,5,6,7-hexahydro-s-indacene |
| Intermediate 52 | 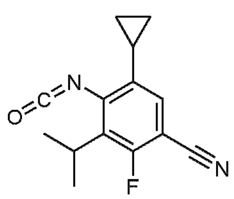 | 5-Cyclopropyl-2-fluoro-4-isocyanato-3-isopropylbenzonitrile |

(continued)

| Intermediate # | Structure | IUPAC Name |
|---|---|---|
| Intermediate 53 | 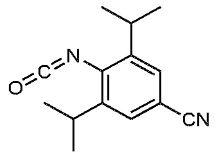 | 4-Isocyanato-3,5-diisopropylbenzonitrile |
| Intermediate 54 | 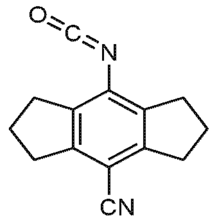 | 1,2,3,5,6,7-Hexahydro-8-isocyanato-*s*-indacene-4-carbonitrile |
| Intermediate 55 | 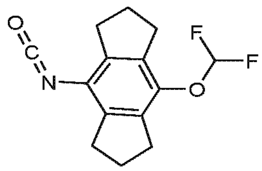 | 4-(Difluoromethoxy)-1,2,3,5,6,7-hexahydro-8-isocyanato-*s*-indacene |
| Intermediate 56 | 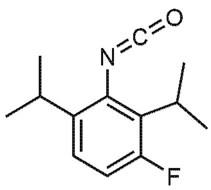 | 1-Fluoro-3-isocyanato-2,4-diisopropylbenzene |
| Intermediate 57 | 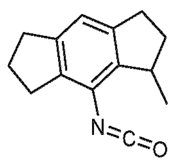 | 1,2,3,5,6,7-Hexahydro-8-isocyanato-1-methyl-*s*-indacene |
| Intermediate 58 | 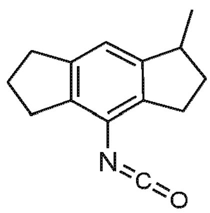 | 1,2,3,5,6,7-Hexahydro-4-isocyanato-1-methyl-*s*-indacene |

The following schemes illustrate additional general methods for the synthesis of compounds of Formula AA:

**Scheme 31:**

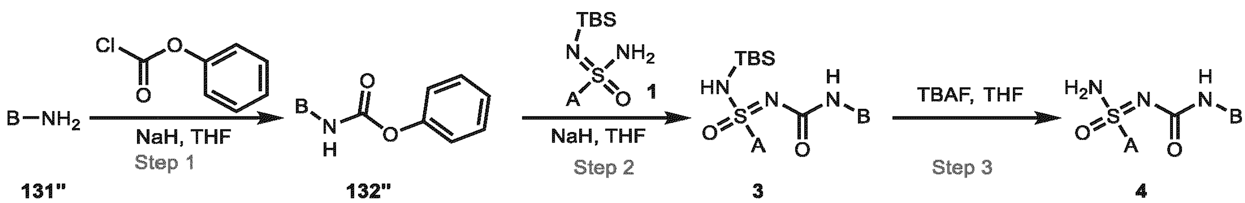


131'' 132'' 3 4

**Scheme 32:**

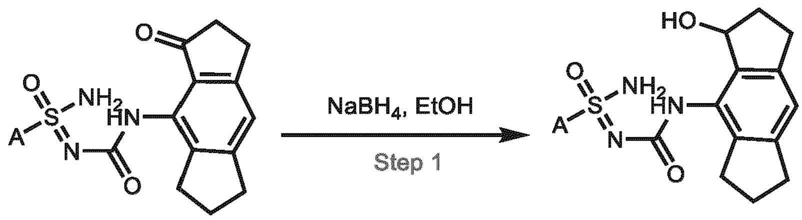


133"

134"

**Scheme 33A:**

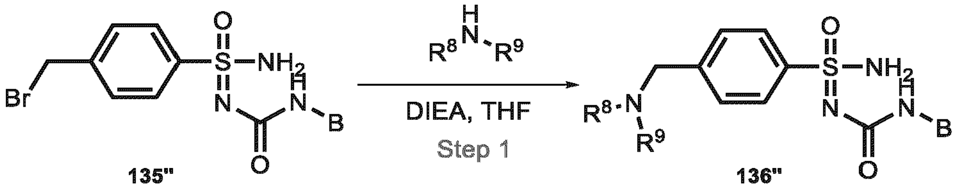


135"

136"

**Scheme 33B:**

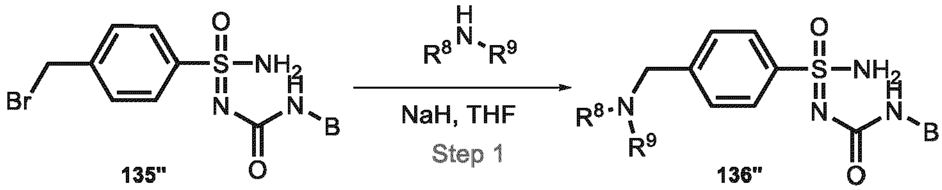


135"

136"

**Scheme 34:**

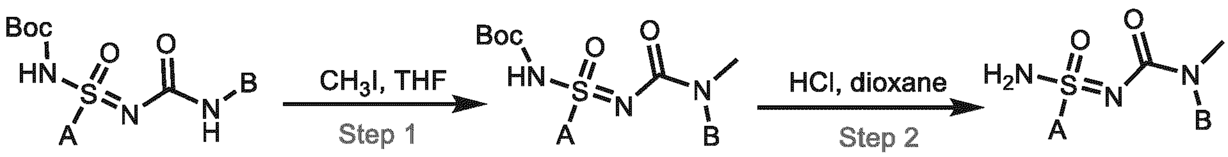


6

138"

139"

**Scheme 35:**

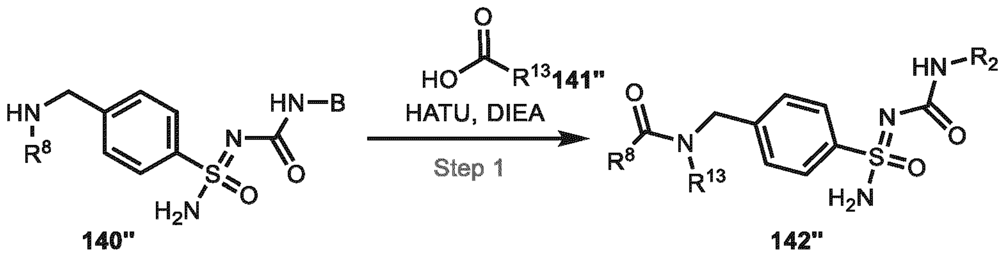


140"

142"

## Scheme 35A:

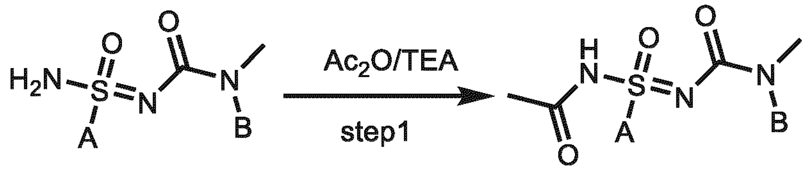


140"A 142"B

**Scheme for the preparation of Sulfonimidamide Intermediates:** Schemes below illustrate the preparation of sulfonimidamide intermediates **59-88** and **112-113.**

## Scheme 36:

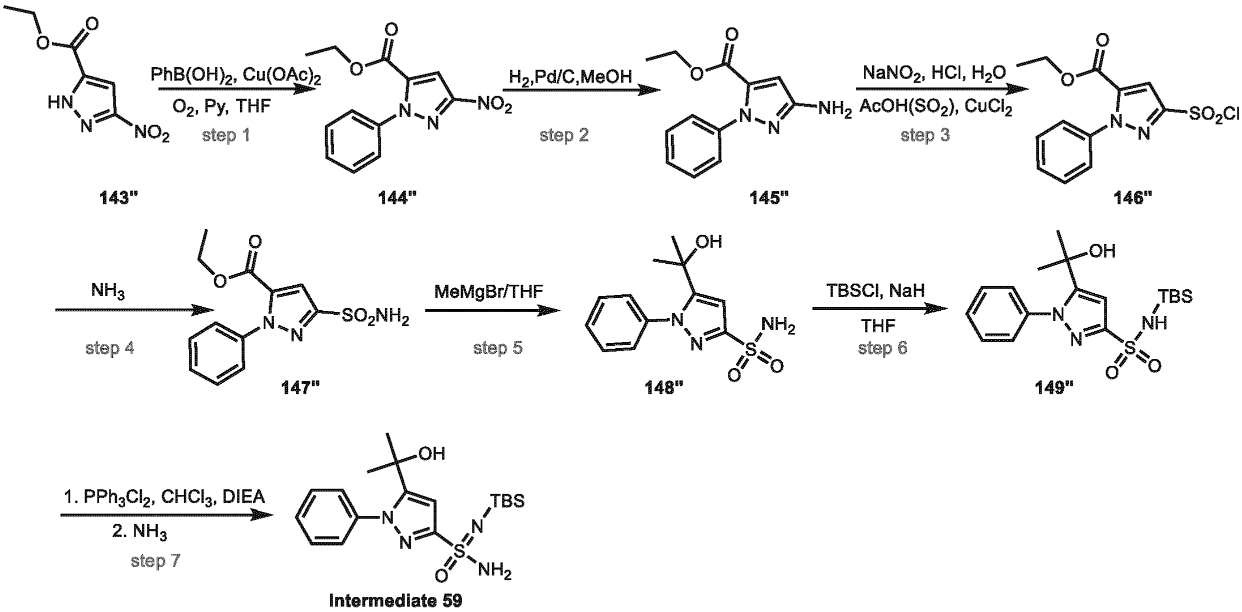


143" 144" 145" 146" 147" 148" 149" Intermediate 59

## Intermediate 59

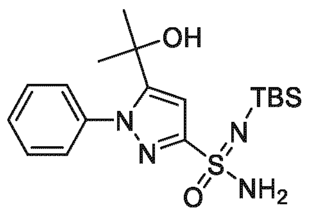

*N'*-(*tert*-butyldimethylsilyl)-5-(2-hydroxypropan-2-yl)-1-phenyl-1H-pyrazole-3-sulfonimidamide

**Step 1: Ethyl 3-nitro-1-phenyl-1H-pyrazole-5-carboxylate**

Into a 250-mL round-bottom flask purged and maintained with an inert atmosphere of nitrogen, was placed ethyl 3-nitro-1H-pyrazole-5-carboxylate (5.0 g, 27.0 mmol), THF (150 mL), phenylboronic acid (6.6 g, 54.1 mmol), $Cu(OAc)_2$ (7.38 g, 40.6 mmol), and pyridine (8.54 g, 108 mmol). The resulting solution was stirred overnight at RT. The resulting mixture was concentrated under vacuum. The residue was eluted from a silica gel column with ethyl acetate/petroleum ether (1:1). This resulted in 3.1 g (44%) of the title compound as an off-white solid. MS-ESI: 262 (M+1).

**Step 2: Ethyl 3-amino-1-phenyl-1H-pyrazole-5-carboxylate**

Into a 100-mL round-bottom flask, was placed ethyl 3-nitro-1-phenyl-1H-pyrazole-5-carboxylate (3.92 g, 15.0 mmol), MeOH (50 mL), and Pd/C (wet 10%wt., 400 mg). The flask was evacuated and filled three times with hydrogen. The resulting solution was stirred overnight at RT. The solids were filtered out. The resulting mixture was concentrated under vacuum. This resulted in 2.8 g (81%) of the title compound as a light yellow solid. MS-ESI: 232 (M+1).

**Step 3: Ethyl 3-(chlorosulfonyl)-1-phenyl-1H-pyrazole-5-carboxylate**

Into a 100-mL round-bottom flask, was placed ethyl 3-amino-1-phenyl-1H-pyrazole-5-carboxylate (1.8 g, 7.78 mmol), HCl (cc. 6.0 mol/L, 15 mL). This was followed by the addition of a solution of $NaNO_2$ (646 mg, 9.36 mmol) in water (2.0 mL) dropwise with stirring at -10 °C. The resulting solution was stirred for 30 min at -10 °C. The above mixture was added to a saturated solution of $SO_2$ in AcOH (20 mL) dropwise with stirring at 0 °C. Then to the above was added $CuCl_2$ (1.05 g, 7.81 mmol). The resulting solution was stirred for 1 h at RT. The reaction was then quenched by the addition of 30 mL of water. The resulting solution was extracted with 3x30 mL of DCM and the organic layers combined and dried over anhydrous sodium sulfate and concentrated under vacuum. This resulted in 2.2 g (90%) of the title compound as a light yellow solid.

**Step 4: Ethyl 1-phenyl-3-sulfamoyl-1H-pyrazole-5-carboxylate**

Into a 100-mL round-bottom flask, was placed a solution of ethyl 3-(chlorosulfonyl)-1-phenyl-1H-pyrazole-5-carboxylate (2.2 g, 6.99 mmol) in DCM (10 mL). Then to the above was introduced $NH_3$ gas bubbled at 0 °C for 10 min. The resulting solution was stirred for 2 h at RT. The resulting mixture was concentrated under vacuum. The residue was eluted from a silica gel column with ethyl acetate/petroleum ether (1:1). This resulted in 1.07 g (52%) of the title compound as a light yellow solid. MS-ESI: 296 (M+1).

**Step 5: 5-(2-Hydroxypropan-2-yl)-1-phenyl-1H-pyrazole-3-sulfonamide**

Into a 100-mL 3-necked round-bottom flask purged and maintained with an inert atmosphere of nitrogen, was placed a solution of ethyl 1-phenyl-3-sulfamoyl-1H-pyrazole-5-carboxylate (1.65 g, 5.59 mmol) in THF (30 mL). This was followed by the addition of MeMgBr/THF (3.0 M, 18.6 mL) dropwise with stirring at 0 °C. The resulting solution was stirred overnight at RT. The reaction was then quenched by the addition of 30 mL of $NH_4Cl$ (sat.). The resulting solution was extracted with 3x30 mL of DCM and the organic layers combined and dried over anhydrous sodium sulfate and concentrated under vacuum. The residue was eluted from a silica gel column with ethyl acetate/petroleum ether (2:1). This resulted in 1.35 g (86%) of the title compound as a yellow solid. MS-ESI: 282 (M+1).

**Step 6: *N*-(*tert*-butyldimethylsilyl)-5-(2-hydroxypropan-2-yl)-1-phenyl-1H-pyrazole-3-sulfonamide**

Into a 100-mL round-bottom flask, was placed 5-(2-hydroxypropan-2-yl)-1-phenyl-1H-pyrazole-3- sulfonamide (500 mg, 1.78 mmol), THF (10 mL). This was followed by the addition of sodium hydride (60% wt. oil dispersion, 86 mg, 3.58 mmol) in portions at 0 °C. Then to the above was added TBSCl (538 mg, 3.57 mmol). The resulting solution was stirred for 2 h at RT. The reaction was then quenched by the addition of 10 mL of water. The resulting solution was extracted with 3x10 mL of DCM. The organic layers were combined, dried over anhydrous sodium sulfate, and concentrated under vacuum. The residue was eluted from a silica gel column with ethyl acetate/petroleum ether (1:2). This resulted in 660 mg (94%) of the title compound as a light yellow solid. MS-ESI: 396 (M+1).

**Step 7: *N'*-(*tert*-butyldimethylsilyl)-5-(2-hydroxypropan-2-yl)-1-phenyl-1H-pyrazole-3-sulfonimidamide**

Into a 100-mL 3-necked round-bottom flask purged and maintained with an inert atmosphere of nitrogen, was placed the solution of $PPh_3Cl_2$ (1.67 g, 5.01 mmol) in chloroform (30 mL). This was followed by the addition of DIEA (1.29 g, 9.98 mmol) dropwise with stirring at RT. The resulting solution was stirred for 10 min at RT and the reaction system was cooled to 0 °C. To this was added a solution of N-(tert-butyldimethylsilyl)-5-(2-hydroxypropan-2-yl)-1-phenyl-1H-pyrazole-3-sulfonamide (660 mg, 1.67 mmol) in chloroform (3.0 mL) dropwise with stirring at 0 °C. The resulting solution was stirred for 30 min at 0 °C. To the mixture was added introduced $NH_3$ gas bubble for 15 min at 0 °C. The resulting solution was stirred for 2 h at RT. The resulting solution was diluted with 30 mL of water. The resulting solution was extracted with 3x30 mL of DCM and the organic layers combined and dried over anhydrous sodium sulfate and concentrated under vacuum. The residue was eluted from a silica gel column with ethyl acetate/petroleum ether (1:1). This resulted in 530 mg (81%) of the title compound as a light yellow solid. MS-ESI: 395 (M+1).

**Scheme 37:**

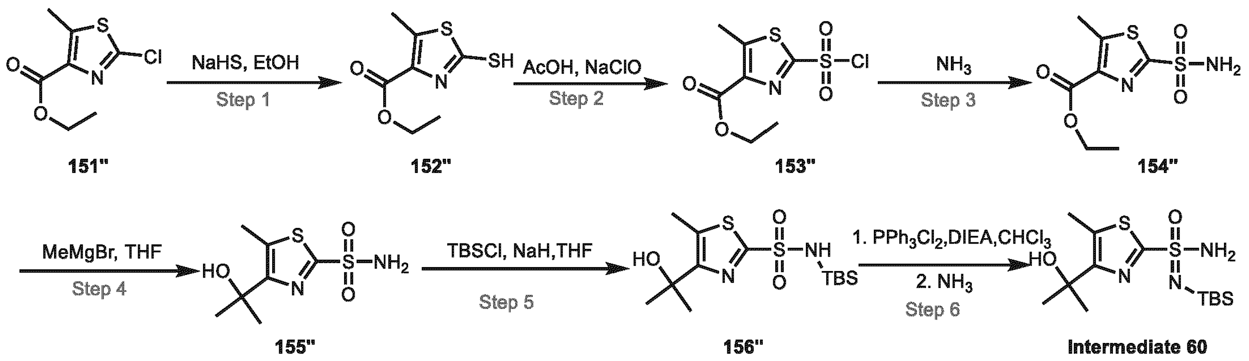


## Intermediate 60

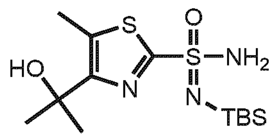

*N'*-(tert-butyldimethylsilyl)-4-(2-hydroxypropan-2-yl)-5-methylthiazole-2-sulfonimidamide

**Steps 1-6** used similar procedures for converting compound **16** to intermediate **2** shown in Scheme 7B to afford intermediate **60** from compound **151"**. MS-ESI: 350 (M+1).

**Scheme 38:**

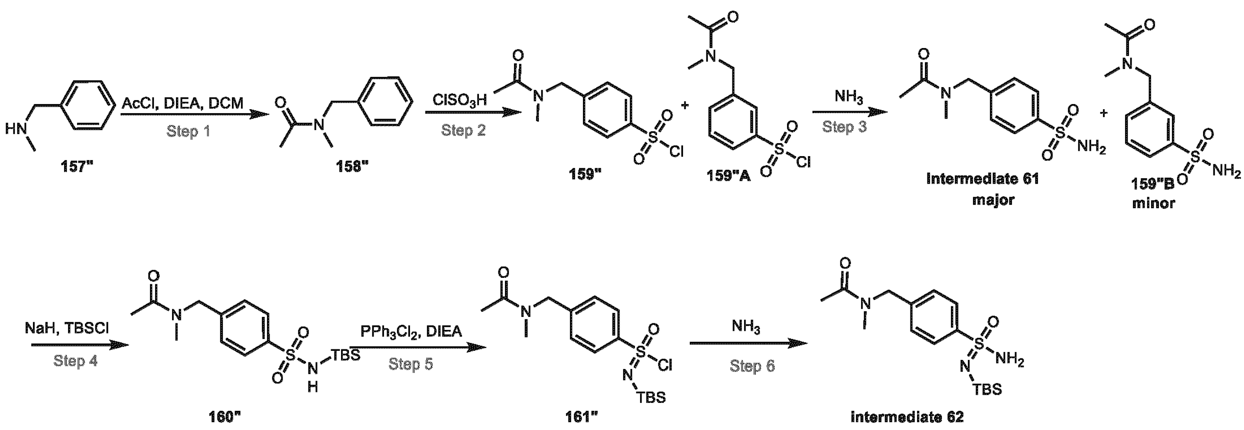


## Intermediate 61

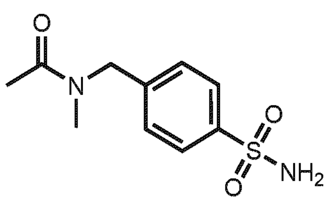

N-methyl-N-(4-sulfamoylbenzyl)acetamide

## Intermediate 62

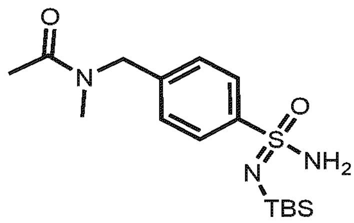

N-(4-(N'-(tert-butyldimethylsilyl)sulfamidimidoyl)benzyl)-N-methylacetamide

**Step 1: N-benzyl-N-methylacetamide**

Into a 1.0 L round-bottom flask were added benzyl(methyl)amine (10 g, 82.5 mmol) and DCM (500 mL) at 0 °C. To this stirred solution were added DIEA (21.3 g, 165 mmol) and acetyl chloride (9.72 g, 124 mmol) in portions at 0 °C. The resulting mixture was stirred for 4 h at RT. The resulting mixture was concentrated under reduced pressure. The residue was eluted from a silica gel column with ethyl acetate/petroleum ether (1:1) to afford the title compound (13 g, 96.5%) as a yellow oil. MS-ESI: 164 (M+1).

**Step 2: 4-((N-methylacetamido)methyl)benzenesulfonyl chloride**

Into a 250 mL round-bottom flask were added N-benzyl-N-methylacetamide (3.0 g, 18.4 mmol,) and DCM (6.0 mL) at 0 °C. To this stirred solution were added $ClSO_2OH$ (6.0 mL) in one portion at 0 °C. The resulting mixture was stirred for 3 h at RT. The reaction was quenched by the addition of water/ice (150 mL) at 0 °C. The resulting solution was extracted with 3x150 mL of DCM and the organic layers combined and dried over anhydrous sodium sulfate and concentrated under vacuum. The crude product of the title compound (2.2 g, 45.7%)) was used in the next step directly without further purification.

**Step 3: N-methyl-N-(4-sulfamoylbenzyl)acetamide**

Into a 250 mL round-bottom flask were added 4-[(N-methylacetamido)methyl]benzene-1-sulfonyl chloride (2.2 g, 8.41 mmol) and DCM (3.0 mL) at 0 °C. To this stirred solution were added $NH_3$(g) in DCM (40 mL) dropwise at 0 °C. The resulting mixture was stirred overnight at RT. The resulting mixture was concentrated under reduced pressure. The residue was eluted from a silica gel column with ethyl acetate/petroleum ether (1:1) to afford the minor compound 159B (122 mg, 6.1%) and the title compound (1.9 g, 93.3%) both as white solids. MS-ESI: 243 (M+1).

**Step 4-6** used similar procedures for converting compound **148"** to intermediate **59** shown in **Scheme 36** to afford intermediate **62** from intermediate **61.** MS-ESI: 356 (M+1).

**Table 10.** Intermediate 62B in the following Table was prepared using the similar procedures for converting compound **157"** to Intermediate **62** shown in **Scheme 38** from compound 159"B which was separated from step 3 in Scheme 38. The Intermediate 63 was prepared using similar procedures for converting compound 157" to Intermediate 62 shown in Scheme 38 from appropriate starting materials.

| Intermediate # | Structure | IUPAC Name | Exact Mass [M+H]+ |
|---|---|---|---|
| **Intermediate 62B** | 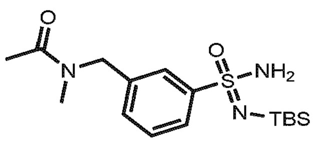 | N-(3-(N'-(tert-butyldimethylsilyl) sulfamimidoyl)benzyl)-N-methylacetamide | 356 |
| **Intermediate 63** | 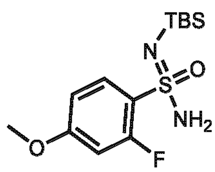 | N'-(tert-butyldimethylsilyl)-2-fluoro-4-methoxybenzenesulfonimidamide | 319 |

**Scheme 39:**

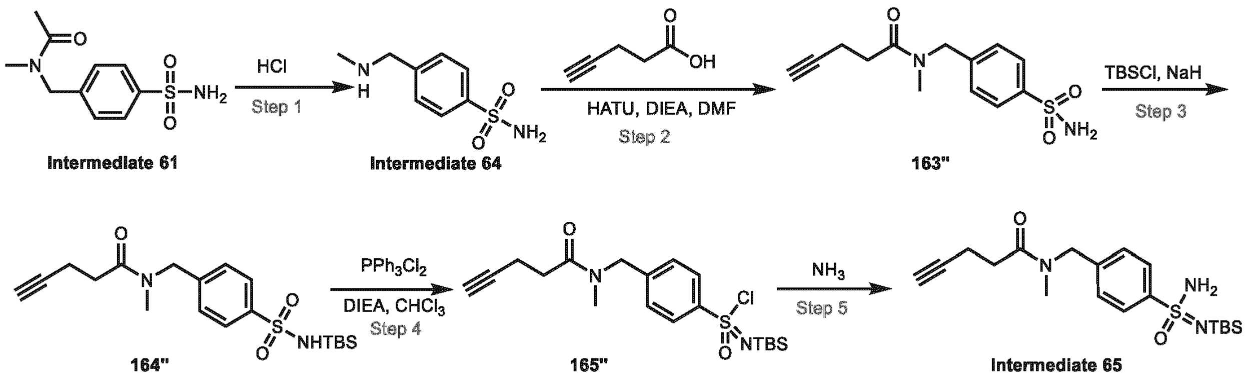


## Intermediate 64

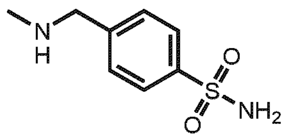

4-((Methylamino)methyl)benzenesulfonamide

## Intermediate 65

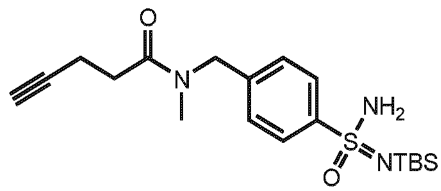

N-(4-(N'-(tert-butyldimethylsilyl)sulfamimidoyl)benzyl)-N-methylpent-4-ynamide

**Step 1: 4-((Methylamino)methyl)benzenesulfonamide**

Into a 500-mL sealed tube, was placed N-methyl-N-[(4-sulfamoylphenyl)methyl]acetamide (5.0 g), hydrogen chloride (200 mL, 12 M). The resulting solution was stirred for 16 h at 100 °C in an oil bath. The resulting mixture was concentrated. This resulted in 5.0 g of the title compound as an off-white crude solid. MS-ESI: 201 (M+1)

**Step 2: *N*-methyl-N-(4-sulfamoylbenzyl)pent-4-ynamide**

Into a 250 mL round-bottom flask was placed 4-((methylamino)methyl)benzenesulfonamide (4.0 g, 20 mmol) in DMF (40 mL ). To this stirred solution was added HATU (6.33 g,16.7 mmol), DIEA (5.16 g, 40 mmol) and pent-4-ynoic acid (2.16 g, 22 mmol). Then the mixture was stirred overnight RT. The resulting solution was diluted with 40 mL of water. The resulting solution was extracted with 3x50 mL of ethyl acetate and the organic layers combined and dried over anhydrous sodium sulfate and concentrated under vacuum. The residue was eluted from a silica gel column with ethyl acetate/petroleum ether (1:1). This resulted in 2.97 g (53%) of the title compound as a light yellow solid. MS-ESI: 281 (M+1).

**Steps 3-5** used similar procedures for converting Intermediate **61** to Intermediate **62** shown in Scheme 38 to afford Intermediate **65** from compound **163".** MS-ESI: 394 (M+1).

**Scheme 40A:**

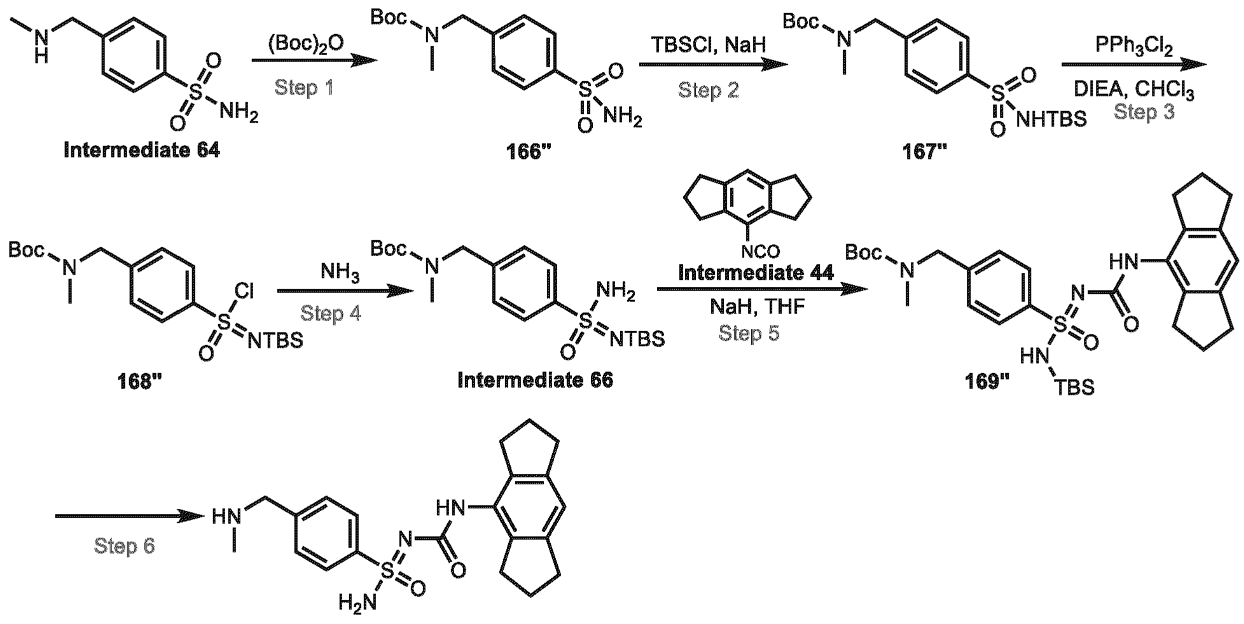


Intermediate 64, 166", 167", 168", Intermediate 66, 169", Intermediate 67

## Intermediate 66

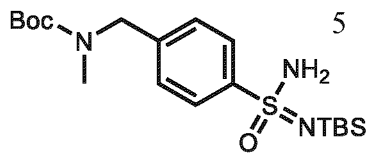

Tert-butyl 4-(N'-(tert-butyldimethylsilyl)sulfamimidoyl)benzyl(methylicarbamate

## Intermediate 67

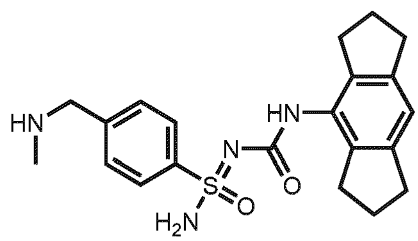

N'-((1,2,3,5,6,7-hexahydro-s-indacen-4-yl)carbamoyl)-4-((methylamino)methyl)benzenesulfonimidamide

**Step 1: *Tert*-butyl methyl(4-sulfamoylbenzyl)carbamate**

Into a 250-mL round-bottom flask, was placed 4-[(methylamino)methyl]benzene-1-sulfonamide (5.0 g, 25 mmol) in DCM (100 mL). To this stirred solution was added di-tert-butyl dicarbonate (6.0 g, 27.5 mmol). The resulting solution was stirred for 5 h at RT. The resulting mixture was concentrated. The residue was eluted from a silica gel column with ethyl acetate/petroleum ether (1:1). This resulted in 5.0 g (66.7 %) of the title compound as a light yellow solid. MS-ESI: 301 (M+1).

**Steps 2-4** used similar procedures for converting compound **148"** to intermediate **59** shown in Scheme 36 to afford Intermediate **66** from compound **166".** MS-ESI: 414 (M+1).

**Step 5: *Tert*-butyl(4-(N-(tert-butyldimethylsilyl)-*N'*-((1,2,3,5,6,7-hexahydro-s-indacen-4-yl)carbamoyl)-sulfamidimidoyl)benzyl)(methyl)carbamate**

Into a 50-mL round-bottom flask, was placed tert-butyl (4-(N'-(tertbutyldimethylsilyl)sulfamidimidoyl)benzyl)(methyl)carbamate (500 mg, 1.21 mmol) in THF (15 mL). To this stirred solution was added 4-isocyanato-1,2,3,5,6,7-hexahydro-s-indacene (343 mg, 1.81 mmol) and NaH (60%wt. oil dispersion, 96.8 mg, 2.42 mmol). The resulting solution was stirred for 3 h at RT. The reaction was quenched by the addition of MeOH (10 mL). This resulted in 500 mg (67.5%) of the title compound as a white crude solid. MS-ESI: 613 (M+1).

**Step 6: *N'*-(1,2,3,5,6,7-hexahydro-*s*-indacen-4-ylcarbamoyl)-4-((methylamino)methyl)benzenesulfonimidamide**

Into a 50-mL round-bottom flask was placed tert-butyl N-[(4-[[(tert-butyldimethylsilyl)amino] ([[(1,2,3,5,6,7-hexahydro-*s*-indacen-4-yl)carbamoyl]imino])oxo-$\lambda^6$-sulfanyl]phenyl)methyl]-N-methylcarbamate (90 mg) and HCl in dioxane (4 M, 5.0 mL). The resulting solution was stirred for 16 h at RT. The resulting mixture was concentrated. The crude product was purified by Prep-HPLC with the following conditions: Column, Sunfire Prep C18 OBD,10 um, 19*250 mm; mobile phase A: water (0.05% TFA) and B: ACN (20% to 50% gradient of B over 17 min); Detector, UV 220/254 nm. This resulted in 30 mg of the title compound as a white solid. MS-ESI: 399 (M+1).

Scheme 42:

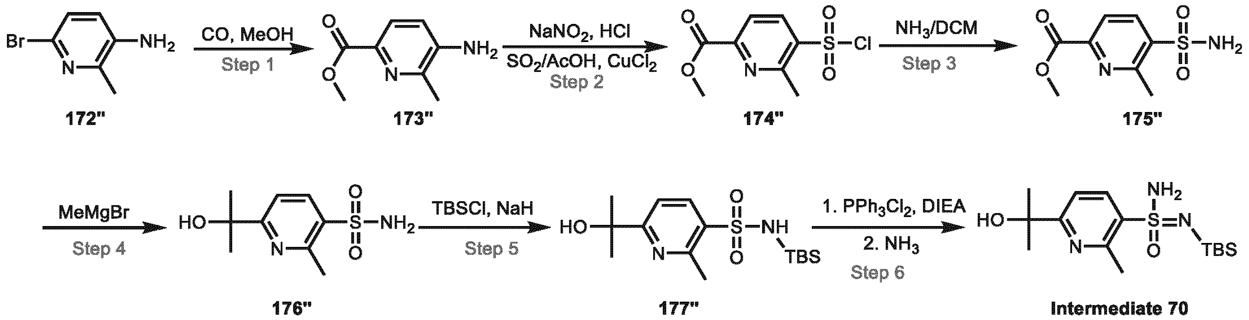


172" 173" 174" 175" 176" 177" Intermediate 70

Intermediate 70

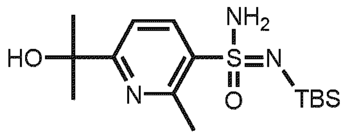

*N'*-(*tert*-butyldimethylsilyl)-6-(2-hydroxypropan-2-yl)-2-methylpyridine-3-sulfonimidamide

**Step 1: Methyl 5-amino-6-methylpicolinate**

Into a 50-mL seal tube was placed methyl 6-bromo-2-methylpyridin-3-amine (500 mg, 2.67 mmol) in MeOH (15 mL) and $Pd(OAc)_2$ (120 mg, 0.53 mmol), dppf (444 mg, 0.80 mmol), TEA (809 mg, 8.01 mmol). The seal tube was evacuated and flushed three times with CO. The resulting solution was stirred for 5 h at 100 °C under 10 atm of CO. Then the solution was concentrated under vacuum. The residue was eluted from a silica gel column with ethyl acetate/petroleum ether (1:1). This resulted in 351 mg (79.2 %) of the title compound as a light yellow solid. MS-ESI: 167 (M+1). **Steps 2-4** used similar procedures for converting compound **27** to Intermediate **30** shown in **Scheme 9** to afford compound **176"** from compound **173".** MS-ESI: 231 (M+1).

**Steps 5-6** used similar procedures for converting compound **148"** to intermediate **59** shown in Scheme 36 to afford Intermediate **70** from compound **176".** MS-ESI: 344 (M+1).

**Table 11.** The Intermediates in the following Table were prepared using the similar procedures for converting compound **172"** to Intermediate **70** shown in Scheme 42 from appropriate starting materials.

| Intermediate # | Structure | IUPAC Name | Exact Mass [M+H]+ |
|---|---|---|---|
| **Intermediate 71** | 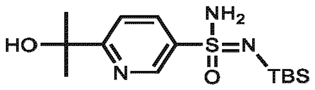 | *N'*-(*tert*-butyldimethylsilyl)-6-(2-hydroxypropan-2-yl)pyridine-3-sulfonimidamide | 330 |

**Scheme 43:**

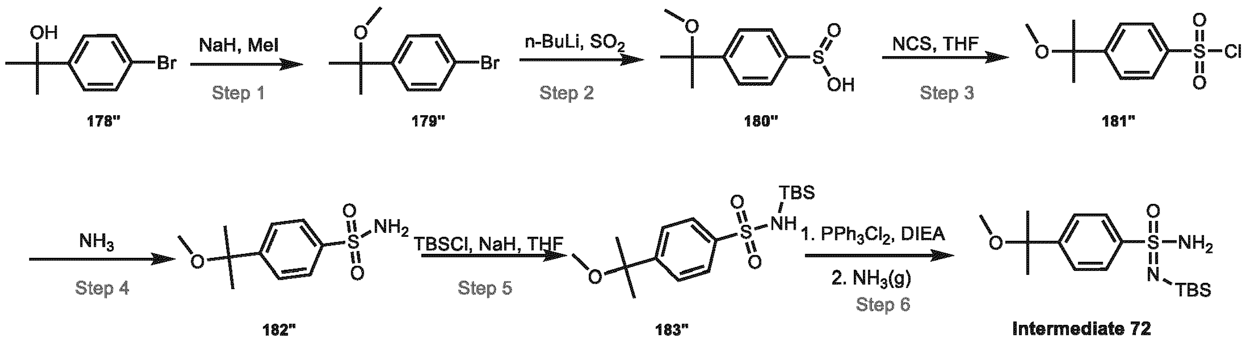


**Intermediate 72**

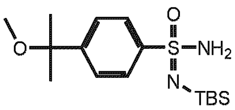

<u>*N'*-(*tert*-butyldimethylsilyl)-4-(2-methoxypropan-2-yl)benzenesulfonimidamide</u>

**Step 1: 1-Bromo-4-(2-methoxypropan-2-yl)benzene**

Into a 250-mL round-bottom flask, was placed a solution of 2-(4-bromophenyl)propan-2-ol (10 g, 46.5 mmol) in THF (50 mL). To this stirred solution was added NaH (60% wt. oil dispersion, 5.19 g, 93 mmol) at 0 °C. The resulting solution was stirred for 30 min at 0 °C. To this stirred solution was added MeI (6.60 g, 46.5 mmol) dropwise with stirring at 0 °C. The resulting solution was allowed to react for an additional 15 h at RT. The resulting solution was quenched with 40 mL of water. The resulting solution was extracted with 3x50 mL of ethyl acetate and the organic layers combined and dried over anhydrous sodium sulfate and concentrated under vacuum. The residue was eluted from a silica gel column with ethyl acetate/petroleum ether (15/85). This resulted in 8.5 g (50.3%) of the title compound as a yellow solid.

**Step 2: 4-(2-Methoxypropan-2-yl)benzenesulfinic acid**

Into a 250-mL 3-necked round-bottom flask purged and maintained with an inert atmosphere of nitrogen, was placed a solution of 1-bromo-4-(2-methoxypropan-2-yl)benzene (5.0 g, 21.8 mmol) in THF (50 mL). To this stirred solution was added n-BuLi (13 mL, 32.7 mmol, 2.5 M) dropwise with stirring at -78 °C. The resulting solution was stirred for 30 min at -78 °C. $SO_2$(g) was introduced into the stirring solution at -78 °C. The resulting solution was allowed to react for an additional 60 min at RT. The resulting mixture was concentrated. This resulted in 6.0 g (crude) of the title compound as a yellow solid. MS-ESI: 213 (M-1)

**Step 3: 4-(2-Methoxypropan-2-yl)benzenesulfonyl chloride**

Into a 50-mL round-bottom flask, was placed 4-(2-methoxypropan-2-yl)benzene-1-sulfinic acid (4.9 g, 22.9 mmol) in THF (50 mL). To this stirred solution was added NCS (4.58 g, 34.3 mmol). The resulting solution was stirred for 30 min at 0 °C. The mixture was allowed to react for an additional 60 min at RT. $NH_3$ (g) was introduced into the reaction solution. The resulting solution was allowed to react for an additional 120 min at RT. The resulting mixture was concentrated. The residue was eluted from a silica gel column with ethyl acetate/petroleum ether (1/4). This resulted in

4.3 g (82 %) of the title compound as a yellow solid.

**Step 4: 4-(2-Methoxypropan-2-yl)benzenesulfonamide**

Into a 250-mL round-bottom flask was placed 4-(2-methoxypropan-2-yl)benzene-1-sulfonyl chloride (4.3 g, 17.3 mmol) in DCM (50 mL). $NH_3$ (g) was introduced into the reaction solution at 0 °C. The resulting solution was stirred for 180 min at RT. The resulting mixture was concentrated. The residue was eluted from a silica gel column with ethyl acetate/petroleum ether (1/3). This resulted in 3.9 g (98.5%) of the title compound as a yellow solid. MS-ESI: 230 (M+1).

**Step 5: *N*-(*tert*-butyldimethylsilyl)-4-(2-methoxypropan-2-yl)benzenesulfonamide**

Into a 100-mL round-bottom flask, was placed a solution of 4-(2-methoxypropan-2-yl)benzene-1-sulfonamide (4.0 g, 17.5 mmol) in THF (40 mL). To this stirred solution was added NaH (1.4 g, 34.9 mmol, 60%wt. oil dispersion) and TBSCl (3.16 g, 21 mmol) at 0 °C. The resulting solution was allowed to react with stirring for 15 h at RT. The resulting solution was quenched with 40 mL of water. The resulting solution was extracted with 3x50 mL of ethyl acetate and the organic layers combined and dried over anhydrous sodium sulfate and concentrated under vacuum. The residue was eluted from a silica gel column with ethyl acetate/petroleum ether (30/70). This resulted in 2.3 g (38.4%) of the title compound as a yellow solid. MS-ESI: 344 (M+1)

**Step 6: *N'*-(*tert*-butyldimethylsilyl)-4-(2-methoxypropan-2-yl)benzenesulfonimidamide**

Into a 500-mL 3-necked round-bottom flask purged and maintained with an inert atmosphere of nitrogen, was placed the solution of $PPh_3Cl_2$ (12.4 g, 37.3 mmol) in chloroform (150 mL). This was followed by the addition of DIEA (9.63 g, 74.5 mmol) dropwise with stirring at RT. The resulting solution was stirred for 10 min at RT and the reaction system was cooled to 0 °C. To this was added a solution of N-(tert-butyldimethylsilyl)-4-(2-methoxypropan-2-yl)benzene-1-sulfonamide (3.2 g, 9.31 mmol) in chloroform (30 mL) dropwise with stirring at 0 °C. The resulting solution was stirred for 30 min at 0 °C. To the mixture was introduced $NH_3$ gas bubble for 15 min at 0 °C. The resulting solution was stirred for 2 h at RT. The resulting solution was diluted with 100 mL of water. The resulting solution was extracted with 3x200 mL of DCM and the organic layers combined and dried over anhydrous sodium sulfate and concentrated under vacuum. The residue was eluted from a silica gel column with ethyl acetate/petroleum ether (36/64). This resulted in 1.4 g (36.5%) of the title compound as a yellow solid. MS-ESI: 343 (M+1)

**Scheme 44:**

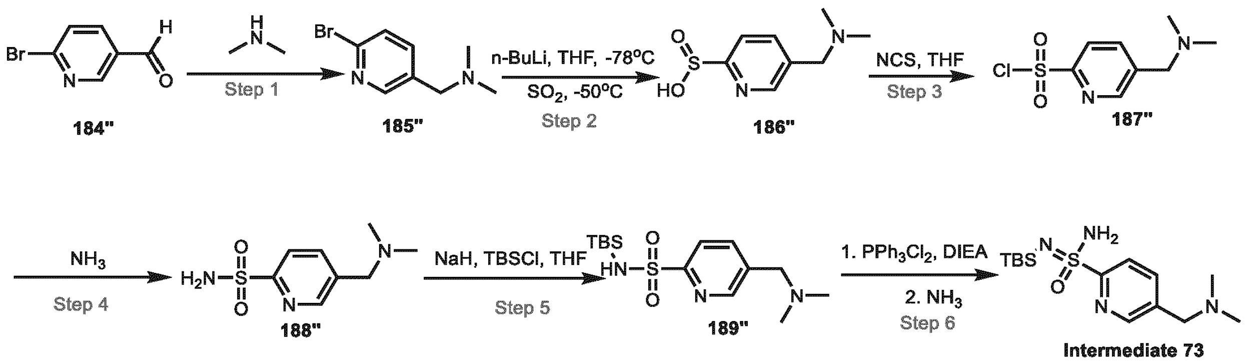


**Intermediate 73**

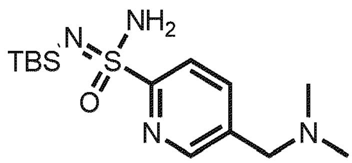

N'-(*tert*-butyldimethylsilyl)-5-((dimethylamino)methyl)pyridine-2-sulfonimidamide

**Step 1: (6-Bromopyridin-3-yl)-N,N-dimethylmethanamine**

Into a 500-mL 3-necked round-bottom flask purged and maintained with an inert atmosphere of n itrogen, were placed $Ti(OEt)_4$ (12.3 g, 53.8 mmol) and dimethylamine (4.85 g, 108 mmol) in Me OH (50 mL) at RT. To a stirred solutionwas added 6-bromopyridine-3-carbaldehyde (5.0 g, 26. 9 mmol) in MeOH (30 mL) dropwise at 0 °C. Then the reaction solution was stirred at RT for 3h. $NaBH_4$ (1.02 g, 26.9 mmol) was added to the mixture and the resulting solution was stirred over night at RT. The reaction was quenched by the addition of water/ice (30mL) at 0 °C. The resultin g mixture was concentrated under reduced pressure. Then the resulting mixture extracted with et hyl acetate (3 x 50mL) and the organic layers combined and dried over anhydrous sodium sulfate and concentrated under vacuum. The residue was eluted from a silica gel column with ethyl aceta te/petroleum ether (5:1) to afford the title compound (3.5 g, 60.5%) as a yellow oil. MS-ESI: 216 /218 (M+1).

**Steps 2-6** used similar procedures for converting compound **179"** to Intermediate **72** shown in **Scheme 43** to afford Intermediate **73** from compound **185.** MS-ESI: 329 (M+1).

**Table 12.** The Intermediates in the following Table were prepared using the similar procedures for converting compound **184"** to Intermediate **73** shown in Scheme 44 from appropriate starting materials.

| Intermediate # | Structure | IUPAC Name | Exact Mass $[M+H]^+$ |
|---|---|---|---|
| **Intermediate 74** | 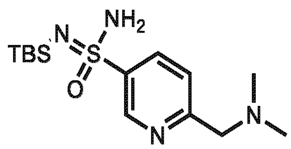 | N'-(*tert*-butyldimethylsilyl)-6-((dimethylamino)methyl)pyridine-3-sulfonimidamide | 329 |

**Scheme 45:**

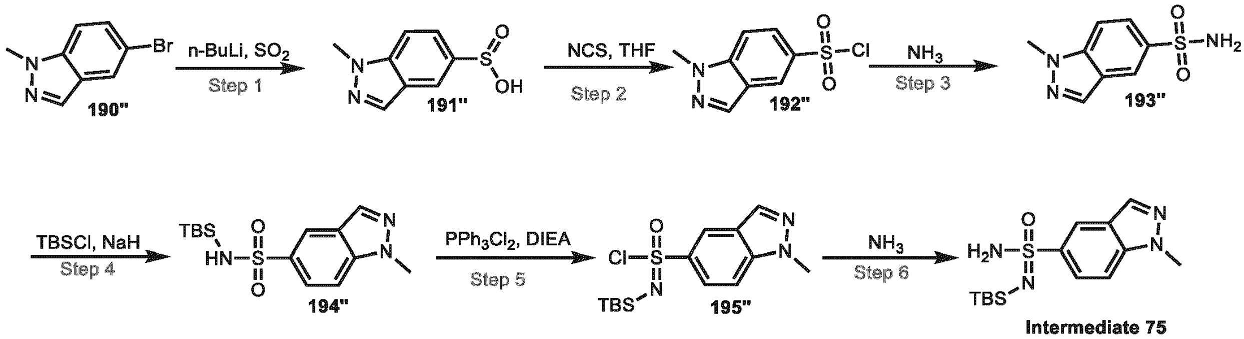


## Intermediate 75

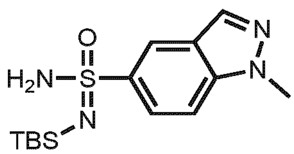

N'-(*tert*-bulyldimethylsilyl)-1-methyl-1H-indazole-5-sulfonimidamide

**Steps 1-6** used similar procedures for converting compound **179"** to Intermediate **72** shown in **Scheme 43** to afford Intermediate **75** from compound **190".** MS-ESI: 325 (M+1).

**Scheme 46:**

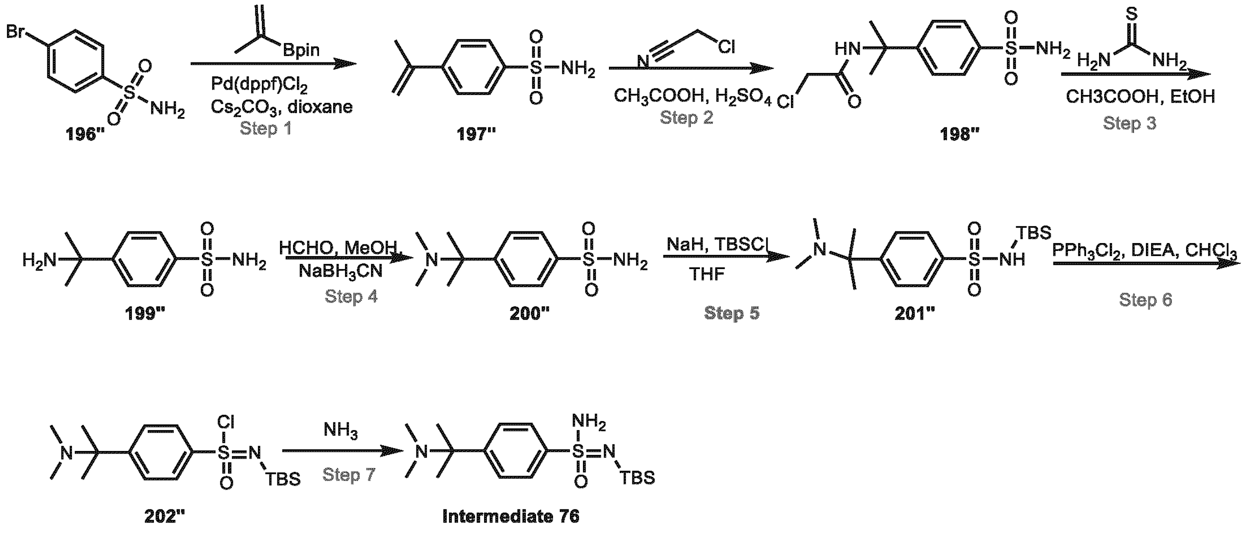


196" 197" 198" 199" 200" 201" 202" Intermediate 76

**Intermediate 76**

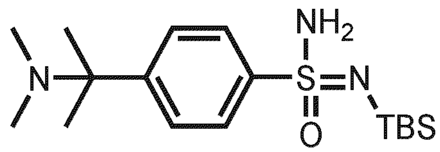

*N'*-(*tert*-butyldimethylsilyl)-4-(2-(dimethylamino)propan-2-yl)benzenesulfonimidamide

**Step 1: 4-(Prop-1-en-2-yl)benzenesulfonamide**

Into a 250-mL round-bottom flask purged and maintained with an inert atmosphere of nitrogen, was placed 4-bromobenzene-1-sulfonamide (5.0 g, 21.2 mmol) in dioxane (100 mL) and $H_2O$ (15 mL). To this stirred solution was added 4,4,5,5-tetramethyl-2-(prop-1-en-2-yl)-1,3,2-dioxaborolane (14.2 g, 84.7 mmol), Pd(dppf)$Cl_2$ (4.65 g, 6.35 mmol) and $Cs_2CO_3$ (13.8 g, 42.4 mmol). The resulting solution was stirred for 15 h at 100 °C. The resulting mixture was concentrated under reduced pressure. The residue was eluted from a silica gel column with ethyl acetate/petroleum ether (40/60). This resulted in 3.6 g (86.2%) of the title compound as a yellow solid. MS-ESI: 198 (M+1).

**Step 2: 2-Chloro-*N*-(2-(4-sulfamoylphenyl)propan-2-yl)acetamide**

Into a 1.0-L round-bottom flask, was placed 4-(prop-1-en-2-yl)benzene-1-sulfonamide (5.0 g, 25.4 mmol) in $H_2SO_4$ (50 mL) and AcOH (250 mL). To the stirred solution was added 2-chloroacetonitrile (38.3 g, 507 mmol). The resulting solution was stirred for 30 min at 0 °C. The resulting solution was allowed to react for an additional 15 h at RT. The pH value of the solution was adjusted to 7 with $Na_2CO_3$ (5.0 M). Then the resulting mixture was extracted with ethyl acetate (3 x 200mL) and the organic layers combined and dried over anhydrous sodium sulfate and concentrated under vacuum. The residue was eluted from a silica gel column with ethyl acetate/petroleum ether (2/3). This resulted in 4.2 g (57%) of the title compound as yellow oil. MS-ESI: 291 (M+1).

**Step 3: 4-(2-Aminopropan-2-yl)benzenesulfonamide**

Into a 250-mL round-bottom flask, was placed 2-chloro-N-[2-(4-sulfamoylphenyl)propan-2-yl]acetamide (4.2 g, 14.5 mmol) in $CH_3COOH$ (15 mL) and ethanol (75 mL). To this stirred solution was added thiourea (1.32 g, 17.3mmol). The resulting solution was stirred for 16 h at 85 °C. The resulting mixture was washed with 100 ml of $H_2O$ and extracted with 3x250 mL of ethyl acetate and the organic layers combined and dried over anhydrous sodium sulfate and concentrated under vacuum. This resulted in 2.3 g (54.3%) of the title compound as a yellow solid. MS-ESI: 215 (M+1).

**Step 4: 4-(2-(Dimethylamino)propan-2-yl)benzenesulfonamide**

Into a 250-mL round-bottom flask, was placed 4-(2-aminopropan-2-yl)benzene-1-sulfonamide (2.14 g, 9.99 mmol) in MeOH (50 mL). To this stirred solution was added HCHO (37%wt., 599 mg, 20 mmol) and $NaBH_3CN$ (1.86 g, 30 mmol). The resulting solution was stirred for 120 min at RT. The resulting mixture was diluted with 100 mL of water and extracted with 3x250 mL of ethyl acetate and the organic layers combined and dried over anhydrous sodium sulfate and concentrated under vacuum The residue was eluted from a silica gel column with ethyl acetate/petroleum ether (30/70). This resulted in 1.0 g (41.3%) of the title compound as a yellow solid. MS-ESI: 243 (M+1).

**Steps 5-7** used similar procedures for converting compound **148"** to intermediate **59** shown in **Scheme 36** to afford Intermediate **76** from compound **200.** MS-ESI: 356 (M+1).

Scheme 47:

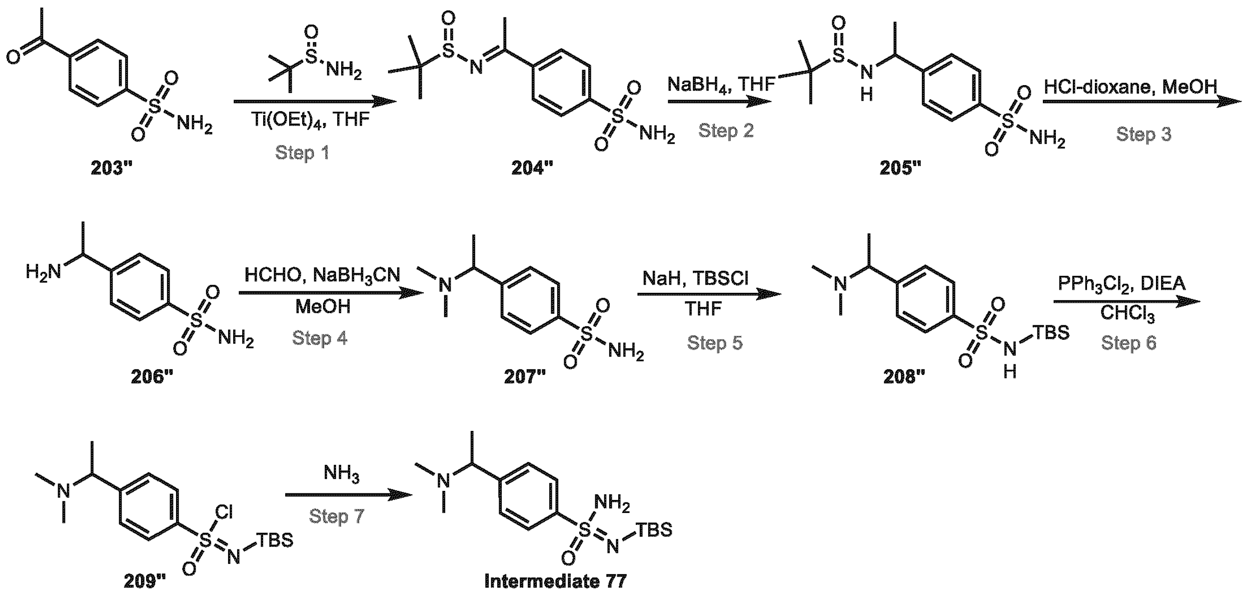


Intermediate 77

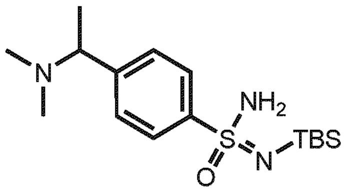

*N'*-(*tert*-butyldimethylsilyl)-4-(1-(dimethylamino)ethyl)benzenesulfonimidamide

**Step** 1: **(E)-4-(1-((*tert*-butylsulfinyl)imino)ethyl)benzenesulfonamide**

Into a 250-mL 3-necked round-bottom flask purged and maintained with an inert atmosphere of nitrogen was added 2-methylpropane-2-sulfinamide (3.04g, 25.1 mmol) in THF (50 mL). To this stirred solution was added $Ti(OEt)_4$ (11.5 g, 50.2 mmol) and 4-acetylbenzene-1-sulfonamide (5.0 g, 25.1 mmol) in portions at RT. The resulting mixture was stirred for overnight at 70 °C under nitrogen atmosphere. The reaction was quenched with Water (20 mL) at 0 °C. The resulting mixture was extracted with ethyl acetate (3 x 30 mL). The combined organic layers were dried over anhydrous $Na_2SO_4$. After filtration, the filtrate was concentrated under reduced pressure. The residue was purified by silica gel column chromatography, eluted with ethyl acetate/petroleum ether (1:1) to afford the title compound (5.0 g,75.8%) as a yellow solid. MS-ESI: 303 (M+1).

**Step 2: 4-(1-((*Tert*-butylsulfinyl)amino)ethyl)benzenesulfonamide**

Into a 500 mL round-bottom flask were added 4-[(1E)-1-[(2-methylpropane-2-sulfinyl)imino]ethyl]benzene -1-sulfonamide (4.65 g, 15.4 mmol) in THF (200 mL) at RT. To this stirred solution was added $NaBH_4$ (1.16 g, 30.8 mmol) in portions at 0 °C under nitrogen atmosphere. The resulting mixture was stirred for 4 h at RT under nitrogen atmosphere. The reaction was quenched by the addition of HCl (2M, 50 mL) at 0 °C. The resulting mixture was extracted with ethyl acetate (3 x 50mL). The combined organic layers were dried over anhydrous $Na_2SO_4$. After filtration, the filtrate was concentrated under reduced pressure to afford the title compound (4.5 g, 96.1%) as a white solid. MS-ESI: 305 (M+1).

**Step 3: 4-(1-Aminoethyl)benzenesulfonamide**

Into a 250 mL round-bottom flask were added 4-[1-[(2-methylpropane-2-sulfinyl)amino]ethyl]benzene-1 -sulfonamide (4.4 g, 14.5 mmol) and MeOH (50 mL) at room temperature. To this stirred solution was added HCl (gas) in 1,4-dioxane (8.0 mL, 26.3 mmol) in one portions at RT. The resulting mixture was stirred overnight at RT. The resulting mixture was concentrated under reduced pressure. The residue was purified by reverse flash chromatography with the following conditions (column, C18 silica gel; mobile phase, MeCN in water, 10% to 50% gradient in 10 min; detector, UV 254 nm.) to afford the title compound (2.6 g, 89.7%) as a white solid. MS-ESI: 201 (M+1).

**Step 4: 4-(1-(Dimethylamino)ethyl)benzenesulfonamide**

Into a 250 mL round-bottom flask was added 4-(1-aminoethyl)benzene-1-sulfonamide (2.0 g, 9.99 mmol) and MeOH (60 mL) at RT. To this stirred solution was added HCHO (37%wt., 1.61 g, 53.6 mmol) and $NaBH_3CN$ (1.25 g, 20 mmol) in portions at RT. The resulting mixture was stirred overnight at RT. The reaction solution was diluted with 100 mL of water and extracted with 3x100 mL of ethyl acetate and the organic layers combined and dried over anhydrous sodium sulfate and concentrated under vacuum. The residue was eluted from a silica gel column with ethyl acetate/petroleum ether. The residue was purified by silica gel column chromatography, eluted with ethyl acetate/petroleum ether (1:2) to afford the title compound (1.5 g, 65.8%) as a white solid. MS-ESI: 229 (M+1).

**Steps 5-7** used similar procedures for converting compound **148"** to intermediate **59** shown in **Scheme 36** to afford Intermediate **77** from compound **207".** MS-ESI: 342 (M+1).

**Scheme 48:**

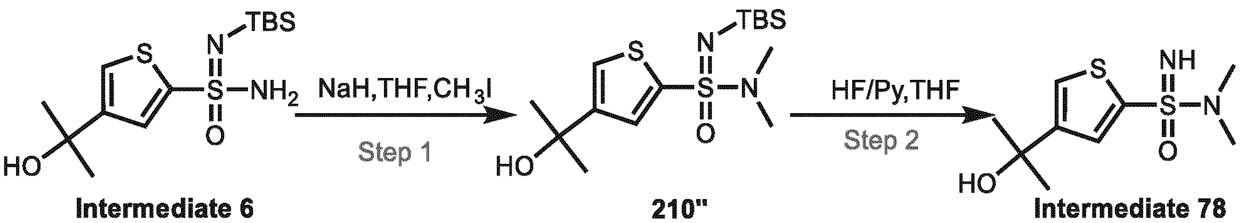


**Intermediate 6** **210"** **Intermediate 78**

**Intermediate 78**

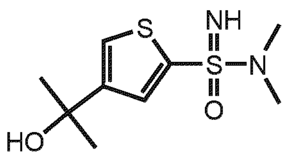

4-(2-Hydroxypropan-2-yl)-*N,N*-dimethylthiophene-2-sulfonimidamide

**Step 1: *N'*-(*tert*-butyldimethylsilyl)-4-(2-hydroxypropan-2-yl)-*N,N*-dimethylthiophene-2-sulfonimidamide**

Into a 50-mL 3-necked round-bottom flask, was placed N-(tert-butyldimethylsilyl)-4-(2-hydroxypropan-2-yl) thiophene-2-sulfonoimidamide (300 mg, 0.90 mmol) in THF (3.0 mL). To the solution were added NaH (60%wt. oil dispersion, 53.8 mg, 1.35 mmol) at -10 °C in ethanol/ice bath. To the solution were added iodomethane (0.50 mL) dropwise with stirring at 0 °C in 30 min. The resulting solution was stirred for 30 min at RT. The reaction was then quenched by the addition of $NH_4Cl$(aq.). The resulting solution was extracted with 3x20 mL of ethyl acetate and the organic layers combined, dried over anhydrous sodium sulfate and concentrated under vacuum. The residue was eluted from a silica gel column with ethyl acetate/petroleum ether (1:5). This resulted in 252 mg (77.5%) of the title compound as a white solid. MS-ESI: 363 (M+1).

**Step 2: *N'*-(*tert*-butyldimethylsilyl)-4-(2-hydroxypropan-2-yl)-*N,N*-dimethylthiophene-2-sulfonimidamide**

Into a 50-mL round-bottom flask, was placed N-(tert-butyldimethylsilyl)-4-(2-hydroxypropan-2-yl)-*N,N* -dimethylthiophene-2-sulfonoimidamide (200 mg, 0.55 mmol) in THF (10 mL). To the solution was added HF/Py (70%wt., 0.10 mL) dropwise with stirring at RT. The resulting solution was stirred for 60 min at RT. The resulting mixture was concentrated under vacuum. The resulting solution was extracted with ethyl acetate (3x10mL), the organic layers combined and dried over anhydrous sodium sulfate. The residue was eluted from a silica gel column with ethyl acetate. This resulted in 127 mg (92.7%) of the title compound as a white solid. MS-ESI: 249 (M+1).

**Scheme 49:**

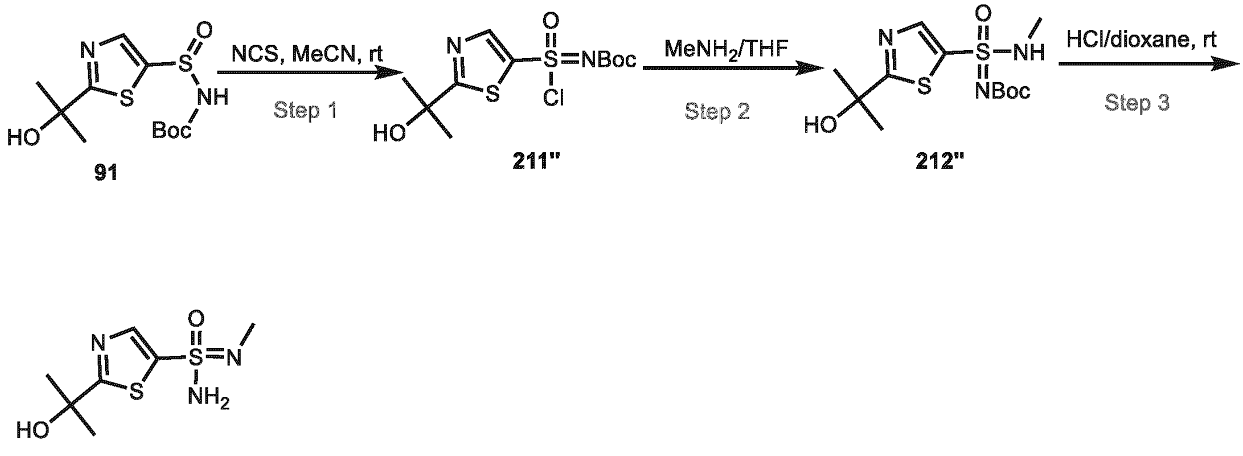


91

211"

212"

Intermediate 79

## Intermediate 79

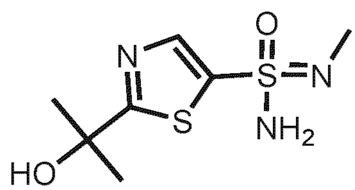

2-(2-Hydroxypropan-2-yl)-*N*-methylthiazole-5-sulfonimidamide

**Step 1: Tert-butyl (chloro(2-(2-hydroxypropan-2-yl)thiazol-5-yl)(oxo)-$\lambda^6$-sulfaneylidene)carbamate**

Into a 1.0-L round-bottom flask, was placed tert-butyl N-[[2-(2-hydroxypropan-2-yl)-1,3-thiazol-5-yl] sulfinyl]carbamate (100 g, 326 mmol) in ACN (500 mL). To the stirred solution was added NCS (65.4 g, 490 mmol). The resulting solution was stirred for 2 h at RT. The resulted solution was concentrated. This resulted in 120 g (crude) of the title compound as yellow oil. MS-ESI: 341/343 (M+1).

**Step 2: *Tert*-butyl((2-(2-hydroxypropan-2-yl)thiazol-5-yl)(methylamino)(oxo)-$\lambda^6$-sulfaneylidene) carbamate**

Into a 250-mL round-bottom flask, was placed tert-butyl (chloro(2-(2-hydroxypropan-2-yl)thiazol-5-yl)(oxo)-$\lambda^6$-sulfaneylidene)carbamate (10 g, 29.3 mmol) in THF (100 mL). To the stirred solution was added $CH_3NH_2$ (1.82 g, 58.6 mmol). The resulting solution was stirred for 2 h at RT. The resulted solution was concentrated. The residue was eluted from a silica gel column with ethyl acetate/ petroleum ether (1:1). This resulted in 6.1 g (62%) of the title compound as a yellow solid. MS-ESI: 336 (M+1).

**Step 3: 2-(2-Hydroxypropan-2-yl)-*N'*-methylthiazole-5-sulfonimidamide**

Into a 100-mL round-bottom flask, was placed *tert*-butyl((2-(2-hydroxypropan-2-yl)thiazol-5-yl)(methylami-

no)(oxo)-λ6-sulfaneylidene) carbamate (3.0 g, 8.94 mmol) in HCl (gas) in 1,4-dioxane (8 mL, 26.3 mmol) in one portion at RT. The resulting solution was stirred for 60 min at RT. The resulting mixture was concentrated under vacuum. This resulted in 2.10 g (crude) of the title compound as a yellow solid. MS-ESI: 236 (M+1).

**Scheme 50A:**

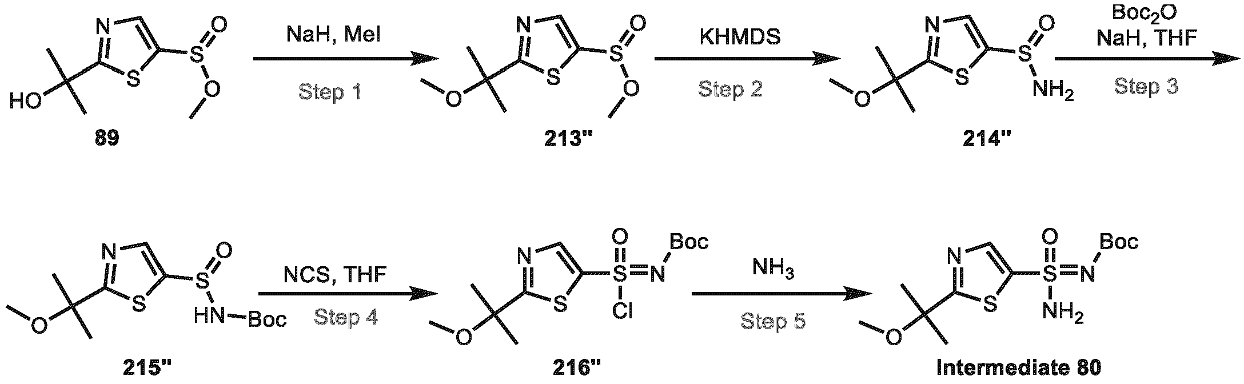


89, 213'', 214'', 215'', 216'', Intermediate 80

## Intermediate 80

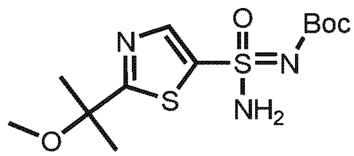

*Tert*-butyl (amino(2-(2-methoxypropan-2-yl)thiazol-5-yl)(oxo)-λ6-sulfaneylidene)carbamate

**Step 1: Methyl 2-(2-methoxypropan-2-yl)thiazole-5-sulfinate**

Into a 1-L round-bottom flask, was placed a solution of methyl 2-(2-hydroxypropan-2-yl)-1,3-thiazole-5-sulfinate (40 g, 181 mmol) in THF (500 mL). To this stirred solution was added NaH (60% wt. oil dispersion, 7.95 g, 199 mmol) in three portions at 0 °C in an ice/ethanol bath. To this reaction solution was added MeI (51.3 g, 362 mmol) dropwise with stirring at 0 °C in an ice/ethanol bath. The resulting solution was stirred for 3 h at RT. The reaction was then quenched by the addition of water (50 mL ) at 0 °C. The resulting solution was extracted with 3x300 mL of ethyl acetate and the organic layers combined and dried over anhydrous sodium sulfate and concentrated under vacuum. This resulted in 32 g (75.3%) of the title compound as a white solid. MS-ESI: 236 (M+1).

**Step 2: 2-(2-Methoxypropan-2-yl)thiazole-5-sulfinamide**

Into a 1-L 3-necked round-bottom flask purged and maintained with an inert atmosphere of nitrogen, was placed a solution of methyl 2-(2-methoxypropan-2-yl)-1,3-thiazole-5-sulfinate (20 g, 85 mmol) in THF (500 mL). This was followed by the addition of KHMDS (500 mL, 1.0 mole, 2 M) dropwise with stirring at -78 °C in a liquid nitrogen/ethanol bath. The resulting solution was stirred for 3 h at -78 °C in a liquid nitrogen/ethanol bath. The reaction was quenched by the addition of water (50 mL). The resulting solution was extracted with 3x300 mL of ethyl acetate dried over anhydrous sodium sulfate and concentrated under vacuum. The residue was eluted from a silica gel column with ethyl acetate/petroleum ether (1:3). This resulted in 14 g (74.8%) of the title compound as a white solid. MS-ESI: 221.0 (M+1).

**Step 3: *Tert*-butyl ((2-(2-methoxypropan-2-yl)thiazol-5-yl)sulfinyl)carbamate**

Into a 500-mL round-bottom flask purged and maintained with an inert atmosphere of nitrogen, was placed a solution of 2-(2-methoxypropan-2-yl)-1,3-thiazole-5-sulfinamide (10 g, 45.4 mmol) in THF (250 mL). To this stirred solution was added NaH (60%wt. oil dispersion, 3.63 g, 90.8 mmol) in three times at 0 °C in an ice/ethanol bath. To this solution was added $Boc_2O$ (9.91 g, 45.4 mmol) in portions at 0 °C in an ice/ethanol bath. The resulting solution was stirred for 3 h at RT. The reaction was then quenched by the addition of water (50 mL). The resulting solution was extracted with 3x300 mL of ethyl acetate concentrated under vacuum. This resulted in 12 g (82.5%) of the title compound as a white solid. MS-ESI: 321.1 (M+1).

**Step 4: *Tert-butyl* (chloro(2-(2-methoxypropan-2-yl)thiazol-5-yl)(oxo)-$\lambda^6$-sulfaneylidene)carbamate**

Into a 500-mL round-bottom flask purged and maintained with an inert atmosphere of nitrogen, was placed a solution of tert-butyl N-[[2-(2-methoxypropan-2-yl)-1,3-thiazol-5-yl]sulfinyl]carbamate (11 g, 34.3 mmol) in THF (200 mL). NCS (13.8 g, 103 mmol) was added to the reaction solution in one portion at RT. The resulting solution was stirred for 3 h at RT. This reaction solution was used to the next step directly without further purification.

**Step 5: *Tert*-butyl (amino(2-(2-methoxypropan-2-yl)thiazol-5-yl)(oxo)-$\lambda^6$-sulfaneylidene)carbamate**

Into a 500-mL round-bottom flask purged and maintained with an inert atmosphere of nitrogen, was placed a solution of tert-butyl *N*-[[2-(2-methoxypropan-2-yl)-1,3-thiazol-5-yl]sulfinyl]carbamate (9.0 g, 28.9mmol) in THF (200 mL). To the mixture was added introduced $NH_3$ gas bubble for 15 min at 0 °C. The resulting solution was stirred for 1 h at RT. The resulting mixture was concentrated. The residue was eluted from a silica gel column with ethyl acetate/petroleum ether (1:1). This resulted in 7g (72.3%) of the title compound as a white solid. MS-ESI: 336.1 (M+1).

**Scheme 51:**

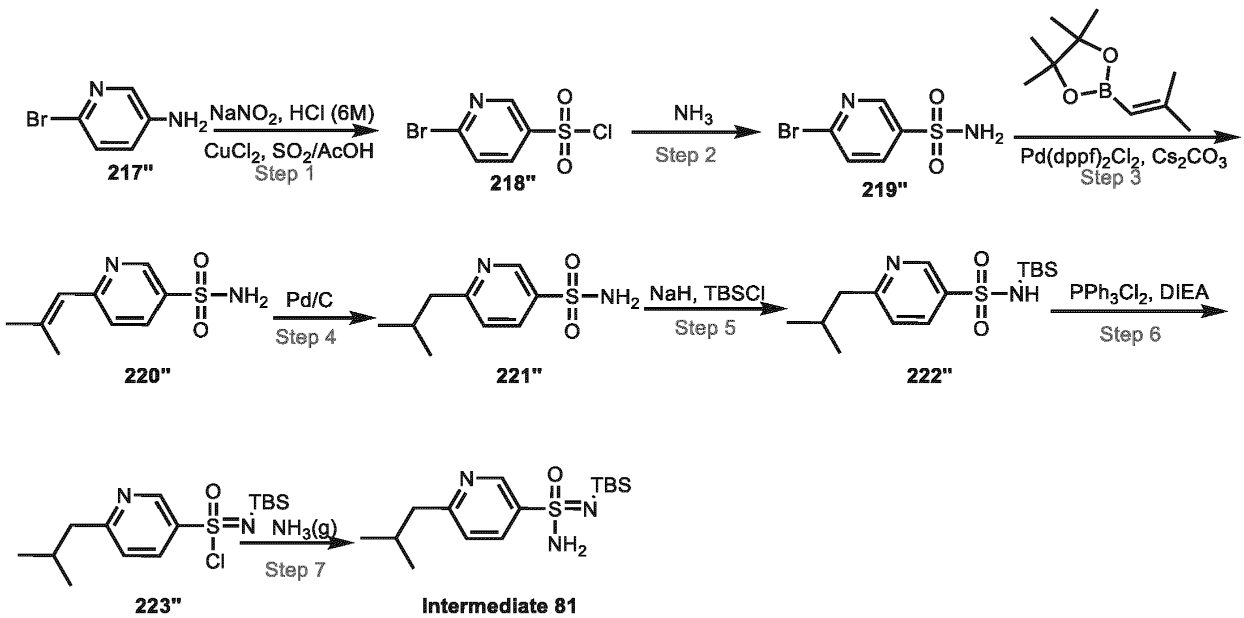


**Intermediate 81**

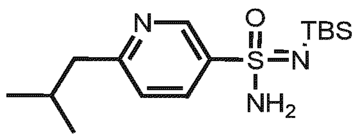

*N'*-(*tert*-butyldimethylsilyl)-6-isobutylpyridine-3-sulfonimidamide

**Steps 1-2** used similar procedures for converting compound **27** to Intermediate **29** shown in **Scheme 9** to afford compound **219"** from compound **217"**. MS-ESI: 238 (M+1).

**Step 3: 6-(2-Methylprop-1-enyl)pyridine-3-sulfonamide**

Into a 500 mL round-bottom flash were added 6-bromopyridine-3-sulfonamide (5.5 g, 23.2 mmol) and dioxane (150 mL) and water (15 mL) at RT. To this solution was added Pd(dppf)$Cl_2$ (1.7 g, 2.32 mmol), Cs2CO3 (15.1 g, 46.4 mmol) and 4,4,5,5-tetramethyl-2-(2-methylprop-1-en-1-yl)-1,3,2-dioxaborolane (8.45 g, 46.4mmol) in one portion at RT under nitrogen atmosphere. The resulting mixture was stirred overnight at 100 °C under nitrogen atmosphere. The resulting mixture was concentrated under reduced pressure. The residue was purified by silica gel column chromatography with ethyl acetate/petroleum ether (1:1) to afford title compound (4.0 g, 81.2%) as a light yellow oil. MS-ESI: 213

(M+1).

**Step 4: 6-Isobutylpyridine-3-sulfonamide**

Into a 250mL 3-necked round-bottom flask was added 6-(2-methylprop-1-en-1-yl)pyridine-3-sulfonamide (4 g, 18.8 mmol) and MeOH (100 mL) at RT under nitrogen atmosphere. To this stirred solution was added Pd/C (wet 10% wt., 900 mg). The flask was evacuated and filled three times with hydrogen. The resulting mixture was stirred overnight at RT under hydrogen atmosphere. The resulting mixture was filtered; the filter cake was washed with MeOH (3x20 mL). The filtrate was concentrated under reduced pressure. The crude product of the title compound (3.8 g) was used to the next step directly without further purification. MS-ESI: 215 (M+1). **Steps 5-7** used similar procedures for converting compound **148"** to intermediate **59** shown in Scheme 36 to afford intermediate **81** from compound **221".** MS-ESI: 328 (M+1).

**Table 13.** The Intermediates in the following Table were prepared using the similar procedures for converting compound **217"** to Intermediate **81** shown in Scheme 51 from appropriate starting materials.

| Intermediate # | Structure | IUPAC Name | Exact Mass $[M+H]^+$ |
|---|---|---|---|
| Intermediate 82 | 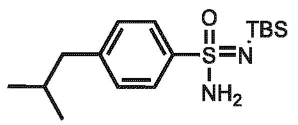 | *N'*-(*tert*-butyldimethylsilyl)-4-isobutylbenzenesulfonimidamide | 327 |

**Scheme 52:**

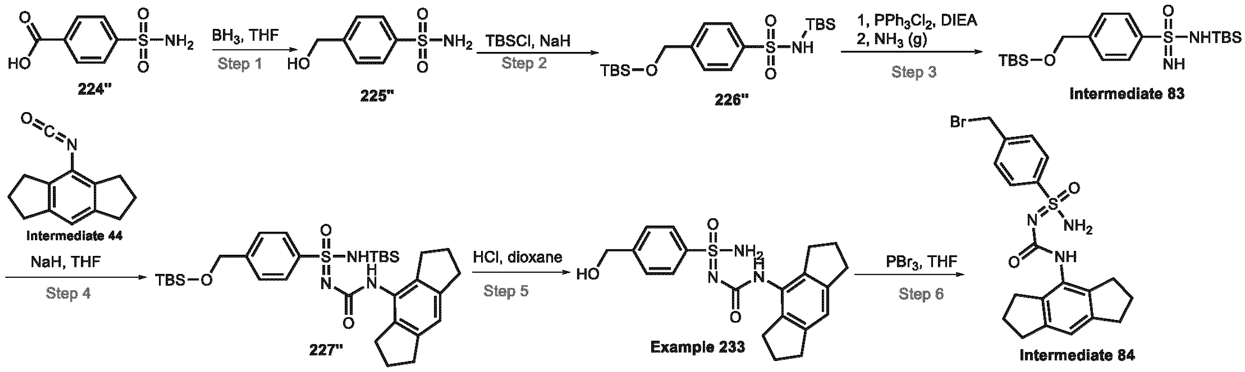


## Intermediate 83

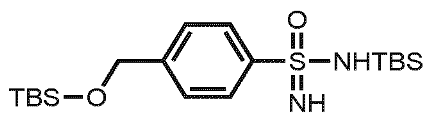

*N*-(*tert*-butyldimethylsilyl)-4-((tert-butyldimethylsilyloxy)methyl)benzenesulfonimidamide

**Example 233 (Compound 342)**

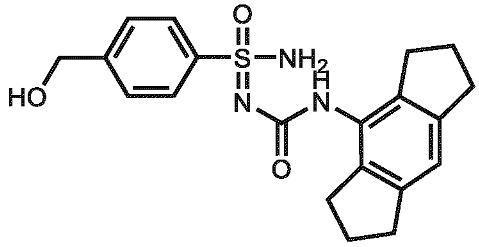

*N'*-((1,2,3,5,6,7-hexahydro-s-indacen-4-yl)carbamoyl)-4-(hydroxymethyl)benzenesulfonimidamide

**Step 1: 4-(Hydroxymethyl)benzenesulfonamide**

Into a 100-mL round-bottom flask, was placed 4-sulfamoylbenzoic acid (1.0 g, 4.97 mmol) in THF (15 mL). This was followed by the addition of $BH_3$-THF (14.3 mL, 149 mmol) dropwise with stirring at 0 °C in an ice/ethanol bath. The resulting solution was stirred for 12 h at RT. The reaction was then quenched by the addition of HCl (50 mL, 2 M) dropwise in an ice bath and stirred for 1h at RT. The mixture was extracted with 8x50 mL of ethyl acetate. The organic layers were combined and concentrated. This resulted in 800 mg (86%) of the title compound as a yellow solid. MS-ESI: 188 (M+1).

**Steps 2-3** used similar procedures for converting compound **148"** to Intermediate **59** shown in Scheme 36 to afford Intermediate **83** from compound **225".** MS-ESI: 415 (M+1).Steps 4-5 used similar procedures for converting compound **166"** to Intermediate **67** shown in **Scheme 40A** to afford compound **Example 233** from Intermediate **83.** MS-ESI: 386 (M+1).

## Intermediate 84

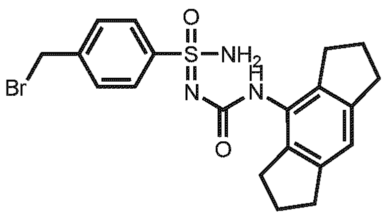

4-(Bromomethyl)-*N'*-(1,2,3,5,6,7-hexahydro-*s*-indacen-4-ylcarbamoyl)benzenesulfonimidamide

**Step 6: 4-(Bromomethyl)-N'-(1,2,3,5,6,7-hexahydro-s-indacen-4-ylcarbamoyl)benzenesulfonimidamide**

Into a 250-mL round-bottom flask purged and maintained with an inert atmosphere of nitrogen, was placed 1-[amino[4-(hydroxymethyl)phenyl]oxo-λ6-sulfanylidene]-3-(1,2,3,5,6,7-hexahydro-s-indacen-4-yl)urea (1.0 g, 2.59 mmol) in THF (50 mL). To the stirred solution was added $PBr_3$ (702 mg, 2.59 mmol) in portions. The resulting solution was stirred for 3 h at RT. The solids were collected by filtration. This resulted in 500 mg (43%) of the title compound as a white solid. MS-ESI: 449/411 (M+1).

## Scheme 53:

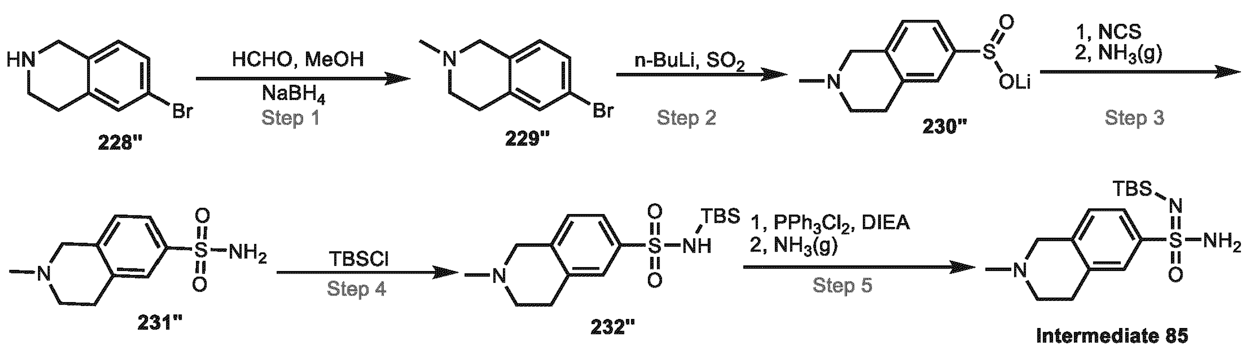

## Intermediate 85

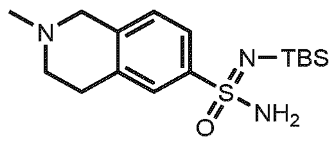

<u>*N'*-(*tert*-bulyldimethylsilyl)-2-methyl-1,2,3,4-tetrahydroisoquinoline-6-sulfonimidamide</u>

**Step 1: 6-Bromo-2-methyl-1,2,3,4-tetrahydroisoquinoline**

Into a 250-mL round-bottom flask, was placed 6-bromo-1,2,3,4-tetrahydroisoquinoline (6.0 g, 28.3 mmol) in MeOH (100 mL) under $N_2$. To the stirred solution was added HCHO (1.02 g, 34 mmol) in portions at RT. The resulting solution was stirred for 4 h, then $NaBH_3CN$ (3.56 g, 56.6 mmol) was added in portions at RT. The resulting solution was stirred overnight at RT. The reaction was then quenched by the addition of water (100 mL) and extracted with 3x150 mL ethyl acetate. The organic layers were combined and dried over anhydrous sodium sulfate and concentrated under vacuum. The crude product was eluted from a silica gel column with acetate/petroleum ether (1:1). This resulted in 5 g (78.2%) of the title compound as a white solid. MS-ESI: 226/228 (M+1).

**teps 2-5** used similar procedures for converting compound **185"** to Intermediate **173"** shown in **Scheme 44** to afford Intermediate **85** from compound **229.** MS-ESI: 238 (M+1).

**Scheme 54:**

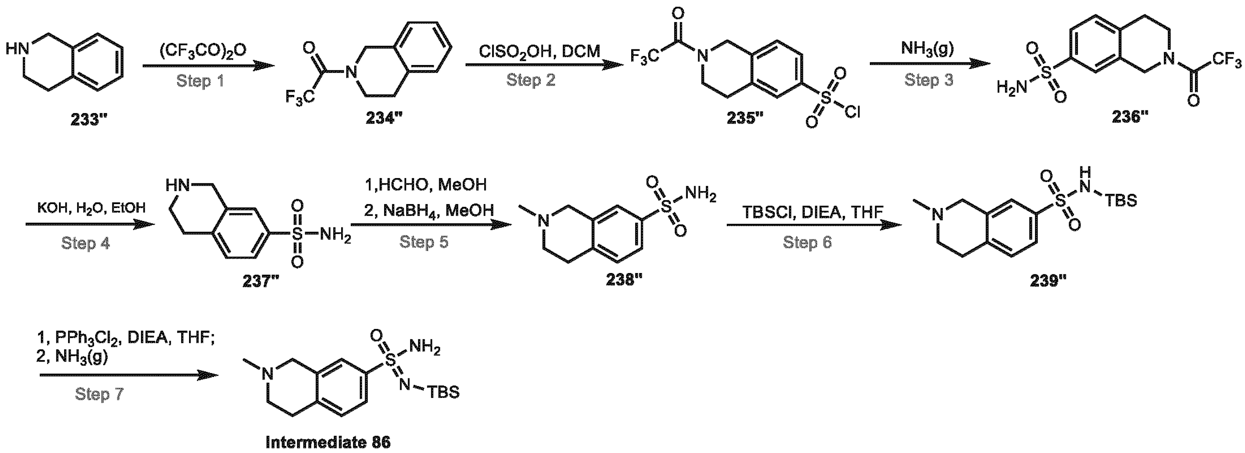


## Intermediate 86

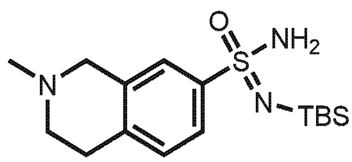

<u>*N'*-(*tert*-butyldimethylsilyl)-2-methyl-1,2,3,4-tetrahydroisoquinoline-7-sulfonimidamide</u>

**Step 1: 1-(3,4-Dihydroisoquinolin-2(1H)-yl)-2,2,2-trifluoroethanone**

Into a 250-mL round-bottom flask purged and maintained with an inert atmosphere of nitrogen, was placed 1,2,3,4-tetrahydroisoquinoline (8.0 g, 60.1 mmol) and 2,2,2-trifluoroacetic anhydride (25.2 g, 120mmol). The resulting solution was stirred for 12 h at RT. The reaction was then quenched by the addition of 100 mL of water/ice. The resulting solution was extracted with 3x100 mL of ethyl acetate and the organic layers were combined and dried over anhydrous

sodium sulfate and concentrated under vacuum. This resulted in 10 g (72.6%) of the title compound as a yellow solid. MS-ESI: 230 (M+1).

**Steps 2-3** used similar procedures for converting compound **158"** to Intermediate **61** shown in **Scheme 38** to afford compound **236"** from compound **234".** MS-ESI: 309 (M+1).

**Step 4: 1,2,3,4-Tetrahydroisoquinoline-7-sulfonamide**

Into a 100-mL round-bottom flask, was placed 2-(2,2,2-trifluoroacetyl)-1,2,3,4-tetrahydroisoquinoline -7-sulfonamide (8.0 g, 26 mmol) in ethanol (12 mL) and $H_2O$ (60 mL). To the stirred solution was added KOH (7.28 g, 123 mmol) in one portion at RT. The resulting solution was stirred for 12 h at RT. The resulting mixture was concentrated. The crude product was applied onto a silica gel column with DCM/MeOH (10:1). This resulted in 5.0 g (90.8%) of the title compound as a light yellow solid.

**Step 5** used similar procedures for converting compound **228"** to compound **229"** shown in **Scheme 53** to afford compound **238"** from compound **237".** MS-ESI: 227 (M+1).

**Steps 6-7** used similar procedures for converting compound **148"** to intermediate **59** shown in **Scheme 36** to afford intermediate **86** from compound **238".** MS-ESI: 340 (M+1).

Scheme 55:

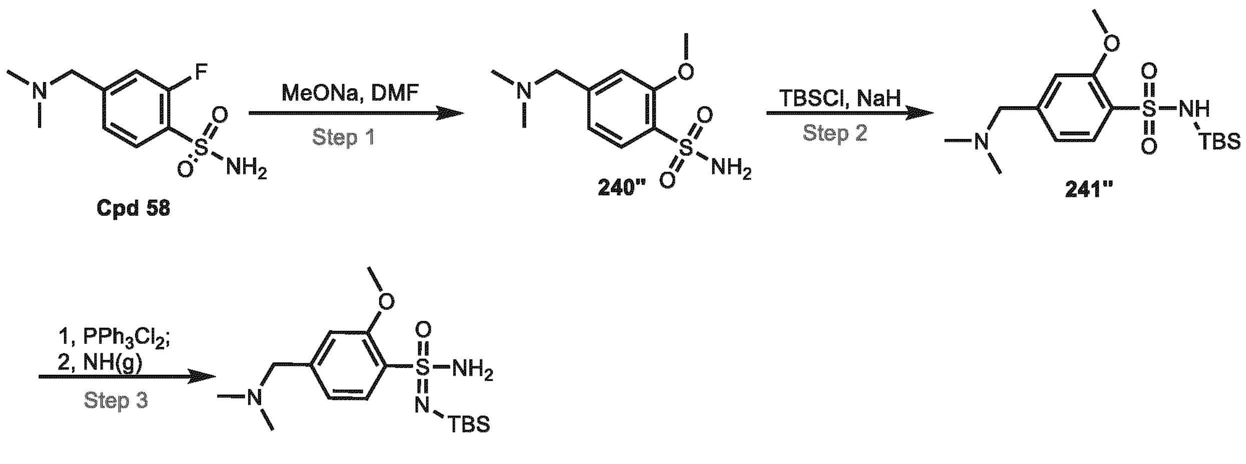


Cpd 58 240" 241" Intermediate 87

Intermediate 87

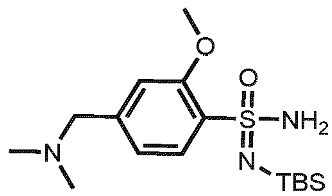

*N'*-(*tert*-butyldimethylsilyl)-2-methyl-1,2,3,4-tetrahydroisoquinoline-7-sulfonimidamide

**Step 1: 4-((Dimethylamino)methyl)-2-methoxybenzenesulfonamide**

Into a 50-mL round-bottom flask, was placed 4-[(dimethylamino)methyl]-2-fluorobenzene-1-sulfonamide (1 g, 4.31 mmol) and DMF (10 mL, 0.14 mmol). Then to the above was added sodium methoxide (2.16 g, 40 mmol). The resulting solution was stirred for 12 h at RT. The reaction was then quenched by the addition of 5.0 mL of water. The residue was eluted from a C18 column with ACN:$H_2O$ (3:7). This resulted in 800 mg (76.1%) of the title compound as a yellow solid. MS-ESI: 245 (M+1).

**Steps 2-3** used similar procedures for converting compound **148"** to intermediate **59** shown in **Scheme 36** to afford intermediate **87** from compound **240".** MS-ESI: 358 (M+1).

## Scheme 56:

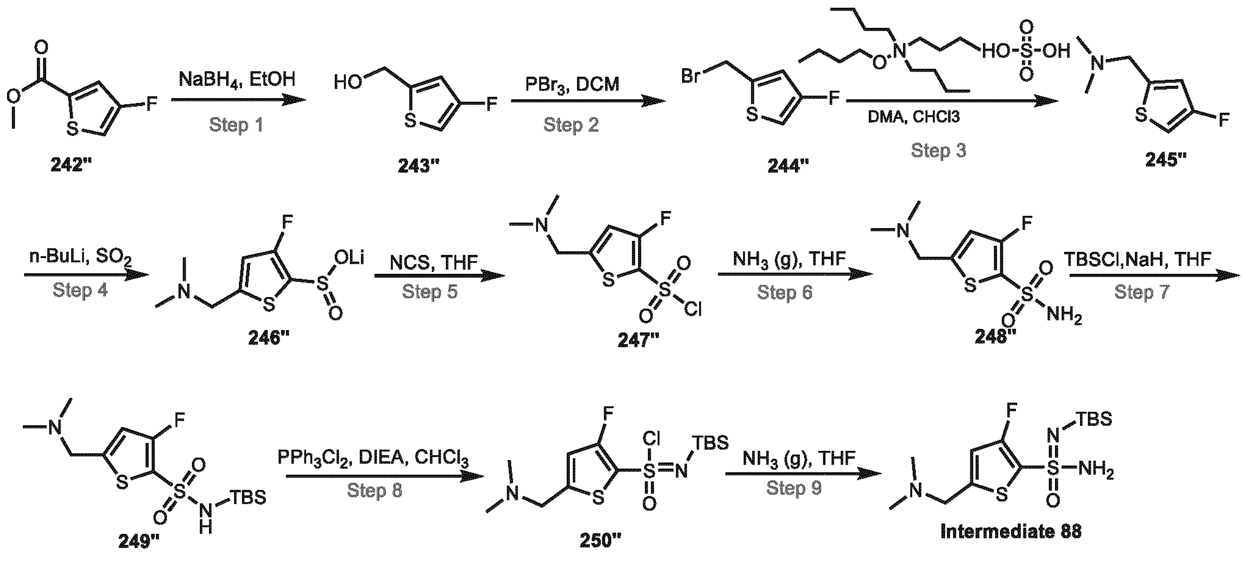


242'' 243'' 244'' 245'' 246'' 247'' 248'' 249'' 250'' Intermediate 88

## Intermediate 88

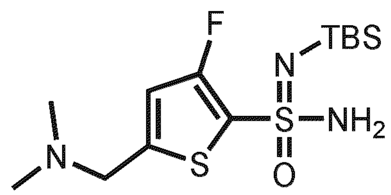

*N'*-(*tert*-bulyldimethylsilyl)-5-((dimethylamino)methyl)-3-fluorothiophene-2-sulfonimidamide

### Step 1: (4-Fluorothiophen-2-yl)methanol

Into a 1000-mL round-bottom flask, was placed methyl 4-fluorothiophene-2-carboxylate (10 g, 62.4 mmol) in ethanol (300 mL). Then to the above solution was added $NaBH_4$ (4.62 g, 125 mmol) in portions at 0 °C in an ice/ethanol bath. The resulting solution was stirred for 30 min at 0 °C and then the reaction solution was allowed to react for an additional 16 h at RT. The reaction was then quenched by the addition of 50 mL of water. Then the mixture was concentrated and extracted with 3x100mL of ethyl acetate and the organic layers were combined and dried over anhydrous sodium sulfate and concentrated under vacuum. This resulted in 6.4 g (77.6%) of the title compound as white oil. MS-ESI: 133 (M+1)

### Step 2: 2-(Bromomethyl)-4-fluorothiophene

Into a 250-mL round-bottom flask, was placed (4-fluorothiophen-2-yl)methanol (8.5 g, 64.3 mmol) in DCM (70 mL). To the stirred solution was added $PBr_3$ (19.2 g, 70.8 mmol) dropwise at 0 °C in an ice/ethanol bath. The resulting solution was stirred for 30 min at 0 °C. The resulting solution was allowed to react for an additional 12 h at RT. The reaction was then quenched by the addition of 50 mL of water. Then the mixture was concentrated and extracted with 3x100mL of ethyl acetate and the organic layers were combined and dried over anhydrous sodium sulfate and concentrated under vacuum. The residue was eluted from a silica gel column with ethyl acetate/petroleum ether (15/85). This resulted in 7.0 g (55.8%) of the title compound as yellow oil. MS-ESI: 194/196 (M+1).

### Step 3: 1-(4-Fluorothiophen-2-yl)-N,N-dimethylmethanamine

Into a 250-mL round-bottom flask, was placed 2-(bromomethyl)-4-fluorothiophene (7.4 g, 37.9 mmol) in $CHCl_3$ (50 mL). To the above solution was added butoxytributyl-l4-azane sulfate (6.76 g, 19 mmol) and DMA (37 mL, 425 mmol) with stirring at RT. The resulting solution was stirred for 2h at 60 °C. The reaction was then quenched by the addition of 50 mL of water. Then the mixture was concentrated and extracted with 3x100mL of ethyl acetate and the organic

layers were combined and dried over anhydrous sodium sulfate and concentrated under vacuum. The residue was eluted from a silica gel column with ethyl acetate/petroleum ether (17/83). This resulted in 6.0 g (99.5%) of the title compound as a yellow solid. MS-ESI: 160 (M+1).

**Step 4: Lithium 5-((dimethylamino)methyl)-3-fluorothiophene-2-sulfinate**

Into a 500-mL 3-necked round-bottom flask purged with and maintained under nitrogen, was placed a solution of [(4-fluorothiophen-2-yl)methyl]dimethylamine (6.2 g, 38.9 mmol) in THF (60 mL). This was followed by the addition of n-BuLi/THF (18.7 mL, 2.5 M) dropwise with stirring at -78 °C in a liquid nitrogen/ethanol bath. The resulting solution was stirred for 30 min at -78 °C. To the above $SO_2$(g) was introduced into the reaction solution at -78 °C. The resulting solution was allowed to react for an additional 2 h at RT. The resulting mixture was concentrated. This resulted in 10 g (crude) of the title compound as a yellow solid. MS-ESI: 222 (M-1).

**Step 5: 5-((Dimethylamino)methyl)-3-fluorothiophene-2-sulfonyl chloride**

Into a 500-mL round-bottom flask, was placed a solution of 5-[(dimethylamino)methyl]-3-fluorothiophene -2-sulfinic acid (10 g, 44.8 mmol) in THF (100 mL). To the above solution was added NCS (7.18 g, 53.8 mmol). The resulting solution was stirred for 30 min at 0 °C and then allowed to react for an additional 2h at RT. This reaction was used for next step without purification.

**Step 6: 5-((Dimethylamino)methyl)-3-fluorothiophene-2-sulfonamide**

Into a 500-mL round-bottom flask, was placed a solution of 5-[(dimethylamino)methyl]-3-fluorothiophene -2-sulfonyl chloride (10 g, 38.8 mmol) in THF (100 mL). To the above $NH_3$ (g) was introduced at RT. The resulting solution was stirred for 30 min at RT. The resulting mixture was concentrated. The residue was eluted from a silica gel column with ethyl acetate/petroleum ether (60/40). This resulted in 2.1 g (22.7%) of the title compound as yellow oil. MS-ESI: 239 (M+1).

**Step 7: *N*-(*tert*-butyldimethylsilyl)-5-((dimethylamino)methyl)-3-fluorothiophene-2-sulfonamide**

Into a 100-mL round-bottom flask, was placed a solution of 5-[(dimethylamino)methyl]-3-fluorothiophene -2-sulfonamide (1.8 g, 7.55 mmol) in THF (30 mL) under $N_2$. To the above solution was added NaH (60%wt. oil dispersion, 640mg, 15 mmol) with stirring at 0 °C. The resulting solution was stirred for 5 min at 0 °C. This was followed by the addition of TBSCl (1.37 g, 9.09 mmol) at 0 °C. The resulting solution was allowed to react for an additional 15 h at RT. The reaction was then quenched by the addition of 20 mL of water. Then the mixture was concentrated and extracted with 3x100mL of ethyl acetate and the organic layers were combined and dried over anhydrous sodium sulfate and concentrated under vacuum. The residue was eluted from a silica gel column with ethyl acetate/petroleum ether (1:1). This resulted in 2.0 g (75.2%) of the title compound as yellow oil. MS-ESI: 353 (M+1).

**Step 8: *N*-(*tert*-butyldimethylsilyl)-5-((dimethylamino)methyl)-3-fluorothiophene-2-sulfonimidoyl chloride**

Into a 500-mL round-bottom flask purged and maintained with an inert atmosphere of nitrogen was placed a solution of $PPh_3Cl_2$ (29.5 g, 88.7 mmol) in $CHCl_3$ (50 mL). To the above solution was added DIEA (17.2 g, 133 mmol) dropwise in an ice/water bath. The solution was stirred at RT for 20 minutes. This was followed by the addition of N-(tert-butyldimethylsilyl)-5-[(dimethylamino)methyl]-3-fluorothiophene- 2-sulfonamide (15.7 g, 44.4 mmol) in $CHCl_3$ (30 mL) at 0 °C. The resulting solution was allowed to react for an additional 30 min at 0 °C. Then the reaction solution was used for next step without purification.

**Step 9: *N'*-(*tert*-butyldimethylsilyl)-5-((dimethylamino)methyl)-3-fluorothiophene-2-sulfonimidamide**

Into a 500-mL round-bottom flask purged and maintained with an inert atmosphere of nitrogen, was placed [(tert-butyldimethylsilyl)imino](chloro)[5-[(dimethylamino)methyl]-3-fluorothiophen-2-yl]-$\lambda^6$-sulfanone (16.5 g, 44.4 mmol) in $CHCl_3$ (80 mL). To the above $NH_3$(g) was introduced at 0 °C for 15min. The resulting solution was stirred for 15 min at 0 °C and then allowed to react for an additional 15 h at RT. The resulting mixture was concentrated. The residue was eluted from a silica gel column with ethyl acetate/ petroleum ether (60/40). This resulted in 5.8 g (37.2 %) of the title compound as a yellow solid. MS-ESI: 352(M+1).

**Scheme 68:**

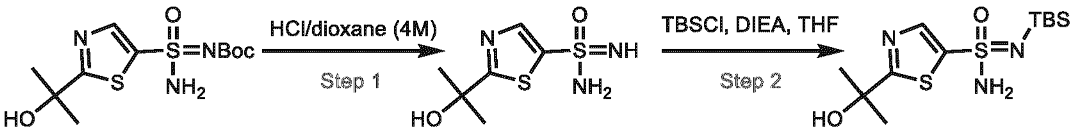


**Intermediate 28** **295"** **Intermediate 112**

## Intermediate 112

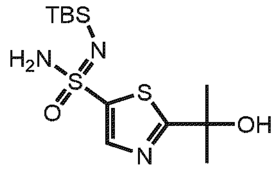

N'-(*tert*-butyldimethylsilyl)-2-(2-hydroxyl)ropan-2-yl)thiazole-5-sulfonimidamide

**Step 1: 2-(2-Hydroxypropan-2-yl)thiazole-5-sulfonimidamide**

Into a 250-mL round-bottom flask, was placed a solution of tert-butyl 2-(2-hydroxypropan-2-yl)thiazole-5-sulfonimidoylcarbamate (3.21 g, 10 mmol) in HCl/dioxane (4 M, 50 mL). The resulting solution was stirred for 1 h at RT. The solution was concentrated to give the title compound (3.2 g, crude, yellow oil). MS-ESI: 222 (M+1).

**Step 2: *N'*-(*tert*-butyldimethylsilyl)-2-(2-hydroxypropan-2-yl)thiazole-5-sulfonimidamide**

Into a 250-mL round-bottom flask, was placed 2-(2-hydroxypropan-2-yl)thiazole-5-sulfonimidamide (3.2 g crude, 10 mmol) in THF (100 mL), DIEA (3.87 g, 30 mmol) was added in at RT. Then TBSCl (3.0 g, 20 mmol) was added to the solution in portions. The resulting solution was stirred for 16 h at RT. The solution was concentrated and the crude product was purified by silica gel column with ethyl acetate/ petroleum ether (1: 1) to give the title compound (2.3 g, yield 70%, yellow solid). MS-ESI: 336 (M+1).

**Scheme 69:**

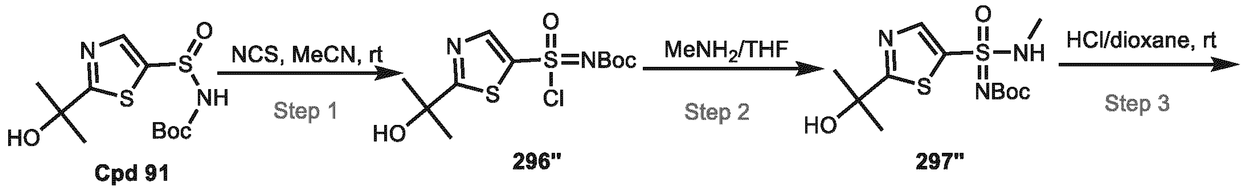


**Cpd 91** **296"** **297"**

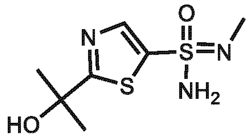

**Intermediate 113**

## Intermediate 113

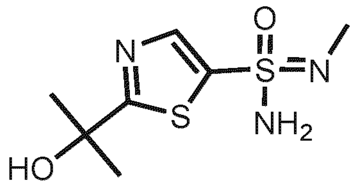

2-(2-Hydroxypropan-2-yl)-*N*-methylthiazole-5-sulfonimidamide

**Step 1: *Tert*-butyl (chloro(2-(2-hydroxypropan-2-yl)thiazol-5-yl)(oxo)-λ⁶-sulfaneylidene)carbamate**

Into a 1-L round-bottom flask, was placed tert-butyl N-[[2-(2-hydroxypropan-2-yl)-1,3-thiazol-5-yl]sulfinyl]carbamate (100 g, 326 mmol) in ACN (500 mL). To the stirred solution was added NCS (65.4 g, 49 mmol). The resulting solution was stirred for 2 h at RT. The resulted solution was concentrated. This resulted in 120 g crude title compound as yellow oil.

**Step 2: *Tert*-((2-(2-hydroxypropan-2-yl)thiazol-5-yl)(methylamino)(oxo)-$\lambda^6$-sulfaneylidene)carbamate**

Into a 250-mL round-bottom flask, was placed *tert*-butyl *N*-[chloro[2-(2-hydroxypropan-2-yl)-1,3-thiazol-5-yl]oxo-$\lambda^6$-sulfanylidene]carbamate (10 g, 29.3 mmol) in THF (100 mL). To the stirred solution was added $CH_3NH_2$ (1.82 g, 58.6 mmol). The resulting solution was stirred for 2 h at RT. The resulted solution was concentrated. The residue was eluted from silica gel with ethyl acetate/ petroleum ether (1:1). This resulted in 6.1 g (62%) of the title compound as a yellow solid. MS-ESI: 336 (M+1).

**Step 3: 2-(2-Hydroxypropan-2-yl)-*N'*-methylthiazole-5-sulfonimidamide**

Into a 100-mL round-bottom flask, was placed tert-butyl((2-(2-hydroxypropan-2-yl)thiazol-5-yl) (methylamino)(oxo)-$\lambda^6$-sulfaneylidene)carbamate (3.0 g, 8.94 mmol) in HCl (gas) in 1,4-dioxane (8.0 mL, 26.3 mmol) in one portion at RT. The resulting solution was stirred for 60 min at RT. The resulting mixture was concentrated under vacuum. This resulted in 2.10 g crude title compound as a yellow solid. MS-ESI: 236 (M+1).

The schemes below illustrate the synthesis of Intermediates **89-96, 101-104, 114-117A,** and **118"-126",** which are isocyanate and precursors thereof as well as other intermediates:

## Scheme 57:

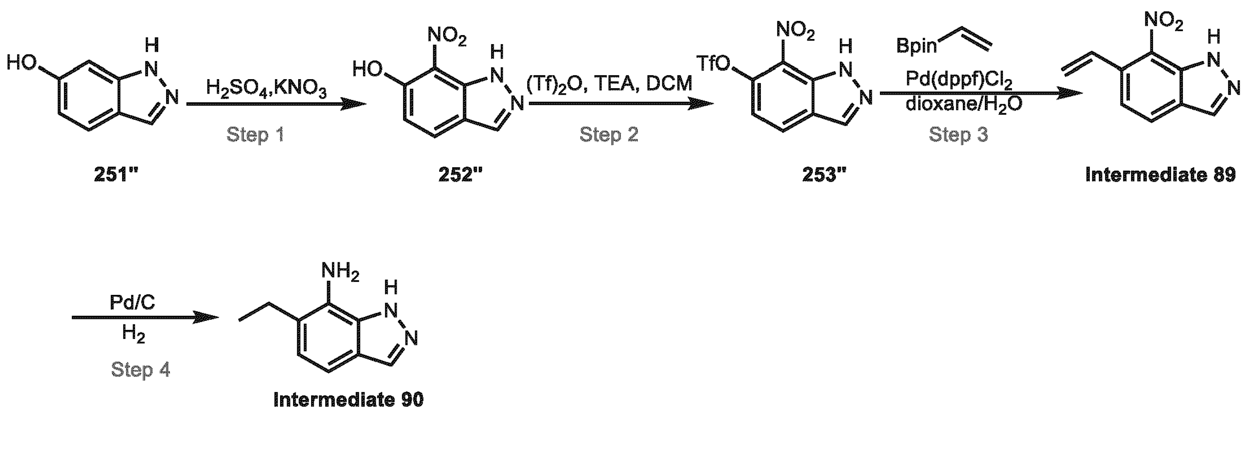


251" 252" 253" Intermediate 89

Intermediate 90

## Intermediate 89

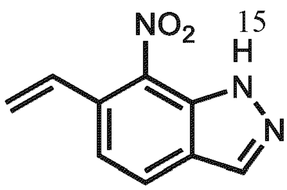

7-Nitro-6-vinyl-1H-indazole

**Step 1: 7-Nitro-1H-indazol-6-ol**

Into a 25-mL round-bottom flask, was placed 1H-indazol-6-ol (500 mg, 3.73 mmol). This was followed by the addition of $H_2SO_4$ (5.0 mL) in several batches at 0 °C. To this was added $KNO_3$ (377 mg, 3.73 mmol) in portions at 0 °C. The resulting solution was stirred for 30 min at 0 °C in a water/ice bath. The reaction was then quenched by the addition of 50 mL of water/ice. The solids were collected by filtration. This resulted in 350 mg (52.4%) of the title compound as a brown solid. MS-ESI: 180 (M+1).

**Step 2: 7-Nitro-1H-indazol-6-yl trifluoromethanesulfonate**

Into a 50-mL round-bottom flask, was placed 7-nitro-1H-indazol-6-ol (350 mg, 1.95 mmol) in DCM (10 mL), TEA (593 mg, 5.86 mmol), $Tf_2O$ (717 mg, 2.54 mmol). The resulting solution was stirred for 16 h at RT. The resulting solution was diluted with 20 mL of $H_2O$. The resulting solution was extracted with 3x20 mL of ethyl acetate dried over anhydrous sodium sulfate and concentrated. The residue was eluted from a silica gel column with ethyl acetate/petroleum ether (1:3). This resulted in 80 mg (13.2%) of the title compound as a yellow solid. MS-ESI: 312 (M+1).

**Step 3: 7-Nitro-6-vinyl-1H-indazole**

Into a 50-mL round-bottom flask purged and maintained with an inert atmosphere of nitrogen, was placed 7-nitro-1H-indazol-6-yl trifluoromethanesulfonate (100 mg, 0.32 mmol) in dioxane (10 mL) and $H_2O$ (2.0 mL), $Cs_2CO_3$ (209 mg, 0.64 mmol), 2-ethenyl-4,4,5,5-tetramethyl-1,3,2-dioxaborolane (59.4 mg, 0.39 mmol), Pd(dppf)$Cl_2$ (23.5 mg, 0.030 mmol). The resulting solution was stirred for 16 h at 90 °C. in an oil bath. Then the mixture was concentrated under vacuum. The residue was eluted from a silica gel column with ethyl acetate/petroleum ether (1:3). This resulted in 50 mg (82.6%) of the title compound as a yellow solid. MS-ESI: 190 (M+1).

## Intermediate 90

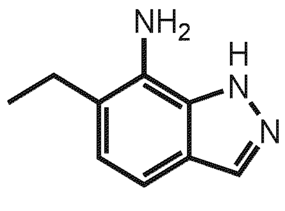

6-Ethyl-1H-indazol-7-amine

**Step 4: 6-Ethyl-1H-indazol-7-amine**

Into a 50-mL round-bottom flask, was placed 6-ethenyl-7-nitro-1H-indazole (50 mg) in MeOH (10 mL), and Pd/C (10% wt., 5.0 mg). The flask was evacuated and flushed three times with hydrogen. The resulting solution was stirred for 12 h at RT under an atmosphere of hydrogen. The Pd/C catalysts were filtered out, the filtrate was concentrated under vacuum. This resulted in 44 mg of the title compound as a yellow solid. MS-ESI: 162 (M+1).

**Scheme 58:**

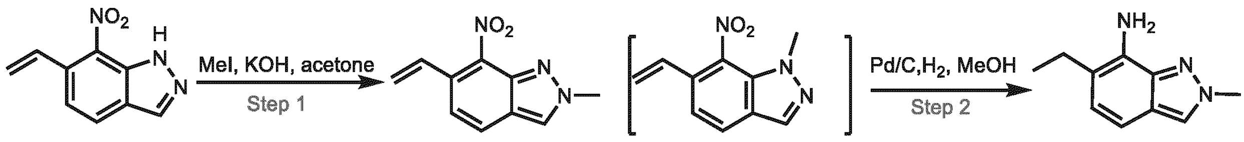


Intermediate 89 254" 255" Intermediate 91

## Intermediate 91

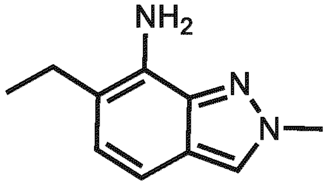

6-Ethyl-2-methyl-2H-indazol-7-amine

**Step 1: 2-Methyl-7-nitro-6-vinyl-2H-indazole**

Into a 50-mL round-bottom flask, was placed 6-ethenyl-7-nitro-1H-indazole (380 mg, 2.01 mmol) in acetone (20 mL), KOH (225 mg, 4.02 mmol). This was followed by the addition of MeI (342 mg, 2.41 mmol) dropwise with stirring. The resulting solution was stirred for 1 h at 0°C in a water/ice bath. The resulting solution was diluted with 20 mL of $H_2O$. The resulting solution was extracted with 3x30 ml of ethyl acetate dried over anhydrous sodium sulfate and concentrated. The residue was eluted from a silica gel column with ethyl acetate/petroleum ether (1:1). This resulted in 210 mg (51.5%) of **254"** as a yellow solid and 180 mg (44%) of **255"** as a yellow solid. MS-ESI: 208 (M+1).

**Step 2: 6-Ethyl-2-methyl-2H-indazol-7-amine**

Into a 50-mL round-bottom flask purged and maintained with an inert atmosphere of $N_2$, was placed 6-ethenyl-1-methyl-7-nitro-1H-indazole (210 mg, 1.03 mmol) in MeOH (15 mL) and Pd/C (10% wt., 50 mg). The flask was evacuated and flushed three times with hydrogen. The resulting solution was stirred for 12 h at RT under an atmosphere of hydrogen. The Pd/C catalysts were filtered out, and the filtrate was concentrated under vacuum. This resulted in 160 mg (88.4%) of the title compound as a yellow solid. MS-ESI: 176 (M+1).

**Table 14.** The Intermediates in the following Table were prepared using the similar procedures for converting compound **254"** to Intermediate **91** shown in **Scheme 58** from **255".**

| Intermediate # | Structure | IUPAC Name | Exact Mass $[M+H]^+$ |
|---|---|---|---|
| Intermediate 92 | 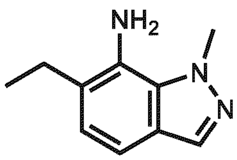 | 6-Ethyl-1-methyl-1H-indazol-7-amine | 176 |

Scheme 59:

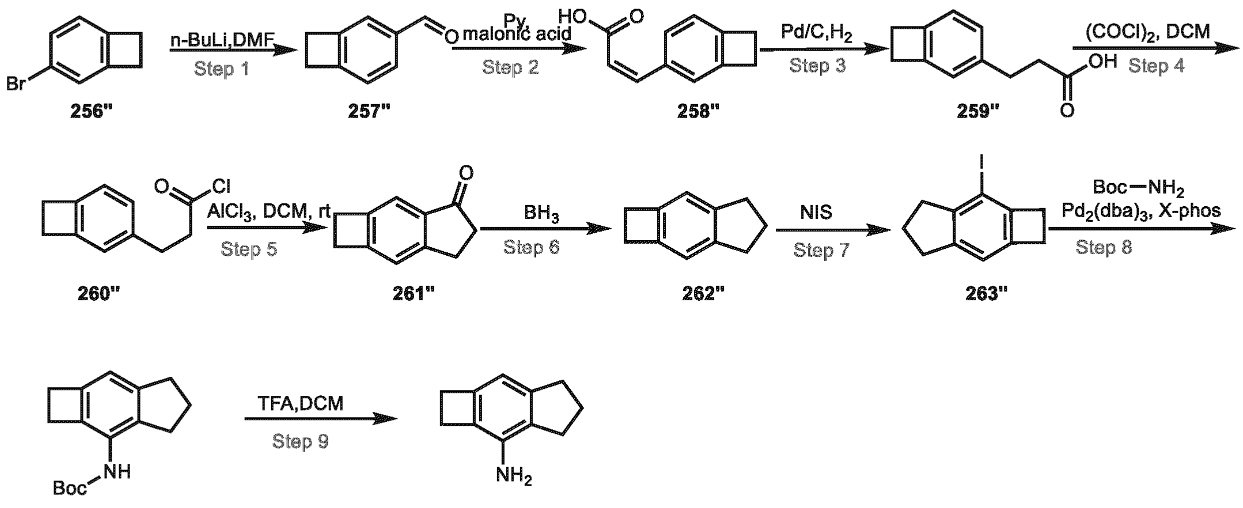


256'' 257'' 258'' 259'' 260'' 261'' 262'' 263'' 264'' Intermediate 93

Intermediate 93

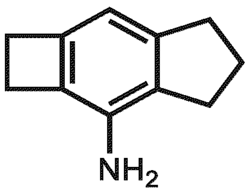

2,4,5,6-Tetrahydro-1H-cyclobuta[f]inden-3-amine

**Step 1: Bicyclo[4.2.0]octa-1(6),2,4-triene-3-carbaldehyde**

Into a 500-mL round-bottom flask purged and maintained with an inert atmosphere of nitrogen, was placed 3-bromobicyclo[4.2.0]octa-1(6),2,4-triene (70 g, 382 mmol) in THF (300 mL). This was followed by the addition of n-BuLi (184 mL, 459 mmol) dropwise with stirring at about -70 °C. After addition, the reaction mixture was stirred at this temperature for 30 min. To this solution was added DMF (36.3 g, 497 mmol) dropwise with stirring at -70 °C. The resulting solution was stirred for 30 min at -70 °C in a liquid nitrogen bath. The reaction was slowly warmed to RT and then quenched by the addition of 100 mL of water. The resulting solution was extracted with 3x200 ml of DCM. The organic layers combined and dried over anhydrous $Na_2SO_4$, and then the organic layers was concentrated. This resulted in 50 g (98.9%) of the title compound as light yellow oil. MS-ESI: 133 (M+1).

**Step 2: (Z)-3-(bicyclo[4.2.0]octa-1(6),2,4-trien-3-yl)acrylic acid**

Into a 250-mL round-bottom flask purged and maintained with an inert atmosphere of nitrogen, was placed bicyclo[4.2.0]octa-1(6),2,4-triene-3-carbaldehyde (1.7 g, 12.9 mmol) in pyridine (20 mL), propanedioic acid (1.99 g, 19.2 mmol) and piperidine (110 mg, 1.29 mmol). The resulting solution was stirred for overnight at 90 °C. in an oil bath. The resulting mixture was concentrated. This resulted in 2.1 g (93.7%) of the title compound as a solid. MS-ESI: 173 (M-1).

**Step 3: 3-(Bicyclo[4.2.0]octa-1(6),2,4-trien-3-yl)propanoic acid**

Into a 250-mL round-bottom flask, was placed 2-(Z or E)-3-[bicyclo[4.2.0]octa-1(6),2,4-trien-3-yl]prop -2-enoic acid (2.1 g, 12.1 mmol) and Pd/C (10% wt., 200 mg). The flask was evacuated and flushed three times with hydrogen. The resulting solution was stirred for 12 h at RT under an atmosphere of hydrogen. The Pd/C catalysts were filtered out, the filtrate was concentrated under vacuum. This resulted in 2.1 g (98.9%) of the title compound as a solid. MS-ESI: 175 (M-1).

**Step 4: 3-(Bicyclo[4.2.0]octa-1(6),2,4-trien-3-yl)propanoyl chloride**

Into a 250-mL round-bottom flask purged and maintained with an inert atmosphere of nitrogen, was placed 3-[bicyclo[4.2.0]octa-1(6),2,4-trien-3-yl]propanoic acid (10 g, 56.8 mmol) in DCM (100 mL). This was followed by the addition of oxalyl chloride (7.2 g, 56.8 mmol) dropwise with stirring at 0 °C. The resulting solution was stirred for 2 h at 0 °C in a water/ice bath. The resulting mixture was concentrated. This resulted in 10 g (90.5%) of the title compound as light yellow oil.

**Step 5: 1,2,5,6-Tetrahydro-4H-cyclobuta[f]inden-4-one**

Into a 100-mL round-bottom flask, was placed 3-[bicyclo[4.2.0]octa-1(6),2,4-trien-3-yl]propanoyl chloride (5.0 g, 25.7 mmol) in DCM (50 mL). This was followed by the addition of $AlCl_3$ (3.4 g, 25.7 mmol) in portions at 0 °C. for 10 min. The resulting solution was stirred for 1 h at 0 °C in a water/ice bath. The reaction was then quenched by the addition of 100 mL of water. The resulting solution was extracted with 2x50 mL of DCM. The organic layers combined and dried over anhydrous $Na_2SO_4$, then concentrated. The residue was eluted from a silica gel column with ethyl acetate/petroleum ether (1:20 to 1:15). This resulted in 3.5 g (86.1%) of the title compound as a white solid. $^1H$ NMR (300 MHz, $CDCl_3$) $\delta$ 7.45 (s, 1H), 7.17 (s, 1H), 3.22 (m, 4H), 3.18 - 3.00 (m, 2H), 2.73 - 2.63 (m, 2H).

**Step 6: 2,4,5,6-Tetrahydro-1H-cyclobuta[f]indene**

Into a 500-mL round-bottom flask purged and maintained with an inert atmosphere of nitrogen, was placed 1,2,5,6-tetrahydrocyclobuta[f]inden-4-one (20 g, 126 mmol) in THF (200 mL). This was followed by the addition of $BH_3$-$Me_2S$ (25.3 mL, 253 mmol, 10 M) dropwise at 0 °C in an ice bath. The resulting solution was stirred for 14 h at 70 °C in an oil bath. The reaction was then quenched by the addition of 20 mL of MeOH. The resulting mixture was concentrated. The residue was eluted from a silica gel column with ethyl acetate/petroleum ether (1:100 to 1:50). This resulted in 15 g (82.3%) of the title compound as colorless oil. $^1H$ NMR (300 MHz, $CDCl_3$) $\delta$ 6.95 (s, 2H), 3.10 (s, 4H), 2.88 (t, J = 7.4 Hz, 4H), 2.03 (p, J = 7.4 Hz, 2H).

**Step 7: 3-Iodo-2,4,5,6-tetrahydro-1H-cyclobuta[f]indene**

Into a 500-mL round-bottom flask, was placed acetic acid (100 mL), 2,4,5,6-tetrahydro-1H-cyclobuta[f]indene (15 g, 104 mmol) and NIS (35.1 g, 156 mmol). The resulting solution was stirred for 3 h at 50 °C in an oil bath. The resulting solution was diluted with 200 mL of water. The mixture was extracted with 3x100 mL of DCM. The organic layers combined and dried over anhydrous $Na_2SO_4$, then concentrated. The residue was eluted from a silica gel column with ethyl acetate/petroleum ether (1:100 to 1:80). This resulted in 5.0 g (17.8%) of the title compound as yellow oil.

**Step 8: *Tert*-butyl (2,4,5,6-tetrahydro-1H-cyclobuta[f]inden-3-yl)carbamate:**

Into a 250-mL round-bottom flask purged and maintained with an inert atmosphere of nitrogen, was placed 3-iodo-2,4,5,6-tetrahydro-1H-cyclobuta[f]indene (5.0 g, 18.5 mmol) in toluene (100 mL), tert-butyl carbamate (6.5 g, 55.5 mmol), X-phos (900 mg, 1.85 mmol), $Pd_2(dba)_3$ (800 mg, 0.93 mmol) , t-BuOK (6.2 g, 55.5 mmol). The resulting solution was stirred for 14 h at 100 °C in an oil bath. The resulting mixture was concentrated. The residue was eluted from a silica gel column with ethyl acetate/petroleum ether (1:50 to 1:20). This resulted in 3.0 g (83.3%) of the title compound as a white solid. MS-ESI: 260 (M+1).

$^1H$ NMR (300 MHz, $CDCl_3$) $\delta$ 6.72 (s, 1H), 6.13 (br, 1H), 3.26 (d, J = 4.5 Hz, 2H), 3.01 (d, J = 4.5 Hz, 2H), 2.90 (t, J = 7.4 Hz, 2H), 2.75 (t, J = 7.4 Hz, 2H), 2.06 (p, J = 7.4 Hz, 2H), 1.52 (s, 9H).

**Step 9: 2,4,5,6-Tetrahydro-1H-cyclobuta[f]inden-3-amine**

Into a 100-mL round-bottom flask, was placed tert-butyl2,4,5,6-tetrahydro-1H-cyclobuta[f]inden-3-ylcarbamate (3.0 g, 11.6 mmol) in DCM (20 mL), 2,2,2-trifluoroacetic acid (5.0 mL). The resulting solution was stirred for 2 h at RT. The resulting solution was diluted with 50 mL of water. The pH value of the solution was adjusted to 10 with sat. aqueous $Na_2CO_3$. The resulting solution was extracted with 3x20 mL of DCM. The organic layers combined and dried over anhydrous $Na_2SO_4$, then concentrated. This resulted in 1.5 g (81.4%) of the title compound as a yellow solid. MS-ESI: 160 (M+1).

**Scheme 60:**

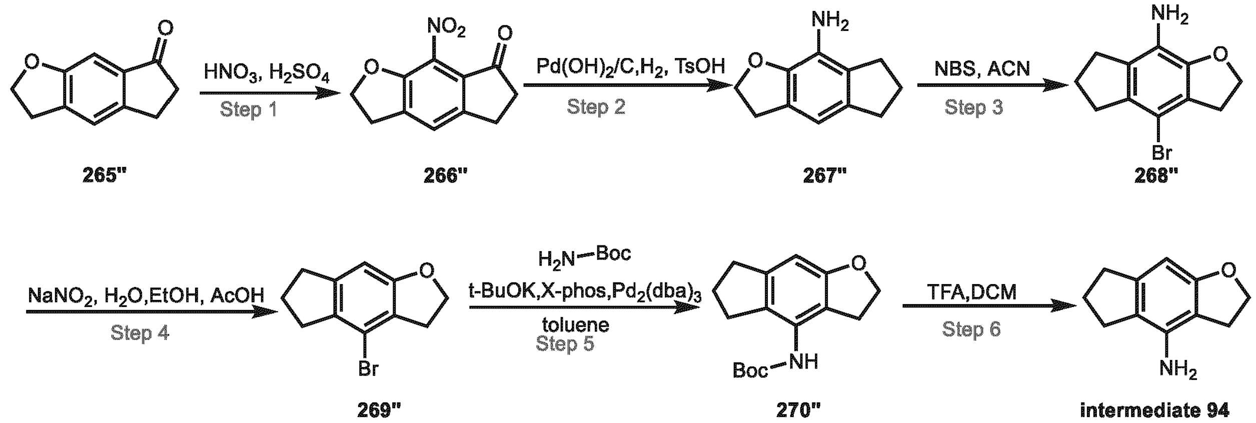


## Intermediate 94

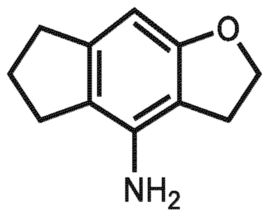

3,5,6,7-tetrahydro-2H-indeno[5,6-b]furan-4-amine

**Step 1: 8-Nitro-2,3,5,6-tetrahydro-7H-indeno[5,6-b]furan-7-one**

Into a 100-mL round-bottom flask, was placed 2H,3H,5H,6H,7H-indeno[5,6-b]furan-7-one (4 g, 23 mmol,) in $H_2SO_4$ (20 mL). This was followed by the addition of $HNO_3$ (2.13 g, 23 mmol, 68%) dropwise with stirring at 0 °C in an ice/ethanol bath. The resulting solution was stirred for 1 h at 0 °C. The reaction was then quenched by the addition of 200 mL of water/ice. The solids were collected by filtration. This resulted in 4.0 g (79.5%) of the title compound as a light brown solid. MS-ESI: 220 (M+1).

**Step 2: 3,5,6,7-Tetrahydro-2H-indeno[5,6-b]furan-8-amine**

Into a 100-mL round-bottom flask purged and maintained with an inert atmosphere of nitrogen, was placed 8-nitro-2H,3H,5H,6H,7H-indeno[5,6-b]furan-7-one (4.0 g, 18.3 mmol) in MeOH (50 mL), TsOH (1.0 mL), $Pd(OH)_2/C$ (20%wt., 1 g). The flask was evacuated and flushed three times with hydrogen. The resulting solution was stirred for 16 h at RT under an atmosphere of hydrogen. The solids were filtered out. The resulting mixture was concentrated. The residue was dissolved in 50 mL of EA. The resulting mixture was washed with 2x50 ml of $NaHCO_3$ and 3 x40 ml of $H_2O$. The mixture was dried over anhydrous sodium sulfate. The residue was eluted from a silica gel column with ethyl acetate/petroleum ether (1:9). This resulted in 1.1 g (34.4%) of the title compound as a yellow solid. MS-ESI: 176 (M+1).

**Step 3: 4-Bromo-3,5,6,7-tetrahydro-2H-indeno[5,6-b]furan-8-amine**

Into a 50-mL round-bottom flask, was placed 2H,3H,5H,6H,7H-indeno[5,6-b]furan-8-amine (1.1 g, 6.28 mmol) in ACN (30 mL) and NBS (1.34 g , 7.53 mmol). The resulting solution was stirred for 2 h at RT. The resulting mixture was concentrated. The residue was eluted from a silica gel column with ethyl acetate/petroleum ether (1:8). This resulted in 83 mg (52 %) of the title compound as a yellow solid. MS-ESI: 254 (M+1).

**Step 4: 4-Bromo-3,5,6,7-tetrahydro-2H-indeno[5,6-b]furan**

Into a 50-mL round-bottom flask, was placed 4-bromo-2H,3H,5H,6H,7H-indeno[5,6-b]furan-8-amine (500 mg, 1.97 mmol) in ethanol (15 mL) and acetic acid (3.0 mL, 0.050 mmol). To the above solution was added $NaNO_2$ (1.36 g,

19.7 mmol) in $H_2O$ (3 mL) dropwise at 0 °C. The resulting solution was stirred for 2 h at RT. The resulting solution was diluted with 30 mL of $H_2O$. The resulting solution was extracted with 3x30 ml of ethyl acetate dried over anhydrous sodium sulfate and concentrated. The residue was eluted from a silica gel column with ethyl acetate/petroleum ether (1:10). This resulted in 100 mg (21.3%) of the title compound as a yellow solid. MS-ESI: 239 (M+1).

**Step 5: *Tert-butyl* (3,5,6,7-tetrahydro-2H-indeno[5,6-b]furan-4-yl)carbamate**

Into a 50-mL round-bottom flask purged and maintained with an inert atmosphere of nitrogen, was placed 4-bromo-2H,3H,5H,6H,7H-indeno[5,6-b]furan (120 mg, 0.50 mmol) in toluene (15 mL), t-BuOK (282 mg, 2.51 mmol), tert-butyl carbamate (588 mg, 5.02 mmol), Xphos (47.8 mg, 0.10 mmol), and $Pd_2(dba)_3CHCl_3$ (104 mg, 0.10 mmol). The resulting solution was stirred for 16 h at 100 °C in an oil bath. The resulting mixture was concentrated. The residue was eluted from a silica gel column with ethyl acetate/petroleum ether (1:5). This resulted in 80 mg (57.9%) of the title compound as a yellow solid. MS-ESI: 276 (M+1).

**Step 6: 3,5,6,7-Tetrahydro-2H-indeno[5,6-b]furan-4-amine**

Into a 50-mL round-bottom flask, was placed tert-butyl N-[2H,3H,5H,6H,7H-indeno[5,6-b]furan-4-yl] carbamate (80 mg, 0.29 mmol) in DCM (8 mL) and TFA (3.0 mL, 0.030 mmol). The resulting solution was stirred for 2 h at room temperature. The resulting mixture was concentrated. The residue was dissolved in 15 mL of DCM. The resulting mixture was washed with 2 x15 ml of NaOH (aq.). The organic layer was dried with $Na_2SO_4$ and then concentrated. This resulted in 50 mg (98.2%) of the title compound as a yellow solid. MS-ESI: 176(M+1).

**Scheme 61:**

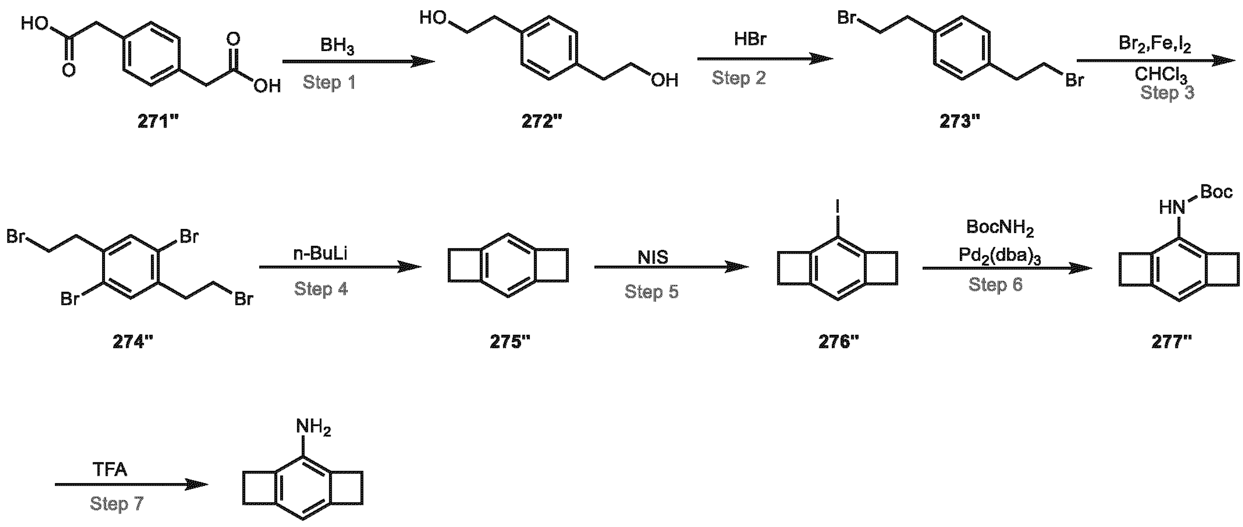


271"

272"

273"

274"

275"

276"

277"

intermediate 95

## Intermediate 95

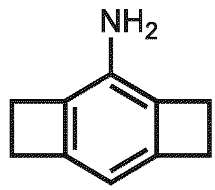

Tricyclo[6.2.0.0$^{3,6}$]deca-1,3(6),7-trien-2-amine

**Step 1: 2,2'-(1,4-Phenylene)bis(ethan-1-ol)**

Into a 1.0-L round-bottom flask purged and maintained with an inert atmosphere of nitrogen, was placed 2-[4-(carboxymethyl)phenyl]acetic acid (40 g, 200 mmol) in THF (500 mL). This was followed by the addition of $BH_3$-$Me_2S$

(60 mL, 600 mmol, 10 M) dropwise with stirring at 0 °C. The resulting solution was stirred for 24 h at RT. The reaction was then quenched by the addition of 200 mL of water. The resulting solution was extracted with 2x150 mL of ethyl acetate. The organic layers combined and dried over anhydrous $Na_2SO_4$, then concentrated. The residue was eluted from a silica gel column with ethyl acetate/petroleum ether (1:10 to 1:3). This resulted in 28 g (81.8%) of the title compound as brown oil. MS-ESI: 167 (M+1).

**Step 2: 1,4-Bis(2-bromoethyl)benzene**

Into a 50-mL round-bottom flask purged and maintained with an inert atmosphere of nitrogen, was placed 2-[4-(2-hydroxyethyl)phenyl]ethan-1-ol (28 g, 168 mmol) in aq. HBr (300 mL, 40%wt.).

The resulting solution was stirred for 5 h at 100 °C in an oil bath. The resulting solution was diluted with 500 mL of water. The resulting solution was extracted with 3x200 mL of DCM. The organic layers combined, then concentrated. This resulted in 40 g (81.4%) of the title compound as a white solid. MS-ESI: 291, 293, 295 (M+1).

**Step 3: 1,4-Dibromo-2,5-bis(2-bromoethyl)benzene**

Into a 500-mL round-bottom flask, was placed 1,4-bis(2-bromoethyl)benzene (30 g, 103 mmol) in trichloromethane (200 mL). To the above solution was added $I_2$ (0.78 g, 3.08 mmol), iron powder (0.75 g, 13.4 mmol), $Br_2$ (41 g, 257 mmol). The resulting solution was stirred for 24 h at RT. The reaction was then quenched by the addition of aqueous $Na_2SO_3$. The resulting solution was extracted with 3x200 mL DCM and the organic layers was combined and dried over anhydrous $Na_2SO_4$ then concentrated. This resulted in 40 g (86.6%) of the title compound as a white solid. MS-ESI: 449/451/453 (M+1).

**Step 4: Tricyclo[6.2.0.0$^{3,6}$]deca-1,3(6),7-triene**

Into a 1000-mL round-bottom flask purged and maintained with an inert atmosphere of nitrogen, was placed 1,4-dibromo-2,5-bis(2-bromoethyl)benzene (40 g, 88.9 mmol) in THF (400 mL). This was followed by the addition of n-BuLi (74.7 mL, 187 mmol, 2.5 M) dropwise with stirring at - 78 °C in a liquid nitrogen bath. The resulting solution was stirred for 30 min at -78 °C. The reaction was then quenched by the addition of aqueous $NH_4Cl$ (300 ml) and extracted with 2x200 mL of DCMDCM and the organic layers was combined and dried over anhydrous $Na_2SO_4$ then concentrated. This resulted in 8.0 g (69.1%) of the title compound as a light yellow solid.

**Step 5: 2-Iodotricyclo[6.2.0.0$^{3,6}$]deca-1,3(6),7-triene**

Into a 250-mL round-bottom flask purged and maintained with an inert atmosphere of nitrogen, was placed tricyclo[6.2.0.0$^{3,6}$]deca-1,3(6),7-triene (8 g, 61.45 mmol) in acetic acid (50 mL) and NIS (20.7 g, 92.2 mmol). The resulting solution was stirred for 3 h at 50 °C in an oil bath. The resulting solution was diluted with 100 mL of water. The reaction was then quenched by the addition of aqueous $Na_2SO_3$. The resulting solution was extracted with 3x50 mL of DCM and the organic layers was combined and dried over anhydrous $Na_2SO_4$ then concentrated. The residue was eluted from a silica gel column with ethyl acetate/petroleum ether (1: 100). This resulted in 2.5 g (18.2%) of the title compound as a white solid.

**Step 6: *Tert*-butyl tricyclo[6.2.0.0$^{3,6}$]deca-1,3(6),7-trien-2-ylcarbamate**

Into a 250-mL round-bottom flask purged and maintained with an inert atmosphere of nitrogen, was placed 2-iodotricyclo[6.2.0.0$^{3,6}$]deca-1,3(6),7-triene (2.5 g, 9.76 mmol) in toluene (50 mL). To the stirred solution was added tert-butyl carbamate (3.43 g, 29.3 mmol), $Pd_2(dba)_3$ (447 mg, 0.49 mmol), Xphos (466 mg, 0.98 mmol), and t-BuOK (3.29g, 29.3 mmol). The resulting solution was stirred for 14 h at 100 °C in an oil bath. The resulting mixture was concentrated. The residue was eluted from a silica gel column with ethyl acetate/petroleum ether (1:50 to 1:30). This resulted in 1.5 g (62.6%) of the title compound as a light yellow solid. MS-ESI: 246 (M+1).

**Step 7: Tricyclo[6.2.0.0$^{3,6}$]deca-1,3(6),7-trien-2-amine**

Into a 50-mL round-bottom flask purged and maintained with an inert atmosphere of nitrogen, was placed tert-butyl N-[tricyclo[6.2.0.0$^{3,6}$]deca-1,3(6),7-trien-2-yl]carbamate (1.5 g, 6.1 mmol) in DCM (20 mL) and 2,2,2-trifluoroacetic acid (4.0 mL). The resulting solution was stirred for 2 h at RT. The resulting mixture was concentrated. This resulted in 800 mg (90.1%) of the title compound as a brown solid. MS-ESI: 146 (M+1).

**Scheme 62A:**

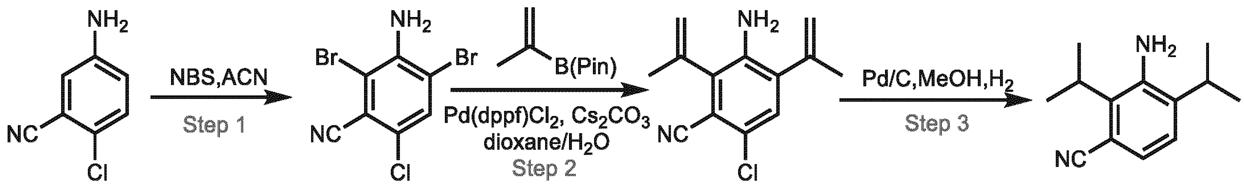


278" 279" 280" **Intermediate 96**

## Intermediate 96

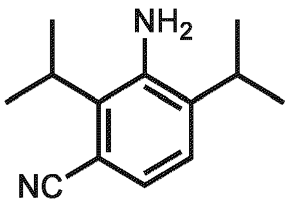

3-Amino-2,4-diisopropylbenzonitrile

**Step 1: 3-Amino-2,4-dibromo-6-chlorobenzonitrile**

Into a 500-mL round-bottom flask, was placed 5-amino-2-chlorobenzonitrile (10 g, 65.8 mmol), ACN (200 mL) and NBS (17.6 g, 98.7 mmol). The resulting solution was stirred for 14 h at RT. The resulting mixture was concentrated. The residue was eluted from a silica gel column with ethyl acetate/petroleum ether (1:15 to 1:5). This resulted in 18 g of the title compound as a yellow solid. MS-ESI: 310, 312 (M+1).

**Step 2: 3-Amino-6-chloro-2,4-di(prop-1-en-2-yl)benzonitrile**

Into a 500-mL round-bottom flask purged and maintained with an inert atmosphere of nitrogen, was placed 3-amino-2,4-dibromo-6-chlorobenzonitrile (15 g, 48 mmol) in dioxane (200 mL) and $H_2O$ (20 mL), 2-(tetramethyl-1,3,2-dioxaborolan-2-yl)prop-2-en-1-ylium (17.6 g, 106 mmol), $Cs_2CO_3$ (47 g, 144 mmol), and Pd(dppf)$Cl_2$ (1.5 g, 4.8 mmol). The resulting solution was stirred for 14 h at 100 °C in an oil bath. The resulting mixture was concentrated. The residue was eluted from a silica gel column with ethyl acetate/ petroleum ether (1:0 to 1:25). This resulted in 10 g of the title compound as brown oil. MS-ESI: 233 (M+1).

**Step 3: 3-Amino-2,4-diisopropylbenzonitrile**

Into a 500-mL round-bottom flask purged and maintained with an inert atmosphere of nitrogen, was placed 3-amino-6-chloro-2,4-bis(prop-1-en-2-yl)benzonitrile (10 g, 43 mmol) in MeOH (50 mL). Then Pd/C (10% wt., 2.0 g) was added. The flask was evacuated and flushed three times with hydrogen. The resulting solution was stirred for 16 h at RT under an atmosphere of hydrogen. The solids were filtered out. The resulting filtrate was concentrated under vacuum. This resulted in 8.0 g of the title compound as brown oil. MS-ESI: 203 (M+1).

**Scheme 65:**

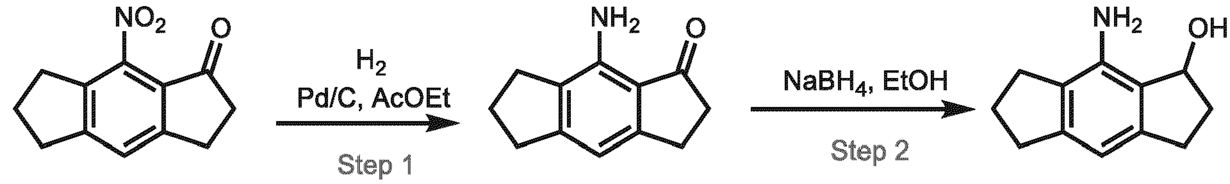


**cpd 114"** **Intermediate 100** **Intermediate 101**

**Intermediate 101**

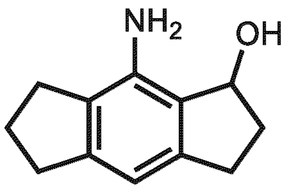

8-Amino- 1,2,3,5,6,7-hexahydro-s-indacen-1-ol

**Step 1: 8-Amino-3,5,6,7-tetrahydro-s-indacen-1(2H)-one**

Into a 250-mL round-bottom flask purged and maintained with an inert atmosphere of hydrogen, was placed a solution of 8-nitro-1,2,3,5,6,7-hexahydro-s-indacen-1-one (700 mg, 3.22 mmol) in MeOH (10 mL), and Pd/C (10% wt., 100 mg). The flask was evacuated and flushed three times with hydrogen. The resulting solution was stirred for 2 h at RT under an atmosphere of hydrogen. The Pd/C catalysts were filtered out, and the filtrate was concentrated under vacuum. This resulted in 550 mg (91.2%) of the title compound as a yellow oil. MS-ESI: 188 (M+1).

**Step 2: 8-Amino-1,2,3,5,6,7-hexahydro-s-indacen-1-ol**

Into a 100 mL round-bottom flask, was placed a solution of 8-amino-3,5,6,7-tetrahydro-s-indacen-1 (2H)-one (2.0 g, 10.7 mmol ) in ethanol. To this solution was added $NaBH_4$(1.9 g, 50 mmol) with stirring in portions at 0 °C in an ice bath. The resulting solution was stirred for 16 h at RT. The reaction was quenched by water (10 mL). The resulting solution was extracted with 3x50 mL of ethyl acetate and the organic layers combined and dried over anhydrous sodium sulfate, and then concentrated under vacuum. This resulted in 1.5 g of the title compound as a yellow solid. MS-ESI: 189 (M+1).

**Scheme 66:**

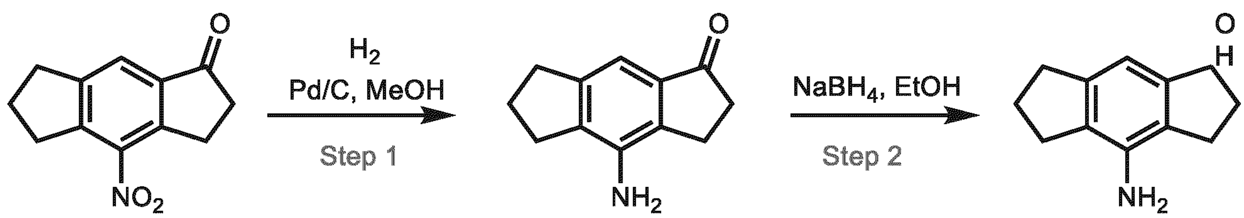


**Cpd 115"** **Intermediate 102** **Intermediate 103**

## Intermediate 103

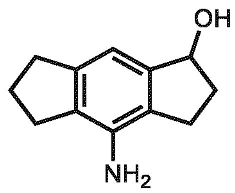

4-Amino-1,2,3,5,6,7-hexahydro-s-indacen-1-ol

**Step 1: 4-Amino-3,5,6,7-tetrahydro-s-indacen-1(2H)-one**

Into a 250-mL round-bottom flask purged and maintained with an inert atmosphere of hydrogen, was placed a solution of 4-nitro-1,2,3,5,6,7-hexahydro-s-indacen-1-one (3.0 g, 13.8 mmol) in MeOH (30 mL), and Pd/C (10% wt., 500 mg). The flask was evacuated and flushed three times with hydrogen. The resulting solution was stirred for 4 h at RT under an atmosphere of hydrogen. The Pd/C catalysts were filtered out, the filtrate was concentrated under vacuum. The residue was eluted from a silica gel column with DCM/MeOH (10:1). This resulted in 2.2 g (85.1%) of the title compound as a white solid. MS-ESI: 187 (M+1).

**Step 2: 4-Amino-1,2,3,5,6,7-hexahydro-*s*-indacen-1-ol**

Into a 100-mL round-bottom flask, was placed a solution of 8-amino-3,5,6,7-tetrahydro-s-indacen-1(2H)-one (2.0 g, 10.7 mmol) in ethanol (20 mL) and $NaBH_4$ (1.9 g, 50 mmol). The resulting solution was stirred for 16h at RT. The reaction was quenched with water. The resulting solution was extracted with 3x50 mL of ethyl acetate and the organic layers combined and dried over anhydrous sodium sulfate. The mixture was concentrated under vacuum. This resulted in 1.36 g of the title compound as a yellow solid. MS-ESI: 190 (M+1).

## Scheme 67:

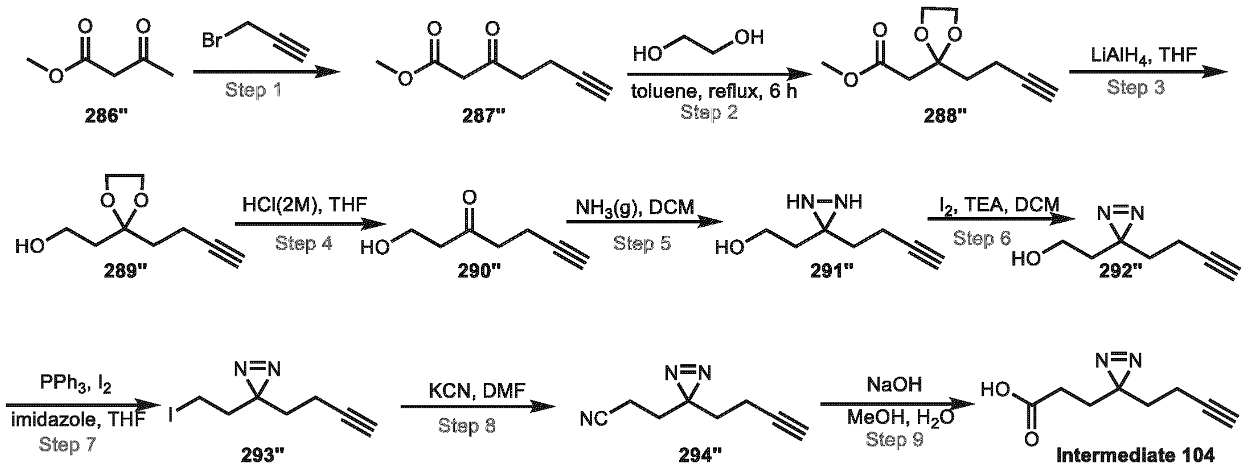


## Intermediate 104

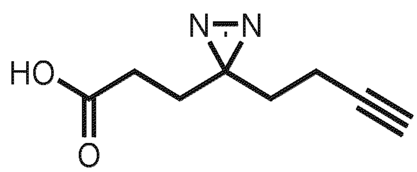

3-(3-(But-3-ynyl)-3H-diazirin-3-yl)propanoic acid

**Step 1: Methyl 3-oxohept-6-ynoate**

Into a 2000-mL 3-neck round-bottom flask purged with and maintained under nitrogen, was placed methyl 3-oxobutanoate (20 g, 172 mmol) in THF (200 mL). To the above solution was added LDA (200 mL, 400 mmol, 2 M) dropwise at -20°C in a dry ice bath. Then reaction was allowed to react at -20 °C for 30 min. Then 3-bromoprop-1-yne (20.5 g, 172 mmol) was added to the reaction solution in portions at -20 °C. The resulting solution was stirred for 3 h at -20 °C in a dry ice bath. The reaction was then quenched by the addition of 500 mL of $NH_4Cl$ solution. The pH value of the solution was adjusted to 3 with HCl (aq). The resulting solution was extracted with 3x200 ml of ethyl acetate and the organic layers was combined and dried over anhydrous $Na_2SO_4$, then concentrated. This resulted in the title compound (2.0 g, 7.53%) as white oil.

**Step 2: Methyl 2-(2-(but-3-ynyl)-1,3-dioxolan-2-yl)acetate**

Into a 500-mL round-bottom flask, was placed methyl 3-oxohept-6-ynoate (20 g, 130 mmol) in toluene (200 mL), ethane-1,2-diol (40.2 g, 649 mmol) and TsOH (2.23 g, 13 mmol). The resulting solution was stirred for 6 h at 120 °C in an oil bath. The resulting solution was diluted with 200 mL of $Et_2O$. The resulting mixture was washed with 3 x100 ml of $NaHCO_3$ and 3x100 ml of saturated NaCl solution. The mixture was dried over anhydrous sodium sulfate and concentrated. This resulted in the title compound (20 g, 77.9%) as yellow oil.

**Step 3: 2-(2-(But-3-ynyl)-1,3-dioxolan-2-yl)ethanol**

Into a 1.0-L 3-necked round-bottom flask purged and maintained with an inert atmosphere of nitrogen, was placed methyl 2-[2-(but-3-yn-1-yl)-1,3-dioxolan-2-yl]acetate (90 g, 454 mmol) in THF (300mL). To this above solution was added $LiAlH_4$ (17.9 g, 472 mmol) in portions with stirring at 0 °C in an ice/ethanol bath. The resulting solution was stirred for 6 h at RT. The reaction was then quenched by the addition of water/ice. The solids were filtered out. The resulting filtrate was concentrated under vacuum. This resulted in the title compound (80 g crude) and used in the next step directly. MS-ESI: 169 (M-1).

**Step 4: 1-Hydroxyhept-6-yn-3-one**

Into a 3.0-L 3-necked round-bottom flask purged and maintained with an inert atmosphere of nitrogen, was placed 2-[2-(but-3-yn-1-yl)-1,3-dioxolan-2-yl]ethan-1-ol (80 g, 470 mmol) in THF (1.0L) and HCl (500 mL). The resulting solution was stirred for 16 h at RT. The resulting solution was diluted with 1.0L of water. The mixture was extracted with 3 ×1.0L of ethyl acetate and the organic layer was combined and dried over anhydrous sodium sulfate. The solids were filtered out. The resulting filtrate was concentrated under vacuum. The residue was eluted from a silica gel column with DCM/petroleum ether (1:1). This resulted in 20 g of the title compound as a white solid. MS-ESI: 125 (M-1).

**Step 5: 2-(3-(But-3-ynyl)diaziridin-3-yl)ethanol**

Into a 500-mL round-bottom flask purged and maintained with an inert atmosphere of nitrogen, was placed 1-hydroxyhept-6-yn-3-one (20 g, 159 mmol) in DCM (250 mL). To the above solution was introduced $NH_3$ (g) for 15 min at -40°C in a liquid nitrogen/ethanol bath. The resulting solution was stirred for 1 h at -40°C and then allowed to react for 16h at RT. The resulting mixture was concentrated. This resulted in 18 g (crude) of the title compound as a white solid. MS-ESI: 141 (M+1).

**Step 6: 2-(3-(But-3-ynyl)-3H-diazirin-3-yl)ethanol**

Into a 500-mL 3-necked round-bottom flask purged and maintained with an inert atmosphere of nitrogen, was placed 2-[3-(but-3-yn-1-yl)diaziridin-3-yl]ethan-1-ol (14.4 g, 114 mmol) in DCM (200 mL), TEA (34.6g, 342 mmol), $I_2$ (58 g, 228 mmol). The resulting solution was stirred for 4 h at RT. The reaction was then quenched by the addition of $Na_2S_2O_3$. The resulting mixture was quenched with 100 mL of water. The resulting solution was extracted with 3x300 mL of DCM and the organic layers combined and dried over anhydrous sodium sulfate. The solids were filtered out. The resulting mixture was concentrated. This resulted in 6.0 g (38%) of the title compound as a white solid. MS-ESI: 139 (M+1).

**Step 7: 3-(But-3-ynyl)-3-(2-iodoethyl)-3H-diazirine**

Into a 250-mL round-bottom flask purged and maintained with an inert atmosphere of nitrogen, was placed 2-[3-(but-3-yn-1-yl)-3H-diazirin-3-yl]ethan-1-ol (5.0 g, 36.2 mmol) in THF (20 mL), imidazole (3.7 g, 54.3 mmol), $I_2$ (9.18 g, 36.2 mmol), $PPh_3$ (14.2 g, 54.3 mmol). The resulting solution was stirred for 16 h at RT. The reaction was then quenched by the addition of 20 mL of saturated $Na_2S_2O_3$ solution. The resulting solution was extracted with 3x50 mL of DCM dried over anhydrous sodium sulfate. The solids were filtered out. The resulting mixture was concentrated. This resulted in 5.0 g (crude) of the title compound as a yellow solid. MS-ESI: 248 (M+1).

**Step 8: 3-(3-(But-3-ynyl)-3H-diazirin-3-yl)propanenitrile**

Into a 500-mL round-bottom flask purged and maintained with an inert atmosphere of nitrogen, was placed 3-(but-3-yn-1-yl)-3-(2-iodoethyl)-3H-diazirine (5.0 g, 20.2 mmol) in DMF (250 mL), KCN (2.62 g, 40.3mmol). The resulting solution was stirred for 16 h at 60 °C in an oil bath. The reaction was then quenched by the addition of 20 mL of $FeSO_4$ solution. The resulting solution was extracted with 3x50 ml of ethyl acetate dried over anhydrous sodium sulfate. The solids were filtered out. The resulting mixture was concentrated. This resulted in 2.0 g (crude) of the title compound as a solid. MS-ESI: 148 (M+1).

**Step 9: 3-(3-(But-3-ynyl)-3H-diazirin-3-yl)propanoic acid**

Into a 100-mL round-bottom flask purged and maintained with an inert atmosphere of nitrogen, was placed 3-[3-(but-3-yn-1-yl)-3H-diazirin-3-yl]propanenitrile (1.0 g, 3.40 mmol) in MeOH (40 mL), NaOH (272 mg, 6.79 mmol). The resulting solution was stirred for 16 h at 90 °C in an oil bath. The resulting solution was concentrated. The residue was eluted from a silica gel column with ethyl acetate/ petroleum ether (1:1). This resulted in 400 mg crude (26.6%) of the title compound as yellow oil. MS-ESI: 167 (M+1).

**Scheme 70:**

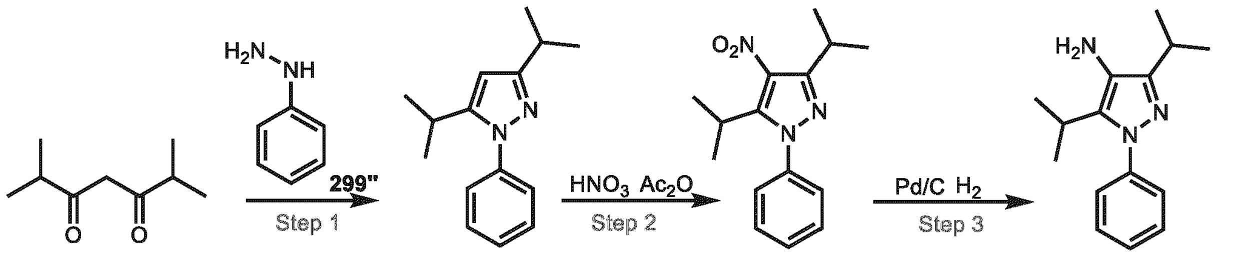


298'' 300'' 301'' Intermediate 114

**Intermediate 114**

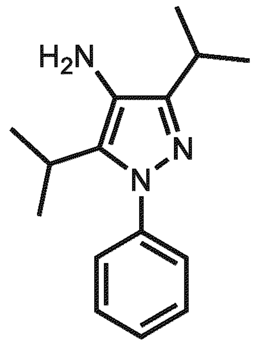

<u>**3,5-Diisopropyl-1-phenyl-1H-pyrazol-4-amine**</u>

**Step 1: 3,5-Diisopropyl-1-phenyl-1H-pyrazole**

Into a 100-mL round-bottom flask, was placed 2-propanol (50 mL), phenylhydrazine (3.81 g, 35.2 mmol) and 2,6-dimethylheptane-3,5-dione (5.0 g, 32.0 mmol). The resulting solution was stirred overnight at 85 °C in an oil bath.

The resulting mixture was concentrated. The residue was dissolved in 100 mL of ethyl acetate. The resulting mixture was washed with 50 mL of $H_2O$. The mixture was dried over anhydrous sodium sulfate and then concentrated. This resulted in 6.9 g (94%) of the title compound as a light yellow oil. MS-ESI: 229 (M+1).

**Step 2: 3,5-Diisopropyl-4-nitro-1-phenyl-1H-pyrazole**

Into a 100-mL round-bottom flask, was placed 1-phenyl-3,5-bis(propan-2-yl)-1H-pyrazole (6.9 g, 30 mmol) in $Ac_2O$ (50 mL). This was followed by the addition of $HNO_3$ (4.07 mL, 91 mmol) dropwise with stirring at 0 °C in 10 min. The resulting solution was stirred for overnight at RT. The residue was dissolved in 150 mL of ethyl acetate. The resulting mixture was washed with 2x100 mL of $H_2O$. The mixture was dried over anhydrous sodium sulfate and then concentrated. This resulted in 3.7 g (44.8%) of the title compound as yellow oil. MS-ESI: 274 (M+1).

**Step 3: 3,5-Diisopropyl-1-phenyl-1H-pyrazol-4-amine**

Into a 250-mL round-bottom flask, was placed 4-nitro-1-phenyl-3,5-bis(propan-2-yl)-1H-pyrazole (3.7 g, 13.5 mmol) in MeOH (100 mL), to the stirred solution was added Pd/C (10%wt., 400 mg). The flask was evacuated and flushed three times with hydrogen. The resulting solution was stirred overnight at RT under an atmosphere of hydrogen. The Pd/C catalysts were filtered out, the filtrate was concentrated under vacuum. This resulted in 2.7 g (82%) of the title compound as a light yellow oil. MS-ESI: 244 (M+1).

**Scheme 72:**

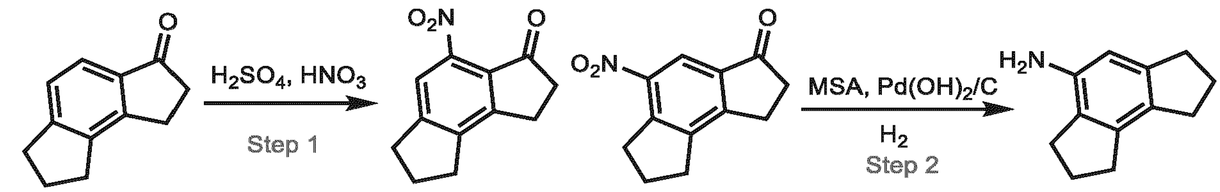


307''    308''    309''    Intermediate 116

**Intermediate 116**

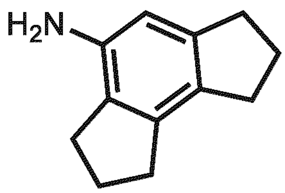

1,2,3,6,7,8-Hexahydro-as-indacen-4-amine

**Step 1: 4-Nitro-1,6,7,8-tetrahydro-as-indacen-3(2H)-one (308) and 5-nitro-1,6,7,8-tetrahydro-as-indacen-3(2H)-one (309")**

Into a 250-mL round-bottom flask was placed a solution of 1,6,7,8-tetrahydro-as-indacen-3(2H)-one (Cpd **307"** was isolated from **113"** in **Scheme 23** by chromatography) (9.8 g, 46.5 mmol) in $H_2SO_4$ (50 mL). Then $HNO_3$ (5.85 g, 92.9 mmol) was added dropwise over 10 min at 0 °C. The resulting solution was stirred for 1 h at 0 °C. The reaction mixture was slowly added to a mixture of water/ice (100 mL) and DCM (50 mL) with ice bath cooling. The organic layer was collected, dried over $Na_2SO_4$ and concentrated under vacuum. This resulted in 11 g (89%) of a mixture of cpd **308"** and cpd **309"** as a yellow solid. The mixture was monitored by TLC (ethyl acetate/ petroleum ether = 1/10, $R_f$ = 0.4),

**Step 2: 1,2,3,6,7,8-hexahydro-as-indacen-4-amine (116)**

Into a 100-mL round-bottom flask was placed a solution of the mixture of 4-nitro-1,6,7,8-tetrahydro-as-indacen-3(2H)-one and 5-nitro-1,6,7,8-tetrahydro-as-indacen-3(2H)-one (2.17 g, 10 mmol) in MeOH (30 mL). To the solution was added MSA (1.15 g, 12 mmol). Then $Pd(OH)_2/C$ (20%wt., 550 mg) was added. The flask was evacuated and filled three times with hydrogen. The resulting mixture was stirred for 16 h at RT under hydrogen (50 psi). The solids were filtered out and washed with MeOH. The MeOH filtrate and wash was diluted with water (50 mL) and the pH was adjusted

to 10.6 with 2 N NaOH. The resulting mixture was filtered and the crude solids were recrystallized from MeOH/water (9:1) with heating. This resulted in 1.38 g (80%) of the title compound as an off-white solid. MS-ESI: 174 (M+1).

**Scheme 73:**

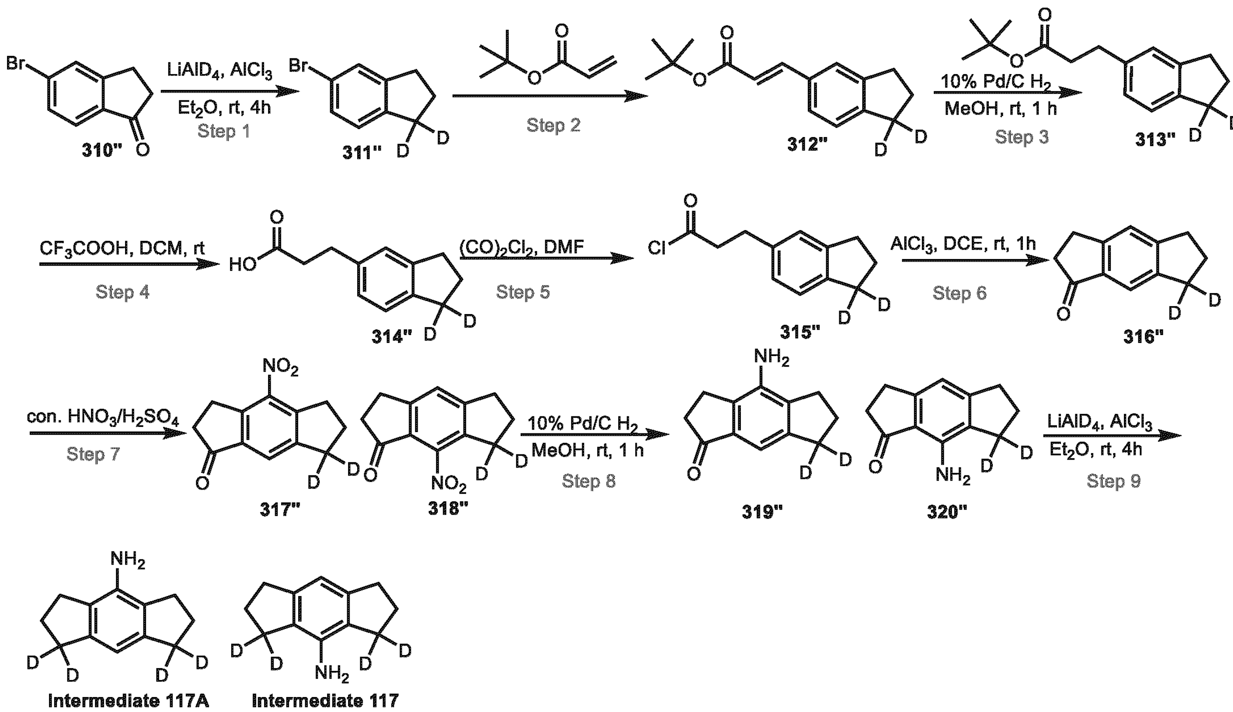


**Intermediate 117**

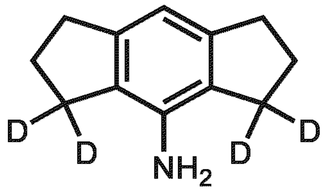

1,2,3,5,6,7-Hexahydro-*s*-indacen-3,3,5,5-$d_4$-4-amine

**Step 1: 5-Bromo-2,3-dihydro-1H-indene-1,1-$d_2$**

Into a 250-mL 3-necked round-bottom flask purged and maintained with an inert atmosphere of nitrogen, was placed a solution of $LiAlD_4$ (1.57 g, 37 mmol) in $Et_2O$ (150 mL). This was followed by the addition of $AlCl_3$ (10.1 g, 76 mmol) in portions at 0 °C in 5 min. To this was added 5-bromo-2,3-dihydro-1H-inden-1-one (4.0 g, 19 mmol) in portions at 0 °C in 5 min. The resulting solution was stirred for 4 h at RT. The reaction mixture was cooled to 0 °C with a water/ice bath. The reaction was then quenched by careful addition of 10 mL of water. The solids were filtered out. The resulting solution was extracted with 3x100 mL of ethyl acetate and concentrated under vacuum. This resulted in 3.5 g (93%) of the title compound as brown oil. MS-ESI: 199/201 (M+1).

**Step 2: Tert-butyl (E)-3-(2,3-dihydro-1H-inden-5-yl-1,1-$d_2$)acrylate**

Into a 250-mL round-bottom flask purged and maintained with an inert atmosphere of nitrogen, was placed a solution of 5-bromo-2,3-dihydro-1H-indene-1,1-$d_2$ (7.0 g, 35 mmol) in DMF (80 mL), to the stirred solution was added tris(4-methylphenyl)phosphane (1.07 g, 3.52 mmol), tert-butyl prop-2-enoate (4.0 mL), triethylamine (5.0 mL) and $Pd(OAc)_2$ (395 mg, 1.76 mmol). The resulting solution was stirred overnight at 100 °C in an oil bath. The resulting mixture was concentrated under vacuum. The resulting solution was extracted with 3x50 mL of ethyl acetate and the organic layers combined and dried over anhydrous sodium sulfate. The residue was eluted from a silica gel column with DCM/petroleum ether (1:1). This resulted in 5.7 g (66%) of the title compound as light yellow oil. MS-ESI: 247 (M+1).

**Step 3: Tert-butyl 3-(2,3-dihydro-1H-inden-5-yl-1,1-d$_2$)propanoate**

Into a 100-mL round-bottom flask purged and maintained with an inert atmosphere of nitrogen, was placed a solution of tert-butyl (E)-3-(2,3-dihydro-1H-inden-5-yl-1,1-*d*$_2$)acrylate (5.8 g, 24 mmol) in MeOH (40 mL), to the stirred solution was added Pd/C (580 mg, 10% wt.). The flask was evacuated and flushed three times with hydrogen. The resulting solution was stirred for 1h at RT under an atmosphere of hydrogen. The Pd/C catalysts were filtered out, the filtrate was concentrated under vacuum. This resulted in 5.7 g (98%) of the title compound as colorless oil. MS-ESI: 249 (M+1).

**Step 4: 3-(2,3-Dihydro-1H-inden-5-yl-1,1-*d*$_2$)propanoic acid**

Into a 100-mL round-bottom flask, was placed a solution of tert-butyl 3-(2,3-dihydro-1H-inden-5-yl-1,1-d$_2$)propanoate (4.3 g, 17.3 mmol) in DCM (50 mL), to the stirred solution was added $CF_3COOH$ (5.5 mL, 74 mmol). The resulting solution was stirred for overnight at RT. The resulting mixture was concentrated under vacuum. This resulted in 3.1 g (93%) of the title compound as an off-white solid. MS-ESI: 191 (M-1).

**Step 5: 3-(2,3-Dihydro-1H-inden-5-yl-1,1-*d*$_2$)propanoyl chloride**

Into a 100-mL round-bottom flask purged and maintained with an inert atmosphere of nitrogen, was placed a solution of 3-(2,3-dihydro-1H-inden-5-yl-1,1-*d*$_2$)propanoic acid (9.0 g, 41.7 mmol) in DCM (40 mL). This was followed by the addition of oxalic dichloride (8.0 mL) at 0 °C. To this was added DMF (0.5 mL) at 0 °C. The resulting solution was stirred for 3 h at RT. The resulting mixture was concentrated under vacuum. This resulted in 4.0 g (41%) of the title compound as brown oil.

**Step 6: 3,5,6,7-Tetrahydro-*s*-indacen-1(2H)-one-7,7-*d*$_2$**

Into a 100-mL round-bottom flask purged and maintained with an inert atmosphere of nitrogen, was placed a solution of 3-(2,3-dihydro-1H-inden-5-yl-1,1-d$_2$)propanoyl chloride (3.9 g, 18 mmol) in DCE (40 mL). This was followed by the addition of $AlCl_3$ (3.3 g, 25 mmol) in portions at 0 °C in 2 min. The resulting solution was stirred for 1 h at RT. The reaction was then quenched by the addition of 200 mL of water/ice. The resulting solution was extracted with 3x50 mL of DCM and the organic layers combined and dried over anhydrous sodium sulfate. The residue was eluted from a silica gel column with ethyl acetate/petroleum ether (2:100). This resulted in 1.5 g (46%) of the title compound as an off-white solid. MS-ESI: 175 (M+1).

**Step 7: 8-Nitro-3,5,6,7-tetrahydro-*s*-indacen-1(2H)-one-7,7-*d*$_2$ (Cpd 318", major) and 4-Nitro-3,5,6,7-tetrahydro-*s*-indacen-1(2H)-one-7,7-*d*$_2$ (Cpd 317", minor)**

Into a 25-mL round-bottom flask, was placed 3,5,6,7-tetrahydro-*s*-indacen-1(2H)-one-7,7-*d*$_2$(120 g). This was followed by the addition of $H_2SO_4$ (8.0 mL) at 0 °C. To this was added $HNO_3$ (2.0 mL) at 0 °C in 2 min. To the mixture was added $H_2SO_4$ (2.0 mL) at 0 °C in 2 min. The resulting solution was stirred for 1 h at 0 °C. The reaction was then quenched by the addition of water/ice. The resulting solution was extracted with 3x50 mL of ethyl acetate dried in an oven under reduced pressure. The residue was separated on silica gel eluted with ethyl acetate/petroleum ether (3:100). This resulted in 870 mg of **cpd 318"** and 290 mg of **cpd 317",** both as yellow solids. **Cpd 317":** $^1$H NMR (300 MHz, $CDCl_3$) δ 7.83 (s, 1H), 3.55 - 3.45 (m, 2H), 3.42 (t, *J* = 7.6 Hz, 2H), 2.84 - 2.74 (m, 2H), 2.22 (t, *J* = 7.6 Hz, 2H). Cpd 318": $^1$H NMR (300 MHz, $CDCl_3$) δ 7.46 (s, 1H), 3.20 - 3.00 (m, 4H), 2.83 - 2.73 (m, 2H), 2.20 (t, *J* = 7.5 Hz, 2H).

**Step 8: 8-Amino-3,5,6,7-tetrahydro-*s*-indacen-1(2H)-one-7,7-*d*$_2$**

Into a 250-mL round-bottom flask purged and maintained with an inert atmosphere of nitrogen, was placed a solution of 8-nitro-3,5,6,7-tetrahydro-*s*-indacen-1(2H)-one-7,7-d$_2$ (870 mg) in MeOH (100 mL), to the stirred solution was added Pd/C (87 mg, 10%wt.). The flask was evacuated and flushed three times with hydrogen. The resulting solution was stirred for 1h at RT under an atmosphere of hydrogen. The Pd/C catalysts were filtered out, the filtrate was concentrated under vacuum. This resulted in 700 mg of the title compound as a yellow solid. MS-ESI: 190 (M+1).

**Step 9: 1,2,3,5,6,7-Hexahydro-*s*-indacen-3,3,5,5-*d₄*-4-amine**

Into a 250-mL round-bottom flask purged and maintained with an inert atmosphere of nitrogen, was placed a solution of $LiAlD_4$ (160 mg, 3.8 mmol) in $Et_2O$ (40 mL). This was followed by the addition of $AlCl_3$ (634 mg, 4.8 mmol) in portions at 0 °C in 2 min. To this solution was added 8-amino-3,5,6,7-tetrahydro-*s*-indacen-1(2H)-one-7,7-$d_2$ (600 mg, 3.17 mmol) at 0 °C. The resulting solution was stirred for 4 h at RT. The reaction was then quenched by the addition of 10 mL of water. The resulting solution was diluted with 20 mL of EtOAc. The solids were filtered out. The resulting solution was extracted with 3x50 mL of ethyl acetate dried over anhydrous sodium sulfate and concentrated under vacuum. The residue was eluted from a silica gel column with ethyl acetate/petroleum ether (5:1). This resulted in 470 mg (78%) of the Intermediate 117 as a yellow solid. MS-ESI: 178 (M+1).

**Intermediate 117A**

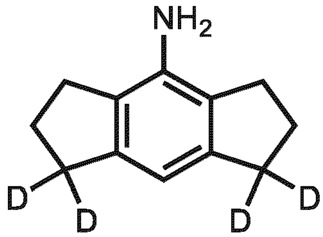

1,2,3,5,6,7-hexahydro-*s*-indacen-1,1,7,7-$d_4$-4-amine

**Intermediate 117A** was prepared starting from compound **317"** and using the same procedure as shown in **scheme 73** above for converting **compound 318"** to **intermediate 117.** MS-ESI: 178 (M+1).

**Table 15.** The Intermediates in the following Table were prepared using similar procedure as shown in **Scheme 30** above for converting compound **130"** to Intermediate **44.**

| Intermediate # | Structure | IUPAC Name |
|---|---|---|
| Intermediate 118" | 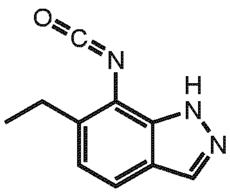 | 6-Ethyl-7-isocyanato-1H-indazole |
| Intermediate 119" | 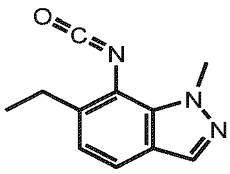 | 6-Ethyl-7-isocyanato-1-methyl-1H-indazole |
| Intermediate 120" | 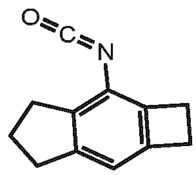 | 3-Isocyanato-2,4,5,6-tetrahydro-1H-cyclobuta[f]indene |
| Intermediate 121" | 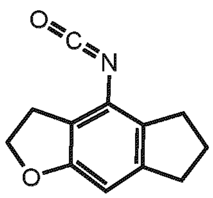 | 4-Isocyanato-3,5,6,7-tetrahydro-2H-indeno [5,6-b]furan |

(continued)

| Intermediate # | Structure | IUPAC Name |
|---|---|---|
| Intermediate 122" | 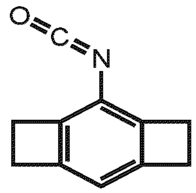 | 2-Isocyanatotricyclo[62.0.03,6]deca-1,3(6), 7-triene |
| Intermediate 123" | 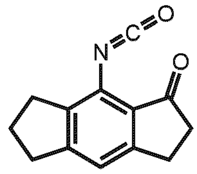 | 8-Isocyanato-2,3,6,7-tetrahydros-indacen-1 (5H)-one |
| Intermediate 124" | 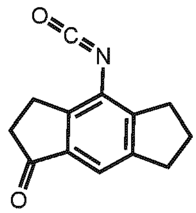 | 4-Isocyanato-2,3,6,7-tetrahydros-indacen-1 (5H)-one |
| Intermediate 125" | 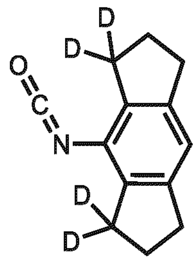 | 4-isocyanato-1,2,3,5,6,7-hexahydro-*s*-indacene-3,3,5,5-$d_4$ |
| Intermediate 126" | 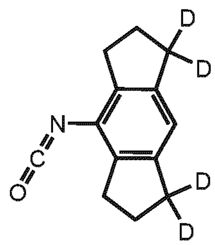 | 4-isocyanato-1,2,3,5,6,7-hexahydro-s-indacene-1,1,7,7-$d_4$ |

Schemes below the synthesis of sulfonimidamide **Intermediates 118-123.**

## Scheme 74:

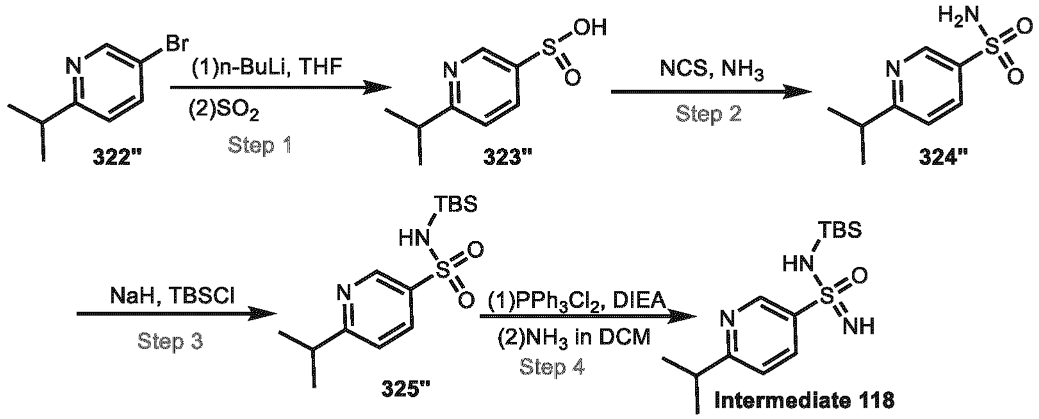


## Intermediate 118

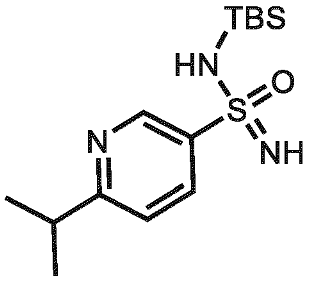

N-(tert-butyldimethylsilyl)-6-isopropylpyridine-3-sulfonimidamide

**Steps 1-4** used similar procedures for converting compound **245"** to Intermediate **88** shown in **Scheme 56** to afford Intermediate **118** from compound **322".** MS-ESI: 314 (M+1).

## Scheme 75:

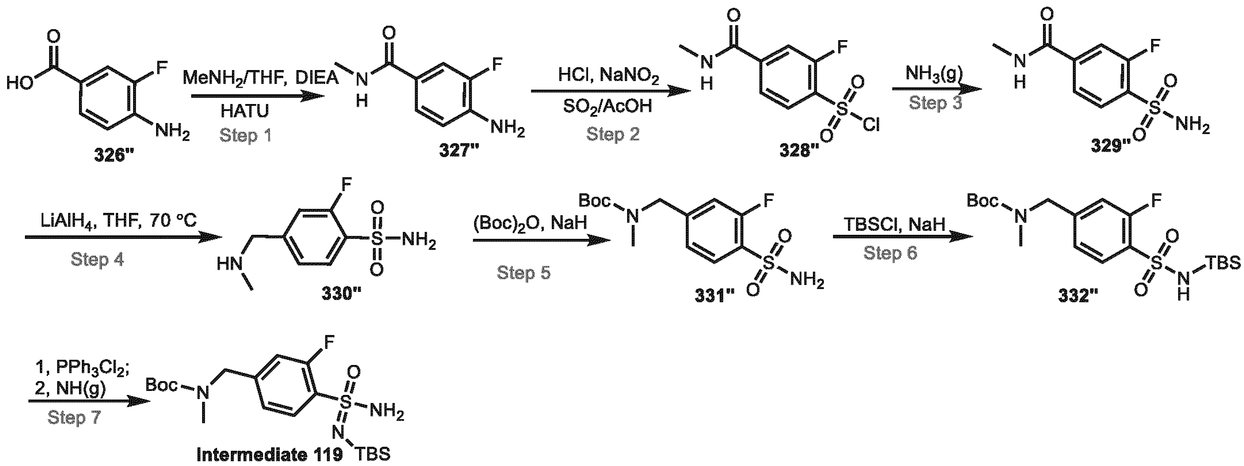

## Intermediate 119

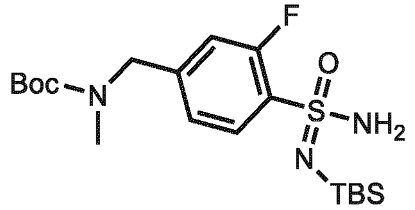

**Step 1: 4-Amino-3-fluoro-N-methylbenzamide**

Into a 500 mL round-bottom flask were added 4-amino-3-fluorobenzoic acid (15 g, 97 mmol) and DMF (100 mL) at RT. To the stirred solution was added HATU (74 mg, 0.19 mmol) and DIEA (25 mg, 0.19 mmol) at 0 °C. To the above mixture was added $MeNH_2$/THF (2M, 97 mL, 194 mmol) in one portion at 0 °C. The resulting mixture was stirred for additional 2 h at RT. The resulting mixture was extracted with EtOAc (3x100 mL). The combined organic layers were dried over anhydrous $Na_2SO_4$. After filtration, the filtrate was concentrated under reduced pressure. The residue was eluted from silica gel column with petroleum ether /EtOAc (1: 1) to afford the title compound (16 g, 98%) as yellow oil. MS-ESI: 169 (M+1).

**Steps 2-3** used similar procedures for converting compound **27** to Intermediate **29** shown in **Scheme 9** to afford compound **329"** from compound **327".** MS-ESI: 233 (M+1).

**Step 4: 2-Fluoro-4-((methylamino)methyl)benzenesulfonamide**

Into a 250-mL round-bottom flask were placed 3-fluoro-N-methyl-4-sulfamoylbenzamide (1.2 g) in THF (40 mL) at 0 °C. To the stirred solution was added $LiAlH_4$ (543 mg, 14 mmol) in portions at 0 °C under nitrogen atmosphere. The resulting mixture was stirred overnight at 70 °C. The reaction was quenched with water (2 mL). The resulting mixture was filtered, the filter cake was washed with EtOAc (3x10 mL). The filtrate was concentrated under reduced pressure. The residue was purified by Prep-TLC (EtOAc/MeOH = 25:1) to afford the title compound (800 mg, 77%) as a white solid. MS-ESI: 219 (M+1).

**Step 5: Tert-butyl (3-fluoro-4-sulfamoylbenzyl)(methyl)carbamate**

Into a 100-mL round-bottom flask were placed 2-fluoro-4-[(methylamino)methyl]benzene-1-sulfonamide (800 mg, 3.7 mmol) in THF (20 mL) at 0 °C. To a stirred solution was added $(Boc)_2O$ (1.5 g, 6.89 mmol) in portions at 0 °C. The resulting mixture was stirred for 2 h at RT and concentrated under reduced pressure. The residue was purified by Prep-TLC (PE/EtOAc 1:1) to afford the title compound (900 mg, 77%) as a white solid. MS-ESI: 319 (M+1).

**Steps 6-7** used similar procedures for converting compound **248"** to Intermediate **88** shown in **Scheme 56** to afford Intermediate **119** from compound **331".** MS-ESI: 432 (M+1).

## Scheme 76:

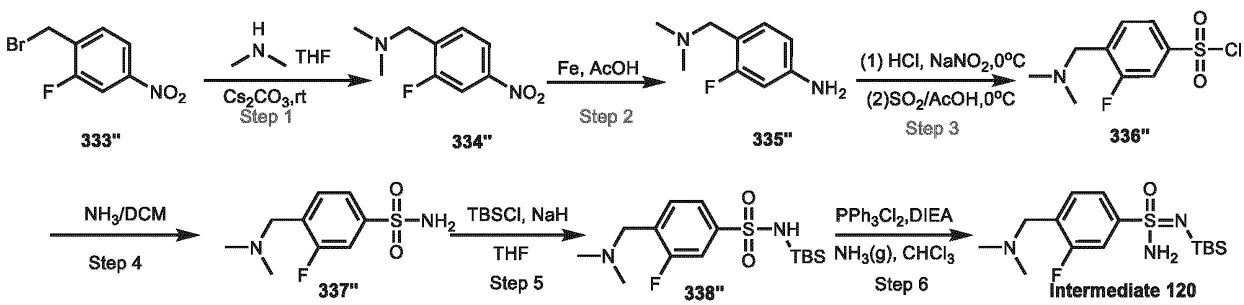

## Intermediate 120

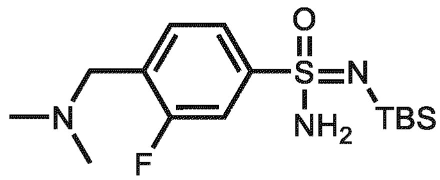

**N'-(tert-butyldimethylsilyl)-4-((dimethylamino)methyl)-3-fluorobenzenesulfonimidamide**

**Step 1: 1-(2-Fluoro-4-nitrophenyl)-N,N-dimethylmethanamine**

Into a 250-mL round-bottom flask, was placed a solution of 1-(bromomethyl)-2-fluoro-4-nitrobenzene (8.0 g, 34 mmol) in MeOH (50 mL). This was followed by the addition of dimethylamine (2 M, 21 mL) dropwise with stirring at 0 °C in 5 min. The resulting solution was stirred for 4 h at RT. The resulting mixture was concentrated under vacuum. This resulted in 7.0 g crude title compound as yellow oil. MS-ESI: 199 (M+1).

**Step 2: 4-((Dimethylamino)methyl)-3-fluoroaniline**

Into a 100-mL round-bottom flask, was placed the solution of [(2-fluoro-4-nitrophenyl)methyl]dimethylamine (7.0 g, 35 mmol) in AcOH (20 mL), to the stirred solution was added iron powder (10 g, 179 mmol). The resulting solution was stirred for 16 h at RT. The solids were filtered out. The resulting filtrate was concentrated under vacuum. The residue was eluted from a silica gel column with DCM/MeOH (9:1). This resulted in 6.5 g crude title compound as yellow oil. MS-ESI: 169 (M+1).

**Steps 3-4** used similar procedures for converting compound **145"** to compound **147"** shown in Scheme 36 to afford compound **337"** from compound **335".** MS-ESI: 233 (M+1).

**Steps 5-6** used similar procedures for converting compound **148"** to Intermediate 59 shown in **Scheme 36** to afford Intermediate **120** from compound **337".** MS-ESI: 233 (M+1).

## Scheme 77:

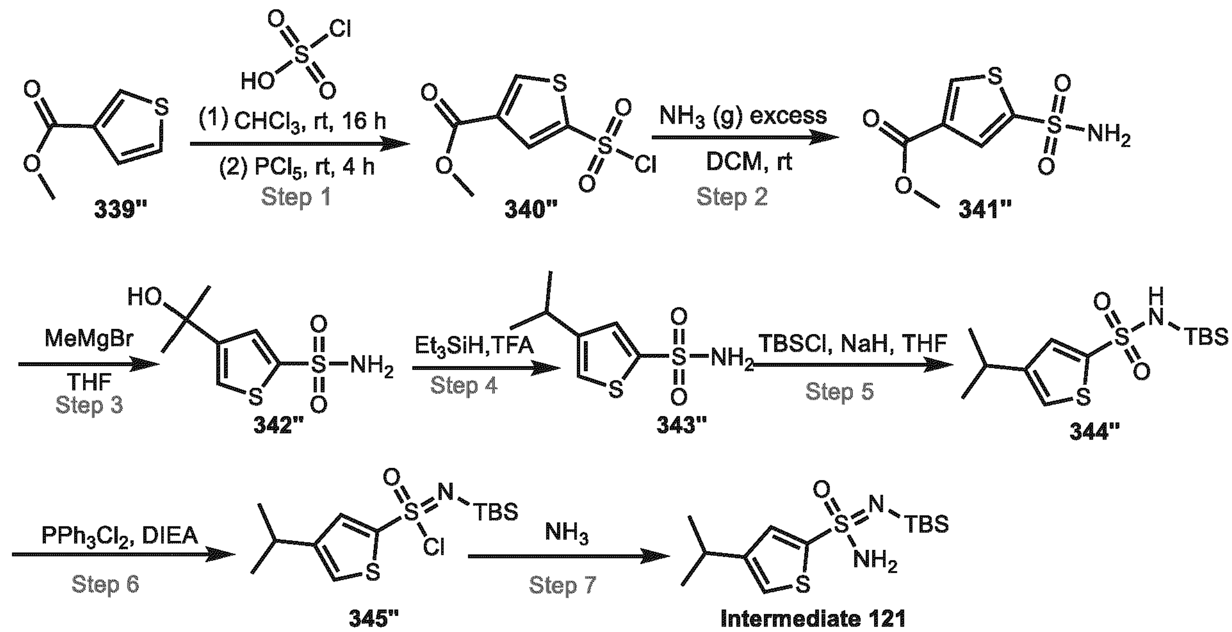


**339"** **340"** **341"** **342"** **343"** **344"** **345"** **Intermediate 121**

## Intermediate 121

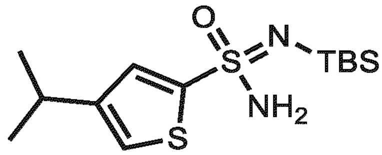

N'-(tert-butyldimethylsilyl)-4-isopropylthiophene-2-sulfonimidamide

**Steps 1-2** used similar procedures for converting compound **158"** to intermediate **61** shown in **Scheme 38** to afford compound **341"** from compound **339".** MS-ESI: 221 (M+1).

**Step 3** used similar procedures for converting compound **147"** to compound **148"** shown in **Scheme 36** to afford compound **342"** from compound **341".** MS-ESI: 221 (M+1).

**Step 4: 4-Isopropylthiophene-2-sulfonamide**

Into a 250-mL round-bottom flask, was placed the solution of 4-(2-hydroxypropan-2-yl)thiophene-2-sulfonamide (1.5 g, 6.79 mmol) in DCM (20 mL). To the stirred solution was added TFA (3.9 g, 34 mmol) and $Et_3SiH$ (2.32 g, 20 mmol). The result solution was stirred overnight at RT. The mixture was concentrated under vacuum. The residue was eluted from silica gel column with a gradient of ethyl acetate/petroleum ether (1:5 to 1:3). This resulted in 1.1 g (79%) of the title compound as a light yellow solid. MS-ESI: 206 (M+1).

**Steps 5-6** used similar procedures for converting compound **148"** to Intermediate **59** shown in **Scheme 36** to afford Intermediate **121** from compound **344".** MS-ESI: 319 (M+1).

## Scheme 78:

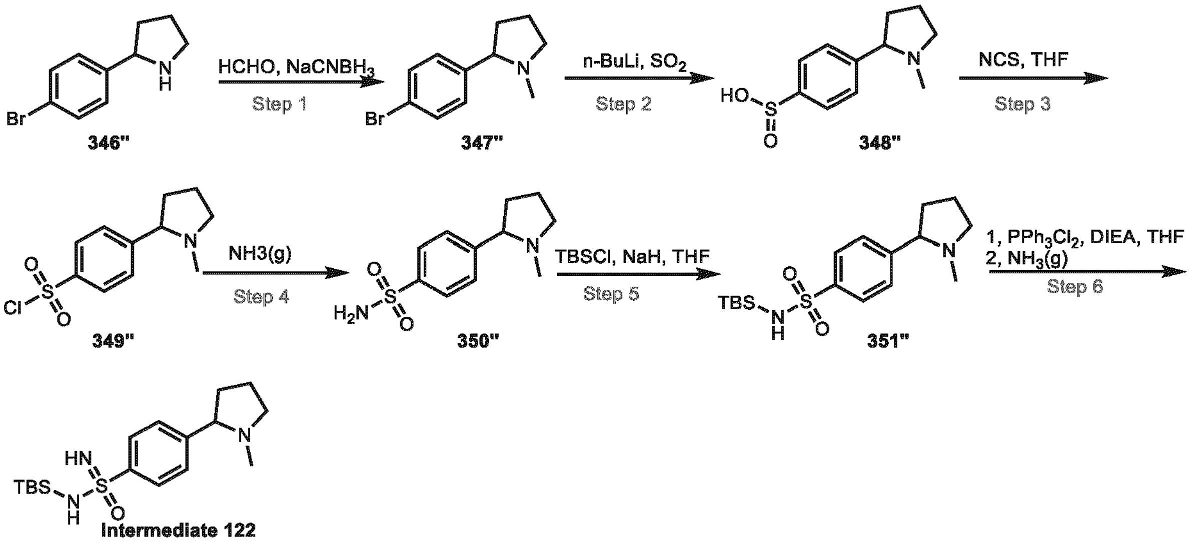


## Intermediate 122

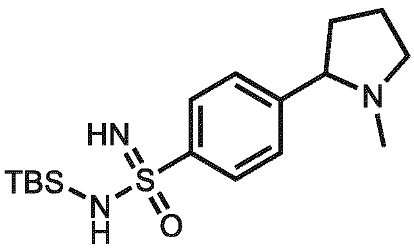

N-(tert-butyldimethylsilyl)-4-(1-methylpyrrolidin-2-yl)benzenesulfonimidamide

**Step 1: 2-(4-Bromophenyl)-1-methylpyrrolidine**

Into a 100-mL round-bottom flask, was placed 2-(4-bromophenyl)pyrrolidine (3.0 g, 13.3 mmol) in HCHO (3.23 g, 37% wt.), to the stirred solution was added $NaBH_3CN$ (2.5 g, 40 mmol). The resulting solution was stirred for 12 h at RT and concentrated under vacuum. The residue was eluted from a silica gel column with ethyl acetate/petroleum ether (1:1). This resulted in 2.8 g (88%) of the title compound as a light yellow solid. MS-ESI: 240/242 (M+1).

**Steps 2-6** used similar procedures for converting compound **245"** to Intermediate **88** shown in **Scheme 56** to afford Intermediate **122"** from compound **347".** MS-ESI: 354 (M+1).

## Scheme 79:

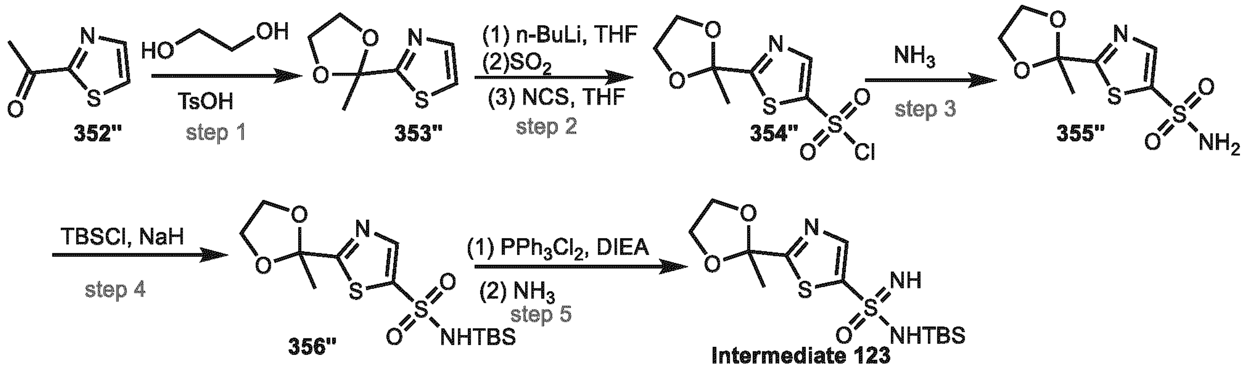


352" 353" 354" 355" 356" Intermediate 123

## Intermediate 123

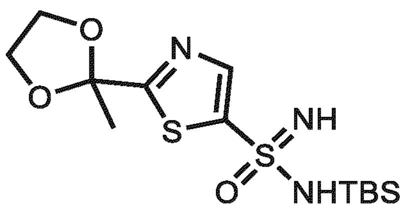

N-(tert-butyldimethylsilyl)-2-(2-methyl-1,3-dioxolan-2-yl)thiazole-5-sulfonimidamide

**Step 1: 2-(2-Methyl-1,3-dioxolan-2-yl)thiazole**

Into a 50-mL round-bottom flask purged and maintained with an inert atmosphere of nitrogen, was placed 1-(1,3-thiazol-2-yl)ethan-1-one (27 g, 212 mmol) in toluene (300 mL), to the stirred solution was added TsOH (2.0 g, 11.6 mmol) and ethane-1,2-diol (40 g, 644 mmol). The resulting solution was stirred for 14 h at 110 °C in an oil bath. The resulting mixture was concentrated under vacuum. The residue was eluted from a silica gel column with ethyl acetate/petroleum ether (1:20 to 1:10). This resulted in 36 g (99%) of the title compound as brown oil. MS-ESI: 172 (M+1).

**Steps 2-5** used similar procedures for converting compound **245"** to Intermediate **88** shown in **Scheme 56** to afford Intermediate **123** from compound **353".** MS-ESI: 363 (M+1).

## Scheme 80:

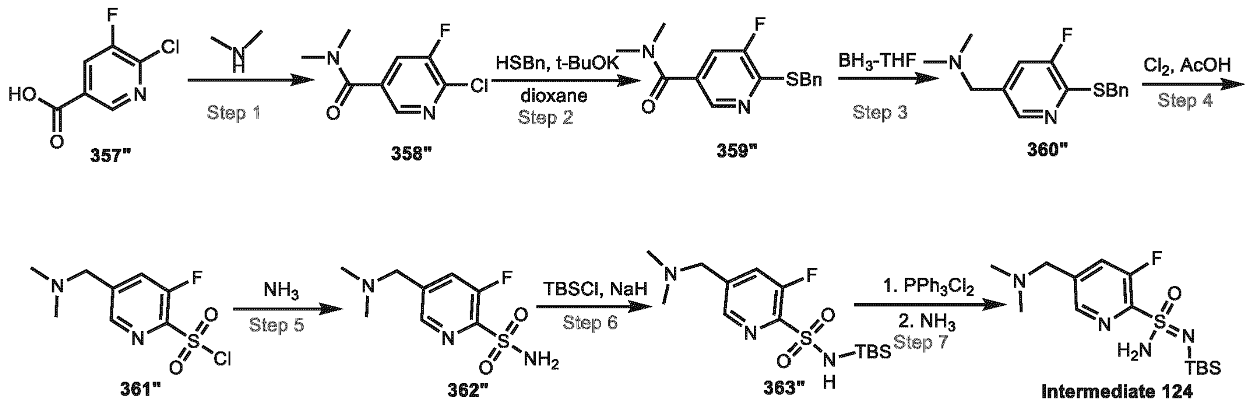


357" 358" 359" 360" 361" 362" 363" Intermediate 124

## Intermediate 124

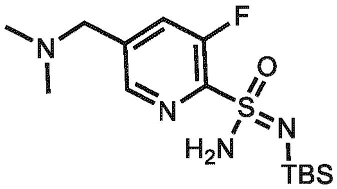

N'-(tert-butyldimethylsilyl)-5-((dimethylamino)methyl)-3-fluoropyridine-2-sulfonimidamide

**Step 1: 6-Chloro-5-fluoro-*N,N*-dimethylnicotinamide**

To a stirred solution of 6-chloro-5-fluoronicotinic acid (10 g, 49.5 mmol) in THF (150 mL) in a 250-mL round-bottom flask under nitrogen was added HATU (28.2 g, 74.3 mmol), DIEA (12.8 g, 99 mmol) and dimethylamine in THF (2 M, 75 mL, 149 mmol) at RT. The resulting solution was stirred for 16 h at RT. The reaction was quenched with water (400 mL). The resulting solution was extracted with 3x400 mL of EtOAc and the organic layers combined and dried over $Na_2SO_4$ and concentrated under vacuum. The residue was eluted from silica gel with EtOAc/PE (1:1). This resulted in 8.0 g (80%) of the title compound as a white solid. MS-ESI: 203/205 (M+1).

**Step 2: 6-(Benzylthio)-5-fluoro-*N,N*-dimethylnicotinamide**

To a stirred solution of 6-chloro-5-fluoro-N,N-dimethylnicotinamide (8.0 g, 39.4 mmol) in dioxane (160 mL) in a 100-mL round-bottom flask under nitrogen was added phenylmethanethiol (9.76 g, 78.8 mmol) and t-BuOK (8.84 g, 78.8 mmol). The resulting solution was stirred for 16 h at 80 °C. The resulting mixture was quenched with water (400 mL) and extracted with 3x500 mL of EtOAc. The organic layer was combined and dried over anhydrous $Na_2SO_4$. The solids were filtered out. The resulting filtrate was concentrated under vacuum. The residue was eluted from silica gel with EtOAc/PE (1:1). This resulted in 6.0 g (52%) of the title compound as yellow oil. MS-ESI: 291 (M+1).

**Step 3: 1-(6-(Benzylthio)-5-fluoropyridin-3-yl)-*N,N*-dimethylmethanamine**

To a stirred solution of 6-(benzylthio)-5-fluoro-N,N-dimethylnicotinamide (6.0 g, 20.7 mmol) in THF (100 mL) in a 250-mL round-bottom flask under nitrogen was added $BH_3$ in THF (1 M, 104 mmol, 104 mL) dropwise at 0 °C. The resulting solution was stirred for 16 h at RT. The reaction was quenched with MeOH (100 mL). The resulting mixture was concentrated under vacuum. The residue was eluted from silica gel with EtOAc/PE (1:1). This resulted in 4.4 g (77.1%) of the title compound as a white solid. MS-ESI: 277 (M+1).

**Step 4: 5-((Dimethylamino)methyl)-3-fluoropyridine-2-sulfonyl chloride**

To a stirred solution of 1-(6-(benzylthio)-5-fluoropyridin-3-yl)-N,N-dimethylmethanamine (4.4 g, 15.9 mmol) in DCM (30 mL), AcOH (15 mL) and $H_2O$ (15 mL) in a 250-mL 3-necked round-bottom flask under nitrogen was introduced $Cl_2$ gas bubbled at 0 °C for 30 min. Then the resulting solution was stirred for another 30 min at 0 °C. The resulting mixture diluted with water (100 mL) and extracted with 3x100 mL DCM. The organic layers were combined and washed

with brine (100 mL), then dried over anhydrous $Na_2SO_4$. This resulted in the title compound in DCM solution which was used for next step directly.

**Step 5: 5-((Dimethylamino)methyl)-3-fluoropyridine-2-sulfonamide**

To a stirred solution of 5-((dimethylamino)methyl)-3-fluoropyridine-2-sulfonyl chloride (crude) in DCM (300 mL) was introduced $NH_3$ gas bubbled for 30 min at 0 °C. The resulting solution was stirred for 2 h at RT. The resulting mixture was concentrated under vacuum. The residue was eluted from silica gel with EtOAc/PE (1:1). This resulted in 1.56 g (42% over two steps) of the title compound as a white solid. MS-ESI: 234 (M+1).

**Step 6: *N*-(tert-butyldimethylsilyl)-5-((dimethylamino)methyl)-3-fluoropyridine-2-sulfonamide**

To a stirred solution of 5-((dimethylamino)methyl)-3-fluoropyridine-2-sulfonamide (233 mg, 1.0 mmol) in THF (10 mL) in a 50-mL round-bottom flask under nitrogen was added NaH (60% dispersion in mineral oil, 120 mg, 3.0 mmol) at 0 °C, to this was added TBSCl (227 mg, 1.50 mmol) at 0 °C. The resulting solution was stirred for 2 h at RT. The resulting mixture was quenched with 10 mL of water. The resulting solution was extracted with 3x10 mL of EtOAc and dried over anhydrous $Na_2SO_4$. The solids were filtered out. The resulting mixture was concentrated under vacuum. The residue was eluted from silica gel with EtOAc/PE (1:1). This resulted in 200 mg (57.5%) of the title compound as a yellow solid. MS-ESI: 348 (M+1).

**Step 7: *N'*-(tert-butyldimethylsilyl)-5-((dimethylamino)methyl)-3-fluoropyridine-2-sulfonimidamide**

To a stirred solution of $PPh_3Cl_2$ (383 mg, 1.15 mmol) in $CHCl_3$ (10 mL) in a 100-mL 3-necked round-bottom flask under nitrogen was added DIEA (371 mg, 2.88 mmol) dropwise with stirring at 0°C. The resulting solution was stirred for 10 min at RT. To this was added a solution of *N*-(tert-butyldimethylsilyl)-5-((dimethylamino)methyl)-3-fluoropyridine-2-sulfonamide (200 mg, 0.58 mmol) in $CHCl_3$ (10 mL) dropwise with stirring at 0°C. The resulting solution was allowed to react, with stirring, for an additional 30 min while the temperature was maintained at 0 °C in a water/ice bath. To the above $NH_3$(g) was bubbled for 10 min at 0°C. The resulting solution was allowed to react, with stirring, for an additional 30 min at RT. The solids were filtered out. The resulting mixture was concentrated under vacuum. The residue was eluted from silica gel with EtOAc/PE (1:1). This resulted in 150 mg (75.0%) of the title compound as an off-white solid. MS-ESI: 347 (M+1).

**Scheme 81:**

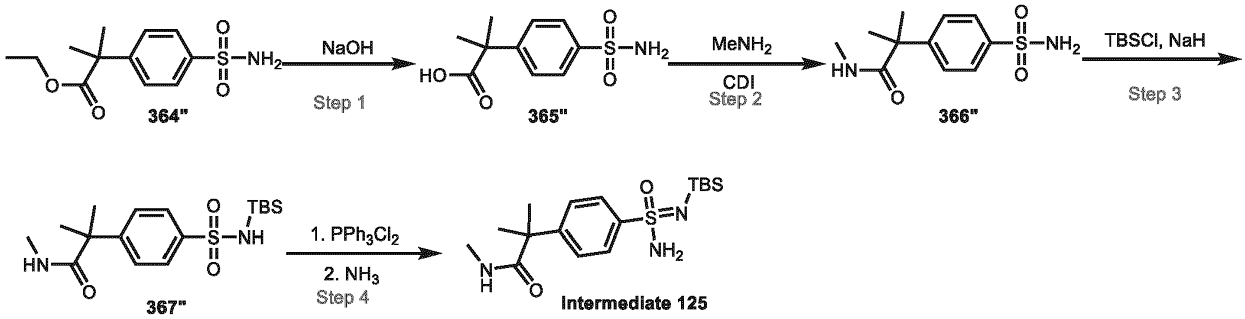


## Intermediate 125

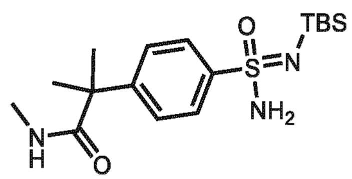

2-(4-(*N'*-(*tert*-butyldimethylsilyl)sulfamidimidoyl)phenyl)-N,2-dimethylpropanamide

**Step 1: 2-Methyl-2-(4-sulfamoylphenyl)propanoic acid**

To a stirred solution of ethyl 2-methyl-2-(4-sulfamoylphenyl) propanoate (5.42 g, 20.0 mmol) in MeOH (60 mL) in a 250-mL round-bottom flask was added the solution of NaOH (8.0 g, 200 mmol) in water (40 mL) dropwise at RT. The resulting solution was stirred for 2 h at 55 °C and the organic solvent was removed under vacuum. The pH value of the solution was adjusted to 1-2 with HCl (2 M). The resulting solution was extracted with 3x200 mL of DCM and the organic layers were combined and dried over $Na_2SO_4$, concentrated under vacuum. This resulted in 3.90 g (80.2%) of the title compound as a light yellow solid. MS-ESI: 242 (M-1)

**Step 2: *N*,2-dimethyl-2-(4-sulfamoylphenyl)propanamide**

To a stirred solution of 2-methyl-2-(4-sulfamoylphenyl)propanoic acid (2.43 g, 10.0 mmol) in THF (60 mL) in a 250-mL round-bottom flask under nitrogen was added CDI (1.94 g, 12.0 mmol) in portions at RT. The resulting solution was stirred for 30 min at RT. Then the reaction was stirred for 30 min at 60 °C. Methanamine in THF (2 M) (40 mL, 80.0 mmol) was added dropwise at RT. The resulting solution was stirred for 16 h at RT. The reaction was then quenched by the addition of 90 mL of water. The resulting solution was extracted with 3x120 mL of DCM and the organic layers were combined and dried over anhydrous $Na_2SO_4$. The solids were filtered out. The resulting mixture was concentrated under vacuum. The residue was eluted from silica gel with EtOAc/PE (1: 1). This resulted in 1.80 g (70.3%) of the title compound as yellow solid. MS-ESI: 257 (M+1).

Steps 3-4 used similar procedures for converting compound **362"** to intermediate **124** shown in Scheme 80 to afford intermediate **125** from compound **366".** MS-ESI: 370 (M+1).

## Scheme 82:

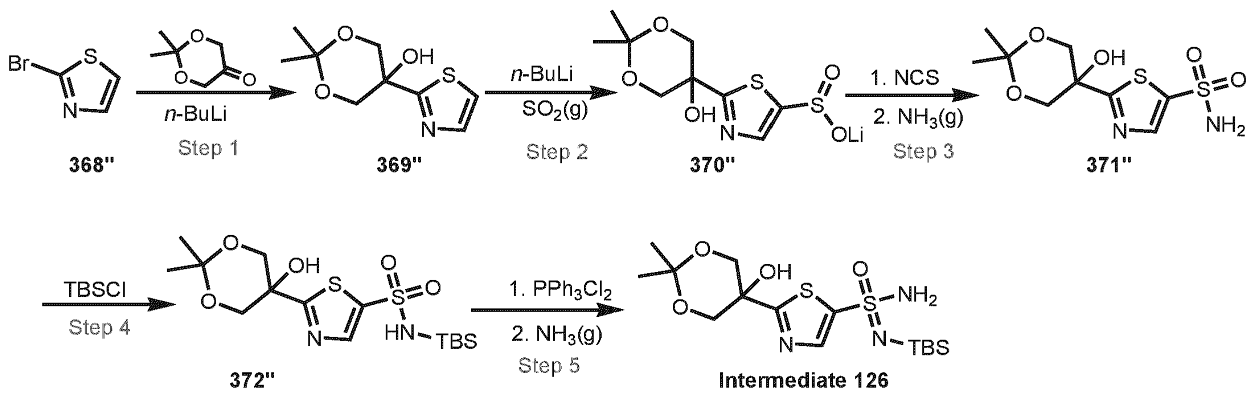


## Intermediate 126

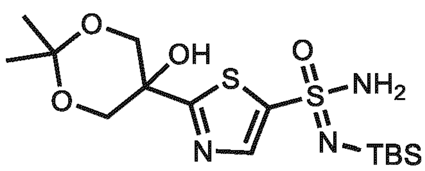

*N'*-(*tert*-bulyldimethylsilyl)-2-(5-hydroxy-2,2-dimethyl-1,3-dioxan-5-yl)thiazole-5-sulfonimidamide

**Step 1: 2,2-Dimethyl-5-(thiazol-2-yl)-1,3-dioxan-5-ol**

To a stirred solution of 2-bromothiazole (4.89 g, 30.0 mmol) in THF (200 mL) in a 500-mL 3-necked round-bottom flask under nitrogen was added n-BuLi/hexane (2.5 M, 12 mL, 30.0 mmol) dropwise with stirring at -78 °C. The resulting solution was stirred for 30 min at -78 °C. Then 2,2-dimethyl-1,3-dioxan-5-one (3.90 g, 30.0 mmol) in THF (10 mL) was added dropwise at -70 °C. The resulting solution was allowed to react, with stirring, for an additional 30 min at

RT. The resulting mixture was quenched with 20 mL of MeOH and concentrated under reduced pressure. The residue was eluted from silica gel with EtOAc/PE (1/10). This resulted in 3.20 g (49.6%) of the title compound as a yellow solid. MS-ESI: 216 (M+1).

**Step 2: Lithium 2-(5-hydroxy-2,2-dimethyl-1,3-dioxan-5-yl)thiazole-5-sulfinate**

To a stirred solution of 2,2-dimethyl-5-(thiazol-2-yl)-1,3-dioxan-5-ol (2.15 g, 10.0 mmol) in THF (100 mL) in a 250-mL 3-necked round-bottom flask under nitrogen was n-BuLi/hexane (2.5 M, 4.0 mL, 10.0 mmol) dropwise with stirring at -70 °C. The resulting solution was stirred for 60 min at -70 °C. Then $SO_2$ (g) was bubbled to the solution at -50 °C for 10 min. The resulting solution was allowed to react, with stirring, for an additional 30 min at 20 °C. The resulting mixture was concentrated under reduced pressure. This resulted in 2.5 g (crude) of the title compound as an off-white solid. MS-ESI: 278 (M-1).

**Step 3: 2-(5-hydroxy-2,2-dimethyl-1,3-dioxan-5-yl)thiazole-5-sulfonamide**

To a stirred solution of lithium 2-(5-hydroxy-2,2-dimethyl-1,3-dioxan-5-yl)thiazole-5-sulfinate (2.5 g, crude) in DCM (100 mL) in a 250-mL round-bottom flask was added NCS (2.67 g, 20.0 mmol) in small portions at RT. The resulting solution was stirred for 2 h at RT.

The resulting solution was diluted with 50 mL of water, then extracted with 3x50 mL of DCM and the organic layers were combined and dried over anhydrous $Na_2SO_4$. Then $NH_3$ (g) was bubbled into reaction mixture for 10 min at 0 °C. The resulting solution was stirred for 2 h at RT. The resulting mixture was concentrated under vacuum. The residue was eluted from silica gel with EtOAc/PE (1/3). This resulted in 1.80 g (61.2% over two steps) of the title compound as a yellow solid. MS-ESI: 293 (M-1).

Step 4-5 used similar procedures for converting compound **362"** to intermediate **124** shown in Scheme 80 to afford intermediate **126** from compound **371"**. MS-ESI: 408 (M+1).

**Scheme 83:**

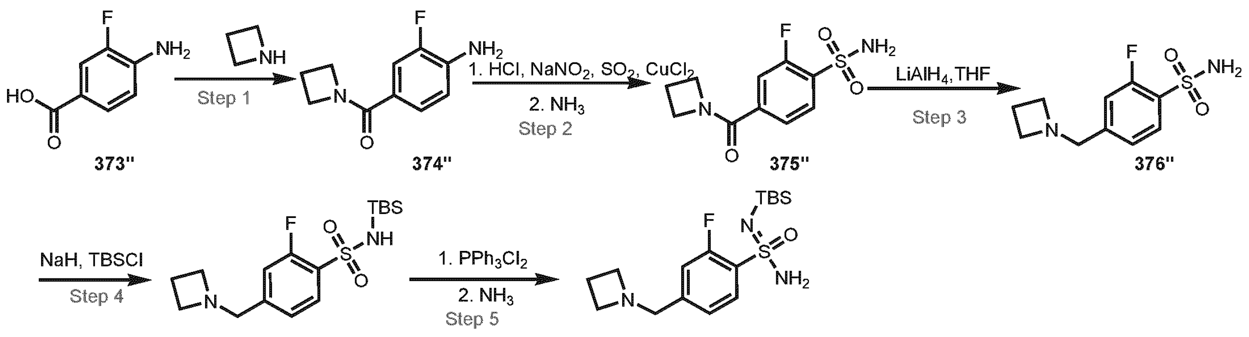


373" 374" 375" 376" 377" Intermediate 127

## Intermediate 127

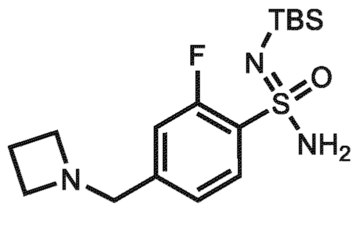

4-(Azetidin-1-ylmethyl)-N'-(tert-bulyldimethylsilyl)-2-fluorobenzenesulfonimidamide

**Step 1: (4-Amino-3-fluorophenyl)(azetidin-1-yl)methanone**

To a stirred solution of 4-amino-3-fluorobenzoic acid (5.0 g, 32.3 mmol) in DMF (50 mL) in a 250 mL round-bottom flask was added DIEA (8.33 g, 64.6 mmol) and HATU (24.5 g, 64.6 mmol) at 0°C. This was followed by the addition of azetidine (3.68 g, 64.6 mmol) in DMF (10 mL) dropwise with stirring at 0°C. The resulting mixture was stirred

for 16 h at RT. The reaction was then quenched by the addition of water (100 mL). The resulting mixture was extracted with EtOAc (5 × 100 mL). The organic layers were combined and dried over anhydrous $Na_2SO_4$ and concentrated under vacuum. The residue was eluted from silica gel with EtOAc/PE (1/2) to afford the title compound (5.71 g, 91.2%) as a light yellow solid. MS-ESI: 195 (M+1).

**Step 2: 4-(Azetidine-1-carbonyl)-2-fluorobenzenesulfonamide**

To a stirred solution of (4-amino-3-fluorophenyl)(azetidin-1-yl)methanone (3.88 g, 20.0 mmol) in HCl (6 M, 30 mL) in a 250-mL 3-necked round-bottom flask under nitrogen was added a solution of $NaNO_2$ (1.66 g, 24.0 mmol) in $H_2O$ (5 mL) dropwise with stirring at 0 °C, the solution was stirred for 30 min, this solution was assigned as solution A. Then $CuCl_2$ (1.34 g, 10.0 mmol) was added to a 250-mL single necked round-bottom flask with AcOH (40 mL) as the solvent. Then $SO_2$ (g) was bubbled to the reaction mixture with stirring at RT for 20 min, this solution was assigned as solution B. To the solution B was added solution A dropwise with stirring at 0 °C. The resulting solution was stirred for 2 h at RT. The reaction mixture was diluted with 100 mL of water, extracted with 3x100 mL of DCM and the organic layers were combined and dried over anhydrous $Na_2SO_4$. To the crude DCM solution (~300 mL) was bubbled $NH_3$ (g) with stirring at 0 °C for 10 min. The resulting solution was stirred for 2 h at RT. The solution was concentrated under vacuum. The crude product was eluted from silica gel with EtOAc/PE (1:1). This resulted in 2.61 g (50.6%) of the title compound as yellow oil. MS-ESI: 257 (M-1).

**Step 3: 4-(Azetidin-1-ylmethyl)-2-fluorobenzenesulfonamide**

To a stirred solution of 4-(azetidine-1-carbonyl)-2-fluorobenzenesulfonamide (1.0 g, 3.88 mmol) in THF (20 mL) was added $LiAlH_4$ (441 mg, 11.6 mmol) in portions at 0 °C. The resulting mixture was stirred for 16 h at 70 °C. The reaction was quenched with 10 g of solid $Na_2SO_4 \cdot 10H_2O$ at 0 °C. The resulting mixture was filtered and the filter cake was washed with EtOAc (2 x 50 mL). The filtrate was concentrated under reduced pressure. The residue was eluted from silica gel with EtOAc/PE (1:1) to afford the title compound (613 mg, 64.8%) as a light yellow solid. MS-ESI: 245 (M+1).

Steps 4-5 used similar procedures for converting compound **362"** to intermediate **124** shown in Scheme 80 to afford intermediate **127** from compound **376".** MS-ESI: 358 (M+1).

**Scheme 84:**

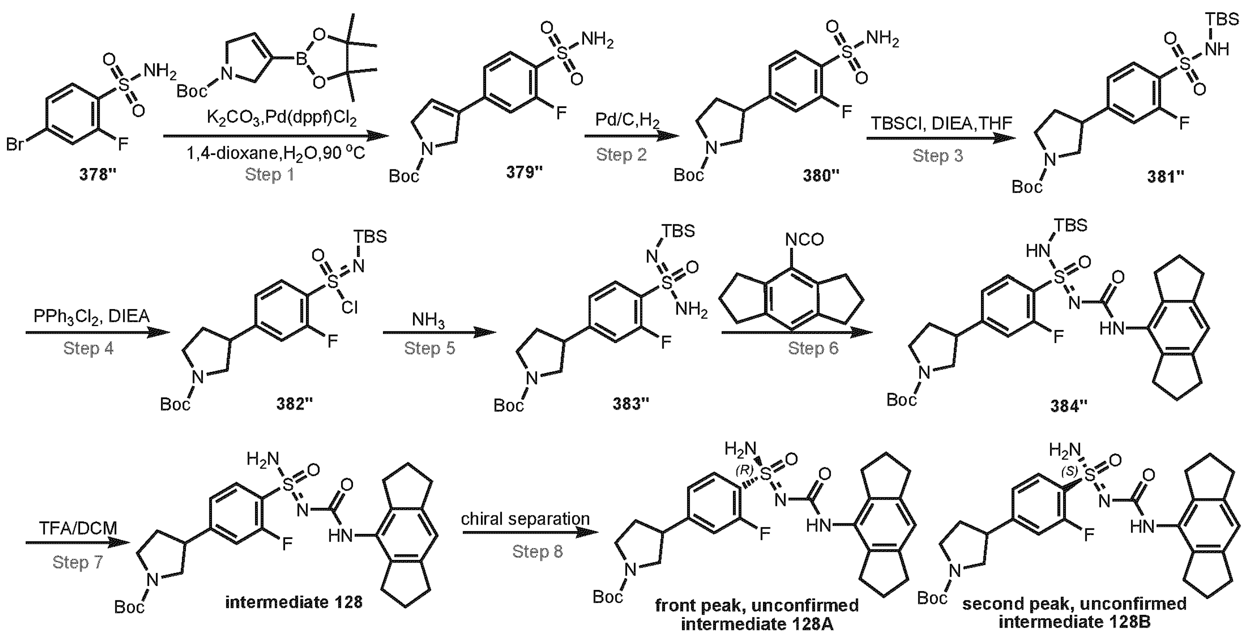

## Intermediate 128

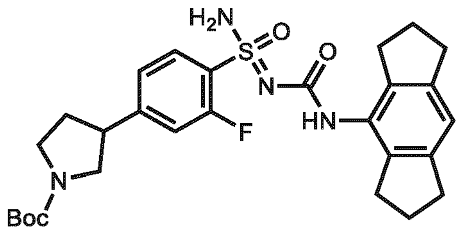

Tert-butyl 3-(3-fluoro-4-(N'-((1,2,3,5,6,7-hexahydro-s-indacen-4-yl)carbamoyl)sulfamidimidoyl)phenyl)-pyrrolidine-1-carboxylate

**Step 1: *Tert*-butyl 3-(3-fluoro-4-sulfamoylphenyl)-2,5-dihydro-1H-pyrrole-1-carboxylate**

To a stirred solution of 4-bromo-2-fluorobenzenesulfonamide (20.0 g, 78.7 mmol) in 1,4-dioxane/$H_2O$ (5:1) (200 mL) in a 250-mL 3-necked round-bottom flask under nitrogen was added tert-butyl 3-(4,4,5,5-tetramethyl-1,3,2-dioxaborolan-2-yl)-2,5-dihydro-1H -pyrrole-1-carboxylate (27.9 g, 94.4 mmol) then $K_2CO_3$ (21.8 g, 157 mmol) and Pd(dppf)$Cl_2$ (11.5 g, 15.7 mmol) were added to the solution at RT. The resulting solution was stirred for 16 h at 90 °C. The insoluble matter was filtered out and the filtrate was concentrated under reduced pressure. The residue was eluted from silica gel with EtOAc/PE (1/3) to give the title compound (7.32 g, 27.2%) as white solid. MS-ESI: 343 (M+1).

**Step 2: *Tert*-butyl 3-(3-fluoro-4-sulfamoylphenyl)pyrrolidine-1-carboxylate**

To a stirred solution of tert-butyl 3-(3-fluoro-4-sulfamoylphenyl)-2,5-dihydro-1H-pyrrole-1-carboxylate (7.30 g, 21.3 mmol) in MeOH (200 mL) in a 500 mL round-bottom flask under nitrogen was added Pd/C (10% wt. 1.0 g) in portions. The flask was evacuated and refilled three times with hydrogen. The resulting solution was stirred for 2 h at RT under atmosphere of hydrogen with a balloon. The solid was filtered out. The resulting mixture was concentrated under reduced pressure. The residue was eluted from silica gel with EtOAc/PE (1:2) to give the title compound (6.30 g, 86.0%) as white solid. MS-ESI: 345 (M+1).

Steps 3-5 used similar procedures for converting compound **362"** to intermediate **124** shown in Scheme 80 to afford compound **383"** from compound **380".** MS-ESI: 458 (M+1).

**Step 6: *Tert*-Butyl 3-(4-(N-(tert-butyldimethylsilyl)-N'-((1,2,3,5,6,7-hexahydro-s-indacen-4-yl) carbamoyl)sulfamidimidoyl)-3-fluorophenyl)pyrrolidine-1-carboxylate**

To a stirred solution of tert-butyl 3-(4-(N'-(tert-butyldimethylsilyl)sulfamidimidoyl)-3-fluorophenyl)- pyrrolidine-1-carboxylate (5.2 g, 11.3 mmol) in THF (100 mL) in a 100-mL 3-necked round-bottom purged with and maintained under nitrogen was added NaH (60% dispersion in mineral oil, 820 mg, 34 mmol) in portions at 0 °C. The reaction mixture was stirred for 15 min at RT. 4-isocyanato-1,2,3,5,6,7-hexahydro- s-indacene (2.7 g, 13.6 mmol) in THF (10 mL) was added dropwise to the solution at 0 °C. The resulting solution was stirred for 60 min at RT. The resulting mixture was quenched with 5 mL of water, then concentrated under reduced pressure. The residue was eluted from silica gel with EtOAc/PE (1:6) to give title compound (5.1 g, 69%) as white solid. MS-ESI: 657 (M+1).

**Step 7: Tert-Butyl 3-(3-fluoro-4-(N'-((1,2,3,5,6,7-hexahydro-s-indacen-4-yl)carbamoyl) sulfamidimidoyl)phenyl)pyrrolidine-1-carboxylate**

To a stirred solution of tert-butyl 3-(4-(N-(tert-butyldimethylsilyl)-N'- ((1,2,3,5,6,7-hexahydro-s-indacen-4-yl)carbamoyl)sulfamidimidoyl)-3-fluorophenyl)pyrrolidine-1 -carboxylate (5.1 g, 7.8 mmol) in DCM (100 mL) in a 500 mL round-bottom flask was added TFA (5 mL) dropwise at 0 °C. The resulting solution was stirred for 60 min at RT. The resulting mixture was concentrated under reduced pressure. The residue was eluted from silica gel with EtOAc/PE (1:8) to give the title compound (1.2 g, 35 %) as white solid. MS-ESI: 543 (M+1).

## Intermediate 128A and 128B

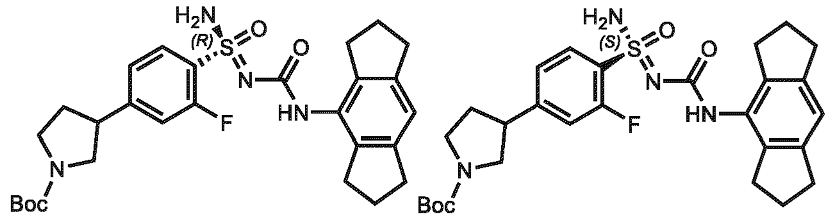

## Intermediate 128A and 128B (stereochemistry not assigned)

(R)- and (S)-*Tert*-butyl 3-(3-fluoro-4-(N'-((1,2,3,5,6,7-hexahydro-*s*-indacen-4-yl)carbamoyl)sulfamidimidoyl)-phenyl)pyrrolidine-1-carboxylate

**Step 8: Tert-butyl 3-(3-fluoro-4-((R)-N'-((1,2,3,5,6,7-hexahydro-s-indacen-4-yl)carbamoyl) sulfamidimidoyl)phenyl)pyrrolidine-1-carboxylate and tert-butyl 3-(3-fluoro-4-((S)-N'-((1,2,3,5,6,7-hexahydro-*s*-indacen-4-yl)carbamoyl)sulfamidimidoyl)phenyl)pyrrolidine-1-carboxylate**

Tert-Butyl 3-(3-fluoro-4-(N'-((1,2,3,5,6,7-hexahydro-s-indacen-4-yl)carbamoyl)sulfamidimidoyl)phenyl) pyrrolidine-1-carboxylate (1.2 g) was resolved by Prep chiral SFC with the following conditions: Column: (R,R)Whelk, 21.1*250 mm, 5 um; Mobile Phase A: $CO_2$, Mobile Phase B: MeOH; Flow rate: 40 mL/min; Gradient: 40% B; 220 nm; Rti:7.14 min **(Intermediate 128A,** 480 mg, 98%de); $Rt_2$:10.3 min **(Intermediate 128B,** 500 mg, 98%de) both as an off white solid. MS-ESI: 543 (M+1).

## Scheme 85:

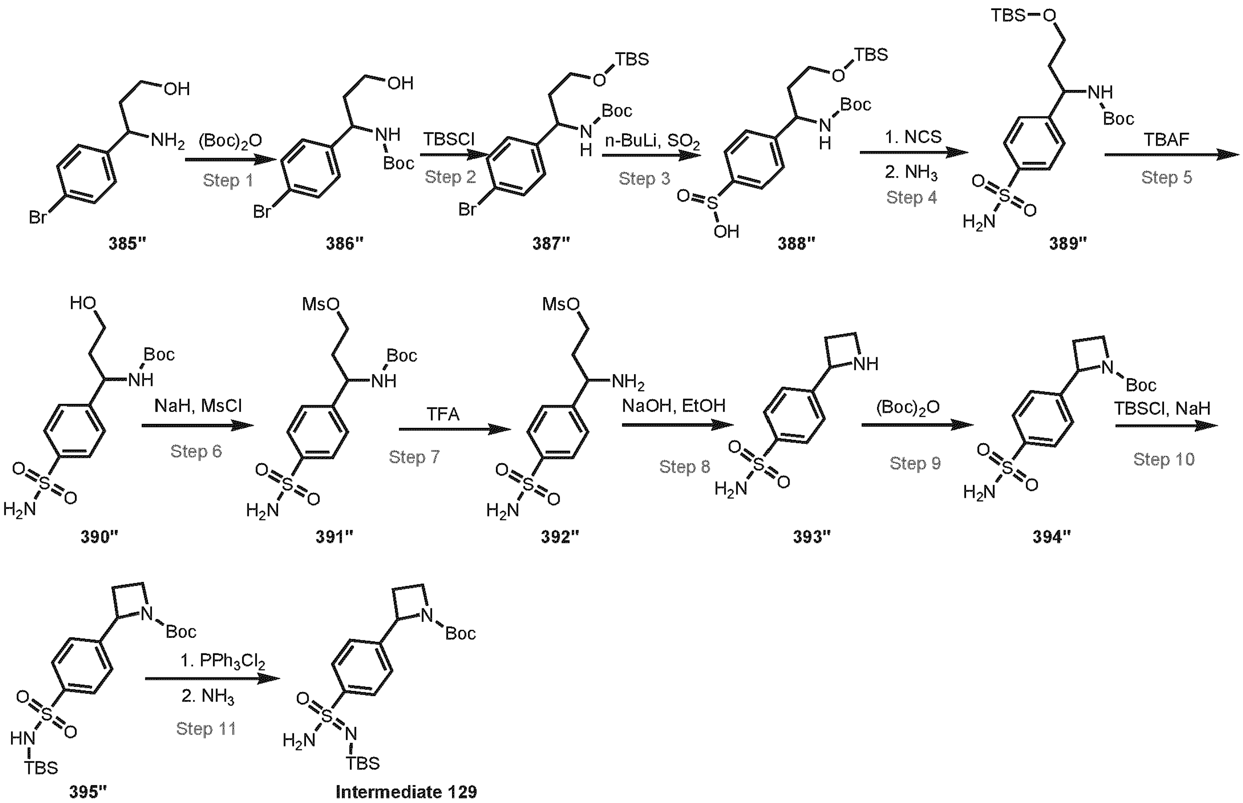

## Intermediate 129

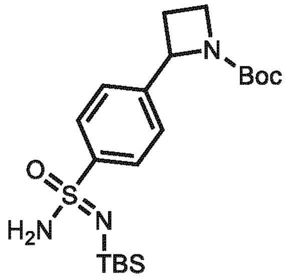

*Tert-butvl* 2-(4-(N'-(tert-butyldimethylsilyl)sulfamidimidoyl)phenyl)azetidine-1-carboxylate

**Step 1: Tert-butyl (1-(4-bromophenyl)-3-hydroxypropyl)carbamate**

To a stirred solution of 3-amino-3-(4-bromophenyl)propan-1-ol (6.90 g, 30.0 mmol) in THF (100 mL) in a 250-mL round-bottom flask under nitrogen was added NaH (60% dispersion in mineral oil, 1.80 g, 45.0 mmol) in portions at 0°C. The resulting solution was stirred for 20 min at RT. This was followed by the addition of $(Boc)_2O$ (9.81 g, 45.0 mmol) in THF (10 mL) dropwise with stirring at 0°C. The resulting solution was stirred for 16 h at RT. The reaction was then quenched by the addition of 50 mL of water. The resulting solution was extracted with 3x100 mL of EtOAc. The organic layers were combined and dried over anhydrous $Na_2SO_4$ and concentrated under vacuum. The crude product was eluted from silica gel with EtOAc/PE (1:4). This resulted in 8.43 g (85.2%) of the title compound as a yellow solid. MS-ESI: 330/332 (M+1).

**Step 2: *Tert-butyl* (1-(4-bromophenyl)-3-((tert-butyldimethylsilyl)oxy)propyl)carbamate**

To a stirred solution of tert-butyl (1-(4-bromophenyl)-3-hydroxypropyl)carbamate (6.60 g, 20.0 mmol) in THF (100 mL) in a 250-mL round-bottom flask under nitrogen was added NaH (60% dispersion in mineral oil, 1.20 g, 30.0 mmol) in portions at 0°C. The resulting solution was stirred for 20 min at RT. This was followed by the addition of TBSCl (4.53 g, 30.0 mmol) in THF (10 mL) dropwise with stirring at 0 °C. The resulting solution was stirred for 2 h at RT. The reaction was then quenched by the addition of 50 mL of water. The resulting solution was extracted with 3x100 mL EtOAc. The organic layers were combined and dried over anhydrous $Na_2SO_4$ and concentrated under vacuum. The crude product was eluted from silica gel with EtOAc/PE (1:5). This resulted in 7.81 g (87.9%) of the title compound as a yellow solid. MS-ESI: 444/446 (M+1).

Steps 3-4 used similar procedures for converting compound **369"** to compound **371"** shown in Scheme 82 to afford compound **389"** from compound **387".** MS-ESI: 445 (M+1).

**Step 5: *Tert-butyl* (3-hydroxy-1-(4-sulfamoylphenyl)propyl)carbamate**

To a stirred solution of tert-butyl (3-((*tert*-butyldimethylsilyl)oxy)-1 -(4-sulfamoylphenyl)propyl)carbamate (1.50 g, 3.37 mmol) in THF (30 mL) in a 100-mL round-bottom flask was added TBAF (1.76 g, 6.74 mmol) in several batches at RT. The resulting solution was stirred for 30 min at RT. The reaction was then quenched by the addition of 50 mL of water. The resulting solution was extracted with 3x150 mL of EtOAc and the organic layers were combined and dried over $Na_2SO_4$ and concentrated under reduced pressure. The residue was eluted from silica gel with DCM/MeOH (20/1). This resulted in 896 mg (80.6%) of the title compound as a yellow solid. MS-ESI: 331 (M+1).

**Step 6: 3-((Tert-butoxycarbonyl)amino)-3-(4-sulfamoylphenyl)propyl methanesulfonate**

To a stirred solution of *tert*-butyl (3-hydroxy-1-(4-sulfamoylphenyl)propyl) carbamate (660 mg, 2.00 mmol) in DCM (20 mL) in a 100-mL round-bottom flask under nitrogen was added TEA (404 mg, 4.00 mmol) at RT, then methanesulfonyl chloride (230 mg, 2.00 mmol) dropwise at 0 °C. The resulting solution was stirred for 4 h at 0 °C. The reaction was then quenched by the addition of 10 mL of water/ice. The resulting solution was extracted with 3x150 mL of EtOAc and the organic layers were combined and dried over anhydrous $Na_2SO_4$ and concentrated. To the residue was added 10 mL of EtOAc/PE (1:5) and the turbid solution was sonicated for 5 min. The product was collected by filtration. This resulted in 635 mg (77.8%) of the title compound as a white solid. MS-ESI: 409 (M+1).

**Step 7: 3-amino-3-(4-sulfamoylphenyl)propyl methanesulfonate**

To a stirred solution of 3-((*tert*-butoxycarbonyl)amino)-3-(4-sulfamoylphenyl)propyl methanesulfonate (635 mg, 1.56 mmol) in DCM (15 ml) in a 100-mL round-bottom flask was added TFA (5 ml) dropwise at RT. The resulting solution was stirred for 16 h at RT. The resulting mixture was concentrated under vacuum. The crude product was used in the next step directly without further purification. This resulted in 500 mg (crude) of the title compound as yellow oil. MS-ESI: 309 (M+1).

**Step 8: 4-(Azetidin-2-yl)benzenesulfonamide**

To a stirred solution of 3-amino-3-(4-sulfamoylphenyl)propyl methanesulfonate (500 mg, crude) in 2-methyl-2-propanol (20 mL) in a 100-mL round-bottom flask. The pH value of the solution was adjusted to 12 with NaOH (4 M). The resulting solution was stirred for 4 h at 60 °C. The resulting solution was extracted with 3x50 mL of EtOAc and the organic layers were combined and dried over anhydrous $Na_2SO_4$ and concentrated. The product was used in the next step directly without further purification. This resulted in 800 mg (crude) of the title compound as yellow oil. MS-ESI: 213 (M+1).

**Step 9: Tert-butyl 2-(4-sulfamoylphenyl)azetidine-1-carboxylate**

To a stirred solution of 4-(azetidin-2-yl)benzenesulfonamide (800 mg, crude) in THF (10 mL) in a 50-mL round-bottom flask was added TEA (253 mg, 2.50 mmol), this was followed by the addition of $(Boc)_2O$ (545 mg, 2.50 mmol) in THF (5 mL) dropwise at RT. The resulting solution was stirred for 2 h at RT. The reaction was then quenched by the addition of 10 mL of water. The resulting solution was extracted with 3x30 mL of EtOAc and the organic layers were combined and concentrated under vacuum. The residue was eluted from silica gel with EtOAc/PE (1:1). This resulted in 200 mg (41% over three steps) of the title compound as a yellow solid. MS-ESI: 313 (M+1).

Steps 10-11 used similar procedures for converting compound **362"** to intermediate **124** shown in Scheme 80 to afford intermediate **129** from compound **394".** MS-ESI: 426 (M+1).

**Scheme 86:**

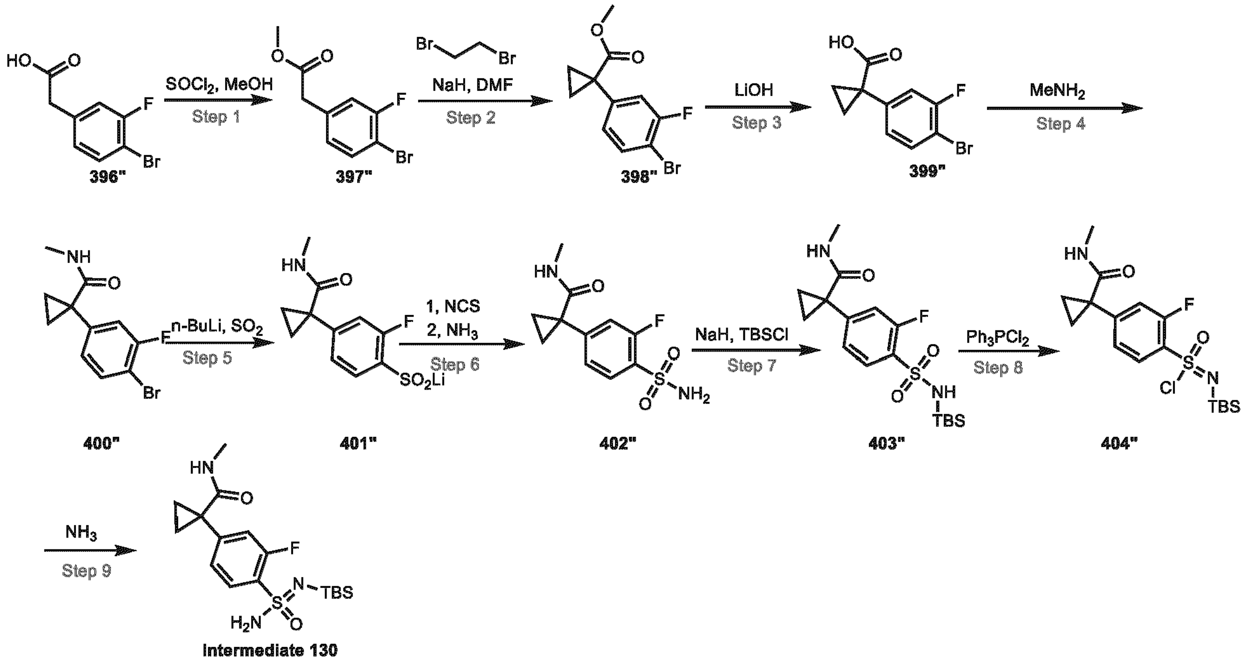


396" 397" 398" 399" 400" 401" 402" 403" 404"

Intermediate 130

## Intermediate 130

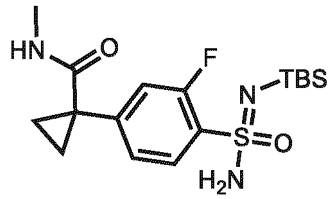

1-(4-(*N'*-(*Tert*-butyldimethylsilyl)sulfamidimidoyl)-3-fluorophenyl)-N-methylcyclopropane-1-carboxamide

**Step 1: Methyl 2-(4-bromo-3-fluorophenyl)acetate**

To a stirred solution of 2-(4-bromo-3-fluorophenyl)acetic acid (10.0 g, 42.9 mmol) in MeOH (200 mL) in a 500-mL round-bottom flask under nitrogen was added $SOCl_2$ (10.2 g, 85.8 mmol) dropwise at 0 °C over 30 min. The resulting solution was stirred for 16 h at RT. The resulting solution was concentrated under vacuum. The crude product was eluted from silica gel with EtOAc/PE (1:1). This resulted in 10.0 g (94.3%) of the title compound as an orange solid. MS-ESI: 247/249 (M+1).

**Step 2: Methyl 1-(4-bromo-3-fluorophenyl)cyclopropane-1-carboxylate**

To a stirred solution of methyl 2-(4-bromo-3-fluorophenyl)acetate (10.0 g, 40.5 mmol) in DMF (200 mL) in a 500-mL round-bottom flask under nitrogen. This was followed by the addition of NaH (60% dispersion in mineral oil, 4.88 g, 122 mmol) in portions at 0°C. To this was added 1,2-dibromoethane (11.4 g, 60.7 mmol) dropwise at 0°C. The resulting solution was stirred for 3 days at RT. The reaction was then quenched with 50 mL of water. The resulting solution was extracted with EtOAc (3x100 mL). The organic layers were combined and washed with brine (300 mL), then dried over anhydrous $Na_2SO_4$ and concentrated under vacuum. The crude product was eluted from silica gel with EtOAc/PE (1:1). This resulted in 5.0 g (45.2%) of the title compound as a yellow solid.

Steps 3-4 used similar procedures for converting compound **364"** to compound **366"** shown in Scheme 81 to afford compound **400"** from compound **398"**. MS-ESI: 272/274 (M+1).

Steps 5-9 used similar procedures for converting compound **369"** to intermediate **126** shown in Scheme 82 to afford intermediate **130** from compound **400"**. MS-ESI: 386 (M+1).

## Scheme 87:

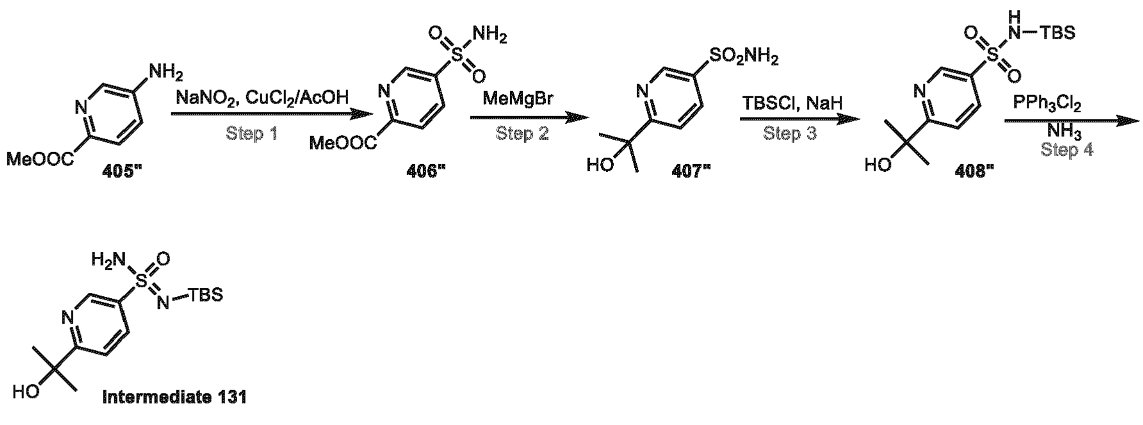


405" 406" 407" 408"

Intermediate 131

## Intermediate 131

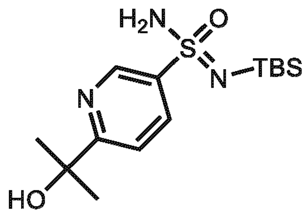

N'-(*tert*-bulyldimethylsilyl)-6-(2-hydroxypropan-2-yl)pyridine-3-sulfonimidamide

Step 1 used similar procedures for converting compound **374"** to compound **375"** shown in Scheme 83 to afford compound **406"** from compound **405".** MS-ESI: 217 (M+1).

**Step 2: 6-(2-Hydroxypropan-2-yl)pyridine-3-sulfonamide**

To a stirred solution of methyl 5-sulfamoylpicolinate (4.80 g, 22.2 mmol) in THF (300 mL) in a 500-mL 3-necked round-bottom flask under nitrogen was added MeMgBr/THF (3 M, 74 mL, 222 mmol) dropwise at 0°C. The resulting solution was stirred for 16 h at RT. The reaction was then quenched with 50 mL of $NH_4Cl$ (aq). The resulting solution was extracted with 3x100 ml of EtOAc and the organic layers were combined and dried over $Na_2SO_4$ and concentrated under vacuum. The residue was eluted from silica gel with DCM/MeOH (60:1). This resulted in 2.46 g (51.3%) of the title compound as a light yellow solid. MS-ESI: 215 (M-1).

Steps 3-4 used similar procedures for converting compound **362"** to intermediate **124** shown in Scheme 80 to afford intermediate **131** from compound **407".** MS-ESI: 330 (M+1).

**Table 30.** The Intermediate 132 was prepared using similar procedures for converting compound **405"** to Intermediate **131** shown in Scheme 87 from appropriate starting materials.

| Intermediate # | Structure | IUPAC Name | Exact Mass $[M+H]^+$ |
|---|---|---|---|
| **Intermediat e 132** | 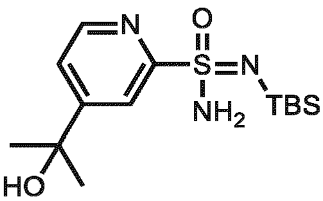 | N'-(tert-butyldimethylsilyl)-4-(2-hydroxypropan-2-yl)pyridine-2-sulfonimidamide | 330 |

## Scheme 88:

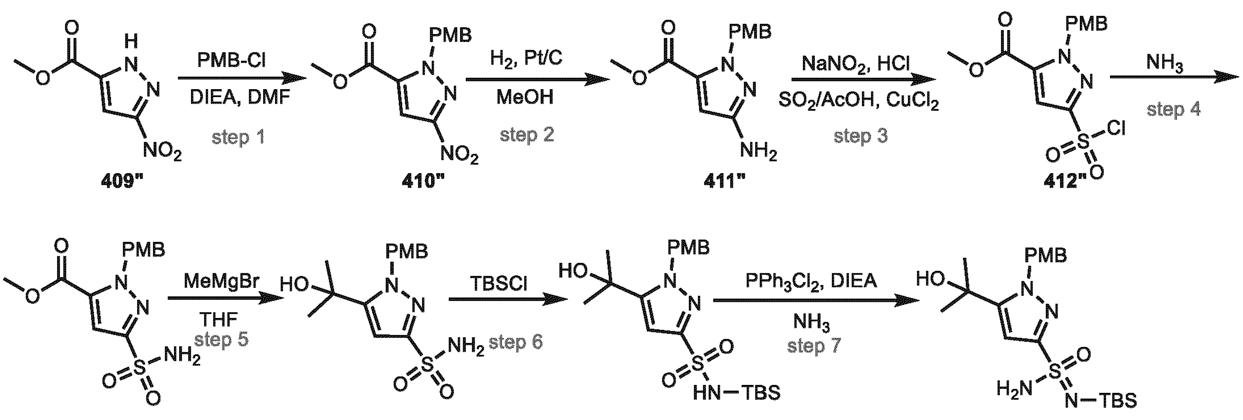


409" 410" 411" 412" 413" 414" 415" Intermediate 133

## Intermediate 133

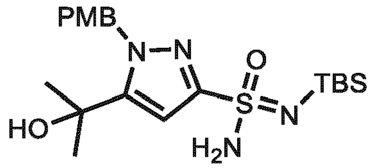

N'-(*tert*-butyldimethylsilyl)-5-(2-hydroxypropan-2-yl)-1-(4-methoxybenzyl)-1H-pyrazole-3-sulfonimidamide

**Step 1: Methyl 1-(4-methoxybenzyl)-3-nitro-1H-pyrazole-5-carboxylate**

To a stirred solution of methyl 3-nitro-1H-pyrazole-5-carboxylate (8.0 g, 46.8 mmol) in DMF (70 mL) in a 250 mL round-bottom flask was added TEA (26.8 g, 187 mmol) and 4-methoxybenzyl chloride (14.64 g, 93.5 mmol) dropwise at RT, the solution was stirred for 16 h at RT. The resulting mixture was quenched with 100 mL of water and extracted with EtOAc (3 x 300 mL). The combined organic layers were washed with brine (300 mL), dried over anhydrous $Na_2SO_4$. After filtration, the filtrate was concentrated under reduced pressure. This resulted in a mixture of the title compound and methyl 1-(4-methoxybenzyl)-5-nitro-1H-pyrazole-3-carboxylate (~6:1) (11.3 g, 82.9%) as a light yellow oil. MS-ESI: 292 (M+1).

**Step 2: Methyl 3-amino-1-(4-methoxybenzyl)-1H-pyrazole-5-carboxylate**

To a stirred solution of the mixture methyl 2-[(4-methoxyphenyl)methyl]-5-nitropyrazole-3-carboxylate and methyl 1-(4-methoxybenzyl)-5-nitro-1H-pyrazole-3-carboxylate (11.3 g, 38.8 mmol) in MeOH (500 mL) in a 1000 mL round-bottom flask under nitrogen was added Pt/C (5% wt., 1.50 g) in portions. The flask was evacuated and flushed 3 times with hydrogen. The solution was stirred for 16 h at RT under hydrogen atmosphere with a balloon. The resulting mixture was filtered, the filter cake was washed with MeOH (3x50 mL). The filtrate was concentrated under reduced pressure. The crude product was eluted from silica gel with EtOAc/PE (1:3). This resulted in the title compound (7.1 g, 70.1%) as a light yellow solid. MS-ESI: 262 (M+1).

Steps 3-7 used similar procedures for converting compound **405"** to Intermediate **131** shown in Scheme 87 to afford Intermediate **133** from compound **411"**. MS-ESI: 439 (M+1).

## Scheme 89:

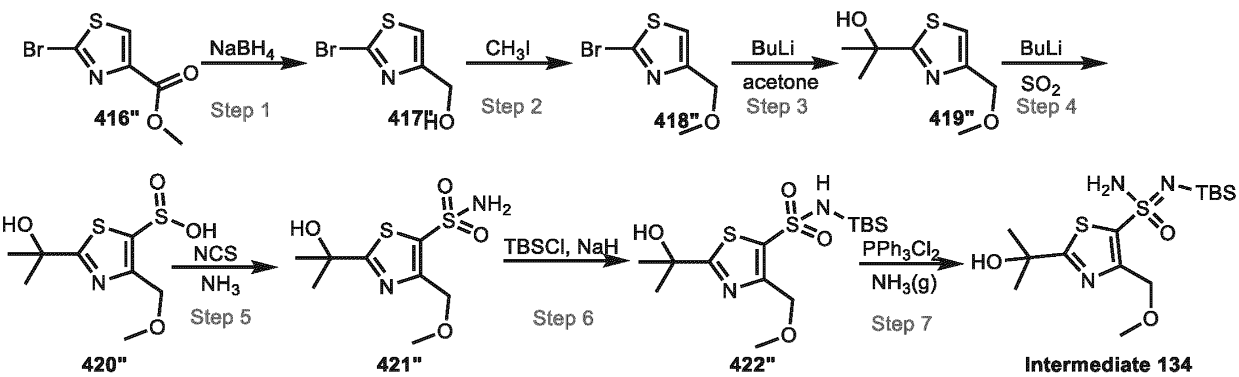


416" 417" 418" 419" 420" 421" 422" Intermediate 134

## Intermediate 134

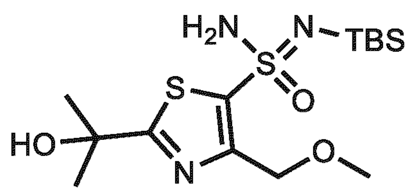

N'-(tert-butyldimethylsilyl)-2-(2-hydroxypropan-2-yl)-4-(methoxymethyl)thiazole-5-sulfonimidamide

**Step 1: (2-Bromothiazol-4-yl) MeOH**

To a stirred solution of methyl 2-bromothiazole-4-carboxylate (10.0 g, 45.0 mmol) in EtOH (150 mL) in a 250-mL round-bottom flask was added $NaBH_4$ (3.42 g, 90.0 mmol) in portions at RT. The resulting solution was stirred for 3 h at RT. The reaction was then quenched by the addition of 200 mL of water. The resulting solution was extracted with 3x300 ml of EtOAc and concentrated. The crude product was eluted from silica gel with EtOAc/PE (1:5). This resulted in 8.0 g (91.6%) of the title compound as yellow oil. MS-ESI: 196/194 (M+1).

**Step 2: 2-Bromo-4-(methoxymethyl)thiazole**

To a stirred solution of (2-bromothiazol-4-yl)methanol (8.0 g, 41.2 mmol) in THF (100 mL) in a 250-mL round-bottom flask under nitrogen was added NaH (60% dispersion in mineral oil, 1.65 g, 82.4 mmol) in portions at 0°C. The resulting solution was stirred for 20 min at RT. This was followed by the addition of $CH_3I$ (6.43 g, 45.3 mmol) dropwise with stirring at 0°C. The resulting solution was stirred for 16 h at RT. The reaction was then quenched with 200 mL of water. The resulting solution was extracted with 3x200 ml of EtOAc and the organic layers combined and concentrated under vacuum. The residue was eluted from silica gel with EtOAc/PE (1:30). This resulted in 7.0 g (81.7%) of the title compound as a light yellow solid. MS-ESI: 210/208 (M+1).

**Step 3: 2-(4-(Methoxymethyl)thiazol-2-yl)propan-2-ol**

To a stirred solution of 2-bromo-4-(methoxymethyl)thiazole (7.0 g, 33.6 mmol) in THF (70 mL) a 250-mL 3-necked round-bottom flask under nitrogen was added n-BuLi/hexane (2.5 M, 14.8 mL, 37.0 mmol) dropwise at -78 °C over 30 min. To this was added acetone (2.15 g, 37.0 mmol) dropwise with stirring at -78 °C. The resulting solution was stirred for 50 min at RT. The reaction was then quenched by the addition of 80 mL of water/ice. The resulting solution was extracted with 3x80 mL of EtOAc. The resulting mixture was washed with 2 x50 mL of water. The mixture was dried over anhydrous $Na_2SO_4$. The solids were filtered out. The resulting mixture was concentrated under vacuum. The residue was eluted from silica gel with EtOAc/PE (1/5). This resulted in 3.20 g (50.8%) of the title compound as yellow oil. MS-ESI: 250 (M-1).

Steps 4-7 used similar procedures for converting compound **369"** to intermediate **126** shown in Scheme 82 to afford intermediate **134** from compound **419".** MS-ESI: 380 (M+1).

## Scheme 90:

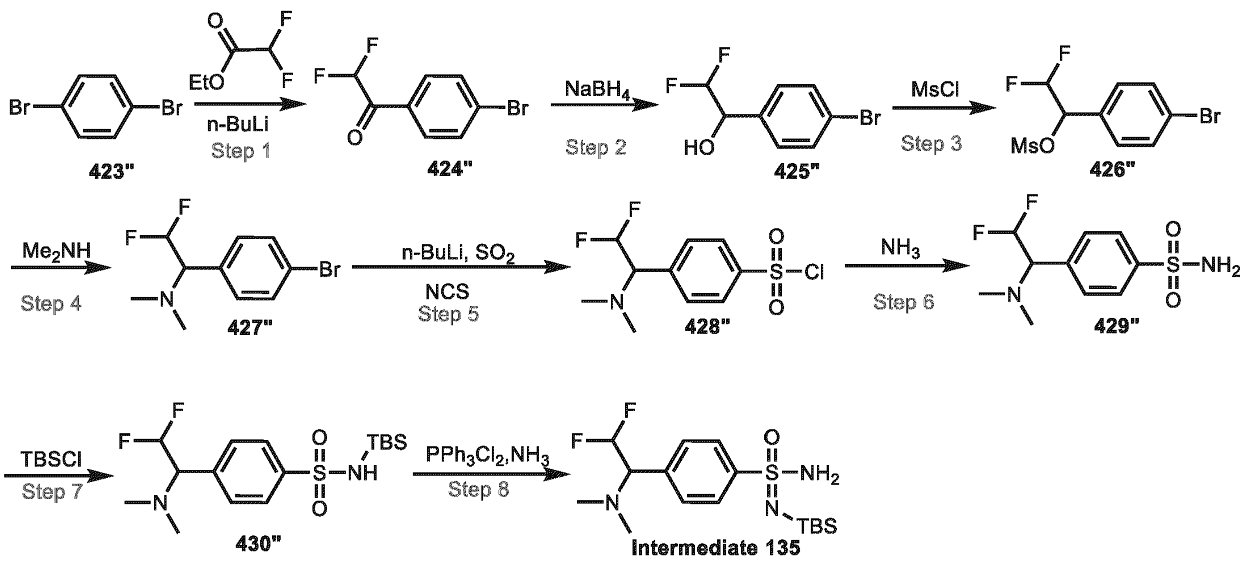

## Intermediate 135

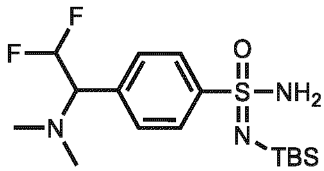

N'-(*tert*-butyldimethylsilyl)-4-(1-(dimethylamino)-2,2-difluoroethyl)benzenesulfonimidamide

**Step 1: 1-(4-Bromophenyl)-2,2-difluoroethan-1-one**

To a stirred solution of 1,4-dibromobenzene (10.0 g, 42.4 mmol) in THF (300 mL) a 500-mL 3-necked round-bottom flask under nitrogen was added n-BuLi/hexane (2.5 M, 18.6 mL, 46.6 mmol) dropwise at -70 °C. The resulting solution was stirred for 30 min at -70 °C. Then to this was added ethyl ethyl 2,2-difluoroacetate (15.7 g, 127 mmol) in THF (20 mL) dropwise at -70 °C. The resulting solution was stirred for 30 min at -70 °C. The reaction was then quenched by the addition of 200 mL of water. The resulting solution was extracted with 3x200 mL of DCM and the combined organic layers dried over $Na_2SO_4$, then concentrated under reduced pressure. This resulted in 6.0 g (crude) of the title compound as a yellow oil. MS-ESI: 235/237 (M+1).

**Step 2: 1-(4-Bromophenyl)-2,2-difluoroethan-1-ol**

To a stirred solution of 1-(4-bromophenyl)-2,2-difluoroethan-1-one (6.0 g, crude) in ethanol (40 mL) in a 50-mL round-bottom flask under nitrogen was added $NaBH_4$ (3.22 g, 84.8 mmol) in portions at 0°C. The resulting solution was stirred for 1 h at RT. The reaction was then quenched by the addition of 50 mL of water. The resulting solution was extracted with 3x20 mL of DCM, the combined organic layers were dried over $Na_2SO_4$ and then concentrated under reduced pressure. The residue was eluted from silica gel with EtOAc/PE (1/5). This resulted in 5 g (49.8% over two steps) of the title compound as brown oil. MS-ESI: 237/239 (M+1).

**Step 3: 1-(4-Bromophenyl)-2,2-difluoroethyl methanesulfonate**

To a stirred solution of 1-(4-bromophenyl)-2,2-difluoroethan-1-ol (4.74 g, 20.0 mmol) in DCM (100 mL) in a 250-mL round-bottom flask was added TEA (6.06 g, 60.0 mmol) at RT. This was followed by the addition of methanesulfonyl chloride (3.45 g, 30.0 mmol) dropwise with stirring at 0°C. The resulting solution was stirred for 30 min at RT. The reaction was then quenched by the addition of 100 mL of water. The resulting solution was extracted with 3x100 mL of DCM, the combined organic layers were dried over $Na_2SO_4$ and then concentrated under vacuum. This resulted in 5.53 g (87.7%) of the title compound as brown oil. MS-ESI: 315/317 (M+1).

**Step 4: 1-(4-Bromophenyl)-2,2-difluoro-*N,N*-dimethylethan-1-amine**

To a stirred solution of 1-(4-bromophenyl)-2,2-difluoroethyl methanesulfonate (5.53 g, 17.6 mmol) in THF (100 mL) in a 250-mL round-bottom flask was added dimethylamine in THF (2 M, 352 mL, 704 mmol) dropwise at 0°C. The resulting solution was stirred for 14 h at 60 °C in an oil bath. The resulting mixture was concentrated under vacuum. The residue was eluted from silica gel with EtOAc/PE (1:1). This resulted in 3.96 g (85.2%) of the title compound as brown oil. MS-ESI: 264/266 (M+1).

Steps 5-8 used similar procedures for converting compound **369"** to Intermediate **126** shown in Scheme 82 to afford Intermediate **135** from compound **427".** MS-ESI: 378 (M+1).

## Scheme 91:

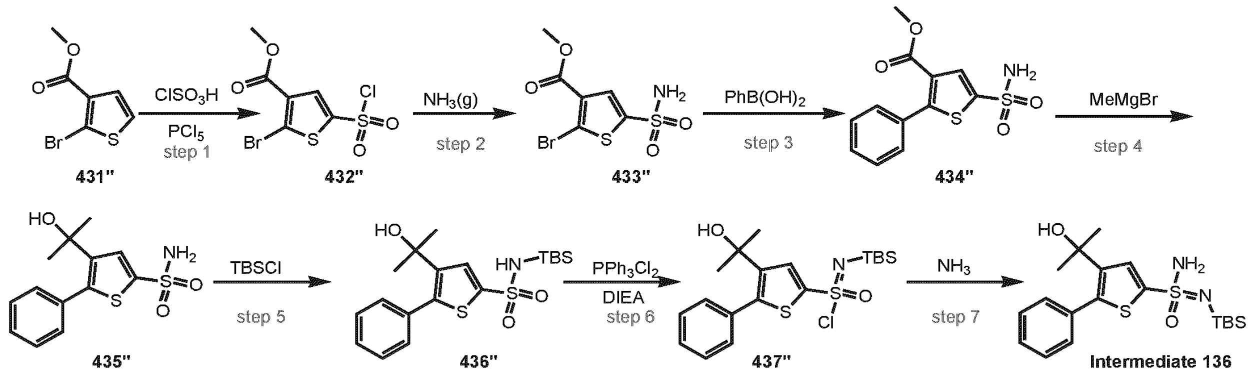


# Intermediate 136

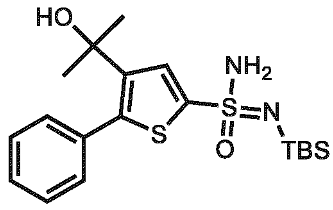

<u>*N'*-(*tert*-butyldimethylsilyl)-4-(2-hydroxypropan-2-yl)-5-phenylthiophene-2-sulfonimidamide</u>

**Step 1: Methyl 2-bromo-5-(chlorosulfonyl)thiophene-3-carboxylate**

To a stirred solution of methyl 2-bromothiophene-3-carboxylate (4.42 g, 20.0 mmol) in $CHCl_3$ (100 mL) in a 250-mL round-bottom flask was added $HSO_3Cl$ (7.02 g, 60.0 mmol) dropwise at 0 °C. The resulting solution was stirred for 16 h at RT. This was followed by the addition of $PCl_5$ (12.5 g, 60.0 mmol) in several batches at 0 °C. The resulting solution was stirred for 3 h at 60 °C in an oil bath. The reaction mixture was poured into 200 mL of water/ice slowly. The resulting solution was extracted with 3x100 mL of DCM, the combined organic layers dried over $Na_2SO_4$ then concentrated under vacuum. This resulted in 4.50 g (crude) of the title compound as light yellow oil.

**Step 2: Methyl 2-bromo-5-sulfamoylthiophene-3-carboxylate**

To a stirred solution of methyl 2-bromo-5-(chlorosulfonyl)thiophene-3-carboxylate (4.50 g, crude) in DCM (100 mL) in a 250-mL round-bottom flask. Then $NH_3$ (g) was introduced to the reaction mixture with stirring at 0 °C for 10 min. The resulting solution was stirred for 2 h at 25 °C, the mixture was concentrated under vacuum. The crude product was eluted from silica gel with EtOAc/PE (1:1). This resulted in 3.04 g (50.6%) of the title compound as yellow solid. MS-ESI: 300/298 (M-1).

**Step 3: Methyl 2-phenyl-5-sulfamoylthiophene-3-carboxylate**

To a stirred solution of methyl 2-bromo-5-sulfamoylthiophene-3-carboxylate (3.0 g, 10.0 mmol) in dioxane (100 mL)/$H_2O$ (10 mL) in a 500-mL 3-necked round-bottom flask under nitrogen was added $Cs_2CO_3$ (8.15 g, 25.0 mmol). Then phenylboronic acid (3.05 g, 25.0 mmol), Xphos (477 mg, 1.0 mmol) and Pd(dppf)$Cl_2$ (732 mg, 1.0 mmol) were added at RT. The resulting solution was stirred for 16 h at 80 °C in an oil bath. The insoluble was filtered out and the filtrate was concentrated under vacuum. The residue was eluted from silica gel with EtOAc/PE (1:3). This resulted in 1.81 g (61.1%) of the title compound as yellow oil. MS-ESI: 296 (M-1).

Steps 4-7 used similar procedures for converting compound **406"** to intermediate **131** shown in Scheme 87 to afford intermediate **136** from compound **434".** MS-ESI: 411 (M+1).

## Scheme 92:

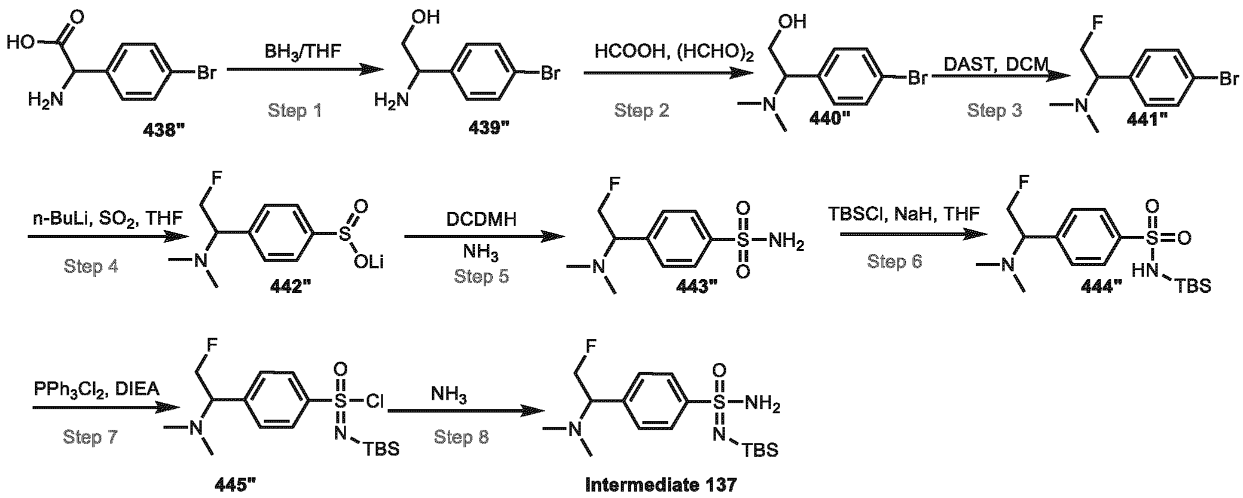


438" 439" 440" 441" 442" 443" 444" 445" Intermediate 137

## Intermediate 137

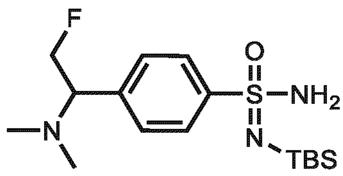

*N'*-(*tert*-butyldimethylsilyl)-4-(1-(dimethylamino)-2-fluoroethyl)benzenesulfonimidamide

**Step 1: 2-Amino-2-(4-bromophenyl)ethan-1-ol**

To a stirred solution of 2-amino-2-(4-bromophenyl)acetic acid (5.0 g, 21.7 mmol) in THF (200 mL) in a 500-mL 3-necked round-bottom flask under nitrogen was added $BH_3$/THF (1M, 108 mmol, 108 mL) dropwise at 0 °C in an ice bath. The resulting solution was stirred for 16 h at 50 °C in an oil bath. The reaction was then quenched by the addition of 50 mL of MeOH. The resulting mixture was concentrated under vacuum. The residue was eluted from silica gel with DCM/MeOH (10:1). This resulted in 2.67 g (56.9%) of the title compound as yellow oil. MS-ESI: 216/218 (M+1).

**Step 2: 2-(4-Bromophenyl)-2-(dimethylamino)ethan-1-ol**

To a stirred solution of 2-amino-2-(4-bromophenyl)ethan-1-ol (2.67 g, 12.4 mmol) in HCOOH (10 mL) in a 100-mL round-bottom flask under nitrogen was added $(HCHO)_n$ (1.12 g, 37.2 mmol) at 0 °C. The resulting solution was stirred for 2 h at 85 °C in an oil bath. The pH value of the solution was adjusted to 10 with $Na_2CO_3$ (5 M). The resulting solution was extracted with 3x100 mL of EtOAc, the combine organic layers was dried over anhydrous $Na_2SO_4$ and concentrated under vacuum. The residue was eluted from silica gel with EtOAc/PE (1:3). This resulted in 2.37 g (78.3%) of the title compound as a yellow solid. MS-ESI: 244/246 (M+1).

**Step 3: 1-(4-Bromophenyl)-2-fluoro-N,N-dimethylethan-1-amine**

To a stirred solution of 2-(4-bromophenyl)-2-(dimethylamino)ethan-1-ol (1.22 g, 5.00 mmol) in DCM (20 mL) in a 100-mL 3-necked round-bottom flask under nitrogen was added DAST (4.03 g, 25.0 mmol) dropwise at 0 °C. The resulting solution was stirred for 16 h at RT. The reaction was then quenched by the addition of 10 mL of water/ice. The pH value of the solution was adjusted to 9 with $NaHCO_3$ (6 M). Then the mixture was extracted with EtOAc (3x50 mL) and the organic layers were combined and dried over anhydrous $Na_2SO_4$, then concentrated under vacuum. The residue was eluted from silica gel with EtOAc/PE (1:1). This resulted in 925 mg (75.2%) of the title compound as brown oil. MS-ESI: 246/248 (M+1).

**Step 4: Lithium 4-(1-(dimethylamino)-2-fluoroethyl)benzenesulfinate**

To a stirred solution of 1-(4-bromophenyl)-2-fluoro-N,N-dimethylethan-1-amine (738 mg, 3.00 mmol) in THF (30 mL) in a 100-mL 3-necked round-bottom flask under nitrogen was added n-BuLi/hexane(2.5 M, 2.40 mL, 6.0 mmol) dropwise at -78 °C. The resulting solution was stirred for 30 min at -78 °C. Then $SO_2$ (g) was bubbled into the reaction solution for 15 min below 0 °C. The resulting solution was allowed to react, with stirring, for an additional 1 h at RT. The reaction was concentrated under vacuum and for next step without purification. MS-ESI: 232 (M+ 1).

**Step 5: 4-(1-(Dimethylamino)-2-fluoroethyl)benzenesulfonamide**

To a stirted solution of Lithium 4-(1-(dimethylamino)-2-fluoroethyl)benzenesulfinate (693 mg, crude) in DCM (40 mL) in a 100-mL round-bottom flask was added DCDMH (1.18 g, 6.00 mmol) at RT. Then the reaction solution was stirred for 2 h at RT. Then $NH_3$ (g) was bubbled into the solution for 10 min at 0 °C. The resulting solution was allowed to react, with stirring, for an additional 1 h at RT. The resulting mixture was concentrated under vacuum. The residue was eluted from silica gel with DCM/MeOH (1:10). This resulted in 163 mg (22.1% over two steps) of the title compound as yellow oil. MS-ESI: 247 (M+1).

Steps 6-8 used similar procedures for converting compound **362"** to intermediate **124** shown in Scheme 80 to afford intermediate **137** from compound **443".** MS-ESI: 360 (M+1).

## Scheme 93:

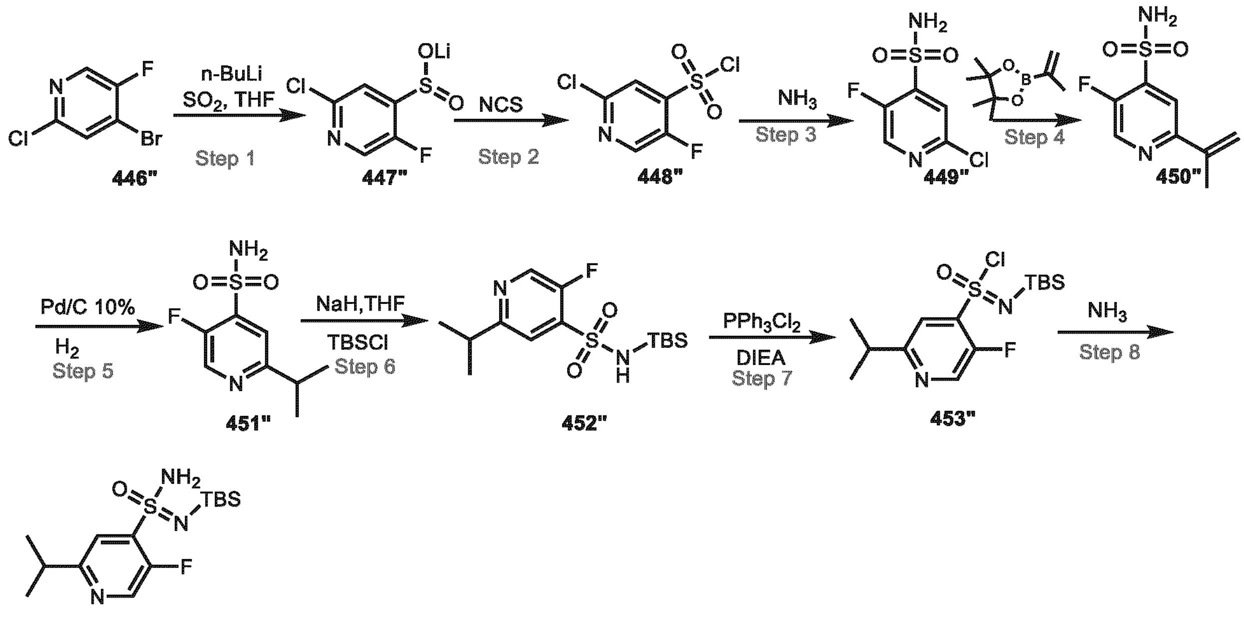


**Intermediate 138**

## Intermediate 138

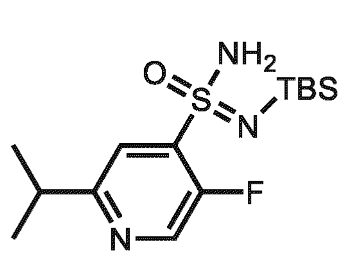

N'-(*tert*-butyldimethylsilyl)-5-fluoro-2-isopropylpyridine-4-sulfonimidamide

Steps 1-3 used similar procedures for converting compound **369"** to compound **371"** shown in Scheme 82 to afford compound **449"** from compound **446".** MS-ESI: 211/213 (M+1).

**Step 4: 5-Fluoro-2-(prop-1-en-2-yl)pyridine-4-sulfonamide**

To a stirred solution of 2-chloro-5-fluoropyridine-4-sulfonamide (1.27 g, 6.00 mmol) in $H_2O$ (15 mL) and dioxane (60 mL) in a 100-mL 3-necked round-bottom flask under nitrogen was added 4,4,5,5-tetramethyl-2-(prop-1-en-2-yl)-1,3,2-dioxaborolane (3.02 g, 18.0 mmol). Then $Cs_2CO_3$ (3.91 g, 12.0 mmol) and $Pd(dppf)Cl_2$ (439 mg, 0.600 mmol) were added. The resulting solution was stirred for 12 h at 80 °C in an oil bath. The resulted mixture was concentrated under vacuum. The resulting solution was diluted with 50 mL of $H_2O$. The resulting solution was extracted with 3x100 mL of DCM, the combined organic layers was dried over anhydrous $Na_2SO_4$ and concentrated under vacuum. The residue was eluted from silica gel with EtOAc/PE (1:9). This resulted in 1.18 g (90.8%) of the title compound as a dark yellow solid. MS-ESI: 217 (M+1).

Step 5: 5-Fluoro-2-isopropylpyridine-4-sulfonamide

To a stirred solution of 5-fluoro-2-(prop-1-en-2-yl)pyridine-4-sulfonamide (1.18 g, 5.46 mmol) in isopropyl alcohol (50 mL) in a 100-mL round-bottom flask under nitrogen was added Pd/C (10%wt., 200 mg), the flask was evacuated and flushed with hydrogen 3 times. The resulting solution was stirred for 3 h at RT under hydrogen with a balloon. The solids were filtered out. The resulting mixture was concentrated under vacuum. The residue was eluted from silica gel with EtOAc/PE (1:9). This resulted in 1.07 g (89.9%) of the title compound as yellow oil. MS-ESI: 219 (M+1).

Steps 6-8 used similar procedures for converting compound **362"** to intermediate **124** shown in Scheme 80 to afford intermediate **138** from compound **451"**. MS-ESI: 332 (M+1).

## Scheme 94:

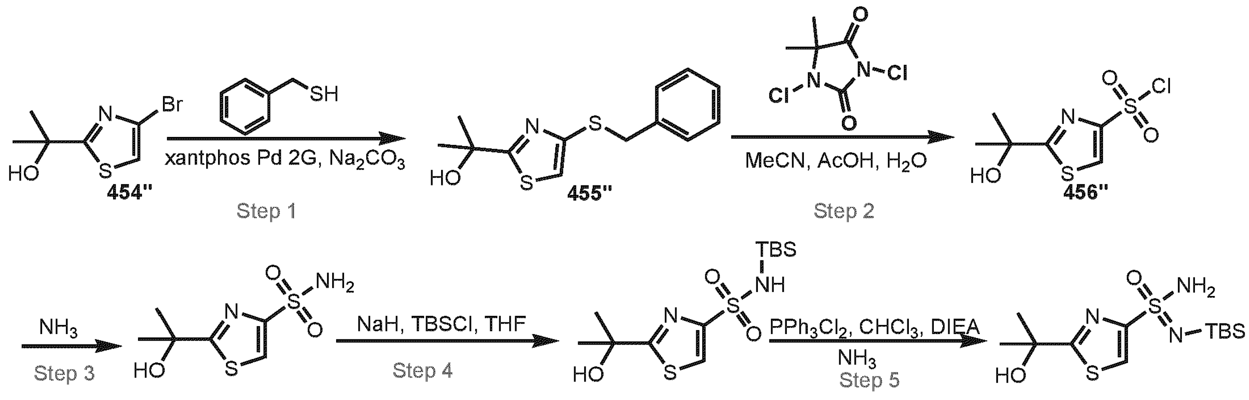


454" 455" 456" 457" 458" Intermediate 139

## Intermediate 139

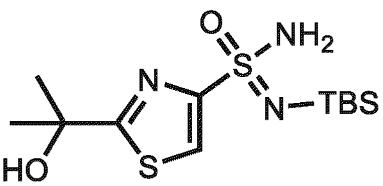

*N'*-(*tert*-buryldimethylsilyl)-2-(2-hydroxypropan-2-yl)thiazole-4-sulfonimidamide

**Step 1: 2-(4-(Benzylthio) thiazol-2-yl)propan-2-ol**

To a stirred solution of 2-(4-bromothiazol-2-yl)propan-2-ol (8.0 g, 36.0 mmol) in 1,2-dimethoxyethane (100 mL) in a 100-mL 3-necked round-bottom flask under nitrogen was added $Na_2CO_3$ (11.4 g, 108 mmol). Then phenylmethanethiol (22.3 g, 180 mmol) and XPhos Pd G2 (3.20 g, 3.60 mmol) were added. The resulting solution was stirred for 16 h at 80 °C in an oil bath. The insoluble was filtered out and the filtrate was concentrated under vacuum. The residue was eluted from silica gel with EtOAc/hexane (1:5). This resulted in 4.99 g (52.3%) of the title compound as a yellow

solid. MS-ESI: 266 (M+1).

**Step 2: 2-(2-Hydroxypropan-2-yl) thiazole-4-sulfonyl chloride**

To a stirred solution of 2-(4-(benzylthio) thiazol-2-yl)propan-2-ol (500 mg, 1.89 mmol) in MeCN (40 mL), $H_2O$ (2 mL), AcOH (0.75 mL) in a 100-mL round-bottom flask was added DCDMH (1.12 g, 5.67 mmol) in MeCN (10 mL) dropwise with stirring at 0 °C. The resulting solution was stirred for 3 h at RT. The resulting mixture was diluted with 30 mL of water. The resulting solution was extracted with 3x30 mL of EtOAc, the combined organic layers was dried over anhydrous $Na_2SO_4$ and concentrated under vacuum. This resulted in 400 mg (crude) of the title compound as a yellow solid.

**Step 3: 2-(2-Hydroxypropan-2-yl) thiazole-4-sulfonamide**

To a stirred solution of 2-(2-hydroxypropan-2-yl) thiazole-4-sulfonyl chloride (400 mg, crude) in DCM (30 mL) in a 100-mL round-bottom flask bubbled $NH_3$ (g) at 0°C for 10 min. The resulting solution was stirred for 20 min at RT. The resulting mixture was concentrated under vacuum. The residue was eluted from silica gel with EtOAc/PE (1:1). This resulted in 150 mg (35.7% for Steps 2&3) of the title compound as a yellow solid. MS-ESI: 221 (M-1).

Steps 4-5 used similar procedures for converting compound 362" to intermediate 124 shown in Scheme 80 to afford intermediate 139 from compound 457". MS-ESI: 336 (M+1).

## Scheme 95:

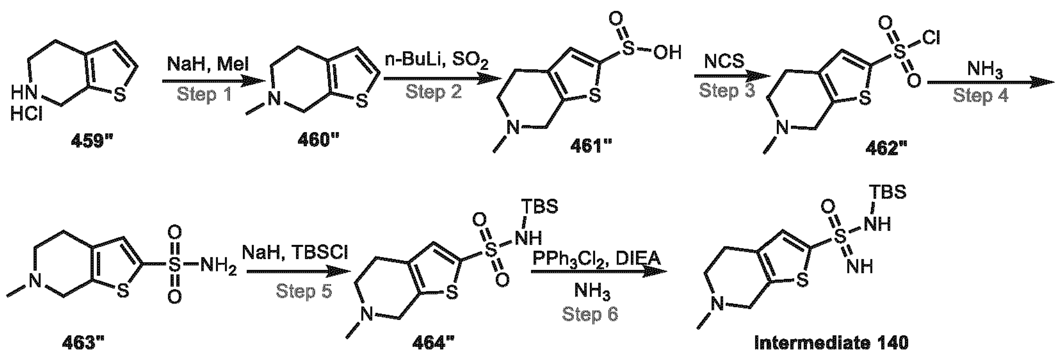


## Intermediate 140

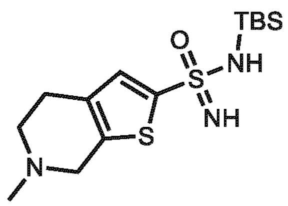

<u>*N*-(*tert*-butyldimethylsilyl)-6-methyl-4,5,6,7-tetrahydrothieno[2,3-c]pyridine-2-sulfonimidamide</u>

**Step 1: 6-Methyl-4,5,6,7-tetrahydrothieno[2,3-c]pyridine**

To a stirred solution of 4,5,6,7-tetrahydrothieno[2,3-c]pyridine hydrochloride (1.76 g, 10.0 mmol) in THF (100 mL) in a 250-mL round-bottom flask under nitrogen was added NaH (60% dispersion in mineral oil, 800 mg, 20.0 mmol) in portions at 0°C. The resulting solution was stirred for 20 min at RT. This was followed by the addition of $CH_3I$ (4.26 g, 30.0 mmol) dropwise with stirring at 0°C. The resulting solution was stirred for 16 h at RT. The reaction was then quenched by the addition of water (100 mL). The resulting solution was extracted with 3x100 ml of EtOAc and the organic layers combined and dried over $Na_2SO_4$, then concentrated under vacuum. The residue was eluted from silica gel with DCM/MeOH (18:1). This resulted in 1.25 g (81.7%) of the title compound as a light yellow solid. MS-ESI: 154 (M+1).

Steps 2-6 used similar procedures for converting compound **369"** to intermediate **126** shown in Scheme 82 to afford intermediate **140** from compound **460"**. MS-ESI: 346 (M+1).

## Scheme 96:

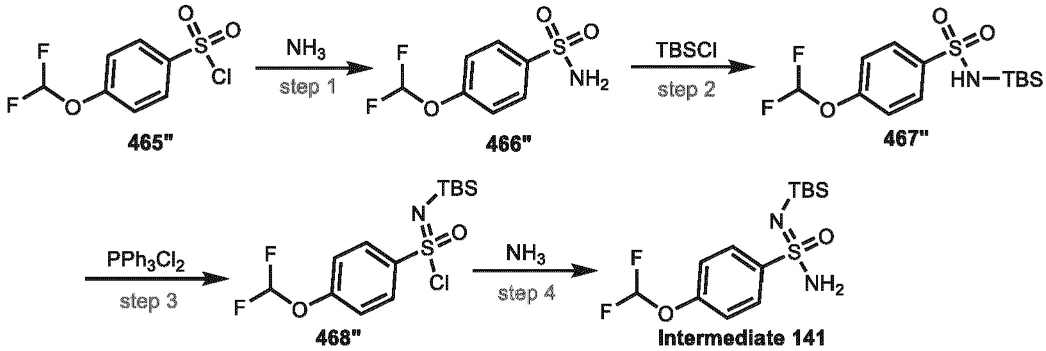


## Intermediate 141

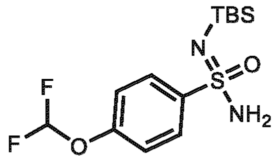

<u>*N'*-(*tert*-butyldimethylsilyl)-4-(difluoromethoxy)benzenesulfonimidamide</u>

**Step 1: 4-(Difluoromethoxy)benzenesulfonamide**

To a stirred solution of 4-(difluoromethoxy)benzenesulfonyl chloride (2.43 g, 10.0 mmol) in THF (100 mL) in a 250-mL round-bottom flask was bubbled $NH_3$ (g) for 10 min with stirring at 0°C. The resulting solution was stirred for 2 h at RT and concentrated under vacuum. The crude product was eluted from silica gel with EtOAc/PE (1:1). This resulted in 1.93 g (86.5%) of the title compound as yellow oil. MS-ESI: 222 (M-1).

Steps 2-4 used similar procedures for converting compound **362"** to intermediate **124** shown in Scheme 80 to afford intermediate **141** from compound **466"**. MS-ESI: 337 (M+1).

## Scheme 97:

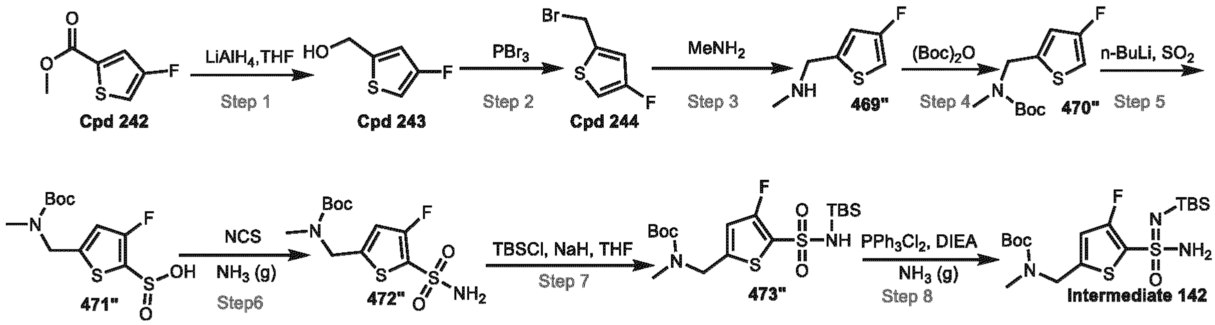


## Intermediate 142

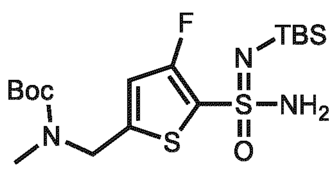

Tert-butyl ((5-(N'-(tert-butyldimethylsilyl)sulfamidimidoyl)-4-fluorothiophen-2-yl)methyl)(methyl)carbamate

**Step 1: (4-Fluorothiophen-2-yl)methanol**

To a stirred solution of methyl 4-fluorothiophene-2-carboxylate (10.0 g, 62.5 mmol) in THF (200 mL) in a 500-mL round-bottom flask under nitrogen was added $LiAlH_4$ (4.75 g, 125 mmol) in portions at 0°C. The resulting solution was stirred for 16 h at RT. The reaction was then quenched with 100 mL of water/ice. The insoluble was filtered out and the filtrate was extracted with EtOAc (3x200 mL). The organic layers were combined and dried over anhydrous $Na_2SO_4$ and concentrated under vacuum. The crude product was eluted from silica gel with EtOAc/PE (1:1). This resulted in 7.0 g (84.8%) of the title compound as a yellow solid. MS-ESI: 133 (M+1).

**Step 2: 2-(Bromomethyl)-4-fluorothiophene**

To a stirred solution of (4-fluorothiophen-2-yl)methanol (7.0 g, 53.0 mmol) in DCM (150 mL) in a 500-mL round-bottom flask was added $PBr_3$ (17.2 g, 63.6 mmol) dropwise at 0°C. The resulting solution was stirred for 3 h at RT. The reaction was then quenched by the addition of 60 mL of water. The resulting solution was extracted with DCM (3x50 mL). The organic layers were combined and dried over anhydrous $Na_2SO_4$ and concentrated under vacuum. The crude product was eluted from silica gel with EtOAc/PE (1:5). This resulted in 8.0 g (77.4%) of the title compound as a yellow solid.

**Step 3: 1-(4-Fluorothiophen-2-yl)-*N*-methylmethanamine**

To a stirred solution of 2-(bromomethyl)-4-fluorothiophene (8.00 g, 41.0 mmol) in THF (100 mL) in a 250-mL round-bottom flask was added methanamine in THF (2 M, 41 mL, 82.0 mmol) at RT. The resulting solution was stirred for 16 h at RT. The reaction was then quenched with 50 mL of water. The resulting solution was extracted with EtOAc (3x100 mL). The organic layers were combined and dried over anhydrous $Na_2SO_4$ and concentrated under vacuum. The crude product was eluted from silica gel with EtOAc/PE (1:1). This resulted in 4.0 g (67.3%) of the title compound as a yellow solid. MS-ESI: 146 (M+1).

**Step 4: *Tert*-butyl ((4-fluorothiophen-2-yl)methyl)(methyl)carbamate**

To a stirred solution of 1-(4-fluorothiophen-2-yl)-*N*-methylmethanamine (4.0 g, 27.6 mmol) in THF (40 mL) in a 100-mL round-bottom flask under nitrogen was added NaH (60% dispersion in mineral oil, 1.66 g, 41.4 mmol) in portions at 0°C. The resulting solution was stirred for 20 min at RT. This was followed by the addition of $(Boc)_2O$ (9.03 g, 41.4 mmol) in THF (5 mL) dropwise with stirring at 0°C. The resulting solution was stirred for 16 h at RT. The reaction was then quenched with 10 mL of water. The resulting solution was extracted with EtOAc. The organic layers were combined and dried over anhydrous $Na_2SO_4$ and concentrated under vacuum. The crude product was eluted from silica gel with EtOAc/PE (1:3). This resulted in 5.0 g (74.0%) of the title compound as a yellow solid. MS-ESI: 246 (M+1).

Steps 5-8 used similar procedures for converting compound **369"** to intermediate **126** shown in Scheme 82 to afford intermediate **142** from compound **470".** MS-ESI: 438 (M+1).

## Scheme 98:

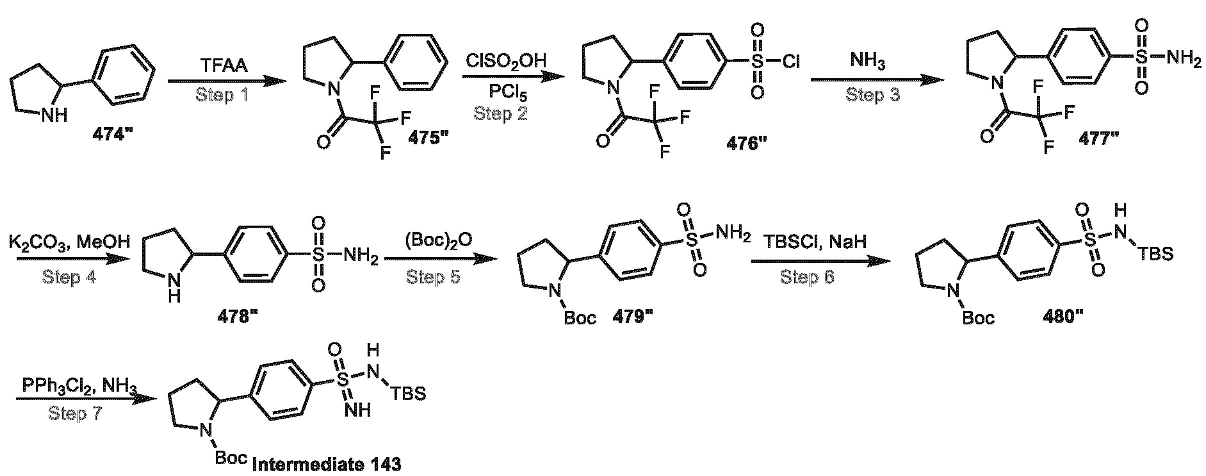

## Intermediate 143

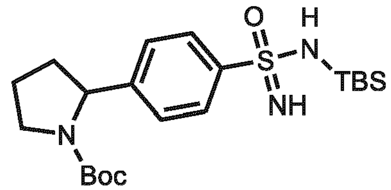

Tert-butyl 2-(4-(N-(tert-butyldimethylsilyl)sulfamidimidoyl)phenyl)pyrrolidine-1-carboxylate

**Step 1: 2,2,2-Trifluoro-1-(2-phenylpyrrolidin-1-yl)ethan-1-one**

To a stirred solution of 2-phenylpyrrolidine (16.5 g, 112 mmol) in DCM (200 mL) in a 500-mL round-bottom flask under nitrogen was added TEA (22.6 g, 224 mmol) followed by TFAA (16.0 mL) dropwise at 0°C. The resulting solution was stirred for 2 h at RT. The reaction was then quenched with 100 mL of water. The resulting solution was extracted with 3x200 mL of DCM. The organic layers were combined and dried over anhydrous $Na_2SO_4$ and concentrated under vacuum. The crude product was eluted from silica gel with EtOAc/PE (1:4). This resulted in 20.0 g (73.5%) of the title compound as yellow oil. MS-ESI: 244 (M+1).

**Step 2: 4-(1-(2,2,2-trifluoroacetyl)pyrrolidin-2-yl)benzenesulfonyl chloride**

To a stirred solution of 2,2,2-trifluoro-1-(2-phenylpyrrolidin-1-yl)ethan-1-one (4.86 g, 20.0 mmol) in $CHCl_3$ (100 mL) in a 250-mL round-bottom flask was added $HSO_3Cl$ (7.02 g, 60.0 mmol) dropwise at 0°C. The resulting solution was stirred for 16 h at RT. This was followed by the addition of $PCl_5$ (12.5 g, 60.0 mmol) in several batches at 0°C. The resulting solution was stirred for 3 h at 60 °C in an oil bath. The reaction mixture was poured into 200 mL of water/ice slowly. The resulting solution was extracted with 3x100 mL of DCM. The organic layers were combined and dried over anhydrous $Na_2SO_4$ and concentrated under vacuum. This resulted in 5.10 g (crude) of the title compound as light yellow oil.

**Step 3: 4-(1-(2,2,2-Trifluoroacetyl)pyrrolidin-2-yl)benzenesulfonamide**

To a stirred solution of 4-(1-(2,2,2-trifluoroacetyl)pyrrolidin-2-yl)benzenesulfonyl chloride (5.10 g, crude) in DCM (100 mL) in a 250-mL round-bottom flask. Then $NH_3$ (g) was introduced into the reaction solution with stirring at 0 °C for 10 min. The resulting solution was stirred for 2 h at RT and concentrated under vacuum. The crude product was eluted from silica gel with EtOAc/PE (1:1). This resulted in 3.26 g (50.6%, over two steps) of the title compound as a yellow solid. MS-ESI: 321 (M-1).

**Step 4: 4-(Pyrrolidin-2-yl)benzenesulfonamide**

To a stirred solution of 4-(1-(2,2,2-trifluoroacetyl)pyrrolidin-2-yl)benzenesulfonamide (3.22 g, 10.0 mmol) in MeOH (30 mL) in a 250-mL round-bottom flask under nitrogen was added $K_2CO_3$ (4.14 g, 30.0 mmol). The resulting solution was stirred for 16 h at RT. The solids were filtered out and the filter cake was washed with 3x100 mL of EtOAc, the filtrate and the wash were combined and concentrated under vacuum. The crude product was eluted from silica gel with EtOAc/PE (1:1). This resulted in 1.86 g (82.1%) of the title compound as yellow oil. MS-ESI: 227 (M+1).

**Step 5: *Tert-butyl* 2-(4-sulfamoylphenyl)pyrrolidine-1-carboxylate**

To a stirred solution of 4-(pyrrolidin-2-yl)benzenesulfonamide (1.13 g, 5.0 mmol) in DCM (30 mL) in a 250-mL round-bottom flask under nitrogen was added $NaHCO_3$ (840 mg, 10.0 mmol) and $Boc_2O$ (1.20 g, 5.50 mmol) in portions at RT. The resulting solution was stirred for 2 h at RT. The reaction was then quenched by the addition of water. The resulting solution was extracted with 3x50 mL of DCM and the organic layers combined and concentrated under vacuum. The crude product was eluted from silica gel with EtOAc/PE (1:1). This resulted in 1.24 g (76.3%) of the title compound as yellow oil. MS-ESI: 327 (M+1).

Steps 6-7 used similar procedures for converting compound **362"** to intermediate **124** shown in Scheme 80 to afford intermediate **143** from compound **479".** MS-ESI: 440 (M+1).

## Scheme 99:

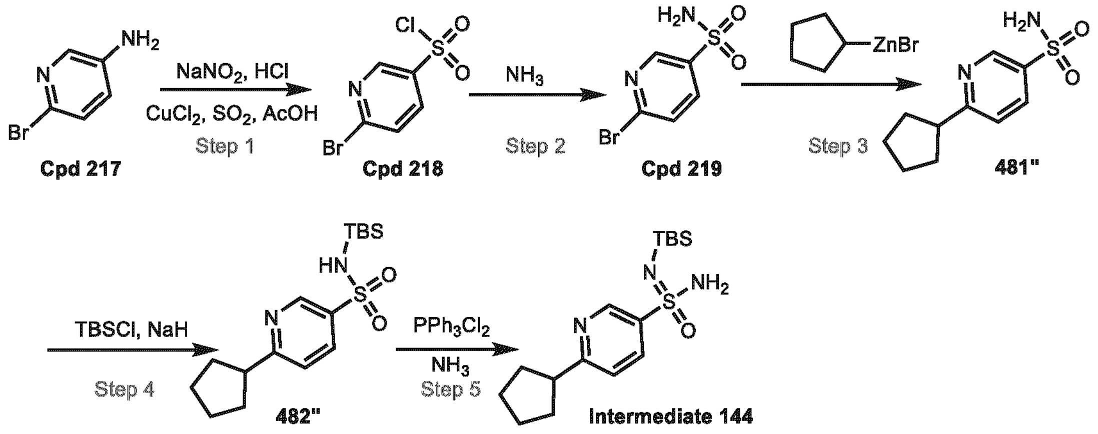


## Intermediate 144

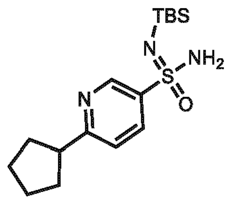

*N'*-(*tert*-butyldimethylsilyl)-6-cyclopenlylpyridine-3-sulfonimidamide

Steps 1-2 used similar procedures for converting compound **374"** to compound **375"** shown in Scheme 83 to afford compound **219** from compound **217.** MS-ESI: 239/237 (M+1).

**Step 3: 6-Cyclopentylpyridine-3-sulfonamide**

To a stirred solution of 6-bromopyridine-3-sulfonamide (2.00 g, 8.44 mmol) in THF (20 mL) in a 250-mL round-bottom flask under nitrogen were added Pd(dppf)$Cl_2$ (309mg, 0.422 mmol). Then CuI (161 mg, 0.844 mmol) was added and cyclopentylzinc(II) bromide (1M in THF, 34.0 mL) dropwise at RT under nitrogen. The resulting mixture was stirred overnight at 80 °C under nitrogen. The resulting mixture was concentrated under reduced pressure. The residue was purified by silica gel column chromatography, eluted with EtOAc/PE (1:2) to afford the title compound (1.70 g, 89.2%) as a yellow solid. MS-ESI: 225 (M-1).

Steps 4-5 used similar procedures for converting compound **362"** to intermediate **124** shown in Scheme 80 to afford intermediate **144** from compound **481".** MS-ESI: 340 (M+1).

## Scheme 100:

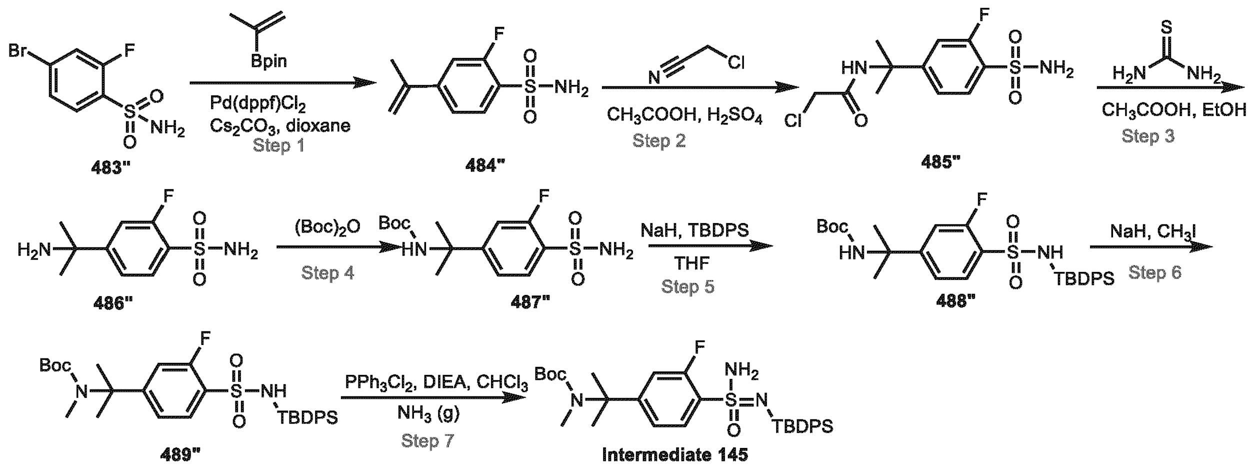


## Intermediate 145

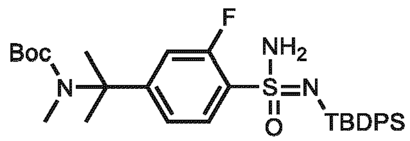

*Tert*-butyl (2-(4-(N'-(tert-butyldiphenylsilyl)sulfamidimidoyl)-3-fluorophenyl)propan-2-yl)(methyl)carbamate

**Step 1: 2-Fluoro-4-(prop-1-en-2-yl)benzenesulfonamide**

To a stirred solution of 4-bromo-2-fluorobenzenesulfonamide (10.2 g, 40.0 mmol) in dioxane (300 mL) and $H_2O$ (30 mL) in a 500-mL round-bottom flask under nitrogen was added $Cs_2CO_3$ (32.6 g, 100 mmol). Then 4,4,5,5-tetramethyl-2-(prop-1-en-2-yl)-1,3,2-dioxaborolane (16.8 g, 100 mmol) and Pd(dppf)$Cl_2$ (1.46 g, 2.00 mmol) were added. The resulting solution was stirred for 16 h at 90 °C in an oil bath. The resulting mixture was concentrated under vacuum. The residue was eluted from silica gel with EtOAc/PE (1:2). This resulted in 6.09 g (70.8%) of the title compound as yellow oil. MS-ESI: 216 (M+1).

**Step 2: 2-Chloro-*N*-(2-(3-fluoro-4-sulfamoylphenyl)propan-2-yl)acetamide**

To a stirred solution of 2-fluoro-4-(prop-1-en-2-yl)benzenesulfonamide (6.02 g, 28.0 mmol) in AcOH (150 mL) in a 500-mL round-bottom flask under nitrogen was added 2-chloroacetonitrile (21.0 g, 280 mmol), then $H_2SO_4$ (30 mL) was added dropwise at 0 °C. The resulting solution was stirred for 12 h at RT. The resulting solution was poured into 200 mL of water/ice. The pH value of the solution was adjusted to 7 with NaOH (6 M). The resulting solution was extracted with 3x300 mL of EtOAc. The organic layers were combined and dried over anhydrous $Na_2SO_4$ and concentrated under vacuum. The residue was eluted from silica gel with DCM/MeOH (10:1). This resulted in 4.42 g (51.1%) of the title compound as a yellow solid. MS-ESI: 309 (M+1).

**Step 3: 4-(2-Aminopropan-2-yl)-2-fluorobenzenesulfonamide**

To a stirred solution of 2-chloro-*N*-(2-(3-fluoro-4-sulfamoylphenyl)propan-2-yl)acetamide (4.02 g, 13.0 mmol) in EtOH (80 mL) and AcOH (16 mL) in a 250-mL round-bottom flask under nitrogen was added thiourea (1.19 g, 15.6 mmol). The resulting solution was stirred for 12 h at 80 °C. The resulting mixture was concentrated and diluted with 200 mL of water. The resulting solution was extracted with 2x200 mL of EtOAc. The organic layers were combined and dried over anhydrous $Na_2SO_4$ and concentrated under vacuum. This resulted in 3.01 g (99.7%) of the title compound as a yellow solid. MS-ESI: 233 (M+1).

**Step 4: *Tert*-butyl (2-(3-fluoro-4-sulfamoylphenyl)propan-2-yl)carbamate**

To a stirred solution of 4-(2-aminopropan-2-yl)-2-fluorobenzenesulfonamide (3.01 g, 13.0 mmol) in THF (50 mL) in a 100-mL round-bottom flask under nitrogen was added TEA (2.63 g, 26.0 mmol), followed by the addition of

$(Boc)_2O$ (4.25 g, 19.5 mmol) in THF (10 mL) dropwise at 0 °C. The resulting solution was stirred for 12 h at RT. The resulting solution was diluted with 100 mL of water. The resulting solution was extracted with 3x100 mL of EtOAc. The organic layers were combined and dried over anhydrous $Na_2SO_4$ and concentrated under vacuum. The residue was eluted from silica gel with EtOAc/PE (1:1). This resulted in 2.81 g (65.2%) of the title compound as yellow oil. MS-ESI: 333 (M+1).

**Step 5: *Tert*-butyl (2-(4-(A-(tert-butyldiphenylsilyl)sulfamoyl)-3-fluorophenyl)propan-2-yl)carbamate**

To a stirred solution of *tert*-butyl (2-(3-fluoro-4-sulfamoylphenyl)propan-2-yl)carbamate (2.80 g, 8.43 mmol) in THF (100 mL) in a 250-mL round-bottom flask under nitrogen was added NaH (60% oil dispersion, 506 mg, 12.6 mmol) in portions at 0 °C. The resulting solution was stirred for 20 min at RT. This was followed by the addition of TBDPSCl (2.78 g, 10.1 mmol) in THF (5 mL) dropwise with stirring at 0 °C. The resulting solution was stirred for 12 h at RT. The reaction was then quenched by the addition of 200 mL of water. The resulting solution was extracted with 3x200 mL of EtOAc. The organic layers were combined and dried over anhydrous $Na_2SO_4$ and concentrated under vacuum. The residue was eluted from silica gel with EtOAc/PE (1:5). This resulted in 3.0 g (62.4%) of the title compound as a yellow solid. MS-ESI: 571 (M+1).

**Step 6: *Tert*-butyl(2-(4-(*N*-(*tert*-butyldiphenylsilyl)sulfamoyl)-3-fluorophenyl)propan-2-yl)(methyl)carbamate**

To a stirred solution of *tert*-butyl (2-(4-(*N*-(tert-butyldiphenylsilyl)sulfamoyl)-3-fluorophenyl)propan-2-yl)carbamate (2.90 g, 5.08 mmol) in THF (100 mL) in a 250-mL round-bottom flask under nitrogen was added NaH (60% oil dispersion, 406 mg, 10.2 mmol) in portions at 0 °C. The resulting solution was stirred for 20 min at RT. This was followed by the addition of $CH_3I$ (866 mg, 6.10 mmol) dropwise with stirring at 0 °C. The resulting solution was stirred for 12 h at RT. The reaction was then quenched by the addition of 100 mL of water. The resulting solution was extracted with 3x100 mL of EtOAc and the combined organic layer concentrated under vacuum. The residue was eluted from silica gel with EtOAc/PE (1:5). This resulted in 2.0 g (67.3%) of the title compound as a yellow solid. MS-ESI: 585 (M+1).

**Step 7: *Tert*-butyl(2-(4-(*N'*-(*tert*-butyldiphenylsilyl)sulfamidimidoyl)-3-fluorophenyl)propan-2-yl) -(methyl)carbamate**

To a stirred solution of $PPh_3Cl_2$ (1.33 g, 4.0 mmol) in $CHCl_3$ (20 mL) in a 250-mL 3-necked round-bottom flask under nitrogen was added DIEA (1.29 g, 10.0 mmol) dropwise at 0 °C. The resulting solution was stirred for 10 min at RT. To this was added a solution of *tert*-butyl(2-(4-(N-(tert-butyldiphenylsilyl)sulfamoyl)-3-fluorophenyl)propan-2-yl)(methyl) carbamate (1.17 g, 2.00 mmol) in $CHCl_3$ (20 mL) dropwise with stirring at 0 °C. The resulting solution was allowed to react, with stirring, for an additional 30 min while the temperature was maintained at 0 °C in a water/ice bath. To the above $NH_3$ (g) was bubbled for 15 min at 0 °C. The resulting solution was allowed to react, with stirring, for an additional 30 min at RT. The solids were filtered out. The resulting mixture was concentrated under vacuum. The residue was eluted from silica gel with EtOAc/PE (1:3). This resulted in 703 mg (60.2%) of the title compound as an off-white solid. MS-ESI: 584 (M+1).

## Scheme 101:

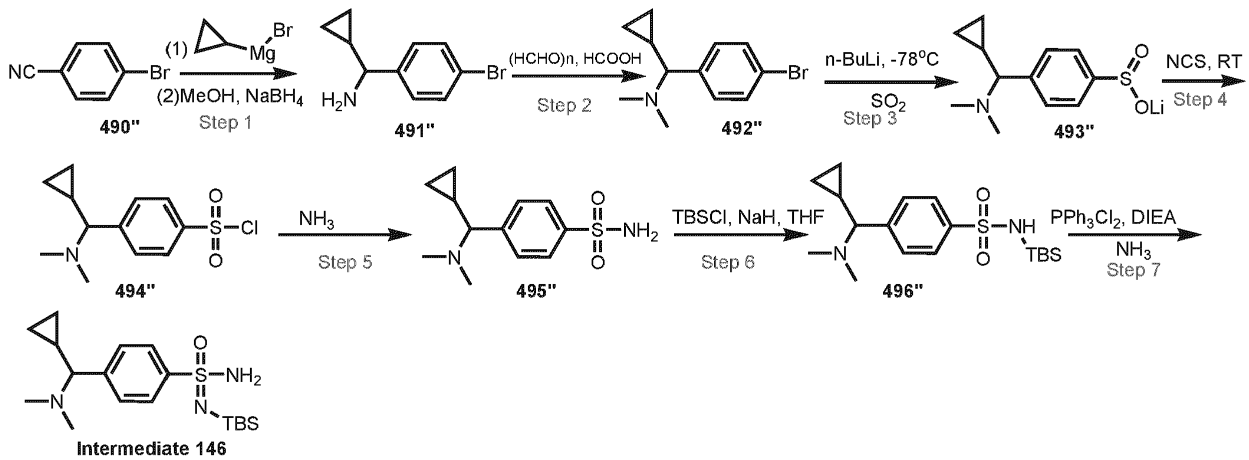

490''
491''
492''
493''
494''
495''
496''
Intermediate 146

## Intermediate 146

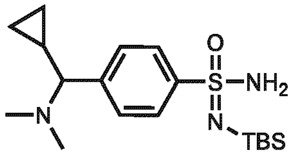

N'-(*tert*-butyldimethylsilyl)-4-(cyclopropyl(dimethylamino)methyl)benzenesulfonimidamide

**Step 1: (4-Bromophenyl)(cyclopropyl)methanamine**

To a stirred solution of 4-bromobenzonitrile (10.0 g, 54.9 mmol) in THF (200 mL) in a 1000-mL 3-necked round-bottom flask under nitrogen was added cyclopropyl magnesium bromide (1 M in THF, 165 mL, 165 mmol) dropwise at 0 °C in 20 min. The resulting solution was stirred for 5 h at 0 °C. To this was added MeOH (200 mL) at 0 °C in 5 min. To the mixture was added $NaBH_4$ (10.4 g, 275 mmol), in portions at 0 °C over 10 min. The resulting solution was allowed to react, with stirring, for an additional 16 h at RT. The reaction was then quenched by the addition of 10 mL of water. The solids were filtered out. The resulting mixture was concentrated under vacuum. The residue was eluted from silica gel with DCM/MeOH (20:1). This resulted in 9.79 g (78.9%) of the title compound as yellow oil. MS-ESI: 226/228 (M+1).

**Step 2: 1-(4-Bromophenyl)-1-cyclopropyl-*N*,*N*-dimethylmethanamine**

To a stirred solution of (4-bromophenyl)(cyclopropyl)methanamine (5.29 g, 23.4 mmol) in DCE (50 mL) in a 250-mL round-bottom flask under nitrogen was added HCOOH (10.8 g, 234 mmol) dropwise at RT. To this was added $(HCHO)_n$ (3.54 g, 39.3 mmol) at RT. The resulting solution was stirred for 1 h at 80 °C. The reaction was then quenched by the addition of 150 mL of water. The resulting solution was extracted with 3x200 mL of DCM. The organic layers were combined and dried over anhydrous $Na_2SO_4$. The solids were filtered out. The resulting mixture was concentrated under vacuum. The residue was eluted from silica gel with EtOAc/PE (2:1). This resulted in 3.30 g (55.4%) of the title compound as a white solid. MS-ESI: 254/256 (M+1).

Steps 3-7 used similar procedures for converting compound **369"** to Intermediate **126** shown in Scheme 82 to afford intermediate **146** from compound **492"**. MS-ESI: 368 (M+1).

**Table 31.** Intermediate 147 was prepared using similar procedures for converting compound **490"** to Intermediate **146** shown in Scheme 101 from appropriate starting materials.

| Intermediate # | Structure | IUPAC Name | Exact Mass[M+H]$^+$ |
|---|---|---|---|
| **Intermediate 147** | 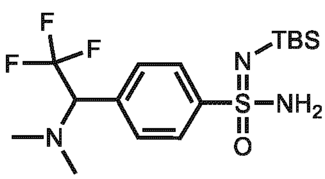 | N'-(tert-butyldimethylsilyl)-4-(1-(dimethylamino)-2,2,2-trifluoroethyl) benzenesulfonimidamide | 396 |

**Scheme 102:**

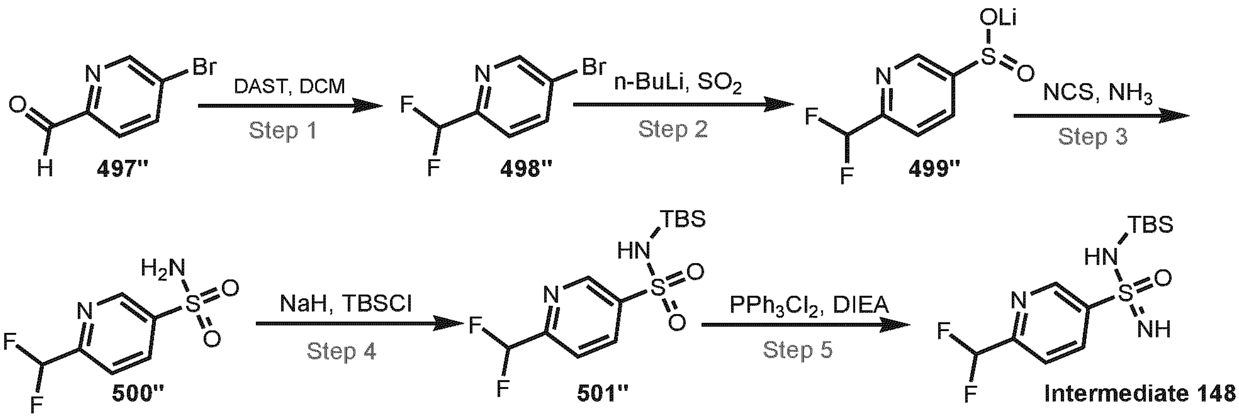


**Intermediate 148**

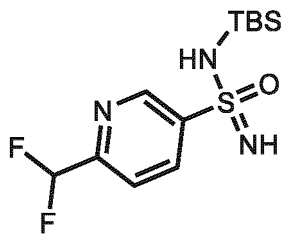

<u>*N*-(*tert*-butyldimethylsilyl)-6-(difluoromethyl)pyridine-3-sulfonimidamide</u>

**Step 1: 5-Bromo-2-(difluoromethyl)pyridine**

To a stirred solution of 5-bromopicolinaldehyde (1.86 g, 10.0 mmol) in THF (50 mL) in a 250-mL round-bottom flask was added DAST (3.22 g, 20.0 mmol) dropwise at 0 °C. The resulting solution was stirred for 6 h at RT. The reaction was then poured into 100 mL of water/ice. The resulting solution was extracted with 3x200 mL of EtOAc and the organic layers combined and dried over anhydrous $Na_2SO_4$ and concentrated under vacuum. This resulted in 2.10 g (crude) of the title compound as yellow oil. MS-ESI: 208/210 (M+1).

Steps 2-5 used similar procedures for converting compound **369"** to intermediate **126** shown in Scheme 82 to afford intermediate **148** from compound **498"**. MS-ESI: 322 (M+1).

## Scheme 103

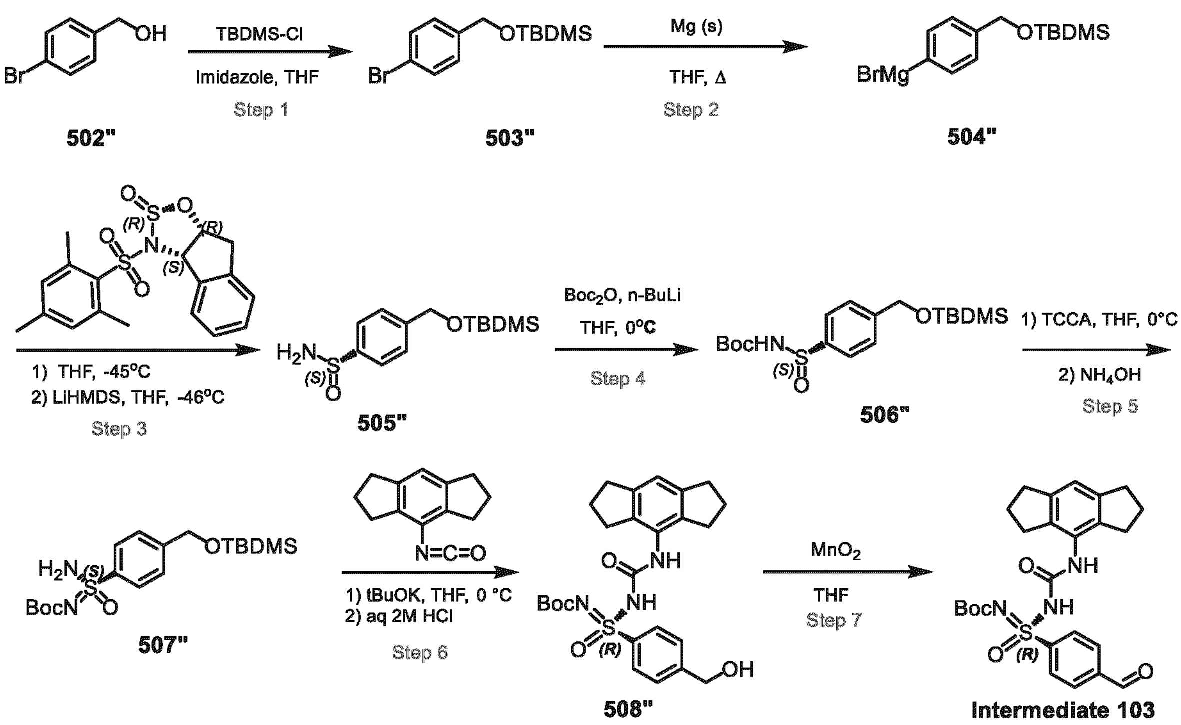


## Intermediate 103

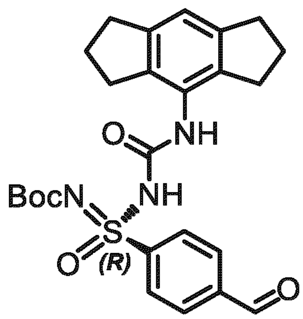

tert-butyl (*R*)-((4-formylphenyl)(3-(1,2,3,5,6,7-hexahydro-*s*-indacen-4-yl)ureido)(oxo)-λ6-sulfanylidene)carbamate

**Step 1: ((4-bromobenzyl)oxy)(*tert*-butyl)dimethylsilane**

Into a 500-mL round bottom flask was placed 4-bromobenzyl alcohol (15 g, 80.2 mmol), imidazole (12.01 g, 176.44 mmol) in DCM (270 mL). TBSCl (13.08 g, 84.21 mmol) was added in one portion and the mixture stirred overnight at RT. MeOH (10 mL) was added and the reaction mixture was stirred for 30 min. The mixture was transferred to a separatory funnel, washed with water (2 x 100 mL) and aq. $NaHCO_3$ (100 mL). The organic phase was dried over anhydrous $Na_2SO_4$ and concentrated under reduced pressure affording the title compound (23.75 g, 98%) as a pale yellow oil. The crude product was used in the next step.

**Step 2: (4-(((*tert*-butyldimethylsilyl)oxy)methyl)phenyl)magnesium bromide**

Magnesium turnings (888 mg, 35.6 mmol) were suspended in a flame-dried 100-mL round bottom flask. Dry THF (10 mL) was added. A solution of ((4-bromobenzyl)oxy)(*tert*-butyl)dimethylsilane (10 g, 33.19 mmol) in 30 mL THF was prepared and was added to the Mg-containing flask via dropping funnel. The mixture was refluxed for 2 h then cooled to RT and left to settle overnight. The Grignard solution was titrated against a known quantity of menthol and a

catalytic amount of 1,10-phenanthroline in THF. Final titer (average of two titrations) was 0.51 M.

**Step 3: (*S*)-4-(((*tert*-butyldimethylsilyl)oxy)methyl)benzenesulfinamide**

In a 100 mL round bottom flask, a solution of (4-(((tert-butyldimethylsilyl)oxy)methyl) phenyl)magnesium bromide (prepared using the procedure described in the step above; 0.86 M in THF, 27 mL, 23 mmol) was diluted with THF (71 mL) and cooled to -46 °C (acetonitrile-dry ice bath). The resulting milky suspension was added via cannula to a solution of (2*R*,3a*S*,8a*R*)-3-(mesitylsulfonyl)-3,3a,8,8a-tetrahydroindeno- [1,2-*d*][1,2,3]oxathiazole 2-oxide (5.88 g, 15.58 mmol; prepared using the procedure detailed in Org. Synth. 2006, 83, 131) in THF (68 mL) keeping the internal temperature of the mixture below -42 °C. After addition, the reaction mixture was stirred at -46 °C for 1 hour. LiHMDS (1 M in toluene, 46.7 mL, 46.7 mmol) was added and the reaction mixture was warmed-up to 0 °C and stirred for 1 h. A saturated solution of $NaHCO_3$ (50 mL) was added and the mixture stirred for 15 min. EtOAc (150 mL) and half-saturated brine (50 mL) were added and the organic layer was separated. The aqueous layer was extracted with EtOAc (2x100 mL). The combined organic layers were dried over anhydrous $Na_2SO_4$ and concentrated under reduced pressure. The residue was purified by flash chromatography with an isocratic mixture of 6% (10% $NH_4OH$ in MeOH) in chloroform affording 2.3 g of the title compound (75%) as an off-white solid. MS-ESI: 286.1 (M+1).

**Step 4: *tert*-butyl (5)-((4-(((*tert*-butyldimethylsilyl)oxy)methyl)phenyl)sulfinyl)carbamate**

In a 500-mL round bottom flask was dissolved (*S*)-4-(((*tert*-butyldimethylsilyl)oxy)methyl) benzenesulfinamide (12.0 g, 42.03 mmol) in THF (350 ml). *n*-BuLi (2.5 M in THF, 35.2 ml, 88.3 mmol) was then added at -78 °C . The mixture was stirred at the same temperature for 10 min. A solution of di-*tert*-butyl carbonate (9.63 g, 44.13 mmol) in THF (21 mL) was added in one portion and the reaction mixture warmed up to 0 °C with stirring. The solution was stirred at 0 °C for 1 h. Formic acid (98%, 4.5 mL, 88.3 mmol) was added, the reaction mixture was stirred 10 min and then diluted with EtOAc (350 mL). Half-saturated brine (100 mL) was added and the phases were separated. The aqueous phase was extracted with EtOAc (2x200 mL). The organic phases were combined, dried over anhydrous $Na_2SO_4$ and concentrated under reduced pressure. The crude was purified by flash chromatography using a gradient of 5-10 % MeOH in DCM affording 12.3 g of the title compound (76 %) as a transparent syrup. MS-ESI: 286.0 (M-Boc).

**Step 5: *tert*-butyl (*S*)-(amino(4-(((*tert*-butyldimethylsilyl)oxy)methyl)phenyl)(oxo)-$\lambda^6$-sulfanylidene)- carbamate**

To a cooled (-10 °C) solution of *tert*-butyl (*S*)-((4-(((*tert*-butyldimethylsilyl)oxy)methyl)phenyl) sulfinyl)carbamate (12.20 g, 31.9 mmol) in THF (50 ml) in a 100-mL round bottom flask was added TCCA (2.52 g, 10.84 mmol, 0.34 eq) in portions. Internal temperature was kept below -5 °C. The mixture was stirred at 0 °C for 60 min, followed by 1 h at RT. Concentrated ammonium hydroxide (14 M, 34.2 mL) was added dropwise and the reaction left to warm to RT overnight. The reaction mixture was diluted with ethyl acetate (400 mL) and washed the organic phase with half-saturated brine (100 mL). The aqueous phase was extracted with EtOAc (300 ml) and the combined organic phases were dried ($Na_2SO_4$), evaporated and the crude was purified by flash chromatography using a gradient of 5-50% EtOAc in hexanes to afford 10 g of the title compound (78%) as a light brown syrup. MS-ESI: 423.1 (M+Na).

**Step 6: *tert*-butyl (*R*)-((3-(1,2,3,5,6,7-hexahydro-s-indacen-4-yl)ureido)(4-(hydroxymethyl)phenyl) (oxo)-$\lambda^6$-sulfanylidene)carbamate**

To a 0 °C (ice bath) solution of *tert*-butyl (*S*)-(amino(4-(((*tert*-butyldimethylsilyl)oxy)methyl) phenyl)(oxo)- $\lambda^6$-sulfanylidene)carbamate (9.77 g, 24.39 mmol) in THF (244 ml) was added dropwise potassium *tert*-butoxide (1 M in THF, 25.6 ml, 25.6 mmol) keeping the internal temperature below -2.2 °C. The reaction mixture was stirred for 1 hour at 0°C. A solution of 4-isocyanato-1,2,3,5,6,7-hexahydro-s-indacene (5.1 g, 25.61 mmol) in THF (66 mL) was added dropwise and the mixture stirred at RT for 1 h, then formic acid was added (1 mL, 25.61 mmol) and the solution stirred for 10 min. The reaction mixture was diluted with EtOAc (300 mL) and washed with half-saturated brine (100 mL). The aqueous phase was extracted with EtOAc (250 mL) and the combined organic phases were dried over anhydrous $Na_2SO_4$, and concentrated under reduced pressure affording the crude TBS ether (13.95 g).

The TBS ether was dissolved in MeOH (211 mL), cooled to 0°C and aqueous HCl (2 M, 280 mL, 558.67 mmol) was added and the reaction mixture was stirred 2 hours at 0°C. The crude mixture was diluted with EtOAc (400 mL), transferred to a separatory funnel and washed with water (100 mL). A white solid precipitated out of solution and the heterogeneous mixture was filtered. The solid was washed with water (100 mL) and EtOAc (100 mL), and dried overnight at low pressure affording 5.96 g of the title compound (53 % over two steps) as a white solid. MS-ESI: 486.3 (M+1).

**Step 7: *tert*-butyl (*R*)-((4-formylphenyl)(3-(1,2,3,5,6,7-hexahydro-s-indacen-4-yl)ureido)(oxo)-$\lambda^6$- sulfanylidene)carbamate**

In a flame-dried 500-mL round bottom flask, $MnO_2$ (12 g, 143 mmol) was suspended in dry toluene (250 mL) under an argon atmosphere. The solvent was removed under reduced pressure at 50 °C, then the flask was kept at high vacuum pump for 1 h. A solution of *tert*-butyl (*R*)-((3-(1,2,3,5,6,7-hexahydro-*s*- indacen-4-yl)ureido)(4-(hydroxymethyl)phenyl)(oxo)-$\lambda^6$-sulfanylidene)carbamate (1.5 g, 3.089 mmol) in dry THF (130 mL) was added to the flask containing the $MnO_2$ under an argon atmosphere. The resulting mixture was vigorously stirred at RT for 3 h. The mixture was filtered through a Celite pad which was washed with MeOH (150 mL) and the filtrate and wash were concentrated under reduced pressure at 30 °C, resulting in isolation of 1.22 g of the title compound as a pale yellow solid (82%). MS-ESI: 484.4 (M+1).

**Scheme 104:**

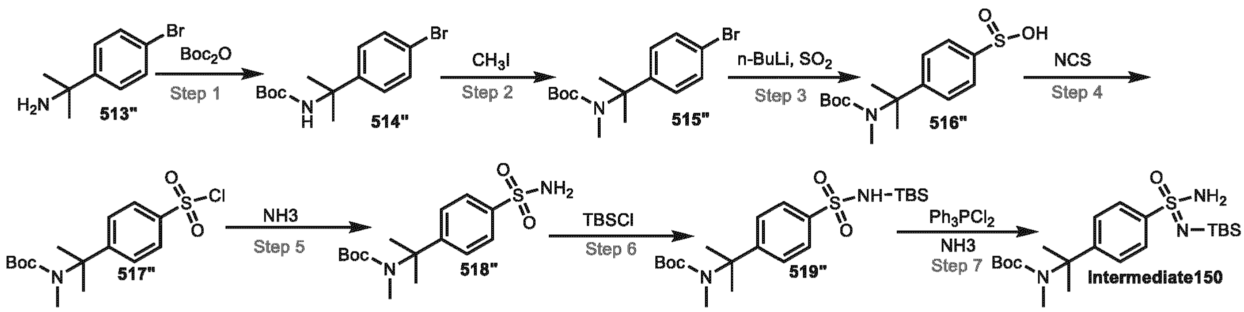


## Intermediate 150

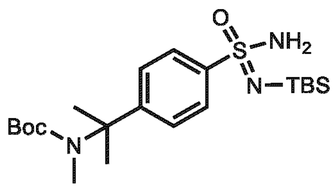

<u>Tert-butyl (2-(4-(*N'*-(tert-butyldimethylsilyl)sulfamidimidoyl)phenyl)propan-2-yl)(methyl)carbamate</u>

**Step 1: *Tert*-butyl (2-(4-bromophenyl)propan-2-yl)carbamate**

To a stirred solution of 2-(4-bromophenyl)propan-2-amine (5.0 g, 23.4 mmol) in DCM (60 mL) in a 250-mL round-bottom flask was added TEA (4.73 g, 46.8 mmol), followed by the addition of $(Boc)_2O$ (8.94 g, 41.0 mmol) in DCM (10 mL) dropwise at 0 °C. The resulting solution was stirred for 15 h at RT. The solution was concentrated under vacuum. The residue was eluted from silica gel with EtOAc/PE (3/7). This resulted in 6.81 g (92.7%) of the title compound as a yellow solid. MS-ESI: 314/316 (M+1).

**Step 2: *Tert*-butyl (2-(4-bromophenyl)propan-2-yl)(methyl)carbamate**

To a stirred solution of *tert*-butyl (2-(4-bromophenyl)propan-2-yl)carbamate (3.14 g, 10.0 mmol) in THF (100 mL) in a 250-mL round-bottom flask under nitrogen was added NaH (60% oil dispersion, 800 mg, 20.0 mmol) in portions at 0 °C. The resulting solution was stirred for 20 min at RT. This was followed by the addition of $CH_3I$ (4.26 g, 30.0 mmol) dropwise with stirring at 0 °C The resulting solution was allowed to react, with stirring, for an additional 16 h at RT. The reaction was then quenched by the addition of 100 mL of water. The resulting solution was extracted with 3x100 mL of EtOAc and the organic layers were combined then dried over anhydrous magnesium sulfate and concentrated under vacuum. The residue was eluted from silica gel with EtOAc/PE (1/4). This resulted in 2.47 g (75.2%) of the title compound as a yellow solid. MS-ESI: 328/330 (M+1).

Steps 3-7 used similar procedures for converting compound **369"** to intermediate **126** shown in Scheme 82 to afford intermediate **150** from compound **515"**. MS-ESI: 442 (M+1).

## Scheme 105:

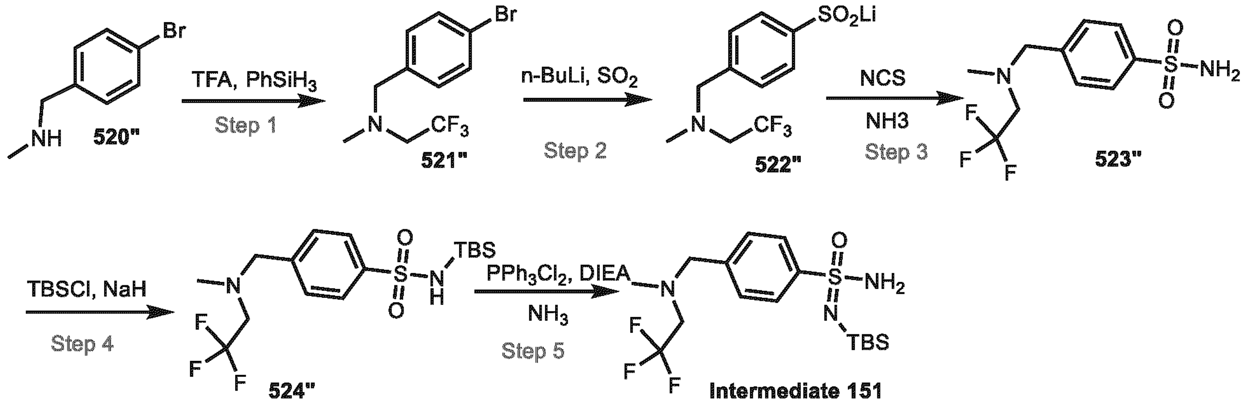


## Intermediate 151

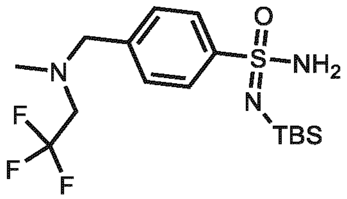

<u>*N'*-(*tert*-butyldimethylsilyl)-4-((methyl(2,2,2-trifluoroethyl)amino)methyl)benzenesulfonimidamide</u>

**Step 1: *N*-(4-bromobenzyl)-2,2,2-trifluoro-*N*-methylethan-1-amine**

To a stirred solution of 1-(4-bromophenyl)-*N*-methylmethanamine (3.90 g, 19.5 mmol) in DCM (200 mL) in a 500-mL round-bottom flask under nitrogen was added TFA (6.67 g, 58.5 mmol) and $PhSiH_3$ (4.21 g, 39.0 mmol) at RT. The resulting solution was stirred for 12 h at 40 °C in an oil bath. The reaction mixture was cooled to 0 °C with a water/ice bath. The pH value of the solution was adjusted to 7 with NaOH (1 M). The resulting solution was extracted with 3x200 mL of DCM, the combined organic layers were dried over anhydrous $Na_2SO_4$ and concentrated under vacuum. The residue was eluted from silica gel with EtOAc/PE (1:10). This resulted in 3.80 g (69.1%) of the title compound as yellow oil. MS-ESI: 282 (M+1).

Steps 2-5 used similar procedures for converting compound **369"** to intermediate **126** shown in Scheme 82 to afford intermediate **151** from compound **521"**. MS-ESI: 396 (M+1).

**Table 32**. Intermediate 152 was prepared using similar procedures for converting compound **520"** to Intermediate **151** shown in Scheme 105 from appropriate starting materials.

| Intermediate # | Structure | IUPAC Name | Exact Mass [M+H] + |
|---|---|---|---|
| **Intermediate 152** | 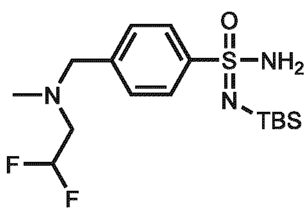 | N'-(tert-butyldimethylsilyl)-4-(((2,2-difluoroethyl)(methyl)amino)methyl) benzenesulfonimidamide | 378 |

## Scheme 106:

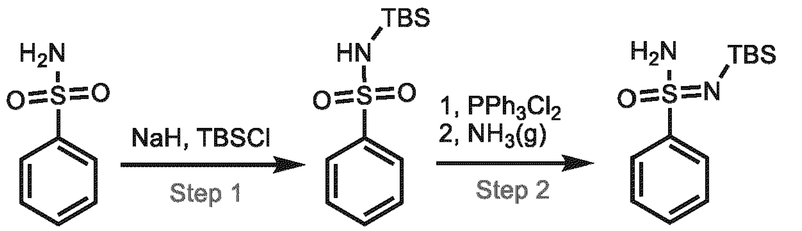


525"  526"  **Intermediate 153**

## Intermediate 153

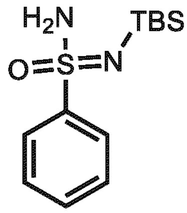

*N'*-(*tert*-butyldimethylsilyl)benzenesulfonimidamide

Steps 1-2 used similar procedures for converting compound **362"** to intermediate **124** shown in Scheme 80 to afford intermediate **153** from compound **525"**. MS-ESI: 271 (M+1).

## Scheme 107:

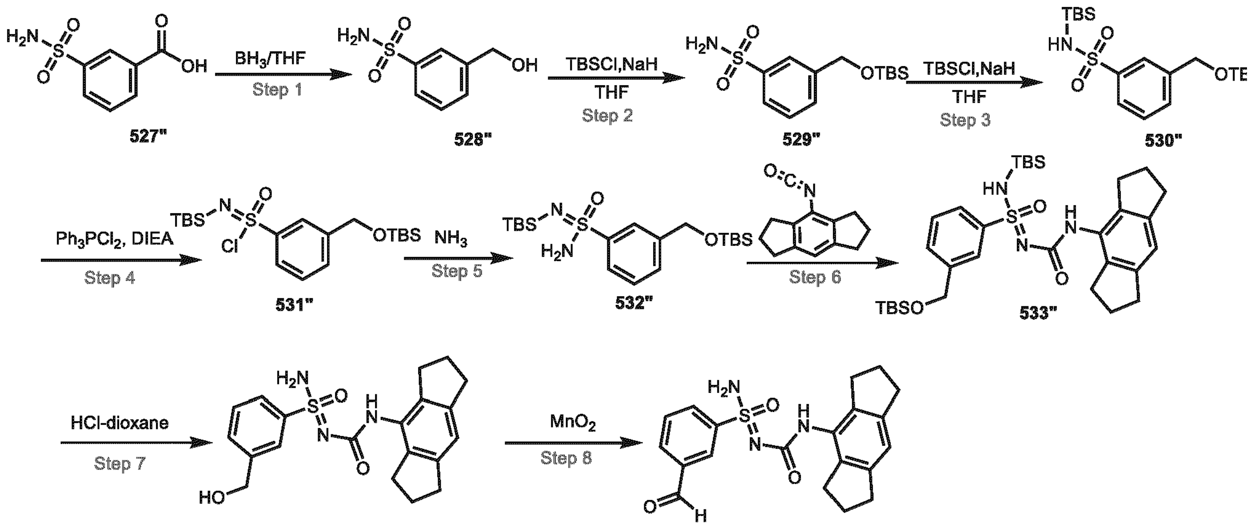


527"  528"  529"  530"  531"  532"  533"  534"  **Intermediate 154**

## Intermediate 154

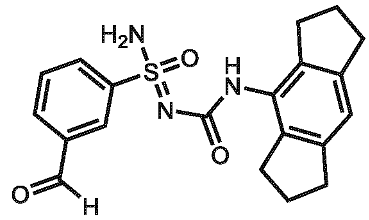

3-Formyl-*N'*-((1,2,3,5,6,7-hexahydro-*s*-indacen-4-yl)carbamoyl)benzenesulfonimidamide

**Step 1: 3-(Hydroxymethyl)benzenesulfonamide**

To a stirred solution of 3-sulfamoylbenzoic acid (10 g, 49.7 mmol) in THF (200 mL) in a 1000 mL 3-necked round-bottom flask under nitrogen was added $BH_3$-THF (1 M, 198 mL, 198 mmol) dropwise at 0 °C in an ice bath. The resulting solution was stirred for 30 min at 0 °C and then allowed to react, with stirring, for an additional 4 h at RT. The reaction was then quenched by the addition of 200 mL of MeOH. The resulting solution was concentrated under vacuum. The residue was eluted from silica gel with EtOAc/PE (1:1). This resulted in 8.8 g (94.6%) of the title compound as yellow oil. MS-ESI: 186 (M-1).

**Step 2: 3-(((Tert-butyldimethylsilyl)oxy)methyl)benzenesulfonamide**

To a stirred solution of 3-(hydroxymethyl)benzenesulfonamide (5.0 g, 26.7 mmol) in THF (50 mL) in a 250-mL round-bottom flask under nitrogen was added NaH (60% oil dispersion, 1.28 g, 53.4 mmol) and TBSCl (6.04 g, 40.1 mmol) in portions at 0 °C in a water/ice bath. The resulting solution was stirred for 12 h at RT. The reaction was then quenched by the addition of 100 mL of water/ice. The resulting solution was extracted with 3x150 mL of EtOAc and the organic layers were combined and dried over anhydrous $Na_2SO_4$ and concentrated under vacuum. The residue was eluted from silica gel with EtOAc/PE (2:1). This resulted in 5.8 g (72%) of the title compound as a white solid. MS-ESI: 302 (M+1).

Steps 3-5 used similar procedures for converting compound **362"** to intermediate **124** shown in Scheme 80 to afford compound **532"** from compound **529"**. MS-ESI: 415 (M+1).

**Step 6: N-(tert-butyldimethylsilyl)-3-(((tert-butyldimethylsilyl)oxy)methyl)-N'-((1,2,3,5,6,7-hexahydro -s-indacen-4-yl)carbamoyl)benzenesulfonimidamide**

To a stirred solution of N'-(tert-butyldimethylsilyl)-3-(((tertbutyldimethylsilyl)oxy)methyl)benzene- sulfonimidamide (2.0 g, 4.82 mmol) in THF (20 mL) in a 100-mL 3-necked round-bottom flask under nitrogen was added NaH (60% oil dispersion, 0.23 g, 9.64 mmol) in portions at 0 °C. The resulting solution was stirred for 15 min at 0 °C in a water/ice bath. Then to the stirred solution was added 4-isocyanato-1,2,3,5,6,7-hexahydro-s-indacene (0.96 g, 4.82 mmol) in THF (5 mL) dropwise at 0 °C. The resulting solution was stirred for 2 h at RT. The reaction was then quenched by the addition of 15 mL of water/ice. The resulting solution was extracted with 3x50 mL of EtOAc and the organic layers were combined and dried over anhydrous $Na_2SO_4$ and concentrated under vacuum. This resulted in 2.25 g (75.9%) of the title compound as a white solid. MS-ESI: 614 (M+1).

**Step 7: N'-((1,2,3,5,6,7-hexahydro-s-indacen-4-yl)carbamoyl)-3-(hydroxymethyl)benzenesulfonimidamide**

To a stirred solution of N-(tert-butyldimethylsilyl)-3-(((tert-butyldimethylsilyl)oxy)methyl)-N'-((1,2,3,5,6,7- hexahydro-s-indacen-4-yl) carbamoyl)benzenesulfonimidamide (500 mg, 0.81 mmol) in THF (10 mL) in a 100-mL round-bottom flask under nitrogen was added HCl in 1,4-dioxane (4 M, 10.0 mL, 40.0 mmol) dropwise at 0 °C. The resulting solution was stirred for 2 h at RT. The resulting mixture was concentrated under vacuum. The residue was eluted from silica gel with DCM/MeOH (10/1). This resulted in 125 mg (39.8%) of the title compound as a white solid. MS-ESI: 386 (M+1).

**Step 8: 3-Formyl-N'-((1,2,3,5,6,7-hexahydro-s-indacen-4-yl)carbamoyl)benzenesulfonimidamide**

To a stirred solution of N'-((1,2,3,5,6,7-hexahydro-s-indacen-4-yl)carbamoyl)-3-(hydroxymethyl)benzene- sulfonimidamide (120 mg, 0.31 mmol) in THF (10 mL) in a 50-mL round-bottom flask under nitrogen was added $MnO_2$ (541 mg, 6.23 mmol) at RT. The resulting solution was stirred for 12 h at RT. The solids were filtered out. The resulting mixture was concentrated under vacuum. The residue was eluted from silica gel with DCM/MeOH (10:1). This resulted in 80 mg (67.0%) of the title compound as a white solid. MS-ESI: 384 (M+1).

## Scheme 108:

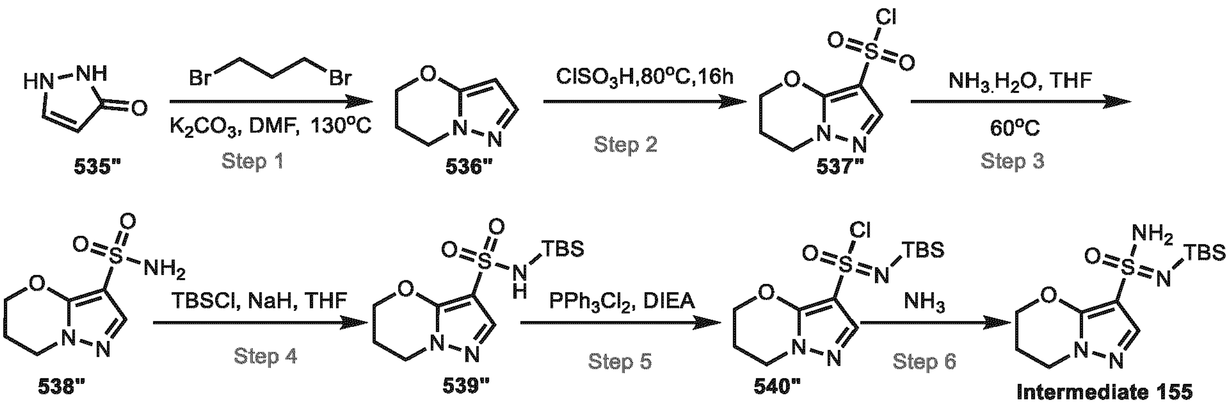


535" 536" 537" 538" 539" 540" Intermediate 155

## Intermediate 155

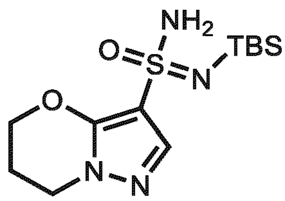

N'-(*tert*-butyldimethylsilyl)-6,7-dihydro-5H-pyrazolo[5,1-b][1,3]oxazine-3-sulfonimidamide

**Step 1: 6,7-Dihydro-5H-pyrazolo[5,1-b][1,3]oxazine**

To a stirred solution of 1,2-dihydro-3H-pyrazol-3-one (42 g, 500 mmol) in DMF (500 mL) in a 1 L 3-neck flask was added $K_2CO_3$ (138 g, 1.0 mol) in portions and 1,3-dibromopropane (111 g, 550 mmol) was added dropwise at RT. The resulting mixture was stirred for 16 h at 130 °C under nitrogen. The insoluble was filtered out, the filtrate was poured into 1500 mL of water, extracted with 3x500 mL of EtOAc. The organic layers combined and dried over anhydrous $Na_2SO_4$ and concentrated under reduced pressure. The residue was eluted from silica gel with PE/EtOAc (20:1) to afford 24.8 g (40%) the title compound as a yellow solid. MS-ESI: 125 (M+1).

**Step 2: 6,7-Dihydro-5H-pyrazolo[5,1-b][1,3]oxazine-3-sulfonyl chloride**

To a stirred solution of 6,7-dihydro-5H-pyrazolo[5,1-b][1,3]oxazine (24 g, 194 mmol) in chlorosulfonic acid (143 mL) in a 1000-mL 3-necked round-bottom flask under nitrogen. The resulting solution was stirred for 16 h at 80 °C under nitrogen. The reaction mixture was poured into 1500 mL of water/ice very slowly, extracted with 3x500 mL of EtOAc. The organic layers were combined and dried over anhydrous $Na_2SO_4$ and concentrated under reduced pressure. The residue was washed with 300 mL of PE. This resulted in the title compound (28.1 g, 65.0%) as a yellow solid. MS-ESI: 223/225 (M+1).

**Step 3: 6,7-Dihydro-SH-pyrazolo[5,1-b][1,3]oxazine-3-sulfonamide**

To a stirred solution of ammonia (30%wt., 100 mL) in a 1000-mL 3-necked round-bottom flask under nitrogen was added 6,7-dihydro-5H-pyrazolo[5,1-b][1,3] oxazine-3-sulfonyl chloride (28 g, 126 mmol) in THF (100 mL) dropwise at RT. The resulting solution was stirred for 16 h at 60 °C under nitrogen. The organic solvent was removed by centration under reduced pressure and then extracted with 3x500 mL of EtOAc. The organic layers were combined and dried over

anhydrous $Na_2SO_4$ and concentrated under reduced pressure. The residue was eluted from gel column with PE/EtOAc (1:1). This resulted in 16.9 g (66%) of the title compound as a light yellow solid. MS-ESI: 202 (M-1).

Steps 4-6 used similar procedures for converting compound **362"** to intermediate **124** shown in Scheme 80 to afford intermediate **155** from compound **538"**. MS-ESI: 317 (M+1).

## Scheme 109:

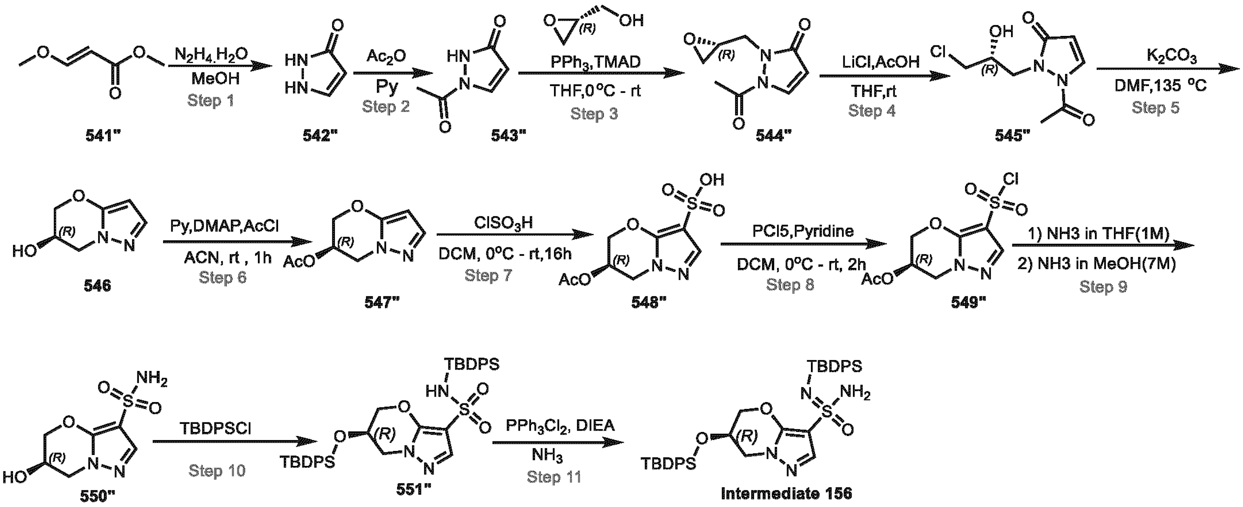


## Intermediate 156

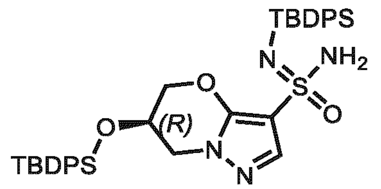

(6*R*)-N'-(tert-butyldiphenylsilyl)-6-((tert-butyldiphenylsilyl)oxy)-6,7-dihydro-5H-pyrazolo[5,1-b][1,3]oxazine-3-sulfonimidamide

### Step 1: 1,2-Dihydropyrazol-5-one

To a 5 L 4-neck flask containing a solution of methyl (*E*)-3-methoxyacrylate (2000 g, 17.2 mol) in MeOH (2.0 L) was added hydrazine hydrate (921 g, 18.4 mol) dropwise at RT under nitrogen. The resulting mixture was stirred for 90 min at 60 °C under nitrogen. The resulting mixture was concentrated under reduced pressure. This resulted in the title compound (1467 g, 68%wt, yield 69%) as an off-white solid. MS-ESI: 85 (M+1).

### Step 2: 2-Acetyl-1,2-dihydropyrazol-5-one

To a 10 L 4-neck flask containing a solution of 1,2-dihydropyrazol-5-one (1467 g, 68%wt, 11.9 mol) in pyridine (6.0 L) was added $Ac_2O$ (1214 g, 11.9 mol) at RT under nitrogen. The resulting mixture was stirred for 1.5 h at 95 °C under nitrogen. The resulting mixture was concentrated under reduced pressure. The residue was slurry with MeOH (1x3000 mL). The resulting mixture was filtered, the filter cake was washed with MeOH (1x500 mL). The filter cake was dried under reduced pressure. This resulted in the title compound (1630 g, 78%wt, 85%) as an off-white solid. MS-ESI: 127 (M+1). $^1H$ NMR (400 MHz, DMSO-$d_6$) δ 8.12 (d, J = 3.0 Hz, 1H), 6.00 (d, J = 3.0 Hz, 1H), 2.48 (s, 3H), 2.44 (s, 1H).

### Step 3: (R)-2-acetyl-1-(oxiran-2-ylmethyl)-1,2-dihydropyrazol-5-one

To a 10 L 4-neck flask containing a solution of 2-acetyl-1,2-dihydropyrazol-5-one (400 g, 78%wt, 3.17 mol) and

R-glycidol (246 g, 3.33 mol) in THF (4.0 L), to the stirred solution was added $PPh_3$ (915 g, 3.49 mol). To the above mixture was added TMAD (705 g, 3488 mmol) in portions at 0 °C. The resulting mixture was stirred for additional 1 h at RT. The resulting mixture was quenched with 1x4.0 L of water. The aqueous layer was extracted with EtOAc (3x1.0 L). The organic layers were combined and washes with brine (1 L), dried over $Na_2SO_4$ and concentrated under reduced pressure. The crude was slurry in PE/EtOAc (10:1) (16 L) for 4 h. The resulting mixture was filtered. The filter cake was washed with PE/EtOAc (10:1) (1x1000 mL). The filtrate was concentrated under reduced pressure. This resulted in the title compound (470 g, 84%wt, 87%) as an off-white solid. MS-ESI: 183 (M+1). $^1$H NMR (400 MHz, DMSO-$d_6$) $\delta$ 8.27 (d, J = 3.0 Hz, 1H), 6.27 (d, J = 3.0 Hz, 1H), 4.56 (dd, J = 11.8, 2.7 Hz, 1H), 4.02 (dd, J = 11.8, 6.8 Hz, 1H), 3.40-3.34 (m, 1H), 2.86 (dd, J = 5.1, 4.3 Hz, 1H), 2.74 (dd, J = 5.1, 2.6 Hz, 1H), 2.54 (s, 3H).

**Step 4: (R)-2-acetyl-1-(3-chloro-2-hydroxypropyl)-1,2-dihydropyrazol-5-one**

To a 10 L 3-neck flask was placed a solution of (R)-2-acetyl-1-(oxiran-2-ylmethyl)-1,2-dihydropyrazol -5-one (500 g, 84%wt, 2.74 mol) in THF (2.5 L), to the stirred solution was added AcOH (494 g, 8233 mmol) dropwise and LiCl (186 g, 4391 mmol) in portions at 0 °C under nitrogen. The resulting mixture was stirred for 16 h at RT under nitrogen. The reaction was quenched with water at RT. The aqueous layer was extracted with EtOAc (3x3.0 L). The organic layers were combined and washed with 2x3.0 L of sat. $NaHCO_3$ and 5.0 L of brine. The organic layer was dried over $Na_2SO_4$ and concentrated under reduced pressure. This resulted in the title compound (552 g, 82%wt, yield 90%) as an off-white solid. MS-ESI: 219 (M+1). $^1$H NMR (400 MHz, $CDCl_3$) $\delta$ 8.10 (d, J = 3.0 Hz, 1H), 6.02 (d, J = 3.0 Hz, 1H), 4.44 (d, J = 5.0 Hz, 2H), 4.29-4.23 (m, 1H), 3.81- 3.67 (m, 2H), 2.61 (s, 3H).

**Step 5: (R)-6,7-dihydro-5H-pyrazolo[5,1-b][1,3]oxazin-6-ol**

To a 10 L 3-neck flask was placed a solution of (R)-2-acetyl-1-(3-chloro-2-hydroxypropyl)-1,2-dihydropyrazol-5-one (500 g, 82%wt, 2.29 mol) in DMF (5.0 L), to the stirred solution was added $K_2CO_3$ (948 g, 6.86 mol) under nitrogen. The resulting mixture was stirred for 16 h at 135 °C under nitrogen and concentrated under reduced pressure. The residue was purified by silica gel column chromatography, eluted with DCM/MeOH (20:1) to afford (152 g, yield 58%) of the title compound as an off-white solid. MS-ESI: 141 (M+1). $^1$H NMR (400 MHz, MeOH-$d_4$) $\delta$ 7.11 (d, J = 2.1 Hz, 1H), 5.31 (d, J = 2.1 Hz, 1H), 4.19 - 4.12 (m, 1H), 4.12 - 3.98 (m, 3H), 3.90 - 3.81 (m, 1H).

**Step 6: (R)-6,7-dihydro-SH-pyrazolo[5,1-b][1,3]oxazin-6-yl acetate**

To a stirred solution of (R)-6,7-dihydro-5H-pyrazolo[5,1-b][1,3]oxazin-6-ol (55.0 g, 392 mmol) in MeCN (825 mL) in a 2-L 3-necked round-bottom flask under nitrogen was added pyridine (93.1 g, 1.18 mol) and DMAP (4.79 g, 39.2 mmol). This was followed by the addition of acetyl chloride (43.1 g, 549 mmol) dropwise with stirring at 0 °C. The resulting solution was stirred for 2 h at RT. LCMS showed reaction was completed. The resulting mixture was concentrated directly. The residue was eluted from silica gel with EtOAc/PE (1:4). This resulted in 58 g (81%) of the title compound as a light yellow solid. MS-ESI: 183 (M+1). $^1$H NMR (400 MHz, $CDCl_3$) $\delta$ 7.39 (d, J = 2.0 Hz, 1H), 5.57 (d, J = 2.0 Hz, 1H), 5.45-5.36 (m, 1H), 4.49-4.41 (m, 1H), 4.40-4.27 (m, 2H), 4.24 (dd, J = 12.1, 1.5 Hz, 1H), 2.14 (s, 3H).

**Step 7: (R)-6-acetoxy-6,7-dihydro-SH-pyrazolo[5,1-b][1,3]oxazine-3-sulfonic acid**

To a stirred solution of (R)-6,7-dihydro-5H-pyrazolo[5,1-b][1,3]oxazin-6-yl acetate (58.0 g, 318 mmol) in DCM (120 mL) in a 2-L 3-necked round-bottom flask under nitrogen.was added chlorosulfonic acid (81.3 g, 700 mmol) dropwise at 0 °C. The resulting solution was stirred for 12 h at RT. LCMS showed the conversion was completed. The reaction mixture was used directly in the next step. MS-ESI: 263 (M+1).

**Step 8: (R)-3-(chlorosulfonyl)-6,7-dihydro-5H-pyrazolo[5,1-b][1,3]oxazin-6-yl acetate**

To a stirred solution of (R)-6-acetoxy-6,7-dihydro-5H-pyrazolo[5,1-b][1,3]oxazine-3-sulfonic acid in DCM (crude from step 7) in a 2-L 3-necked round-bottom flask under nitrogen was added pyridine (55.1 g, 696 mmol) dropwise at 0 °C. To this was added $PCl_5$ (144 g, 696 mmol) in portions at 0 °C. The resulting solution was stirred for 2 h at RT. The reaction was then quenched by the addition of 1 L of water/ice. The resulting solution was extracted with 3x300 mL of EtOAc. The resulting mixture was washed with 2 x500 ml of $NaHCO_3$ and 1 x500 mL of $H_2O$. The resulting mixture was washed with 1x500 mL of NaCl (aq.). The mixture was dried over anhydrous $Na_2SO_4$ and concentrated under vacuum. This resulted in 76 g (crude) of the title compound as a light yellow solid. MS-ESI: 281/283 (M+1).

**Step 9: (R)-6-hydroxy-6,7-dihydro-SH-pyrazolo[5,1-b][1,3]oxazine-3-sulfonamide**

To a stirred solution of $NH_3$ in THF (1M, 730 ml) in a 3-L round-bottom flask was added (R)-3-(chloro- sulfonyl)-6,7-dihydro-5H-pyrazolo[5,1-b][1,3] oxazin-6-yl acetate (73.0 g) in several batches. The flask was then filled with $NH_3$ (balloon). The resulting solution was stirred overnight at 40 °C in an oil bath. After reaction completed, the solids were filtered out. The filtrate was concentrated. The residue was diluted with $NH_3$ (7 M in MeOH, 730 mL). The resulting solution was stirred for 2h at RT. LC showed the reaction was complete. The MeOH solution was concentrated under reduced pressure to a final volume of about 100 mL. $Et_2O$ (360 ml) was charged to the resulting solution and kept stirring for 30 min. The mixture was filtered, and the cake washed with $Et_2O$ (100 mL). The white solid was dried under vacuum. This resulted in 37 g (53% for 3 steps) of the title compound as a white solid. MS-ESI: 220 (M+1). $^1H$ NMR (300 MHz, DMSO-$d_6$) $\delta$ 7.48 (s, 1H), 7.06 (s, 2H), 5.64 (d, $J$ = 2.3 Hz, 1H), 4.30 (s, 3H), 4.30 - 4.18 (m, 1H), 4.00 - 3.90 (m, 1H).

**Step 10: (*R*)-*N*-(*tert*-butyldiphenylsilyl)-6-((*tert*-butyldiphenylsilyl)oxy)-6,7-dihydro-5*H*-pyrazolo[5,1-*b*] -[1,3]oxazine-3-sulfonamide**

To a stirred solution of (R)-6-hydroxy-6,7-dihydro-5H-pyrazolo[5,1-b][1,3] oxazine-3-sulfonamide (500 mg, 2.28 mmol) in DMF (20 mL) in a 50-mL round-bottom flask was added DBU (2.08 g, 13.7 mmol) and TBDPSCl (5.02 g, 18.2 mmol) at 0 °C. The resulting solution was stirred for 2 h at RT and then diluted with 30 mL of $H_2O$. The resulting solution was extracted with 3x100 mL of EtOAc. The combined organic layers were dried over anhydrous $Na_2SO_4$ and concentrated under reduced pressure. The residue was eluted from silica gel with EtOAc/PE (1:1). This resulted in 530 mg (33.3%) of the title compound as a solid. MS-ESI: 696 (M+1).

**Step 11: (6*R*)-*N'*-(*tert*-butyldiphenylsilyl)-6-((*tert*-butyldiphenylsilyl)oxy)-6,7-dihydro-5*H*-pyrazolo [5,1-*b*][1,3]oxazine-3-sulfonimidamide**

To a stirred solution of $PPh_3Cl_2$ (759 mg, 2.28 mmol) in DCE (30 mL) in a 100-mL 3-necked round-bottom flask under nitrogen was added DIEA (492 mg, 3.81 mmol) at 0 °C. The resulting solution was stirred for 10 min at RT. Then (R)-N-(tert-butyldiphenylsilyl)-6-((tert-butyldiphenylsilyl)oxy)-6,7-dihydro-5H- pyrazolo[5,1-b][1,3] oxazine-3-sulfonamide (530 mg, 0.76 mmol) in $CHCl_3$ (10 mL) was added at 0 °C. The resulting solution was stirred for 30 min at 0 °C. Then $NH_3$ (g) was bubbled to the reaction mixture for 15 min at 0 °C. The resulting solution was stirred for 2 h at RT. The solids were filtered out. The resulting mixture was concentrated under vacuum. The residue was eluted from silica gel with EtOAc/PE (1:1). This resulted in 400 mg (75.6%) of the title compound as a white solid. MS-ESI: 695 (M+1).

**Scheme 110:**

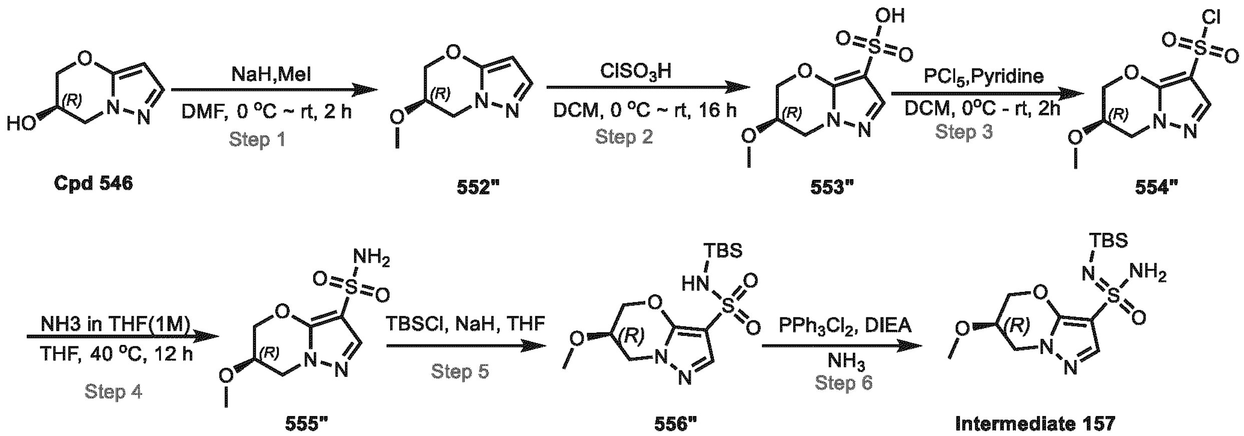


Cpd 546 552" 553" 554"

555" 556" Intermediate 157

## Intermediate 157

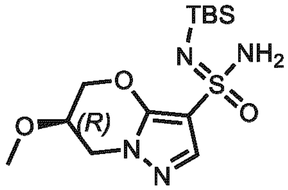

(6*R*)-N'-(tert-butyldimethylsilyl)-6-methoxy-6,7-dihydro-5H-pyrazolo[5,1-b][1,3]oxazine-3-sulfonimidamide

**Step 1: (R)-6-methoxy-6,7-dihydro-5H-pyrazolo[5,1-b][1,3]oxazine**

To a stirred solution of (R)-6,7-dihydro-5H-pyrazolo[5,1-b][1,3]oxazin-6-ol (40 g, 285 mmol) in DMF (400 mL) in a 1 L 4-neck round-bottom flask was added NaH (60% oil dispersion, 13.7 g, 342 mmol) in portions at 0 °C. The resulting mixture was stirred for 1 h at RT. To the above mixture was added MeI (48.7 g, 343 mmol) dropwise at RT. The resulting mixture was stirred for additional 2 h at RT. The reaction was quenched with AcOH (3.66 g, 57.1 mmol) at 0 °C and concentrated under reduced pressure. The residue was eluted from silica gel with PE/EtOAc (1:2) to afford the title compound (34.6 g, 79%) as a light yellow solid. MS-ESI: 155 (M+1).

Steps 2-4 used similar procedures for converting compound **547"** to compound **550"** shown in Scheme 109 to afford intermediate 159 from compound 562". MS-ESI: 232 (M-1).

Steps 5-6 used similar procedures for converting compound **362"** to intermediate **124** shown in Scheme 80 to afford intermediate **157** from compound **555"**. MS-ESI: 347 (M+1).

**Scheme 111:**

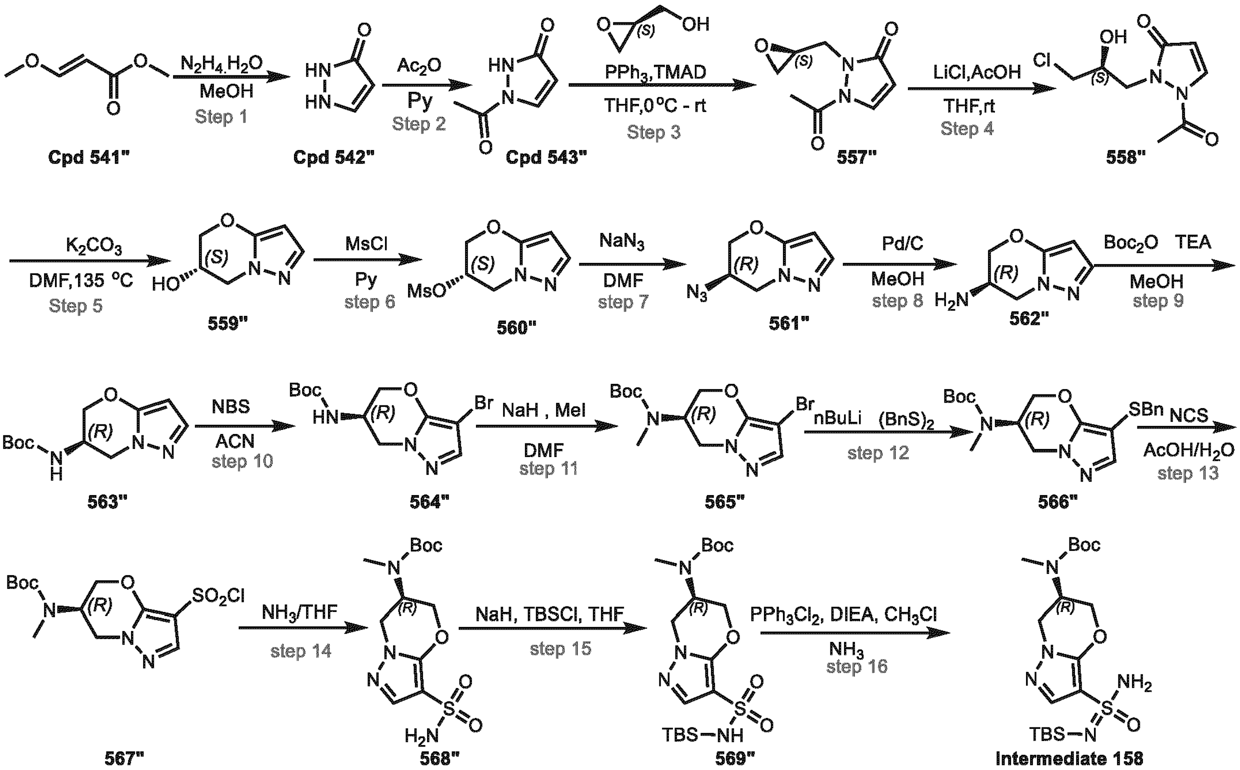


## Intermediate 158

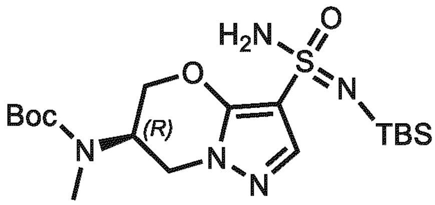

Tert-butyl ((6R)-3-(N'-(tert-butyldimethylsilyl)sulfamidimidoyl)-6,7-dihydro-5H-pyrazolo[5,1-b][1,3]oxazin-6-yl)(methyl)carbamate

Steps 1-5 used similar procedures for converting compound **541"** to compound **546"** shown in Scheme 109 to afford compound **559"** from compound **541"**. MS-ESI: 141 (M+1).

**Step 6: (*S*)-6,7-dihydro-5H-pyrazolo[5,1-b][1,3]oxazin-6-yl methanesulfonate**

To a stirred solution of (*S*)-6,7-dihydro-5H-pyrazolo[5,1-b][1,3]oxazin-6-ol (40 g, 285 mmol) in pyridine (280 mL) in a 500 mL 3-neck flask under nitrogen was added MsCl (39 g, 343 mmol) dropwise at RT. The resulting mixture was stirred for 30 min at RT. The resulting mixture was concentrated under reduced pressure. The crude was diluted with 500 mL of water. The resulting mixture was extracted with EtOAc (3 x 300 mL). The combined organic layers were washed with water (3x200 mL) and brine 1x100 mL, dried over anhydrous $Na_2SO_4$. After filtration, the filtrate was concentrated under reduced pressure. This resulted in the title compound (57 g, 92%) as an off-white solid. MS-ESI: 219 (M+1).

**Step 7: (*R*)-6-azido-6,7-dihydro-5H-pyrazolo[5,1-b][1,3]oxazine**

To a stirred solution of (*S*)-6,7-dihydro-5H-pyrazolo[5,1-b][1,3]oxazin-6-yl methanesulfonate (46 g, 211 mmol) in DMF (313 mL) in a 1 L 3-neck flask under nitrogen was added $NaN_3$ (21 g, 316 mmol) at RT. The resulting mixture was stirred for 6 h at 80 °C. LCMS showed reaction was complete. This was used directly in the next step. MS-ESI: 166 (M+1).

**Step 8: (*R*)-6,7-dihydro-SH-pyrazolo[5,1-b][1,3]oxazin-6-amine**

To a stirred solution of (*R*)-6-azido-6,7-dihydro-5H-pyrazolo[5,1-b][1,3]oxazine in DMF from the last step was added MeOH (313 mL) followed by Pd/C (10%wt., 10 g) under nitrogen. The mixture was hydrogenated at RT for 5 h under hydrogen atmosphere using a hydrogen balloon. LCMS showed reaction was complete. The mixture was filtered through a pad of Celite and concentrated under reduced pressure to remove low boiling solvent. This resulted in the title compound in DMF solution which was used directly in the next step. MS-ESI: 140 (M+1).

**Step 9: *Tert*-butyl (*R*)-(6,7-dihydro-SH-pyrazolo[5,1-b][1,3]oxazin-6-yl)carbamate**

To a stirred solution of (*R*)-6,7-dihydro-SH-pyrazolo[5,1-b][1,3]oxazin-6-amine in DMF from the last step was added MeOH (313 mL) followed by TEA (50 g, 496 mmol) and di-tert-butyl dicarbonate (79 g, 364 mmol) at RT under nitrogen. The resulting mixture was stirred for 16 h at RT. The resulting mixture was quenched with 500 mL of water. The resulting mixture was extracted with EtOAc (3 x 300 mL). The combined organic layers were washed with $H_2O$ (2x600 mL) and brine (1x600 mL), dried over anhydrous $Na_2SO_4$. After filtration, the filtrate was concentrated under reduced pressure. This resulted in the title compound (45.9 g, 91%, for 3 steps) as an off-white solid. MS-ESI: 240 (M+1).

**Step 10: *Tert*-butyl (R)-(3-bromo-6,7-dihydro-5H-pyrazolo[5,1-b][1,3]oxazin-6-yl)carbamate**

To a stirred solution of *tert*-butyl (R)-(6,7-dihydro-SH-pyrazolo[5,1-b][1,3] oxazin-6-yl)carbamate (140 g, 585 mmol) in MeCN (2.1 L) in a 3 L 3-neck flask under nitrogen was added NBS (115 g, 644 mmol) at 0 °C. The resulting mixture was stirred for 2 h at RT. The resulting mixture was diluted with 2000 mL of water. The resulting mixture was extracted with EtOAc (3 x 800 mL). The combined organic layers were washed with brine (1x800 mL), dried over anhydrous $Na_2SO_4$. After filtration, the filtrate was concentrated under reduced pressure. This resulted in the title compound (167 g, 90%) as an off-white solid. MS-ESI: 318/320 (M+1).

**Step 11: *Tert*-butyl (R)-(3-bromo-6,7-dihydro-5H-pyrazolo[5,1-b][1,3]oxazin-6-yl)(methyl)carbamate**

To a stirred solution of *tert*-butyl(*R*)-(3-bromo-6,7-dihydro-5H-pyrazolo-[5,1-b][1,3]oxazin-6-yl)carbamate (170 g, 534 mmol) in DMF (1.19 L) in a 3 L 3-neck flask under nitrogen was added sodium hydride (60% oil dispersion, 26 g, 650 mmol) in portions at 0 °C. The mixture was stirred for 1 h at 0 °C. Then MeI (379 g, 2.67 mol) was added and the mixture was allowed to warm to RT and stirred for 2 h. The reaction mixture was quenched by water and extracted with EtOAc (3*800 mL), the organic layers were washed with $H_2O$ (3x500 mL) and brine (1x800 mL), dried over anhydrous $Na_2SO_4$. After filtration, the filtrate was concentrated under reduced pressure. The residue was purified by silica gel column chromatography, eluted with PE/EtOAc (8:1) to afford the title compound (153 g, 86%) as an off-white solid. MS-ESI: 332/334 (M+1).

**Step 12: *Tert*-butyl(R)-(3-(benzylthio)-6,7-dihydro-5H-pyrazolo[5,1-b][1,3]oxazin-6-yl)(methyl) carbamate**

To a stirred solution of *tert*-butyl(*R*)-(3-bromo-6,7-dihydro-5H-pyrazolo-[5,1-b][1,3] oxazin-6-yl)(methyl)carbamate (130 g, 391 mmol) in THF (1.3 L) in a 3 L 3-neck flask under nitrogen was added n-BuLi (188 mL, 470 mmol, 2.5 mol/L) dropwise at -78 °C. The resulting mixture was stirred for 1 h at -78 °C. To the above mixture was added bis(phenylmethyl) disulfide (145 g, 587 mmol) in THF (300 mL) dropwise at -78 °C. The resulting mixture was stirred for additional 2 h at RT. The reaction was quenched by the addition of sat. $NH_4Cl$ (aq.) (500 mL) at RT. The resulting mixture was extracted with EtOAc (3 x 500 mL). The combined organic layers were washed with brine (1x800 mL), dried over anhydrous $Na_2SO_4$. After filtration, the filtrate was concentrated under reduced pressure. The residue was purified by silica gel column chromatography, eluted with PE/EtOAc (6:1) to afford the title compound (106 g, 72%) as an off-white solid. MS-ESI: 376 (M+1).

**Step 13: Tert-butyl (R)-(3-(chlorosulfonyl)-6,7-dihydro-5H-pyrazolo[5,1-b][1,3]oxazin-6-yl)(methyl) carbamate**

To a stirred solution of tert-butyl (R)-(3-(benzylthio)-6,7-dihydro-SH-pyrazolo[5,1-b][1,3]oxazin-6-yl)- (methyl) carbamate (110 g, 293 mmol) in AcOH (3.67 L)/$H_2O$ (1.83 L) in a 10 L 3-neck flask under nitrogen was added NCS (155 g, 1.17 mol) in portions at 0 °C. The resulting mixture was stirred for 1 h at RT. The reaction was quenched with water/ice at RT. The resulting mixture was extracted with MTBE (3 × 1 L). The combined organic layers were washed with water (2 x 1.0 L), $NaHCO_3$ (L) and brine (1×1 L), dried over anhydrous $Na_2SO_4$. After filtration, the filtrate was concentrated under reduced pressure. This resulted in the title compound (80 g, crude ) as a yellow solid. MS-ESI: 374 (M+Na).

**Step 14: Tert-butyl (R)-methyl(3-sulfamoyl-6,7-dihydro-5H-pyrazolo[5,1-b][1,3]oxazin-6-yl)carbamate**

To a stirred solution of tert-butyl (R)-(3-(chlorosulfonyl)-6,7-dihydro-5H-pyrazolo[5,1-b][1,3]oxazin- 6-yl)(methyl) carbamate (80 g, 227 mmol) in $NH_3$ in THF (1 M, 800 mL, 800 mmol) in a 2 L 3-neck flask under nitrogen. The flask was then filled with $NH_3$ (balloon). The resulting mixture was stirred for overnight at 60 °C. The reaction mixture was quenched by water and extracted with MTBE (3*800 mL), the organic layers were washed with $H_2O$ (3x500 mL) and brine (1x800 mL), dried over anhydrous $Na_2SO_4$. After filtration, the filtrate was concentrated under reduced pressure. The precipitated solid was slurry with MTBE (1x100 mL). This resulted in the title compound (36 g, for 2 steps) as an off-white solid. MS-ESI: 333 (M+1).

Steps 15-16 used similar procedures for converting compound **362"** to intermediate **124** shown in Scheme 80 to afford intermediate **158** from compound 568. MS-ESI: 446 (M+1).

## Scheme 112:

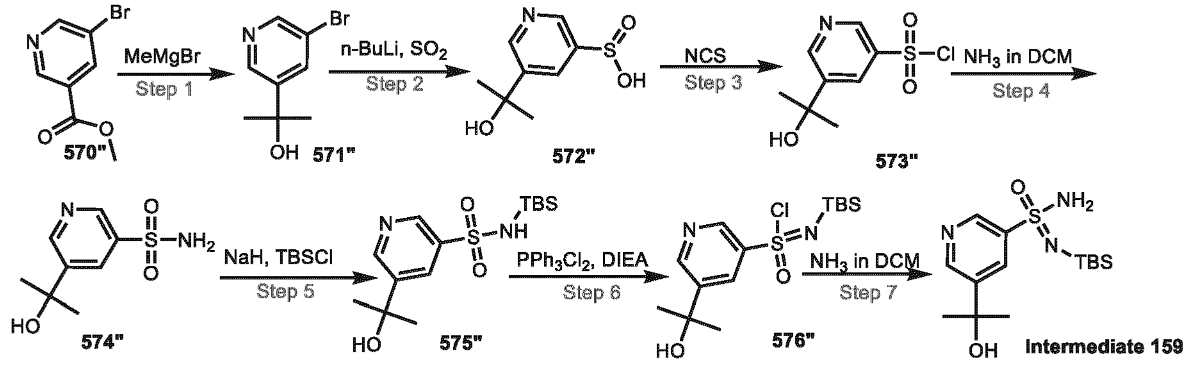


570" 571" 572" 573" 574" 575" 576" Intermediate 159

## Intermediate 159

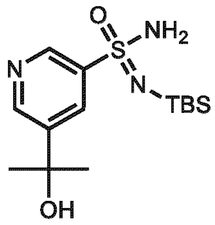

*N'*-(*tert*-butyldimethylsilyl)-5-(2-hydroxypropan-2-yl)pyridine-3-sulfonimidamide

Step 1 used similar procedures for converting compound **406"** to compound **407"** shown in Scheme 87 to afford compound **571"** from compound 570". MS-ESI: 215/217 (M+1).

Steps 2-7 used similar procedures for converting compound **369"** to intermediate **126** shown in Scheme 82 to afford intermediate **159** from compound 571". MS-ESI: 330 (M+1).

## Scheme 113:

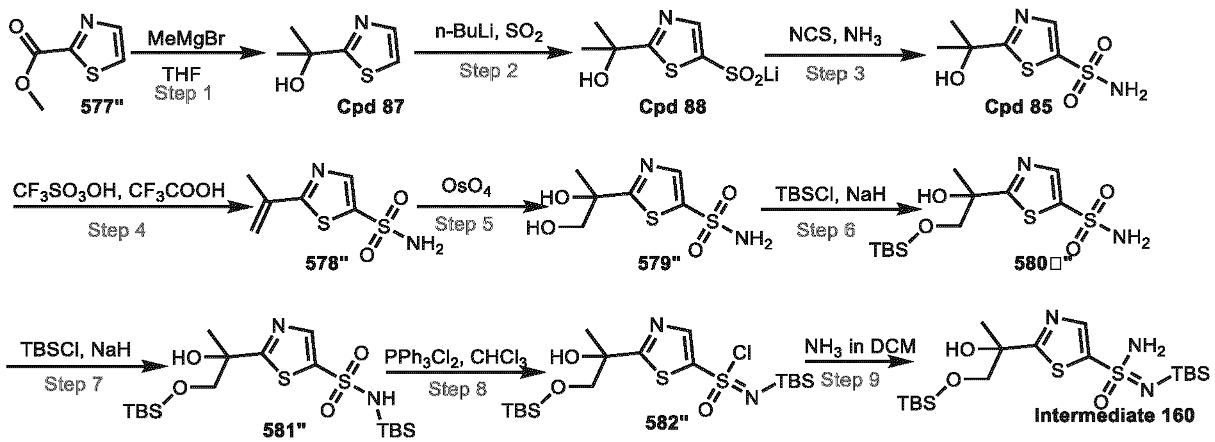


## Intermediate 160

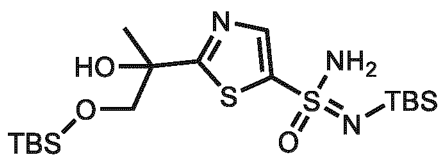

*N'*-(*tert*-butyldimethylsilyl)-2-(1-((tert-butyldimethylsilyl)oxy)-2-hydroxypropan-2-yl)thiazole-5-sulfonimidamide

Step 1 used similar procedures for converting compound **406"** to compound **407"** shown in Scheme 87 to afford compound **87** from compound 577". MS-ESI: 144 (M+1).

Steps 2-3 used similar procedures for converting compound **369"** to compound **371"** shown in Scheme 82 to afford compound **85** from compound **87**. MS-ESI: 223 (M+1).

**Step 4: 2-(Prop-1-en-2-yl)thiazole-5-sulfonamide**

To a stirred solution of 2-(2-hydroxypropan-2-yl)thiazole-5-sulfonamide (50 g, 225 mmol,) in trifluoromethanesulfonoperoxoic acid (60 mL) in a 1-L round-bottom flask under nitrogen was added TFA (60 mL). The resulting solution was stirred for 16 h at 50 °C in an oil bath. The pH value of the solution was adjusted to 8 with NaOH (3 %wt.) solution. The resulting solution was extracted with 3x500 mL of DCM and the organic layers were combined and concentrated under vacuum. The residue was eluted from silica gel with EtOAc/PE (1:2). This resulted in 10 g (21%) of the title compound as an off-white solid. MS-ESI: 205 (M+1).

**Step 5: 2-(1,2-Dihydroxypropan-2-yl)thiazole-5-sulfonamide**

Into a 500-mL round-bottom flask, was placed 2-(prop-1-en-2-yl)thiazole-5-sulfonamide (10 g, 49 mmol) in t-BuOH (40 mL) and acetone (40 mL), to the stirred solution was added NMO (11.5 g, 97.9 mmol) and the resulting solution was stirred for 15 min at RT. Then to this was added a solution of $OsO_4$ (1.24 g, 4.9 mmol) in $H_2O$ (60 mL) at RT. The resulting solution was stirred overnight at RT. The reaction was then quenched by the addition of the saturation solution of $Na_2S_2O_3$ (50 mL). The resulting solution was extracted with EtOAc (3x100 mL). The organic layers were combined and dried over anhydrous $Na_2SO_4$ and concentrated under vacuum. The crude product was eluted from silica gel with MeOH/DCM (7:100). This resulted in 5 g (43%) of the title compound as yellow oil. MS-ESI: 239 (M+1).

Steps 6-9 used similar procedures for converting compound **362"** to intermediate **124** shown in Scheme 80 to afford intermediate **160** from compound 579". MS-ESI: 466 (M+1).

**Table 33**. The Intermediate 161 was prepared using similar procedures for converting compound **577"** to Intermediate **160** shown in Scheme 113 from appropriate starting materials.

| Intermediate # | Structure | IUPAC Name | Exact Mass [M+H]+ |
|---|---|---|---|
| **Intermediate 161** | 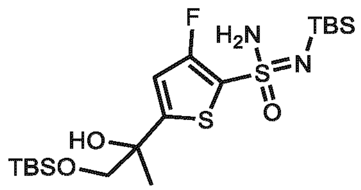 | *N'*-(*tert*-butyldimethylsilyl)-5-(1-((tert-butyldimethylsilyl)oxy)-2-hydroxypropan-2-yl)-3-fluorothiophene-2-sulfonimidamide | 483 |

# Scheme 114:

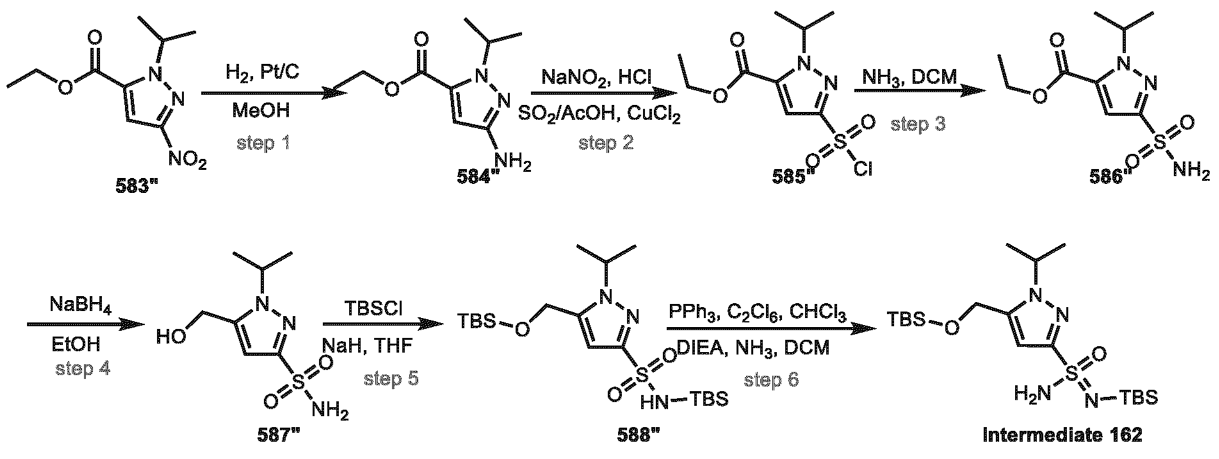


## Intermediate 162

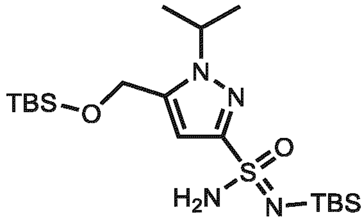

N'-(*tert*-butyldimethylsilyl)-5-(((tert-butyldimethylsilyl)oxy)methyl)-1-isopropyl-1H-pyrazole-3-sulfonimidamide

Steps 1-3 used similar procedures for converting compound **410"** to compound **413"** shown in Scheme 88 to afford compound **586"** from compound 583". MS-ESI: 262 (M+1).

Step 4 used similar procedures for converting compound **416"** to compound **417"** shown in Scheme 89 to afford compound **587"** from compound 586". MS-ESI: 220 (M+1).

Steps 5-6 used similar procedures for converting compound **362"** to intermediate **124** shown in Scheme 80 to afford intermediate **167** from compound 594". MS-ESI: 447 (M+1).

## Scheme 115:

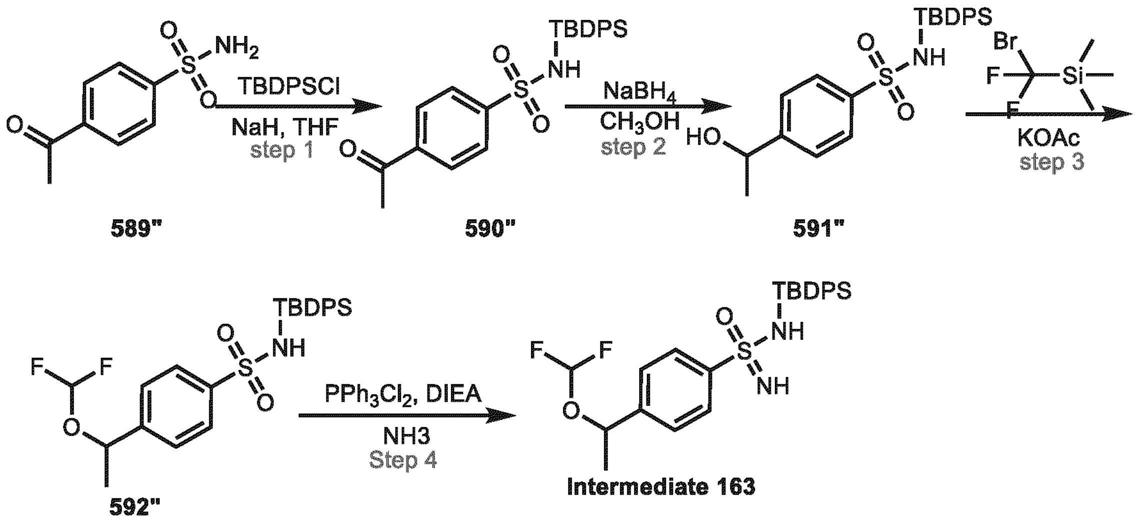


589" 590" 591"

592" Intermediate 163

## Intermediate 163

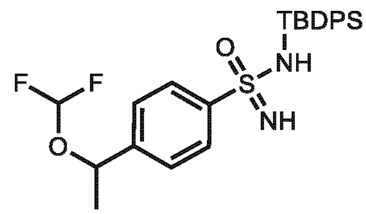

N-(*tert*-butyldiphenylsilyl)-4-(1-(difluoromethoxy)ethyl)benzenesulfonimidamide

Step 1 used similar procedures for converting intermediate **550"** to compound **551"** shown in Scheme 109 to afford compound **590"** from compound 589". MS-ESI: 438 (M+1).

### Step 2: *N*-(*tert*-butyldiphenylsilyl)-4-(1-hydroxyethyl)benzenesulfonamide

To a stirred solution of 4-acetyl-N-(tert-butyldiphenylsilyl)benzenesulfonamide (12 g, 27.5 mmol) in MeOH (100 mL) in a 500-mL round-bottom flask under nitrogen was added $NaBH_4$ (2.09 g, 54.9 mmol). The resulting solution was stirred for 2 h at RT. The reaction was then quenched by the addition of 200 mL of water. The resulting solution was extracted with 3x200 mL of EtOAc and the organic layers were combined and concentrated under vacuum. This resulted in 8.0 g of the title compound as a yellow solid. MS-ESI: 440 (M+1).

### Step 3: *N*-(*tert*-butyldiphenylsilyl)-4-(1-(difluoromethoxy)ethyl)benzenesulfonamide

To a stirred solution of *N*-(*tert*-butyldiphenylsilyl)-4-(1-hydroxyethyl)benzenesulfonamide (3.5 g, 7.97 mmol) in DCM (50 mL) and $H_2O$ (50 mL) in a 250-mL round-bottom flask under nitrogen was added KOAc (3.12 g, 31.9 mmol) and (bromodifluoromethyl)trimethylsilane (6.47 g, 3.24 mmol). The resulting solution was stirred for 24 h at RT. The resulting solution was extracted with 3x100 mL of DCM and the organic layers were combined and concentrated under

vacuum. The residue was eluted from silica gel column with PE/EtOAc (3:1) to afford the title compound (2.81 g, yield 72%) as an off-white solid MS-ESI: 490 (M+1).

Step 4 used similar procedures for converting compound **362"** to Intermediate **124** shown in Scheme 80 to afford Intermediate **163** from compound 592". MS-ESI: 489 (M+1).

## Scheme 116:

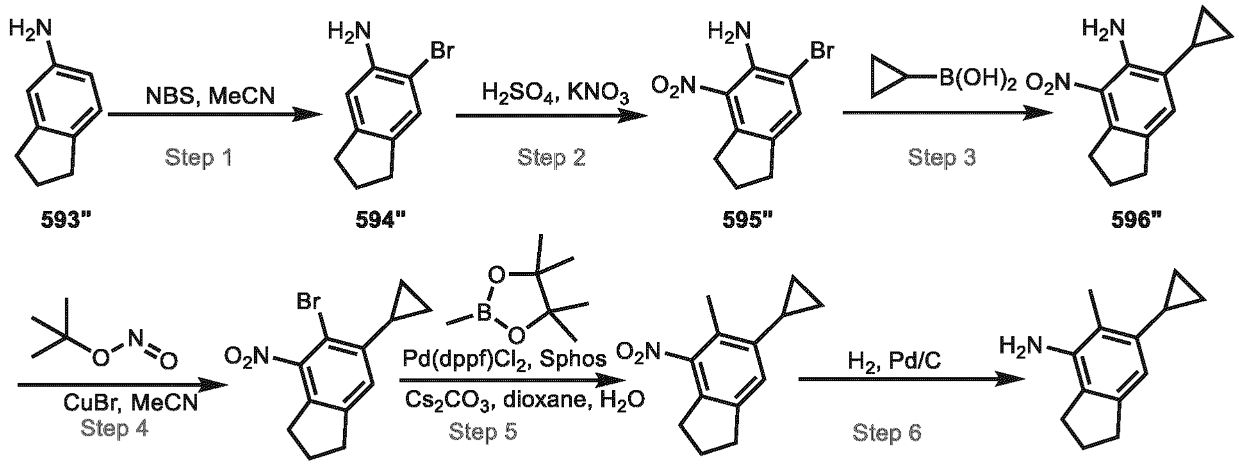


593" 594" 595" 596"

597" 598" Intermediate 164

## Intermediate 164

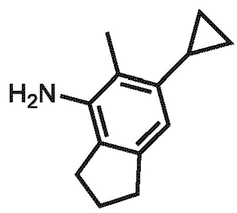

6-Cyclopropyl-5-methyl-2,3-dihydro-1H-inden-4-amine

### Step 1: 6-Bromo-2,3-dihydro-1H-inden-5-amine

To a stirred solution of 2,3-dihydro-1H-inden-5-amine (5.0 g, 37.5 mmol) in MeCN (300 mL) in a 500-mL round-bottom flask under nitrogen was added a solution of NBS (6.68 g, 37.5 mmol) in MeCN/THF (1:1) (50 mL) dropwise at 0 °C. The resulting solution was stirred for 1 h at 0 °C in a water/ice bath. The reaction was then quenched by the addition of 100 mL of sat. $NaHCO_3$ (aq). The resulting solution was extracted with 3x100 mL of EtOAc and the organic layers were combined and concentrated under vacuum. The residue was eluted from silica gel with EtOAc/PE (1:20). This resulted in 7.0 g (87.9%) of the title compound as purple oil. MS-ESI: 212/214 (M+1).

### Step 2: 6-Bromo-4-nitro-2,3-dihydro-1H-inden-5-amine

To a stirred solution of 6-bromo-2,3-dihydro-1H-inden-5-amine (1.92 g, 9.05 mmol) in cc. $H_2SO_4$ (10 mL) in a 50-mL round-bottom flask was added $KNO_3$ (1.1 g, 10.9 mmol) at 0 °C. The resulting solution was stirred for 16 h at RT. The resulting solution was poured into 100 mL of water/ice slowly. The pH value of the solution was adjusted to 8 with sat. $NaHCO_3$ (aq.). The resulting solution was extracted with 3x100 mL of EtOAc. The combined organic phase was dried over anhydrous $Na_2SO_4$ and concentrated under reduced pressure. The residue was eluted from silica gel with EtOAc/PE (1:5). This resulted in 980 mg (42.1%) of the title compound as a red solid. MS-ESI: 256/258 (M+1).

**Step 3: 6-Cyclopropyl-4-nitro-2,3-dihydro-1H-inden-5-amine**

To a stirred solution of 6-bromo-4-nitro-2,3-dihydro-1H-inden-5-amine (0.98 g, 3.81 mmol) in toluene (50 mL) and $H_2O$ (7 mL) in a 100-mL round-bottom flask under nitrogen was added cyclopropylboronic acid (0.49 g, 5.70 mmol). Then Pd(dppf)$Cl_2$ (278 mg, 0.38 mmol), SPhos (156 mg, 0.381 mmo) and $Cs_2CO_3$ (2.48 g, 7.62 mmol) were added. The resulting solution was stirred for 16 h at 80 °C under nitrogen. The insoluble was filtered out, the filter cake was washed with 200 mL of EtOAc. The filtrate was concentrated under reduced pressure. The residue was eluted from silica gel with EtOAc/PE (1:3). This resulted in 946 mg (95.2%) of the title compound as a red solid. MS-ESI: 219 (M+1).

**Step 4: 5-Bromo-6-cyclopropyl-4-nitro-2,3-dihydro-1H-indene**

To a stirred solution of 6-cyclopropyl-4-nitro-2,3-dihydro-1H-inden-5-amine (946 mg, 4.33 mmol) in MeCN (50 mL) in a 100-mL round-bottom flask under nitrogen was added tert-butyl nitrite (670 mg, 6.50 mmol) dropwise at 0 °C, CuBr (746 mg, 5.20 mmol) was added in portions at 0 °C. The resulting solution was stirred for 3 h at 60 °C. The resulting mixture was concentrated under vacuum. The residue was eluted from a silica gel with EtOAc/PE (1:10). This resulted in 750 mg (61.3%) of the title compound as red oil. $^1H$ NMR (400 MHz, DMSO-$d_6$) $\delta$ 7.18 (s, 1H), 3.00-2.80 (m, 4H), 2.20-2.00 (m, 3H), 1.10-0.95 (m, 2H), 0.80-0.65 (m, 2H).

**Step 5: 6-Cyclopropyl-5-methyl-4-nitro-2,3-dihydro-1H-indene**

To a stirred solution of 5-bromo-6-cyclopropyl-4-nitro-2,3-dihydro-1H-indene (700 mg, 2.48 mmol) in toluene (42 mL) and $H_2O$ (7 mL) in a 100-mL round-bottom flask under nitrogen was added 2,4,4,5,5-penta- methyl-1,3,2-dioxaborolane (528 mg, 3.72 mmol). Then $Cs_2CO_3$ (1.62 g, 4.96 mmol), Pd(dppf)$Cl_2$ (181 mg, 0.25 mmol) and SPhos (101 mg, 0.25 mmol) were added. The resulting solution was stirred for 16 h at 80 °C under nitrogen. The resulting mixture was concentrated under vacuum. The residue was eluted from silica gel with EtOAc/PE (1:3). This resulted in 265 mg (49.2%) of the title compound as dark yellow oil. $^1H$ NMR (400 MHz, DMSO-$d_6$) $\delta$ 7.16 (s, 1H), 2.96-2.80 (m, 4H), 2.32 (s, 3H), 2.10-1.98 (m, 2H), 1.98-1.85 (m, 1H), 1.01-0.89 (m, 2H), 0.68-0.59 (m, 2H).

**Step 6: 6-Cyclopropyl-5-methyl-2,3-dihydro-1H-inden-4-amine**

To a stirred solution of 6-cyclopropyl-5-methyl-4-nitro-2,3-dihydro-1H-indene (265 mg, 1.22 mmol) in MeOH (25 mL) in a 100-mL round-bottom flask under nitrogen was added Pd/C (10% wt., 50 mg), the flask was evacuated and flushed with hydrogen 3 times. The resulting solution was stirred for 16 h at RT under hydrogen with a balloon. The solids were filtered out. The resulting mixture was concentrated under vacuum. This resulted in 187 mg (81.9%) of the title compound as dark yellow oil. MS-ESI: 188 (M+1).

**Scheme 117:**

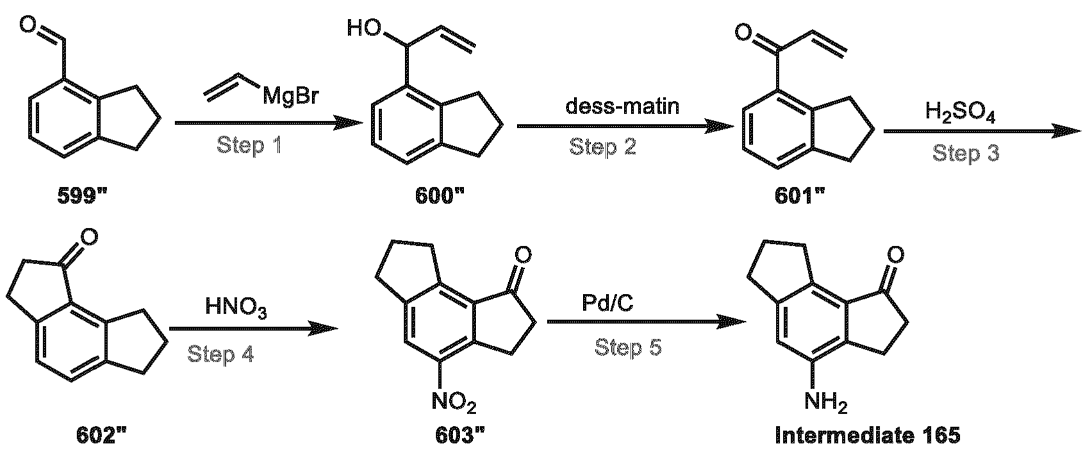

599"
600"
601"
602"
603"
Intermediate 165

## Intermediate 165

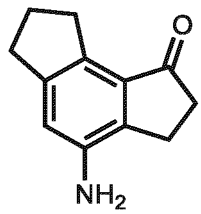

4-Amino-3,6,7,8-tetrahydro-as-indacen-1(2H)-one

**Step 1: 1-(2,3-Dihydro-1H-inden-4-yl)prop-2-en-1-ol**

To a stirred solution of 2,3-dihydro-1H-indene-4-carbaldehyde (4.0 g, 27.4 mmol) in THF (50 mL) in a 100-mL round-bottom flask under nitrogen was added bromo(ethenyl)magnesium (27.4 mL, 27.4 mmol, 1.0 M in THF) dropwise at 0 °C. The resulting solution was stirred for 2 h at RT.

The reaction was then quenched by the addition of 100 mL of water/ice. The resulting solution was extracted with 3x50 mL of DCM and the organic layers were combined then concentrated under vacuum. This resulted in 3.5 g (73.4%) of the title compound as brown oil. MS-ESI: 175 (M+1).

**Step 2: 1-(2,3-Dihydro-1H-inden-4-yl)prop-2-en-1-one**

To a stirred solution of 1-(2,3-dihydro-1H-inden-4-yl)prop-2-en-1-ol (3.50 g, 20.1 mmol) in DCM (50 mL) in a 100-mL round-bottom flask under nitrogen was added Dess-Martin (8.52 g, 20.1 mmol) in portions at 0 °C. The resulting solution was stirred for 1 h at RT. The resulting mixture was quenched by addition of sat. $Na_2S_2O_3$. The mixture was extracted with 3x200 mL EtOAc and the organic layers were combined and dried over anhydrous $Na_2SO_4$ and concentrated under vacuum. The residue was eluted from a silica gel with EtOAc/PE (1:50 to 1 :30). This resulted in 3 g (86.7%) of the title compound as a yellow solid. MS-ESI: 173 (M+1).

**Step 3: 3,6,7,8-Tetrahydro-as-indacen-1(2H)-one**

To a stirred solution of sulfuric acid (30 mL) in a 100-mL round-bottom flask under nitrogen was added a solution of 1-(2,3-dihydro-1H-inden-4-yl)prop-2-en-1-ol (3.0 g, 17.4 mmol) in DCM (5 mL) dropwise at 0 °C. The resulting solution was stirred for 3 h at 50 °C in an oil bath. The resulting solution was poured into 200 mL of water/ice slowly. The resulting solution was extracted with 3x200 mL of DCM and concentrated under vacuum. The residue was eluted from silica gel with EtOAc/PE (1:30 to 1:10). This resulted in 1.0 g (33.3%) of the title compound as a yellow solid. MS-ESI: 173 (M+1).

**Step 4: 4-Nitro-3,6,7,8-tetrahydro-*as*-indacen-1(2H)-one**

To a stirred solution of sulfuric acid (5.00 mL) in a 100-mL round-bottom flask under nitrogen was added 3,6,7,8-tetrahydro-*as*-indacen-1(2H)-one (500 mg, 2.9 mmol) in portions at 0 °C. To this was added nitric acid (365 mg, 5.8 mmol) dropwise with stirring at 0 °C. The resulting solution was stirred for 1 h at 0 °C in a water/ice bath. The resulting solution was poured into 20 mL of water/ice slowly. The resulting solution was extracted with 3x20 mL of DCM concentrated under vacuum. This resulted in 400 mg (63%) of the title compound as a brown solid. MS-ESI: 218 (M+1).

**Step 5: 4-Amino-3,6,7,8-tetrahydro-*as*-indacen-1(2H)-one**

To a stirred solution of 4-nitro-3,6,7,8-tetrahydro-*as*-indacen-1(2H)-one (400 mg, 1.84 mmol) in MeOH (20 mL) in a 100-mL round-bottom flask under nitrogen was added Pd/C (10% wt., 100 mg), the flask was evacuated and flushed with hydrogen 3 times. The resulting solution was stirred for 16 h at RT under hydrogen with a balloon. The solids were collected by filtration. The resulting mixture was concentrated under vacuum. This resulted in 300 mg (87%) of the title compound as a brown solid. MS-ESI: 188 (M+1).

## Scheme 118:

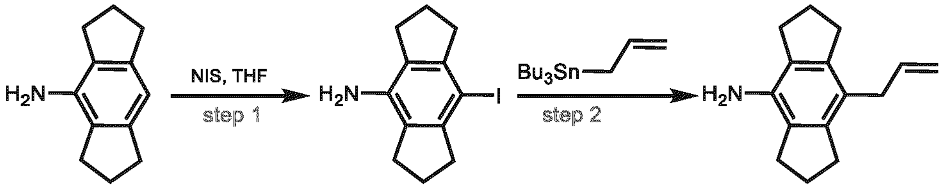


604"    605"    Intermediate 166

## Intermediate 166

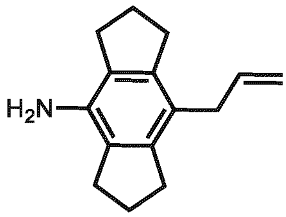

8-Allyl-1,2,3,5,6,7-hexahydro-s-indacen-4-amine

**Step 1: 8-Iodo-1,2,3,5,6,7-hexahydro-*s*-indacen-4-amine**

To a stirred solution of 1,2,3,5,6,7-hexahydro-*s*-indacen-4-amine (2.01 g, 12 mmol) in N,N-dimethylformamide (10 mL) in a 50-mL round-bottom flask was added NIS (2.97 g, 13.2 mmol). The resulting solution was stirred for 3 h at RT. The resulting solution was diluted with 100 mL of EtOAc. The resulting mixture was washed with 100 mL of brine. The resulting mixture was concentrated under vacuum. The residue was eluted from silica gel with EtOAc/PE (1:20). This resulted in 2.84 g (79%) of the title compound as an orange solid. MS-ESI: 300 (M+1).

**Step 2: 8-Allyl-1,2,3,5,6,7-hexahydro-s-indacen-4-amine**

To a stirred solution of 8-iodo-1,2,3,5,6,7-hexahydro-s-indacen-4-amine (460 mg, 1.54 mmol) and $PPh_3$ (202 mg, 0.77 mmol) in DMF(15 mL) in 50 mL round-bottom flask under nitrogen was added $Pd(pph_3)_2Cl_2$ (216 mg, 0.31 mmol) and tributyl(prop-2-en-1-yl)stannane (432 mg, 1.3 mmol) at RT. The resulting mixture was stirred for 16 h at 120 °C under nitrogen. The resulting mixture was diluted with water (40 mL) and extracted with EtOAc (3 x 80 mL). The combined organic layers were washed with brine (40 mL), dried over anhydrous $Na_2SO_4$. After filtration, the filtrate was concentrated under reduced pressure. The residue was eluted from silica gel with EtOAc/PE (1:2) to afford the title compound (200 mg, 61%) as a brown solid. MS-ESI: 214 (M+ 1).

## Scheme 119:

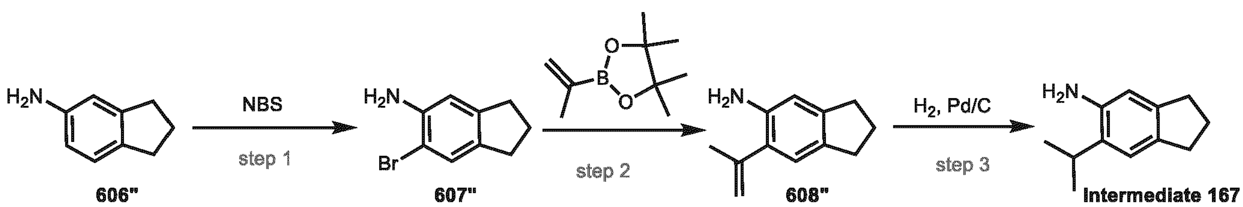


606"    607"    608"    Intermediate 167

## Intermediate 167

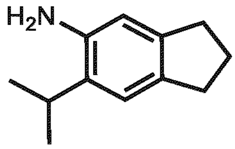

6-Isopropyl-2,3-dihydro-1H-inden-5-amine

**Step 1: 6-Bromo-2,3-dihydro-1H-inden-5-amine**

To a stirred solution of 2,3-dihydro-1H-inden-5-amine (5 g, 37.5 mmol) in MeCN (300 mL) in a 500-mL round-bottom flask was added a solution of NBS (6.68 g, 37.5 mmol) in MeCN/THF (1:1, 40 mL) dropwise at 0 °C. The resulting solution was stirred for 2 h at 0 °C in a water/ice bath. The reaction was then quenched by the addition of 100 mL of sat. $NaHCO_3$ (aq). The resulting solution was extracted with 3x100 mL of EtOAc and the organic phase was combined and dried over anhydrous $Na_2SO_4$, then concentrated under vacuum. The residue was eluted from silica gel with EtOAc/PE (1 :20). This resulted in 7.2 g (90%) of the title compound as purple oil. MS-ESI: 212 (M+1).

**Step 2: 6-(Prop-1-en-2-yl)-2,3-dihydro-1H-inden-5-amine**

To a stirred solution of 6-bromo-2,3-dihydro-1H-inden-5-amine (7.5 g, 35.4 mmol) in 1,4-dioxane (100 mL) and $H_2O$ (10 mL) in a 250-mL round-bottom flask under nitrogen was added $Cs_2CO_3$ (23.1 g, 70.8 mmol). Then 4,4,5,5-tetramethyl-2-(prop-1-en-2-yl)-1,3,2 -dioxaborolane (8.9 g, 53.1 mmol) and Pd(dppf)$Cl_2$ (2.6 g, 3.54 mmol) were added. The resulting solution was stirred for 16 h at 80 °C. The reaction was then quenched by the addition of 200 mL of water. The solids were filtered out. The resulting solution was extracted with 3x300 mL of EtOAc and the organic layers were combined and dried over anhydrous $Na_2SO_4$ and concentrated under vacuum. The residue was eluted from silica gel with EtOAc/PE (1:3). This resulted in 5.8 g (94.8%) of the title compound as yellow oil. MS-ESI: 174 (M+1).

**Step 3: 6-Isopropyl-2,3-dihydro-1H-inden-5-amine**

To a stirred solution of 6-(prop-1-en-2-yl)-2,3-dihydro-1H-inden-5-amine (5.8 g, 33.5 mmol) in MeOH (100 mL) in a 250-mL round-bottom flask under nitrogen was added Pd/C (10% wt., 0.58 g). The flask was evacuated and flushed with hydrogen 3 times. The resulting solution was stirred for 16 h at RT under hydrogen with a balloon. The solids were filtered out. The resulting mixture was concentrated under vacuum. This resulted in 5.77 g (98.3%) of the title compound as yellow oil. MS-ESI: 176 (M+1).

## Scheme 120:

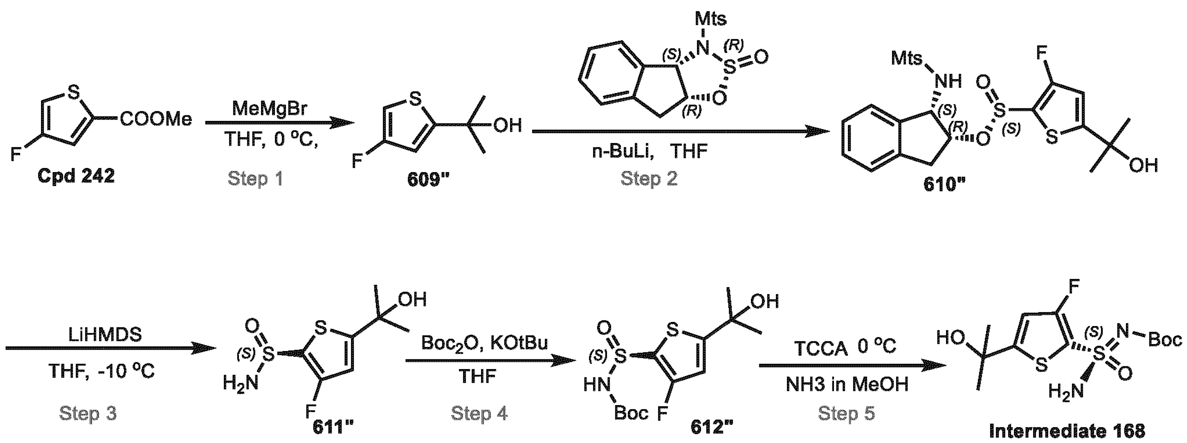

## Intermediate 168

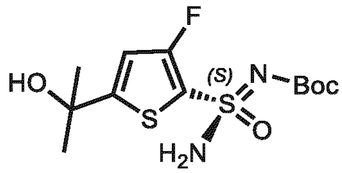

Tert-butyl (S)-(amino(3-fluoro-5-(2-hydroxypropan-2-yl)thiophen-2-yl)(oxo)-$\lambda^6$-sulfaneylidene)carbamate

**Step 1: 2-(4-Fluorothiophen-2-yl)propan-2-ol**

To a 250 mL 3-neck flask placed a solution of methyl 4-fluorothiophene-2-carboxylate (5.5 g, 34.3 mmol) in THF (55 mL) was added MeMgBr (1M in THF) (86 mL, 86 mmol) dropwise at 0 °C under nitrogen. The resulting solution was stirred for 3 h at 0 °C under nitrogen. LC showed reaction is completed. The resulting mixture was quenched with cold sat. aq. $NH_4Cl$ (100 mL) and extracted with EtOAc (3 x 50 mL). The combined organic layers were washed with brine (2 x 50 mL). The organic layer was dried over by anhydrous $Na_2SO_4$. After filtration, the filtrate was concentrated under reduced pressure. The crude product was re-crystallized from PE/EtOAc (100:1) to afford (4.0 g, 72%) of the title compound as a light yellow solid. GCMS: 160 [M]. $^1$H NMR (400 MHz, DMSO-$d_6$) $\delta$ 6.91 (d, $J$ = 1.7 Hz, 1H), 6.84 (d, $J$ = 1.7 Hz, 1H), 5.52 (s, 1H), 1.47 (s, 6H).

**Step 2: (1S,2R)-1-((2,4,6-trimethylphenyl)sulfonamido)-2,3-dihydro-1H-inden-2-yl (S)-3-fluoro-5-(2-hydroxypropan-2-yl)thiophene-2-sulfinate**

To a 100 mL 3-neck flask placed a solution of 2-(4-fluorothiophen-2-yl)propan-2-ol (2.0 g, 12.5mmol) in THF (30 mL) was added LDA (2 M in hexane, 12.5 mL, 25 mmol) dropwise at -78 °C over 1 min under nitrogen. The resulting solution was stirred for 30 min at -78 °C, this solution was assigned as A. The solution of (2R,3aS,8aR)-3-(mesitylsulfonyl)-3,3a,8,8a-tetrahydroindeno[1,2-d][1,2,3] oxathiazole 2-oxide (4.7 g, 12.5 mmol) in THF (10 mL) was transferred into the solution A, while the internal temperature of the resulting solution was kept below -65 °C under nitrogen. The resulting solution was stirred for 30 min at -70 °C under nitrogen. LC showed the reaction to be complete. The resulting mixture was quenched with cool sat. aq. $NH_4Cl$ (50 mL) and extracted with EtOAc (3 x 50 mL). The combined organic layers were washed with brine (2 x 50 mL). The organic layer was dried over by anhydrous $Na_2SO_4$. After filtration, the filtrate was concentrated under reduced pressure. The crude product was re-crystallized from PE/EtOAc (10:1) to afford (2.6 g, 38%, de=100%) of the title compound as a white solid. MS-ESI: 560 $[M+Na]^+$. $^1$H NMR (400 MHz, DMSO-$d_6$) $\delta$ 8.19 (d, $J$ = 9.7 Hz, 1H), 7.27 - 7.17 (m, 2H), 7.18 - 7.12 (m, 1H), 7.04 (s, 2H), 7.01 (s, 1H), 6.92 (d, $J$ = 7.4 Hz, 1H), 5.86 (s, 1H), 4.98 - 4.89 (m, 1H), 4.80 (dd, $J$ = 9.6, 5.1 Hz, 1H), 3.22 (dd, $J$ = 16.7, 4.8 Hz, 1H), 3.08 (dd, $J$ = 16.7, 2.2 Hz, 1H), 2.61 (s, 6H), 2.28 (s, 3H), 1.49 (s, 3H), 1.48 (s, 3H).

**Step 3: (S)-3-fluoro-5-(2-hydroxypropan-2-yl)thiophene-2-sulfinamide**

The solution of (1S,2R)-1-((2,4,6-trimethylphenyl)sulfonamido)-2,3-dihydro-1H-inden-2-yl-(S)-3-fluoro-5 -(2-hydroxypropan-2-yl)thiophene-2-sulfinate (1.5 g, 2.79 mmol) in THF (9 mL) was added in one portion to a stirred 50 mL of 3-neck flask placed a solution of NaHMDS (2M in THF) (5.6 ml, 11.2 mmol) in THF (15 mL) at -10 °C under nitrogen. The resulting solution was stirred for 1 h at -10 °C under nitrogen. LC showed the reaction to be complete. The resulting mixture was quenched with AcOH (704 mg, 11.7 mmol) and MeOH (1.5 mL) below 0 °C. The resulting mixture was diluted with water (50 mL) and extracted with EtOAc (3 x 50 mL). The combined organic layers were washed with brine (2 x 50 mL). The organic layer was dried over anhydrous $Na_2SO_4$. After filtration, the filtrate was concentrated under reduced pressure. The crude product was re-crystallized from PE/EtOAc (3:1) to afford (452 mg, 72%, ee=85.4%) of the title compound as a white solid. MS-ESI: 224 (M+1). $^1$H NMR (400 MHz, DMSO-$d_6$) $\delta$ 6.92 (s, 1H), 6.61 (s, 2H), 5.70 (s, 1H), 1.46 (s, 3H), 1.45 (s, 3H).

**Step 4: Tert-butyl (S)-((3-fluoro-5-(2-hydroxypropan-2-yl)thiophen-2-yl)sulfinyl)carbamate**

To a 25 mL 3-neck flask was added (S)-3-fluoro-5-(2-hydroxypropan-2-yl)thiophene-2-sulfinamide (200 mg, 0.62 mmol, ee=85.4%), this was followed by the addition of 3.0 mL of dry THF under nitrogen. The reaction mixture was cooled to 0 °C and KOtBu (120 mg, 0.74 mmol) was added (internal temperature was controlled below 0 °C). The solution was stirred for 30 min then the $Boc_2O$ (195 mg, 0.62 mmol) in 1.0 mL of THF was added. The ice bath was removed

and the reaction mixture was stirred at RT for 1 h. LC showed the reaction to be complete. The resulting mixture was quenched with aq. $NH_4Cl$ (25 mL) and extracted with EtOAc (3 x 5 mL). The combined organic layers were washed with brine (2 x 5 mL). The organic layer was dried over anhydrous $Na_2SO_4$. After filtration, the filtrate was concentrated under reduced pressure. The crude product was re-crystallized from PE/EtOAc (5:1) to afford (49 mg, 17%, ee=97.4%) of the title compound as a white solid. MS-ESI: 386 $[M+Na+MeCN]^+$, $^1H$ NMR (300 MHz, DMSO-$d_6$) $\delta$ 10.96 (s, 1H), 7.03 (s, 1H), 5.85 (s, 1H), 1.50 (s, 3H), 1.48 (s, 3H), 1.46 (s, 9H).

**Step 5: *Tert*-butyl (*S*)-(amino(3-fluoro-5-(2-hydroxypropan-2-yl)thiophen-2-yl)(oxo)-$\lambda^6$-sulfaneylidene) carbamate**

To a 10 mL 3-neck flask was added *tert*-butyl (S)-((3-fluoro-5-(2-hydroxypropan-2-yl)thiophen-2-yl) sulfinyl)carbamate (15 mg, 0.046 mmol, ee=97.4%), this was followed by the addition of dry THF (0.3 mL) under nitrogen. The reaction mixture was cooled to 0 °C and TCCA (3.8 mg, 0.016 mmol) was added portion-wise to avoid any exotherm and extensive over-chlorination. The reaction was stirred at 0 °C for 1 h. This solution was assigned as A. The solution A was cooled to -50 °C and the solution of $NH_3$ (7 M in MeOH) was added dropwise via syringe. The reaction was stirred at -50 °C for 1h. LC showed the reaction to be complete. After filtration, the filtrate was purified by Prep-HPLC to afford (3.0 mg, 19% , ee=97.9%) of the title compound as a white solid. MS-ESI: 339 (M+1). $^1H$ NMR (400 MHz, MeOH-$d_4$) $\delta$ 6.81 (s, 1H), 1.58 (s, 6H), 1.39 (s, 9H).

**Scheme 121:**

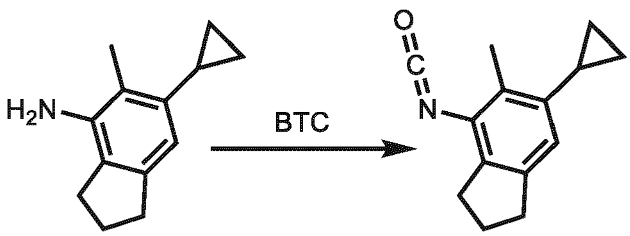


intermediate 164 intermediate 169

**Intermediate 169**

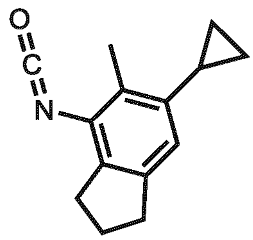

6-Cyclopropyl-4-isocyanato-5-methyl-2,3-dihydro-1H-indene

To a stirred solution of 6-cyclopropyl-5-methyl-2,3-dihydro-1H-inden-4-amine (187 mg, 1.0 mmol) in THF (30 mL) in a 100-mL round-bottom flask under nitrogen was added TEA (101 mg, 1.0 mmol) and triphosgene (148 mg, 0.5.0 mmol). The resulting solution was stirred for 2 h at 70 °C in an oil bath. The resulting mixture was concentrated under vacuum. This resulted in 213 mg (crude) of the title compound as yellow oil. This was used in the next step without further purification.

**Table 34**. The Intermediates in the following Table were prepared using the similar procedures for converting Intermediate **164** to Intermediate **169** shown in Scheme 121.

| Intermediate # | Structure | IUPAC Name |
|---|---|---|
| **Intermediate 170** | 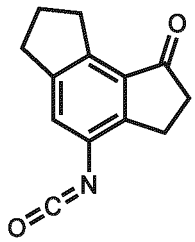 | 4-Isocyanato-3,6,7,8-tetrahydro-*as*-indacen-1(2H)-one |
| **Intermediate 171** | 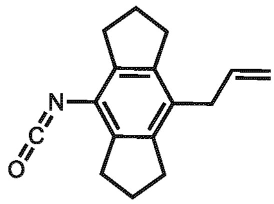 | 4-Allyl-8-isocyanato-1,2,3,5,6,7-hexahydro-*s*-indacene |
| **Intermediate 172** | 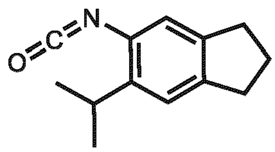 | 5-Isocyanato-6-isopropyl-2,3-dihydro-1H-indene |

**Scheme 122:**

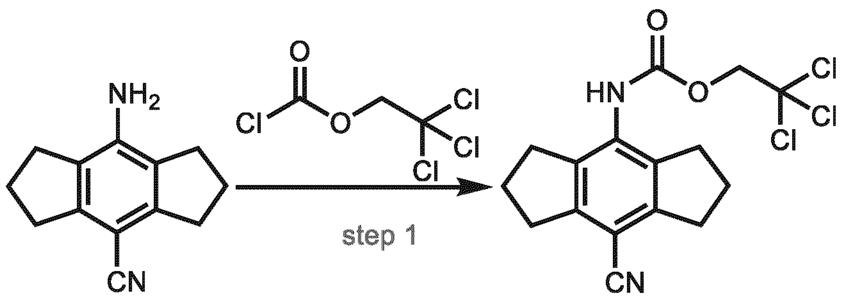


**intermediate 40** **intermediate 173**

**Intermediate 173**

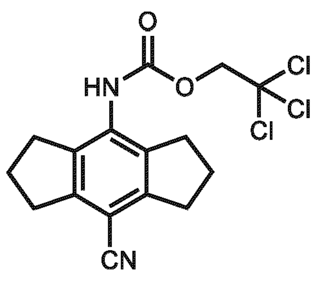

2,2,2-Trichloroethyl (8-cyano-1,2,3,5,6,7-hexahydro-s-indacen-4-yl)carbamate

To a stirred solution of 8-amino-1,2,3,5,6,7-hexahydro-s-indacene-4-carbonitrile (700 mg, 3.54 mmol) in THF (10 mL) in a 100-mL round-bottom flask was added DIEA (912 mg, 7.07 mmol) and 2,2,2-trichloroethyl chloroformate

(1.49 g, 7.07 mmol). The resulting solution was stirred for 12 h at RT. The reaction was then quenched by the addition of 20 mL of water. The resulting solution was extracted with 3x30 mL of EtOAc and the organic layers combined and dried over anhydrous $Na_2SO_4$ and concentrated under vacuum. The residue was eluted from silica gel with EtOAc/PE (1:3). This resulted in 1.1 g (83.3%) of the title compound as yellow solid. MS-ESI: 373/375/377 (M+1).

**Table 34**. The Intermediates in the following Table were prepared using the similar procedures for converting Intermediate **40** to Intermediate **173** shown in Scheme 122.

| Intermediate # | Structure | IUPAC Name | Exact Mass $[M+H]^+$ |
|---|---|---|---|
| **Intermediate 174** | 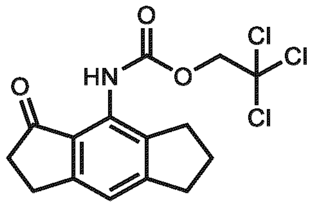 | 2,2,2-Trichloroethyl (3-oxo-1,2,3,5,6,7-hexahydro-*s*-indacen-4-yl)carbamate | 362/354/ 367 |
| **Intermediate 175** | 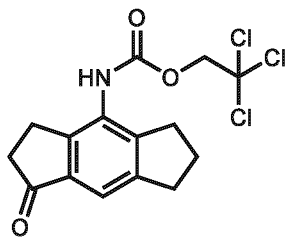 | 2,2,2-Trichloroethyl (1-oxo-1,2,3,5,6,7-hexahydro-*s*-indacen-4-yl)carbamate | 362/364/ 367 |

## Scheme 123:

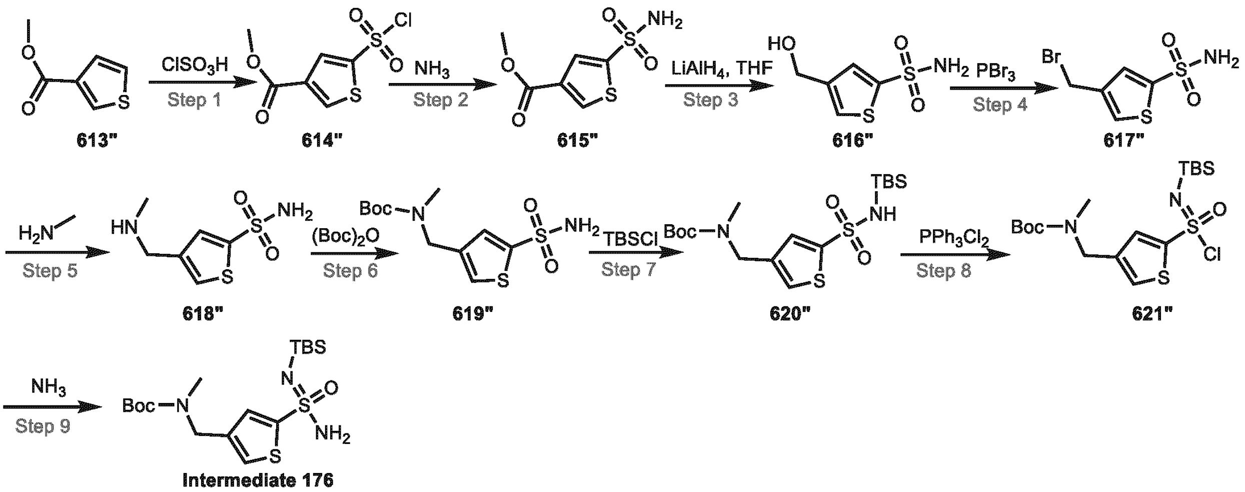


## Intermediate 176

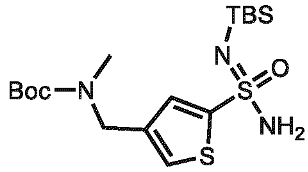

Tert-butyl ((5-(N'-(tert-butyldimethylsilyl)sulfamidimidoyl)thiophen-3-yl)methyl)(methyl)carbamate

Steps 1-2 used similar procedures for converting compound **431"** to compound **433"** shown in Scheme 91 to afford compound **615"** from compound **613"**. MS-ESI: 219 (M-1).

Steps 3-6 used similar procedures for converting compound **242** to compound **470"** shown in Scheme 97 to afford intermediate **619"** from compound **615"**. MS-ESI: 307 (M+1).

Steps 7-9 used similar procedures for converting compound **362"** to intermediate **124** shown in Scheme 80 to afford intermediate **176** from compound **619"**. MS-ESI: 420 (M+1).

## Scheme 124:

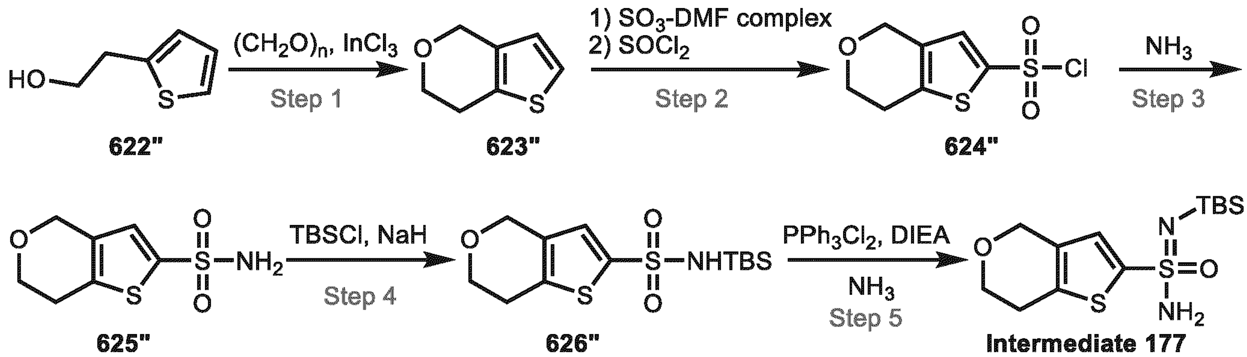


## Intermediate 177

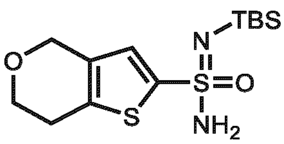

N'-(tert-butyldimethylsilyl)-6,7-dihydro-4H-thieno[3,2-c]pyran-2-sulfonimidamide

**Step 1: 6,7-Dihydro-4H-thieno[3,2-c]pyran**

To a stirred solution of 2-(thiophen-2-yl)ethan-1-ol (10 g, 78 mmol) in ACN (400 mL) in a 1-L round-bottom flask under nitrogen was added paraformaldehyde (3.02 g, 34 mmol) at RT, followed by the addition of $InCl_3$ (863 mg, 3.9 mmol) in portions at 0 °C. The resulting solution was stirred for 2 h at 70 °C. The solids were filtered out. The resulting mixture was concentrated under vacuum. The residue was eluted from silica gel with EtOAc/PE (1:10). This resulted in 6.0 g (55%) of the title compound as brown oil. MS-ESI: 141 (M+1).

**Step 2: 6,7-Dihydro-4H-thieno[3,2-c]pyran-2-sulfonyl chloride**

To a stirred solution of 6,7-dihydro-4H-thieno[3,2-c]pyran (2.0 g, 14 mmol) in DCE (100 mL) under nitrogen was added N,N-dimethylformamide sulfur trioxide complex ($SO_3$-DMF complex) (2.62 g, 17 mmol) dropwise at RT. The resulting solution was stirred for 1 h at RT. Then to the stirred solution was added $SOCl_2$ (2.04 g, 17 mmol) dropwise at 0 °C. The resulting solution was stirred for 4 h at 50 °C. The reaction solution was quenched with ice/water (20 mL). The resulting mixture was extracted with EtOAc (4x100 mL). The organic layers were combined and concentrated under vacuum. This resulted in 2.1 g (crude) of the title compound as a yellow solid.

**Step 3: 6,7-Dihydro-4H-thieno[3,2-c]pyran-2-sulfonamide**

To stirred solution of 6,7-dihydro-4H-thieno[3,2-c]pyran-2-sulfonyl chloride (2.1 g, crude from last step) in DCM (100 mL) was introduced $NH_3$ gas bubbled for 15 min at 0 °C. The resulting solution was stirred overnight at RT. The reaction solution was concentrated under vacuum. The residue was eluted from silica gel with EtOAc/PE (1:1). This resulted in 800 mg (25.6% over two steps) of the title compound as a yellow solid. MS-ESI: 218 (M-1).

Steps 4-5 used similar procedures for converting compound **362"** to intermediate **124** shown in Scheme 80 to afford intermediate **177** from compound **625"**. MS-ESI: 333 (M+1).

## Scheme 125:

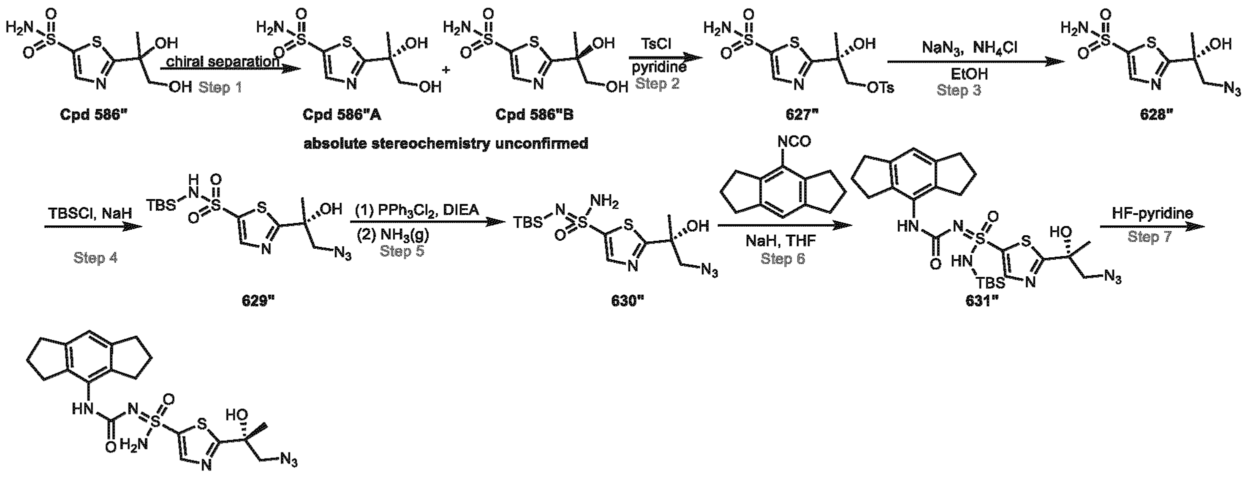


Cpd 586" Cpd 586"A + Cpd 586"B 627" 628"

absolute stereochemistry unconfirmed

629" 630" 631"

Intermediate 178

## Intermediate 178

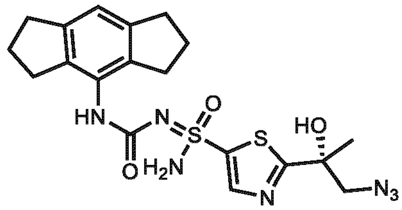

2-((R)-1-azido-2-hydroxypropan-2-yl)-N'-((1,2,3,5,6,7-hexahydro-*s*-indacen-4-yl)carbamoyl)thiazole-5-sulfonimidamide

**Step 1: (R) and (S)-2-(1,2-dihydroxypropan-2-yl)thiazole-5-sulfonamide**

**Compound 586"** (10 g) was resolved by Prep-Chiral-HPLC with the following conditions: CHIRALPAK AD, 5*25 cm, 5 um; Mobile Phase A: $CO_2$, Mobile Phase B: MeOH(2 mM $NH_3$-MeOH); Flow rate: 200 mL/min; Gradient:40% B; 220 nm; Rti: 3.5 (**Compound 586"A**); $Rt_2$: 5.6 (**Compound 586"B**). This resulted in 4.1 g (99%ee) of **Compound 586"A** and 4.0 g (98%ee) of **Compound 586"B** both as a white solid. MS-ESI: 237 (M-1).

**Step 2: (R)-2-hydroxy-2-(5-sulfamoylthiazol-2-yl)propyl 4-methylbenzenesulfonate**

To a stirred solution of (R)-2-(1,2-dihydroxypropan-2-yl)thiazole-5-sulfonamide (4.0 g, 17 mmol) in pyridine (40 mL) in a 250-mL round-bottom flask under nitrogen was added 4-methylbenzenesulfonyl chloride (4.8 g, 25 mmol) in portions at 0 °C. The resulting solution was stirred overnight at RT. The resulting mixture was quenched with HCl (1 M, 40 mL). The resulting mixture was extracted with 3x40 mL ethyl acetate. The combined organic layer was dried over anhydrous $Na_2SO_4$. After filtration, the filtrate was concentrated under vacuum. The residue was eluted from silica gel with PE/EtOAc (1:1). This resulted in 5.2 g (79%) of the title compound as a yellow solid. MS-ESI: 391 (M-1).

**Sep 3: (R)-2-(1-azido-2-hydroxypropan-2-yl)thiazole-5-sulfonamide**

To a stirred solution of (R)-2-hydroxy-2-(5-sulfamoylthiazol-2-yl)propyl 4-methylbenzenesulfonate (3.2 g, 8.2 mmol) in EtOH (40 mL) in a 250-mL round-bottom flask under nitrogen was added $NH_4Cl$ (2.18 g, 41 mmol) at RT, followed by the addition of $NaN_3$(1.3 g, 20 mmol) in portions at 0 °C carefully. The resulting solution was stirred overnight at 80 °C. The resulting mixture was diluted with water (30 mL). The resulting mixture was extracted with 3x30 mL ethyl acetate. The combined organic layer was dried over anhydrous $Na_2SO_4$. After filtration, the filtrate was concentrated under reduced pressure. The residue was eluted from silica gel with PE/EtOAc (1:1). This resulted in 2.1 g (98%) of the

title compound as a yellow solid. MS-ESI: 264 (M+1).

Steps 4-5 used similar procedures for converting compound **362"** to intermediate **124** shown in Scheme 80 to afford compound **630"** from compound **628"**. MS-ESI: 377 (M+1).

**Step 6: 2-((R)-1-azido-2-hydroxypropan-2-yl)-N-(tert-butyldimethylsilyl)-N'-((1,2,3,5,6,7-hexahydro-s- indacen-4-yl)carbamoyl)thiazole-5-sulfonimidamide**

To a stirred solution of 2-((R)-1-azido-2-hydroxypropan-2-yl)-N'-(tertbutyldimethylsilyl)thiazole-5- sulfonimidamide (1.7 g, 4.5 mmol) in THF (10 mL) in a 100-mL round-bottom flask under nitrogen was added NaH (60% wt. dispersion in mineral oil, 540 mg, 13.5 mmol) in portions at 0 °C. The resulting solution was stirred for 5 min at 0 °C. To the above solution was added 4-isocyanato-1,2,3,5,6,7-hexahydro-s-indacene (900 mg, 4.5 mmol) in small portions at 0 °C. The resulting mixture was stirred for 1 h at RT. The reaction was quenched with water/ice (10 mL) at 0 °C. The resulting mixture was extracted with ethyl acetate (3 x 15 mL). The combined organic layer was dried over anhydrous $Na_2SO_4$. After filtration, the filtrate was concentrated under reduced pressure. This resulted in 3.0 g (crude) of the title compound as yellow oil. MS-ESI: 576 (M+1).

**Step 7: 2-((R)-1-azido-2-hydroxypropan-2-yl)-N'-((1,2,3,5,6,7-hexahydro-s-indacen-4-yl)carbamoyl) thiazole-5-sulfonimidamide**

To a stirred solution of 2-((R)-1-azido-2-hydroxypropan-2-yl)-N-(tert-butyldimethylsilyl)-N'-((1,2,3,5,6,7- hexahydro-s-indacen-4-yl)carbamoyl)thiazole-5-sulfonimidamide (3.0 g, crude) in THF (20 mL) in a 100-mL round-bottom flask was added HF-pyridine (2.0 mL) dropwise at °C. The resulting solution was stirred for 1 h at RT. The resulting mixture was concentrated under vacuum. The residue was eluted from silica gel with DCM/MeOH (10:1). This resulted in 1.6 g (76.8% over two steps) of the title compound as a yellow solid. MS-ESI: 462 (M+1).

**Scheme 126:**

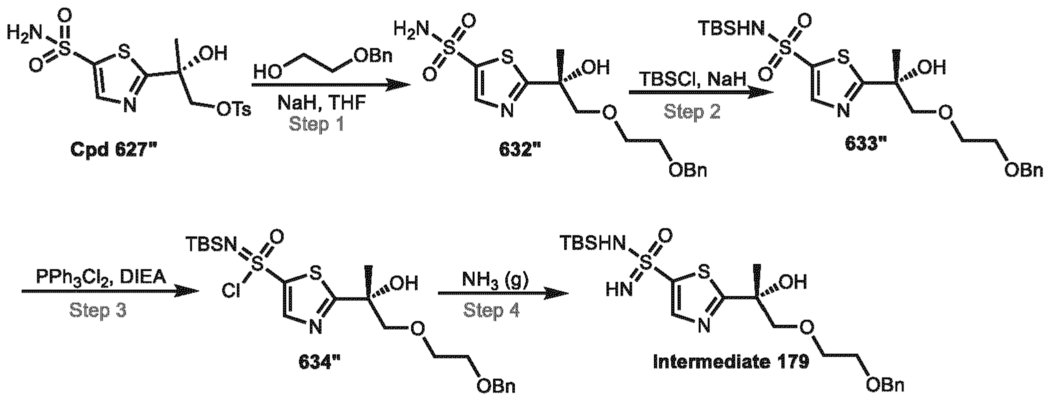


**Intermediate 179**

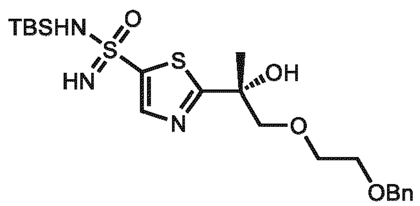

2-((R)-1-(2-(benzyloxy)ethoxy)-2-hydroxypropan-2-yl)-N-(tert-butyldimethylsilyl)thiazole-5-sulfonimidamide

**Step 1: (R)-2-(1-(2-(benzyloxy)ethoxy)-2-hydroxypropan-2-yl)thiazole-5-sulfonamide**

To a stirred solution of 2-(benzyloxy)ethan-1-ol (3.88 g, 25.5 mmol) in THF (30 mL) in a 100-mL round-bottom flask under nitrogen was added NaH (60% wt. dispersion in mineral oil, 2.04 g, 51 mmol) in portions at 0 °C. The resulting mixture was stirred for 30 min at 0 °C. (R)-2-hydroxy-2-(5-sulfamoyl- thiazol-2-yl)propyl 4-methylbenzenesulfonate (2.0 g, 5.1 mmol) in THF (10 mL) was added to the solution dropwise at 0 °C. The resulting solution was stirred for 32 h at

35 °C. The reaction was quenched with ice/water (100 mL) at 0 °C. The resulting mixture was extracted with ethyl acetate (3 x 200 mL). The combined organic layers were washed with brine (2x200 mL) and dried over anhydrous $Na_2SO_4$. After filtration, the filtrate was concentrated under reduced pressure. The residue was eluted from silica gel with PE/EtOAc (1:2). This resulted in 1.8 g (95%) of the title compound as a yellow solid. MS-ESI: 371 (M-1). Steps 2-4 used similar procedures for converting compound **362"** to intermediate **124** shown in Scheme 80 to afford intermediate **179"** from compound 632". MS-ESI: 486 (M+1).

**Table 35**. The Intermediate 180 was prepared using similar procedures for converting compound **627"** to Intermediate **179** shown in Scheme 126 from appropriate starting materials.

| Intermediate # | Structure | IUPAC Name | Exact Mass $[M+H]^+$ |
|---|---|---|---|
| **Intermediate 180** | 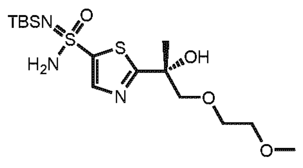 | N-(tert-bulyldimethylsilyl)-2-((R)-2-hydroxy-1-(2-methoxyethoxy)propan-2-yl)thiazole-5-sulfonimidamide | 410 |

## Scheme 127:

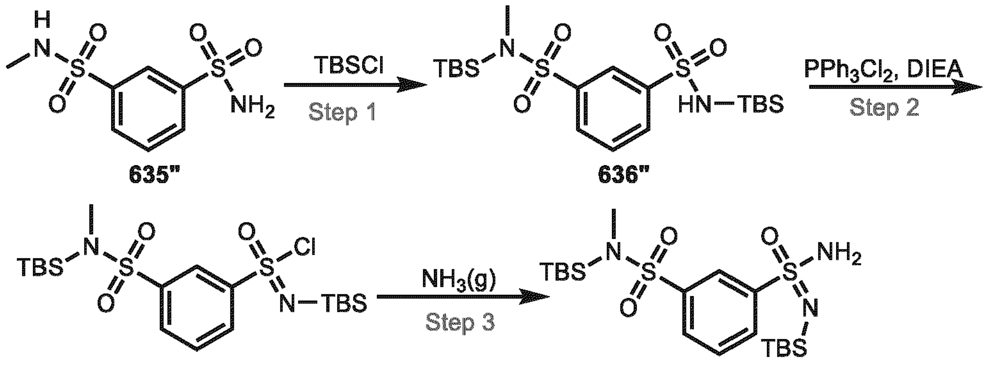


635"

636"

637"

**Intermediate 181**

## Intermediate 181

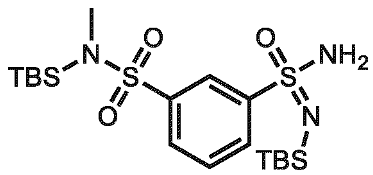

N-(tert-bulyldimethyl silyl)-3 -(N'-(tert-bulyldimethyl silyl) sulfamidimidoyl)-N-methylbenzenesulfonamide

**Step 1: $N_1$,$N_3$-bis(tert-butyldimethylsilyl)-N1-methylbenzene-1,3-disulfonamide**

To a stirred solution of N-methylbenzene-1,3-disulfonamide (220 mg, 0.88 mmol) in THF (6 mL) in a 25-mL round-bottom flask under nitrogen was added NaH (60% wt. dispersion in mineral oil, 211 mg, 5.28 mmol) in portions at 0 °C. The resulting solution was stirred for 20 min at RT. To the solution was added TBSCl (397 mg, 2.64 mmol) in portions at 0 °C. The resulting solution was stirred overnight at RT. The reaction solution was quenched with 10 mL water. The mixture was extracted with 3x20 mL ethyl acetate. The organic layer was dried with anhydrous $Na_2SO_4$ and concentrated under vacuum. The residue was eluted from silica gel with PE/EtOAc (1:2). This resulted in 400 mg (95%) of the title compound as yellow oil. MS-ESI: 479 (M+1). Steps 2-4 used similar procedures for converting compound **363"** to intermediate **124** shown in Scheme 80 to afford intermediate **181** from compound 636". MS-ESI: 478 (M+1).

**Table 36**. The Intermediate 146 was prepared using similar procedures for converting compound **459"** to Intermediate **139** shown in Scheme 95 from appropriate starting materials.

| Intermediate # | Structure | IUPAC Name | Exact Mass $[M+H]^+$ |
|---|---|---|---|
| **Intermediate 182** | 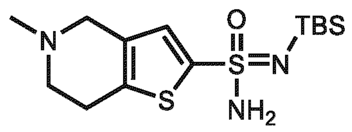 | N'-(tert-butyldimethylsilyl)-5-methyl-4,5,6,7-tetrahydrothieno [3,2-c] pyridine-2-sulfonimidamide | 346 |

**Scheme for the preparation of Key Intermediates:** Schemes below illustrate the preparation of key intermediates.

## Scheme 128:

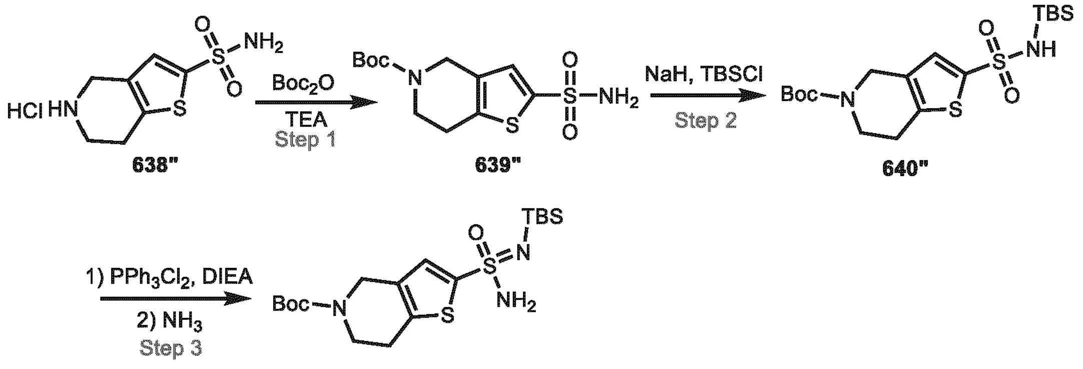


**Intermediate 183**

## Intermediate 183

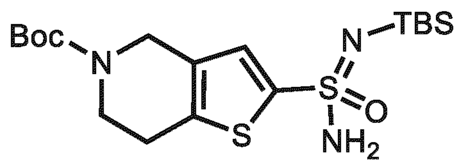

Tert-butyl 2-(N'-(tert-butyldimethylsilyl)sulfamidimidoyl)-6,7-dihydrothieno[3,2-c]pyridine-5(4H)-carboxylate

**Step 1: Tert-butyl 2-sulfamoyl-6,7-dihydrothieno[3,2-c]pyridine-5(4H)-carboxylate**

To a stirred solution of 4,5,6,7-tetrahydrothieno[3,2-c]pyridine-2-sulfonamide hydrochloride (1.0 g, 4.58 mmol, an intermediate to Intermediate 182) in THF (20 mL) in a 100-mL round-bottom flask were added TEA (1.39 g, 13.7 mmol) at RT, followed by the addition of $Boc_2O$ (1.0 g, 4.58 mmol) in portions at RT. The reaction mixture was stirred for 2 h at RT and then was concentrated under vacuum. The residue was eluted from silica gel with EtOAc (100%). This resulted in 1.0 g (69%) of the title compound as a light yellow solid. MS-ESI: 319 (M+1).

**Step 2: Tert-butyl 2-(N-(tert-butyldimethylsilyl)sulfamoyl)-6,7-dihydrothieno[3,2-c]pyridine-5(4H)- carboxylate**

To a stirred solution of tert-butyl 2-sulfamoyl-6,7-dihydrothieno[3,2-c]pyridine-5(4H)-carboxylate (500 mg, 1.57 mmol) in THF (15 mL) in a 100-mL round-bottom flask under nitrogen was added NaH (60% wt. dispersion in mineral oil, 126 mg, 3.14 mmol) in portions at 0 °C. The resulting mixture was stirred for 10 min at RT, followed by the addition of TBSCl (473 mg, 3.14 mmol) in portions at 0 °C. The reaction mixture was stirred for 2 h at RT. The reaction was quenched with water (10 mL) at 0 °C. The mixture was extracted with EtOAc (3x20 mL) and the organic layers were combined. The organic layer was dried over anhydrous $Na_2SO_4$ and concentrated under vacuum. This resulted in 723

mg (crude) of the title compound as light yellow liquid. MS-ESI: 433 (M+1).

**Step 3: Tert-butyl 2-(N-(tert-butyldimethylsilyl)sulfamidimidoyl)-6,7-dihydrothieno[3,2-c]pyridine-5 (4H)-carboxylate**

To a stirred mixture of $PPh_3Cl_2$ (693 mg, 2.08 mmol) in $CHCl_3$ (10 mL) a 100-mL 3-necked round-bottom flask under nitrogen was added DIEA (537 mg, 4.16 mmol) dropwise at 0 °C. The reaction mixture was stirred for 15 min at 0 °C. To the above mixture was added tert-butyl 2-(N-(tert-butyldimethylsilyl)sulfamoyl)-6,7-dihydrothieno[3,2-c]pyridine-5(4H)-carboxylate (723 mg, crude) in $CHCl_3$ (5.0 mL) dropwise over 5 min at 0 °C. The reaction mixture was stirred for additional 30 min at 0 °C. $NH_3$ (gas) was bubbled into the above mixture for 15 min at 0 °C. The reaction mixture was stirred overnight at RT. The reaction mixture was filtered, the filter cake was washed with DCM (10 mL). The filtrate and wash were combined and concentrated under reduced pressure. The residue was eluted from silica gel with EtOAc (100%). This resulted in 300 mg (44%, over two steps) of the title compound as a light yellow solid. MS-ESI: 432 (M+1).

## Scheme 129:

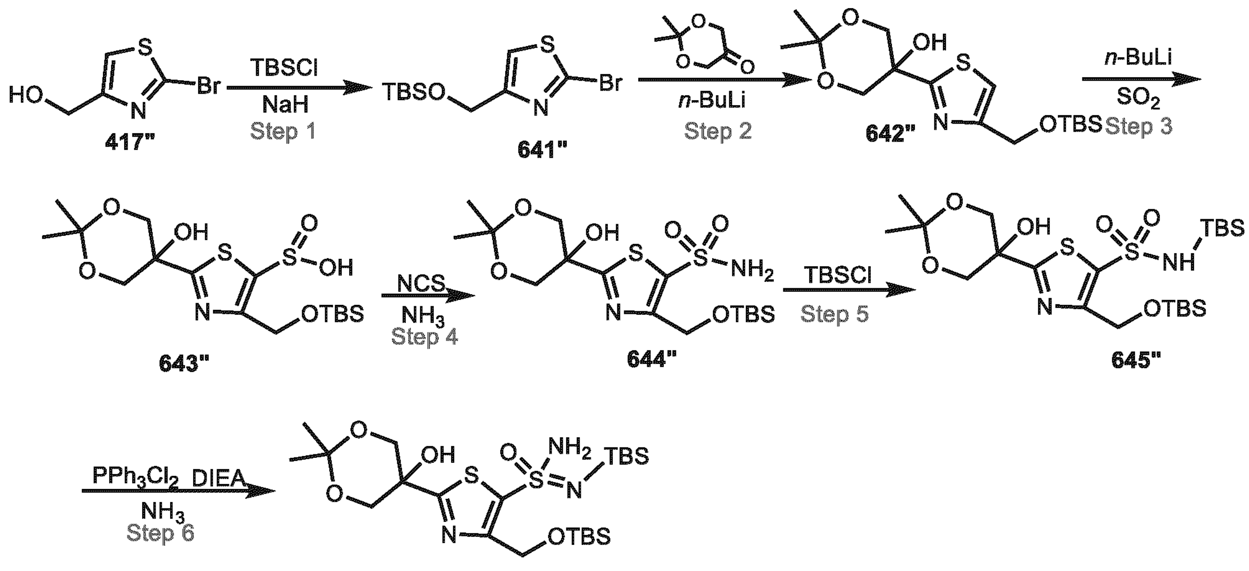


**Intermediate 184**

## Intermediate 184

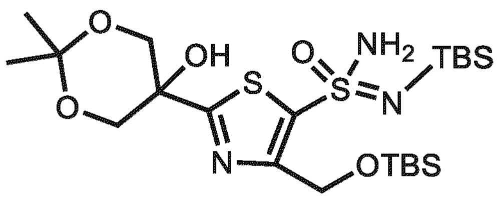

N'-(tert-butyldimethylsilyl)-4-(((tert-butyldimethylsilyl)oxy)methyl)-2-(5-hydroxy-2,2-dimethyl-1,3-dioxan-5-yl)thiazole-5-sulfonimidamide

**Step 1: 2-Bromo-4-(((tert-butyldimethylsilyl)oxy)methyl)thiazole**

To a stirred solution of (2-bromothiazol-4-yl)methanol (13 g, 67 mmol) in THF (150 mL) in a 250-mL round-bottom flask under nitrogen was added NaH (60% wt. dispersion in mineral oil, 8.0 g, 201 mmol) in portions at 0 °C in an ice/water bath. The reaction mixture was stirred for 10 min at RT. Then to the above mixture was added TBSCl (30.3 g, 201 mmol) in portions at 0 °C. The reaction mixture was stirred for 2 h at RT. The reaction was quenched with 200 mL of water. The mixture was extracted with 3x300 mL of EtOAc and the organic layers were combined, dried over

anhydrous $Na_2SO_4$, and concentrated under vacuum. The residue was eluted from silica gel with EtOAc/PE (1 :30). This resulted in 15 g (73%) of the title compound as yellow oil. MS-ESI: 308/310 (M+1).

**Step 2: 5-(4-(((Tert-butyldimethylsilyl)oxy)methyl)thiazol-2-yl)-2,2-dimethyl-1,3-dioxan-5-ol**

To a stirred solution of 2-bromo-4-(((tert-butyldimethylsilyl)oxy)methyl)thiazole (1.4 g, 4.55 mmol) in THF (30 mL) in a 100-mL 3-necked round-bottom flask under nitrogen was added n-BuLi in hexane (2.5 M, 1.8 mL, 4.55 mmol) dropwise at -78 °C. The reaction solution was stirred for 30 min at -78 °C. Then to the solution was added 2,2-dimethyl-1,3-dioxan-5-one (621 mg, 4.78 mmol) in THF (5 mL) dropwise at -78 °C. The reaction mixture was stirred for 1 h at -78 °C. The reaction was quenched with $H_2O$ (20 mL) at 0 °C. The resulting mixture was extracted with EtOAc (3x30 mL). The combined organic layers were washed with brine (50 mL), dried over anhydrous $Na_2SO_4$ and concentrated under vacuum. The residue was purified by prep-TLC (PE/EtOAc 10: 1). This resulted in 1.1 g (67%) of the title compound as brown oil. MS-ESI: 360 (M+1).

**Step 3: 4-(((Tert-butyldimethylsilyl)oxy)methyl)-2-(5-hydroxy-2,2-dimethyl-1,3-dioxan-5-yl)thiazole-5-sulfinic acid, lithium salt**

To a stirred solution of 5-(4-(((tert-butyldimethylsilyl)oxy)methyl)thiazol-2-yl)-2,2-dimethyl-1,3-dioxan-5-ol (1.1 g, 3.06 mmol) in THF (30 mL) in a 100-mL 3-necked round-bottom flask under nitrogen was added n-BuLi in hexane (2.5 M, 2.7 mL, 6.73 mmol) dropwise at -78 °C. The reaction solution was stirred for 30 min at -78 °C. Then $SO_2$ (g) was bubbled to the solution at - 50 °C for 20 min. The reaction mixture was allowed to react with stirring for an additional 2 h at RT. The mixture was concentrated directly under vacuum. This resulted in 1.5 g (crude) of the title compound as a yellow solid. MS-ESI: 422 (M-1).

**Step 4: 4-(((Tert-butyldimethylsilyl)oxy)methyl)-2-(5-hydroxy-2,2-dimethyl-1,3-dioxan-5-yl)thiazole-5- sulfonyl chloride**

To a stirred solution of 4-(((tert-butyldimethylsilyl)oxy)methyl)-2-(5-hydroxy-2,2-dimethyl-1,3-dioxan-5-yl) thiazole-5-sulfinic acid, lithium salt (1.5 g, crude) in THF (20 mL) in a 100-mL round-bottom flask was added NCS (610 mg, 4.59 mmol) in portions at 0 °C. The reaction mixture was stirred for 2 h at 0 °C. To the reaction solution was added $NH_3$ in THF (0.5 M, 50 mL) in portions at 0 °C. The reaction mixture was stirred for 2 h at 0 °C. The mixture was concentrated under vacuum. The residue was purified by prep-TLC (PE/EtOAc 7: 1). This resulted in 685 mg (51%, over two steps) of the title compound as a brown solid. MS-ESI: 439 (M+1).

Steps 5-6 used similar procedures for converting compound **639"** to Intermediate **183** shown in Scheme 128 to afford Intermediate **184** from compound **644".** MS-ESI: 552 (M+1).

**Scheme 130:**

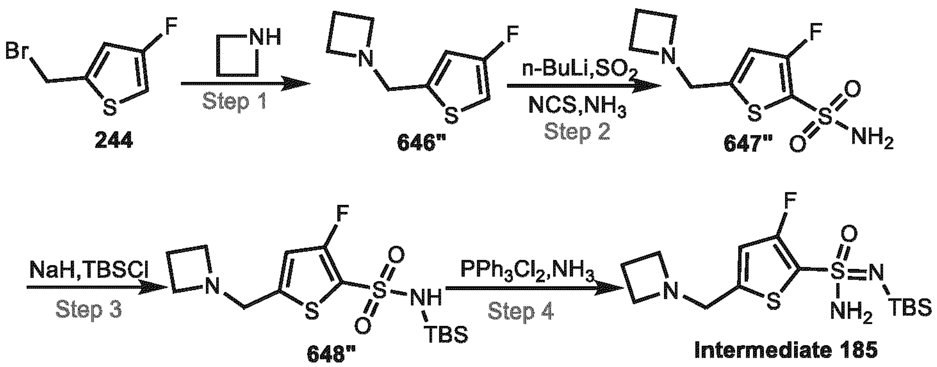

## Intermediate 185

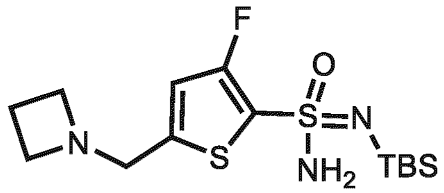

5-(Azetidin-1-ylmethyl)-N'-(tert-bulyldimethylsilyl)-3-fluorothiophene-2-sulfonimidamide

**Step 1: 1-((4-Fluorothiophen-2-yl)methyl)azetidine**

To a stirred solution of 2-(bromomethyl)-4-fluorothiophene (2.4 g, 12.4 mmol) in THF (25 mL) in a 100-mL round-bottom flask under nitrogen were added azetidine (1.06 g, 18.6 mmol) and TEA (2.49 g, 25 mmol) in portions at RT. The reaction solution was stirred for 2 h at RT. The reaction solution was concentrated under vacuum. The residue was eluted from silica gel with EtOAc/PE (1:1). This resulted in 2.0 g (94%) of the title compound as yellow oil. MS-ESI: 172 (M+1).

Steps 2-4 used similar procedures for converting compound **642"** to Intermediate **184** shown in Scheme 129 to afford Intermediate **185** from compound **646".** MS-ESI: 364 (M+1).

**Scheme 131:**

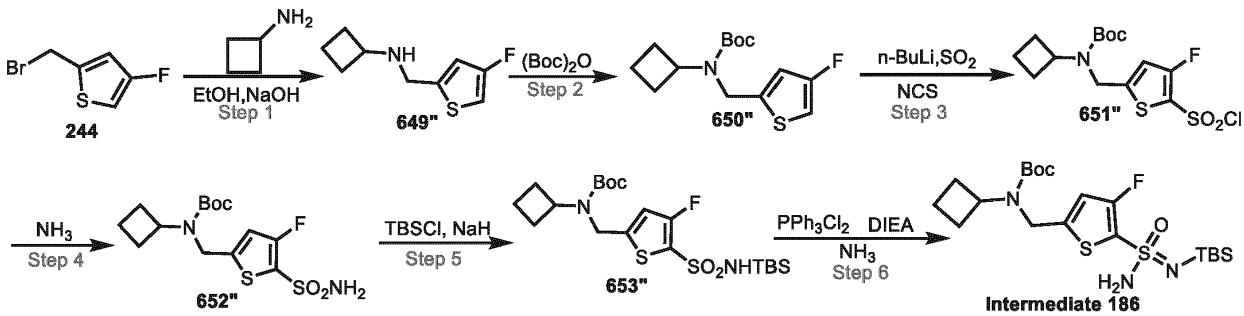


## Intermediate 186

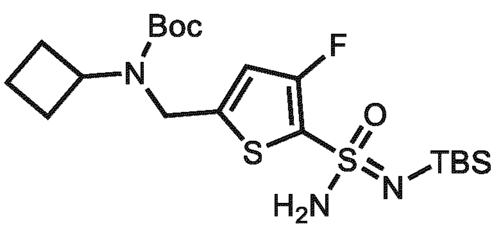

Tert-butyl ((5-(N'-(tert-butyldimethylsilyl)sulfamidimidoyl)-4-fluorothiophen-2-yl)methyl)(cyclobutyl)carbamate

**Step 1: N-((4-fluorothiophen-2-yl)methyl)cyclobutanamine**

To a stirred solution of 2-(bromomethyl)-4-fluorothiophene (4.5 g, 23 mmol) in EtOH (100 mL) in a 250-mL round-bottom flask under nitrogen were added cyclobutanamine (2.0 g, 28 mmol) at 0 °C, followed by the addition of NaOH (1.5 g, 38 mmol) in portions at 0 °C. The reaction mixture was stirred overnight at RT. The reaction mixture was concentrated under vacuum. The residue was eluted from silica gel with EtOAc/PE (1:3). This resulted in 3.5 g (82%) of the title compound as a yellow solid. MS-ESI: 186 (M+1).

**Step 2: Tert-butyl cyclobutyl((4-fluorothiophen-2-yl)methyl)carbamate**

To stirred solution of N-((4-fluorothiophen-2-yl)methyl)cyclobutanamine (3.5 g, 19 mmol) in DCM (50 mL) in a 250-mL round-bottom flask under nitrogen were added $Et_3N$ (5.4 mL, 38 mmol) at RT, followed by the addition of $Boc_2O$ (5.0 g, 23 mmol) at RT. The reaction mixture was stirred for 3 h at RT. The reaction was quenched with 100 mL of water. The mixture was extracted with 3x100 mL of DCM and the organic layers were combined and concentrated under vacuum. The residue was eluted from silica gel with EtOAc/PE (5:1). This resulted in 4.6 g (85%) of the title compound as a yellow solid. MS-ESI: 286 (M+1).

Steps 3-6 used similar procedures for converting compound **642"** to Intermediate **184** shown in Scheme 129 to afford Intermediate **186** from compound **650".** MS-ESI: 478 (M+1).

**Scheme 132:**

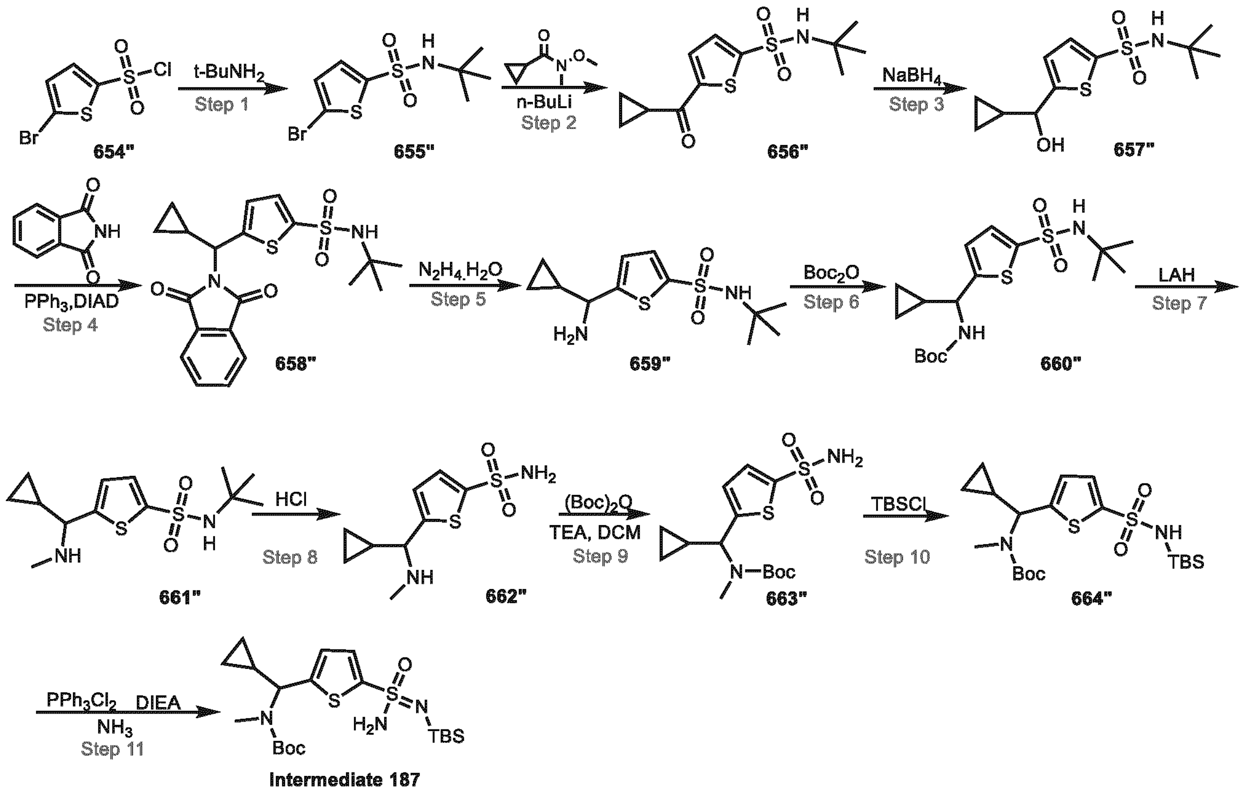


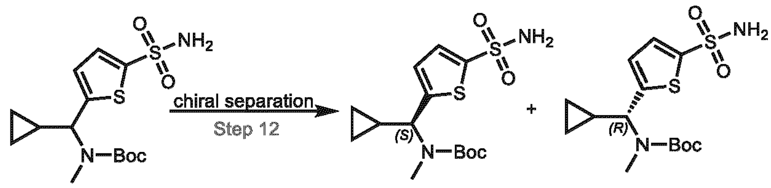


663" 663"F 663"S

**Intermediate 187**

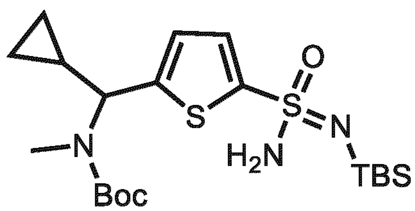

Tert-butyl ((5-(N'-(tert-butyldimethylsilyl)sulfamidimidoyl)thiophen-2-yl)(cyclopropyl)methyl)(methyl)carbamate

**Step 1: 5-Bromo-N-(tert-butyl)thiophene-2-sulfonamide**

To a stirred solution of 5-bromothiophene-2-sulfonyl chloride (20 g, 77 mmol) in DCM (200 mL) in a 1-L round-bottom flask were added 2-methylpropan-2-amine (8.43 g, 116 mmol) at RT, followed by the addition of TEA (15.6 g, 154 mmol) at RT. The reaction mixture was stirred overnight at RT and then was quenched with 10 mL of water. The mixture was extracted with 3x150 mL of DCM and the organic layers were combined and dried over anhydrous $Na_2SO_4$ and concentrated under vacuum. This resulted in 20.1 g (88%) of the title compound as a red solid. MS-ESI: 298/300 (M+1).

**Step 2: N-(tert-butyl)-5-(cyclopropanecarbonyl)thiophene-2-sulfonamide**

To a stirred solution of 5-bromo-N-(tert-butyl)thiophene-2-sulfonamide (20 g, 67 mmol) in THF (500 mL) in a 1-L 3-necked round-bottom flask under nitrogen was added n-BuLi in hexane (2.5 M, 67 mL, 168 mmol) dropwise at -78 °C. The reaction solution was stirred for 1 h at -78 °C. To the reaction solution was added N-methoxy-N-methylcyclopropanecarboxamide (35 g, 270 mmol) in THF (20 mL) dropwise at -78 °C. Then the reaction mixture was stirred for 1 h at -78 °C. The reaction was quenched with 20 mL of MeOH. The mixture was concentrated under vacuum. The residue was eluted from silica gel with EtOAc/PE (21%). This resulted in 7.1 g (37%) of the title compound as a white solid. MS-ESI: 288 (M+1).

**Step 3: N-(tert-butyl)-5-(cyclopropyl(hydroxy)methyl)thiophene-2-sulfonamide**

To a stirred solution of N-(tert-butyl)-5-(cyclopropanecarbonyl)thiophene-2-sulfonamide (2.0 g, 6.96 mmol) in MeOH (40 mL) in a 100-mL round-bottom flask under nitrogen was added $NaBH_4$ (529 mg, 14 mmol) at 0 °C. The reaction mixture was stirred for 2 h at RT. The reaction was quenched with 10 mL of water. The mixture was extracted with 3x100 mL of EtOAc and the organic layers were combined. The organic layer was dried over anhydrous $Na_2SO_4$ and concentrated under vacuum. This resulted in 1.8 g (89%) of the title compound as yellow oil. MS-ESI: 290 (M+1).

**Step 4: N-(tert-butyl)-5-(cyclopropyl(1,3-dioxoisoindolin-2-yl)methyl)thiophene-2-sulfonamide**

To a stirred solution of N-(tert-butyl)-5-(cyclopropyl(hydroxy)methyl)thiophene-2-sulfonamide (1.2 g, 4.15 mmol) in THF (24 mL) in a 100-mL round-bottom flask under nitrogen were added isoindoline-1,3-dione (610 mg, 4.15 mmol) and $PPh_3$ (1.63 g, 6.22 mmol) at RT. To the stirred mixture was added DIAD (1.26 g, 6.22 mmol) dropwise at 0 °C. The reaction mixture was stirred overnight at RT. The mixture was concentrated under vacuum. The residue was eluted from silica gel with EtOAc/PE (1:6). This resulted in 1.7 g (98%) of the title compound as a yellow solid. MS-ESI: 419 (M+1).

**Step 5: 5-(Amino(cyclopropyl)methyl)-N-(tert-butyl)thiophene-2-sulfonamide**

To a stirred solution of N-(tert-butyl)-5-(cyclopropyl(1,3-dioxoisoindolin-2-yl)methyl)thiophene-2- sulfonamide (200 mg, 0.48 mmol) in EtOH (10 mL) in a 50-mL round-bottom flask was added $NH_2NH_2.H_2O$ (80%wt., 180 mg, 2.88 mmol). The reaction solution was stirred for 3 h at 80 °C. The reaction solution was concentrated under vacuum. The residue was dissolved in 10 mL of DCM. The solids were filtered out and the filtrate was concentrated under vacuum. This resulted in 100 mg (73%) of the title compound as yellow oil. MS-ESI: 289 (M+1).

**Step 6: Tert-butyl ((5-(N-(tert-butyl)sulfamoyl)thiophen-2-yl)(cyclopropyl)methyl)carbamate**

To a stirred solution of 5-(amino(cyclopropyl)methyl)-N-(tert-butyl)thiophene-2-sulfonamide (100 mg, 0.35 mmol) in DCM (10 mL) in a 50-mL round-bottom flask were added TEA (70 mg, 0.69 mmol) at RT, followed by the addition of $Boc_2O$ (151 mg, 0.69 mmol) at RT. The reaction solution was stirred overnight at 40 °C. The reaction solution was diluted with $H_2O$ (10 mL). The mixture was extracted with 3x20 mL of DCM and the organic layers were combined. The organic layer was dried over anhydrous $Na_2SO_4$ and concentrated under vacuum. The residue was eluted from silica gel with EtOAc/PE (1/3). This resulted in 100 mg (74%) of the title compound as yellow oil. MS-ESI: 389 (M+1).

**Step 7: N-(tert-butyl)-5-(cyclopropyl(methylamino)methyl)thiophene-2-sulfonamide**

To a stirred solution of tert-butyl ((5-(N-(tert-butyl)sulfamoyl)thiophen-2-yl)(cyclopropyl)methyl)carbamate (100 mg, 0.26 mmol) in THF (10 mL) in a 50-mL round-bottom flask under nitrogen was added $LiAlH_4$ (30 mg, 0.78 mmol) at

0 °C. The reaction mixture was stirred for 2 h at 80 °C. The reaction was quenched with 5.0 mL of water. The reaction mixture was concentrated under vacuum. The residue was dissolved in 10 mL of MeOH. The solids were filtered and the filtrate was concentrated under vacuum. This resulted in 74 mg (94%) of the title compound as off-white oil. MS-ESI: 303 (M+1).

**Step 8: 5-(Cyclopropyl(methylamino)methyl)thiophene-2-sulfonamide**

A solution of N-(tert-butyl)-5-(cyclopropyl(methylamino)methyl)thiophene-2-sulfonamide (74 mg, 0.24 mmol) in HCl in 1,4-dioxane (4 M, 5.0 mL, 2.0 mmol) in a 50-mL round-bottom flask was stirred overnight at RT. The reaction mixture was concentrated under vacuum. The residue was eluted from silica gel with DCM/MeOH (10:1). This resulted in 53 mg (89%) of the title compound as yellow oil. MS-ESI: 247 (M+1).

**Step 9: Tert-butyl (cyclopropyl(5-sulfamoylthiophen-2-yl)methyl)(methyl)carbamate**

To a stirred solution of 5-(cyclopropyl(methylamino)methyl)thiophene-2-sulfonamide (53 mg, 0.21 mmol) in DCM (8 mL) in a 25-mL round-bottom flask was added TEA (43 mg, 0.43 mmol) at RT, followed by the addition of $Boc_2O$ (69 mg, 0.32 mmol) at RT. The reaction solution was stirred overnight at 40 °C. The reaction was diluted with $H_2O$ (8 mL). The mixture was extracted with 3x8 mL of DCM and the organic layers combined and dried over anhydrous $Na_2SO_4$ and concentrated under vacuum. The residue was eluted from silica gel with EtOAc/PE (1/3). This resulted in 51 mg (70%) of the title compound as yellow oil. MS-ESI: 347 (M+1).

Steps 10-11 used similar procedures for converting compound **639"** to Intermediate **183** shown in Scheme 128 to afford Intermediate **187** from compound **663".** MS-ESI: 460 (M+1).

**Step 12: Tert-butyl (S) and (R)-(cyclopropyl(5-sulfamoylthiophen-2-yl)methyl)(methyl)carbamate**

Tert-butyl (cyclopropyl(5-sulfamoylthiophen-2-yl)methyl)(methyl)carbamate **(663",** 1.0 g) was resolved by Chiral-Prep-HPLC using the following conditions: Column CHIRALPAK IG, 2*25 cm, 5 um; Mobile Phase A: Hex (8 mM $NH_3$·MeOH), Mobile Phase B: EtOH; Flow rate: 20 mL/min; Gradient: 20 B to 20 B over 17 min; UV 254/220 nm; Rti: 8.479 (**663"F**); $Rt_2$: 10.122 (**663"S)**; Injection Volumn:0.5 ml; Number of runs:39. This resulted in 417 mg (98% ee) of **663"F** and 407 mg (99% ee) of **663"S** both as yellow solid. MS-ESI: 347 (M+1). The stereochemistry of **663"F** and **663"S** were assigned arbitrarily for registration purpose.

**Table 54** The Intermediates in the following table were prepared using the similar procedures for converting compound **633"** to Intermediate **187** shown in Scheme 132 starting from **663"F** or **663"S.**

| Intermediate # | Structure | IUPAC Name | Exact Mass $[M-H]^-$ |
|---|---|---|---|
| **Intermediate 187F** | 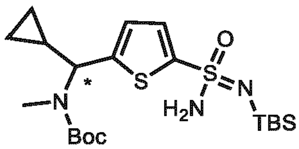 | Tert-butyl ((1S) or (1R)-(5-(N'-(tertbutyldimethylsilyl) sulfamidimidoyl)thiophen-2-yl)(cyclopropyl) methyl)(methyl)carbamate | 460 |
| **Intermediate 187S** | 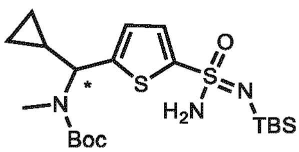 | Tert-butyl ((1R) or (1S)-(5-(N'-(tertbutyldimethylsilyl) sulfamidimidoyl)thiophen-2-yl)(cyclopropyl) methyl)(methyl)carbamate | 460 |

## Scheme 133:

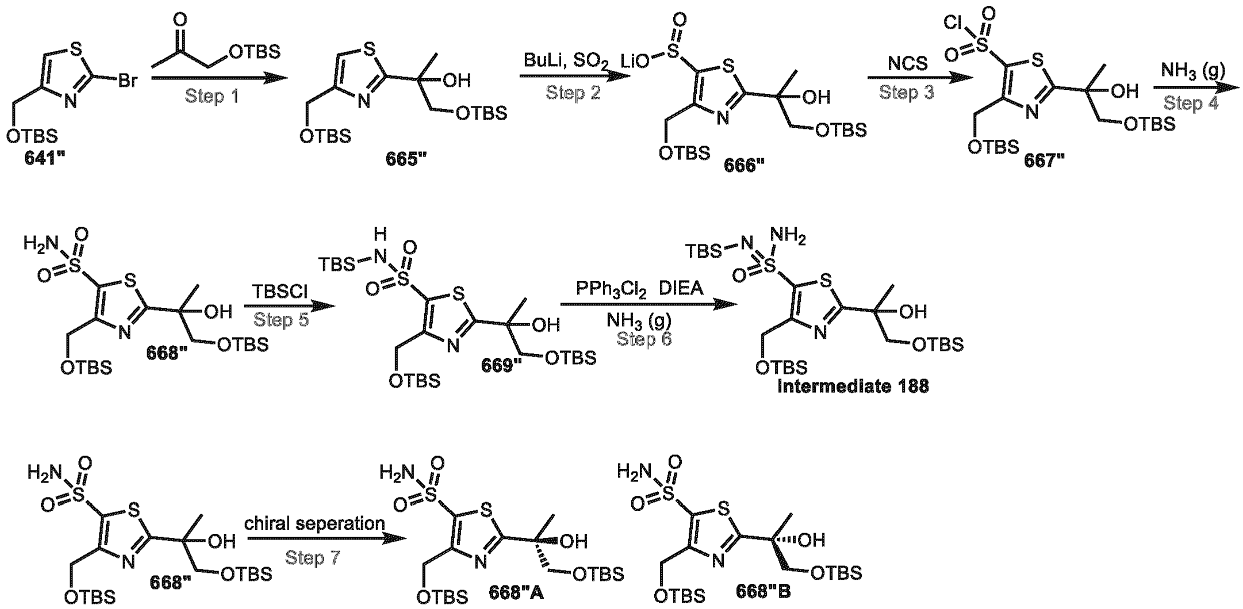


641" 665" 666" 667" 668" 669" Intermediate 188 668" 668"A 668"B

**absolute stereochemistry unconfirmed**

## Intermediate 188

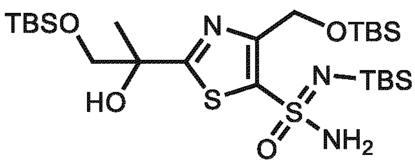

### Step 1: 1-((Tert-butyldimethylsilyl)oxy)-2-(4-(((tert-butyldimethylsilyl)oxy)methyl)thiazol-2-yl)propan -2-ol

To a stirred solution of 2-bromo-4-(((tert-butyldimethylsilyl)oxy)methyl)thiazole (300 mg, 0.97 mmol) in THF (10 mL) in a 50-mL 3-necked round-bottom flask under nitrogen was added n-BuLi in hexane (2.5 M, 0.4 mL, 0.97 mmol) dropwise at -78 °C. The reaction solution was stirred for 30 min at -78 °C. To the solution was added 1-((tertbutyldimethylsilyl)oxy)propan-2-one (183 mg, 0.97 mmol) at -78 °C over 10 min. The reaction mixture was stirred for 12 h at RT. The reaction was quenched with 10 mL of $H_2O$ (10 mL). The mixture was extracted with 3x20 mL of DCM and the organic layers were combined and concentrated under vacuum. The residue was eluted from silica gel with EtOAc/PE (1:10). This resulted in 250 mg (62%) of the title compound as yellow oil. MS-ESI: 418 (M+1).

Steps 2-6 used similar procedures for converting compound **642"** to Intermediate **184** shown in Scheme 129 to afford Intermediate **188** from compound **665".** MS-ESI: 610 (M+1).

### Step 7: (S) and (R)-2-(1-((tert-butyldimethylsilyl)oxy)-2-hydroxypropan-2-yl)-4-(((tertbutyldimethylsilyl)oxy)methyl)thiazole-5-sulfonamide

2-(1-((Tert-butyldimethylsilyl)oxy)-2-hydroxypropan-2-yl)-4-(((tertbutyldimethylsilyl)oxy)methyl)thiazole-5-sulfonamide **(668",** 1.0 g) was resolved by Prep-SFC using the following conditions: Column: Lux Cellulose-4, 5*25 cm, 5 um; Mobile Phase A: $CO_2$, Mobile Phase B: MeOH; Flow rate: 150 mL/min; Gradient: 25% B; 220 nm; Rti: 3.81 min. (**668"A**); $Rt_2$: 4.88 min. (**668"B)**; Injection Volume: 1.5 ml; number of runs: 40. This resulted in 420 mg (98%ee) of **668"A** and 428 mg (99%ee) of **668"B** both as yellow solid. MS-ESI: 497 (M+1).

**Table 55** The intermediates in the following table were prepared using the similar procedures for converting compound **668"** to Intermediate **188** shown in Scheme 133 starting from **668"A** or **668"B.**

| Intermediate # | Structure | IUPAC Name | Exact Mass [M-H]- |
|---|---|---|---|
| **Intermediate 188A** | 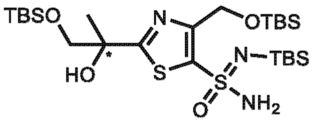 | (R) or (S) N'-(tert-butyldimethylsilyl)-2-(1-((tertbutyldimethylsilyl)oxy)-2-hydroxypropan-2-yl)-4-(((tertbutyldimethylsilyl) oxy)methyl)thiazole-5-sulfonimidamide | 610 |
| **Intermediate 188B** | 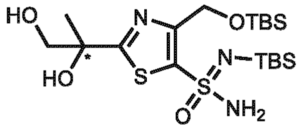 | (S) or (R) N'-(tert-butyldimethylsilyl)-2-(1-((tertbutyldimethylsilyl)oxy)-2-hydroxypropan-2-yl)-4-(((tertbutyldimethylsilyl) oxy)methyl)thiazole-5-sulfonimidamide | 610 |

## Scheme 134:

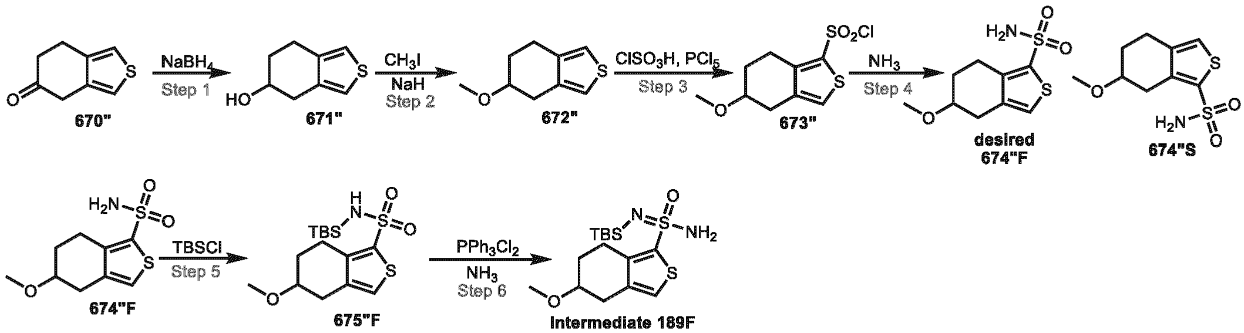


## Intermediate 189F

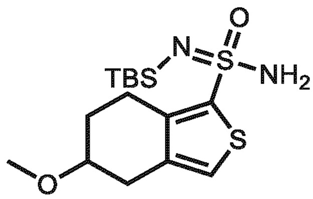

N'-(tert-buiyldimethylsilyl)-5-methoxy-4,5,6,7-tetrahydrobenzo[c]thiophene-1-sulfonimidamide

**Step 1: 4,5,6,7-Tetrahydrobenzo[c]thiophen-5-ol**

To a stirred solution of 6,7-dihydrobenzo[c]thiophen-5(4H)-one (3.7 g, 24 mmol) in MeOH (100 mL) in a 250-mL round-bottom flask under nitrogen was added $NaBH_4$ (1.09 g, 29 mmol) in portions at 0 °C. The resulting mixture was stirred for 5 h at RT. The reaction was quenched with $H_2O$ (5.0 mL). The mixture was extracted with 3x50 mL of DCM and the organic layers were combined, dried over anhydrous $Na_2SO_4$, and concentrated under vacuum. The residue was eluted from silica gel with EtOAc/PE (1 :40). This resulted in 3.0 g (80%) of the title compound as yellow oil. MS-ESI: 155 (M+1).

**Step 2: 5-Methoxy-4,5,6,7-tetrahydrobenzo[c]thiophene**

To a stirred solution of 4,5,6,7-tetrahydrobenzo[c]thiophen-5-ol (3.0 g, 19.5 mmol) in THF (30 mL) in a 100-mL round-bottom flask was added NaH (60% wt. dispersion in mineral oil, 1.95 g, 49 mmol) at 0 °C. The reaction mixture was stirred for 10 min at RT, followed by the addition of $CH_3I$ (4.15 g, 29.2 mmol) dropwsie at RT. The reaction mixture was stirred for 5 h at RT. The reaction was then quenched with $H_2O$ (30 mL). The mixture was extracted with 3x50 mL

of DCM and the organic layers were combined, dried over anhydrous $Na_2SO_4$, and concentrated under vacuum. The residue was eluted from silica gel with EtOAc/PE (1:80). This resulted in 3.0 g (91%) of the title compound as yellow oil. MS-ESI: 169 (M+1).

**Step 3: 5-Methoxy-4,5,6,7-tetrahydrobenzo[c]thiophene-1-sulfonyl chloride and its regioiosmer**

To a stirred solution of 5-methoxy-4,5,6,7-tetrahydrobenzo[c]thiophene (3.0 g, 18 mmol) in $CHCl_3$ (30 mL) in a 100-mL 3-necked round-bottom flask was added $ClSO_3H$ (2.49 g, 21 mmol) dropwise at -10 °C. The reaction mixture was stirred for 48 h at RT. Then to the above mixture was added $PCl_5$ (7.36 g, 35.7 mmol) at -10 °C. The reaction mixture was stirred for 3 h at 50 °C and then poured into 200 mL of water/ice. The mixture was extracted with 3x200 mL of DCM. The organic layers were combined, dried over anhydrous $Na_2SO_4$, and then concentrated under vacuum. This resulted in 1.5 g (crude) of the title compound as light brown oil. This crude product was used in the next step.

**Step 4: 5-Methoxy-4,5,6,7-tetrahydrobenzo[c]thiophene-1-sulfonamide and 6-methoxy-4,5,6,7- tetrahydrobenzo[c]thiophene-1-sulfonamide and its regio-isomer**

To a stirred solution of 5-methoxy-4,5,6,7-tetrahydrobenzo[c]thiophene-1-sulfonyl chloride from Step 3 above (1.5 g, crude) in DCM (50 mL) in a 250-mL round-bottom flask was bubbled $NH_3$ (g) for 15 min at 0 °C. The reaction mixture was stirred for 3 h at RT and concentrated under vacuum. The residue was eluted from silica gel with a gradient of EtOAc/PE (1:4 to 1:2). This resulted in 650 mg (15% over two steps) of **674"F** and 620 mg (14%) of **674"S,** both as a yellow solid. MS-ESI: 248 (M+1). The regiochemistry of **674"F** and **674"S** were assigned based on NMR including COSY and NOESY.

Compound **674"F:** $^1$H NMR (300 MHz, DMSO-$d_6$) δ 7.46 (br s, 2H), 7.34 (s, 1H), 3.75-3.55 (m, 1H), 3.26 (s, 3H), 2.99-2.80 (m, 3H), 2.71 (dd, $J$ = 16.3, 5.9 Hz, 1H), 1.95-1.75 (m, 2H).

NOESY: Ar-H 7.34 (s, 1H) correlated with ring-methylene 2.71 (dd, $J$ = 16.3, 5.9 Hz, 1H), exchangeable proton $NH_2$ correlated with ring-methylene 2.99-2.80 (m, 3H).

Compound **674"S:** $^1$H NMR (300 MHz, DMSO-$d_6$) δ 7.50 (br s, 2H), 7.32 (s, 1H), 3.85-3.55 (m, 1H), 3.30 (s, 3H), 3.08 (dd, $J$ = 17.8, 4.7 Hz, 1H), 2.92 (dd, $J$ = 17.7, 5.9 Hz, 1H), 2.80-2.55 (m, 2H), 1.90-1.75 (m, 2H).

NOESY: Ar-H 7.32 (s, 1H) correlated with ring-methylene 2.80-2.55 (m, 2H), exchangeable proton $NH_2$ correlated with ring-methylene 3.08 (dd, $J$ = 17.8, 4.7 Hz, 1H) and 2.92 (dd, $J$ = 17.7, 5.9 Hz, 1H).

Steps 5-6 used similar procedures for converting compound **639"** to Intermediate **183** shown in Scheme 128 to afford Intermediate **189F** from compound **674"F.** MS-ESI: 361 (M+1).

**Table 56** The Intermediates in the following table were prepared using the similar procedures for converting compound **674"F** to Intermediate **189F** shown in Scheme 134 starting from **674"S.**

| Intermediate # | Structure | IUPAC Name | Exact Mass [M-H]- |
|---|---|---|---|
| **Intermediat e 189S** | 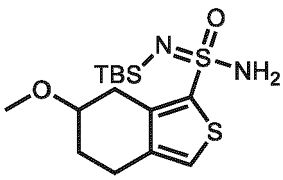 | N'-(tert-butyldimethylsilyl)-6-methoxy-4,5,6,7-tetrahydrobenzo[c] thiophene-1-sulfonimidamide | 361 |

**Scheme 135**

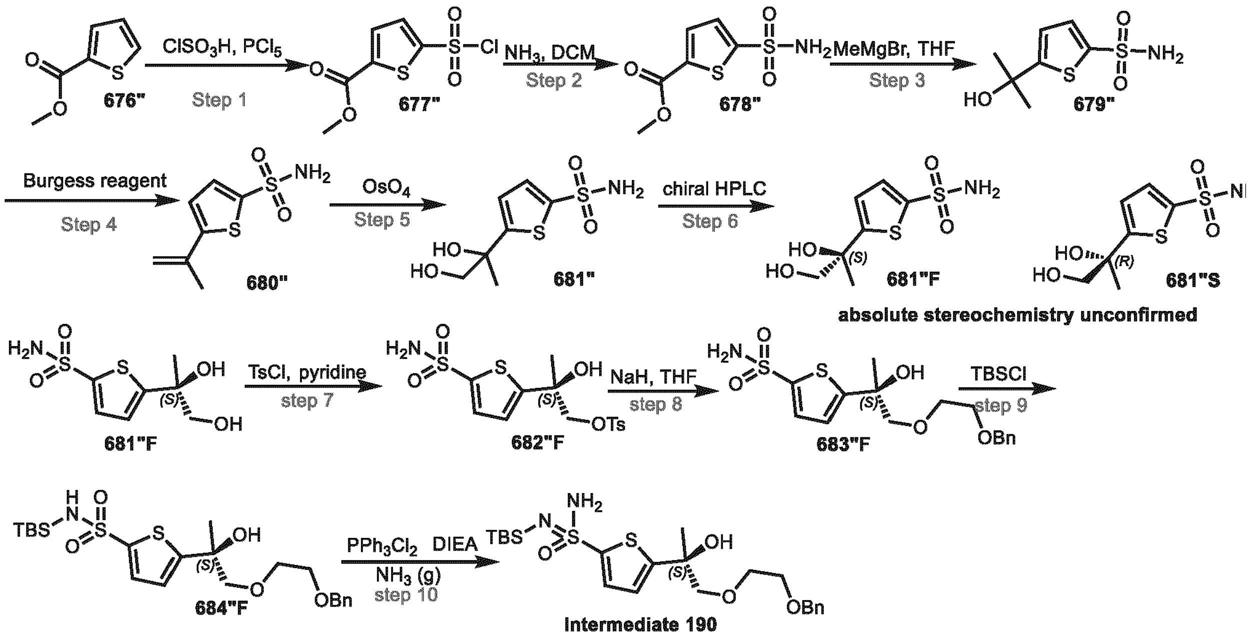


## Intermediate 190

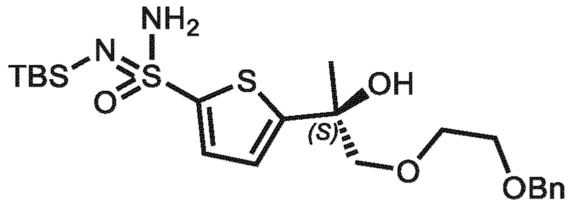

5-((S)-1-(2-(benzyloxy(ethoxy)-2-hydroxypropan-2-yl)-N'-(tert-butyldimethylsilyl)thiophene-2-sulfonimidamide

Steps 1-2 used similar procedures for converting compound **672"** to compound **674"** shown in Scheme 134 to afford compound **678"** from compound **676".** MS-ESI: 220 (M-1).

**Step 3: 5-(2-Hydroxypropan-2-yl)thiophene-2-sulfonamide**

To a stirred solution of methyl 5-sulfamoylthiophene-2-carboxylate (1.0 g, 4.52 mmol) in THF (40 mL) in a 100-mL 3-necked round-bottom flask under nitrogen was added $CH_3MgBr$ in THF (3.0 M, 7.5 mL, 22.6 mmol) dropwise at 0 °C. The reaction mixture was stirred overnight at RT, quenched with sat. $NH_4Cl$ (40 mL) at 0 °C, and then extracted with EtOAc (3x40 mL). The organic layers were combined, dried over anhydrous $Na_2SO_4$, and concentrated under vacuum. The residue was eluted from silica gel with EtOAc/PE (1:2). This resulted in 500 mg (50%) of the title compound as a yellow solid. MS-ESI: 222 (M+1).

**Step 4: 5-(Prop-1-en-2-yl)thiophene-2-sulfonamide**

To a stirred solution of 5-(2-hydroxypropan-2-yl)thiophene-2-sulfonamide (500 mg, 2.25 mmol) in THF (20 mL) in a 100-mL round-bottom flask under nitrogen was added Burgess reagent (1.15 g, 4.5 mmol) in portions at RT. The reaction mixture was stirred overnight at RT. The reaction mixture was quenched with $H_2O$ (10 mL) and extracted with EtOAc (3x20 mL). The organic layers were dried over anhydrous $Na_2SO_4$ and concentrated under vacuum. The residue was purified by prep-TLC (PE/EtOAc=1:2). This resulted in 420 mg (91.5%) of the title compound as a white solid. MS-ESI: 204 (M+1).

**Step 5: 5-(1,2-Dihydroxypropan-2-yl)thiophene-2-sulfonamide**

To a stirred solution of 5-(prop-1-en-2-yl)thiophene-2-sulfonamide (420 mg, 2.06 mmol) in t-BuOH (4.0 mL)

and acetone (4.0 mL) in a 50-mL round-bottom flask was added NMO (483 mg, 4.12 mmol) and the reaction solution was stirred for 15 min at RT. Then to this was added a solution of $OsO_4$ (53 mg, 0.21 mmol) in $H_2O$ (3.0 mL) dropwise at RT. The reaction mixture was stirred overnight at RT. The reaction was quenched with sat. aq. $Na_2S_2O_3$ (5.0 mL). The mixture was extracted with 3x10 mL EtOAc. The organic layers were combined and dried over anhydrous $Na_2SO_4$ and concentrated under vacuum. The crude product was eluted from silica gel with MeOH/DCM (7:100). This resulted in 245 mg (50%) of the title compound as yellow oil. MS-ESI: 238 (M+1).

**Step 6: (S) and (R)-5-(1,2-dihydroxypropan-2-yl)thiophene-2-sulfonamide**

5-(1,2-Dihydroxypropan-2-yl)thiophene-2-sulfonamide (**681"**, 245 mg) was resolved by Prep-Chiral HPLC using the following conditions: CHIRALPAK AD-H SFC, 5*25 cm, 5 um; Mobile Phase A: $CO_2$, Mobile Phase B: MeOH:ACN=1:1 (2 mM $NH_3$-MeOH); Flow rate: 150 mL/min; Gradient: 50% B; 220 nm; Rti: 4.1 min (**681"F**); $Rt_2$: 7.4 min (**681"S**). This resulted in 100 mg (99%ee) of **681"F** and 110 mg (98%ee) of **681"S,** both as yellow oil. Absolute stereochemistry of both were unconfirmed. MS-ESI: 236 (M-1).

**Step 7: (S)-2-hydroxy-2-(5-sulfamoylthiophen-2-yl)propyl 4-methylbenzenesulfonate**

To a stirred solution of (S)-5-(1,2-dihydroxypropan-2-yl)thiophene-2-sulfonamide (**681"F,** 100 mg, 0.42 mmol) in pyridine (8.0 mL) in a 50-mL round-bottom flask was added TsCl (239 mg, 1.26 mmol) at RT. The reaction mixture was stirred for 16 h at RT, diluted with 20 mL of $H_2O$, and then extracted with 3x20 mL of EtOAc. The organic layers were combined and concentrated under vacuum. The residue was eluted from silica gel with EtOAc/PE (1:10). This resulted in 125 mg (76%) of the title compound as yellow oil. MS-ESI: 392 (M+1).

**Step 8: (S)-5-(1-(2-(benzyloxy)ethoxy)-2-hydroxypropan-2-yl)thiophene-2-sulfonamide**

To a stirred solution of 2-(benzyloxy)ethan-1-ol (243 mg, 1.6 mmol) in THF(10 mL) in a 50-mL round-bottom flask under nitrogen was added NaH (60% wt. dispersion in mineral oil, 128 mg, 3.2 mmol) at 0 °C. The reaction mixture was stirred for 30 min at 0 °C, followed by the addition of (S)-2-hydroxy-2-(5-sulfamoylthiophen-2-yl)propyl 4-methylbenzenesulfonate (125 mg, 0.32 mmol) at 0 °C. The reaction mixture was stirred for 32 h at 35 °C. The reaction was quenched with $H_2O$ (10 mL) at 0 °C. The mixture was extracted with EtOAc (3 x 10 mL). The combined organic layers were washed with brine (2x10 mL) and dried over anhydrous $Na_2SO_4$ and concentrated under vacuum. The residue was eluted from silica gel with PE/EtOAc (1:2). This resulted in 113 mg (95%) of the title compound as yellow oil. MS-ESI: 372 (M+1).

Steps 9-10 used similar procedures for converting compound **639"** to Intermediate **183** shown in Scheme 128 to afford Intermediate **190** from compound **683"F.** MS-ESI: 485 (M+1).

**Table 57** The intermediates in the following table were prepared using the similar procedures for converting compound **681"F** to Intermediate **190** shown in Scheme 135 starting from **586"B.**

| Intermediate # | Structure | IUPAC Name | Exact Mass $[M-H]^-$ |
|---|---|---|---|
| **Intermediat e 191** | 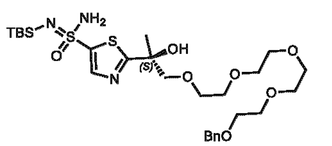 | N'-(tert-butyldimethylsilyl)-2-((S)-16-hydroxy-1-phenyl-2,5,8,11,14-pentaoxaheptadecan-16-yl)thiazole-5-sulfonimidamide | 618 |

# Scheme 136:

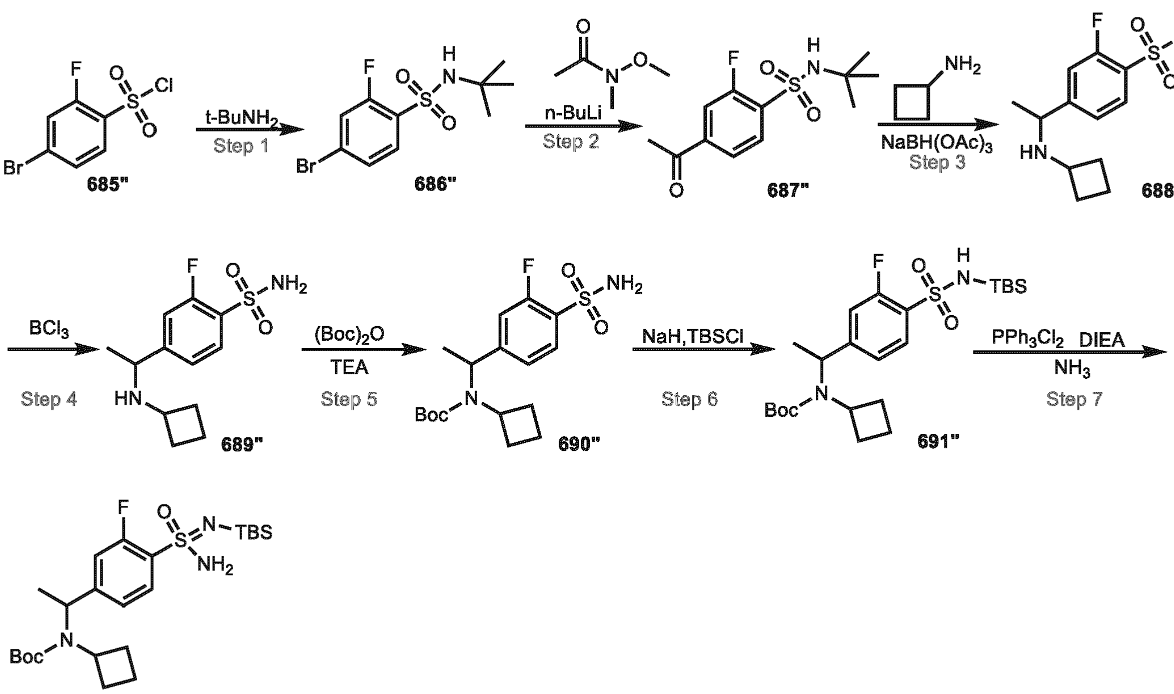


**Intermediate 192**

## Intermediate 192

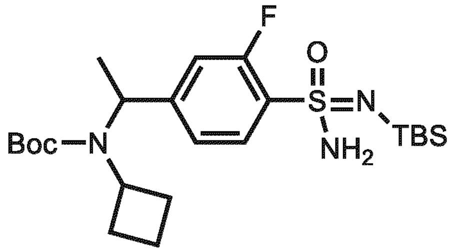

Tert-butyl (1-(4-(N'-(tert-bulyldimethylsilyl)sulfamidimidoyl)-3-fluorophenyl)ethyl)(cyclobutyl)carbamate

Steps 1-2 used similar procedures for converting compound **654"** to compound **656"** shown in Scheme 132 to afford compound **687"** from compound **685".** MS-ESI: 274 (M+1).

**Step 3: N-(tert-butyl)-4-(1-(cyclobutylamino)ethyl)-2-fluorobenzenesulfonamide**

To a stirred solution 4-acetyl-N-tert-butyl-2-fluorobenzenesulfonamide (3.6 g, 13 mmol) in DCM (100 mL) in a 250-mL round-bottom flask under nitrogen were added cyclobutylamine (1.87 g, 26 mmol) at RT, followed by the addition of HCOOH (1.33 g, 29 mmol) dropwsie at RT. The reaction solution was stirred overnight at RT. To the above solution was added $NaBH(OAc)_3$ (5.86 g, 28 mmol) in portions at RT. The reaction mixture was stirred for additional 2 days at 35 °C. The mixture was adjusted to pH 9 with NaOH (2 M). The mixture was extracted with EtOAc (3 x 100 mL). The combined organic layers were dried over anhydrous $Na_2SO_4$ and concentrated under vacuum. The residue was purified by prep-TLC (PE/EtOAc 2: 1). This resulted in 1.5 g (35%) of the title compound as a yellow solid. MS-ESI: 329 (M+1).

**Step 4: 4-(1-(Cyclobutylamino)ethyl)-2-fluorobenzenesulfonamide**

To a stirred solution of N-(tert-butyl)-4-(1-(cyclobutylamino)ethyl)-2-fluorobenzenesulfonamide (1.5 g, 4.57 mmol) in DCM (20 mL) in a 250-mL round-bottom flask was added $BCl_3$ in DCM (1 M, 20 mL, 20 mmol) dropwise at 0 °C. The reaction solution was stirred for 2 h at RT. The residue was adjusted to pH 9 with NaOH (2 M). The mixture was extracted with EtOAc (3x80 mL). The combined organic layers were dried over anhydrous $Na_2SO_4$ and concentrated

under vacuum. This resulted in 1.2 g (crude) of the title compound as yellow oil. MS-ESI: 273 (M+1).

Steps 5-7 used similar procedures for converting compound **662"** to Intermediate **187** shown in Scheme 132 to afford Intermediate **192** from compound **689".** MS-ESI: 486 (M+1).

**Scheme 137:**

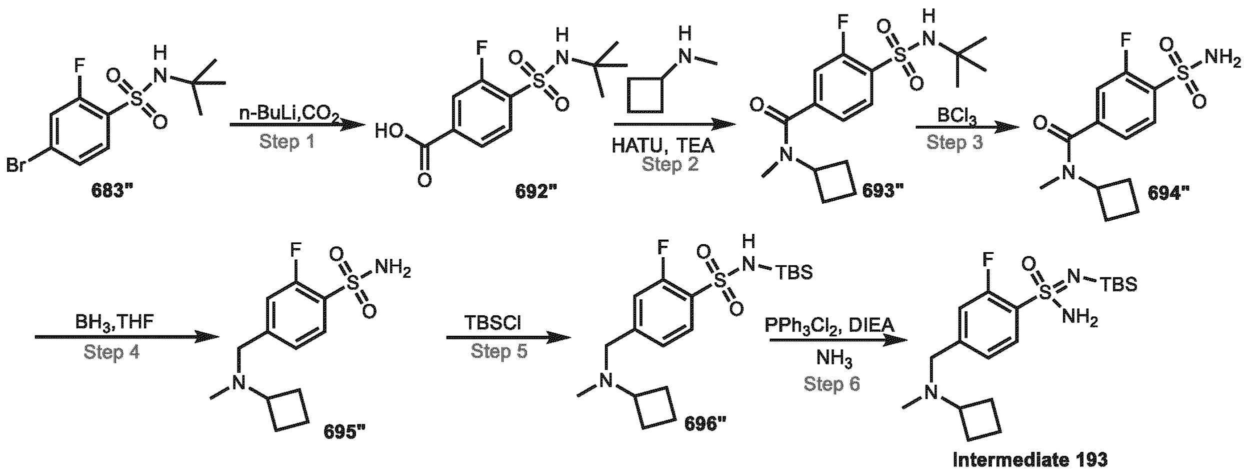


683" 692" 693" 694" 695" 696" Intermediate 193

**Intermediate 193**

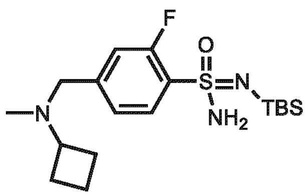

N'-(tert-butyldimethylsilyl)-4-((cyclobutyl(methyl)amino)methyl)-2-fluorobenzenesulfonimidamide

**Step 1: 4-(N-(tert-butyl)sulfamoyl)-3-fluorobenzoic acid**

To a stirred solution of 4-bromo-N-tert-butyl-2-fluorobenzenesulfonamide (7.7 g, 25 mmol) in THF (40 mL) in a 100-mL 3-necked round-bottom flask under nitrogen was added n-BuLi in hexane (2.5 M, 20 mL, 50 mmol) dropwise at -78 °C. The reaction solution was stirred for 30 min at -78 °C. $CO_2$ (g) was bubbled into the above solution for 20 min at -30 °C. The reaction mixture was stirred for additional 3 h at RT. The pH value of the solution was adjusted to 10 with NaOH (1 M). The mixture was extracted with 3x30 mL of DCM. The pH value of the solution was adjusted to 3 with HCl (1 M). The mixture was extracted with 3x100 mL of EtOAc. The organic layer was dried over anhydrous $Na_2SO_4$ and concentrated under vacuum. This resulted in 5.0 g (74%) of the title compound as a yellow solid. MS-ESI: 274 (M-1).

**Step 2: 4-(N-(tert-butyl)sulfamoyl)-N-cyclobutyl-3-fluoro-N-methylbenzamide**

To a stirred solution of 4-(N-(tert-butyl)sulfamoyl)-3-fluorobenzoic acid (4.9 g, 18 mmol) in DCM (50 mL) in a 250-mL round-bottom flask were added N-methylcyclobutanamine (1.83 g, 21.6 mmol), HATU (8.8 g, 23 mmol) and TEA (5.4 g, 53 mmol). The reaction mixture was stirred overnight at RT. The reaction was quenched with $H_2O$ (50 mL). The mixture was extracted with DCM (3x80 mL). The organic layers were dried over anhydrous $Na_2SO_4$ and concentrated under vacuum. The residue was eluted from silica gel with EtOAc/PE (2/3). This resulted in 2.58 g (42%) of the title compound as a yellow solid. MS-ESI: 343 (M+1).

Step 3 used similar procedures for converting compound **688"** to compound **689"** shown in Scheme 136 to afford compound **694"** from compound **693".** MS-ESI: 287 (M+1).

**Step 4: 4-((Cyclobutyl(methyl)amino)methyl)-2-fluorobenzenesulfonamide**

To a stirred solution of N-cyclobutyl-3-fluoro-N-methyl-4-sulfamoylbenzamide (2.2 g, 7.7 mmol) in THF (30 mL) in a 100-mL round-bottom flask under nitrogen was added $BH_3$-THF (1 M, 15 mL, 15 mmol) at 0 °C. The reaction mixture was stirred for 30 min at 0 °C. The reaction mixture was stirred overnight at RT. The reaction was quenched with aq. HCl (2 M, 50 mL) at 0 °C. The reaction mixture was stirred for 10 min at RT. The pH value of the solution was adjusted to 9 with NaOH (5 M). The mixture was extracted with 3x50 mL of EtOAc and the organic layers were combined. The organic layer was dried over anhydrous $Na_2SO_4$ and concentrated under vacuum. This resulted in 1.74 g (83%) of the title compound as a yellow solid. MS-ESI: 273 (M+1).

Steps 5-6 used similar procedures for converting compound **639"** to Intermediate **183** shown in Scheme 128 to afford Intermediate **193** from compound **695"**. MS-ESI: 386 (M+1).

**Scheme 138:**

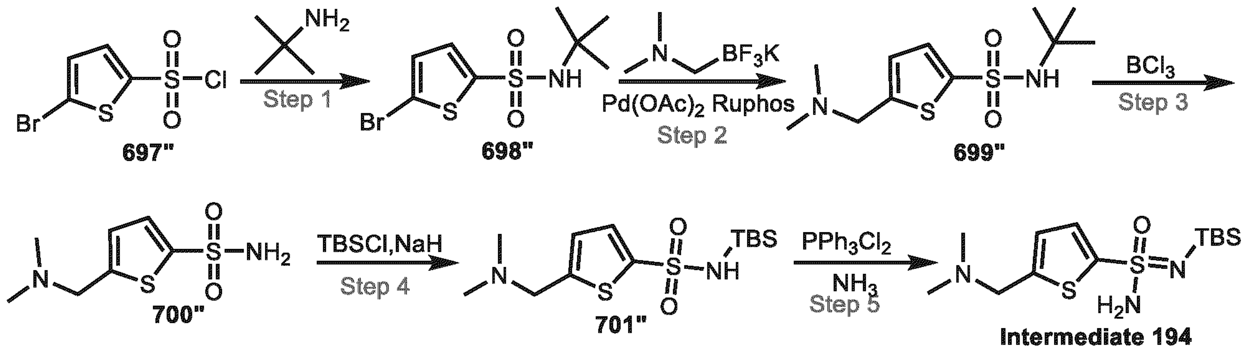


**Intermediate 194**

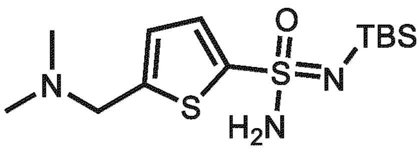

N-(tert-butyldimethylsilyl)-5-((dimethylamino)methyl)thiophene-2-sulfonimidamide

Step 1 used similar procedures for converting compound **654"** to compound **655"** shown in Scheme 132 to afford compound **698"** from compound **697"**. MS-ESI: 297/299 (M+1).

**Step 2: N-(tert-butyl)-5-((dimethylamino)methyl)thiophene-2-sulfonamide**

To a stirred solution of 5-bromo-N-(tert-butyl)thiophene-2-sulfonamide (1.15 g, 3.86 mmol) in dioxane (30 mL) and $H_2O$ (3.0 mL) in a 100-mL round-bottom flask under nitrogen were added N,N-dimethyl-1-(trifluoro-$\lambda_4$-boraneyl)methanamine, potassium salt (764 mg, 4.63 mmol) and $Cs_2CO_3$ (3.77 g, 11.7 mmol) in portions at RT. Then Ruphos (360 mg, 0.77 mmol) and $Pd(AcO)_2$ (173 mg, 0.77 mmol) were added to the mixture. The reaction mixture was stirred overnight at 100 °C. The solids were filtered out and the filtrate was concentrated under vacuum. The residue was eluted from silica gel with EtOAc/PE (2/1). This resulted in 120 mg (11%) of the title compound as yellow oil. MS-ESI: 277 (M+1).

Step 3 used similar procedures for converting compound **688"** to compound **689"** shown in Scheme 136 to afford compound **700"** from compound **699"**. MS-ESI: 221 (M+1).

Steps 4-5 used similar procedures for converting compound **639"** to Intermediate **183** shown in Scheme 128 to afford Intermediate **194** from compound **700"**. MS-ESI: 334 (M+1).

## Scheme 139

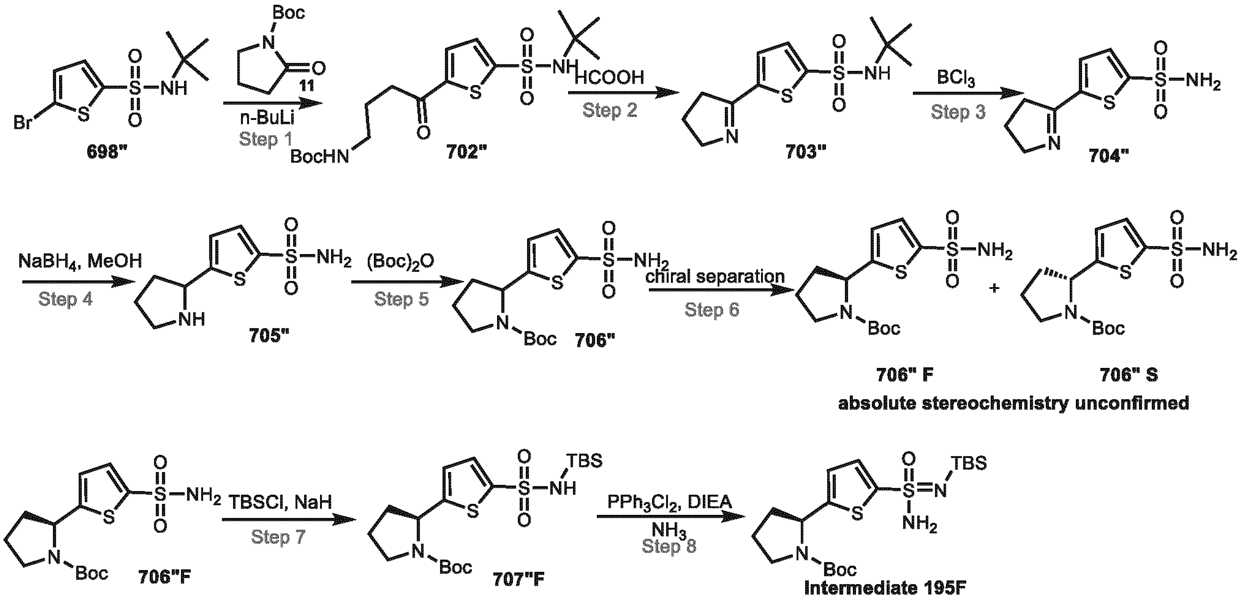


## Intermediate 195F

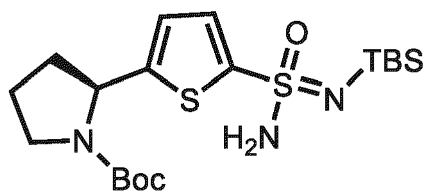

Tert-butyl (2S)-2-(5-(N-(tert-butyldimethylsilyl)sulfamidimidoyl)thiophen-2-yl)pyrrolidine-1-carboxylate

**Step 1: Tert-butyl (4-(5-(N-(tert-butyl)sulfamoyl)thiophen-2-yl)-4-oxobutyl)carbamate**

To a stirred solution of 5-bromo-N-(tert-butyl)thiophene-2-sulfonamide (5.0 g, 16.8 mmol) in THF (50 mL) in a 250-mL 3-necked round-bottom flask under nitrogen was added n-BuLi in hexane (2.5 M, 13 mL, 32.5 mmol) dropwise at -78 °C. The reaction mixture was stirred for 1 h at -78 °C. Then to the mixture was added tert-butyl 2-oxopyrrolidine-1-carboxylate (6.21 g, 33.6 mmol) at -78 °C. The reaction mixture was stirred for 2 h at RT and then quenched with $H_2O$ (50 mL). The mixture was extracted with EtOAc (3x100 mL). The organic layer was dried over anhydrous $Na_2SO_4$ and concentrated under vacuum. The residue was eluted from silica gel with EtOAc/PE (1:1). This resulted in 1.8 g (27%) of the title compound as a yellow solid. MS-ESI: 405 (M+1).

**Step 2: N-(tert-butyl)-5-(3,4-dihydro-2H-pyrrol-5-yl)thiophene-2-sulfonamide**

A solution of tert-butyl (4-(5-(N-(tert-butyl)sulfamoyl)thiophen-2-yl)-4-oxobutyl)carbamate (1.8 g, 4.45 mmol) in formic acid (20 mL) in a 100-mL round-bottom flask under nitrogen was stirred for 12 h at RT. The pH value of the solution was adjusted to 9 with sat.$Na_2CO_3$ (aq). The mixture was extracted with EtOAc (3x100 mL) and the organic layers were combined. The organic layer was dried over anhydrous $Na_2SO_4$ and concentrated under vacuum. The residue was eluted from silica gel with DCM/MeOH (10:1). This resulted in 1.0 g (78%) of the title compound as a yellow solid. MS-ESI: 287 (M+1).

**Step 3: 5-(3,4-Dihydro-2H-pyrrol-5-yl)thiophene-2-sulfonamide**

To a stirred solution of N-(tert-butyl)-5-(3,4-dihydro-2H-pyrrol-5-yl)thiophene-2-sulfonamide (1.0 g, 3.49 mmol) in DCM (10 mL) in a 100-mL round-bottom flask was added $BCl_3$ in DCM (1 M, 10 mL, 10 mmol) dropwise at 0 °C. The

reaction mixture was stirred for 2 h at RT. The reaction was quenched with 10 mL of MeOH. The mixture was concentrated under vacuum. The residue was eluted from silica gel with DCM/MeOH (10:1). This resulted in 600 mg (75%) of the title compound as a yellow solid. MS-ESI: 231 (M+1).

**Step 4: 5-(Pyrrolidin-2-yl)thiophene-2-sulfonamide**

To a stirred solution of 5-(3,4-dihydro-2H-pyrrol-5-yl)thiophene-2-sulfonamide (600 mg, 2.6 mmol) in MeOH (10 mL) in a 50-mL round-bottom flask under nitrogen was added $NaBH_4$ (197 mg, 5. 21 mmol) in portions at 0 °C. The reaction mixture was stirred for 2 h at RT. The reaction mixture was diluted with 10 mL of water. The mixture was extracted with 3x50 mL of EtOAc and the organic layers were combined. The organic layer was dried over anhydrous $Na_2SO_4$ and concentrated under vacuum. This resulted in 500 mg (83%) of the title compound as yellow oil. MS-ESI: 233 (M+1).

Step 5 used similar procedures for converting compound **662"** to compound **663"** shown in Scheme 132 to afford compound **706"** from compound **705".** MS-ESI: 331 (M-1).

**Step 6: Tert-butyl (S)-2-(5-sulfamoylthiophen-2-yl)pyrrolidine-1-carboxylate and tert-butyl (R)-2- (5-sulfamoylthiophen-2-yl)pyrrolidine-1-carboxylate**

Tert-butyl 2-(5-sulfamoylthiophen-2-yl)pyrrolidine-1-carboxylate (**706"**, 600 mg) was resolved by Prep-SFC using the following conditions: Column, CHIRALPAK AD-H SFC, 5*25 cm, 5 um; Mobile Phase A: $CO_2$, Mobile Phase B: MeOH (2 mM $NH_3$-MeOH); Flow rate: 200 mL/min; Gradient: 24% B; UV 254 nm; Rti: 3.5 min (**706"F**); $Rt_2$: 5.6 min (**706"S**). This resulted in 250 mg (98%ee) of **706"F** and 240 mg (99%ee) of **706"S,** both as a yellow solid. Absolute stereochemistry of both enantiomers were assigned arbitrarily. MS-ESI: 333 (M+1).

Steps 7-8 used similar procedures for converting compound **639"** to Intermediate **183** shown in Scheme 128 to afford Intermediate **195F** from compound **706"F.** MS-ESI: 446 (M+1).

**Table 58** The intermediates in the following table were prepared using the similar procedures for converting compound **706"F** to Intermediate **195F** shown in Scheme 139 starting from **706"S.**

| Intermediate # | Structure | IUPAC Name | Exact Mass [M-H]$^-$ |
|---|---|---|---|
| **Intermediate 1955** | 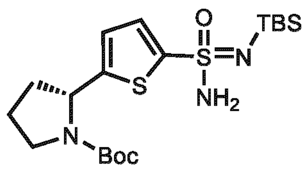 | Tert-butyl (2R)-2-(5-(N-(tertbutyldimethylsilyl) sulfamidimidoyl)thiophen-2-yl)pyrrolidine-1-carboxylate | 446 |

**Scheme 140:**

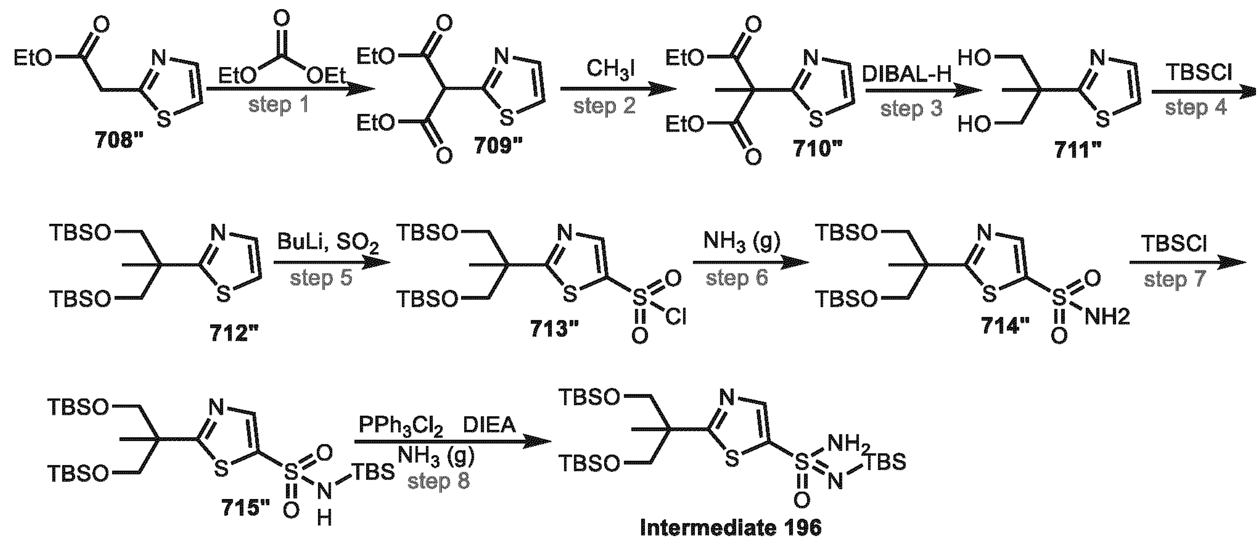


## Intermediate 196

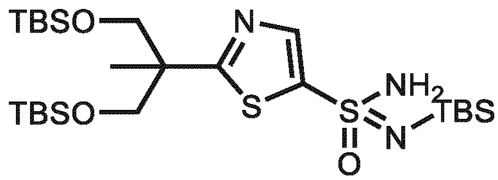

N'-(tert-bulyldimethylsilyl)-2-(2,2,3,3,6,9,9, 10, 1 0-nonamethyl-4,8-dioxa-3,9-disilaundecan-6-yl)thiazole-5-sulfonimidamide

**Step 1: Diethyl 2-(thiazol-2-yl)malonate**

To a stirred solution of ethyl 2-(1,3-thiazol-2-yl)acetate (5.0 g, 29 mmol) in THF (20 mL) in a 100-mL 3-necked round-bottom flask under nitrogen was added NaH (60% wt. dispersion in mineral oil, 2.34 g, 58 mmol) in portions at 0 °C. The reaction mixture was stirred for 30 min at 0 °C. Then diethyl carbonate (50 mL) was added to the above mixture. The reaction mixture was stirred for 10 h at 65 °C. The reaction was quenched with 50 mL of water and extracted with 3x50 mL of EtOAc. The organic layers were combined, dried over anhydrous $Na_2SO_4$, and concentrated under vacuum. This resulted in 7.59 g (crude) of the title compound as a light yellow solid. MS-ESI: 244 (M+1).

**Step 2: Diethyl 2-methyl-2-(thiazol-2-yl)malonate**

To a stirred solution of diethyl 2-(thiazol-2-yl)malonate (2.0 g, 8.22 mmol) in THF (30 mL) in a 250-mL 3-necked round-bottom flask under nitrogen were added DBU (2.5 g, 16 mmol) at RT, followed by the addition of $CH_3I$ (4.67 g, 33 mmol) dropwise at 0 °C. The reaction mixture was stirred for 2 h at RT and then quenched with 20 mL of water/ice. The mixture was extracted with 3x50 mL of EtOAc and the organic layers were combined, dried over anhydrous $Na_2SO_4$, and concentrated under vacuum. The crude product was purified by TLC with EtOAc/PE (1/2). This resulted in 1.1 g (52%) of the title compound as light yellow oil. MS-ESI: 258 (M+1).

**Step 3: 2-Methyl-2-(thiazol-2-yl)propane-1,3-diol**

To a stirred solution of diethyl 2-methyl-2-(thiazol-2-yl)malonate (1.1 g, 4.23 mmol) in THF (25 mL) in a 250-mL 3-necked round-bottom flask under nitrogen was added DIBAL-H in hexane (1 M, 25 mL, 25 mmol) in portions at 0 °C in an ice/water bath over 10 min. The reaction mixture was stirred for 2 h at RT. The reaction was then quenched with 20 mL of MeOH. The mixture was concentrated under vacuum. The crude product was purified by RP-HPLC with ACN/$H_2O$ (9/1). This resulted in 384 mg (60%) of the title compound as light yellow oil. MS-ESI: 174 (M+1).

**Step 4: 2-(2,2,3,3,6,9,9,10,10-Nonamethyl-4,8-dioxa-3,9-disilaundecan-6-yl)thiazole**

To a stirred solution of 2-methyl-2-(thiazol-2-yl)propane-1,3-diol (384 mg, 2.22 mmol) in THF (10 mL) in a 50-mL round-bottom flask under nitrogen was added NaH (60%wt. dispersion in mineral oil, 532 mg, 13.3 mmol) in portions at 0 °C in an ice/water bath. The reaction mixture was stirred for 10 min at 0 °C. To the above mixture was added TBSCl (3.34 g, 22 mmol) in portions at 0 °C. The reaction mixture was stirred for 4 h at RT. The reaction was quenched with 20 mL of water/ice. The mixture was extracted with 3x50 mL of EtOAc and the organic layers were combined, dried over anhydrous $Na_2SO_4$, and concentrated under vacuum. This resulted in 846 mg (crude) of the title compound as light yellow oil. MS-ESI: 402 (M+1).

Steps 5-8 used similar procedures for converting compound **642"** to Intermediate **184** shown in Scheme 129 to afford Intermediate **196** from compound **712".** MS-ESI: 594 (M+1).

## Scheme 141:

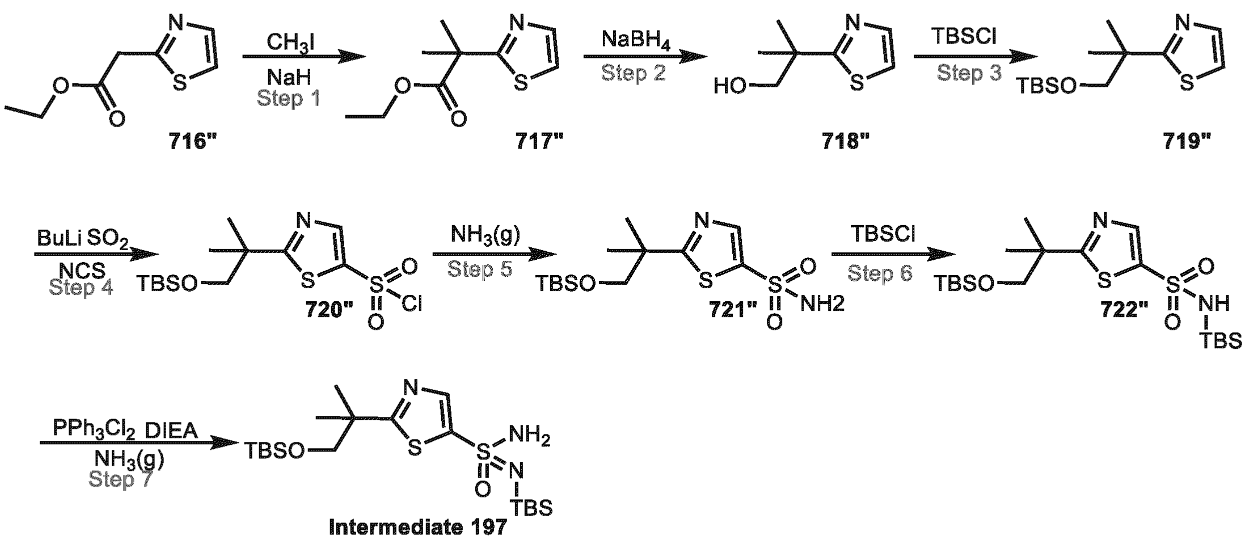


## Intermediate 197

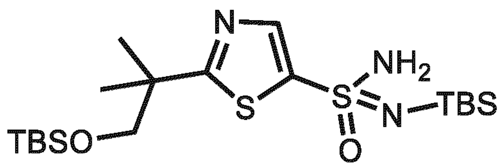

N'-(tert-butyldimethylsilyl)-2-(1-((tert-butyldimethylsilyl)oxy)-2-methylpropan-2-yl)thiazole-5-sulfonimidamide

**Step 1: Ethyl 2-methyl-2-(thiazol-2-yl)propanoate**

To a stirred solution of ethyl 2-(thiazol-2-yl)acetate (2.5 g, 14.6 mmol) in THF (30 mL) in a 100-mL round-bottom flask under nitrogen was added NaH (60% wt. dispersion in mineral, 3.5 g, 87.6 mmol) in portions at 0 °C. The reaction mixture was stirred for 10 min at RT. Then to the above was added $CH_3I$ (21 g, 146 mmol) dropwise at 0 °C. The reaction mixture was stirred overnight at RT. The reaction was quenched with ice/water at 0 °C. The mixture was extracted with EtOAc (3 x 100 mL). The combined organic layers were washed with brine (2x30 mL) and dried over anhydrous $Na_2SO_4$. After filtration, the filtrate was concentrated under vacuum. The residue was eluted from silica gel with EtOAc/PE (1:10). This resulted in 1.1 g (38%) of the title compound as a yellow solid. MS-ESI: 200 (M+1).

**Step 2: 2-Methyl-2-(thiazol-2-yl)propan-1-ol**

To a stirred solution of ethyl 2-methyl-2-(thiazol-2-yl)propanoate (1.3 g, 6.5 mmol) in THF (15 mL) in a 50-mL round-bottom flask was added $NaBH_4$ (1.48 g, 39 mmol) in portions at 0 °C. The reaction mixture was stirred for 2 h at

0 °C. The reaction was quenched with ice/water at 0 °C. The mixture was extracted with 3x50 mL of EtOAc. The organic layers were combined, dried over anhydrous $Na_2SO_4$, and concentrated under vacuum. The residue was eluted from silica gel with EtOAc/PE (1:3). This resulted in 600 mg (58%) of the title compound as a yellow solid. MS-ESI: 158 (M+1).

Steps 3-7 used similar procedures for converting compound **711"** to Intermediate **196** shown in Scheme 140 to afford Intermediate **197** from compound **718".** MS-ESI: 464 (M+1).

**Scheme 142:**

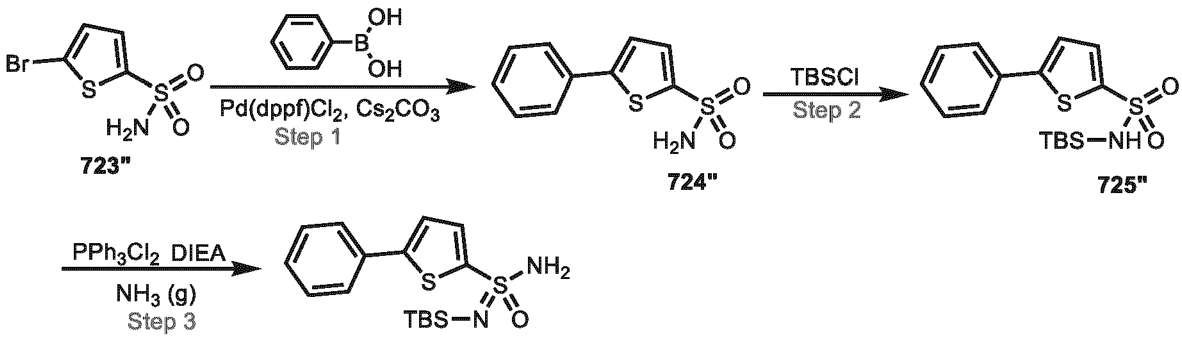


723"

724"

725"

Intermediate 198

## Intermediate 198

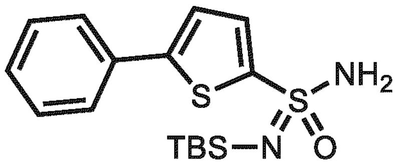

N'-(tert-butyldimethylsilyl)-5-phenylthiophene-2-sulfonimidamide

**Step 1: 5-Phenylthiophene-2-sulfonamide**

To a stirred solution of 5-bromothiophene-2-sulfonamide (500 mg, 2.07 mmol) in dioxane (20 mL) and $H_2O$ (2.0 mL) in a 50 mL 3-necked round-bottom flask under nitrogen were added phenylboronic acid (504 mg, 4.13 mmol), $Pd(dppf)Cl_2$ (151 mg, 0.21 mmol) and $Cs_2CO_3$ (1.35 g, 4.13 mmol) at RT. The reaction mixture was stirred overnight at 100 °C. The reaction was diluted with $H_2O$ (20 mL) and the mixture was extracted with 3x20 mL of EtOAc. The combined organic layer was dried over anhydrous $Na_2SO_4$ and concentrated under vacuum. The residue was eluted from silica gel with PE/EtOAc (5:1). This resulted in 450 mg (91 %) as a white solid. MS-ESI: 238 (M-1).

Steps 2-3 used similar procedures for converting compound **639"** to Intermediate **183** shown in Scheme 128 to afford Intermediate **198** from compound **724.** MS-ESI: 353 (M+1).

## Scheme 143:

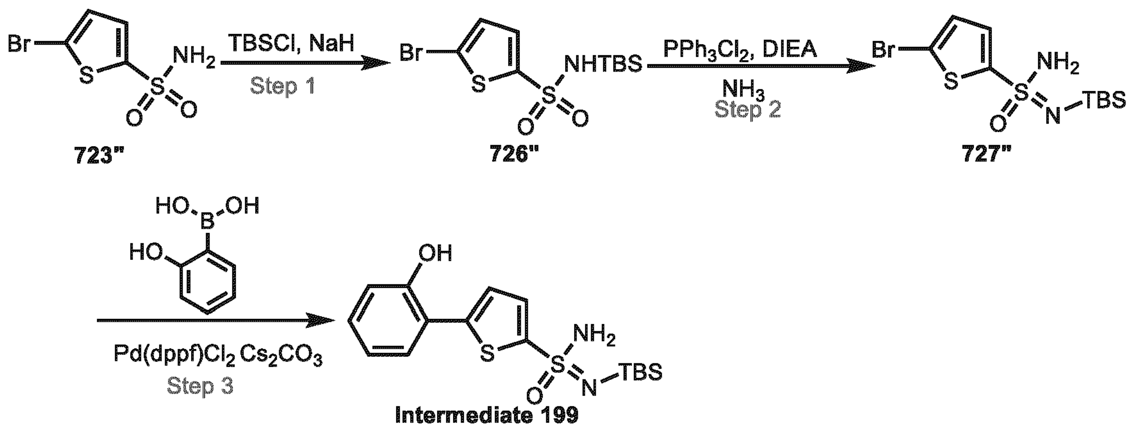


## Intermediate 199

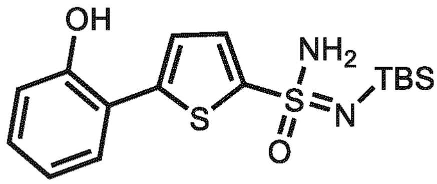

N-(tert-butyldimethylsilyl)-5-(2-hydroxyphenyl)thiophene-2-sulfonimidamide

Steps 1-2 used similar procedures for converting compound 639" to Intermediate **183** shown in Scheme 128 to afford compound **727"** from compound **723".** MS-ESI: 355/357 (M+1).

Step 3 used similar procedures for converting compound **723"** to compound **724"** shown in Scheme 142 to afford Intermediate **199** from compound **727".** MS-ESI: 369 (M+1).

**Table 59** The intermediates in the following table were prepared using the similar procedures for converting compound **723"** to Intermediate **199** shown in Scheme 143.

| Intermediate # | Structure | IUPAC Name | Exact Mass [M-H]- |
| --- | --- | --- | --- |
| **Intermediate 200** | 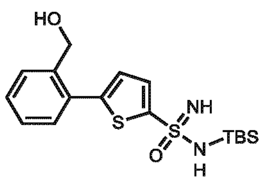 | N-(tert-butyldimethylsilyl)-5-(2-(hydroxymethyl)phenyl)thiophene-2-sulfonimidamide | 383 |

## Scheme 144:

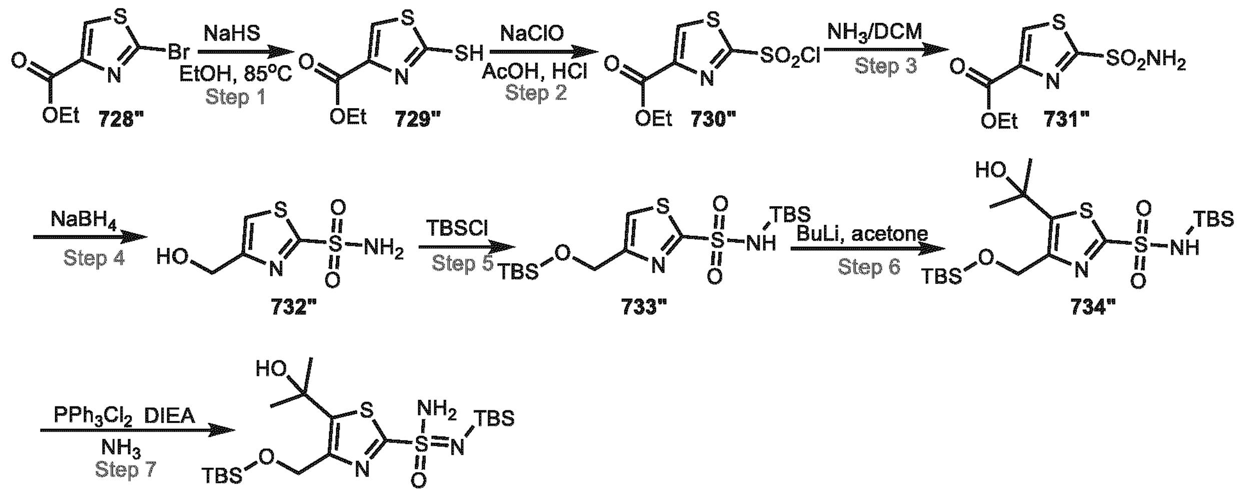


Intermediate 201

## Intermediate 201

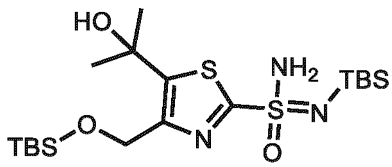

N'-(tert-butyldimethylsilyl)-4-(((tert-butyldimethylsilyl)oxy)methyl)-5-(2-hydroxypropan-2-yl)thiazole-2-sulfonimidamide

**Step 1: Ethyl 2-mercaptothiazole-4-carboxylate**

To a stirred solution of ethyl 2-bromothiazole-4-carboxylate (100 g, 424 mmol) in EtOH (1.0 L) in a 2000-mL round-bottom flask was added NaSH (119 g, 2.12 mol) in portions at RT. The reaction mixture was stirred for 3 h at 85 °C and then concentrated under vacuum. The residue was dissolved in 500 mL of $H_2O$. The pH value of the solution was adjusted to 3 with HCl (1 M). The solids were collected by filtration. This resulted in 80 g (99.8%) of the title compound as a yellow solid. MS-ESI: 190 (M+1).

**Step 2: Ethyl 2-(chlorosulfonyl)thiazole-4-carboxylate**

To a stirred solution of ethyl 2-mercaptothiazole-4-carboxylate (80 g, 421 mmol) in HCl (12 M, 800 mL) and AcOH (800 mL) in a 5000-mL round-bottom flask was added NaClO in $H_2O$ (13%wt., 1.2 L, 1.78 mol) dropwise at 0 °C. The reaction mixture was stirred for 2 h at 0 °C. The residue was dissolved in 1.6 L of $H_2O$. The mixture was extracted with 2x2.0 L of DCM. The organic layers were dried over anhydrous $Na_2SO_4$ and concentrated under vacuum. This resulted in 36 g (crude) of the title compound as yellow oil.

**Step 3: Ethyl 2-sulfamoylthiazole-4-carboxylate**

To a stirred solution of ethyl 2-(chlorosulfonyl)thiazole-4-carboxylate (36 g, crude) in DCM (500 mL) in a 1.0-L round-bottom flask was introduced $NH_3$(g) bubbled for 20 min at 0 °C. The reaction mixture was stirred for 1 h at 0 °C. The mixture was concentrated under vacuum. The residue was eluted from silica gel with DCM/MeOH (10:1). This resulted in 18 g (18%, over two steps) of the title compound as a white solid. MS-ESI: 235 (M-1).

**Step 4: 4-(Hydroxymethyl)thiazole-2-sulfonamide**

To a stirred solution of ethyl 2-sulfamoylthiazole-4-carboxylate (12 g, 51 mmol) in EtOH (500 mL) in a 1-L round-bottom flask under nitrogen was added $NaBH_4$ (15.5 g, 408 mmol) in portions at 0 °C. The reaction mixture was stirred

for 4 h at 0 °C. The reaction was quenched with 100 mL of water/ice. The reaction mixture was concentrated under vacuum. The pH value of the mixture was adjusted to 5 with HCl (6 M). The mixture was concentrated under vacuum. The solids were dissolved with MeOH (200 mL). The residue was eluted from silica gel with EtOAc (100%). This resulted in 8.0 g (81%) of the title compound as a white solid. MS-ESI: 193 (M-1).

**Step 5: N-(tert-butyldimethylsilyl)-4-(((tert-butyldimethylsilyl)oxy)methyl)thiazole-2-sulfonamide**

To a stirred solution of 4-(hydroxymethyl)thiazole-2-sulfonamide (4.6 g, 24 mmol) in THF (100 mL) in a 250-mL round-bottom flask under nitrogen was added NaH (60% wt. dispersion in mineral oil, 7.68 g, 192 mmol) in several batches at 0 °C. The mixture was stirred for 10 min at RT, followed by the addition of TBSCl (10.8 g, 72 mmol) in portions at 0 °C. The reaction mixture was stirred for 1 h at RT. The reaction was quenched with ice/water (100 mL). The mixture was extracted with EtOAc (3x100 mL). The organic layers were dried over anhydrous $Na_2SO_4$ and concentrated under vacuum. The residue was eluted from silica gel with EtOAc/PE (1:3). This resulted in 4.5 g (45%) of the title compound as yellow oil. MS-ESI: 421 (M-1).

**Step 6: N-(tert-butyldimethylsilyl)-4-(((tert-butyldimethylsilyl)oxy)methyl)-5-(2-hydroxypropan-2-yl) thiazole-2-sulfonamide**

To a stirred solution of N-(tert-butyldimethylsilyl)-4-(((tertbutyldimethylsilyl)oxy)methyl)thiazole-2- sulfonamide (3.0 g, 7.1 mmol) in THF (20 mL) in a 100-mL 3-necked round-bottom flask under nitrogen was added n-BuLi in hexane (2.5 M, 5.7 mL, 14.2 mmol) dropwise at -78 °C. The reaction solution was stirred for 1 h at -78 °C. Then to this solution was added acetone (4.2 g, 71 mmol) at -78 °C. The reaction mixture was stirred for 1 h at -78 °C. The reaction was quenched with 20 mL of water/ice. The mixture was extracted with 3x100 mL of EtOAc. The organic layers was dried over anhydrous $Na_2SO_4$ and concentrated under vacuum. The residue was eluted from silica gel with EtOAc/PE (10:1). This resulted in 1.48 g (43%) of the title compound as yellow oil. MS-ESI: 481 (M+1).

Step 7 used similar procedures for converting compound **640"** to Intermediate **183** shown in Scheme 128 to afford Intermediate **201** from compound **734"**. MS-ESI: 480 (M+1).

**Scheme 145:**

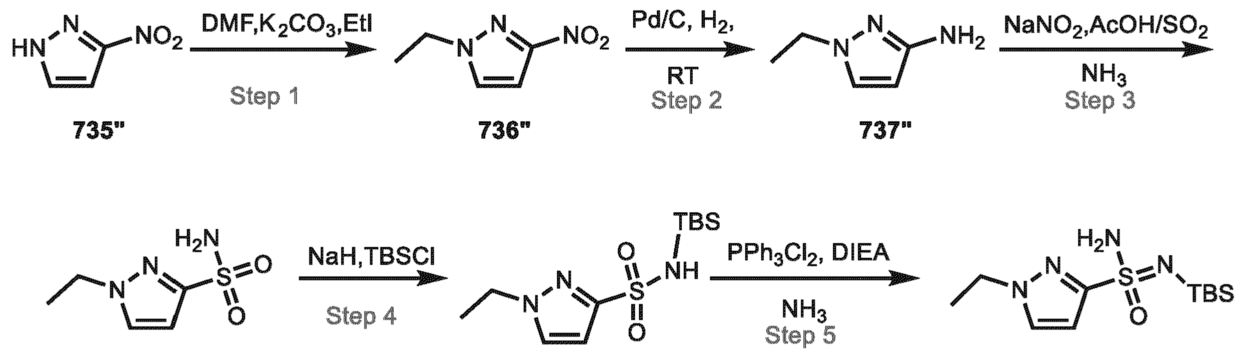


**735"** **736"** **737"** **738"** **739"** **Intermediate 202**

**Intermediate 202**

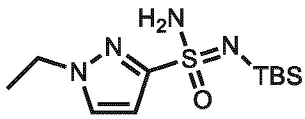

N'-(tert-butyldimethylsilyl)-1-ethyl-1H-pyrazole-3-sulfonimidamide

**Step 1: 1-Ethyl-3-nitro-1H-pyrazole**

To a stirred solution of 3-nitro-1H-pyrazole (20 g, 177 mmol) in DMF (100 mL) in a 250-mL round-bottom flask were added $K_2CO_3$ (49 g, 354 mmol) in portions at RT, followed by the addition of iodoethane (55 g, 354 mmol) dropwise at RT. The reaction mixture was stirred for 16 h at 80 °C in an oil bath. The reaction mixture was diluted with 200 mL of

$H_2O$. The mixture was extracted with 3x200 mL of EtOAc and the organic layers were combined and dried over anhydrous $Na_2SO_4$. The mixture was concentrated under vacuum. The residue was eluted from silica gel with EtOAc/PE (1:4). This resulted in 22.6 g (91%) of the title compound as yellow oil. MS-ESI: 142 (M+1). $^1H$ NMR (400 MHz, DMSO-$d_6$) δ 8.06 (d, *J* = 2.5 Hz, 1H), 7.04 (d, *J* = 2.5 Hz, 1H), 4.27 (q, *J* = 7.3 Hz, 2H), 1.42 (t, *J* = 7.3 Hz, 3H).

**Step 2: 1-Ethyl-1H-pyrazol-3-amine**

To a stirred solution of 1-ethyl-3-nitro-1H-pyrazole (22 g, 156 mmol) in MeOH (100 mL) in a 250-mL round-bottom flask under nitrogen was added Pd/C (10% wt., 2.2 g) in portions at 0 °C. The flask was evacuated and refilled three times with hydrogen. The reaction mixture was stirred overnight at RT under atmosphere of hydrogen with a balloon. The solids were filtered out. The filtrate was concentrated under vacuum. This resulted in 13.8 g (80%) of the title compound as yellow oil. MS-ESI: 112 (M+1).

**Step 3: 1-Ethyl-1H-pyrazole-3-sulfonamide**

To a stirred solution of 1-ethyl-1H-pyrazol-3-amine (3.4 g, 31 mmol,) in HCl (6 M, 20 mL) in a 100-mL round-bottom flask was added $NaNO_2$ (2.53 g, 37 mmol) in $H_2O$ (5.0 mL) dropwise slowly at 0 °C over 25 min. The reaction solution was stirred for 40 min at 0 °C, this solution was assigned as solution A. Then $CuCl_2$ (8.23 g, 61 mmol) was added to a 250-mL single necked round-bottom flask with AcOH (100 mL) as the solvent, $SO_2$ (g) was bubbled to the mixture with stirring at 0 °C for 20 min, this mixture was assigned as solution B. To the mixture B was added solution A dropwise with stirring at 0 °C. The reaction mixture was stirred for additional 2 h at 0 °C. The reaction mixture was diluted with 100 mL of $H_2O$. The mixture was extracted with 3x150 mL of DCM. The organic layers were dried over anhydrous $Na_2SO_4$ and concentrated under vacuum. The residue was dissolved in 30 mL of DCM and $NH_3$ (g) was bubbled for 15 min at 0 °C. The reaction was stirred for 2 h at RT. The reaction mixture was concentrated under vacuum. The residue was eluted from silica gel with PE/EtOAc (3:1). This resulted in 1.75 g (33%) of the title compound as a yellow solid. MS-ESI: 174 (M-1).

Steps 4-5 used similar procedures for converting compound **639"** to Intermediate **183** shown in Scheme 128 to afford Intermediate 202 from compound **738".** MS-ESI: 289 (M+1).

**Scheme 146:**

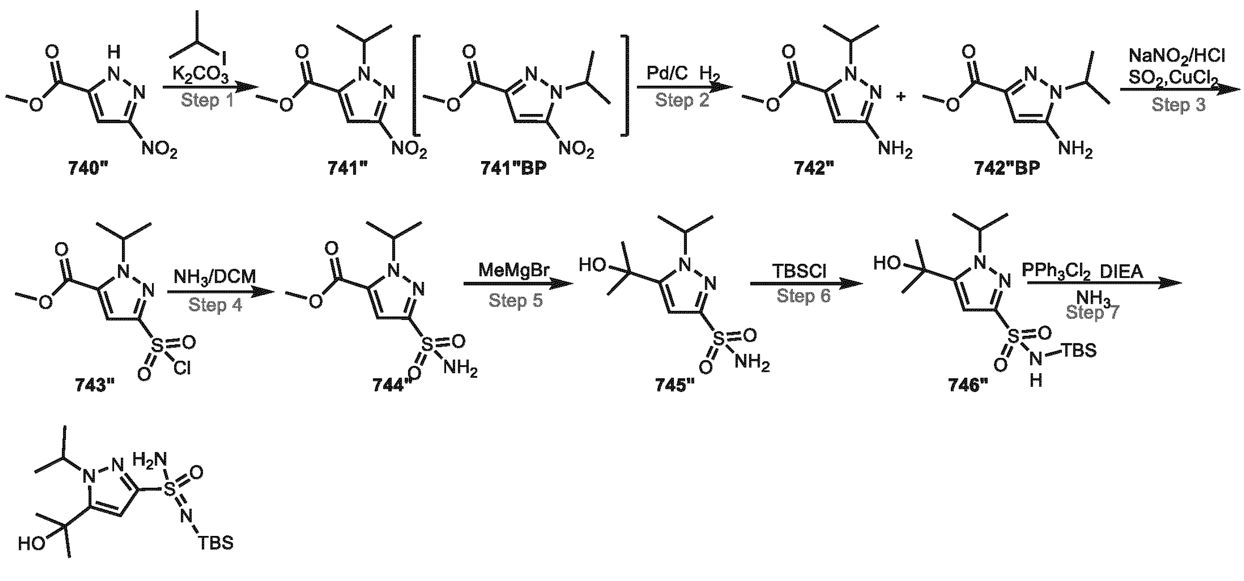


740" 741" 741"BP 742" 742"BP 743" 744" 745" 746"

Intermediate 203

## Intermediate 203

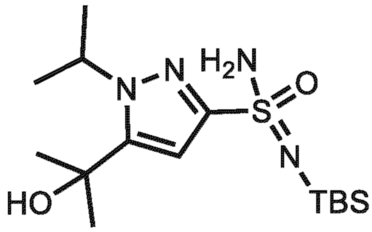

N'-(tert-butyldimethylsilyl)-5-(2-hydroxypropan-2-yl)-1-isopropyl-1H-pyrazole-3-sulfonimidamide

Steps 1-2 used similar procedures for converting compound **735"** to compound **737"** shown in Scheme 145 to afford compound **742"** from compound **740".** Compound 742" (5 g) and Compound 742"BP (0.5 g) were separated from silica gel eluted with PE/EtOAc (5:1). MS-ESI: 184 (M+1). The regiochemistry of **742"** and **742"BP** were assigned based on NMR including NOESY.

Compound 742": $^1$H NMR (300 MHz, DMSO-$d_6$) δ 5.93 (s, 1H), 5.33-5.10 (m, 1H), 4.83 (s, 2H), 3.77 (s, 3H), 1.31 (d, *J* = 6.3 Hz, 6H).

NOESY: exchangeable protons $NH_2$4.83 (s, 2H) has no correlation with i-Pr's CH at 5.33-5.10 (m, 1H) and O-$CH_3$ 3.77 (s, 3H) has correlation with i-Pr's $CH_3$ at 1.31 (d, *J* = 6.3 Hz, 6H).

Compound 742"BP: $^1$H NMR (300 MHz, DMSO-$d_6$) δ 5.72 (s, 1H), 5.35 (s, 2H), 4.59-4.32 (m, 1H), 3.71 (s, 3H), 1.30 (d, *J* = 6.6 Hz, 6H).

NOESY: exchangeable protons $NH_2$ 5.35 (s, 2H) has correlation with i-Pr's CH at 4.59-4.32 (m, 1H) and O-$CH_3$ 3.71 (s, 3H) has no correlation with i-Pr's $CH_3$ at 1.30 (d, *J* = 6.6 Hz, 6H).

Steps 3-4 used similar procedures for converting compound **737"** to compound **738"** shown in Scheme 145 to afford compound **744"** from compound **742".** MS-ESI: 246 (M-1).

**Step 5: 5-(2-Hydroxypropan-2-yl)-1-isopropyl-1H-pyrazole-3-sulfonamide**

To a stirred solution of methyl 1-isopropyl-3-sulfamoyl-1H-pyrazole-5-carboxylate (430 mg, 1.74 mmol) in THF (50 mL) in a 100-mL 3-necked round-bottom flask under nitrogen was added MeMgBr in THF (3.0 M, 3.5 mL, 10 mmol) at 0 °C. The reaction mixture was stirred for 16 h at RT. The reaction mixture was quenched with water/ice at 0 °C. The pH value of the solution was adjusted to 6 with HCl (6 M). The reaction mixture was extracted with 3x50 mL of EtOAc. The combined organic layers were dried over anhydrous $Na_2SO_4$ and concentrated under vacuum. The residue was eluted from silica gel with EtOAc/PE (3:1). This resulted in 400 mg (93%) of the title compound as a yellow solid. MS-ESI: 246 (M-1).

Steps 6-7 used similar procedures for converting compound **639"** to Intermediate **183** shown in Scheme 128 to afford Intermediate **203** from compound **745".** MS-ESI: 361 (M+1).

## Scheme 147:

## Intermediate 204

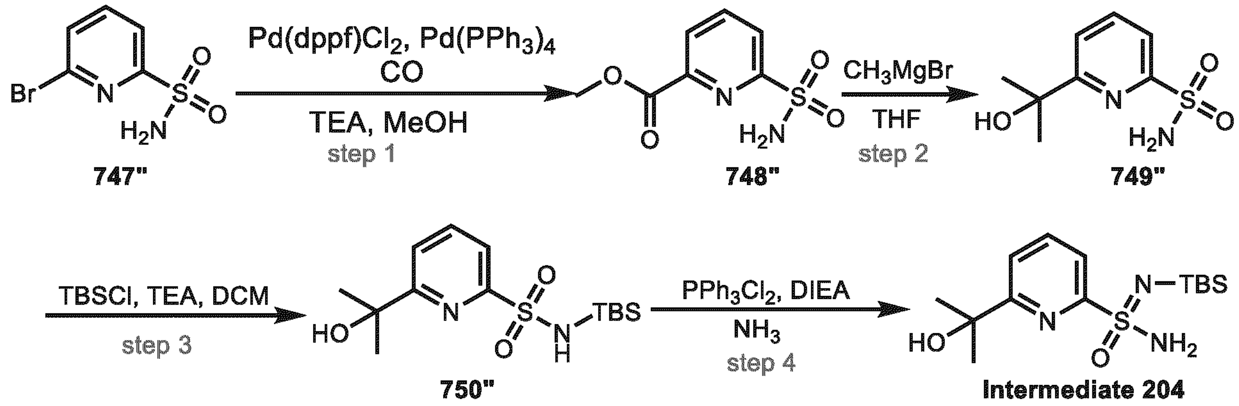


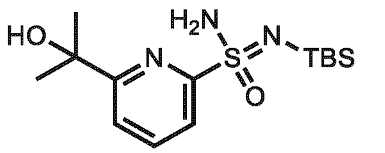

N'-(tert-butyldimethylsilyl)-6-(2-hydroxypropan-2-yl)pyridine-2-sulfonimidamide

**Step 1: Methyl 6-sulfamoylpicolinate**

To a stirred solution of 6-bromopyridine-2-sulfonamide (5.0 g, 21 mmol) in MeOH (200 mL) in 500 mL-sealed tube under nitrogen were added $Pd(dppf)Cl_2$ (1.54 g, 2.1 mmol), $Pd(PPh_3)_4$ (2.44 g, 2.1 mmol) and TEA (11 g, 105 mmol) at RT. Then the sealed tube was evacuated and refilled three times with CO. The reaction mixture was stirred overnight at 80 °C under CO atmosphere (10 atm). The reaction mixture was concentrated under vacuum. The residue was eluted from silica gel with PE/EtOAc (1:2). This resulted in 1.8 g (39%) of the title compound as a light yellow solid. MS-ESI: 215 (M-1).

Steps 2-4 used similar procedures for converting compound **744** to Intermediate **203** shown in Scheme 146 to afford Intermediate **204** from compound **748".** MS-ESI: 330 (M+1).

## Scheme 148:

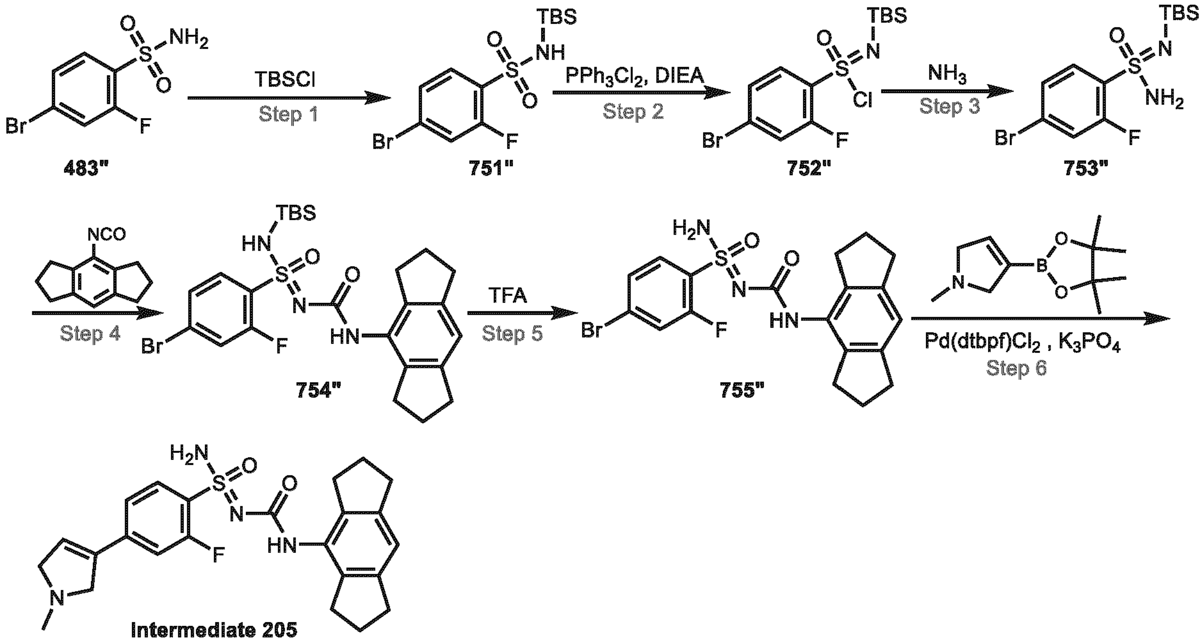


483" 751" 752" 753" 754" 755" Intermediate 205

## Intermediate 205

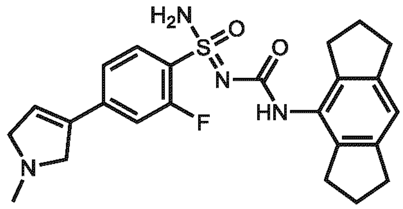

2-Fluoro-N'-((1,2,3,5,6,7-hexahydro-s-indacen-4-yl)carbamoyl)-4-(1-methyl-2,5-dihydro-1H-pyrrol-3-yl)benzenesulfonimidamide

Steps 1-3 used similar procedures for converting compound **639"** to Intermediate **183** shown in Scheme 128 to afford compound **753"** from compound **483".** MS-ESI: 367/369 (M+1).

### Step 4: 4-Bromo-N-(tert-butyldimethylsilyl)-2-fluoro-N'-((1,2,3,5,6,7-hexahydro-s-indacen-4-yl) carbamoyl)benzenesulfonimidamide

To a stirred solution of 4-bromo-N'-(tert-butyldimethylsilyl)-2-fluorobenzenesulfonimidamide (3.0 g, 8.2 mmol) in THF (50 mL) in a 250-mL round-bottom flask under nitrogen was added NaH (60% wt. dispersion in mineral oil, 1.0 g, 25 mmol) in portions at 0 °C. The reaction mixture was stirred for 10 min at RT. Then to the mixture was added 4-isocyanato-1,2,3,5,6,7-hexahydro-s-indacene (1.95 g, 9.8 mmol) in portions at 0 °C. The reaction mixture was stirred for 1 h at RT. The reaction mixture was quenched with $H_2O$ (50 mL) and then extracted with EtOAc (3x100 mL). The combined organic layers were dried over anhydrous $Na_2SO_4$ and concentrated under vacuum. The residue was eluted from silica gel column with EtOAc/PE (1:5). This resulted in 2.8 g (61%) of the title compound as a white solid. MS-ESI: 566/568 (M+1).

### Step 5: 4-Bromo-2-fluoro-N'-((1,2,3,5,6,7-hexahydro-s-indacen-4-yl)carbamoyl) benzenesulfonimidamide

To a stirred solution of 4-bromo-N-(tert-butyldimethylsilyl)-2-fluoro-N'-((1,2,3,5,6,7-hexahydro-s-indacen -4-yl)carbamoyl)benzenesulfonimidamide (2.8 g, 4.94 mmol) in DCM (100 mL) in a 250-mL round-bottom flask was added TFA (56 mg, 0.49 mmol) at RT. The reaction mixture was stirred for 1 h at RT and then concentrated under vacuum. The residue was eluted from silica gel with EtOAc/PE (1:8). This resulted in 1.9 g (85%) of the title compound as a yellow solid. MS-ESI: 452/454 (M+1).

**Step 6: 2-Fluoro-N'-((1,2,3,5,6,7-hexahydro-s-indacen-4-yl)carbamoyl)-4-(1-methyl-2,5-dihydro-1H-pyrrol-3-yl)benzenesulfonimidamide**

To a stirred solution of 4-bromo-2-fluoro-N'-((1,2,3,5,6,7-hexahydro-s-indacen-4-yl)carbamoyl) benzenesulfonimidamide (1.9 g, 4.2 mmol) in dioxane (90 mL) and $H_2O$ (10 mL) in a 250-mL round-bottom flask under nitrogen were added $K_3PO_4$ (1.78 g, 8.4 mmol), Pd(dtbpf)$Cl_2$ (274 mg, 0.42 mmol) and 1-methyl-3-(4,4,5,5-tetramethyl-1,3,2-dioxaborolan-2-yl)-2,5-dihydro-1H-pyrrole (966 mg, 4.62 mmol) at RT. The reaction mixture was stirred for 5 h at 60 °C and then concentrated under vacuum. The resulted was dissolved in 100 mL $H_2O$ and extracted with EtOAc (3x100 mL). The combined organic layers were dried over $Na_2SO_4$ and concentrated under vacuum. The residue was eluted from silica gel with EtOAc/PE (1:1). This resulted in 100 mg (5.3%) of the title compound as a yellow solid. MS-ESI: 455 (M+1).

## Scheme 149:

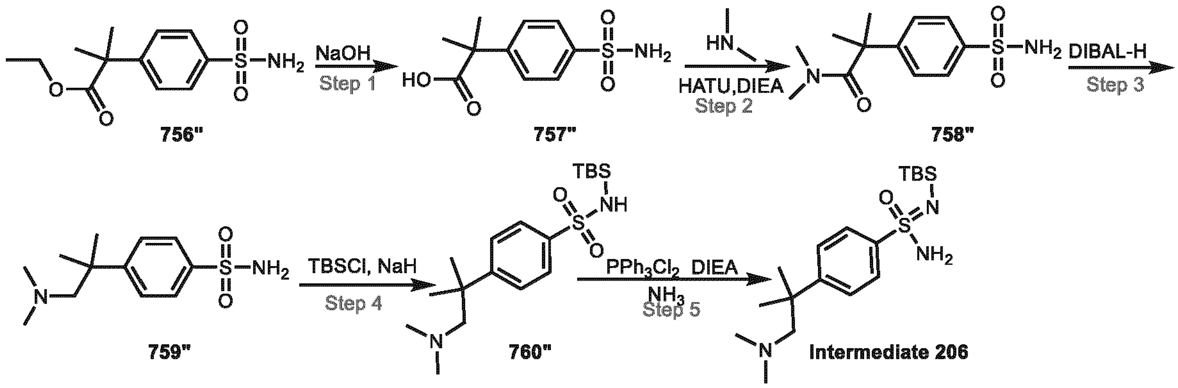


## Intermediate 206

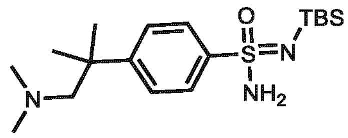

N'-(tert-butyldimethylsilyl)-4-(1-(dimethylamino)-2-methylpropan-2-yl)benzenesulfonimidamide

**Step 1: 2-Methyl-2-(4-sulfamoylphenyl)propanoic acid**

To a stirred solution of ethyl 2-methyl-2-(4-sulfamoylphenyl)propanoate (7.0 g, 26 mmol) in MeOH (140 mL) and $H_2O$ (93 mL) in a 500-mL round-bottom flask was added NaOH (10 g, 258 mmol) at 0 °C. The reaction mixture was stirred for 90 min at 55 °C in an oil bath. The pH value of the mixture was adjusted to 2-3 with HCl (2 M). To the mixture was added additional 200 mL of MeOH. The solids were filtered out. The filtrate was concentrated under vacuum. This resulted in 5.0 g (80%) of the title compound as a white crude solid. MS-ESI: 242 (M-1).

**Step 2: N,N,2-trimethyl-2-(4-sulfamoylphenyl)propanamide**

To a stirred solution of 2-methyl-2-(4-sulfamoylphenyl)propanoic acid (3.0 g, 12 mmol) in THF (150 mL) in a 250-mL round-bottom flask were added HATU (6.8 g, 18 mmol), DIEA (3.19 g, 25 mmol) and dimethylamine (4.44 g, 99 mmol) at RT. The reaction solution was stirred for 12 h at RT. The resulting solution was concentrated under vacuum. The residue was eluted from silica gel with DCM/MeOH (30:1). This resulted in 2.5 g (75%) of the title compound as a white solid. MS-ESI: 269 (M-1).

**Step 3: 4-(1-(Dimethylamino)-2-methylpropan-2-yl)benzenesulfonamide**

To a stirred solution of N,N,2-trimethyl-2-(4-sulfamoylphenyl)propanamide (2.5 g, 9.25 mmol) in THF (30 mL) in a 250-mL 3-necked round-bottom flask under nitrogen was added DIBAL-H in hexane (1 M, 46 mL, 46.2 mmol) at

-10 °C. The reaction mixture was stirred for 12 h at RT. The reaction was quenched with 15 mL of MeOH. The reaction mixture was concentrated under vacuum. The residue was diluted with 200 mL of HCl (2 M). The mixture was extracted with 3x200 mL of EtOAc. The combined organic layers were dried over anhydrous $Na_2SO_4$ and concentrated under vacuum. The residue was eluted from silica gel with DCM/MeOH (10:1). This resulted in 1.50 g (63%) of the title compound as a yellow solid. MS-ESI: 255 (M-1).

Steps 4-5 used similar procedures for converting compound **639"** to Intermediate **183** shown in Scheme 128 to afford Intermediate **206** from compound **759".** MS-ESI: 370 (M+1).

## Scheme 150:

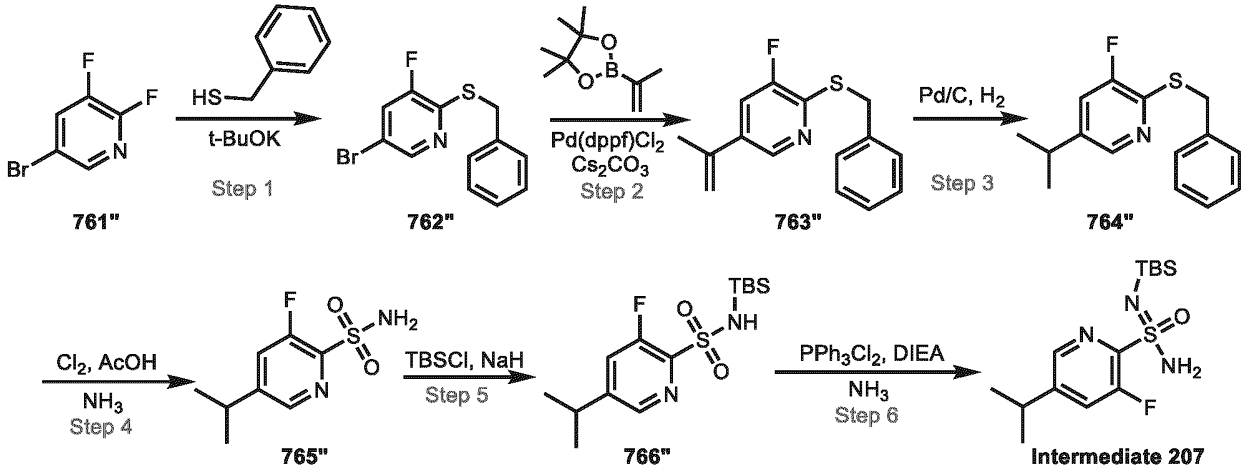


## Intermediate 207

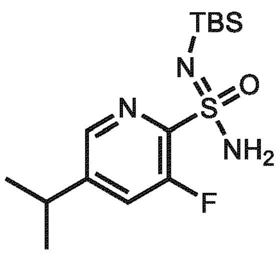

N'-(tert-butyldimethylsilyl)-3-fluoro-5-isopropylpyridine-2-sulfonimidamide

**Step 1: 2-(Benzylthio)-5-bromo-3-fluoropyridine**

To a stirred solution of 5-bromo-2,3-difluoropyridine (20 g, 103 mmol) in dioxane (80 mL) in a 500-mL round-bottom flask under nitrogen were added t-BuOK (11.6 g, 103 mmol) in portions at 0 °C, followed by phenylmethanethiol (12.8 g, 103 mmol) in portions at RT. The reaction mixture was stirred overnight at 75 °C and then quenched with $H_2O$ (200 mL). The mixture was extracted with EtOAc (3x200 mL). The organic layers were dried over anhydrous $Na_2SO_4$ and concentrated under vacuum. The residue was eluted from silica gel with EtOAc/PE (1:1). This resulted in 18 g (59%) of the title compound as colorless oil. MS-ESI: 298/300 (M+1).

**Step 2: 2-(Benzylthio)-3-fluoro-5-(prop-1-en-2-yl)pyridine**

To a stirred solution of 2-(benzylthio)-5-bromo-3-fluoropyridine (18 g, 48 mmol) in dioxane (300 mL) and $H_2O$ (60 mL) in a 1-L round-bottom flask under nitrogen were added $Cs_2CO_3$ (47 g, 145 mmol) and $Pd(dppf)Cl_2$ (5.3 g, 7.2 mmol). Then to the above mixture was added 4,4,5,5-tetramethyl-2-(prop-1-en-2-yl) -1,3,2-dioxaborolane (24 g, 145 mmol) at RT. The reaction mixture was stirred overnight at 90 °C. The reaction mixture was extracted with 4x300 mL of EtOAc and the organic layers combined and concentrated under vacuum. The residue was eluted from silica gel with EtOAc/PE (1:2). This resulted in 10 g (80%) of the title compound as brown oil. MS-ESI: 260 (M+1).

**Step 3: 2-(Benzylthio)-3-fluoro-5-isopropylpyridine**

To a stirred solution of 2-(benzylthio)-3-fluoro-5-(prop-1-en-2-yl)pyridine (10 g, 39 mmol) in MeOH (20 mL) in a 300-mL sealed tube under nitrogen were added AcOH (1.0 mL) and Pd/C (10%wt., 2.1 g) at RT. The sealed tube was evacuated and refilled three times with hydrogen. The resulting mixture was stirred for 24 h at 40 °C under atmosphere of hydrogen with a balloon. The solids were filtered out. The filtrate was concentrated under vacuum. This resulted in 5.0 g (49%) of the title compound as brown oil. MS-ESI: 262 (M+1).

**Step 4: 3-Fluoro-5-isopropylpyridine-2-sulfonamide**

To a stirred solution of 2-(benzylthio)-3-fluoro-5-isopropylpyridine (500 mg, 1.9 mmol) in DCM (20 mL) and AcOH (20.00 mL) and $H_2O$ (20 mL) in a 250-mL 3-necked round-bottom flask was introduced $Cl_2$ bubbled for 30 min at 0 °C. The mixture was extracted with DCM (3x40 mL). The combined organic layers were washed with brine (3x100 mL). The organic layer was dried over anhydrous $Na_2SO_4$. Then the solids were filtered out, $NH_3$ (g) was bubbled into the filtrate for 20 min at 0 °C. The reaction mixture was stirred overnight at RT. The mixture was concentrated under vacuum. The residue was eluted from silica gel with EtOAc (100%). This resulted in 50 mg (12%) of the title compound as yellow oil. MS-ESI: 217 (M-1).

Steps 5-6 used similar procedures for converting compound **639"** to Intermediate **183** shown in Scheme 128 to afford Intermediate **207** from compound **765"**. MS-ESI: 332 (M+1).

**Scheme 151:**

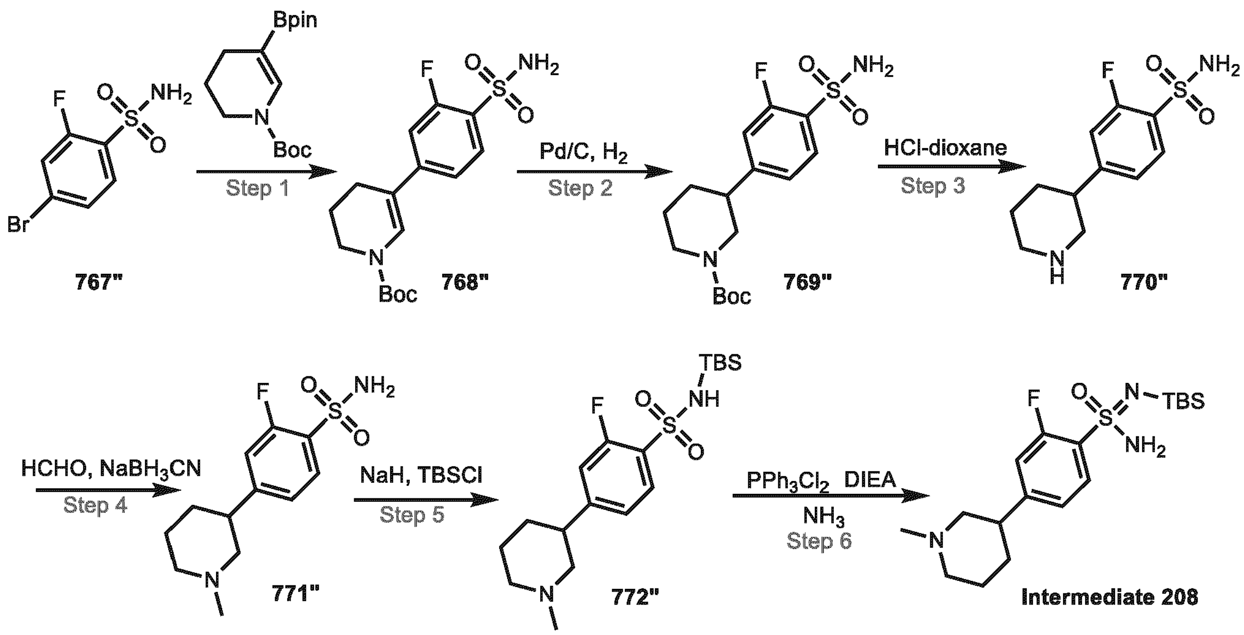


**Intermediate 208**

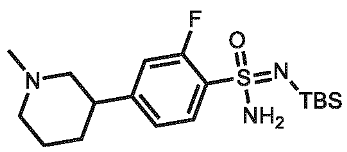

N'-(tert-butyldimethylsilyl)-2-fluoro-4-(1-methylpiperidin-3-yl)benzenesulfonimidamide

Steps 1-2 used similar procedures for converting compound **762"** to compound **764"** shown in Scheme 150 to afford compound **769"** from compound **767"**. MS-ESI: 357 (M-1).

**Step 3: 2-Fluoro-4-(piperidin-3-yl)benzenesulfonamide**

To a stirred solution of tert-butyl 3-(3-fluoro-4-sulfamoylphenyl)piperidine-1-carboxylate (500 mg, 1.4 mmol) in DCM (5.0 mL) in a 25-mL round-bottom flask was added HCl in dioxane (4 M, 3.5 mL, 14 mmol) dropwise at RT. The reaction mixture was stirred for 2 h at RT and then diluted with 5.0 mL of water. The mixture was extracted with 3x10 mL of DCM. The organic layer was dried over anhydrous $Na_2SO_4$ and concentrated under vacuum. This resulted in 400 mg (crude) of the title compound as a yellow solid. MS-ESI: 257 (M-1).

**Step 4: 2-Fluoro-4-(1-methylpiperidin-3-yl)benzenesulfonamide**

To a stirred solution of 2-fluoro-4-(piperidin-3-yl)benzenesulfonamide (400 mg, crude) in MeOH (5.0 mL) in a 25-mL round-bottom flask under nitrogen was added HCHO (269 mg, 8.4 mmol) in portions at RT. The resulting solution was stirred overnight at RT. Then to the solution was added $NaBH_3CN$ (173 mg, 2.8 mmol) at RT. The reaction mixture was stirred for 2 h at RT. The mixture was concentrated under vacuum. The reaction was quenched with $H_2O$ (10 mL). The mixture was extracted with EtOAc (3x20 mL). The organic layer was dried over anhydrous $Na_2SO_4$ and concentrated under vacuum. The residue was eluted from silica gel with DCM/MeOH (10:1). This resulted in 300 mg (79%) of the title compound as a yellow solid. MS-ESI: 271 (M-1).

Steps 5-6 used similar procedures for converting compound **639"** to Intermediate **183** shown in Scheme 128 to afford Intermediate **208** from compound **771"**. MS-ESI: 386 (M+1).

**Scheme 152:**

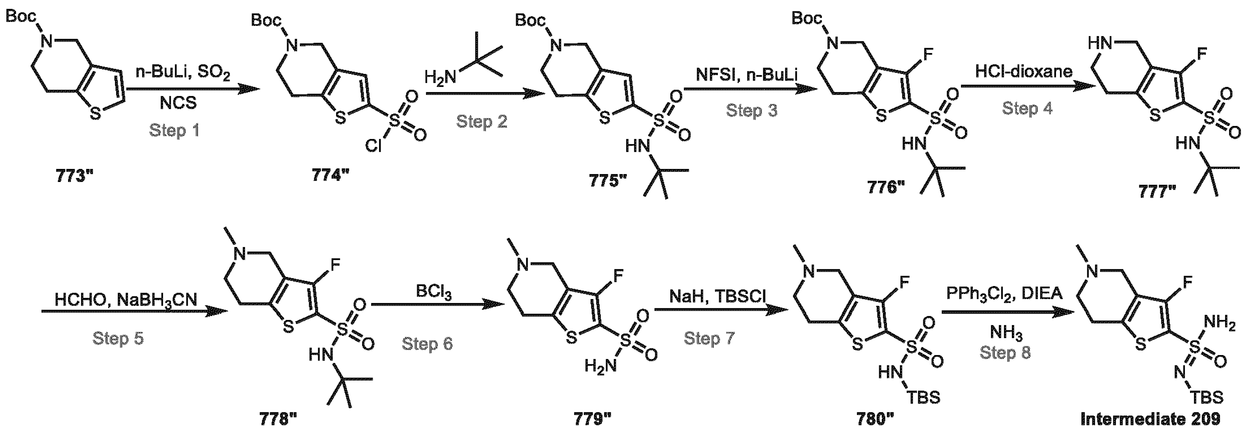


**Intermediate 209**

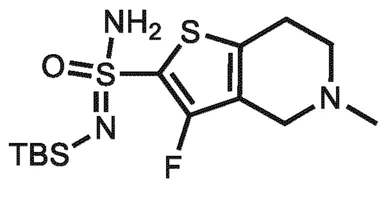

N'-(tert-butyldimethylsilyl)-3-fluoro-5-methyl-4,5,6,7-tetrahydrothieno[3,2-c]pyridine-2-sulfonimidamide

Steps 1-2 used similar procedures for converting compound **642"** to compound **644"** shown in Scheme 129 to afford compound **775"** from compound **773"**. MS-ESI: 375 (M+1).

**Step 3: Tert-butyl 2-(N-(tert-butyl)sulfamoyl)-3-fluoro-6,7-dihydrothieno[3,2-c]pyridine-5(4H)- carboxylate**

To a stirred solution of tert-butyl 2-(N-(tert-butyl)sulfamoyl)-6,7-dihydrothieno[3,2-c]pyridine-5(4H)- carboxylate (4.0 g, 10.7 mmol) in THF (40 mL) in a 250-mL 3-necked round-bottom flask under nitrogen was added n-BuLi in hexane (2.5 M, 13 mL, 32 mmol) dropwise at -78 °C. The reaction mixture was stirred for 30 min at -78 °C. Then to the reaction solution was added NFSI (6.74 g, 21.4 mmol) in THF (10 mL) dropwise at -78 °C. The reaction mixture was stirred for

2 h at RT and then quenched with $H_2O$ (40 mL). The mixture was extracted with EtOAc (3x100 mL) and the organic layers were combined, dried over anhydrous $Na_2SO_4$, and concentrated under vacuum. The residue was eluted from silica gel with EtOAc/PE (1:1). This resulted in 1.7 g (41%) of the title compound as yellow oil. MS-ESI: 392 (M+1).

**Step 4: N-(tert-butyl)-3-fluoro-4,5,6,7-tetrahydrothieno[3,2-c]pyridine-2-sulfonamide**

To a stirred solution of tert-butyl 2-(N-(tert-butyl)sulfamoyl)-3-fluoro-6,7-dihydrothieno[3,2-c]pyridine-5(4H)-carboxylate (300 mg, 0.76 mmol) in DCM (5.0 mL) in a 25-mL round-bottom flask was added HCl in dioxane (4 M, 0.75 mL) dropwise at RT. The reaction mixture was stirred for 1 h at RT. The reaction mixture was concentrated under vacuum. This resulted in 200 mg (89%) of the title compound as yellow oil. MS-ESI: 293 (M+1).

**Step 5: N-(tert-butyl)-3-fluoro-5-methyl-4,5,6,7-tetrahydrothieno[3,2-c]pyridine-2-sulfonamide**

To a stirred solution of N-(tert-butyl)-3-fluoro-4,5,6,7-tetrahydrothieno[3,2-c]pyridine-2-sulfonamide (200 mg, 0.68 mmol) in MeOH (2.0 mL) in a 25-mL round-bottom flask under nitrogen was added HCHO (31 mg, 1.03 mmol) in portions. The reaction mixture was stirred for 1 h at RT. To above mixture was added $NaBH_3CN$ (64 mg, 1.03 mmol) in portions at RT. The reaction mixture was stirred for 2 h at RT. The reaction mixture was concentrated under vacuum. The residue was eluted from silica gel with EtOAc/PE (1:1). This resulted in 200 mg (95%) of the title compound as yellow oil. MS-ESI: 307 (M+1).

Step 6 used similar procedures for converting compound **703"** to compound **704"** shown in Scheme 139 to afford compound **779"** from compound **778"**. MS-ESI: 249 (M-1).

Steps 7-8 used similar procedures for converting compound **639"** to Intermediate **183** shown in Scheme 128 to afford Intermediate **209** from compound **779"**. MS-ESI: 364 (M+1).

**Scheme 153:**

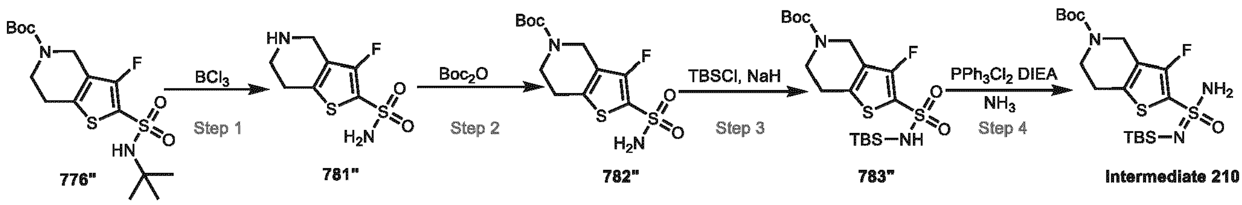


**Intermediate 210**

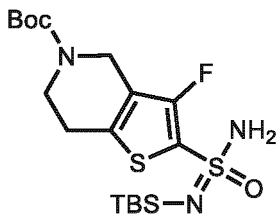

Tert-butyl 2-(N-(tert-butyldimethylsilyl)sulfamidimidoyl)-3-fluoro-6,7-dihydrothieno[3,2-c]pyridine-5(4H)-carboxylate

Steps 1-4 used similar procedures for converting compound **688"** to Intermediate **192** shown in Scheme 136 to afford Intermediate **210** from compound **776"**. MS-ESI: 450 (M+1).

**Scheme 154:**

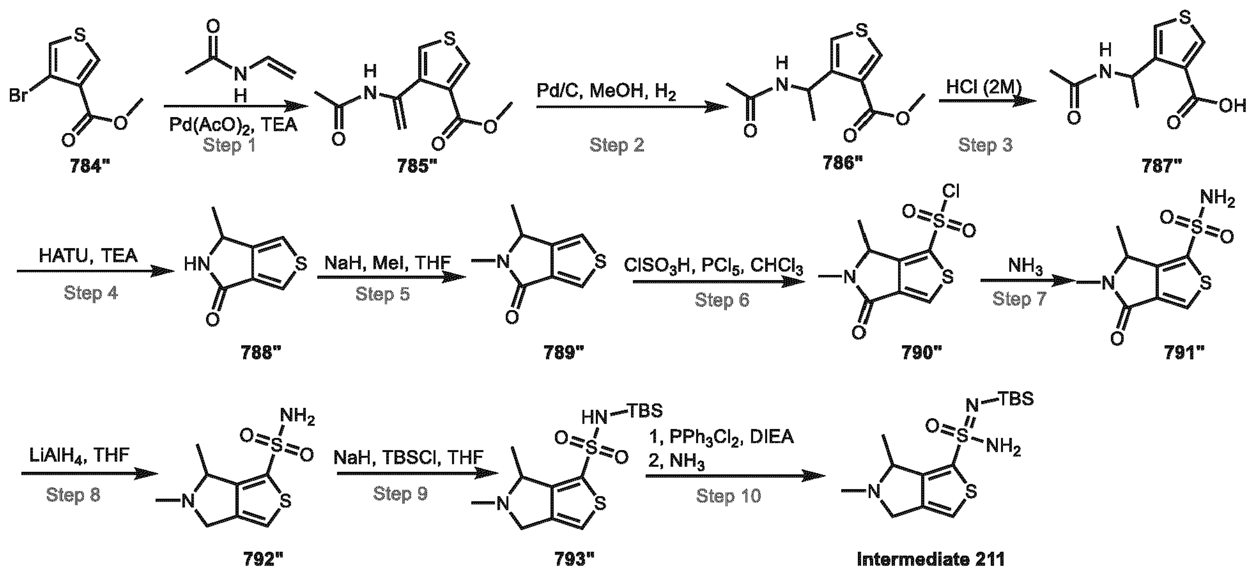


## Intermediate 211

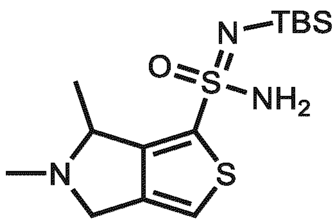

N-(tert-butyldimethylsilyl)-5,6-dimethyl-5,6-dihydro-4H-thieno[3,4-c]pyrrole-1-sulfonimidamide

**Step 1: Methyl 4-(1-acetamidovinyl)thiophene-3-carboxylate**

To a stirred solution of methyl 4-bromothiophene-3-carboxylate (10 g, 45 mmol) in DMF (100 mL) in a 250-mL round-bottom flask under nitrogen were added tris(2-methoxyphenyl)phosphine (3.19 g, 9.05 mmol) and N-vinylacetamide (2.24 g, 226 mmol) at RT. To the above mixture were added Pd(AcO)$_2$ (1.02 g, 4.5 mmol) and TEA (6.0 mL, 43 mmol) at RT. Then the reaction mixture was stirred overnight at 80 °C. The reaction mixture was quenched with $H_2O$ (100 mL). The mixture was extracted with EtOAc (3x200 mL) and the organic layers were combined. The organic layer was dried over anhydrous $Na_2SO_4$ and concentrated under vacuum. The residue was eluted from silica gel with PE/EtOAc (1:1). This resulted in 6.0 g (60%) of the title compound as yellow liquid. MS-ESI: 226 (M+1).

**Step 2: Methyl 4-(1-acetamidoethyl)thiophene-3-carboxylate**

To a stirred solution of methyl 4-(1-acetamidovinyl)thiophene-3-carboxylate (5.0 g, 22 mmol) in MeOH (100 mL) in a 250-mL round-bottom flask under nitrogen was added Pd/C (10% wt., 500 mg) in portions at 0 °C. The flask was evacuated and refilled three times with hydrogen. The reaction mixture was stirred for 2 days at RT under atmosphere of hydrogen with a balloon. The reaction mixture was filtered and the filter cake was washed with MeOH (3x10 mL). The filtrate was concentrated under reduced pressure. The residue was eluted from silica gel with PE/EtOAc (1:1). This resulted in 3.3 g (66%) of the title compound as a yellow solid. MS-ESI: 228 (M+1).

**Step 3: 4-(1-Acetamidoethyl)thiophene-3-carboxylic acid**

A solution of methyl 4-(1-acetamidoethyl)thiophene-3-carboxylate (3.2 g, 14 mmol) in HCl (2 M, 30 mL) in a 100-mL round-bottom flask under nitrogen was stirred overnight at 100 °C. The resulting mixture was concentrated under vacuum. This resulted in 3.0 g (crude) of the title compound as a brown solid. MS-ESI: 212 (M-1).

**Step 4: 6-Methyl-5,6-dihydro-4H-thieno[3,4-c]pyrrol-4-one**

To a stirred solution of 4-(1-acetamidoethyl)thiophene-3-carboxylic acid (3.0 g, crude) in DCM (200 mL) were added HATU (8.02 g, 21 mmol) and TEA (4.27 g, 42 mmol) in portions at RT. The reaction mixture was stirred overnight at RT. The reaction mixture was concentrated under vacuum. The residue was eluted from silica gel with PE/EtOAc (1:1). This resulted in 1.9 g (88%, over two steps) of the title compound as a light yellow solid. MS-ESI: 154 (M+1).

Step 5 used similar procedures for converting compound **770"** to compound **771"** shown in Scheme 151 to afford compound **789"** from compound **788".** MS-ESI: 168 (M+1).

Steps 6-7 used similar procedures for converting compound **672"** to compound **674"** shown in Scheme 134 to afford compound **791"** from compound **789".** MS-ESI: 245 (M-1).

**Step 8: 5,6-Dimethyl-5,6-dihydro-4H-thieno[3,4-c]pyrrole-1-sulfonamide**

To a stirred solution of 5,6-dimethyl-4-oxo-5,6-dihydro-4H-thieno[3,4-c]pyrrole-1-sulfonamide (486 mg, 1.97 mmol) in THF (5.0 mL) under nitrogen was added $LiAlH_4$ (225 mg, 5.91 mmol) in portions at 0 °C. The reaction mixture was stirred overnight at 70 °C. The reaction mixture was quenched with $H_2O$ (0.3 mL) and aq. NaOH (25% wt., 0.3 mL) at 0 °C. The mixture was concentrated under vacuum. The residue was eluted from silica gel with DCM/MeOH (12:1). This resulted in 300 mg (65%) of the title compound as yellow oil. MS-ESI: 231 (M-1).

Steps 9-10 used similar procedures for converting compound **639"** to Intermediate **183** shown in Scheme 128 to afford Intermediate **211** from compound **792".** MS-ESI: 346 (M+1).

## Scheme 155:

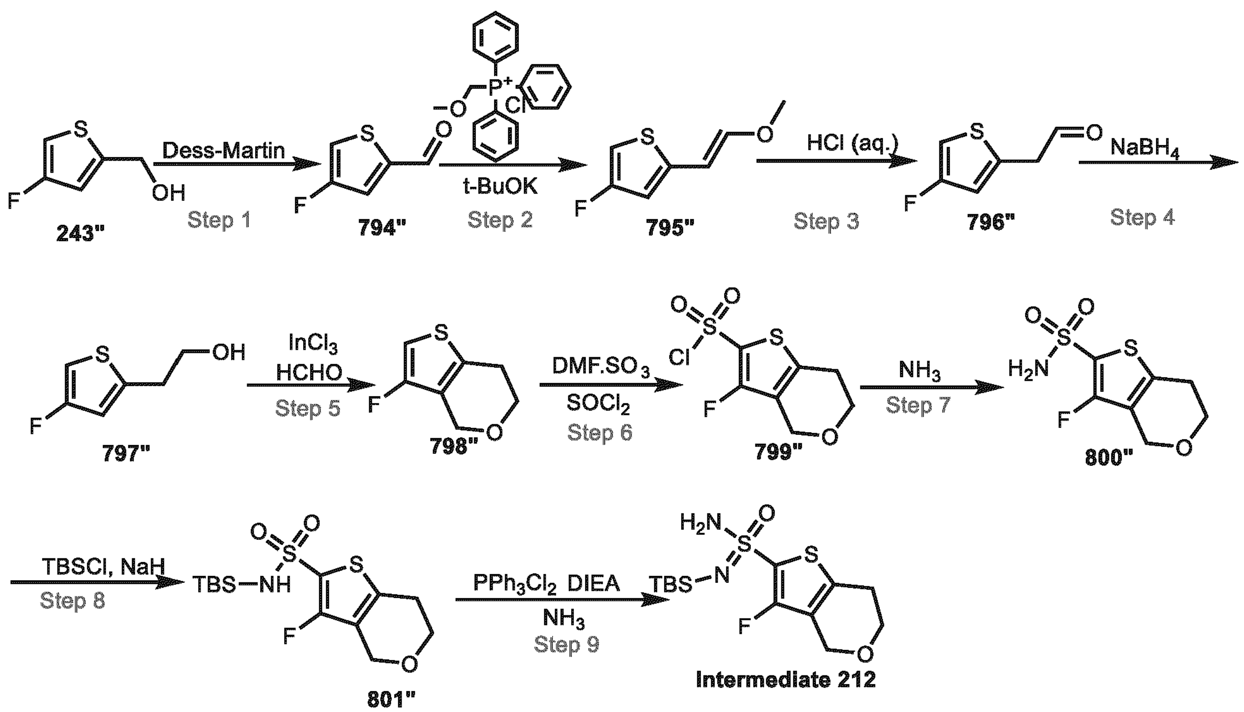


## Intermediate 212

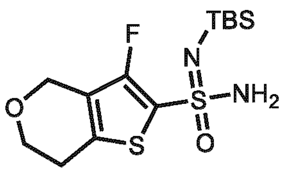

N'-(tert-buiyldimethylsilyl)-3-fluoro-6,7-dihydro-4H-thieno[3,2-c]pyran-2-sulfonimidamide

**Step 1: 4-Fluorothiophene-2-carbaldehyde**

To a stirred solution of (4-fluorothiophen-2-yl)methanol (7.5 g, 56.8 mmol) in DCM (300 mL) in a 1 L round-bottom flask under nitrogen was added Dess-Martin reagent (48 g, 113 mmol) in portions at 0 °C. The reaction mixture was stirred for 1 h at RT and then quenched with sat. aq. $Na_2S_2O_3$ (200 mL). The mixture was extracted with DCM (3x200 mL) and the organic layer combined. The organic layer was dried over anhydrous $Na_2SO_4$ and concentrated under vacuum. The residue was eluted from silica gel with PE/EtOAc (1:1). This resulted in 6.75 g (91%) of the title compound as yellow oil. MS-ESI: 131 (M+1).

**Step 2: (E)-4-fluoro-2-(2-methoxyvinyl)thiophene**

To a stirred solution of 4-fluorothiophene-2-carbaldehyde (5.0 g, 38.4 mmol) in THF (300 mL) in a 1 L round-bottom flask under nitrogen were added (methoxymethyl)triphenylphosphanium chloride (20 g, 58 mmol) in portions at 0 °C, followed by the addition of t-BuOK (6.47 g, 58 mmol) in portions at 0 °C. The reaction mixture was stirred for 4 h at 15 °C, quenched with $H_2O$ (200 mL), and extracted with EtOAc (3x200 mL). The combined organic layers were dried over anhydrous $Na_2SO_4$ and concentrated under vacuum. The residue was eluted from silica gel with EtOAc/PE (1:9). This resulted in 5.0 g (82%) of the title compound as brown oil. MS-ESI: 159 (M+1).

**Step 3: 2-(4-Fluorothiophen-2-yl)acetaldehyde**

To a stirred solution of (E)-4-fluoro-2-(2-methoxyvinyl)thiophene (5.0 g, 32 mmol) in dioxane (36 mL) in a 250 mL round-bottom flask was added aq. HCl (4 M, 36 mL) at 0 °C. The reaction mixture was stirred for 4 h at 15 °C. The mixture was extracted with EtOAc (3x100 mL) and the organic layers were combined and concentrated under vacuum. This resulted in 1.5 g (33%) of the title compound as brown oil. MS-ESI: 145 (M+1).

Step 4 used similar procedures for converting compound **731"** to compound **732"** shown in Scheme 144 to afford compound **797"** from compound **796".** MS-ESI: 147 (M+1).

**Step 5: 3-Fluoro-6,7-dihydro-4H-thieno[3,2-c]pyran**

To a stirred solution of 2-(4-fluorothiophen-2-yl)ethan-1-ol (300 mg, 2.05 mmol) in ACN (30 mL) in a 100-mL round-bottom flask were added $InCl_3$ (902 mg, 4.1 mmol) at RT, followed by the addition of paraformaldehyde (131 mg, 4.1 mmol) in portions at RT. The reaction mixture was stirred overnight at 70 °C. The reaction mixture was quenched with $H_2O$ (30 mL) and extracted with EtOAc (3x50 mL). The combined organic layers were combined and dried over anhydrous $Na_2SO_4$ and concentrated under vacuum. The residue was eluted from silica gel with EtOAc/PE (8:92). This resulted in 240 mg (74%) of the title compound as colorless oil. MS-ESI: 159 (M+1).

**Step 6: 3-Fluoro-6,7-dihydro-4H-thieno[3,2-c]pyran-2-sulfonyl chloride**

To a stirred solution of 3-fluoro-6,7-dihydro-4H-thieno[3,2-c]pyran (300 mg, 1.9 mmol) in 1,2-dichloroethane (50 mL) in a 100-mL round-bottom flask was added sulfur trioxide DMF complex (349 mg, 2.28 mmol) in portions at RT. The reaction mixture was stirred for 30 min at RT. To above mixture was added $SOCl_2$ (271 mg, 2.3 mmol) dropwise at 0 °C. The reaction mixture was stirred overnight at 50 °C and then concentrated under vacuum. This resulted in 260 mg (crude) of the title compound as a yellow solid.

**Step 7: 3-Fluoro-6,7-dihydro-4H-thieno[3,2-c]pyran-2-sulfonamide**

To a stirred solution of 3-fluoro-6,7-dihydro-4H-thieno[3,2-c]pyran-2-sulfonyl chloride (260 mg, crude) in DCM (10 mL) in a 50-mL round-bottom flask was introduced $NH_3$ (g) bubbled for 10 min at 0 °C. The reaction mixture was stirred for 2 h at 15 °C. The reaction mixture was quenched with $H_2O$ (10 mL). The mixture was extracted with DCM (3x10 mL) and the organic layers were combined and concentrated under vacuum. The residue was eluted from silica gel with EtOAc/PE (1:2). This resulted in 200 mg (44%, over two steps) of the title compound as a yellow solid. MS-ESI: 236 (M-1).

Steps 8-9 used similar procedures for converting compound **639"** to Intermediate **183** shown in Scheme 128 to afford Intermediate **212** from compound **800".** MS-ESI: 351 (M+1).

**Scheme 156:**

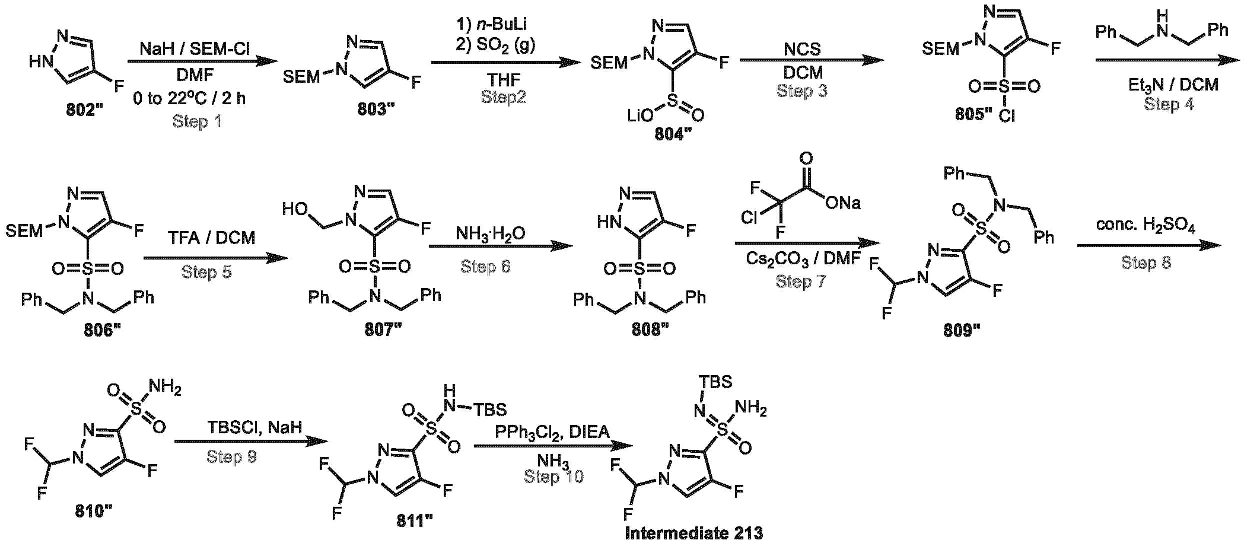


## Intermediate 213

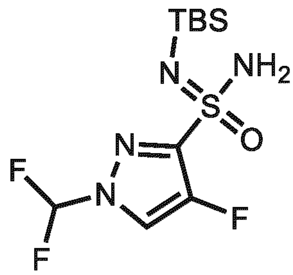

N'-(tert-butyldimethylsilyl)-1-(difluoromethyl)-4-fluoro-1H-pyrazole-3-sulfonimidamide

**Step 1: 4-Fluoro-1-((2-(trimethylsilyl)ethoxy)methyl)-1H-pyrazole**

To a stirred solution of 4-fluoro-1H-pyrazole (10 g, 116 mmol) in DMF (100 mL) under nitrogen was added NaH (60% wt., dispersion in mineral oil, 10.7 g, 267 mmol) at 0 °C over 10 min. The reaction mixture was stirred for 30 min at 10 °C. To this mixture was added SEM-Cl (45 g, 267 mmol) dropwise at 0 °C over 10 min. The reaction mixture was stirred overnight at 7 °C. The reaction was quenched with 100 mL of water, extracted with 3x100 mL of EtOAc. The organic layers were combined, washed with 5x100 mL of brine, dried over anhydrous $Na_2SO_4$, and concentrated under vacuum. The residue was eluted from silica gel with EtOAc/PE (1:100). This resulted in 27.4 g (crude) of the title compound as a light yellow liquid. MS-ESI: 217 (M+1).

Steps 2-3 used similar procedures for converting compound **719"** to compound **720"** shown in Scheme 141 to afford compound **805"** from compound **803".**

**Step 4: N,N-dibenzyl-4-fluoro-1-((2-(trimethylsilyl)ethoxy)methyl)-1H-pyrazole-5-sulfonamide**

To a stirred solution of 4-fluoro-1-((2-(trimethylsilyl)ethoxy)methyl)-1H-pyrazole-5-sulfonyl chloride (20.4 g, 65 mmol) in DCM (200 mL) was added $Et_3N$ (8.85 g, 87 mmol) dropwise with stirring at 0 °C over 5 min. To this mixture was added dibenzylamine (17 g, 84 mmol) dropwise with stirring at 0 °C over 5 min. The reaction mixture was stirred for 1 h at 8 °C. The reaction was quenched with 300 mL of water. The mixture was extracted with 3x300 mL of DCM and the organic layers were combined. The organic layer was dried over anhydrous $Na_2SO_4$ and concentrated under vacuum. The residue was eluted from silica gel with EtOAc/PE (1:19). This resulted in 22.5 g (73%) of the title compound as light yellow oil. MS-ESI: 346 (M+1).

**Step 5: N,N-dibenzyl-4-fluoro-1-(hydroxymethyl)-1H-pyrazole-5-sulfonamide**

To a stirred solution of N,N-dibenzyl-4-fluoro-1-((2-(trimethylsilyl)ethoxy)methyl)-1H-pyrazole-5- sulfonamide (22.5 g, 47.3 mmol) in DCM (25 mL) was added TFA (25 mL) dropwise at RT. The reaction mixture was stirred overnight at RT and then concentrated under vacuum. The residue was eluted from silica gel with EtOAc/PE (1:4). This resulted in 15 g (84%) of the title compound as yellow oil. MS-ESI: 376 (M+1).

**Step 6: N,N-dibenzyl-4-fluoro-1H-pyrazole-5-sulfonamide**

To a stirred solution of N,N-dibenzyl-4-fluoro-1-(hydroxymethyl)-1H-pyrazole-5-sulfonamide (15 g, 40 mmol) in dioxane (50 mL) was added $NH_3 \cdot H_2O$ (30% wt., 50 mL) dropwise at RT. The reaction mixture was stirred for 3 h at RT and then concentrated under vacuum. The residue was eluted from silica gel with EtOAc/PE (1:1). This resulted in 12 g (87%) of the title compound as a white solid. MS-ESI: 346 (M+1).

**Step 7: N,N-dibenzyl-1-(difluoromethyl)-4-fluoro-1H-pyrazole-3-sulfonamide**

To a stirred solution of N,N-dibenzyl-4-fluoro-1H-pyrazole-5-sulfonamide (6.6 g, 19 mmol) in DMF (100 mL) were added $Cs_2CO_3$ (18.7 g, 57 mmol) in portions at RT, followed by the addition of sodium 2-chloro-2,2-difluoroacetate (4.38 g, 29 mmol) in portions at RT. The reaction mixture was stirred for 3 h at 110 °C. The reaction mixture was quenched with 100 mL of water/ice at 0 °C. The mixture was extracted with 3x100 mL of EtOAc and the organic layers were combined. The organic layer was washed with 5x100 ml of brine, dried over anhydrous $Na_2SO_4$, and concentrated under vacuum. The residue was eluted from silica gel with EtOAc/PE (1:5). This resulted in 2.98 g (39%) of the title compound as a white solid. MS-ESI: 396 (M+1).

**Step 8: 1-(Difluoromethyl)-4-fluoro-1H-pyrazole-3-sulfonamide**

To a stirred solution of N,N-dibenzyl-1-(difluoromethyl)-4-fluoro-1H-pyrazole-3-sulfonamide (2.97 g, 7.5 mmol) in DCM (30 mL) was added cc.$H_2SO_4$ (98% wt., 30 mL) dropwise at 0 °C. The reaction mixture was stirred for 90 min at RT. The reaction mixture was quenched with 20 mL of water/ice. The pH value of the solution was adjusted to 7 with NaOH (5 M). The mixture was extracted with EtOAc (3x100 mL). The organic layer was dried over anhydrous $Na_2SO_4$ and concentrated under vacuum. The residue was eluted from silica gel with DCM/MeOH (15:1). This resulted in 1.31 g (81%) of the title compound as a white solid. MS-ESI: 214 (M-1).

Steps 9-10 used similar procedures for converting compound **639"** to Intermediate **183** shown in Scheme 128 to afford Intermediate **213** from compound **810".** MS-ESI: 329 (M+1).

**Scheme 157:**

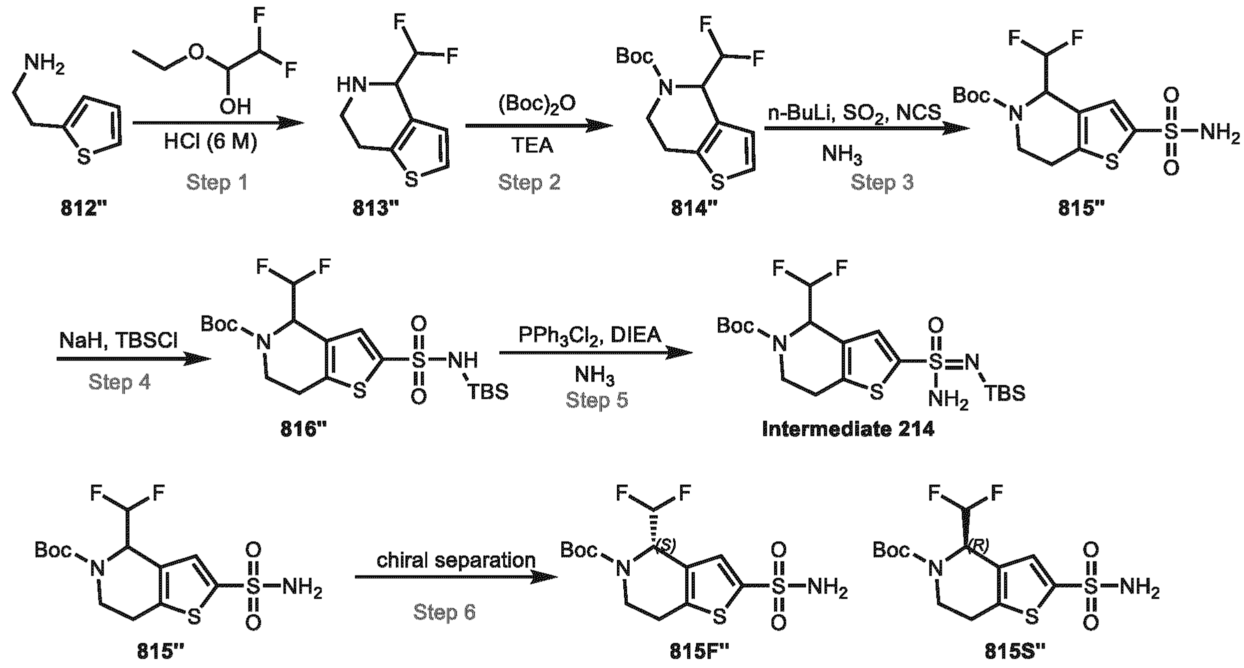


812'' 813'' 814'' 815''

816'' Intermediate 214

815'' 815F'' 815S''

## Intermediate 214

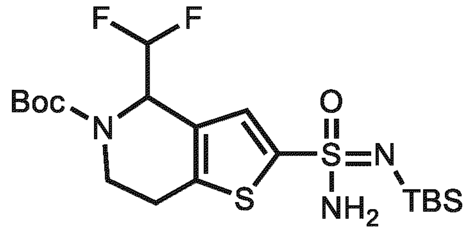

Tert-butyl 2-(N'-(tert-butyldimethylsilyl)sulfamidimidoyl)-4-(difluoromethyl)-6,7-dihydrothieno[3,2-c]pyridine-5(4H)-carboxylate

**Step 1: 4-(Difluoromethyl)-4,5,6,7-tetrahydrothieno[3,2-c]pyridine**

To a stirred solution of 2-(thiophen-2-yl)ethan-1-amine (5.00 g, 39.3 mmol) in HCl (6 M, 40 mL) and water (40 mL) was added 1-ethoxy-2,2-difluoroethan-1-ol (5.45 g, 43.2 mmol) dropwise at RT. The reaction mixture was stirred for 12 h at RT and then diluted with 30 mL of water. The pH value of the solution was adjusted to 7 with NaOH (2 M). The resulting solution was extracted with 3x200 mL of EtOAc; the combined organic layers were dried over anhydrous $Na_2SO_4$ and concentrated under vacuum. This resulted in 3 g (40.3%) of the title compound as yellow oil. MS-ESI: 190 (M+1).

**Step 2: Tert-butyl 4-(difluoromethyl)-6,7-dihydrothieno[3,2-c]pyridine-5(4H)-carboxylate**

To a stirred solution of 4-(difluoromethyl)-4,5,6,7-tetrahydrothieno[3,2-c]pyridine (2.00 g, 10.6 mmol) in DCM (20 mL) was added TEA (2.14 g, 21.2 mmol) at RT, followed by the addition of $(Boc)_2O$ (3.46 g, 15.9 mmol) in 10 mL DCM dropwise at RT. The resulting solution was stirred for 2 h at RT. The reaction was quenched with water 30 mL, and then extracted with EtOAc (3x50 mL). The combined organic layers were dried over anhydrous $Na_2SO_4$ and concentrated under vacuum. The residue was eluted from a silica gel column with EtOAc/PE (1:10). This resulted in 2.20 g (71.9%) of the title compound as yellow oil. MS-ESI: 290 (M+1).

**Step 3: Tert-butyl 4-(difluoromethyl)-2-sulfamoyl-6,7-dihydrothieno[3,2-c]pyridine-5(4H)-carboxylate**

To a stirred solution of tert-butyl 4-(difluoromethyl)-6,7-dihydrothieno[3,2-c]pyridine-5(4H)-carboxylate (2.20 g, 7.60 mmol) in THF (20 mL) under nitrogen was added n-BuLi (2.5 M in hexane, 6.1 mL, 15.2 mmol) dropwise at -78 °C and stirred for 30 min at -78 °C. To the above solution was bubbled $SO_2$ (g) for 15 min at -50 °C. The reaction mixture was stirred for 1 h at RT, and then concentrated under vacuum. To the above residue was added DCM (20 mL), followed by NCS (1.52 g, 11.4 mmol) in small portions at 0 °C. The reaction mixture was stirred for 1 h, and then $NH_3$ (g) was bubbled for 15 min at 0 °C. The resulting mixture was further stirred for 2 h at RT. The reaction was quenched with water (50 mL) and extracted with DCM (3x100 mL). The combined organic layers were dried over anhydrous $Na_2SO_4$ and concentrated under reduced pressure. The crude product was purified by Prep-HPLC with the following conditions: Column, XBridge Shield RP18 OBD, 30*150 mm, 5 um; mobile phase, Water (10 mM $NH_4HCO_3$+0.1%$NH_3 \cdot H_2O$) and ACN (35% to 40% Phase B over 7 min); Detector, UV 254 nm. This resulted in 500 mg (19.6%) of the title compound as a yellow solid. MS-ESI: 369 (M+1).

Steps 4-5 used similar procedures for converting compound **639"** to Intermediate **183** shown in Scheme 128 to afford Intermediate **214** from compound **815".** MS-ESI: 482 (M+1).

**Step6:(S)and(R)tert-butyl4-(difluoromethyl)-2-sulfamoyl-6,7-dihydrothieno[3,2-c]pyridine-5(4H)-carboxylate**

Compound **815"** (120 mg) was resolved by chiral prep-HPLC using the following conditions: Column CHIRALPAK IA, 3*25 cm, 5 um; Mobile Phase A: Hex (8 mM $NH_3 \cdot$MeOH), Mobile Phase B: EtOH; Flow rate: 40 mL/min; Gradient: 30% B to 30% B over 12 min; 254/220 nm; RT1: 7.85 min (Compound 815F"); RT2: 9.3 min (Compound 815S"). This resulted in 25.5 mg of Compound **815F"** and 27.0 mg of Compound **815S";** both as white solid. MS-ESI: 369 (M+1). The stereochemistry assignments of **815F"** and **815F"** are arbitrary.

**Table 74** The intermediates in the following table were prepared using the similar procedures for converting compound **815"** to Intermediate **214** shown in Scheme 157 starting from **815F"** and **815S"..**

| Intermediate # | Structure | IUPAC Name | Exact Mass [M+H]+ |
|---|---|---|---|
| **Intermediate 214F** | 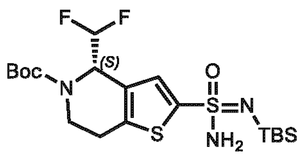 | (S) or (R) tert-butyl 2-(N'-(tert-butyldimethylsilyl)sulfamidimidoyl)-4-(difluoromethyl)-6,7-dihydrothieno[3,2-c] pyridine-5(4H)-carboxylate | 482 |
| **Intermediate 214S** | 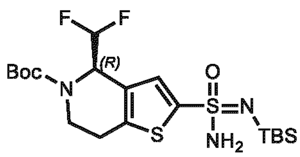 | (R) or (S) tert-butyl 2-(N'-(tert-butyldimethylsilyl)sulfamidimidoyl)-4-(difluoromethyl)-6,7-dihydrothieno[3,2-c] pyridine-5(4H)-carboxylate | 482 |

## Scheme 158:

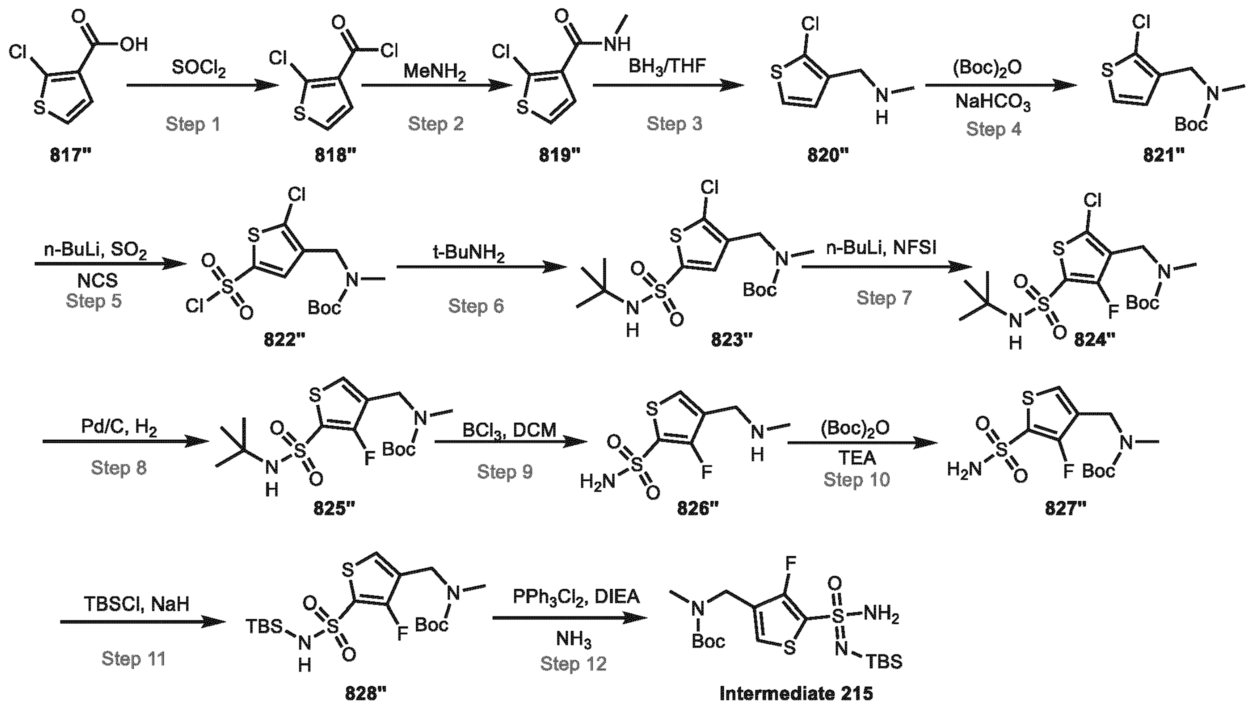


## Intermediate 215

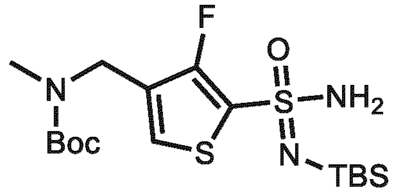

Tert-butyl ((5-(N'-(tert-butyldimethylsilyl)sulfamidimidoyl)-4-fluorothiophen-3-yl)methyl)(methyl)carbamate

**Step 1: 2-Chlorothiophene-3-carbonyl chloride**

To a stirred solution of 2-chlorothiophene-3-carboxylic acid (5.00 g, 30.9 mmol) in DCM (300 mL) under nitrogen was added $SOCl_2$ (12.3 g, 104 mmol) dropwise at 0 °C. The resulting solution was stirred for 1 h at RT. The resulting mixture was concentrated under vacuum. This resulted in 5.00 g (crude) of the title compound as a yellow solid.

**Step 2: 2-Chloro-N-methylthiophene-3-carboxamide**

To a stirred solution of methanamine (2 M in THF, 150 mL, 300 mmol) under nitrogen was added 2-chlorothiophene-3-carbonyl chloride (5.00 g, crude from last step) in THF (10 mL) dropwise at 0 °C. The resulting solution was stirred for 16 h at RT. The resulting mixture was concentrated under vacuum. The residue was eluted from a silica gel column with EtOAc/PE (1:1). This resulted in 3 g (55.6% over 2 steps) of the title compound as a white solid. MS-ESI: 176/178 (M+1).

**Step 3: 1-(2-Chlorothiophen-3-yl)-N-methylmethanamine**

To a stirred solution of 2-chloro-N-methylthiophene-3-carboxamide (3.00 g, 17.1 mmol) in THF (150 mL) under nitrogen was added $BH_3$-THF (1 M, 34.2 mL, 34.2 mmol) dropwise at 0 °C. The resulting solution was stirred for 16 h at RT, then quenched with 10 mL of MeOH. The resulting mixture was concentrated. The residue was eluted from a silica gel column with EtOAc/PE (1:1). This resulted in 2.0 g (72.5%) of the title compound as a white solid. MS-ESI: 162/164 (M+1).

**Step 4: Tert-butyl ((2-chlorothiophen-3-yl)methyl)(methyl)carbamate**

To a stirred solution of 1-(2-chlorothiophen-3-yl)-N-methylmethanamine (2.00 g, 12.4 mmol) in dioxane (100 mL) and water (10 mL) under nitrogen was added $NaHCO_3$ (3.12 g, 37.1 mmol) in portions at RT, followed by the addition of $(Boc)_2O$ (4.05 g, 18.6 mmol) in dioxane (10 mL) dropwise at RT. The resulting solution was stirred for 16 h at RT. The resulting mixture was diluted with 50 ml of water, and extracted with 3x50 mL of DCM. The combined organic layers were dried over anhydrous $Na_2SO_4$. The resulting mixture was concentrated under vacuum. The residue was eluted from a silica gel column with EtOAc/PE (1:1). This resulted in 2.5 g (77.2%) of the title compound as a white solid. MS-ESI: 262/264 (M+1).

**Step 5: Tert-butyl ((2-chloro-5-(chlorosulfonyl)thiophen-3-yl)methyl)(methyl)carbamate**

To a stirred solution of tert-butyl ((2-chlorothiophen-3-yl)methyl)(methyl)carbamate (2.50 g, 9.58 mmol) in THF (100 mL) under nitrogen was added n-BuLi (2.5 M in hexane, 9.60 mL, 24.0 mmol) dropwise at -78 °C, the resulting solution was stirred for 1 h at -78 °C. To the above solution was bubbled $SO_2$ (g) at -50 °C for 15 min. The resulting solution was stirred for 1 h at RT. The resulting mixture was concentrated under vacuum. To the above residue was added DCM (50 mL), followed was added NCS (2.55 g, 19.2 mmol) in small portions at 0 °C. The resulting mixture was stirred for 2 h at RT, then concentrated under vacuum. This resulted in 3.00 g (crude) of the title compound as a yellow solid. This compound was monitored by LC-MS after adding 7 M $MeOH/NH_3$ to an aliquot of the reaction mixture, MS-ESI: 341/343 (M+1).

**Step 6: Tert-butyl ((5-(N-(tert-butyl)sulfamoyl)-2-chlorothiophen-3-yl)methyl)(methyl)carbamate**

To a stirred solution of tert-butyl ((2-chloro-5-(chlorosulfonyl)thiophen-3-yl)methyl)(methyl)carbamate (3.00 g, crude from last step) in DCM (100 mL) under nitrogen was added *t*-$BuNH_2$ (1.02 g, 13.8 mmol) dropwise at 0 °C. The resulting solution was stirred for 16 h at RT. The resulting mixture was diluted with 100 mL of water and extracted with 3x100 mL of DCM. The combined organic layers were dried over anhydrous $Na_2SO_4$. The resulting mixture was concentrated under vacuum. The residue was eluted from a silica gel column with EtOAc/PE (1:1). This resulted in 1.80 g (45.3% over 2 steps) of the title compound as a white solid. MS-ESI: 397/399 (M+1).

**Step 7: Tert-butyl ((5-(N-(tert-butyl)sulfamoyl)-2-chloro-4-fluorothiophen-3-yl)methyl)(methyl)carbamate**

To a stirred solution of tert-butyl ((5-(N-(tert-butyl)sulfamoyl)-2-chlorothiophen-3-yl)methyl)(methyl)- carbamate (1.80 g, 4.55 mmol) in THF (100 mL) under nitrogen was added n-BuLi (2.5 M in hexane, 3.64 mL, 9.10 mmol) dropwise at -78 °C. The resulting solution was stirred for 1 h at -78 °C. NFSI (2.87 g, 9.10 mmol) in THF (15 mL) was added dropwise at -78 °C under nitrogen. The resulting solution was stirred for 16 h at RT. The reaction mixture was quenched with 20 mL of water, extracted with 3x100 mL of EtOAc. The combined organic layers were dried over anhydrous $Na_2SO_4$ and concentrated under vacuum. The residue was eluted from silica gel with EtOAc/PE (1:1). This resulted in 1.2 g (63.8%) of the title compound as a white solid. MS-ESI: 415/417 (M+1).

**Step 8: Tert-butyl ((5-(N-(tert-butyl)sulfamoyl)-4-fluorothiophen-3-yl)methyl)(methyl)carbamate**

To a stirred solution of tert-butyl ((5-(N-(tert-butyl)sulfamoyl)-2-chloro-4-fluorothiophen-3-yl)methyl)- (methyl)carbamate (1.2 g, 2.90 mmol) in MeOH (100 mL) under nitrogen was added Pd/C (10%wt., 240 mg) in portions at RT. The flask was evacuated and flushed with hydrogen 3 times. The resulting solution was stirred 16 h at RT under hydrogen with a balloon. The Pd/C catalysts were filtered out, the filtrate was concentrated under vacuum. The residue was eluted from silica gel with EtOAc/PE (1:1). This resulted in 700 mg (63.6%) of the title compound as a white solid. MS-ESI: 381 (M+1).

**Step 9: 3-Fluoro-4-((methylamino)methyl)thiophene-2-sulfonamide**

To a stirred solution of tert-butyl ((5-(N-(tert-butyl)sulfamoyl)-4-fluorothiophen-3-yl)methyl)(methyl)- carbamate (700 mg, 1.84 mmol) in DCM (10 mL) was added $BCl_3$ (1M in DCM, 5 mL, 5 mmol) dropwise at 0 °C. The resulting solution was stirred for 16 h at RT. The reaction was then quenched by the addition of 10 mL of MeOH and concentrated under vacuum. This resulted in 450 mg (crude) of the title compound as yellow oil which was used in next step without further purification. MS-ESI: 225 (M+1).

**Step 10: Tert-butyl ((4-fluoro-5-sulfamoylthiophen-3-yl)methyl)(methyl)carbamate**

To a stirred solution of 3-fluoro-4-((methylamino)methyl)thiophene-2-sulfonamide (450 mg, crude from last step) in DCM (10 mL) was added TEA (362 mg, 3.58 mmol), followed by $(Boc)_2O$ (781 mg, 3.58 mmol) in DCM (5 mL) dropwise at RT. The resulting solution was stirred for 16 h at RT. The resulting solution was extracted with 3x100 mL of EtOAc and the organic layers were combined, dried over anhydrous $Na_2SO_4$, and concentrated under vacuum. The residue was purified by Prep-TLC (PE/EtOAc 1:1). This resulted in 500 mg (83.7% over 2 steps) of the title compound as yellow oil. MS-ESI: 325 (M+1).

Steps 11-12 used similar procedures for converting compound **639"** to Intermediate **183** shown in Scheme **128** to afford Intermediate **215** from compound **827"**. MS-ESI: 438 (M+1).

## Scheme 159:

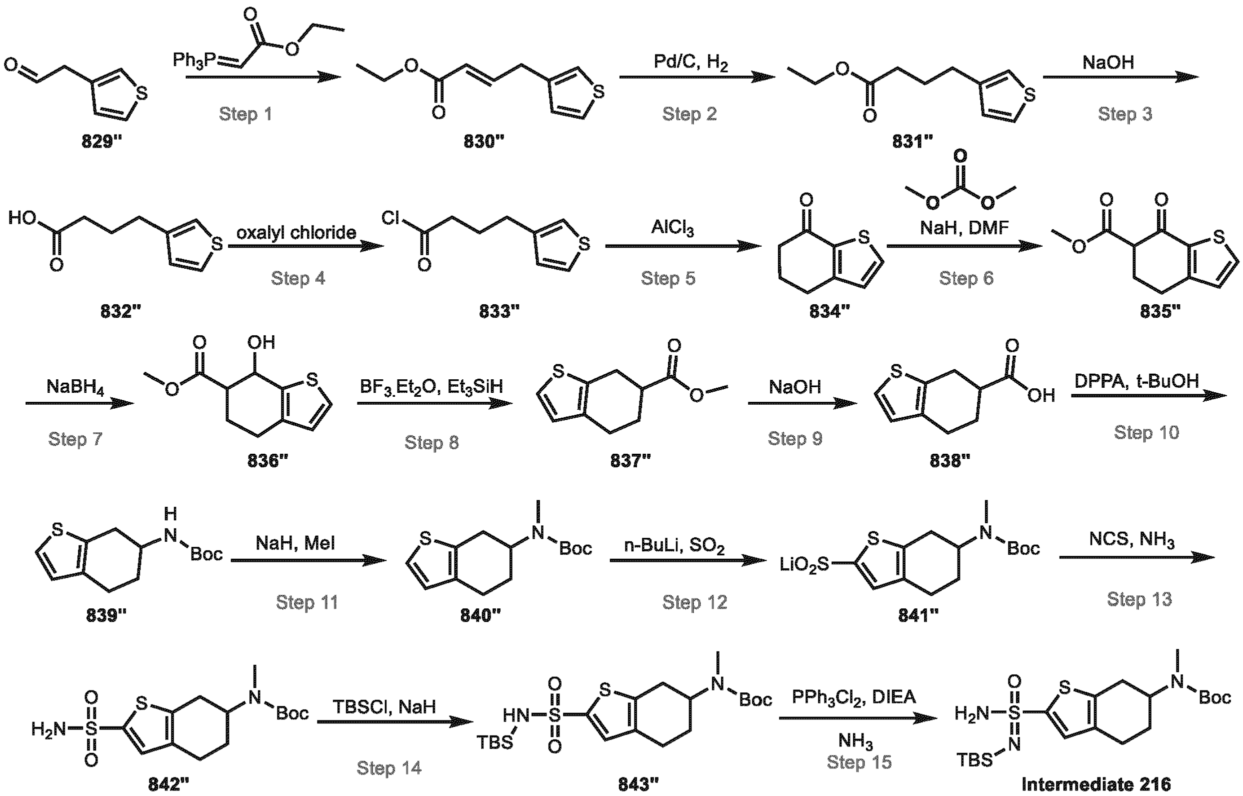


## Intermediate 216

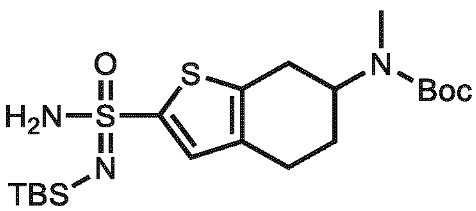

Tert-butyl (2-(N'-(tert-butyldimethylsilyl)sulfamidimidoyl)-4,5,6,7-tetrahydrobenzolb1thiophen-6-yl)(methyl)carbamate

**Step 1: Ethyl *(E)*-4-(thiophen-3-yl)but-2-enoate**

To a stirred solution of 2-(thiophen-3-yl)acetaldehyde (5.00 g, 39.7 mmol) in THF (100 mL) under nitrogen was added ethyl 2-(triphenyl-$\lambda^5$-phosphaneylidene)acetate (13.8 g, 39.7 mmol) in THF (10 mL) dropwise at 0 °C. The resulting solution was stirred for 3 h at RT. The resulting solution was extracted with 5x100 mL of EtOAc and the organic layers

combined and dried over anhydrous $Na_2SO_4$ and concentrated under vacuum. The residue was eluted from silica gel with EtOAc/PE (1:4). This resulted in 4.25 g (54.6%) of the title compound as yellow oil. GC-MS-EI: 196 (M).

**Step 2: Ethyl 4-(thiophen-3-yl)butanoate**

To a stirred solution of ethyl *(E)*-4-(thiophen-3-yl)but-2-enoate (3.00 g, 15.3 mmol) in MeOH (50 mL) under nitrogen was added Pd/C (10%wt., 240 mg) in portions at RT. The flask was evacuated and flushed with hydrogen 3 times. The resulting solution was stirred 16 h at RT under hydrogen with a balloon. The Pd/C catalysts were filtered out, the filtrate was concentrated under vacuum. The residue was eluted from a silica gel column with EtOAc/PE (1:4). This resulted in 2.15 g (71.0%) of the title compound as yellow oil. GC-MS-EI: 198 (M).

**Step 3: 4-(Thiophen-3-yl)butanoic acid**

To a stirred solution of ethyl 4-(thiophen-3-yl)butanoate (5.00 g, 25.3 mmol) in MeOH (50 mL) was added aq. NaOH (4 M, 30 mL) dropwise at 0 °C. The resulting mixture was stirred for 16 h at RT. The pH value of the mixture was adjusted to 5 with HCl (6 M). The mixture was extracted with EtOAc (3x300 mL). The organic layers were combined and dried over anhydrous $Na_2SO_4$ and concentrated under vacuum. The residue was eluted from silica gel with PE/EtOAc (1:1). This resulted in (4.08 g, 95%) of the title compound as yellow oil. MS-ESI: 169 (M-1).

**Step 4: 4-(Thiophen-3-yl)butanoyl chloride**

To a stirred solution of 4-(thiophen-3-yl)butanoic acid (4.70 g, 27.6 mmol) in DCM (60 mL) and DMF (cat., 0.1 mL) under nitrogen was added oxalyl chloride (6.96 g, 55.2 mmol) dropwise at 0 °C. The resulting mixture was stirred for 2 h at RT. The resulting mixture was concentrated under vacuum. The crude product was used in next step without further purification. This compound was monitored by LC-MS after adding 7M MeOH/$NH_3$ to an aliquot of the reaction mixture MS-ESI: 170 (M+1).

**Step 5: 5,6-Dihydrobenzo[b]thiophen-7(4H)-one**

To a stirred solution of 4-(thiophen-3-yl)butanoyl chloride (6.00 g, 31.9 mmol) in DCM (100 mL) under nitrogen was added anhydrous $AlCl_3$ (8.48 g, 64.2 mmol) in portions at 0 °C. The resulting solution was stirred for 2 h at RT, then quenched with 300 mL of water/ice. The resulting solution was extracted with 3x300 mL of DCM and the organic layers were combined and concentrated under vacuum. This resulted in 4.2 g (86.6% over 2 steps) of the title compound as brown oil. MS-ESI: 153 (M+1).

**Step 6: Methyl 7-oxo-4,5,6,7-tetrahydrobenzo[b]thiophene-6-carboxylate**

To a stirred solution of 5,6-dihydrobenzo[b]thiophen-7(4H)-one (2.80 g, 18.4 mmol) in DMF (20 mL) under nitrogen was added NaH (60%wt. dispersion in mineral oil, 1.68 g, 42.0 mmol) in portions at 0 °C. The resulting solution was stirred for 30 min at 10 °C, then dimethyl carbonate (9.94 g, 110 mmol) was added dropwise at 0 °C. The resulting solution was stirred for 3 h at RT. The reaction was quenched with 10 mL of water. The resulting solution was extracted with 3x20 mL of EtOAc and the organic layers were combined and concentrated under vacuum. The residue was eluted from silica gel with EtOAc/PE (1:5). This resulted in 3.3 g (85.3%) of the title compound as yellow oil. $^1H$ NMR (400 MHz, DMSO-$d_6$) $\delta$ 8.06 (d, *J* = 5.0 Hz, 1H), 7.17 (d, *J* = 5.0 Hz, 1H), 3.81 (dd, *J* = 8.8, 6.3 Hz, 1H), 3.69 (s, 3H), 3.02-2.85 (m, 2H), 2.40-2.25 (m, 2H).

**Step 7: Methyl 7-hydroxy-4,5,6,7-tetrahydrobenzo[b]thiophene-6-carboxylate**

To a stirred solution of methyl 7-oxo-4,5,6,7-tetrahydrobenzo[b]thiophene-6-carboxylate (5.00 g, 23.8 mmol) in THF (30 mL) and MeOH (20 mL) was added $NaBH_4$ (0.90 g, 23.7 mmol) in portions at 0 °C. The resulting solution was stirred for 3 h at RT. The reaction mixture was quenched with 10 mL of water, organic solvent was removed under vacuum, then the residue was extracted with EtOAc (3x20 mL). The organic layers were combined and concentrated under vacuum. The residue was eluted from silica gel with EtOAc/PE (3:7). This resulted in 1.8 g (35.7%) of the title compound as yellow oil. MS-ESI: 213 (M+1).

**Step 8: Methyl 4,5,6,7-tetrahydrobenzo[b]thiophene-6-carboxylate**

To a stirred solution of methyl 7-hydroxy-4,5,6,7-tetrahydrobenzo[b]thiophene-6-carboxylate (1.40 g, 6.60

mmol) in DCM (40 mL) was added $Et_3SiH$ (1.53 g, 13.2 mmol) and $BF_3$-$Et_2O$ (47%wt. in $Et_2O$, 3.99 g, 13.2 mmol) dropwise at 0 °C. The resulting solution was stirred for 16 h at 0 °C and then quenched with sat. $NaHCO_3$ (10 mL) and extracted with EtOAc (3x30 mL). The organic layers were combined, dried over anhydrous $Na_2SO_4$, and concentrated under vacuum. The residue was eluted from silica gel with EtOAc/PE (30:70). This resulted in 1.2 g (92.7%) of the title compound as nearly colorless oil. MS-ESI: 197 (M+1).

**Step 9: 4,5,6,7-Tetrahydrobenzo[b]thiophene-6-carboxylic acid**

To a stirred solution of methyl 4,5,6,7-tetrahydrobenzo[b]thiophene-6-carboxylate (1.20 g, 6.12 mmol) in MeOH (50 mL) was added NaOH (4 M, 10 mL, 40 mmol) dropwise at RT. The resulting solution was stirred for 1 h at RT. The pH value of the mixture was adjusted to 6 with HCl (6 M) and extracted with 3x80 mL of EtOAc. The organic layers were combined, dried over anhydrous $Na_2SO_4$, and concentrated under vacuum. This resulted in 1.10 g (98.7%) of the title compound as brown oil. MS-ESI: 181 (M-1).

**Step 10: Tert-butyl (4,5,6,7-tetrahydrobenzo[b]thiophen-6-yl)carbamate**

To a stirred solution of 4,5,6,7-tetrahydrobenzo[b]thiophene-6-carboxylic acid (500 mg, 2.75 mmol) in toluene (20 mL) under nitrogen was added TEA (306 mg, 3.03 mmol) and t-BuOH (407 mg, 5.50 mmol) at RT. DPPA (831 mg, 3.02 mmol) in toluene (5 mL) was added dropwise at RT. The resulting solution was stirred for 16 h at 90 °C. The reaction mixture was concentrated under vacuum. The residue was eluted from silica gel with EtOAc/PE (5:95). This resulted in 540 mg (77.7%) of the title compound as yellow oil. MS-ESI: 254 (M+1).

**Step 11: Tert-butyl methyl(4,5,6,7-tetrahydrobenzo[b]thiophen-6-yl)carbamate**

To a stirred solution of tert-butyl (4,5,6,7-tetrahydrobenzo[b]thiophen-6-yl)carbamate (1.00 g, 3.95 mmol) in THF (20 mL) under nitrogen was added NaH (60%wt. dispersion in mineral oil, 395 mg, 9.87 mmol) in portions at 0 °C, followed by the addition of MeI (2.81 g, 19.8 mmol) dropwise at 0 °C. The resulting solution was stirred for 16 h at RT. The reaction mixture was quenched with 5 mL of water. The resulting solution was further diluted with 30 mL of water and extracted with 3x80 mL of EtOAc. The organic layers were combined and concentrated under vacuum. This resulted in 1.02 g (96.6%) of the title compound as yellow oil. MS-ESI: 268 (M+1).

Steps 12-15 used similar procedures for converting compound **814"** to Intermediate **214** shown in Scheme **157** to afford Intermediate **216** from compound **840".** MS-ESI: 460 (M+1).

## Scheme 160:

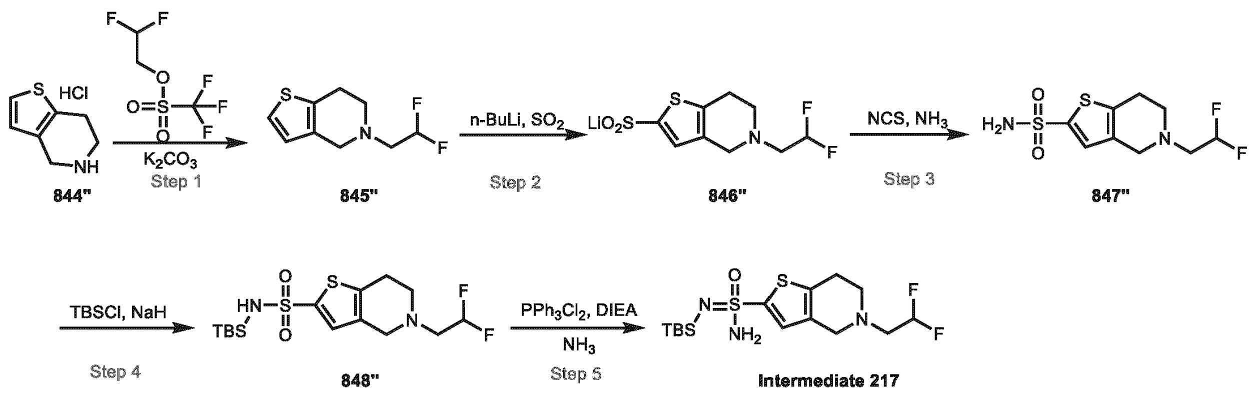


## Intermediate 217

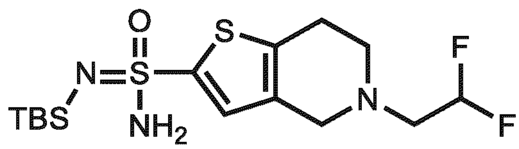

N'-(tert-butyldimethylsilyl)-5-(2,2-difluoroethyl)-4,5,6,7-tetrahydrothieno[3,2-c]pytridine-2-sulfonimidamide

**Step 1: 5-(2,2-Difluoroethyl)-4,5,6,7-tetrahydrothieno[3,2-c]pyridine**

To a stirred solution of 4,5,6,7-tetrahydrothieno[3,2-c]pyridine hydrochloride (5.0 g, 28.5 mmol) in isopropyl alcohol (200 mL) was added $K_2CO_3$ (9.83 g, 71.3 mmol) in portions and 2,2-difluoroethyl trifluoromethanesulfonate (12.2 g, 57.0 mmol) dropwise at RT. The resulting solution was stirred for 3 h at 65 °C. The reaction was quenched with 10 mL of water and extracted with 3x150 mL of DCM. The organic layers were combined, dried over anhydrous $Na_2SO_4$, and concentrated under vacuum. The residue was eluted from silica gel with EtOAc/PE (1:1). This resulted in 2.7 g (46.7%) of the title compound as yellow oil. MS-ESI: 204 (M+1). $^1H$ NMR (400 MHz, DMSO-$d_6$) δ 7.26 (d, *J* = 5.1 Hz, 1H), 6.77 (d, *J* = 5.1 Hz, 1H), 6.18 (tt, *J* = 55.8, 4.3 Hz, 1H), 3.64-3.59 (m, 2H), 2.97-2.81 (m, 4H), 2.81- 2.74 (m, 2H).

Steps 2-5 used similar procedures for converting compound **814"** to Intermediate **214** shown in Scheme **157** to afford Intermediate **217** from compound **845".** MS-ESI: 396 (M+1).

Compound **847"** $^1H$ NMR (300 MHz, DMSO-$d_6$) δ 7.58 (s, 2H), 7.24 (s, 1H), 6.19 (tt, *J* = 55.8, 4.3 Hz, 1H), 3.64-3.59 (m, 2H), 3.03-2.78 (m, 6H).

## Scheme 161:

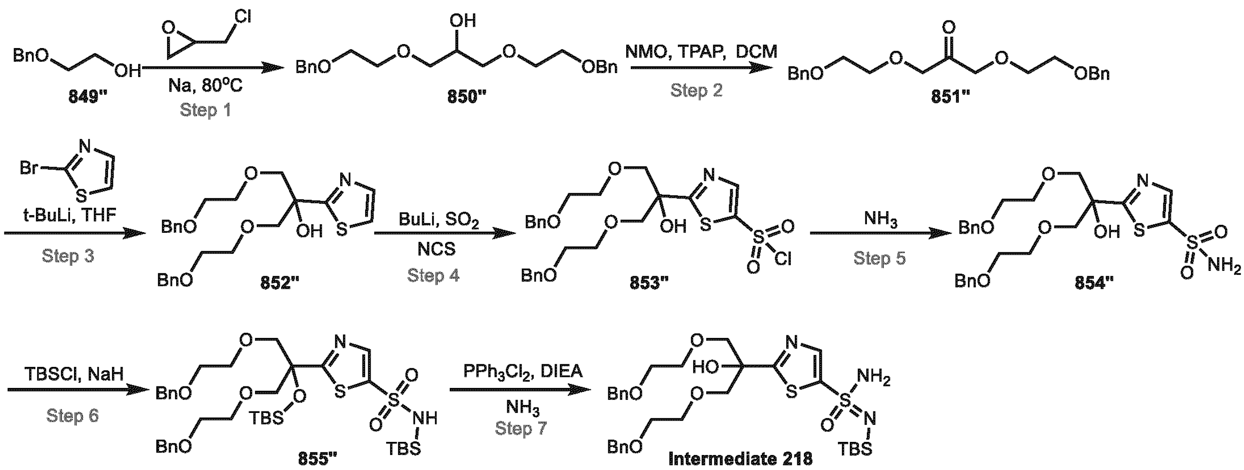


849" 850" 851" 852" 853" 854" 855" Intermediate 218

## Intermediate 218

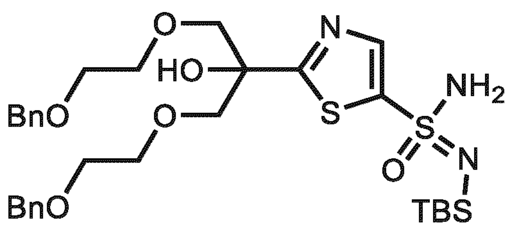

N'-(tert-butyldimethylsilyl)-2-(7-hydroxy-1,13-diphenyl-2,5,9,12-tetraoxatridecan-7-yl)thiazole-5-sulfonimidamide

**Step 1: 1,13-Diphenyl-2,5,9,12-tetraoxatridecan-7-ol**

To a stirred solution of 2-(benzyloxy)ethan-1-ol (26.0 mL, 183 mmol) under nitrogen was added Na (2.20 g, 95.7 mmol) at 100 °C, followed by the addition of 2-(chloromethyl)oxirane (2.40 mL, 30.8 mmol) dropwise at 80 °C for 16 h. The reaction was quenched with MeOH (20 mL) at 0 °C. The resulting mixture concentrated and diluted with 20 mL of water, then extracted with EtOAc (3x20 mL). The combined organic layers were washed with brine (2x20 mL), dried over anhydrous $Na_2SO_4$, and concentrated under vacuum. The residue was purified by RP-HPLC using the following conditions: column, $C_{18}$ silica gel; mobile phase, 10 mM $NH_4HCO_3$ in water and ACN (10% ACN to 60% gradient over 30 min); detector, UV 210 nm. This resulted in 5.60 g (50.5%) of the title compound as a dark yellow oil. $^1H$ NMR (300 MHz, DMSO-$d_6$) δ 7.48-7.12 (m, 10H), 4.75 (d, *J* = 5.2 Hz, 1H), 4.48 (s, 4H), 3.80-3.65 (m, 1H), 3.60-3.50 (m, 8H),

3.46-3.28 (m, 4H).

**Step 2: 1,13-Diphenyl-2,5,9,12-tetraoxatridecan-7-one**

To a stirred solution of 1,13-diphenyl-2,5,9,12-tetraoxatridecan-7-ol (2.70 g, 7.50 mmol) in DCM (20 mL) was added NMO (1.76 g, 15.0 mmol) and TPAP (0.527 g, 1.50 mmol) dropwise at RT. The resulting solution was stirred for 3 h at RT. The reaction was quenched with water (20 mL) and then extracted with 3x20 mL of EtOAc. The organic layers were combined, dried over anhydrous $Na_2SO_4$, and concentrated under vacuum. The residue was purified by RP-HPLC with the following conditions: column, $C_{18}$ silica gel; mobile phase, 10 mM $NH_3HCO_3$ in water and ACN (5% ACN to 55% gradient over 30 min); detector, UV 220 nm. This resulted in 800 mg (29.8%) of the title compound as light yellow oil. $^1H$ NMR (400 MHz, DMSO-$d_6$) δ 7.55-7.15 (m, 10H), 4.50 (s, 4H), 4.25 (s, 4H), 3.65-3.50 (m, 8H).

**Step 3: 1,13-Diphenyl-7-(thiazol-2-yl)-2,5,9,12-tetraoxatridecan-7-ol**

To a stirred solution of 2-bromothiazole (3.00 g, 18.3 mmol) in THF (30 mL) under nitrogen was added t-BuLi (1.3 M in n-pentane, 15.4 mL, 20.1 mmol) dropwise at -78 °C, followed by the addition of 1,13-diphenyl-2,5,9,12-tetraoxatridecan-7-one (6.55 g, 18.3 mmol) dropwise at -78 °C. The resulting mixture was stirred for 3 h at -78 °C. The reaction was quenched with water (10 mL). The resulting mixture was extracted with EtOAc (3x30 mL). The combined organic layers were washed with brine (3x20 mL), dried over anhydrous $Na_2SO_4$, and concentrated under vacuum. The residue was eluted from silica gel column with PE/EtOAC (1:1) to afford 3.7 g (46.7%) the title compound as a light yellow oil. MS-ESI: 444 (M+1). $^1H$ NMR (300 MHz, DMSO-$d_6$) δ 7.72 (d, *J* = 3.2 Hz, 1H), 7.61 (d, *J* = 3.2 Hz, 1H), 7.38-7.20 (m, 10H), 5.96 (s, 1H), 4.43 (s, 4H), 3.85-3.69 (m, 4H), 3.62-3.39 (m, 8H).

Steps 4-7 used similar procedures for converting compound **814"** to Intermediate **214** shown in Scheme **157** to afford Intermediate **218** from compound **852"**. MS-ESI: 636 (M+1).

**Scheme 162:**

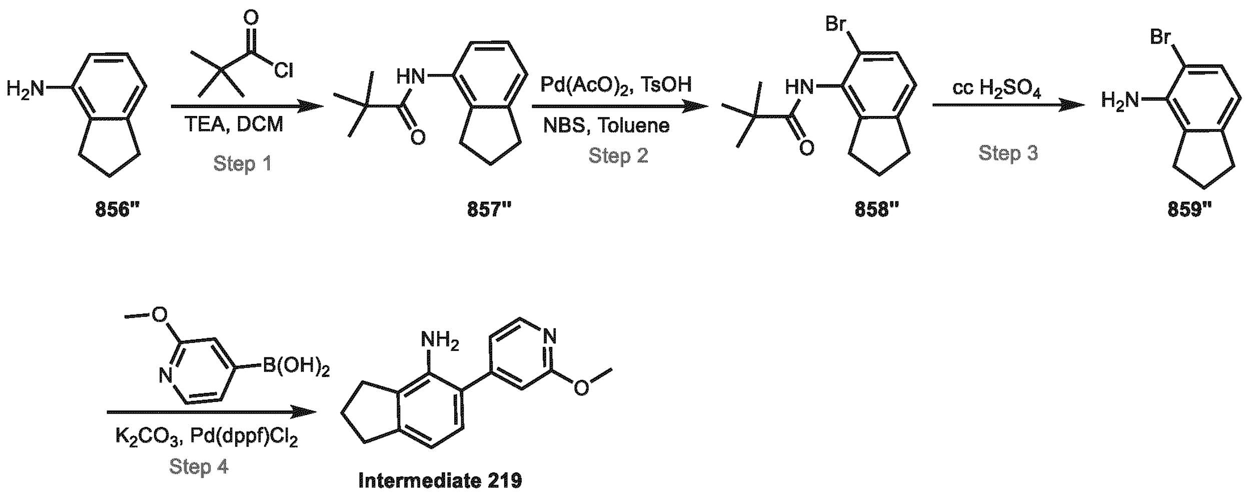


Intermediate 219

## Intermediate 219

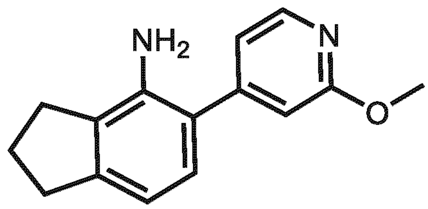

5-(2-Methoxypyridin-4-yl)-2,3-dihydro-1H-inden-4-amine

**Step 1: N-(2,3-dihydro-1H-inden-4-yl)pivalamide**

To a stirred solution of 2,3-dihydro-1H-inden-4-amine (7.00 g, 52.6 mmol) in THF (200 mL) was added TEA (7.98 g, 78.8 mmol) at RT. This was followed by the addition of pivaloyl chloride (7.60 g, 63.1 mmol) dropwise by syringe at RT. The reaction mixture was stirred at RT for 6 h. The reaction was quenched with water (200 mL). The resulting solution was extracted with 3x200 mL of DCM, the organic layers were combined, dried over anhydrous $Na_2SO_4$, and concentrated under vacuum. The reaction mixture was eluted from silica gel with PE/EtOAC (1:10). This result in 21 g (95.0%) of the title compound as a brown solid. MS-ESI: 218 (M+1)

**Step 2: N-(5-bromo-2,3-dihydro-1H-inden-4-yl)pivalamide**

To a stirred solution of N-(2,3-dihydro-1H-inden-4-yl)pivalamide (13.5 g, 62.1 mmol) in toluene (200 mL) was added $Pd(OAc)_2$ (0.70 g, 3.11 mmol), NBS (13.3 g, 74.5 mmol) and TsOH (5.33 g, 31.0 mmol) in portions at RT. The resulting mixture was stirred under air for 16 h at RT. The reaction was then quenched with aq. $NaHCO_3$ (sat., 20 mL). The resulting solution was extracted with 3x20 mL of EtOAc. The organic layers were combined, washed with brine (2x20 mL), dried over anhydrous $Na_2SO_4$, and concentrated under vacuum. The residue was eluted from a silica gel column with EtOAc/PE (1 :20). This resulted in 12.0 g (65.0%) of the title compound as a light yellow solid. MS-ESI: 296/298 (M+1). $^1H$ NMR (400 MHz, DMSO-$d_6$) $\delta$ 9.09 (s, 1H), 7.40 (d, $J$ = 7.9 Hz, 1H), 7.07 (d, $J$ = 8.0 Hz, 1H), 2.87 (t, $J$ = 7.4 Hz, 2H), 2.70 (t, $J$ = 7.5 Hz, 2H), 2.08-1.92 (m, 2H), 1.23 (s, 9H).

**Step 3: 5-Bromo-2,3-dihydro-1H-inden-4-amine**

To a stirred solution of N-(5-bromo-2,3-dihydro-1H-inden-4-yl)pivalamide (2.00 g, 6.75 mmol) in MeOH (10 mL) was added $H_2SO_4$ (conc., 10 mL) dropwise at 0 °C. The resulting solution was stirred for 16 h at 100 °C. The reaction mixture was cooled to 0 °C and added dropwise to 100 mL of water carefully. The pH value of the mixture was adjusted to 14 with NaOH (2 M). The resulting mixture was extracted with 3x150 mL of EtOAc, the organic layers were combined and dried over anhydrous $Na_2SO_4$. The residue was eluted from silica gel with EtOAc/PE (1:15). This resulted in 1.16 g (81.0%) of the title compound as a yellow solid. LCMS-ESI: 212 (M+1). $^1H$ NMR (400 MHz, DMSO-$d_6$) $\delta$ 7.11 (d, $J$ = 7.9 Hz, 1H), 6.41 (d, $J$ = 7.9 Hz, 1H), 4.98 (s, 2H), 2.76 (t, $J$ = 7.5 Hz, 2H), 2.70 (t, $J$ = 7.4 Hz, 2H), 2.10-1.90 (m, 2H).

**Step 4: 5-(2-Methoxypyridin-4-yl)-2,3-dihydro-1H-inden-4-amine**

To a stirred solution of 5-bromo-2,3-dihydro-1H-inden-4-amine (1.50 g, 7.07 mmol) in dioxane (30 mL) and water (6 mL) was added $K_2CO_3$ (3.23 g, 23.3 mmol) and (2-methoxypyridin-4-yl)boronic acid (1.62 g, 10.6 mmol) in portions at RT. The mixture was degassed with nitrogen for 15 min before $Pd(dppf)Cl_2$ (517 mg, 0.71 mmol) was added. The resulting mixture was stirred for 2 h at 80 °C under nitrogen. The resulting mixture was diluted with 150 mL of water and extracted with 3x120 mL of EtOAc, the organic layers were combined, dried over anhydrous $Na_2SO_4$, and concentrated under vacuum. The residue was eluted from silica gel with EtOAc /PE (1:10). This resulted in 1.37 g (62.9%) of the title compound as a light yellow solid. LCMS-ESI: 241 (M+1).

**Scheme 163:**

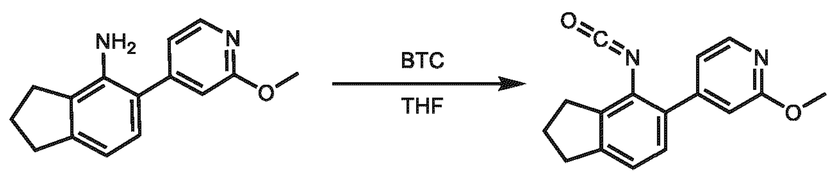


Intermediate 219

Intermediate 220

## Intermediate 220

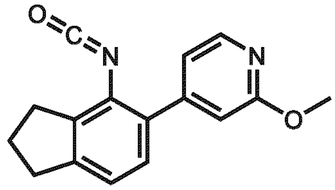

4-(4-Isocyanato-2,3-dihydro-1H-inden-5-yl)-2-methoxypyridine

To a stirred solution of 5-(2-methoxypyridin-4-yl)-2,3-dihydro-1H-inden-4-amine (165 mg, 0.68 mmol) in THF (5 mL) under nitrogen was added BTC (102 mg, 0.34 mmol) in portions at RT. The resulting solution was stirred for 2 h at 80 °C. The resulting mixture was concentrated under vacuum. The crude product was used for next step without purification. This compound was monitored by LC-MS after adding MeOH to an aliquot of the reaction mixture, MS-ESI: 299 (M+1)

**Schemes of Sulfonimidamide and Aniline Intermediates:** Schemes **164-175** illustrate the preparations of sulfonimidamide and aniline intermediates.

## Scheme 164:

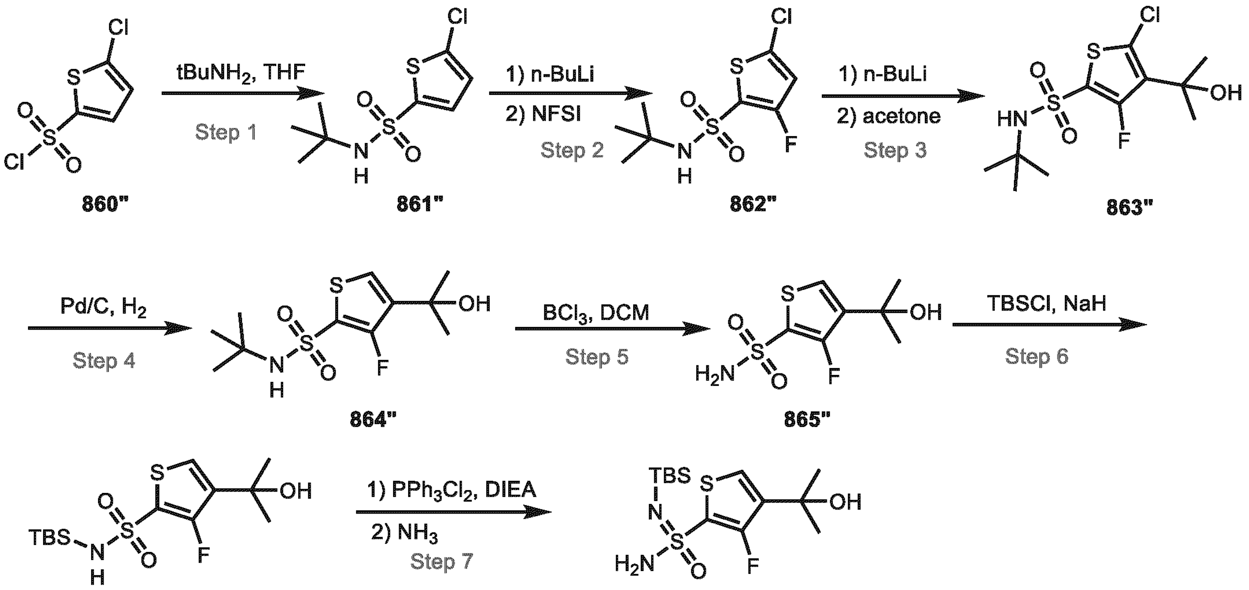


## Intermediate 221

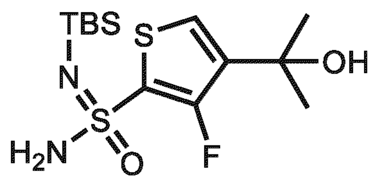

N'-(tert-bulyldimethylsilyl)-3-fluoro-4-(2-hydroxypropan-2-yl)thiophene-2-sulfonimidamide

**Step 1: N-(tert-butyl)-5-chlorothiophene-2-sulfonamide**

To a stirred solution of t-BuNH$_2$ (20.2 g, 276 mmol) in DCM (180 mL) under nitrogen was added TEA (42.0 g,

415 mmol), followed by the addition of 5-chlorothiophene-2-sulfonyl chloride (10.0 g, 46.3 mmol) in DCM (20 mL) dropwise at 0 °C. The resulting solution was stirred for 16 h at 35 °C. The reaction was quenched with water/ice (200 mL) and extracted with DCM (2x200 mL). The combined organic layers were dried over anhydrous $Na_2SO_4$ and concentrated under vacuum. The residue was eluted from a silica gel column with EtOAc/PE (1:10). This resulted in 11.0 g (93.9%) of the title compound as a yellow solid. MS-ESI: 252/254 (M-1). $^1H$ NMR (400 MHz, DMSO-$d_6$) δ 7.90 (s, 1H), 7.46 (d, *J* = 4.0 Hz, 1H), 7.21 (d, *J* = 4.1 Hz, 1H), 1.17 (s, 9H).

**Step 2: N-(tert-butyl)-5-chloro-3-fluorothiophene-2-sulfonamide**

To a stirred solution of N-(tert-butyl)-5-chlorothiophene-2-sulfonamide (11.0 g, 43.5 mmol) in THF (200 mL) under nitrogen was added n-BuLi (2.5 M in hexane, 52.4 mL, 131 mmol) dropwise at -78 °C. The resulting solution was stirred at -78 °C for 1 h. To the above solution was added NFSI (41.6 g, 131 mmol) in THF (100 mL) dropwise at -78 °C. The resulting solution was slowly warmed to RT, and stirred at RT for another 1 h. The reaction mixture was then quenched with water/ice (250 mL) and extracted with EtOAc (3x300 mL). The combined organic layers were dried over anhydrous $Na_2SO_4$ and concentrated under vacuum. The residue was eluted from a silica gel column with EtOAc/PE (1:20). This resulted in 6.2 g (43.3%) of the title compound as a yellow solid. MS-ESI: 270/272 (M-1).

**Step 3: N-(tert-butyl)-5-chloro-3-fluoro-4-(2-hydroxypropan-2-yl)thiophene-2-sulfonamide**

To a stirred solution of N-(tert-butyl)-5-chloro-3-fluorothiophene-2-sulfonamide (6.2 g, 22.8 mmol) in THF (200 mL) under nitrogen was added n-BuLi (2.5 M in hexane, 91.2 mL, 228 mmol) dropwise at -78 °C. The resulting solution was stirred for 50 min at -78 °C. To this was added acetone (53.0 g, 913 mmol) dropwise with stirring at -78 °C. The resulting solution was stirred for additional 1.5 h at -60 °C. The reaction was then quenched with water/ice (200 mL) and extracted with EtOAc (2x300 mL). The combined organic layers were dried over anhydrous $Na_2SO_4$ and concentrated under vacuum. The residue was eluted from a silica gel column with EtOAc/PE (1:5). This resulted in 4.4 g (65.4 %) of the title compound as yellow oil. MS-ESI: 328/330 (M-1).

**Step 4: N-(tert-butyl)-3-fluoro-4-(2-hydroxypropan-2-yl)thiophene-2-sulfonamide**

To a stirred solution of N-(tert-butyl)-5-chloro-3-fluoro-4-(2-hydroxypropan-2-yl)thiophene-2-sulfonamide (4.3 g, 13.1 mmol) in MeOH (80 mL) under nitrogen was added Pd/C (10% wt, 470 mg) in portions at RT. The flask was evacuated and refilled with hydrogen three times. The resulting mixture was stirred for 16 h at RT under hydrogen with a balloon. The solids were filtered out and the filtrate was concentrated under vacuum. This resulted in 3.88 g (crude) of the title compound as a white solid. MS-ESI: 294 (M-1).

**Step 5: 3-Fluoro-4-(2-hydroxypropan-2-yl)thiophene-2-sulfonamide**

To a stirred solution of N-(tert-butyl)-3-fluoro-4-(2-hydroxypropan-2-yl)thiophene-2-sulfonamide (3.88 g, 13.1 mmol) in DCM (78 mL) under nitrogen was added $BCl_3$ (1 M in DCM, 39 mL, 39 mmol) dropwise at 0 °C. The resulting solution was stirred for 3 h at RT. The reaction was then quenched with water/ice (80 mL) and extracted with EtOAc (2x100 mL). The combined organic layers were dried over anhydrous $Na_2SO_4$ and concentrated under vacuum. The residue was eluted from a silica gel column with EtOAc/PE (2:3). This resulted in 1.9 g (60.7% over 2 steps) of the title compound as yellow oil. MS-ESI: 238 (M-1).

**Step 6: N-(tert-butyldimethylsilyl)-3-fluoro-4-(2-hydroxypropan-2-yl)thiophene-2-sulfonamide**

To a stirred solution of 3-fluoro-4-(2-hydroxypropan-2-yl)thiophene-2-sulfonamide (1.89 g, 7.91 mmol) in THF (40 mL) under nitrogen was added NaH (60% wt., 630 mg, 15.9 mmol) in portions at 0 °C, followed by the addition of TBSCl (2.39 g, 15.9 mmol) in THF (5 mL) dropwise with stirring at 0 °C. The resulting solution was stirred for 2 h at RT. The reaction was quenched with water/ice (50 mL). The resulting solution was extracted with EtOAc (2x50 mL). The organic layers were combined and dried over anhydrous $Na_2SO_4$. The resulting solution was concentrated under vacuum. The residue was eluted from a silica gel column with EtOAc/PE (1:3). This resulted in 2.1 g (75.2%) of the title compound as a white solid. MS-ESI: 352 (M-1). $^1H$ NMR (400 MHz, DMSO-$d_6$) δ 8.13 (s, 1H), 7.56 (d, *J* = 5.1 Hz, 1H), 5.38 (s, 1H), 1.43 (s, 6H), 0.89 (s, 9H), 0.16 (s, 6H).

**Step 7: N'-(tert-butyldimethylsilyl)-3-fluoro-4-(2-hydroxypropan-2-yl)thiophene-2-sulfonimidamide**

To a stirred solution of $PPh_3Cl_2$ (1.41 g, 4.24 mmol) in $CHCl_3$ (15 mL) under nitrogen was added DIEA (1.83

g, 14.1 mmol) dropwise at 0 °C. The resulting solution was stirred for 15 min at 0 °C. To the above solution was added N-(tert-butyldimethylsilyl)-2-(difluoromethyl)thiazole-5-sulfonamide (1.0 g, 2.83 mmol) in $CHCl_3$ (10 mL) dropwise at 0 °C. The resulting solution was stirred for 2 h at 0 °C. $NH_3$ (g) was bubbled into the reaction solution for 10 min at 0 °C. Then the solution was stirred for another 30 min at RT. The solids were filtered out, the filtrate was diluted with water (20 mL). The resulting solution was extracted with DCM (3x20 mL). The organic layers were dried over anhydrous $Na_2SO_4$ and concentrated under vacuum. The residue was eluted from a silica gel column with EtOAc/PE (1:1). This resulted in 800 mg (80.1%) of the title compound as an off-white solid. MS-ESI: 353 (M+1).

**Scheme 165:**

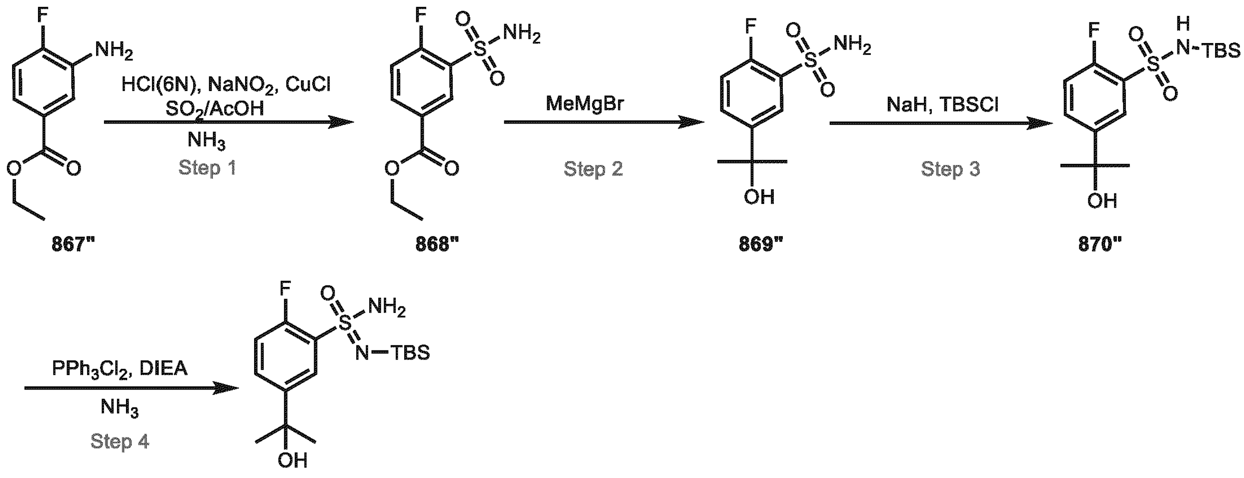


867" 868" 869" 870"

Intermediate 222

## Intermediate 222

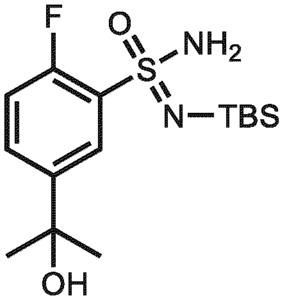

N'-(tert-butyldimethylsilyl)-2-fluoro-5-(2-hydroxypropan-2-yl)benzenesulfonimidamide

**Step 1: Ethyl 4-fluoro-3-sulfamoylbenzoate**

To a stirred solution of ethyl 3-amino-4-fluorobenzoate (500 mg, 2.73 mmol) in HCl (6 M, 5 mL) was added $NaNO_2$ (226 mg, 3.28 mmol) in water (0.5 mL) dropwise at 0 °C. The resulting solution was stirred for 30 min at 0 °C, the mixture was assigned as A. $SO_2$ (g) was bubbled in AcOH (10 mL) for 5 min at 0 °C, then to the solution was added CuCl (272 mg, 2.73 mmol) at 0 °C, this mixture was assigned as B. Mixture A was added to mixture B dropwise at 0 °C. The resulting mixture was stirred for 30 min at RT. The reaction mixture diluted with water (20 mL), then extracted with DCM (3x30 mL). The organic layers were combined and dried over anhydrous $Na_2SO_4$. To the above solution was bubbled $NH_3$ at 0 °C for 3 min. The resulting mixture was stirred for 30 min at RT. The resulting mixture was concentrated under vacuum. The residue was eluted from a silica gel column with EtOAc/PE (1:3). This resulted in 500 mg (74.1%) of the title compound as a white solid. MS-ESI: 246 (M-1).

**Step 2: 2-Fluoro-5-(2-hydroxypropan-2-yl)benzenesulfonamide**

To a stirred solution of ethyl 4-fluoro-3-sulfamoylbenzoate (500 mg, 2.02 mmol) in THF (40 mL) was added MeMgBr (3 M in THF, 5.4 mL, 16.2 mmol) dropwise at 0 °C. The resulting solution was stirred for 16 h at RT. The resulting

mixture was quenched with 30 mL of saturated $NH_4Cl$ (aq.), extracted with 3x50 mL of EtOAc. The organic layers were combined and dried over anhydrous $Na_2SO_4$. The resulting solution was concentrated under vacuum. The residue was eluted from a silica gel column with EtOAc/PE (1:3). This resulted in 420 mg (89.2%) of the title compound as a white solid. MS-ESI: 232 (M-1).

Steps 3-4 used similar procedures for converting compound **639"** to Intermediate **183** shown in Scheme 128 to afford Intermediate **222** from compound **869".** MS-ESI: 347 (M+1).

**Scheme 166:**

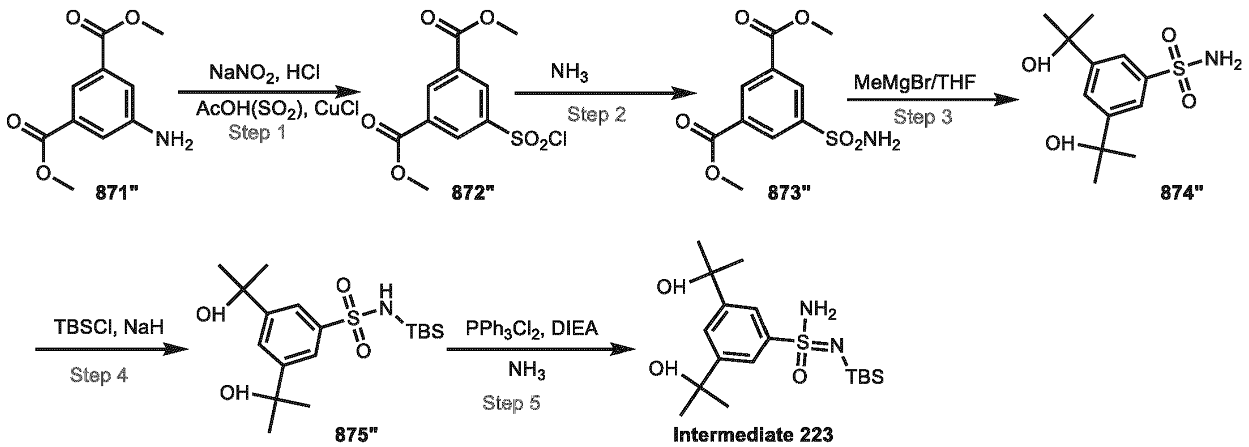


**Intermediate 223**

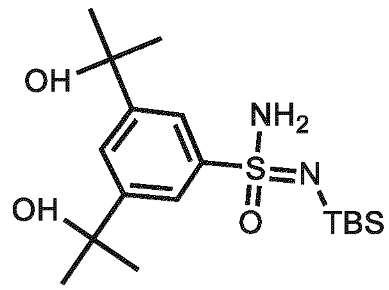

N'-(tert-butyldimethylsilyl)-3,5-bis(2-hydroxypropan-2-yl)benzenesulfonimidamide

Steps 1-2 used similar procedures for converting compound **867"** to compound **868"** shown in Scheme 165 to afford compound **873"** from compound **871".** MS-ESI: 272 (M-1).

**Step 3: 3,5-Bis(2-hydroxypropan-2-yl)benzenesulfonamide**

To a stirred solution of dimethyl 5-sulfamoylisophthalate (5 g, 18.3 mmol) in THF (100 mL) was added MeMgBr (3 M in THF, 120 mL, 360 mmol) dropwise at 0 °C. The resulting mixture was stirred for 48 h at RT. The reaction mixture was quenched with 200 mL of sat. $NH_4Cl$ (aq.), then extracted with 3x200 mL of EtOAc. The organic layers were combined and dried over anhydrous $Na_2SO_4$. The resulting solution was concentrated under vacuum. The crude product was purified by reverse column using the following conditions: Column, $C_{18}$ silica gel; mobile phase, ACN and water: increasing to ACN/water from 0/100 to 90/10 over 30 min; Detector, UV 254 nm. This resulted in 2.5 g (50.0%) of the title compound as a white solid. MS-ESI: 274 (M+1).

Steps 4-5 used similar procedures for converting compound **639"** to Intermediate **183** shown in Scheme 128 to afford Intermediate **223** from compound **874".** MS-ESI: 387 (M+1).

**Scheme 167:**

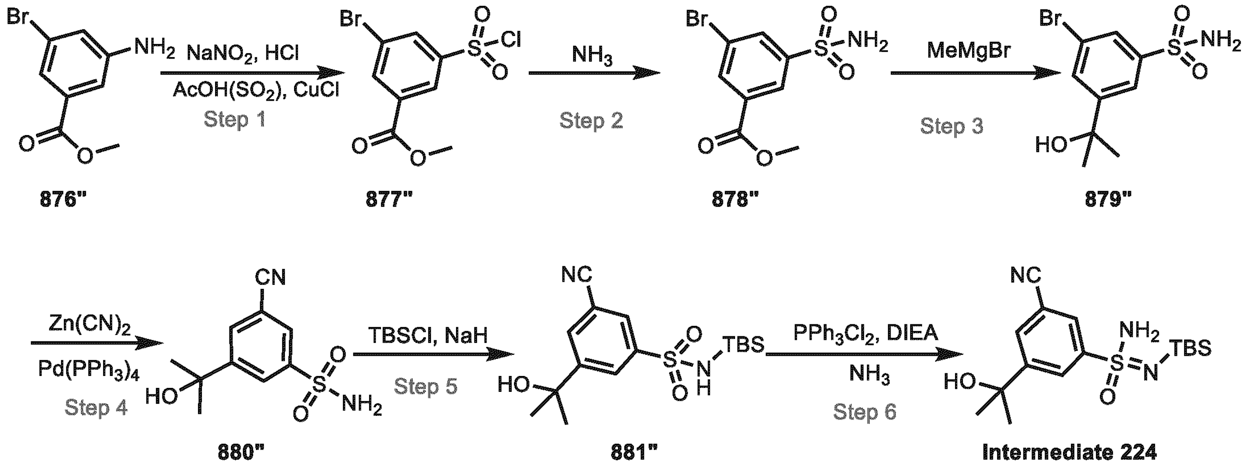


## Intermediate 224

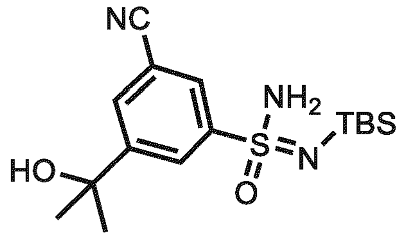

N'-(tert-butyldimethylsilyl)-3-cyano-5-(2-hydroxypropan-2-yl)benzenesulfonimidamide

Steps 1-2 used similar procedures for converting compound **867"** to compound **869"** shown in Scheme 165 to afford compound **879"** from compound **876".** MS-ESI: 292/294 (M-1).

**Step 4: 3-Cyano-5-(2-hydroxypropan-2-yl)benzenesulfonamide**

To a stirred solution of 3-bromo-5-(2-hydroxypropan-2-yl)benzenesulfonamide (294 mg, 1.00 mmol) in DMF (5 mL) under nitrogen was added $Zn(CN)_2$ (232 mg, 2.00 mmol) and $Pd(PPh_3)_4$ (231 mg, 0.20 mmol). The resulting mixture was stirred for 5 h at 80 °C. The reaction mixture was quenched with 20 mL of water, and then extracted with 3x20 ml of EtOAc. The organic layers were combined and dried over anhydrous $Na_2SO_4$. The resulting solution was concentrated under vacuum. The residue was eluted from a silica gel with EtOAc/PE (2:1). This resulted in 150 mg (62.5%) of the title compound as a yellow solid. MS-ESI: 239/240 (M-1).

Steps 5-6 used similar procedures for converting compound **639"** to Intermediate **183** shown in Scheme 128 to afford Intermediate **224** from compound **880".** MS-ESI: 354 (M+1).

**Scheme 168:**

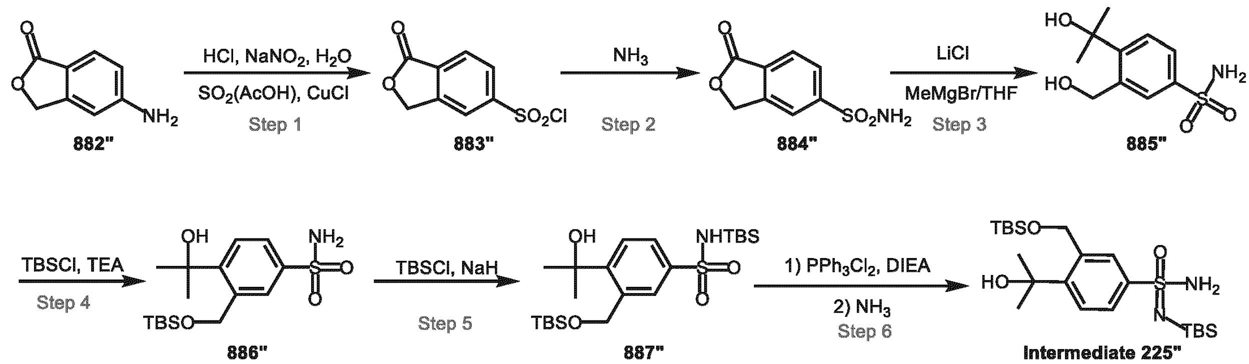

## Intermediate 225

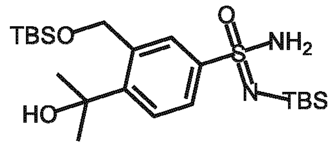

N'-(tert-butyldimethylsilyl)-3-(((tert-butyldimethylsilyl)oxy)methyl)-4-(2-hydroxypropan-2-yl)benzenesulfonimidamide

Steps 1-2 used similar procedures for converting compound **867"** to compound **868"** shown in Scheme 165 to afford compound **884"** from compound **882".** MS-ESI: 212 (M-1).

**Step 3: 3-(Hydroxymethyl)-4-(2-hydroxypropan-2-yl)benzenesulfonamide**

To a stirred mixture of 1-oxo-1,3-dihydroisobenzofuran-5-sulfonamide (1.3 g, 6.10 mmol) and LiCl (119 mg, 2.81 mmol) in THF (40 mL) under nitrogen was added MeMgBr (3 M in THF, 40.7 mL, 122 mmol) dropwise at 0 °C. The resulting mixture was stirred for 16 h at RT. The reaction was quenched with 50 mL of sat. $NH_4Cl$ (aq.) at 0 °C. The resulting mixture was extracted with 3 × 100 mL of EtOAc. The organic layers were combined and dried over anhydrous $Na_2SO_4$. The resulting solution was concentrated under vacuum. This resulted in 1.1 g (73.6%) of the title compound as red oil. MS-ESI: 246 (M+1).

**Step 4: 3-(((Tert-butyldimethylsilyl)oxy)methyl)-4-(2-hydroxypropan-2-yl)benzenesulfonamide**

To a stirred solution of 3-(hydroxymethyl)-4-(2-hydroxypropan-2-yl)benzenesulfonamide (1.1 g, 4.49 mol) in DCM (40 mL) under nitrogen was added TEA (1.36 g, 13.5 mmol) and TBSCl (1.01 g, 6.74 mmol) at RT. The resulting mixture was stirred for 6 h at RT. The reaction mixture was quenched with 40 mL of water, extracted with 3 × 30 mL of DCM. The organic layers were combined and dried over anhydrous $Na_2SO_4$. The resulting solution was concentrated under vacuum. The residue was eluted from a silica gel column with DCM/MeOH (100:1). This resulted in 1.3 g (80.6%) of the title compound as an off-white solid. MS-ESI: 360 (M+1).

Steps 5-6 used similar procedures for converting compound **639"** to Intermediate **183** shown in Scheme 128 to afford Intermediate **225** from compound **886".** MS-ESI: 473 (M+1).

**Scheme 169:**

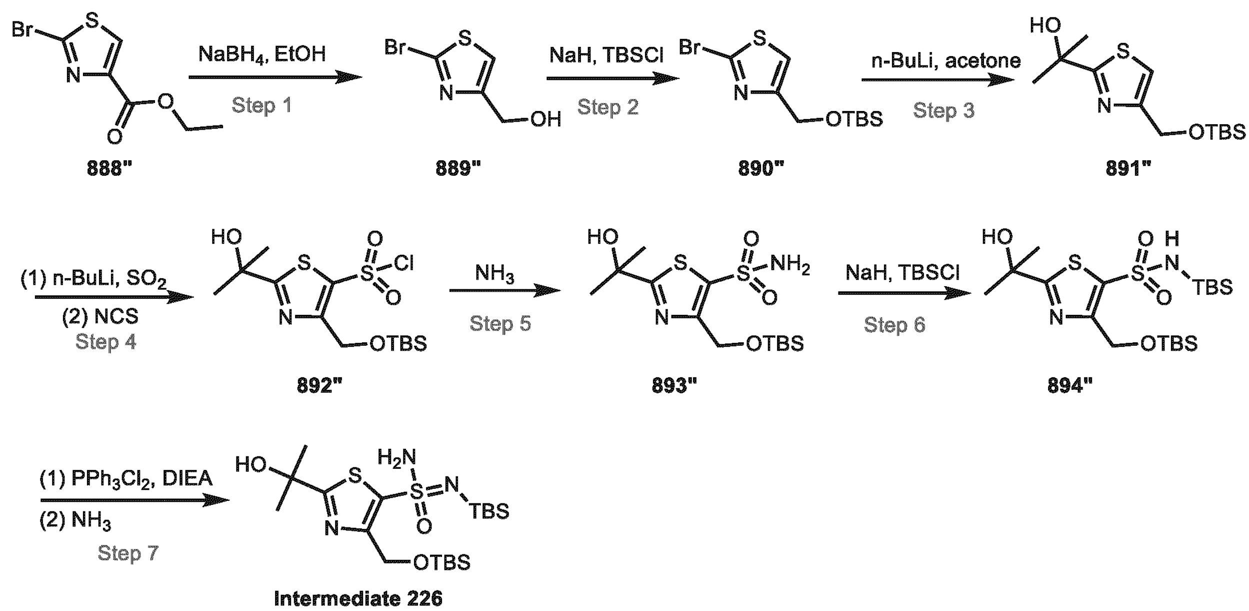


## Intermediate 226

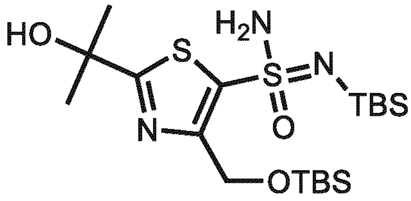

N'-(tert-butyldimethylsilyl)-4-(((tert-butyldimethylsilyl)oxy)methyl)-2-(2-hydroxypropan-2-yl)thiazole-5-sulfonimidamide

**Step 1: (2-Bromothiazol-4-yl)methanol**

To a stirred solution of ethyl 2-bromothiazole-4-carboxylate (3.0 g, 12.8 mmol) in EtOH (30 mL) was added $NaBH_4$ (1.0 g, 25.4 mmol) in portions at 0 °C. The resulting solution was stirred for 3 h at RT. The reaction was quenched with 100 mL of water at 0 °C. Then extracted with 3x100 ml of EtOAc, the organic layers were combined and dried over anhydrous $Na_2SO_4$. The resulting solution was concentrated under vacuum. This resulted in 2 g (81.0%) of the title compound as yellow oil. MS-ESI: 194/196 (M+1).

**Step 2: 2-Bromo-4-(((tert-butyldimethylsilyl)oxy)methyl)thiazole**

To a stirred solution of (2-bromothiazol-4-yl)methanol (2.0 g, 10.3 mmol) in THF (20 mL) under nitrogen was added NaH (60% wt., 1.2 g, 30.9 mmol) in portions at 0 °C. After stirring for 15 minutes at RT, a solution of TBSCl (4.7 g, 30.9 mmol) in THF (5 mL) was added dropwise at 0 °C. The resulting solution was stirred for 2 h at RT. The reaction was then quenched with 50 mL of water. The resulting solution was extracted with 3x100 mL of EtOAc. The organic layers were combined and dried over anhydrous $Na_2SO_4$ and concentrated under vacuum. The residue was eluted from a silica gel column EtOAc/PE (1 :30). This resulted in 2.5 g (79%) of the title compound as yellow oil. MS-ESI: 310/308 (M+1).

**Step 3: 2-(4-(((Tert-butyldimethylsilyl)oxy)methyl)thiazol-2-yl)propan-2-ol**

To a stirred mixture of 2-bromo-4-(((tert-butyldimethylsilyl)oxy)methyl)thiazole (2.5 g, 8.14 mmol) in THF (30 mL) under nitrogen was added n-BuLi (2.5 M in hexane, 4.86 mL, 12.2 mmol) dropwise at -78 °C. After stirring for 30 min at -78 °C, to the above was added acetone (1.8 g, 32.4 mmol) dropwise at -78 °C. The resulting mixture was stirred for 1 h at RT. The resulting mixture was quenched with 50 mL of water, extracted with 3x100 ml of EtOAc. The organic layers were combined and dried over anhydrous $Na_2SO_4$. The resulting solution was concentrated under vacuum. The residue was eluted from a silica gel column with EtOAc/PE (1:10). This resulted in 2 g (85.6%) of the title compound as yellow oil. MS-ESI: 288 (M+1). Steps 4-7 used similar procedures for converting compound **665"** to Intermediate **188** shown in Scheme 133 to afford Intermediate **226** from compound **891".** MS-ESI: 480 (M+1).

**Scheme 170:**

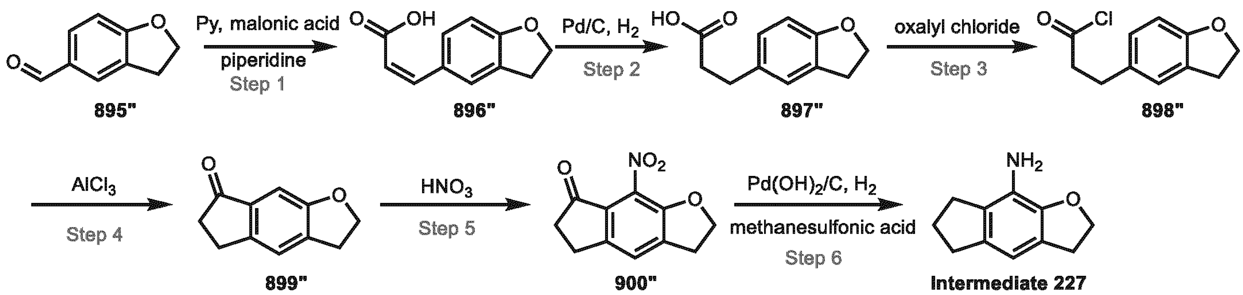

## Intermediate 227

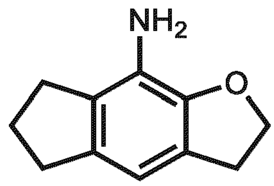

3, 5,6, 7-Tetrahydro-2H-indeno[5,6-b]furan-8-amine

**Step 1: (Z)-3-(2,3-Dihydrobenzofuran-5-yl)acrylic acid**

To a stirred solution of 2,3-dihydrobenzofuran-5-carbaldehyde (25 g, 169 mmol) in pyridine (200 mL) under nitrogen was added malonic acid (21.5 g, 203 mmol) and piperidine (1.4 g, 16.44 mmol). The resulting solution was stirred for 16 h at 100 °C. The resulting mixture was cooled to RT, diluted with water (200 mL), and extracted with EtOAc (3x200 mL). The organic layers were combined and dried over anhydrous $Na_2SO_4$ then concentrated under vacuum. This resulted in 30 g (93.4%) of the title compound as a yellow solid. MS-ESI: 189 (M-1).

**Step 2: 3-(2,3-Dihydrobenzofuran-5-yl)propanoic acid**

To a stirred solution of (Z)-3-(2,3-dihydrobenzofuran-5-yl)acrylic acid (25 g, 132 mmol) in MeOH (200 mL) under nitrogen was added Pd/C (10%wt., 4 g). The flask was evacuated and refilled with hydrogen three times. The resulting solution was stirred for 16 h at RT under hydrogen with a balloon. The solids were filtered out and the filtrate was concentrated under vacuum. This resulted in 25 g (98.6%) of the title compound as a light yellow solid. MS-ESI: 191 (M-1).

**Step 3: 3-(2,3-Dihydrobenzofuran-5-yl)propanoyl chloride**

To a stirred solution of 3-(2,3-dihydrobenzofuran-5-yl)propanoic acid (20 g, 104 mmol) in DCM (200 mL) under nitrogen was added oxalyl chloride (13.21 g, 104 mmol). The resulting solution was stirred for 2 h at RT. The resulting mixture was concentrated under vacuum. This resulted in 20 g (crude) of the title compound as a light yellow liquid which was used for next step without further purification.

**Step 4: 2,3,5,6-Tetrahydro-7H-indeno[5,6-b]furan-7-one**

To a stirred solution of 3-(2,3-dihydrobenzofuran-5-yl)propanoyl chloride (crude from last step) in DCM (200 mL) under nitrogen was added $AlCl_3$ (12.7 g, 95.2 mmol) in portions at 0 °C. The resulting solution was stirred for 2 h at 0 °C. The reaction was quenched with 200 mL of water/ice, extracted with 3x200 mL of DCM. The organic layers were combined and dried over anhydrous $Na_2SO_4$. The resulting solution was concentrated under vacuum. The residue was eluted from a silica gel column with EtOAc/PE (1:3). This resulted in 10 g (54.6% over two steps) of the title compound as a yellow solid. MS-ESI: 175 (M+1).

**Step 5: 8-Nitro-2,3,5,6-tetrahydro-7H-indeno[5,6-b]furan-7-one**

To a stirred solution of 2,3,5,6-tetrahydro-7H-indeno[5,6-b]furan-7-one (5 g, 28.7 mmol) in conc. $H_2SO_4$ (20 mL) was added HNOs (1.99 g, 31.6 mmol) dropwise at 0 °C. The resulting solution was stirred for 2 h at 0 °C. The resulting solution was diluted with 200 mL of ice/water, extracted with 3x100 mL of DCM. The organic layers were combined and dried over anhydrous $Na_2SO_4$. The resulting mixture was concentrated under vacuum. This resulted in 5 g (79.6%) of the title compound as a light yellow solid. MS-ESI: 220 (M+1).

**Step 6: 3,5,6,7-Tetrahydro-2H-indeno[5,6-b]furan-8-amine**

To a stirred solution of 8-nitro-2,3,5,6-tetrahydro-7H-indeno[5,6-b]furan-7-one (5 g, 22.8 mmol) in MeOH (50 mL) was added methanesulfonic acid (675 mg, 7.0 mmol) and $Pd(OH)_2/C$ (20%wt., 1 g). The flask was evacuated and refilled with hydrogen three times. The resulting solution was stirred for 16 h at RT under hydrogen with a balloon. The pH value of the solution was adjusted to 9 with 1M NaOH (aq.). The solids were filtered out and the filtrate was concentrated under vacuum. This resulted in 3.50 g (87.7%) of the title compound as a yellow solid. MS-ESI: 176 (M+1).

## Scheme 171:

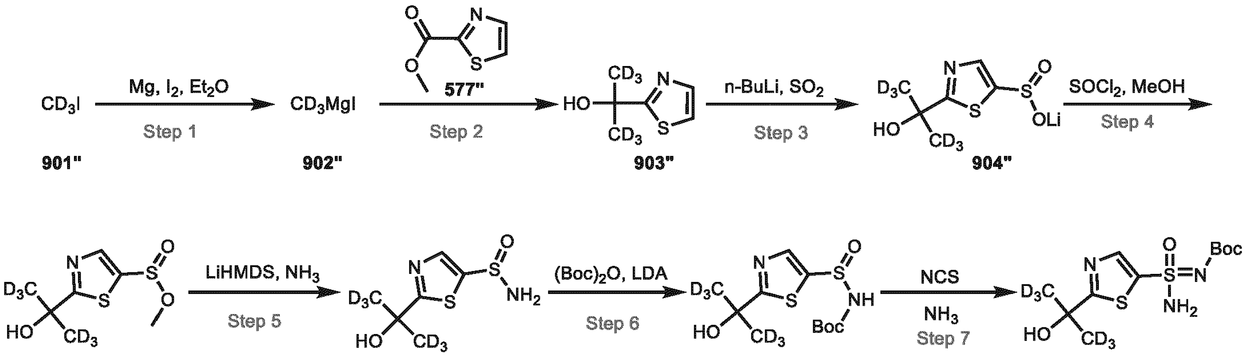


901" 902" 903" 904"

905" 906" 907" Intermediate 228

## Intermediate 228

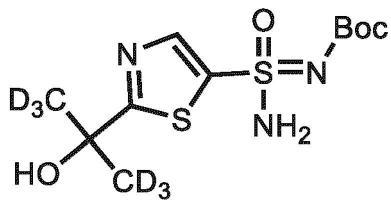

Tert-butyl (amino(2-(2-hydroxypropan-2-yl-1,1,1,3,3,3-$d_6$)thiazol-5-yl)(oxo)- $\lambda^6$-sulfaneylidene)carbamate

**Step 1: (Methyl-$d_3$)magnesium iodide**

To a stirred mixture of Mg turnings (10.0 g, 410 mmol) and iodine (10.0 mg, a small crystal) in $Et_2O$ (1.3 L) under nitrogen was added $CD_3I$ (50 g, 345 mmol) in $Et_2O$ (100 mL) dropwise at 0 °C. The resulting mixture was stirred for 2 h at RT. This resulted in the title compound (crude in $Et_2O$, ~0.25 M) as a grey solution which was used for next step without further purification.

**Step 2: 2-(Thiazol-2-yl)propan-1,1,1,3,3,3-$d_6$-2-ol**

To a stirred solution of (methyl-$d_3$)magnesium iodide (crude 0.25 M in $Et_2O$, 1.4 L, 350 mmol) under nitrogen was added methyl thiazole-2-carboxylate (10.7 g, 74.8 mmol) in THF (150 mL) dropwise at 0 °C. The resulting solution was stirred for 2 h at RT. The reaction was quenched with 200 mL of water at 0 °C. Then extracted with 2×1 L of EtOAc, the organic layers were combined and dried over anhydrous $Na_2SO_4$. The resulting mixture was concentrated under vacuum. The residue was eluted from a silica gel column with EtOAc/PE (1:10). This resulted in 9 g (80.7%) of the title compound as a white solid. MS-ESI: 150 (M+1).

Steps 3-7 used similar procedures for converting compound **87** to Intermediate **28** shown in Scheme 16 to afford Intermediate **228** from compound **903"**. MS-ESI: 328 (M+1).

**Scheme 172:**

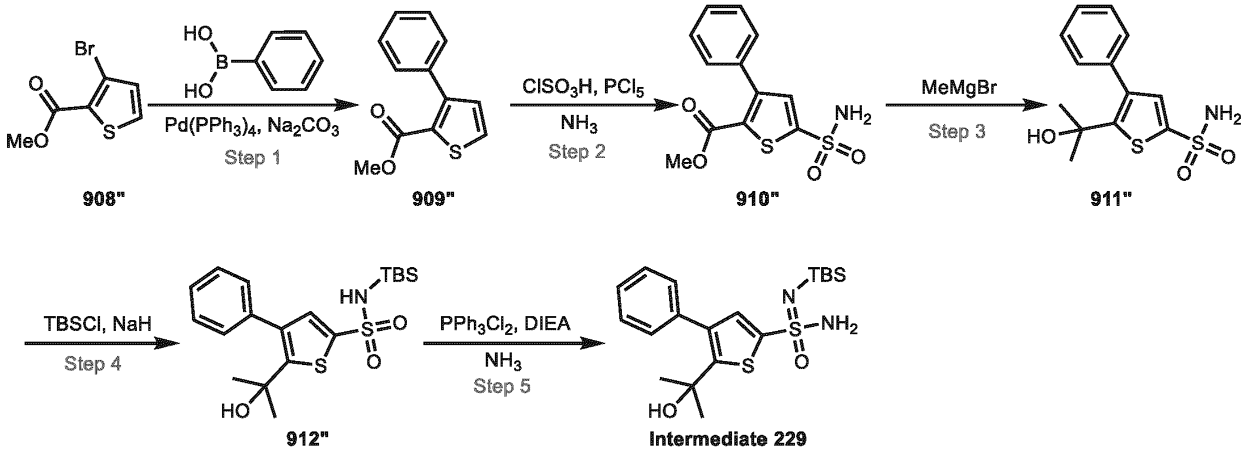


## Intermediate 229

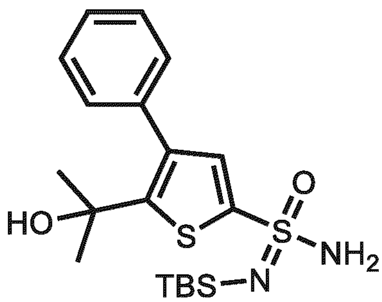

N'-(tert-butyldimethylsilyl)-5-(2-hydroxypropan-2-yl)-4-phenylthiophene-2-sulfonimidamide

**Step 1: Methyl 3-phenylthiophene-2-carboxylate**

To a stirred solution of methyl 3-bromothiophene-2-carboxylate (3.0 g, 13.6 mmol) in dioxane (40 mL) and $H_2O$ (13 mL) under nitrogen was added phenylboronic acid (1.99 g, 16.3 mmol), $Na_2CO_3$ (2.16 g, 20.4 mmol) and $Pd(PPh_3)_4$ (784 mg, 0.679 mmol). The resulting mixture was stirred for 5 h at 90 °C. The reaction was diluted with 50 mL of water. Then extracted with 3x50 mL of EtOAc, the organic layers were combined and dried over anhydrous $Na_2SO_4$. The resulting mixture was concentrated under vacuum. The residue was eluted from silica gel column with EtOAc/PE (1/3). This resulted in 2.8 g (94.4%) of the title compound as a light yellow solid.MS-ESI: 219 (M+1).

**Step 2: Methyl 3-phenyl-5-sulfamoylthiophene-2-carboxylate**

To a stirred solution of methyl 3-phenylthiophene-2-carboxylate (2.8 g, 12.8 mmol) in $CHCl_3$ (30 mL) under nitrogen was added chlorosulfonic acid (3.0 g, 25.6 mmol) dropwise at 0 °C. The solution was stirred for 2 h at RT, then $PCl_5$ (13.2 g, 64.0 mmol) was added in portions at 70 °C. The resulting mixture was stirred for 3 h at 70 °C. The reaction mixture was slowly poured into 100 mL of ice/water. Then extracted with 3x50 mL of DCM, the organic layer were combined and dried over anhydrous $Na_2SO_4$. The resulting solution was concentrated under vacuum. The residue was dissolved in THF (50 mL), then $NH_3$ (g) was bubbled for 5 min at 0 °C. The resulting mixture was stirred for 1 h at RT. The resulting mixture was concentrated under vacuum. The residue was eluted from silica gel column with EtOAc/PE (1/2). This resulted in 2.0 g (52.6%) of the title compound as a light yellow solid. MS-ESI: 296 (M-1).

Steps 3-5 used similar procedures for converting compound **868"** to Intermediate **222** shown in Scheme 165 to afford Intermediate **229** from compound **910"**. MS-ESI: 411 (M+1).

**Scheme 173:**

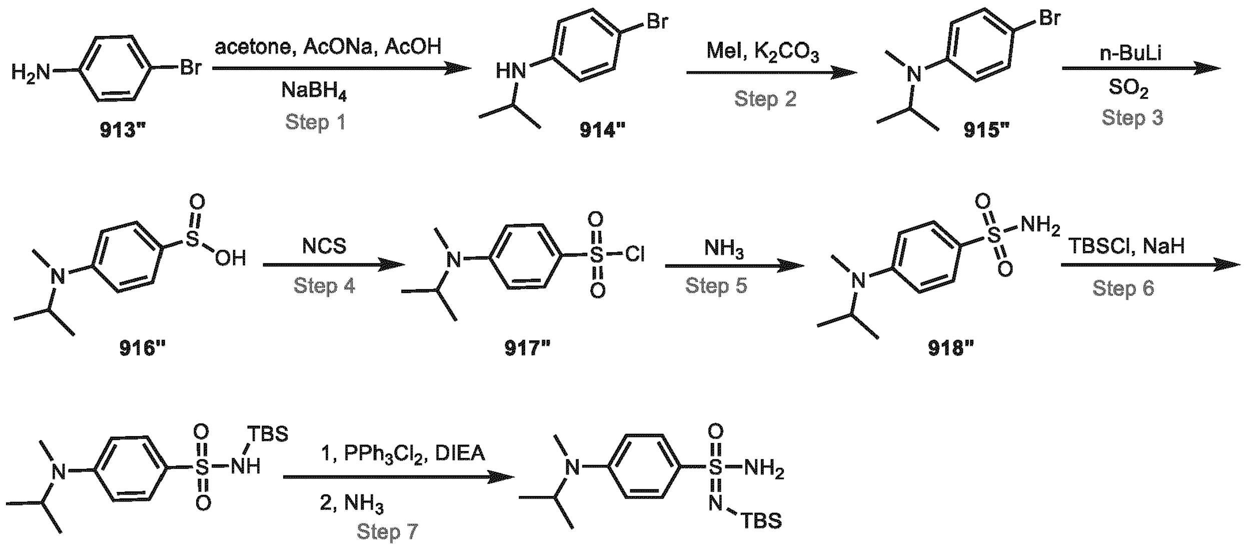


913" 914" 915" 916" 917" 918" 919" Intermediate 230

## Intermediate 230

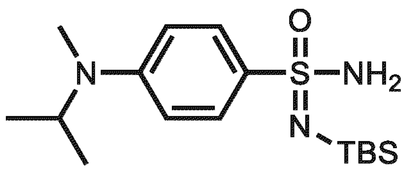

N-(tert-butyldimethylsilyl)-4-(isopropyl(methyl)amino)benzenesulfonimidamide

**Step 1: 4-Bromo-N-isopropylaniline**

To a stirred solution of 4-bromoaniline (7.6 g, 44.2 mmol) in EtOH (50 mL) and $H_2O$ (50 mL) under nitrogen was added AcONa (10.9 g, 133 mmol), AcOH (40 mL) and acetone (12.8 g, 221 mmol) at 0 °C. The resulting mixture was stirred for 30 min at RT. To the above mixture was added $NaBH_4$ (8.36 g, 221 mmol) in portions slowly at 0 °C. The resulting mixture was stirred for 2 h at 0 °C. The reaction was quenched with 50 mL of NaOH (2 M, aq.), extracted with 3 × 100 mL of EtOAc. The organic layers were combined and dried over anhydrous $Na_2SO_4$. The resulting solution was concentrated under vacuum. The residue was eluted from a silica gel column with EtOAc/PE (1:1). This resulted in 9 g (95.6%) of the title compound as a yellow solid. MS-ESI: 214/216 (M+1).

**Step 2: 4-Bromo-N-isopropyl-N-methylaniline**

To a stirred solution of 4-bromo-N-isopropylaniline (9 g, 42.3 mmol) in DMF (100 mL) under nitrogen was added $K_2CO_3$ (11.7 g, 84.6 mmol), followed by the addition of MeI (30.1 g, 212 mmol) dropwise at 0 °C. The resulting mixture was stirred for 16 h at 80 °C. The reaction was quenched with 150 mL of water/ice, extracted with 3 × 100 mL of EtOAc. The organic layers were combined and dried over anhydrous $Na_2SO_4$. The resulting solution was concentrated under vacuum. The residue was eluted from a silica gel column with EtOAc/PE (1:1). This resulted in 6.18 g (64.4%) of the title compound as a yellow oil. MS-ESI: 228/230 (M+1).

Steps 3-7 used similar procedures for converting compound **642"** to Intermediate **184** shown in Scheme 129 to afford Intermediate **230** from compound **915"**. MS-ESI: 342 (M+1).

Scheme 174:

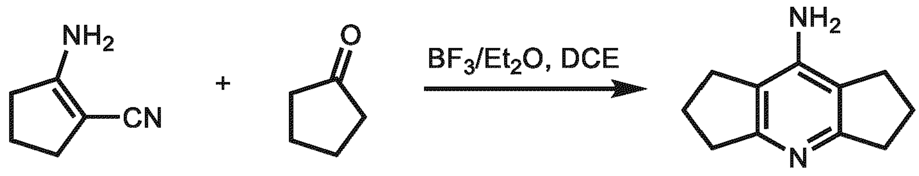


920"

921"

Intermediate 231

Intermediate 231

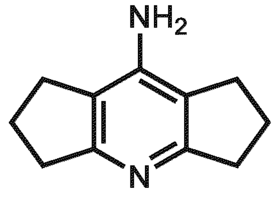

2,3,5,6,7-Hexahydrodicyclopenta[b,e]pyridin-8-amine

To a mixture of 2-aminocyclopent-1-ene-1-carbonitrile (5.4 g, 50 mmol) in DCE (125 mL) under nitrogen was added cyclopentanone (8.4 g, 100 mmol) at RT, followed by the addition of $BF_3.Et_2O$ (47%wt., 100 mmol, 14.5 g) dropwise at 0 °C. The resulting mixture was heat to 75 °C for 6 h. The reaction cooled to RT, then quenched with water/ice (100 mL), extracted with DCM (2x50 mL) to remove the impurities. The aqueous phase was collected and its pH value adjusted to 14 with NaOH (6 M). A solid was precipitated and collected by filtration. The filter cake was washed with water (150 mL) then dried under infra-red light. This resulted in 7.0 g of the title compound (80%) as a light yellow solid. MS-ESI: 175 (M+1).

Scheme 175:

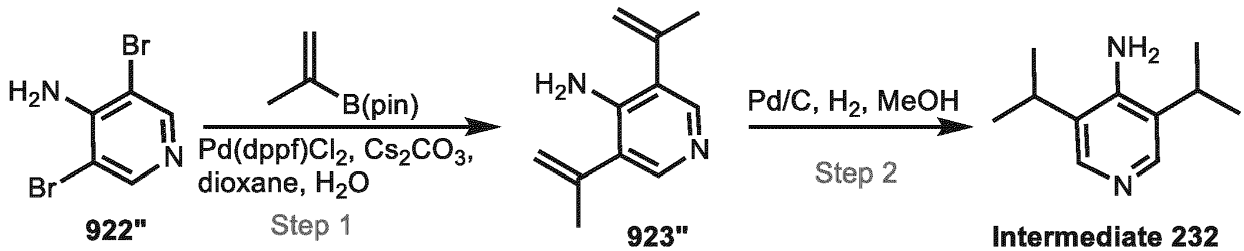


922"

923"

Intermediate 232

Intermediate 232

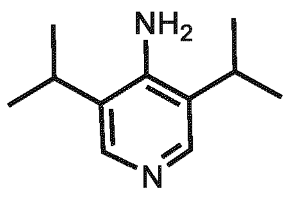

3,5 -Diisopropylpyridin-4-amine

**Step 1: 3,5-Di(prop-1-en-2-yl)pyridin-4-amine**

To a stirred solution of 3,5-dibromopyridin-4-amine (5.0 g, 20 mmol) in dioxane (150 mL) and water (15 mL) under nitrogen was added 4,4,5,5-tetramethyl-2-(prop-1-en-2-yl)-1,3,2-dioxaborolane (10 g, 60 mmol), $Cs_2CO_3$ (19.6 g, 60 mmol) and $Pd(dppf)Cl_2$ (1.46 g, 2.0 mmol). The resulting solution was stirred for 15 h at 90 °C. The resulting mixture was concentrated under vacuum. The residue was eluted from a silica gel with EtOAc/PE (1:3). This resulted in 3.0 g (87%) of the title compound as light yellow oil. MS-ESI: 175 (M+1).

**Step 2: 3,5-Diisopropylpyridin-4-amine**

To a stirred solution of 3,5-di(prop-1-en-2-yl)pyridin-4-amine (3.0 g, 17.2 mmol) in MeOH (50 mL) under nitrogen was added Pd/C (wet. 10% wt., 300 mg). The flask was evacuated and flushed three times with hydrogen. The resulting solution was stirred for 16 h at RT under hydrogen with a balloon. The Pd/C catalysts were filtered out, the filtrate was concentrated under vacuum. This resulted in 2.8 g (91%) of the title compound as a light yellow solid. MS-ESI: 178 (M+1).

**Reagent 1**

Dichlorotriphenylphosphorane

This reagent was either purchased or prepared using the following procedure:

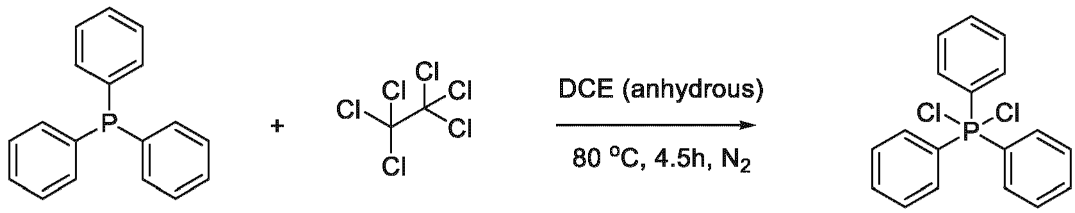


An oven dried 40 mL vial equipped with a stir bar was capped with a rubber septum and flushed with nitrogen. At room temperature, a solution of $PPh_3$ (0.85 g, 3.2 mmol) in anhydrous 1,2-dichloroethane (5 mL) was introduced *via* syringe. The reaction vessel was immersed in an ice / water bath and cooled for 5 min. A solution of hexachloroethane (0.76 g, 3.2 mmol) in anhydrous 1,2-dichloroethane (5 mL) was introduced dropwise *via* syringe. After the addition was complete the reaction mixture was stirred at the same temperature for an additional 5 min and then placed into a preheated block set at 80 °C. Heating was continued for 4.5 h, at which time the reaction was assumed to be complete. The light golden clear solution was cooled to ambient temperature. The reagent thus prepared was transferred *via* syringe in subsequent reactions without any work up or purification. The total volume of the reaction mixture was 11 mL for the molar calculations for next steps. This solution containing $PPh_3Cl_2$ was stored under nitrogen at room temperature until used.

**Reagent 2**

Polymer-bound dichlorotriphenylphosphorane

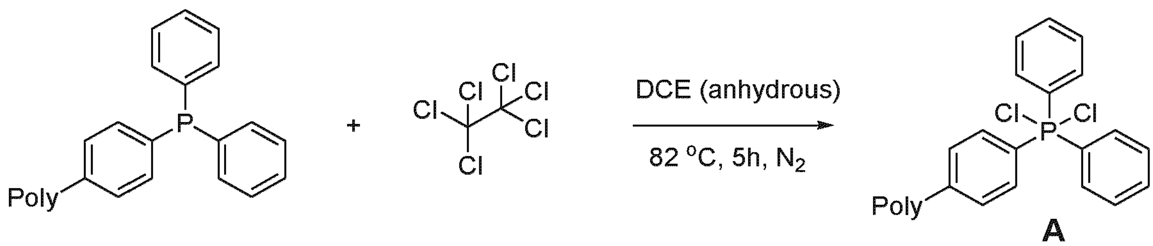


Polystyrene bound $PPh_3$ (0.32 g, 0.32 mmol) was suspended in anhydrous dichloroethane (6 mL) and shaked on a shaker for 5 mins. It was then filtered and the process was repeated again to swell the polymer. Filtered resin was suspended in anhydrous dichloroethane (6 mL) a third time and the whole suspension was transferred into an oven dried 40 mL vial with a stir bar via pipette. The vial was capped with a rubber septum and connected to a steady flow of nitrogen. The reaction vessel was immersed in an ice/water bath and cooled down for 10 min. A solution of hexachloroethane (0.076 g, 0.32 mmol) in anhydrous 1,2-dichloroethane (2 mL) was introduced drop wise via syringe. After the addition was complete the reaction mixture was placed in an already heated block set at 82 °C for 5 h. At this point the reaction is assumed to be completed. It was gradually brought to room temperature and used in the next step as is. This reagent was used at 1.5 equiv. with respect to sulfonamide in the next step.

**Synthetic Examples**

Except where mentioned to refer to an example compound falling under claim 1, all Examples are merely disclosed for reference purposes and do not form part of the invention.

**Example 1**

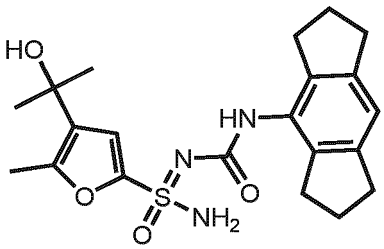

**Example 1 (181):** N'-(1,2,3,5,6,7-hexahydro-*s*-indacen-4-ylcarbamoyl)-4-(2-hydroxypropan-2-yl)-5-methylfuran-2-sulfonimidamide

**Example 1** was synthesized according to the general method shown in **Scheme 1,** as illustrated below.

**Examples 2 and 3**

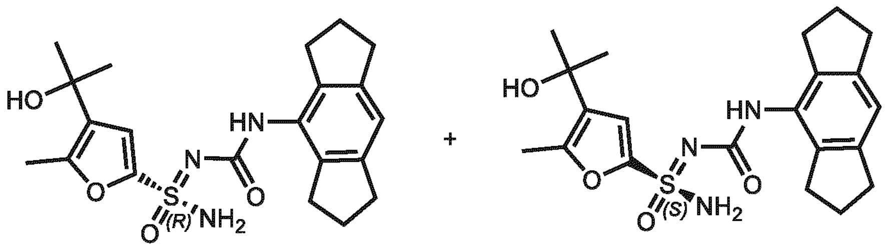

**Examples 2 and 3**

**Examples 2 (181a) and 3 (181b):** (S)- and (R)- N'-(1,2,3,5,6,7-hexahydro-*s*-indacen-4-ylcarbamoyl)-4-(2-hydroxypropan-2-yl)-5-methylfuran-2-sulfonimidamide

Examples 2 and 3 were prepared through chiral separation of Example 1 as illustrated below.

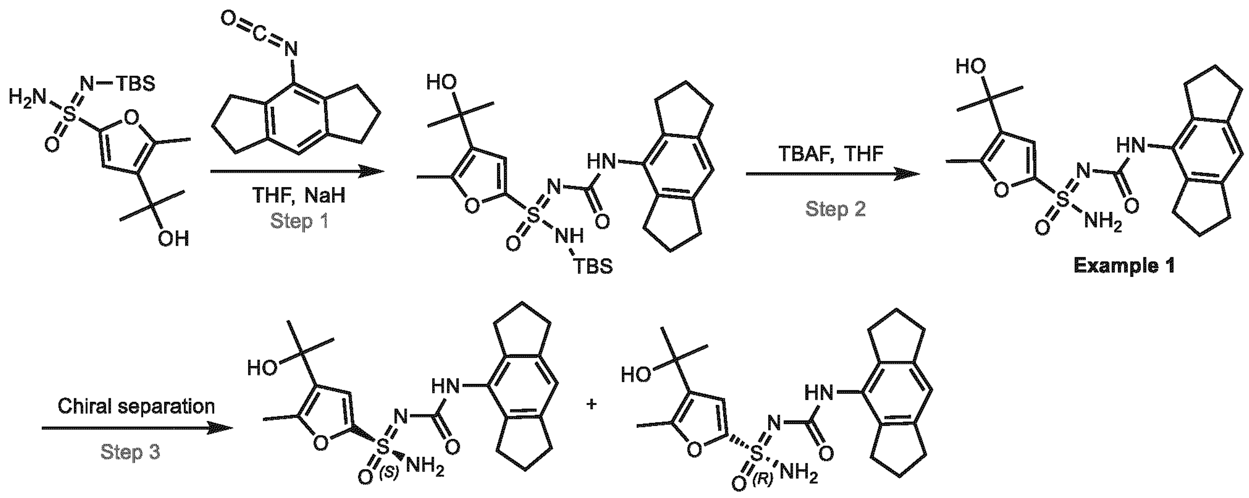


**Examples 2 and 3**

**Step 1: N'-(*tert*-butyldimethylsilyl)-N-(1,2,3,5,6,7-hexahydro-s-indacen-4-ylcarbamoyl)-4-(2-hydroxypropan-2-yl)-5-methylfuran-2-sulfonimidamide**

Into a 50-mL round-bottom flask was placed N'-(tert-butyldimethylsilyl)-4-(2-hydroxypropan-2-yl)-5-methylfuran-2-sulfonimidamide (200 mg, 0.6 mmol), THF (10 mL), NaH (60% wt, 48 mg, 1.2 mmol). This was followed by the addition of a solution of 4-isocyanato-1,2,3,5,6,7-hexahydro-s-indacene (120 mg, 0.6 mmol) in THF (1 mL) dropwise with stirring at RT. The resulting solution was stirred for 12 h at RT. The reaction was then quenched by the addition of 10 mL of $NH_4Cl$ (sat.). The resulting solution was extracted with 3x10 mL of DCM and the organic layers combined and concentrated under vacuum. The residue was applied onto a silica gel column and eluted with ethyl acetate/petroleum ether (1:10 to 1:3). This resulted in 140 mg (43.8%) of the title compound as brown oil. MS-ESI: 532.0 (M-1).

**Step 2: N'-(1,2,3,5,6,7-hexahydro-s-indacen-4-ylcarbamoyl)-4-(2-hydroxypropan-2-yl)-5-methylfuran-2-sulfonimidamide**

Into a 50-mL round-bottom flask was placed N'-(*tert*-butyldimethylsilyl)-N-(1,2,3,5,6,7-hexahydro-s-indacen-4-ylcarbamoyl)-4-(2-hydroxypropan-2-yl)-5-methylfuran-2-sulfonimidamide (130 g, 0.2 mmol), THF (10 mL), and TBAF (300 mg, 0.5 mmol). The resulting solution was stirred for 2 h at RT and then concentrated under vacuum. The crude product was purified by Prep-HPLC using method E eluted with a gradient of 30~60% ACN. This resulted in 82 mg (80.3%) of Example 1 as a white solid.

**Example 1:** MS-ESI: 418.0 (M+1). $^1H$ NMR (400 MHz, DMSO-$d_6$) $\delta$ 8.39 (s, 1H), 7.57 (s, 2H), 6.87 (s, 1H), 6.85 (s, 1H), 5.04 (s, 1H), 2.79 (t, $J$ = 7.4 Hz, 4H), 2.71 - 2.63 (m, 4H), 2.42 (s, 3H), 1.94 (tt, $J$ = 7.4 and 7.4 Hz, 4H), 1.40 (s, 6H).

**Step 3: Chiral separation**

The product obtained as described in the previous step (70 mg) was resolved by Chiral-Prep-HPLC using the following conditions: Column, ChiralPak ID, 2*25 cm, 5 um; mobile phase, Hex and EtOH (hold 20% EtOH over 18 min); Flow rate, 20 mL/min; Detector, UV 254/220 nm. This resulted in 26.8 mg of Example 2 (front peak, 99% ee) as a white solid and 27.7 mg (second peak, 99.3% ee) of Example 3 as a white solid.

**Example 2:** MS-ESI: 418.0 (M+1). $^1H$ NMR (400 MHz, DMSO-$d_6$) $\delta$ 8.36 (s, 1H), 7.57 (s, 2H), 6.87 (s, 1H), 6.85 (s, 1H), 5.03 (s, 1H), 2.78 (t, $J$ = 7.2 Hz, 4H), 2.73 - 2.60 (m, 4H), 2.41 (s, 3H), 1.93 (tt, $J$ = 7.2 and 7.2 Hz, 4H), 1.39 (s, 6H).

**Example 3:** MS-ESI: 418.0 (M+1). $^1H$ NMR (400 MHz, DMSO-$d_6$) $\delta$ 8.39 (s, 1H), 7.58 (s, 2H), 6.87 (s, 1H), 6.85 (s, 1H), 5.03 (s, 1H), 2.78 (t, $J$ = 7.2 Hz, 4H), 2.73 - 2.60 (m, 4H), 2.41 (s, 3H), 1.93 (tt, $J$ = 7.2 and 7.2 Hz, 4H), 1.39 (s, 6H).

Single crystal X-ray crystallographic analysis was performed on compound **181a.** FIG 1 shows ball and stick models of the asymmetrical unit containing two crystallographically independent molecules of compound **181a,** with hydrogen atoms omitted for clarity. Table M below shows fractional atomic coordinates of compound **181a.**

Table M.

| Fractional Atomic Coordinates (×10⁴) and Equivalent Isotropic Displacement Parameters (Å²×10³) for Example 2. $U_{eq}$ is defined as 1/3 of the trace of the orthogonalised $U_{IJ}$ tensor. | | | | |
|---|---|---|---|---|
| **Atom** | ***x*** | ***y*** | ***z*** | **U(eq)** |
| S1 | 722.5(7) | 5368.3(5) | 6903.3(4) | 14.52(18) |
| S2 | 4304.8(7) | 505.4(5) | 3262.9(4) | 16.15(18) |
| O1 | 2143(2) | 6680.8(16) | 8220.2(13) | 16.1(4) |
| O2 | -195(2) | 4624.4(17) | 6478.0(14) | 21.9(5) |
| O5 | 2874(2) | 1624.4(17) | 1805.2(15) | 22.8(5) |
| O6 | 5238(2) | -141.6(18) | 3795.4(15) | 25.6(5) |
| O3 | 1492(3) | 5769.7(18) | 5397.8(14) | 25.5(5) |
| O7 | 2974(2) | 2151.0(17) | 3638.1(14) | 24.6(5) |
| N1 | 51(2) | 7218.5(19) | 8513.8(16) | 14.6(5) |
| N2 | 59(3) | 5986.0(18) | 7536.5(16) | 15.3(5) |
| O4 | 2422(3) | 8513(2) | 4297.8(17) | 34.3(6) |
| N4 | 4956(2) | 2247(2) | 1576.1(16) | 16.9(5) |
| O8 | 2771(3) | 3430(2) | 6070.3(18) | 36.7(6) |
| N5 | 4980(3) | 1071.7(19) | 2602.6(17) | 16.6(5) |
| N3 | 2120(3) | 4817(2) | 7347.5(17) | 16.3(5) |
| C13 | 854(3) | 6633(2) | 8105.0(18) | 12.9(6) |
| C1 | 605(3) | 7947(2) | 9133.7(19) | 14.4(6) |
| N6 | 2978(3) | -121(2) | 2801.8(19) | 20.2(6) |
| C22 | 4388(3) | 2952(2) | 936.5(19) | 16.2(6) |
| C24 | 5733(3) | 2203(2) | -207(2) | 18.3(6) |
| C34 | 4164(3) | 1656(2) | 1979(2) | 16.6(6) |
| C11 | -695(3) | 7200(2) | 10304.5(19) | 17.2(6) |
| C12 | 267(3) | 7915(2) | 9953.6(19) | 14.2(6) |
| C23 | 4754(3) | 2918(2) | 127(2) | 17.0(6) |
| C27 | 4221(3) | 3614(2) | -494(2) | 18.1(6) |
| C8 | 800(3) | 8626(2) | 10566(2) | 17.0(6) |
| C28 | 3315(3) | 4357(2) | -324(2) | 18.6(6) |
| C4 | 2436(4) | 10034(2) | 8218(2) | 23.3(7) |
| C7 | 1688(3) | 9377(2) | 10382(2) | 16.9(6) |
| C29 | 2969(3) | 4399(2) | 492(2) | 18.0(6) |
| C9 | 237(3) | 8445(2) | 11388(2) | 20.4(6) |
| C38 | 2557(3) | 2633(3) | 4320(2) | 24.9(7) |
| C2 | 1458(3) | 8717(2) | 8931.9(19) | 15.1(6) |
| C6 | 2005(3) | 9409(2) | 9557(2) | 17.2(6) |
| C26 | 4804(3) | 3424(2) | -1310(2) | 21.8(7) |
| C31 | 2476(4) | 5023(2) | 1822(2) | 24.4(7) |

(continued)

| Fractional Atomic Coordinates (×10⁴) and Equivalent Isotropic Displacement Parameters (Å²×10³) for Example 2. $U_{eq}$ is defined as 1/3 of the trace of the orthogonalised $U_{IJ}$ tensor. | | | | |
|---|---|---|---|---|
| **Atom** | ***x*** | ***y*** | ***z*** | **U(eq)** |
| C5 | 2927(3) | 10137(2) | 9193(2) | 19.6(6) |
| C16 | 2044(3) | 7389(3) | 5427(2) | 22.4(7) |
| C25 | 5416(4) | 2367(3) | -1181(2) | 24.1(7) |
| C15 | 1514(3) | 7144(2) | 6188(2) | 21.6(6) |
| C33 | 3503(3) | 3713(2) | 1124(2) | 16.9(6) |
| C37 | 3005(3) | 2117(3) | 5067(2) | 23.8(7) |
| C30 | 2028(3) | 5128(2) | 844(2) | 20.5(6) |
| C10 | -360(4) | 7379(2) | 11275(2) | 23.9(7) |
| C36 | 3748(3) | 1285(3) | 4821(2) | 24.5(7) |
| C17 | 2020(4) | 6535(3) | 4974(2) | 28.3(7) |
| C14 | 1181(3) | 6178(2) | 6137.8(19) | 19.0(6) |
| C35 | 3710(3) | 1326(2) | 3973(2) | 23.7(7) |
| C19 | 2583(3) | 8401(3) | 5214(2) | 26.0(7) |
| C3 | 1902(3) | 8960(2) | 8090(2) | 19.0(6) |
| C32 | 3002(3) | 3944(2) | 1954(2) | 21.0(6) |
| C40 | 2768(4) | 2390(3) | 5955(2) | 33.7(8) |
| C20 | 1804(4) | 9231(3) | 5566(3) | 34.6(8) |
| C39 | 1810(4) | 3575(3) | 4092(3) | 35.7(8) |
| C42 | 1313(4) | 2062(3) | 6087(3) | 40.3(9) |
| C21 | 4139(4) | 8447(3) | 5541(3) | 40.0(9) |
| C18 | 2406(5) | 6256(3) | 4130(3) | 44.7(10) |
| C41 | 3893(5) | 1934(4) | 6622(3) | 54.3(12) |

Single crystal X-ray crystallographic analysis was performed on compound **181b.** FIG 2 shows ball and stick models of the asymmetrical unit containing two crystallographically independent molecules of compound **181b,** with hydrogen atoms omitted for clarity. Table N below shows fractional atomic coordinates of compound **181b.**

Table N.

| Fractional Atomic Coordinates (×10⁴) and Equivalent Isotropic Displacement Parameters (Å²×10³) for Example 3. $U_{eq}$ is defined as 1/3 of the trace of the orthogonalised $U_{IJ}$ tensor. | | | | |
|---|---|---|---|---|
| **Atom** | ***x*** | ***y*** | ***z*** | **U(eq)** |
| S1 | 9264.0(7) | 4621.3(5) | 3094.0(4) | 16.15(17) |
| S2 | 5705.1(7) | 9485.8(5) | 6733.7(4) | 19.00(17) |
| O1 | 7853(2) | 3305.2(16) | 1778.9(13) | 18.6(4) |
| O7 | 7027(2) | 7842.4(18) | 6357.2(15) | 26.4(5) |
| O2 | 10182(2) | 5364.5(17) | 3520.0(14) | 23.6(5) |
| O5 | 7131(2) | 8368.0(19) | 8192.5(15) | 25.5(5) |
| O3 | 8512(3) | 4220.7(18) | 4605.0(14) | 26.6(5) |

(continued)

| Fractional Atomic Coordinates (×10[4]) and Equivalent Isotropic Displacement Parameters (Å[2]×10[3]) for Example 3. $U_{eq}$ is defined as 1/3 of the trace of the orthogonalised $U_{IJ}$ tensor. | | | | |
|---|---|---|---|---|
| Atom | *x* | *y* | *z* | U(eq) |
| O6 | 4770(2) | 10133.7(19) | 6200.8(15) | 28.4(5) |
| O8 | 7211(3) | 6563(2) | 3921.7(19) | 38.6(7) |
| O4 | 7597(3) | 1484(2) | 5713.0(18) | 37.3(6) |
| N2 | 9933(3) | 4006.4(19) | 2465.8(16) | 17.8(5) |
| N1 | 9943(2) | 2773(2) | 1482.7(16) | 16.3(5) |
| N4 | 5051(3) | 7745(2) | 8421.8(17) | 20.2(5) |
| N3 | 7870(3) | 5173(2) | 2653.4(17) | 18.4(5) |
| N5 | 5031(3) | 8923(2) | 7390.2(17) | 19.9(5) |
| C14 | 9136(3) | 3353(2) | 1894.3(18) | 15.8(6) |
| C1 | 9391(3) | 2043(2) | 864.7(19) | 17.4(6) |
| N6 | 7031(3) | 10109(2) | 7191.6(19) | 23.0(6) |
| C30 | 5618(3) | 7045(2) | 9058(2) | 19.3(6) |
| C6 | 9205(3) | 1370(2) | -570(2) | 20.1(6) |
| C53 | 7446(4) | 7363(3) | 5675(2) | 26.1(7) |
| C32 | 4273(3) | 7792(2) | 10199(2) | 20.6(6) |
| C2 | 9731(3) | 2078(2) | 44.2(19) | 16.8(6) |
| C43 | 5846(3) | 8333(2) | 8016(2) | 20.2(6) |
| C3 | 10685(3) | 2795(2) | -304(2) | 20.3(6) |
| C37 | 7028(3) | 5597(2) | 9506(2) | 21.2(6) |
| C7 | 8316(3) | 620(2) | -386(2) | 20.2(6) |
| C35 | 5773(3) | 6383(2) | 10493(2) | 20.7(6) |
| C10 | 7573(4) | -36(3) | 1780(2) | 27.4(7) |
| C36 | 6681(3) | 5639(2) | 10322(2) | 21.2(6) |
| C22 | 8481(3) | 2845(3) | 3816(2) | 23.0(6) |
| C8 | 8002(3) | 584(2) | 440(2) | 20.3(6) |
| C39 | 7525(4) | 4977(3) | 8177(2) | 28.0(7) |
| C31 | 5248(3) | 7078(2) | 9867(2) | 19.6(6) |
| C52 | 6981(3) | 7875(3) | 4927(2) | 24.2(7) |
| C12 | 8541(3) | 1280(2) | 1066.4(19) | 18.0(6) |
| C34 | 5191(4) | 6574(3) | 11302(2) | 24.8(7) |
| C51 | 6252(4) | 8707(3) | 5170(2) | 26.7(7) |
| C33 | 4585(4) | 7630(3) | 11175(2) | 27.6(7) |
| C24 | 7990(4) | 3461(3) | 5032(2) | 30.5(8) |
| C23 | 7962(3) | 2603(3) | 4580(2) | 24.0(7) |
| C50 | 6302(4) | 8662(3) | 6020(2) | 25.4(7) |
| C9 | 7077(3) | -142(2) | 804(2) | 23.6(7) |

(continued)

| Fractional Atomic Coordinates (×$10^4$) and Equivalent Isotropic Displacement Parameters (Å$^2$×$10^3$) for Example 3. $U_{eq}$ is defined as 1/3 of the trace of the orthogonalised $U_{IJ}$ tensor. | | | | |
|---|---|---|---|---|
| **Atom** | ***x*** | ***y*** | ***z*** | **U(eq)** |
| C38 | 7972(3) | 4873(2) | 9155(2) | 23.5(7) |
| C5 | 9763(4) | 1551(3) | -1391(2) | 24.9(7) |
| C41 | 6502(3) | 6286(2) | 8872(2) | 20.5(6) |
| C21 | 8811(3) | 3816(2) | 3866.6(19) | 20.4(6) |
| C4 | 10356(4) | 2619(3) | -1277(2) | 28.3(7) |
| C11 | 8099(3) | 1036(2) | 1909(2) | 22.6(6) |
| C40 | 7006(3) | 6055(3) | 8044(2) | 24.5(6) |
| C25 | 7419(4) | 1599(3) | 4793(2) | 26.8(7) |
| C58 | 8189(4) | 6425(3) | 5905(3) | 37.6(9) |
| C54 | 7221(4) | 7601(3) | 4036(2) | 34.1(8) |
| C27 | 8195(4) | 774(3) | 4438(3) | 38.1(9) |
| C29 | 7607(6) | 3737(3) | 5874(3) | 46.7(10) |
| C56 | 8674(4) | 7924(3) | 3907(3) | 42.4(10) |
| C28 | 5872(4) | 1551(3) | 4471(3) | 44.2(10) |
| C57 | 6101(6) | 8060(4) | 3369(3) | 58.9(14) |

**Example 4**

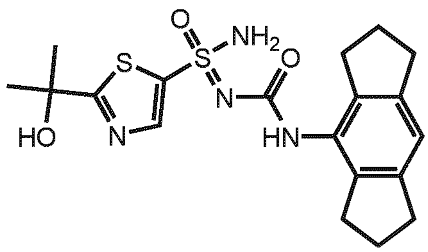

**Example 4 (101'):** N'-(1,2,3,5,6,7-hexahydro-*s*-indacen-4-ylcarbamoyl)-2-(2-hydroxypropan-2-yl)thiazole-5-sulfonimidamide

**Example 4** (above) was synthesized according to the general methods in **Schemes 2 and 3,** as illustrated in **Route 1** and **Route 2** below.

**Examples 5 and 6**

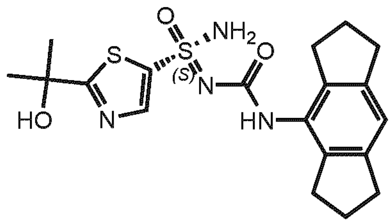
+
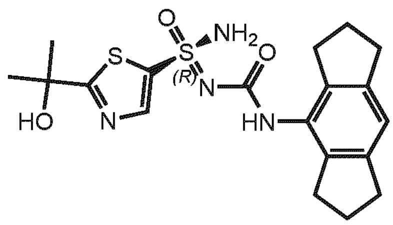

**Examples 5 and a (stereochemistry not assigned)**

**Examples 5 (101) and 6 (102):** (S)- and (R)-N'-(1,2,3,5,6,7-hexahvdro-s-indacen-4-ylcarbamoyl)-2-(2-hydroxypropan-2-yl)thiazole-5-sulfonimidamide

**Examples 5 and 6** (above) were synthesized according to general methods shown in **Schemes 2 and 3,** as illustrated in **Route 1** and **Route 2** below.

**Example 7**

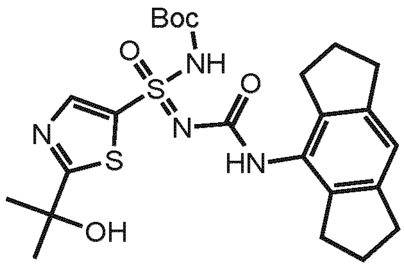

**Example 7 (194):** Tert-butyl N-(1,2,3,5,6,7-hexahydro-s-indacen-4-ylcarbamoyl)-2-(2-hydroxypropan-2-yl)thiazole-5-sulfonimidoylcarbamate

**Example 7** was synthesized according to general method shown in Scheme 3, as illustrated in **Route 2** below.

**Route 1**

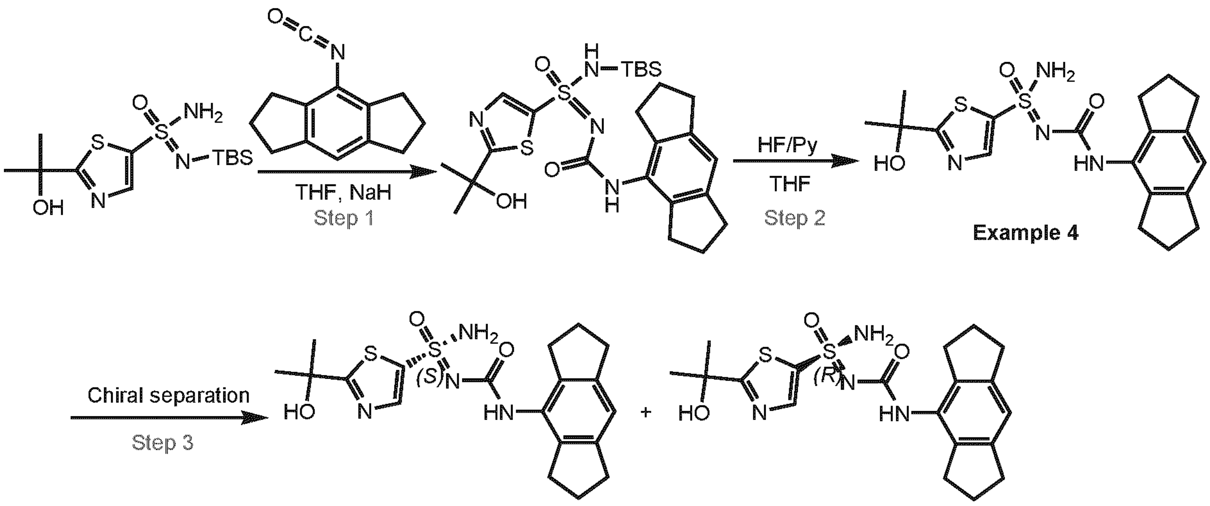


**Example 4**

**Examples 5 and 6 (stereochemistry not assigned)**

**Step 1: N-(*tert*-butyldimethylsilyl)-N'-(1,2,3,5,6,7-hexahydro-s-indacen-4-ylcarbamoyl)-2-(2-hydroxypropan-2-yl)thiazole-5-sulfonimidamide**

Into a 50-mL round-bottom flask was placed a solution of N'-(tert-butyldimethylsilyl)-2-(2-hydroxypropan-2-yl)thiazole-5-sulfonimidamide (336 mg, 1.0 mmol) in THF (10 mL). To this solution was added NaH (60% wt, 80 mg, 2.0 mmol) in portions at 0°C. The solution was stirred at 0°C for 15 minutes, and this was followed by the addition of a solution of 4-isocyanato-1,2,3,5,6,7-hexahydro-s-indacene (209 mg, 1.1 mmol) in THF (5 mL) dropwise with stirring at RT. The resulting solution was stirred for 12 h at RT. The reaction was then quenched by the addition of 10 mL of $NH_4Cl$ (sat.). The resulting solution was extracted with 3x10 mL of DCM and the combined organic layers were concentrated under vacuum. This resulted in 535 mg (crude) of the title compound as a brown oil. MS-ESI: 535.0 (M+1).

**Step 2: N'-(1,2,3,5,6,7-hexahydro-s-indacen-4-ylcarbamoyl)-2-(2-hydroxypropan-2-yl)thiazole-5-sulfonimidamide**

Into a 50-mL round-bottom flask was placed a solution of N-(tert-butyldimethylsilyl)-N'-(1,2,3,5,6,7-hexahydro-s-indacen-4-ylcarbamoyl)-2-(2-hydroxypropan-2-yl)thiazole-5-sulfonimidamide (535 mg, crude, 1.0 mmol) in THF (10 mL). To this solution was added HF/Py (70% wt, 143 mg, 5.0 mmol) dropwise at 0°C. The solution was stirred at RT for 4 h. The reaction was then quenched by the addition of 10 mL of water. The resulting solution was extracted with 3x10 mL of ethyl acetate and the combined organic layers were concentrated under vacuum. The crude product was purified by Prep-HPLC using Method E with ACN/water (20% to 60% in 10 minutes). This resulted in 189 mg (45%, 2 steps) of Example 4 as a white solid.

**Example 4:** MS-ESI: 421.0 (M+1). $^1$H NMR (400 MHz, DMSO-$d_6$) $\delta$ 8.46 (br s, 1H), 8.04 (s, 1H), 7.80 (br s, 2H), 6.86 (s, 1H) 6.28 (s, 1H), 2.88 - 2.71 (m, 4H), 2.71 - 2.56 (m, 4H), 2.02-1.80 (m, 4H), 1.49 (s, 6H).

**Step 2: Chiral separation.**

The product obtained as described in the previous step (189 mg) was resolved by Chiral-Prep-HPLC using the following conditions: Column, CHIRAL Cellulose-SB, 2*25 cm, 5 um; mobile phase, Hex (0.1%DEA) and EtOH (hold 20% EtOH over 16 min); Flow rate, 20 mL/min; Detector, UV 254/220 nm. This resulted in 70 mg of Example 5 (front peak, 99% ee **101**) as a white solid and 65 mg of Example 6 (second peak, 97.5% ee **102**) as a white solid. Absolute stereochemistry of these two isomers has not been assigned.

**Example 5:** MS-ESI: 421.0 (M+1). $^1$H NMR (400 MHz, DMSO-$d_6$) $\delta$ 8.43 (br s, 1H), 8.05 (s, 1H), 7.83 (br s, 2H), 6.87 (s, 1H) 6.29 (s, 1H), 2.82 - 2.71 (m, 4H), 2.71 - 2.56 (m, 4H), 2.02 - 1.80 (m, 4H), 1.50 (s, 6H).

**Example 6:** MS-ESI: 421.0 (M+1). $^1$H NMR (400 MHz, DMSO-$d_6$) $\delta$ 8.41 (br s, 1H), 8.05 (s, 1H), 7.83 (s, 2H), 6.87 (s, 1H) 6.27 (s, 1H), 2.82 - 2.71 (m, 4H), 2.71 - 2.56 (m, 4H), 2.02 - 1.80 (m, 4H), 1.50 (s, 6H).

**Route 2:**

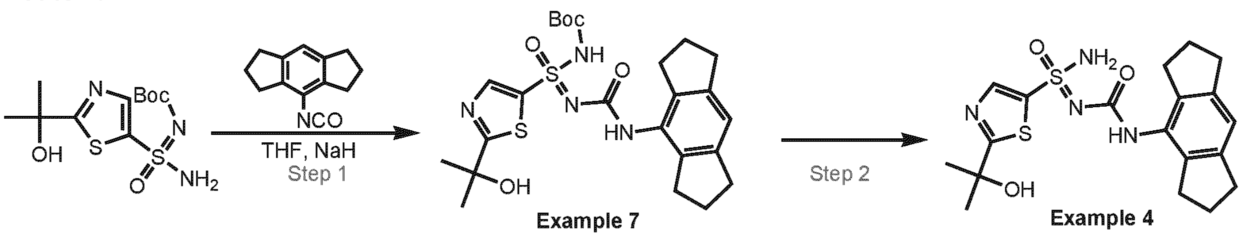


Example 7

Example 4

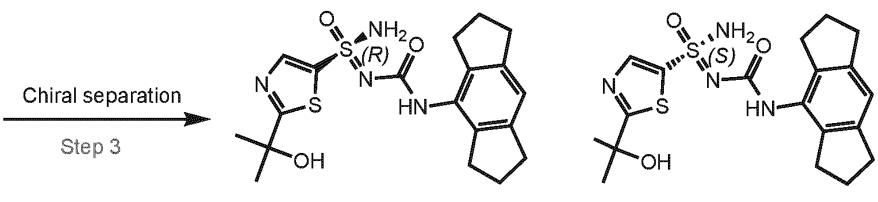


**Examples 5 and 6 (stereochemistry not assigned)**

**Step 1: Tert-butyl N-(1,2,3,5,6,7-hexahydro-s-indacen-4-ylcarbamoyl)-2-(2-hydroxypropan-2-yl)thiazole-5-sulfonimidoylcarbamate**

Tert-butyl (amino(2-(2-hydroxypropan-2-yl)thiazol-5-yl)(oxo)-$\lambda^6$-sulfaneylidene)carbamate (12 g, 37 mmol) was dissolved in dried THF (200 mL). To the solution was added NaH (17.7 g, 60%, 44 mmol) in portions at 0°C under nitrogen atmosphere, and then the mixture was stirred at 0°C for 0.5 h. Freshly prepared 4-isocyanato-1,2,3,5,6,7-hexahydro-s-indacene (7.4 g, 37 mmol) was dissolved in dried THF (50 mL) and the solution was added to the front mixture dropwise at 0°C. The mixture was stirred at RT for 1 h. The reaction was quenched with ice-water (100 mL), and the pH value of the resulting solution was adjusted to 6 with $HCO_2H$. The solution was extracted with EtOAc (3x200 mL) and the combined organic layers were dried over anhydrous $Na_2SO_4$ and concentrated to give 17.5 g of Example 7 as a crude grey solid.

**Example 7:** MS-ESI: 521.0 (M+1). $^1H$ NMR (300 MHz, MeOD-$d_4$) δ 8.14 (s, 1H), 6.89 (s, 1H), 3.00 - 2.60 (m, 8H), 2.20 - 1.90 (m, 4H), 1.51 (s, 6H), 1.37 (s, 9H).

**Step 2: N'-(1,2,3,5,6,7-hexahydro-s-indacen-4-ylcarbamoyl)-2-(2-hydroxypropan-2-yl)thiazole-5-sulfonimidamide**

The crude tert-butyl (N-((1,2,3,5,6,7-hexahydro-s-indacen-4-yl)carbamoyl)-2-(2-hydroxypropan-2-yl)-thiazole-5-sulfonimidoyl)carbamate (crude 17.5 g) was dissolved in THF (200 mL). To the solution was added HCl (200 mL, 4M in 1,4-dioxane) at RT. The mixture was stirred at RT overnight and concentrated. The residue was purified with $SiO_2$-gel column and eluted with MeOH/DCM (5%) and further purified by reverse column with MeOH/water (50% to 80% in 50 minutes) to give 12 g of **Example 4** (51%, 2 steps) as a white solid.

**Example 4:** MS-ESI: 421.0 (M+1). $^1H$ NMR (400 MHz, DMSO-$d_6$) δ 8.46 (br s, 1H), 8.04 (s, 1H), 7.80 (br s, 2H), 6.86 (s, 1H) 6.28 (s, 1H), 2.88 - 2.71 (m, 4H), 2.71 - 2.56 (m, 4H), 2.02-1.80 (m, 4H), 1.49 (s, 6H).

**Step 3: Chiral separation.**

The product obtained as described in the previous step (12 g) was resolved by Chiral-Prep-SFC using the following conditions: Column, CHIRALPAK IF, 2*25cm, 5um; Mobile Phase A: $CO_2$: 60, Mobile Phase B: MeOH (2mM $NH_3$-MeOH): 40; Flow rate: 40 mL/min; Detector, UV 220 nm. This resulted in 3.8 g of Example 6 (front peak, 99% ee **102**) as a white solid and 4.6 g of Example 5 (second peak, 97.5% ee **101**) as a white solid. Absolute stereochemistry of these two isomers has not been assigned.

**Example 5:** MS-ESI: 421.0 (M+1). $^1H$ NMR (400 MHz, DMSO-$d_6$) δ 8.43 (br s, 1H), 8.05 (s, 1H), 7.83 (br s, 2H), 6.87 (s, 1H) 6.29 (s, 1H), 2.82 - 2.71 (m, 4H), 2.71 - 2.56 (m, 4H), 2.02-1.80 (m, 4H), 1.50 (s, 6H).

**Example 6:** MS-ESI: 421.0 (M+1). $^1H$ NMR (400 MHz, DMSO-$d_6$) δ 8.41 (br s, 1H), 8.05 (s, 1H), 7.83 (s, 2H), 6.87 (s, 1H) 6.27 (s, 1H), 2.82 - 2.71 (m, 4H), 2.71 - 2.56 (m, 4H), 2.02 - 1.80 (m, 4H), 1.50 (s, 6H).

**Example 8**

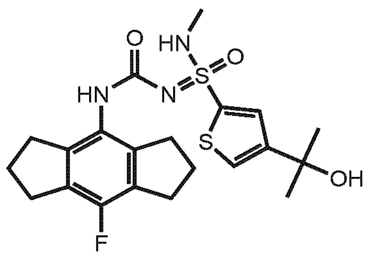

**Example 8 (270):** N'-(8-fluoro-1,2,3,5,6,7-hexahydro-*s*-indacen-4-ylcarbamoyl)-4-(2-hydroxypropan-2-yl)-N-methylthiophene-2-sulfonimidamide (Scheme 4)

**Example 8** was synthesized according to the general method shown in **Scheme 4.**

Into a 50-mL round-bottom flask purged with and maintained under nitrogen was placed a solution of 4-fluoro-8-isocyanato-1,2,3,5,6,7-hexahydro-s-indacene (110 mg, 0.51 mmol) in DCM (5 mL). To the solution were added TEA (153 mg, 1.51 mmol) and 4-(2-hydroxypropan-2-yl)-N'-methylthiophene-2-sulfonimidamide (120 mg, 0.51 mmol). The

resulting solution was stirred for 14 h at RT and then was concentrated under vacuum. The crude product was purified by Prep-HPLC using method E eluted with a gradient of 30~74% ACN. This resulted in 80 mg (35%) of Example 8 as a white solid.

**Example 8:** MS-ESI: 450.1 (M-1). $^1$H NMR (400 MHz, DMSO-$d_6$) δ 8.50 (br s, 1H), 7.64 (s, 1H), 7.59-7.50 (m, 2H), 5.23 (s, 1H), 2.84-2.69 (m, 8H), 2.50 (s, 3H), 1.99 (t, $J$ = 7.2 Hz, 4H), 1.42 (d, $J$ = 2.8 Hz, 6H)

**Example 9 (204)**

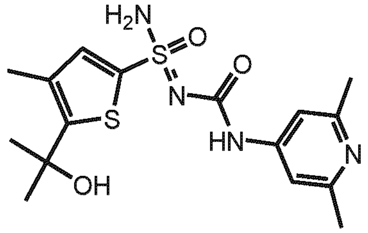

N'-((2,6-dimethylpyridin-4-yl)carbamoyl)-4-methyl-5-(2-hydroxypropan-2-yl)thiophene-2-sulfonimidamide (Scheme 5)

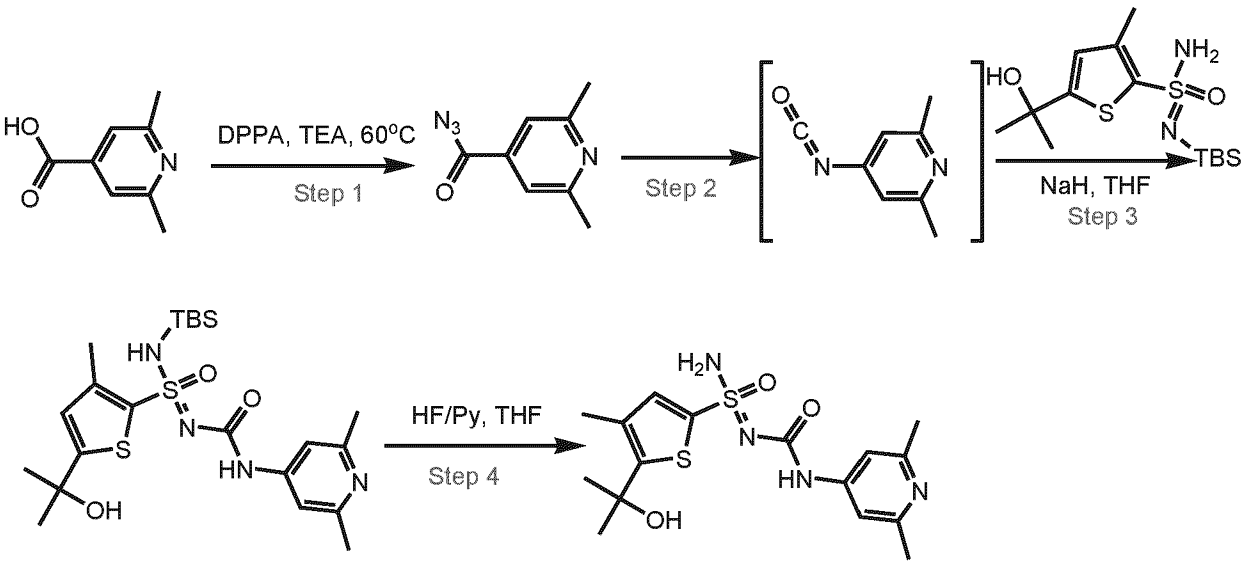


**Example 9**

**Step 1: 4-Azido-2,6-dimethylpyridine**

To the solution of 2,6-dimethylpyridine-4-carboxylic acid (151 mg, 1.0 mmol) in dried toluene (15 mL). To the solution was added DPPA (825 mg, 3.0 mmol) and TEA (303 mg, 3.0 mmol). The mixture was stirred at 60°C for 4 h. The solution was concentrated under vacuum. This gave 900 mg (crude) of the title compound as yellow oil.

Step 2 & 3: N-(tert-butyldimethylsilyl)-N'-((2,6-dimethylpyridin-4-yl)carbamoyl)-5-(2-hydroxypropan-2-yl)-3-methylthiophene-2-sulfonimidamide

The 4-azido-2,6-dimethylpyridine (900 mg, crude) was dissolved in THF (20 mL). To the solution was added N'-(tert-butyldimethylsilyl)-5-(2-hydroxypropan-2-yl)-3-methylthiophene-2-sulfonimidamide (349 mg, 1.0 mmol) and NaOH (120 mg, 3.0 mmol). The mixture was stirred at 50°C for 12 h. The solution was diluted with water 20 mL, then the resulting solution was extracted with 3×20 mL of ethyl acetate. The organic layers were combined, dried over anhydrous $Na_2SO_4$, then concentrated under vacuum. This gave 500 mg (crude) of the title compound as a yellow solid. MS-ESI: 497.0 (M+1).

**Step 4: N'-((2,6-dimethylpyridin-4-yl)carbamoyl)-4-methyl-5-(2-hydroxypropan-2-yl)thiophene-2-sulfonimidamide**

Into a 50-mL round-bottom flask was placed a solution of N-(*tert*-butyldimethylsilyl)-N'-((2,6-dimethylpyridin-4-yl)carbamoyl)-4-methyl-5-(2-hydroxypropan-2-yl)thiophene-2-sulfonimidamide (500 mg, crude) in THF (10 mL), to this solution was added HF/Py (70% wt, 143 mg, 5.0 mmol) dropwise at 0°C. The solution was stirred at RT for 4 h. The reaction was then quenched by the addition of 10 mL of water. The resulting solution was extracted with 3x10 mL of ethyl acetate and the combined organic layers were concentrated under vacuum. The crude product was purified by Prep-HPLC using method E eluted with a gradient of ACN/water (10% to 30% in 10 minutes). This resulted in 15 mg (4%, 4 steps) of Example 9 as a white solid. MS-ESI: 383.0 (M+1). $^{1}H$ NMR (300 MHz, DMSO-$d_6$) $\delta$ 9.31 (s, 1H), 7.53 (br s, 2H), 7.31 (s, 1H), 7.14 (s, 2H), 5.81 (s, 1H), 2.28 (s, 6H), 2.23 (s, 3H), 1.50 (s, 6H).

**Table 16.** Examples in the following table were prepared using similar conditions as described in **Example 1** and **Scheme 1** from appropriate starting materials.

| Example # | Final Target Number | Structure | IUPAC Name | Exact Mass $[M+H]^+$ |
|---|---|---|---|---|
| 10 | 180 | 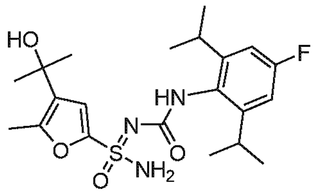 | N'-(4-fluoro-2,6-diisopropylphenylcarbamoyl)-4-(2-hydroxypropan-2-yl)-5-methylfuran-2-sulfonimidamide | 440.2 |
| 11 | 190 | 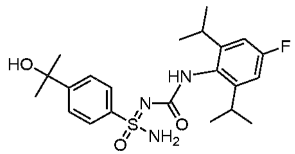 | N'-(4-fluoro-2,6-diisopropylphenylcarbamoyl)-4-(2-hydroxypropan-2-yl) benzenesulfonimidamide | 436.2 |
| 12 | 182 | 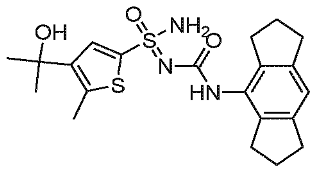 | N'-(1,2,3,5,6,7-hexahydro-s-indacen-4-ylcarbamoyl)-4-(2-hydroxypropan-2-yl)-5-methylthiophene-2-sulfonimidamide | 434.1 |
| 13 | 191 | 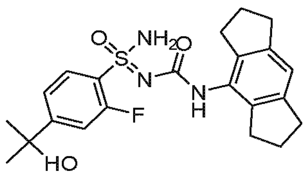 | 2-fluoro-N'-(1,2,3,5,6,7-hexahydro-*s*-indacen-4-ylcarbamoyl)-4-(2-hydroxypropan-2-yl)benzenesulfonimidamide | 432.2 |
| 14 | 177 | 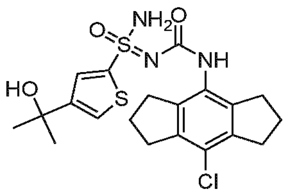 | N'-(8-chloro-1,2,3,5,6,7-hexahydro-*s*-indacen-4-ylcarbamoyl)-4-(2-hydroxypropan-2-yl)thiophene-2-sulfonimidamide | 452.0(M-1) |

(continued)

| Example # | Final Target Number | Structure | IUPAC Name | Exact Mass $[M+H]^+$ |
|---|---|---|---|---|
| 15 | 185 | 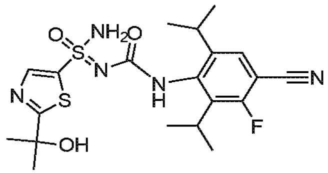 | N'-(4-cyano-3-fluoro-2,6-diisopropylphenylcarbamoyl)-2-(2-hydroxypropan-2-yl)thiazole-5-sulfonimidamide | 468.2 |
| 16 | 186 | 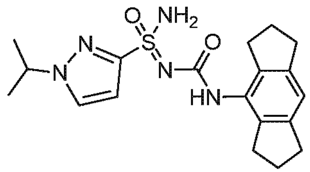 | N'-(1,2,3,5,6,7-hexahydro-s-indacen-4-ylcarbamoyl)-1-isopropyl-1H-pyrazole-3-sulfonimidamide | 388.1 |
| 17 | 187 | 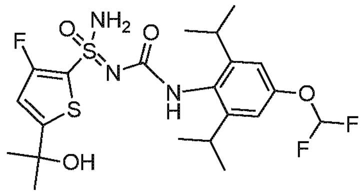 | N'-(4-(difluoromethoxy)-2,6-diisopropylphenylcarbamoyl)-3-fluoro-5-(2-hydroxypropan-2-yl)thiophene-2-sulfonimidamide | 508.2 |
| 18 | 188 | 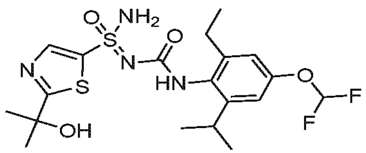 | N'-(4-(difluoromethoxy)-2-ethyl-6-isopropylphenylcarbamoyl)-2-(2-hydroxypropan-2-yl)thiazole-5-sulfonimidamide | 477.1 |
| 19 | 192 | 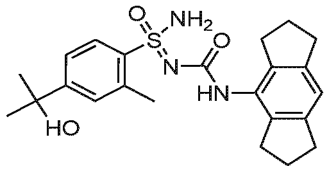 | N'-(1,2,3,5,6,7-hexahydro-s-indacen-4-ylcarbamoyl)-4-(2-hydroxypropan-2-yl)-2-methylbenzenesulfonimidamide | 426.2(M-1) |

(continued)

| Example # | Final Target Number | Structure | IUPAC Name | Exact Mass $[M+H]^+$ |
|---|---|---|---|---|
| 20 | 189 | 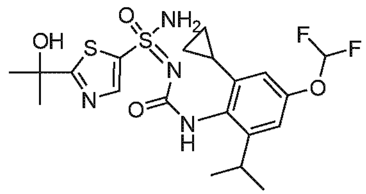 | N'-(2-cyclopropyl-4-(difluoromethoxy)-6-isopropylphenylcarbamoyl)-2-(2-hydroxypropan-2-yl)thiazole-5-sulfonimidamide | 487.1(M-1) |
| 21 | 178 | 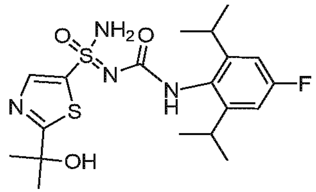 | N'-(4-fluoro-2,6-diisopropylphenylcarbamoyl)-2-(2-hydroxypropan-2-yl) thiazole-5-sulfonimidamide | 441.1(M-1) |
| 22 | 193 | 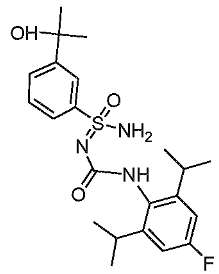 | N'-(4-fluoro-2,6-diisopropylphenylcarbamoyl)-3-(2-hydroxypropan-2-yl) benzenesulfonimidamide | 436.1 |
| 23 | 170 | 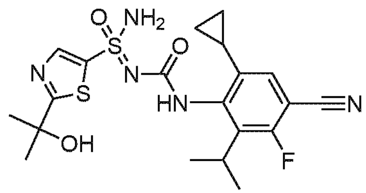 | N'-(4-cyano-6-cyclopropyl-3 -fluoro-2-isopropylphenylcarbamoyl)-2-(2-hydroxypropan-2-yl)thiazole-5-sulfonimidamide | 466.1 |

(continued)

| Example # | Final Target Number | Structure | IUPAC Name | Exact Mass [M+H]+ |
|---|---|---|---|---|
| 24 | 168 | 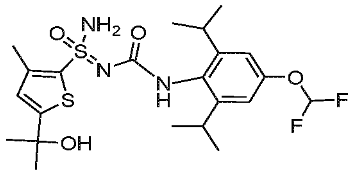 | N'-(4-(difluoromethoxy)-2,6-diisopropylphenylcarbamoyl)-5-(2-hydroxypropan-2-yl)-3 -methylthiophene-2-sulfonimidamide | 504.3 |
| 25 | 171 | 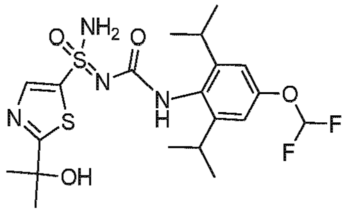 | N'-(4-(difluoromethoxy)-2,6-diisopropylphenylcarbamoyl)-2-(2-hydroxypropan-2-yl)thiazole-5-sulfonimidamide | 491.1 |
| 26 | 122 | 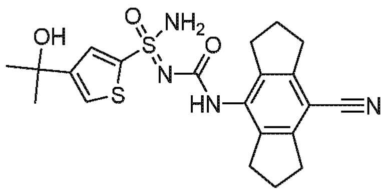 | N'-(8-cyano-1,2,3,5,6,7-hexahydro-*s*-indacen-4-ylcarbamoyl)-4-(2-hydroxypropan-2-yl)thiophene-2-sulfonimidamide | 443.1 (M-1) |
| 27 | 120 | 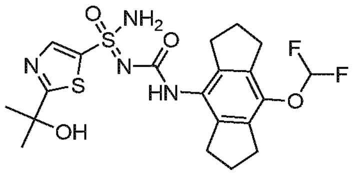 | N'-(8-(difluoromethoxy)-1,2,3,5,6,7-hexahydro-*s*-indacen-4-ylcarbamoyl)-2-(2-hydroxypropan-2-yl)thiazole-5-sulfonimidamide | 487.1 |
| 28 | 125 | 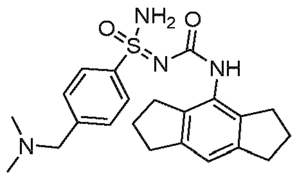 | 4-((dimethylamino)methyl)-N'-(1,2,3,5,6,7-hexahydro-*s*-indacen-4-ylcarbamoyl)benzenesulfonimidamide | 413.3 |

(continued)

| Example # | Final Target Number | Structure | IUPAC Name | Exact Mass $[M+H]^+$ |
|---|---|---|---|---|
| 29 | 129 | 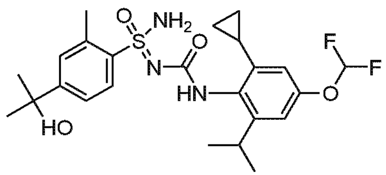 | N'-(2-cyclopropyl-4-(difluoromethoxy)-6-isopropylphenylcarbamoyl)-4-(2-hydroxypropan-2-yl)-2-methylbenzenesulfonimidamide | 496.2 |
| 30 | 213 | 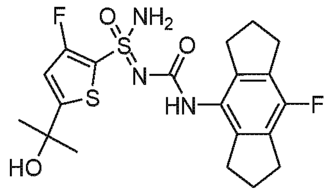 | 3-fluoro-N'-(8-fluoro-1,2,3,5,6,7-hexahydro-*s*-indacen-4-ylcarbamoyl)-5-(2-hydroxypropan-2-yl)thiophene-2-sulfonimidamide | 456.1 |
| 31 | 207 | 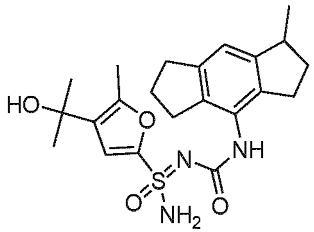 | 4-(2-hydroxypropan-2-yl)-5-methyl-N'-((3-methyl-1,2,3,5,6,7-hexahydro-s-indacen-4-yl)carbamoyl)furan-2-sulfonimidamide | 432.2 |
| 32 | 195 | 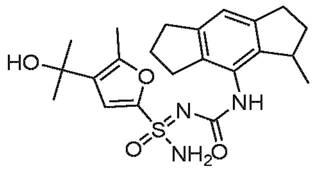 | 4-(2-hydroxypropan-2-yl)-5-methyl-N'-((1-methyl-1,2,3,5,6,7-hexahydro-s-indacen-4-yl)carbamoyl)furan-2-sulfonimidamide | 432.2 |

**Table 17.** Examples in the following table were prepared using similar conditions as described in **Example 4 - route 1** and **Scheme 2** from appropriate starting materials.

| Example # | Final Target Number | Structure | IUPAC Name | Exact Mass $[M+H]^+$ |
|---|---|---|---|---|
| 33 | 179 | 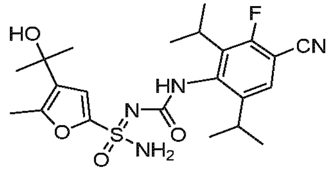 | N'-(4-cyano-3-fluoro-2,6-diisopropylphenylcarbamoyl)-4-(2-hydroxypropan-2-yl)-5-methylfuran-2-sulfonimidamide | 465.2 |
| 34 | 105 | 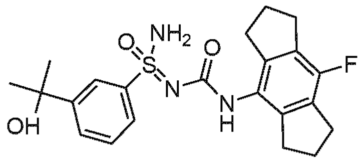 | N'-(8-fluoro-1,2,3,5,6,7-hexahydro-s-indacen-4-ylcarbamoyl)-3-(2-hydroxypropan-2-yl)benzenesulfonimidamide | 432.2 |
| 35 | 121 | 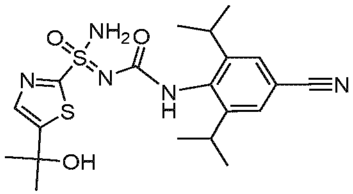 | N'-(4-cyano-2,6-diisopropylphenylcarbamoyl)-5-(2-hydroxypropan-2-yl) thiazole-2-sulfonimidamide | 448.1 (M-1) |
| 36 | 145 | 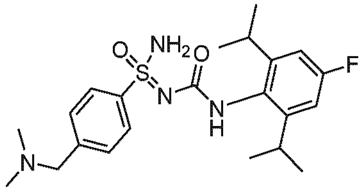 | 4-((dimethylamino)methyl)-N'-(4-fluoro-2,6-diisopropylphenylcarbamoyl) benzenesul fonimidamide | 435.2 |
| 37 | 131 | 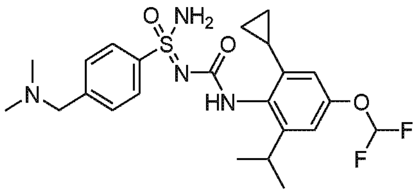 | N'-(2-cyclopropyl-4-(difluoromethoxy)-6-isopropylphenylcarbamoyl)-4-((dimethylamino)methyl)benzenesulfoni midamide | 481.3 |

(continued)

| Example # | Final Target Number | Structure | IUPAC Name | Exact Mass [M+H]+ |
|---|---|---|---|---|
| 38 | 132 | 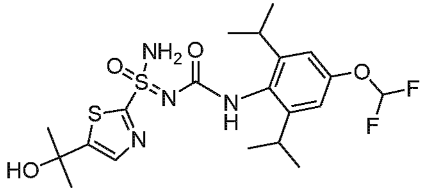 | N'-(4-(difluoromethoxy)-2,6-diisopropylphenylcarbamoyl)-5-(2-hydroxypropan-2-yl)thiazole-2-sulfonimidamide | 489.1 (M-1) |
| 39 | 144 | 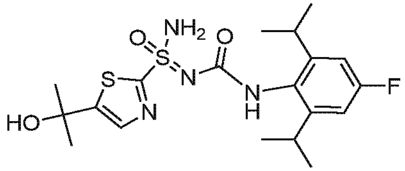 | N'-(4-fluoro-2,6-diisopropylphenylcarbamoyl)-5-(2-hydroxypropan-2-yl) thiazole-2-sulfonimidamide | 441.1 (M-1) |
| 40 | 149 | 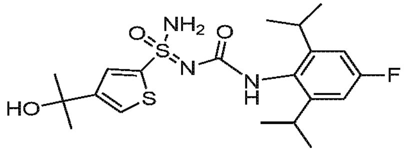 | N'-(4-fluoro-2,6-diisopropylphenylcarbamoyl)-4-(2-hydroxypropan-2-yl) thiophene-2-sulfonimidamide | 440.1 (M-1) |
| 41 | 152 | 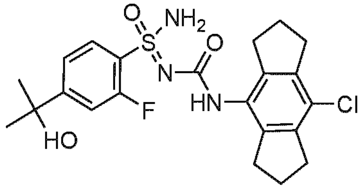 | N'-(8-chloro-1,2,3,5,6,7-hexahydro-*s*-indacen-4-ylcarbamoyl)-2-fluoro-4-(2-hydroxypropan-2-yl)benzenesulfonimidamide | 466.2 |
| 42 | 150 | 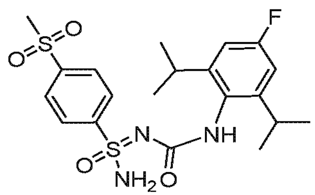 | N'-(4-fluoro-2,6-diisopropylphenylcarbamoyl)-4-(methylsulfonyl) benzenesulfonimidamid e | 454.1 (M-1) |

(continued)

| Example # | Final Target Number | Structure | IUPAC Name | Exact Mass [M+H]+ |
|---|---|---|---|---|
| 43 | 167 | 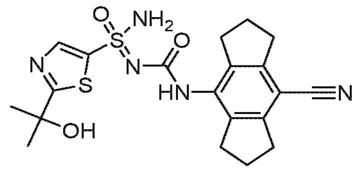 | N'-(8-cyano-1,2,3,5,6,7-hexahydro-*s*-indacen-4-ylcarbamoyl)-2-(2-hydroxypropan-2-yl)thiazole-5-sulfonimidamide | 444.2 (M-1) |
| 44 | 106 | 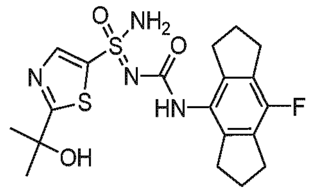 | N'-(8-fluoro-1,2,3,5,6,7-hexahydro-*s*-indacen-4-ylcarbamoyl)-2-(2-hydroxypropan-2-yl)thiazole-5-sulfonimidamide | 437.1 (M-1) |
| 45 | 107 | 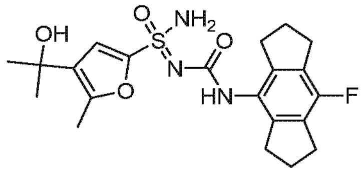 | N'-(8-fluoro-1,2,3,5,6,7-hexahydro-*s*-indacen-4-ylcarbamoyl)-4-(2-hydroxypropan-2-yl)-5-methylfuran-2-sulfonimidamide | 436.2 |
| 46 | 110 | 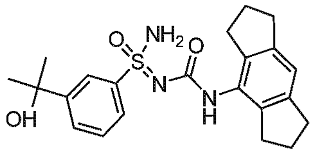 | N'-(1,2,3,5,6,7-hexahydro-*s*-indacen-4-ylcarbamoyl)-3 -(2-hydroxypropan-2-yl)benzenesulfonimidamide | 414.2 |
| 47 | 151 | 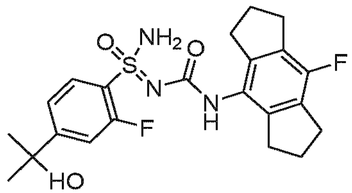 | 2-fluoro-N'-(8-fluoro-1,2,3,5,6,7-hexahydro-s-indacen-4-ylcarbamoyl)-4-(2-hydroxypropan-2-yl)benzenesulfonimidamide | 448.1 (M-1) |

(continued)

| Example # | Final Target Number | Structure | IUPAC Name | Exact Mass $[M+H]^+$ |
|---|---|---|---|---|
| 48 | 154 | 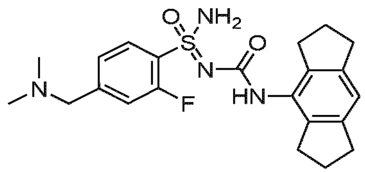 | 4-((dimethylamino)methyl)-2-fluoro-N'-(1,2,3,5,6,7-hexahydro-s-indacen-4-ylcarbamoyl)benzenesulfonimidamide | 431.2 |
| 49 | 148 | 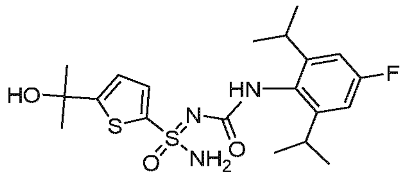 | N'-(4-fluoro-2,6-diisopropylphenylcarbamoyl)-5-(2-hydroxypropan-2-yl) thiophene-2-sulfonimidamide | 442.2 |
| 50 | 153 | 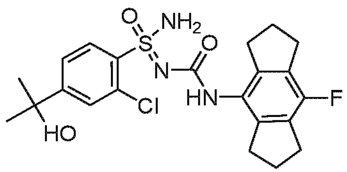 | 2-chloro-N'-(8-fluoro-1,2,3,5,6,7-hexahydro-*s*-indacen-4-ylcarbamoyl)-4-(2-hydroxypropan-2-yl)benzenesulfonimidamide | 464.1 (M-1) |
| 51 | 109 | 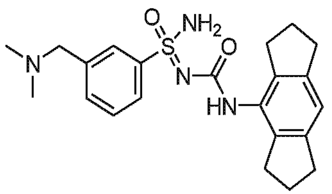 | 3 -((dimethylamino)methyl)-N'-(1,2,3,5,6,7-hexahydro-s-indacen-4-ylcarbamoyl)benzenesulfonimidamide | 411.1 (M-1) |
| 52 | 135 | 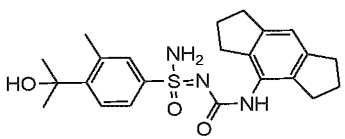 | N'-((1,2,3,5,6,7-hexahydro-s-indacen-4-yl)carbamoyl)-4-(2-hydroxypropan-2-yl)-3-methylbenzenesulfonimidamide | 428.2 |

(continued)

| Example # | Final Target Number | Structure | IUPAC Name | Exact Mass [M+H]+ |
|---|---|---|---|---|
| 53 | 134 | 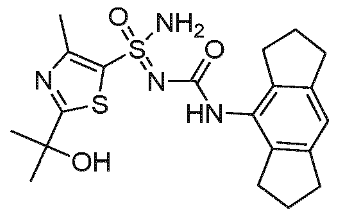 | N'-((1,2,3,5,6,7-hexahydro-s-indacen-4-yl)carbamoyl)-2-(2-hydroxypropan-2-yl)-4-methylthiazole-5-sulfonimidamide | 435.1 |
| 54 | 130 | 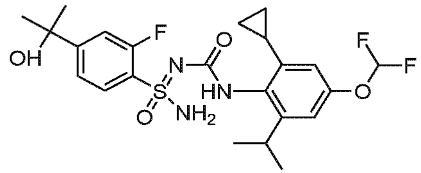 | N'-((2-cyclopropyl-4-(difluoromethoxy)-6-isopropylphenyl)carbamoyl)-2-fluoro-4-(2-hydroxypropan-2-yl)benzenesulfonimidamide | 500.2 |
| 55 | 212 | 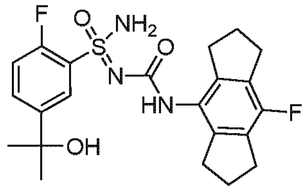 | 2-fluoro-N'-((8-fluoro-1,2,3,5,6,7-hexahydro-s-indacen-4-yl)carbamoyl)-5-(2-hydroxypropan-2-yl)benzenesulfonimidamide | 450.2 |
| 56 | 205 | 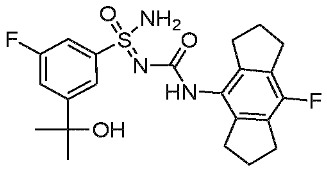 | 3-fluoro-N'-((8-fluoro-1,2,3,5,6,7-hexahydro-s-indacen-4-yl)carbamoyl)-5-(2-hydroxypropan-2-yl)benzenesulfonimidamide | 450.2 |
| 57 | 143 | 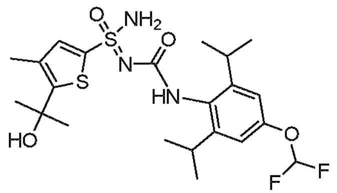 | N'-((4-(difluoromethoxy)-2,6-diisopropylphenyl)carbamoyl)-5-(2-hydroxypropan-2-yl)-4-methylthiophene-2-sulfonimidamide | 504.2 |

(continued)

| Example # | Final Target Number | Structure | IUPAC Name | Exact Mass $[M+H]^+$ |
|---|---|---|---|---|
| 58 | 206 | 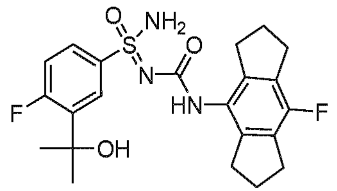 | 4-fluoro-N'-((8-fluoro-1,2,3,5,6,7-hexahydro-s-indacen-4-yl)carbamoyl)-3-(2-hydroxypropan-2-yl)benzenesulfonimidamide | 450.2 |
| 59 | 108 | 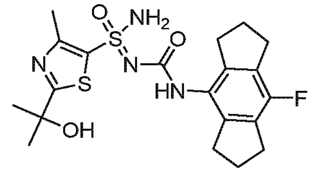 | N'-((8-fluoro-1,2,3,5,6,7-hexahydro-s-indacen-4-yl)carbamoyl)-2-(2-hydroxypropan-2-yl)-4-methylthiazole-5-sulfonimidamide | 453.1 |
| 60 | 202 | 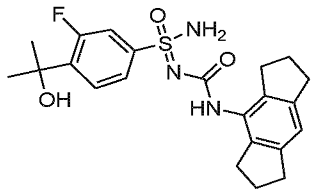 | 3-fluoro-N'-((1,2,3,5,6,7-hexahydro-s-indacen-4-yl)carbamoyl)-4-(2-hydroxypropan-2-yl)benzenesulfonimidamide | 432.2 |
| 61 | 208 | 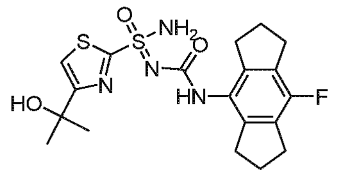 | N'-((8-fluoro-1,2,3,5,6,7-hexahydro-s-indacen-4-yl)carbamoyl)-4-(2-hydroxypropan-2-yl)thiazole-2-sulfonimidamide | 439.1 |
| 62 | 197 | 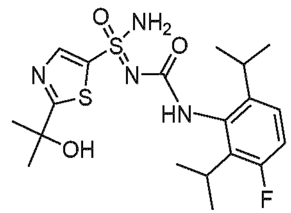 | N'-((3-fluoro-2,6-diisopropylphenyl)carbamoyl)-2-(2-hydroxypropan-2-yl)thiazole-5-sulfonimidamide | 443.2 |

(continued)

| Example # | Final Target Number | Structure | IUPAC Name | Exact Mass [M+H]+ |
|---|---|---|---|---|
| 63 | 196 | 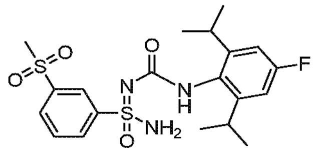 | N'-((4-fluoro-2,6-diisopropylphenyl)carbamoyl)-3 - (methylsulfonyl) benzenesulfonimidamid e | 456.1 |
| 64 | 124 | 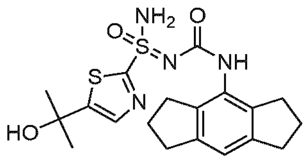 | N'-((1,2,3,5,6,7-hexahydro-s-indacen-4-yl)carbamoyl)-5-(2-hydroxypropan-2-yl)thiazole-2-sulfonimidamide | 421.1 |
| 65 | 173 | 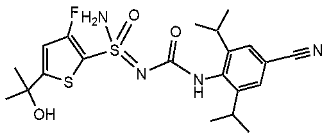 | N'-((4-cyano-2,6-diisopropylphenyl)carbamoyl)-3-fluoro-5-(2-hydroxypropan-2-yl)thiophene-2-sulfonimidamide | 467.2 |
| 66 | 172 | 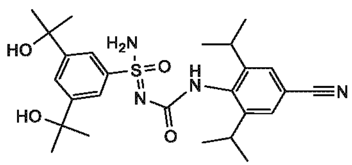 | N'-((4-cyano-2,6-diisopropylphenyl)carbamoyl)-3,5-bis(2-hydroxypropan-2-yl)benzenesulfonimidamide | 501.2 |
| 67 | 174 | 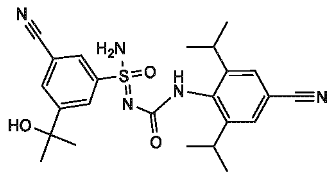 | 3-cyano-N'-((4-cyano-2,6-diisopropylphenyl)carbamoyl)-5-(2-hydroxypropan-2-yl)benzenesulfonimidamide | 468.2 |
| 68 | 158 | 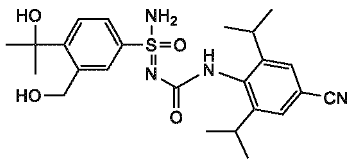 | N'-((4-cyano-2,6-diisopropylphenyl)carbamoyl)-3 - (hydroxymethyl)-4-(2-hydroxypropan-2-yl)benzenesulfonimidamide | 473.2 |

(continued)

| Example # | Final Target Number | Structure | IUPAC Name | Exact Mass $[M+H]^+$ |
|---|---|---|---|---|
| 69 | 220 | 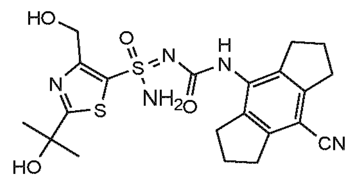 | N'-((8-cyano- 1,2,3,5,6,7-hexahydro-s-indacen-4-yl)carbamoyl)-4-(hydroxymethyl)-2-(2-hydroxypropan-2-yl)thiazole-5-sulfonimidamide | 476.1 |
| 70 | 157 | 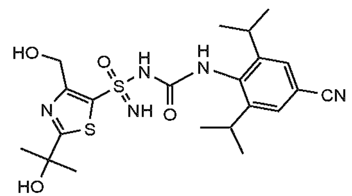 | N-((4-cyano-2,6-diisopropylphenyl)carbamoyl)-4-(hydroxymethyl)-2-(2-hydroxypropan-2-yl)thiazole-5-sulfonimidamide | 480.2 |
| 71 | 161 | 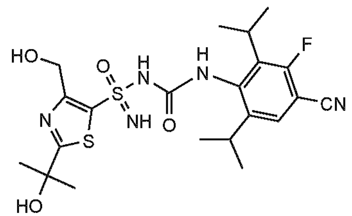 | N-((4-cyano-3-fluoro-2,6-diisopropylphenyl)carbamoyl)-4-(hydroxymethyl)-2-(2-hydroxypropan-2-yl)thiazole-5-sulfonimidamide | 498.2 |
| 72 | 159 | 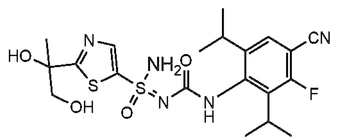 | N'-((4-cyano-3-fluoro-2,6-diisopropylphenyl)carbamoyl)-2-(1,2-dihydroxypropan-2-yl)thiazole-5-sulfonimidamide | 484.1 |
| 73 | 165 | 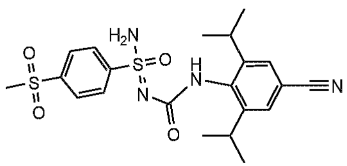 | N'-((4-cyano-2,6-diisopropylphenyl)carbamoyl)-4-(methylsulfonyl) benzenesulfonimidamid e | 463.1 |

(continued)

| Example # | Final Target Number | Structure | IUPAC Name | Exact Mass $[M+H]^+$ |
|---|---|---|---|---|
| 74 | 183 | 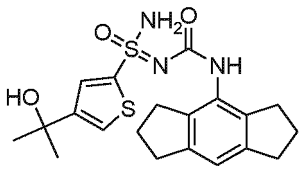 | N'-(1,2,3,5,6,7-hexahydro-s-indacen-4-ylcarbamoyl)-4-(2-hydroxypropan-2-yl)thiophene-2-sulfonimidamide | 418.1(M-1) |
| 75 | 176 | 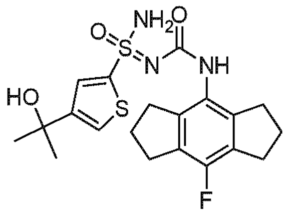 | N'-(8-fluoro-1,2,3,5,6,7-hexahydro-s-indacen-4-ylcarbamoyl)-4-(2-hydroxypropan-2-yl)thiophene-2-sulfonimidamide | 438.0 |
| 76 | 136 | 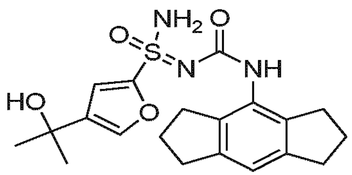 | N'-(1,2,3,5,6,7-hexahydro-s-indacen-4-ylcarbamoyl)-4-(2-hydroxypropan-2-yl)furan-2-sulfonimidamide | 404.2 |
| 77 | 209 | 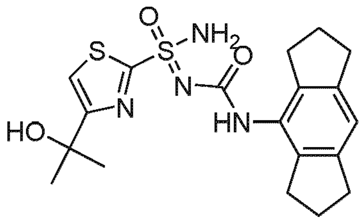 | N'-(1,2,3,5,6,7-hexahydro-s-indacen-4-ylcarbamoyl)-4-(2-hydroxypropan-2-yl)thiazole-2-sulfonimidamide | 421.1 |

**Table 18.** Examples in the following table were prepared using similar conditions as described in Example 9 and Scheme **5** from appropriate materials.

| Example # | Final Target Number | Structure | IUPAC Name | Exact Mass $[M+H]^+$ |
|---|---|---|---|---|
| 78 | 203 | 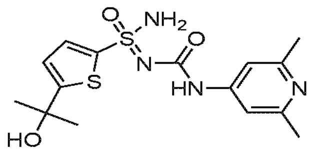 | N-((2,6-dimethylpyridin-4-yl) carbamoyl)-5-(2-hydroxypropan-2-yl) thiophene-2-sulfonimidamide | 369.1 |

**Table 19.** Examples in the following table were obtained from chiral HPLC resolutions of racemic examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined.

| Ex. # | Final Target Number | Structure | IUPAC Name | Column | Eluents | LC-MS $[M+H]^+$ |
|---|---|---|---|---|---|---|
| 79 | 180a or 180b | 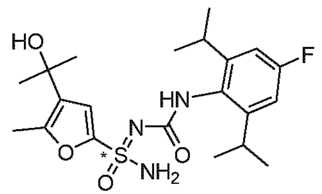 | (S)-or (R)-N'-(4-fluoro-2,6-diisopropylphenylcarbamoyl)-4-(2-hydroxypropan-2-yl)-5-methylfuran-2-sulfonimidamide | ChiralPak ID, 2*25cm, Sum | 20% EtOH in Hex | 440.3 |
| 80 | 180b or 180a | 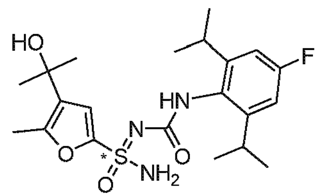 | (R)-or (S)-N'-(4-fluoro-2,6-diisopropylphenylcarbamoyl)-4-(2-hydroxypropan-2-yl)-5-methylfuran-2-sulfonimidamide | ChiralPak ID, 2*25cm, Sum | 20% EtOH in Hex | 440.3 |
| 81 | 179a or 179b | 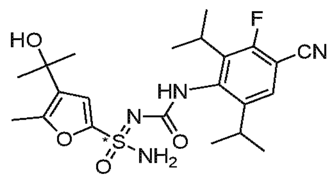 | (S)-or (R)-N'-(4-cyano-3-fluoro-2,6-diisopropylphenylcarbamoyl)-4-(2-hydroxypropan-2-yl)-5-methylfuran-2-sulfonimidamide | ChiralPak ID, 2*25cm, Sum | 23% EtOH in Hex | 465.3 |
| 82 | 179b or 179a | 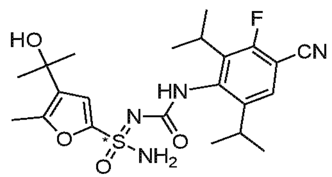 | (R)-or (S)-N'-(4-cyano-3-fluoro-2,6-diisopropylphenylcarbamoyl)-4-(2-hydroxypropan-2-yl)-5-methylfuran-2-sulfonimidamide | ChiralPak ID, 2*25cm, Sum | 23% EtOH in Hex | 465.3 |
| 83 | 190a or 190b | 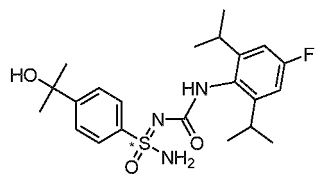 | (S)-or (R)-N'-(4-fluoro-2,6-diisopropylphenylcarbamoyl)-4-(2-hydroxypropan-2-yl)benzenesulfonimidamide | ChiralPak ID, 2*25cm, Sum | 20% EtOH in Hex | 436.2 |

(continued)

| Ex. # | Final Target Number | Structure | IUPAC Name | Column | Eluents | LC-MS $[M+H]^+$ |
|---|---|---|---|---|---|---|
| 84 | 190b or 190a | 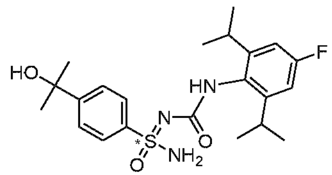 | (R)-or (S)-N'-(4-fluoro-2,6-diisopropylphenylcarbamoyl)-4-(2-hydroxypropan-2-yl)benzenesulfonimidamide | ChiralPak ID, 2*25cm, Sum | 20% EtOH in Hex | 436.2 |
| 85 | 182a or 182b | 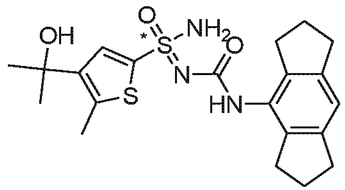 | (S)-or (R)-N'-(1,2,3,5,6,7-hexahydro-s-indacen-4-ylcarbamoyl)-4-(2-hydroxypropan-2-yl)-5-methylthiophene-2-sulfonimidamide | ChiralPak IC, 2*25cm, Sum | 20% EtOH in Hex | 434.1 |
| 86 | 182b or 182a | 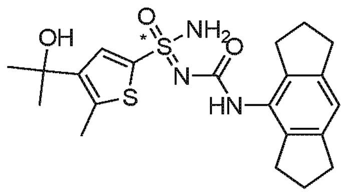 | (R)-or (S)-N'-(1,2,3,5,6,7-hexahydro-*s*-indacen-4-ylcarbamoyl)-4-(2-hydroxypropan-2-yl)-5-methylthiophene-2-sulfonimidamide | ChiralPak IC, 2*25cm, Sum | 20% EtOH in Hex | 434.1 |
| 87 | 191a or 191b | 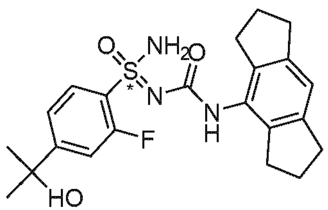 | (S)-or (R)-2-fluoro-N'-(1,2,3,5,6,7-hexahydro-*s*-indacen-4-ylcarbamoyl)-4-(2-hydroxypropan-2-yl) benzenesulfonimidamide | ChiralPak IG, 2*25cm, Sum | 30% IPA in Hex(0.1 %DEA) | 430.1 (M-1) |
| 88 | 191b or 191a | 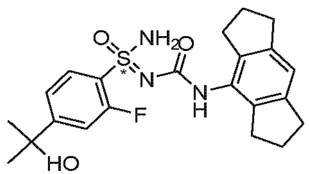 | (R)-or (S)-2-fluoro-N'-(1,2,3,5,6,7-hexahydro-*s*-indacen-4-ylcarbamoyl)-4-(2-hydroxypropan-2-yl) benzenesulfonimidamide | ChiralPak IG, 2*25cm, Sum | 30% IPA in Hex(0.1 %DEA) | 430.1 (M-1) |

(continued)

| Ex. # | Final Target Number | Structure | IUPAC Name | Column | Eluents | LC-MS [M+H]+ |
|---|---|---|---|---|---|---|
| 89 | 177a or 177b | 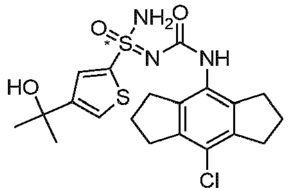 | (S)-or (R)-N'-(8-chloro-1,2,3,5,6,7-hexahydro-*s*-indacen-4-ylcarbamoyl)-4-(2-hydroxypropan-2-yl) thiophene-2-sulfonimidamide | ChiralPak ID, 2*25cm, Sum | 20% EtOH in Hex(0.1 %DEA) | 452.0 (M-1) |
| 90 | 177b or 177a | 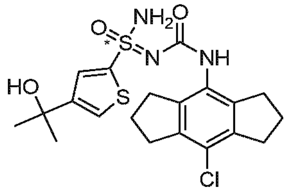 | (R)-or (S)-N'-(8-chloro-1,2,3,5,6,7-hexahydro-*s*-indacen-4-ylcarbamoyl)-4-(2-hydroxypropan-2-yl) thiophene-2-sulfonimidamide | ChiralPak ID, 2*25cm, Sum | 20% EtOH in Hex(0.1 %DEA) | 452.0 (M-1) |
| 91 | 185a or 185b | 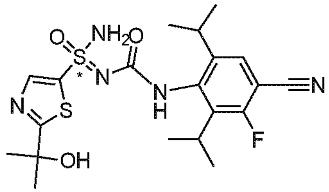 | (S)-or (R)-N'-(4-cyano-3-fluoro-2,6-diisopropylphenylcarbamoyl)-2-(2-hydroxypropan-2-yl)thiazole-5-sulfonimidamide | ChiralPak ID, 2*25cm, Sum | 30% EtOH in Hex(0.1 %DEA) | 466.1 (M-1) |
| 92 | 185b or 185a | 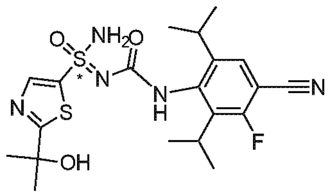 | (R)-or (S)-N'-(4-cyano-3-fluoro-2,6-diisopropylphenylcarbamoyl)-2-(2-hydroxypropan-2-yl)thiazole-5-sulfonimidamide | ChiralPak ID, 2*25cm, Sum | 30% EtOH in Hex(0.1 %DEA) | 466.1 (M-1) |
| 93 | 186a or 186b | 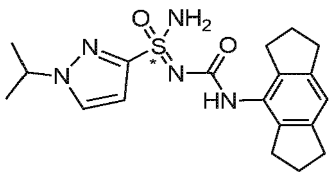 | (S)-or (R)-N'-(1,2,3,5,6,7-hexahydro-*s*-indacen-4-ylcarbamoyl)-1-isopropyl-1H-pyrazole-3-sulfonimidamide | ChiralPak IG, 2*25cm, Sum | 30% EtOH in Hex(0.1 %DEA) | 388.1 |

(continued)

| Ex. # | Final Target Number | Structure | IUPAC Name | Column | Eluents | LC-MS $[M+H]^+$ |
|---|---|---|---|---|---|---|
| 94 | 186b or 186a | 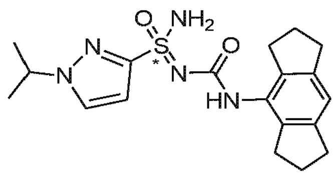 | (R)-or (S)-N'-(1,2,3,5,6,7-hexahydro-*s*-indacen-4-ylcarbamoyl)-1-isopropyl-1H-pyrazole-3-sulfonimidamide | ChiralPak IG, 2*25cm, Sum | 30% EtOH in Hex(0.1 %DEA) | 388.1 |
| 95 | 187a or 187b | 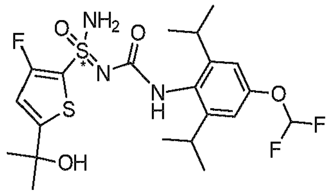 | (S)-or (R)-N'-(4-(difluoromethoxy)-2,6-diisopropylphenylcarbamoyl)-3-fluoro-5-(2-hydroxypropan-2-yl)thiophene-2-sulfonimidamide | ChiralPak ID, 2*25cm, Sum | 20% EtOH in Hex(0.1 %DEA) | 508.2 |
| 96 | 187b or 187a | 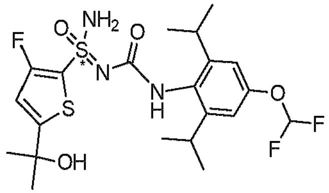 | (R)-or (S)-N'-(4-(difluoromethoxy)-2,6-diisopropylphenylcarbamoyl)-3-fluoro-5-(2-hydroxypropan-2-yl)thiophene-2-sulfonimidamide | ChiralPak ID, 2*25cm, Sum | 20% EtOH in Hex(0.1 %DEA) | 508.2 |
| 97 | 188a or 188b | 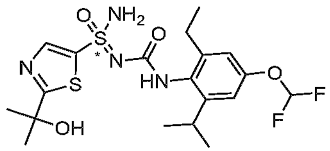 | (S)-or (R)-N'-(4-(difluoromethoxy)-2-ethyl-6-isopropylphenylcarbamoyl)-2-(2-hydroxypropan-2-yl)thiazole-5-sulfonimidamide | ChiralPak ID, 2*25cm, Sum | 30% IPA in Hex(0.1 %DEA) | 477.2 |
| 98 | 188b or 188a | 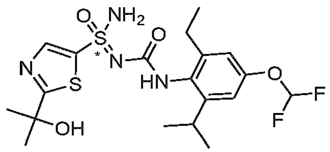 | (R)-or (S)-N'-(4-(difluoromethoxy)-2-ethyl-6-isopropylphenylcarbamoyl)-2-(2-hydroxypropan-2-yl)thiazole-5-sulfonimidamide | ChiralPak ID, 2*25cm, Sum | 30% IPA in Hex(0.1 %DEA) | 477.2 |

(continued)

| Ex. # | Final Target Number | Structure | IUPAC Name | Column | Eluents | LC-MS $[M+H]^+$ |
|---|---|---|---|---|---|---|
| 99 | 192a or 192b | 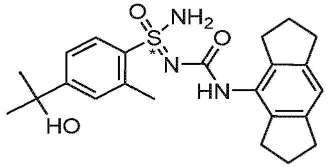 | (S)-or (R)-N'-(1,2,3,5,6,7-hexahydro-*s*-indacen-4-ylcarbamoyl)-4-(2-hydroxypropan-2-yl)-2-methylbenzenesulfonimidamide | ChiralPak ID, 2*25cm, Sum | 30% EtOH in Hex(0.1 %DEA) | 428.2 |
| 100 | 192b or 192a | 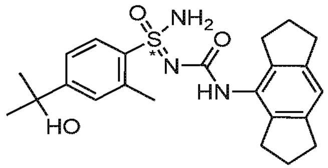 | (R)-or (S)-N'-(1,2,3,5,6,7-hexahydro-*s*-indacen-4-ylcarbamoyl)-4-(2-hydroxypropan-2-yl)-2-methylbenzenesulfonimidamide | ChiralPak ID, 2*25cm, Sum | 30% EtOH in Hex(0.1 %DEA) | 428.2 |
| 101 | 189a or 189b | 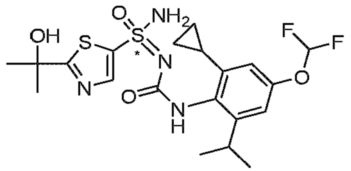 | (S)-or(R)-N'-(2-cyclopropyl-4-(difluoromethoxy)-6-isopropylphenylcarbamoyl)-2-(2-hydroxypropan-2-yl)thiazole-5-sulfonimidamide | ChiralPak ID, 2*25cm, Sum | 50% IPA in Hex(0.1 %DEA) | 489.3 |
| 102 | 189b or 189a | 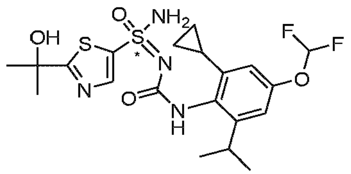 | (R)-or (S)-N'-(2-cyclopropyl-4-(difluoromethoxy)-6-isopropylphenylcarbamoyl)-2-(2-hydroxypropan-2-yl)thiazole-5-sulfonimidamide | ChiralPak ID, 2*25cm, Sum | 50% IPA in Hex(0.1 %DEA) | 489.2 |
| 103 | 178a or 178b | 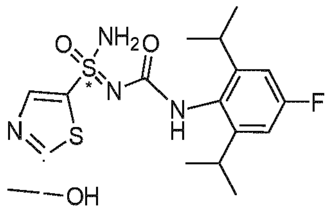 | (S)-or (R)-N'-(4-fluoro-2,6-diisopropylphenylcarbamoyl)-2-(2-hydroxypropan-2-yl)thiazole-5-sulfonimidamide | ChiralPak ID, 2*25cm, Sum | 30% IPA (0.1% DEA) in Hex:DCM=3:1 | 443.2 |

(continued)

| Ex. # | Final Target Number | Structure | IUPAC Name | Column | Eluents | LC-MS $[M+H]^+$ |
|---|---|---|---|---|---|---|
| 104 | 178b or 178a | 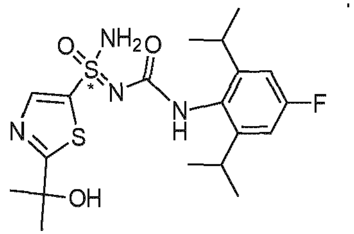 | (R)-or (S)-N'-(4-fluoro-2,6-diisopropylphenylcarbamoyl)-2-(2-hydroxypropan-2-yl)thiazole-5-sulfonimidamide | ChiralPak ID, 2*25cm, Sum | 30% IPA (0.1% DEA) in Hex:DCM=3:1 | 443.1 |
| 105 | 193a or 193b | 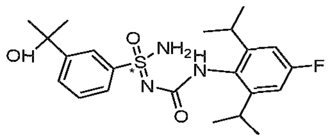 | (S)-or (R)-N'-(4-fluoro-2,6-diisopropylphenylcarbamoyl)-3-(2-hydroxypropan-2-yl)benzenesulfonimidamide | ChiralPak IG, 2*25cm, Sum | 20% IPA in Hex(0.1 %DEA) | 436.2 |
| 106 | 193b or 193a | 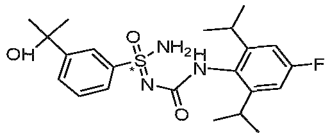 | (R)-or (S)-N'-(4-fluoro-2,6-diisopropylphenylcarbamoyl)-3-(2-hydroxypropan-2-yl)benzenesulfonimidamide | ChiralPak IG, 2*25cm, Sum | 20% IPA in Hex(0.1 %DEA) | 436.2 |
| 107 | 170a or 170b | 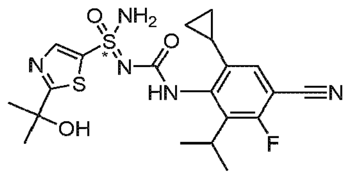 | (S)-or (R)-N'-(4-cyano-6-cyclopropyl-3-fluoro-2-isopropylphenylcarbamoyl)-2-(2-hydroxypropan-2-yl)thiazole-5-sulfonimidamide | ChiralPak IG, 2*25cm, Sum | 15% EtOH in Hex(0.1 %DEA) | 466.1 |
| 108 | 170b or 170a | 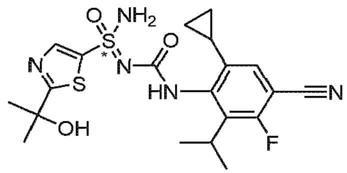 | (R)-or (S)-N'-(4-cyano-6-cyclopropyl-3-fluoro-2-isopropylphenylcarbamoyl)-2-(2-hydroxypropan-2-yl)thiazole-5-sulfonimidamide | ChiralPak IG, 2*25cm, Sum | 15% EtOH in Hex(0.1 %DEA) | 466.1 |

(continued)

| Ex. # | Final Target Number | Structure | IUPAC Name | Column | Eluents | LC-MS $[M+H]^+$ |
|---|---|---|---|---|---|---|
| 109 | 168a or 168b | 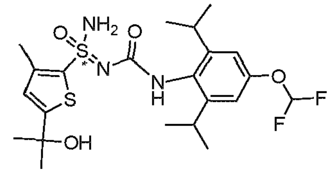 | (S)-or (R)-N'-(4-(difluoromethoxy)-2,6-diisopropylphenylcarbamoyl)-5-(2-hydroxypropan-2-yl)-3-methylthiophene-2-sulfonimidamide | ChiralPak ID, 2*25cm, Sum | 30% IPA in Hex(0.1 %DEA) | 504.2 |
| 110 | 168b or 168a | 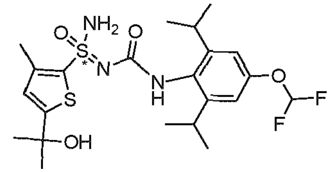 | (R)-or (S)-N'-(4-(difluoromethoxy)-2,6-diisopropylphenylcarbamoyl)-5-(2-hydroxypropan-2-yl)-3-methylthiophene-2-sulfonimidamide | ChiralPak ID, 2*25cm, Sum | 30% IPA in Hex(0.1 %DEA) | 504.2 |
| 111 | 171a or 171b | 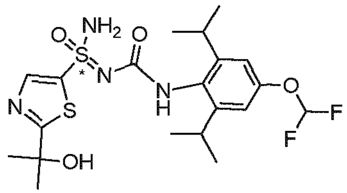 | (S)-or (R)-N'-(4-(difluoromethoxy)-2,6-diisopropylphenylcarbamoyl)-2-(2-hydroxypropan-2-yl)thiazole-5-sulfonimidamide | ChiralPak ID, 2*25cm, Sum | 50% IPA in Hex:DCM=1:1 | 489.1 (M-1) |
| 112 | 171b or 171a | 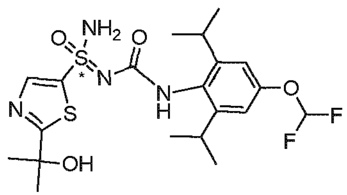 | (R)-or (S)-N'-(4-(difluoromethoxy)-2,6-diisopropylphenylcarbamoyl)-2-(2-hydroxypropan-2-yl)thiazole-5-sulfonimidamide | ChiralPak ID, 2*25cm, Sum | 50% IPA in Hex:DCM=1:1 | 489.1 (M-1) |
| 113 | 122a or 122b | 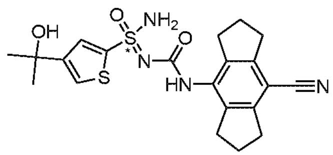 | (S)-or (R)-N'-(8-cyano-1,2,3,5,6,7-hexahydro-*s*-indacen-4-ylcarbamoyl)-4-(2-hydroxypropan-2-yl) thiophene-2-sulfonimidamide | ChiralPak ID, 2*25cm, Sum | 30% IPA in Hex | 443.1 (M-1) |

(continued)

| Ex. # | Final Target Number | Structure | IUPAC Name | Column | Eluents | LC-MS $[M+H]^+$ |
|---|---|---|---|---|---|---|
| 114 | 122b or 122a | 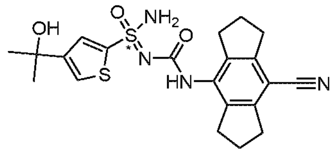 | (R)-or (S)-N'-(8-cyano-1,2,3,5,6,7-hexahydro-*s*-indacen-4-ylcarbamoyl)-4-(2-hydroxypropan-2-yl) thiophene-2-sulfonimidamide | ChiralPak ID, 2*25cm, Sum | 30% IPA in Hex | 443.1 (M-1) |
| 115 | 120a or 120b | 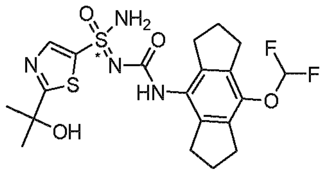 | (S)-or (R)-N'-(8-(difluoromethoxy)-1,2,3,5,6,7-hexahydro-*s*-indacen-4-ylcarbamoyl)-2-(2-hydroxypropan-2-yl)thiazole-5-sulfonimidamide | Chiral ART Cellulose-SB 2*25cm, Sum | 20% EtOH in Hex(0.1 %DEA) | 485.1 (M-1) |
| 116 | 120b or 120a | 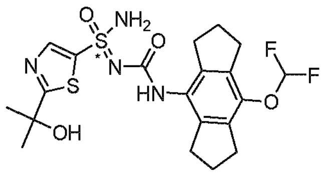 | (R)-or (S)-N'-(8-(difluoromethoxy)-1,2,3,5,6,7-hexahydro-*s*-indacen-4-ylcarbamoyl)-2-(2-hydroxypropan-2-yl)thiazole-5-sulfonimidamide | Chiral ART Cellulose-SB 2*25cm, Sum | 20% EtOH in Hex(0.1 %DEA) | 485.1 (M-1) |
| 117 | 125a or 125b | 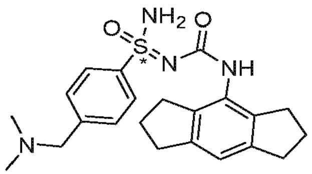 | (S)-or (R)-4-((dimethylamino) methyl)-N'-(1,2,3,5,6,7-hexahydro-s-indacen-4-ylcarbamoyl)benzenesulfonimidamide | ChiralPak ID, 2*25cm, Sum | 50% IPA in Hex:DCM=3:1 | 413.2 |
| 118 | 125b or 125a | 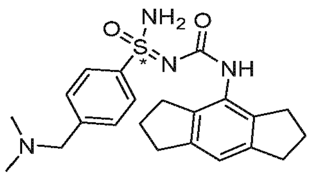 | (R)-or (S)-4-((dimethylamino) methyl)-N'-(1,2,3,5,6,7-hexahydro-s-indacen-4-ylcarbamoyl)benzenesulfonimidamide | ChiralPak ID, 2*25cm, Sum | 50% IPA in Hex:DCM=3:1 | 413.2 |

(continued)

| Ex. # | Final Target Number | Structure | IUPAC Name | Column | Eluents | LC-MS $[M+H]^+$ |
|---|---|---|---|---|---|---|
| 119 | 129a or 129b | 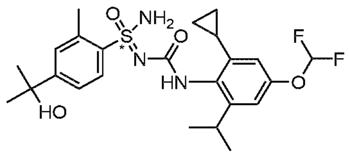 | (S)-or (R)-N'-(2-cyclopropyl-4-(difluoromethoxy)-6-isopropylphenylcarbamoyl)-4-(2-hydroxypropan-2-yl)-2-methylbenzenesulfonimidamide | ChiralPak ID, 2*25cm, Sum | 50% IPA in Hex:DCM=3:1 | 496.2 |
| 120 | 129b or 129a | 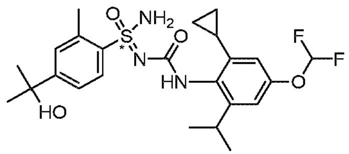 | (R)-or (S)-N'-(2-cyclopropyl-4-(difluoromethoxy)-6-isopropylphenylcarbamoyl)-4-(2-hydroxypropan-2-yl)-2-methylbenzenesulfonimidamide | ChiralPak ID, 2*25cm, Sum | 50% IPA in Hex:DCM=3:1 | 496.2 |
| 121 | 112a or 112b | 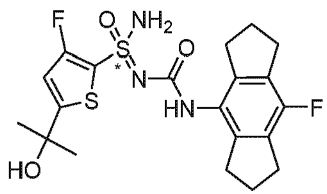 | (S)-or (R)-3-fluoro-N'-(8-fluoro-1,2,3,5,6,7-hexahydro-s-indacen-4-ylcarbamoyl)-5-(2-hydroxypropan-2-yl)thiophene-2-sulfonimidamide | ChiralPak IG, 2*25cm, Sum | 15% EtOH in Hex(0.1 %DEA) | 456.1 |
| 122 | 112b or 112a | 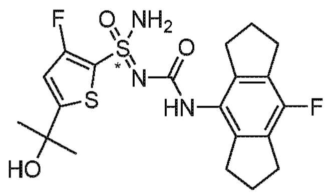 | (R)-or (S)-3-fluoro-N'-(8-fluoro-1,2,3,5,6,7-hexahydro-s-indacen-4-ylcarbamoyl)-5-(2-hydroxypropan-2-yl)thiophene-2-sulfonimidamide | ChiralPak IG, 2*25cm, Sum | 15% EtOH in Hex(0.1 %DEA) | 456.1 |
| 128 | 105a or 105b | 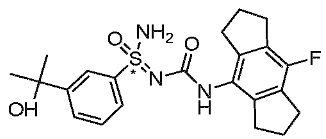 | (S)-or (R)-N'-(8-fluoro-1,2,3,5,6,7-hexahydro-*s*-indacen-4-ylcarbamoyl)-3 -(2-hydroxypropan-2-yl) benzenesulfonimidamide | ChiralPak ID, 2*25cm, Sum | 40% EtOH in Hex | 432.1 |

(continued)

| Ex. # | Final Target Number | Structure | IUPAC Name | Column | Eluents | LC-MS $[M+H]^+$ |
|---|---|---|---|---|---|---|
| 129 | 105b or 105a | 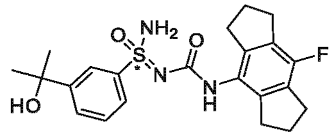 | (R)-or (S)-N'-(8-fluoro-1,2,3,5,6,7-hexahydro-*s*-indacen-4-ylcarbamoyl)-3 -(2-hydroxypropan-2-yl) benzenesulfonimidamide | ChiralPak ID, 2*25cm, Sum | 40% EtOH in Hex | 432.1 |
| 130 | 121a or 121b | 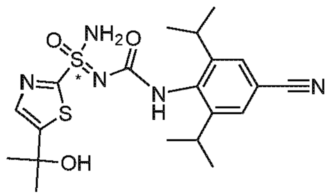 | (S)-or (R)-N'-(4-cyano-2,6-diisopropylphenylcarbamoyl)-5-(2-hydroxypropan-2-yl)thiazole-2-sulfonimidamide | ChiralPak ID, 2*25cm, Sum | 40% EtOH in Hex | 448.1 (M-1) |
| 131 | 121b or 121a | 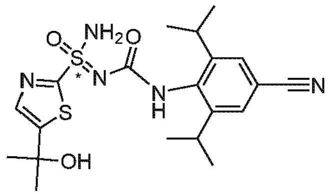 | (R)-or (S)-N'-(4-cyano-2,6-diisopropylphenylcarbamoyl)-5-(2-hydroxypropan-2-yl)thiazole-2-sulfonimidamide | ChiralPak ID, 2*25cm, Sum | 40% EtOH in Hex | 448.1 (M-1) |
| 132 | 145a or 145b | 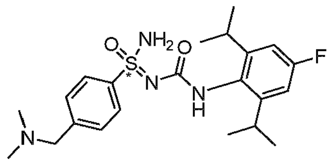 | (S)-or (R)-4-((dimethylamino)methyl)-N'-(4-fluoro-2,6-diisopropylphenylcarbamoyl)benzenesulfoni midamide | ChiralPak IG, 2*25cm, Sum | 30% EtOH in Hex | 435.2 |
| 133 | 145b or 145a | 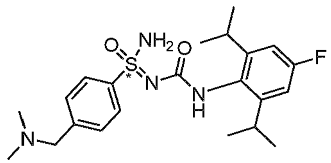 | (R)-or (S)-4-((dimethylamino)methyl)-N'-(4-fluoro-2,6-diisopropylphenylcarbamoyl)benzenesulfoni midamide | ChiralPak IG, 2*25cm, Sum | 30% EtOH in Hex | 435.2 |

(continued)

| Ex. # | Final Target Number | Structure | IUPAC Name | Column | Eluents | LC-MS $[M+H]^+$ |
|---|---|---|---|---|---|---|
| 134 | 131a or 131b | 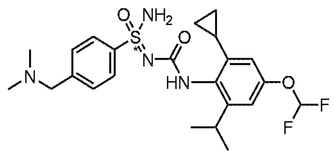 | (S)-or (R)-N'-(2-cyclopropyl-4-(difluoromethoxy)-6-isopropylphenylcarbamoyl)-4-((dimethylamino) methyl)benzenesulfonimid amide | ChiralPak IG, 2*25cm, Sum | 50% EtOH in Hex | 481.2 |
| 135 | 131b or 131a | 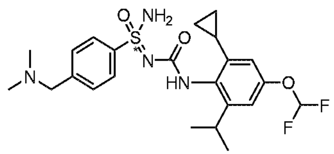 | (R)-or (S)-N'-(2-cyclopropyl-4-(difluoromethoxy)-6-isopropylphenylcarbamoyl)-4-((dimethylamino) methyl)benzenesulfonimid amide | ChiralPak IG, 2*25cm, Sum | 50% EtOH in Hex | 481.2 |
| 136 | 225a or 225b | 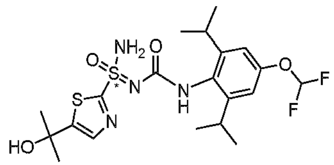 | (S)-or (R)-N'-(4-(difluoromethoxy)-2,6-diisopropylphenylcarbamoyl)-5-(2-hydroxypropan-2-yl)thiazole-2-sulfonimidamide | ChiralPak IF, 2*25cm, Sum | 20% MeOH (0.1%TF A) in $CO_2$ | 489.1 (M-1) |
| 137 | 225b or 225a | 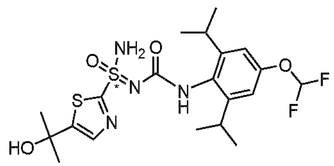 | (R)-or (S)-N'-(4-(difluoromethoxy)-2,6-diisopropylphenylcarbamoyl)-5-(2-hydroxypropan-2-yl)thiazole-2-sulfonimidamide | ChiralPak IF, 2*25cm, Sum | 20% MeOH (0.1%TF A) in $CO_2$ | 489.1 (M-1) |
| 138 | 144a or 144b | 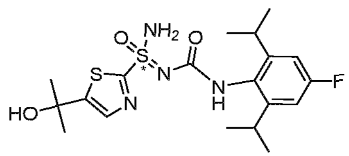 | (S)-or (R)-N'-(4-fluoro-2,6-diisopropylphenylcarbamoyl)-5-(2-hydroxypropan-2-yl)thiazole-2-sulfonimidamide | ChiralPak IF, 2*25cm, Sum | 20% MeOH (0.1%TF A) in $CO_2$ | 443.2 |

(continued)

| Ex. # | Final Target Number | Structure | IUPAC Name | Column | Eluents | LC-MS $[M+H]^+$ |
|---|---|---|---|---|---|---|
| 139 | 144b or 144a | 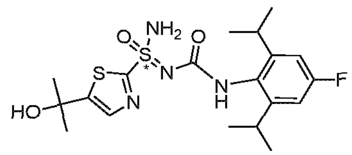 | (R)-or (S)-N'-(4-fluoro-2,6-diisopropylphenylcarbamoyl)-5-(2-hydroxypropan-2 -yl)thiazole-2 - sulfonimidamide | ChiralPak IF, 2*25cm, Sum | 20% MeOH (0.1%TF A) in $CO_2$ | 443.1 |
| 140 | 149a or 149b | 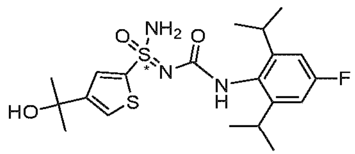 | (S)-or (R)-N'-(4-fluoro-2,6-diisopropylphenylcarbamoyl)-4-(2-hydroxypropan-2-yl)thiophene-2-sulfonimidamide | ChiralPak ID, 2*25cm, Sum | 30% EtOH in Hex | 440.1 (M-1) |
| 141 | 149b or 149a | 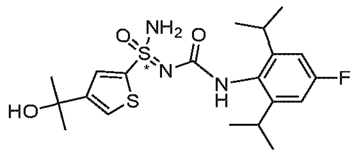 | (R)-or (S)-N'-(4-fluoro-2,6-diisopropylphenylcarbamoyl)-4-(2-hydroxypropan-2-yl)thiophene-2-sulfonimidamide | ChiralPak ID, 2*25cm, Sum | 30% EtOH in Hex | 440.1 (M-1) |
| 142 | 152a or 152b | 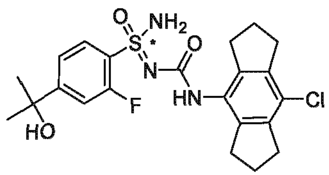 | (S)-or (R)-N'-(4-fluoro-2,6-diisopropylphenylcarbamoyl)-4-(2-hydroxypropan-2-yl)thiophene-2-sulfonimidamide | ChiralPak IG, 2*25cm, Sum | 30% IPA in Hex | 466.2 |
| 143 | 152b or 152a | 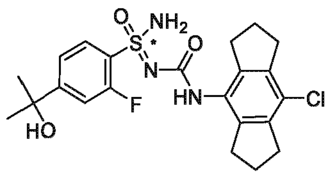 | (R)-or (S)-N'-(4-fluoro-2,6-diisopropylphenylcarbamoyl)-4-(2-hydroxypropan-2-yl)thiophene-2-sulfonimidamide | ChiralPak IG, 2*25cm, Sum | 30% IPA in Hex | 466.2 |

(continued)

| Ex. # | Final Target Number | Structure | IUPAC Name | Column | Eluents | LC-MS $[M+H]^+$ |
|---|---|---|---|---|---|---|
| 144 | 151a' or 151b' | 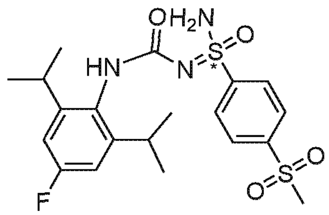 | (S)-or (R)-N'-(4-fluoro-2,6-diisopropylphenylcarbamoyl)-4-(methylsulfonyl) benzenesulfonimidamide | Lux 5u Cellulose-4, AXIA Packed, 2.12*25cm, Sum | 35% MeOH (2 mM $NH_3$) in $CO_2$ | 454.1 (M-1) |
| 145 | 151b' or 151a' | 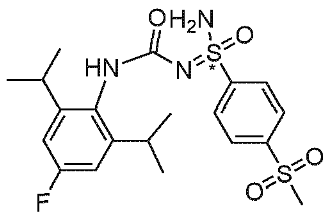 | (R)-or (S)-N'-(4-fluoro-2,6-diisopropylphenylcarbamoyl)-4-(methylsulfonyl) benzenesulfonimidamide | Lux 5u Cellulose-4, AXIA Packed, 2.12*25cm, Sum | 35% MeOH (2 mM $NH_3$) in $CO_2$ | 454.1 (M-1) |
| 146 | 167a or 167b | 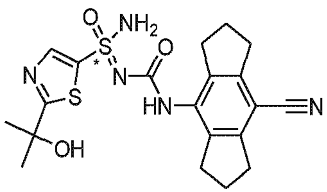 | (S)-or (R)-N'-(8-cyano-1,2,3,5,6,7-hexahydro-*s*-indacen-4-ylcarbamoyl)-2-(2-hydroxypropan-2-yl) thiazole-5-sulfonimidamide | ChiralPak IC, 2*25cm, Sum | 30% EtOH in Hex(0.1 %DEA) | 444.1 (M-1) |
| 147 | 167b or 167a | 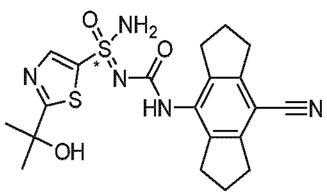 | (R)-or (S)-N'-(8-cyano-1,2,3,5,6,7-hexahydro-*s*-indacen-4-ylcarbamoyl)-2-(2-hydroxypropan-2-yl) thiazole-5-sulfonimidamide | ChiralPak IC, 2*25cm, Sum | 30% EtOH in Hex(0.1 %DEA) | 444.1 (M-1) |

(continued)

| Ex. # | Final Target Number | Structure | IUPAC Name | Column | Eluents | LC-MS $[M+H]^+$ |
|---|---|---|---|---|---|---|
| 148 | 107a or 107b | 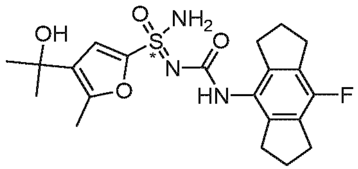 | (S)-or (R)-N'-(8-fluoro-1,2,3,5,6,7-hexahydro-*s*-indacen-4-ylcarbamoyl)-4-(2-hydroxypropan-2-yl)-5-methylfuran-2-sulfonimidamide | ChiralPak ID, 2*25cm, Sum | 50% IPA in Hex | 434.1 (M-1) |
| 149 | 107b or 107a | 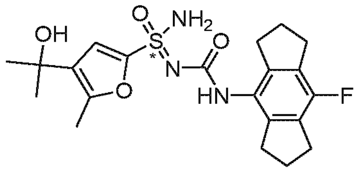 | (R)-or (S)-N'-(8-fluoro-1,2,3,5,6,7-hexahydro-*s*-indacen-4-ylcarbamoyl)-4-(2-hydroxypropan-2-yl)-5-methylfuran-2-sulfonimidamide | ChiralPak ID, 2*25cm, Sum | 50% IPA in Hex | 434.1 (M-1) |
| 150 | 110a or 110b | 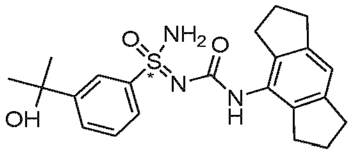 | (S)-or (R)-N'-(1,2,3,5,6,7-hexahydro-*s*-indacen-4-ylcarbamoyl)-3-(2-hydroxypropan-2-yl) benzenesulfonimidamide | ChiralPak IF, 2*25cm, Sum | 30% EtOH in Hex | 412.1 (M-1) |
| 151 | 110b or 110a | 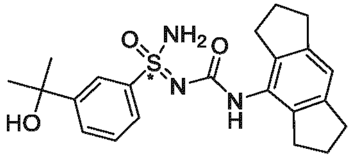 | (R)-or (S)-N'-(1,2,3,5,6,7-hexahydro-*s*-indacen-4-ylcarbamoyl)-3-(2-hydroxypropan-2-yl) benzenesulfonimidamide | ChiralPak IF, 2*25cm, Sum | 30% EtOH in Hex | 412.1 (M-1) |
| 152 | 151a or 151b | 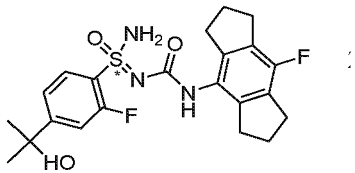 | (S)-or (R)-2-fluoro-N'-(8-fluoro-1,2,3,5,6,7-hexahydro-*s*-indacen-4-ylcarbamoyl)-4-(2-hydroxypropan-2-yl)benzenesulfonimidamide | ChiralPak IG, 2*25cm, Sum | 30% IPA in Hex | 448.1 (M-1) |

(continued)

| Ex. # | Final Target Number | Structure | IUPAC Name | Column | Eluents | LC-MS $[M+H]^+$ |
|---|---|---|---|---|---|---|
| 153 | 151b or 151a | 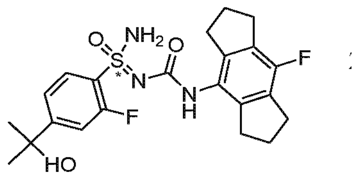 | (R)-or (S)-2-fluoro-N'-(8-fluoro-1,2,3,5,6,7-hexahydro-*s*-indacen-4-ylcarbamoyl)-4-(2-hydroxypropan-2-yl)benzenesulfonimidamide | ChiralPak IG, 2*25cm, 5um | 30% IPA in Hex | 448.1 (M-1) |
| 154 | 154a or 154b | 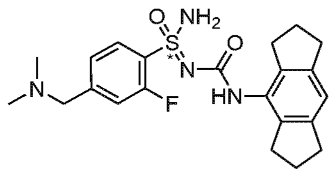 | (S)-or (R)-4-((dimethylamino)methyl)-2-fluoro-N'-(1,2,3,5,6,7-hexahydro-s-indacen-4-ylcarbamoyl)benzenesulfonimidamide | ChiralPak IG, 2*25cm, 5um | 30% EtOH in Hex | 431.2 |
| 155 | 154b or 154a | 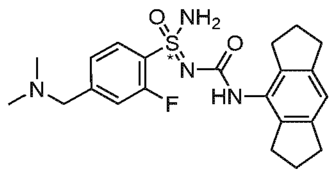 | (R)-or (S)-4-((dimethylamino)methyl)-2-fluoro-N'-(1,2,3,5,6,7-hexahydro-s-indacen-4-ylcarbamoyl)benzenesulfonimidamide | ChiralPak IG, 2*25cm, 5um | 30% EtOH in Hex | 431.2 |
| 156 | 148a or 148b | 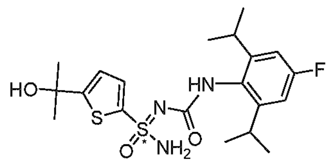 | (S)-or (R)-N'-(4-fluoro-2,6-diisopropylphenylcarbamoyl)-5-(2-hydroxypropan-2-yl)thiophene-2-sulfonimidamide | ChiralPak ID, 2*25cm, 5um | 30% EtOH in Hex | 442.1 |
| 157 | 148b or 148a | 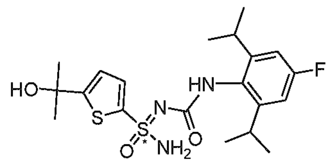 | (R)-or (S)-N'-(4-fluoro-2,6-diisopropylphenylcarbamoyl)-5-(2-hydroxypropan-2-yl)thiophene-2-sulfonimidamide | ChiralPak ID, 2*25cm, 5um | 30% EtOH in Hex | 442.1 |

(continued)

| Ex. # | Final Target Number | Structure | IUPAC Name | Column | Eluents | LC-MS $[M+H]^+$ |
|---|---|---|---|---|---|---|
| 158 | 153a or 153b | 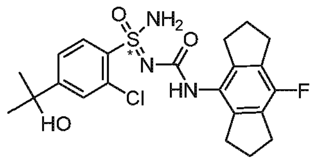 | (S)-or (R)-2-chloro-N'-(8-fluoro-1,2,3,5,6,7-hexahydro-s-indacen-4-ylcarbamoyl)-4-(2-hydroxypropan-2-yl)benzenesulfonimidamide | ChiralPak IF, 2*25cm, Sum | 30% EtOH in Hex | 464.1 (M-1) |
| 159 | 153a or 153b | 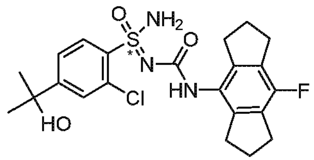 | (R)-or (S)-2-chloro-N'-(8-fluoro-1,2,3,5,6,7-hexahydro-s-indacen-4-ylcarbamoyl)-4-(2-hydroxypropan-2-yl)benzenesulfonimidamide | ChiralPak IF, 2*25cm, Sum | 30% EtOH in Hex | 464.1 (M-1) |
| 160 | 109a or 109b | 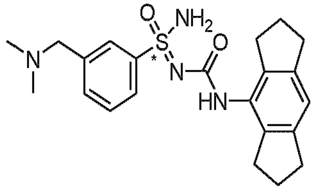 | (S)-or (R)-3-((dimethylamino) methyl)-N'-(1,2,3,5,6,7-hexahydro-s-indacen-4-ylcarbamoyl)benzenesulfonimidamide | ChiralPak ID, 2*25cm, Sum | 50% EtOH in Hex(0.1 %DEA) | 413.1 |
| 161 | 109b or 109a | 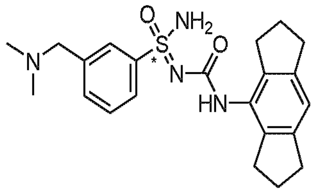 | (R)-or (S)-3-((dimethylamino) methyl)-N'-(1,2,3,5,6,7-hexahydro-s-indacen-4-ylcarbamoyl)benzenesulfonimidamide | ChiralPak ID, 2*25cm, Sum | 50% EtOH in Hex(0.1 %DEA) | 413.1 |
| 162 | 135a or 135b | 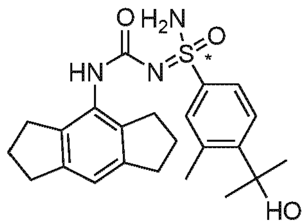 | (S)-or (R)-N'-((1,2,3,5,6,7-hexahydro-s-indacen-4-yl)carbamoyl)-4-(2-hydroxypropan-2-yl)-3-methylbenzenesulfonimidamide | ChiralPak ID, 2*25cm, Sum | 30% IPA in Hex | 428.2 |

(continued)

| Ex. # | Final Target Number | Structure | IUPAC Name | Column | Eluents | LC-MS $[M+H]^+$ |
|---|---|---|---|---|---|---|
| 163 | 135b or 135a | 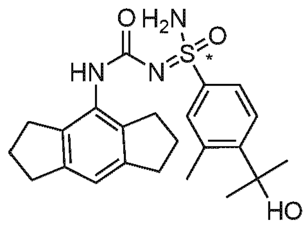 | (R)-or (S)-N'-((1,2,3,5,6,7-hexahydro-s-indacen-4-yl)carbamoyl)-4-(2-hydroxypropan-2-yl)-3-methylbenzenesulfonimidamide | ChiralPak ID, 2*25cm, Sum | 30% IPA in Hex | 428.2 |
| 164 | 134a or 134b | 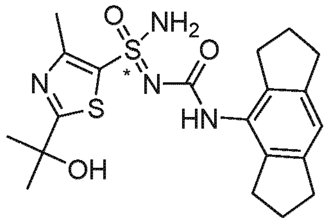 | (S)-or (R)-N'-((1,2,3,5,6,7-hexahydro-s-indacen-4-yl)carbamoyl)-2-(2-hydroxypropan-2-yl)-4-methylthiazole-5-sulfonimidamide | ChiralPak ID, 2*25cm, Sum | 30% IPA in Hex | 435.1 |
| 165 | 134b or 134a | 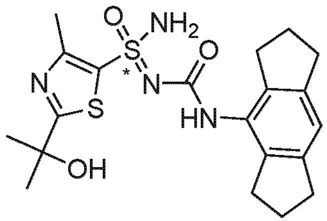 | (R)-or (S)-N'-((1,2,3,5,6,7-hexahydro-s-indacen-4-yl)carbamoyl)-2-(2-hydroxypropan-2-yl)-4-methylthiazole-5-sulfonimidamide | ChiralPak ID, 2*25cm, Sum | 30% IPA in Hex | 435.1 |
| 166 | 130a or 130b | 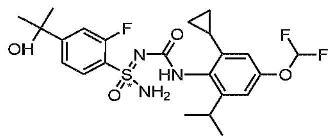 | (S)-or (R)-N'-((2-cyclopropyl-4-(difluoromethoxy)-6-isopropylphenyl)carbamoyl)-2-fluoro-4-(2-hydroxypropan-2-yl)benzenesulfonimidamide | ChiralPak ID, 2*25cm, Sum | 40% IPA in Hex | 500.2 |
| 167 | 130b or 130a | 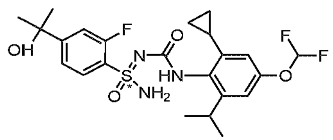 | (R)-or (S)-N'-((2-cyclopropyl-4-(difluoromethoxy)-6-isopropylphenyl)carbamoyl)-2-fluoro-4-(2-hydroxypropan-2-yl)benzenesulfonimidamide | ChiralPak ID, 2*25cm, Sum | 40% IPA in Hex | 500.2 |

(continued)

| Ex. # | Final Target Number | Structure | IUPAC Name | Column | Eluents | LC-MS $[M+H]^+$ |
|---|---|---|---|---|---|---|
| 168 | 212a or 212b | 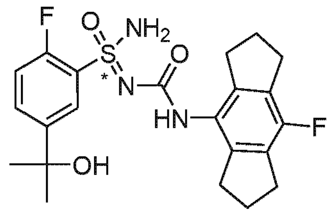 | (S)-or (R)-2-fluoro-N'-((8-fluoro-1,2,3,5,6,7-hexahydro-s-indacen-4-yl)carbamoyl)-5-(2-hydroxypropan-2-yl)benzenesulfonimidamide | ChiralPak ID, 2*25cm, Sum | 40% EtOH in Hex | 450.2 |
| 169 | 212b or 212a | 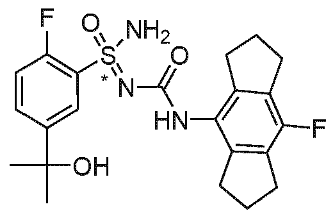 | (S)-or (R)-2-fluoro-N'-((8-fluoro-1,2,3,5,6,7-hexahydro-s-indacen-4-yl)carbamoyl)-5-(2-hydroxypropan-2-yl)benzenesulfonimidamide | ChiralPak ID, 2*25cm, Sum | 40% EtOH in Hex | 450.2 |
| 170 | 205a or 205b | 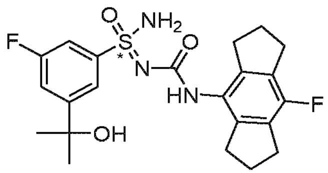 | (R)-or (S)-3-fluoro-N'-((8-fluoro-1,2,3,5,6,7-hexahydro-s-indacen-4-yl)carbamoyl)-5-(2-hydroxypropan-2-yl)benzenesulfonimidamide | Chiral ART Cellulose-SB, 2*25cm, Sum | 30% EtOH in Hex | 450.2 |
| 171 | 205a or 205b | 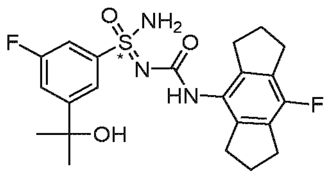 | (S)-or (R)-3-fluoro-N'-((8-fluoro-1,2,3,5,6,7-hexahydro-s-indacen-4-yl)carbamoyl)-5-(2-hydroxypropan-2-yl)benzenesulfonimidamide | Chiral ART Cellulose-SB, 2*25cm, Sum | 40% EtOH in Hex | 450.2 |
| 172 | 143a or 143b | 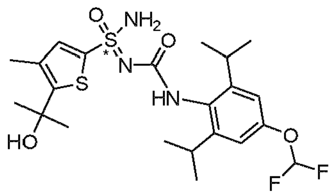 | (S)-or (R)-N'-((4-(difluoromethoxy)-2,6-diisopropylphenyl)carbamoyl)-5-(2-hydroxypropan-2-yl)-4-methylthiophene-2-sulfonimidamide | ChiralPak ID, 2*25cm, Sum | 30% EtOH in Hex | 504.2 |

(continued)

| Ex. # | Final Target Number | Structure | IUPAC Name | Column | Eluents | LC-MS $[M+H]^+$ |
|---|---|---|---|---|---|---|
| 173 | 143b or 143a | 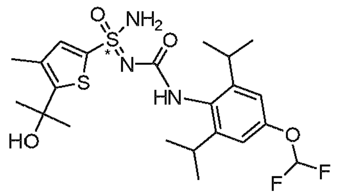 | (R)-or (S)-N'-((4-(difluoromethoxy)-2,6-diisopropylphenyl)carbamoyl)-5-(2-hydroxypropan-2-yl)-4-methylthiophene-2-sulfonimidamide | ChiralPak ID, 2*25cm, Sum | 30% EtOH in Hex | 504.2 |
| 174 | 206a or 206b | 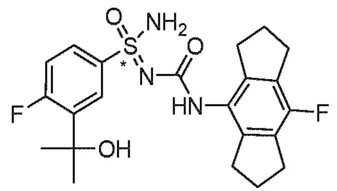 | (S)-or (R)-4-fluoro-N'-((8-fluoro-1,2,3,5,6,7-hexahydro-s-indacen-4-yl)carbamoyl)-3-(2-hydroxypropan-2-yl)benzenesulfonimidamide | ChiralPak ID, 2*25cm, Sum | 30% EtOH in Hex(8m M $NH_3$. MeOH) | 450.2 |
| 175 | 206b or 206a | 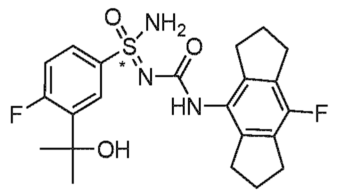 | (R)-or (S)-4-fluoro-N'-((8-fluoro-1,2,3,5,6,7-hexahydro-s-indacen-4-yl)carbamoyl)-3-(2-hydroxypropan-2-yl)benzenesulfonimidamide | ChiralPak ID, 2*25cm, Sum | 30% EtOH in Hex(8m M $NH_3$. MeOH) | 450.2 |
| 176 | 108a or 108b | 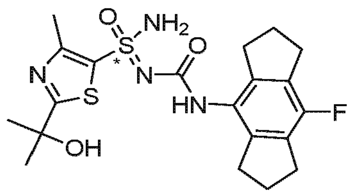 | (S)-or (R)-N'-((8-fluoro-1,2,3,5,6,7-hexahydro-s-indacen-4-yl)carbamoyl)-2-(2-hydroxypropan-2-yl)-4-methylthiazole-5-sulfonimidamide | ChiralPak IG, 2*25cm, Sum | 30% IPA in Hex | 453.1 |
| 177 | 108b or 108a | 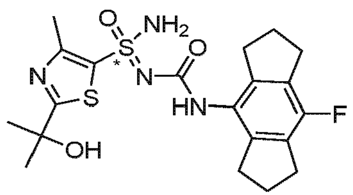 | (R)-or (S)-N'-((8-fluoro-1,2,3,5,6,7-hexahydro-s-indacen-4-yl)carbamoyl)-2-(2-hydroxypropan-2-yl)-4-methylthiazole-5-sulfonimidamide | ChiralPak IG, 2*25cm, Sum | 30% IPA in Hex | 453.1 |

(continued)

| Ex. # | Final Target Number | Structure | IUPAC Name | Column | Eluents | LC-MS $[M+H]^+$ |
|---|---|---|---|---|---|---|
| 178 | 202a or 202b | 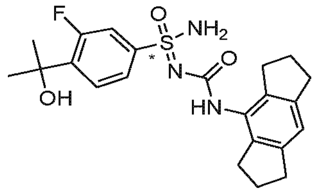 | (S)-or (R)-3-fluoro-N'-((1,2,3,5,6,7-hexahydro-s-indacen-4-yl)carbamoyl)-4-(2-hydroxypropan-2-yl) benzenesulfonimidamide | Chiral ART Cellulose-SB, 2*25cm, Sum | 50% EtOH in Hex(8m M $NH_3$. MeOH) | 432.2 |
| 179 | 202b or 202a | 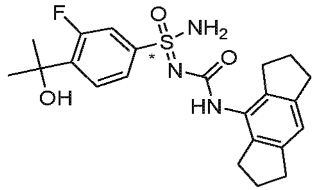 | (R)-or (S)-3-fluoro-N'-((1,2,3,5,6,7-hexahydro-s-indacen-4-yl)carbamoyl)-4-(2-hydroxypropan-2-yl) benzenesulfonimidamide | Chiral ART Cellulose-SB, 2*25cm, Sum | 50% EtOH in Hex(8m M $NH_3$. MeOH) | 432.2 |
| 180 | 116a or 116b | 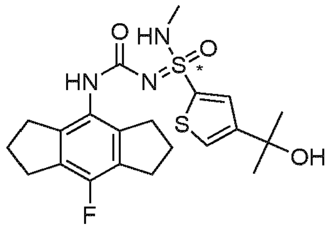 | (S)-or (R)-N'-((8-fluoro-1,2,3,5,6,7-hexahydro-s-indacen-4-yl)carbamoyl)-4-(2-hydroxypropan-2-yl)-N-methylthiophene-2-sulfonimidamide | ChiralPak IG, 2*25cm, Sum | 30% EtOH in Hex | 452.1 |
| 181 | 116b or 116a | 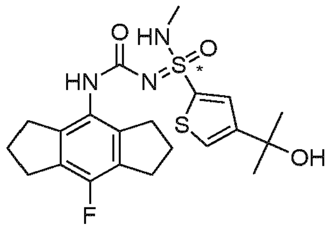 | (R)-or (S)-N'-((8-fluoro-1,2,3,5,6,7-hexahydro-s-indacen-4-yl)carbamoyl)-4-(2-hydroxypropan-2-yl)-N-methylthiophene-2-sulfonimidamide | ChiralPak IG, 2*25cm, Sum | 30% EtOH in Hex | 452.1 |

(continued)

| Ex. # | Final Target Number | Structure | IUPAC Name | Column | Eluents | LC-MS $[M+H]^+$ |
|---|---|---|---|---|---|---|
| 182 | 173a or 173b | 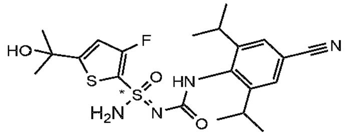 | (S)-or (R)-N'-((4-cyano-2,6-diisopropylphenyl) carbamoyl)-3-fluoro-5-(2-hydroxypropan-2-yl) thiophene-2-sulfonimidamide | ChiralPak ID, 2*25cm, Sum | 15% EtOH in Hex(0.1 %DEA) | 467.2 |
| 183 | 173b or 173a | 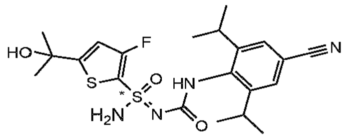 | (R)-or (S)-N'-((4-cyano-2,6-diisopropylphenyl) carbamoyl)-3-fluoro-5-(2-hydroxypropan-2-yl) thiophene-2-sulfonimidamide | ChiralPak ID, 2*25cm, Sum | 15% EtOH in Hex(0.1 %DEA) | 467.2 |
| 184 | 174a or 174b | 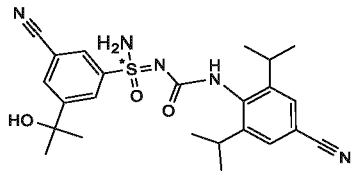 | (S)-or (R)-3-cyano-N'-((4-cyano-2,6-diisopropylphenyl)carbamoyl)-5-(2-hydroxypropan-2-yl)benzenesulfonimidamide | ChiralPak IG, 2*25cm, Sum | 15% EtOH in Hex(0.1 %DEA) | 468.2 |
| 185 | 174b or 174a | 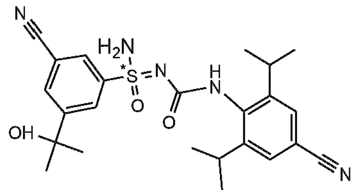 | (R)-or (S)-3-cyano-N'-((4-cyano-2,6-diisopropylphenyl)carbamoyl)-5-(2-hydroxypropan-2-yl)benzenesulfonimidamide | ChiralPak IG, 2*25cm, Sum | 15% EtOH in Hex(0.1 %DEA) | 468.2 |
| 186 | 223a or 223b | 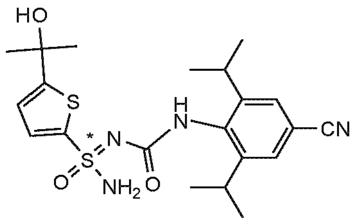 | (S)-or (R)-N'-((4-cyano-2,6-diisopropylphenyl) carbamoyl)-5-(2-hydroxypropan-2-yl)thiophene-2-sulfonimidamide | ChiralPak ID, 2*25cm, Sum | 30% EtOH in Hex(0.1 %DEA) | 449.2 |

(continued)

| Ex. # | Final Target Number | Structure | IUPAC Name | Column | Eluents | LC-MS $[M+H]^+$ |
|---|---|---|---|---|---|---|
| 187 | 223b or 223a | 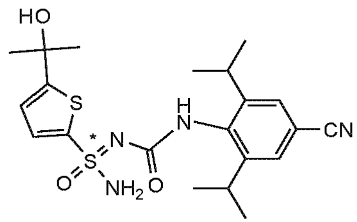 | (R)-or (S)-N'-((4-cyano-2,6-diisopropylphenyl) carbamoyl)-5-(2-hydroxypropan-2-yl)thiophene-2-sulfonimidamide | ChiralPak ID, 2*25cm, Sum | 30% EtOH in Hex(0.1 %DEA) | 449.2 |
| 188 | 158a or 158b | 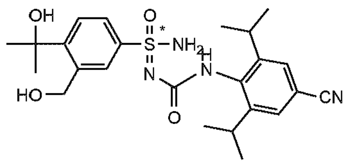 | (S)-or (R)-N'-((4-cyano-2,6-diisopropylphenyl) carbamoyl)-3-(hydroxymethyl)-4-(2-hydroxypropan-2-yl)benzenesulfonimidamide | ChiralPak ID, 2*25cm, Sum | 30% EtOH in Hex(0.1 %DEA) | 473.2 |
| 189 | 158b or 158a | 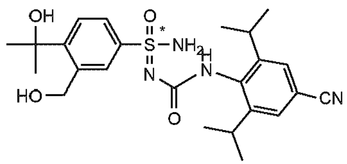 | (R)-or (S)-N'-((4-cyano-2,6-diisopropylphenyl) carbamoyl)-3-(hydroxymethyl)-4-(2-hydroxypropan-2-yl)benzenesulfonimidamide | ChiralPak ID, 2*25cm, Sum | 30% EtOH in Hex(0.1 %DEA) | 473.2 |
| 190 | 220a or 220b | 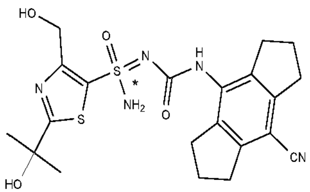 | (S)-or (R)-N'-((8-cyano-1,2,3,5,6,7-hexahydro-s-indacen-4-yl)carbamoyl)-4-(hydroxymethyl)-2-(2-hydroxypropan-2-yl)thiazole-5-sulfonimidamide | ChiralPak IF, 2*25cm, Sum | MeOH (0.1%DE A) | 476.1 |
| 191 | 220b or 220a | 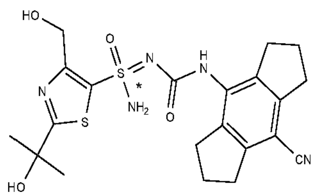 | (R)-or (S)-N'-((8-cyano-1,2,3,5,6,7-hexahydro-s-indacen-4-yl)carbamoyl)-4-(hydroxymethyl)-2-(2-hydroxypropan-2-yl)thiazole-5-sulfonimidamide | ChiralPak IF, 2*25cm, Sum | MeOH (0.1%DE A) | 476.1 |

(continued)

| Ex. # | Final Target Number | Structure | IUPAC Name | Column | Eluents | LC-MS $[M+H]^+$ |
|---|---|---|---|---|---|---|
| 192 | 157a or 157b | 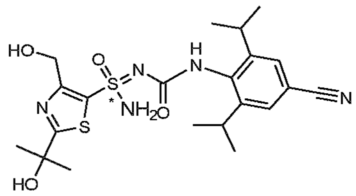 | (S)-or (R)-N'-((4-cyano-2,6-diisopropylphenyl) carbamoyl)-4-(hydroxymethyl)-2-(2-hydroxypropan-2-yl)thiazole-5-sulfonimidamide | ChiralPak IC, 2*25cm, Sum | 15% EtOH in Hex | 480.2 |
| 193 | 157b or 157a | 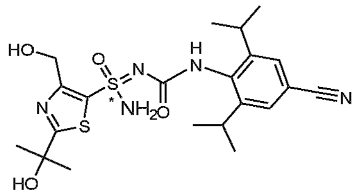 | (R)-or (S)-N'-((4-cyano-2,6-diisopropylphenyl) carbamoyl)-4-(hydroxymethyl)-2-(2-hydroxypropan-2-yl)thiazole-5-sulfonimidamide | ChiralPak IC, 2*25cm, Sum | 15% EtOH in Hex | 480.2 |
| 194 | 161a or 161b | 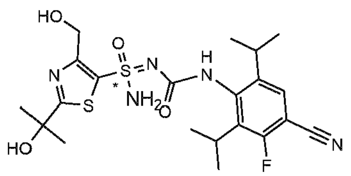 | (S)-or (R)-N'-((4-cyano-3-fluoro-2,6-diisopropylphenyl)carbamoyl)-4-(hydroxymethyl)-2-(2-hydroxypropan-2-yl)thiazole-5-sulfonimidamide | ChiralPak IC, 2*25cm, Sum | 15% EtOH in Hex | 498.2 |
| 195 | 161b or 161a | 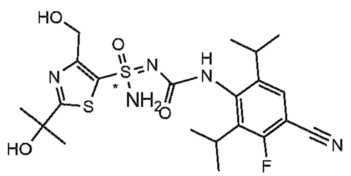 | (R)-or (S)-N'-((4-cyano-3-fluoro-2,6-diisopropylphenyl)carbamoyl)-4-(hydroxymethyl)-2-(2-hydroxypropan-2-yl)thiazole-5-sulfonimidamide | ChiralPak IC, 2*25cm, Sum | 15% EtOH in Hex | 498.2 |
| 196 | 165a or 165b | 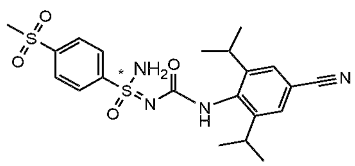 | (S)-or (R)-N'-((4-cyano-2,6-diisopropylphenyl) carbamoyl)-4-(methylsulfonyl) benzenesulfonimidamide | ChiralPak IG, 2*25cm, Sum | 30% EtOH in Hex(0.1 %DEA) | 463.1 |

(continued)

| Ex. # | Final Target Number | Structure | IUPAC Name | Column | Eluents | LC-MS $[M+H]^+$ |
|---|---|---|---|---|---|---|
| 197 | 165b or 165a | 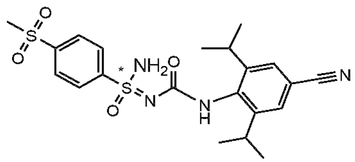 | (R)-or (S)-N'-((4-cyano-2,6-diisopropylphenyl) carbamoyl)-4-(methylsulfonyl) benzenesulfonimidamide | ChiralPak IG, 2*25cm, Sum | 30% EtOH in Hex(0.1 %DEA) | 463.1 |
| 198 | 172a or 172b | 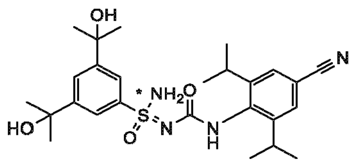 | N'-((4-cyano-2,6-diisopropylphenyl)carbamoyl)-3,5-bis(2-hydroxypropan-2-yl) benzenesulfonimidamide | ChiralPak IC, 2*25cm, Sum | 15% EtOH in Hex(0.1 %DEA) | 501.2 |
| 199 | 172b or 172a | 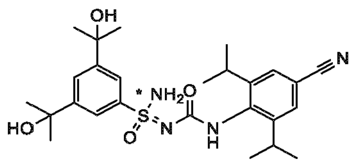 | N'-((4-cyano-2,6-diisopropylphenyl)carbamoyl)-3,5-bis(2-hydroxypropan-2-yl) benzenesulfonimidamide | ChiralPak IC, 2*25cm, Sum | 15% EtOH in Hex(0.1 %DEA) | 501.2 |
| 200 | 106a or 106b | 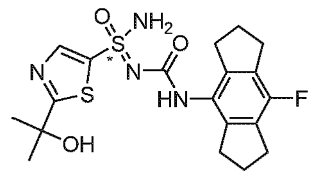 | (R)-or (S)-N'-(8-fluoro-1,2,3,5,6,7-hexahydro-s-indacen-4-ylcarbamoyl)-2-(2-hydroxypropan-2-yl) thiazole-5-sulfonimidamide | ChiralPak AD-H, 2*25cm, Sum | 25% EtOH in CO2 | 439.2 |
| 201 | 106b or 106a | 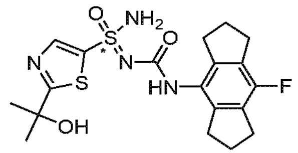 | (S)-or (R)-N'-(8-fluoro-1,2,3,5,6,7-hexahydro-s-indacen-4-ylcarbamoyl)-2-(2-hydroxypropan-2-yl) thiazole-5-sulfonimidamide | ChiralPak AD-H, 2*25cm, Sum | 25% EtOH in $CO_2$ | 439.2 |

(continued)

| Ex. # | Final Target Number | Structure | IUPAC Name | Column | Eluents | LC-MS $[M+H]^+$ |
|---|---|---|---|---|---|---|
| 202 | 136a or 136b | 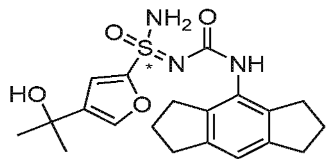 | (S)-or (R)-N'-(1,2,3,5,6,7-hexahydro-*s*-indacen-4-ylcarbamoyl)-4-(2-hydroxypropan-2-yl)furan-2-sulfonimidamide | Chiral ART Cellulose-SB, 2*25cm, Sum | 20% EtOH in Hex(0.2 %DEA) | 404.2 |
| 203 | 136b or 136a | 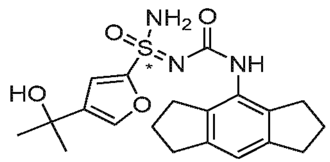 | (R)-or (S)-N'-(1,2,3,5,6,7-hexahydro-*s*-indacen-4-ylcarbamoyl)-4-(2-hydroxypropan-2-yl)furan-2-sulfonimidamide | Chiral ART Cellulose-SB, 2*25cm, Sum | 20% EtOH in Hex(0.2 %DEA) | 404.2 |
| 204 | 183a or 183b | 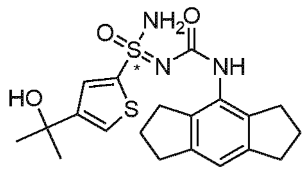 | (R)-or (S)-N'-(1,2,3,5,6,7-hexahydro-s-indacen-4-ylcarbamoyl)-4-(2-hydroxypropan-2-yl)thiophene-2-sulfonimidamide | ChiralPak ID, 2*25cm, Sum | 20% EtOH in Hex(0.1 %DEA) | 418.1 (M-1) |
| 205 | 183a or 183b | 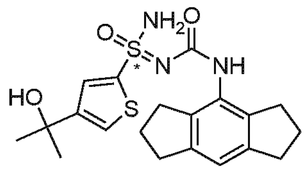 | (S)-or (R)-N'-(1,2,3,5,6,7-hexahydro-s-indacen-4-ylcarbamoyl)-4-(2-hydroxypropan-2-yl)thiophene-2-sulfonimidamide | ChiralPak ID, 2*25cm, Sum | 20% EtOH in Hex(0.1 %DEA) | 418.1 (M-1) |
| 206 | 176a or 176b | 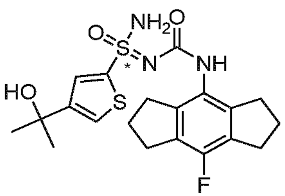 | (S)-or (R)-N'-(8-fluoro-1,2,3,5,6,7-hexahydro-s-indacen-4-ylcarbamoyl)-4-(2-hydroxypropan-2-yl) thiophene-2-sulfonimidamide | ChiralPak IG, 2*25cm, Sum | 30% EtOH in Hex | 438.2 |

(continued)

| Ex. # | Final Target Number | Structure | IUPAC Name | Column | Eluents | LC-MS $[M+H]^+$ |
|---|---|---|---|---|---|---|
| 207 | 176b or 176a | 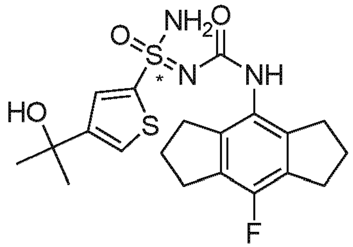 | (R)-or (S)-N'-(8-fluoro-1,2,3,5,6,7-hexahydro-s-indacen-4-ylcarbamoyl)-4-(2-hydroxypropan-2-yl) thiophene-2-sulfonimidamide | ChiralPak IG, 2*25cm, Sum | 30% EtOH in Hex | 438.2 |

**Example 77:** MS-ESI: 421.1 (M+1). $^1$H NMR (400 MHz, DMSO-$d_6$) δ 8.41 (br s, 1H), 7.74 (br s, 2H), 7.68 (s, 1H), 6.87 (s, 1H), 5.36 (s, 1H), 3.02 - 2.50 (m, 8H), 2.10 - 1.80 (m, 4H), 1.48 (s, 6H).

**Example 200:** MS-ESI: 439.2 (M+1). 1H NMR (400 MHz, DMSO-$d_6$) δ 8.38 (br, 1H), 8.02 (s, 1H), 7.75 (br, 1H), 6.27 (s, 1H), 2.81 (t, J= 7.6 Hz, 4H), 2.70 (t, J= 6.8 Hz, 4H), 2.02-1.95 (m, 4H), 1.50 (s, 6H).

**Example 203:** MS-ESI: 404.2 (M+1). $^1$H NMR (300 MHz, DMSO-$d_6$) δ 8.42 (br s, 1H), 7.76 (s, 1H), 7.72 (s, 2H), 7.01 (s, 1H), 6.88 (s, 1H), 5.11 (s, 1H), 2.90 - 2.72 (m, 4H), 2.72 - 2.60 (m, 4H), 2.10 -1.80 (m, 4H), 1.46 (s, 6H).

**Example 205:** MS-ESI: 418.1 (M-1). $^1$H NMR (400 MHz, DMSO-$d_6$) δ 8.39 (br s, 1H), 7.68 (s, 2H), 7.63 (s, 1H), 7.59 (s, 1H), 6.88 (s, 1H), 5.23 (s, 1H), 2.95 - 2.75 (m, 4H), 2.75 - 2.60 (m, 4H), 2.05 - 1.80 (m, 4H), 1.43 (s, 6H).

**Example 206:** MS-ESI: 438.2 (M+1). $^1$H NMR (300 MHz, DMSO-$d_6$) δ 8.41 (br s, 1H), 7.65 (s, 2H), 7.59 (s, 1H), 7.55 (s, 1H), 5.20 (s, 1H), 2.90 - 2.60 (m, 8H), 2.10 - 1.80 (m, 4H), 1.39 (s, 6H).

**Example 208 (Compound 221)**

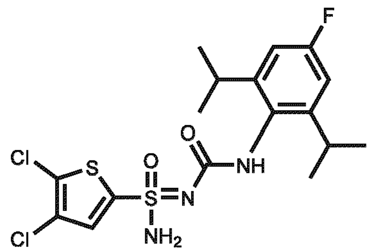

4,5-Dichloro-N'-((4-fluoro-2,6-diisopropylphenyl)carbamoyl)thiophene-2-sulfonimidamide

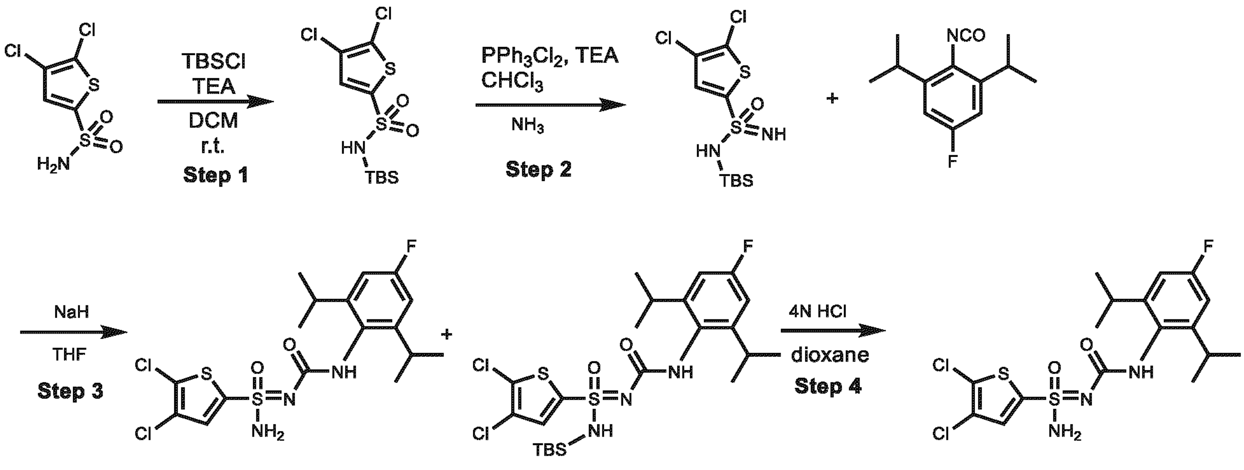


**Step 1: N-(tert-butyldimethylsilyl)-4,5-dichlorothiophene-2-sulfonamide**

4,5-Dichlorothiophene-2-sulfonamide (50 mg, 0.22 mmol) was dissolved in anhydrous $CH_2Cl_2$ (2 mL). Triethylamine (0.090 mL, 0.65 mmol) and TBSCl (38 mg, 0.25 mmol) were added and the resulting mixture was stirred overnight at room temperature, or until the reaction was complete as indicated by LCMS(Method F: m/Z=424.1 $[M+DMSO+H]^+$, retention time = 3.70 min). The reaction mixture was used in the next step as is.

**Step 2: N-(tert-butyldimethylsilyl)-4,5-dichlorothiophene-2-sulfonimidamide**

In an oven-dried vial under nitrogen, a solution of $PPh_3Cl_2$ (143 mg, 0.44 mmol) was prepared in dichloroethane (1.5 mL). Triethylamine (0.120 mL, 0.86 mmol) was introduced in a steady stream via syringe at 0 °C. The reaction mixture was stirred at room temperature for 10 min. The reaction mixture was then cooled in an ice / water bath for 2 min and the reaction mixture of TBS protected sulfonamide (prepared in 2 mL DCM) from **step 1** was introduced *via* syringe rapidly drop by drop (addition time < 30 seconds). The resulting mixture was stirred at 0 °C for 30 min, at which

time anhydrous ammonia was bubbled into the reaction mixture for 45 seconds. The suspension thus formed was stirred in an ice / water bath for 30 min and then warmed to room temperature and centrifuged to remove solids. The supernatant was concentrated *in vacuo* and dried under high vacuum for 30 min.

**Step 3: 4,5-Dichloro-N'-((4-fluoro-2,6-diisopropylphenyl)carbamoyl)thiophene- 2-sulfonimidamide and N-(tert-butyldimethylsilyl)-4,5-dichloro- N'-((4-fluoro- 2,6-diisopropyl phenyl)carbamoyl)thiophene-2-sulfonimidamide**

To the crude reaction mixture from step 2 was added anhydrous THF (1.5 mL) and the resulting solution was stirred in an ice / water bath for 5 min, at which time NaH (17 mg, 0.44 mmol) was added. After 2 min stirring, a solution of 5-fluoro-2-isocyanato-1,3-diisopropylbenzene (36.5 mg, 0.165 mmol) in THF (3 ml) was added dropwise at 0 °C. The resulting mixture was brought to room temperature and stirred for 30 min to give a mixture of crude products. LC-MS (Method F): m/Z=451.8 $[M+H]^+$, retention time = 6.18 min; for TBS-protected product, 566.4 $[M+H]^+$, retention time = 9.25 min.

**Step 4: 4,5-Dichloro-N'-((4-fluoro-2,6-diisopropylphenyl)carbamoyl)thiophene- 2-sulfonimidamide**

To the reaction mixture from step 3 was carefully added 4N HCl in dioxane (0.3 mL) and the resulting mixture was stirred at room temperature for approximately 30 min until the completion of reaction, as determined by LCMS analysis (Method F: 451.8 $[M+H]^+$, retention time = 6.18 min). The reaction mixture was then concentrated *in vacuo.* DMSO (0.5 mL) was added to the residue and the resulting solution was purified on a prep-HPLC to afford the title compound. LC-MS: 451 $[M+H]^+$.

**Table 20.** Examples in the following table were prepared using similar procedures as described in **Example 208** above starting from appropriate sulfonamides.

| Ex. # | Final Target # | | IUPAC Name | Exact Mass $[M+H]^+$ |
|---|---|---|---|---|
| 209 | 219 | | N'-((4-fluoro-2,6-diisopropylphenyl)carbamoyl)-1,3 -dimethyl- 1H-pyrazole-4-sulfonimidamide | 396.05 |
| 210 | 217 | | N'-((4-fluoro-2,6-diisopropylphenyl)carbamoyl) naphthalene-2-sulfonimidamide | 428.17 |
| 211 | 216 | | N'-((4-fluoro-2,6-diisopropylphenyl)carbamoyl)-2,3-dihydrobenzofuran-5-sulfonimidamide | 420.07 |
| 212 | 215 | | N'-((4-fluoro-2,6-diisopropylphenyl)carbamoyl)-[1,1'-biphenyl]-2-sulfonimidamide | 454.28 |
| 213 | 218 | | N'-((4-fluoro-2,6-diisopropylphenyl)carbamoyl)-2-(methoxymethyl)benzenesulfonimidamide | 422.17 |
| 214 | 214 | | 2,5-dichloro-N'-((4-fluoro-2,6-diisopropylphenyl) carbamoyl)thiophene-3 -sulfonimidamide | 452.18 |
| 215 | 211 | | N'-((4-fluoro-2,6-diisopropylphenyl) carbamoyl) pyridine-3-sulfonimidamide | 379.24 |
| 216 | 210 | | N'-((4-fluoro-2,6-diisopropylphenyl)carbamoyl) benzo [d] [1,3]dioxole-5-sulfonimidamide | 422.17 |
| 217 | 201 | | N'-((4-fluoro-2,6-diisopropylphenyl) carbamoyl)-2,5-dimethylfuran-3-sulfonimidamide | 396.40 |
| 218 | 200 | | N'-((4-fluoro-2,6-diisopropylphenyl) carbamoyl) quinoline-3-sulfonimidamide | 429.40 |
| 219 | 199 | | N'-((4-fluoro-2,6-diisopropylphenyl)carbamoyl)-6,7-dihydro-5H-pyrrolo[1,2-a]imidazole-3 -sulfonimidamide | 408.40 |
| 220 | 198 | | N'-((4-fluoro-2,6-diisopropylphenyl)carbamoyl)-5-methylpyridine-2-sulfonimidamide | 393.40 |

**Example 221 (Compound 141)**

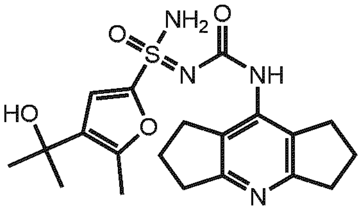

*N'*-((1,2,3,5,6,7-hexahydrodicyclopenta[b,e]pyridin-8-yl)carbamoyl)-4-(2-hydroxypropan-2-yl)-5-methylfuran-2-sulfonimidamide (**Scheme 31**)

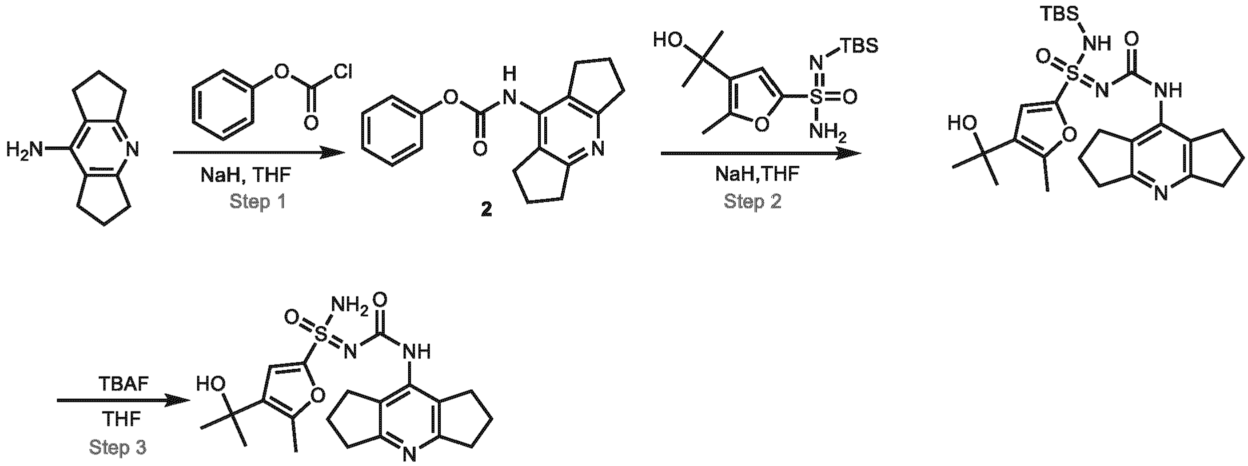


**Example 221**

**Step 1: Phenyl (1,2,3,5,6,7-hexahydrodicyclopenta[b,e]pyridin-8-yl)carbamate**

Into a 50-mL 3-necked round-bottom flask purged and maintained with nitrogen, was placed 1,2,3,5,6,7-hexahydrodicyclopenta[b,e]pyridin-8-amine (50 mg, 0.29 mmol) in THF (10 mL), to this was added NaH (60%wt. oil dispersion, 22.8 mg, 0.57 mmol) at 0 °C; and then phenyl chloroformate (67.4 mg, 0.43 mmol,) in THF (2.0 mL) was added dropwise at 0 °C. The resulting solution was stirred for 2 h at RT. This reaction solution was used for next step directly without any purification.

**Step 2: *N*-(tert-butyldimethylsilyl)-*N'*-((1,2,3,5,6,7-hexahydrodicyclopenta[b,e]pyridin-8-yl)carbamoyl) -4-(2-hydroxypropan-2-yl)-5-methylfuran-2-sulfonimidamide**

Into a 50-mL 3-necked round-bottom flask purged and maintained with nitrogen, was placed N-(tert-butyldimethylsilyl)-4-(2-hydroxypropan-2-yl)-5-methylfuran-2-sulfonoimidamide (96 mg, 0.29 mmol) in THF (10 mL). To this was added NaH (60%wt. oil dispersion, 23.2 mg, 0.58 mmol) at 0 °C, followed by phenyl (1,2,3,5,6,7-hexahydrodicyclopenta[b,e]pyridin-8-yl)carbamate (127 mg, 0.43 mmol) crude in THF from via syringe rapidly drop by drop. The resulting mixture was stirred for 16 h at RT. The reaction was then quenched by the addition of 5.0 mL of water. The resulting solution was extracted with 4x10 ml of ethyl acetate. The combined organic layers were dried over anhydrous sodium sulfate and concentrated under vacuum. The residue was eluted from a silica gel column with ethyl acetate/petroleum ether (1;1). This resulted in 50 mg (38.4%) of the title compound as an off-white solid. MS-ESI: 533(M+1).

**Step 3: *N'*-((1,2,3,5,6,7-hexahydrodicyclopenta[b,e]pyridin-8-yl)carbamoyl)-4-(2-hydroxypropan-2-yl) -5-methylfuran-2-sulfonimidamide**

Into a 50-mL round-bottom flask, was placed N-(tert-butyldimethylsilyl)-N'-((1,2,3,5,6,7 -hexahydrodicyclopenta[b,e]pyridin-8-yl)carbamoyl)-4-(2-hydroxypropan-2-yl)-5-methylfuran-2-sulfonimidamide (58 mg, 0.11 mmol) in THF (10 mL), to this was added TBAF (28.8 mg, 0.11 mmol). The resulting solution was stirred for 1 h at RT. The resulting mixture was concentrated under vacuum. The residue was eluted from a silica gel column with DCM/MeOH (10:1). The crude product was further purified by Prep-HPLC with the following conditions: Column: XBridge Prep OBD C18 Column 19*250 mm, 5um; Mobile Phase A: water (10 mM $NH_4HCO_3$), Mobile Phase B: ACN; Flow rate: 25 mL/min; Gradient: 11% B to 40% B in 7 min; UV 254/210 nm; Rt: 6 min. This resulted in 25 mg (54.87%) of Example 221 as a white solid. MS-ESI: 419 (M+1). $^1$H NMR (400 MHz, DMSO-$d_6$, ppm) δ: 8.82 (s, 1H), 7.65 (s, 2H), 6.90 (s, 1H), 5.03 (s, 1H), 2.82-2.78 (m, 4H), 2.76-2.67 (m, 4H), 2.41 (s, 3H), 2.00-1.92 (m, 4H), 1.39 (s, 6H).

**Table 21.** Examples in the following table were prepared using similar conditions as described in Example **221** and Scheme **31** from appropriate starting materials.

| Ex. # | Final Target # | Structure | IUPAC Name | Exact Mass [M+H]$^+$ |
|---|---|---|---|---|
| 222 | 140 | 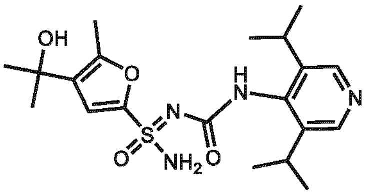 | N'-((3,5-diisopropylpyridin-4-yl) carbamoyl)-4-(2-hydroxypropan-2-yl)-5-methylfuran-2-sulfonimidamide | 423 |

**Example 223 (Compound 321)**

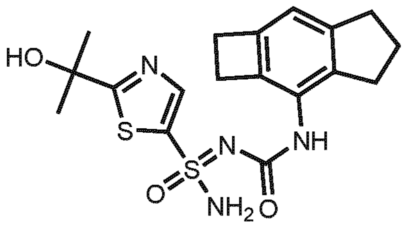

2-(2-Hydroxypropan-2-yl)-*N'*-((2,4,5,6-tetrahydro-1*H*-cyclobuta[f]inden-3-yl)carbamoyl)thiazole-5-sulfonimidamide (Scheme 3A)

**Examples 224 and 225 (Compound 321b and 321a)**

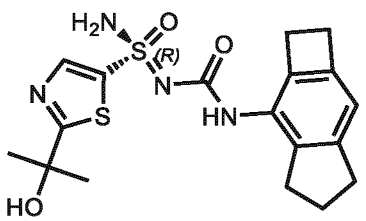

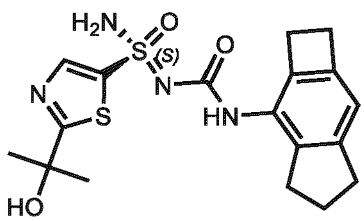

**Examples 224 and 225 (stereochemistry tentatively assigned)**

(R)- and (S)- 2-(2-hydroxypropan-2-yl)-*N'*-((2,4,5,6-tetrahydro-1*H*-cyclobuta[f]inden-3-yl)carbamoyl)thiazole-5-sulfonimidamide

## Route 1:

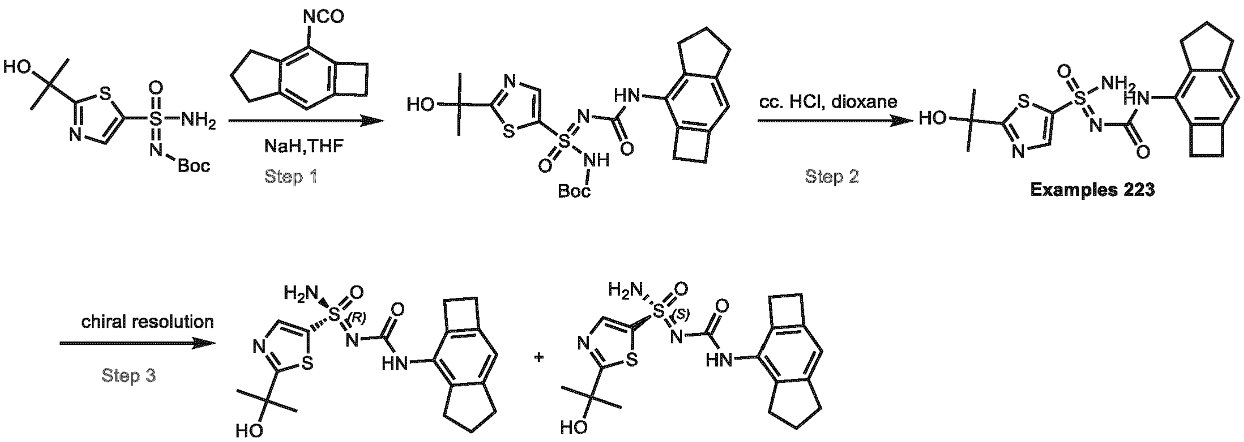


**Examples 223**

+

**Examples 224 and 225 (stereochemistry tentatively assigned)**

**Step 1: *Tert*-butyl(2-(2-hydroxypropan-2-yl)-*N*-((2,4,5,6-tetrahydro-1*H*-cyclobuta[f]inden-3-yl) carbamoyl)thiazole-5-sulfonimidoyl)carbamate**

Into a 100-mL round-bottom flask purged and maintained with an inert atmosphere of nitrogen, was placed *tert*-butyl *N*-[amino[2-(2-hydroxypropan-2-yl)-1,3-thiazol-5-yl]oxo-$\lambda^6$-sulfanylidene]carbamate (1.39g, 4.32 mmol) in THF (50 mL). To this solution was added NaH (60%wt. oil dispersion, 518 mg, 13mmol) at 0 °C, followed by the addition of 3-isocyanato-2,4,5,6-tetrahydro-1H-cyclobuta[f]indene (800 mg, 4.32 mmol) in THF (5.0 mL) dropwise at 0 °C. The resulting solution was stirred for 14 h at RT. The reaction was then quenched by the addition of 100 mL of water. The resulting solution was extracted with 3x50 mL of DCM. The organic layers combined and dried over anhydrous $Na_2SO_4$, then concentrated. The residue was eluted from a silica gel column with ethyl acetate/petroleum ether (1:5 to 1:1). This resulted in 2.0 g (91%) of title compound as a light yellow solid. MS-ESI: 507 (M+1).

**Step 2: 2-(2-Hydroxypropan-2-yl)-*N'*-((2,4,5,6-tetrahydro-1*H*-cyclobuta[f]inden-3-yl)carbamoyl) thiazole-5-sulfonimidamide**

Into a 100-mL round-bottom flask purged and maintained with an inert atmosphere of nitrogen, was placed tert-butyl(2-(2-hydroxypropan-2-yl)-N-((2,4,5,6-tetrahydro-1H-cyclobuta[f]inden-3-yl)carbamoyl) thiazole-5-sulfonimidoyl)carbamate (2.2 g, 4.34 mmol) in dioxane (40 mL). To this was added conc. HCl (8 mL,12 M) dropwise at 0 °C. The resulting solution was stirred for 14 h at RT. The resulting solution was diluted with 100 mL of water. The resulting solution was extracted with 3x50 mL of DCM. The organic layers combined and dried over anhydrous $Na_2SO_4$, then concentrated. The crude product was purified by HP-Flash with the following conditions: Column, C18 silica gel; mobile phase, ACN:$H_2O$=25:75 increasing to ACN:$H_2O$=55:45 within 25 ; Detector, UV 254 nm. This resulted in 1.5 g (85%) of Example 223. MS-ESI: 407 (M+1). $^1H$ NMR (300 MHz, DMSO-$d_6$) $\delta$ 8.35 (s, 1H), 8.05 (s, 1H), 7.74 (s, 2H), 6.66 (s, 1H), 6.25 (s, 1H), 3.06 - 2.94 (m, 2H), 2.93 - 2.84 (m, 2H), 2.82 - 2.60 (m, 4H), 2.03 - 1.79 (m, 2H), 1.50 (s, 6H).

**Step 3: Chiral resolution**

Example 223 (1.5 g) was separated with the followed condition: Column: CHIRALPAK IG, 20*250 mm, 5 um; Mobile Phase A: $CO_2$: 60, Mobile Phase B: MeOH-----Preparative: 40; Flow rate: 50 mL/min; 220 nm. The resulting solution was stirred for 20 min at 10 °C. This resulted in 546 mg (99%ee, 36.4%) of Example 224 (RTi: 3.47 min) as a white solid and 595 mg (99%ee, 39.6%) of Example 225 ($RT_2$: 5.35 min) as a white solid. The absolute stereochemistry was tentatively assigned.

**Example 224:** MS-ESI: 407.1 (M+1). $^1H$ NMR (300 MHz, DMSO-$d_6$) $\delta$ 8.35 (s, 1H), 8.05 (s, 1H), 7.74 (s, 2H), 6.66 (s, 1H), 6.25 (s, 1H), 3.06 - 2.94 (m, 2H), 2.93 - 2.84 (m, 2H), 2.82 - 2.60 (m, 4H), 2.03 - 1.79 (m, 2H), 1.50 (s, 6H).

**Example 225:** MS-ESI: 407.1 (M+1). $^1H$ NMR (300 MHz, DMSO-$d_6$) $\delta$ 8.35 (s, 1H), 8.05 (s, 1H), 7.74 (s, 2H), 6.66 (s, 1H), 6.25 (s, 1H), 3.06 - 2.94 (m, 2H), 2.93 - 2.84 (m, 2H), 2.82 - 2.60 (m, 4H), 2.03 - 1.79 (m, 2H), 1.50 (s, 6H).

**Route 2:**

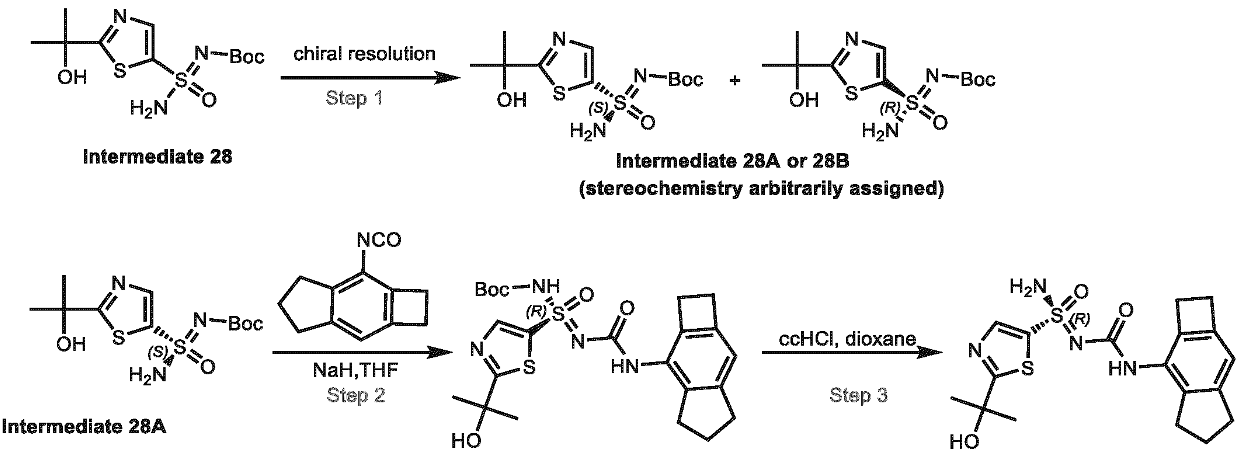


**Intermediate 28**

**Intermediate 28A or 28B (stereochemistry arbitrarily assigned)**

**Intermediate 28A**

**Example 224**

**Step 1: Chiral resolution (R) and (S)-tert-butyl(amino(2-(2-hydroxypropan-2-yl)thiazol-5-yl)(oxo)-λ$^6$-sulfaneylidene)carbamate**

The product10 g of **Intermediate 28** was separated with the followed condition: Column: CHIRALPAK IC, 5*25 cm,5 um; Mobile Phase A:$CO_2$ :55, Mobile Phase B: EtOH:HeX=1:1:45; Flow rate: 150 mL/min; UV 220 nm; Rti: 5.13 (**Intermediate 28A**); $Rt_2$: 5.65 (**Intermediate 28B**). This resulted in 3 g (99.5% ee, 60%) of **28A,** and 3 g (99.0 % ee, 60%) **of 28B.**

**Step 2: *Tert*-butyl (R)-(2-(2-hydroxypropan-2-yl)-*N*-((2,4,5,6-tetrahydro-1*H*-cyclobuta[f]inden-3-yl) carbamoyl)thiazole-5-sulfonimidoyl)carbamate**

Into a 100-mL round-bottom flask purged and maintained with an inert atmosphere of nitrogen, was placed **intermediate 28A** (>99% ee, 1.67 g, 5.20 mmol) in THF (50 mL), NaH (60% wt. oil dispersion, 624 mg, 15.6 mmol) was added at 0 °C, this was followed by the addition of 3-isocyanato-2,4,5,6-tetrahydro-1H-cyclobuta[f]indene (850 mg, crude) in THF (5 mL) dropwise at 0 °C. The resulting solution was stirred for 14 h at RT. The reaction was then quenched by the addition of 100 mL of water. The resulting solution was extracted with 3x100 mL of DCM. The organic layers combined and dried over anhydrous $Na_2SO_4$, then concentrated. This resulted in 2.2 g (83.5%) of title compound as a light yellow solid. MS-ESI: 507 (M+1).

**Step 3: (R)-2-(2-hydroxypropan-2-yl)-N'-((2,4,5,6-tetrahydro-1H- cyclobuta[f]inden-3-yl)carbamoyl) thiazole-5-sulfonimidamide**

Into a 100-mL round-bottom flask purged and maintained with an inert atmosphere of nitrogen, was placed tert-butyl (S)-(2-(2-hydroxypropan-2-yl)-N-((2,4,5,6-tetrahydro-1H-cyclobuta[f] inden-3-yl)carbamoyl) thiazole-5-sulfonimidoyl)carbamate (2.2 g, 4.34 mmol) in dioxane (40 mL), to this was added conc. HCl (8 mL, 12M) dropwise at 0 °C. The resulting solution was stirred for 8 h below 10 °C. The resulting solution was diluted with 100 mL of water. The resulting solution was extracted with 3x100 mL of DCM. The organic layers combined and dried over anhydrous $Na_2SO_4$, then concentrated. The crude product was purified by HP-Flash with the following conditions: Column, C18 silica gel; mobile phase, MeCN:water=25:75 increasing to MeCN:water=55:45 within 30 min; Detector, UV 210 nm. This resulted in 1.37 g (77.3%) of Example 224 (99.4% ee) as a white solid. MS-ESI: 407 (M+1).

$^1$H NMR (300 MHz, DMSO-$d_6$) δ 8.43 (s, 1H), 8.09 (s, 1H), 7.90 (s, 2H), 6.67 (s, 1H), 6.29 (s, 1H), 2.92 (d, *J* = 3.9 Hz, 2H), 2.89 (d, *J* = 3.9 Hz, 2H), 2.90 - 2.55 (m, 4H), 2.00 - 1.75 (m, 6H), 1.50 (s, 6H).

**Table 22.** Examples in the following table were prepared using similar conditions as described in Example **223- Route 1** and Scheme **3A** from appropriate starting materials.

| Ex. # | Final Target # | Structure | IUPAC Name | Exact Mass [M+H]+ |
|---|---|---|---|---|
| 226 | 329 | 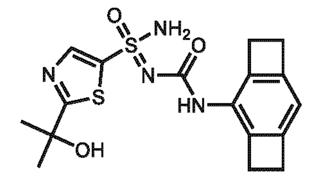 | 2-(2-Hydroxypropan-2-yl)-N'-(tricyclo [6.2.0.03,6]deca-1,3(6),7-trien-2-ylcarbamoyl)triazole-5-sulfonimidamide | 393 |
| 227 | 375 | 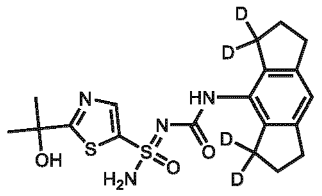 | N'-((1,2,3,5,6,7-hexahydro-*s*-indacen-4-yl-3,3,5,5-$d_4$)carbamoyl)-2-(2-hydroxypropan-2-yl)thiazole-5-sulfonimidamide | 425 |
| 228 | 376 | 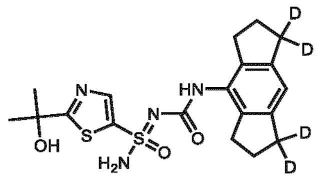 | N'-((1,2,3,5,6,7-hexahydro-s-indacen-4-yl-1,1,7,7-$d_4$)carbamoyl)-2-(2-hydroxypropan-2-yl)thiazole-5-sulfonimidamide | 425 |

**Example 229 (Compound 307)**

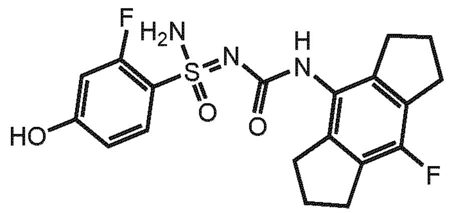

2-Fluoro-*N'*-((8-fluoro-1,2,3,5,6,7-hexahydro-*s*-indacen-4-yl)carbamoyl)-4-hydroxybenzenesulfonimidamide (Scheme 3B)

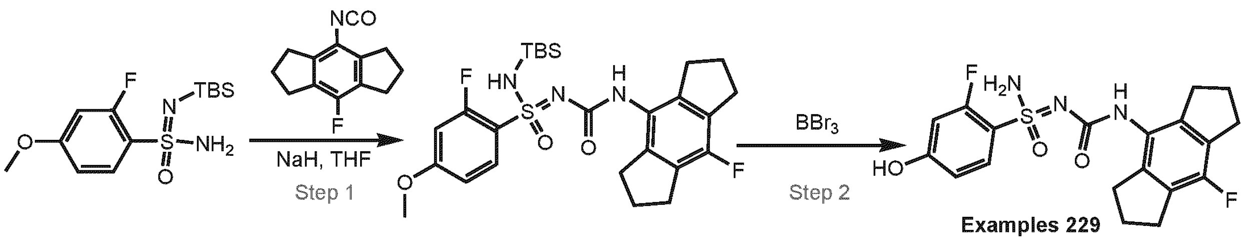


**Step1: *N*-(*tert*-butyldimethylsilyl)-2-fluoro-*N'*-(8-fluoro-1,2,3,5,6,7-hexahydro-*s*-indacen-4-ylcarbamoyl)-4-methoxybenzenesulfonimidamide**

Into a 50-mL round-bottom flask, was placed a solution of N-(tert-butyldimethylsilyl)-2-fluoro-4-methoxybenzene-1-sulfonoimidamide (139 mg, 0.44 mmol) in THF (5.0 mL). To this solution was added NaH (60%wt. oil dispersion,

35.2 mg, 0.44 mmol) at 0 °C.This was followed by the addition of 4-fluoro-8-isocyanato-1,2,3,5,6,7-hexahydro-s-indacene (95 mg, 0.44 mmol) in THF (5 mL) dropwise at RT. The resulting solution was stirred for 1 h at RT. The reaction was then quenched by the addition of 100 mL of water. The resulting solution was extracted with 3x50 mL of ethyl acetate. The organic layers combined and dried over anhydrous $Na_2SO_4$, and then concentrated. The residue was eluted from a silica gel column with ethyl acetate/petroleum ether (1:5 to 1:1). This resulted in 120 mg (51.2%) of the title compound as yellow oil. MS-ESI: 536 (M+1).

**Step 2: 2-Fluoro-*N'*-(8-fluoro-1,2,3,5,6,7-hexahydro-*s*-indacen-4-ylcarbamoyl)-4-hydroxybenzenesulfonimidamide**

Into a 50-mL round-bottom flask, was placed a solution of 1-[[(*tert*-butyldimethylsilyl)imino](2-fluoro-4- methoxybenzene)sulfinyl]-3-(8-fluoro-1,2,3,5,6,7-hexahydro-*s*-indacen-4-yl)urea (120 mg, 0.22 mmol) in ACN (5.0 mL), to this solution was added $BBr_3$ (561 mg, 2.24 mmol) dropwise at 0 °C. The resulting solution was stirred for 2 h at RT. The reaction was then quenched by the addition of 5 mL of MeOH. The resulting mixture was concentrated. The crude product (100 mg) was purified by Prep-HPLC under the following conditions: Column, XBridge Prep OBD C18, 19*250 mm, 5 um; mobile phase: water (10 mM $NH_4HCO_3$) and ACN (25% to 43% ACN gradient in 7 min); Detector, UV. This resulted in 17.7 mg (19.4%) of Example 229 as a white solid. MS-ESI: 408 (M+1).

**Example 230 (Compound 323)**

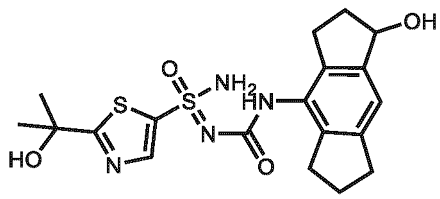

*N'*-((1-hydroxy-1,2,3,5,6,7-hexahydro-*s*-indacen-4-yl)carbamoyl)-2-(2-hydroxypropan-2-yl)thiazole-5-sulfonimidamide (Scheme 32)

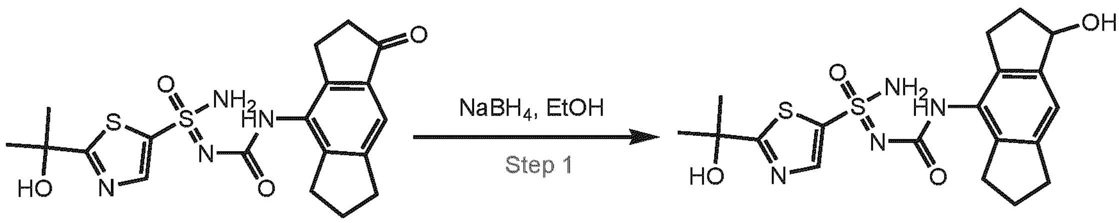


Example 293

Examples 230

Into a 50-mL round-bottom flask, was placed 2-(2-hydroxypropan-2-yl)-N'-((1-oxo-1,2,3,5,6,7-hexahydro-*s*-indacen-4-yl)carbamoyl)thiazole-5-sulfonimidamide (100 mg, 0.23 mmol) in ethanol (10 mL). To this solution was added $NaBH_4$ (17.4 mg, 0.46 mmol) in portions at 0 °C. The resulting solution was stirred for 2 h at RT. The crude product (5 mL) was purified by Flash-Prep-HPLC with the following conditions: Column: XBridge Prep OBD C18 Column 30×150mm 5um; Mobile Phase A: water (10 mM $NH_4HCO_3$), Mobile Phase B: ACN; Flow rate: 60 mL/min; Gradient: 10% to 28% B in 7 min; 210/254 nm; Rt: 6.00 min. This resulted in 180 mg of the title compound (Example 230) as a solid. MS-ESI: 437.1 (M+1).

$^1H$ NMR (400 MHz, DMSO-$d_6$) $\delta$ 8.51 (br s, 1H), 8.04 (s, 1H), 7.82 (br s, 2H), 6.97 (s, 1H), 6.28 (s, 1H), 5.07 (d, *J* = 5.6 Hz, 1H), 5.05-4.85 (m, 1H),2.95-2.75 (m, 2H), 2.75-50 (m, 4H), 2.35-2.15 (m, 1H), 2.00-1.80 (m, 2H), 1.80-1.60 (m, 1H), 1.51 (s, 6H).

**Example 231 (Compound 338)**

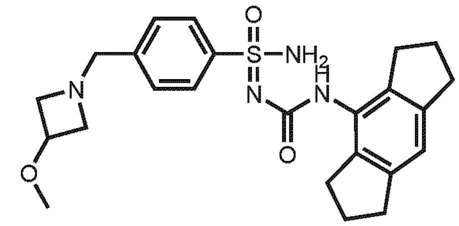

N'-((1,2,3,5,6,7-hexahydro-s-indacen-4-yl)carbamoyl)-4-((3-methoxyazetidin-1-yl)methyl)benzenesulfonimidamide (Scheme 33A)

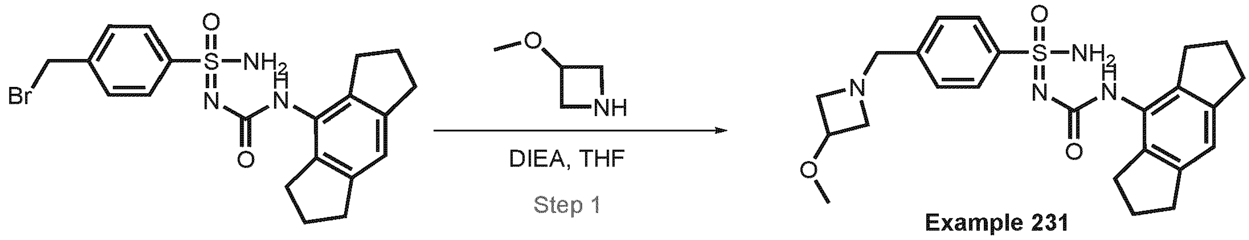


Into a 50-mL round-bottom flask purged and maintained with an inert atmosphere of nitrogen, was placed 1-[amino[4-(bromomethyl)phenyl]oxo-$\lambda^6$-sulfanylidene]-3-(1,2,3,5,6,7-hexahydro-s-indacen-4-yl)urea(50 mg, 0.11 mmol) in THF(5 mL). To this solution was added DIEA (28.4 mg, 0.22mmol) and 3-methoxyazetidine(10.5 mg, 0.12 mmol) at RT. The resulting solution was stirred for 1 h at 65 °C. The resulting mixture was concentrated. The crude product was purified by Prep-HPLC with the following conditions: Column: XBridge Prep C18 OBD Column 19×100mm Sum 13nm; Mobile Phase A: water (10 mM $NH_4HCO_3$mM+0.1%$NH_3\cdot H_2O$), Mobile Phase B: ACN; Flow rate: 25 mL/min; Gradient: 30% to 37% B in 9.5 min; 254/210 nm; Rt: 9.62 min This resulted in 5 mg of Example **231** as a white solid. MS-ESI: 455 (M+1). $^1$H NMR (300MHz , DMSO-$d_6$) δ: 8.27 (br s, 1H), 7.81 (d, J = 8.4 Hz, 2H), 7.45 (d, J = 8.4 Hz, 2H), 7.34 (s, 2H), 6.85 (s, 1H), 4.02-3.94 (m, 1H), 3.67 (s, 2H), 3.51-3.46 (m, 2H), 3.14(s, 3H), 2.95-2.80 (m, 2H), 2.78-2.73 (m, 4H), 2.69-2.63 (m, 4H), 1.96-1.88 (m, 4H).

**Table 23.** Examples in the following table were prepared using similar conditions as described in Example **231** and Scheme **33A** from appropriate starting materials.

| Ex. # | Final Target # | Structure | IUPAC Name | Exact Mass [M+H]+ |
|---|---|---|---|---|
| 232 | 341 | 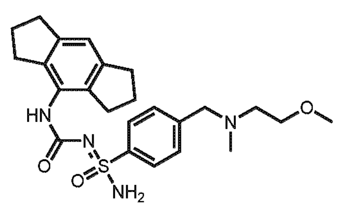 | N'-(1,2,3,5,6,7-hexahydro-s-indacen-4-ylcarbamoyl)-4-(((2-methoxyethyl)(methyl) amino)methyl)benzenesulfonimidamide | 457 |
| 233 | 342 | 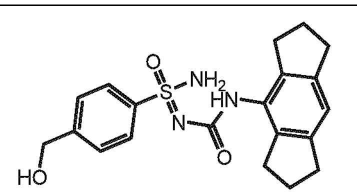 | N'-(1,2,3,5,6,7-hexahydro-s-indacen-4-ylcarbamoyl)-4-(hydroxymethyl) benzenesulfonimidamide | 386 |

(continued)

| Ex. # | Final Target # | Structure | IUPAC Name | Exact Mass [M+H]+ |
|---|---|---|---|---|
| 234 | 345 | 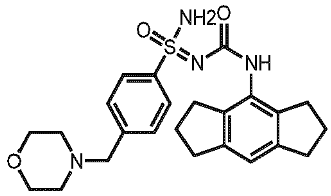 | *N'*-(1,2,3,5,6,7-hexahydro-*s*-indacen-4-ylcarbamoyl)-4-(morpholinomethyl) benzenesulfonimidamide | 455 |
| 235 | 346 | 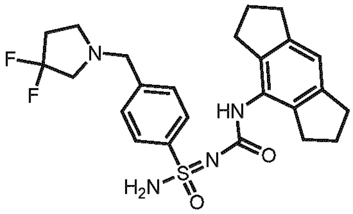 | 4-((3,3-Difluoropyrrolidin-1-yl) methyl)-*N'*-(1,2,3,5,6,7-hexahydro-s-indacen-4-ylcarbamoyl) benzenesulfonimidamide | 475 |
| 236 | 347 | 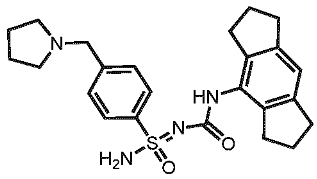 | *N'*-(1,2,3,5,6,7-hexahydro-*s*-indacen-4-ylcarbamoyl)-4-(pyrrolidin-1-ylmethyl) benzenesulfonimidamide | 439 |
| 237 | 348 | 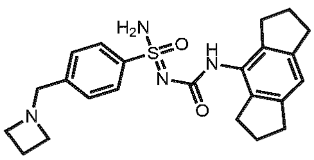 | 4-(Azetidin-1-ylmethyl)-*N'*-(1,2,3,5,6,7-hexahydro-*s*-indacen-4-ylcarbamoyl) benzenesulfonimidamide | 425 |
| 238 | 403 | 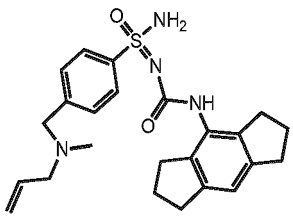 | 4-((Allyl(methyl)amino) methyl)-*N'*-(1,2,3,5,6,7-hexahydro-*s*-indacen-4-ylcarbamoyl) benzenesulfonimidamide | 439 |
| 239 | 402 | 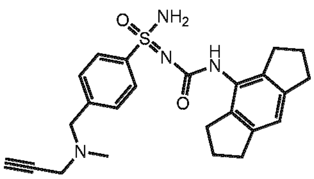 | *N'*-(1,2,3,5,6,7-hexahydro-*s*-indacen-4-ylcarbamoyl)-4-((methyl(prop-2-ynyl)amino) methyl)benzenesulfonimidamide | 437 |
| 240 | 350 | 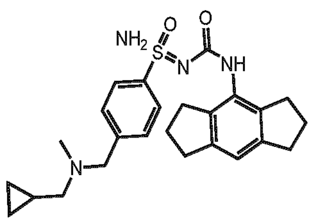 | 4-(((Cyclopropylmethyl)(methyl)amino) methyl)-*N'*-(1,2,3,5,6,7-hexahydro-s-indacen-4-ylcarbamoyl) benzenesulfonimidamide | 453 |

(continued)

| Ex. # | Final Target # | Structure | IUPAC Name | Exact Mass [M+H]+ |
|---|---|---|---|---|
| 241 | 322 | 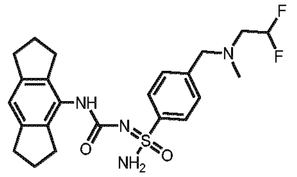 | 4-(((2,2-Difluoroethyl)(methyl)amino) methyl)-N'-(1,2,3,5,6,7-hexahydro-s-indacen-4-ylcarbamoyl) benzenesulfonimidamide | 463 |
| 242 | 351 | 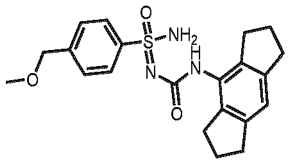 | *N'*-(1,2,3,5,6,7-hexahydro-*s*-indacen-4-ylcarbamoyl)-4-(methoxymethyl) benzenesulfonimidamide | 400 |
| 243 | 358 | 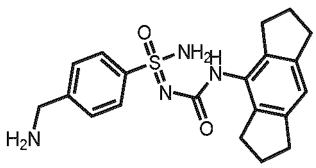 | 4-(Aminomethyl)-N'-(1,2,3,5,6,7-hexahydro-*s*-indacen-4-ylcarbamoyl) benzenesulfonimidamide | 385 |

**Example 244 (Compound 401)**

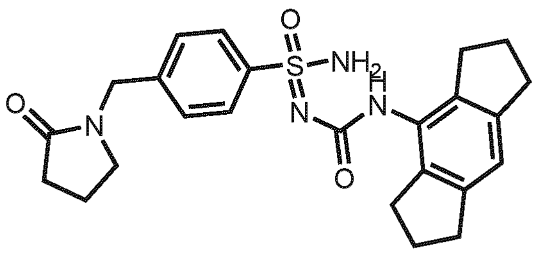

*N'*-((1,2,3,5,6,7-hexahydro-*s*-indacen-4-yl)carbamoyl)-4-((2-oxopyrrolidin-1-yl)methyl)benzenesulfonimidamide (Scheme 33B)

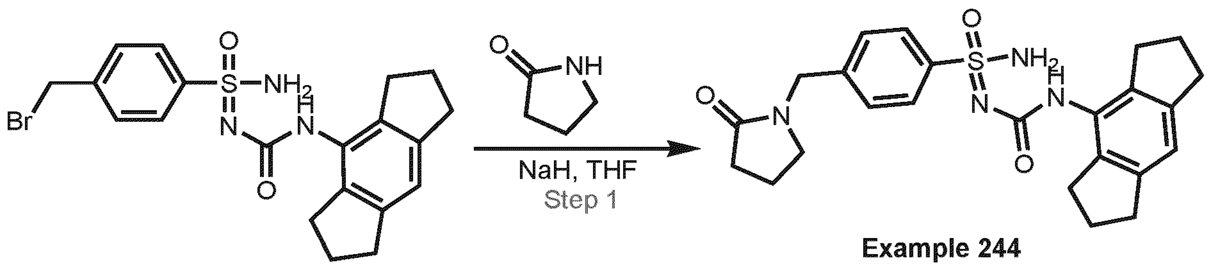


Into a 40-mL sealed tube purged and maintained with an inert atmosphere of nitrogen, was placed 1-[amino[4-(bromomethyl)phenyl]oxo-$\lambda^6$-sulfanylidene]-3-(1,2,3,5,6,7-hexahydro-*s*-indacen-4-yl)urea (200 mg, 0.45 mmol) in THF (10 mL), to this stirred solution was added DIEA (173 mg, 1.34 mmol) and pyrrolidin-2-one (114 mg, 1.34 mmol) at RT. The resulting solution was stirred for 3 h at 60 °C. The resulting mixture was concentrated. The crude product was purified by Prep-HPLC with the following conditions: Column, XBridge Prep OBD C18, 30x150 mm 5 um; mobile

phase, water (10 mM $NH_4HCO_3$) and ACN (25% to 44% ACN gradient in 7 min); Detector, UV. This resulted in 10 mg (4.95%) of **Example 244** as a white solid. MS-ESI: 453 (M+1).

$^1H$ NMR (400 MHz, DMSO-$d_6$) δ: 8.26 (br s, 1H), 7.83 (d, J = 8.0 Hz, 2H), 7.40 (d, J = 8.0 Hz, 2H), 7.27 (br s, 2H), 6.85 (s, 1H), 4.43 (s, 2H), 3.26-3.22 (m, 2H), 2.78-2.74 (m, 4H), 2.65-2.61 (m, 4H), 2.30 (t, J = 8.20 Hz, 2H), 1.98-1.89 (m, 6H).

**Example 245 (Compound 404)**

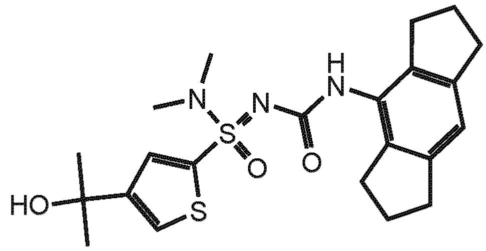

N'-((1,2,3,5,6,7-hexahydro-s-indacen-4-yl)carbamoyl)-4-(2-hydroxypropan-2-yl)-N,N-dimethylthiophene-2-sulfonimidamide (Scheme 4A)

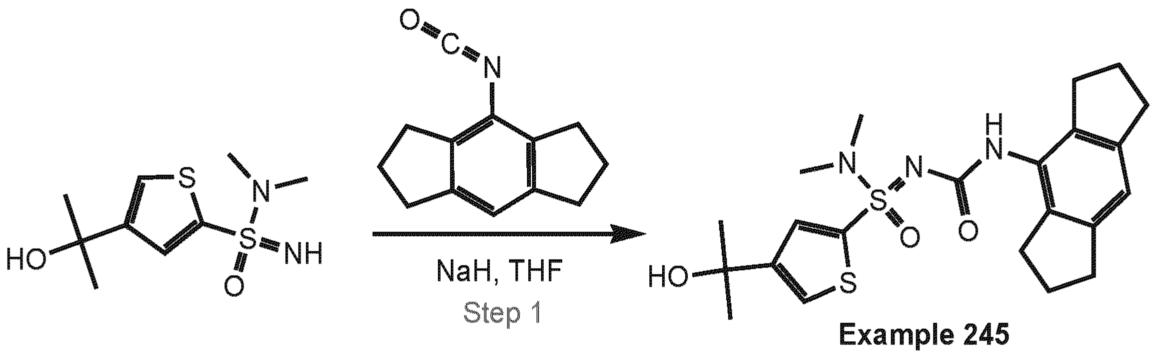


Into a 50-mL 3-necked round-bottom flask, was placed a solution of 4-(2-hydroxypropan-2-yl)-N,N- dimethylthiophene-2-sulfonoimidamide (125 mg, 0.50 mmol) in THF (2.0 mL). To this was added NaH (60%wt. oil dispersion, 30.2 mg, 0.75 mmol) in several batches at 0 °C in an ice/water bath. To the mixture was added 4-isocyanato-1,2,3,5,6,7-hexahydro-s-indacene (110 mg, 0.55 mmol) at 0 °C in an ice/water bath. The resulting solution was stirred for 30 min at 0 °C in a water/ice bath. The reaction was then quenched by the addition of $NH_4Cl$ (aq.). The resulting solution was extracted with ethyl acetate and the organic layers combined, the organic layer was dried over anhydrous sodium sulfate and concentrated under vacuum. The crude product was purified by Prep-HPLC with the following conditions: X Bridge Prep $C_{18}$ OBD, 19*150mm 5 um; mobile phase, water (10 mM $NH_4HCO_3$) and ACN (10% to 80% in 6 min); Detector, UV 254 nm. This resulted in 90 mg (39.9%) of **Example 245** as a white powder. MS-ESI: 448.2 (M+1). $^1H$ NMR (DMSO-$d_6$, 300 MHz): δ 8.60 (br s, 1H), 7.71 (s, 1H), 7.58 (br s, 1H), 6.88 (s, 1H), 5.21 (s, 1H), 2.86-2.70 (m, 8H), 2.70 (s, 6H), 1.98-1.90 (m, 4H), 1.3 (s, 6H).

**Example 246 (Compound 331)**

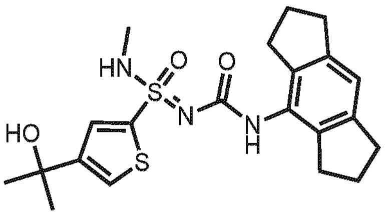

N'-((1,2,3,5,6,7-hexahydro-*s*-indacen-4-yl)carbamoyl)-4-(2-hydroxypropan-2-yl)-N-methylthiophene-2-sulfonimidamide (Scheme 4)

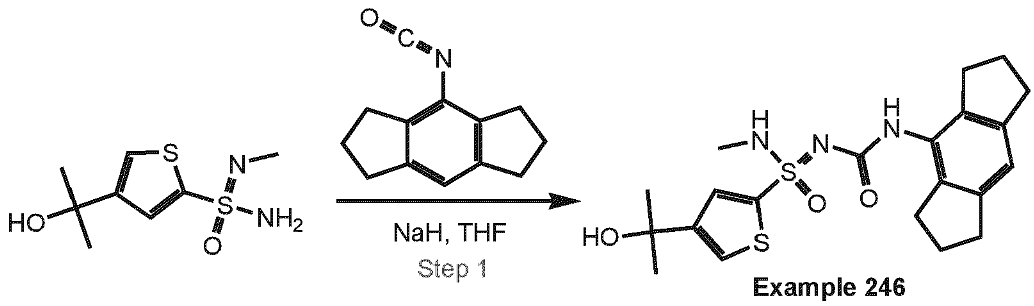


**Example 246**

Into a 50-mL 3-necked round-bottom flask purged and maintained with an inert atmosphere of nitrogen, was placed 4-(2-hydroxypropan-2-yl)-N-methylthiophene-2-sulfonoimidamide (106 mg, 0.45 mmol) in THF (4.0 mL). This was followed by the addition of NaH (60%wt. oil dispersion, 23.5 mg, 0.59 mmol) in several batches at 0 °C in a water/ice bath. To this was added a solution of 4-isocyanato-1,2,3,5,6,7-hexahydro-s-indacene (99.1 mg, 0.50 mmol) in THF (2.0 mL) dropwise with stirring at 0 °C. The resulting solution was stirred for 30 min at 0 °C in a water/ice bath. The reaction was then quenched by the addition of water/ice. The resulting solution was extracted with ethyl acetate and the organic layers combined, the organic layer was dried over anhydrous sodium sulfate and concentrated under vacuum. The crude product was purified by Prep-HPLC with the following conditions: Column, X Bridge Shield RP18 OBD, 19x250 mm, 10 um; mobile phase, water (10 mM $NH_4HCO_3$+0.1%$NH_3.H_2O$) and ACN (43% to 67% ACN gradient in 6 min); Detector, UV 254 nm. This resulted in 80 mg (40.79%) of **Example 246** as a white solid. MS-ESI: 434.15 (M+1). $^1$H NMR (DMSO-$d_6$, 300 MHz): δ 8.55 (br s, 1H) 7.65 (s, 1H), 7.59 (s, 1H), 7.53 (s, 1H), 6.89 (s, 1H), 5.22 (s, 1H) 2.63-2.85 (m, 8H) 2.49 (s, 3H) 2.00-1.80 (m, 4H) 1.31 (s, 6H).

**Table 24.** Examples in the following table were prepared using similar conditions as described in Example **246** and Scheme **4** from appropriate starting materials.

| Example # | Final Target Number | Structure | IUPAC Name | Exact Mass $[M+H]^+$ |
|---|---|---|---|---|
| 247 | 339 | 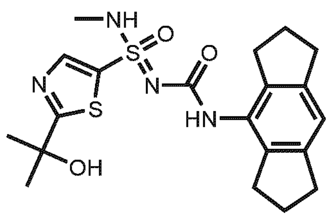 | N'-((1,2,3,5,6,7-hexahydro-*s*-indacen-4-yl)carbamoyl)-2-(2-hydroxypropan-2-yl)-N-methylthiazole-5-sulfonimidamide | 435 |

**Example 248 (Compound 405)**

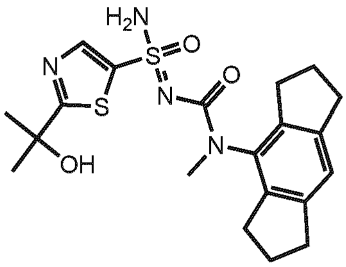

*N'*-((1,2,3,5,6,7-hexahydro-*s*-indacen-4-yl)(methyl)carbamoyl)-2-(2-hydroxypropan-2-yl)thiazole-5-sulfonimidamide (Scheme 34)

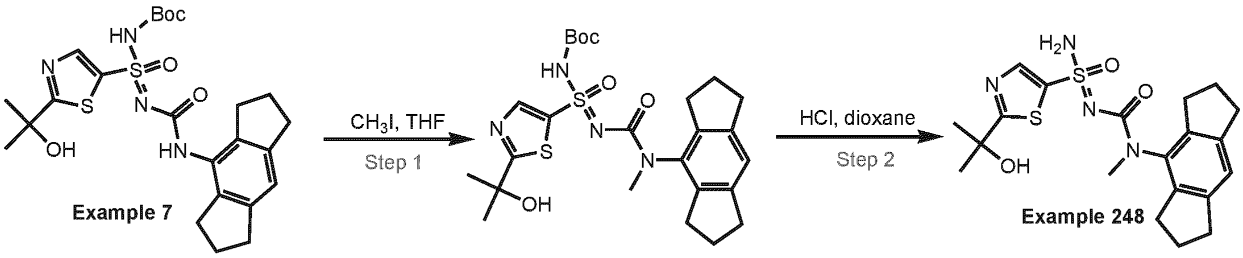


**Step 1: *Tert*-butyl(N-((1,2,3,5,6,7-hexahydro-*s*-indacen -4-yl)(methyl)carbamoyl)-2- (2-hydroxypropan -2-yl)thiazole-5-sulfonimidoyl)carbamate**

Into a 50-mL round-bottom flask, was placed *tert*-butyl N-([[(1,2,3,5,6,7-hexahydro-*s*-indacen-4-yl) carbamoyl]imino][2-(2-hydroxypropan-2-yl)-1,3-thiazol-5-yl]oxo-$\lambda^6$-sulfanyl)carbamate (200 mg, 0.38 mmol) in THF (10 mL), to this stirred solution was added $CH_3I$ (60 mg, 0.42 mmol) dropwise at 0 °C. The resulting solution was stirred for 1 d at RT. The resulting mixture was concentrated. This resulted in 100 mg (49%) of the title compound as a solid. MS-ESI: 535 (M+1).

**Step 2: *N'*-((1,2,3,5,6,7-hexahydro-*s*-indacen-4-yl)(methyl)carbamoyl)-2-(2-hydroxypropan-2-yl) thiazole-5-sulfonimidamide**

Into a 25-mL round-bottom flask, was placed tert-butyl N-([[(1,2,3,5,6,7-hexahydro-*s*-indacen-4-yl) (methyl)carbamoyl]imino][2-(2-hydroxypropan-2-yl)-1,3-thiazol-5-yl]oxo-$\lambda^6$-sulfanyl)carbamate (100 mg) in HCl (4M, 10 mL). The resulting solution was stirred for 5 h at RT. The crude product was purified by Prep-HPLC with the following conditions: Column, XBridge Prep C18 OBD, 5um, 19*150 mm; mobile phase, water (10 mM $NH_4HCO_3$mM) and ACN (22% to 53% ACN gradient in 7 min); Detector, UV. This resulted in 15.7 mg of **Example 248** as a solid. MS-ESI: 435 (M+1).

**Table 25.** Example 249 was isolated as a side product from the preparation of Example 248.

| Example # | Final Target Number | Structure | IUPAC Name | Exact Mass $[M+H]^+$ |
|---|---|---|---|---|
| 249 | 406 | 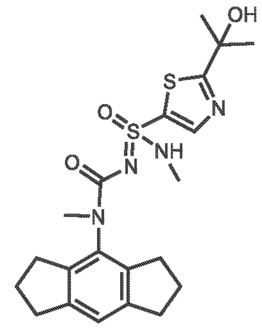 | N'-((1,2,3,5,6,7-hexahydro-s-indacen-4-yl)(methyl)carbamoyl)-2-(2-hydroxypropan-2-yl)-N-methylthiazole-5-sulfonimidamide | 449 |

**Example 250 (Compound 324)**

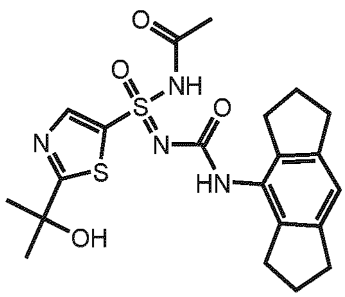

<u>*N*-(*N*-((1,2,3,5,6,7-hexahydro-*s*-indacen-4-yl)carbamoyl)-2-(2-hydroxypropan-2-yl)thiazole-5-sulfonimidoyl)acetamide (Scheme 35A)</u>

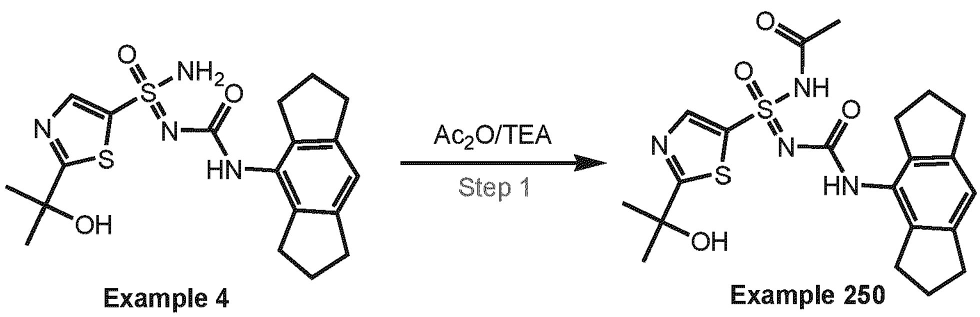


Into a 50-mL round-bottom flask purged and maintained with an inert atmosphere of nitrogen, was placed a mixture of N'-((1,2,3,5,6,7-hexahydro-s-indacen-4-yl)carbamoyl)-2-(2-hydroxypropan-2-yl)thiazole-5- sulfonimidamide (200 mg, 0.48 mmol) and TEA (96 mg, 0.96 mmol) in DCM (20 mL). To the stirred solution, $Ac_2O$ (74 mg, 0.72 mmol) was added dropwise at 0°C. The resulting solution was stirred overnight. Then 80 mg of the product was obtained by Prep-HPLC with the following conditions: Column: XBridge Prep C18 OBD Column 19×150 mm 5um; Mobile Phase A: water (10 mM $NH_4HCO_3$), Mobile Phase B: ACN; Flow rate: 25 mL/min; Gradient: 18% B to 41% B in 7 min; 254/210 nm; Rt: 5.05 min, this resulted in 100 mg of the **Example 250** as a white solid. MS-ESI: 462.14 (M+1). $^1H$ NMR (300 MHz, $CD_3OD$-*$d_4$*) δ: 8.11 (s, 1H), 6.89 (s, 1H), 2.92 - 2.69 (m, 8H), 2.09 - 2.01 (m, 4H), 1.99 (s, 3H), 1.60 (d, *J* = 2.3 Hz, 6H).

**Example 251 (Compound 407)**

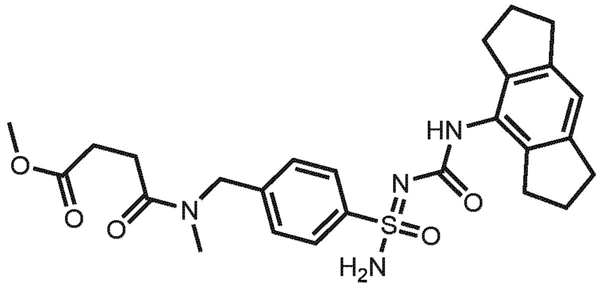

methyl 4-((4-(*N'*-((1,2,3,5,6,7-hexahydro-*s*-indacen-4-yl)carbamoyl)sulfamidimidoyl)benzyl)(methyl)amino)-4-oxobutanoate (Scheme 35)

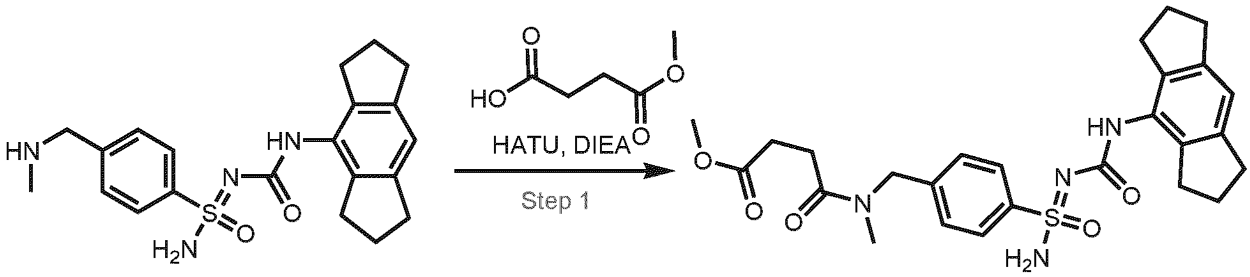


**Example 251**

Into a 8-mL round-bottom flask, was placed a solution of 1-[amino([4-[(methylamino)methyl]-phenyl])oxo-λ$^6$-sulfanylidene]-3-(1,2,3,5,6,7-hexahydro-s-indacen-4-yl)urea (100 mg, 0.25 mmol), methyl 4-chloro-4-oxobutanoate (37.8 mg, 0.25 mmol) in DMF (10 mL), to this stirred solution was added HATU (191 mg, 0.50 mmol) and DIEA (64.9 mg, 0.50 mmol). The resulting solution was stirred for 20 min at RT. The crude product was purified by Prep-HPLC with the following conditions: Column, XBridge Shield RP18 OBD, 19*250 mm,10 um; mobile phase, water (10 mM $NH_4HCO_3$) and ACN (15% to 75% ACN gradient in 7 min); Detector, UV 250 nm. This resulted in 4.2 mg (3.27%) of **Example 251** as a white solid. MS-ESI: 513 (M+1). $^1$H NMR (300MHz, $CD_3OD$-$d_4$) δ: 8.02-7.94 (m, 2H), 7.49-7.41 (m, 2H), 6.89 (s, 1H), 4.68 (s, 2H), 3.68 (s, 3H), 3.04 (s, 3H), 2.85-2.80 (m, 4H), 2.75-2.60 (m, 8H), 2.03-1.97 (m, 4H).

**Example 252 (Compound 410)**

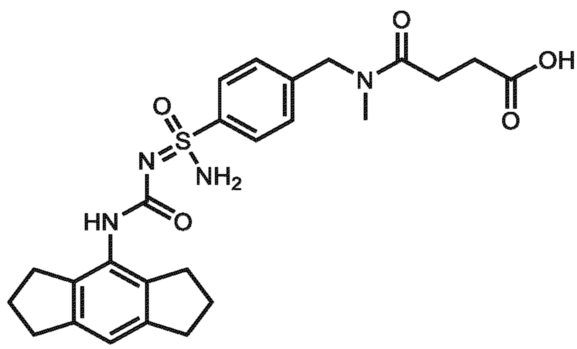

4-((4-(N'-(1,2,3,5,6,7-hexahydro-*s*-indacen-4-ylcarbamoyl)sulfamimidoyl)benzyl)(methyl)amino)-4-oxobutanoic acid

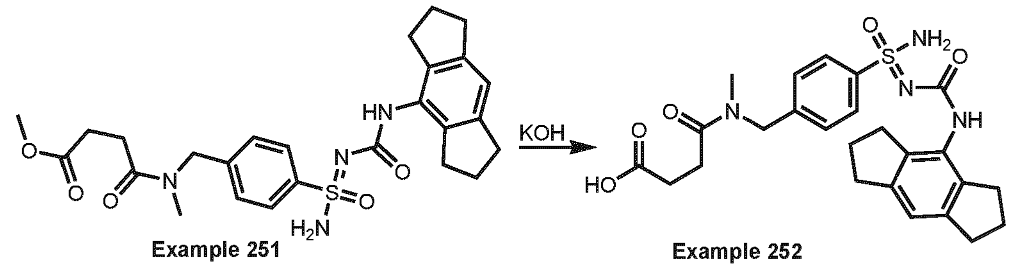


Into a 50-mL round-bottom flask, was placed a solution of methyl 3-[([4-[amino([[(1,2,3,5,6,7-hexahydro-*s*-indacen-4-yl)carbamoyl]imino])oxo-$\lambda^6$-sulfanyl]phenyl]methyl)(methyl)-carbamoyl]propanoate (80 mg, 0.16 mmol) in THF (3.0 mL) and $H_2O$ (3.0 mL), to the stirred solution was added KOH (17.5 mg, 0.31 mmol). The resulting solution was stirred for 120 min at RT. The crude product was purified by Prep-HPLC with the following conditions: Column, XBridge Shield RP18 OBD, 19*250 mm,10 um; mobile phase, water (10 mM $NH_4HCO_3$) and ACN (15% to 75% gradient in 7 min); Detector, UV250 nm. This resulted in 39 mg (50%) of Example 252 as a white solid. MS-ESI: 499 (M+1). $^1$H-NMR (300 MHz, $CD_3OD$-$d_4$) δ: 8.10-7.80 (m, 2H), 7.55-7.30 (m, 2H), 6.89 (s, 1H), 4.68 (s, 2H), 3.04 (s, 3H), 2.90-2.60 (m, 12H), 2.10-1.80 (m, 4H).

**Example 253 (Compound 408)**

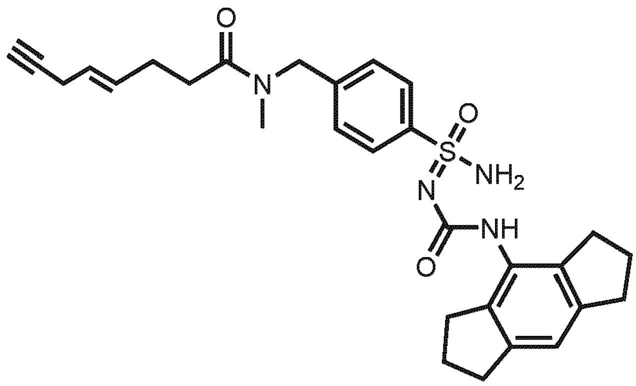

(E)-N-(4-(N'-((1,2,3,5,6,7-hexahydro-s-indacen-4-yl)carbamoyl)sulfamidimidoyl)benzyl)-N-methyloct-4-en-7-ynamide (Scheme 35)

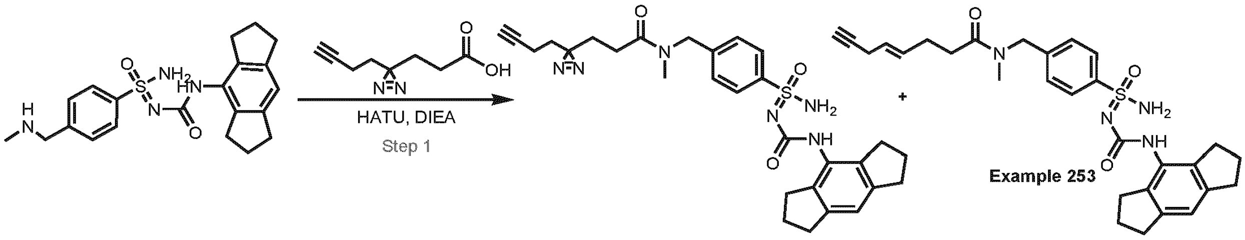


**Example 253** was prepared using similar conditions as described in Example **251** and Scheme **35** from 3-(3-(but-3-ynyl)-3*H*-diazirin-3-yl)propanoic acid and **Intermediate 67.** MS-ESI: 519 (M+1)

**Table 26.** Examples in the following table were prepared using similar conditions as described in Example **4 - route 1** and Scheme **2** from appropriate starting materials.

| Example # | Final Target Number | Structure | IUPAC Name | Exact Mass [M+H]+ |
|---|---|---|---|---|
| 254 | 308 | 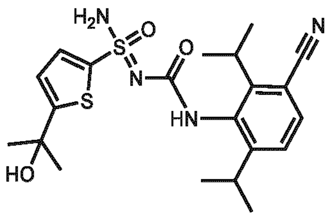 | N'-((3-cyano-2,6-diisopropylphenyl) carbamoyl)-5-(2-hydroxypropan-2-yl) thiophene-2-sulfonimidamide | 449 |
| 255 | 311 | 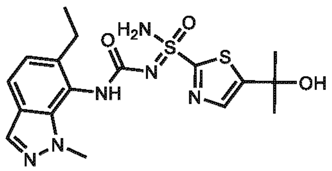 | N'-((6-ethyl-1-methyl-1H-indazol-7-yl) carbamoyl)-5-(2-hydroxypropan-2-yl) thiazole-2-sulfonimidamide | 423 |
| 256 | 312 | 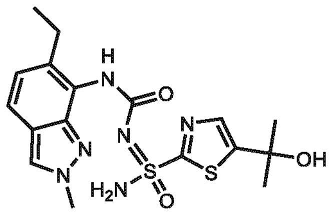 | N'-((6-ethyl-2-methyl-2H-indazol-7-yl) carbamoyl)-5-(2-hydroxypropan-2-yl) thiazole-2-sulfonimidamide | 423 |
| 257 | 327 | 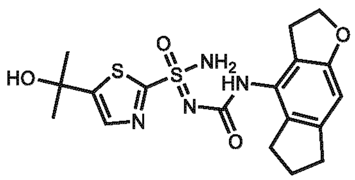 | 5-(2-hydroxypropan-2-yl)-N'-((3,5,6,7-tetrahydro-2H-indeno[5,6-b]furan-4-yl)carbamoyl)thiazole-2-sulfonimidamide | 423 |
| 258 | 326 | 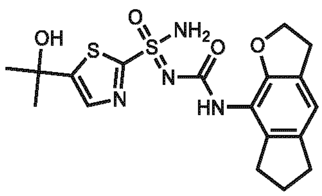 | 5-(2-Hydroxypropan-2-yl)-N'-((3,5,6,7-tetrahydro-2H-indeno [5,6-b]furan-8-yl)carbamoyl)thiazole-2-sulfonimidamide | 423 |
| 259 | 139 | 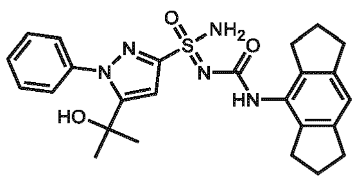 | N'-((1,2,3,5,6,7-hexahydro-s-indacen-4-yl)carbamoyl)-5-(2-hydroxypropan-2-yl)-1-phenyl-1H-pyrazole-3-sulfonimidamide | 480 |
| 260 | 137 | 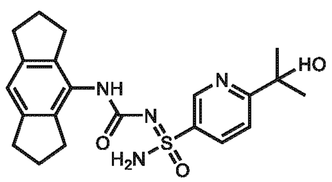 | N'-((1,2,3,5,6,7-hexahydro-s-indacen-4-yl)carbamoyl)-6-(2-hydroxypropan-2-yl)pyridine-3-sulfonimidamide | 415 |

(continued)

| Example # | Final Target Number | Structure | IUPAC Name | Exact Mass [M+H]+ |
|---|---|---|---|---|
| 261 | 409 | 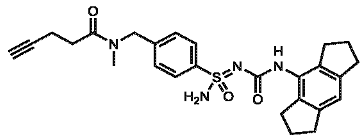 | N-(4-(N'-((1,2,3,5,6,7-hexahydro-s-indacen-4-yl)carbamoyl) sulfamidimidoyl)benzyl)-N-methylpent-4-ynamide | 479 |
| 262 | 303 | 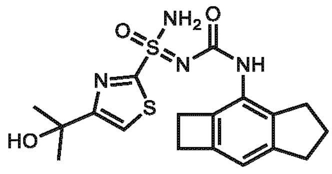 | 4-(2-Hydroxypropan-2-yl)-N'-((2,4,5,6-tetrahydro- 1H-cyclobuta[f]inden-3 - yl)carbamoyl) thiazole-2-sulfonimidamide | 407 |
| 263 | 325 | 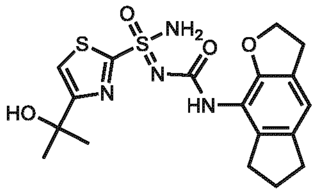 | 4-(2-Hydroxypropan-2-yl)-N'-((3,5,6,7-tetrahydro-2H-indeno [5,6-b]furan-8-yl)carbamoyl)thiazole-2-sulfonimidamide | 423 |
| 264 | 138 | 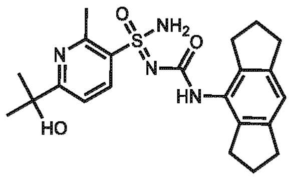 | N'-((1,2,3,5,6,7-hexahydro-*s*-indacen-4-yl)carbamoyl)-6-(2-hydroxypropan-2-yl)-2-methylpyridine-3-sulfonimidamide | 429 |
| 265 | 332 | 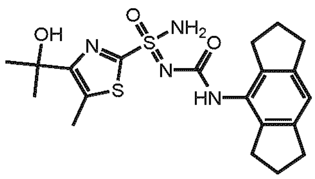 | N'-(1,2,3,5,6,7-hexahydro-*s*-indacen-4-ylcarbamoyl)-4-(2-hydroxypropan-2-yl)-5-methylt hiazole-2-sulfonimidamide | 435 |
| 266 | 334 | 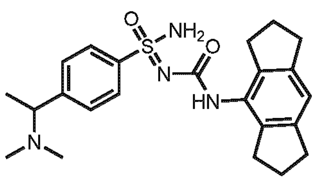 | 4-(1-(Dimethylamino) ethyl)-N'-(1,2,3,5,6,7-hexahydro-*s*-indacen-4-ylcarbamoyl) benzenesulfonimidamide | 427 |
| 267 | 335 | 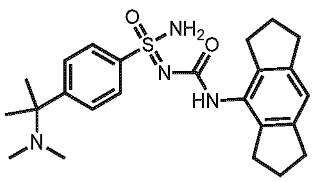 | 4-(2-(Dimethylamino)propan-2-yl)-N'-(1,2,3,5,6,7-hexahydro-*s*-indacen-4-ylcarbamoyl) benzenesulfonimidamide | 441 |
| 268 | 337 | 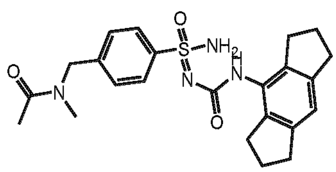 | N-(4-(N'-(1,2,3,5,6,7-hexahydro-*s*-indacen-4-ylcarbamoyl) sulfamimidoyl)benzyl)-N-methylacetamide | 441 |

(continued)

| Example # | Final Target Number | Structure | IUPAC Name | Exact Mass [M+H]+ |
|---|---|---|---|---|
| 269 | 113 | 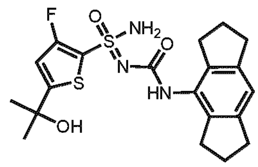 | 3-Fluoro-N'-(1,2,3,5,6,7-hexahydro-*s*-indacen-4-ylcarbamoyl)-5-(2-hydroxypropan-2-yl)thiophene-2-sulfonimidamide | 438 |
| 270 | 343 | 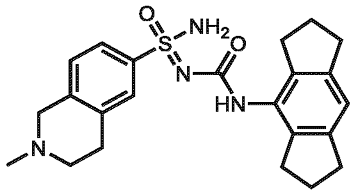 | N'-((1,2,3,5,6,7-hexahydro-*s*-indacen-4-yl)carbamoyl)-2-methyl-1,2,3,4-tetrahydroisoquinoline-6-sulfonimidamide | 425 |
| 271 | 349 | 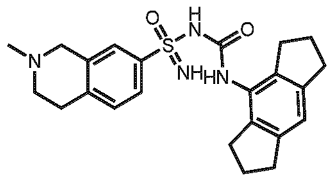 | N-(1,2,3,5,6,7-hexahydro-*s*-indacen-4-ylcarbamoyl)-2-methyl-1,2,3,4-tetrahydroisoquinoline-7-sulfonimidamide | 425 |
| 272 | 344 | 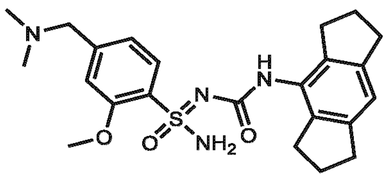 | 4-((Dimethylamino) methyl)-N'-((1,2,3,5,6,7-hexahydro-*s*-indacen-4-yl)carbamoyl)-2-methoxybenzenesulfonimidamide | 443 |
| 273 | 359 | 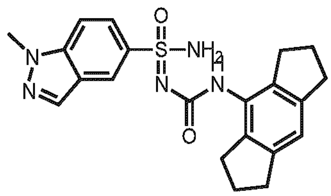 | N'-(1,2,3,5,6,7-hexahydro-*s*-indacen-4-ylcarbamoyl)-1-methyl-1H-indazole-5-sulfonimidamide | 410 |
| 274 | 352 | 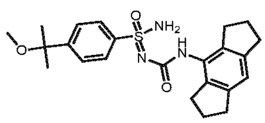 | N'-(1,2,3,5,6,7-hexahydro-*s*-indacen-4-ylcarbamoyl)-4-(2-methoxypropan-2-yl)benzenesulfonimidamide | 428 |
| 275 | 354 | 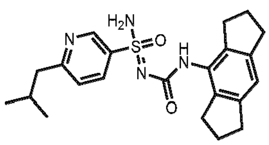 | N'-(1,2,3,5,6,7-hexahydro-*s*-indacen-4-ylcarbamoyl)-6-isobutylpyridine-3-sulfonimidamide | 413 |
| 276 | 355 | 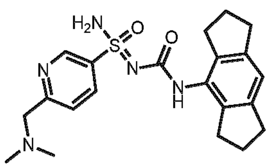 | 6-((Dimethylamino) methyl)-N'-(1,2,3,5,6,7-hexahydro-*s*-indacen-4-ylcarbamoyl)pyridine-3-sulfonimidamide | 414 |

(continued)

| Example # | Final Target Number | Structure | IUPAC Name | Exact Mass [M+H]+ |
|---|---|---|---|---|
| 277 | 356 | 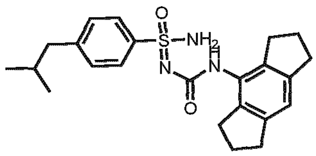 | N'-(1,2,3,5,6,7-hexahydro-*s*-indacen-4-ylcarbamoyl)-4-isobutylbenzenesulfonimidamide | 412 |
| 278 | 357 | 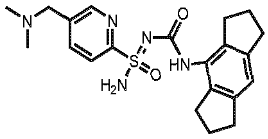 | 5-((Dimethylamino) methyl)-N'-(1,2,3,5,6,7-hexahydro-*s*-indacen-4-ylcarbamoyl)pyridine-2-sulfonimidamide | 414 |
| 279 | 340 | 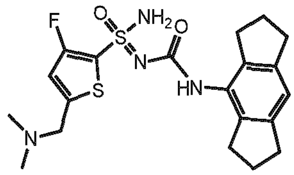 | 5-((Dimethylamino)methyl)-3-fluoro-N'-(1,2,3,5,6,7-hexahydro-*s*-indacen-4-ylcarbamoyl)thiophene-2-sulfonimidamide | 437 |
| 280 | 377 | 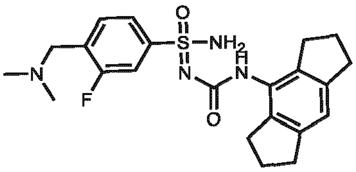 | 4-((dimethylamino)methyl)-3-fluoro-N'-((1,2,3,5,6,7-hexahydro-*s*-indacen-4-yl)carbamoyl) benzenesulfonimidamide | 431 |
| 281 | 378 | 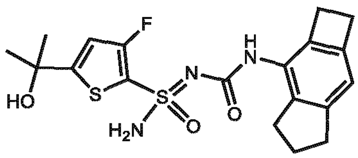 | 3-fluoro-5-(2-hydroxypropan-2-yl)-N'-((2,4,5,6-tetrahydro-1H-cyclobuta[f]inden-3-yl)carbamoyl) thiophene-2-sulfonimidamide | 424 |
| 282 | 379 | 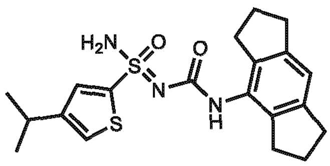 | N'-((1,2,3,5,6,7-hexahydro-*s*-indacen-4-yl)carbamoyl)-4-isopropylthiophene-2-sulfonimidamide | 404 |
| 283 | 380 | 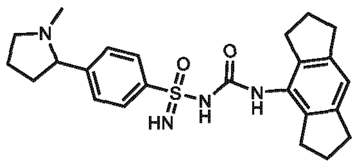 | N-((1,2,3,5,6,7-hexahydro-*s*-indacen-4-yl)carbamoyl)-4-(1-methylpyrrolidin-2-yl)benzenesulfonimidamide | 439 |
| 284 | 353 | 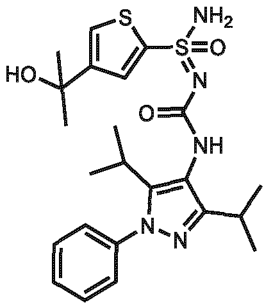 | N'-((3,5-diisopropyl-1-phenyl-1H-pyrazol-4-yl)carbamoyl)-4-(2-hydroxypropan-2-yl)thiophene-2-sulfonimidamide | 490 |

(continued)

| Example # | Final Target Number | Structure | IUPAC Name | Exact Mass [M+H]+ |
|---|---|---|---|---|
| 285 | 333 | 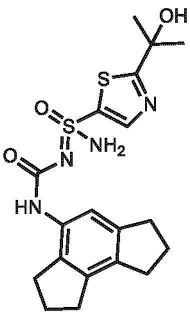 | N'-((1,2,3,6,7,8-hexahydro-as-indacen-4-yl)carbamoyl)-2-(2-hydroxypropan-2-yl)thiazole-5-sulfonimidamide | 421 |
| 287 | 382 | 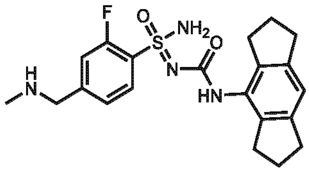 | 2-fluoro-N'-((1,2,3,5,6,7-hexahydro-s-indacen-4-yl)carbamoyl)-4-((methylamino)methyl) benzenesulfonimi damide | 417 |
| 288 | 383 | 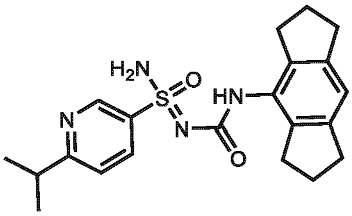 | N'-((1,2,3,5,6,7-hexahydro-s-indacen-4-yl)carbamoyl)-6-isopropylpyridine-3-sulfonimidamide | 399 |

**Table 27.** Examples in the following table were prepared using similar conditions as described in Example **4** - route **2** and Scheme **3** from appropriate starting materials.

| Example # | Final Target Number | Structure | IUPAC Name | Exact Mass [M+H]+ |
|---|---|---|---|---|
| 289 | 315 | 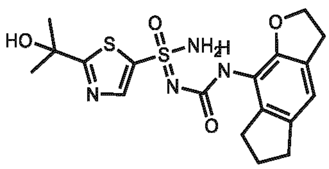 | 2-(2-Hydroxypropan-2-yl)-N'-((3,5,6,7-tetrahydro-2H-indeno[5,6-b] furan-8-yl)carbamoyl)thiazole-5-sulfonimidamide | 423 |
| 290 | 316 | 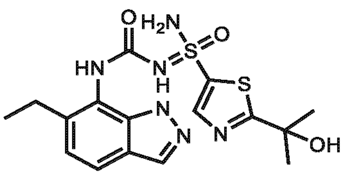 | N'-((6-ethyl-1H-indazol-7-yl) carbamoyl)-2-(2-hydroxypropan-2-yl)thiazole-5-sulfonimidamide | 409 |
| 291 | 317 | 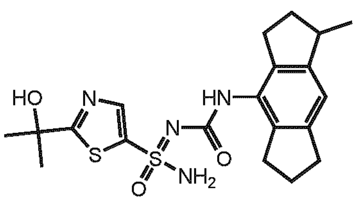 | 2-(2-Hydroxypropan-2-yl)-N'-((1-methyl-1,2,3,5,6,7-hexahydro-*s*-indacen-4-yl)carbamoyl)thiazole-5-sulfonimidamide | 435 |

(continued)

| Example # | Final Target Number | Structure | IUPAC Name | Exact Mass [M+H]+ |
|---|---|---|---|---|
| 292 | 319 | 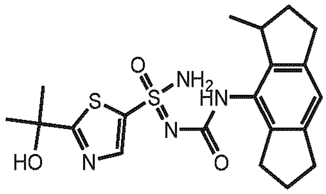 | 2-(2-Hydroxypropan-2-yl)-N'-((3-methyl-1,2,3,5,6,7-hexahydro-*s*-indacen-4-yl)carbamoyl)thiazole-5-sulfonimidamide | 435 |
| 293 | 320 | 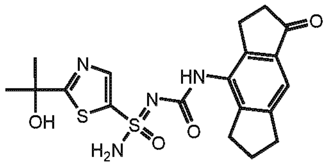 | 2-(2-Hydroxypropan-2-yl)-N'-((1-oxo-1,2,3,5,6,7-hexahydro-*s*-indacen-4-yl)carbamoyl)thiazole-5-sulfonimidamide | 435 |
| 294 | 336 | 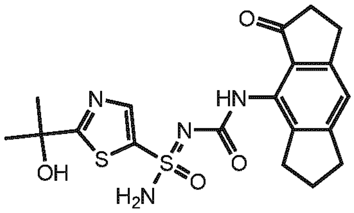 | 2-(2-Hydroxypropan-2-yl)-N'-((3-oxo-1,2,3,5,6,7-hexahydro-*s*-indacen-4-yl)carbamoyl)thiazole-5-sulfonimidamide | 435 |
| 295 | 330 | 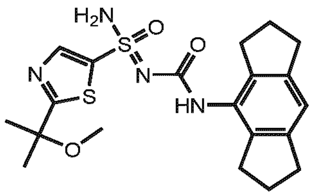 | N'-((1,2,3,5,6,7-hexahydro-*s*-indacen-4-yl)carbamoyl)-2-(2-methoxypropan-2-yl)thiazole-5-sulfonimidamide | 435 |

**Table 28.** Examples in the following table were obtained from chiral HPLC resolutions of racemic examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. For mixtures contained two chiral centers and if two columns are used for separating the four diastereomers, the individual isomers are listed in the order of faster column 1/faster column 2; faster column 1/slower column 2; slower column 1/faster column 2; followed by slower column 1/slower column 2.

| Ex. # | Final Target Number | Structure | IUPAC Name | Column | Eluents | LC-MS $[M+H]^+$ |
|---|---|---|---|---|---|---|
| 296 | 364a | 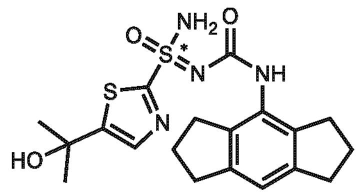 | (R) or (S) -N'-((1,2,3,5,6,7-hexahydro-*s*-indacen-4-yl)carbamoyl)-5-(2-hydroxypropan-2-yl)thiazole-2-sulfonimidamide | CHIRALP AK IG 2 *25 cm (5 um) | 50% MeOH (8 mM $NH_3$-MeOH) in $CO_2$# | 421 |
| 297 | 364b | 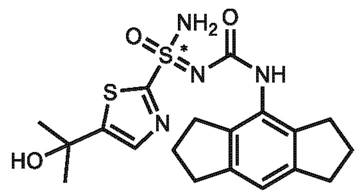 | (S) or (R)-N'-((1,2,3,5,6,7-hexahydro-*s*-indacen-4-yl)carbamoyl)-5-(2-hydroxypropan-2-yl)thiazole-2-sulfonimidamide | CHIRALP AK IG 2 *25 cm (5 um) | 50% MeOH (8 mM $NH_3$.MeO H) in $CO_2$ | 421 |
| 298 | 365a | 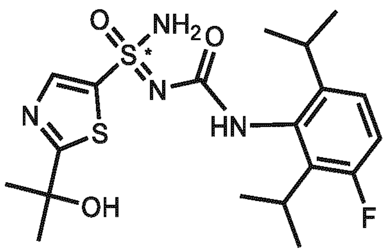 | (R) or (S) - N'-((3-fluoro-2,6-diisopropylphenyl)carbamoyl) -2-(2-hydroxypropan-2-yl)thiazole-5-sulfonimidamide | Chiralpak ID, 2*25 cm, 5 um | IPA in Hex:DCM =5:1 | 443 |

(continued)

| Ex. # | Final Target Number | Structure | IUPAC Name | Column | Eluents | LC-MS $[M+H]^+$ |
|---|---|---|---|---|---|---|
| 299 | 365b | 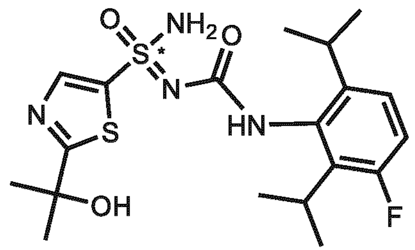 | (S) or (R) - N'-((3-fluoro-2,6-diisopropylphenyl)carbamoyl) -2-(2-hydroxypropan-2-yl)thiazole-5-sulfonimidamide | Chiralpak ID, 2*25 cm, 5 um | IPA in Hex:DCM =5:1 | 443 |
| 300 | 308a | 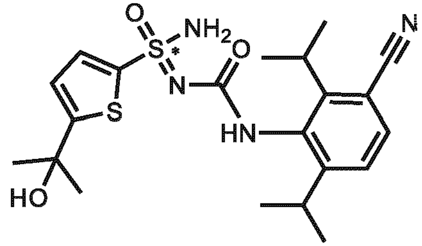 | (R) or (S) - N'-((3-cyano-2,6-diisopropylphenyl)carbamoyl) -5-(2-hydroxypropan-2- yl)thiophene-2-sulfonimidamide | CHIRALP AK IG, 20*250 mm, 5 um | 30% EtOH in Hex (0.1% DEA) | 449 |
| 301 | 308b | 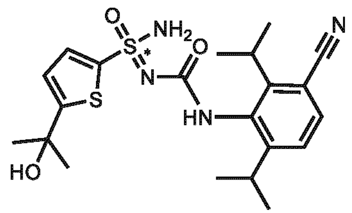 | (S) or (R) -N'-((3-fluoro-2,6-diisopropylphenyl)carbamoyl) -2-(2-hydroxypropan-2-yl)thiazole-5-sulfonimidamide | CHIRALP AKIG, 20*250 mm, 5 um | 30% EtOH in Hex (0.1% DEA) | 449 |

(continued)

| Ex. # | Final Target Number | Structure | IUPAC Name | Column | Eluents | LC-MS $[M+H]^+$ |
|---|---|---|---|---|---|---|
| 126 | 195a | 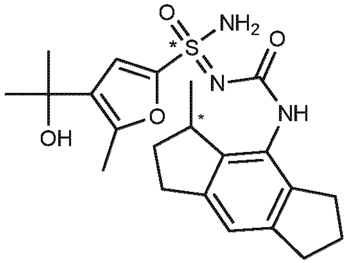 | Two isomers of (S, S)-and (S, R)-or (R, S)-and (R, R) 4-(2-hydroxypropan-2-yl)-5-methyl-N'-((3-methyl-1,2,3,5,6,7-hexahydro-s-indacen-4-yl)carbamoyl) furan-2-sulfonimidamide | CHIRALA RT Cellulose-SB, 2*25 cm, 5 um | MeOH (0.1% DEA); 1st and 2nd peaks | 432 |
| 127 | 195e | 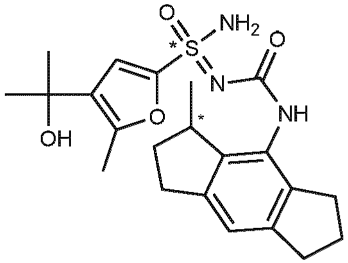 | Two isomers of (R, S)-and (R, R)-or (S, S)-and (S, R) 4-(2-hydroxypropan-2-yl)-5-methyl-N'-((3-methyl-1,2,3,5,6,7-hexahydro-s-indacen-4-yl)carbamoyl) furan-2-sulfonimidamide | | MeOH (0.1%DEA ); 3rd peak | 432 |
| 302 | 195ba | 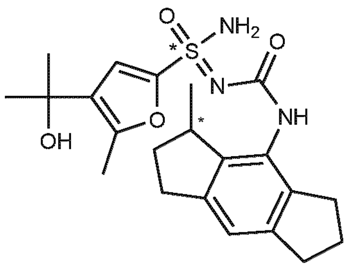 | (R, R) or (R, S) or (S, S) or (S, R)- 4-(2-hydroxypropan-2-yl)-5-methyl-N'-((3-methyl-1,2,3,5,6,7-hexahydro-s-indacen-4-yl)carbamoyl)furan-2-sulfonimidamide resolved from example 127 | Phenomene x Lux 5u Cellulose-4 , AXIA Packed 2.12*25 cm, 5 um | 40%MeOH in $CO_2$ | 432 |

(continued)

| Ex. # | Final Target Number | Structure | IUPAC Name | Column | Eluents | LC-MS $[M+H]^+$ |
|---|---|---|---|---|---|---|
| 303 | 195bb | 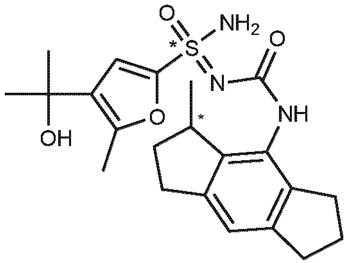 | (R, S) or (R, R) or (S, R) or (S, S)- 4-(2-hydroxypropan-2-yl)-5-methyl-N'-((3-methyl-1,2,3,5,6,7-hexahydro-s-indacen-4-yl)carbamoyl)furan-2-sulfonimidamide resolved from example 127 | Phenomene x Lux 5u Cellulose-4 , AXIA Packed 2.12*25 cm, 5 um | 40%MeOH in $CO_2$ | 432 |
| 123 | 207c | 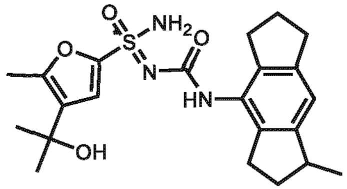 | Two isomers of (R, S)-and (R, R) 4-(2-hydroxypropan-2-yl)-5-methyl-N'-(1-methyl-1,2,3,5,6,7-hexahydros-indacen-4-ylcarbamoyl)furan-2-sulfonimidamide | ChiralPak IC, 2*25cm, 5um | 50% EtOH in MTBE; 1st and 2nd peaks | 432.2 |
| 124 | 207aa | 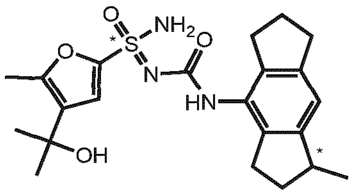 | (S, S)-or (S, R)-4-(2-hydroxypropan-2-yl)-5-methyl-N'-(1-methyl-1,2,3,5,6,7-hexahydros-indacen-4-ylcarbamoyl) furan-2-sulfonimidamide | | 50% EtOH in MTBE; 3rd peak | 432.2 |
| 125 | 207b | 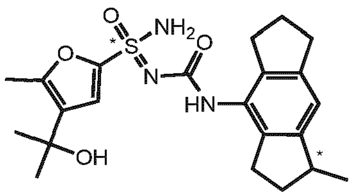 | (S, R)-or (S, S)-4-(2-hydroxypropan-2-yl)-5-methyl-N'-(1-methyl-1,2,3,5,6,7-hexahydros-indacen-4-ylcarbamoyl) furan-2-sulfonimidamide | | 50% EtOH in MTBE; 4th peak | 432.2 |

(continued)

| Ex. # | Final Target Number | Structure | IUPAC Name | Column | Eluents | LC-MS $[M+H]^+$ |
|---|---|---|---|---|---|---|
| 304 | 207a | 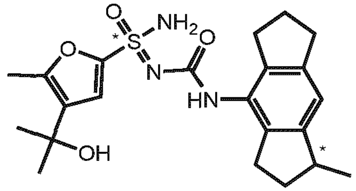 | (R, R) or (R, S) -4-(2-hydroxypropan-2-yl)-5- methyl-N'-((3-methyl-1,2,3,5,6,7-hexahydro-*s*-indacen-4-yl)carbamoyl) furan-2-sulfonimidamide; resolved from example 123 | CHIRALP AK IG, 20*250 mm, 5 um | EtOH in Hex (0.1% FA) | 432 |
| 305 | 207bb | 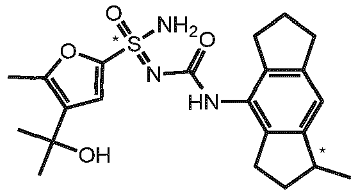 | (R, S) or (R, R)-4-(2-hydroxypropan-2-yl)-5- methyl-N'-((3-methyl-1,2,3,5,6,7-hexahydro-*s*-indacen-4-yl)carbamoyl) furan-2-sulfonimidamide; resolved from example 123 | CHIRALP AK IG, 20*250 mm, 5 um | EtOH in Hex (0.1% FA) | 432 |
| 306 | 366a | 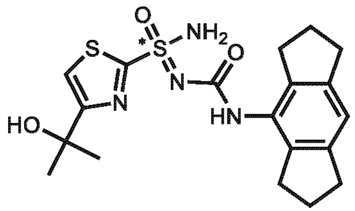 | (R) or (S) -N'-((1,2,3,5,6,7-hexahydro-*s*-indacen-4-yl)carbamoyl)-4-(2-hydroxypropan-2-yl)thiazole-2-sulfonimidamide | CHIRALP AK AS-H, 2*25 cm (5 um) | 35% IPA (2 mM $NH_3$-MeOH) in $CO_2$ | 421 |
| 307 | 366b | 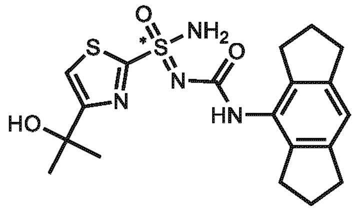 | (S) or (R) -N'-((1,2,3,5,6,7-hexahydro-*s*-indacen-4-yl)carbamoyl)-4-(2-hydroxypropan-2-yl)thiazole-2-sulfonimidamide | CHIRALP AK AS-H, 2*25 cm (5 um) | 35% IPA (2 mM $NH_3$-MeOH) in $CO_2$ | 421 |

(continued)

| Ex. # | Final Target Number | Structure | IUPAC Name | Column | Eluents | LC-MS $[M+H]^+$ |
|---|---|---|---|---|---|---|
| 308 | 139a | 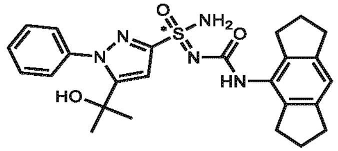 | (S) or (R)-N'-((1,2,3,5,6,7-hexahydro-*s*-indacen-4-yl)carbamoyl)-5-(2-hydroxypropan-2-yl)-1-phenyl- 1H-pyrazole-3 - sulfonimidamide | CHIRALP AK AS-H, 2*25 cm (5 um) | EtOH in Hex (0.1% DEA) | 480 |
| 309 | 139b | 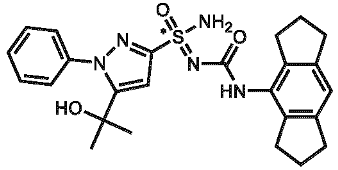 | (R) or (S)-N'-((1,2,3,5,6,7-hexahydro-*s*-indacen-4-yl)carbamoyl)-5-(2-hydroxypropan-2-yl)-1-phenyl- 1H-pyrazole-3 - sulfonimidamide | CHIRALP AK AS-H, 2*25 cm (5 um) | EtOH in Hex (0.1% DEA) | 480 |
| 310 | 367a | 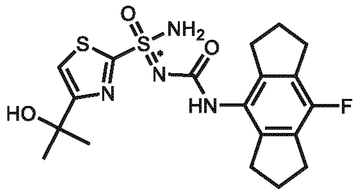 | (R) or (S) -N'-((8-fluoro-1,2,3,5,6,7-hexahydro-*s*-indacen-4-yl)carbamoyl)-4-(2-hydroxypropan-2-yl)thiazole-2-sulfonimidamide | Chiralpak AS-H 2*25 cm (5 um) | 35% IPA in $CO_2$ | 439 |
| 311 | 367b | 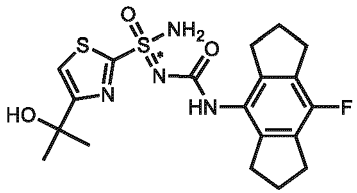 | (S) or (R) -N'-((8-fluoro-1,2,3,5,6,7-hexahydro-*s*-indacen-4-yl)carbamoyl)-4-(2-hydroxypropan-2-yl)thiazole-2-sulfonimidamide | Chiralpak ASH2*25cm ( 5um) | 35% IPA in $CO_2$ | 439 |
| 312 | 409b | 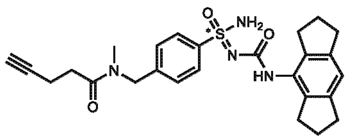 | (S) or (R)-N-(4-(N'-((1,2,3,5,6,7-hexahydro-*s*-indacen-4-yl)carbamoyl) sulfamidimidoy l)benzyl)-N-methylpent-4-ynamide | CHIRAL ART Cellulose-SB, 2*25 cm, 5 um | EtOH in Hex (8 mM $NH_3$-MeOH) | 479 |

(continued)

| Ex. # | Final Target Number | Structure | IUPAC Name | Column | Eluents | LC-MS $[M+H]^+$ |
|---|---|---|---|---|---|---|
| 313 | 409a | 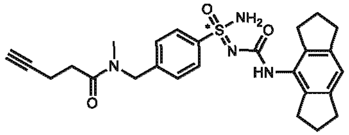 | (R) or (S)-N-(4-(N'-((1,2,3,5,6,7-hexahydro-*s*-indacen-4-yl)carbamoyl) sulfamidimidoy l)benzyl)-N-methylpent-4-ynamide | CHIRAL ART Cellulose-SB, 2*25 cm, 5 um | EtOH in Hex (8 mM $NH_3$.MeO H) | 479 |
| 314 | 369a | 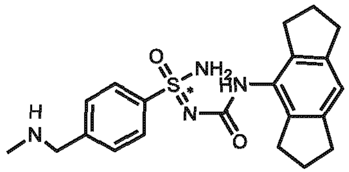 | (S) or (R)-N'-((1,2,3,5,6,7-hexahydro-*s*-indacen-4-yl)carbamoyl)-4-((methylamino)methyl)benze nesulfonimidamide | Chiralpak ID-2, 2*25cm, Sum | EtOH in Hex (8 mM $NH_3$.MeO H) | 399 |
| 315 | 369b | 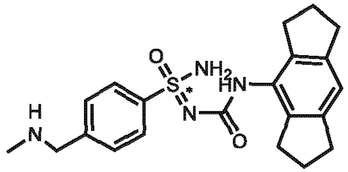 | (R) or (S)-N'-((1,2,3,5,6,7-hexahydro-*s*-indacen-4-yl)carbamoyl)-4-((methylamino)methyl)benze nesulfonimidamide | Chiralpak ID-2, 2*25 cm, 5 um | EtOH in Hex (8 mM $NH_3$.MeO H) | 399 |
| 316 | 159a | 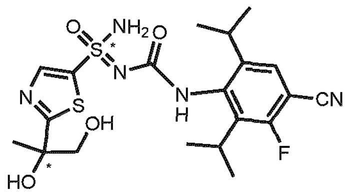 | Two isomers of (R, R) or (R, S) or (S, S) or (S, R)-N'-((4-cyano-3-fluoro-2,6-diisopropylphenyl)carbamoyl) -2-(1,2-dihydroxypropan-2-yl)thiazole-5-sulfonimidamide | CHIRAL ART Cellulose-SB, 2*25 cm, 5 um | EtOH in Hex (0.1% FA) 1st and 2nd peak | 484 |
| 317 | 159ab | 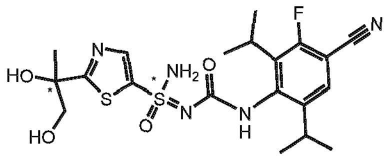 | (R, R) or (R, S) or (S, S) or (S, R)-N-((4-cyano-3-fluoro-2,6-diisopropylphenyl) carbamoyl) -2-(1,2-dihydroxypropan-2-yl) thiazole-5-sulfonimidamide | CHIRAL ART Cellulose-SB, 2*25 cm, 5 um | EtOH in Hex (0.1% FA) 3rd peak | 484 |

(continued)

| Ex. # | Final Target Number | Structure | IUPAC Name | Column | Eluents | LC-MS $[M+H]^+$ |
|---|---|---|---|---|---|---|
| 318 | 159ba | 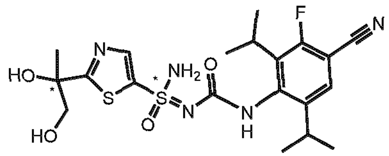 | (S, S) or (S, R) or (R, R) or (R, S)-N-((4-cyano-3-fluoro-2,6-diisopropylphenyl) carbamoyl) -2-(1,2-dihydroxypropan-2-yl) thiazole-5-sulfonimidamide | CHIRAL ART Cellulose-SB, 2*25 cm, 5 um | EtOH in Hex (0.1% FA) 4th peak | 484 |
| 319 | 137a | 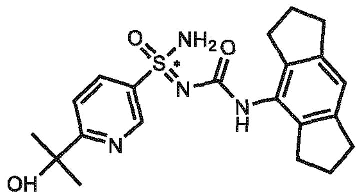 | (S) or (R)-N'-((1,2,3,5,6,7-hexahydro-s-indacen-4-yl)carbamoyl)-6-(2-hydroxypropan-2-yl)pyridine-3-sulfonimidamide | CHIRAL ART Cellulose-SB, 2*25 cm, 5 um | EtOH in Hex (0.1%F A) | 415 |
| 320 | 137b | 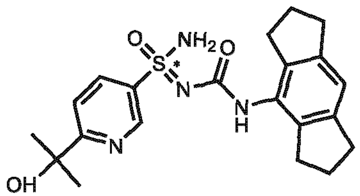 | (R) or (S)-N'-((1,2,3,5,6,7-hexahydro-s-indacen-4-yl)carbamoyl)-6-(2-hydroxypropan-2-yl)pyridine-3 -sulfonimidamide | CHIRAL ART Cellulose-SB, 2*25 cm, 5 um | EtOH in Hex (0.1% FA) | 415 |
| 321 | 317ab | 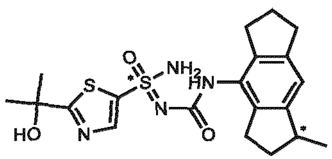 | (S, S) or (S, R)-2-(2-hydroxypropan-2-yl)-N'-((1-methyl-1,2,3,5,6,7-hexahydro-s-indacen-4-yl)carbamoyl)thiazole-5-sulfonimidamide (from Example 291) | 1st and 2nd peak (two isomers) Faster-eluting on column 1: CHIRAL ART Cellulose-SB, 2*25 cm, 5 um, IPA in Hex (0.1% FA). Separated further on column 2: CHIRALPAK IE, EtOH in MTBE (0.1% FA) to obtain single isomers. | | 435 |
| 322 | 317aa | 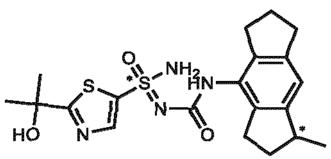 | (S, R) or (S, S)-2-(2-hydroxypropan-2-yl)-N'-((1-methyl-1,2,3,5,6,7-hexahydro-s-indacen-4-yl)carbamoyl)thiazole-5-sulfonimidamide (from Example 291) | | | 435 |

(continued)

| Ex. # | Final Target Number | Structure | IUPAC Name | Column | Eluents | LC-MS $[M+H]^+$ |
|---|---|---|---|---|---|---|
| 323 | 317bb | 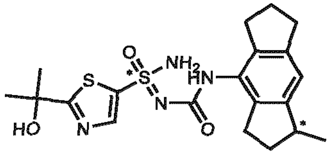 | (R, R) or (R, S) -2-(2-hydroxypropan-2-yl)-N'-((1-methyl-1,2,3,5,6,7-hexahydro-s-indacen-4-yl)carbamoyl)thiazole-5-sulfonimidamide (from Example 291) | CHIRAL ART Cellulose-SB, 2*25 cm, 5 um | IPA in Hex (0.1% FA) 3nd peak | 435 |
| 324 | 317ba | 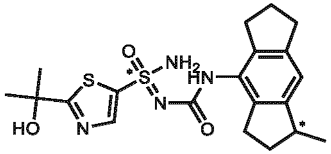 | (R, S) or (R, R)-2-(2-hydroxypropan-2-yl)-N'-((1-methyl-1,2,3,5,6,7-hexahydro-s-indacen-4-yl)carbamoyl)thiazole-5-sulfonimidamide (from Example 291) | | IPA in Hex (0.1% FA) 4th peak | 435 |
| 325 | 316a | 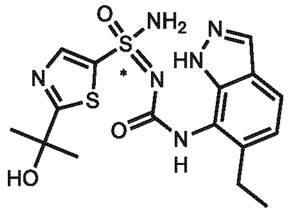 | (S) or (R)-N'-((6-ethyl-1H-indazol-7-yl) carbamoyl)-2-(2-hydroxypropan-2-yl) thiazole-5 -sulfonimidamide | Chiralpak ID, 2*25 cm, 5 um | EtOH in Hex (0.1% FA) | 409 |
| 326 | 316b | 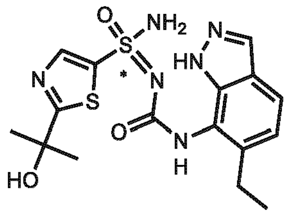 | (R) or (S)-N'-((6-ethyl-1H-indazol-7-yl) carbamoyl)-2-(2-hydroxypropan-2-yl) thiazole-5 -sulfonimidamide | Chiralpak ID, 2*25 cm, 5 um | EtOH in Hex (0.1% FA) | 409 |
| 327 | 373a | 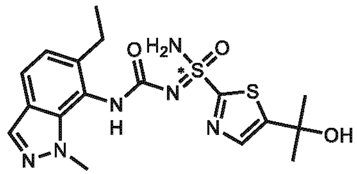 | (S) or (R)-N'-((6-ethyl-1-methyl-1H-indazol-7-yl)carbamoyl)-5-(2-hydroxypropan-2-yl)thiazole-2-sulfonimidamide | CHIRALP AKIG, 20*250 mm, 5 um | EtOH in Hex (0.1% FA) | 423 |

(continued)

| Ex. # | Final Target Number | Structure | IUPAC Name | Column | Eluents | LC-MS $[M+H]^+$ |
|---|---|---|---|---|---|---|
| 328 | 373b | 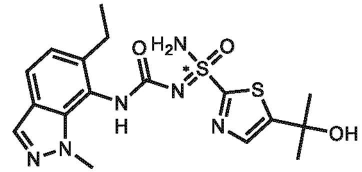 | (R) or (S)-N'-((6-ethyl-1-methyl-1H-indazol-7-yl)carbamoyl)-5-(2-hydroxypropan-2-yl)thiazole-2-sulfonimidamide | CHIRALP AKIG, 20*250 mm, 5 um | EtOH in Hex (0.1% FA) | 423 |
| 329 | 374a | 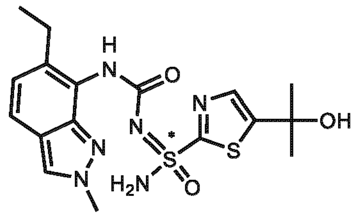 | (S) or (R)-N'-((6-ethyl-2-methyl-2H-indazol-7-yl)carbamoyl)-5-(2-hydroxypropan-2-yl)thiazole-2-sulfonimidamide | CHIRAL ART Cellulose-SB S-5um, 250*20 mm | EtOH in Hex (0.1% FA) | 423 |
| 330 | 374b | 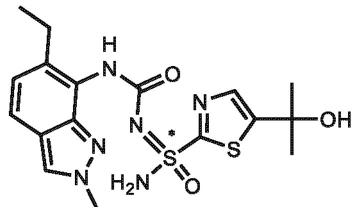 | (R) or (S)-N'-((6-ethyl-2-methyl-2H-indazol-7-yl)carbamoyl)-5-(2-hydroxypropan-2-yl)thiazole-2-sulfonimidamide | CHIRAL ART Cellulose-SB S-5um, 250*20 mm | EtOH in Hex (0.1% FA) | 423 |
| 331 | 319ab | 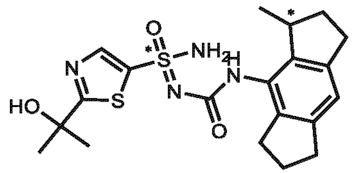 | (S, S) or (S, R)-2-(2-hydroxypropan-2-yl)-N'-((3 - methyl-1,2,3,5,6,7-hexahydro-s-indacen-4-yl)carbamoyl)thiazole-5-sulfonimidamide | 1st peak CHIRAL ART Cellulose-SB, 2*25 cm, 5 um | EtOH in Hex (0.1% FA) | 435 |

(continued)

| Ex. # | Final Target Number | Structure | IUPAC Name | Column | Eluents | LC-MS $[M+H]^+$ |
|---|---|---|---|---|---|---|
| 332 | 319aa | 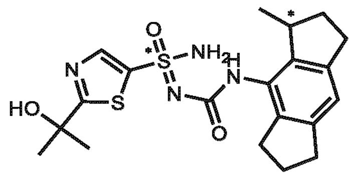 | (R, R) or (R, S)-2-(2-hydroxypropan-2-yl)-N'-((3 - methyl-1,2,3,5,6,7-hexahydro-s-indacen-4-yl)carbamoyl)thiazole-5-sulfonimidamide | 2nd peak CHIRAL ART Cellulose-SB, 2*25 cm, 5 um | EtOH in Hex (0.1% FA) | 435 |
| 333 | 319bb | 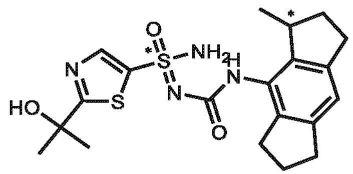 | (S, R) or (S, S)-2-(2-hydroxypropan-2-yl)-N'-((3 - methyl-1,2,3,5,6,7-hexahydro-s-indacen-4-yl)carbamoyl)thiazole-5-sulfonimidamide | 3rd peak CHIRAL ART Cellulose-SB, 2*25 cm, 5 um | EtOH in Hex (0.1% FA) | 435 |
| 334 | 319ba | 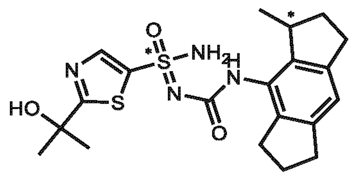 | (R, S) or (R, R)-2-(2-hydroxypropan-2-yl)-N'-((3 - methyl-1,2,3,5,6,7-hexahydro-s-indacen-4-yl)carbamoyl)thiazole-5-sulfonimidamide | 4th peak CHIRAL ART Cellulose-SB, 2*25 cm, 5 um | EtOH in Hex (0.1% FA) | 435 |
| 335 | 320a | 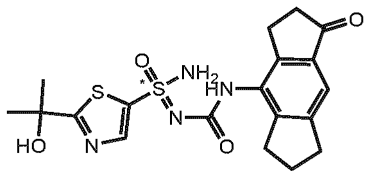 | (S) or (R)- 2-(2-Hydroxypropan-2-yl)-N'-((1-oxo-1,2,3,5,6,7-hexahydro-s-indacen-4-yl)carbamoyl)thiazole-5-sulfonimidamide from Example 293 | Chiralpak IA, 2*25 cm, 5 um | EtOH in Hex (0.1% FA) | 435 |
| 336 | 320b | 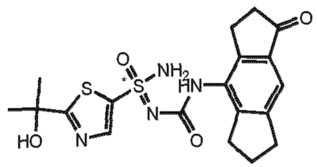 | (R) or (S)- 2-(2-Hydroxypropan-2-yl)-N'-((1-oxo-1,2,3,5,6,7-hexahydro-s-indacen-4-yl)carbamoyl)thiazole-5-sulfonimidamide from Example 293 | Chiralpak IA, 2*25 cm, 5 um | EtOH in Hex (0.1% FA) | 435 |

(continued)

| Ex. # | Final Target Number | Structure | IUPAC Name | Column | Eluents | LC-MS $[M+H]^+$ |
|---|---|---|---|---|---|---|
| 337 | 323ab | 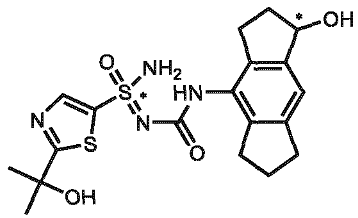 | (R, R) or (R, S)- N'-((1-hydroxy-1,2,3,5,6,7-hexahydro-s-indacen-4-yl) carbamoyl)-2-(2-hydroxypropan-2-yl) thiazole-5-sulfonimidamide (from example 336) | CHIRALP AK AD, 2*25 cm, 5 um | EtOH (0.1% DEA) in $CO_2$, 1st peak | 437 |
| 338 | 323bb | 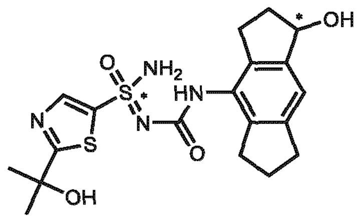 | (R, S) or (R, R)- N'-((1-hydroxy-1,2,3,5,6,7-hexahydro-s-indacen-4-yl) carbamoyl)-2-(2-hydroxypropan-2-yl) thiazole-5-sulfonimidamide (from example 336) | | EtOH (0.1% DEA) in $CO_2$, 2nd peak | 437 |
| 339 | 323aa | 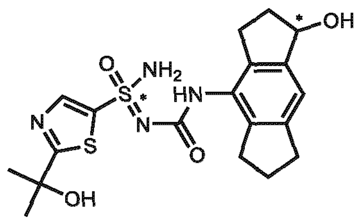 | (S, S) or (S, R)- N'-((1-hydroxy-1,2,3,5,6,7-hexahydro-s-indacen-4-yl) carbamoyl)-2-(2-hydroxypropan-2-yl) thiazole-5-sulfonimidamide (from example 335) | CHIRALP AKAD, 2*25 cm, 5 um | EtOH (0.1% DEA) in $CO_2$, 1st peak | 437 |
| 340 | 323ba | 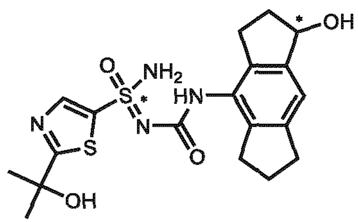 | (S, R) or (S, S)- N'-((1-hydroxy-1,2,3,5,6,7-hexahydro-s-indacen-4-yl) carbamoyl)-2-(2-hydroxypropan-2-yl) thiazole-5-sulfonimidamide (from example 335) | | EtOH (0.1% DEA) in $CO_2$, 2nd peak | 437 |

(continued)

| Ex. # | Final Target Number | Structure | IUPAC Name | Column | Eluents | LC-MS $[M+H]^+$ |
|---|---|---|---|---|---|---|
| 341 | 303a | 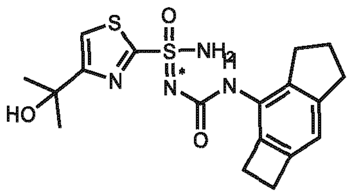 | (R) or (S)-4-(2-hydroxypropan-2-yl)-N'-((2,4,5,6-tetrahydro-1H-cyclobuta [f]inden-3-yl)carbamoyl)thiazole-2-sulfonimidamide | Chiralpak ID, 2*25cm, Sum | EtOH in Hex (0.1% FA) | 407 |
| 342 | 303b | 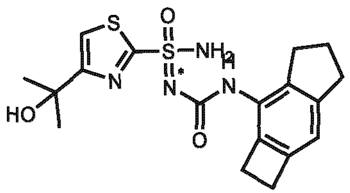 | (R) or (S)-4-(2-hydroxypropan-2-yl)-N'-((2,4,5,6-tetrahydro-1H-cyclobuta [f]inden-3-yl)carbamoyl)thiazole-2-sulfonimidamide | Chiralpak ID, 2*25cm, Sum | EtOH in Hex (0.1% FA) | 407 |
| 343 | 315a | 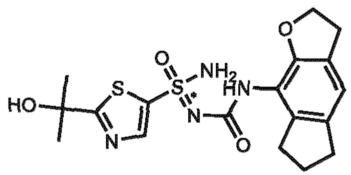 | (R) or (S)-2-(2-hydroxypropan-2-yl)-N'-((3,5,6,7-tetrahydro-2H-indeno[5,6-b]furan-8-yl)carbamoyl)thiazole-5-sulfonimidamide | Chiralpak ID, 2*25 cm, 5 um | EtOH in Hex (0.1% FA) | 423 |
| 344 | 315b | 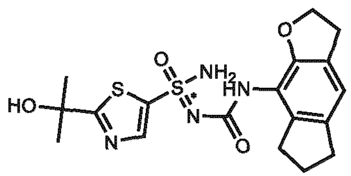 | (R) or (S)-2-(2-hydroxypropan-2-yl)-N'-((3,5,6,7-tetrahydro-2H-indeno [5,6-b]furan-8-yl)carbamoyl)thiazole-5-sulfonimidamide | Chiralpak ID, 2*25 cm, 5 um | EtOH in Hex (0.1% FA) | 423 |
| 345 | 138a | 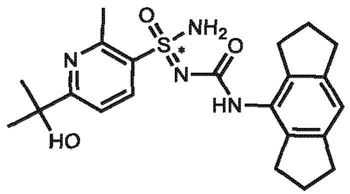 | (R) or (S)-N'-((1,2,3,5,6,7-hexahydro-s-indacen-4-yl)carbamoyl)-6-(2-hydroxypropan-2-yl)-2-methylpyridine-3-sulfonimidamide | CHIRALP AK IF, 2*25 cm, 5 um | EtOH in Hex (8 mM $NH_3$-MeOH) | 429 |

(continued)

| Ex. # | Final Target Number | Structure | IUPAC Name | Column | Eluents | LC-MS $[M+H]^+$ |
|---|---|---|---|---|---|---|
| 346 | 138b | 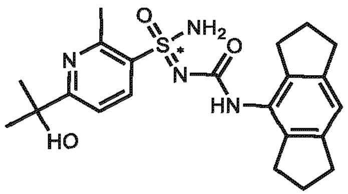 | (R) or (S)-N'-((1,2,3,5,6,7-hexahydro-s-indacen-4-yl)carbamoyl)-6-(2-hydroxypropan-2-yl)-2-methylpyridine-3 -sulfonimidamide | CHIRALP AK IF, 2*25 cm, 5 um | EtOH in Hex (8 mM $NH_3$.MeO H) | 429 |
| 347 | 328a | 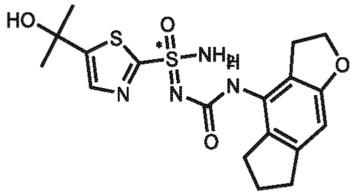 | (R) or (S)-5-(2-hydroxypropan-2-yl)-N'-((3,5,6,7-tetrahydro-2H-indeno [5,6-b]furan-4-yl)carbamoyl)thiazole-2-sulfonimidamide | CHIRALP AKIC, 2*25 cm, 5 um | EtOH in Hex (0.1% FA) | 423 |
| 348 | 328b | 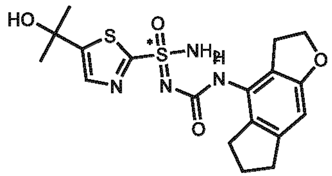 | (S) or (R)-5-(2-hydroxypropan-2-yl)-N'-((3,5,6,7-tetrahydro-2H-indeno [5,6-b]furan-4-yl)carbamoyl)thiazole-2-sulfonimidamide | CHIRALP AKIC, 2*25 cm, 5 um | EtOH in Hex (0.1% FA) | 423 |
| 349 | 326b | 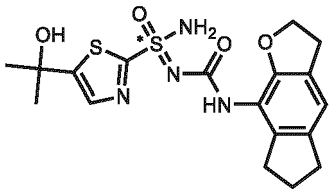 | (S) or (R)-5-(2-hydroxypropan-2-yl)-N'-((3,5,6,7-tetrahydro-2H-indeno[5,6-b]furan-8-yl)carbamoyl)thiazole-2-sulfonimidamide | CHIRALP AK IG, 20*250 mm, 5 um | IPA in Hex:DCM =5:1 (0.1% FA) | 423 |
| 350 | 326a | 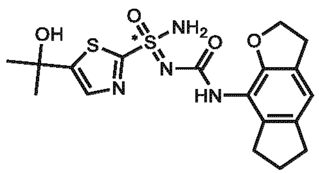 | (R) or (S)-5-(2-hydroxypropan-2-yl)-N'-((3,5,6,7-tetrahydro-2H-indeno[5,6-b]furan-8-yl)carbamoyl)thiazole-2-sulfonimidamide | CHIRALP AK IG, 20*250 mm, 5 um | IPA in Hex:DCM =5:1 (0.1% FA) | 423 |

(continued)

| Ex. # | Final Target Number | Structure | IUPAC Name | Column | Eluents | LC-MS $[M+H]^+$ |
|---|---|---|---|---|---|---|
| 351 | 318a | 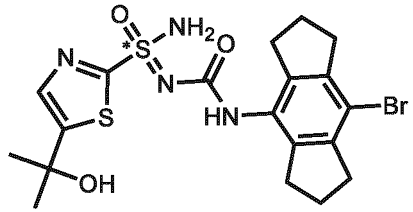 | (S) or (R)-N'-((8-bromo-1,2,3,5,6,7-hexahydro-s-indacen-4-yl)carbamoyl)-5-(2-hydroxypropan-2-yl)thiazole-2-sulfonimidamide | CHIRAL ART Cellulose-SB S-5um, 2*25 cm, 5 um | EtOH in Hex (8 mM $NH_3$.MeO H) | 499 |
| 352 | 318b | 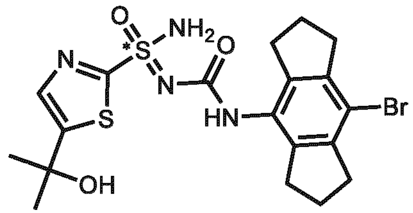 | (R) or (S)-N'-((8-bromo-1,2,3,5,6,7-hexahydro-s-indacen-4-yl)carbamoyl)-5-(2-hydroxypropan-2-yl)thiazole-2-sulfonimidamide | CHIRAL ART Cellulose-SB S-5um, 2*25 cm, 5 um | EtOH in Hex (8 mM $NH_3$.MeO H) | 499 |
| 353 | 325a | 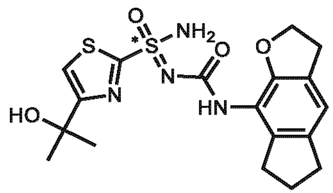 | (S) or (R)-4-(2-hydroxypropan-2-yl)-N'-((3,5,6,7-tetrahydro-2H-indeno [5,6-b]furan-8-yl)carbamoyl)thiazole-2-sulfonimidamide | CHIRAL ART Cellulose-SB, 2*25 cm, 5 um | EtOH in Hex (0.1% FA) | 423 |
| 354 | 325b | 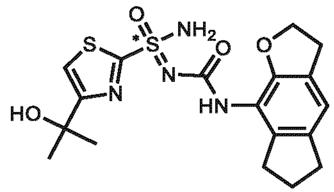 | (R) or (S)-4-(2-hydroxypropan-2-yl)-N'-((3,5,6,7-tetrahydro-2H-indeno [5,6-b]furan-8-yl)carbamoyl)thiazole-2-sulfonimidamide | CHIRAL ART Cellulose-SB, 2*25 cm, 5 um | EtOH in Hex (0.1% FA) | 423 |

(continued)

| Ex. # | Final Target Number | Structure | IUPAC Name | Column | Eluents | LC-MS $[M+H]^+$ |
|---|---|---|---|---|---|---|
| 355 | 329a | 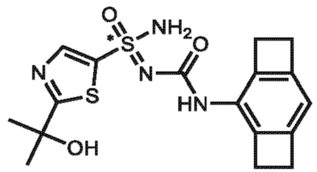 | (R) or (S) -2-(2-hydroxypropan-2-yl)-N'-(tricyclo[6.2.0.03,6]deca-1,3(6),7-trien-2-ylcarbamoyl)thiazole-5-sulfonimidamide | Chiralpak ID, 2*25 cm, 5 um | IPA in Hex (0.1% FA) | 393 |
| 356 | 329b | 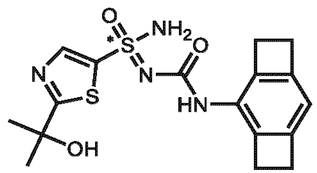 | (S) or (R) -2-(2-hydroxypropan-2-yl)-N'-(tricyclo[6.2.0.03,6]deca-1,3(6),7-trien-2-ylcarbamoyl)thiazole-5-sulfonimidamide | Chiralpak ID, 2*25 cm, 5 um | IPA in Hex (0.1% FA) | 393 |
| 357 | 404b | 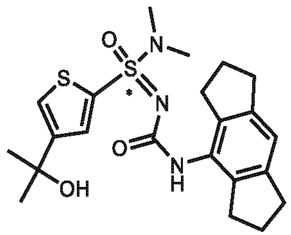 | (R) or (S) -N'-((1,2,3,5,6,7-hexahydro-s-indacen-4-yl)carbamoyl)-4-(2-hydroxypropan-2-yl)-N,N-dimethylthiophene-2-sulfonimidamide | CHIRALP AKIG, 20*250 mm, 5 um | IPA in Hex:DCM =3:1 (10 mM $NH_3$-MeOH) | 448 |
| 358 | 404a | 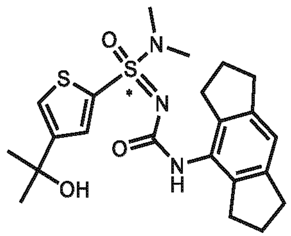 | (S) or (R) -N'-((1,2,3,5,6,7-hexahydro-s-indacen-4-yl)carbamoyl)-4-(2-hydroxypropan-2-yl)-N,N-dimethylthiophene-2-sulfonimidamide | CHIRALP AKIG, 20*250 mm, 5 um | IPA in Hex:DCM =3:1 (10 mM $NH_3$-MeOH) | 448 |

(continued)

| Ex. # | Final Target Number | Structure | IUPAC Name | Column | Eluents | LC-MS $[M+H]^+$ |
|---|---|---|---|---|---|---|
| 359 | 332a | 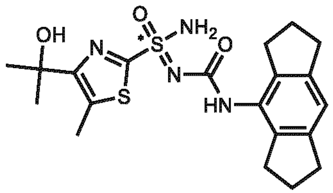 | (R) or (S) -N'-((1,2,3,5,6,7-hexahydro-s-indacen-4-yl)carbamoyl)-4-(2-hydroxypropan-2-yl)-5 - methylthiazole-2-sulfonimidamide | Chiralpak ID, 2*25 cm, 5 um | IPA in Hex (0.1% FA) | 435 |
| 360 | 332b | 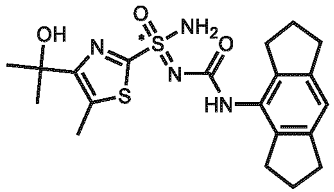 | (S) or (R) -N'-((1,2,3,5,6,7-hexahydro-s-indacen-4-yl)carbamoyl)-4-(2-hydroxypropan-2-yl)-5 - methylthiazole-2-sulfonimidamide | Chiralpak ID, 2*25 cm, 5 um | IPA in Hex (0.1% FA) | 435 |
| 361 | 335a | 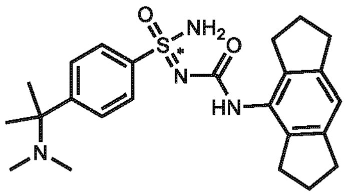 | (R) or (S) -4-(2-(dimethylamino)propan-2-yl)-N'-((1,2,3,5,6,7-hexahydro-s-indacen-4-yl)carbamoyl)benzenesulfoni midamide | CHIRALP AK IG, 2.0*25 cm (5 um) | IPA in Hex (8 mM $NH_3$-MeOH) | 441 |
| 362 | 335b | 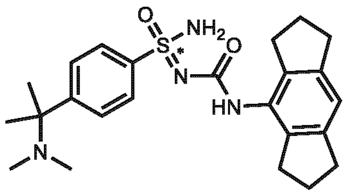 | (S) or (R) -4-(2-(dimethylamino)propan-2-yl)-N'-((1,2,3,5,6,7-hexahydro-s-indacen-4-yl)carbamoyl)benzenesulfoni midamide | CHIRALP AKIG, 2.0*25 cm (5 um) | IPA in Hex (8 mM $NH_3$-MeOH | 441 |

(continued)

| Ex. # | Final Target Number | Structure | IUPAC Name | Column | Eluents | LC-MS $[M+H]^+$ |
|---|---|---|---|---|---|---|
| 363 | 336a | 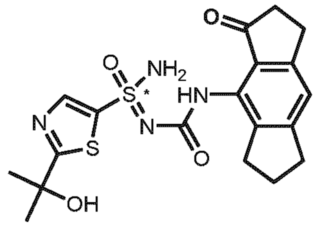 | (S) or (R)- 2-(2-Hydroxypropan-2-yl)-N'-((3 - oxo-1,2,3,5,6,7-hexahydro-s-indacen-4-yl)carbamoyl)thiazole-5-sulfonimidamide | CHIRAL ART Cellulose-SB, 2*25 cm, 5 um | EtOH in MTBE (10 mM $NH_3$-MeOH) | 435 |
| 364 | 336b | 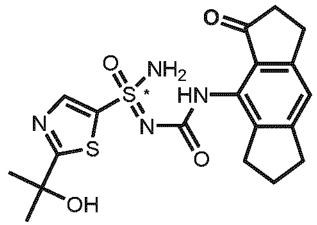 | (R) or (S)- 2-(2-Hydroxypropan-2-yl)-N'-((3 - oxo-1,2,3,5,6,7-hexahydro-s-indacen-4-yl)carbamoyl)thiazole-5-sulfonimidamide | CHIRAL ART Cellulose-SB, 2*25 cm, 5 um | EtOH in MTBE (10 mM $NH_3$-MeOH) | 435 |
| 365 | 337a | 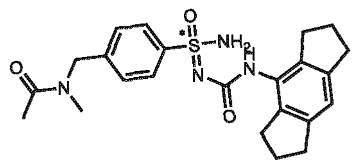 | (S) or (R)-N-(4-(N'-((1,2,3,5,6,7-hexahydro-s-indacen-4-yl)carbamoyl) sulfamidimidoy l)benzyl)-N-methylacetamide | CHIRAL ART Cellulose-SB, 2*25 cm, 5 um | EtOH in Hex (8 mM $NH_3$-MeOH) | 441 |
| 366 | 337b | 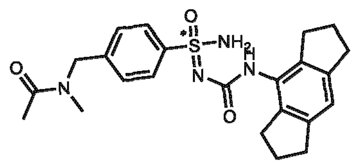 | (R) or (S)-N-(4-(N'-((1,2,3,5,6,7-hexahydro-s-indacen-4-yl)carbamoyl) sulfamidimidoy l)benzyl)-N-methylacetamide | CHIRAL ART Cellulose-SB, 2*25 cm, 5 um | EtOH in Hex (8 mM $NH_3$-MeOH) | 441 |
| 367 | 371a | 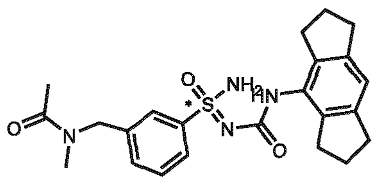 | (S) or (R)-N-(3-(N'-((1,2,3,5,6,7-hexahydro-s-indacen-4-yl)carbamoyl) sulfamidimidoy l)benzyl)-N-methylacetamide | CHIRAL ART Cellulose-SB, 2*25 cm, 5 um | EtOH in Hex (8 mM $NH_3$-MeOH) | 441 |

(continued)

| Ex. # | Final Target Number | Structure | IUPAC Name | Column | Eluents | LC-MS $[M+H]^+$ |
|---|---|---|---|---|---|---|
| 368 | 371b | 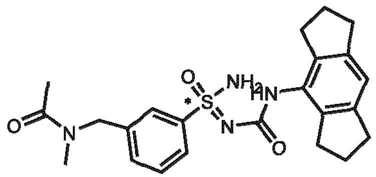 | (R) or (S)-N-(3-(N'-((1,2,3,5,6,7-hexahydro-s-indacen-4-yl)carbamoyl) sulfamidimidoy l)benzyl)-N-methylacetamide | CHIRAL ART Cellulose-SB, 2*25 cm, 5 um | EtOH in Hex (8 mM $NH_3$-MeOH) | 441 |
| 369 | 372a | 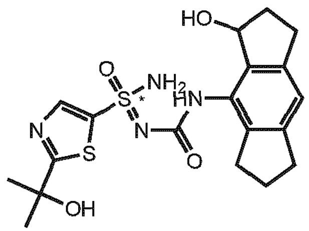 | (S, R/S) or (R, R/S)- N'-((3-hydroxy-1,2,3,5,6,7-hexahydro-s-indacen-4-yl) carbamoyl)-2-(2-hydroxypropan-2-yl) thiazole-5 -sulfonimidamide | Obtained from Example 363 | N/A | 435 (M-1) |
| 370 | 372b | 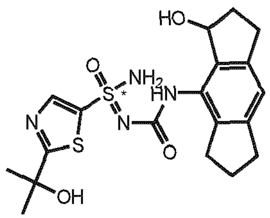 | (R, R/S) or (S, R/S)- N'-((3-hydroxy-1,2,3,5,6,7-hexahydro-s-indacen-4-yl) carbamoyl)-2-(2-hydroxypropan-2-yl) thiazole-5-sulfonimidamide | Obtained from Example 364 | N/A | 435 (M-1) |
| 371 | 334a | 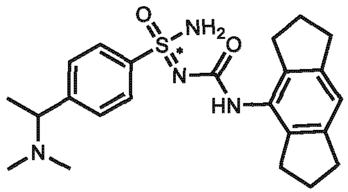 | (S) or (R)-4-(1-(dimethylamino) ethyl)-N'-((1,2,3,5,6,7-hexahydro-s-indacen-4-yl)carbamoyl) benzenesulfonimidamide | CHIRAL ART Cellulose-SB, 2*25 cm, 5 um | IPA in Hex (8 mM $NH_3$-MeOH) | 427 |

(continued)

| Ex. # | Final Target Number | Structure | IUPAC Name | Column | Eluents | LC-MS $[M+H]^+$ |
|---|---|---|---|---|---|---|
| 372 | 334b | 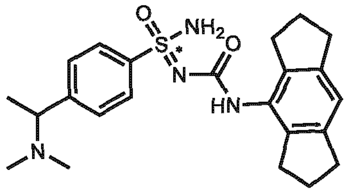 | (R) or (S)-4-(1-(dimethylamino) ethyl)-N'-((1,2,3,5,6,7-hexahydro-s-indacen-4-yl)carbamoyl)benzenesulfoni midamide | CHIRAL ART Cellulose-SB, 2*25 cm, 5 um | IPA in Hex (8 mM $NH_3$-MeOH) | 427 |
| 373 | 339a | 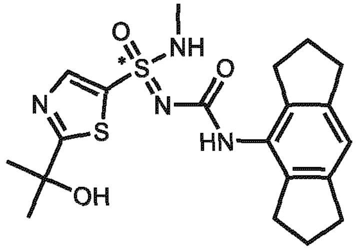 | (R) or (S) -N'-((1,2,3,5,6,7-hexahydro-s-indacen-4-yl)carbamoyl)-2-(2-hydroxypropan-2-yl)-N-methylthiazole-5-sulfonimidamide | CHIRALP AK IE, 2*25 cm, 5 um | IPA in Hex (8 mM $NH_3$-MeOH) | 435 |
| 374 | 339b | 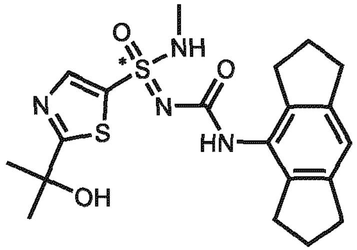 | (S) or (R)-N'-((1,2,3,5,6,7-hexahydro-s-indacen-4-yl)carbamoyl)-2-(2-hydroxypropan-2-yl)-N-methylthiazole-5-sulfonimidamide | CHIRALP AK IE, 2*25 cm, 5 um | IPA in Hex (8 mM $NH_3$-MeOH) | 435 |
| 375 | 334ab | 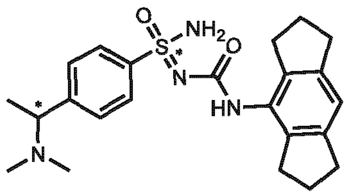 | (S, R) or (S, S) or (R, S) or (R, R)-4-(1-(dimethylamino) ethyl)-N'-((1,2,3,5,6,7-hexahydro-s-indacen-4-yl)carbamoyl)benzenesulfoni midamide | CHIRALP AKIG, 20*250 mm, 5 um | EtOH in Hex (8 mM $NH_3$-MeOH) | 427 |

(continued)

| Ex. # | Final Target Number | Structure | IUPAC Name | Column | Eluents | LC-MS $[M+H]^+$ |
|---|---|---|---|---|---|---|
| 376 | 334aa | 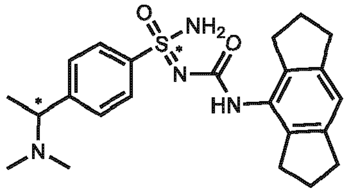 | (S, S) or (S, R) or (R, R) or (R, S)-4-(1-(dimethylamino) ethyl)-N'-((1,2,3,5,6,7-hexahydro-s-indacen-4-yl)carbamoyl)benzenesulfoni midamide | CHIRALP AKIG, 20*250 mm, 5 um | EtOH in Hex (8 mM $NH_3$-MeOH) | 427 |
| 377 | 334bb | 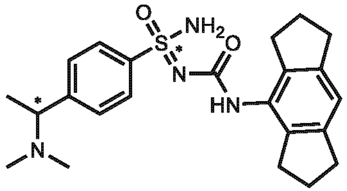 | (R, R) or (R, S) or (S, S) or (S, R)-4-(1-(dimethylamino) ethyl)-N'-((1,2,3,5,6,7-hexahydro-s-indacen-4-yl)carbamoyl)benzenesulfoni midamide | CHIRALP AKIG, 20*250 mm, 5 um | EtOH in Hex (8 mM $NH_3$-MeOH) | 427 |
| 378 | 334ba | 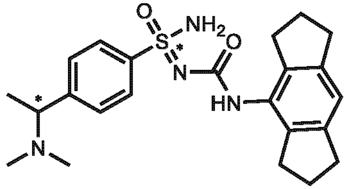 | (R, S) or (R, R) or (S, R) or (S, S)-4-(1-(dimethylamino)ethyl)-N'-((l, 2,3,5,6,7-hexahydro-s-indacen-4-yl) carbamoyl)benzenesulfoni midamide | CHIRALP AKIG, 20*250 mm, 5 um | EtOH in Hex (8 mM $NH_3$-MeOH) | 427 |
| 379 | 338a | 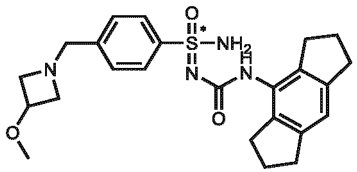 | (R) or (S) -N'-((1,2,3,5,6,7-hexahydro-s-indacen-4-yl)carbamoyl)-4-((3-methoxyazetidin-1 - yl)methyl) benzenesulfonimid amide | CHIRALP AK IF, 2*25 cm, 5 um | EtOH in Hex (8 mM $NH_3$-MeOH) | 455 |
| 380 | 338b | 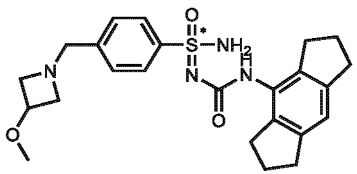 | (S) or (R) -N'-((1,2,3,5,6,7-hexahydro-s-indacen-4-yl)carbamoyl)-4-((3-methoxyazetidin-1 - yl)methyl) benzenesulfonimid amide | CHIRALP AK IF, 2*25 cm, 5 um | EtOH in Hex (8 mM $NH_3$-MeOH) | 455 |

(continued)

| Ex. # | Final Target Number | Structure | IUPAC Name | Column | Eluents | LC-MS $[M+H]^+$ |
|---|---|---|---|---|---|---|
| 381 | 340a | 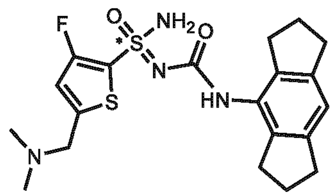 | (R) or (S) -5-((dimethylamino)methyl)-3 -fluoro-N'-((1,2,3,5,6,7-hexahydro-s-indacen-4-yl)carbamoyl)thiophene-2-sulfonimidamide | CHIRALP AK IG, 20*250 mm, 5 um | Hex (0.1% DEA): EtO H=50:50 | 437 |
| 382 | 340b | 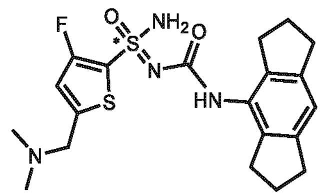 | (S) or (R) -5-((dimethylamino)methyl)-3 -fluoro-N'-((1,2,3,5,6,7-hexahydro-s-indacen-4-yl)carbamoyl)thiophene-2-sulfonimidamide | CHIRALP AKIG, 20*250 mm, 5 um | Hex (0.1% DEA): EtO H=50:50 | 437 |
| 383 | 361b | 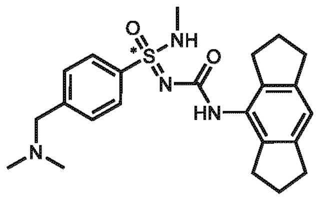 | (R) or (S) -4-((dimethylamino) methyl)-N'-((l,2,3,5,6,7-hexahydro-s-indacen-4-yl)carbamoyl)-N-methylbenzenesulfonimidami de | CHIRALP AK IE, 2*25 cm, 5 um | EtOH in Hex (8 mM $NH_3$-MeOH) | 427 |
| 384 | 361a | 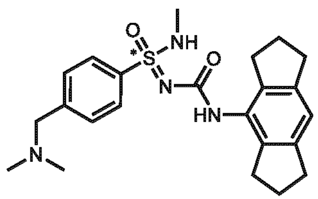 | (S) or (R) -4-((dimethylamino) methyl)-N'-((1,2,3,5,6,7-hexahydro-s-indacen-4-yl)carbamoyl)-N-methylbenzenesulfonimidami de | CHIRALP AK IE, 2*25 cm, 5 um | EtOH in Hex (8 mM $NH_3$-MeOH) | 427 |

(continued)

| Ex. # | Final Target Number | Structure | IUPAC Name | Column | Eluents | LC-MS $[M+H]^+$ |
|---|---|---|---|---|---|---|
| 385 | 113a | 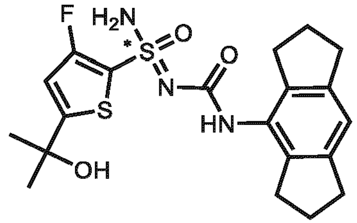 | (R) or (S)-3-fluoro-N'-((1,2,3,5,6,7-hexahydro-s-indacen-4-yl)carbamoyl)-5-(2-hydroxypropan-2-yl)thiophene-2-sulfonimidamide | CHIRALP AK IG, 20*250 mm, 5 um | IPA in Hex (8 mM $NH_3$-MeOH) | 438 |
| 386 | 113b | 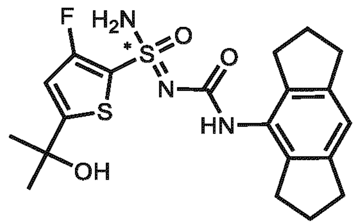 | (S) or (R) -3-fluoro-N'-((1,2,3,5,6,7-hexahydro-s-indacen-4-yl)carbamoyl)-5-(2-hydroxypropan-2-yl)thiophene-2-sulfonimidamide | CHIRALP AK IG, 20*250 mm, 5 um | IPA in Hex (8 mM $NH_3$-MeOH) | 438 |
| 387 | 330a | 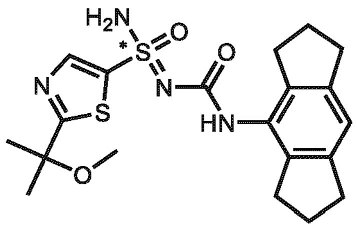 | (S) or (R)-N'-((1,2,3,5,6,7-hexahydro-s-indacen-4-yl)carbamoyl)-2-(2-methoxypropan-2-yl)thiazole-5 -sulfonimidamide | CHIRAL ART Cellulose-SB, 2*25 cm, 5 um | EtOH in Hex (8 mM $NH_3$-MeOH) | 435 |
| 388 | 330b | 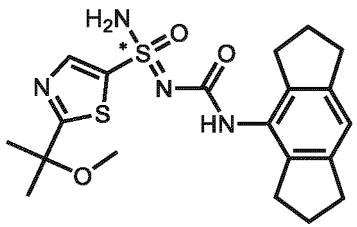 | (R) or (S)-N'-((1,2,3,5,6,7-hexahydro-s-indacen-4-yl)carbamoyl)-2-(2-methoxypropan-2-yl)thiazole-5-sulfonimidamide | CHIRAL ART Cellulose-SB, 2*25 cm, 5 um | EtOH in Hex (8 mM $NH_3$-MeOH) | 435 |

(continued)

| Ex. # | Final Target Number | Structure | IUPAC Name | Column | Eluents | LC-MS $[M+H]^+$ |
|---|---|---|---|---|---|---|
| 389 | 341a | 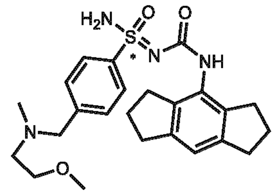 | (S) or (R)-N'-((1,2,3,5,6,7-hexahydro-s-indacen-4-yl)carbamoyl)-4-(((2-methoxyethyl)(methyl)amino) methyl) benzenesulfonimidam ide | CHIRALC ellulose-SB 4.6*100 mm 3 um | Hex (0.1% DEA): EtO H=70:30 | 457 |
| 390 | 341b | 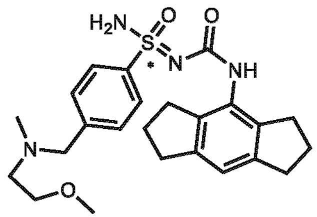 | (R) or (S)-N'-((1,2,3,5,6,7-hexahydro-s-indacen-4-yl)carbamoyl)-4-(((2-methoxyethyl)(methyl)amino) methyl) benzenesulfonimidam ide | CHIRALC ellulose-SB 4.6*100 mm 3 um | Hex (0.1% DEA): EtO H=70:30 | 457 |
| 391 | 360ba | 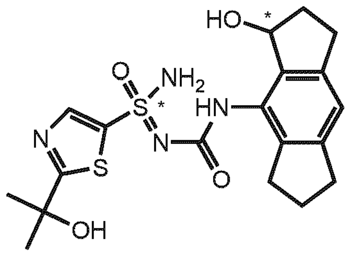 | (R, R) or (R, S) or (S, S) or (S, R)-N'-((3-hydroxy-1,2,3,5,6,7-hexahydro-s-indacen-4-yl)carbamoyl)-2-(2-hydroxypropan-2-yl)thiazole-5-sulfonimidamide (from Example 370) | CHIRALP AK IG, 20*250 mm, 5 um | EtOH in MTBE (10 mM $NH_3$-MeOH) | 437 |
| 392 | 360bb | 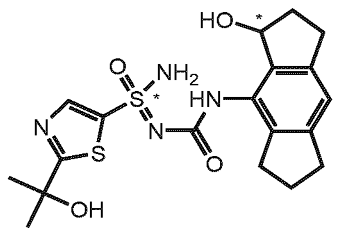 | (R, S) or (R, R) or (S, R) or (S, S)- N'-((3-hydroxy-1,2,3,5,6,7-hexahydro-s-indacen-4-yl)carbamoyl)-2-(2-hydroxypropan-2-yl)thiazole-5-sulfonimidamide (from Example 370) | CHIRALP AK IG, 20*250 mm, 5 um | EtOH in MTBE (10 mM $NH_3$-MeOH) | 437 |

(continued)

| Ex. # | Final Target Number | Structure | IUPAC Name | Column | Eluents | LC-MS $[M+H]^+$ |
|---|---|---|---|---|---|---|
| 393 | 363b | 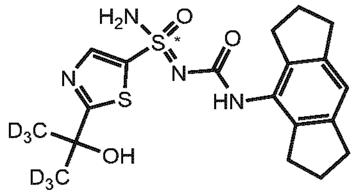 | (R)-N'-((1,2,3,5,6,7-hexahydro-s-indacen-4-yl)carbamoyl)-2-(2-hydroxypropan-2-yl-1,1,1,3,3,3-$d_6$) thiazole-5-sulfonimidamide | CHIRALP AK IF, 2*25 cm, 5 um | 40% MeOH in $CO_2$ | 427 |
| 394 | 363a | 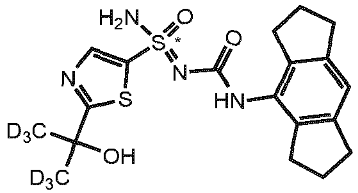 | (S)- N'-((1,2,3,5,6,7-hexahydro-s-indacen-4-yl)carbamoyl)-2-(2-hydroxypropan-2-yl-1,1,1,3,3,3-$d_6$) thiazole-5-sulfonimidamide | CHIRALP AK IF, 2*25 cm, 5 um | 40% MeOH in $CO_2$ | 427 |
| 395 | 343a | 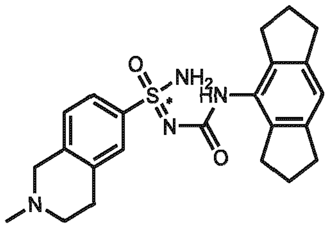 | (R) or (S)-N'-((1,2,3,5,6,7-hexahydro-s-indacen-4-yl)carbamoyl)-2-methyl-1,2,3,4-tetrahydroisoquinoline-6-sulfonimidamide | CHIRALP AK IG, 20*250mm ,5 um | EtOH in Hex (8 mM $NH_3$-MeOH | 425 |
| 396 | 343b | 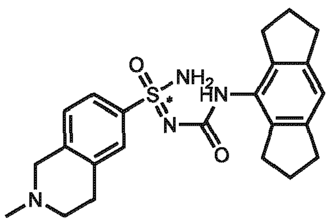 | (S) or (R)-N'-((1,2,3,5,6,7-hexahydro-s-indacen-4-yl)carbamoyl)-2-methyl-1,2,3,4-tetrahydroisoquinoline-6-sulfonimidamide | CHIRALP AKIG, 20*250mm ,5 um | EtOH in Hex (8 mM $NH_3$-MeOH | 425 |

(continued)

| Ex. # | Final Target Number | Structure | IUPAC Name | Column | Eluents | LC-MS $[M+H]^+$ |
|---|---|---|---|---|---|---|
| 397 | 359a | 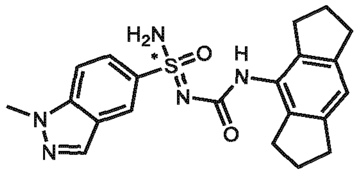 | (R) or (S)-N'-((1,2,3,5,6,7-hexahydro-s-indacen-4-yl)carbamoyl)-1-methyl-1H-indazole-5-sulfonimidamide | Chiralpakl D, 2*25 cm, 5 um | IPA in Hex:DCM =3:1 (10 mM $NH_3$-MeOH) | 410 |
| 398 | 359b | 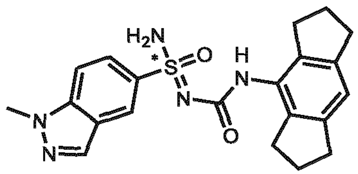 | (S) or (R)-N'-((1,2,3,5,6,7-hexahydro-s-indacen-4-yl)carbamoyl)-1-methyl-1H-indazole-5-sulfonimidamide | Chiralpak ID, 2*25cm, 5 um | IPA in Hex:DCM =3:1 (10 mM $NH_3$-MeOH) | 410 |
| 399 | 352a | 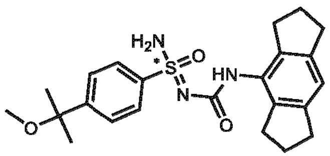 | (R) or (S) -N'-((1,2,3,5,6,7-hexahydro-s-indacen-4-yl)carbamoyl)-4-(2-methoxypropan-2-yl) benzenesulfonimidamide | CHIRALP AKIG, 2.0*25 cm (5 um) | Hex (0.1% DEA): IPA =70:30 | 428 |
| 400 | 352b | 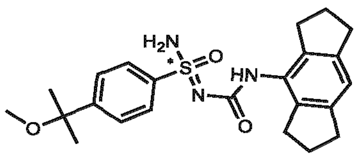 | (S) or (R) -N'-((1,2,3,5,6,7-hexahydro-s-indacen-4-yl)carbamoyl)-4-(2-methoxypropan-2-yl) benzenesulfonimidamide | CHIRALP AK IG, 2.0*25 cm (5 um) | Hex (0.1% DE A): IPA=70 :30 | 428 |
| 401 | 383a | 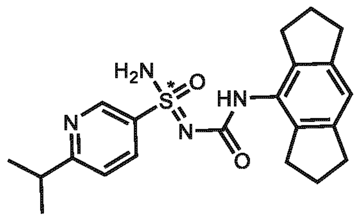 | (R) or (S) -N'-((1,2,3,5,6,7-hexahydro-s-indacen-4-yl)carbamoyl)-6-isopropylpyridine-3 - sulfonimidamide | CHIRALP AK IG, 2*25 cm, 5 um | EtOH in Hex (8 mM $NH_3$-MeOH) | 399 |

(continued)

| Ex. # | Final Target Number | Structure | IUPAC Name | Column | Eluents | LC-MS $[M+H]^+$ |
|---|---|---|---|---|---|---|
| 402 | 383b | 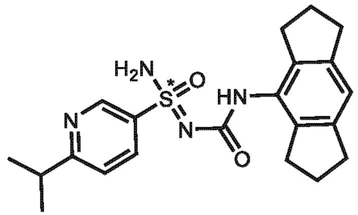 | (S) or (R) -N'-((1,2,3,5,6,7-hexahydro-s-indacen-4-yl)carbamoyl)-6-isopropylpyridine-3 - sulfonimidamide | CHIRALP AK IG, 2*25 cm, 5 um | EtOH in Hex (8 mM $NH_3$-MeOH) | 399 |
| 403 | 382a | 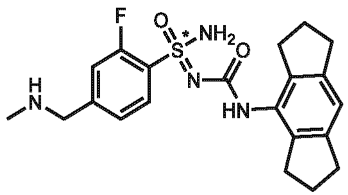 | (R) or (S) -2-fluoro-N'-((l,2,3,5,6,7-hexahydro-s-indacen-4-yl)carbamoyl)-4-((methylamino)methyl)benze nesulfonimidamide | CHIRALP AK IG, 2*25 cm, 5 um | EtOH in Hex (8 mM $NH_3$-MeOH) | 417 |
| 404 | 382b | 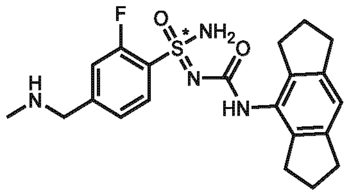 | (S) or (R) -2-fluoro-N'-((1,2,3,5,6,7-hexahydro-s-indacen-4-yl)carbamoyl)-4-((methylamino)methyl)benze nesulfonimidamide | CHIRALP AK IG, 2*25 cm, 5 um | EtOH in Hex (8 mM $NH_3$.MeO H) | 417 |
| 405 | 379a | 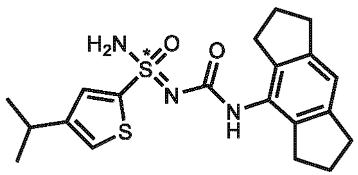 | (R) or (S) -N'-((1,2,3,5,6,7-hexahydro-s-indacen-4-yl)carbamoyl)-4-isopropylthiophene-2-sulfonimidamide | CHIRALP AKIG, 2.0*25 cm (5 um) | EtOH in Hex (8 mM $NH_3$-MeOH) | 404 |
| 406 | 379b | 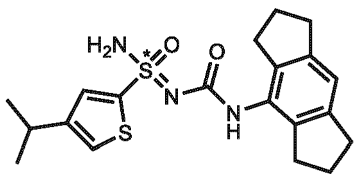 | (S) or (R) -N'-((1,2,3,5,6,7-hexahydro-s-indacen-4-yl)carbamoyl)-4-isopropylthiophene-2-sulfonimidamide | CHIRALP AKIG, 2.0*25 cm (5 um) | EtOH in Hex (8 mM $NH_3$-MeOH) | 404 |

(continued)

| Ex. # | Final Target Number | Structure | IUPAC Name | Column | Eluents | LC-MS $[M+H]^+$ |
|---|---|---|---|---|---|---|
| 407 | 380a | 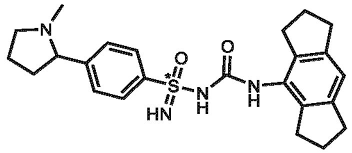 | (R, R) or (R, S) or (S, S) or (S, R)-N-((1,2,3,5,6,7-hexahydro-s-indacen-4-yl)carbamoyl)-4-(1-methylpyrrolidin-2-yl)benzenesulfonimidamide | CHIRALP AKIG, 20*250 mm, 5 um | IPA in Hex:DCM =5:1 (10 mM $NH_3$-MeOH) | 439 |
| 408 | 380b | 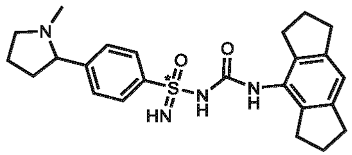 | (S, R) or (S, S) or (R, S) or (R, R)-N-((1,2,3,5,6,7-hexahydro-s-indacen-4-yl)carbamoyl)-4-(1-methylpyrrolidin-2-yl)benzenesulfonimidamide | CHIRALP AKIG, 20*250 mm, 5 um | IPA in Hex:DCM =5:1 (10 mM $NH_3$-MeOH) | 439 |
| 409 | 380c | 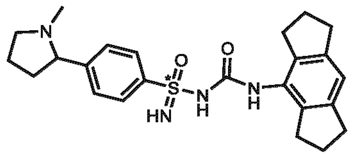 | (R, S) or (S, R) or (S, R) or (R, R)-N-((1,2,3,5,6,7-hexahydro-s-indacen-4-yl)carbamoyl)-4-(1-methylpyrrolidin-2-yl)benzenesulfonimidamide | CHIRALP AKIG, 20*250 mm, 5 um | IPA in Hex:DCM =5:1 (10 mM $NH_3$-MeOH) | 439 |
| 410 | 380d | 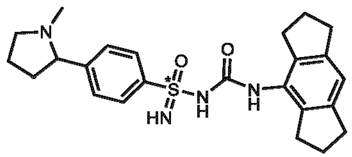 | (R, S) or (S, R) or (R,S or (S, S)-N-((1,2,3,5,6,7-hexahydro-s-indacen-4-yl)carbamoyl)-4-(1-methylpyrrolidin-2-yl)benzenesulfonimidamide | CHIRALP AKIG, 20*250 mm, 5 um | IPA in Hex:DCM =5:1 (10 mM $NH_3$-MeOH) | 439 |
| 411 | 384a | 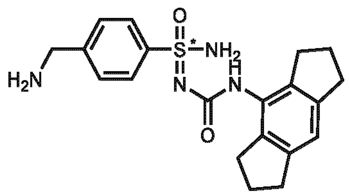 | (R) or (S) -4-(aminomethyl)-N'-((1,2,3,5,6,7-hexahydro-s-indacen-4-yl)carbamoyl) benzenesulfoni midamide | CHIRALP AK IG, 2*25 cm, 5 um | EtOH in Hex (8 mM $NH_3$-MeOH) | 385 |

(continued)

| Ex. # | Final Target Number | Structure | IUPAC Name | Column | Eluents | LC-MS $[M+H]^+$ |
|---|---|---|---|---|---|---|
| 412 | 384b | 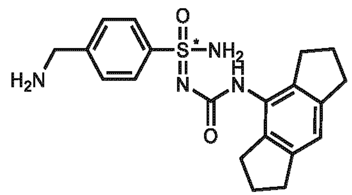 | (S) or (R) -4-(aminomethyl)-N'-((1,2,3,5,6,7-hexahydro-s-indacen-4-yl)carbamoyl) benzenesulfoni midamide | CHIRALP AK IG, 2*25 cm, 5 um | EtOH in Hex (8 mM $NH_3$-MeOH) | 385 |
| 413 | 357a | 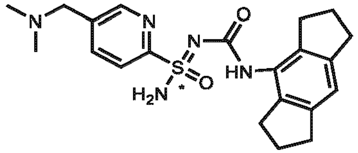 | (R) or (S) -5-((dimethylamino) methyl)-N'-((l,2,3,5,6,7-hexahydro-s-indacen-4-yl)carbamoyl)pyridine-2-sulfonimidamide | CHIRALP AK AD-H, 2.0.*25 cm | EtOH in Hex (8 mM $NH_3$-MeOH) | 414 |
| 414 | 357b | 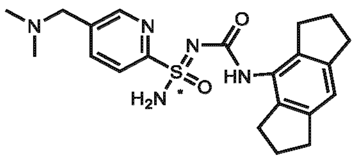 | (S) or (R) -5-((dimethylamino) methyl)-N'-((l,2,3,5,6,7-hexahydro-s-indacen-4-yl)carbamoyl)pyridine-2-sulfonimidamide | CHIRALP AK AD-H, 2.0.*25 cm | EtOH in Hex (8 mM $NH_3$-MeOH) | 414 |
| 415 | 354a | 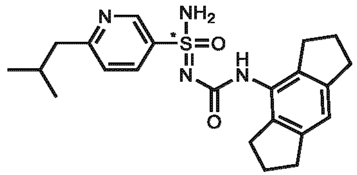 | (R) or (S) -N'-((1,2,3,5,6,7-hexahydro-s-indacen-4-yl)carbamoyl)-6-isobutylpyridine-3- sulfonimidamide | CHIRAL ART Cellulose-SB, 2*25 cm, 5 um | EtOH in Hex ( 0.1% DEA ) | 413 |
| 416 | 354b | 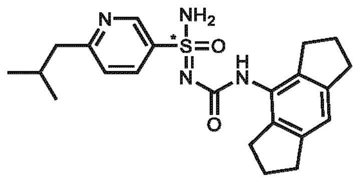 | (S) or (R) -N'-((1,2,3,5,6,7-hexahydro-s-indacen-4-yl)carbamoyl)-6-isobutylpyridine-3 - sulfonimidamide | CHIRAL ART Cellulose-SB, 2*25 cm, 5 um | EtOH in Hex ( 0.1% DEA ) | 413 |

(continued)

| Ex. # | Final Target Number | Structure | IUPAC Name | Column | Eluents | LC-MS $[M+H]^+$ |
|---|---|---|---|---|---|---|
| 417 | 387a | 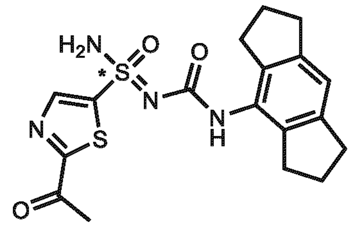 | (R) or (S) -2-acetyl-N'-((l,2,3,5,6,7-hexahydro-s-indacen-4-yl)carbamoyl) thiazole-5-sulfonimidamide | CHIRAL ART Cellulose-SB, 2*25 cm, 5 um | IPA in $CO_2$ | 405 |
| 418 | 387b | 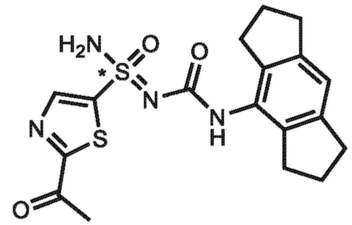 | (S) or (R) -2-acetyl-N'-((1,2,3,5,6,7-hexahydro-s-indacen-4-yl)carbamoyl) thiazole-5-sulfonimidamide | CHIRAL ART Cellulose-SB, 2*25 cm, 5 um | IPA in $CO_2$ | 405 |
| 419 | 333a | 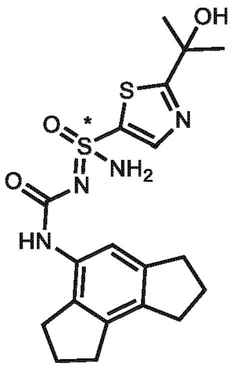 | (R) or (S) -N'-((1,2,3,6,7,8-hexahydro-as-indacen-4-yl)carbamoyl)-2-(2-hydroxypropan-2-yl)thiazole-5-sulfonimidamide | CHIRALP AK IF, 5*25 cm, 5 um | EtOH in Hex (8 mM $NH_3$-MeOH) | 421 |

(continued)

| Ex. # | Final Target Number | Structure | IUPAC Name | Column | Eluents | LC-MS [M+H]+ |
|---|---|---|---|---|---|---|
| 420 | 333b | 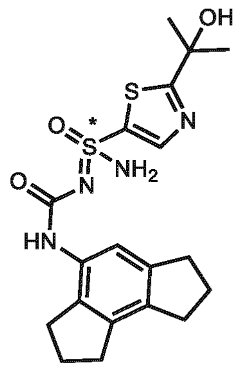 | (S) or (R) -N'-((1,2,3,6,7,8-hexahydro-as-indacen-4-yl)carbamoyl)-2-(2-hydroxypropan-2-yl)thiazole-5 -sulfonimidamide | CHIRALP AK IF, 5*25 cm, 5 um | EtOH in Hex (8 mM $NH_3$-MeOH) | 421 |
| 421 | 375a | 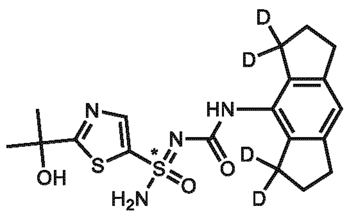 | (R) or (S) -N'-((1,2,3,5,6,7-hexahydro-s-indacen-4-yl-3,3,5,5-$d_4$)carbamoyl)-2-(2-hydroxypropan-2-yl)thiazole-5 -sulfonimidamide | CHIRALP AK IF, 2*25 cm, 5 um | MeOH (2 mM $NH_3$-MeOH) in $CO_2$ | 425 |
| 422 | 375b | 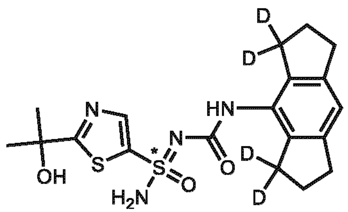 | (S) or (R) -N'-((1,2,3,5,6,7-hexahydro-s-indacen-4-yl-3,3,5,5-$d_4$)carbamoyl)-2-(2-hydroxypropan-2-yl)thiazole-5-sulfonimidamide | CHIRALP AK IF, 2*25 cm, 5 um | MeOH (2 mM $NH_3$-MeOH) in $CO_2$ | 425 |

(continued)

| Ex. # | Final Target Number | Structure | IUPAC Name | Column | Eluents | LC-MS $[M+H]^+$ |
|---|---|---|---|---|---|---|
| 423 | 376a | 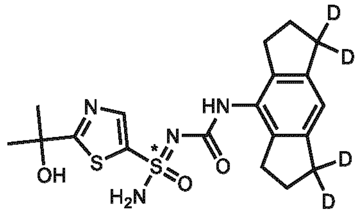 | (R) or (S) -N'-((1,2,3,5,6,7-hexahydro-s-indacen-4-yl-1, 1,7,7-$d_4$)carbamoyl)-2-(2-hydroxypropan-2-yl)thiazole-5-sulfonimidamide | CHIRALP AK ID, 2*25 cm (5 um) | MeOH (2 mM $NH_3$-MeOH) in $CO_2$ | 425 |
| 424 | 376b | 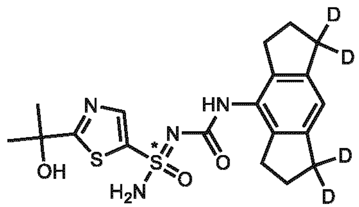 | (S) or (R) -N'-((1,2,3,5,6,7-hexahydro-s-indacen-4-yl-1,1,7,7-$d_4$)carbamoyl)-2-(2-hydroxypropan-2-yl)thiazole-5-sulfonimidamide | CHIRALP AK ID, 2*25 cm (5 um) | MeOH (2 mM $NH_3$-MeOH) in $CO_2$ | 425 |

# The amount of $NH_3$ in this chiral chromatographic solvent and similar solvents were adjusted by adding 2 M $NH_3$ in methanol to the desired $NH_3$ concentration. In this case, the resulting concentration of $NH_3$ in methanol is 8 mM.

Example 425 (Compound 318)

1-{Amino[5-(2-hydroxypropan-2-yl)-1,3-thiazol-2-yl]oxo-λ6-sulfanylidene}-3-(8-bromo-1,2,3,5,6,7-hexahydro-s-indacen-4-yl)urea

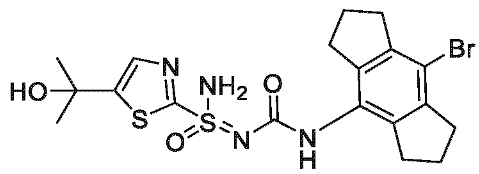

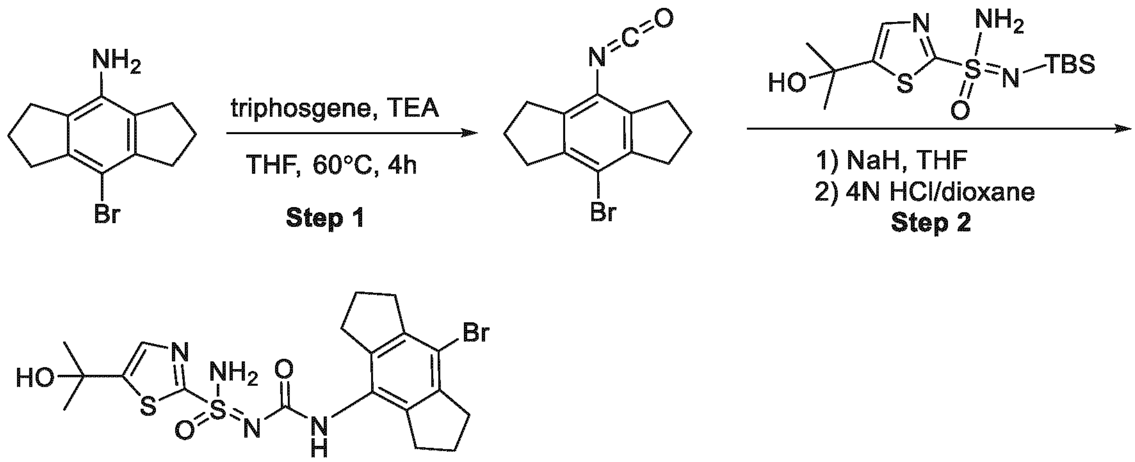


Step 1: **4-Bromo-1,2,3,5,6,7-hexahydro-8-isocyanato-s-indacene**

To a solution of 8-bromo-1,2,3,5,6,7-hexahydros-indacen-4-amine (1.5 g, 5.94 mmol) in anhydrous THF (50 mL) was added triethylamine (1.07 mL, 7.73 mmol) and triphosgene (882 mg, 2.97 mmol) at room temperature. The resulting mixture was then stirred at 60 °C for 4h. Reaction mixture was then brought to room temperature and used directly in the next step.

Step 2: **1-{Amino[5-(2-hydroxypropan-2-yl)-1,3-thiazol-2-yl]oxo-λ6-sulfanylidene}-3-(8-bromo-1,2,3,5,6,7-hexahydro-s-indacen-4-yl)urea**

To a solution of N-(tert-butyldimethylsilyl)-5-(2-hydroxypropan-2-yl)-1,3-thiazole-2-sulfonoimidamide (400 mg, 1.2 mmol) in anhydrous THF (10 mL) was added NaH (60% wt. oil dispersioin, 96 mg, 2.4 mmol) at room temperature. After 5 min, a solution of 4-bromo-1,2,3,5,6,7-hexahydro-8-isocyanato-s-indacene (2 mL, 2 mmol, from Step 1) was added drop wise. The resulting mixture was stirred at room temperature for 20 min before quenching carefully with 4 M HCl solution in dioxane (3 mL). Saturated aqueous ammonium chloride was added and the mixture was extracted with dichloromethane (15 mL x 3). The combined organic layers were washed with brine, dried over $MgSO_4$, filtered, and concentrated *in vacuo*. The crude product was purified by prep-HPLC to obtain the titled compound (280 mg, 47%). LCMS: $[M+H]^+$= 499.3.

**Example** 426 (Compound 313)

1-{Amino[5-(2-hydroxypropan-2-yl)-13-thiazol-2-yl]oxo-λ6-sulfanylidene}-3-[7-(3,4-dimethylphenyl)-2,3-dihydro-1H-inden-4-yl]urea

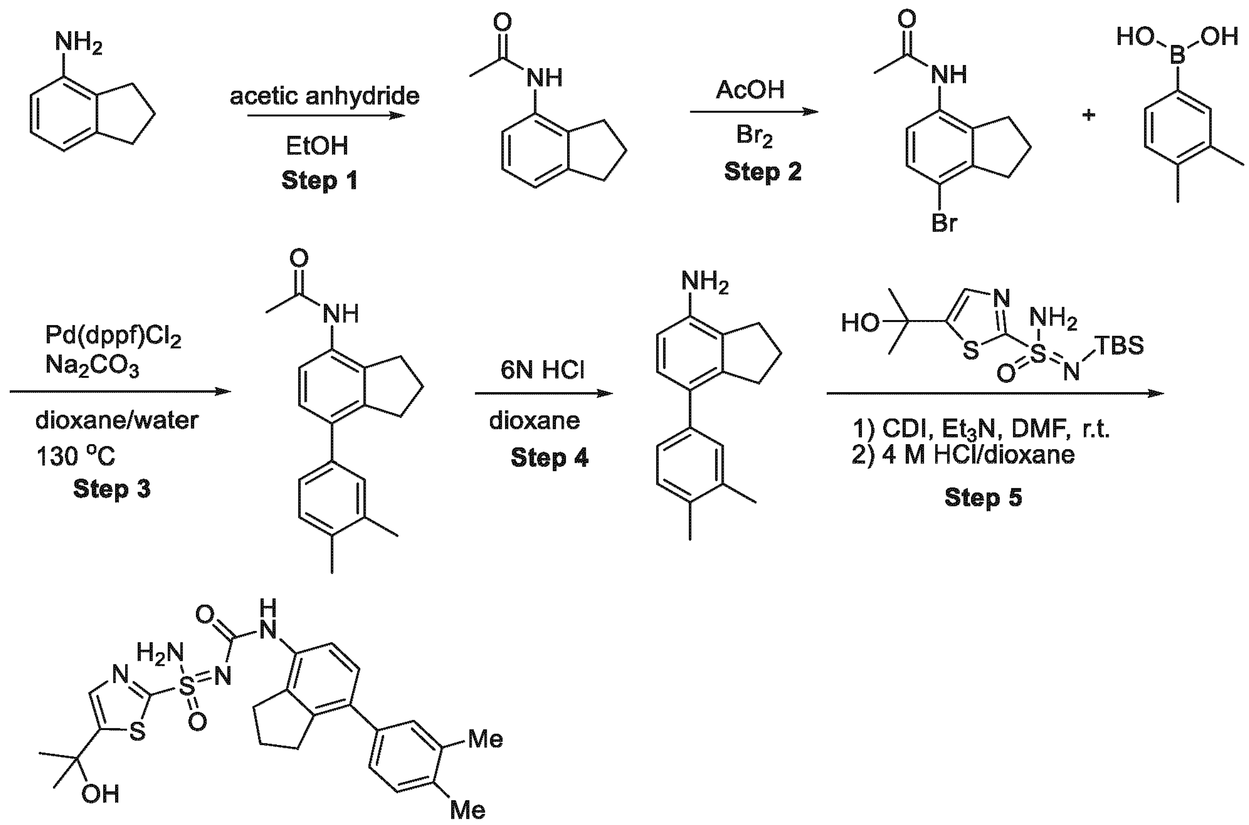


**Step 1: N-(2,3-dihydro-1H-inden-4-yl)acetamide**

To a solution of 2,3-dihydro-1H-inden-4-amine (3.4 g, 26 mmol) in ethanol (45 mL) was added a solution of acetic anhydride (4.9 mL, 52 mmol) in ethanol (15 mL) dropwise at 0 °C. The resulting mixture was gradually warmed up to RT and stirred for 15 h. Solvent was removed under reduced pressure and the residue was triturated with diethyl ether to afford titled compound as off white solid (3 g, 66%). LCMS $[M+H]^+$= 176.3.

**Step 2: N-(4-bromo-2,3-dihydro-1H-inden-7-yl)acetamide**

Into a 250-mL round-bottom flask was added N-(2,3-dihydro-1H-inden-4-yl)acetamide (3 g, 17.1 mmol) and acetic acid (45 mL). The resulting solution was cooled to 0 °C and then a solution of bromine (5.4 g, 34.2 mmol) in acetic acid (12 mL) was added dropwise with stirring over 10 min.

The cooling bath was removed and the reaction mixture was stirred at RT for 1h. Water was added and the resulting precipitates of product were collected by filtration and dried under vacuum to afford titled compound as off white solid (3.9 g, 90%). LCMS $[M+H]^+$= 254.4.

**Step 3: N-(2,3-dihydro-4-(3,4-dimethylphenyl)-1H-inden-7-yl)acetamide**

A mixture of N-(4-bromo-2,3-dihydro-1H-inden-7-yl)acetamide (1 g, 3.9 mmol), 3,4-dimethylphenylboronic acid (700 mg, 4.68 mmol), Pd(dppf)$Cl_2$.DCM (160 mg, 0.19 mmol), sodium carbonate (900 mg, 8.58 mmol as 2 M aqueous solution) in dioxane (12 mL) was stirred at 100 °C in an oil bath for 72 h. The reaction mixture was brought to RT, water (20 mL) was added and extracted with EtOAc (15 mL x 3). The combined organic layers were washed with brine, dried over $MgSO_4$, filtered, and concentrated *in vacuo.* The crude product was purified by silica gel flash chromatography using 0- 30% gradient of EtOAc in hexanes to afford titled compound (880 mg, 81%). LCMS $[M+H]^+$= 280.6.

**Step 4: 2,3-Dihydro-7-(3,4-dimethylphenyl)-1H-inden-4-amine**

A solution of N-(2,3-dihydro-4-(3,4-dimethylphenyl)-1H-inden-7-yl)acetamide (880 mg, 3.15 mmol) in 6 N HCl (20 mL) was stirred at 100 °C for 40 h. After consumption of the starting material, the reaction mixture was cooled to 0 °C and adjusted to pH = 8 with 10 M aqueous sodium hydroxide solution. The precipitates formed were collected, washed

with water and dried under vacuum to afford the titled compound (81 mg, 67%) as tan colored powder. LCMS $[M+H]^+$ = 238.3.

**Step 5: 1-{Amino[5-(2-hydroxypropan-2-yl)-1,3-thiazol-2-yl]oxo-λ⁶-sulfanylidene}-3-[7-(3,4-dimethylphenyl)-2,3-dihydro-1H-inden-4-yl] urea**

To a solution of N-(tert-butyldimethylsilyl)-5-(2-hydroxypropan-2-yl)-1,3-thiazole-2-sulfonoimidamide (42 mg, 0.13 mmol) in DMF (1 mL) was added $Et_3N$ (35 uL, 0.25 mmol) and the resulting mixture was stirred at room temperature for 10 min, followed by the addition of CDI (41 mg, 0.25 mmol). The reaction mixture was further stirred at RT for 1 h, and then 2,3-dihydro-7-(3,4-dimethylphenyl)-1H-inden-4-amine (30 mg, 0.13 mmol) was added. The resulting reaction mixture was stirred overnight at room temperature. The presence of desired product was then confirmed by LC-MS. The reaction mixture was quenched with 4 M HCl in dioxane (1 mL) and stirred for 30 min to de-protect the TBS group which indicated the formation of desired product on LCMS. The crude product was purified by preparative HPLC to provide titled compound (16.4 mg, 27%). LCMS $[M+H]^+$= 485.49.

**Example 427 (Compound 314)**

1-{Amino[5-(2-hydroxypropan-2-yl)-1,3-thiazol-2-yl]oxo-$\lambda^6$-sulfanylidene}-3-[8-(3,4-dimethylphenyl)-1,2,3,5,6,7-hexahydro-s-indacen-4-yl]urea

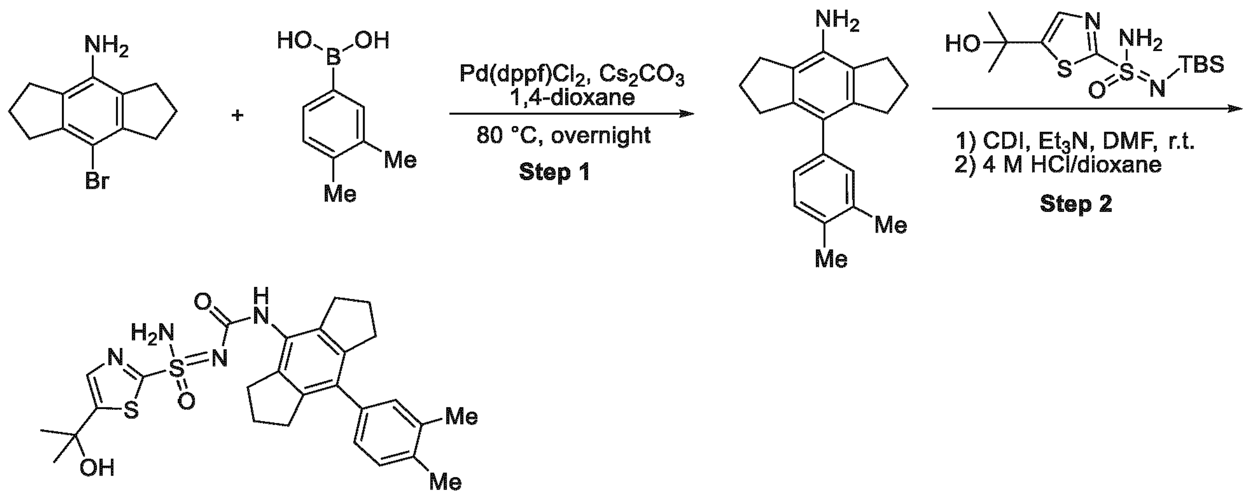


**Step 1: 1,2,3,5,6,7-Hexahydro-8-(3,4-dimethylphenyl)-s-indacen-4-amine**

8-Bromo-1,2,3,5,6,7-hexahydro-s-indacen-4-amine (105 mg, 0.42 mmol), 3,4-dimethylphenyl-boronic acid (187 mg, 1.25 mmol), $Pd(dppf)Cl_2$ (30.4 mg, 0.04 mmol) and dioxane (1.5 mL) were added to a reaction vial. Cesium carbonate (1.24 mL, 1 M in $H_2O$) was then added and the reaction mixture was stirred at 80 °C for 16 h. Reaction mixture was brought to RT and filtered through a small bed of Celite and rinsed with dioxane (5 mL). Water (5 mL) was added to the filtrates and extracted with diethyl ether (5 mL x 3). The combined organic layers were washed with brine, dried over anhydrous $MgSO_4$, filtered, and concentrated *in vacuo* to provide titled compound which was used in the next step without any purification. LCMS $[M+H]^+$= 278.4.

**Step 2: 1-{Amino[5-(2-hydroxypropan-2-yl)-1,3-thiazol-2-yl]oxo-$\lambda^6$-sulfanylidene}-3-[8-(3,4-dimethylphenyl)-1,2,3,5,6,7-hexahydro-s-indacen-4-yl]urea**

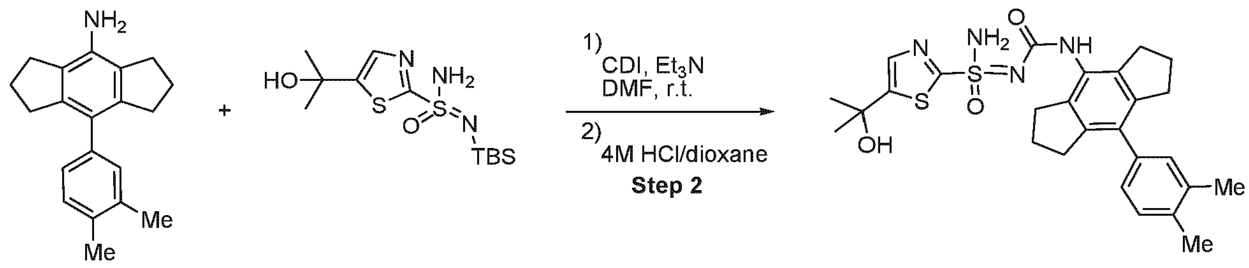


The title product was obtained using similar procedure as in Step 5 Example 426. LCMS: $[M+H]^+$ = 525.42.

**Example 428 (Compound 309)**

3-[Amino(dimethyl-1,3-thiazol-5-yl)oxo-λ$^6$-sulfanylidene]-1-[4-fluoro-2,6-bis(propan-2-yl)phenyl]urea

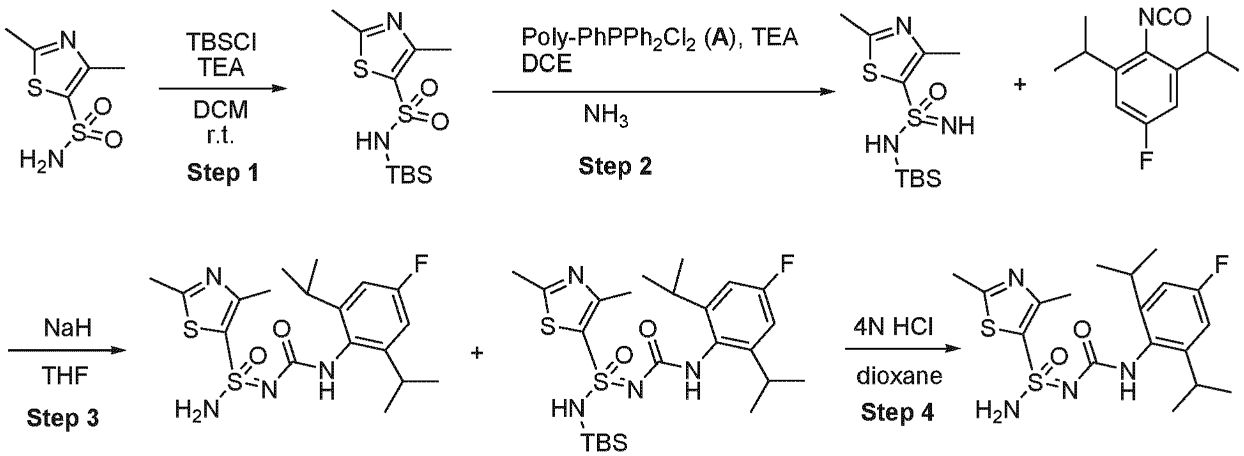


**Step 1: N-(tert-butyldimethylsilyl)-2,4-dimethyl-1,3-thiazole-5-sulfonamide**

Dimethyl-1,3-thiazole-5-sulfonamide (41.4 mg, 0.22 mmol) was dissolved in anhydrous $CH_2Cl_2$ (2 mL). Triethylamine (0.090 mL, 0.65 mmol) and TBSCl (38 mg, 0.25 mol) were added and the resulting mixture was stirred at 50 °C for 18 h. Reaction mixture was brought to RT and used directly in the next step. LCMS: $[M+H]^+$= 307.2.

**Step 2: N-(tert-butyldimethylsilyl)-2,4-dimethyl-1,3-thiazole-5-sulfonoimidamide**

Polymer bound dichlorotriphenylphosphorane reaction mixture (described for Reagent 2) was cooled in an ice/water bath under nitrogen. Triethylamine (0.1 mL, 0.72 mmol, 2.25 equiv.) was added slowly via syringe. Resulting mixture was stirred at 0 °C for 10 min and then the reaction mixture from Step 1 above was added dropwise via syringe. This reaction mixture was further stirred at 0 °C for 30 min and then a steady stream of anhydrous ammonia was bubbled into the reaction mixture for 3 min. Reaction vial was screw capped and stirred in ice/water bath for 2h. Reaction mixture was warmed up to room temperature, carefully opened and filtered to remove resin. The cloudy filtrate was centrifuged to remove any solids. Supernatant was concentrated *in vacuo* and dried under high vacuum for 1 h and used directly in the next step. LCMS: $[M+H]^+$= 306.8.

**Step 3: 3-{[(Tert-butyldimethylsilyl)amino](dimethyl-1,3-thiazol-5-yl)oxo-λ$^6$-sulfanylidene}-1- [4-fluoro-2,6-bis(propan-2-yl)phenyl] urea**

To the crude reaction mixture from Step 2 was added anhydrous THF (1.5 mL) and the resulting mixture was stirred in an ice/water bath for 5 min. NaH (17 mg, 0.44 mmol) was added and after 2 min of stirring a solution of isocyanate (0.165 mmol) in THF (3 ml) was added dropwise at 0 °C. The resulting mixture was brought to RT and stirred for 15 min to give a mixture of crude products. LCMS: $[M+H]^+$= 527.5; for de-protected product, $[M+H]^+$= 413.5.

**Step 4: 3-[amino(dimethyl-1,3-thiazol-5-yl)oxo-λ⁶-sulfanylidene]-1-[4-fluoro-2,6-bis(propan-2-yl)phenyl] urea**

To the reaction from Step 3 was carefully added 4N HCl in dioxane (0.3 mL) and the resulting mixture was stirred at RT for 30 min or till the completion of reaction as determined by the LCMS analysis ($[M+H]^+$= 413.5). Reaction mix was then concentrated *in vacuo.* DMSO (0.8 mL) was added to the residue and purified by prep-HPLC to afford titled compound (10 mg).

Examples in the following table were prepared using similar procedures described in Example 428.

**Table 29.**

| Example # | Final Target # | IUPAC Name | Structure | LCMS: $M+H^+$ |
|---|---|---|---|---|
| 428 | 309 | 3-[amino (dimethyl-1,3 -thiazol-5-yl) oxo-$\lambda^6$-sulfanylidene]-1-[4-fluoro-2,6-bis(propan-2-yl)phenyl]urea | 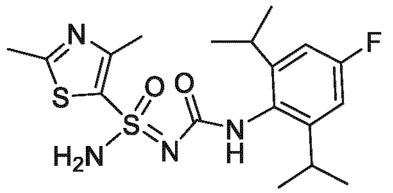 | 413.16 |
| 430 | 310 | 3-[amino({1-methyl-1H-pyrazolo [3,4-b]pyridin-5-yl})oxo-$\lambda^6$-sulfanylidene]-1-[4-fluoro-2,6-bis(propan-2-yl) phenyl]urea | 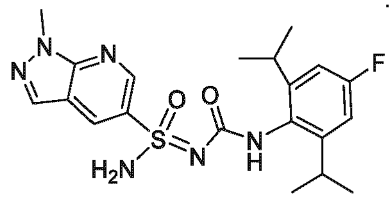 | 433.27 |
| 431 | 306 | 1-{amino[5-(dimethylamino) naphthalen-1-yl]oxo-$\lambda^6$-sulfanylidene}-3-[4-fluoro-2,6-bis (propan-2-yl)phenyl]urea | 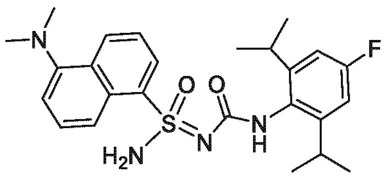 | 471.70 |

**Example 281 (Compound 378)**

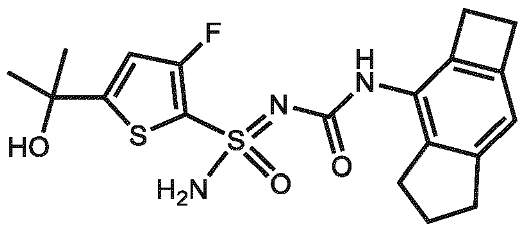

3-Fluoro-5-(2-hydroxypropan-2-yl)-N'-((2,4,5,6-tetrahydro-1H-cyclobuta[f]inden-3-vl)carbamoyl)thiophene-2-sulfonimidamide (Scheme 1)

**Examples 432 (Compound 378a) and Example 433 (Compound 378b)**

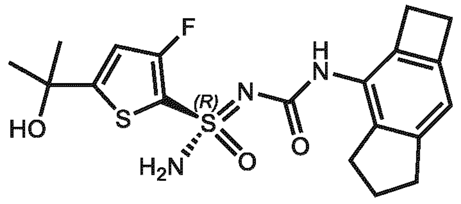 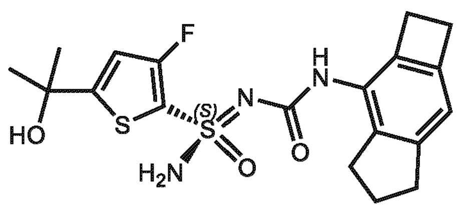

**Example 432** **Example 433**
**(stereochemistry tentatively assigned)**

(R)- and (S)-3-Fluoro-5-(2-hydroxypropan-2-yl)-N'-((2,4,5,6-tetrahydro-1H-cyclobuta[f]inden-3-yl) -carbamoyl)thiophene-2-sulfonimidamide

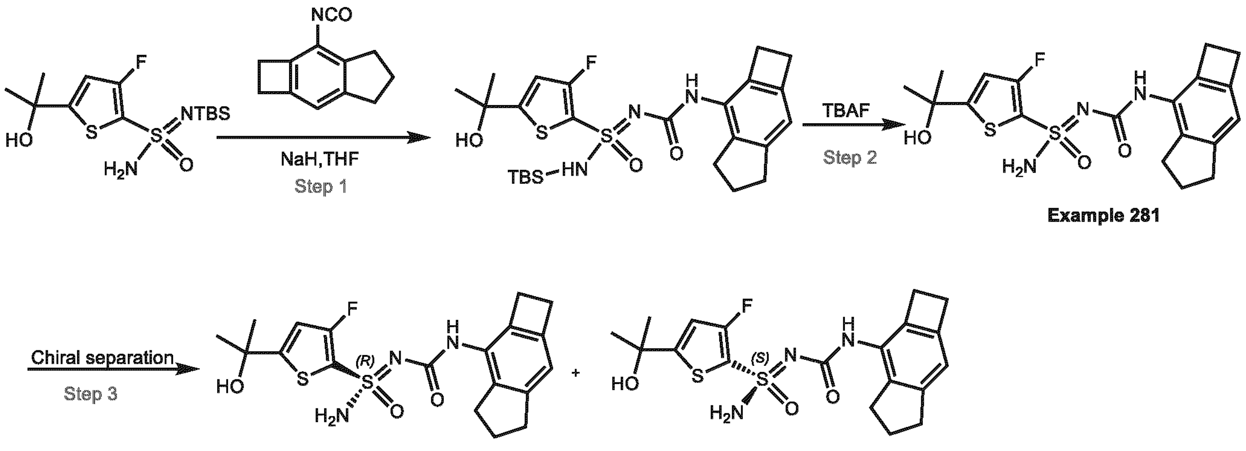


Example 281

Example 432 Example 433

**Step 1: N-(tert-butyldimethylsilyl)-3-fluoro-5-(2-hydroxypropan-2-yl)-N'-(2,4,5,6-tetrahydro-1H -cyclobuta[flinden-3-ylcarbamoyl)thiophene-2-sulfonimidamide**

To a stirred solution of N'-(tert-butyldimethylsilyl)-3-fluoro-5-(2-hydroxypropan-2-yl)thiophene-2 -sulfonimidamide (200 mg, 0.57 mmol) in THF (15 mL) in a 100 mL 3-necked round-bottom flask under nitrogen was added NaH (60% oil dispersion, 68 mg, 1.70 mmol) in one portion at 0 °C, then 3-isocyanato-2,4,5,6-tetrahydro-1H-cyclobuta[f]indene (105 mg, 0.57 mmol) in THF (2.0 mL) was added dropwise at 0 °C. The resulting solution was stirred for 2 h at RT. The reaction was then quenched by the addition of 10 mL of water. The resulting solution was extracted with 3x50 mL of EtOAc and the organic layers were combined and dried over anhydrous $Na_2SO_4$ and concentrated under reduced pressure. The crude product was purified by Prep-TLC (PE/EtOAc = 1:1). This resulted in 110 mg (36.1%) of the title compound as a yellow solid. MS-ESI: 538 (M+1).

**Step 2: 3-Fluoro-5-(2-hydroxypropan-2-yl)-N'-((2,4,5,6-tetrahydro-1H-cyclobuta[f]inden-3-yl) carbamoyl)thiophene-2-sulfonimidamide**

To a stirred solution of N-(tert-butyldimethylsilyl)-3-fluoro-5-(2-hydroxypropan-2-yl)-N'-(2,4,5,6- tetrahydro-1H-cyclobuta[f]inden-3-ylcarbamoyl)thiophene-2-sulfonimidamide (100 mg, 0.19 mmol) in THF (15 mL) in a 50-mL round-bottom flask was added TBAF (146 mg, 0.56 mmol) in one portion at RT. The resulting solution was stirred for 2 h at RT. The resulting mixture was concentrated under vacuum. The residue was eluted from silica gel with EtOAc/PE (3:2). The crude product was purified by Prep-HPLC with the following conditions: XBridge Prep C18 OBD Column, 19*150 mm*5um; mobile phase, Water (10 mM$NH_4HCO_3$+0.1%$NH_3H_2O$) and MeCN (25% to 41% Phase B over 7 min); Detector, UV 254/210 nm. This resulted in 60 mg (76.1%) of **Example 281** as a white solid. MS-ESI: 424 (M+1). $^1H$ NMR (400 MHz, DMSO-$d_6$) $\delta$ 8.31 (s, 1H), 7.69 (br s, 2H), 6.95 (s, 1H), 6.66 (s, 1H), 5.86 (s, 1H), 3.05-2.93 (m, 2H), 2.93-2.85 (m, 2H), 2.83-2.63 (m, 4H), 2.00-1.80 (m, 2H), 1.47 (s, 3H), 1.46 (s, 3H).

**Step 3: Chiral separation**

3-Fluoro-5-(2-hydroxypropan-2-yl)-N'-(2,4,5,6-tetrahydro-1H-cyclobuta[f]inden-3-ylcarbamoyl)thiophene-2-sulfonimidamide (60 mg) was resolved by Chiral HPLC with the following conditions: Column: CHIRALPAK IG, 20*250 mm, 5 um; Mobile Phase A: Hex (8 mM $NH_3$·MeOH), Mobile Phase B: IPA; Flow rate: 20 mL/min; Gradient: 30 B to 30 B in 13 min; 220/254 nm; Rt1: 6.2 min **(Example 432);** Rt2: 9.46 min **(Example 433).** This resulted in 15 mg of Example 432 and 16 mg of Example 433, both as white solids.

**Example 432:** MS-ESI: 424 (M+1). $^1$H NMR (400 MHz, DMSO-$d_6$) δ 8.32 (s, 1H), 7.79 (br s, 2H), 6.97 (s, 1H), 6.67 (s, 1H), 5.88 (s, 1H), 3.05-2.93 (m, 2H), 2.93-2.85 (m, 2H), 2.83-2.63 (m, 4H), 2.00-1.80 (m, 2H), 1.48 (s, 3H), 1.47 (s, 3H).

**Example 433:** MS-ESI: 424 (M+1). $^1$H NMR (400 MHz, DMSO-$d_6$) δ 8.30 (s, 1H), 7.80 (s, 2H), 6.97 (s, 1H), 6.67 (s, 1H), 5.86 (s, 1H), 3.05-2.93 (m, 2H), 2.93-2.85 (m, 2H), 2.85-2.65 (m, 4H), 2.00-1.80 (m, 2H), 1.48 (s, 3H), 1.47 (s, 3H).

**Table 37.** Examples in the following table were prepared using similar conditions as described in Example **281** and Scheme **1** from appropriate starting materials.

| Ex. # | Cmpd # | Structure | IUPAC Name | Exact Mass [M+H]+ |
|---|---|---|---|---|
| 434 | 858 | 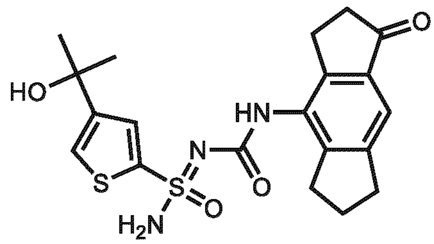 | 4-(2-Hydroxypropan-2-yl)-N'-((1-oxo-1,2,3,5,6,7- hexahydro-*s*-indacen-4-yl) carbamoyl)thiophene-2-sulfonimidamide | 434 |
| 435 | 837 | 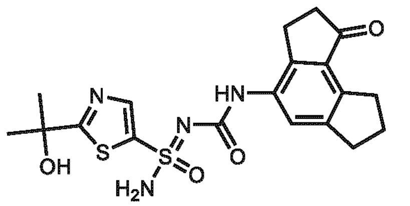 | 2-(2-hydroxypropan-2-yl)-N'-((1-oxo-1,2,3,6,7,8-hexahydro-*as*-indacen-4-yl) carbamoyl)thiazole-5-sulfonimidamide | 435 |
| 436 | 829 | 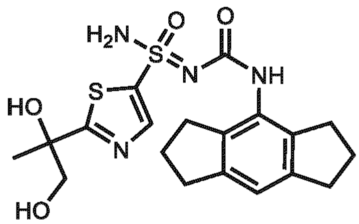 | 2-(1,2-Dihydroxypropan-2-yl)-N'-((1,2,3,5,6,7-hexahydro-*s*-indacen-4-yl)carbamoyl)thiazole-5-sulfonimidamide | 437 |
| 437 | 842 | 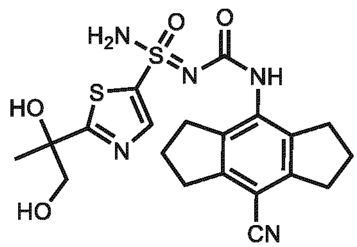 | N'-((8-cyano-1,2,3,5,6,7-hexahydro-*s*-indacen-4-yl)carbamoyl)-2-(1,2-dihydroxypropan-2-yl)thiazole-5-sulfonimidamide | 462 |

(continued)

| Ex. # | Cmpd # | Structure | IUPAC Name | Exact Mass [M+H]+ |
|---|---|---|---|---|
| 438 | 859 | 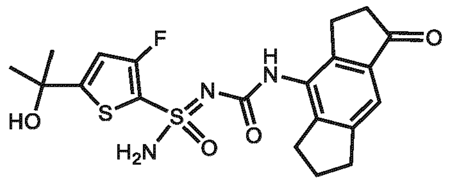 | 3-Fluoro-5-(2-hydroxypropan-2-yl)-N'-((1-oxo-1,2,3,5,6,7-hexahydro-*s*-indacen-4-yl)carbamoyl)thiophene-2-sulfonimidamide | 452 |
| 439 | 873 | 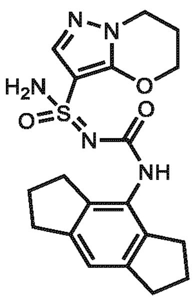 | N-(amino(6,7-dihydro-5H-pyrazolo [5,1-b][1,3]oxazin-3-yl)(oxo)-λ6-sulfaneylidene)-1,2,3,5,6,7-hexahydro-*s*-indacene-4-carboxamide | 387 |
| 440 | 834 | 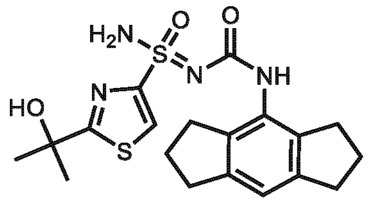 | N'-((1,2,3,5,6,7-hexahydro-*s*-indacen-4-yl)carbamoyl)-2-(2-hydroxypropan-2-yl)thiazole-4-sulfonimidamide | 421 |
| 441 | 869c | 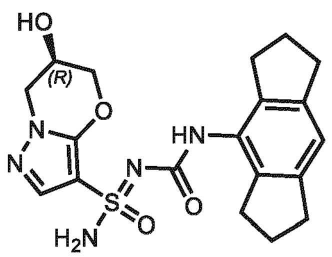 | (6R)-N'-((1,2,3,5,6,7-hexahydro-*s*-indacen-4-yl)carbamoyl)-6-hydroxy-6,7-dihydro-5H-pyrazolo[5,1-b][1,3] oxazine-3-sulfonimidamide | 418 |
| 442 | 817 | 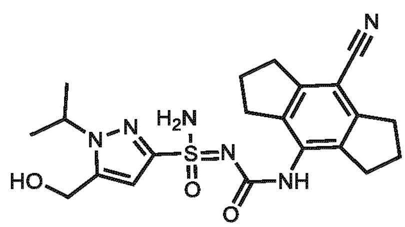 | N'-((8-cyano-1,2,3,5,6,7-hexahydro-*s*-indacen-4-yl)carbamoyl)-5-(hydroxymethyl)-1-isopropyl-1H-pyrazole-3-sulfonimidamide | 443 |
| 443 | 411 | 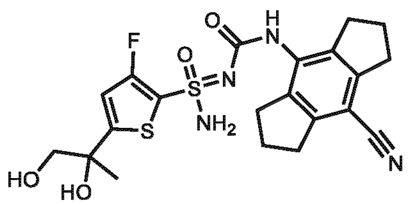 | N'-((8-cyano-1,2,3,5,6,7-hexahydro-s-indacen-4-yl)carbamoyl)-5-(1,2-dihydroxypropan-2-yl)-3-fluorothiophene-2-sulfonimidamide | 479 |

**Example 444 (Compound 802)**

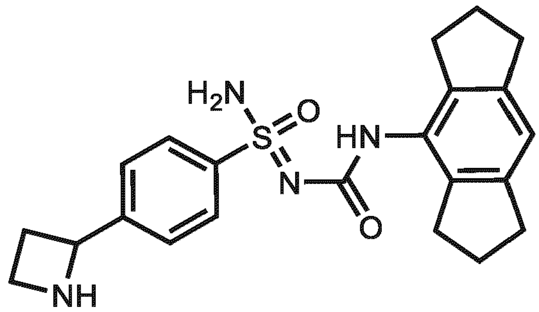

4-(Azetidin-2-yl)-N'-((1,2,3,5,6,7-hexahydro-s-indacen-4-yl)carbamoyl)benzenesulfonimidamide (Scheme I)

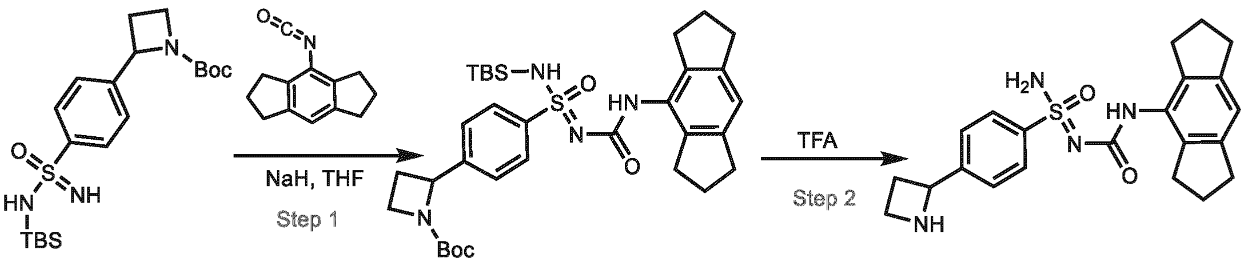


Example 444

**Step 1: *Tert*-butyl 2-(4-(N-(tert-butyldimethylsilyl)-N'-((1,2,3,5,6,7-hexahydro-s-indacen-4-yl) carbamoyl)sulfamidimidoyl)phenyl)azetidine-1-carboxylate**

To a stirred solution of tert-butyl 2-(4-(N-(tertbutyldimethylsilyl)sulfamidimidoyl)phenyl)azetidine-1- carboxylate (80 mg, 0.19 mmol) in THF (5 mL) in a 100 mL 3 necked round-bottom flask under nitrogen was added NaH (60% oil dispersion, 15.2 mg, 0.38 mmol) in one portion at 0 °C, 4-isocyanato-1,2,3,5,6,7-hexahydro-s-indacene (39.3 mg, 0.19 mmol) in THF (2 mL) was added dropwise at 0 °C. The resulting solution was stirred for 2 h at RT. The reaction was then quenched by the addition of 5 mL of water. The resulting solution was extracted with 3x50 mL of EtOAc. The organic layers were combined and dried over anhydrous $Na_2SO_4$ and concentrated under reduced pressure. The residue was eluted from silica gel with EtOAc/PE (1/1). This resulted in 90 mg (76.6%) of the title compound as a yellow solid. MS-ESI: 625 (M+1)

**Step 2: 4-(Azetidin-2-yl)-N'-((1,2,3,5,6,7-hexahydro-s-indacen-4-yl)carbamoylbenzenesulfonimidamide**

To a stirred solution of tert-butyl 2-(4-(N-(tert-butyldimethylsilyl)-N'-((1,2,3,5,6,7-hexahydro-s-indacen-4- yl)carbamoyl)sulfamidimidoyl)phenyl)azetidine-1-carboxylate (90 mg, 0.14 mmol) in DCM (10 mL) in a 25-mL round-bottom flask was added TFA (2 mL) dropwise at 0 °C. The resulting solution was stirred for 10 min at 0 °C. The resulting mixture was concentrated under reduced pressure below 30 °C. The crude product was purified by Prep-HPLC with the following conditions: XBridge Prep OBD C18 Column, 30*150 mm 5 um; mobile phase, water (10 mM $NH_4HCO_3$+0.1%$NH_3 \cdot H_2O$) and MeCN (20% to 40% Phase B over 7 min); Detector, UV 254/220 nm. This resulted in 6.10 mg (10.3%) of **Example 444** as a white solid. MS-ESI: 411 (M+1).

**Example 445 (Compound 870a)**

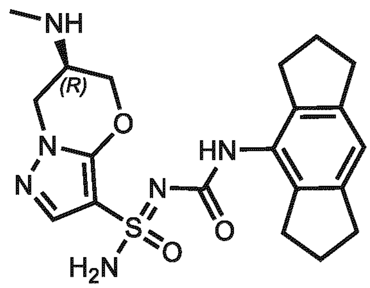

(6R)-*N'*-((1,2,3,5,6,7-hexahydro-s-indacen-4-yl)carbamoyl)-6-(methylamino)-6,7-dihydro-5H-pyrazolo[5,1-b][1,3]oxazine-3-sulfonimidamide (Scheme II)

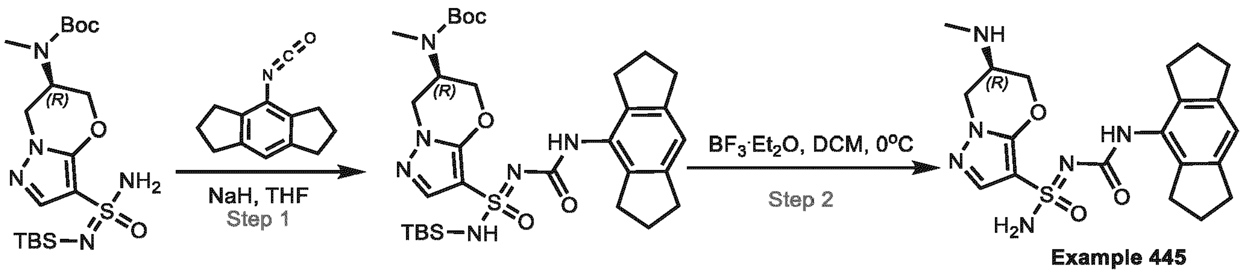


**Example 445**

**Step 1: Tert-butyl (R)-3-(N-(tert-butyldimethylsilyl)-N'-(1,2,3,5,6,7-hexahydro-s-indacen-4-ylcarbamoyl)sulfamimidoyl)-6,7-dihydro-5H-pyrazolo[5,1-b] [1,3]oxazin-6-yl(methyl)carbamate**

To a stirred solution of tert-butyl (R)-3-(N'-(tert-butyldimethylsilyl)sulfamimidoyl)-6,7-dihydro-5H- pyrazolo[5,1-b][1,3]oxazin-6-yl-(methyl)carbamate (1.10 g, 3.31 mmol) in THF (50 mL) in a 100-mL 3 necked round-bottom flask under nitrogen was added NaH (60% oil dispersion, 397 mg, 9.93 mmol) in portions at 0 °C. The resulting solution was stirred for 20 min at RT. To this was added 4-isocyanato-1,2,3,5,6,7-hexahydro-s-indacene (1.32 g, 6.62 mmol) in THF (5 mL) dropwise at 0 °C. The resulting solution was allowed to react, with stirring, for an additional 16 h at RT. The reaction was then quenched by the addition of 20 mL of water. The resulting solution was extracted with 3x30 mL of EtOAc. The organic layers were combined and dried over anhydrous $Na_2SO_4$ and concentrated under reduced pressure. The residue was eluted from silica gel with EtOAc/PE (2:3). This resulted in 556 mg (35%) of the title compound as an off-white solid. MS-ESI: 645 (M+1).

**Step 2: (6R)-N'-(1,2,3,5,6,7-hexahydro-s-indacen-4-ylcarbamoyl)-6-(methylamino)-6,7-dihydro-5H- pyrazolo[5,1-b][1,3]oxazine-3-sulfonimidamide**

To a stirred solution of tert-butyl (R)-3-(N-(tert-butyldimethylsilyl)-N'- (1,2,3,5,6,7-hexahydro-s-indacen-4-ylcarbamoyl)sulfamimidoyl)-6,7-dihydro-5H-pyrazolo[5,1-b][1,3]oxazin-6-yl(methyl)carbamate (280 mg, 0.43 mmol) in DCM (10 mL) in a 50-mL round-bottom flask was added $BF_3.Et_2O$ (47%wt., 289 mg, 2.0 mmol) dropwise at 0 °C. The resulting solution was stirred for 2 h at 0 °C. The reaction was then quenched by the addition of 0.5 mL of sat. $NaHCO_3$ (aq). The resulting mixture was concentrated under reduced pressure. The crude product was purified by Prep-HPLC with the following conditions: XBridge Prep OBD C18 Column, 30*150 mm, 5um; mobile phase, water (10 mM $NH_4HCO_3$+0.1%$NH_3 \cdot H_2O$) and MeCN (10% to 33% Phase B over 7 min); Detector, UV (254/220 nm). This resulted in 80 mg (40.9%) of **Example 445** as an off-white solid. MS-ESI: 431 (M+1). $^1$H NMR (400 MHz, DMSO-$d_6$) $\delta$ 8.18 (br s, 1H), 7.52 (s, 1H), 7.25 (br s, 2H), 6.86 (s, 1H), 4.40-4.32 (m, 1H), 4.32-4.19 (m, 2H), 4.00-3.90 (m, 1H), 3.25-3.10 (m, 1H), 2.78 (t, *J* = 7.4 Hz, 4H), 2.75 - 2.60 (m, 4H), 2.35 (d, *J* = 2.2 Hz, 3H), 2.00 - 1.88 (m, 4H).

**Example 446 (Compound 846)**

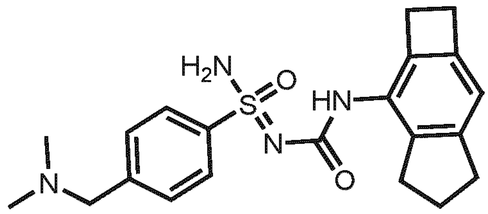

4-((Dimethylamino)methyl)-N'-(2,4,5,6-tetrahydro-1H-cyclobuta[f]inden-3-ylcarbamoyl)benzenesulfonimidamide (Scheme 3)

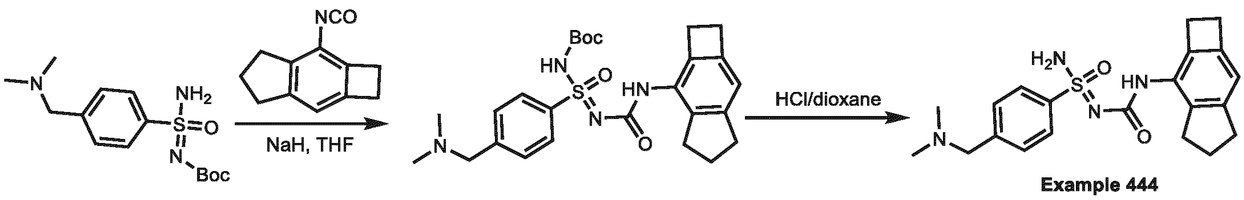


**Step 1: Tert-butyl 4-((dimethylamino)methyl)-N-(2,4,5,6-tetrahydro-1H-cyclobuta[fJinden-3- ylcarbamoyl)phenylsulfonimidoylcarbamate**

To a stirred solution of tert-butyl (amino(4-((dimethylamino)methyl)phenyl)(oxo)-$\lambda^6$-sulfaneylidene)carbamate (100 mg, 0.32 mmol) in THF (5.0 mL) a 100 mL 3 necked round-bottom flask under nitrogen was added NaH (60% oil dispersion, 25.6 mg, 0.64 mmol) in one portion at 0 °C, then 3-isocyanato-2,4,5,6-tetrahydro-1H-cyclobuta[fJindene (60 mg, 0.32 mmol) in THF (5 mL) was added dropwise at 0 °C. The resulting solution was stirred for 2 h at RT. The reaction was then quenched by the addition of 10 mL of water. The resulting solution was extracted with 3x100 mL of EtOAc and the organic layers were combined and dried over anhydrous $Na_2SO_4$ and concentrated under reduced pressure. This resulted in 60 mg (37.7%) of the title compound as yellow oil. MS-ESI: 499 (M+1).

**Step 2: 4-((Dimethylamino)methyl)-N'-((2,4,5,6-tetrahydro-1H-cyclobuta[f]inden-3-yl)carbamoyl) benzenesulfonimidamide**

To a stirred solution of tert-butyl 4-((dimethylamino)methyl)-N-(2,4,5,6 -tetrahydro-1H-cyclobuta[f]inden-3-ylcarbamoyl)phenylsulfonimidoylcarbamate (60 mg, 0.12 mmol) in THF (5 mL) in a 50-mL round-bottom flask was added HCl in 1,4-dioxane (4 M, 2.0 mL) dropwise at 0 °C. The resulting solution was stirred for 30 min at RT. The resulting mixture was concentrated under reduced pressure. The crude product was purified by Prep-HPLC with the following conditions: XBridge Shield RP18 OBD Column, 19*250 mm, 10 um; Mobile Phase A: water (10 mM $NH_4HCO_3$), Mobile Phase B: MeCN; Flow rate: 25 mL/min; Gradient: 46% B to 47% B in 7 min; UV 210/254 nm. This resulted in 22.8 mg (45.1%) of **Example 446** as a white solid. MS-ESI: 399 (M+1). $^1H$ NMR (400 MHz, MeOH-$d_4$) $\delta$ 7.98 (d, $J$ = 8.3 Hz, 2H), 7.53 (d, $J$ = 8.3 Hz, 2H), 6.65 (s, 1H), 3.61 (s, 2H), 3.01 (s, 2H), 2.92 (s, 2H), 2.84 (t, $J$ = 7.4 Hz, 2H), 2.75 (t, $J$ = 7.3 Hz, 2H), 2.29 (s, 6H), 2.06-1.90 (m, 2H).

**Table 38.** Examples in the following table were prepared using similar conditions as described in Example **446** and Scheme 3 from appropriate starting materials.

| Ex. # | Cmpd # | Structure | IUPAC Name | Exact Mass [M+H]+ |
|---|---|---|---|---|
| 447 | 847 | 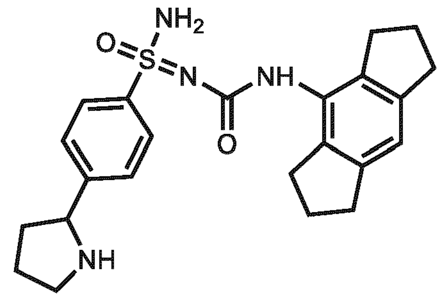 | N'-((1,2,3,5,6,7-hexahydro-s-indacen-4-yl)carbamoyl)-4-(pyrrolidin-2-yl) benzenesulfonimidamide | 425 |
| 448 | 814 | 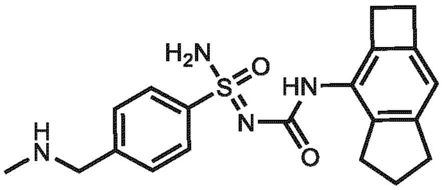 | 4-((Methylamino)methyl)-N'-((2,4,5,6-tetrahydro-1H-cyclobuta[f]inden-3-yl) carbamoyl)benzenesulfonimidamide | 385 |
| 449 | 845 | 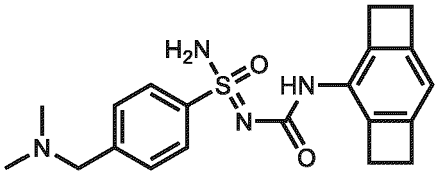 | 4-((Dimethylamino) methyl)-N'-(tricyclo [6.2.0.03,6] deca-1, 3 (6),7-trien-2-ylcarbamoyl) benzenesulfonimidamide | 385 |
| 450 | 857 | 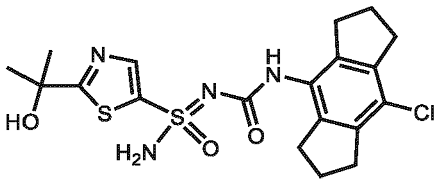 | N'-((8-chloro-1,2,3,5,6,7-hexahydro-s-indacen-4-yl)carbamoyl)-2-(2-hydroxypropan-2-yl)thiazole-5-sulfonimidamide | 455/457 |

**Example 451 (Compound 853)**

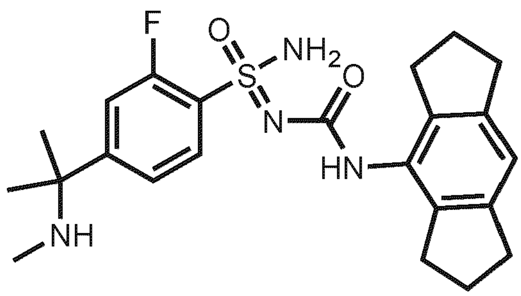

2-Fluoro-N'-(1,2,3,5,6,7-hexahydros-indacen-4-ylcarbamoyl)-4-(2-(methylamino)propan-2-yl)benzenesulfonimidamideonimidamide (Scheme III)

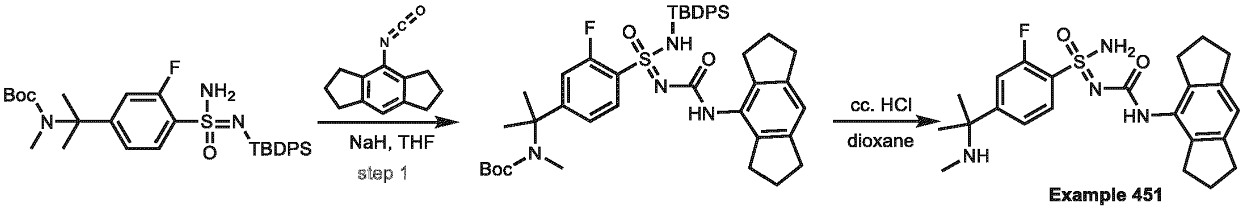

Example 451

**Step 1: *Tert*-butyl 2-(4-(N-(tert-butyldiphenylsilyl)-N'-(1,2,3,5,6,7-hexahydro-s-indacen-4-ylcarbamoyl) sulfamimidoyl)-3-fluorophenyl)propan-2-yl(methyl)carbamate**

To a stirred solution of tert-butyl 2-(4-(N'-(tert-butyldiphenylsilyl)sulfamimidoyl)-3-fluorophenyl)propan-2-yl(methyl) carbamate (400 mg, 0.69 mmol) in THF (10 mL) in a 100-mL round-bottom flask under nitrogen was added NaH (60% oil dispersion, 82.2 mg, 2.06 mmol) in portions at 0 °C, then 4-isocyanato-1,2,3,5,6,7-hexahydro-s-indacene (136 mg, 0.685 mmol) in THF (5 mL) was added dropwise at 0 °C. The resulting solution was stirred for 2 h at RT. The reaction was then quenched by the addition of 10 mL of water. The resulting solution was extracted with 3x30 mL of EtOAc. The organic layers were combined and dried over anhydrous $Na_2SO_4$ and concentrated under reduced pressure. This resulted in 400 mg (74.6%) of the title compound as a yellow solid. MS-ESI: 783 (M+1).

**Step 2: 2-Fluoro-N'-(1,2,3,5,6,7-hexahydro-s-indacen-4-ylcarbamoyl)-4-(2-(methylamino)propan-2-yl) benzenesulfonimidamide**

To a stirred solution of tert-butyl 2-(4-(N-(tert-butyldiphenylsilyl)-N'-(1,2,3,5,6,7-hexahydro-s-indacen- 4-ylcarbamoyl)sulfamimidoyl)-3 -fluorophenyl)propan-2-yl(methyl)carbamate (80 mg, 0.1 mmol) in dioxane (5 mL) a 25-mL round-bottom flask under nitrogen was added cc. HCl (0.09 mL, 1.02 mmol) dropwise at 0 °C. The resulting solution was stirred for 30 min at RT. The pH value of the solution was adjusted to 7 with sat. $NaHCO_3$ solution. The resulting solution was extracted with 3x20 mL of EtOAc. The organic layers were combined and dried over anhydrous $Na_2SO_4$ and concentrated under reduced pressure. The crude product was purified by Prep-HPLC with the following conditions: XBridge Prep C18 OBD Column, 198*150 mm, 5um; mobile phase, water (10 mM $NH_4HCO_3$+0.1%$NH_3 \cdot H_2O$) and MeCN (28% to 49% Phase B over 7 min); Detector, UV 254 nm. This resulted in 11.8 mg (25.9%) of **Example 451** as a white solid. MS-ESI: 445 (M+1). $^1$H NMR (400 MHz, DMSO-$d_6$) δ 8.30 (br s, 1H), 7.76 (dd, *J* = 8.0, 8.0 Hz, 1H), 7.55 (br s, 2H), 7.43 (dd, *J* = 12.5, 1.6 Hz, 1H), 7.39 (dd, *J* = 8.3, 1.8 Hz, 1H), 6.85 (s, 1H), 2.75 (t, *J* = 7.4 Hz, 4H), 2.62 (t, *J* = 7.6 Hz, 4H), 1.99 (s, 3H), 1.95-1.80 (m, 4H), 1.34 (s, 3H), 1.33 (s, 3H).

**Table 39.** Examples in the following table were prepared using similar conditions as described in Example **451** and Scheme **III** from appropriate starting materials.

| Ex. # | Cmpd # | Structure | IUPAC Name | Exact Mass [M+H]+ |
|---|---|---|---|---|
| 452 | 826 | 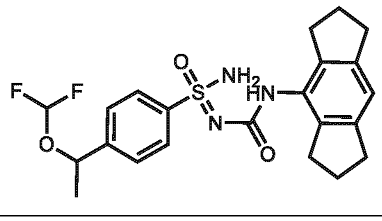 | 4-(1-(Difluoromethoxy) ethyl)-N'-((1,2,3,5,6,7-hexahydro-s-indacen-4-yl)carbamoyl) benzenesulfonimidamide | 450 |

**Example 453 (Compound 840)**

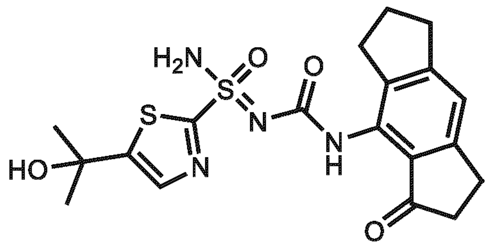

5-(2-Hydroxypropan-2-yl)-N'-((3-oxo-1,2,3,5,6,7-hexahydro-s-indacen-4-yl)carbamoyl)thiazole-2-sulfonimidamide (Scheme IV)

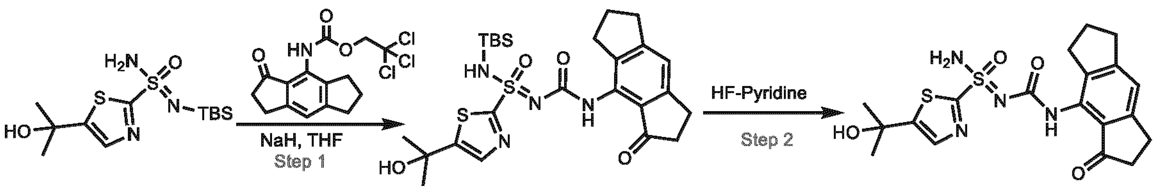


**Step 1: N-(tert-butyldimethylsilyl)-5-(2-hydroxypropan-2-yl)-N'-((3-oxo-1,2,3,5,6,7-hexahydro-s -indacen-4-yl)carbamoyl)thiazole-2-sulfonimidamide**

To a stirred solution of N'-(tert-butyldimethylsilyl)-5-(2-hydroxypropan-2-yl)thiazole-2-sulfonimidamide (1.40 g, 4.17 mmol) in THF (20 mL) in a 100 mL 3 necked round-bottom flask under nitrogen was added NaH (60% oil dispersion, 333 mg, 8.34 mmol) in portions at 0 °C, then 2,2,2-trichloroethyl (3-oxo-1,2,3,5,6,7-hexahydro-s-indacen-4-yl)carbamate (1.82 g, 5.01 mmol) in THF (10 mL) was added dropwise at 0 °C. The resulting solution was stirred for 2 h at RT. The reaction was then quenched by the addition of $Na_2SO_4 \cdot 10H_2O$ (1.6 g, 5.0 mmol). The solids were filtered out. The resulting mixture was concentrated under vacuum. This resulted in 3.0 g (crude) of the title compound as a light yellow crude oil. MS-ESI: 549 (M+1)

**Step 2: 5-(2-Hydroxypropan-2-yl)-N'-((3-oxo-1,2,3,5,6,7-hexahydro-s-indacen-4-yl)carbamoyl) thiazole-2-sulfonimidamide**

To a stirred solution of N-(tert-butyldimethylsilyl)-5-(2-hydroxypropan-2-yl)-N'- ((3-oxo-1,2,3,5,6,7-hexahydro-s-indacen-4-yl)carbamoyl)thiazole-2-sulfonimidamide (1.40 g, 2.55 mmol, crude) in DCM (10 mL) in a 100-mL round-bottom flask was added HF-Pyridine (70% wt., 73 mg, 2.55 mmol). The resulting solution was stirred for 16 h at RT. The solids were filtered out. The resulting mixture was concentrated under vacuum. The crude product was purified by Prep-HPLC with the following conditions: XBridge Prep OBD C18 Column, 30*150 mm, 5um; mobile phase, water (10 mM $NH_4HCO_3$) and MeCN (22% to 24% Phase B over 7 min); Detector, UV 220 nm. This resulted in 540 mg (28.8% over 2 steps) of Example 453 as a white solid. MS-ESI: 435 (M+1). $^1H$ NMR (400 MHz, MeOH-$d_4$) $\delta$ 7.78 (s, 1H), 7.13 (s, 1H), 3.07-3.04 (m, 2H), 2.91 (t, $J$ = 7.3 Hz, 2H), 2.82-2.78 (m, 2H), 2.70- 2.68 (m, 2H), 2.04-2.01 (m, 2H), 1.66 (s, 6H).

**Table 40.** Examples in the following table were prepared using similar conditions as described in Example **453** and Scheme **IV** from appropriate starting materials.

| Ex. # | Cmpd # | Structure | IUPAC Name | Exact Mass [M+H]+ |
|---|---|---|---|---|
| 454 | 805 | 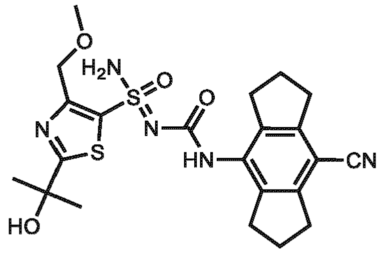 | N'-((8-cyano-1,2,3,5,6,7-hexahydro-s-indacen-4-yl)carbamoyl)-2-(2-hydroxypropan-2-yl)-4-(methoxymethyl) thiazole-5-sulfonimidamide | 490 |
| 455 | 851 | 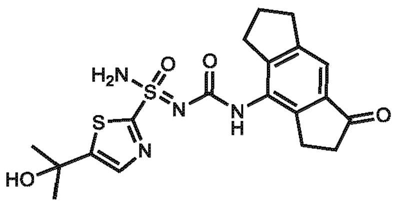 | 5-(2-Hydroxypropan-2-yl)-N'-((1-oxo-1,2,3,5,6,7-hexahydro-s-indacen-4-yl) carbamoyl)thiazole-2-sulfonimidamide | 435 |
| 456 | 818 | 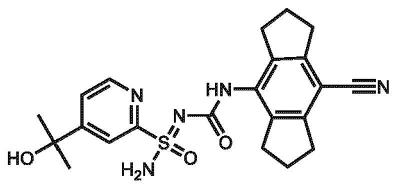 | N'-((8-cyano-1,2,3,5,6,7-hexahydro-s-indacen-4-yl)carbamoyl)-4-(2-hydroxypropan-2-yl)pyridine-2-sulfonimidamide | 440 |
| 457 | 816 | 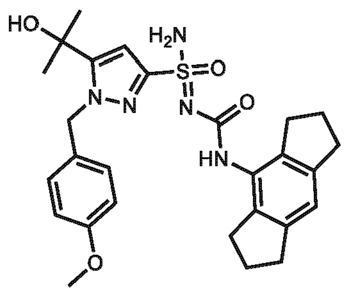 | N'-((1,2,3,5,6,7-hexahydro-s-indacen-4-yl)carbamoyl)-5-(2-hydroxypropan-2-yl)-1-(4-methoxybenzyl)-1H-pyrazole-3-sulfonimidamide | 524 |

**Example 458 (Compound 828)**

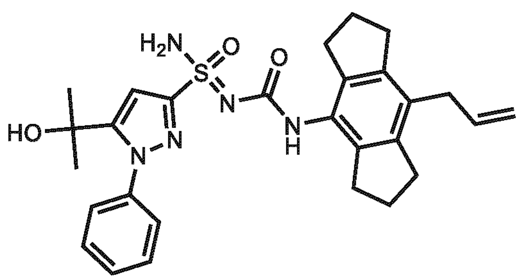

N'-((8-allyl-1,2,3,5,6,7-hexahydro-s-indacen-4-yl)carbamoyl)-5-(2-hydroxypropan-2-yl)-1-phenvl-1H-pvrazole-3-sulfonimidamide (Scheme 2)

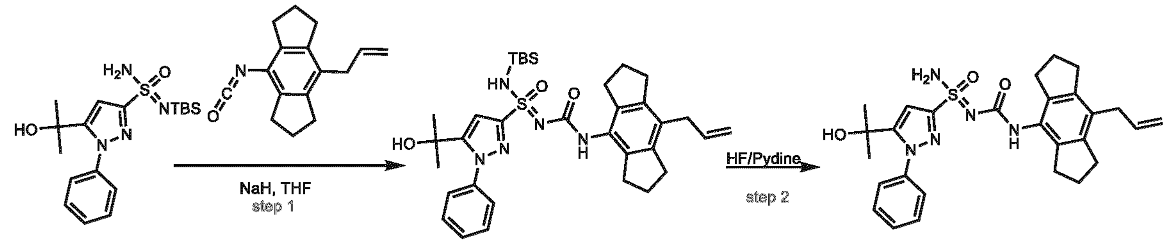


Example 458

**Step 1: N'-((8-allyl-1,2,3,5,6,7-hexahydro-s-indacen-4-yl)carbamoyl)-N-(tertbutyldimethylsilyl)-5- (2-hydroxypropan-2-yl)-1-phenyl-1H-pyrazole-3-sulfonimidamide**

To a stirred solution of N'-(tert-butyldimethylsilyl)-5-(2-hydroxypropan-2-yl)-1-phenyl-1H-pyrazole-3 -sulfonimidamide (82.4 mg, 0.21 mmol) in THF (5. 0 mL) in a 100 mL 3 necked round-bottom flask under nitrogen was added NaH (60% oil dispersion, 25.0 mg, 0.63 mmol) in one portion at 0 °C. The resulting solution was stirred for 30 min at 0 °C. To the above mixture was added 4-allyl-8-isocyanato-1,2,3,5,6,7-hexahydro-s-indacene (50.0 mg, 0.21 mmol) at 0 °C. The resulting mixture was stirred for additional 4 h at RT under nitrogen atmosphere. The reaction was quenched with 5 mL of water at 0 °C. The resulting mixture was extracted with EtOAc (3 x 15 mL). The combined organic layers were washed with brine (2x15 mL), dried over anhydrous $Na_2SO_4$. After filtration, the filtrate was concentrated under reduced pressure. This resulted in 112 mg (85%) crude title compound as a yellow solid. MS-ESI: 634 (M+1).

**Step 2: N'-((8-allyl-1,2,3,5,6,7-hexahydro-s-indacen-4-yl)carbamoyl)-5-(2-hydroxypropan-2-yl) -1-phenyl-1H-pyrazole-3-sulfonimidamide**

To a stirred solution of N'-((8-allyl-1,2,3,5,6,7-hexahydro-s-indacen-4-yl)carbamoyl)-N-(tert-butyl- dimethylsilyl)-5-(2-hydroxypropan-2-yl)-1-phenyl-1H-pyrazole-3-sulfonimidamide (110 mg, 0.17 mmol) in THF (5 mL) in a 50-mL round-bottom flask was added HF/Py (70% wt., 25 mg, 0.87 mmol) at 0 °C. The resulting solution was stirred for 1 h at 25 °C. The resulting mixture was concentrated under reduced pressure. The crude product was purified by Prep-HPLC with the following conditions: Sunfire prep C18 column 30*150, 5um; Mobile Phase A: water (0.1% FA), Mobile Phase B: MeCN; Flow rate: 60 mL/min; Gradient: 53% B to 55% B in 10 min; 254/210 nm; Rt: 7.73 min; Detector, 254/210 um. This resulted in 31.7 mg (35.0%) of **Example 458** as a white solid. MS-ESI: 520 (M+1). $^1H$ NMR (400 MHz, DMSO-$d_6$) δ 8.31 (s, 1H), 7.53 (s, 7H), 6.72 (s, 1H), 5.90-5.65 (m, 1H), 5.46 (s, 1H), 4.98-4.88 (m, 2H), 3.23 (dt, *J* = 6.5, 1.7 Hz, 2H), 2.80-2.72 (m, 4H), 2.72-2.62 (m, 4H), 1.98-1.85 (m, 4H), 1.35 (s, 3H), 1.34 (s, 3H).

**Table 41.** Example 459 was obtained during the Pre-HPLC purification from isomerizaton of Example 458.

| Ex. # | Cmpd # | Structure | IUPAC Name | Exact Mass $[M+H]^+$ |
|---|---|---|---|---|
| 459 | 843 | 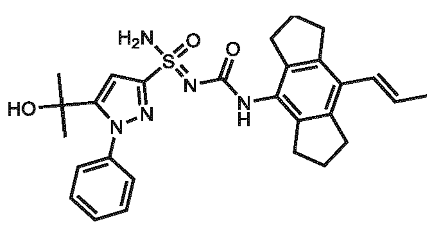 | (E)-5-(2-hydroxypropan-2-yl)-1 -phenyl-N'-((8-(prop-1 -en-1 - yl)-1,2,3,5,6,7-hexahydro-s-indacen-4-yl) carbamoyl)-1H-pyrazole-3 -sulfonimidamide | 520 |

**Table 42.** Examples in the following table were prepared using similar conditions as described in Example **458** and Scheme **2** from appropriate starting materials.

| Ex. # | Cmpd # | Structure | IUPAC Name | Exact Mass [M+H]+ |
|---|---|---|---|---|
| 460 | 801 | 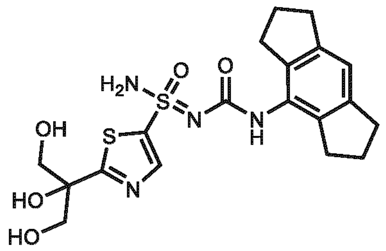 | N'-((1,2,3,5,6,7-hexahydro-s-indacen-4-yl)carbamoyl)-2-(1,2,3 -trihydroxypropan-2-yl)thiazole-5-sulfonimidamide | 453 |
| 461 | 806 | 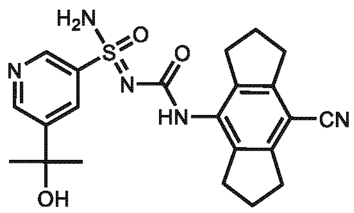 | N'-((8-cyano-1,2,3,5,6,7-hexahydro-s-indacen-4-yl)carbamoyl)-5-(2-hydroxypropan-2-yl)pyridine-3-sulfonimidamide | 440 |
| 462 | 807 | 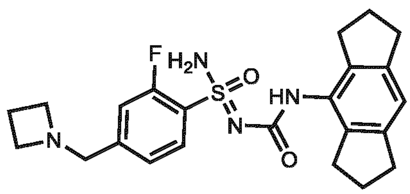 | 4-(azetidin-1-ylmethyl)-2-fluoro-N'-((1,2,3,5,6,7-hexahydro-s-indacen-4-yl)carbamoyl)benzenesulfonimidamide | 443 |
| 463 | 808 | 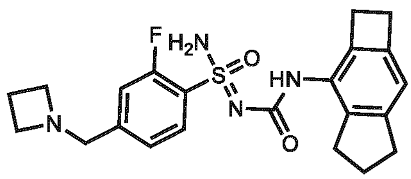 | 4-(azetidin-1-ylmethyl)-2-fluoro-N'-((2,4,5,6-tetrahydro-1H-cyclobuta[f] inden-3-yl)carbamoyl) benzenesulfonimidamide | 429 |
| 464 | 852 | 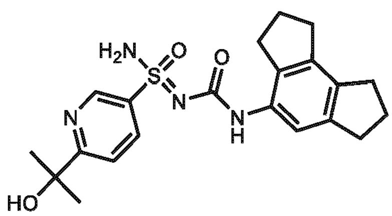 | N'-((1,2,3,6,7,8-hexahydro-as-indacen-4-yl)carbamoyl)-6-(2-hydroxypropan-2-yl)pyridine-3-sulfonimidamide | 415 |
| 465 | 803 | 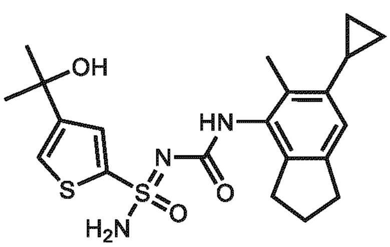 | N'-((6-cyclopropyl-5-methyl-2,3-dihydro-1H-inden-4-yl)carbamoyl)-4-(2-hydroxypropan-2-yl)thiophene-2-sulfonimidamide | 434 |

(continued)

| Ex. # | Cmpd # | Structure | IUPAC Name | Exact Mass [M+H]+ |
|---|---|---|---|---|
| 466 | 811 | 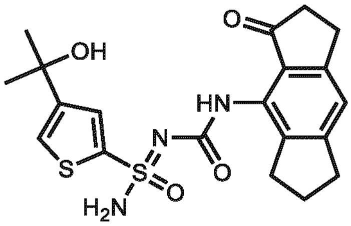 | 4-(2-Hydroxypropan-2-yl)-N'-((3-oxo-1,2,3,5,6,7-hexahydro-s-indacen-4-yl) carbamoyl)thiophene-2-sulfonimidamide | 434 |
| 467 | 835 | 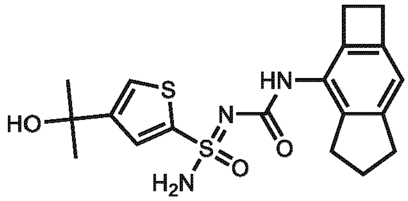 | 4-(2-Hydroxypropan-2-yl)-N'-((2,4,5,6-tetrahydro-1H-cyclobuta[f]inden-3-yl) carbamoyl)thiophene-2-sulfonimidamide | 406 |
| 468 | 836 | 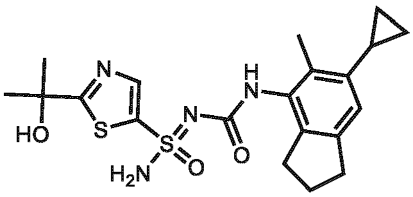 | N'-((6-cyclopropyl-5-methyl-2,3-dihydro-1H-inden-4-yl)carbamoyl)-2-(2-hydroxypropan-2-yl)thiazole-5-sulfonimidamide | 435 |
| 469 | 804 | 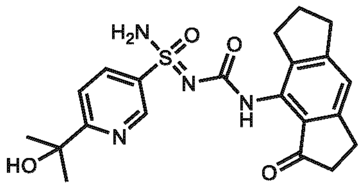 | 6-(2-Hydroxypropan-2-yl)-N'-((3-oxo-1,2,3,5,6,7-hexahydro-s-indacen-4-yl) carbamoyl)pyridine-3-sulfonimidamide | 429 |
| 470 | 822 | 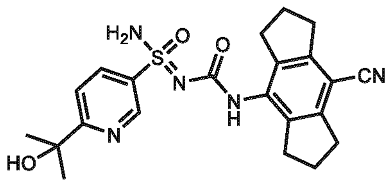 | N'-((8-cyano-1,2,3,5,6,7-hexahydro-s-indacen-4-yl)carbamoyl)-6-(2-hydroxypropan-2-yl)pyridine-3-sulfonimidamide | 440 |
| 471 | 871d | 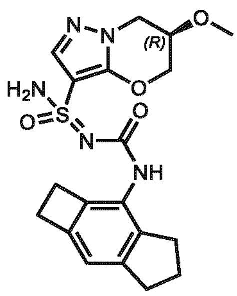 | N-(amino((R)-6-methoxy-6,7-dihydro-5H-pyrazolo[5,1-b][1,3]oxazin-3-yl)(oxo)-$\lambda^6$-sulfaneylidene)-2,4,5,6-tetrahydro-1H-cyclobuta[f]indene-3-carboxamide | 403 |

(continued)

| Ex. # | Cmpd # | Structure | IUPAC Name | Exact Mass [M+H]+ |
|---|---|---|---|---|
| 472 | 872a | 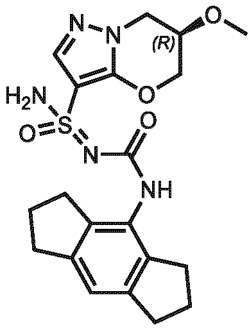 | N-(amino((R)-6-methoxy-6,7-dihydro-5H-pyrazolo[5,1-b][1,3]oxazin-3-yl)(oxo)-λ6-sulfaneyfidene)-1,2,3,5,6,7-hexahydro-s-indacene-4-carboxamide | 416 |
| 473 | 815 | 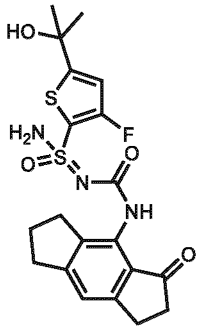 | N-(amino(3-fluoro-5-(2-hydroxypropan-2-yl)thiophen-2-yl)(oxo)-λ6-sulfaneylidene)-1,2,3,5,6,7-hexahydro-s-indacene-4-carboxamide | 423 |
| 474 | 819 | 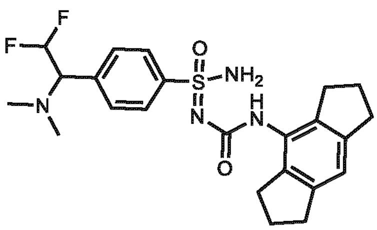 | 4-(1-(Dimethylamino)-2,2-difluoroethyl)-N'-((1,2,3,5,6,7-hexahydro-s-indacen-4-yl)carbamoyl) benzenesulfonimidamide | 463 |
| 475 | 820 | 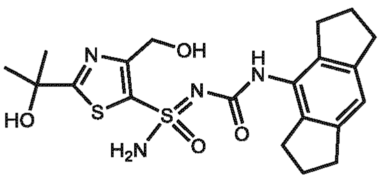 | N'-((1,2,3,5,6,7-hexahydro-s-indacen-4-yl)carbamoyl)-4-(hydroxymethyl)-2-(2-hydroxypropan-2-yl)thiazole-5-sulfonimidamide | 451 |
| 476 | 501 | 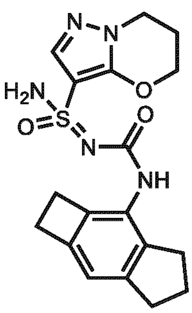 | N-(amino(6,7-dihydro-5H-pyrazolo [5, 1-b] [1,3]oxazin-3-yl)(oxo)-λ6-sulfaneyfidene)-2,4,5,6-tetrahydro-1H-cyclobuta[f]indene-3 -carboxamide<br><br>**COMPOUND ACCORDING TO THE INVENTION** | 373 |

(continued)

| Ex. # | Cmpd # | Structure | IUPAC Name | Exact Mass [M+H]+ |
|---|---|---|---|---|
| 477 | 821 | 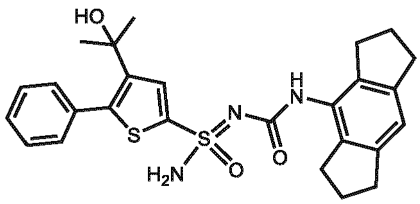 | N'-((1,2,3,5,6,7-hexahydro-s-indacen-4-yl)carbamoyl)-4-(2-hydroxypropan-2-yl)-5-phenylthiophene-2-sulfonimidamide | 496 |
| 478 | 833 | 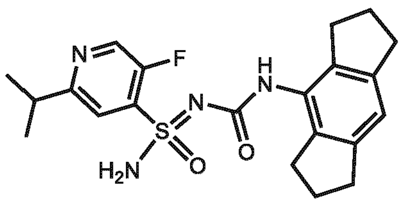 | 5-Fluoro-N'-((1,2,3,5,6,7-hexahydro-s-indacen-4-yl)carbamoyl)-2-isopropylpyridine-4-sulfonimidamide | 417 |
| 479 | 413 | 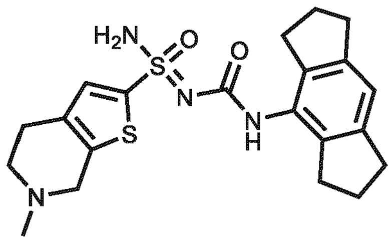 | N'-((1,2,3,5,6,7-hexahydro-s-indacen-4-yl)carbamoyl)-6-methyl-4,5,6,7-tetrahydrothieno[2,3-c]pyridine-2-sulfonimidamide | 431 |
| 480 | 831 | 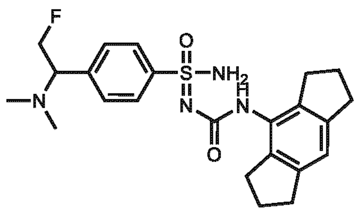 | 4-(1-(Dimethylamino)-2-fluoroethyl)-N'-((1,2,3,5,6,7-hexahydro-s-indacen-4-yl)carbamoyl) benzenesulfonimidamide | 445 |
| 481 | 412 | 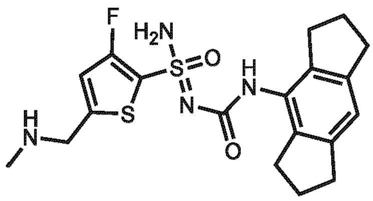 | 3-Fluoro-N'-((1,2,3,5,6,7-hexahydro-s-indacen-4-yl)carbamoyl)-5-((methylamino)methyl)thiophene-2-sulfonimidamide | 423 |
| 482 | 849 | 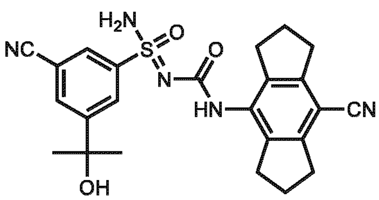 | 3-Cyano-N'-((8-cyano-1,2,3,5,6,7-hexahydro-s-indacen-4-yl)carbamoyl)-5-(2-hydroxypropan-2-yl) benzenesulfonimidamide | 464 |
| 483 | 850 | 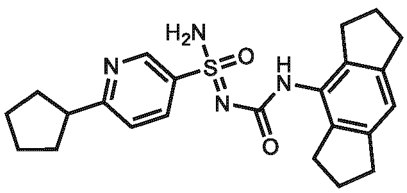 | 6-Cyclopentyl-N'-((1,2,3,5,6,7-hexahydro-s-indacen-4-yl)carbamoyl) pyridine-3-sulfonimidamide | 424 |

(continued)

| Ex. # | Cmpd # | Structure | IUPAC Name | Exact Mass [M+H]+ |
|---|---|---|---|---|
| 484 | 854 | 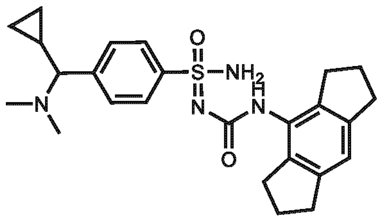 | 4-(Cyclopropyl(dimethylamino) methyl)-N'-((1,2,3,5,6,7-hexahydro-s-indacen-4-yl)carbamoyl) benzenesulfonimidamide | 453 |
| 485 | 855 | 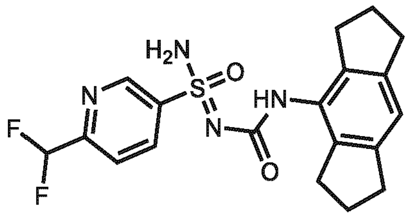 | 6-(Difluoromethyl)-N'-((1,2,3,5,6,7-hexahydro-s-indacen-4-yl)carbamoyl) pyridine-3-sulfonimidamide | 407 |
| 486 | 860 | 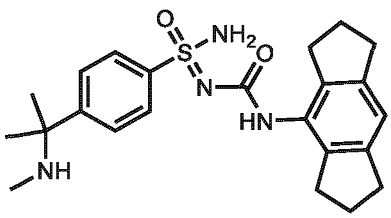 | N'-((1,2,3,5,6,7-hexahydro-s-indacen-4-yl)carbamoyl)-4-(2-(methylamino) propan-2-yl)benzenesulfonimidamide | 427 |
| 487 | 861 | 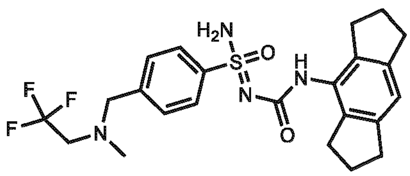 | N'-((1,2,3,5,6,7-hexahydro-s-indacen-4-yl)carbamoyl)-4-((methyl(2,2,2-trifluoroethyl)amino)methyl) benzenesulfonimidamide | 481 |
| 488 | 862 | 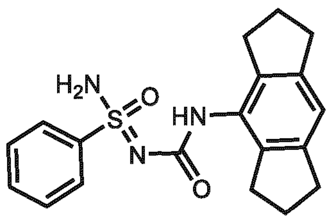 | N'-((1,2,3,5,6,7-hexahydro-s-indacen-4-yl)carbamoyl)benzenesulfonimidamide | 356 |
| 489 | 863 | 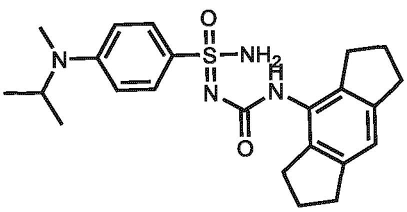 | N'-((1,2,3,5,6,7-hexahydro-s-indacen-4-yl)carbamoyl)-4-(isopropyl(methyl) amino)benzenesulfonimidamide | 427 |
| 490 | 844 | 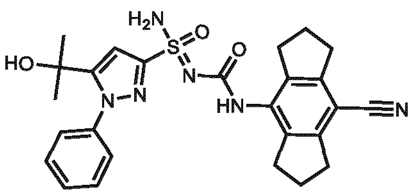 | N'-((8-cyano-1,2,3,5,6,7-hexahydro-s-indacen-4-yl)carbamoyl)-5-(2-hydroxypropan-2-yl)-1 -phenyl- 1H-pyrazole-3 -sulfonimidamide | 505 |

(continued)

| Ex. # | Cmpd # | Structure | IUPAC Name | Exact Mass [M+H]+ |
|---|---|---|---|---|
| 491 | | 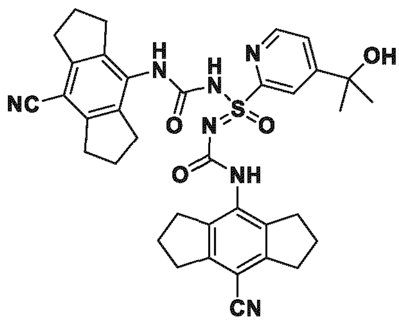 | N'-((8-cyano-1,2,3,5,6,7-hexahydro-s-indacen-4-yl)carbamoyl)-4-(2-hydroxypropan-2-yl)pyridine-2-sulfonimidamide | 440 |
| 492 | 856 | 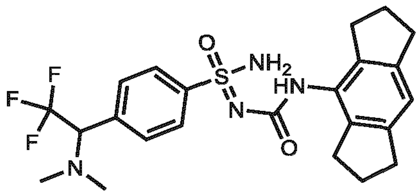 | 4-(1-(Dimethylamino)-2,2,2-trifluoroethyl)-N'-((1,2,3,5,6,7-hexahydro-s-indacen-4-yl)carbamoyl) benzenesulfonimidamide | 481 |
| 493 | 841 | 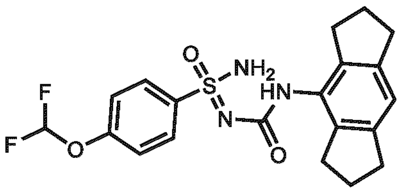 | 4-(Difluoromethoxy)-N'-((1,2,3,5,6,7-hexahydro-s-indacen-4-yl)carbamoyl) benzenesulfonimidamide | 422 |
| 494 | 812 | 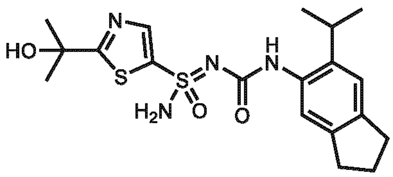 | 2-(2-Hydroxypropan-2-yl)-N'-((6-isopropyl-2,3-dihydro-1H-inden-5-yl) carbamoyl)thiazole-5-sulfonimidamide | 423 |

Example 495 (Compound 810)

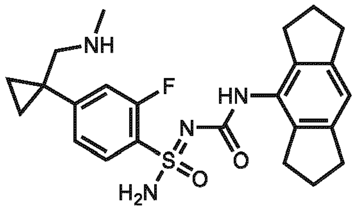

2-Fluoro-N'-((1,2,3,5,6,7-hexahydro-s-indacen-4-yl)carbamoyl)-4-(1-((methylamino)methyl)cyclopropyl)benzenesulfonimidamide (Scheme V)

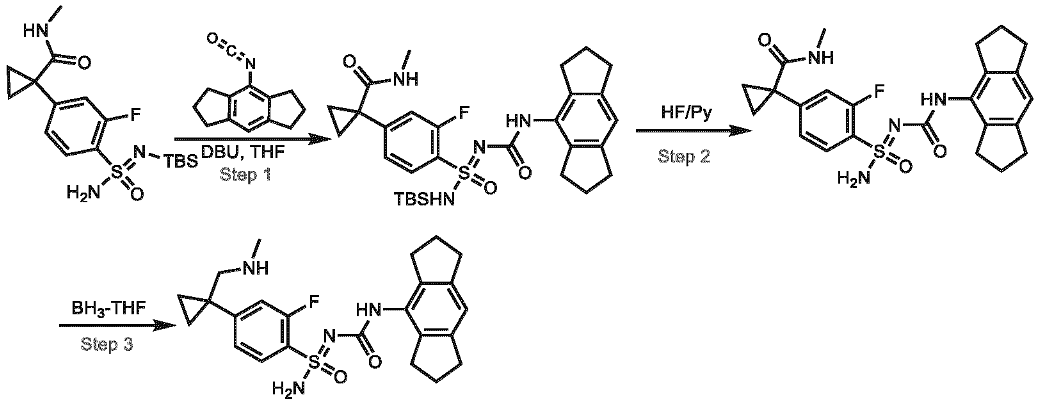


**Example 495**

**Step 1: 1-(4-(N-(tert-butyldimethylsilyl)-N'-((1,2,3,5,6,7-hexahydro-s-indacen-4-yl)carbamoyl) sulfamidimidoyl)-3-fluorophenyl)-N-methylcyclopropane-1-carboxamide**

To a stirred solution of 1-(4-(N'-(tert-butyldimethylsilyl)sulfamidimidoyl) -3-fluorophenyl)-N-methylcyclopropane-1-carboxamide (100 mg, 0.26 mmol) in THF (5 mL) in a 25-mL round-bottom flask was added DBU (12.5 mg, 0.52 mmol) and 4-isocyanato-1,2,3,5,6,7-hexahydro-s-indacene (51.7 mg, 0.26 mmol). The resulting solution was stirred for 16 h at RT. The resulting mixture was used in the next step directly without further purification. MS-ESI: 585 (M+1).

**Step 2: 1-(3-Fluoro-4-(N'-((1,2,3,5,6,7-hexahydro-s-indacen-4-yl)carbamoyl)sulfamidimidoyl)phenyl) -N-methylcyclopropane-1-carboxamide**

To the solution from the step 1 was added HF-Pyridine (70% wt., 37 mg, 1.3 mmol). The resulting solution was stirred for 1 h at RT. The reaction was then quenched by the addition of 5 mL of water. The resulting solution was extracted with 3x15 mL of EtOAc. The organic layers were combined and dried over anhydrous $Na_2SO_4$ and concentrated under vacuum. The crude product was eluted from silica gel with PE/EtOAc (1:3). This resulted in 73.3 mg (62.1% over 2 steps) of the title compound as a yellow solid. MS-ESI: 471 (M+1).

**Step 3: 2-Fluoro-N'-((1,2,3,5,6,7-hexahydro-s-indacen-4-yl)carbamoyl)-4-(1-((methylamino)methyl) cyclopropyl)benzenesulfonimidamide**

To a stirred solution of 1-(3-fluoro-4-(N'-((1,2,3,5,6,7-hexahydro-s-indacen-4-yl)carbamoyl)-sulfamidimidoyl)phenyl)-N-methylcyclopropane-1-carboxamide (47.1 mg, 0.10 mmol) in THF (2 mL) in a 8-mL sealed tube purged with and maintained under nitrogen was added $BH_3$-THF (1 M, 0.5 mL, 0.50 mmol) dropwise at 0 °C. The resulting solution was stirred for 16 h at 30 °C. The reaction was then quenched by the addition of 10 mL of water. The resulting solution was extracted with 3x15 mL of EtOAc. The organic layers were combined and dried over anhydrous $Na_2SO_4$ and concentrated under vacuum. The crude product was purified by Prep-HPLC with the following conditions: Sunfire prep C18 column 30*150, 5um; mobile phase, $H_2O$/MeCN = 90:10 increasing to $H_2O$/MeCN = 10:90 within 30 min; Detector, 254 nm. This resulted in 18.3 mg (40.0%) of **Example 495** as a solid. MS-ESI: 457 (M+1).

**Table 43.** Examples in the following table were prepared using similar conditions as described in Example **495** and Scheme V from appropriate starting materials.

| Ex. # | Cmpd # | Structure | IUPAC Name | Exact Mass [M+H]+ |
|---|---|---|---|---|
| 496 | 813 | 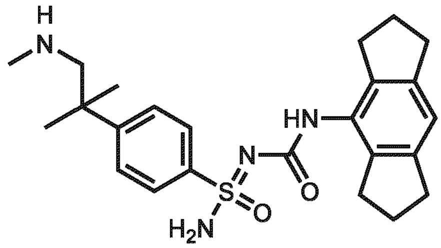 | N'-((1,2,3,5,6,7-hexahydro-s-indacen-4-yl)carbamoyl)-4-(2-methyl-1 -(methylamino)propan-2-yl) benzenesulfonimidamide | 441 |

**Example 497 (Compound 809)**

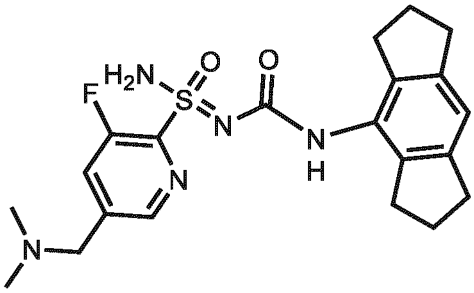

5-((Dimethylamino)methyl)-3-fluoro-N'-((1,2,3,5,6,7-hexahydro-s-indacen-4-yl)carbamoyl)pyridine-2-sulfonimidamide (Scheme VI)

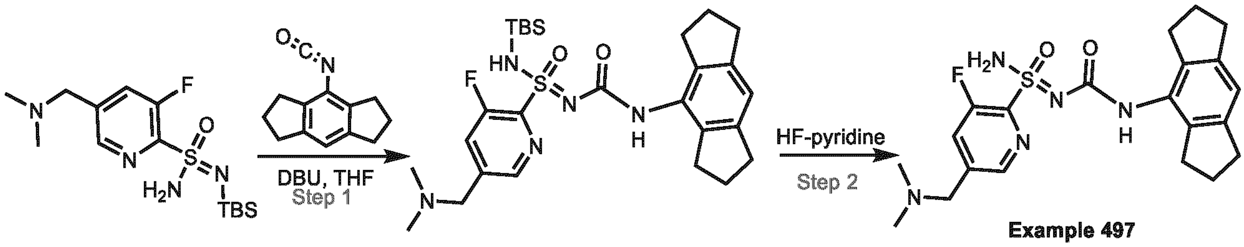


Step 1-2 used similar procedures as step 1-2 shown in **Example 495** to afford **Example 497.** MS-ESI: 432 (M+1). $^1$H NMR (400 MHz, DMSO-$d_6$) δ 8.78 (s, 1H), 8.53 (br s, 1H), 8.34 (dd, *J* = 10.4, 1.2 Hz, 1H), 7.78 (s, 2H), 6.86 (s, 1H), 4.62 (s, 2H), 2.81 (s, 6H), 2.81-2.70 (m, 4H), 2.70-2.50 (m, 4H), 2.00-1.80 (m, 4H)

**Example 499 (Compound 909)**

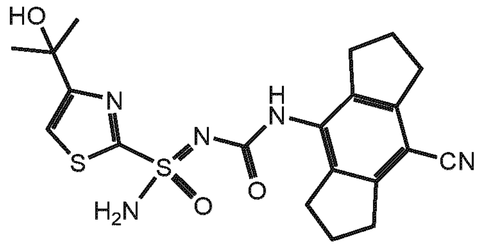

N'-((8-Cyano-1,2,3,5,6,7-hexahydro-s-indacen-4-yl)carbamoyl)-4-(2-hydroxypropan-2-yl)thiazole-2-sulfonimidamide (Scheme VIII)

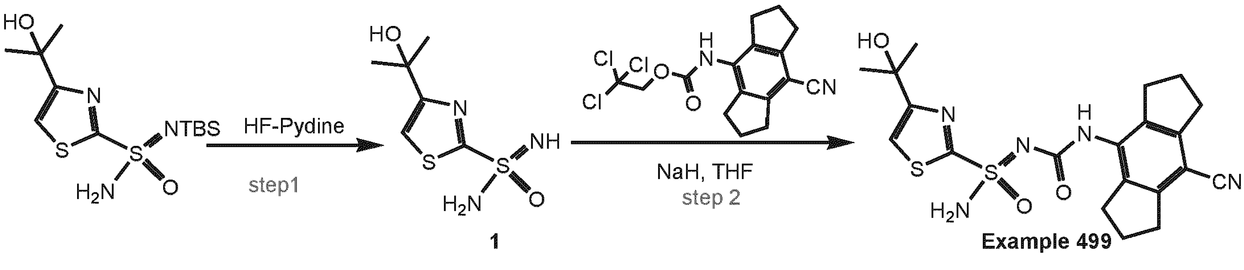


1

Example 499

**Step 1: 4-(2-Hydroxypropan-2-yl)thiazole-2-sulfonimidamide**

To a stirred solution of N'-(tert-butyldimethylsilyl)-4-(2-hydroxypropan -2-yl)thiazole-2-sulfonimidamide (485 mg, 1.44 mmol) in THF (10 mL) in a 25-mL round-bottom flask was added HF/Py (70%wt., 206 mg, 7.2 mmol). The resulting solution was stirred for 1 h at RT. The resulting mixture was concentrated under reduced pressure. The residue was eluted from silica gel with DCM/MeOH (10:1). This resulted in 299 mg (93.8%) of the title compound as a yellow solid. MS-ESI: 222 (M+1).

**Step 2: *N*-((8-cyano-1,2,3,5,6,7-hexahydro-s-indacen-4-yl)carbamoyl)-4-(2-hydroxypropan-2-yl) thiazole-2-sulfonimidamide**

To a stirred solution of 4-(2-hydroxypropan-2-yl)thiazole-2-sulfonimidamide (221 mg, 1.00 mmol) in THF (20 mL) in a 50-mL round-bottom flask under nitrogen was added NaH (60% dispersion in mineral oil, 120 mg, 3.00 mmol) in one portion at 0 °C, 2,2,2-trichloroethyl (8-cyano-1,2,3,5,6,7-hexahydro-s-indacen-4- yl)carbamate (374 mg, 1.00 mmol) was added to the solution in portions at 0 °C. The resulting solution was stirred for 60 min at RT. The resulting mixture was quenched with 5 mL of water then concentrated under reduced pressure. The crude product was purified by Prep-HPLC with the following conditions: XBridge Shield RP18 OBD Column, 19*250 mm, 10 um; mobile phase, water (10 mM $NH_4HCO_3$) and MeCN (13% to 34% Phase B over 7 min); Detector, 254/220 nm. This resulted in 134 mg (30.1%) of **Example 499** as an off-white solid. MS-ESI: 446 (M+1). $^1$H NMR (400 MHz, DMSO-$d_6$) δ 8.90 (s, 1H), 7.98 (s, 2H), 7.72 (s, 1H), 5.37 (s, 1H), 2.93 (t, $J$=7.4 Hz, 4H), 2.70 (t, $J$=7.7 Hz, 4H), 2.10-1.90 (m, 4H), 1.47 (s, 3H), 1.46 (s, 3H).

**Example 500 (Compound 838)**

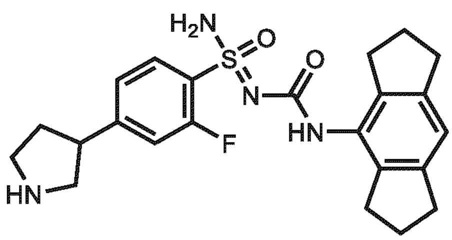

2-Fluoro-N'-((1,2,3,5,6,7-hexahydro-s-indacen-4-yl)carbamoyl)-4-(pyrrolidin-3-yl)benzenesulfonimidamide (Scheme IX)

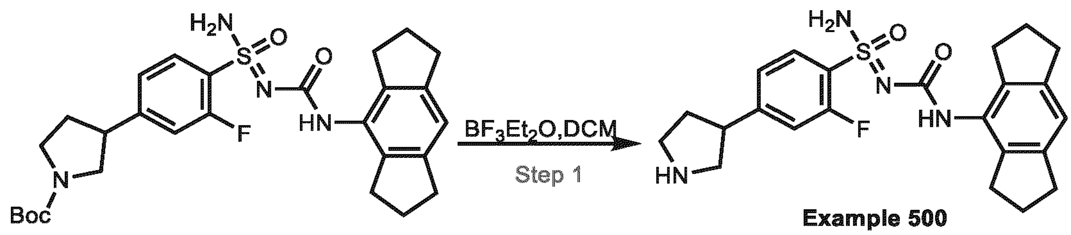


**Step 1: 2-Fluoro-N'-((1,2,3,5,6,7-hexahydro-s-indacen-4-yl)carbamoyl)-4-(pyrrolidin-3-yl) benzenesulfonimidamide**

To a stirred solution of tert-butyl 3-(3-fluoro-4-(N'-((1,2,3,5,6,7-hexahydro-s-indacen -4-yl)carbamoyl)sulfamidimidoyl)phenyl)pyrrolidine-1-carboxylate (272 mg, 0.50 mmol) in DCM (10 mL) in a 50-mL round-bottom flask was added $BF_3 \cdot Et_2O$ (47%wt., 362 mg, 2.50 mmol) dropwise at 0 °C. The resulting solution was stirred for 2 h at 0 °C. The reaction was then quenched by the addition of 0.2 mL of sat. $NaHCO_3$ solution. The resulting mixture was concentrated under vacuum. The crude product was purified by Prep-HPLC with the following conditions: XBridge Prep OBD C18 Column, 30*150mm, 5um; mobile phase, water (10 mM $NH_4HCO_3$+0.1%$NH_3 \cdot H_2O$) and MeCN (10% to 33% Phase B over 7 min); Detector, UV (254/220 nm). This resulted in 90.8 mg (41.0%) of **Example 500** as an off-white solid. MS-ESI: 443(M+1). $^1H$ NMR (300 MHz, MeOH-$d_4$) δ 7.98 (d, *J* = 7.7 Hz, 1H), 7.45-7.25 (m, 2H), 6.91 (s, 1H), 3.82-3.56 (m, 3H), 3.50-3.10 (m, 2H), 2.90-2.65 (m, 8H), 2.60-2.30 (m, 2H), 2.10-1.90 (m, 4H).

**Table 44.** Example **501** and Example **502** in the following table were prepared using similar conditions as described in Example **500** and Scheme **IX** from the corresponding chiral Intermediate **128B** and Intermediate **128A.**

| Ex. # | Cmpd # | Structure | IUPAC Name | Exact Mass [M+H]$^+$ |
|---|---|---|---|---|
| 501 | 838e | 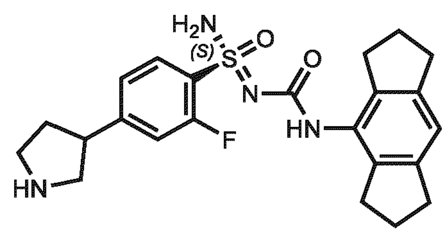 | (S) or (R) -2-fluoro-N'-((1,2,3,5,6,7-hexahydro-s-indacen-4-yl) carbamoyl)-4-(pyrrolidin-3-yl) benzenesulfonimidamide | 443 |
| 502 | 838b | 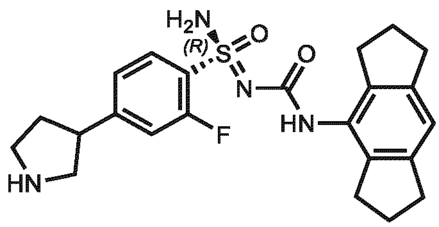 | (R) or (S) -2-fluoro-N'-((1,2,3,5,6,7-hexahydro-s-indacen-4-yl) carbamoyl)-4-(pyrrolidin-3-yl) benzenesulfonimidamide | 443 |

Example 503 (Compound 832)

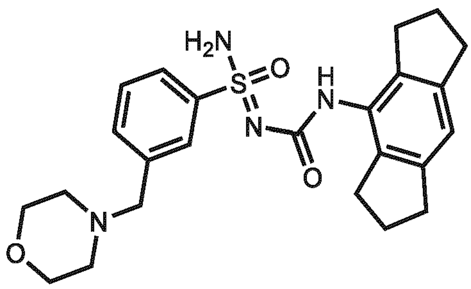

N'-((1,2,3,5,6,7-hexahydro-s-indacen-4-yl)carbamoyl)-3-(morpholinomethyl)benzenesulfonimidamide (Scheme X)

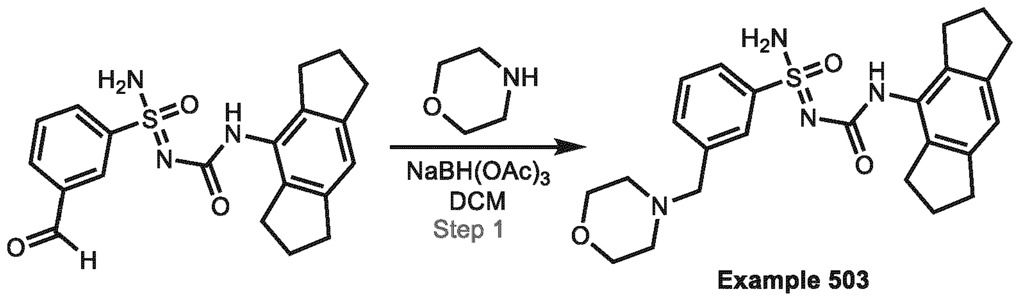


**Example 503**

**Step** 1: **N'-((1,2,3,5,6,7-hexahydro-s-indacen-4-yl)carbamoyl)-3-(morpholinomethyl)benzene -sulfonimidamide**

To a stirred solution of 3-formyl-N'-((1,2,3,5,6,7-hexahydro-s-indacen-4-yl)carbamoyl)benzenesulfonimidamide (50 mg, 0.13 mmol) in DCE (5 mL) in a 25-mL round-bottom flask under nitrogen was added morpholine (33.9 mg, 0.39 mmol) at RT. The resulting solution was stirred overnight at RT. Then $NaBH(OAc)_3$ (55.1 mg, 0.26 mmol) was added to the mixture at 0 °C. The resulting solution was stirred for 12 h at RT. The resulting solution was extracted with 3x10 mL of EtOAc and the organic layers were combined and dried over anhydrous $Na_2SO_4$ and concentrated under vacuum. The residue was eluted from silica gel with DCM/MeOH (10:1), further purified by Prep-HPLC with the following conditions: XBridge Prep OBD C18 Column, 30* 150 mm, 5um; mobile phase, water (10 mM $NH_4HCO_3$+0.1%$NH_3 \cdot H_2O$) and MeCN (10% to 33% Phase B over 7 min); Detector, UV (254/220 nm). This resulted in 27.9 mg (47.2%) of **Example 503** as a yellow solid. MS-ESI: 455 (M+1).

**Table 45.** Examples in the following table were prepared using similar conditions as described in Example **503** and Scheme **X** from appropriate starting materials.

| Ex. # | Cmpd # | Structure | IUPAC Name | Exact Mass [M+H]+ |
|---|---|---|---|---|
| 504 | 830 | 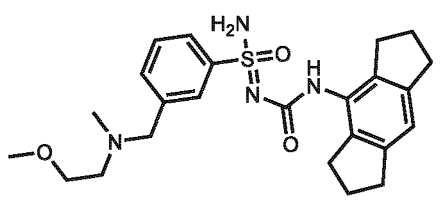 | N'-((1,2,3,5,6,7-hexahydro-s-indacen-4-yl)carbamoyl)-3-(((2-methoxyethyl)(methyl)amino)methyl) benzenesulfonimida mide | 457 |
| 505 | 825 | 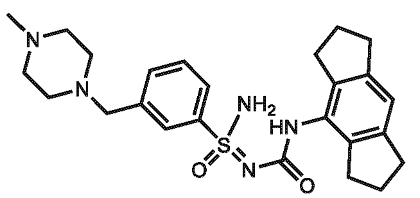 | N'-((1,2,3,5,6,7-hexahydro-s-indacen-4-yl)carbamoyl)-3-((4-methylpiperazin-1-yl)methyl)benzenesulfonimidamide | 468 |

(continued)

| Ex. # | Cmpd # | Structure | IUPAC Name | Exact Mass [M+H]+ |
|---|---|---|---|---|
| 506 | 824 | 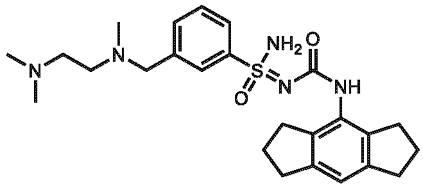 | 3-(((2-(Dimethylamino)ethyl)(methyl) amino)methyl)-N'-((1,2,3,5,6,7-hexahydro-s-indacen-4-yl)carbamoyl) benzenesulfonimidamide | 470 |
| 507 | 823 | 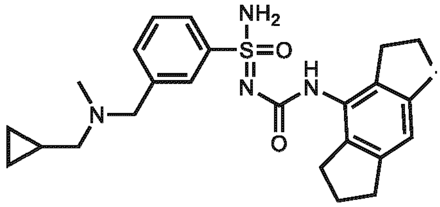 | 3-(((Cyclopropylmethyl)(methyl)amino) methyl)-N'-((1,2,3,5,6,7-hexahydro-s-indacen-4-yl)carbamoyl) benzenesulfonimidamide | 453 |
| 508 | 848 | 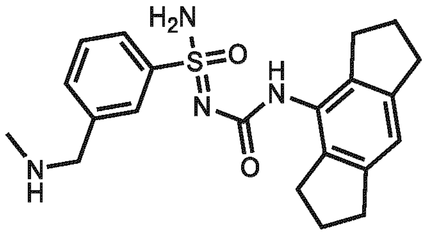 | N'-((1,2,3,5,6,7-hexahydro-s-indacen-4-yl)carbamoyl)-3-((methylamino) methyl)benzenesulfonimidamide | 399 |

**Example 509 (Compound 8681)**

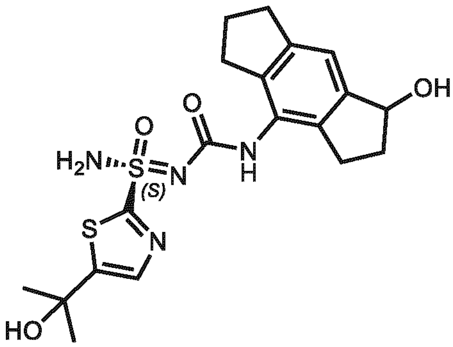

(S)-N'-((1-hydroxy-1,2,3,5,6,7-hexahydro-s-indacen-4-yl)carbamoyl)-5-(2-hydroxypropan-2-yl)thiazole-2-sulfonimidamide (Scheme 32)

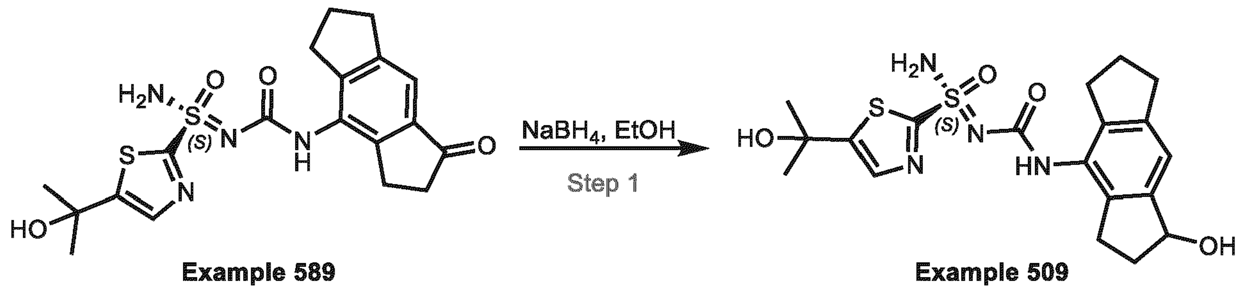


Example 589 Example 509

To a stirred solution of (S)-5-(2-hydroxypropan-2-yl)-N'-((1-oxo-1,2,3,5,6,7-hexahydro-s-indacen-4- yl)carbamoyl)thiazole-2-sulfonimidamide (180 mg, 0.41 mmol) in EtOH (10 mL) in a 50-mL round-bottom flask was added $NaBH_4$ (31.3 mg, 0.828 mmol) in portions at 0 °C. The resulting solution was stirred for 2 h at RT. The reaction was then quenched with 5 mL of water. The solids were filtered out. The resulting mixture was concentrated under vacuum. The crude product was purified by Prep-HPLC with the following conditions: XBridge Prep C18 OBD Column, 19*150 mm 5um; mobile phase, water (10 mM $NH_4HCO_3$+0.1% $NH_3{\cdot}H_2O$) and MeCN (5% to 24% Phase B over 7 min); Detector, UV 254 nm. This resulted in 130 mg (71.9%) of **Example 509** as a white solid. MS-ESI: 437 (M+1). $^1$H NMR (400 MHz, DMSO-$d_6$) δ 8.49 (br s, 1H), 7.79 (s, 1H), 7.65 (br s, 2H), 6.97 (s, 1H), 5.90 (s, 1H), 5.10 (d, $J$ = 5.6 Hz, 1H), 5.00-4.90 (m, 1H), 3.90-3.80 (m, 2H), 3.80-3.60 (m, 4H), 2.35-2.25 (m, 1H), 2.05-1.85 (m, 2H), 1.75-1.60 (m, 1H), 1.55 (s, 6H).

**Table 46.** Examples in the following table were prepared using similar conditions as described in Example **509** and Scheme **32** from appropriate chiral starting ketones.

| Ex. # | Cmpd # | Structure | IUPAC Name | Exact Mass $[M+H]^+$ |
|---|---|---|---|---|
| 510 | 867f | 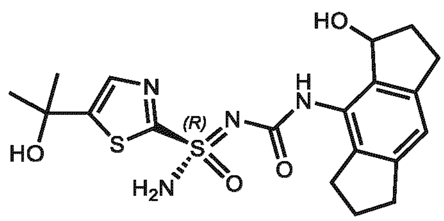 | (R)-N'-((3-hydroxy-1,2,3,5,6,7-hexahydro-*s*-indacen-4-yl) carbamoyl)-5-(2-hydroxypropan-2-yl)thiazole-2-sulfonimidamide | 437 |
| 511 | 867e | 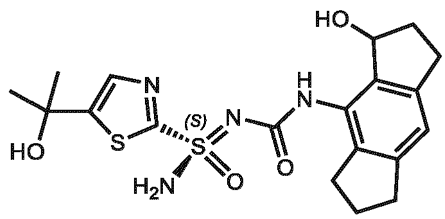 | (S)-N'-((3-hydroxy-1,2,3,5,6,7-hexahydro-*s*-indacen-4-yl) carbamoyl)-5-(2-hydroxypropan-2-yl)thiazole-2-sulfonimidamide | 437 |
| 512 | 868e | 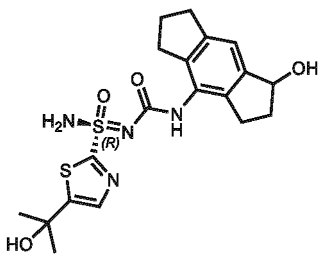 | (R)-N'-((1-hydroxy-1,2,3,5,6,7-hexahydro-*s*-indacen-4-yl) carbamoyl)-5-(2-hydroxypropan-2-yl)thiazole-2-sulfonimidamide | 437 |

**Example 513 (Compound 839)**

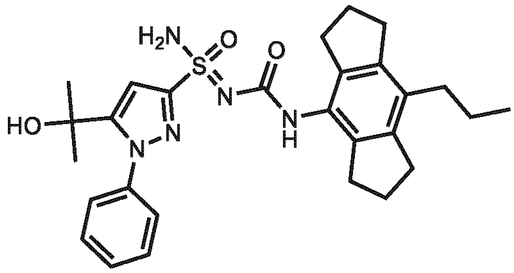

5-(2-hydroxypropan-2-yl)-1-phenyl-N'-((8-propyl-1,2,3,5,6,7-hexahydro-*s*-indacen-4-yl)carbamoyl)-1H-pyrazole-3-sulfonimidamide (Scheme XI)

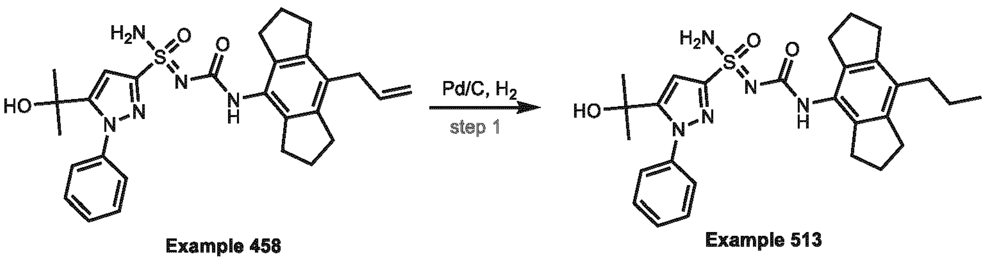


To a stirred solution of *N'*-((8-allyl-1,2,3,5,6,7-hexahydro-*s*-indacen-4-yl)carbamoyl)-5- (2-hydroxypropan-2-yl)-1-phenyl-1H-pyrazole-3-sulfonimidamide (20 mg, 0.038 mmol) in MeOH (10 mL) 50 mL round-bottom flask under nitrogen was added Pd/C (10% wt., 5.0 mg), the flask was excavated and flushed with hydrogen 3 times. The resulting mixture was stirred for 16 h at RT under hydrogen with a balloon. The resulting mixture was filtered; the filter cake was washed with MeOH (3x5 mL). The filtrate and wash were concentrated under reduced pressure. The crude product was purified by Prep-HPLC using the following conditions: XBridge Shield RP18 OBD Column 19*250 mm, 10 um; Mobile Phase A: water (10 mM $NH_4HCO_3$), Mobile Phase B: MeCN; Flow rate: 25 mL/min; Gradient: 50% B to 52% B in 10 min; 210/254 nm; Rt: 11.45 min. This resulted in **Example 513** (6.5 mg, 32.4%) as a white solid. MS-ESI: 522 (M+1). $^1$H NMR (400 MHz, MeOH-$d_4$) δ 7.60-7.48 (m, 5H), 6.87 (s, 1H), 2.84 (t, *J*= 7.4 Hz, 4H), 2.82-2.70 (m, 4H), 2.57-2.48 (m, 2H), 2.08-1.96 (m, 4H), 1.60-1.45 (m, 2H), 1.45 (s, 3H), 1.44 (s, 3H), 0.95 (t, *J* = 7.4 Hz, 3H).

**Table 47.** Example 514 was obtained from Step 3 in Route 2 in Example 224.

| Example # | Compound # | Structure | IUPAC Name | Exact Mass [M+H]+ |
|---|---|---|---|---|
| 514 | 865a | 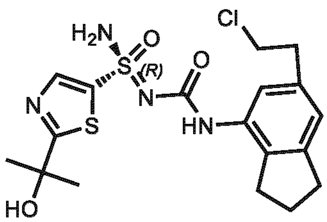 | (R)-N'-((6-(2-chloroethyl)-2,3-dihydro-1H-inden-4-yl)carbamoyl)-2-(2-hydroxypropan-2-yl)thiazole-5-sulfonimidamide | 443/445 |

**Table 48.** Examples in the following table were obtained from chiral HPLC resolutions of racemic examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative.

| Ex. # | Cmpd # | Structure | IUPAC Name | Column | Eluents | LC-MS $[M+H]^+$ |
|---|---|---|---|---|---|---|
| 515 | 869a | 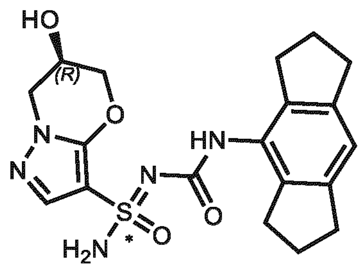 | (R,6R) or (S,6R)-N'-((1,2,3,5,6,7-hexahydro-*s*-indacen-4-yl)carbamoyl)-6-hydroxy-6,7-dihydro-5H-pyrazolo[5,1-b][1,3]oxazine-3-sulfonimidamide | CHIRA LPAK ID, 2*25 cm (5 um) | EtOH in Hex (8 mM $NH_3$·MeOH )# | 418 |
| 516 | 869b | 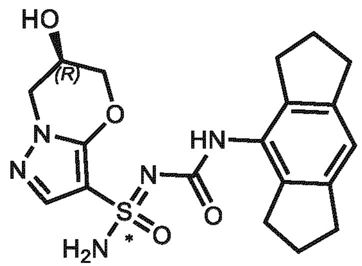 | (S,6R) or (R,6R)-N'-((1,2,3,5,6,7-hexahydro-*s*-indacen-4-yl)carbamoyl)-6-hydroxy-6,7-dihydro-5H-pyrazolo[5,1-b][1,3]oxazine-3-sulfonimidamide | CHIRA LPAK ID, 2*25 cm (5 um) | EtOH in Hex (8 mM $NH_3$·MeOH ) | 418 |
| 517 | 809b | 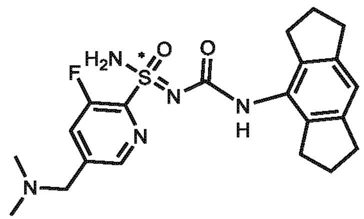 | (S) or (R)-5-((dimethylamino)methyl)-3-fluoro-N'-((1,2,3,5,6,7-hexahydro-*s*-indacen-4-yl)carbamoyl)pyridine-2-sulfonimidamide | CHIRA LPAK IG, 20*250 mm, 5 um | EtOH in Hex (0.1% FA) | 432 |

(continued)

| Ex. # | Cmpd # | Structure | IUPAC Name | Column | Eluents | LC-MS $[M+H]^+$ |
|---|---|---|---|---|---|---|
| 518 | 809a | 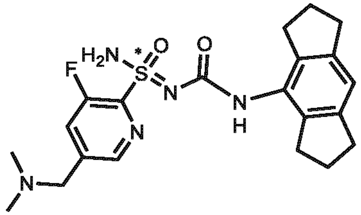 | (R) or (S)-5-((dimethylamino)methyl)-3-fluoro-N'-((1,2,3,5,6,7-hexahydro-*s*-indacen-4-yl) carbamoyl)pyridine-2-sulfonimidamide | CHIRA LPAK IG, 20*250 mm, 5 um | EtOH in Hex (0.1% FA) | 432 |
| 519 | 813b | 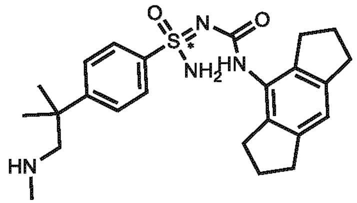 | (R) or (S)-N'-((1,2,3,5,6,7-hexahydro-*s*-indacen-4-yl)carbamoyl)-4-(2-methyl-1-(methylamino)propan-2-yl) benzenesulfonimidamide | CHIRA LPAK ID 2* 25 cm (5 um) | IPA in Hex:DCM= 5:1 (10 mM $NH_3 \cdot MeOH$ ) | 441 |
| 520 | 813a | 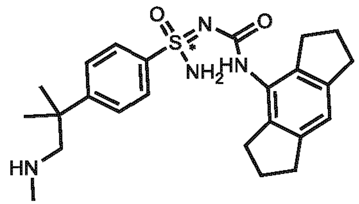 | (S) or (R)-N'-((1,2,3,5,6,7-hexahydro-*s*-indacen-4-yl)carbamoyl)-4-(2-methyl-1-(methylamino)propan-2-yl) benzenesulfonimidamide | CHIRA LPAK ID 2* 25 cm (5 um) | IPA in Hex:DCM= 5:1 (10 mM $NH_3 \cdot MeOH$ ) | 441 |
| 498 | 803b | 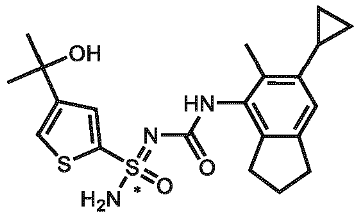 | (R) or (S)-N'-((6-cyclopropyl-5-methyl-2,3-dihydro-1H-inden-4-yl)carbamoyl)-4-(2-hydroxypropan-2-yl)thiophene-2-sulfonimidamide | Chiralpa k ID, 2*25 cm, 5 um | 50% IPA in Hex (0.1% FA) | 434 |

(continued)

| Ex. # | Cmpd # | Structure | IUPAC Name | Column | Eluents | LC-MS $[M+H]^+$ |
|---|---|---|---|---|---|---|
| 521 | 803a | 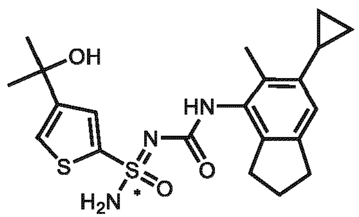 | (S) or (R)-N'-((6-cyclopropyl-5-methyl-2,3-dihydro-1H-inden-4-yl)carbamoyl)-4-(2-hydroxypropan-2-yl)thiophene-2-sulfonimidamide | Chiralpa k ID, 2*25 cm, 5 um | 50% IPA in Hex (0.1% FA) | 434 |
| 522 | 806a | 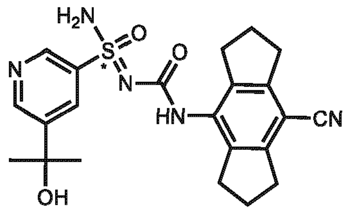 | (R) or (S)-N'-((8-cyano-1,2,3,5,6,7-hexahydro-*s*-indacen-4-yl)carbamoyl)-5-(2-hydroxypropan-2-yl)pyridine-3-sulfonimidamide | CHIRA LPAK ID, 2*25 cm (5 um) | IPA in Hex (0.1% FA) | 439 |
| 523 | 806b | 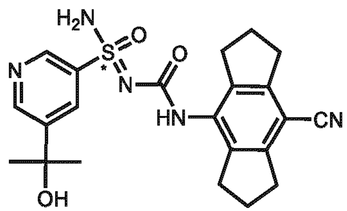 | (S) or (R)-N'-((8-cyano-1,2,3,5,6,7-hexahydro-*s*-indacen-4-yl)carbamoyl)-5-(2-hydroxypropan-2-yl)pyridine-3-sulfonimidamide | CHIRA LPAK ID, 2*25 cm (5 um) | IPA in Hex (0.1% FA) | 439 |
| 524 | 808b | 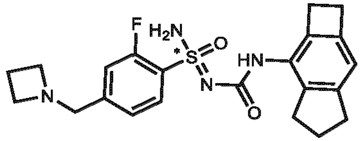 | (R) or (S)-4-(azetidin-1-ylmethyl)-2-fluoro-N'-((2,4,5,6-tetrahydro-1H-cyclobuta[f]inden-3-yl)carbamoyl)benzenesulfonimidamide | CHIRA LPAK IG, 2*25 cm, 5 um | EtOH in Hex (8 mM $NH_3$·MeOH ) | 429 |
| 525 | 808a | 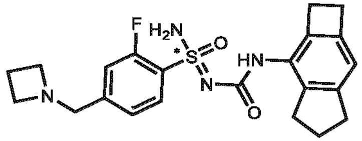 | (S) or (R)-4-(azetidin-1-ylmethyl)-2-fluoro-N'-((2,4,5,6-tetrahydro-1H-cyclobuta[f]inden-3-yl)carbamoyl)benzenesulfonimidamide | CHIRA LPAK IG, 2*25 cm, 5 um | EtOH in Hex (8 mM $NH_3$·MeOH ) | 429 |

(continued)

| Ex. # | Cmpd # | Structure | IUPAC Name | Column | Eluents | LC-MS $[M+H]^+$ |
|---|---|---|---|---|---|---|
| 526 | 812b | 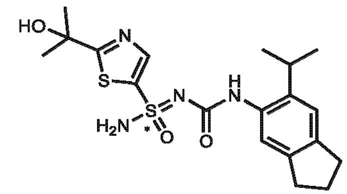 | (R) or (S)-2-(2-hydroxypropan-2-yl)-N'-((6-isopropyl-2,3-dihydro-1H-inden-5 -yl) carbamoyl)thiazole-5 - sulfonimidamide | Chiralpa k ID-2, 2*25 cm, 5 um | EtOH in Hex (0.1% FA) | 423 |
| 527 | 812a | 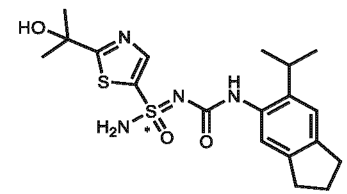 | (S) or (R)-2-(2-hydroxypropan-2-yl)-N'-((6-isopropyl-2,3-dihydro-1H-inden-5 -yl) carbamoyl)thiazole-5 - sulfonimidamide | Chiralpa k ID-2, 2*25 cm, 5 um | EtOH in Hex (0.1% FA) | 423 |
| 528 | 810b | 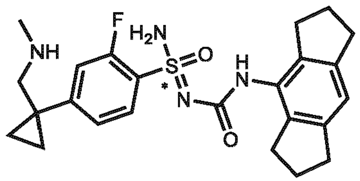 | (R) or (S)-2-fluoro-N'-((1,2,3,5,6,7-hexahydro-*s*-indacen-4-yl)carbamoyl)-4-(1-((methylamino)methyl)cyclopropyl)benzenesulfonimidamide | CHIRA LPAK IG, 2.0*25 cm (5 um) | EtOH in MTBE (10 mM $NH_3$·MeOH ) | 457 |
| 529 | 810a | 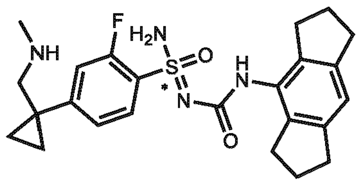 | (S) or (R)-2-fluoro-N'-((1,2,3,5,6,7-hexahydro-*s*-indacen-4-yl)carbamoyl)-4-(1-((methylamino)methyl)cyclopropyl)benzenesulfonimidamide | CHIRA LPAK IG, 2.0*25 cm (5 um) | EtOH in MTBE (10 mM $NH_3$·MeOH ) | 457 |
| 530 | 811b | 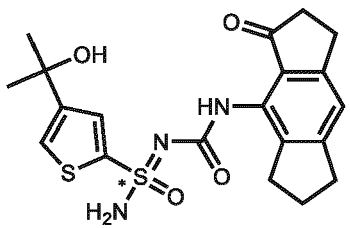 | (R) or (S)-4-(2-hydroxypropan-2-yl)-N'-((3-oxo-1,2,3,5,6,7-hexahydro-*s*-indacen-4-yl) carbamoyl)thiophene-2-sulfonimidamide | CHIRA LPAK IG, 5*25 cm, 5 um | EtOH in Hex (0.1% FA) | 434 |

(continued)

| Ex. # | Cmpd # | Structure | IUPAC Name | Column | Eluents | LC-MS $[M+H]^+$ |
|---|---|---|---|---|---|---|
| 531 | 811a | 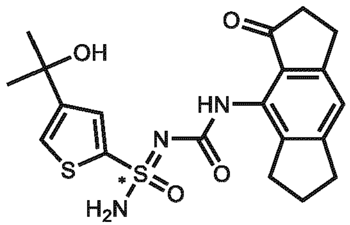 | (S) or (R)-4-(2-hydroxypropan-2-yl)-N'-((3-oxo-1,2,3,5,6,7-hexahydro-*s*-indacen-4-yl) carbamoyl)thiophene-2-sulfonimidamide | CHIRA LPAK IG, 5*25 cm, 5 um | EtOH in Hex (0.1% FA) | 434 |
| 532 | 805a | 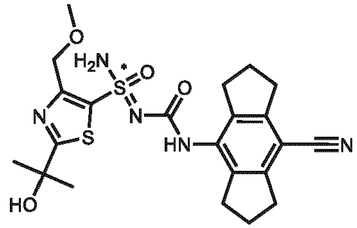 | (S) or (R)-N'-((8-cyano-1,2,3,5,6,7-hexahydro-*s*-indacen-4-yl)carbamoyl)-2-(2-hydroxypropan-2-yl)-4-(methoxymethyl) thiazole-5-sulfonimidamide | CHIRA L ART Cellulos e-SB, 2*25 cm, 5 um | EtOH in Hex (0.1% FA) | 490 |
| 533 | 805b | 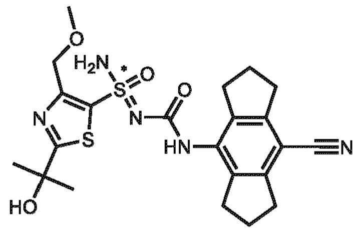 | (R) or (S)-N'-((8-cyano-1,2,3,5,6,7-hexahydro-*s*-indacen-4-yl)carbamoyl)-2-(2-hydroxypropan-2-yl)-4-(methoxymethyl) thiazole-5-sulfonimidamide | CHIRA L ART Cellulos e-SB, 2*25 cm, 5 um | EtOH in Hex (0.1% FA) | 490 |
| 534 | 871b | 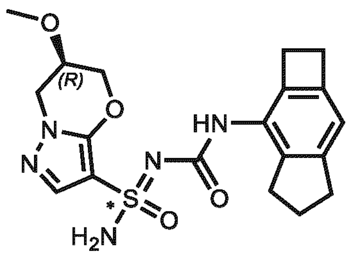 | (R,6R) or (S,6R)-6-methoxy-N'-((2,4,5,6-tetrahydro-1H-cyclobuta[f]inden-3 yl) carbamoyl)-6,7-dihydro-5H-pyrazolo[5,1-b][1,3]oxazine-3-sulfonimidamide | CHIRA LPAK ID, 2*25 cm (5 um) | EtOH in Hex: DCM= 5:1 (10mM $NH_3$·MeOH ) | 418 |

(continued)

| Ex. # | Cmpd # | Structure | IUPAC Name | Column | Eluents | LC-MS [M+H]+ |
|---|---|---|---|---|---|---|
| 535 | 871a | 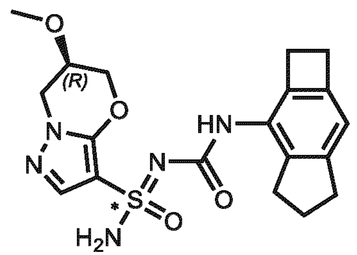 | (S,6R) or (R,6R)-6-methoxy-N'-((2,4,5,6-tetrahydro-1H-cyclobuta[f]inden-3-yl) carbamoyl)-6,7-dihydro-5H-pyrazolo[5,1-b] [1,3]oxazine-3-sulfonimidamide | CHIRA LPAK ID, 2*25 cm (5 um) | EtOH in Hex: DCM= 5:1 (10mM $NH_3$·MeOH ) | 418 |
| 536 | 814b | 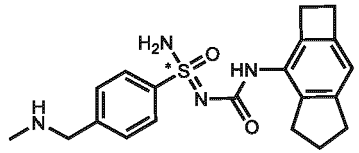 | (S) or (R)-4-((methylamino) methyl)-N'-((2,4,5,6-tetrahydro-1H-cyclobuta [f]inden-3-yl)carbamoyl) benzenesulfonimidamide | chiralpa k AS-H, 2*25 cm (5 um) | 43% IPA in $CO_2$ | 385 |
| 537 | 814a | 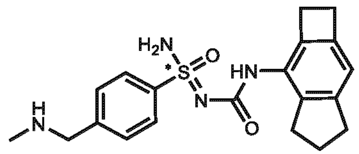 | (R) or (S)-4-((methylamino) methyl)-N'-((2,4,5,6-tetrahydro-1H-cyclobuta [f]inden-3-yl)carbamoyl) benzenesulfonimidamide | chiralpa k AS-H, 2*25 cm (5 um) | 43% IPA in $CO_2$ | 385 |
| 538 | 815b | 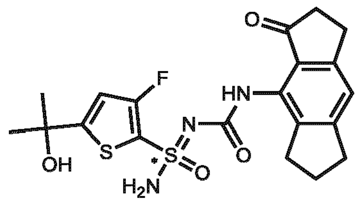 | (S) or (R)-3-fluoro-5-(2-hydroxypropan-2-yl)-N'-((3 -oxo-1,2,3,5,6,7-hexahydro-*s*-indacen-4-yl)carbamoyl)thiophene-2-sulfonimidamide | CHIRA LPAK AS-H, 2.0*25 cm (5 um) | 43% IPA in $CO_2$ | 452 |
| 539 | 815a | 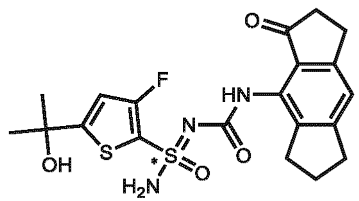 | (R) or (S)-3-fluoro-5-(2-hydroxypropan-2-yl)-N'-((3 -oxo-1,2,3,5,6,7-hexahydro-*s*-indacen-4-yl)carbamoyl)thiophene-2-sulfonimidamide | CHIRA LPAK AS-H, 2.0*25 cm (5 um) | 43% IPA in $CO_2$ | 452 |

(continued)

| Ex. # | Cmpd # | Structure | IUPAC Name | Column | Eluents | LC-MS [M+H]+ |
|---|---|---|---|---|---|---|
| 540 | 866a | 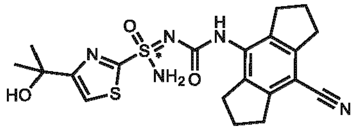 | (S) or (R)-N'-((8-cyano-1,2,3,5,6,7-hexahydro-*s*-indacen-4-yl)carbamoyl)-4-(2-hydroxypropan-2-yl)thiazole-2-sulfonimidamide | CHIRA L ART Cellulos e-SB, 2*25 cm, 5 um | EtOH in Hex (0.1% FA) | 446 |
| 541 | 866b | 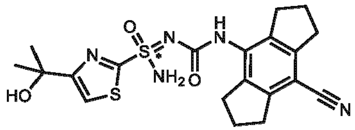 | (R) or (S)-N'-((8-cyano-1,2,3,5,6,7-hexahydro-*s*-indacen-4-yl)carbamoyl)-4-(2-hydroxypropan-2-yl)thiazole-2-sulfonimidamide | CHIRA L ART Cellulos e-SB, 2*25 cm, 5 um | EtOH in Hex (0.1% FA) | 446 |
| 542 | 820b | 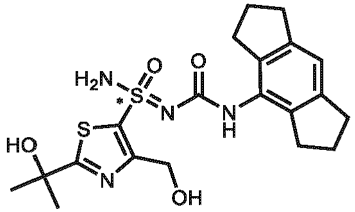 | (S) or (R)-N'-((1,2,3,5,6,7-hexahydro-*s*-indacen-4-yl)carbamoyl)-4-(hydroxymethyl)-2-(2-hydroxypropan-2-yl) thiazole-5-sulfonimidamide | CHIRA L ART Cellulos e-SB, 2*25 cm, 5 um | EtOH in Hex (0.1% FA) | 451 |
| 543 | 820a | 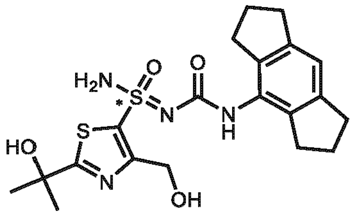 | (R) or (S)-N'-((1,2,3,5,6,7-hexahydro-*s*-indacen-4-yl)carbamoyl)-4-(hydroxymethyl)-2-(2-hydroxypropan-2-yl) thiazole-5-sulfonimidamide | CHIRA L ART Cellulos e-SB, 2*25 cm, 5 um | EtOH in Hex (0.1% FA) | 451 |
| 544 | 822b | 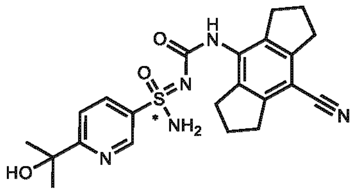 | (S) or (R)-N'-((8-cyano-1,2,3,5,6,7-hexahydro-*s*-indacen-4-yl)carbamoyl)-6-(2-hydroxypropan-2-yl)pyridine-3-sulfonimidamide | CHIRA L ART Cellulos e-SB, 2*25 cm, 5 um | EtOH in Hex (0.1% FA) | 440 |

(continued)

| Ex. # | Cmpd # | Structure | IUPAC Name | Column | Eluents | LC-MS $[M+H]^+$ |
|---|---|---|---|---|---|---|
| 545 | 822a | 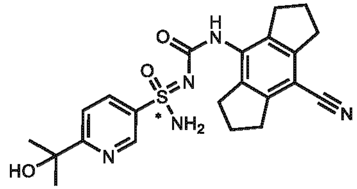 | (R) or (S)-N'-((8-cyano-1,2,3,5,6,7-hexahydro-s-indacen-4-yl)carbamoyl)-6-(2-hydroxypropan-2-yl)pyridine-3-sulfonimidamide | CHIRA L ART Cellulos e-SB, 2*25 cm, 5 um | EtOH in Hex (0.1% FA) | 440 |
| 546 | 501b | 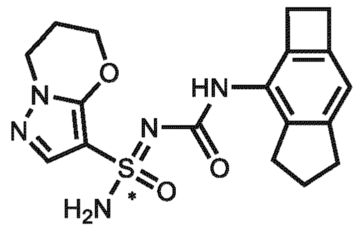 | (R) or (S)-N'-((2,4,5,6-tetrahydro-1H-cyclobuta[f]inden-3-yl)carbamoyl)-6,7-dihydro-5H-pyrazolo [5,1-b][1,3]oxazine-3-sulfonimidamide **COMPOUND ACCORDING TO THE INVENTION** | CHIRA LPAK ID, 2*25 cm (5 um) | IPA in MTBE (0.1% FA) | 388 |
| 547 | 501a | 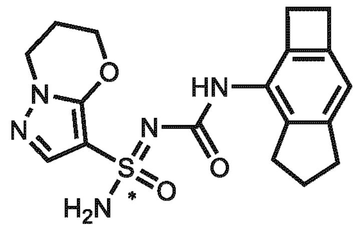 | (S) or (R)-N'-((2,4,5,6-tetrahydro-1H-cyclobuta[f]inden-3-yl)carbamoyl)-6,7-dihydro-5H-pyrazolo [5,1-b][1,3]oxazine-3-sulfonimidamide **COMPOUND ACCORDING TO THE INVENTION** | CHIRA LPAK ID, 2*25 cm (5 um) | IPA in MTBE (0.1% FA) | 388 |
| 548 | 817b | 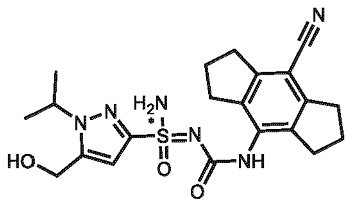 | (R) or (S)-N'-((8-cyano-1,2,3,5,6,7-hexahydro-*s*-indacen-4-yl)carbamoyl)-5-(hydroxymethyl)-1-isopropyl-1H-pyrazole-3 -sulfonimidamide | CHIRA LPAK IC, 2*25 cm, 5 um | EtOH in MTBE (0.1% FA) | 443 |

(continued)

| Ex. # | Cmpd # | Structure | IUPAC Name | Column | Eluents | LC-MS $[M+H]^+$ |
|---|---|---|---|---|---|---|
| 549 | 817a | 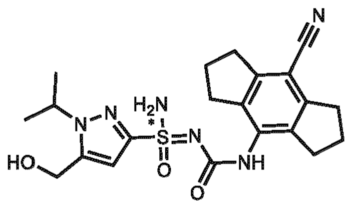 | (S) or (R)-N'-((8-cyano-1,2,3,5,6,7-hexahydro-*s*-indacen-4-yl)carbamoyl)-5-(hydroxymethyl)-1-isopropyl-1H-pyrazole-3-sulfonimidamide | CHIRA LPAK IC, 2*25 cm, 5 um | EtOH in MTBE (0.1% FA) | 443 |
| 550 | 832b | 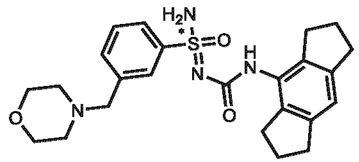 | (R) or (S)-N'-((1,2,3,5,6,7-hexahydro-*s*-indacen-4-yl)carbamoyl)-3-(morpholinomethyl)benzenesulfonimidamide | CHIRA LPAK ID, 0.46*10 cm; 3 um | EtOH in Hex (0.1% FA) | 455 |
| 551 | 832a | 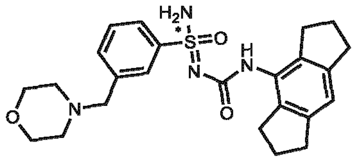 | (S) or (R)-N'-((1,2,3,5,6,7-hexahydro-*s*-indacen-4-yl)carbamoyl)-3-(morpholinomethyl)benzenesulfonimidamide | CHIRA LPAK ID, 0.46*10 cm; 3 um | EtOH in Hex (0.1% FA) | 455 |
| 552 | 821a | 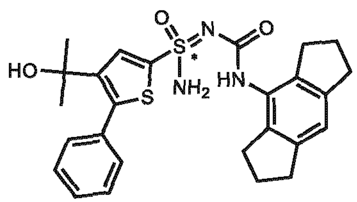 | (R) or (S)-N'-((1,2,3,5,6,7-hexahydro-*s*-indacen-4-yl)carbamoyl)-4-(2-hydroxypropan-2-yl)-5-phenylthiophene-2-sulfonimidamide | CHIRA LPAK IG, 20*250 mm, 5 um | EtOH in Hex (0.1% FA) | 496 |
| 553 | 821b | 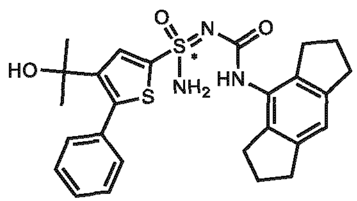 | (S) or (R)-N'-((1,2,3,5,6,7-hexahydro-*s*-indacen-4-yl)carbamoyl)-4-(2-hydroxypropan-2-yl)-5-phenylthiophene-2-sulfonimidamide | CHIRA LPAK IG, 20*250 mm, 5 um | EtOH in Hex (0.1% FA) | 496 |

(continued)

| Ex. # | Cmpd # | Structure | IUPAC Name | Column | Eluents | LC-MS $[M+H]^+$ |
|---|---|---|---|---|---|---|
| 554 | 833b | 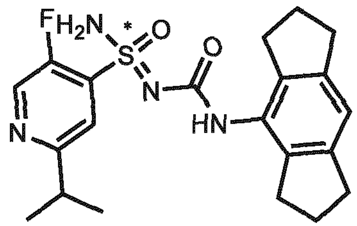 | (S) or (R)-5-fluoro-N'-((1,2,3,5,6,7-hexahydro-s-indacen-4-yl)carbamoyl)-2-isopropylpyridine-4-sulfonimidamide | CHIRA L ART Cellulos e-SB, 2*25 cm, 5 um | EtOH in Hex (0.1% FA) | 417 |
| 555 | 833a | 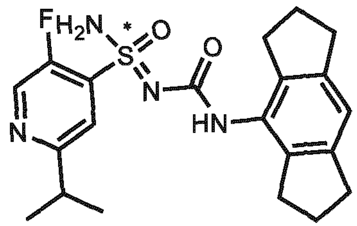 | (R) or (S)-5-fluoro-N'-((1,2,3,5,6,7-hexahydro-*s*-indacen-4-yl)carbamoyl)-2-isopropylpyridine-4-sulfonimidamide | CHIRA L ART Cellulos e-SB, 2*25 cm, 5 um | EtOH in Hex (0.1% FA) | 417 |
| 556 | 834b | 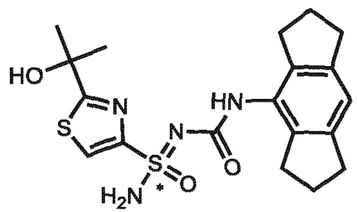 | (S) or (R)-N'-((1,2,3,5,6,7-hexahydro-*s*-indacen-4-yl)carbamoyl)-2-(2-hydroxypropan-2-yl)thiazole-4-sulfonimidamide | (R,R)-WHELK -O1-Kromasi, 5*25 cm (5 um) | IPA in Hex (8 mM $NH_3$·MeOH ) | 421 |
| 557 | 834a | 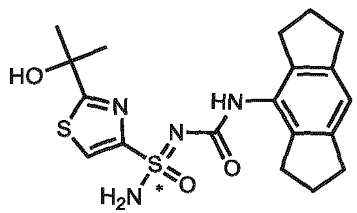 | (R) or (S)-N'-((1,2,3,5,6,7-hexahydro-*s*-indacen-4-yl)carbamoyl)-2-(2-hydroxypropan-2-yl)thiazole-4-sulfonimidamide | (R,R)-WHELK -O1-Kromasi, 5*25 cm (5 um) | IPA in Hex (8 mM $NH_3$·MeOH ) | 421 |

(continued)

| Ex. # | Cmpd # | Structure | IUPAC Name | Column | Eluents | LC-MS $[M+H]^+$ |
|---|---|---|---|---|---|---|
| 558 | 413a | 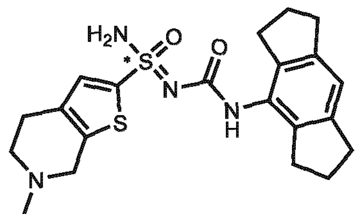 | (R) or (S)-N'-((1,2,3,5,6,7-hexahydro-*s*-indacen-4-yl)carbamoyl)-6-methyl-4,5,6,7-tetrahydrothieno[2,3-c]pyridine-2-sulfonimidamide | Chiralpa k ID, 2*25 cm, 5 um | EtOH in Hex (8 mM $NH_3$·MeOH ) | 431 |
| 559 | 413b | 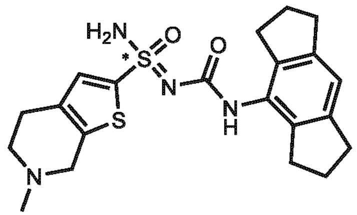 | (S) or (R)-N'-((1,2,3,5,6,7-hexahydro-*s*-indacen-4-yl)carbamoyl)-6-methyl-4,5,6,7-tetrahydrothieno[2,3-c]pyridine-2-sulfonimidamide | Chiralpa k ID, 2*25 cm, 5 um | EtOH in Hex (8 mM $NH_3$·MeOH ) | 431 |
| 560 | 835b | 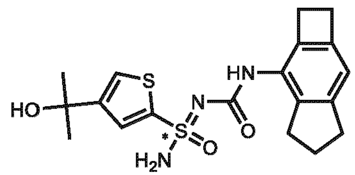 | (R) or (S)-4-(2-hydroxypropan-2-yl)-N'-((2,4,5,6-tetrahydro-1H-cyclobuta[f]inden-3-yl)carbamoyl)thiophene-2-sulfonimidamide | CHIRA LPAK IF, 2*25 cm, 5 um | 40% MeOH (2 mM $NH_3$·MeOH ) in $CO_2$ | 406 |
| 561 | 835a | 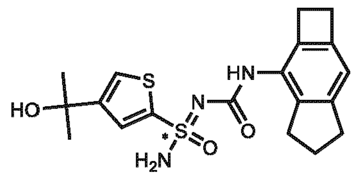 | (S) or (R)-4-(2-hydroxypropan-2-yl)-N'-((2,4,5,6-tetrahydro-1H-cyclobuta[f]inden-3-yl)carbamoyl)thiophene-2-sulfonimidamide | CHIRA LPAK IF, 2*25 cm, 5 um | 40% MeOH (2 mM $NH_3$·MeOH ) in $CO_2$ | 406 |
| 562 | 836b | 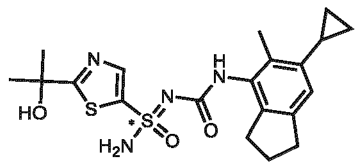 | (S) or (R)-N'-((6-cyclopropyl-5-methyl-2,3-dihydro-1H-inden-4-yl)carbamoyl)-2-(2-hydroxypropan-2-yl)thiazole-5-sulfonimidamide | CHIRA LPAK ID, 2*25 cm (5 um) | EtOH in MTBE (10 mM $NH_3$·MeOH ) | 435 |

(continued)

| Ex. # | Cmpd # | Structure | IUPAC Name | Column | Eluents | LC-MS $[M+H]^+$ |
|---|---|---|---|---|---|---|
| 563 | 836a | 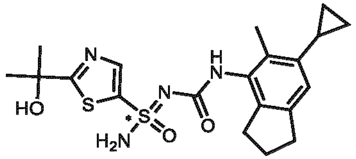 | (R) or (S)-N'-((6-cyclopropyl-5-methyl-2,3-dihydro-1H-inden-4-yl)carbamoyl)-2-(2-hydroxypropan-2-yl)thiazole-5-sulfonimidamide | CHIRA LPAK ID, 2*25 cm (5 um) | EtOH in MTBE (10 mM $NH_3$·MeOH ) | 435 |
| 564 | 837b | 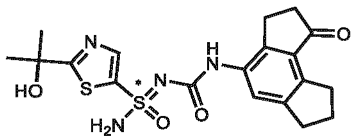 | (S) or (R)-2-(2-hydroxypropan-2-yl)-N'-((1-oxo-1,2,3,6,7,8-hexahydro-*as*-indacen-4-yl) carbamoyl)thiazole-5-sulfonimidamide | CHIRA LPAK IA, 2*25 cm, 5 um | EtOH in Hex (8 mM $NH_3$·MeOH ) | 435 |
| 565 | 837a | 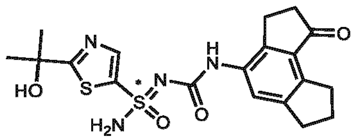 | (R) or (S)-2-(2-hydroxypropan-2-yl)-N'-((1-oxo-1,2,3,6,7,8-hexahydro-as-indacen-4-yl) carbamoyl)thiazole-5-sulfonimidamide | CHIRA LPAK IA, 2*25 cm, 5 um | EtOH in Hex (8 mM $NH_3$·MeOH ) | 435 |
| 566 | 828a | 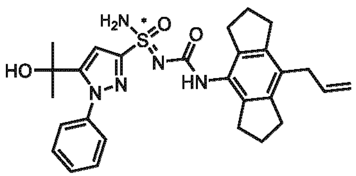 | (R) or (S)-N'-((8-allyl-1,2,3,5,6,7-hexahydro-*s*-indacen-4-yl)carbamoyl)-5-(2-hydroxypropan-2-yl)-1-phenyl-1H-pyrazole-3-sulfonimidamide | CHIRA LPAK IC, 2*25 cm, 5 um | IPA in MTBE (0.1% FA) | 520 |
| 567 | 828b | 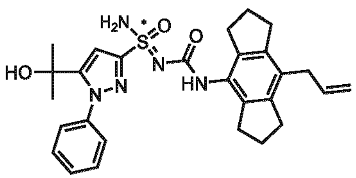 | (S) or (R)-N'-((8-allyl-1,2,3,5,6,7-hexahydro-*s*-indacen-4-yl)carbamoyl)-5-(2-hydroxypropan-2-yl)-1-phenyl-1H-pyrazole-3-sulfonimidamide | CHIRA LPAK IC, 2*25 cm, 5 um | IPA in MTBE (0.1% FA) | 520 |

(continued)

| Ex. # | Cmpd # | Structure | IUPAC Name | Column | Eluents | LC-MS $[M+H]^+$ |
|---|---|---|---|---|---|---|
| 568 | 873b | 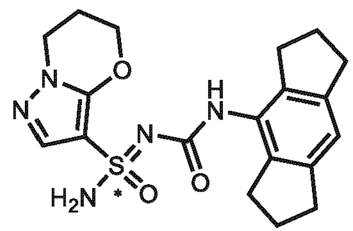 | (S) or (R)-N'-((1,2,3,5,6,7-hexahydro-*s*-indacen-4-yl)carbamoyl)-6,7-dihydro-5H-pyrazolo [5,1-b][1,3]oxazine-3-sulfonimidamide | Chiralpa k AD-H, 2*25 cm (5 um) | 50% EtOH (2 mM $NH_3$·MeOH ) in $CO_2$ | 402 |
| 569 | 873a | 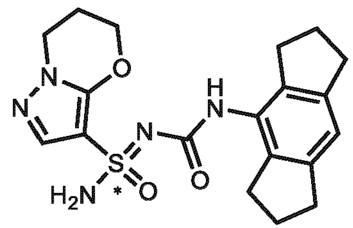 | (R) or (S)-N'-((1,2,3,5,6,7-hexahydro-*s*-indacen-4-yl)carbamoyl)-6,7-dihydro-5H-pyrazolo [5,1-b][1,3]oxazine-3-sulfonimidamide | Chiralpa k AD-H, 2*25 cm (5 um) | 50% EtOH (2 mM $NH_3$·MeOH ) in $CO_2$ | 402 |
| 570 | 412a | 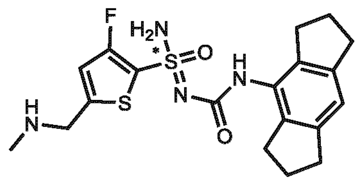 | (R) or (S)-3-fluoro-N'-((1,2,3,5,6,7-hexahydro-*s*-indacen-4-yl)carbamoyl)-5-((methylamino) methyl)thiophene-2-sulfonimidamide | CHIRA LPAK ID, 2*25 cm (5 um) | 40% MeOH:DCM=1:1 (2 mM $NH_3$·MeOH ) in $CO_2$ | 423 |
| 571 | 412b | 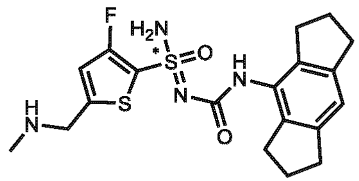 | (S) or (R)-3-fluoro-N'-((1,2,3,5,6,7-hexahydro-*s*-indacen-4-yl)carbamoyl)-5-((methylamino) methyl)thiophene-2-sulfonimidamide | CHIRA LPAK ID, 2*25 cm (5 um) | 40% MeOH:DCM=1:1 (2 mM $NH_3$·MeOH ) in $CO_2$ | 423 |
| 572 | 840b | 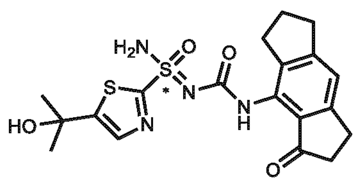 | (R) or (S)-5-(2-hydroxypropan-2-yl)-N'-((3-oxo-1,2,3,5,6,7-hexahydro-*s*-indacen-4-yl) carbamoyl)thiazole-2-sulfonimidamide | CHIRA LPAK AS-H, 2*25 cm (5 um) | 40% IPA (2 mM $NH_3$·MeOH ) in $CO_2$ | 435 |

(continued)

| Ex. # | Cmpd # | Structure | IUPAC Name | Column | Eluents | LC-MS $[M+H]^+$ |
|---|---|---|---|---|---|---|
| 573 | 840a | 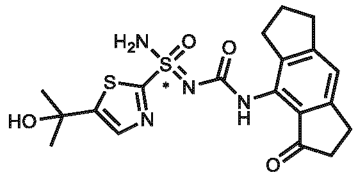 | (S) or (R)-5-(2-hydroxypropan-2-yl)-N'-((3-oxo-1,2,3,5,6,7-hexahydro-*s*-indacen-4-yl) carbamoyl)thiazole-2-sulfonimidamide | CHIRA LPAK AS-H, 2*25 cm (5 um) | 40% IPA (2 mM $NH_3$·MeOH ) in $CO_2$ | 435 |
| 574 | 844b | 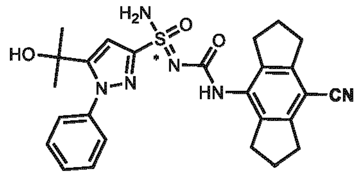 | (S) or (R)-N'-((8-cyano-1,2,3,5,6,7-hexahydro-*s*-indacen-4-yl)carbamoyl)-5-(2-hydroxypropan-2-yl)-1-phenyl-1H-pyrazole-3 -sulfonimidamide | CHIRA L ART Cellulos e-SB, 2*25 cm, 5 um | IPA in MTBE (0.1% FA) | 505 |
| 575 | 844a | 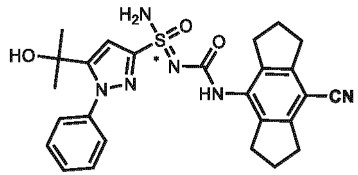 | (R) or (S)-N'-((8-cyano-1,2,3,5,6,7-hexahydro-*s*-indacen-4-yl)carbamoyl)-5-(2-hydroxypropan-2-yl)-1-phenyl-1H-pyrazole-3 -sulfonimidamide | CHIRA L ART Cellulos e-SB, 2*25 cm, 5 um | IPA in MTBE (0.1% FA) | 505 |
| 576 | 841b | 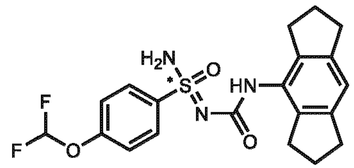 | (R) or (S)- 4-(difluoromethoxy)-N'-((1,2,3,5,6,7-hexahydro-*s*-indacen-4-yl)carbamoyl) benzenesulfonimidamide | CHIRA LPAK IG, 2*25 cm, 5 um | ETOH in Hex (8 mM $NH_3$·MeOH | 422 |
| 577 | 841a | 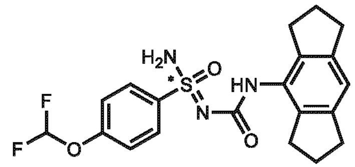 | (S) or (R)- 4-(difluoromethoxy)-N'-((1,2,3,5,6,7-hexahydro-*s*-indacen-4-yl)carbamoyl) benzenesulfonimidamide | CHIRA LPAK IG, 2*25 cm, 5 um | EtOH in Hex (8 mM $NH_3$·MeOH ) | 422 |

(continued)

| Ex. # | Cmpd # | Structure | IUPAC Name | Column | Eluents | LC-MS $[M+H]^+$ |
|---|---|---|---|---|---|---|
| 578 | 849b | 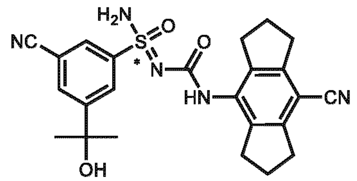 | (R) or (S)-3-cyano-N'-((8-cyano-1,2,3,5,6,7-hexahydro-*s*-indacen-4-yl)carbamoyl)-5-(2-hydroxypropan-2-yl)benzenesulfonimidamide | CHIRA LPAK IC, 2*25 cm, 5 um | EtOH in Hex (8 mM $NH_3 \cdot MeOH$ ) | 464 |
| 579 | 849a | 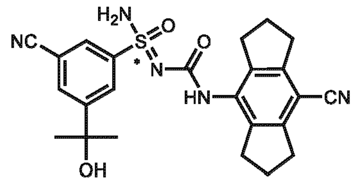 | (S) or (R)-3-cyano-N'-((8-cyano-1,2,3,5,6,7-hexahydro-*s*-indacen-4-yl)carbamoyl)-5-(2-hydroxypropan-2-yl)benzenesulfonimidamide | CHIRA LPAK IC, 2*25 cm, 5 um | EtOH in Hex (8 mM $NH_3 \cdot MeOH$ ) | 464 |
| 580 | 846b | 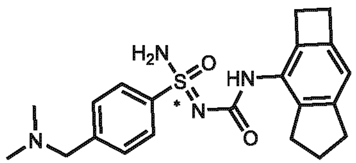 | (R) or (S)-4-((dimethylamino) methyl)-N'-((2,4,5,6-tetrahydro-1H-cyclobuta [f]inden-3-yl)carbamoyl) benzenesulfonimidamide | Chiralpa k ID, 2*25 cm, 5 um | EtOH in Hex (8 mM $NH_3 \cdot MeOH$ ) | 399 |
| 581 | 846a | 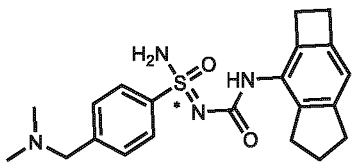 | (S) or (R)-4-((dimethylamino) methyl)-N'-((2,4,5,6-tetrahydro-1H-cyclobuta [f]inden-3-yl)carbamoyl) benzenesulfonimidamide | Chiralpa k ID, 2*25 cm, 5 um | EtOH in Hex (8 mM $NH_3 \cdot MeOH$ ) | 399 |
| 582 | 845a | 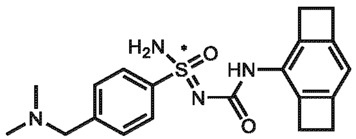 | (R) or (S)-4-((dimethylamino) methyl)-N'-(tricyclo [62.0.03,6] deca-1,3 (6),7-trien-2-ylcarbamoyl)benzenesulfonimidamide | CHIRALPAKID2*25 cm, 5 um | 30% EtOH in Hex (0.1% DEA) | 385 |
| 583 | 845b | 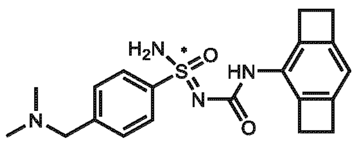 | (S) or (R)-4-((dimethylamino) methyl)-N'-(tricyclo [62.0.03,6] deca-1,3 (6),7-trien-2-ylcarbamoyl)benzenesulfonimidamide | CHIRALPAKID2*25 cm, 5 um | 30% EtOH in Hex (0.1% DEA) | 385 |

(continued)

| Ex. # | Cmpd # | Structure | IUPAC Name | Column | Eluents | LC-MS $[M+H]^+$ |
|---|---|---|---|---|---|---|
| 584 | 349b | 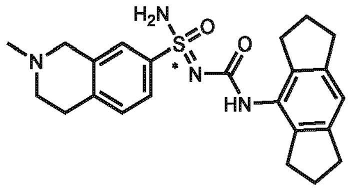 | (S) or (R)-N'-((1,2,3,5,6,7-hexahydro-*s*-indacen-4-yl)carbamoyl)-2-methyl-1,2,3,4-tetrahydroisoquinoline-7-sulfonimidamide | (R,R)Whelk, 21.1*250mm, 5 um | EtOH in Hex (8 mM $NH_3$·MeOH ) | 425 |
| 585 | 349a | 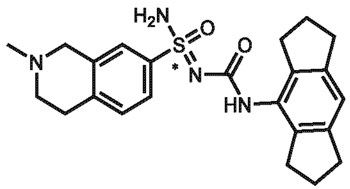 | (R) or (S)-N'-((1,2,3,5,6,7-hexahydro-*s*-indacen-4-yl)carbamoyl)-2-methyl-1,2,3,4-tetrahydroisoquinoline-7-sulfonimidamide | (R,R)Whelk, 21.1*250mm, 5 um | EtOH in Hex (8 mM $NH_3$·MeOH ) | 425 |
| 586 | 850b | 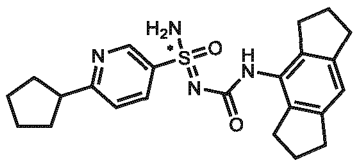 | (S) or (R)-6-cyclopentyl-N'-((1,2,3,5,6,7-hexahydro-*s*-indacen-4-yl)carbamoyl) pyridine-3-sulfonimidamide | CHIRAL ART Cellulos e-SB, 2*25 cm, 5 um | EtOH in Hex (0.1% FA) | 425 |
| 587 | 850a | 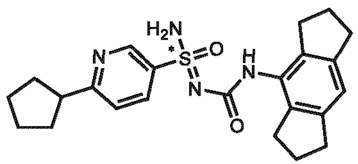 | (R) or (S)-6-cyclopentyl-N'-((1,2,3,5,6,7-hexahydro-*s*-indacen-4-yl)carbamoyl) pyridine-3-sulfonimidamide | CHIRAL ART Cellulos e-SB, 2*25 cm, 5 um | EtOH in Hex (0.1% FA) | 425 |
| 588 | 851b | 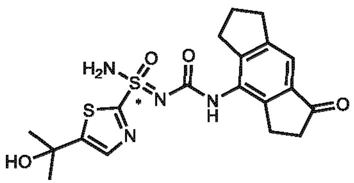 | (S) or (R)-5-(2-hydroxypropan-2-yl)-N'-((1-oxo-1,2,3,5,6,7-hexahydro-*s*-indacen-4-yl) carbamoyl)thiazole-2-sulfonimidamide | CHIRAL ART Cellulos e-SB, 2*25 cm, 5 um | EtOH in Hex (0.1% FA) | 435 |

(continued)

| Ex. # | Cmpd # | Structure | IUPAC Name | Column | Eluents | LC-MS $[M+H]^+$ |
|---|---|---|---|---|---|---|
| 589 | 851a | 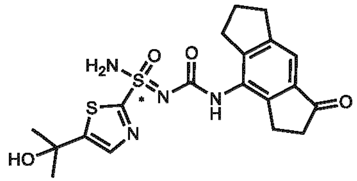 | (R) or (S)-5-(2-hydroxypropan-2-yl)-N'-((1-oxo-1,2,3,5,6,7-hexahydro-*s*-indacen-4-yl) carbamoyl)thiazole-2-sulfonimidamide | CHIRA L ART Cellulos e-SB, 2*25 cm, 5 um | EtOH in Hex (0.1% FA) | 435 |
| 590 | 852b | 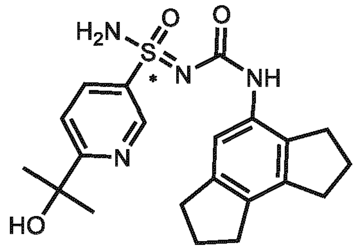 | (S) or (R)-N'-((1,2,3,6,7,8-hexahydro-*as*-indacen-4-yl)carbamoyl)-6-(2-hydroxypropan-2-yl)pyridine-3 -sulfonimidamide | (R,R)W helk, 21.1*25 0 mm, 5 um | EtOH in Hex (0.1% FA) | 415 |
| 591 | 852a | 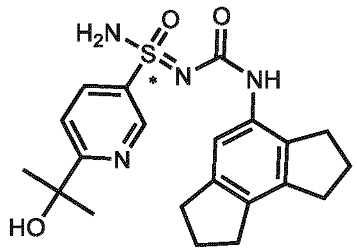 | (R) or (S)-N'-((1,2,3,6,7,8-hexahydro-*as*-indacen-4-yl)carbamoyl)-6-(2-hydroxypropan-2-yl)pyridine-3 -sulfonimidamide | (R,R)W helk, 21.1*25 0 mm, 5 um | EtOH in Hex (0.1% FA) | 415 |
| 592 | 853b | 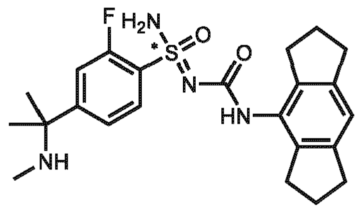 | (S) or (R)-2-fluoro-N'-((1,2,3,5,6,7-hexahydro-*s*-indacen-4-yl)carbamoyl)-4-(2-(methylamino)propan-2-yl) benzenesulfonimidamide | (R,R)W helk, 21.1*25 0 mm, 5 um | EtOH in Hex (8 mM $NH_3$·MeOH ) | 445 |

(continued)

| Ex. # | Cmpd # | Structure | IUPAC Name | Column | Eluents | LC-MS $[M+H]^+$ |
|---|---|---|---|---|---|---|
| 593 | 853a | 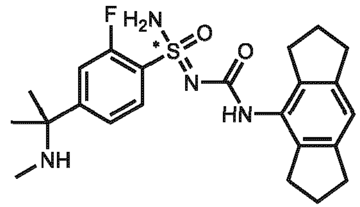 | (R) or (S)-2-fluoro-N'-((1,2,3,5,6,7-hexahydro-*s*-indacen-4-yl)carbamoyl)-4-(2-(methylamino)propan-2-yl) benzenesulfonimidamide | (R,R)W helk, 21.1 *25 0 mm, 5 um | EtOH in Hex (8 mM $NH_3$·MeOH ) | 445 |
| 594 | 855b | 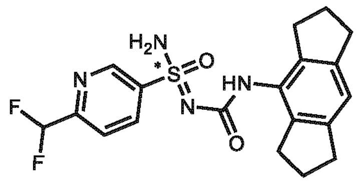 | (S) or (R)-6-(difluoromethyl)-N'-((1,2,3,5,6,7-hexahydro-*s*-indacen-4-yl)carbamoyl) pyridine-3-sulfonimidamide | CHIRA L ART Cellulos e-SB, 2*25 cm, 5 um | EtOH in Hex (8 mM $NH_3$·MeOH ) | 407 |
| 595 | 855a | 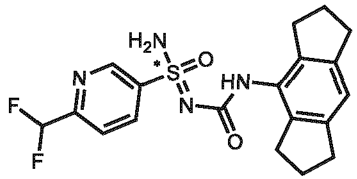 | (R) or (S)-6-(difluoromethyl)-N'-((1,2,3,5,6,7-hexahydro-*s*-indacen-4-yl)carbamoyl) pyridine-3-sulfonimidamide | CHIRA L ART Cellulos e-SB, 2*25 cm, 5 um | EtOH in Hex (8 mM $NH_3$·MeOH ) | 407 |
| 596 | 857b | 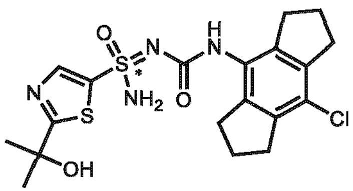 | (S) or (R)-N'-((8-chloro-1,2,3,5,6,7-hexahydro-*s*-indacen-4-yl)carbamoyl)-2-(2-hydroxypropan-2-yl)thiazole-5-sulfonimidamide | Chiralpa k ID, 2*25 cm, 5 um | IPA in MTBE (10 mM $NH_3$·MeOH ) | 455/457 |
| 597 | 857a | 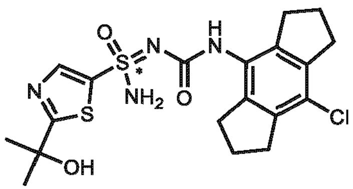 | (R) or (S)-N'-((8-chloro-1,2,3,5,6,7-hexahydro-*s*-indacen-4-yl)carbamoyl)-2-(2-hydroxypropan-2-yl)thiazole-5-sulfonimidamide | Chiralpa k ID, 2*25 cm, 5 um | IPA in MTBE (10 mM $NH_3$·MeOH ) | 455/457 |

(continued)

| Ex. # | Cmpd # | Structure | IUPAC Name | Column | Eluents | LC-MS $[M+H]^+$ |
|---|---|---|---|---|---|---|
| 598 | 858b | 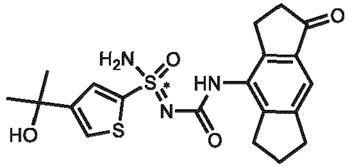 | (R) or (S)-4-(2-hydroxypropan-2-yl)-N'-((1-oxo-1,2,3,5,6,7-hexahydro-*s*-indacen-4-yl) carbamoyl)thiophene-2-sulfonimidamide | CHIRA LPAK IE, 2*25 cm, 5 um | EtOH in Hex (8 mM $NH_3$·MeOH ) | 434 |
| 599 | 858a | 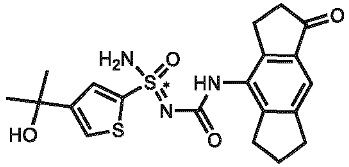 | (S) or (R)-4-(2-hydroxypropan-2-yl)-N'-((1-oxo-1,2,3,5,6,7-hexahydro-*s*-indacen-4-yl) carbamoyl)thiophene-2-sulfonimidamide | CHIRA LPAK IE, 2*25 cm, 5 um | EtOH in Hex (8 mM $NH_3$·MeOH ) | 434 |
| 600 | 859b | 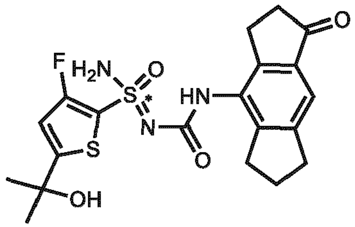 | (R) or (S)-3-fluoro-5-(2-hydroxypropan-2-yl)-N'-((1-oxo-1,2,3,5,6,7-hexahydro-*s*-indacen-4-yl)carbamoyl)thiophene-2-sulfonimidamide | CHIRA L ART Cellulos e-SB, 2*25 cm, 5 um | EtOH in Hex (0.1% FA) | 452 |
| 601 | 859a | 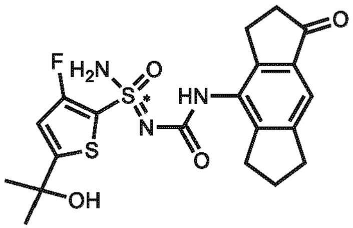 | (S) or (R)-3-fluoro-5-(2-hydroxypropan-2-yl)-N'-((1-oxo-1,2,3,5,6,7-hexahydro-*s*-indacen-4-yl)carbamoyl)thiophene-2-sulfonimidamide | CHIRA L ART Cellulos e-SB, 2*25 cm, 5 um | EtOH in Hex (0.1% FA) | 452 |

(continued)

| Ex. # | Cmpd # | Structure | IUPAC Name | Column | Eluents | LC-MS $[M+H]^+$ |
|---|---|---|---|---|---|---|
| 602 | 860b | 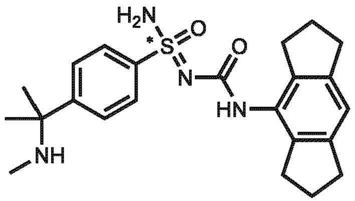 | (R) or (S)-N'-((1,2,3,5,6,7-hexahydro-*s*-indacen-4-yl)carbamoyl)-4-(2-(methylamino)propan-2-yl) benzenesulfonimidamide | CHIRA LPAK IG, 20*250 mm, 5 um | IPA in MTBE (10 mM $NH_3$·MeOH ) | 427 |
| 603 | 860a | 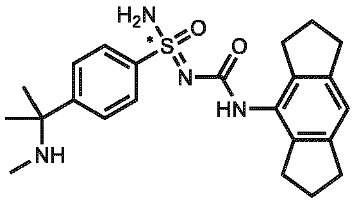 | (S) or (R)-N'-((1,2,3,5,6,7-hexahydro-*s*-indacen-4-yl)carbamoyl)-4-(2-(methylamino)propan-2-yl) benzenesulfonimidamide | CHIRA LPAK IG, 20*250 mm, 5 um | IPA in MTBE (10 mM $NH_3$·MeOH ) | 427 |
| 604 | 377b | 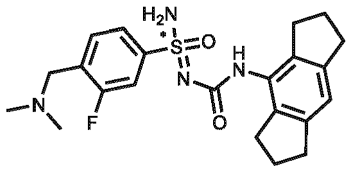 | (S) or (R)-4-((dimethylamino)methyl)-3-fluoro-N'-((1,2,3,5,6,7-hexahydro-*s*-indacen-4-yl) carbamoyl)benzenesulfonimidamide | CHIRA L ART Cellulos e-SB, 2*25 cm, 5 um | EtOH in Hex (8 mM $NH_3$·MeOH ) | 431 |
| 605 | 377a | 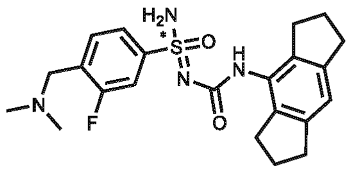 | (R) or (S)-4-((dimethylamino)methyl)-3-fluoro-N'-((1,2,3,5,6,7-hexahydro-*s*-indacen-4-yl) carbamoyl)benzenesulfonimidamide | CHIRA L ART Cellulos e-SB, 2*25 cm, 5 um | EtOH in Hex (8 mM $NH_3$·MeOH ) | 431 |
| 606 | 344a | 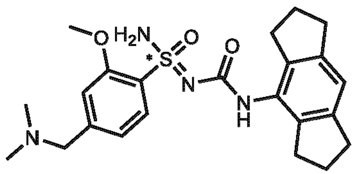 | (R) or (S)-4-((dimethylamino) methyl)-N'-((1,2,3,5,6,7-hexahydro-*s*-indacen-4-yl)carbamoyl)-2-methoxybenzenesulfonimidamide | CHIRA LPAK IG, 20*250 mm, 5 um | EtOH in Hex (2 mM $NH_3$) | 443 |

(continued)

| Ex. # | Cmpd # | Structure | IUPAC Name | Column | Eluents | LC-MS $[M+H]^+$ |
|---|---|---|---|---|---|---|
| 607 | 344b | 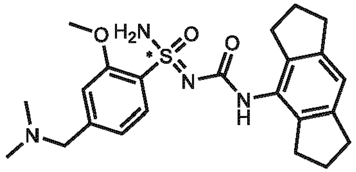 | (S) or (R)-4-((dimethylamino) methyl)-N'-((1,2,3,5,6,7-hexahydro-*s*-indacen-4-yl)carbamoyl)-2-methoxybenzenesulfonimidamide | CHIRA LPAK IG, 20*250 mm, 5 um | EtOH in Hex (2 mM $NH_3$) | 443 |
| 608 | 861b | 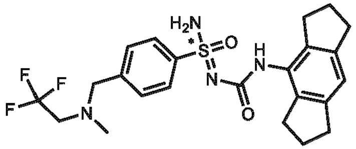 | (R) or (R)-N'-((1,2,3,5,6,7-hexahydro-*s*-indacen-4-yl)carbamoyl)-4-((methyl(2,2,2-trifluoroethyl)amino)methyl)benzenesu lfonimidamide | CHIRA LPAK IG, 2*25 cm (5 um) | EtOH in Hex (8 mM $NH_3$·MeOH ) | 481 |
| 609 | 861a | 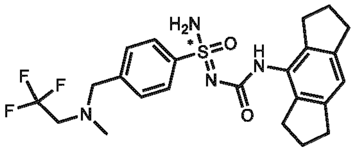 | (S) or (R)-N'-((1,2,3,5,6,7-hexahydro-*s*-indacen-4-yl)carbamoyl)-4-((methyl(2,2,2-trifluoroethyl)amino)methyl)benzenesu lfonimidamide | CHIRA LPAK IG, 2*25 cm (5 um) | EtOH in Hex (8 mM $NH_3$·MeOH ) | 481 |
| 610 | 322a | 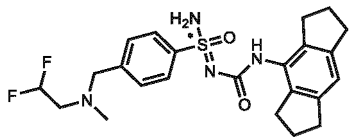 | (R) or (S)-4-(((2,2-difluoroethyl)(methyl) amino)methyl)-N'-((1,2,3,5,6,7-hexahydro-*s*-indacen-4-yl)carbamoyl) benzenesulfonimidamide | CHIRA LPAK IF, 2*25 cm, 5 um | EtOH in Hex (8 mM $NH_3$·MeOH ) | 463 |
| 611 | 322b | 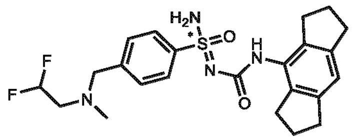 | (S) or (R)-4-(((2,2-difluoroethyl)(methyl) amino)methyl)-N'-((1,2,3,5,6,7-hexahydro-*s*-indacen-4-yl)carbamoyl) benzenesulfonimidamide | CHIRA LPAK IF, 2*25 cm, 5 um | EtOH in Hex (8 mM $NH_3$·MeOH ) | 463 |

(continued)

| Ex. # | Cmpd # | Structure | IUPAC Name | Column | Eluents | LC-MS $[M+H]^+$ |
|---|---|---|---|---|---|---|
| 612 | 862b | 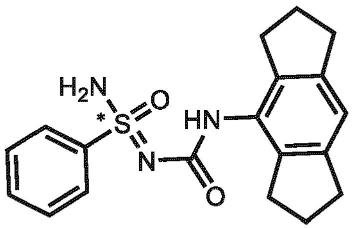 | (R) or (S)-N'-((1,2,3,5,6,7-hexahydro-*s*-indacen-4-yl)carbamoyl) benzenesulfonimidamide | CHIRA LPAK IA, 2*25 cm, 5 um | EtOH in Hex (8 mM $NH_3$·MeOH ) | 356 |
| 613 | 862a | 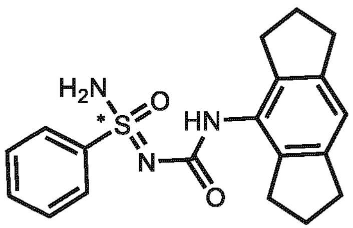 | (S) or (R)-N'-((1,2,3,5,6,7-hexahydro-*s*-indacen-4-yl)carbamoyl) benzenesulfonimidamide | CHIRA LPAK IA, 2*25 cm, 5 um | EtOH in Hex (8 mM $NH_3$·MeOH ) | 356 |
| 614 | 863b | 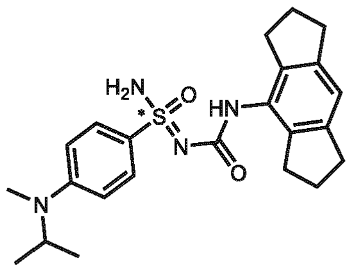 | (S) or (R)-N'-((1,2,3,5,6,7-hexahydro-*s*-indacen-4-yl)carbamoyl)-4-(isopropyl (methyl)amino)benzenesulfo nimidamide | (R,R)W helk, 21.1*25 0 mm, 5 um | EtOH in Hex (8 mM $NH_3$·MeOH ) | 427 |
| 615 | 863a | 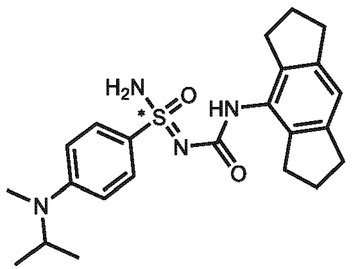 | (R) or (S)-N'-((1,2,3,5,6,7-hexahydro-*s*-indacen-4-yl)carbamoyl)-4-(isopropyl (methyl)amino)benzenesulfo nimidamide | (R,R)W helk, 21.1*25 0 mm, 5 um | EtOH in Hex (8 mM $NH_3$·MeOH ) | 427 |

(continued)

| Ex. # | Cmpd # | Structure | IUPAC Name | Column | Eluents | LC-MS $[M+H]^+$ |
|---|---|---|---|---|---|---|
| 616 | 355b | 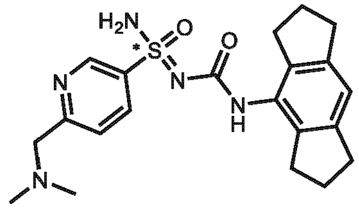 | (S) or (R)-6-((dimethylamino) methyl)-N'-((1,2,3,5,6,7-hexahydro-*s*-indacen-4-yl)carbamoyl)pyridine-3-sulfonimidamide | CHIRA L ART Cellulos e-SB, 2*25 cm, 5 um | EtOH in Hex (8 mM $NH_3$·MeOH ) | 414 |
| 617 | 355a | 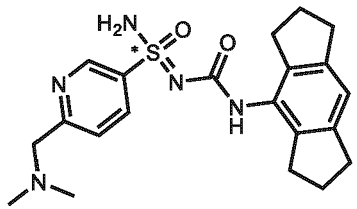 | (R) or (S)-6-((dimethylamino) methyl)-N'-((1,2,3,5,6,7-hexahydro-*s*-indacen-4-yl)carbamoyl)pyridine-3-sulfonimidamide | CHIRA L ART Cellulos e-SB, 2*25 cm, 5 um | EtOH in Hex (8 mM $NH_3$·MeOH ) | 414 |
| 618 | 356b | 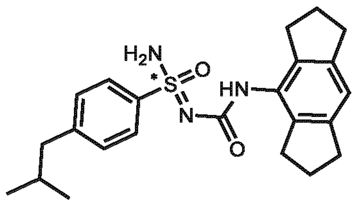 | (S) or (R)-N'-((1,2,3,5,6,7-hexahydro-*s*-indacen-4-yl)carbamoyl)-4-isobutylbenzenesulfonimidamide | CHIRA LPAK IF, 2*25 cm, 5 um | EtOH in Hex (8 mM $NH_3$·MeOH | 412 |
| 619 | 356a | 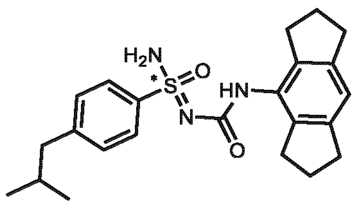 | (R) or (S)-N'-((1,2,3,5,6,7-hexahydro-*s*-indacen-4-yl)carbamoyl)-4-isobutylbenzenesulfonimidamide | CHIRA LPAK IF, 2*25 cm, 5 um | EtOH in Hex (8 mM $NH_3$·MeOH ) | 412 |

(continued)

| Ex. # | Cmpd # | Structure | IUPAC Name | Column | Eluents | LC-MS $[M+H]^+$ |
|---|---|---|---|---|---|---|
| 620 | 346a | 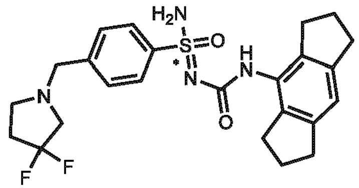 | (R) or (S)-4-((3,3-difluoropyrrolidin-1-yl) methyl)-N'-((1,2,3,5,6,7-hexahydro-*s*-indacen-4-yl)carbamoyl) benzenesulfonimidamide | CHIRA LPAK IF, 2*25 cm, 5 um | EtOH in Hex | 475 |
| 621 | 346b | 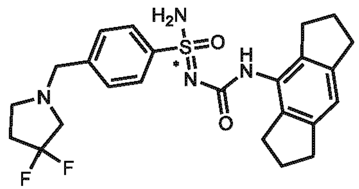 | (S) or (R)-4-((3,3-difluoropyrrolidin-1-yl) methyl)-N'-((1,2,3,5,6,7-hexahydro-*s*-indacen-4-yl)carbamoyl) benzenesulfonimidamide | CHIRA LPAK IF, 2*25 cm, 5 um | EtOH in Hex | 475 |
| 622 | 83 8f | 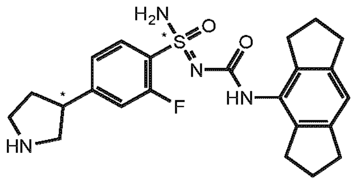 | (R,S) or (S,S) -2-fluoro-N'-((1,2,3,5,6,7-hexahydro-*s*-indacen-4-yl)carbamoyl)-4-(-pyrrolidin-3-yl)benzenesulfonimidamide | CHIRA LPAK IC, 2*25 cm, 5 um; from Ex. 501 | IPA in Hex (0.1% FA) | 443 |
| 623 | 838d | 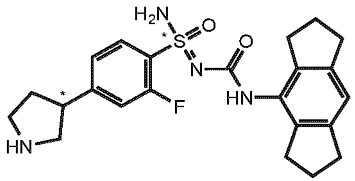 | (S,S) or (R,S) -2-fluoro-N'-((1,2,3,5,6,7-hexahydro-*s*-indacen-4-yl)carbamoyl)-4-(-pyrrolidin-3-yl)benzenesulfonimidamide | CHIRA LPAK IC, 2*25 cm, 5 um; from Ex. 501 | IPA in Hex (0.1% FA) | 443 |
| 624 | 838c | 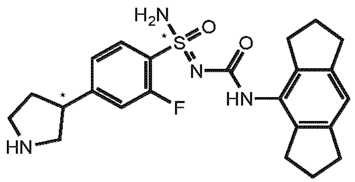 | (R,R) or (S,R) -2-fluoro-N'-((1,2,3,5,6,7-hexahydro-*s*-indacen-4-yl)carbamoyl)-4-pyrrolidin-3-yl)benzenesulfonimidamide | CHIRA L PAK IA, 2*25 cm, 5 um; from Ex. 502 | EtOH in MTBE (0.1% FA) | 443 |

(continued)

| Ex. # | Cmpd # | Structure | IUPAC Name | Column | Eluents | LC-MS $[M+H]^+$ |
|---|---|---|---|---|---|---|
| 625 | 838a | 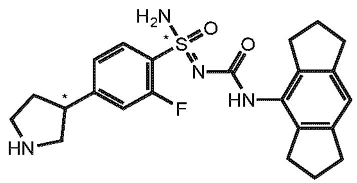 | (S,R) or (R,R) -2-fluoro-N'-((1,2,3,5,6,7-hexahydro-*s*-indacen-4-yl)carbamoyl)-4-pyrrolidin-3-yl)benzenesulfonimidamide | CHIRA L PAK IA, 2*25 cm, 5 um; from Ex. 502 | EtOH in MTBE (0.1% FA) | 443 |
| 626 | 829b | 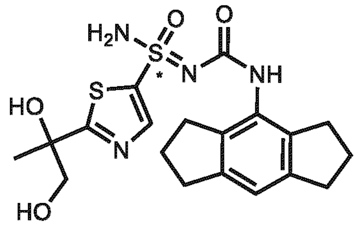 | (R,R) and (S,R)-2-(1,2-dihydroxypropan-2-yl)-N'-((1,2,3,5,6,7-hexahydro-*s*-indacen-4-yl)carbamoyl)thiazole-5-sulfonimidamide (mixture of two isomers) | 1st and 2nd peak CHIRA LPAK IF, 2*25 cm 5 um; from Ex. 436 | EtOH in Hex (0.1% FA) | 437 |
| 627 | 829c | 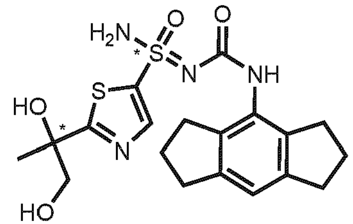 | (R,S) or (S,S)-1,2-dihydroxypropan-2-yl)-N'-((1,2,3,5,6,7-hexahydro-*s*-indacen-4-yl)carbamoyl)thiazole-5-sulfonimidamide | 3rd peak CHIRA LPAK IF, 2*25 cm 5 um; from Ex. 436 | EtOH in Hex (0.1% FA) | 437 |
| 628 | 829a | 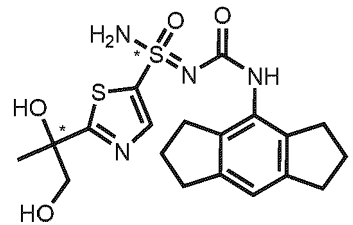 | (S,S) or (R,S)-1,2-dihydroxypropan-2-yl)-N'-((1,2,3,5,6,7-hexahydro-*s*-indacen-4-yl)carbamoyl)thiazole-5-sulfonimidamide | 4th peak CHIRA LPAK IF, 2*25 cm 5 um; from Ex. 436 | EtOH in Hex (0.1% FA) | 437 |

(continued)

| Ex. # | Cmpd # | Structure | IUPAC Name | Column | Eluents | LC-MS $[M+H]^+$ |
|---|---|---|---|---|---|---|
| 629 | 411a | 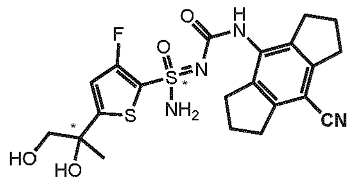 | (S,R) or (R,R) -N'-((8-cyano-1,2,3,5,6,7-hexahydro-*s*-indacen-4-yl)carbamoyl)-5-(1,2-dihydroxypropan-2-yl)-3-fluorothiophene-2-sulfonimidamide | 1st peak CHIRA LPAK IG, 20*250 mm, 5 um; from Ex. 443 | EtOH in Hex (0.1% FA) | 479 |
| 630 | 411b | 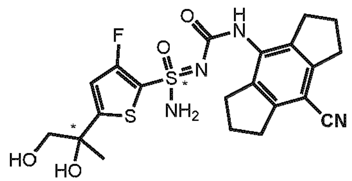 | (R,R) or (S,R) -N'-((8-cyano-1,2,3,5,6,7-hexahydro-*s*-indacen-4-yl)carbamoyl)-5-(1,2-dihydroxypropan-2-yl)-3-fluorothiophene-2-sulfonimidamide | 2nd peak CHIRA LPAK IG, 20*250 mm, 5 um; from Ex. 443 | EtOH in Hex (0.1% FA) | 479 |
| 631 | 411e | 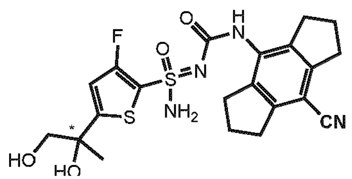 | (R,S) and (S,S) -N'-((8-cyano-1,2,3,5,6,7-hexahydro-*s*-indacen-4-yl)carbamoyl)-5-(1,2-dihydroxypropan-2-yl)-3 -fluorothiophene-2-sulfonimidamide (mixture of two isomers) | 3rd and 4th peak CHIRA LPAK IG, 20*250 mm, 5 um; from Ex. 443 | EtOH in Hex (0.1% FA) | 479 |
| 632 | 411d | 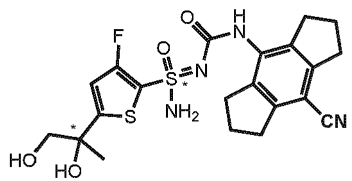 | (S,S) or (R,S) -N'-((8-cyano-1,2,3,5,6,7-hexahydro-*s*-indacen-4-yl)carbamoyl)-5-(1,2-dihydroxypropan-2-yl)-3 - fluorothiophene-2-sulfonimidamide | 1st peak Chiralpa k IC, 2*25 cm, 5 um; from Ex. 631 | EtOH in Hex (0.1% FA) | 479 |
| 633 | 411c | 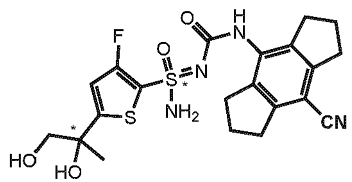 | (R,S) or (S,S)-N'-((8-cyano-1,2,3,5,6,7-hexahydro-*s*-indacen-4-yl)carbamoyl)-5-(1,2-dihydroxypropan-2-yl)-3 - fluorothiophene-2-sulfonimidamide | 2nd peak Chiralpa k IC, 2*25 cm, 5 um; from Ex. 631 | EtOH in Hex (0.1% FA) | 479 |

(continued)

| Ex. # | Cmpd # | Structure | IUPAC Name | Column | Eluents | LC-MS [M+H]+ |
|---|---|---|---|---|---|---|
| 634 | 842a | 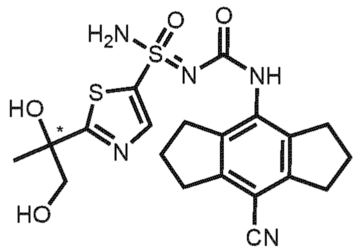 | (R,R) and (S,R) -N'-((8-cyano-1,2,3,5,6,7-hexahydro-*s*-indacen-4-yl)carbamoyl)-2-(1,2-dihydroxypropan-2-yl)thiazole-5-sulfonimidamide (mixture of two isomers) | 1st and 2nd peak CHIRA LPAK IE, 2*25 cm, 5 um; from Ex. 437 | EtOH in Hex (0.1% FA) | 462 |
| 635 | 842b | 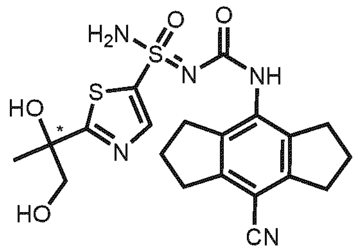 | (R,S) and (S,S) -N'-((8-cyano-1,2,3,5,6,7-hexahydro-*s*-indacen-4-yl)carbamoyl)-2-(-1,2-dihydroxypropan-2-yl)thiazole-5-sulfonimidamide (mixture of two isomers) | 3rd and 4th peak CHIRA LPAK IE, 2*25 cm, 5 um; from Ex. 437 | EtOH in Hex (0.1% FA) | 462 |
| 636 | 842d | 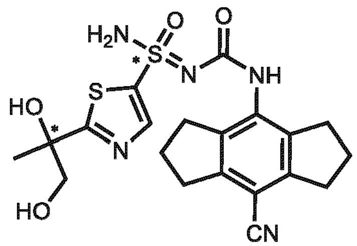 | (S,R) or (R,R)-N'-((8-cyano-1,2,3,5,6,7-hexahydro-*s*-indacen-4-yl)carbamoyl)-2-(-1,2-dihydroxypropan-2-yl)thiazole-5-sulfonimidamide | 1st peak Chiralpa k IA, 2*25 cm, 5 um; from Ex. 634 | IPA in Hex (0.1% FA) | 462 |
| 637 | 842c | 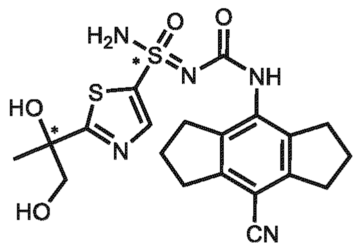 | (R,R) or (S,R)-N'-((8-cyano-1,2,3,5,6,7-hexahydro-*s*-indacen-4-yl)carbamoyl)-2-(-1,2-dihydroxypropan-2-yl)thiazole-5-sulfonimidamide | 2nd peak Chiralpa k IA, 2*25 cm, 5 um; from Ex. 634 | IPA in Hex (0.1% FA) | 462 |

(continued)

| Ex. # | Cmpd # | Structure | IUPAC Name | Column | Eluents | LC-MS $[M+H]^+$ |
|---|---|---|---|---|---|---|
| 638 | 826f | 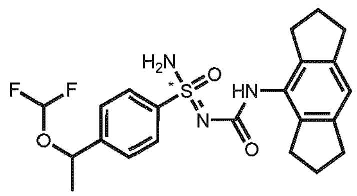 | (R,R) and (S,R)-4-(1-(difluoromethoxy) ethyl)-N'-((1,2,3,5,6,7-hexahydro-*s*-indacen-4-yl)carbamoyl)benzenesulfonimidamide (mixture of two isomers) | 1st and 2nd peak CHIRA LPAK IG, 2*25 cm (5 um); from Ex. 452 | EtOH in Hex (8 mM $NH_3$·MeOH | 450 |
| 639 | 826d | 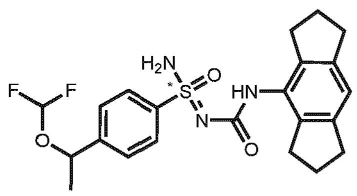 | (R,S) and (S,S)-4-(1-(difluoromethoxy) ethyl)-N'-((1,2,3,5,6,7-hexahydro-*s*-indacen-4-yl)carbamoyl)benzenesulfonimidamide (mixture of two isomers) | 3rd and 4th peak CHIRA LPAK IG, 2*25 cm (5 um); from Ex. 452 | EtOH in Hex (8 mM $NH_3$·MeOH | 450 |
| 640 | 826b | 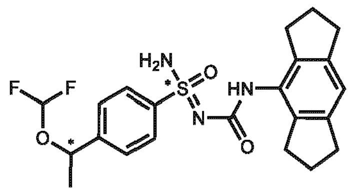 | (S,R) or (R,R)-4-(-1-(difluoromethoxy) ethyl)-N'-((1,2,3,5,6,7-hexahydro-*s*-indacen-4-yl)carbamoyl)benzenesulfonimidamide | 1st peak CHIRA LPAK IA, 2*25 cm, 5 um; from Ex. 638 | EtOH in MTBE (10 mM $NH_3$·MeOH ) | 450 |
| 641 | 826a | 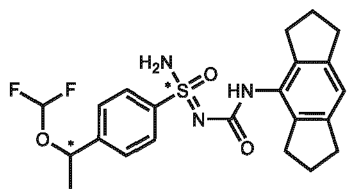 | (R,R) or (S,R)-4-(-1-(difluoromethoxy) ethyl)-N'-((1,2,3,5,6,7-hexahydro-*s*-indacen-4-yl)carbamoyl)benzenesulfonimidamide | 2nd peak CHIRA LPAK IA, 2*25 cm, 5 um; from Ex. 638 | EtOH in MTBE (10 mM $NH_3$·MeOH ) | 450 |
| 642 | 826e | 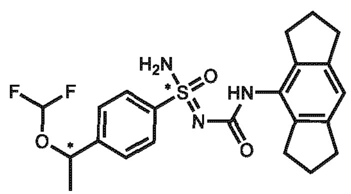 | (S,S) or (R,S)-4-(1-(difluoromethoxy) ethyl)-N'-((1,2,3,5,6,7-hexahydro-*s*-indacen-4-yl)carbamoyl)benzenesulfonimidamide | 1st peak CHIRA LPAK IG, 20*250 mm, 5 um; from Ex. 639 | IPA in Hex (0.1% FA) | 450 |

(continued)

| Ex. # | Cmpd # | Structure | IUPAC Name | Column | Eluents | LC-MS $[M+H]^+$ |
|---|---|---|---|---|---|---|
| 643 | 826c | 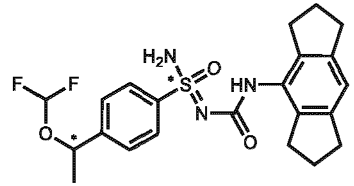 | (R,S) or (S,S)-4-(-1-(difluoromethoxy) ethyl)-N'-((1,2,3,5,6,7-hexahydro-*s*-indacen-4-yl)carbamoyl)benzenesulfonimidamide | 2nd peak CHIRA LPAK IG, 20*250 mm, 5 um; from Ex. 639 | IPA in Hex (0.1% FA) | 450 |
| 644 | 819d | 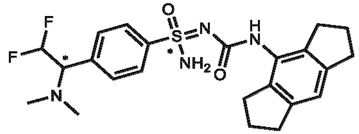 | (R,R) or (S,R)-4-(-1-(dimethylamino)-2,2-difluoroethyl)-N'-((1,2,3,5,6,7-hexahydro-*s*-indacen-4-yl)carbamoyl) benzenesulfonimidamide | Column 2: Chiralpak ID, 2*25 cm, 5 um, IPA in Hex (0.1% FA) to separate two fast-co-eluting isomers from Column 1 CHIRALPAK IG, 2*25 cm (5 um), EtOH in Hex (0.1% FA) | | 463 |
| 645 | 819c | 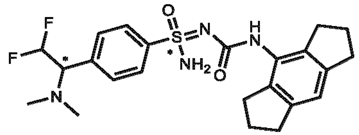 | (S,S) or (R,S)-4-(-1-(dimethylamino)-2,2-difluoroethyl)-N'-((1,2,3,5,6,7-hexahydro-*s*-indacen-4-yl)carbamoyl) benzenesulfonimidamide | | | 463 |
| 646 | 819b | 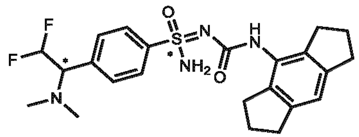 | (R,S) or (S,S)-4-(-1-(dimethylamino)-2,2-difluoroethyl)-N'-((1,2,3,5,6,7-hexahydro-s-indacen-4-yl)carbamoyl) benzenesulfonimidamide | Column 2: Chiralpak ID, 2*25 cm, 5 um, IPA in Hex (0.1% FA) to separate two slow-co-eluting isomers from Column 1 CHIRALPAK IG, 2*25 cm (5 um), EtOH in Hex (0.1% FA) | | 463 |
| 647 | 819a | 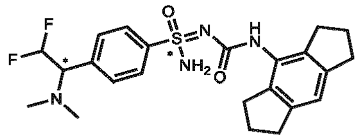 | (S,R) or (R,R)-4-(1-(dimethylamino)-2,2-difluoroethyl)-N'-((1,2,3,5,6,7-hexahydro-*s*-indacen-4-yl)carbamoyl) benzenesulfonimidamide | | | 463 |
| 648 | 847b | 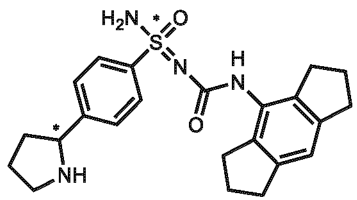 | (R,R) or (S,R)-N'-((1,2,3,5,6,7-hexahydro-*s*-indacen-4-yl)carbamoyl)-4-(pyrrolidin-2-yl)benzenesulfonimidamide | 1st peak Ultimate Diol, 20*250 mm; 5 um | 35% MeOH (2 mM $NH_3$) in $CO_2$ | 425 |

(continued)

| Ex. # | Cmpd # | Structure | IUPAC Name | Column | Eluents | LC-MS $[M+H]^+$ |
|---|---|---|---|---|---|---|
| 649 | 847a | 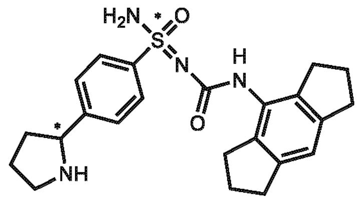 | (S,R) or (R,R) and (S,S) or (R,S) -N'-((1,2,3,5,6,7-hexahydro-*s*-indacen-4-yl) carbamoyl)-4-(pyrrolidin-2-yl) benzenesulfonimidamide (mixture of two isomers) | 2nd and 3rd peak Ultimate Diol, 20*250 mm; 5 um | 35% MeOH (2 mM $NH_3$) in $CO_2$ | 425 |
| 650 | 847b | 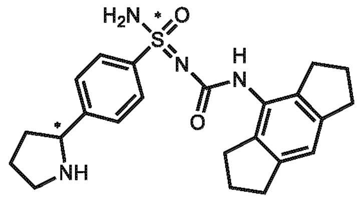 | (R,S) or (S,S)-N'-((1,2,3,5,6,7-hexahydro-*s*-indacen-4-yl)carbamoyl)-4-(pyrrolidin-2-yl) benzenesulfonimidamide | 4th peak Ultimate Diol, 20*250 mm; 5 um | 35% MeOH (2 mM $NH_3$) in $CO_2$ | 425 |
| 651 | 867d | 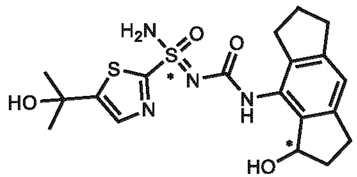 | (R,S) or (R,R)-N'-((3-hydroxy-1,2,3,5,6,7-hexahydro-*s*-indacen-4-yl)carbamoyl)-5-(2-hydroxypropan-2-yl)thiazole-2-sulfonimidamide | 1st peak CHIRA L ART Cellulos e-SB, 2*25 cm, 5 um; from Ex. 510 | 35% IPA (2 mM $NH_3 \cdot MeOH$ ) in $CO_2$ | 437 |
| 652 | 867c | 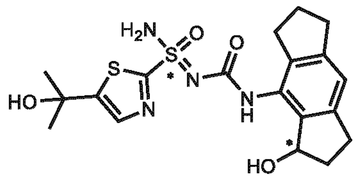 | (R,R) or (R,S)-N'-((3-hydroxy-1,2,3,5,6,7-hexahydro-*s*-indacen-4-yl)carbamoyl)-5-(2-hydroxypropan-2-yl)thiazole-2-sulfonimidamide | 2nd peak CHIRA L ART Cellulos e-SB, 2*25 cm, 5 um; from Ex. 510 | 35% IPA (2 mM $NH_3 \cdot MeOH$ ) in $CO_2$ | 437 |
| 653 | 867b | 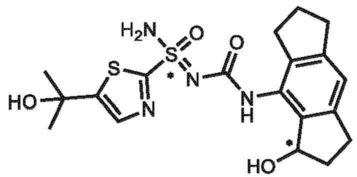 | (S,S) or (S,R)-N'-((3-hydroxy-1,2,3,5,6,7-hexahydro-*s*-indacen-4-yl)carbamoyl)-5-(2-hydroxypropan-2-yl)thiazole-2-sulfonimidamide | 1st peak CHIRA LPAK AS, 2*25 cm (5 um); from Ex. 511 | 30% IPA (2 mM $NH_3 \cdot MeOH$ ) in $CO_2$ | 437 |

(continued)

| Ex. # | Cmpd # | Structure | IUPAC Name | Column | Eluents | LC-MS $[M+H]^+$ |
|---|---|---|---|---|---|---|
| 654 | 867a | 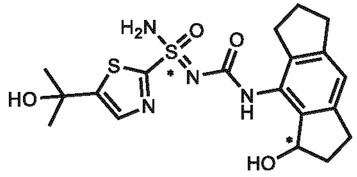 | (S,R) or (S,S)-N'-((-3-hydroxy-1,2,3,5,6,7-hexahydro-*s*-indacen-4-yl)carbamoyl)-5-(2-hydroxypropan-2-yl)thiazole-2-sulfonimidamide | 2nd peak CHIRA LPAK AS, 2*25 cm (5 um); from Ex. 511 | 30% IPA (2 mM $NH_3 \cdot MeOH$ ) in $CO_2$ | 437 |
| 655 | 868d | 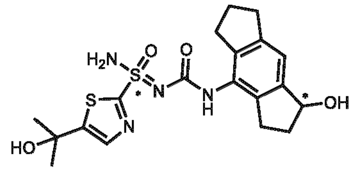 | (S,R) or (S,S)-N'-((1-hydroxy-1,2,3,5,6,7-hexahydro-*s*-indacen-4-yl)carbamoyl)-5-(2-hydroxypropan-2-yl)thiazole-2-sulfonimidamide | 1st peak Chiralpa k AD, 2*25 cm (5 um); from Ex. 509 | 35% MeOH (2 mM $NH_3$) in $CO_2$ | 437 |
| 656 | 868c | 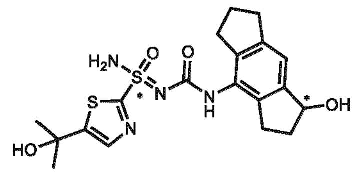 | (S,S) or (S,R)-N'-((1-hydroxy-1,2,3,5,6,7-hexahydro-*s*-indacen-4-yl)carbamoyl)-5-(2-hydroxypropan-2-yl)thiazole-2-sulfonimidamide | 2nd peak Chiralpa k AD, 2*25 cm (5 um); from Ex. 509 | 35% MeOH (2 mM $NH_3$) in $CO_2$ | 437 |
| 657 | 868b | 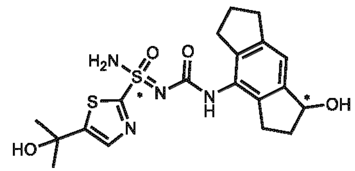 | (R,R) or (R,S)-N'-((1-hydroxy-1,2,3,5,6,7-hexahydro-*s*-indacen-4-yl)carbamoyl)-5-(2-hydroxypropan-2-yl)thiazole-2-sulfonimidamide | 3rd peak Chiralpa k AD, 2*25 cm (5 um); from Ex. 512 | 40% MeOH (2 mM $NH_3$) in $CO_2$ | 437 |
| 658 | 868a | 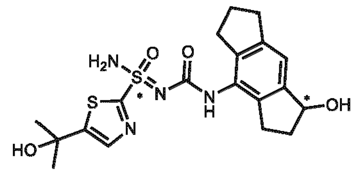 | (R,S) or (R,R)-N'-((1-hydroxy-1,2,3,5,6,7-hexahydro-*s*-indacen-4-yl)carbamoyl)-5-(2-hydroxypropan-2-yl)thiazole-2-sulfonimidamide | 4th peak Chiralpa k AD, 2*25 cm (5 um); from Ex. 512 | 40% MeOH (2 mM $NH_3$) in $CO_2$ | 437 |

(continued)

| Ex. # | Cmpd # | Structure | IUPAC Name | Column | Eluents | LC-MS $[M+H]^+$ |
|---|---|---|---|---|---|---|
| 659 | 854d | 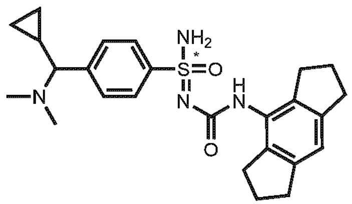 | (R,S) and (S,S)-4-(cyclopropyl (dimethylamino)methyl)-N'-((1,2,3,5,6,7-hexahydro-*s*-indacen-4-yl)carbamoyl) benzenesulfonimidamide (mixture of two isomers) | 1st peak CHIRA L ART Cellulos e-SB, 2*25 cm, 5 um | IPA in Hex (8 mM $NH_3$·MeOH ) | 453 |
| 660 | 854c | 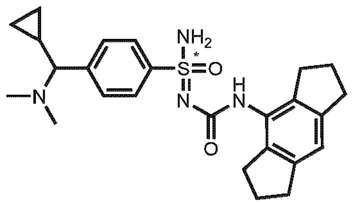 | (R,R) and (S,R)-4-(cyclopropyl (dimethylamino)methyl)-N'-((1,2,3,5,6,7-hexahydro-*s*-indacen-4-yl)carbamoyl) benzenesulfonimidamide (mixture of two isomers) | 2nd peak CHIRA L ART Cellulos e-SB, 2*25 cm, 5 um | IPA in Hex (8 mM $NH_3$·MeOH ) | 453 |
| 661 | 854b | 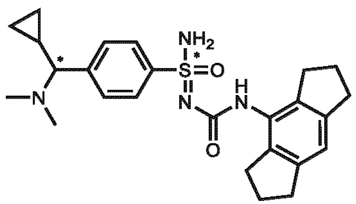 | (S,R) or (R,R)-4-(cyclopropyl(dimethylamino) methyl)-N'-((1,2,3,5,6,7-hexahydro-*s*-indacen-4-yl)carbamoyl) benzenesulfonimidamide | 1st peak Chiralpa k IG, 2*25 cm, 5 um; from Ex. 660 | EtOH in Hex (8 mM $NH_3$·MeOH ) | 453 |
| 662 | 854a | 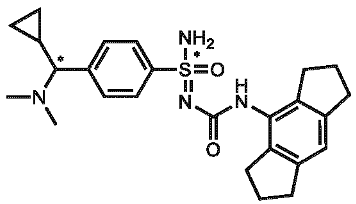 | (R,R) or (S,R)-4-(cyclopropyl(dimethylamino) methyl)-N'-((1,2,3,5,6,7-hexahydro-*s*-indacen-4-yl)carbamoyl) benzenesulfonimidamide | 2nd peak Chiralpa k IG, 2*25 cm, 5 um; from Ex. 660 | EtOH in Hex (8 mM $NH_3$·MeOH ) | 453 |

(continued)

| Ex. # | Cmpd # | Structure | IUPAC Name | Column | Eluents | LC-MS $[M+H]^+$ |
|---|---|---|---|---|---|---|
| 663 | 864a | 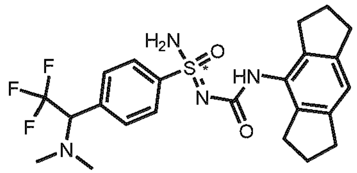 | (R,S) and (S,S)-4-(1-(dimethylamino)-2,2,2-trifluoroethyl)-N'-((1,2,3,5,6,7-hexahydro-*s*-indacen-4-yl)carbamoyl) benzenesulfonimidamide (mixture of two isomers) | 1st peak CHIRA L ART Cellulos e-SB, 2*25 cm, 5 um | EtOH in Hex (8 mM $NH_3$·MeOH ) | 481 |
| 664 | 856e | 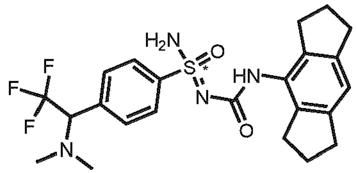 | (R,R) and (S,R)-4-(1-(dimethylamino)-2,2,2-trifluoroethyl)-N'-((1,2,3,5,6,7-hexahydro-*s*-indacen-4-yl)carbamoyl) benzenesulfonimidamide (mixture of two isomers) | 2nd peak CHIRA L ART Cellulos e-SB, 2*25 cm, 5 um | EtOH in Hex (8 mM $NH_3$·MeOH ) | 481 |
| 665 | 856d | 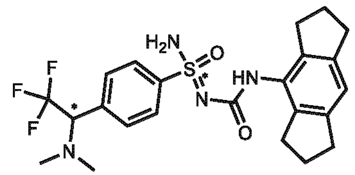 | (S,S) or (R,S)-4-(1-(dimethylamino)-2,2,2-trifluoroethyl)-N'-((1,2,3,5,6,7-hexahydro-*s*-indacen-4-yl)carbamoyl) benzenesulfonimidamide | 1st peak Chiralpa k IG, 2*25 cm, 5 um; from Ex. 663 | EtOH in MTBE (10 mM $NH_3$·MeOH ) | 481 |
| 666 | 856c | 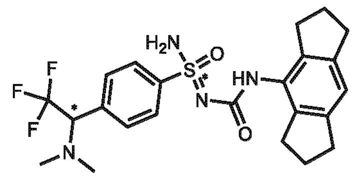 | (R,S) or (S,S)-4-(1-(dimethylamino)-2,2,2-trifluoroethyl)-N'-((1,2,3,5,6,7-hexahydro-*s*-indacen-4-yl)carbamoyl) benzenesulfonimidamide | 2nd peak Chiralpa k IA, 2*25 cm, 5 um; from Ex. 663 | EtOH in MTBE (10 mM $NH_3$-MeOH) | 481 |
| 667 | 856b | 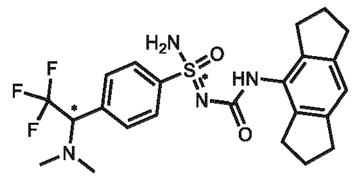 | (S,R) or (R,R) -4-(1-(dimethylamino)-2,2,2-trifluoroethyl)-N'-((1,2,3,5,6,7-hexahydro-*s*-indacen-4-yl)carbamoyl) benzenesulfonimidamide | 1st peak Chiralpa k IA, 2*25 cm, 5 um; from Ex. 664 | EtOH in MTBE (10 mM $NH_3$·MeOH ) | 481 |

(continued)

| Ex. # | Cmpd # | Structure | IUPAC Name | Column | Eluents | LC-MS $[M+H]^+$ |
|---|---|---|---|---|---|---|
| 668 | 856a | 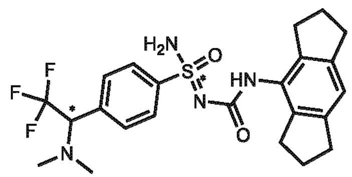 | (R,R) or (S,R) -4-(1-(dimethylamino)-2,2,2-trifluoroethyl)-N'-((1,2,3,5,6,7-hexahydro-*s*-indacen-4-yl)carbamoyl) benzenesulfonimidamide | 2nd peak Chiralpa k IA, 2*25 cm, 5 um; from Ex. 664 | EtOH in MTBE (10 mM $NH_3$·MeOH ) | 481 |

# The amount of $NH_3$ in this chiral chromatographic solvent and similar solvents were adjusted by adding 2 M $NH_3$ in MeOH to the desired $NH_3$ concentration. In this case, the resulting concentration of $NH_3$ in MeOH is 8 mM.

**Example 669 (Compound 901)**

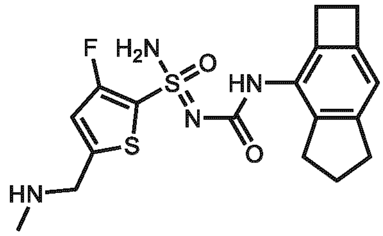

3-Fluoro-5-((methylamino)methyl)-N'-((2,4,5,6-tetrahydro-1H-cyclobuta[f]inden-3-yl)carbamoyl)thiophene-2-sulfonimidamide (Scheme XII)

Examples **670 (Compound 901a) and Examples 671 (Compound** 901b)

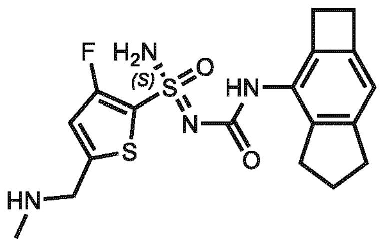

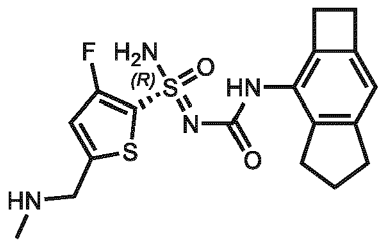

**Examples 670 and 671 (stereochemistry not assigned)**

(S)- and (R)- 3-Fluoro-5-((methylamino)methyl)-N'-((2,4,5,6-tetrahydro-1H-cyclobuta[f]inden-3-yl)carbamoyl)thiophene-2-sulfonimidamide

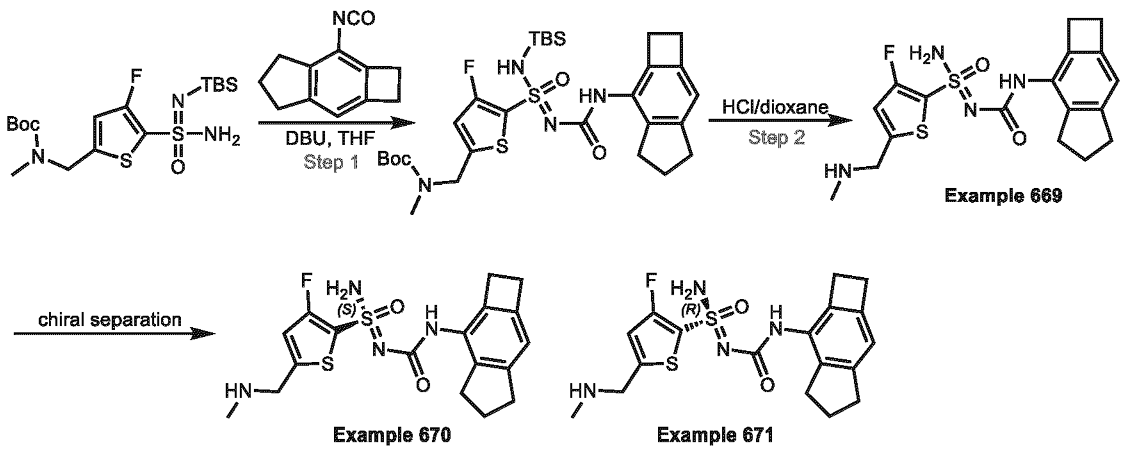


**Step 1: Tert-butyl((5-(N-(tert-butyldimethylsilyl)-N'-((2,4,5,6-tetrahydro-1H-cyclobuta[f]inden-3-yl) carbamoyl)sulfamidimidoyl)-4-fluorothiophen-2-yl)methyl)(methyl)carbamate**

To a stirred solution of tert-butyl ((5-(N'-(tert-butyldimethylsilyl)sulfamidimidoyl)-4-fluorothiophen-2-yl) methyl)(methyl)carbamate (200 mg, 0.46 mmol) in THF (5.0 mL) in a 25-mL round-bottom flask was added DBU (139 mg, 0.91 mmol) at RT, followed by the addition of 3-isocyanato-2,4,5,6-tetrahydro-1H-cyclobuta[f]indene (85 mg, 0.46 mmol)

in THF (3 mL) dropwise at 0 °C. The resulting solution was stirred overnight at RT. The reaction was quenched with water (5 mL). The resulting solution was extracted with 3x10 mL ethyl acetate. The organic layers were combined and dried over anhydrous sodium sulfate and concentrated under vacuum. This resulted in 180 mg (crude) of the title compound as a white solid. MS-ESI: 538 (M+1).

**Step 2: 3-Fluoro-5-((methylamino)methyl)-N'-((2,4,5,6-tetrahydro-1H-cyclobuta[f]inden-3-yl) carbamoyl)thiophene-2-sulfonimidamide**

To a stirred solution of tert-butyl ((5-(N-(tert-butyldimethylsilyl)-N'-((2,4,5,6-tetrahydro-1H-cyclobuta[f] inden-3-yl)carbamoyl)sulfamidimidoyl)-4-fluorothiophen-2-yl)methyl)(methyl)carbamate (180 mg, crude) in THF (5 mL) in a 25-mL round-bottom flask was added HCl in 1,4-dioxane (4 M, 5.0 mL) dropwise at 0 °C. The resulting solution was stirred overnight at RT. The resulting mixture was concentrated under vacuum. The crude product was purified by Prep-HPLC with the following conditions: XBridge Prep OBD C18 Column, 30*150mm 5um; mobile phase, water (10 mM $NH_4HCO_3$+0.1%$NH_3.H_2O$) and ACN (20% to 30% Phase B over 7 min); Detector, UV 254/210 nm. This resulted in 65 mg (34.8% over two steps) of **Example 669** as a white solid. MS-ESI: 424 (M+1). $^1$H NMR (300 MHz, DMSO-$d_6$) δ 8.26 (br s, 1H), 6.93 (s, 1H), 6.66 (s, 1H), 3.79 (s, 2H), 3.00-2.94 (m, 2H), 2.92-2.85 (m, 2H), 2.83-2.67 (m, 4H), 2.29 (s, 3H), 1.96-1.83 (m, 2H).

**Step 3: Chiral separation**

Example **669** (60 mg) was resolved by Prep-Chiral-HPLC with the following conditions: CHIRALPAK ID, 2*25cm(5 um); Mobile Phase A: Hex:DCM=3:1(10 mM $NH_3$-MeOH), Mobile Phase B: EtOH; Flow rate: 20 mL/min; Gradient: 30% B to 30% B over 23 min; Detector: UV 254/220 nm. Rti: 6.2 **(Example 670)**; $Rt_2$: 9.46 **(Example 671).** This resulted in 22.1 mg (99%ee) of (Example 670) as a white solid. This resulted in 22.3 mg (99%ee) of (Example 671) as a white solid.

**Example 670** MS-ESI: 424 (M+1). $^1$H NMR (400 MHz, DMSO-$d_6$) δ 8.25 (br s, 1H), 6.92 (s, 1H), 6.65 (s, 1H), 3.78 (s, 2H), 3.00-2.94 (m, 2H), 2.91-2.84 (m, 2H), 2.83-2.64 (m, 4H), 2.29 (s, 3H), 1.95-1.85 (m, 2H).

**Example 671** MS-ESI: 424 (M+1). $^1$H NMR (400 MHz, DMSO-$d_6$) δ 8.28 (br s, 1H), 6.94 (s, 1H), 6.66 (s, 1H), 3.79 (s, 2H), 3.00-2.93 (m, 2H), 2.91-2.85 (m, 2H), 2.83-2.67 (m, 4H), 2.29 (s, 3H), 1.96-1.84 (m, 2H).

**Table 49.** Examples in the following table were prepared using similar conditions as described in Example **669** and Scheme **XII** from appropriate starting materials.

| Example # | Cmpd # | Structure | IUPAC Name | Exact Mass $[M+H]^+$ |
|---|---|---|---|---|
| 672 | 910 | 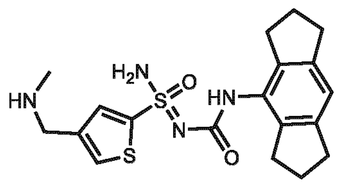 | N'-((1,2,3,5,6,7-hexahydro-*s*-indacen-4-yl)carbamoyl)-4-((methylamino) methyl)thiophene-2-sulfonimidamide | 405 |

**Example 673 (Compound 922a)**

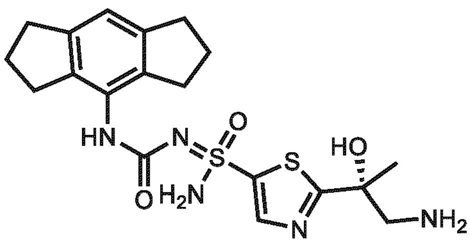

2-((R)-1-amino-2-hydroxypropan-2-yl)-N'-((1,2,3,5,6,7-hexahydro-*s*-indacen-4-yl)carbamoyl)thiazole-5-sulfonimidamide (Scheme XIII)

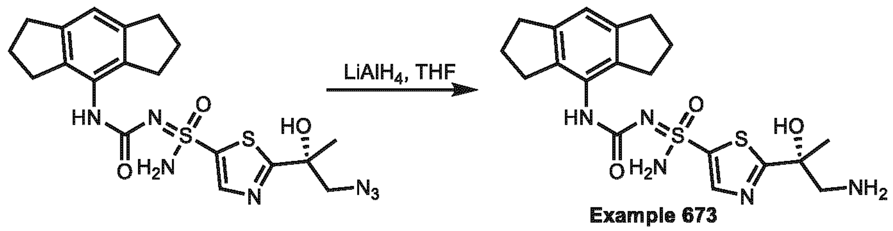


To a stirred solution of 2-((R)-1-azido-2-hydroxypropan-2-yl)-N'-((1,2,3,5,6,7-hexahydro-s-indacen-4-yl) carbamoyl)thiazole-5-sulfonimidamide (100 mg, 0.22 mmol) in THF (10 mL) in a 50-mL round-bottom flask under nitrogen was added $LiAlH_4$ (17 mg, 0.44 mmol) at 0 °C. The resulting solution was stirred for 2 h at 0 °C. The reaction was quenched with water/ice at 0 °C. The resulting mixture was filtered. The filter cake was washed with water (3x5 mL). The resulting mixture was concentrated under reduced pressure. The crude product was purified by Prep-HPLC with the following conditions: Sunfire prep C18 column, 30*150, 5 um; Mobile Phase A: Water (0.1%FA), Mobile Phase B: ACN; Flow rate: 60 mL/min; Gradient: 20% B to 37% B over 7 min; Rti:4.72, UV 254/210 nm. This resulted in 8.2 mg (8.7%) of **Example 673** as a white solid. MS-ESI: 436 (M+1). $^1H$ NMR (400 MHz, DMSO-$d_6$) δ: 8.46 (br, s, 1H), 8.18 (s, 1H), 7.17 (br, s, 4H), 6.89 (s, 1H), 3.10-2.98 (m, 2H), 2.84-2.74 (m, 4H), 2.73-2.61 (m, 4H), 2.00-1.87 (m, 4H), 1.53 (s, 3H).

**Example 674 (Compound 896)**

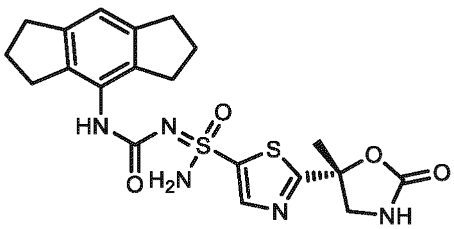

N'-((1,2,3,5,6,7-hexahydro-*s*-indacen-4-yl)carbamoyl)-2-((R)-5-methyl-2-oxooxazolidin-5-yl)thiazole-5-sulfonimidamide (Scheme XIV)

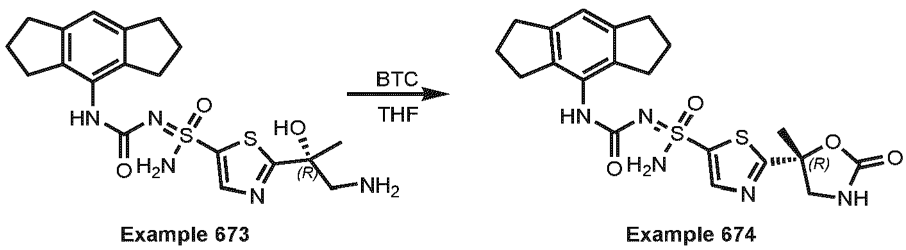


To a stirred solution of 2-((R)-1-amino-2-hydroxypropan-2-yl)-N'-((1,2,3,5,6,7-hexahydro-s-indacen-4-yl) carbamoyl)thiazole-5-sulfonimidamide (Ex. 673, 80 mg, 0.18 mmol) in THF (10 mL) in a 50-mL round-bottom flask under nitrogen was added bis(trichloromethyl) carbonate (23 mg, 0.09 mmol) at 0 °C. The resulting mixture was stirred for 2 h at RT. The resulting mixture was concentrated under vacuum. The crude product was purified by Prep-HPLC with the following conditions: XBridge Prep C18 OBD Column, 19×150 mm 5 um; Mobile Phase A: Water (10 mM $NH_4HCO_3$+0.1%$NH_3.H_2O$), Mobile Phase B: ACN; Flow rate: 25 mL/min; Gradient: 13% B to 36% B over 7 min; Rti:6.47, UV 254/210 nm. This resulted in 3.7 mg (4.4%) of **Example 674** as a white solid. MS-ESI: 462 (M+1). $^1H$ NMR (400

MHz, DMSO-$d_6$) δ 8.47 (br s, 1H), 8.20 (s, 1H), 7.96 (s, 1H), 7.86 (br s, 2H), 6.88 (s, 1H), 3.79-3.71 (m, 2H), 2.83-2.74 (m, 4H), 2.72-2.60 (m, 4H), 1.99-1.87 (m, 4H), 1.79 (s, 3H).

**Example 675 (Compound 894)**

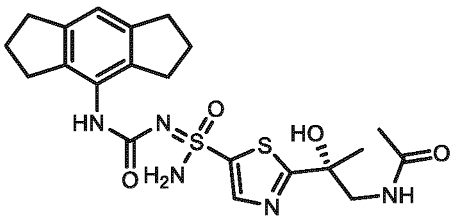

N-((2R)-2-(5-(N'-((1,2,3,5,6,7-hexahydro-s-indacen-4-yl)carbamoyl)sulfamidimidoyl)thiazol-2-yl)-2-hydroxypropyl)acetamide (Scheme XV)

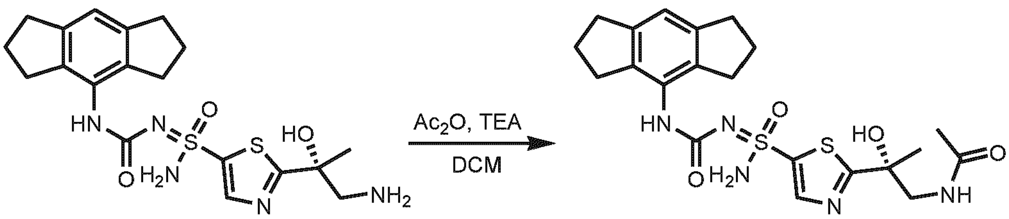


Example 673 Example 675

To a stirred solution of 2-((R)-1-amino-2-hydroxypropan-2-yl)-N'-((1,2,3,5,6,7-hexahydro-s-indacen-4-yl) carbamoyl)thiazole-5-sulfonimidamide (100 mg, 0.23 mmol) in DCM (10 mL) in a 50-mL round-bottom flask under nitrogen was added TEA (70 mg, 0.69 mmol) at RT, followed by the addition of $Ac_2O$ (23 mg, 0.23 mmol) dropwise at 0 °C. The resulting solution was stirred for 1 h at RT. The resulting mixture was concentrated under vacuum. The crude product was purified by Prep-HPLC with the following conditions: XBridge Prep C18 OBD Column, 19×150 mm 5 um; Mobile Phase A: Water (10 mM $NH_4HCO_3$+0.1%$NH_3.H_2O$), Mobile Phase B: ACN; Flow rate: 25 mL/min; Gradient: 12% B to 20% B over 10 min; $Rt_1$: 12.43 min, Detector, UV 254/210 nm. This resulted in 5.3 mg (4.83%) of **Example 675** as a white solid. MS-ESI: 478 (M+1). 1H NMR (400 MHz, DMSO-d6) δ 8.41 (br, s, 1H), 8.08 (s, 1H), 7.98 (br s, 1H), 7.82 (br, s, 2H), 6.88 (s, 1H), 6.52 (s, 1H), 3.60-3.47 (m, 1H), 3.31-3.25 (m, 1H), 2.85-2.73 (m, 4H), 2.72-2.60 (m, 4H), 2.00-1.87 (m, 4H), 1.83 (s, 3H), 1.45 (s, 3H).

**Example 676 (Compound 894a)**

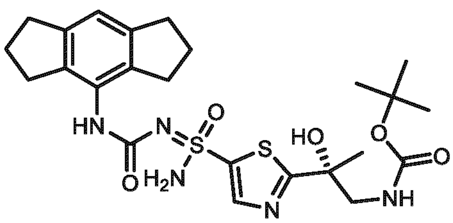

Tert-butyl ((2R)-2-(5-(N'-((1,2,3,5,6,7-hexahydro-*s*-indacen-4-yl)carbamoyl)sulfamidimidoyl)thiazol-2-yl)-2-hydroxypropyl)carbamate (Scheme XVI)

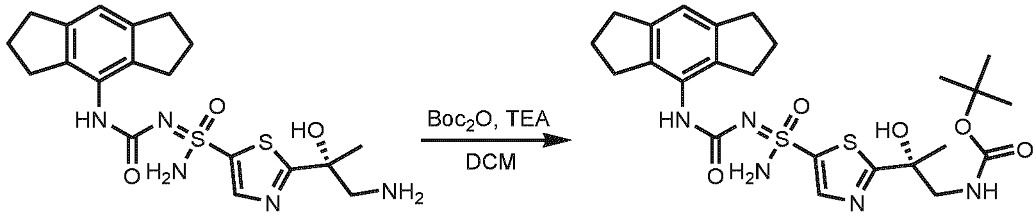


**Example 673** **Example 676**

To a stirred solution of 2-((R)-1-amino-2-hydroxypropan-2-yl)-N'-((1,2,3,5,6,7-hexahydro-s-indacen-4-yl) carbamoyl)thiazole-5-sulfonimidamide (100 mg, 0.23 mmol) in DCM (10 mL) in a 50-mL round-bottom flask was added TEA (70 mg, 0.69 mmol) at RT, followed by the addition of $Boc_2O$ (50 mg, 0.23 mmol) in THF (5 mL) dropwise at 0 °C. The resulting solution was stirred for 1 h at RT. The resulting mixture was concentrated under reduced pressure. The crude product was purified by Prep-HPLC with the following conditions: XSelect CSH Prep C18 OBD Column, 19*250 mm, 5 um; Mobile Phase A: Water (0.1% FA), Mobile Phase B: ACN; Flow rate: 25 mL/min; Gradient: 45% B to 70% B over 7 min; Rti: 6.28 min, UV 254/210 nm. This resulted in 5.5 mg (4.47%) of **Example 676** as a white solid. MS-ESI: 536 (M+1). $^1H$ NMR (400 MHz, DMSO-$d_6$) δ 8.42 (br, s, 1H), 8.08 (s, 1H), 7.87 (br, s, 2H), 6.88 (s, 1H), 6.73 (br s, 1H), 6.33 (s, 1H), 3.45-3.34 (m, 1H), 3.25-3.15 (m, 1H), 2.84-2.75 (m, 4H), 2.72-2.61 (m, 4H), 1.99-1.87 (m, 4H), 1.44 (s, 3H), 1.37 (s, 9H).

**Example 677 (Compound 918a)**

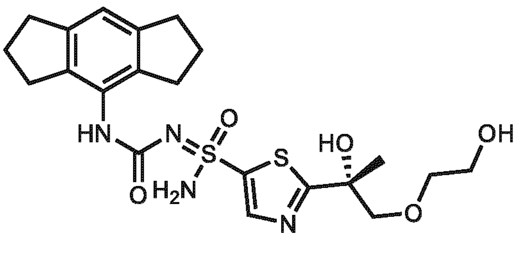

N'-((1,2,3,5,6,7-hexahydro-*s*-indacen-4-yl)carbamoyl)-2-((R)-2-hydroxy-1-(2-hydroxyethoxy)propan-2-yl)thiazole-5-sulfonimidamide (Scheme XVII)

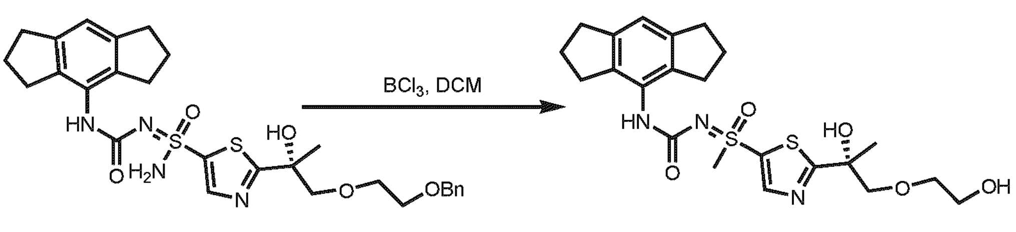


**Example 683** **Example 677**

To a stirred solution of 2-((R)-1-(2-(benzyloxy)ethoxy)-2-hydroxypropan-2-yl)-N'-((1,2,3,5,6,7-hexahydro-s- indacen-4-yl)carbamoyl)thiazole-5-sulfonimidamide (80 mg, 0.14 mmol) in DCM (3.0 mL) in a 50-mL round-bottom flask under nitrogen was added trichloroborane (0.50 mL) dropwise at 0 °C. The resulting mixture was stirred for 16 h at RT. The reaction was quenched with water at 0 °C. The resulting mixture was extracted with DCM (3x30 mL). The combined organic layers were washed with brine (1x30 mL) and dried over anhydrous $Na_2SO_4$. After filtration, the filtrate was

concentrated under reduced pressure. The crude product was purified by Prep-HPLC with the following conditions: XBridge Prep C18 OBD Column, 19×150mm 5um; Mobile Phase A: Water(10 mM $NH_4HCO_3$+0.1%$NH_3.H_2O$), Mobile Phase B: ACN; Flow rate: 25 mL/min; Gradient:8 B to 38 B over 7 min; Rti: 5.50; Detector, UV 254/210 nm. This resulted in 33.3 mg (49%) of **Example 677** as a white solid. MS-ESI: 480 (M+1).

$^1$H NMR (400 MHz, DMSO-$d_6$) δ 8.42 (br s, 1H), 8.08 (s, 1H), 7.85 (br s, 2H), 6.88 (s, 1H), 6.28 (s, 1H), 4.58 (br s, 1H), 3.69-3.58 (m, 2H), 3.61-3.44 (m, 4H), 2.84-2.75 (m, 4H), 2.72-2.61 (m, 4H), 1.99-1.87 (m, 4H), 1.47 (s, 3H).

**Table 50.** Examples in the following table were prepared using similar conditions as described in Example **451** and Scheme **III** from appropriate starting materials.

| Example # | Cmpd # | Structure | IUPAC Name | Exact Mass [M+H]+ |
|---|---|---|---|---|
| 678 | 905 | 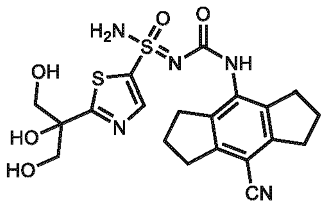 | N'-((8-cyano-1,2,3,5,6,7-hexahydro-*s*-indacen-4-yl)carbamoyl)-2-(1,2,3-trihydroxypropan-2-yl)thiazole-5-sulfonimidamide | 478 |

**Table 51.** Examples in the following table were prepared using similar conditions as described in Example **453** and Scheme **IV** from appropriate starting materials.

| Example # | Cmpd # | Structure | IUPAC Name | Exact Mass [M+H]+ |
|---|---|---|---|---|
| 679 | 915 | 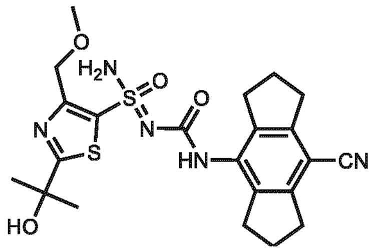 | N'-((8-cyano-1,2,3,5,6,7-hexahydro-*s*-indacen-4-yl) carbamoyl)-2-(2-hydroxypropan-2-yl)-4-(methoxymethyl)thiazole-5-sulfonimidamide | 490 |
| 680 | 912 | 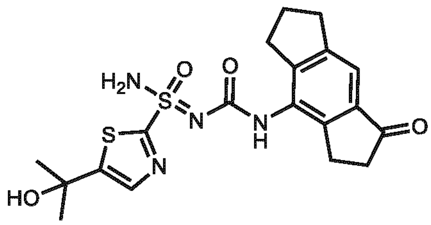 | 5-(2-Hydroxypropan-2-yl)-N'-((1-oxo-1,2,3,5,6,7-hexahydro-*s*-indacen-4-yl)carbamoyl)thiazole-2-sulfonimidamide | 435 |
| 681 | 906 | 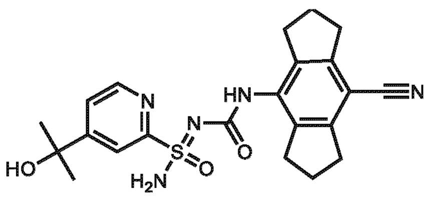 | N'-((8-cyano-1,2,3,5,6,7-hexahydro-*s*-indacen-4-yl) carbamoyl)-4-(2-hydroxypropan-2-yl)pyridine-2-sulfonimidamide | 440 |

(continued)

| Example # | Cmpd # | Structure | IUPAC Name | Exact Mass [M+H]+ |
|---|---|---|---|---|
| 682 | 897 | 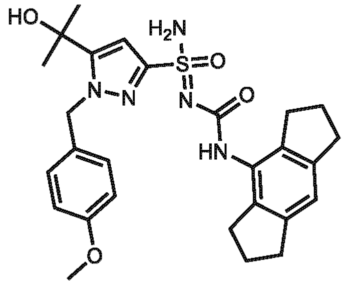 | N'-((1,2,3,5,6,7-hexahydro-*s*-indacen-4-yl)carbamoyl)-5-(2-hydroxypropan-2-yl)-1-(4-methoxybenzyl)- 1H-pyrazole-3 -sulfonimidamide | 524 |
| 683 | 923a | 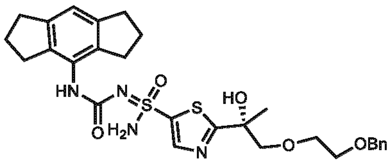 | 2-((R)-1-(2-(benzyloxy)ethoxy)-2-hydroxypropan-2-yl)-N'-((1,2,3,5,6,7-hexahydro-*s*-indacen-4-yl)carbamoyl)thiazole-5-sulfonimidamide | 571 |
| 684 | 924a | 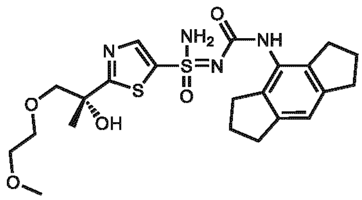 | N'-((1,2,3,5,6,7-hexahydro-*s*-indacen-4-yl)carbamoyl)-2-((R)-2-hydroxy-1-(2-methoxyethoxy) propan-2-yl)thiazole-5-sulfonimidamide | 495 |

**Table 52.** Examples in the following table were obtained from chiral HPLC resolutions of racemic examples described above. The chiral column and eluents are listed in the table. As a convention, the faster-eluting enantiomer is always listed first in the table followed by the 5 slower-eluting enantiomer of the pair. The symbol * at a chiral center denotes that this chiral center has been resolved and the absolute stereochemistry at that center has not been determined. Assigned stereochemistry in compound names are tentative.

| Ex. # | Cmpd # | Structure | IUPAC Name | Column | Eluents | LC-MS $[M+H]^+$ |
|---|---|---|---|---|---|---|
| 685 | 912b | 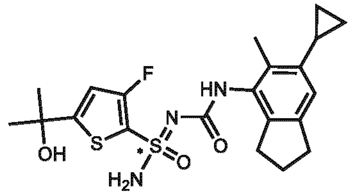 | (R) or (S)-N'-((6-cyclopropyl-5-methyl-2,3-dihydro-1H-inden-4-yl)carbamoyl)-3-fluoro-5-(2-hydroxypropan-2-yl)thiophene-2-sulfonimidamide | CHIRALP AKIF, 2*25cm, Sum | 20% EtOH in Hex (0.1% FA) | 452 |
| 686 | 912a | 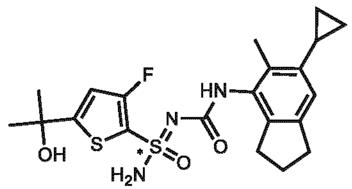 | (S) or (R)-N'-((6-cyclopropyl-5-methyl-2,3-dihydro-1H-inden-4-yl)carbamoyl)-3-fluoro-5-(2-hydroxypropan-2-yl)thiophene-2-sulfonimidamide | CHIRALP AK IF, 2*25cm, Sum | 20% EtOH in Hex (0.1% FA) | 452 |
| 687 | 906b | 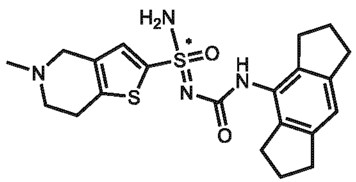 | (R) or (S)-N'-((1,2,3,5,6,7-hexahydro-*s*-indacen-4-yl)carbamoyl)-5-methyl-4,5,6,7-tetrahydrothieno [3,2-c]pyridine-2-sulfonimidamide | CHIRALP AK ID, 2.0cm I.D*25cm L (5um) | 20% EtOH in Hex: DCM (3:1, 10 mM $NH_3$-MeOH) | 431 |
| 688 | 906a | 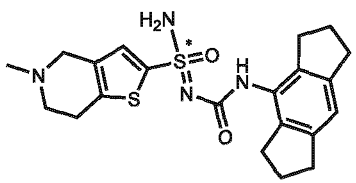 | (S) or (R)-N'-((1,2,3,5,6,7-hexahydro-*s*-indacen-4-yl)carbamoyl)-5-methyl-4,5,6,7-tetrahydrothieno[3,2-c]pyridine-2-sulfonimidamide | CHIRALP AK ID, 2.0*25cm L (5um) | 20% EtOH in Hex: DCM (3:1, 10 mM $NH_3$-MeOH) | 431 |

(continued)

| Ex. # | Cmpd # | Structure | IUPAC Name | Column | Eluents | LC-MS $[M+H]^+$ |
|---|---|---|---|---|---|---|
| 691 | 910b | 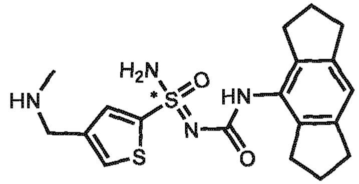 | (R) or (S)-N'-((1,2,3,5,6,7-hexahydro-*s*-indacen-4-yl)carbamoyl)-4-((methylamino)methyl) thiophene-2-sulfonimidamide | CHIRALP AK ID, 2*25cm(5u m) | 30% EtOH in Hex: DCM (3:1, 10 mM $NH_3$-MeOH) | 405 |
| 692 | 910a | 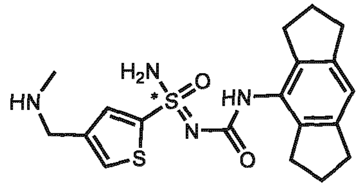 | (S) or (R)-N'-((1,2,3,5,6,7-hexahydro-*s*-indacen-4-yl)carbamoyl)-4-((methylamino)methyl) thiophene-2-sulfonimidamide | CHIRALP AK ID, 2*25cm(5u m) | 30% EtOH in Hex: DCM (3:1, 10 mM $NH_3$-MeOH) | 405 |
| 693 | 897a | 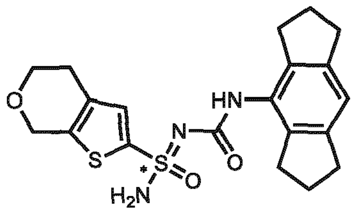 | (R) or (S)-N'-((1,2,3,5,6,7-hexahydro-*s*-indacen-4-yl)carbamoyl)-4,7-dihydro-5H-thieno [2,3 -c] pyran-2-sulfonimidamide | CHIRALP AKIG, 20*250mm, 5 um | 50% EtOH in Hex (0.1% FA) | 418 |
| 694 | 897b | 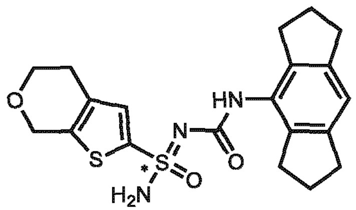 | (S) or (R)-N'-((1,2,3,5,6,7-hexahydro-*s*-indacen-4-yl)carbamoyl)-4,7-dihydro-5H-thieno [2,3 -c] pyran-2-sulfonimidamide | CHIRALP AKIG, 20*250mm, 5 um | 50% EtOH in Hex (0.1% FA) | 418 |

(continued)

| Ex. # | Cmpd # | Structure | IUPAC Name | Column | Eluents | LC-MS $[M+H]^+$ |
|---|---|---|---|---|---|---|
| 695 | 872c | 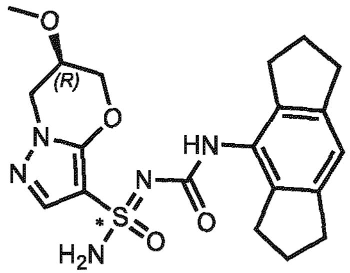 | (R) or (S)- (6R)-N'-((1,2,3,5,6,7-hexahydro-*s*-indacen-4-yl)carbamoyl)-6-methoxy-6,7-dihydro-5H-pyrazolo [5,1-b][1,3]oxazine-3-sulfonimidamide | CHIRALP AKIC, 3*25cm, 5u m | 30% EtOH in Hex (8 mM $NH_3$·MeOH ) | 432 |
| 696 | 872b | 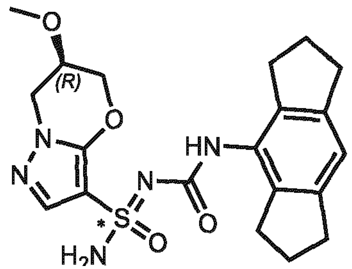 | (S) or (R)- (6R)-N'-((1,2,3,5,6,7-hexahydro-*s*-indacen-4-yl)carbamoyl)-6-methoxy-6,7-dihydro-5H-pyrazolo[5,1-b][1,3]oxazine-3-sulfonimidamide | CHIRALP AKIC, 3*25cm, 5u m | 30% EtOH in Hex (8 mM $NH_3$·MeOH ) | 432 |
| 697 | 870c | 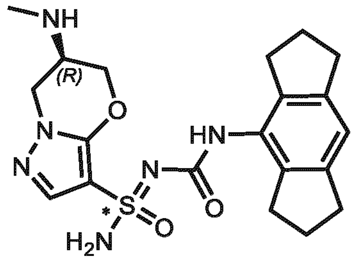 | (R) or (S)- (6R)-N'-((1,2,3,5,6,7-hexahydro-*s*-indacen-4-yl)carbamoyl)-6-(methylamino)-6,7-dihydro-5H-pyrazolo[5,1-b] [1,3]oxazine-3-sulfonimidamide | CHIRALP AK ID, 2*25cm(5u m) | 20% EtOH in Hex: DCM (3:1, 10 mM $NH_3$-MeOH) | 431 |

(continued)

| Ex. # | Cmpd # | Structure | IUPAC Name | Column | Eluents | LC-MS $[M+H]^+$ |
|---|---|---|---|---|---|---|
| 698 | 870b | 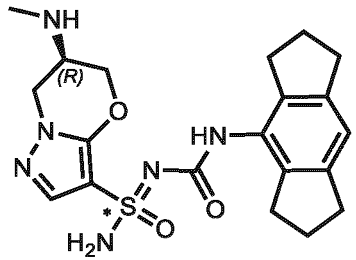 | (S) or (R)- (6R)-N'-((1,2,3,5,6,7-hexahydro-*s*-indacen-4-yl)carbamoyl)-6-(methylamino)-6,7-dihydro-5H-pyrazolo[5,1-b] [1,3]oxazine-3-sulfonimidamide | CHIRALP AK ID, 2*25cm(5u m) | 20% EtOH in Hex: DCM (3:1, 10 mM $NH_3$-MeOH) | 431 |

**Examples 699-709**

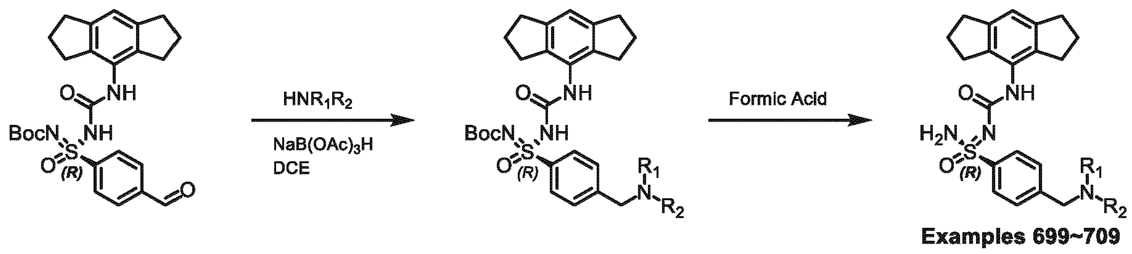


$HNR_1R_2$ = prop-2-ene-1-amine, ethanamine, 2-methoxyethanamine, propan-2-amine, butan-2-amine, tetrahydro-2*H*-pyran-4-amine, cyclobutanamine, *N*-methylcyclobutanamine, cycloheptanamine, 1-methylpiperazine, 2,2-difluoroethan-1-amine

## General procedure for reductive amination

In a flame-dried 25 mL round bottom flask, a primary or secondary amine (0.41 mmol) and sodium triacetoxyborohydride (75 mg, 0.354 mmol) were added to a solution of tert-butyl (*R*)-((4-formylphenyl)(3-(1,2,3,5,6,7-hexahydro-*s*-indacen-4-yl)ureido)(oxo)-$\lambda^6$-sulfanylidene)carbamate (100 mg, 0.207 mmol) in dry DCE (6 mL) under an argon atmosphere. The flask was sealed and the resulting cloudy mixture was stirred at RT for 18h.

In cases where the reduction rate was extremely slow, additional portion of $NaBH_4$ (15 mg, 0.41 mmol) was added to the reaction flask and the resulting mixture was stirred at RT for additional 24 h.

The reaction was quenched with $NaHCO_3$ sat. solution (2.5 mL) and the product was extracted with EtOAc (3 $\times$ 10 mL). The combined organic layers were washed with water (5 mL) and brine (5 mL), dried over $Na_2SO_4$, filtered and concentrated under reduced pressure at 30 °C, to give the products as pale yellow solids.

## General procedure for Boc-deprotection

In a 20 mL vial, the product of reductive amination (0.156 mmol) was dissolved in formic acid (0.9 mL) and the resulting solution was stirred at room temperature overnight (16 h). The solution was concentrated in vacuo at room temperature affording a pale yellow oil, which was triturated in EtOAc resulting in the formation of a white solid. The product was purified by reverse phase flash chromatography using a gradient of 70-100% $CH_3CN$ in $H_2O$ (formic acid 0.1%) generating the desired products as a white powders. Compounds were isolated as formate salts except where indicated.

Examples below were prepared from *tert*-butyl (*R*)-((4-formylphenyl)(3-(1,2,3,5,6,7-hexahydro-*s*-indacen-4-yl)ureido)(oxo)-$\lambda^6$-sulfanylidene)carbamate following the general procedures for reductive amination and then Boc-deprotection above.

**Example 699: (R)-4-((allylamino)methyl)-N'-((1,2,3,5,6,7-hexahydro-s-indacen-4-yl)carbamoyl)- benzenesulfonimidamide**

**Example 700: (R)-4-((ethylamino)methyl)-N'-((1,2,3,5,6,7-hexahydro-s-indacen-4-yl)carbamoyl)- benzenesulfonimidamide**

**Example 701: (*R*)-*N'*-((1,2,3,5,6,7-hexahydro-*s*-indacen-4-yl)carbamoyl)-4-(((2-methoxyethyl)amino)- methyl)benzenesulfonimidamide**

**Example 702: (*R*)-4-((*sec*-butylamino)methyl)-*N'*-((1,2,3,5,6,7-hexahydro-*s*-indacen-4-yl)carbamoyl)- benzenesulfonimidamide**

**Example 703: (R)-N'-((1,2,3,5,6,7-hexahydro-s-indacen-4-yl)carbamoyl)-4-(((tetrahydro-2*H*-pyran-4- yl)-amino)methyl)benzenesulfonimidamide**

**Example 704: (*R*)-4-((cycloheptylamino)methyl)-*N'*-((1,2,3,5,6,7-hexahydro-*s*-indacen-4-yl)- carbamoyl)-benzenesulfonimidamide**

**Example 705: (*R*)-4-((cyclobutylamino)methyl)-*N'*-((1,2,3,5,6,7-hexahydro-*s*-indacen-4-yl)carbamoyl)- benzenesulfonimidamide**

**Example 706: (*R*)-*N'*-((1,2,3,5,6,7-hexahydro-*s*-indacen-4-yl)carbamoyl)-4-((isopropylamino)methyl)- benzenesulfonimidamide**

**Example 707: (*R*)-*N'*-((1,2,3,5,6,7-hexahydro-*s*-indacen-4-yl)carbamoyl)-4-((4-methylpiperazin-1-yl)- methyl)benzenesulfonimidamide**

**Example 708: (*R*)-4-(((2,2-difluoroethyl)amino)methyl)-*N'*-((1,2,3,5,6,7-hexahydro-*s*-indacen-4-yl)- carbamoyl)benzenesulfonimidamide**

**Example 709: (*R*)-4-((cyclobutyl(methyl)amino)methyl)-*N'*-((1,2,3,5,6,7-hexahydro-*s*-indacen-4-yl)- carbamoyl)benzenesulfonimidamide**

Table 53: Structure and analytical data for Examples 699 to 709

| Example | Cmpd # | Structure | $^1$H-NMR (400 MHz, $CD_3OD$) δ: ppm | MS-ESI |
|---|---|---|---|---|
| 699 | 885a | 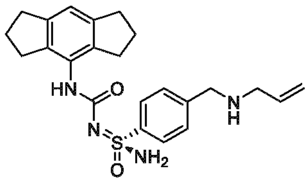 | 8.48 ( br s, 1H), 8.04 ( br s, 2H), 7.64 (d, *J* = 8.2 Hz, 2H), 6.90 (s, 1H), 6.01-5.90 (m, 1H), 5.50-5.42 (m, 2H), 4.18 (s, 2H), 3.60 (d, *J* = 6.7 Hz, 2H), 2.84-2.81 (m, 4H), 2.78-2.67 (m, 4H), 2.04-1.97 (m, 4H). | 425.3 |
| 700 | 884a | 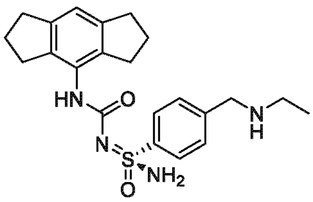 | 8.53 ( br s, 1H), 8.04 ( br s, 2H), 7.64 (d, *J* = 8.1 Hz, 2H), 6.90 (s, 1H), 4.61 ( br s, 2H), 4.18 (s, 2H), 3.02 (q, *J* = 7.3 Hz, 2H), 2.84-2.80 (m, 4H), 2.78-2.68 (m, 4H), 2.04-1.97 (m, 4H), 1.29 (t, *J* = 7.3 Hz, 3H). | 413.3 |
| 701 | 883a | 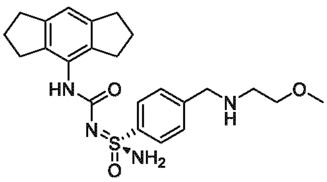 | 8.02 ( br s, 2H), 7.63 (d, *J* = 8.1 Hz, 2H), 6.90 (s, 1H), 4.61 ( br s, 3H), 4.16 (s, 2H), 3.62-3.59 (m, 2H), 3.39 (s, 3H), 3.09-3.06 (m, 2H), 2.84-2.80 (m, 4H), 2.76-2.69 (m, 4H), 2.04-1.96 (m, 4H). | 443.3 |
| 702 | 882a | 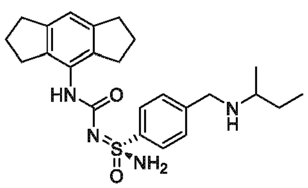 | 8.51 (s, 1H), 8.05 ( br s, 2H), 7.67 (d, *J* = 8.2 Hz, 2H), 6.90 (s, 1H), 4.62 ( br s, 2H), 4.29 (d, *J* = 13.4 Hz, 1H, AB system), 4.23 (d, *J* = 13.4 Hz, 1H, AB system), 3.22-3.13 (m, 1H), 2.84-2.80 (m, 4H), 2.78-2.69 (m, 4H), 2.04-1.97 (m, 4H), 1.94-1.84 (m, 1 H), 1.63-1.51 (m, 1H), 1.35 (d, *J* = 6.6 Hz, 3H), 1.02 (t, *J* = 7.5 Hz, 3H). | 441.3 |

(continued)

| Example | Cmpd # | Structure | $^1$H-NMR (400 MHz, $CD_3OD$) δ: ppm | MS-ESI |
|---|---|---|---|---|
| 703 | 880a | 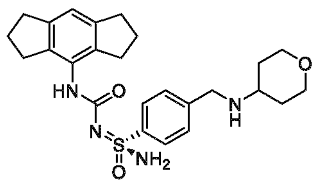 | 8.43 (s, 1H), 8.05 ( br s, 2H), 7.67 (d, *J* = 8.1 Hz, 2H), 6.90 (s, 1H), 4.26 (s, 2H), 4.03 (dd, *J* = 11.8, 4.4 Hz, 2H), 3.43 (t, J = 11.8 Hz, 2H), 3.38-3.33 (m, 1H), 2.84-2.81 (m, 4H), 2.78-2.69 (m, 4H), 2.08-1.96 (m, 6H), 1.67 (qd, *J* = 12.1, 4.6 Hz, 2H). | 469.4 |
| 704 | 879a | 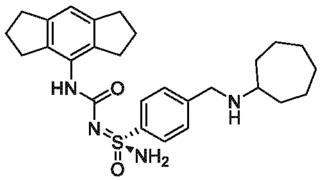 | 8.51 (s, 1H), 8.04 ( br s, 2H), 7.66 (d, *J* = 8.2 Hz, 2H), 6.90 (s, 1H), 4.60 (br s, 3H), 4.25 (s, 2H), 3.27-3.19 (m, 1H), 2.84-2.80 (m, 4H), 2.78-2.68 (m, 4H), 2.18-2.11 (m, 2H), 2.04-1.97 (m, 4H), 1.85-1.77 (m, 2H), 1.72-1.50 (m, 8H). | 481.4 |
| 705 | 878a | 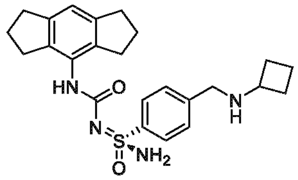 | 8.48 (s, 1H), 8.04 ( br s, 2H), 7.64 (d, *J* = 8.2 Hz, 2H), 6.90 (s, 1H), 4.12 (s, 2H), 3.73 (quin, *J* = 8.1 Hz, 1H), 2.84-2.80 (m, 4H), 2.78-2.68 (m, 4H), 2.34-2.28 (m, 2H), 2.21-2.11 (m, 2H), 2.04-1.97 (m, 4H), 1.93-1.84 (m, 2H). | 439.3 |
| 706 | 877a | 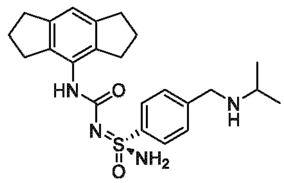 | 8.51 (s, 1H), 8.05 ( br s, 2H), 7.67 (d, *J* = 8.1 Hz, 2H), 6.90 (s, 1H), 4.25 (s, 2H), 3.40 (sep, *J* = 6.5 Hz, 1H), 2.84-2.80 (m, 4H), 2.77-2.69 (m, 4H), 2.04-1.97 (m, 4H), 1.37 (d, *J* = 6.5 Hz, 6H). | 427.3 |
| 707 | 876a | 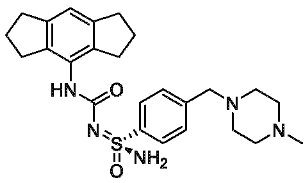 | 8.45 ( br s, 1H), 7.96 ( br s, 2H), 7.55 (d, *J* = 8.1 Hz, 2H), 6.90 (s, 1H), 3.68 (s, 2H), 3.08 ( br s, 4H), 2.84-2.80 (m, 4H), 2.77-2.62 (m, 8H), 2.71 (s, 3H), 2.03-1.96 (m, 4H). | 468.4 |
| 708 | 881a | 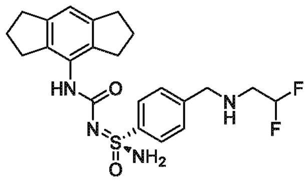 | 8.38 ( br s, 1H), 7.96 ( br s, 2H), 7.55 (d, *J* = 8.1 Hz, 2H), 6.89 (s, 1H), 5.91 (tt, *J* = 56.2, 4.2 Hz, 1H), 3.92 (s, 2H), 2.91 (td, *J* = 15.5, 4.2 Hz, 2H), 2.84-2.80 (m, 4H), 2.77-2.68 (m, 4H), 2.04-1.96 (m, 4H). | 449.3 |
| 709 | 827a | 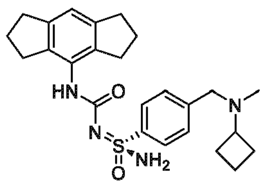 | 7.98 ( br s, 2H), 7.55 (d, *J* = 8.1 Hz, 2H), 6.89 (s, 1H), 3.66 (s, 2H), 3.14-3.06 (m, 1H), 2.84-2.80 (m, 4H), 2.76-2.68 (m, 4H), 2.15 (s, 3H), 2.14-2.09 (m, 2H), 2.05-1.96 (m, 6H), 1.79-1.68 (m, 2H). | 453.3 |

**Examples 710 (Compound 875a) and 711 (Compound 875b)**

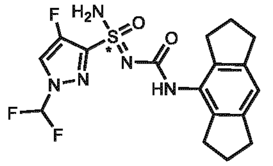

and

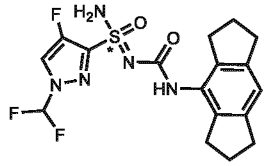

(R)- and (S)-1-(Difluoromethyl)-4-fluoro-*N*'-(1,2,3,5,6,7-hexahydro-*s*-indacen-4-yl)carbamoyl)-1H- pyrazole-3-sulfonimidamide

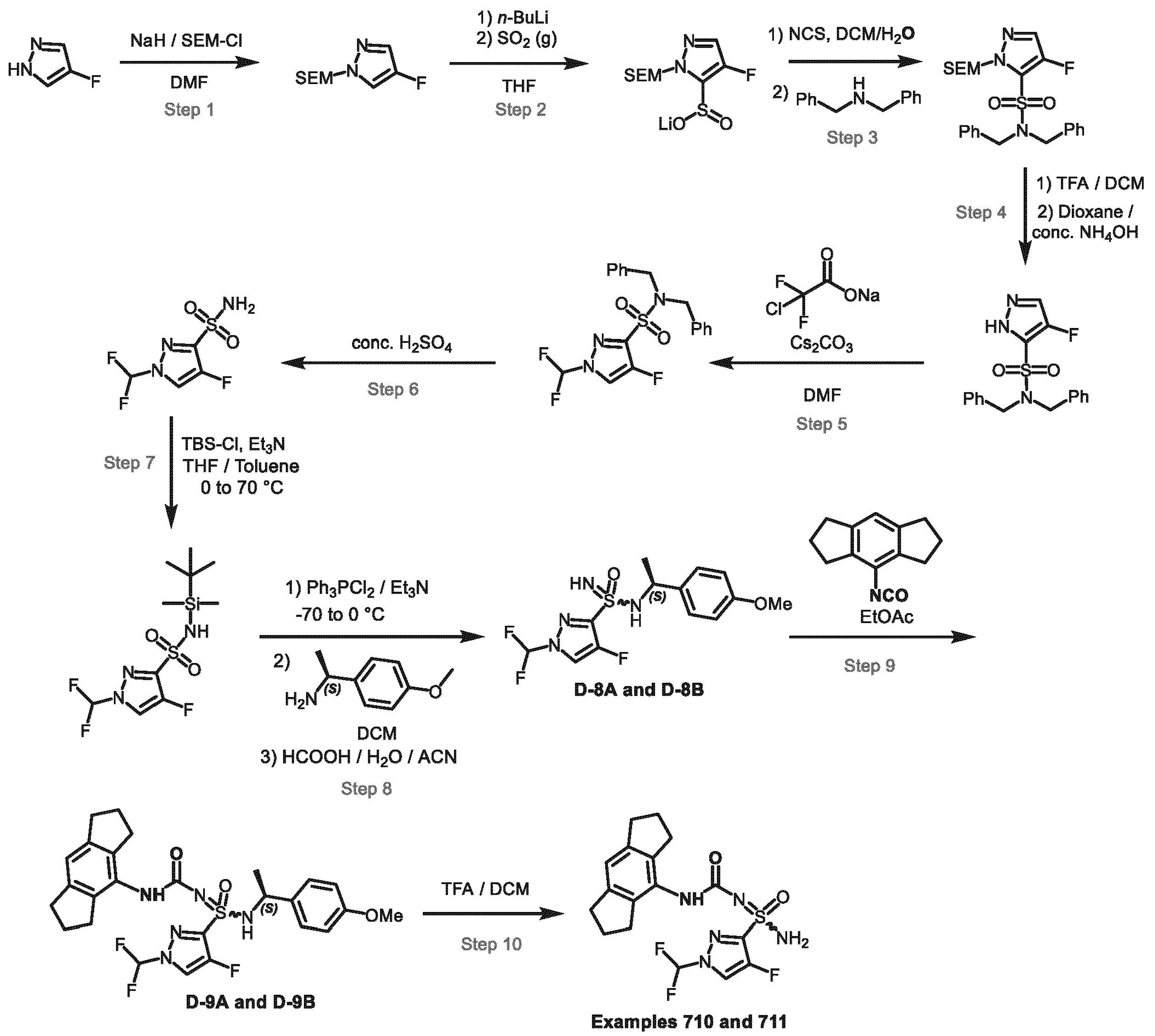


**Step 1: 4-Fluoro-1-((2-(trimethylsilyl)ethoxy)methyl)-1*H*-pyrazole**

In a 1-L flask, a cold (0 °C) solution of 4-fluoro-1*H*-pyrazole (14.14 g, 115.0 mmol) in dry DMF (150 mL) was treated carefully portion-wise with NaH (60% in oil, 5.31 g, 132.8 mmol) over 20 min. The suspension was then treated dropwise with SEM-Cl (23.0 mL, 129.7 mmol) keeping the internal temperature below 10 °C. The suspension was left to warm to RT and stirred for 7 h. The reaction was diluted with EtOAc (500 mL) and quenched with half-saturated brine (1.4 L). The organic layer was separated and washed again with half-saturated brine (2x500 mL) and brine (300 mL). The aqueous phases were back extracted with EtOAc (2x500 mL). The organic layers were combined and dried over $Na_2SO_4$, filtered and concentrated in vacuo. Purification on normal phase silica using a gradient of 100% hexanes to

30% EtOAc / hexanes gave 23.0 g of the title compound as a colorless oil (85%). MS-ESI: 217.1 (M+1).

**Step 2: lithium 4-fluoro-1-((2-(trimethylsilyl)ethoxy)methyl)-1*H*-pyrazole-5-sulfinate**

In a 1-L round bottom flask, a cold (-70 °C) solution of 4-fluoro-1-((2-(trimethylsilyl)ethoxy)methyl)- 1*H*-pyrazole (19.98 g, 92.36 mmol) in dry THF (150 mL) was treated dropwise with n-BuLi (2.5 M in hexanes, 40.0 mL, 100.00 mmol) over 20 min by the keeping internal temperature below -65 °C. After 1 h at -70 °C, an atmosphere of $SO_2$ (g) was introduced via balloon. After stirring for 20 min at -70 °C the dry-ice bath was removed, and the reaction mixture was left to warm to RT. The suspension was left to stir at RT for 30 min. The crude reaction mixture was concentrated *in vacuo* to provide a beige solid. The material was mixed with DCM (200 mL) and sonicated for 15 min and triturated over 5 h. The solid was collected by filtration and after drying under vacuum 24.39 g (86%) of the title compound was isolated as a beige solid. The product was used as is in the next step.

**Step 3: *N*,*N*-dibenzyl-4-fluoro-1-((2-(trimethylsilyl)ethoxy)methyl)-1*H*-pyrazole-5-sulfonamide**

In a 100-mL round bottom flask, a cold (0 °C, ice-water bath) suspension of lithium 4-fluoro-1-((2-(trimethylsilyl)ethoxy)methyl)-1*H*-pyrazole-5-sulfinate (24.4 g, 79.76 mmol) in a mixture of DCM (200 mL) and water (100 mL) was treated in two portions with NCS (11.98 g, 89.715 mmol) over 3-5 min under vigorous stirring by keeping internal temperature below 10 °C. The biphasic solution was stirred for 1 h.

To the biphasic reaction mixture cooled at 0 °C, $Et_3N$ (15 mL, 107.6 mmol) was added in one portion followed by *N*,*N*-dibenzylamine (20.0 mL, 104.0 mmol). Internal temperature was kept below 7-8 °C. Then, the ice-water bath was removed and the reaction was left to warm to RT over 1h. The biphasic solution was diluted with more DCM (100 mL) and water (100 mL). The organic layer was separated and washed with water (100 mL) again. The aqueous layers were back extracted with DCM (2x100 mL). The organic layers were combined, cooled to 0 °C, and treated slowly by the addition of aq. HCl 0.5 N (120 mL, 60 mmol) over 5-10 min by keeping internal temperature below 10 °C. The organic layer was separated (500 mL) and washed with half-saturated brine (100 mL). The aqueous layers were back extracted with DCM (100 mL).

The organic layers were combined, dried over $Na_2SO_4$, concentrated and purified through the use of a short silica plug, eluting with dichloromethane. 31.88 g (80%) of the title compound was isolated as a colorless oil. MS-ESI: 498.2 (M+Na).

**Step 4: *N*,*N*-dibenzyl-4-fluoro-1*H*-pyrazole-5-sulfonamide**

In a 1000-ml round bottom flask, a cold (0 °C) solution of *N*,*N*-dibenzyl-4-fluoro-1-((2-(trimethylsilyl)- ethoxy)methyl)-1*H*-pyrazole-5-sulfonamide (31.76 g, 66.77 mmol) in DCM (100 mL) was treated slowly with TFA (50 mL, 653 mmol). The ice-water bath was removed and the reaction was left to warm to RT and stirred for 2 h. The reaction was concentrated *in vacuo* and the residue was dissolved in 1,4-dioxane (45 mL), cooled to 10 °C, and treated very slowly over 5 min with conc. $NH_4OH$ (28-30%, 45 mL, 335 mmol). The mixture was stirred for 2 h at RT. The reaction mixture was diluted with water (100 mL) and brine (100 mL) and extracted with EtOAc (200 mL). The organic layer was washed with brine (100 mL). The aqueous phases were back extracted with EtOAc (150 mL). The organic layers were combined, dried over $Na_2SO_4$, filtered and concentrated *in vacuo.*

After evaporation, a waxy gummy white solid was obtained. This crude material was purified by normal phase silica column chromatography, eluting with EtOAc / hexanes (1:2). The title compound was isolated as a white solid (17.62 g, 72%). MS-ESI: 346.1 (M+1).

**Step 5: *N*,*N*-dibenzyl-1-(difluoromethyl)-4-fluoro-1*H*-pyrazole-3-sulfonamide**

In a 1-L round bottom flask equipped with a condenser, a suspension of *N*,*N*-dibenzyl-4-fluoro-1*H*-pyrazole-5-sulfonamide (17.49 g, 50.638 mmol), sodium chlorodifluoroacetate (9.48 g, 62.18 mmol) and $Cs_2CO_3$ (20.07 g, 61.60 mmol) in anhydrous DMF (70 mL) was heated slowly to 130 °C over 2 h. The reaction was maintained at this temperature for 16 h. The brown suspension was cooled to RT and poured into half-saturated brine (700 mL) and extracted with EtOAc (250 mL). The organic layer was washed again with half-saturated brine (300 mL) and then brine (150 mL). The aqueous phases were back extracted with EtOAc (200 mL). The organic layers were combined and dried over $Na_2SO_4$. After concentration, the resulting dark residue was purified by silica column chromatography, eluting with EtOAc / hexanes (1:1). After evaporation, the title compound was isolated as a brown oil (15.12g, 65%). MS-ESI: 418.1 (M+1).

**Step 6: 1-(difluoromethyl)-4-fluoro-1H pyrazole-3-sulfonamide**

In a 1-L round bottom flask (1000 mL), finely powdered *N*,*N*-dibenzyl-1-(difluoromethyl)-4-fluoro-1*H*- pyrazole-3-sulfonamide (15.1 g, 32.9 mmol) was cooled to 0 °C, treated with cold conc. $H_2SO_4$ (90 mL, 1620 mmol) and stirred at 0 °C for 60 min. The yellow suspension was added dropwise to ice and the suspension was diluted with water (1.5 L) and solid NaCl (200 g) was added. The aqueous phase was extracted with EtOAc (800 mL) and washed with brine (200 mL). The aqueous phases were back extracted with EtOAc (2x400 mL). The organic layers were combined, dried over $Na_2SO_4$, filtered through Celite and concentrated *in vacuo.* The residue was re-dissolved in EtOAc (200 mL) and decolorized using active charcoal. The solution was concentrated in vacuo. The crude was triturated with DCM (100 mL) and heptane (150 mL). After filtration and drying, the title compound was isolated (4.807 g, 66%) as a beige solid. MS-ESI: 216.1 (M+1).

**Step 7: *N*-(*tert*-butyldimethylsilyl)-1-(difluoromethyl)-4-fluoro-1*H*-pyrazole-3-sulfonamide**

In a 100-mL round bottom flask, a cold (0 °C) solution of 1-(difluoromethyl)-4-fluoro-1H-pyrazole-3- sulfonamide (1.015 g, 4.716 mmol) in THF (4 mL) was treated dropwise with $Et_3N$ (1.4 mL, 10 mmol) keeping internal temperature below 5 °C. After 5 min, a solution of *tert*-butyldimethylsilyl chloride (975 mg, 6.469 mmol) in toluene (0.9 mL) was added via a cannula by keeping internal temperature below 7 °C. The suspension was warmed to RT and heated to 70 °C for 3 h.

The reaction mixture was diluted with MTBE (50 mL). The organic phase was washed twice with aq. $NaHCO_3$ (2x40 mL), and brine (40 mL). The organic phase was dried over $Na_2SO_4$, treated with active charcoal (160 mg), filtered through Celite and concentrated *in vacuo.* The residue was co-evaporated with heptane. The solid obtained was triturated with heptane (45 mL) and MTBE (3 mL) overnight. After filtration and drying, the title compound was isolated as a beige solid (1.07 g, 67%). MS-ESI: 330.1 (M+1).

**Step 8: (*R*)-1-(difluoromethyl)-4-fluoro-N-((*S*)-1-(4-methoxyphenyl)ethyl)-1*H*-pyrazole-3-sulfonimidamide and (*S*)-1-(difluoromethyl)-4-fluoro-N-((*S*)-1-(4-methoxyphenyl)ethyl)-1*H*-pyrazole- 3-sulfonimidamide (D-8A and D-8B)**

In a 25-mL round bottom flask, a cold (0 °C) solution of $PPh_3Cl_2$ (826 mg, 2.48 mmol) in dry DCM (4 mL) was treated dropwise with $Et_3N$ (0.52 mL, 3.73 mmol). The ice water bath was removed and a suspension was obtained. The reaction was allowed to warm to RT and stirred for 10 min at this temperature. The white suspension was cooled to -70 °C and a solution of N-(tertbutyldimethylsilyl)-1-(difluoromethyl)-4-fluoro- 1*H*-pyrazole-3-sulfonamide (405 mg, 1.23 mmol) in DCM (2 mL) was added via a cannula by keeping internal temperature below -50 °C. The dry ice bath was removed and the reaction was allowed to warm to 0 °C. The reaction was stirred at 0 °C for 30 min. Then, the reaction mixture was cooled back to -70 °C and (*S*)-(-)-4-Methoxy-$\alpha$-methylbenzylamine (0.55 mL, 3.72 mmol) was added dropwise by keeping internal temperature below -45 °C. After 5 min, the dry ice bath was removed and the suspension was left to warm to 0 °C. The suspension was stirred at 0 °C for 30 min and left to warm to RT for an additional stirring period of 90 min.

The solvent was removed under reduced pressure to give a crude residue which was treated with a mixture of ACN (4 mL), water (2 mL) and HCOOH (0.46 mL, 12.2 mmol). The reaction was stirred at RT for 2 h. The reaction was concentrated under vacuum to remove half the volume of ACN and, after, diluted with EtOAc (50 mL), washed with 1% HCOOH in half-saturated brine (2 $\times$ 30mL), aq. $NaHCO_3$:brine (1:1) (2 $\times$ 30 mL) and brine (25 mL). The aqueous phases were back extracted with EtOAc (50 mL). The organic layers were combined and dried over $Na_2SO_4$, filtered and concentrated *in vacuo.* The crude residue was dissolved in DCM / hexanes (1:1) and purified on silica gel column by eluting with a gradient of 100% hexanes to 90% EtOAc / hexanes. The title compounds were isolated as white solids (first eluting diastereomer D-8A, $R_f$ = 0.60 (DCM/EtOAc = 1:1), 104.9 mg, 24% yield, and second eluting diastereomer D-8B, $R_f$ = 0.51 (DCM/EtOAc = 1:1), 73 mg, 17% yield). Absolute stereochemistry was not determined. MS-ESI: 371.1 (M+Na).

**Step 9: (*R*)-1-(difluoromethyl)-4-fluoro-*N'*-((1,2,3,5,6,7-hexahydro-*s*-indacen-4-yl)carbamoyl)-*N*-((*S*)- 1-(4-methoxyphenyl)ethyl)-1*H*-pyrazole-3-sulfonimidamide and (*S*)-1-(difluoromethyl)-4-fluoro-*N'*- ((1,2,3,5,6,7-hexahydro-*s*-indacen-4-yl)carbamoyl)-N-((S)-1-(4-methoxyphenyl)ethyl)-1*H*-pyrazole-3-sulfonimidamide (I-9A and I-9B)**

In a 25 mL round bottom flask, a solution of 1-(difluoromethyl)-4-fluoro-N'-((1,2,3,5,6,7-hexahydro- *s*-indacen-4-yl)carbamoyl)-N-((*S*)-1-(4-methoxyphenyl)ethyl)-1H-pyrazole-3-sulfonimidamide (diastereomer **D-8A**, 49.4 mg, 0.142 mmol) in EtOAc (1.5 mL) was treated with 4-isocyanato-1,2,3,5,6,7-hexahydro- s-indacene (33.5 mg, 0.168 mmol) in one portion. The reaction was stirred for 1h at RT. Then, heptane (2 mL) was added dropwise over 2-3 min and a white solid was observed. The mixture was stirred at RT for a total of 2 h. The white solid was collected by filtration and the

material was washed with EtOAc / heptane (1 mL / 2 mL) and dried under high vacuum to give the title compound as diastereomer **D-9A** (43.4 mg, 55%) as a white solid. Similarly, 40 mg of Diastereomer B gave diastereomer **D-9B** (43.9 mg, 69%). MS-ESI: 548.2 (M+1).

**Step 10: (*S*)-1-(difluoromethyl)-4-fluoro-*N*'-((1,2,3,5,6,7-hexahydro-*s*-indacen-4-yl)carbamoyl)-1*H*- pyrazole-3-sulfonimidamide and (*R*)-1-(difluoromethyl)-4-fluoro-*N*'-((1,2,3,5,6,7-hexahydro-*s*-indacen- 4-yl)-carbamoyl)-1*H*-pyrazole-3-sulfonimidamide (Examples 710 and 711)**

In a 5-mL round bottom flask, a cold (0 °C) suspension of diastereomer **D9-A** (31.4 mg, 0.057 mmol) in DCM (1 mL) was treated dropwise with TFA (0.4 mL, 5.2 mmol). The resulting solution was left to warm to RT by removing the ice-water bath after 5 min. The solution was stirred at RT for 2 h. Then, the reaction mixture was added to a 50 mL flask containing heptane (20 mL) and the solution was concentrated *in vacuo.* The material was suspended in DCM (20 mL) and heptane (20 mL) was added and triturated overnight at RT. The white solid was collected by filtration, washed with DCM / heptane (1:1, 2x2 mL) to afford 18.6 mg (76%) of the title product (Example 710). Absolute stereochemistry was not determined. $^1$H NMR (400 MHz, DMSO): $\delta$ 8.62 (d; J = 4.59 Hz; 1 H); 8.45 (br s; 1 H); 7.90 (s; 2 H); 7.83 (t; J = 58.27 Hz; 1 H); 6.86 (s; 1 H); 2.75 (t; J = 7.35 Hz; 4 H); 2.56-2.69 (m; 4 H); 1.86-1.93 (m; 4 H). MS(ESI): 414.1 (M+1).

Example 711 was generated from diastereomer **D-9B** using the same procedure, yielding 17.6 mg (73%) of product. Absolute stereochemistry was not determined. $^1$H NMR (400 MHz, DMSO): $\delta$ 8.62 (d; J = 4.57 Hz; 1 H); 8.46 (br s; 1 H); 7.90 (s; 2 H); 7.83 (t; J = 58.25 Hz; 1 H); 6.85 (s; 1 H); 2.75 (t; J = 7.32 Hz; 4 H); 2.54-2.68 (m; 4 H); 1.86-1.93 (m; 4 H). MS(ESI): 414.1 (M+ 1).

**Example 712 (Compound 886a)**

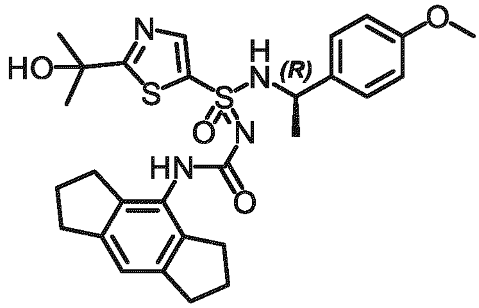

*N*'-((1,2,3,5,6,7-hexahydro-*s*-indacen-4-yl)carbamoyl)-2-(2-hydroxypropan-2-yl)-*N*-((*R*)-1-(4-methoxyphenyl)ethyl)thiazole-5-sulfonimidamide

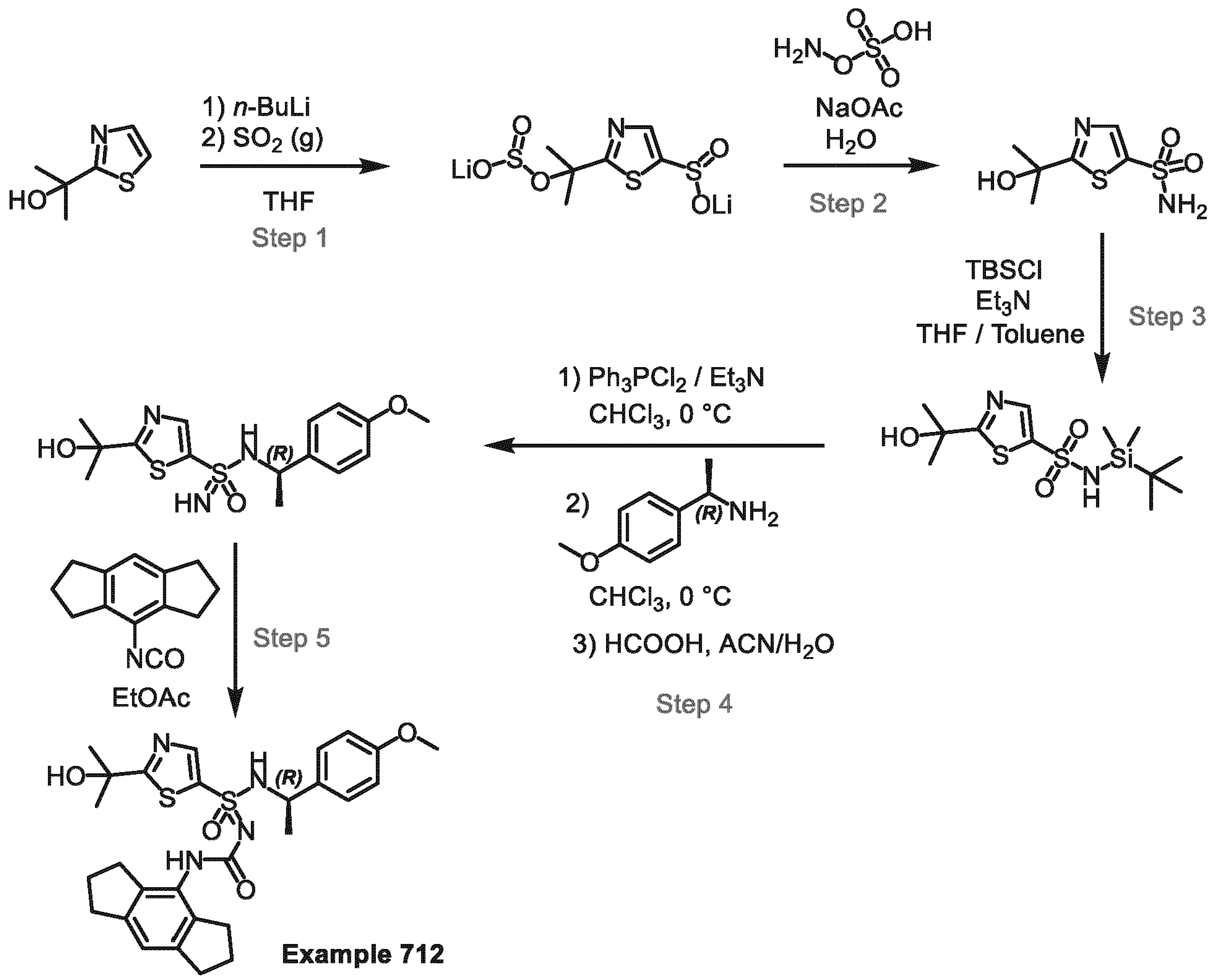


**Example 712**

**Step 1: lithium 2-(5-sulfinatothiazol-2-yl)propan-2-yl sulfite**

In a flame-dried 500 mL round bottom flask, a cold (-70 °C) solution of 2-(thiazol-2-yl)propan-2-ol (10.101 g, 70.538 mmol) in dry THF (100 mL) was treated dropwise with n-BuLi (2.5 M in hexanes, 60 mL, 150 mmol). After 1 h at -70 °C, an atmosphere of $SO_2$ (g) was introduced for 15 min using a balloon. Then, while maintaining the $SO_2$ atmosphere, the dry-ice bath was removed and the suspension was allowed to warm to RT over 30 minutes. The suspension was cooled to 0°C and collected by filtration and washed with $Et_2O$. The very fine off-white powder was dried under high vacuum to give 23.4 g (94%) of the title compound as an off-white powder. MS(ESI): 208.0 (M+1).

**Step 2: 2-(2-hydroxypropan-2-yl)thiazole-5-sulfonamide**

In a 100 mL round bottom flask immersed in a cool water bath, a suspension of hydroxylamine-O-sulfonic acid (18.64 g, 164.8 mmol) in water (50 mL) was treated with NaOAc (9.007 g, 109.8 mmol) followed by addition of lithium 2-(5-sulfinatothiazol-2-yl)propan-2-yl sulfite (15.10 g, 55.13 mmol). The white suspension was stirred at RT for 1 h. The reaction mixture was diluted with more water and extracted with EtOAc. The organic layer was separated and washed with water and brine. The aqueous phases (pH around 1-2) were back extracted with EtOAc. The organic layers were combined and dried over $Na_2SO_4$, filtered and concentrated *in vacuo*. The off-white solid was triturated in $CHCl_3$ (250 mL) for 1 h under reflux. The hot suspension was cooled to RT and the material was collected by filtration. The filtered material was dried under high vacuum to give the title compound as an off-white solid (9.11 g, 72%). MS(ESI): 223.0 (M+ 1).

**Step 3: *N*-(*tert*-butyldimethylsilyl)-2-(2-hydroxypropan-2-yl)thiazole-5-sulfonamide**

In a 100-mL round-bottom flask, a cloudy solution of 2-(2-hydroxypropan-2-yl)thiazole-5-sulfonamide (8.110 g, 36.49 mmol) in THF (30 mL) was treated with $Et_3N$ (11.5 mL, 82.5 mmol). The mixture was cooled to 0 °C and a solution

of tert-butyl dimethyl silyl chloride (7.55 g, 50.1 mmol) in toluene (10 mL) was added. The suspension was warmed to RT and heated to 70 °C for 3 h.

The reaction mixture was diluted with MTBE. The organic phase was washed twice with aq. $NaHCO_3$, and brine, dried over $Na_2SO_4$, filtered through Celite® and concentrated *in vacuo.* The beige solid was recrystallized by dissolving in MTBE (20-40 mL) and adding heptane (300 mL). After filtration and drying, the title compound was isolated as an off-white solid (11.35 g, 91%). MS(ESI): 337.1 (M+1).

**Step 4: 2-(2-hydroxypropan-2-yl)-*N*-((*R*)-1-(4-methoxyphenyl)ethyl)thiazole-5-sulfonimidamide**

In a 10 mL round-bottom flask, a suspension of $PPh_3Cl_2$ (380 mg, 1.140 mmol) in dry $CHCl_3$ (2 mL) was treated with $Et_3N$ (0.25 mL, 1.79 mmol). The mixture was stirred for 10 min at RT. The white suspension was cooled to 0 °C and a solution of *N*-(*tert*-butyldimethylsilyl)-2-(2-hydroxypropan-2-yl)-thiazole-5- sulfonamide (201 mg, 0.597 mmol) in $CHCl_3$ (1 mL) was added via a cannula; a yellow solution was immediately obtained. The reaction mixture was stirred for 20 min at 0 °C. To the reaction mixture was added (*R*)-(+)-4-methoxy-α-methylbenzylamine (0.175 mL, 1.185 mmol) in one portion at 0 °C. The mixture was stirred at 0 °C for 30 min and warmed slowly to RT for 3 h. After an additional 6 h of stirring at RT, extra (R)-(+)-4-Methoxy-α-methylbenzylamine (0.045 mL, 0.305 mmol) was added and stirring continued for another 16 hours.

The solvent was removed under reduced pressure to give a crude residue which was dissolved in ACN (2 mL). To the suspension was added water (1 mL) and HCOOH (0.225 mL, 5.96 mmol) and a yellow cloudy solution was obtained. The reaction was stirred at RT for 1 h. The reaction was diluted with EtOAc, washed with 1% HCOOH in half-saturated brine (2 × 50 mL), aq. $NaHCO_3$ : brine (1:1) (2 × 50 mL) and brine. The aqueous phases were back extracted with EtOAc. The organic layers were combined and dried over $Na_2SO_4$. The title compound was isolated as a 1:1 mixture of diastereomers (212 mg, quantitative) and was brought to the next step as is. MS(ESI): 356.1 (M+1).

**Step 5: *N*-((1,2,3,5,6,7-hexahydro-s-indacen-4-yl)carbamoyl)-2-(2-hydroxypropan-2-yl)-*N*-((*R*)-1-(4-methoxyphenyl)ethyl)thiazole-5-sulfonimidamide (Example 712)**

In a 25 mL round-bottom flask, a mixture of crude 2-(2-hydroxypropan-2-yl)-*N*-((*R*)-1-(4-methoxyphenyl)-ethyl)thiazole-5-sulfonimidamide (0.597 mmol) and 4-isocyanato-1,2,3,5,6,7-hexahydro-s-indacene (138 mg, 0.693 mmol) was dissolved in EtOAc (2 mL). The clear reaction solution was stirred at RT for 3 h. A white precipitate was observed to form after 30 min. The suspension was diluted with more EtOAc (4 mL) and collected by filtration. The white solid was washed with more EtOAc (3x), dried under high vacuum overnight giving 207 mg (61%) of the title compound as a white solid (1: 1 mixture of diastereomers as measured by proton NMR). MS(ESI): 555.2 (M+1).

**Example 713 (Compound 874b)**

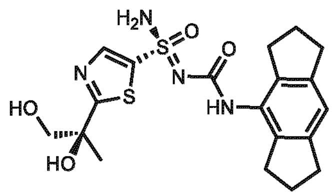

(*R*)-2-((*S*)-1,2-dihydroxypropan-2-yl)-*N'*-((1,2,3,5,6,7-hexahydro-*s*-indacen-4-yl)carbamoyl)thiazole-5-sulfonimidamide

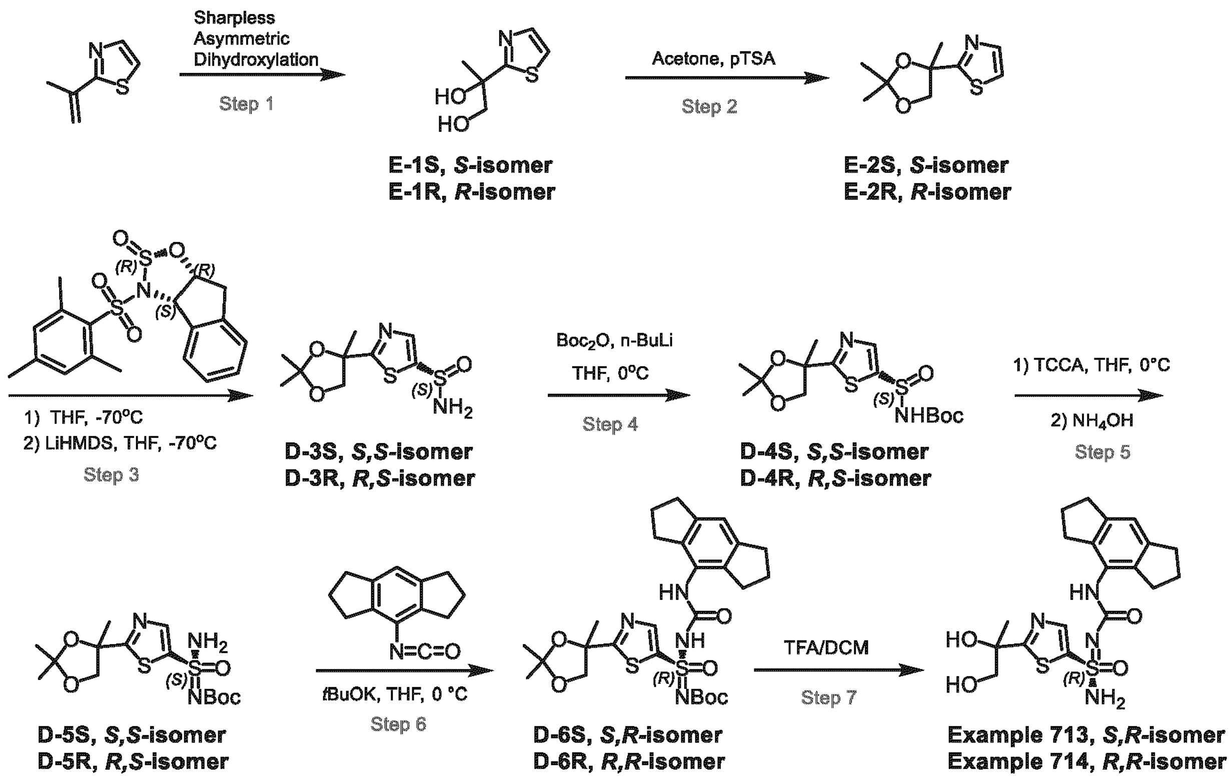


**E-1S, *S*-isomer**
**E-1R, *R*-isomer**
**E-2S, *S*-isomer**
**E-2R, *R*-isomer**
**D-3S, *S,S*-isomer**
**D-3R, *R,S*-isomer**
**D-4S, *S,S*-isomer**
**D-4R, *R,S*-isomer**
**D-5S, *S,S*-isomer**
**D-5R, *R,S*-isomer**
**D-6S, *S,R*-isomer**
**D-6R, *R,R*-isomer**
**Example 713, *S,R*-isomer**
**Example 714, *R,R*-isomer**

**Step 1: (*S*)-2-(thiazol-2-yl)propane-1,2-diol (E-1S)**

In a 1 L round-bottom flask, t-BuOH (230 mL), water (230 mL) and THF (230 mL) was added, AD-mix-beta (111.8 g, 1.4 g per mmol of propenyl thiazole), followed by methanesulfonamide (7.60 g, 79.88 mmol) and $(DHQD)_2AQN$ (2.74 g, 3.195 mmol). The reaction mixture was cooled to 0 °C with a mechanical stirrer. Into the cooled reaction mixture was added 2-propenyl thiazole (10 g, 79.8 mmol) and stirred for approximately 1 h at this temperature and then the ice-water bath was removed and stirred at RT for 24h. The reaction mixture was diluted with $H_2O$ (100 mL) and was extracted with EtOAc (3 × 400mL). The combined organic extracts were washed with $H_2O$ (75 mL) and brine (100 mL), dried over $Na_2SO_4$ and concentrated to give a yellowish orange solid. The crude material was purified by column chromatography using silica gel (20% methanol in DCM) to give 9.2 g of the product with 80% ee. Crystallization from mixture of ethyl acetate (140 mL), methanol (10 mL) and hexanes (420 mL) gave 6.4 g (70%) as a white solid with 99% ee. Chiral HPLC conditions; 70% hexanes +30% ethanol (0.1% DEA), Chiralpak IF column, flow rate; 1 mL/min, run time; 15 mins, Rt = 10.3 min).

**Step 2: (*S*)-2-(2,2,4-trimethyl-1,3-dioxolan-4-yl)thiazole (E-2S)**

In a 25 mL single neck round bottom flask was charged (*S*)-2-(thiazol-2-yl)propane-1,2-diol (6.4 g, 40.2 mmol, ee 99%) followed by acetone (50 mL) and stirred to get a clear solution at RT. pTSA (690 mg, 4.02 mmol) was added and stirred for 20 h at 40°C. The reaction mixture then cooled to RT, diluted with ethyl acetate (150 mL) and washed with satd. $NaHCO_3$ solution (20 mL). The collected organic layer was washed with brine solution (50 mL), dried over anhydrous $Na_2SO_4$ and concentrated to give a crude oil. The crude oil was purified on silica gel by eluting from 100% hexanes to 20% ethyl acetate to the title compounds as a colorless oil (4.8 g, 60%). The material was used in the next step as is.

**Step 3: (S)-2-((S)-2,2,4-trimethyl-1,3-dioxolan-4-yl)thiazole-5-sulfinamide (D-3S)**

This step was performed using the procedure described in Scheme 103, Step 3 from 300 mg of (*S*)-2-(2,2,4-trimethyl-1,3-dioxolan-4-yl)thiazole from the Step above to yield the title compound as a white solid (130 mg, 33%). MS(ESI): 263.0 (M+1).

**Step 4: *tert*-butyl ((S)-(2-((S)-2,2,4-trimethyl-1,3-dioxolan-4-yl)thiazol-5-yl)sulfinyl)-carbamate (D-4S)**

This step was performed using the procedure described in Scheme 103, Step 4 from 100 mg of (S)-2-((S)-2,2,4-trimethyl-1,3-dioxolan-4-yl)thiazole-5-sulfinamide (D-3S) to yield the title compound as a colourless viscous oil (110 mg, 80%).

**Step 5: *tert*-butyl ((S)-amino(oxo)(2-((S)-2,2,4-trimethyl-1,3-dioxolan-4-yl)thiazol-5-yl)-)λ$^6$-sulfaneylidene)carbamate (D-5S)**

This step was performed using the procedure described in Scheme 103, Step 5 from 110 mg of tert-butyl ((S)-(2-((S)-2,2,4-trimethyl-1,3-dioxolan-4-yl)thiazol-5-yl)sulfinyl)-carbamate (**D-4S**) to yield the title compound as an off-white solid (63 mg, 60%). MS(ESI): 378.1 (M+1).

**Step 6: *tert*-butyl ((R)-(3-(1,2,3,5,6,7-hexahydro-s-indacen-4-yl)ureido)(oxo)(2-((S)-2,2,4-trimethyl-1,3-dioxolan-4-yl)thiazol-5-yl)-λ$^6$-sulfaneylidene)carbamate (D-6S)**

In a 10 mL round bottom flask was charged *tert*-butyl ((S)-amino(oxo)(2-((S)-2,2,4-trimethyl-1,3-dioxolan-4-yl)thiazol-5-yl)-λ$^6$-sulfaneylidene)carbamate (**D-5S**, 25 mg, 0.06 mmol) followed by THF (2.0 mL) and cooled to 0 $^0$C with stirring. Charged 1 M potassium tert-butoxide solution in THF (0.070 mL) and reaction mixture was stirred for 30 min at 0 $^0$C. The solution of 4-isocyanato-1,2,3,5,6,7-hexahydro-s-indacene (13.2 mg in 0.5 mL THF) was added at 0$^0$C and then stirred for approx. 1 hours at RT. The reaction mixture was quenched with 1 M formic acid in THF (0.070 mL) at 0$^0$C and then extracted with ethyl acetate (25 mL) and filtered through Celite. The filtrate was concentrated under reduced pressure to afford an off-white solid. The residue was purified by column chromatography using a gradient of 100% ethyl acetate to 1% methanol in ethyl acetate. The title compound (25 mg, 66%) was isolated as an off-white solid and used in the next step as is.

**Step 7: (*R*)-2-((*S*)-1,2-dihydroxypropan-2-yl)-*N'*-((1,2,3,5,6,7-hexahydro-*s*-indacen-4-yl)carbamoyl)- thiazole-5-sulfonimidamide (Example 713)**

In a 10 mL round-bottom flask was charged tert-butyl ((R)-(3-(1,2,3,5,6,7-hexahydro-s-indacen-4-yl)ureido)(oxo)(2-((S)-2,2,4-trimethyl-1,3-dioxolan-4-yl)thiazol-5-yl)-λ$^6$-sulfaneylidene)carbamate (**D-6S**, 25 mg, 0.043 mmol) followed by anhydrous DCM (3 mL) and the reaction suspension was cooled to 0°-5°C under an argon atmosphere. TFA was slowly added (0.05 mL) and stirred for 1 hour at 0 °C. Added more TFA (0.3 mL) and reaction was stirred for additional 3 hours. Added slowly another 0.45 mL of TFA in to the reaction mixture and stirred for additional 2.5 hours at 0 °C. The reaction solution was concentrated under reduced pressure and repeatedly co-evaporated with acetonitrile (3 × 5 mL) and concentrated.

The residue was dissolved DMSO and purified by C18 RP-HPLC by eluting with 100% water (0.1% TFA) to 95% acetonitrile in water. After lyophilization, the title compound was isolated as a white solid (12 mg, 58%). $^1$H NMR (DMSO-$d_6$, 400 MHz): δ 8.41 (br s, 1H), 8.04 (s, 1H), 7.82 (s, 2H), 6.86 (s, 1H), 6.12 (s, 1H), 5.00 (t, *J* = 6.0 Hz, 1H), 3.53 (d, *J*= 6.1 Hz, 2H), 2.76 (t, *J* = 7.4 Hz, 4H), 2.66 (s, 4H), 1.92 (p, *J*= 7.4 Hz, 4H), 1.43 (s, 3H). MS(ESI): 437.1 (M+1).

**Example 714 (Compound 874a)**

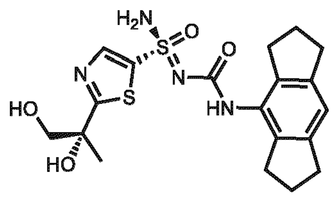

(*R*)-2-((*R*)-1,2-dihydroxypropan-2-yl)-*N'*-((1,2,3,5,6,7-hexahydro-*s*-indacen-4-yl)carbamoyl)thiazole-5-sulfonimidamide

This compound was prepared as illustrated in Example 713 Steps 1-7 with replacement of reagents AD-mix-beta and $(DHQD)_2AQN$ in Step 1 with AD-mix-alpha and $(DHQ)_2AQN$. $^1$H NMR (400 MHz, DMSO): δ 8.41 (br s, 1H), 8.05 (s, 1H), 7.82 (br s, 2H), 6.86 (s, 1H), 6.12 (s, 1H), 4.99 (t, *J* = 6.08 Hz, 1 H), 3.53 (d, *J* = 6.07 Hz; 2H), 2.76 (t; J =

7.39 Hz, 4H), 2.55-2.70 (m, 4H), 1.92 (p, J = 7.35 Hz, 4H), 1.43 (s, 3H). MS(ESI): 437.1 (M+1).

**Example 715**

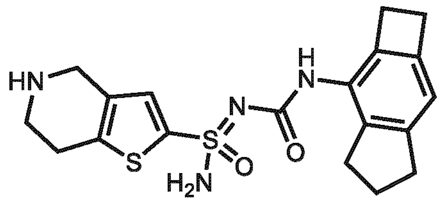

N'-((2,4,5,6-tetrahydro-1H-cyclobuta[f]inden-3-yl)carbamoyl)-4,5,6,7-tetrahydrothieno[3,2-clpyridine-2-sulfonimidamide (Scheme XVIII)

**Examples 716 and Example 717**

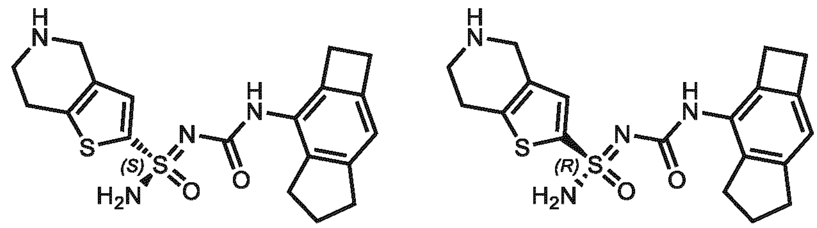

**Example 716** **Example 717**
**(stereochemistry tentatively assigned)**

(S)- and (R)-N'-((2,4,5,6-tetrahydro-1H-cyclobutalf|inden-3-yl)carbamoyl)-4,5,6,7-tetrahydrothieno[3,2-c]pyridine-2-sulfonimidamide

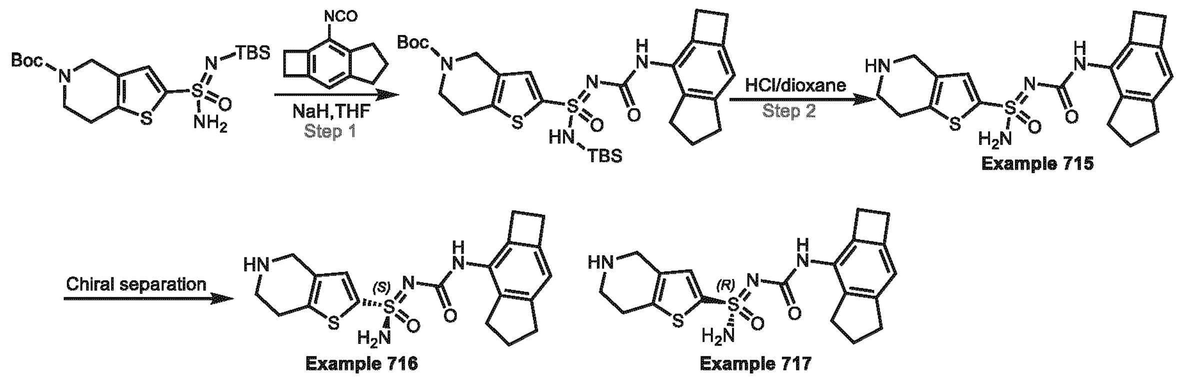


**Step 1: Tert-butyl 2-(N-(tert-butyldimethylsilyl)-N'-((2,4,5,6-tetrahydro-1H-cyclobuta[f]inden-3-yl) carbamoyl)sulfamidimidoyl)-6,7-dihydrothieno[3,2-c]pyridine-5(4H)-carboxylate**

To a stirred solution of tert-butyl 2-(N'-(tert-butyldimethylsilyl)sulfamidimidoyl)-6,7-dihydrothieno[3,2-c] pyridine-5(4H)-carboxylate (200 mg, 0.46 mmol) in THF (6.0 mL) in a 50-mL round-bottom flask under nitrogen was added NaH (60% wt. dispersion in mineral oil, 37 mg, 0.93 mmol) in portions at 0 °C. The reaction mixture was stirred for 10 min at RT. Then to this was added a mixture of 3-isocyanato-2,4,5,6-tetrahydro-1H-cyclobuta[f]indene (86 mg, 0.46 mmol) in THF (2.0 mL) dropwise with stirring at 0 °C. The reaction mixture was stirred for 2 h at RT and then quenched with 1.0

mL of water. The resulting mixture was extracted with 3x25 mL of EtOAc and the organic layers were combined. The organic layer was dried over anhydrous $Na_2SO_4$ and concentrated under vacuum. The crude product was eluted from silica gel with DCM/MeOH (10:1). This resulted in 130 mg (46%) of the title compound as a light yellow solid. MS-ESI: 617 (M+1).

**Step 2: N'-((2,4,5,6-tetrahydro-1H-cyclobuta[f]inden-3-yl)carbamoyl)-4,5,6,7-tetrahydrothieno[3,2-c] pyridine-2-sulfonimidamide**

A solution of tert-butyl 2-(N-(tert-butyldimethylsilyl)-N'-((2,4,5,6-tetrahydro-1H-cyclobuta[f]inden-3-yl) carbamoyl)sulfamidimidoyl)-6,7-dihydrothieno[3,2-c]pyridine-5(4H)-carboxylate (130 mg, 0.21 mmol) in HCl in dioxane (4 M, 8.0 mL) in a 25-mL round-bottom flask was stirred for 30 min at RT. The reaction mixture was concentrated under vacuum. The crude product was purified by Prep-HPLC using the following conditions: XBridge Prep C18 OBD Column, 19×150 mm 5 um; Mobile Phase A: water (10 mM $NH_4HCO_3$ + 0.1%$NH_3 \cdot H_2O$), Mobile Phase B: ACN; Flow rate: 25 mL/min; Gradient: 19% B to 38% B over 7 min; UV 210/254 nm; Rti: 6.92 min. This resulted in 50 mg (59%) of **Example 715** as a white solid. MS-ESI: 403 (M+1). $^1H$ NMR (400 MHz, DMSO-$d_6$) δ 8.27 (s, 1H), 7.31 (s, 1H), 6.66 (s, 1H), 3.72 (s, 2H), 3.10-2.99 (m, 2H), 2.99-2.91 (m, 2H), 2.91-2.79 (m, 2H), 2.85-2.66 (m, 6H), 1.96-1.86 (m, 2H).

**Step 3: Chiral separation**

**Example 715** (40 mg) was resolved by Prep-Chiral-HPLC with the following conditions: CHIRALPAK IG, 2*25 cm, 5 um; Mobile Phase A: $CO_2$, Mobile Phase B: MeOH:ACN=1:1 (2 mM $NH_3$-MeOH); Flow rate: 45 mL/min; Gradient: 50% B; UV 220 nm; Rti: 8.28 min.

(**Example 716**); $Rt_2$: 11.31 min. **(Example 717).** This resulted in 12.8 mg (99.9%ee) of **Example 716** followed by 14.8 mg (97.5%ee) of **Example 717,** both as a white solid.

**Example 716:** MS-ESI: 403 (M+1). $^1H$ NMR (400 MHz, DMSO-$d_6$) δ 8.19 (s, 1H), 7.26 (s, 1H), 6.64 (s, 1H), 3.70 (s, 2H), 3.10-3.00 (m, 2H), 3.00-2.90 (m, 2H), 2.90-2.81 (m, 2H), 2.82-2.65 (m, 6H), 1.95-1.83 (m, 2H).

**Example 717:** MS-ESI: 403 (M+1). $^1H$ NMR (400 MHz, DMSO-$d_6$) δ 8.17 (s, 1H), 7.24 (s, 1H), 6.63 (s, 1H), 3.69 (s, 2H), 3.13-3.00 (m, 2H), 3.00-2.90 (m, 2H), 2.90-2.81 (m, 2H), 2.82-2.65 (m, 6H), 1.95-1.84 (m, 2H).

**Table 60** Examples in the following table were prepared using similar conditions as described in Example **715** and Scheme **XVIII** from appropriate starting materials.

| Example # | Structure | IUPAC Name | Exact Mass [M+H]+ |
|---|---|---|---|
| 718 | 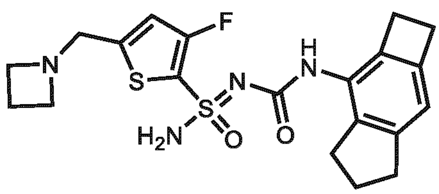 | 5-(Azetidin-1-ylmethyl)-3-fluoro-N'-((2,4,5,6-tetrahydro-1H-cyclobuta[f] inden-3-yl)carbamoyl)thiophene-2-sulfonimidamide | 435 |
| 719 | 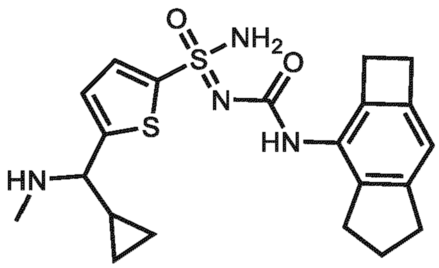 | 5-(Cyclopropyl(methylamino) methyl)-N'-((2,4,5,6-tetrahydro-1H-cyclobuta[f]inden-3-yl)carbamoyl) thiophene-2-sulfonimidamide | 431 |

(continued)

| Example # | Structure | IUPAC Name | Exact Mass [M+H]+ |
|---|---|---|---|
| 720 | 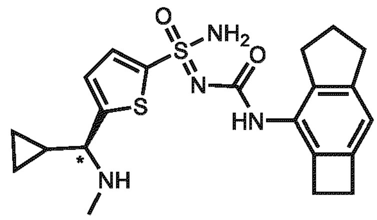 | 5-((S) or (R)-cyclopropyl(methylamino) methyl)-N'-((2,4,5,6-tetrahydro-1H-cyclobuta[f]inden-3- yl)carbamoyl) thiophene-2-sulfonimidamide | 431 |
| 721 | 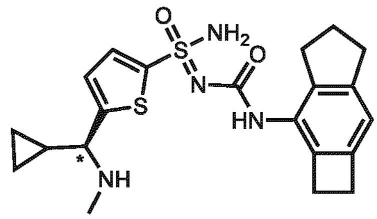 | 5-((R) or (S)-cyclopropyl(methylamino) methyl)-N'-((2,4,5,6-tetrahydro-1H-cyclobuta[f]inden-3- yl)carbamoyl) thiophene-2-sulfonimidamide | 431 |
| 722 | 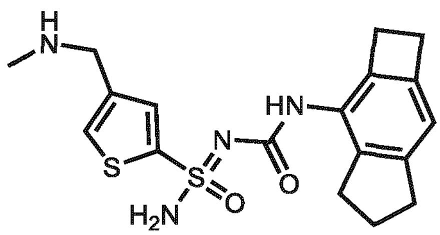 | 4-((Methylamino)methyl)-N'-((2,4,5,6-tetrahydro-1H-cyclobuta[f]inden-3-yl) carbamoyl)thiophene-2-sulfonimidamide | 391 |
| 723 | 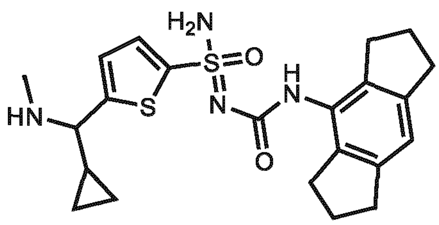 | 5-(cyclopropyl(methylamino) methyl)-N'-((1,2,3,5,6,7-hexahydro-*s*-indacen-4-yl)carbamoyl)thiophene-2-sulfonimidamide | 445 |
| 724 | 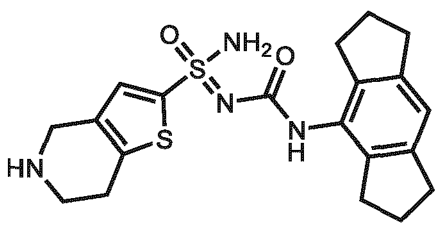 | N'-((1,2,3,5,6,7-hexahydro-*s*-indacen-4-yl)carbamoyl)-4,5,6,7-tetrahydrothieno [3,2-c]pyridine-2-sulfonimidamide | 417 |
| 725 | 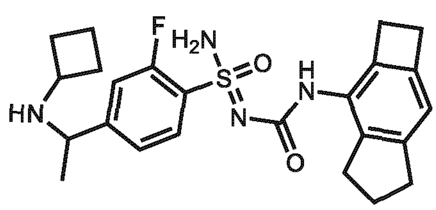 | 4-(1-(cyclobutylamino)ethyl)-2-fluoro-N'-((2,4,5,6-tetrahydro-1H-cyclobuta[f] inden-3-yl)carbamoyl) benzenesulfonimidamide | 457 |

(continued)

| Example # | Structure | IUPAC Name | Exact Mass $[M+H]^+$ |
|---|---|---|---|
| 726 | 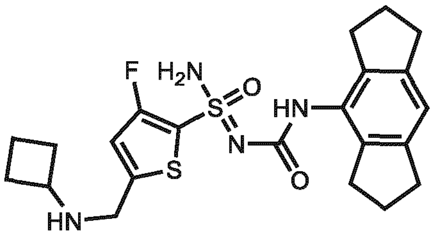 | 5-((Cyclobutylamino)methyl)-3-fluoro-N'-((1,2,3,5,6,7-hexahydro-*s*-indacen-4-yl)carbamoyl)thiophene-2-sulfonimidamide | 463 |
| 727 | 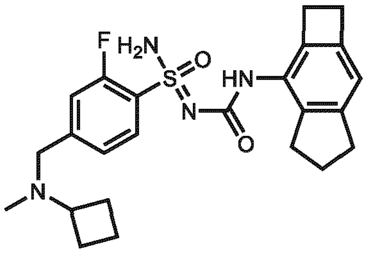 | 4-((cyclobutyl(methyl)amino)methyl)-2-fluoro-N'-((2,4,5,6-tetrahydro-1H-cyclobuta[f]inden-3- yl)carbamoyl) benzenesulfonimidamide | 457 |
| 728 | 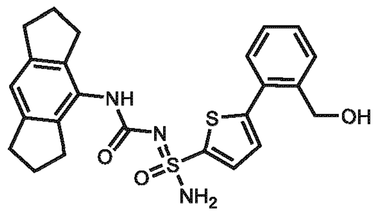 | N'-((1,2,3,5,6,7-hexahydro-*s*-indacen-4-yl)carbamoyl)-5-(2-(hydroxymethyl) phenyl)thiophene-2-sulfonimidamide | 468 |
| 729 | 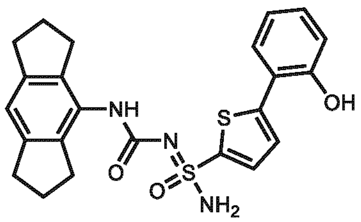 | N'-((1,2,3,5,6,7-hexahydro-*s*-indacen-4-yl)carbamoyl)-5-(2-hydroxyphenyl) thiophene-2-sulfonimidamide | 454 |
| 730 | 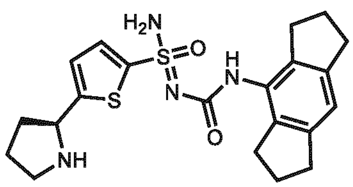 | N'-((1,2,3,5,6,7-hexahydro-*s*-indacen-4-yl)carbamoyl)-5-((S)-pyrrolidin-2-yl) thiophene-2-sulfonimidamide (made from chiral Intermediate 195F) | 431 |
| 731 | 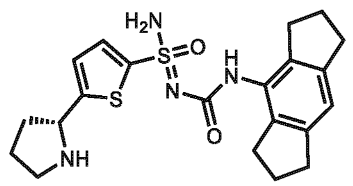 | N'-((1,2,3,5,6,7-hexahydro-*s*-indacen-4-yl)carbamoyl)-5-((R)-pyrrolidin-2-yl) thiophene-2-sulfonimidamide (made from chiral Intermediate 1955) | 431 |

(continued)

| Example # | Structure | IUPAC Name | Exact Mass [M+H]+ |
|---|---|---|---|
| 732 | 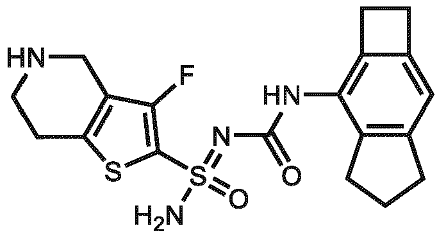 | 3-Fluoro-N'-((2,4,5,6-tetrahydro-1H-cyclobuta[f]inden-3-yl)carbamoyl)-4,5,6,7-tetrahydrothieno[3,2-c]pyridine-2-sulfonimidamide | 421 |

**Example 733**

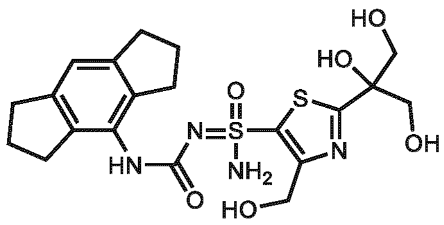

N'-((1,2,3,5,6,7-hexahydro-*s*-indacen-4-yl)carbamoyl)-4-(hydroxymethyl)-2-(1,2,3 - trihydroxypropan-2-yl)thiazole-5-sulfonimidamide (Scheme XIX)

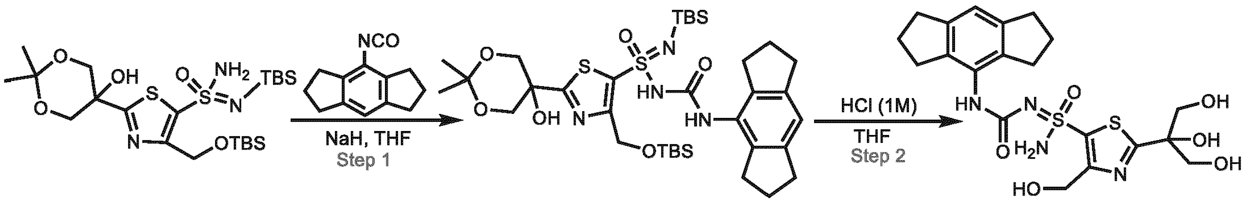


**Step 1: N'-(tert-butyldimethylsilyl)-4-(((tert-butyldimethylsilyl)oxy)methyl)-N-((1,2,3,5,6,7-hexahydro -*s*-indacen-4-yl)carbamoyl)-2-(5-hydroxy-2,2-dimethyl-1,3-dioxan-5-yl)thiazole-5-sulfonimidamide**

To a stirred solution of N'-(tert-butyldimethylsilyl)-4-(((tert-butyldimethylsilyl)oxy)methyl)-2-(5-hydroxy -2,2-dimethyl-1,3-dioxan-5-yl)thiazole-5-sulfonimidamide (150 mg, 0.27 mmol) in THF (10 mL) in a 50-mL round-bottom flask under nitrogen was added NaH (60% wt. dispersion in mineral, 43.2 mg, 1.08 mmol) in portions at 0 °C. The resulting solution was stirred for 10 min at RT. Then to the above mixture was added 4-isocyanato-1,2,3,5,6,7-hexahydro-s-indacene (81 mg, 0.41 mmol) at RT. The resulting mixture was stirred for 4 h at RT. The reaction mixture was quenched with 2.0 mL of $H_2O$. The mixture was concentrated under vacuum. The residue was purified by Prep-TLC (PE/EtOAc 1:1). This resulted in 110 mg (54%) of the title compound as an off-white solid. MS-ESI: 751 (M+1).

**Step 2: N'-((1,2,3,5,6,7-hexahydro-*s*-indacen-4-yl)carbamoyl)-4-(hydroxymethyl)-2-(1,2,3-trihydroxypropan-2-yl)thiazole-5-sulfonimidamide**

To a stirred solution of N'-(tert-butyldimethylsilyl)-4-(((tert-butyldimethylsilyl)oxy)methyl)-N-((1,2,3,5,6,7- hexahydro-s-indacen-4-yl)carbamoyl)-2-(5-hydroxy-2,2-dimethyl-1,3-dioxan-5-yl)thiazole-5-sulfonimidamide (110 mg, 0.15

mmol) in THF (12 mL) in a 50-mL round-bottom flask was added HCl in dioxane (4 M, 4 mL) dropwise at RT. The reaction solution was stirred for 2 h at RT. The reaction solution was concentrated under vacuum. The crude product was purified by Prep-HPLC using the following conditions: XBridge Prep OBD C18 Column, 30×150 mm 5 um; Mobile Phase A: water (10 mM $NH_4HCO_3$ + 0.1%$NH_3 \cdot H_2O$), Mobile Phase B: ACN; Flow rate: 60 mL/min; Gradient: 5% B to 35% B over 7 min; UV 210/254 nm; Rti: 6.2 min. This resulted in 32 mg (46%) of **Example 733** as a white solid. MS-ESI: 483 (M+1). $^1H$ NMR (400 MHz, MeOH-$d_4$) δ 6.92 (s, 1H), 5.01-4.90 (m, 2H), 3.92-3.81 (m, 4H), 2.91-2.70 (m, 8H), 2.09-1.98 (m, 4H).

**Table 61** Examples in the following table were prepared using similar conditions as described in Example **733** and Scheme **XIX** from appropriate starting materials.

| Example # | Structure | IUPAC Name | Exact Mass [M+H]+ |
|---|---|---|---|
| 734 | 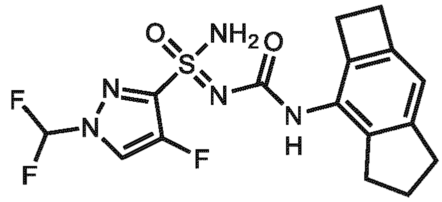 | 1-(Difluoromethyl)-4-fluoro-N'-((2,4,5,6-tetrahydro-1H-cyclobuta[f]inden-3-yl) carbamoyl)-1H-pyrazole-3-sulfonimidamide | 400 |

**Example 735**

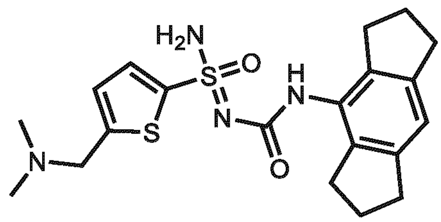

5-((Dimethylamino)methyl)-N'-((1,2,3,5,6,7-hexahydro-*s*-indacen-4-yl)carbamoyl)thioiphene-2-sulfonimidamide (Scheme XX)

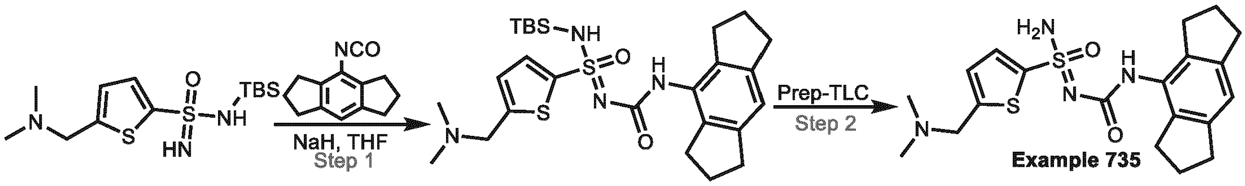


**Step 1: N-(tert-butyldimethylsilyl)-5-((dimethylamino)methyl)-N'-((1,2,3,5,6,7-hexahydro-*s*-indacen-4- yl)carbamoyl)thiophene-2-sulfonimidamide**

To a stirred solution of N-(tert-butyldimethylsilyl)-5-((dimethylamino)methyl)thiophene-2-sulfonimidamide (800 mg, 2.4 mmol) in THF (50 mL) in a 100-mL round-bottom flask under nitrogen was added NaH (60% wt. dispersion in mineral oil, 192 mg, 4.8 mmol) in portions at 0 °C. The reaction mixture was stirred for 10 min at RT. Then to the reaction mixture was added 4-isocyanato-1,2,3,5,6,7-hexahydro-s-indacene (382 mg, 1.92 mmol) in portions at 0 °C. The reaction mixture was stirred for 2 h at RT and then quenched with 5.0 mL of water. The mixture was extracted with 3x150 mL of EtOAc and the organic layers were combined. The organic layer was dried over anhydrous $Na_2SO_4$ and concentrated under vacuum. This resulted in 950 mg (crude) of the title compound as yellow oil which was used in the next step without further purification. MS-ESI: 533 (M+1).

**Step 2: 5-((Dimethylamino)methyl)-N'-((1,2,3,5,6,7-hexahydro-*s*-indacen-4-yl)carbamoyl)thiophene -2-sulfonimidamide**

The crude product of N-(tert-butyldimethylsilyl)-5-((dimethylamino)methyl)-N'-((1,2,3,5,6,7-hexahydro-s- indacen-4-yl)carbamoyl)thiophene-2-sulfonimidamide (950 mg, crude) was applied on Prep-TLC and using DCM/MeOH (10:1) to keep Rf= 0.4~0.5, then left the product on TLC overnight at RT. The TBS group was removed on TLC to give the final product which was then eluted from TLC with DCM/MeOH (10:1). The final product was further purified by Prep-HPLC using the following conditions: XSelect CSH Prep C18 OBD Column, 5 um, 19* 150 mm; Mobile Phase A: water (10 mM $NH_4HCO_3$ + 0.1%$NH_3 \cdot H_2O$), Mobile Phase B: ACN; Flow rate: 25 mL/min; Gradient: 38 B to 50 B over 7 min; UV 210/254 nm; Rt: 5.53 min. This resulted in 280 mg (28 %, over two steps) of **Example 735** as a white solid. MS-ESI: 419 (M+1). $^1$H NMR (400 MHz, DMSO-$d_6$) δ 8.41 (br s, 1H), 7.82 (br s, 2H), 7.63 (d, *J* = 3.6 Hz, 1H), 7.49 (d, *J* = 4.0 Hz, 1H), 6.88 (s, 1H), 4.73 (s, 2H), 2.89-2.72 (m, 4H), 2.82 (s, 6H), 2.71-2.61 (m, 4H), 1.98-1.87 (m, 4H).

**Table 62** Examples in the following table were prepared using similar conditions as described in Example **735** and Scheme **XX** from appropriate starting materials.

| Example # | Structure | IUPAC Name | Exact Mass $[M+H]^+$ |
|---|---|---|---|
| 736 | 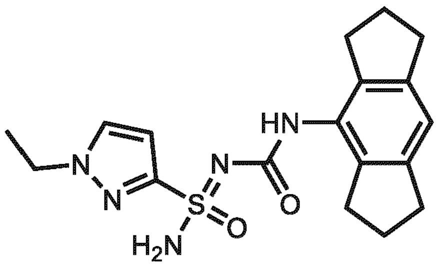 | 1-Ethyl-N'-((1,2,3,5,6,7-hexahydro-*s*-indacen-4-yl)carbamoyl)-1H-pyrazole-3-sulfonimidamide | 374 |
| 737 | 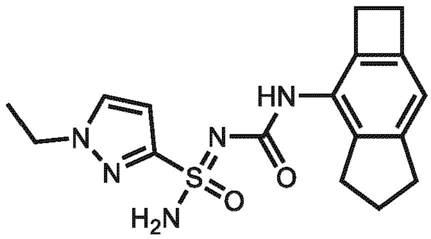 | 1-Ethyl-N'-((2,4,5,6-tetrahydro-1H-cyclobuta[f]inden-3-yl)carbamoyl)-1H-pyrazole-3 -sulfonimidamide | 360 |

**Example 738**

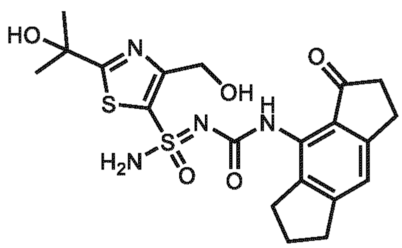

4-(Hydroxymethyl)-2-(2-hydroxypropan-2-yl)-N'-((3-oxo-1,2,3,5,6,7-hexahydro-*s*-indacen-4-yl)carbamoyl)thiazole-5-sulfonimidamide (Scheme XXI)

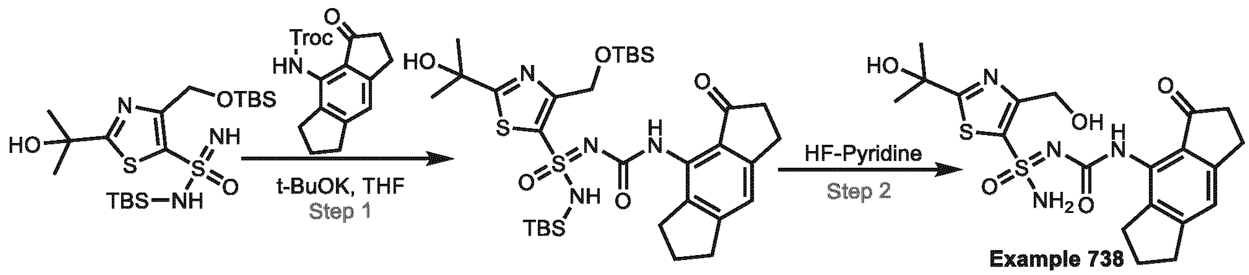


**Step 1: N-(tert-butyldimethylsilyl)-4-(((tert-butyldimethylsilyl)oxy)methyl)-2-(2-hydroxypropan-2-yl)-N'-((3-oxo-1,2,3,5,6,7-hexahydro-*s*-indacen-4-yl)carbamoyl)thiazole-5-sulfonimidamide**

To a stirred solution of N-(tert-butyldimethylsilyl)-4-(((tert-butyldimethylsilyl)oxy)methyl)-2-(2-hydroxypropan-2-yl)thiazole-5-sulfonimidamide (200 mg, 0.42 mmol) in THF (20 mL) in a 100-mL round-bottom flask under nitrogen was added t-BuOK (94 mg, 0.83 mmol) in portions at 0 °C. The reaction mixture was stirred for 10 min at RT. Then to the above mixture was added 2,2,2-trichloroethyl (3-oxo-1,2,3,5,6,7-hexahydro-s-indacen-4-yl)carbamate (174 mg, 0.50 mmol) at RT. The reaction mixture was stirred for 4 h at RT. The reaction mixture was diluted with 20 mL of $H_2O$. The mixture was extracted with 3x30 mL of EtOAc and the organic layers were combined. The organic layer was dried over anhydrous $Na_2SO_4$ and concentrated under vacuum. The crude product was purified by Prep-TLC with EtOAc/PE (1/1). This resulted in 239 mg (82%) of the title compound as a light yellow solid. MS-ESI: 693 (M+1).

**Step 2: 4-(Hydroxymethyl)-2-(2-hydroxypropan-2-yl)-N'-((3-oxo-1,2,3,5,6,7-hexahydro-*s*-indacen-4-yl) carbamoyl)thiazole-5-sulfonimidamide**

To a stirred solution of N-(tert-butyldimethylsilyl)-4-(((tert-butyldimethylsilyl)oxy)methyl)-2-(2-hydroxypropan-2-yl)-N'-((3-oxo-1,2,3,5,6,7-hexahydro-s-indacen-4-yl)carbamoyl)thiazole-5-sulfonimidamide (20 mg, 0.029 mmol) in THF (5.0 mL) in a 25-mL round-bottom flask was added HF/Py (70% wt., 0.10 mL, 3.85 mmol). The reaction solution was stirred for 2 h at RT. The reaction solution was concentrated under vacuum. The crude product was purified by Prep-HPLC using the following conditions: XBridge Prep C18 OBD Column, 19*150 mm, 5 um; mobile phase, water (10 mL $NH_4HCO_3$ + 0.1%$NH_3 \cdot H_2O$) and ACN (5% Phase B up to 30% over 7 min). This resulted in 7.0 mg (52%) of **Example 738** as a white solid. MS-ESI: 465 (M+1). $^1H$ NMR (300 MHz, MeOH-$d_4$) $\delta$ 7.13 (s, 1H), 4.99-4.86 (m, 2H), 3.07-2.99 (m, 2H), 2.96-2.87 (m, 2H), 2.84-2.74 (m, 2H), 2.71-2.63 (m, 2H), 2.08-1.93 (m, 2H), 1.58 (s, 6H).

**Table 63** Examples in the following table were prepared using similar conditions as described in Example **738** and Scheme **XXI** from appropriate starting materials.

| Example # | Structure | IUPAC Name | Exact Mass [M+H]+ |
|---|---|---|---|
| 739 | 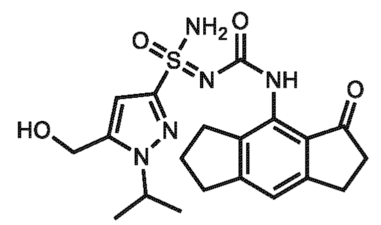 | 5-(Hydroxymethyl)-1 -isopropyl-N'-((3- oxo-1,2,3,5,6,7-hexahydro-*s*-indacen-4-yl) carbamoyl)-1H-pyrazole-3-sulfonimidamide | 432 |

**Example 740**

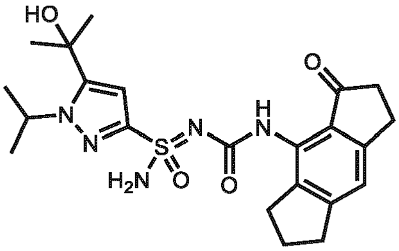

5-(2-Hydroxypropan-2-yl)-1-isopropyl-N'-((3-oxo-1,2,3,5,6,7-hexahydro-*s*-indacen-4-yl)carbamoyl)-1H-pyrazole-3-sulfonimidamide (Scheme XXII)

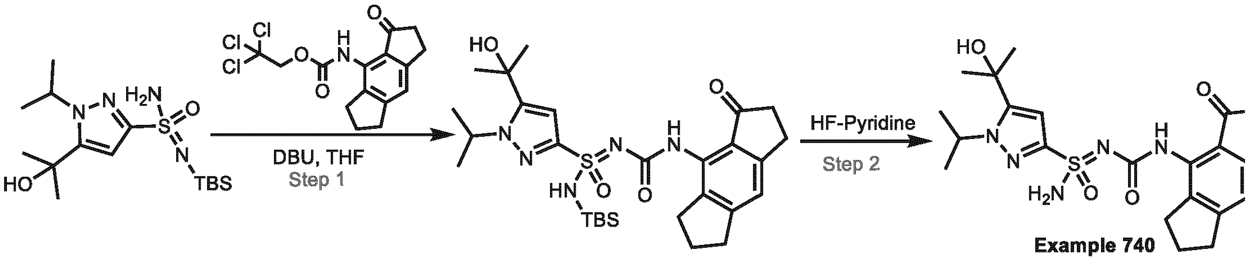


**Step 1: N-(tert-butyldimethylsilyl)-5-(2-hydroxypropan-2-yl)-1-isopropyl-N'-((3-oxo-1,2,3,5,6,7- hexahydro-*s*-indacen-4-yl)carbamoyl)-1H-pyrazole-3-sulfonimidamide**

To a stirred solution of N'-(tert-butyldimethylsilyl)-5-(2-hydroxypropan-2-yl)-1-isopropyl-1H-pyrazole-3- sulfonimidamide (250 mg, 0.69 mmol) in THF (8.0 mL) in a 25-mL round-bottom flask under nitrogen were added DBU (233 mg, 1.53 mmol) and 2,2,2-trichloroethyl (3-oxo-1,2,3,5,6,7-hexahydro-*s*-indacen-4-yl) carbamate (251 mg, 0.69 mmol) in portions at 0 °C in an ice/water bath. The reaction mixture was stirred overnight at RT. The reaction mixture was diluted with $H_2O$ (8 mL). The mixture was extracted with EtOAc (3 × 10 mL). The combined organic layers were washed with brine (3x15 mL) and dried over anhydrous $Na_2SO_4$. After filtration, the filtrate was concentrated under reduced pressure. The residue was eluted silica gel with PE/EtOAc (3:1). This resulted in 80 mg (20%) of the title compound as a yellow solid. MS-ESI: 574 (M+1).

**Step 2: 5-(2-Hydroxypropan-2-yl)-1-isopropyl-N'-((3-oxo-1,2,3,5,6,7-hexahydro-*s*-indacen-4-yl) carbamoyl)-1H-pyrazole-3-sulfonimidamide**

To a stirred solution of N-(tert-butyldimethylsilyl)-5-(2-hydroxypropan-2-yl)-1-isopropyl-N'-((3-oxo- 1,2,3,5,6,7-hexahydro-*s*-indacen-4-yl)carbamoyl)-1H-pyrazole-3-sulfonimidamide (80 mg, 0.14 mmol) in THF (5.0 mL) was added HF-Pyridine (70% wt., 0.03 mL, 1.12 mmol) at RT. The reaction mixture was stirred for 2 h at RT. The pH value of the mixture was adjusted to pH 7 with sat. $NaHCO_3$ (aq.). The mixture was extracted with EtOAc (3 × 10 mL). The combined organic layers were washed with brine (5.0 mL) and dried over anhydrous $Na_2SO_4$. After filtration, the filtrate was concentrated under reduced pressure. The crude product (50 mg) was purified by Prep-HPLC using the following conditions: XBridge Prep OBD C18 Column, 30×150 mm, 5 um; Mobile Phase A: water (10 mM $NH_4HCO_3$ + 0.1%$NH_3 \cdot H_2O$), Mobile Phase B: ACN; Flow rate: 60 mL/min; Gradient: 30% B to 45% B over 7 min; 254/210 nm; Rti: 6.43 min. This resulted in 25.6 mg (40%) of **Example 740** as a white solid. MS-ESI: 399 (M+1). $^1H$ NMR (400 MHz, MeOH-$d_4$) δ 7.12 (s, 1H), 6.60 (s, 1H), 5.36-5.28 (m, 1H), 3.05 (t, *J* = 5.6 Hz, 2H), 2.94 (t, *J* = 7.6 Hz, 2H), 2.90-2.80 (m, 2H), 2.77-2.66 (m, 2H), 2.08-2.02 (m, 2H), 1.62 (s, 6H), 1.50 (d, *J* = 6.8 Hz, 3H), 1.47 (d, *J* = 6.8 Hz, 3H).

**Example 741**

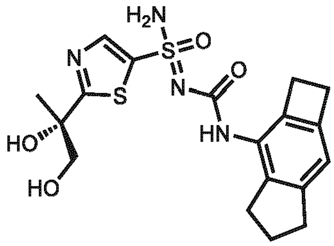

2-((S)-1,2-dihydroxypropan-2-yl)-N'-((2,4,5,6-tetrahydro-1H-cyclobuta[f]inden-3-yl)carbamoyl)thiazole-5-sulfonimidamide (Scheme XXIII)

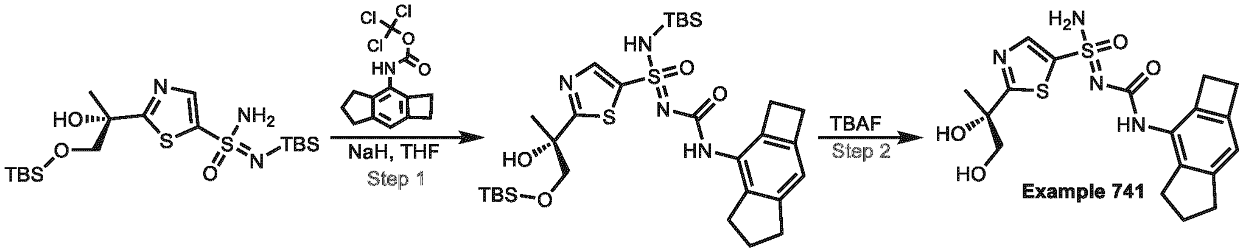


**Step 1: N-(tert-butyldimethylsilyl)-2-((S)-1-((tert-butyldimethylsilyl)oxy)-2-hydroxypropan-2-yl)-N'-((2,4,5,6-tetrahydro-1H-cyclobuta[f]inden-3-yl)carbamoyl)thiazole-5-sulfonimidamide**

To a stirred solution of N'-(tert-butyldimethylsilyl)-2-((S)-1-((tert-butyldimethylsilyl)oxy)-2-hydroxypropan-2-yl)thiazole-5-sulfonimidamide (500 mg, 1.07 mmol) in THF (10 mL) in a 50-mL round-bottom flask under nitrogen was added NaH (60% wt. dispersion in mineral oil, 87 mg, 2.15 mmol) in portions at 0 °C. The reaction mixture was stirred for 10 min at RT. To the above mixture was added trichloromethyl (2,4,5,6-tetrahydro-1H-cyclobuta[f]inden-3-yl)carbamate (359 mg, 1.07 mmol) in portions at 0 °C. The reaction mixture was stirred for 2 h at RT. The reaction was quenched with 10 mL of water. The mixture was extracted with 3x20 mL of EtOAc. The organic layers were dried over anhydrous $Na_2SO_4$ and concentrated under vacuum. The residue was eluted from silica gel with EtOAc/PE (2:1). This resulted in 300 mg (43%) of the title compound as light brown oil. MS-ESI: 651 (M+1)

**Step 2: 2-((S)-1,2-dihydroxypropan-2-yt)-N'-((2,4,5,6-tetrahydro-1H-cydobuta[f]inden-3-yl) carbamoyl)thiazole-5-sulfonimidamide**

To a stirred solution of N-(tert-butyldimethylsilyl)-2-((S)-1-((tert-butyldimethylsilyl)oxy)-2-hydroxypropan-2-yl)-N'-((2,4,5,6-tetrahydro-1H-cyclobuta[f]inden-3-yl)carbamoyl)thiazole-5-sulfonimidamide (300 mg, 0.46 mmol) in THF (10 mL) in a 50-mL round-bottom flask was added TBAF (480 mg, 1.84 mmol) at RT. The reaction mixture was stirred for 2 h at RT. The reaction mixture was concentrated under vacuum. The residue was eluted from silica gel with EtOAc (100%). The product was further purified by Flash-Prep-HPLC using the following conditions: XSelect CSH Prep $C_{18}$ OBD Column, 5 um, 19*150 mm; Mobile Phase A: water (10 mM $NH_4HCO_3$+0.1%$NH_3 \cdot H_2O$), Mobile Phase B: ACN; Flow rate: 25 mL/min; Gradient: 25% B to 42% B over 7 min; 254/210 nm; Rti: 5.32 min. This resulted in 130 mg (67%) of **Example 741** as an off-white solid. MS-ESI: 423 (M+1). $^1$H NMR (400 MHz, DMSO-$d_6$) δ 8.42 (br s, 1H), 8.11 (s, 1H), 7.90 (br s, 2H), 6.67 (s, 1H), 6.14 (s, 1H), 5.00 (t, *J* = 6.0 Hz, 1H), 3.55 (d, *J* = 6.0 Hz, 2H), 3.05-2.95 (m, 2H), 2.95-2.85 (m, 2H), 2.82-2.60 (m, 4H), 1.95-1.85 (m, 2H), 1.45 (s, 3H).

**Table 64** Examples in the following table were prepared using similar conditions as described in Example **741** and Scheme **XXIII** from appropriate starting materials.

| Example # | Structure | IUPAC Name | Exact Mass $[M+H]^+$ |
|---|---|---|---|
| 742 | 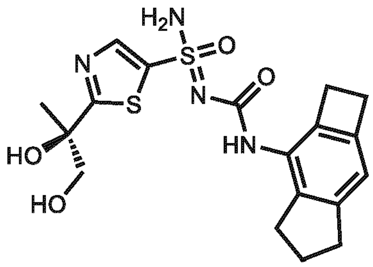 | 2-((R)-1,2-dihydroxypropan-2-yl)-N'-((2,4,5,6-tetrahydro-1H-cyclobuta[f] inden-3- yl)carbamoyl)thiazole-5-sulfonimidamide | 423 |

**Example 743**

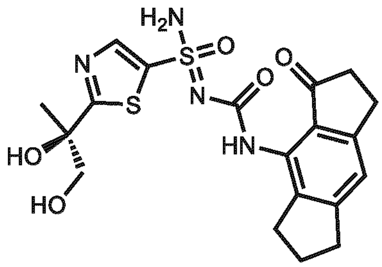

2-((R)-1,2-dihydroxypropan-2-yl)-N'-((3-oxo-1,2,3,5,6,7-hexahydro-*s*-indacen-4-yl)carbamoyl)thiazole-5-sulfonimidamide (Scheme XXIV)

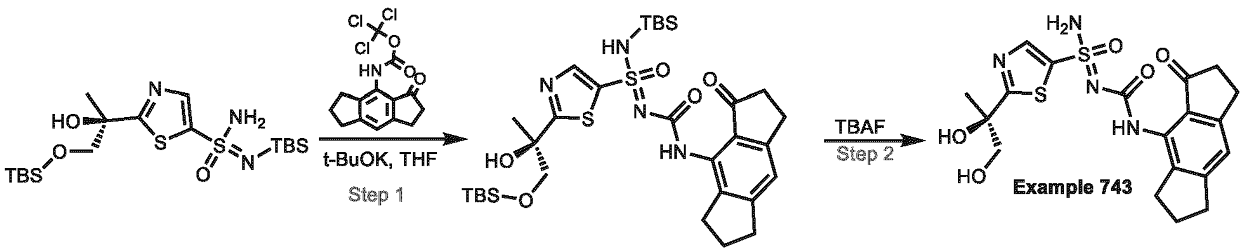


**Step 1: N-(tert-butyldimethylsilyl)-2-((R)-1-((tert-butyldimethylsilyl)oxy)-2-hydroxypropan-2-yl)-N'- ((3-oxo-1,2,3,5,6,7-hexahydro-s-indacen-4-yl)carbamoyl)thiazole-5-sulfonimidamide**

To a stirred solution of N'-(tert-butyldimethylsilyl)-2-((R)-1-((tert-butyldimethylsilyl)oxy)-2-hydroxypropan-2-yl)thiazole-5-sulfonimidamide (300 mg, 0.64 mmol) in THF (10 mL) in a 100-mL round-bottom flask under nitrogen was added t-BuOK (217 mg, 1.93 mmol) in portions at 0 °C. The reaction mixture was stirred for 10 min at RT. To the reaction mixture was added trichloromethyl (3-oxo-1,2,3,5,6,7-hexahydro-s-indacen-4-yl)carbamate (202 mg, 0.58 mmol) in portions at 0 °C. The reaction mixture was stirred for 16 h at RT. The reaction mixture was quenched with $H_2O$ (10 mL). The mixture was extracted with 5x20 mL of EtOAc and the organic layers were combined. The organic layer was dried with anhydrous $Na_2SO_4$ and concentrated under vacuum. This resulted in 400 mg (91%) of the title compound as a brown solid. MS-ESI: 679 (M+1).

**Step 2: 2-((R)-1,2-dihydroxypropan-2-yl)-N'-((3-oxo-1,2,3,5,6,7-hexahydro-*s*-indacen-4-yl)carbamoyl) thiazole-5-sulfonimidamide**

To a stirred solution of N-(tert-butyldimethylsilyl)-2-((R)-1-((tert-butyldimethylsilyl)oxy)-2-hydroxypropan -2-yl)-N'-((3-oxo-1,2,3,5,6,7-hexahydro-*s*-indacen-4-yl)carbamoyl)thiazole-5-sulfonimidamide (200 mg, 0.30 mmol) in THF (10 mL) in a 100-mL round-bottom flask was added TBAF (509 mg, 1.95 mmol) at RT. The reaction solution was stirred for 2 h at RT. The reaction mixture was diluted with $H_2O$ (10 mL). The mixture was extracted with 5x20 mL of EtOAc and the organic layers were combined. The organic layer was dried over anhydrous $Na_2SO_4$ and concentrated under vacuum. The residue was eluted from silica gel with DCM:MeOH (20:1). The product was further purified by Prep-HPLC using the following conditions: XBridge Prep C18 OBD Column, 19×150 mm, 5 um; Mobile Phase A: water (10 mM $NH_4HCO_3$+0.1%$NH_3 \cdot H_2O$), Mobile Phase B: ACN; Flow rate: 25 mL/min; Gradient: 5% B to 30% B over 7 min; UV 254/210 nm; Rti: 5.78 min. This resulted in 80 mg (60%) of **Example 743** as a white solid. MS-ESI: 451 (M+1). $^1$H NMR (400 MHz, DMSO-$d_6$) δ 9.01 (s, 1H), 8.07 (s, 1H), 7.65 (br s, 2H), 7.09 (s, 1H), 6.12 (s, 1H), 4.99 (br s, 1H), 3.54 (s, 2H), 2.96 (t, *J* = 5.2 Hz, 2H), 2.87 (t, *J* = 7.2 Hz, 2H), 2.83-2.61 (m, 4H), 1.99-1.93 (m, 2H), 1.44 (s, 3H).

**Table 65** Examples in the following table were prepared using similar conditions as described in Example **743** and Scheme **XXIV** from appropriate starting materials.

| Example # | Structure | IUPAC Name | Exact Mass [M+H]+ |
|---|---|---|---|
| 744 | 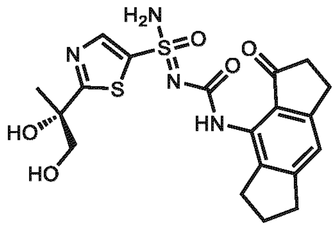 | 2-((S)-1,2-dihydroxypropan-2-yl)-N'-((3-oxo-1,2,3,5,6,7-hexahydro-*s*-indacen-4-yl) carbamoyl)thiazole-5-sulfonimidamide | 451 |

**Example 745**

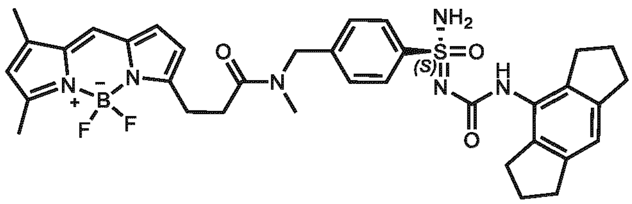

(S)-3-(5,5-difluoro-7,9-dimethyl-5*H*-5λ4,6λ4-dipyrrolo[1,2-c:2',1'-f][1,3,2]diazaborinin-3-yl)-N-(4-(N'-(1,2,3,5,6,7-hexahydro-*s*-indacen-4-yl)carbamoyl)sulfamidimidoyl)benzyl)-N-methylpropanamide (Scheme XXV)

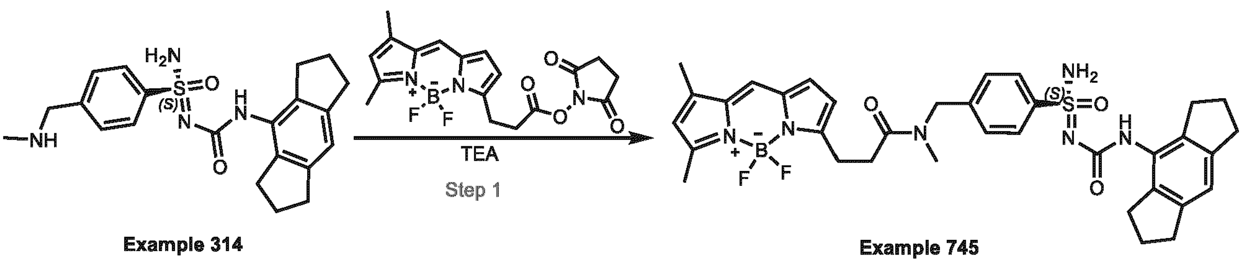


Example 314

Example 745

**Step 1: (S)-3-(5,5-difluoro-7,9-dimethyl-5*H*-5$\lambda_4$,6$\lambda_4$-dipyrrolo[1,2-c:2',1'-f][1,3,2]diazaborinin-3-yl)-N-(4-(N'-((1,2,3,5,6,7-hexahydro-*s*-indacen-4-yl)carbamoyl)sulfamidimidoyl)benzyl)-N-methylpropanamide**

To a stirred solution of (S)-N'-((1,2,3,5,6,7-hexahydro-s-indacen-4-yl)carbamoyl)-4-((methylamino)methyl) benzenesulfonimidamide (12 mg, 0.030 mmol) in DMF (0.50 mL) in a 25-mL round-bottom flask were added 2,5-dioxopyrrolidin-1-yl 3-(5,5-difluoro-7,9-dimethyl-5*H*-5$\lambda_4$,6$\lambda_4$-dipyrrolo[1,2-c:2',1'-f][1,3,2] diazaborinin-3-yl)propanoate (11 mg, 0.027 mmol) and TEA (6.09 mg, 0.060 mmol) at RT. The reaction solution was stirred for 25 h at RT. The solution was purified by Prep-HPLC using the following conditions: XBridge Prep OBD C18 Column, 19*250 mm, 5 um; mobile phase, water (10 mM $NH_4HCO_3$+0.1%$NH_3 \cdot H_2O$) and ACN (54% PhaseB up to 69% over 10 min); UV 254/210. This resulted in 5.8 mg (29%) of **Example 745** as a red solid. MS-ESI: 673 (M+1). $^1$H NMR (400 MHz, MeOH-*d*$_4$) $\delta$ 7.95 (d, *J* = 8.1 Hz, 2H), 7.47-7.35 (m, 3H), 7.01-6.88 (m, 2H), 6.35-6.20 (m, 2H), 4.79-4.61 (m, 2H), 3.29-3.20 (m, 2H), 3.00 (s, 3H), 2.94-2.60 (m, 10H), 2.53 (s, 3H), 2.28 (s, 3H), 2.05-1.94 (m, 4H).

**Example 746 and Example 747**

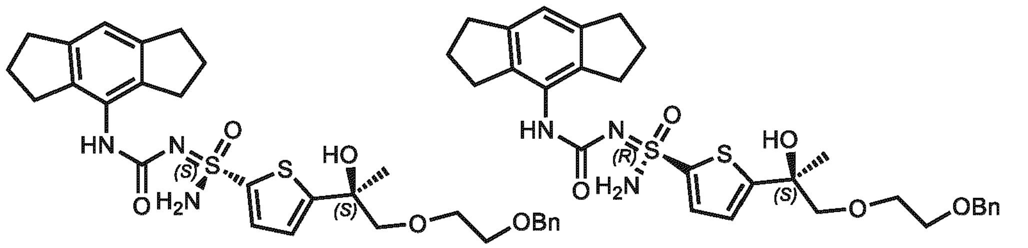

**Example 746** **Example 747**

**(stereochemistry tentatively assigned)**

(S) and (R)-5-((S)-1-(2-(benzyloxy)ethoxy)-2-hydroxypropan-2-yl)-N'-((1,2,3,5,6,7-hexahydro-*s*-indacen-4-yl)carbamoyl)thiophene-2-sulfonimidamide (Scheme 2)

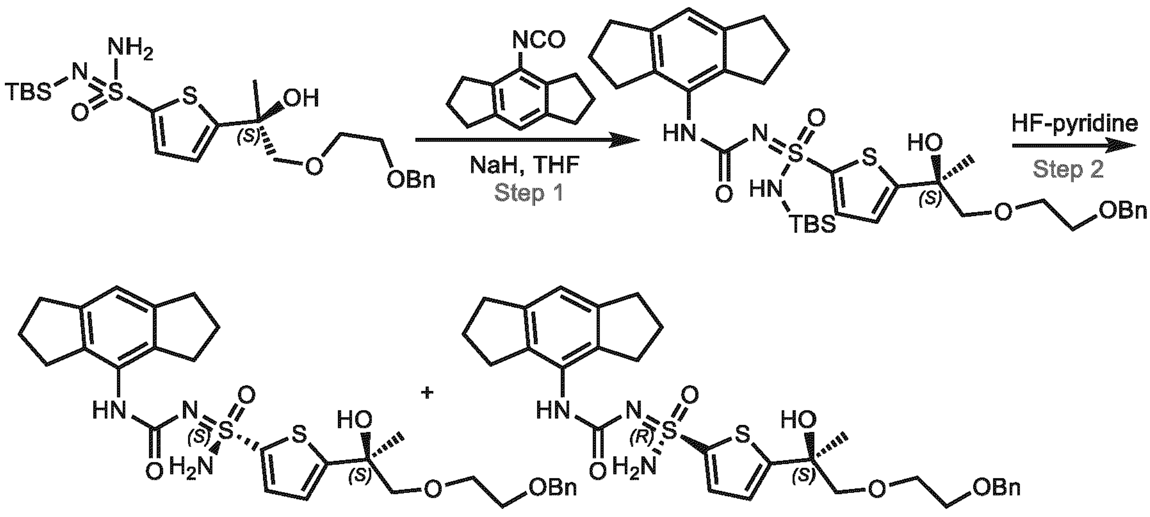


**Example 746** **Example 747**

**Step 1: 5-((S)-1-(2-(benzyloxy)ethoxy)-2-hydroxypropan-2-yl)-N-(tert-butyldimethylsilyl)-N'-((1,2,3,5,6,7-hexahydro-*s*-indacen-4-yl)carbamoyl)thiophene-2-sulfonimidamide**

To a stirred solution of 5-((S)-1-(2-(benzyloxy)ethoxy)-2-hydroxypropan-2-yl)-N'-(tertbutyldimethylsilyl) thiophene-2-sulfonimidamide (350 mg, 0.72 mmol) in THF (8 mL) in a 25-mL round-bottom flask under nitrogen was added

NaH (60% wt. dispersion in mineral oil, 52 mg, 2.17 mmol) in portions 0 °C. The reaction mixture was stirred for 10 min at RT. To above mixture was added 4-isocyanato-1,2,3,5,6,7-hexahydro-s-indacene (144 mg, 0.72 mmol) in portions at 0 °C. The reaction mixture was stirred for 3 h at RT. The reaction was quenched with 10 mL of water/ice at 0 °C. The mixture was extracted with EtOAc (3 × 30 mL). The combined organic layers were washed with brine (3x 15 mL) and dried over anhydrous $Na_2SO_4$. After filtration, the filtrate was concentrated under reduced pressure. This resulted in 469 mg (crude) of the title compound as a light yellow solid. MS-ESI: 684 (M+1).

**Step 2: (S) and (R)-5-((S)-1-(2-(benzyloxy)ethoxy)-2-hydroxypropan-2-yl)-N'-((1,2,3,5,6,7-hexahydro-*s*-indacen-4-yl)carbamoyl)thiophene-2-sulfonimidamide**

To a stirred solution of 5-((S)-1-(2-(benzyloxy)ethoxy)-2-hydroxypropan-2-yl)-N-(tert-butyldimethyl- silyl)-N'-((1,2,3,5,6,7-hexahydro-s-indacen-4-yl)carbamoyl)thiophene-2-sulfonimidamide (50 mg, crude) in THF (5.0 mL) in a 25-mL round-bottom flask was added HF/Py (70%wt., 0.1 mL, 3.7 mmol). The reaction mixture was stirred for 1 h at RT. The reaction mixture was concentrated under vacuum. The crude product was purified by Prep-HPLC using the following conditions: XSelect CSH Prep C18 OBD Column, 5 um, 19*150 mm; Mobile Phase A: water (10 mM $NH_4HCO_3$+0.1%$NH_3 \cdot H_2O$), Mobile Phase B: ACN; Flow rate: 25 mL/min; Gradient: 30% B to 50% B over 7 min; UV, 210/254 nm; Rti: 6.37 min. This resulted in 10 mg (25%) of **Example 746** and 10 mg (25%) of **Example 747,** both as a white solid. MS-ESI: 570 (M+1).

**Example 746** $^1$H NMR (400 MHz, MeOH-$d_4$) δ 7.77 (s, 1H), 7.65 (s, 1H), 7.37-7.25 (m, 5H), 6.92 (s, 1H), 4.54 (s, 2H), 3.67-3.49 (m, 6H), 2.91-2.79 (m, 8H), 2.10-1.98 (m, 4H), 1.52 (s, 3H). **Example 747** $^1$H NMR (400 MHz, MeOH-$d_4$) δ 7.56 (d, *J* = 3.6 Hz, 1H), 7.40-7.25 (m, 5H), 7.00 (d, *J* = 4.0 Hz, 1H), 6.92 (s, 1H), 4.55 (s, 2H), 3.72-3.58 (m, 6H), 2.90-2.71 (m, 8H), 2.10-1.95 (m, 4H), 1.60 (s, 3H).

**Example 748**

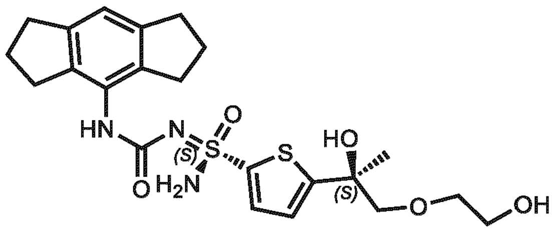

(S)-N'-((1,2,3,5,6,7-hexahydro-*s*-indacen-4-yl)carbamoyl)-5-((S)-2-hydroxy-1-(2-hvdroxvethoxv)propan-2-yl)thiophene-2-sulfonimidamide (Scheme XVII)

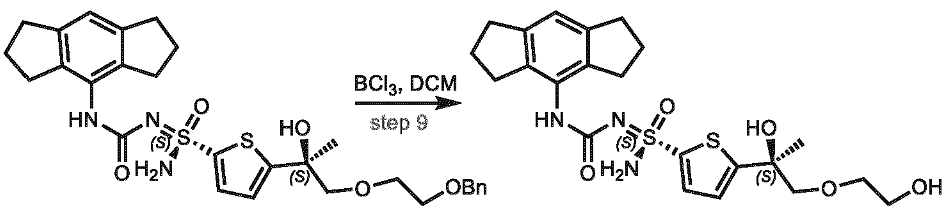


Example 746 Example 748

**Step 1: (S)-N'-((1,2,3,5,6,7-hexahydro-s-indacen-4-yl)carbamoyl)-5-((S)-2-hydroxy-1-(2-hydroxyethoxy)propan-2-yl)thiophene-2-sulfonimidamide**

To a stirred solution of (S)-5-((S)-1-(2-(benzyloxy)ethoxy)-2-hydroxypropan-2-yl)-N'-((1,2,3,5,6,7- hexahydro-s-indacen-4-yl)carbamoyl)thiophene-2-sulfonimidamide (20 mg, 0.035 mmol) in DCM (10 mL) was added $BCl_3$ in DCM (1 M, 0.1 mL, 0.1 mmol) dropwise at 0°C under nitrogen atmosphere. The reaction mixture was stirred for 5 h at 0 °C. The reaction was quenched with water/ice at 0°C. The mixture was extracted with DCM (3x10 mL). The combined organic

layers were washed with brine (3 × 10 mL) and dried over anhydrous $Na_2SO_4$. After filtration, the filtrate was concentrated under reduced pressure. The crude product (20 mg) was purified by Prep-HPLC using the following conditions: XSelect CSH Prep C18 OBD Column, 5 um, 19*150 mm; Mobile Phase A: water (10 mM, $NH_4HCO_3$+0.1%$NH_3 \cdot H_2O$), Mobile Phase B: ACN; Flow rate: 25 mL/min; Gradient: 32% B to 40% B over 10 min; UV 210/254 nm; Rti: 10.63 min. This resulted in 1.7 mg (10%) of **Example 748** as a white solid. MS-ESI: 480 (M+1). $^1$H NMR (300 MHz, MeOH-$d_4$) δ 7.87 (s, 1H), 7.78 (s, 1H), 6.94 (s, 1H), 3.69-3.50 (m, 6H), 2.91-2.70 (m, 8H), 2.11-1.96 (m, 4H), 1.54 (s, 3H).

**Table 66** Examples in the following table were prepared using similar conditions as described in Example **748** and Scheme **XVII** from Example 747 and Example 759.

| Example # | Structure | IUPAC Name | Exact Mass [M+H]+ |
|---|---|---|---|
| 749 | 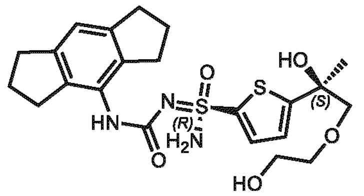 | (R)-N'-((1,2,3,5,6,7-hexahydro-*s*-indacen-4-yl)carbamoyl)-5-((S)-2-hydroxy-1-(2-hydroxyethoxy)propan-2-yl)thiophene-2-sulfonimidamide | 480 |
| 750 | 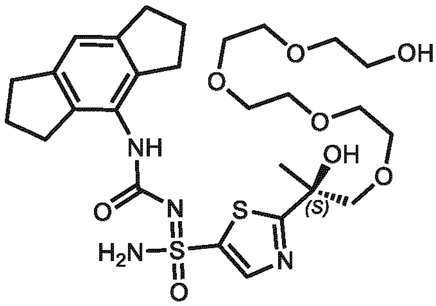 | 2-((S)-1, 14-dihydroxy-3,6,9, 12-tetraoxapentadecan-14-yl)-N'-((1,2,3,5,6,7-hexahydro-*s*-indacen-4-yl)carbamoyl)thiazole-5-sulfonimidamide | 613 |
| 751 | 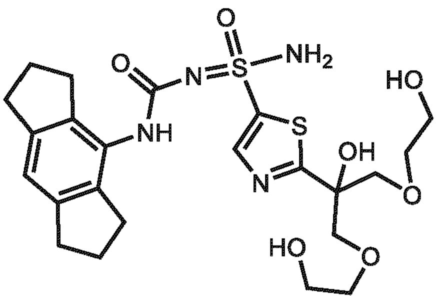 | N'-((1,2,3,5,6,7-hexahydro-s-indacen-4-yl)carbamoyl)-2-(2-hydroxy-1,3-bis(2-hydroxyethoxy)propan-2-yl)thiazole-5-sulfonimidamide | 541 |

**Example 752**

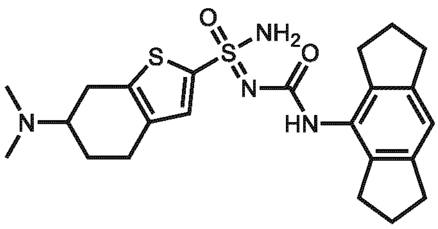

6-(Dimethylamino)-N'-((1,2,3,5,6,7-hexahydro-s-indacen-4-yl)carbamoyl)-4,5,6,7-tetrahydrobenzorblthiophene-2-sulfonimidamide (Scheme XXVI)

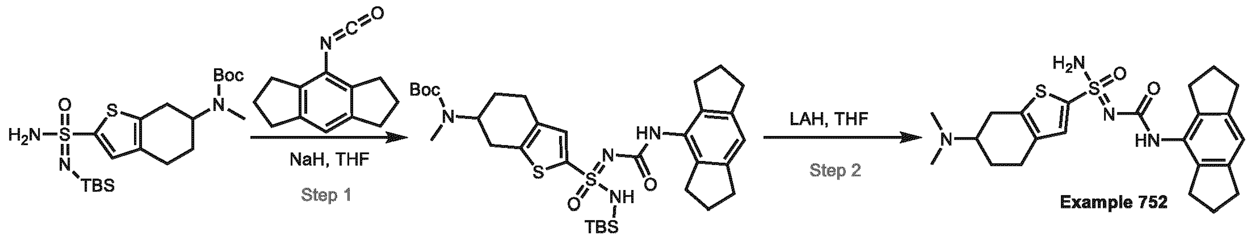


**Step 1: Tert-butyl (2-(N-(tert-butyldimethylsilyl)-N'-((1,2,3,5,6,7-hexahydro-s-indacen-4-yl)- carbamoyl)sulfamidimidoyl)-4,5,6,7-tetrahydrobenzo[b]thiophen-6-yl)(methyl)carbamate**

To a stirred solution of tert-butyl (2-(N'-(tert-butyldimethylsilyl)sulfamidimidoyl)-4,5,6,7-tetrahydro- benzo[b]thiophen-6-yl)(methyl)carbamate (30 mg, 0.065 mmol) in THF (5 mL) under nitrogen was added NaH (60%wt dispersion in mineral oil, 5.24 mg, 0.131 mmol) in portions at 0 °C. The resulting mixture was stirred for 10 min at 0 °C, then 4-isocyanato-1,2,3,5,6,7-hexahydro-s-indacene (20 mg, 0.098 mmol) was added in one portion at 0 °C. The resulting solution was stirred for 16 h at RT, then quenched with 10 mL of water. The resulting solution was extracted with 3x50 mL of EtOAc. The organic layers were combined, dried over anhydrous sodium sulfate, and concentrated under vacuum. This resulted in 40 mg (93.1%) of the title compound as a yellow solid. MS-ESI: 659 (M+1).

**Step 2: 6-(Dimethylamino)-N'-((1,2,3,5,6,7-hexahydro-s-indacen-4-yl)carbamoyl)-4,5,6,7-tetrahydro- benzo[b]thiophene-2-sulfonimidamide**

To a stirred solution of tert-butyl (2-(N-(tert-butyldimethylsilyl)-N'-((1,2,3,5,6,7-hexahydro-s-indacen- 4-yl)carbamoyl)sulfamidimidoyl)-4,5,6,7-tetrahydrobenzo[b]thiophen-6-yl)(methyl)carbamate (20 mg, 0.044 mmol) in THF (10 mL) under nitrogen was added LAH (8.4 mg, 0.22 mmol) in one portion at 0 °C. The resulting mixture was stirred for 16 h at RT. The reaction mixture was quenched with water (1 mL) and diluted with EtOAc (50 mL). The solids were filtered out and the filtrate was concentrated under vacuum. The residue was purified by Prep-HPLC using the following conditions: XBridge Shield RP18 OBD Column, 30*150 mm, 5 um; Mobile Phase A: water (10 mM $NH_4HCO_3$), Mobile Phase B: ACN; Flow rate: 60 mL/min; Gradient: 25% B to 55% B over 7 min; 254/220 nm. This resulted in 3.5 mg (25%) of **Example 752** as white solid. MS-ESI: 459 (M+1).

**Table 67** Examples in the following table were prepared using similar conditions as described in Example **1** and Scheme **1** from appropriate starting materials.

| Example # | Structure | IUPAC Name | Exact Mass $[M+H]^+$ |
|---|---|---|---|
| 753 | 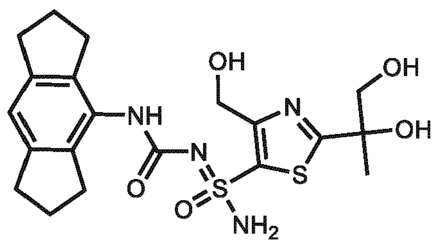 | 2-(1,2-Dihydroxypropan-2-yl)-N'-((1,2,3,5,6,7-hexahydro-*s*-indacen-4-yl)carbamoyl)-4-(hydroxymethyl)thiazole-5 -sulfonimidamide | 467 |
| 754 | 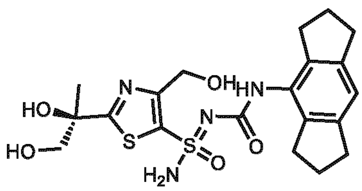 | 2-((R)-1,2-dihydroxypropan-2-yl)-N'-((1,2,3,5,6,7-hexahydro-*s*-indacen-4-yl)carbamoyl)-4-(hydroxymethyl)thiazole-5 -sulfonimidamide | 467 |

(continued)

| Example # | Structure | IUPAC Name | Exact Mass [M+H]+ |
|---|---|---|---|
| 755 | 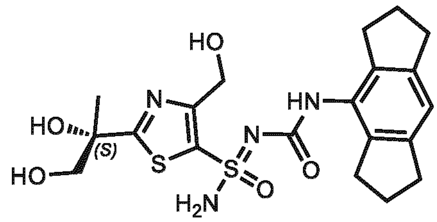 | 2-((S)-1,2-dihydroxypropan-2-yl)-N'-((1,2,3,5,6,7-hexahydro-*s*-indacen-4-yl)carbamoyl)-4-(hydroxymethyl)thiazole-5-sulfonimidamide | 467 |
| 756 | 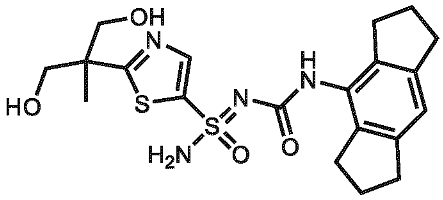 | 2-(1,3-Dihydroxy-2-methylpropan-2-yl)-N'-((1,2,3,5,6,7-hexahydro-*s*-indacen-4-yl)carbamoyl)thiazole-5-sulfonimidamide | 451 |
| 757 | 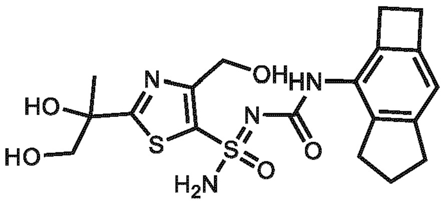 | 2-(1,2-Dihydroxypropan-2-yl)-4-(hydroxymethyl)-N'-((2,4,5,6-tetrahydro-1H-cyclobuta[f]inden-3-yl)carbamoyl) thiazole-5-sulfonimidamide | 453 |

**Table 68** Examples in the following table were prepared using similar conditions as described in Example **4** and Scheme **2** from appropriate starting materials.

| Example # | Structure | IUPAC Name | Exact Mass [M+H]+ |
|---|---|---|---|
| 758 | 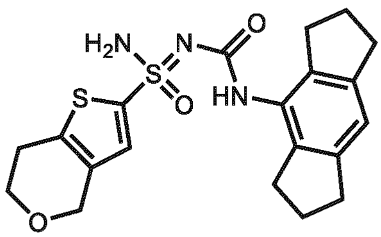 | N'-((1,2,3,5,6,7-hexahydro-*s*-indacen-4-yl) carbamoyl)-6,7-dihydro-4H-thieno [3,2-c] pyran-2-sulfonimidamide | 418 |
| 759 | 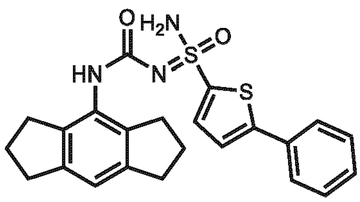 | N'-((1,2,3,5,6,7-hexahydro-*s*-indacen-4-yl) carbamoyl)-5-phenylthiophene-2-sulfonimidamide | 438 |

(continued)

| Example # | Structure | IUPAC Name | Exact Mass [M+H]+ |
|---|---|---|---|
| 760 | 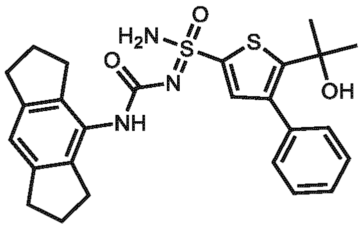 | N'-((1,2,3,5,6,7-hexahydro-*s*-indacen-4-yl) carbamoyl)-5-(2-hydroxypropan-2-yl)-4-phenylthiophene-2-sulfonimidamide | 496 |
| 761 | 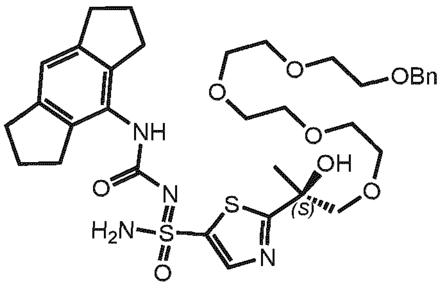 | N-((1,2,3,5,6,7-hexahydro-*s*-indacen-4-yl) carbamoyl)-2-((S)-16-hydroxy-1-phenyl-2,5,8,11,14-pentaoxaheptadecan-16-yl) thiazole-5-sulfonimidamide | 703 |
| 762 | 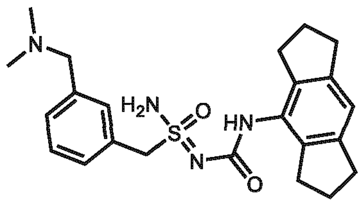 | 1-(3 -((Dimethylamino)methyl) phenyl)-N'-((1,2,3,5,6,7-hexahydro-*s*-indacen-4-yl)carbamoyl) methanesulfonimidamide | 426 |
| 763 | 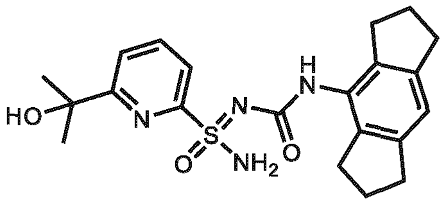 | N'-((1,2,3,5,6,7-hexahydro-*s*-indacen-4-yl) carbamoyl)-6-(2-hydroxypropan-2-yl) pyridine-2-sulfonimidamide | 415 |
| 764 | 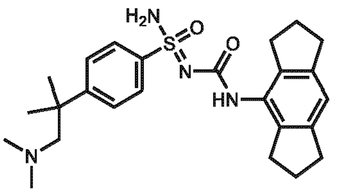 | 4-(1-(Dimethylamino)-2-methylpropan-2-yl)-N'-((1,2,3,5,6,7-hexahydro-*s*-indacen-4-yl)carbamoyl)benzenesulfonimidamide | 455 |
| 765 | 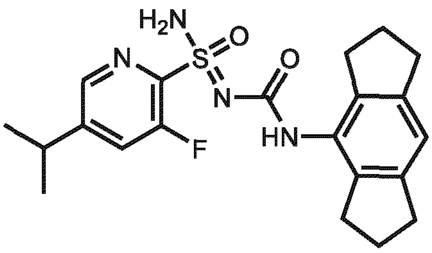 | 3-Fluoro-N'-((1,2,3,5,6,7-hexahydro-*s*-indacen-4-yl)carbamoyl)-5-isopropylpyridine-2-sulfonimidamide | 417 |

(continued)

| Example # | Structure | IUPAC Name | Exact Mass [M+H]+ |
|---|---|---|---|
| 766 | 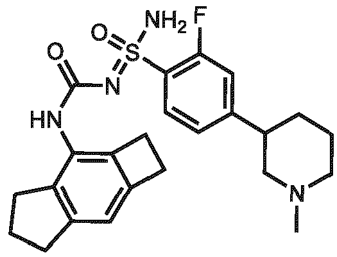 | 2-Fluoro-4-(1-methylpiperidin-3 -yl)-N'-((2,4,5,6-tetrahydro-1H-cyclobuta[f] inden-3 - yl)carbamoyl) benzenesulfonimidamide | 457 |
| 767 | 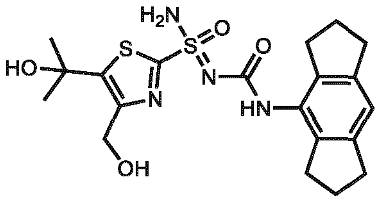 | N'-((1,2,3,5,6,7-hexahydro-*s*-indacen-4-yl) carbamoyl)-4-(hydroxymethyl)-5-(2-hydroxypropan-2-yl)thiazole-2-sulfonimidamide | 451 |
| 768 | 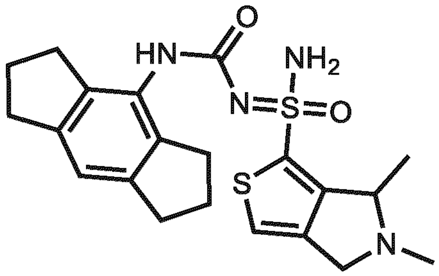 | N'-((1,2,3,5,6,7-hexahydro-*s*-indacen-4-yl) carbamoyl)-5,6-dimethyl-5,6-dihydro-4H-thieno[3,4-c]pyrrole-1-sulfonimidamide | 431 |
| 769 | 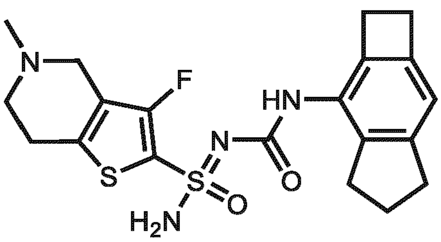 | 3-Fluoro-5-methyl-N'-((2,4,5,6-tetrahydro-1H-cyclobuta[f]inden-3-yl)carbamoyl)-4,5,6,7-tetrahydrothieno [3,2-c]pyridine-2-sulfonimidamide | 435 |
| 770 | 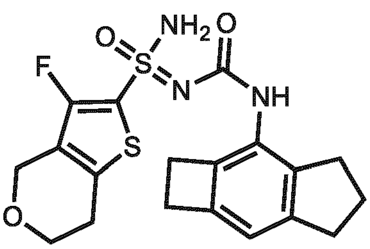 | 3-Fluoro-N'-((2,4,5,6-tetrahydro-1H-cyclobuta[f]inden-3-yl)carbamoyl)-6,7-dihydro-4H-thieno [3,2-c]pyran-2-sulfonimidamide | 422 |

**Table 69** Examples in the following table were prepared using similar conditions as described in Example **251** and Scheme **35** from appropriate starting materials.

| Example # | Structure | IUPAC Name | Exact Mass [M+H]+ |
|---|---|---|---|
| 771 | 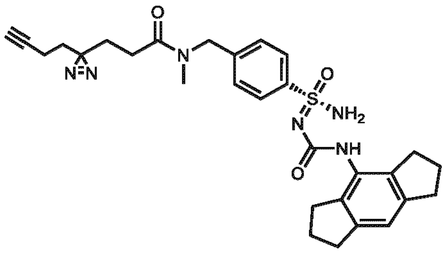 | (S)-3-(3-(but-3-yn-1-yl)-3H-diazirin-3-yl)-N-(4-(N'-((1,2,3,5,6,7-hexahydro-*s*-indacen-4-yl)carbamoyl)sulfamidimidoyl) benzyl)-N-methylpropanamide | 547 |

**Table 70** Examples in the following table were prepared using similar conditions as described in Example **453** and Scheme **IV** from appropriate starting materials.

| Example # | Structure | IUPAC Name | Exact Mass [M+H]+ |
|---|---|---|---|
| 772 | 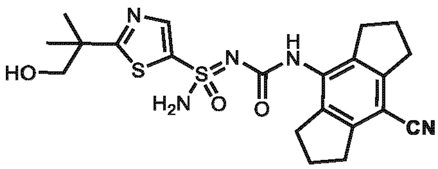 | N'-((8-cyano-1,2,3,5,6,7-hexahydro-*s*-indacen-4-yl)carbamoyl)-2-(1-hydroxy-2-methylpropan-2-yl)thiazole-5-sulfonimidamide | 460 |
| 773 | 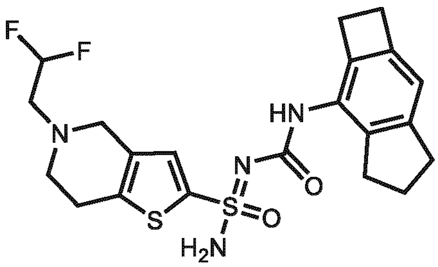 | 5-(2,2-Difluoroethyl)-N'-((2,4,5,6-tetrahydro-1H-cyclobuta[f]inden-3-yl) carbamoyl)-4,5,6,7-tetrahydrothieno [3,2-c] pyridine-2-sulfonimidamide | 467 |

**Table 71** Examples in the following table were prepared using similar conditions as described in Example **497** and Scheme **VI** from appropriate starting materials.

| Example # | Structure | IUPAC Name | Exact Mass [M+H]+ |
|---|---|---|---|
| 774 | 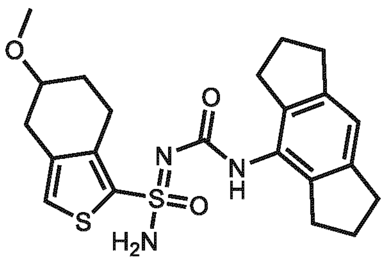 | N'-((1,2,3,5,6,7-hexahydro-*s*-indacen-4-yl) carbamoyl)-5-methoxy-4,5,6,7-tetrahydrobenzo[c]thiophene-1-sulfonimidamide | 446 |

(continued)

| Example # | Structure | IUPAC Name | Exact Mass [M+H]+ |
|---|---|---|---|
| 775 | 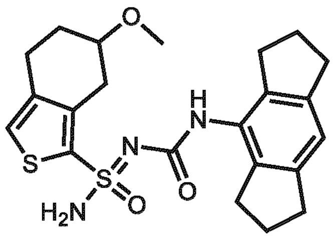 | N'-((1,2,3,5,6,7-hexahydro-*s*-indacen-4-yl) carbamoyl)-6-methoxy-4,5,6,7-tetrahydrobenzo[c]thiophene-1-sulfonimidamide | 446 |

**Table 72** Examples in the following table were prepared using similar conditions as described in Example **513** and Scheme **XI** from appropriate starting materials.

| Example # | Structure | IUPAC Name | Exact Mass [M+H]+ |
|---|---|---|---|
| 776 | 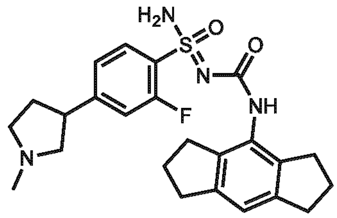 | 2-Fluoro-N'-((1,2,3,5,6,7-hexahydro-*s*-indacen-4-yl)carbamoyl)-4-(1-methylpyrrolidin-3 - yl) benzenesulfonimidamide | 457 |

**Table 73** Examples in the following table were prepared using similar conditions as described in Example **446** and Scheme **3** from appropriate starting materials.

| Example # | Structure | IUPAC Name | Exact Mass [M+H]+ |
|---|---|---|---|
| 777 | 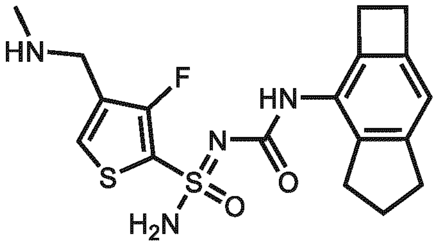 | 3-Fluoro-4-((methylamino) methyl)-N'-((2,4,5,6-tetrahydro-1H-cyclobuta [f]inden-3-yl)carbamoyl)thiophene-2-sulfonimidamide | 409 |
| 778 | 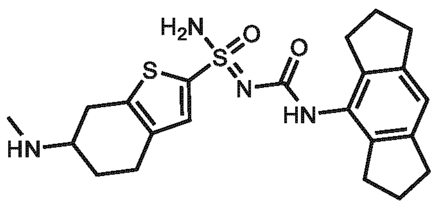 | N'-((1,2,3,5,6,7-hexahydro-s-indacen-4-yl) carbamoyl)-6-(methylamino)-4,5,6,7-tetrahydrobenzo [b]thiophene-2-sulfonimidamide | 445 |

(continued)

| Example # | Structure | IUPAC Name | Exact Mass [M+H]+ |
|---|---|---|---|
| 779 | 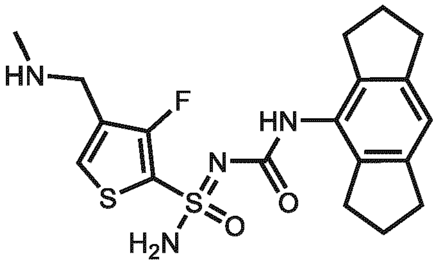 | 3-Fluoro-N'-((1,2,3,5,6,7-hexahydro-s-indacen-4-yl)carbamoyl)-4-((methylamino) methyl)thiophene-2-sulfonimidamide | 423 |
| 780 | 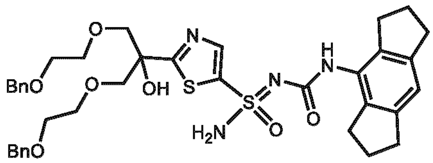 | N'-((1,2,3,5,6,7-hexahydro-s-indacen-4-yl) carbamoyl)-2-(7-hydroxy-1,13-diphenyl-2,5,9,12-tetraoxatridecan-7-yl)thiazole-5-sulfonimidamide | 721 |
| 781 | 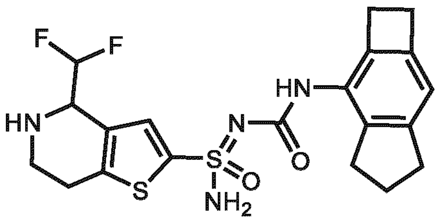 | 4-(Difluoromethyl)-N'-((2,4,5,6-tetrahydro-1H-cyclobuta[f]inden-3-yl)carbamoyl)-4,5,6,7-tetrahydrothieno[3,2-c]pyridine-2-sulfonimidamide | 453 |
| 782 | 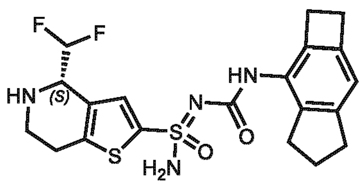 | (S) or (R) -4-(difluoromethyl)-N'-((2,4,5,6-tetrahydro-1H-cyclobuta[f]inden-3-yl) carbamoyl)-4,5,6,7-tetrahydrothieno[3,2-c] pyridine-2-sulfonimidamide Prepared from Intermediate 214F | 453 |
| 783 | 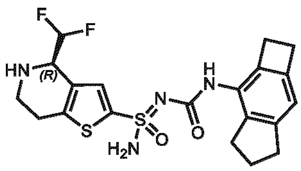 | (R) or (S) -4-(difluoromethyl)-N'-((2,4,5,6-tetrahydro-1H-cyclobuta[f]inden-3-yl) carbamoyl)-4,5,6,7-tetrahydrothieno[3,2-c] pyridine-2-sulfonimidamide Prepared from Intermediate 214S | 453 |

**Table 74** Examples in the following table were prepared using similar conditions as described in Example **451** and Scheme **III** from appropriate starting materials.

| Example # | Structure | IUPAC Name | Exact Mass [M+H]+ |
|---|---|---|---|
| 784 | 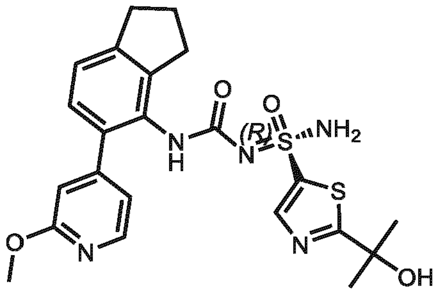 | (R)-2-(2-hydroxypropan-2-yl)-N'-((5-(2-metho xypyridin-4-yl)-2,3 -dihydro- 1H-inden-4-yl)carbamoyl)thiazole-5-sulfonimidamide | 488 |

**Table 75** Examples in the following table were obtained from chiral HPLC resolutions of racemic examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative. Some carbon stereocenters were assigned arbitrarily for registration purpose, such as the carbon centers for Examples 874~877.

| Ex. # | Structure | IUPAC Name | Column | Eluents | LC-MS $[M+H]^+$ |
|---|---|---|---|---|---|
| 787 | 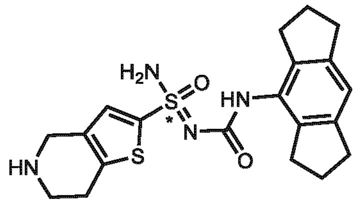 | (R) or (S)-N'-((1,2,3,5,6,7-hexahydro-*s*-indacen-4-yl)carbamoyl)-4,5,6,7-tetrahydrothieno[3,2-c] pyridine-2-sulfonimidamide | CHIRALPA K IG, 2*25 cm, 5 um | 50% in MeOH (8 mM $NH_3$·MeOH) in $CO_2$ | 417 |
| 788 | 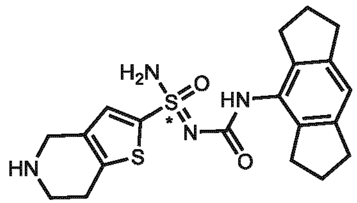 | (S) or (R)-N'-((1,2,3,5,6,7-hexahydro-*s*-indacen-4-yl)carbamoyl)-4,5,6,7-tetrahydrothieno[3,2-c] pyridine-2-sulfonimidamide | CHIRALPA K IG, 2*25 cm, 5 um | 50% in MeOH (8 mM $NH_3$·MeOH) in $CO_2$ | 417 |
| 789 | 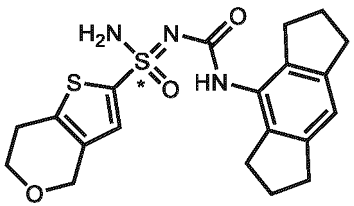 | (R) or (S)-N'-((1,2,3,5,6,7-hexahydro-*s*-indacen-4-yl)carbamoyl)-6,7-dihydro-4H-thieno [3,2-c] pyran-2-sulfonimidamide | CHIRALPA K IG, 2*25 cm, 5 um | 50% EtOH in Hex (0.1% FA) | 418 |
| 790 | 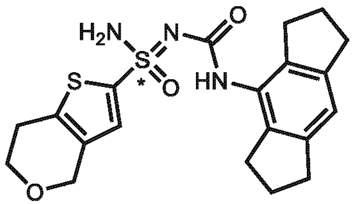 | (S) or (R)-N'-((1,2,3,5,6,7-hexahydro-*s*-indacen-4-yl)carbamoyl)-6,7-dihydro-4H-thieno [3,2-c] pyran-2-sulfonimidamide | CHIRALPA K IG, 2*25 cm, 5 um | 50% EtOH in Hex (0.1% FA) | 418 |

(continued)

| Ex. # | Structure | IUPAC Name | Column | Eluents | LC-MS $[M+H]^+$ |
|---|---|---|---|---|---|
| 791 | 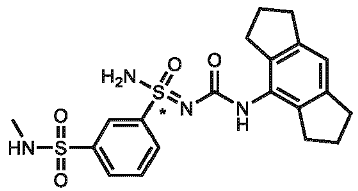 | (R) or (S)-3-(N'-((1,2,3,5,6,7-hexahydro-*s*-indacen-4-yl)carbamoyl)sulfamidimidoyl)-N-methylbenzenesulfonamide | CHIRALPA K IE, 2*25 cm, 5 um | 30% EtOH in Hex (0.1% FA) | 449 |
| 792 | 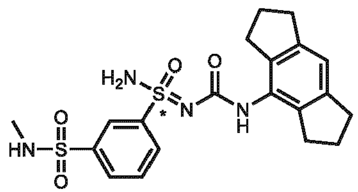 | (S) or (R)-3-(N'-((1,2,3,5,6,7-hexahydro-*s*-indacen-4-yl)carbamoyl)sulfamidimidoyl)-N-methylbenzenesulfonamide | CHIRALPA K IE, 2*25 cm, 5 um | 30% EtOH in Hex (0.1% FA) | 449 |
| 793 | 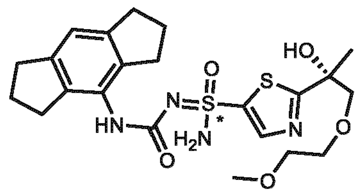 | (S) or (R)-N'-((1,2,3,5,6,7-hexahydro-*s*-indacen-4-yl)carbamoyl)-2-((R)-2-hydroxy-1-(2-methoxyethoxy)propan-2-yl)thiazole-5 -sulfonimidamide | CHIRALPA KIA, 2*25 cm, 5 um | 30% EtOH in Hex (0.1% FA) | 495 |
| 794 | 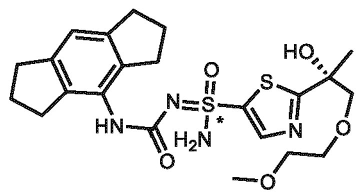 | (R) or (S)-N'-((1,2,3,5,6,7-hexahydro-*s*-indacen-4-yl)carbamoyl)-2-((R)-2-hydroxy-1-(2-methoxyethoxy)propan-2-yl)thiazole-5 -sulfonimidamide | CHIRALPA KIA, 2*25 cm, 5 um | 30% EtOH in Hex (0.1% FA) | 495 |
| 795 | 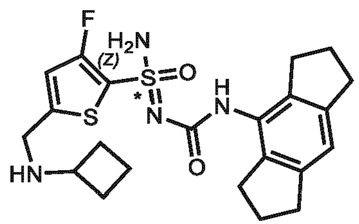 | (R) or (S)-5-((cyclobutylamino)methyl)-3-fluoro-N'-((1,2,3,5,6,7-hexahydro-*s*-indacen-4-yl) carbamoyl)thiophene-2-sulfonimidamide | Chiralpak ID, 2*25 cm, 5 um | 50% EtOH in Hex (0.1% FA) | 463 |

(continued)

| Ex. # | Structure | IUPAC Name | Column | Eluents | LC-MS $[M+H]^+$ |
|---|---|---|---|---|---|
| 796 | 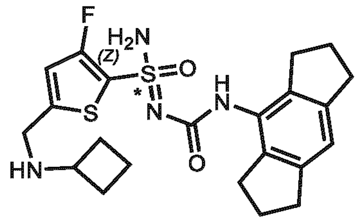 | (S) or (R)-5-((cyclobutylamino)methyl)-3-fluoro-N'-((1,2,3,5,6,7-hexahydro-*s*-indacen-4-yl) carbamoyl)thiophene-2-sulfonimidamide | Chiralpak ID, 2*25 cm, 5 um | 50% EtOH in Hex (0.1% FA) | 463 |
| 797 | 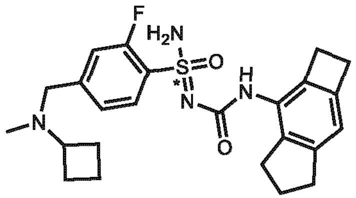 | (R) or (S)-4-((cyclobutyl(methyl)amino)methyl)-2-fluoro-N'-((2,4,5,6-tetrahydro-1H-cyclobuta[f] inden-3-yl)carbamoyl)benzenesulfonimidamide | CHIRALPA KIG, 2*25 cm, 5 um | 30% EtOH in Hex (0.1% FA) | 457 |
| 798 | 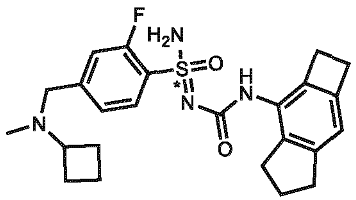 | (S) or (R)-4-((cyclobutyl(methyl)amino)methyl)-2-fluoro-N'-((2,4,5,6-tetrahydro-1H-cyclobuta[f] inden-3-yl)carbamoyl)benzenesulfonimidamide | CHIRALPA K IG, 2*25 cm, 5 um | 30% EtOH in Hex (0.1% FA) | 457 |
| 799 | 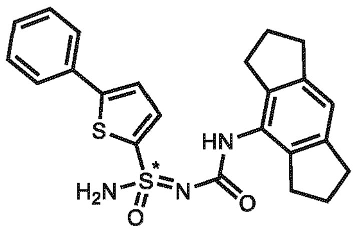 | (R) or (S)-N'-((1,2,3,5,6,7-hexahydro-*s*-indacen-4-yl)carbamoyl)-5-phenylthiophene-2-sulfonimidamide | CHIRALPA K ID, 4.6*50 mm, 5 um | 30% EtOH in Hex (0.1% FA) | 438 |

(continued)

| Ex. # | Structure | IUPAC Name | Column | Eluents | LC-MS [M+H]+ |
|---|---|---|---|---|---|
| 800 | 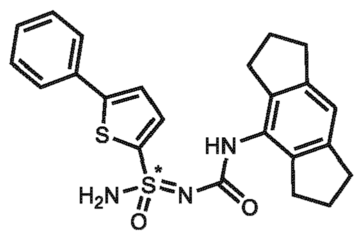 | (S) or (R)-N'-((1,2,3,5,6,7-hexahydro-*s*-indacen-4-yl)carbamoyl)-5-phenylthiophene-2-sulfonimidamide | CHIRALPA K ID, 4.6*50 mm, 5 um | 30% EtOH in Hex (0.1% FA) | 438 |
| 801 | 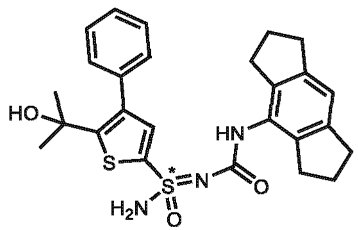 | (S) or (R)-N'-((1,2,3,5,6,7-hexahydro-*s*-indacen-4-yl)carbamoyl)-5-(2-hydroxypropan-2-yl)-4-phenylthiophene-2-sulfonimidamide | CHIRALPA KIA, 2*25 cm, 5 um | 30% EtOH in Hex (0.1% FA) | 496 |
| 802 | 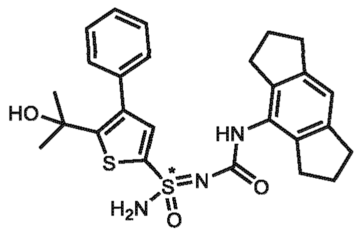 | (R) or (S)-N'-((1,2,3,5,6,7-hexahydro-*s*-indacen-4-yl)carbamoyl)-5-(2-hydroxypropan-2-yl)-4-phenylthiophene-2-sulfonimidamide | CHIRALPA KIA, 2*25 cm, 5 um | 30% EtOH in Hex (0.1% FA) | 496 |
| 803 | 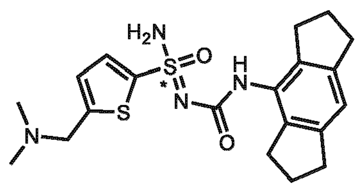 | (R) or (S)-5-((dimethylamino) methyl)-N'-((1,2,3,5,6,7-hexahydro-*s*-indacen-4-yl)carbamoyl)thiophene-2-sulfonimidamide | CHIRALPA K IE, 2*25 cm, 5 um | 50% EtOH in Hex (0.1% FA) | 419 |

(continued)

| Ex. # | Structure | IUPAC Name | Column | Eluents | LC-MS [M+H]+ |
|---|---|---|---|---|---|
| 804 | 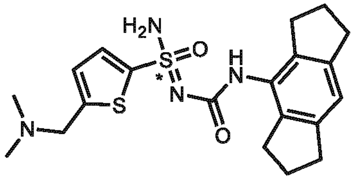 | (S) or (R)-5-((dimethylamino) methyl)-N'-((1,2,3,5,6,7-hexahydro-*s*-indacen-4-yl)carbamoyl)thiophene-2-sulfonimidamide | CHIRALPA K IE, 2*25 cm, 5 um | 50% EtOH in Hex (0.1% FA) | 419 |
| 805 | 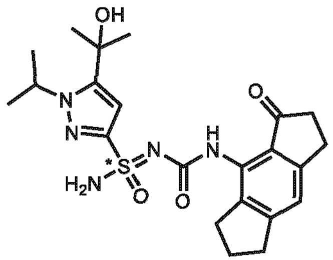 | (R) or (S)-5-(2-hydroxypropan-2-yl)-1-isopropyl-N'-((3-oxo-1,2,3,5,6,7-hexahydro-*s*-indacen-4-yl)carbamoyl)-1H-pyrazole-3-sulfonimidamide | CHIRALPA K IG, 2*25 cm, 5 um | 30% EtOH in Hex (0.1% FA) | 460 |
| 806 | 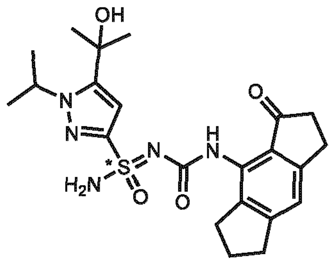 | (S) or (R)-5-(2-hydroxypropan-2-yl)-1-isopropyl-N'-((3-oxo-1,2,3,5,6,7-hexahydro-*s*-indacen-4-yl)carbamoyl)-1H-pyrazole-3 -sulfonimidamide | CHIRALPA K IG, 2*25 cm, 5 um | 30% EtOH in Hex (0.1% FA) | 460 |
| 807 | 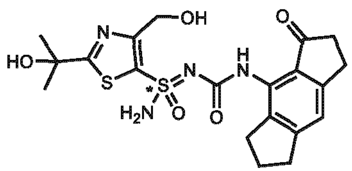 | (S) or (R)-4-(hydroxymethyl)-2-(2-hydroxypropan-2-yl)-N'-((3 -oxo-1,2,3,5,6,7-hexahydro-*s*-indacen-4-yl)carbamoyl)thiazole-5-sulfonimidamide | CHIRAL ART Cellulose-SB, 2*25 cm, 5 um | 30% EtOH in Hex (0.1% FA) | 465 |

(continued)

| Ex. # | Structure | IUPAC Name | Column | Eluents | LC-MS $[M+H]^+$ |
|---|---|---|---|---|---|
| 808 | 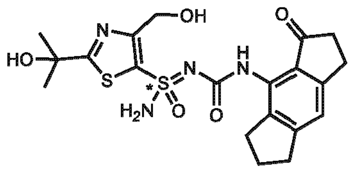 | (R) or (S)-4-(hydroxymethyl)-2-(2-hydroxypropan-2-yl)-N'-((3 -oxo-1,2,3,5,6,7-hexahydro-*s*-indacen-4-yl)carbamoyl)thiazole-5-sulfonimidamide | CHIRAL ART Cellulose-SB, 2*25 cm, 5 um | 30% EtOH in Hex (0.1% FA) | 465 |
| 809 | 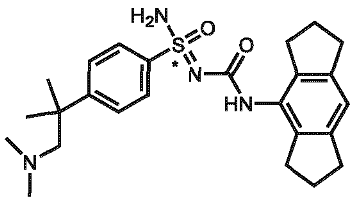 | (R) or (S)-4-(1-(dimethylamino)-2-methylpropan-2-yl)-N'-((1,2,3,5,6,7-hexahydro-*s*-indacen-4-yl) carbamoyl)benzenesulfonimidamide | CHIRALPA KIG, 3*25 cm, 5 um | 30% EtOH in Hex (0.1% FA) | 455 |
| 810 | 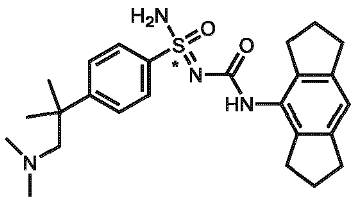 | (S) or (R)-4-(1-(dimethylamino)-2-methylpropan-2-yl)-N'-((1,2,3,5,6,7-hexahydro-*s*-indacen-4-yl) carbamoyl)benzenesulfonimidamide | CHIRALPA KIG, 3*25 cm, 5 um | 30% EtOH in Hex (0.1% FA) | 455 |
| 811 | 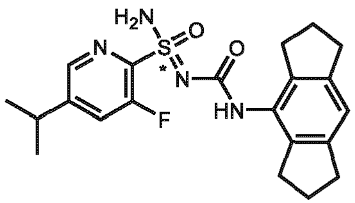 | (R) or (S)-3-fluoro-N'-((1,2,3,5,6,7-hexahydro-*s*-indacen-4-yl)carbamoyl)-5-isopropylpyridine-2-sulfonimidamide | CHIRALPA K ID, 2*25 cm, 5 um | 30% EtOH in Hex (0.1% FA) | 417 |
| 812 | 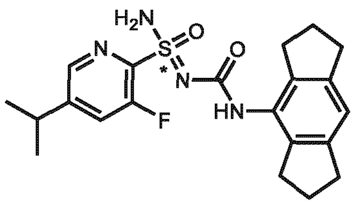 | (S) or (R)-3-fluoro-N'-((1,2,3,5,6,7-hexahydro-*s*-indacen-4-yl)carbamoyl)-5-isopropylpyridine-2-sulfonimidamide | CHIRALPA K ID, 2*25 cm, 5 um | 30% EtOH in Hex (0.1% FA) | 417 |

(continued)

| Ex. # | Structure | IUPAC Name | Column | Eluents | LC-MS $[M+H]^+$ |
|---|---|---|---|---|---|
| 813 | 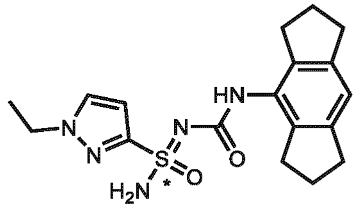 | (R) or (S)-1-ethyl-N'-((1,2,3,5,6,7-hexahydro-*s*-indacen-4-yl)carbamoyl)-1H-pyrazole-3-sulfonimidamide | CHIRALPA KIG, 2*25 cm, 5 um | 40% MeOH(8m M $NH_3$·MeOH) in $CO_2$ | 374 |
| 814 | 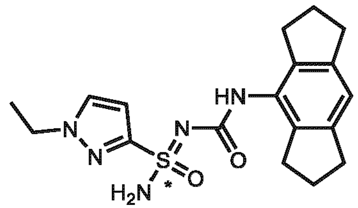 | (S) or (R)-1-ethyl-N'-((1,2,3,5,6,7-hexahydro-*s*-indacen-4-yl)carbamoyl)-1H-pyrazole-3-sulfonimidamide | CHIRALPA K IG, 2*25 cm, 5 um | 40% MeOH(8m M $NH_3$·MeOH) in $CO_2$ | 374 |
| 815 | 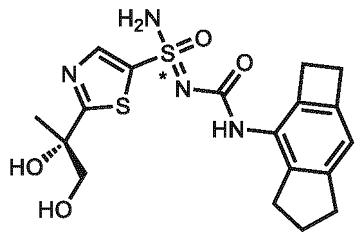 | (R) or (S)-2-((S)-1,2-dihydroxypropan-2-yl)-N'-((2,4,5,6-tetrahydro-1H-cyclobuta[f]inden-3-yl)carbamoyl)thiazole-5-sulfonimidamide | CHIRALPA K IF, 4.6*50 mm, 3 um | 20% EtOH in Hex (0.1% FA) | 423 |
| 816 | 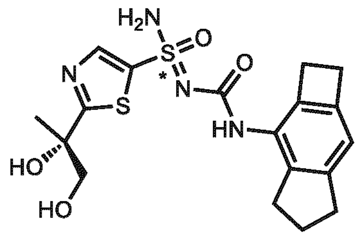 | (S) or (R)-2-((S)-1,2-dihydroxypropan-2-yl)-N'-((2,4,5,6-tetrahydro-1H-cyclobuta[f]inden-3-yl)carbamoyl)thiazole-5-sulfonimidamide | CHIRALPA K IF, 4.6*50 mm, 3 um | 20% EtOH in Hex (0.1% FA) | 423 |

(continued)

| Ex. # | Structure | IUPAC Name | Column | Eluents | LC-MS $[M+H]^+$ |
|---|---|---|---|---|---|
| 817 | 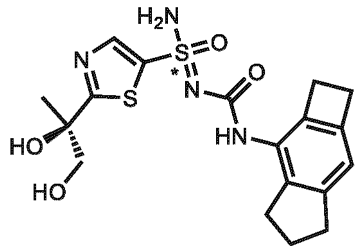 | (R) or (S)-2-((R)-1,2-dihydroxypropan-2-yl)-N'-((2,4,5,6-tetrahydro-1H-cyclobuta[f]inden-3-yl)carbamoyl)thiazole-5-sulfonimidamide | CHIRALPA K IF, 2*25 cm, 5 um | 30% EtOH in Hex (0.1% FA) | 423 |
| 818 | 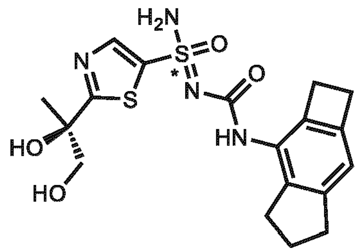 | (S) or (R)-2-((R)-1,2-dihydroxypropan-2-yl)-N'-((2,4,5,6-tetrahydro-1H-cyclobuta[f]inden-3-yl)carbamoyl)thiazole-5-sulfonimidamide | CHIRALPA K IF, 2*25 cm, 5 um | 30% EtOH in Hex (0.1% FA) | 423 |
| 819 | 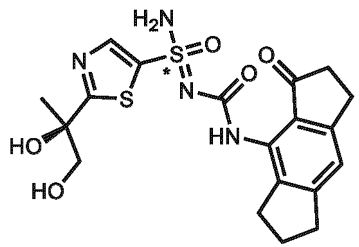 | (R) or (S)-2-((R)-1,2-dihydroxypropan-2-yl)-N'-((3-oxo-1,2,3,5,6,7-hexahydro-*s*-indacen-4-yl)carbamoyl)thiazole-5-sulfonimidamide (from Example 743) | CHIRALPA K ID, 2*25 cm, 5 um | 30% EtOH in Hex (0.1% FA) | 451 |
| 820 | 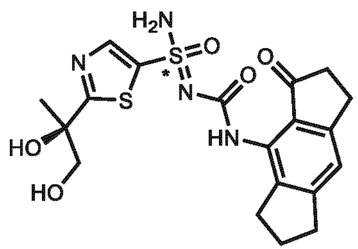 | (S) or (R)-2-((R)-1,2-dihydroxypropan-2-yl)-N'-((3-oxo-1,2,3,5,6,7-hexahydro-*s*-indacen-4-yl)carbamoyl)thiazole-5-sulfonimidamide (from Example 743) | CHIRALPA K ID, 2*25 cm, 5 um | 30% EtOH in Hex (0.1% FA) | 451 |

(continued)

| Ex. # | Structure | IUPAC Name | Column | Eluents | LC-MS $[M+H]^+$ |
|---|---|---|---|---|---|
| 821 | 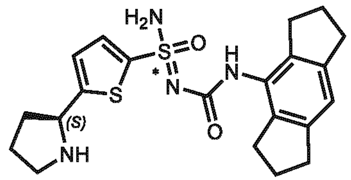 | (R) or (S) -N'-((1,2,3,5,6,7-hexahydro-*s*-indacen-4-yl)carbamoyl)-5-((S)-pyrrolidin-2-yl)thiophene-2-sulfonimidamide (separated from chiral Ex.730) | CHIRALPA KIG, 2*25 cm, 5 um | 40 % EtOH in Hex (8 mM $NH_3$·MeOH) | 431 |
| 822 | 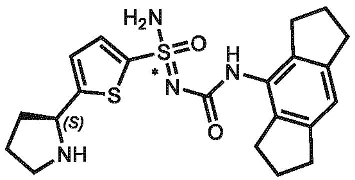 | (S) or (R) -N'-((1,2,3,5,6,7-hexahydro-*s*-indacen-4-yl)carbamoyl)-5-((S)-pyrrolidin-2-yl)thiophene-2-sulfonimidamide (separated from chiral Ex.730) | CHIRALPA K IG, 2*25 cm, 5 um | 40 % EtOH in Hex (8 mM $NH_3$·MeOH) | 431 |
| 823 | 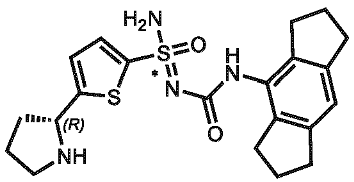 | (R) or (S) -N'-((1,2,3,5,6,7-hexahydro-*s*-indacen-4-yl)carbamoyl)-5-((R)-pyrrolidin-2-yl)thiophene-2-sulfonimidamide (separated from chiral Ex.731) | Chiralpak ID, 2*25 cm, 5 um | 10% EtOH in Hex:DCM= 3:1 (10 mM $NH_3$-MeOH) | 431 |
| 824 | 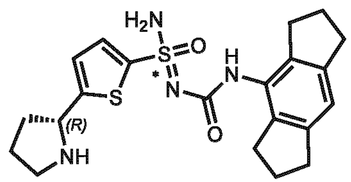 | (S) or (R) -N'-((1,2,3,5,6,7-hexahydro-*s*-indacen-4-yl)carbamoyl)-5-((R)-pyrrolidin-2-yl)thiophene-2-sulfonimidamide (separated from chiral Ex.731) | Chiralpak ID, 2*25 cm, 5 um | 10% EtOH in Hex:DCM= 3:1 (10 mM $NH_3$-MeOH) | 431 |
| 825 | 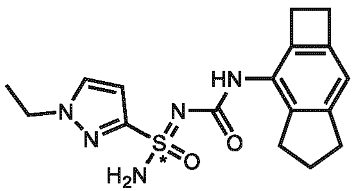 | (R) or (S) -1-ethyl-N'-((2,4,5,6-tetrahydro-1H-cyclobuta[f]inden-3-yl)carbamoyl)-1H-pyrazole-3 -sulfonimidamide | CHIRALPA K IE, 3*25 cm, 5 um | 50% EtOH in Hex:DCM= 3:1 (10 mM $NH_3$-MEOH) | 360 |

(continued)

| Ex. # | Structure | IUPAC Name | Column | Eluents | LC-MS $[M+H]^+$ |
|---|---|---|---|---|---|
| 826 | 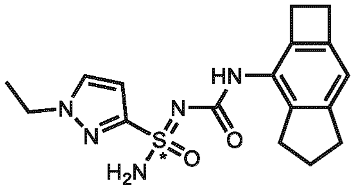 | (S) or (R) -1-ethyl-N'-((2,4,5,6-tetrahydro-1H-cyclobuta[f]inden-3-yl)carbamoyl)-1H-pyrazole-3 -sulfonimidamide | CHIRALPA K IE, 3*25 cm, 5 um | 50% EtOH in Hex:DCM= 3:1 (10 mM $NH_3$-MEOH) | 360 |
| 827 | 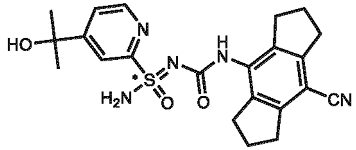 | (R) or (S)-N'-((8-cyano-1,2,3,5,6,7-hexahydro-*s*-indacen-4-yl)carbamoyl)-4-(2-hydroxypropan-2-yl)pyridine-2-sulfonimidamide | CHIRALPA K IF, 2*25 cm, 5 um | 45% EtOH (2 mM $NH_3$-MeOH) in $CO_2$ | 440 |
| 828 | 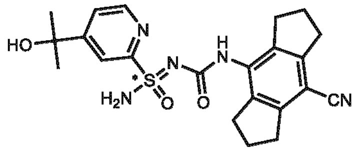 | (S) or (R)-N'-((8-cyano-1,2,3,5,6,7-hexahydro-*s*-indacen-4-yl)carbamoyl)-4-(2-hydroxypropan-2-yl)pyridine-2-sulfonimidamide | CHIRALPA K IF, 2*25 cm, 5 um | 45% EtOH (2 mM $NH_3$-MeOH) in $CO_2$ | 440 |
| 829 | 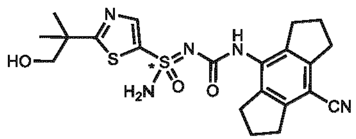 | (S) or (R)-N'-((8-cyano-1,2,3,5,6,7-hexahydro-*s*-indacen-4-yl)carbamoyl)-2-(1- hydroxy-2-methylpropan-2-yl)thiazole-5-sulfonimidamide | CHIRAL ART Cellulose-SB, 2*25 cm, 5 um | 30% EtOH in Hex (0.1% FA) | 460 |
| 830 | 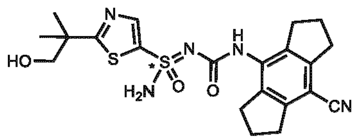 | (R) or (S)-N'-((8-cyano-1,2,3,5,6,7-hexahydro-*s*-indacen-4-yl)carbamoyl)-2-(1-hydroxy-2-methylpropan-2-yl)thiazole-5-sulfonimidamide | CHIRAL ART Cellulose-SB, 2*25 cm, 5 um | 30% EtOH in Hex (0.1% FA) | 460 |
| 831 | 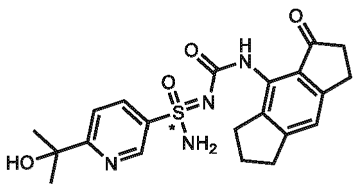 | (S) or (R)-6-(2-hydroxypropan-2-yl)-N'-((3-oxo-1,2,3,5,6,7-hexahydro-*s*-indacen-4-yl) carbamoyl)pyridine-3-sulfonimidamide | CHIRAL ART Cellulose-SB, 2*25 cm, 5 um | 30% EtOH in Hex (0.1% FA) | 429 |

(continued)

| Ex. # | Structure | IUPAC Name | Column | Eluents | LC-MS $[M+H]^+$ |
|---|---|---|---|---|---|
| 832 | 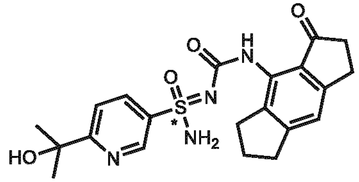 | (R) or (S)-6-(2-hydroxypropan-2-yl)-N'-((3-oxo-1,2,3,5,6,7-hexahydro-*s*-indacen-4-yl) carbamoyl)pyridine-3-sulfonimidamide | CHIRAL ART Cellulose-SB, 2*25 cm, 5 um | 30% EtOH in Hex (0.1% FA) | |
| 833 | 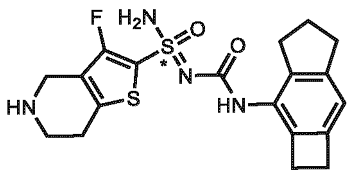 | (R) or (S)-3-fluoro-N'-((2,4,5,6-tetrahydro-1H-cyclobuta[f]inden-3-yl)carbamoyl)-4,5,6,7-tetrahydrothieno[3,2-c]pyridine-2-sulfonimidamide | CHIRALPA K IC, 2*25 cm, 5 um | 15% MeOH in Hex:DCM= 3:1 (10 mM $NH_3$-MeOH) | 421 |
| 834 | 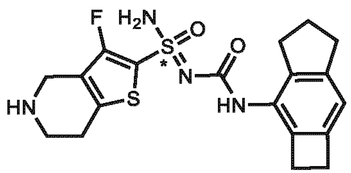 | (S) or (R)-3-fluoro-N'-((2,4,5,6-tetrahydro-1H-cyclobuta[f]inden-3-yl)carbamoyl)-4,5,6,7-tetrahydrothieno[3,2-c]pyridine-2-sulfonimidamide | CHIRALPA K IC, 2*25 cm, 5 um | 15% MeOH in Hex:DCM= 3:1 (10 mM $NH_3$-MeOH) | 421 |
| 835 | 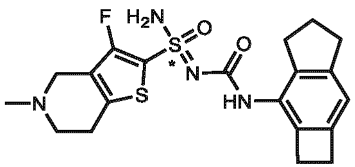 | (R) or (S)-3-fluoro-5-methyl-N'-((2,4,5,6-tetrahydro-1H-cyclobuta[f]inden-3 - yl) carbamoyl)-4,5,6,7-tetrahydrothieno[3,2-c] pyridine-2-sulfonimidamide | CHIRALPA K IE, 2*25 cm, 5 um | 50% EtOH in Hex (0.1% FA) | 435 |
| 836 | 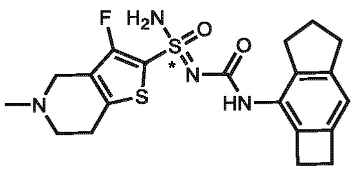 | (S) or (R)-3-fluoro-5-methyl-N'-((2,4,5,6-tetrahydro-1H-cyclobuta[f]inden-3 -yl) carbamoyl)-4,5,6,7-tetrahydrothieno[3,2-c] pyridine-2-sulfonimidamide | CHIRALPA K IE, 2*25 cm, 5 um | 50% EtOH in Hex (0.1% FA) | 435 |

(continued)

| Ex. # | Structure | IUPAC Name | Column | Eluents | LC-MS $[M+H]^+$ |
|---|---|---|---|---|---|
| 837 | 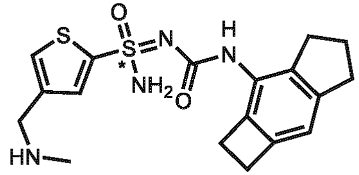 | (R) or (S)-4-((methylamino)methyl)-N'-((2,4,5,6-tetrahydro-1H-cyclobuta[f]inden-3-yl)carbamoyl) thiophene-2-sulfonimidamide | CHIRALPA KIG, 2*25 cm, 5 um | 50% EtOH (8mM $NH_3 \cdot MeOH$) in $CO_2$ | 391 |
| 838 | 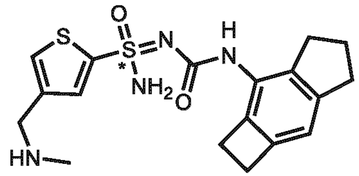 | (S) or (R)-4-((methylamino)methyl)-N'-((2,4,5,6-tetrahydro-1H-cyclobuta[f]inden-3-yl)carbamoyl) thiophene-2-sulfonimidamide | CHIRALPA K IG, 2*25 cm, 5 um | 50% EtOH (8mM $NH_3 \cdot MeOH$) in $CO_2$ | 391 |
| 839 | 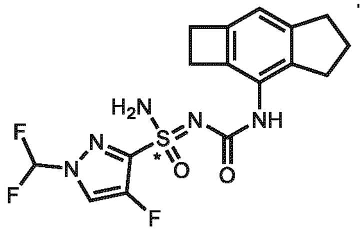 | (R) or (S)-1-(difluoromethyl)-4-fluoro-N'-((2,4,5,6-tetrahydro-1H-cyclobuta[f]inden-3-yl)carbamoyl)-1H-pyrazole-3 - sulfonimidamide | Chiralpak ID, 2*25 cm, 5 um | 30% EtOH in Hex (0.1% FA) | 400 |
| 840 | 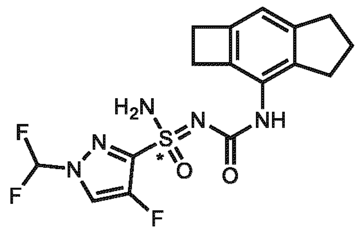 | (S) or (R)-1-(difluoromethyl)-4-fluoro-N'-((2,4,5,6-tetrahydro-1H-cyclobuta[f]inden-3-yl)carbamoyl)-1H-pyrazole-3 - sulfonimidamide | Chiralpak ID, 2*25 cm, 5 um | 30% EtOH in Hex (0.1% FA) | 400 |

(continued)

| Ex. # | Structure | IUPAC Name | Column | Eluents | LC-MS $[M+H]^+$ |
|---|---|---|---|---|---|
| 841 | 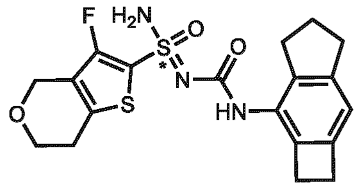 | (R) or (S)-3-fluoro-N'-((2,4,5,6-tetrahydro-1H-cyclobuta[f]inden-3-yl)carbamoyl)-6,7-dihydro-4H-thieno[3,2-c]pyran-2-sulfonimidamide | CHIRALPA K IE, 2*25 cm, 5 um | 50% EtOH in Hex (0.1% FA) | 422 |
| 842 | 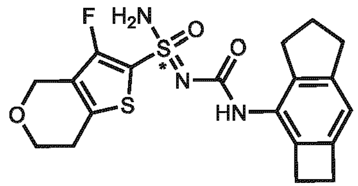 | (S) or (R)-3-fluoro-N'-((2,4,5,6-tetrahydro-1H-cyclobuta[f]inden-3-yl)carbamoyl)-6,7-dihydro-4H-thieno[3,2-c]pyran-2-sulfonimidamide | CHIRALPA K IE, 2*25 cm, 5 um | 50% EtOH in Hex (0.1% FA) | 422 |
| 843 | 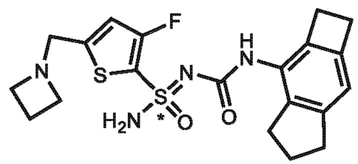 | (R) or (S)-5-(azetidin-1-ylmethyl)-3-fluoro-N'-((2,4,5,6-tetrahydro-1H-cyclobuta[f]inden-3-yl)carbamoyl)thiophene-2-sulfonimidamide | CHIRALPA K IC, 2*25 cm, 5 um | 50% EtOH in Hex (0.1% IPA) | 435 |
| 844 | 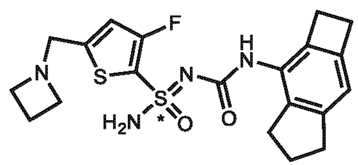 | (S) or (R)-5-(azetidin-1-ylmethyl)-3-fluoro-N'-((2,4,5,6-tetrahydro-1H-cyclobuta[f]inden-3-yl)carbamoyl)thiophene-2-sulfonimidamide | CHIRALPA K IC, 2*25 cm, 5 um | 50% EtOH in Hex (0.1% IPA) | 435 |
| 845 | 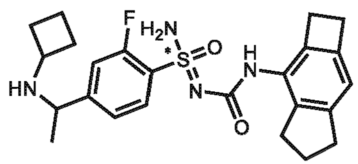 | (R/S, R)-4-(1-(cyclobutylamino)ethyl)-2-fluoro-N'-((2,4,5,6-tetrahydro-1H-cyclobuta[f]inden-3-yl)carbamoyl)benzenesulfonimidamide | 1st and 2nd peak CHIRALPA K IG, 2*25 cm, 5 um | 50% EtOH in Hex (8 mM $NH_3$·MeOH) | 457 |

(continued)

| Ex. # | Structure | IUPAC Name | Column | Eluents | LC-MS [M+H]$^+$ |
|---|---|---|---|---|---|
| 846 | 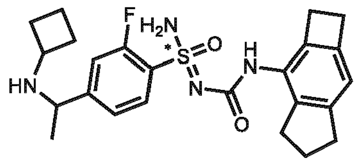 | (R/S, S)-4-(1-(cyclobutylamino)ethyl)-2-fluoro-N'-((2,4,5,6-tetrahydro-1H-cyclobuta[flinden-3-yl)carbamoyl)benzenesulfonimidamide | 3rd and 4th peak CHIRALPA K IG, 2*25 cm, 5 um | 50% EtOH in Hex (8 mM $NH_3$·MeOH) | 457 |
| 847 | 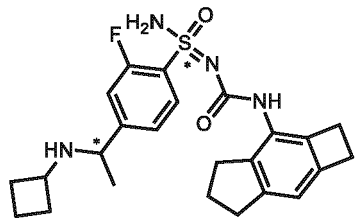 | (R, R) or (S, R) -4-(1-(cyclobutylamino)ethyl)-2-fluoro-N'-((2,4,5,6-tetrahydro-1H-cyclobuta[f] inden-3 -yl)carbamoyl)benzenesulfonimidamide (Separated from Ex. 832) | Chiralpak AD, 2*25 cm, 5 um | 20% EtOH in Hex (8 mM $NH_3$·MeOH) | 457 |
| 848 | 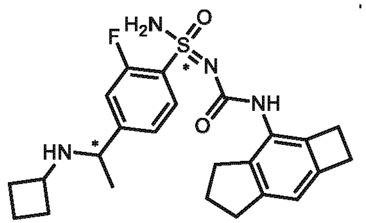 | (S, R) or (R, R) -4-(1-(cyclobutylamino)ethyl)-2-fluoro-N'-((2,4,5,6-tetrahydro-1H-cyclobuta[f] inden-3 -yl)carbamoyl)benzenesulfonimidamide (Separated from Ex. 832) | Chiralpak AD, 2*25 cm, 5 um | 20% EtOH in Hex (8 mM $NH_3$·MeOH) | 457 |
| 849 | 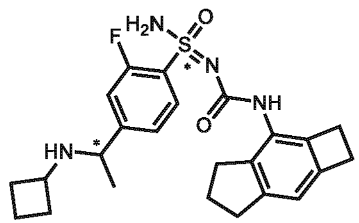 | (S, S) or (R, S) -4-(1-(cyclobutylamino)ethyl)-2-fluoro-N'-((2,4,5,6-tetrahydro-1H-cyclobuta[f] inden-3 -yl)carbamoyl)benzenesulfonimidamide (Separated from Ex. 833) | Chiralpak AD, 2*25 cm, 5 um | 30% EtOH in Hex (8 mM $NH_3$·MeOH) | 457 |

(continued)

| Ex. # | Structure | IUPAC Name | Column | Eluents | LC-MS $[M+H]^+$ |
|---|---|---|---|---|---|
| 850 | 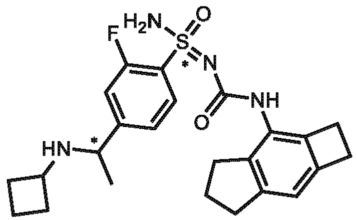 | (R, S) or (S, S) -4-(1-(cyclobutylamino)ethyl)-2-fluoro-N'-((2,4,5,6-tetrahydro-1H-cyclobuta[f]inden-3 -yl)carbamoyl)benzenesulfonimidamide (Separated from Ex. 833) | Chiralpak AD, 2*25 cm, 5 um | 30% EtOH in Hex (8 mM $NH_3$·MeOH) | 457 |
| 851 | 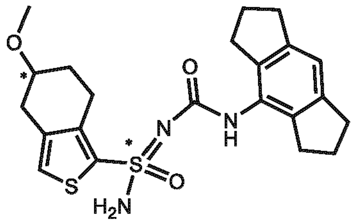 | (R, R) or (S, R)-N'-((1,2,3,5,6,7-hexahydro-*s*-indacen-4yl)carbamoyl)-5-methoxy-4,5,6,7-tetrahydrobenzo[c]thiophene-1-sulfonimidamide | Column 2: CHIRALPAK IF, 2*25 cm, 5 um, 20% EtOH (10 mM $NH_3$·MeOH) in (Hex:DCM=3:1) to separate two fasting-co-eluting isomers from Column 1: CHIRALPAK ID 2*25 cm, 5 um, 50% EtOH (0.1% DEA) in (Hex/DCM 1/1) | | 446 |
| 852 | 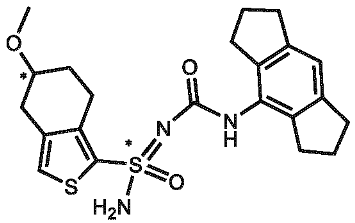 | (S, R) or (R, R)-N'-((1,2,3,5,6,7-hexahydro-*s*-indacen-4yl)carbamoyl)-5-methoxy-4,5,6,7-tetrahydrobenzo[c]thiophene-1-sulfonimidamide | | | 446 |

(continued)

| Ex. # | Structure | IUPAC Name | Column | Eluents | LC-MS $[M+H]^+$ |
|---|---|---|---|---|---|
| 853 | 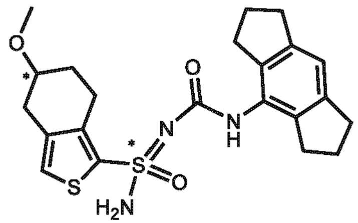 | (R, S) or (S, S)-N'-((1,2,3,5,6,7-hexahydro-*s*-indacen-4-yl)carbamoyl)-5-methoxy-4,5,6,7-tetrahydrobenzo[c]thiophene-1-sulfonimidamide | Column 2: CHIRALPAK ID, 2*25 cm, 5 um, 50% EtOH (10 mM $NH_3$-MeOH) in (Hex:DCM=1:1) to separate two slow-co-eluting isomers from Column 1: CHIRALPAK ID 2*25 cm, 5 um, 50% EtOH (0.1% DEA) in (Hex/DCM 1/1) | | 446 |
| 854 | 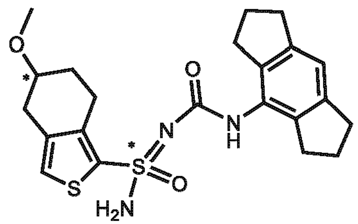 | (S, S) or (R, S)-N'-((1,2,3,5,6,7-hexahydro-*s*-indacen-4-yl)carbamoyl)-5-methoxy-4,5,6,7-tetrahydrobenzo[c]thiophene-1-sulfonimidamide | | | 446 |
| 855 | 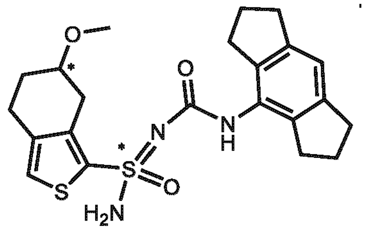 | (R, S) or (S, S)-N'-((1,2,3,5,6,7-hexahydro-*s*-indacen-4-yl)carbamoyl)-6-methoxy-4,5,6,7-tetrahydrobenzo[c]thiophene-1-sulfonimidamide | Column 2: CHIRALPAK IG, 2*25 cm, 5 um 20% EtOH (10 mM $NH_3$-MeOH) in (Hex:DCM=1:1) to separate two fasting-co-eluting isomers from Column 1: CHIRALPAK ID, 2*25 cm, 5 um, MTBE (0.1% DEA): EtOH=80:20 | | 446 |
| 856 | 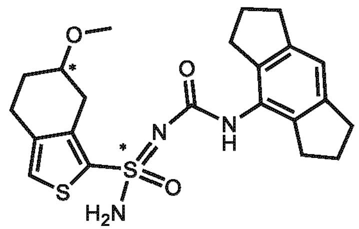 | (S, S) or (R, S)-N'-((1,2,3,5,6,7-hexahydro-*s*-indacen-4-yl)carbamoyl)-6-methoxy-4,5,6,7-tetrahydrobenzo[c]thiophene-1-sulfonimidamide | | | 446 |

(continued)

| Ex. # | Structure | IUPAC Name | Column | Eluents | LC-MS $[M+H]^+$ |
|---|---|---|---|---|---|
| 857 | 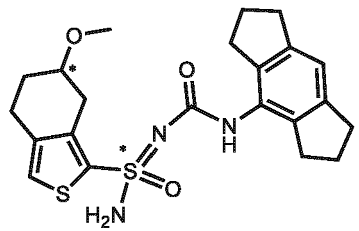 | (R, R) or (S, R)-N'-((1,2,3,5,6,7-hexahydro-*s*-indacen-4yl)carbamoyl)-6-methoxy-4,5,6,7-tetrahydrobenzo[c]thiophene-1-sulfonimidamide | Column 2: CHIRALPAK ID, 2*25 cm, 5 um 20% EtOH in MTBE (10 mM $NH_3$-MeOH) to separate two slow-co-eluting isomers from Column 1: CHIRALPAK ID, 2*25 cm, 5 um, MTBE (0.1% DEA): EtOH=80:20 | | 446 |
| 858 | 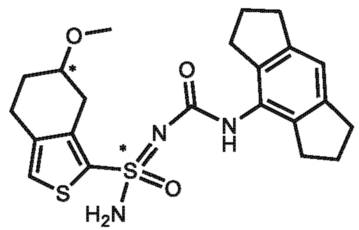 | (S, R) or (R, R)-N'-((1,2,3,5,6,7-hexahydro-*s*-indacen-4yl)carbamoyl)-6-methoxy-4,5,6,7-tetrahydrobenzo[c]thiophene-1-sulfonimidamide | | | 446 |
| 859 | 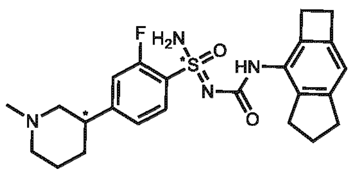 | (S, R) or (R, R)-2-fluoro-4-(1-methylpiperidin-3-yl)-N'-((2,4,5,6-tetrahydro-1H-cyclobuta[f]inden-3-yl)carbamoyl)benzenesulfonimidamide | 1st peak from CHIRALPAK IG, 2*25 cm, 5 um, 40% EtOH in Hex (8 mM $NH_3 \cdot MeOH$) | | 457 |
| 860 | 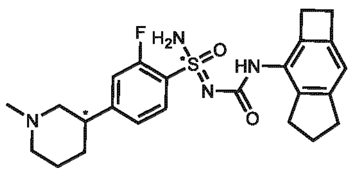 | (R, S) or (S, S)-2-fluoro-4-(1-methylpiperidin-3-yl)-N'-((2,4,5,6-tetrahydro-1H-cyclobuta[f]inden-3-yl)carbamoyl)benzenesulfonimidamide | Column 2: Reg-AD, 30*250 mm, 5 um, 15% IPA in Hex (8 mM $NH_3 \cdot MeOH$) to separate 2nd and 3rd peak from Column 1: CHIRALPAK IG, 2*25 cm, 5 um, EtOH in Hex (8 mM $NH_3 \cdot MeOH$) | | 457 |
| 861 | 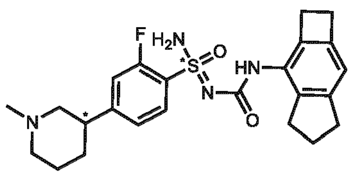 | (S, S) or (R, S)-2-fluoro-4-(1-methylpiperidin-3-yl)-N'-((2,4,5,6-tetrahydro-1H-cyclobuta[f]inden-3-yl)carbamoyl)benzenesulfonimidamide | | | 457 |

(continued)

| Ex. # | Structure | IUPAC Name | Column | Eluents | LC-MS $[M+H]^+$ |
|---|---|---|---|---|---|
| 862 | 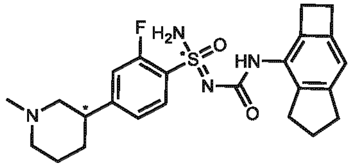 | (R, R) or (S, R)-2-fluoro-4-(1-methylpiperidin-3-yl)-N'-((2,4,5,6-tetrahydro-1H-cyclobuta[f]inden-3-yl)carbamoyl)benzenesulfonimidamide | 4th peak from CHIRALPAK IG, 2*25 cm, 5 um, EtOH in Hex (8 mM $NH_3 \cdot MeOH$) | | 457 |
| 863 | 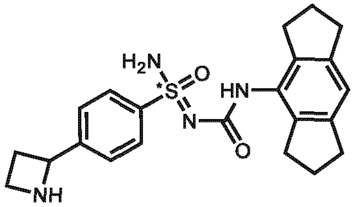 | (S/R, R)-4-(azetidin-2-yl)-N'-((1,2,3,5,6,7-hexahydro-s-indacen-4-yl)carbamoyl) benzenesulfonimidamide | 1st and 2nd peak CHIRALPA K ID, 2*25 cm, 5 um | 40% IPA in Hex:DCM=3 : 1 (10 mM $NH_3$-MeOH) | 411 |
| 864 | 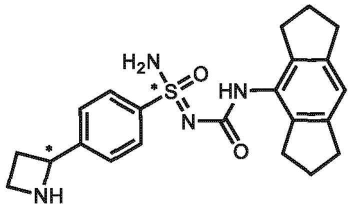 | (R, S) or (S, S)-4-(azetidin-2-yl)-N'-((1,2,3,5,6,7-hexahydro-s-indacen-4-yl)carbamoyl) benzenesulfonimidamide | 3rd peak CHIRALPA K ID, 2*25 cm, 5 um | 40% IPA in Hex:DCM=3 : 1 (10 mM $NH_3$-MeOH) | 411 |
| 865 | 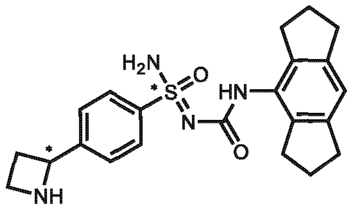 | (S, S) or (R, S)-4-(azetidin-2-yl)-N'-((1,2,3,5,6,7-hexahydro-s-indacen-4-yl)carbamoyl) benzenesulfonimidamide | 4th peak CHIRALPA K ID, 2*25 cm, 5 um | 40% IPA in Hex:DCM=3 : 1 (10 mM $NH_3$-MeOH) | 411 |

(continued)

| Ex. # | Structure | IUPAC Name | Column | Eluents | LC-MS $[M+H]^+$ |
|---|---|---|---|---|---|
| 866 | 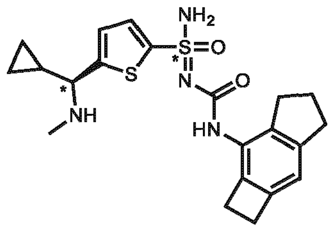 | (S, R) or (R, R)-5-(cyclopropyl(methylamino) methyl)-N'-((2,4,5,6-tetrahydro-1H-cyclobuta[f] inden-3 -yl)carbamoyl)thiophene-2-sulfonimidamide (Separated from Ex. 720) | CHIRALPA K IG, 2*25 cm, 5 um | 40% EtOH in Hex (8 mM $NH_3$·MeOH) | 416 |
| 867 | 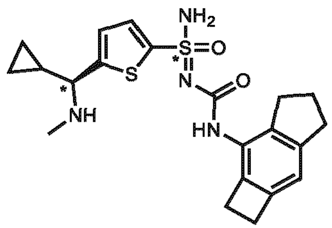 | (S, S) or (R, S)-5-(cyclopropyl(methylamino) methyl)-N'-((2,4,5,6-tetrahydro-1H-cyclobuta[f] inden-3 -yl)carbamoyl)thiophene-2-sulfonimidamide (Separated from Ex. 720) | CHIRALPA K IG, 2*25 cm, 5 um | 40% EtOH in Hex (8 mM $NH_3$·MeOH) | 416 |
| 868 | 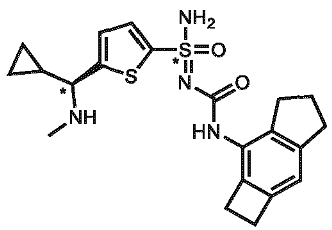 | (R, R) or (S, R)-5-(cyclopropyl(methylamino) methyl)-N'-((2,4,5,6-tetrahydro-1H-cyclobuta[f] inden-3 -yl)carbamoyl)thiophene-2-sulfonimidamide (Separated from Ex. 721) | CHIRALPA K IG, 2*25 cm, 5 um | 40% EtOH in Hex (8 mM $NH_3$·MeOH) | 416 |
| 869 | 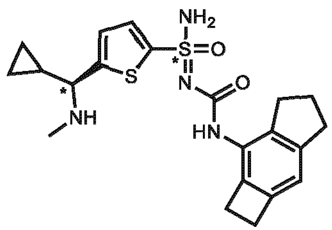 | (R, S) or (S, S)-5-(cyclopropyl(methylamino) methyl)-N'-((2,4,5,6-tetrahydro-1H-cyclobuta[f] inden-3 -yl)carbamoyl)thiophene-2-sulfonimidamide (Separated from Ex. 721) | CHIRALPA K IG, 2*25 cm, 5 um | 40% EtOH in Hex (8 mM $NH_3$·MeOH) | 416 |

(continued)

| Ex. # | Structure | IUPAC Name | Column | Eluents | LC-MS $[M+H]^+$ |
|---|---|---|---|---|---|
| 870 | 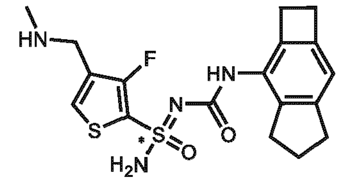 | (R) or (S) -3-fluoro-4-((methylamino) methyl)-N'-((2,4,5,6-tetrahydro-1H-cyclobuta[f] inden-3-yl)carbamoyl)thiophene-2-sulfonimidamide | CHIRALPA K IC, 3*25 cm, 5 um | 30% IPA in Hex:DCM=3 : 1 (10 mM $NH_3$-MeOH) | 409 |
| 871 | 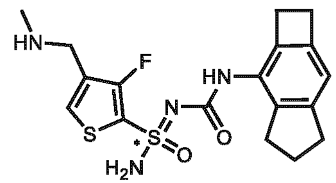 | (S) or (R) -3-fluoro-4-((methylamino) methyl)-N'-((2,4,5,6-tetrahydro-1H-cyclobuta[f] inden-3-yl)carbamoyl)thiophene-2-sulfonimidamide | CHIRALPA K IC, 3*25 cm, 5 um | 30% IPA in Hex:DCM=3 : 1 (10 mM $NH_3$-MeOH) | 409 |
| 872 | 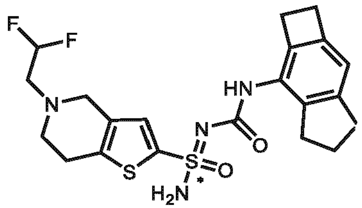 | (R) or (S) -5-(2,2-difluoroethyl)-N'-((2,4,5,6-tetrahydro-1H-cyclobuta[f]inden-3-yl) carbamoyl)-4,5,6,7-tetrahydrothieno[3,2-c] pyridine-2-sulfonimidamide | CHIRALPA K ID, 2*25 cm, 5 um | 10% IPA in Hex:DCM=3 : 1 (10 mM $NH_3$-MeOH) | 467 |
| 873 | 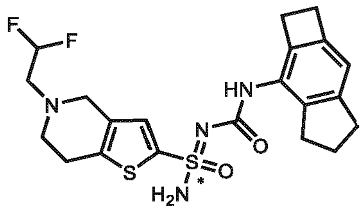 | (S) or (R) -5-(2,2-difluoroethyl)-N'-((2,4,5,6-tetrahydro-1H-cyclobuta[f]inden-3-yl) carbamoyl)-4,5,6,7-tetrahydrothieno[3,2-c] pyridine-2-sulfonimidamide | CHIRALPA K ID, 2*25 cm, 5 um | 10% IPA in Hex:DCM=3 : 1 (10 mM $NH_3$-MeOH) | 467 |
| 874 | 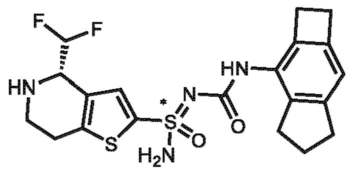 | (R or S,4S)-4-(difluoromethyl)-N'-((2,4,5,6-tetrahydro-1H-cyclobuta[f]inden-3-yl) carbamoyl)-4,5,6,7-tetrahydrothieno[3,2-c] pyridine-2-sulfonimidamide Resolved from Example 782 | CHIRALPA K IC, 2*25 cm, 5 um | 30% EtOH in Hex (0.1% FA) | 453 |

(continued)

| Ex. # | Structure | IUPAC Name | Column | Eluents | LC-MS $[M+H]^+$ |
|---|---|---|---|---|---|
| 875 | 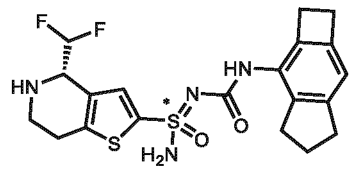 | (S or R,4S)-4-(difluoromethyl)-N'-((2,4,5,6-tetrahydro-1H-cyclobuta[f]inden-3-yl) carbamoyl)-4,5,6,7-tetrahydrothieno[3,2-c] pyridine-2-sulfonimidamide Resolved from Example 782 | CHIRALPA K IC, 2*25 cm, 5 um | 30% EtOH in Hex (0.1% FA) | 453 |
| 876 | 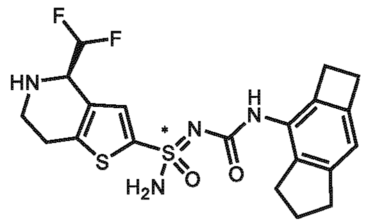 | (R or S,4R)-4-(difluoromethyl)-N'-((2,4,5,6-tetrahydro-1H-cyclobuta[f]inden-3-yl) carbamoyl)-4,5,6,7-tetrahydrothieno[3,2-c] pyridine-2-sulfonimidamide Resolved from Example 783 | CHIRALPA K IE, 2*25 cm, 5um | 30% EtOH in Hex (0.1% FA) | 453 |
| 877 | 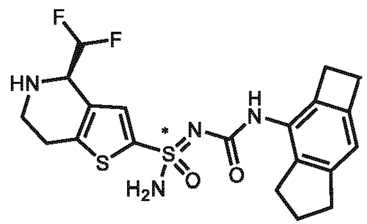 | (S or R,4R)-4-(difluoromethyl)-N'-((2,4,5,6-tetrahydro-1H-cyclobuta[f]inden-3-yl) carbamoyl)-4,5,6,7-tetrahydrothieno[3,2-c] pyridine-2-sulfonimidamide Resolved from Example 783 | CHIRALPA K IE, 2*25 cm, 5um | 30% EtOH in Hex (0.1% FA) | 453 |

**Table 76** Examples in the following table were prepared using similar conditions as described in Example **735** and Scheme **XX** from appropriate starting materials.

| Ex. # | Compound # | Structure | IUPAC Name | Exact Mass $[M+H]^+$ |
|---|---|---|---|---|
| 878 | **1100** | 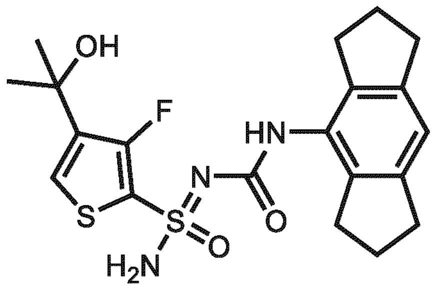 | 3-Fluoro-N'-((1,2,3,5,6,7-hexahydro-*s*-indacen-4-yl) carbamoyl)-4-(2-hydroxypropan-2-yl)thiophene-2-sulfonimidamide | 438 |
| 879 | **1101** | 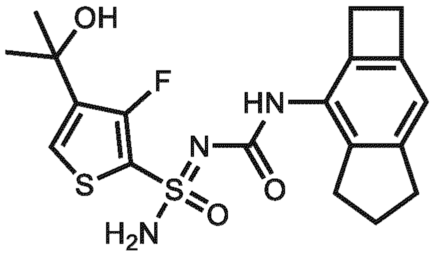 | 3-Fluoro-4-(2-hydroxypropan-2-yl)-N'-((2,4,5,6-tetrahydro-1H-cyclobuta[f]inden-3-yl)carbamoyl) thiophene-2-sulfonimidamide | 424 |

**Table 77** Examples in the following table were obtained from chiral HPLC resolutions of racemic examples described above. TheF chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative.

| Ex. # | Compound # | Structure | IUPAC Name | Column | Eluents | LC-MS $[M+H]^+$ |
|---|---|---|---|---|---|---|
| 880 | **1102** | 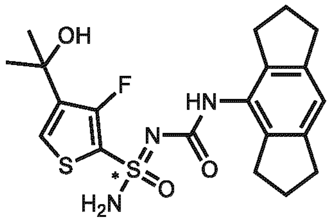 | (R) or (S)- 3-fluoro-N'-((1,2,3,5,6,7-hexahydro-s-indacen-4-yl)carbamoyl)-4-(2-hydroxypropan-2-yl)thiophene-2-sulfonimidamide | Chiralpak ID, 2*25 cm, 5 um | 50% IPA in Hex (0.1% FA) | 438 |
| 881 | **1103** | 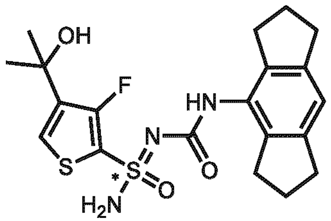 | (S) or (R)- 3-fluoro-N'-((1,2,3,5,6,7-hexahydro-s-indacen-4-yl)carbamoyl)-4-(2-hydroxypropan-2-yl)thiophene-2-sulfonimidamide | Chiralpak ID, 2*25 cm, 5 um | 50% IPA in Hex (0.1% FA) | 438 |
| 882 | **1104** | 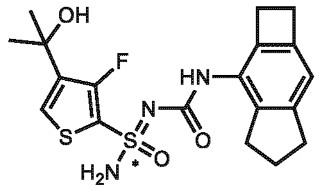 | (R) or (S)- 3-fluoro-4-(2-hydroxypropan-2-yl)-N'-((2,4,5,6-tetrahydro-1H-cyclobuta[f]inden-3-yl)carbamoyl)thiophene-2-sulfonimidamide | CHIRALPAK IF, 2*25 cm, 5 um | 20% EtOH in Hex (0.1%FA) | 424 |
| 883 | **1105** | 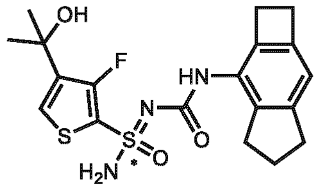 | (S) or (R)- 3-fluoro-4-(2-hydroxypropan-2-yl)-N'-((2,4,5,6-tetrahydro-1H-cyclobuta[f]inden-3-yl)carbamoyl)thiophene-2-sulfonimidamide | CHIRALPAK IF, 2*25 cm, 5 um | 20% EtOH in Hex (0.1%FA) | 424 |

The following protocol is suitable for testing the activity of the compounds disclosed herein.

**Procedure 1: IL-1β production in PMA-differentiated THP-1 cells stimulated with Gramicidin.**

THP-1 cells were purchased from the American Type Culture Collection and sub-cultured according to instructions from the supplier. Cells were cultured in complete RPMI 1640 (containing 10% heat inactivated FBS, penicillin (100 units/ml) and streptomycin (100 μg/ml)), and maintained in log phase prior to experimental setup. Prior to the experiment, compounds were dissolved in dimethyl sulfoxide (DMSO) to generate a 30mM stock. The compound stock was first pre-diluted in DMSO to 3, 0.34, 0.042 and 0.0083 mM intermediate concentrations and subsequently spotted using Echo550 liquid handler into an empty 384-well assay plate to achieve desired final concentration (e.g. 100, 33, 11, 3.7, 1.2, 0.41, 0.14, 0.046, 0.015, 0.0051, 0.0017 μM). DMSO was backfilled in the plate to achieve a final DMSO assay concentration of 0.37%. The plate was then sealed and stored at room temperature until required.

THP-1 cells were treated with PMA (Phorbol 12-myristate 13-acetate) (20 ng/ml) for 16-18 hours. On the day of the experiment the media was removed and adherent cells were detached with trypsin for 5 minutes. Cells were then harvested, washed with complete RPMI 1640, spun down, and resuspended in RPMI 1640 (containing 2% heat inactivated FBS, penicillin (100 units/ml) and streptomycin (100 μg/ml) . The cells were plated in the 384-well assay plate containing the spotted compounds at a density of 50,000 cells/well (final assay volume 50 μl). Cells were incubated with compounds for 1 hour and then stimulated with gramicidin (5μM) (Enzo) for 2 hours. Plates were then centrifuged at 340g for 5 min. Cell free supernatant (40μL) was collected using a 96-channel PlateMaster (Gilson) and the production of IL-1β was evaluated by HTRF (cisbio). The plates were incubated for 18 h at 4°C and read using the preset HTRF program (donor emission at 620 nm, acceptor emission at 668 nm) of the SpectraMax i3x spectrophotometer (Molecular Devices, software SoftMax 6). A vehicle only control and a dose titration of CRID3 (100 - 0.0017 μM) were run concurrently with each experiment. Data was normalized to vehicle-treated samples (equivalent to 0% inhibition) and CRID3 at 100 μM (equivalent to 100% inhibition). Compounds exhibited a concentration-dependent inhibition of IL-1β production in PMA-differentiated THP-1 cells.

**Procedure 2: IL-1β production in PMA-differentiated THP-1 cells stimulated with Gramicidin.**

THP-1 cells were purchased from the American Type Culture Collection and sub-cultured according to instructions from the supplier. Prior to experiments, cells were cultured in complete RPMI 1640 (containing 10% heat inactivated FBS, penicillin (100 units/ml) and streptomycin (100 μg/ml)), and maintained in log phase prior to experimental setup. Prior to the experiment THP-1 were treated with PMA (Phorbol 12-myristate 13-acetate) (20 ng/ml) for 16-18 hours. Compounds were dissolved in dimethyl sulfoxide (DMSO) to generate a 30mM stock. On the day of the experiment the media was removed and adherent cells were detached with trypsin for 5 minutes. Cells were then harvested, washed with complete RPMI 1640, spun down, resuspended in RPMI 1640 (containing 2% heat inactivated FBS, penicillin (100 units/ml) and streptomycin (100 μg/ml) . The cells were plated in a 384-well plate at a density of 50,000 cells/well (final assay volume 50 μl). Compounds were first dissolved in assay medium to obtain a 5x top concentration of 500μM. 10 step dilutions (1:3) were then undertaken in assay medium containing 1.67% DMSO. 5x compound solutions were added to the culture medium to achieve desired final concentration (e.g. 100, 33, 11, 3.7, 1.2, 0.41, 0.14, 0.046, 0.015, 0.0051, 0.0017 μM). Final DMSO concentration was at 0.37%. Cells were incubated with compounds for 1 hour and then stimulated with gramicidin (5μM) (Enzo) for 2 hours. Plates were then centrifuged at 340g for 5 min. Cell free supernatant (40μL) was collected using a 96-channel PlateMaster (Gilson) and the production of IL-1β was evaluated by HTRF (cisbio). A vehicle only control and a dose titration of CRID3 (100 - 0.0017 μM) were run concurrently with each experiment. Data was normalized to vehicle-treated samples (equivalent to 0% inhibition) and CRID3 at 100 μM (equivalent to 100% inhibition). Compounds exhibited a concentration-dependent inhibition of IL-1β production in PMA-differentiated THP-1 cells.

**Procedure 3**

**1. Experimental procedure**

**1.1 Cell Culture**

1) Culture THP-1 cells in the complete RPMI-1640 medium with 10% FBS at 37°C, 5% $CO_2$.

2) Passage the cells every 3 days by inoculating $3\times10^5$ cells per ml.

### 1.2 Compound Preparation

Prepare the 3-fold serial dilution of the compounds with DMSO in a 384-well LDV Microplate using TECAN EVO system to generate the compound source plate with 10 concentrations. Top concentration is 30 mM. FIG. 3 depicts the layout of the microplate.

### 1.3 Cell preparation

1) Centrifuge THP-1 cells at 350g for 5 min.

2) Re-suspend cells with complete RMPI-1640 medium, and count cells.

3) Seed cells in T225 flask, about $2.5\times10^7$ per flask, treat cells with 20ng/ml PMA (final DMSO concentration< 1%).

4) Incubate overnight.

### 1.4 THP-1 Stimulation

1) Wash adherent THP-1 cells with PBS, and detach cells with 4ml trypsin for T225 flask.

2) Centrifuge cells at 350g for 5 min, re-suspend cells with RPMI-1640 containing 2% FBS and count cells with trypan blue.

3) Transfer 50 nl/well the serial dilution of test compound to 384-well plate by Echo; For the high control and first point of CRID3 (MCC950), transfer 165 nl, then backfill to make the DMSO concentration is consistent in all wells, the plate layout is as below.

4) Seed 50k cells in 40ul RPMI-1640 with 2% FBS per well in 384-well plate.

5) Incubate for 1h at 37°C, 5% $CO_2$.

6) Prepare 5x gramicidin, add 10 μl per well, the final concentration is 5 μM, incubate for 2hrs at 37°C, 5% $CO_2$.

7) Centrifuge at 350 g for 1 min.

8) Pipet 16μl supernatant by apricot, and transfer into white 384 proxiplate. FIG. 3 depicts the layout of the plates: HC: 100 μM CRID3 (MCC950) + 5 μM gramicidin LC : 5 μM Gramicidin.

### 1.5 IL-1β detection

1) Homogenize the 5x diluent #5 with a vortex and add 1 volume of stock solution in 4 volumes of distilled water.

2) Thaw 20x stock solution of anti-IL1β-Cryptate-antibody and anti-IL1β XL-antibody. Dilute these two antibodies to 1x with detection buffer #3.

3) Pre-mix the two ready-to-use antibody solutions just prior to use.

4) Dispense 4ul of pre-mixed Anti-IL1β antibodies working solution into all wells.

5) Seal the plate and incubate overnight at 4 °C.

6) Read the cell plate using EnVison and plot Readout vs. the test compound concentration to calculate the $IC_{50}$.

2. Data Analysis:

1. $IC_{50}$ of compounds can be calculated using the following formulas
Formula for $IC_{50}$

$$\% \text{ inhibition} = 100 - 100 \text{ x } [HC_{ave} - \text{Readout} / (HC_{ave} - LC_{ave})]$$

2. Fit the normalized data in a dose-response manner using XLfit, and calculate the compound concentration. Table B2 shows the biological activity of compounds in hTHP-1 assay containing 2% fetal bovine serum. <0.008 μM = "++++++"; ≥0.008 and <0.04 μM = "+++++"; ≥0.04 and <0.2 μM = "++++"; ≥0.2 and <1 μM = "+++"; ≥1 and <5 μM = "++"; ≥5 and <30 μM = "+".

**Table B2.**

| Example # | Compound # | $IC_{50}$ (uM) | Example # | Compound # | $IC_{50}$ (uM) |
|---|---|---|---|---|---|
| 476 | 501 | +++ | 649 | 847a | + |
| 546 | 501b | +++ | | 818b | +++ |
| 547 | 501a | ++ | | 875 | |

**Study Example 1.**

The CARD8 gene is located within the inflammatory bowel disease (IBD) 6 linkage region on chromosome 19. CARD8 interacts with NLRP3, and Apoptosis-associated Speck-like protein to form a caspase-1 activating complex termed the NLRP3 inflammasome. The NLRP3 inflammasome mediates the production and secretion of interleukin-1β, by processing pro-IL-1β into mature secreted IL-1β. In addition to its role in the inflammasome, CARD8 is also a potent inhibitor of nuclear factor NF-κB. NF-κB activation is essential for the production of pro-IL-1β. Since over-production of IL-1β and dyregulation of NF-κB are hallmarks of Crohn's disease, CARD8 is herein considered to be a risk gene for inflammatory bowel disease. A significant association of CARD8 with Crohn's disease was detected in two British studies with a risk effect for the minor allele of the non-synonymous single-nucleotide polymorphism (SNP) of a C allele at rs2043211. This SNP introduces a premature stop codon, resulting in the expression of a severely truncated protein. This variant CARD8 protein is unable to suppress NF-κB activity, leading to constitutive production of pro-IL-ββ , which is a substrate for the NLRP3 inflammasome. It is believed that a gain-of-function mutation in an NLRP3 gene (e.g., any of the gain-of-function mutations described herein, e.g., any of the gain-of-function mutations in an NLRP3 gene described herein) combined with a loss-of-function mutation in a CARD8 gene (e.g., a C allele at rs2043211) results in the development of diseases related to increased NLRP3 inflammasome expression and/or activity. Patients having, e.g., a gain-of-function mutation in an NLRP3 gene and/or a loss-of-function mutation in a CARD8 gene are predicted to show improved therapeutic response to treatment with an NLRP3 antagonist.

A study is designed to determine: whether NLRP3 antagonists inhibit inflammasome function and inflammatory activity in cells and biopsy specimens from patients with Crohn's disease or ulcerative colitis; and whether the specific genetic variants identify patients with Crohn's disease or ulcerative colitis who are most likely to respond to treatment with an NLRP3 antagonist.

The secondary objectives of this study are to: determine if an NLRP3 antagonist reduces inflammasome activity in Crohn's disease and ulcerative biopsy samples (comparing Crohn's disease and ulcerative colitis results with control patient results); determine if an NLRP3 antagonist reduced inflammatory cytokine RNA and protein expression in Crohn's disease and ulcerative colitis samples; determine if baseline (no ex vivo treatment) RNA levels of NLRP3, ASC, and IL-1β are greater in biopsy samples from patients with anti-TNFα agent resistance status; and stratify the results according to presence of specific genetic mutations in genes encoding ATG16L1, NLRP3, and CARD8 (e.g., any of the mutations in the ATG16L1 gene, NLRP3 gene, and CARD8 gene described herein).

***Methods***

- Evaluation of baseline expression of NLRP3 RNA and quantify inhibition of inflammasome activity by an NLRP3 antagonist in biopsies of disease tissue from patients with Crohn's disease and ulcerative colitis.
- Determine if NLRP3 antagonist treatment reduces the inflammatory response in biopsies of disease from patients with Crohn's disease based on decreased expression of inflammatory gene RNA measured with Nanostring.
- Sequence patient DNA to detect specific genetic mutations in the ATG16L1 gene, NLRP3 gene, and CARD8 gene (e.g., any of the exemplary mutations in these genes described herein) and then stratify the results of functional assays according to the presence of these genetic mutations.

***Experimental Design***

- Human subjects and tissue:
  Endoscopic or surgical biopsies from areas of disease in patients with Crohn's disease and ulcerative colitis who are either anti-TNFα treatment naive or resistant to anti-TNFα treatment; additionally biopsies from control patients (surveillance colonoscopy or inflammation-free areas from patients with colorectal cancer) are studied.

- Ex vivo Treatment Model:
  Organ or LPMC culture as determined appropriate

- Endpoints to be measured:

  *Before ex vivo treatment*-- NLRP3 RNA level
  *After ex vivo treatment*- inflammasome activity (either processed IL-1β, processed caspase-1, or secreted IL-1β); RNA for inflammatory cytokines (Nanostring); viable T cell number and/or T cell apoptosis.

- Data Analysis Plan:

  - Determine if NLRP3 antagonist treatment decreases processed IL-1β, processed caspase-1 or secreted IL-1β, and inflammatory cytokine RNA levels.
  - Stratify response data according to treatment status at biopsy and the presence of genetic mutations in the NLRP3 gene, CARD8 gene, and ATG16L1 gene (e.g., any of the exemplary genetic mutations of these genes described herein).

**Study Example 2. Treatment of anti-TNFα resistant patients with NLRP3 antagonists**

PLoS One 2009 Nov 24;4(11):e7984, describes that mucosal biopsies were obtained at endoscopy in actively inflamed mucosa from patients with Ulcerative Colitis, refractory to corticosteroids and/or immunosuppression, before and 4-6 weeks after their first infliximab (an anti-TNFα agent) infusion and in normal mucosa from control patients. The patients in this study were classified for response to infliximab based on endoscopic and histologic findings at 4-6 weeks after first infliximab treatment as responder or non-responder. Transcriptomic RNA expression levels of these biopsies were accessed by the inventors of the invention disclosed herein from GSE 16879, the publically available Gene Expression Omnibus (https://www.ncbi.nlm.nih.gov/geo/geo2r/?acc=GSE16879). Expression levels of RNA encoding NLRP3 and IL-1β were determined using GEO2R (a tool available on the same website), based on probe sets 207075_at and 205067_at, respectively. It was surprisingly found that in Crohn's disease patients that are non-responsive to the infliximab (an anti-TNFα agent) have higher expression of NLRP3 and IL-1β RNA than responsive patients (Figs. 4 and 5). Similar surprising results of higher expression of NLRP3 and IL-1β RNA in UC patients that are non-responsive to infliximab (an anti-TNFα agent) compared to infliximab (an anti-TNFα agent) responsive patients (Figs. 6 and 7) were found.

Said higher levels of NLRP3 and IL-1β RNA expression levels in anti-TNFα agent non-responders, is hypothesised herein to lead to NLRP3 activation which in turns leads ot release of IL-1β that induces IL-23 production, leading to said resistance to anti-TNFα agents. Therefore, treatment of Crohn's and UC anti-TNFα non-responders with an NLRP3 antagonist would prevent this cascade, and thus prevent development of non-responsiveness to anti-TNFα agents. Indeed, resistance to anti-TNFα agents is common in other inflammatory or autoimmune diseases. Therefore, use of an NLRP3 antagonist for the treatment of inflammatory or autoimmune diseases will block the mechanism leading to non-responsiveness to anti-TNFα□agents. Consequently, use of NLRP3 antagonists will increase the sensitivity of patients with inflammatory or autoimmune diseases to anti-TNFα agents, resulting in a reduced dose of anti-TNFα agents for the treatment of these diseases. Therefore, a combination of an NLRP3 antagonist and an anti-TNFα agent can be

used in the treatment of diseases wherein TNFα is overexpressed, such as inflammatory or autoimmune diseases, to avoid such non-responsive development of patients to anti-TNFα agents. Preferably, this combination threatment can be used in the treatment of IBD, for example Crohn's disease and UC.

Further, use of NLRP3 antagonists offers an alternative to anti-TNFα agents for the treatment of diseases wherein TNFα is overexpressed. Therefore, NLRP3 antagonists offers an alternative to anti-TNFα agents inflammatory or autoimmune diseases, such as IBD (e.g. Crohn's disease and UC).

Systemtic anti-TNFα agents are also known to increase the risk of infection. Gut restricted NLRP3 antagonists, however, offers a gut targeted treatment (i.e. non-systemic treatment), preventing such infections. Therefore, treatment of TNFα gut diseases, such as IBD (i.e. Crohn's disease and UC), with gut restricted NLRP3 antagonists has the additional advantage of reducing the risk of infection compared to anti-TNFα agents.

**Proposed Experiment:**

Determine the expression of NLRP3 and caspase-1 in LPMCs and epithelial cells in patients with non-active disease, in patients with active disease, in patients with active disease resistant to corticosteroids, patients with active disease resistant to TNF-blocking agents. The expression of NLRP3 and caspase-1 in LPMCs and epithelial cells will be analyzed by RNAScope technology. The expression of active NLRP3 signature genes will be analyzed by Nanostring technology. A pilot analysis to determine feasibility will be performed with 5 samples from control, 5 samples from active CD lesions, and 5 samples from active UC lesions.

**Study Example 3.**

It is presented that NLRP3 antagonists reverse resistance to anti-TNF induced T cell depletion/apoptosis in biopsy samples from IBD patients whose disease is clinically considered resistant or unresponsive to anti-TNF therapy.

A study is designed to determine: whether NLRP3 antagonists inhibit inflammasome function and inflammatory activity in cells and biopsy specimens from patients with Crohn's disease or ulcerative colitis; and whether an NLRP3 antagonist will synergize with anti-TNFα therapy in patients with Crohn's disease or ulcerative colitis.

The secondary objectives of this study are to: determine if an NLRP3 antagonist reduces inflammasome activity in Crohn's disease and ulcerative biopsy samples (comparing Crohn's disease and ulcerative colitis results with control patient results); determine if an NLRP3 antagonist reduced inflammatory cytokine RNA and protein expression in Crohn's disease and ulcerative colitis samples; determine if an NLRP3 antagonist in the absence of co-treatment with anti-TNFα antibody induces T cell depletion in Crohn's disease and ulcerative colitis biopsy samples; and determine if baseline (no ex vivo treatment) RNA levels of NLRP3, ASC, and IL-10 are greater in biopsy samples from patients with anti-TNFα agent resistance status.

***Methods***

- Evaluation of baseline expression of NLRP3 RNA and quantify inhibition of inflammasome activity by an NLRP3 antagonist in biopsies of disease tissue from patients with Crohn's disease and ulcerative colitis.
- Determine if there is synergy between an NLRP3 antagonist and anti-TNF antibody with respect to effects on T cell depletion/apoptosis in biopsies of disease from patients with Crohn's disease and ulcerative colitis.
- Determine if NLRP3 antagonist treatment reduces the inflammatory response in biopsies of disease from patients with Crohn's disease based on decreased expression of inflammatory gene RNA measured with Nanostring.

***Experimental Design***

- Human subjects and tissue:
  Endoscopic or surgical biopsies from areas of disease in patients with Crohn's disease and ulcerative colitis who are either anti-TNFα treatment naive or resistant to anti-TNFα treatment; additionally biopsies from control patients (surveillance colonoscopy or inflammation-free areas from patients with colorectal cancer) are studied.

- Ex vivo Treatment Model:
  Organ or LPMC culture as determined appropriate

- Ex vivo Treatments:
  NLRP3 antagonist (2 concentrations), negative control (vehicle), positive control (caspase-1 inhibitor) each in the presence or absence of anti-TNF antibody at a concentration appropriate to distinguish differences in the T cell apoptotic between biopsies from anti-TNF resistant and anti-TNF-sensitive Crohn's disease patients. Each treatment condition is evaluated in a minimum in duplicate samples.

- Endpoints to be measured:

  *Before ex vivo treatment*-- NLRP3 RNA level
  *After ex vivo treatment*- inflammasome activity (either processed IL-1β, processed caspase-1, or secreted IL-1β); RNA for inflammatory cytokines (Nanostring); viable T cell number and/or T cell apoptosis.

- Data Analysis Plan:

  - Determine if NLRP3 antagonist co-treatment increases T cell apoptosis/deletion in response to anti-TNF.
  - Determine if the level of NLRP3 RNA expression is greater in TNF-resistant Crohn's disease and ulcerative colitis samples compared to anti-TNF treatment-naive samples.
  - Determine if NLRP3 antagonist treatment decreases processed IL-1β, processed caspase-1 or secreted IL-1β, and inflammatory cytokine RNA levels.

**Biological Assay** - Nigericin-stimulated IL-1β secretion assay in THP-1 cells

Monocytic THP-1 cells (ATCC: TIB-202) were maintained according to providers' instructions in RPMI media (RPMI/Hepes +10% fetal bovine serum + Sodium Pyruvate + 0.05 mM Beta-mercaptoethanol (1000x stock) + Pen-Strep). Cells were differentiated in bulk with 0.5 μM phorbol 12-myristate 13-acetate (PMA; Sigma # P8139) for 3 hours, media was exchanged, and cells were plated at 50,000 cells per well in a 384-well flat-bottom cell culture plates (Greiner, #781986), and allowed to differentiate overnight. Compound in a 1:3.16 serial dilution series in DMSO was added 1:100 to the cells and incubated for 1 hour. The NLRP3 inflammasome was activated with the addition of 15 μM (final concentration) Nigericin (Enzo Life Sciences, #BML-CA421-0005), and cells were incubated for 3 hours. 10 μL supernatant was removed, and IL-1β levels were monitored using an HTRF assay (CisBio, #62II,1PEC) according to manufacturers' instructions. Viability and pyroptosis was monitored with the addition of PrestoBlue cell viability reagent (Life Technologies, #A13261) directly to the cell culture plate.

## Claims

**1.** A compound selected from the group consisting of the compounds below:

| | | | |
|---|---|---|---|
| 501 | 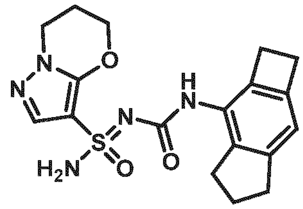 | 618 | 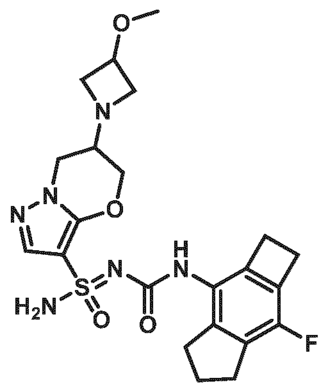 |

(continued)

| | | | |
|---|---|---|---|
| 502 | 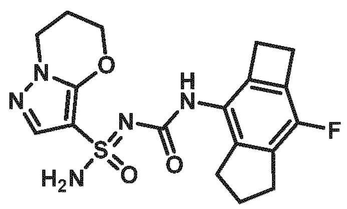 | 619 | 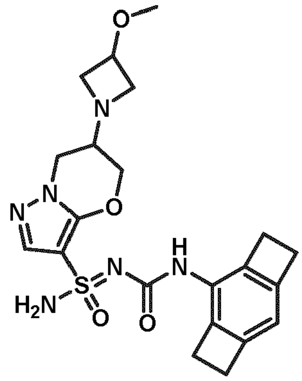 |
| 503 | 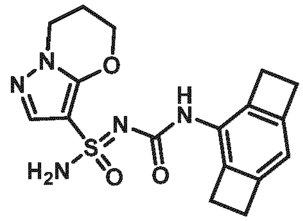 | 620 | 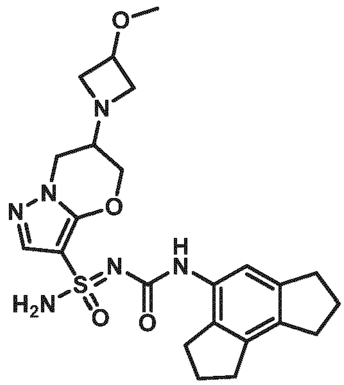 |
| 504 | 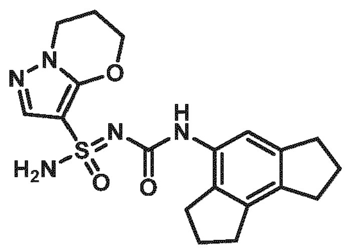 | 621 | 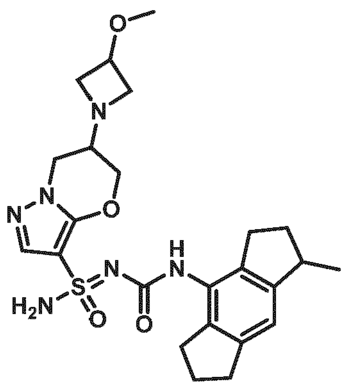 |
| 505 | 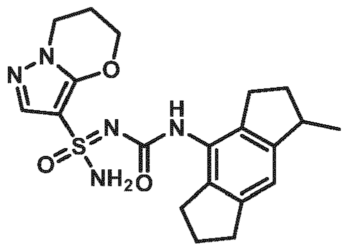 | 622 | 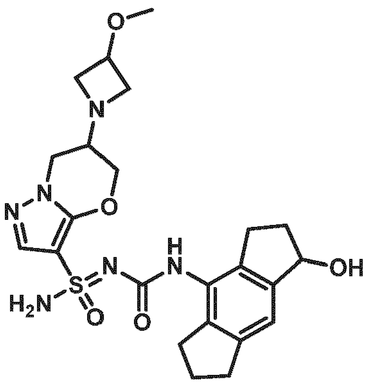 |

(continued)

| | | | |
|---|---|---|---|
| 506 | 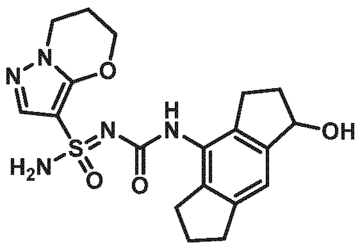 | 623 | 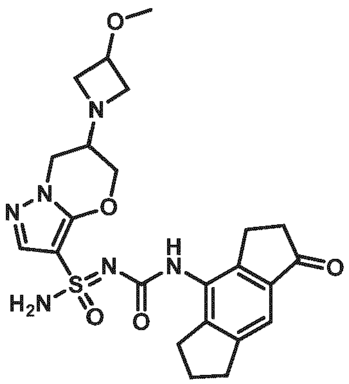 |
| 507 | 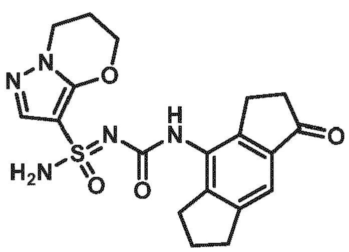 | 624 | 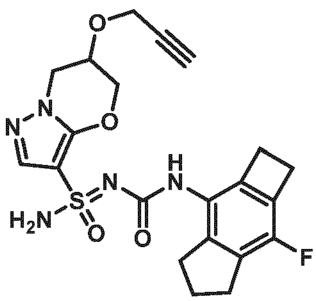 |
| 508 | 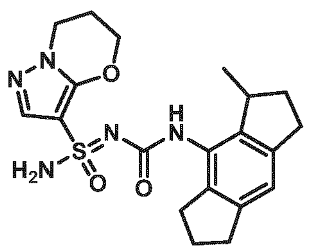 | 625 | 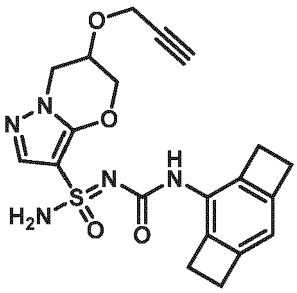 |
| 509 | 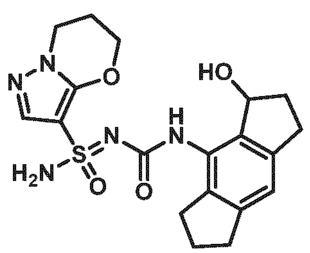 | 626 | 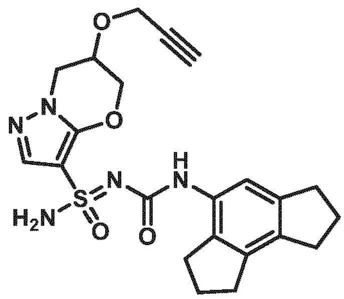 |
| 510 | 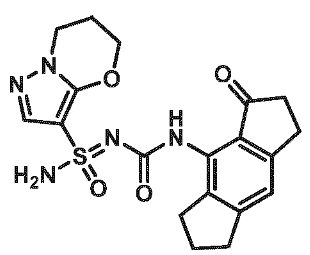 | 627 | 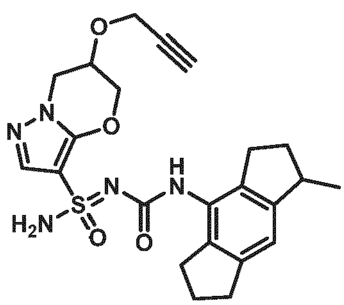 |

(continued)

| | | | |
|---|---|---|---|
| 511 | 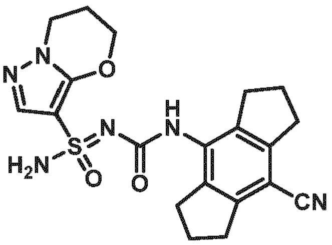 | 628 | 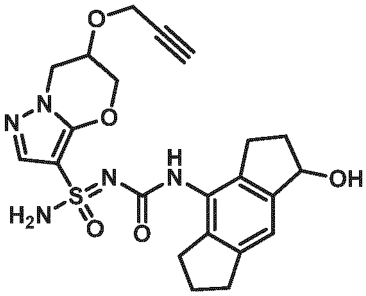 |
| 512 | 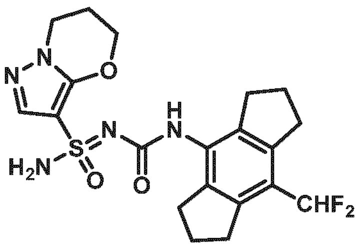 | 629 | 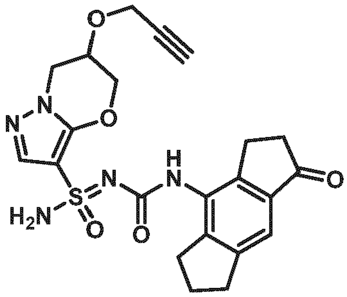 |
| 513 | 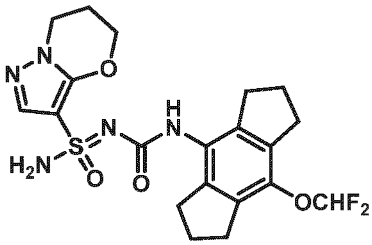 | 630 | 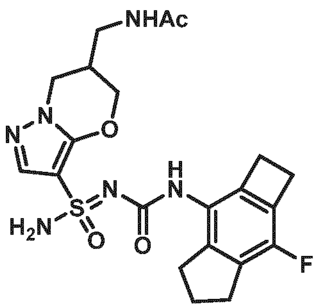 |
| 514 | 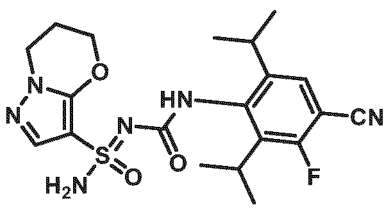 | 631 | 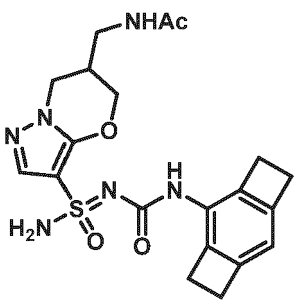 |
| 515 | 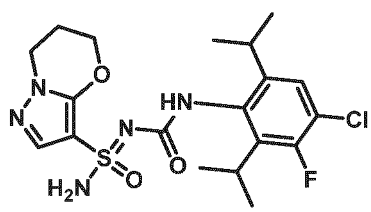 | 632 | 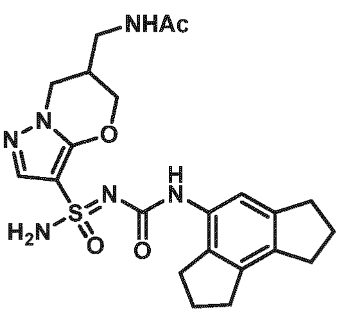 |

(continued)

| | | | |
|---|---|---|---|
| 516 | 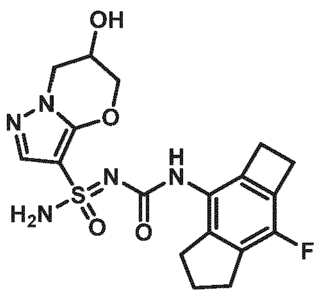 | 633 | 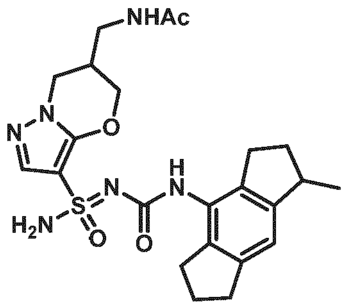 |
| 517 | 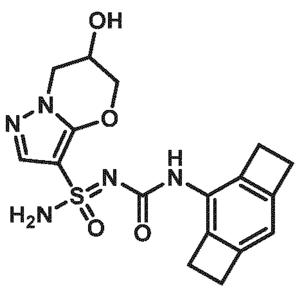 | 634 | 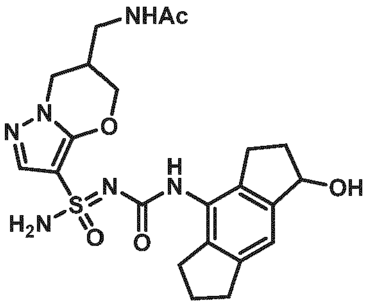 |
| 518 | 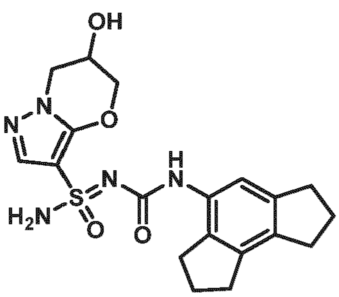 | 635 | 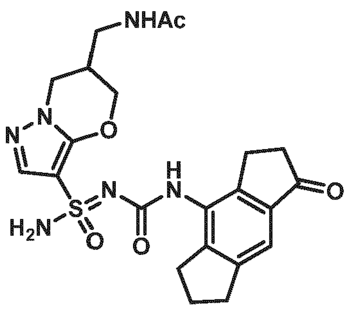 |
| 519 | 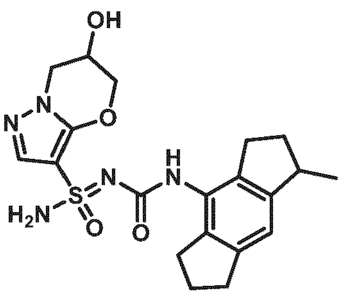 | 636 | 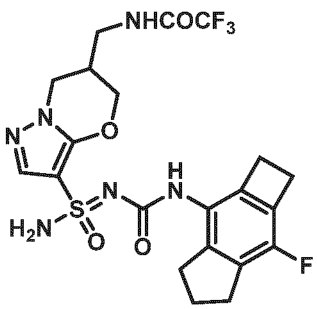 |
| 520 | 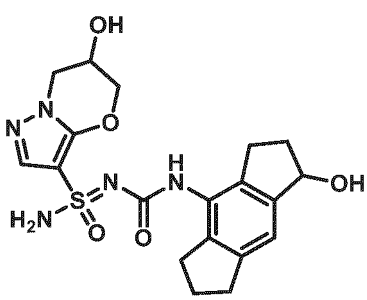 | 637 | 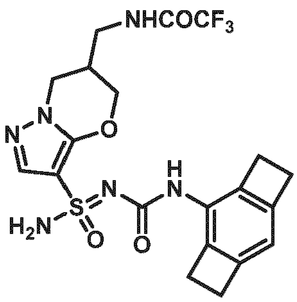 |

(continued)

| | | | |
|---|---|---|---|
| 521 | 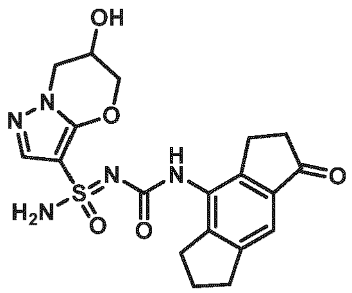 | 638 | 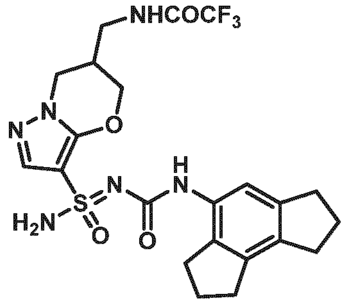 |
| 522 | 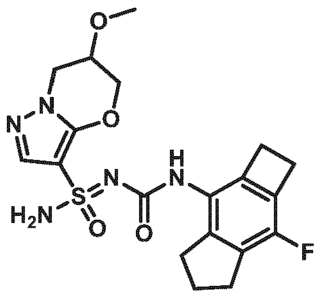 | 639 | 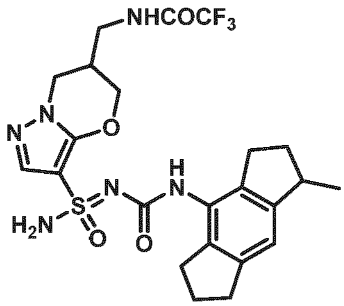 |
| 523 | 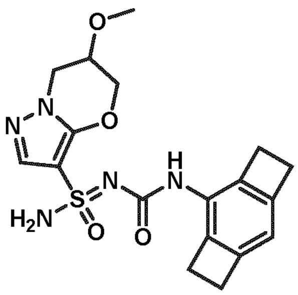 | 640 | 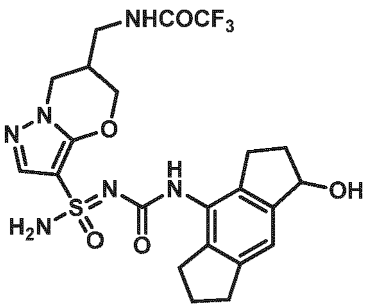 |
| 524 | 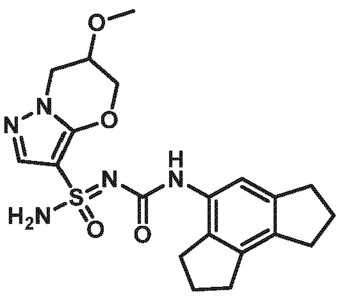 | 641 | 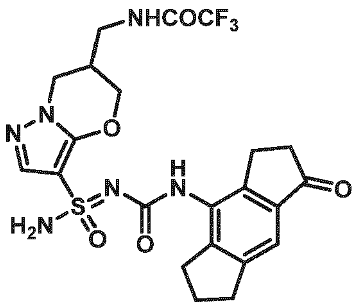 |
| 525 | 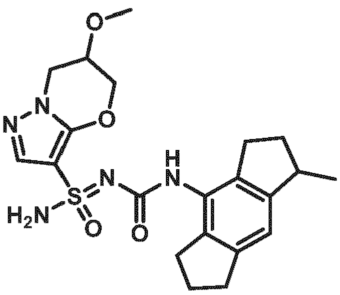 | 642 | 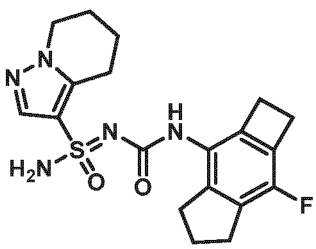 |

(continued)

| | | | |
|---|---|---|---|
| 526 | 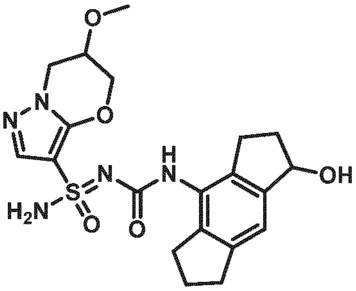 | 643 | 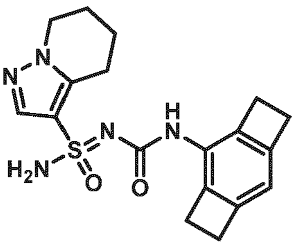 |
| 527 | 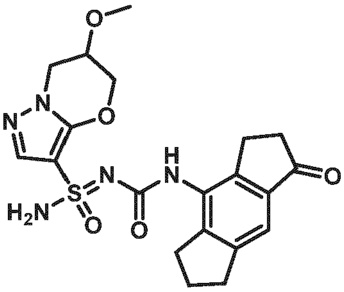 | 644 | 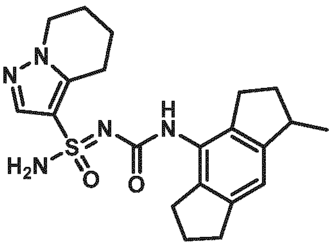 |
| 528 | 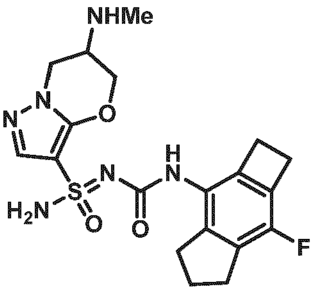 | 645 | 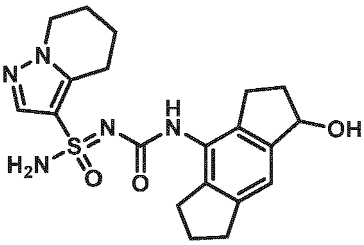 |
| 529 | 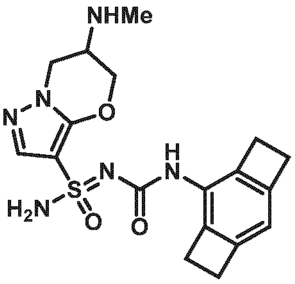 | 646 | 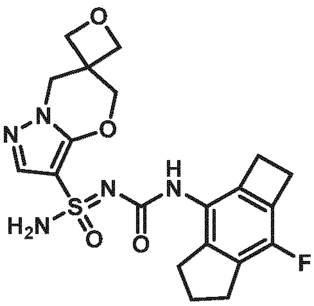 |
| 530 | 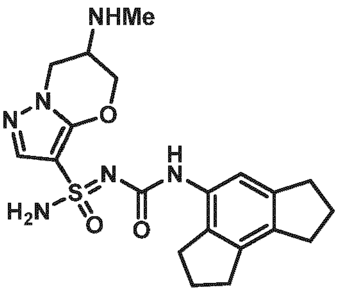 | 647 | 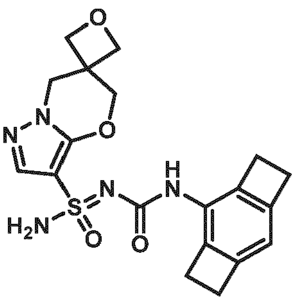 |

(continued)

| | | | |
|---|---|---|---|
| 531 | 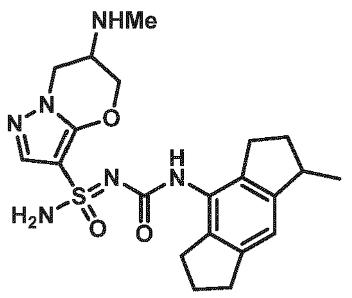 | 648 | 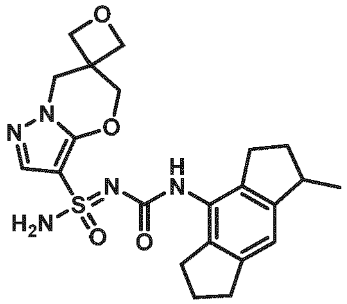 |
| 532 | 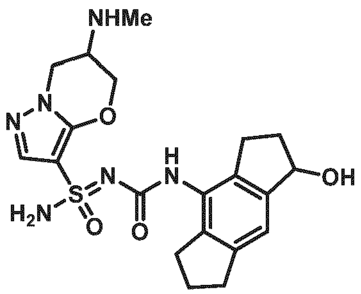 | 649 | 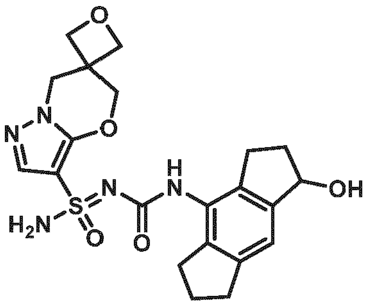 |
| 533 | 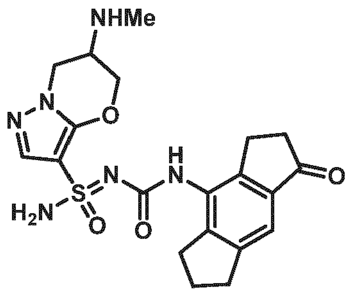 | 650 | 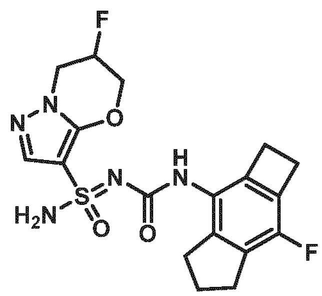 |
| 534 | 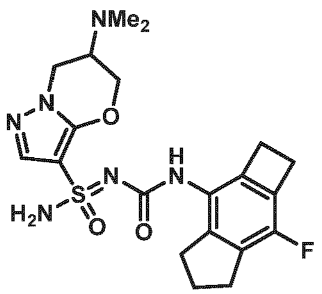 | 651 | 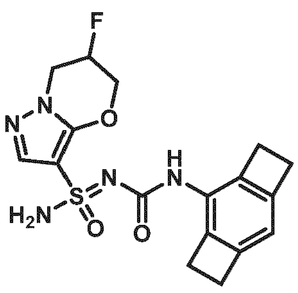 |
| 535 | 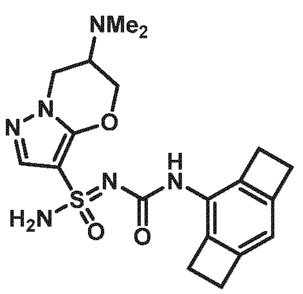 | 652 | 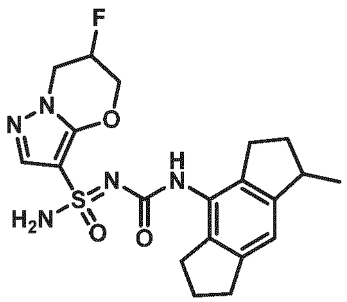 |

(continued)

| | | | |
|---|---|---|---|
| 536 | 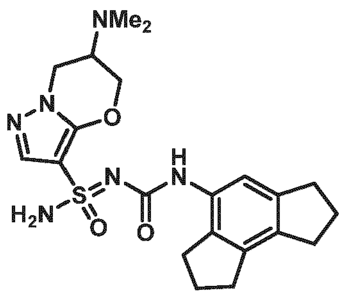 | 653 | 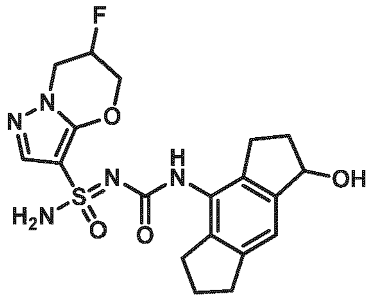 |
| 537 | 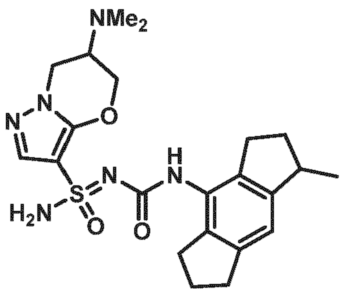 | 654 | 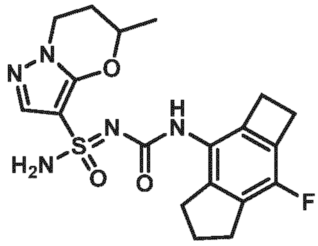 |
| 538 | 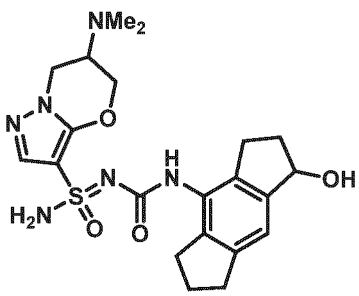 | 655 | 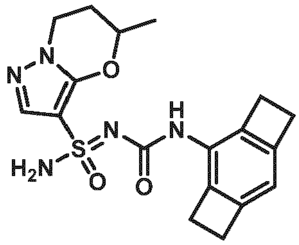 |
| 539 | 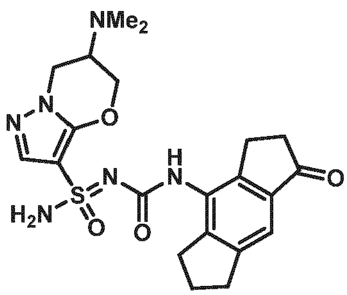 | 656 | 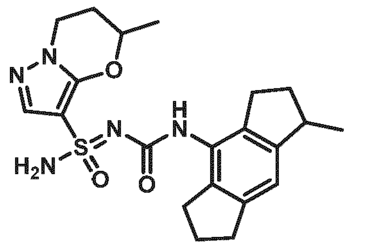 |
| 540 | 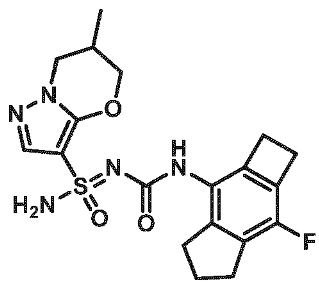 | 657 | 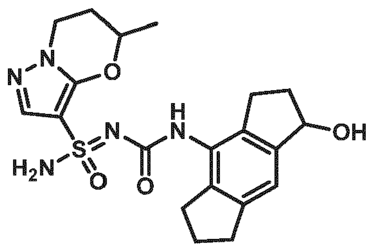 |

(continued)

| | | | |
|---|---|---|---|
| 541 | 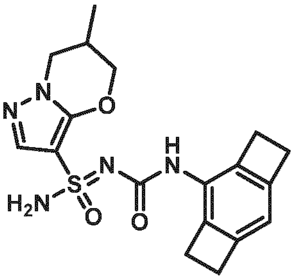 | 658 | 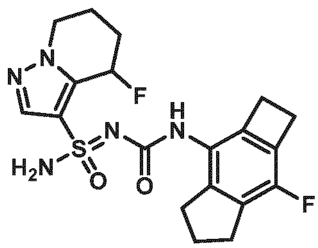 |
| 542 | 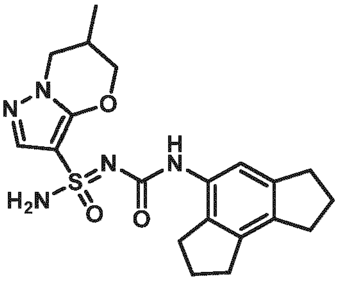 | 659 | 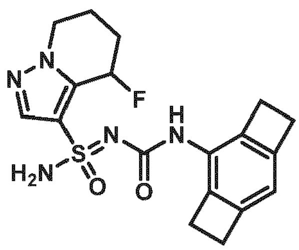 |
| 543 | 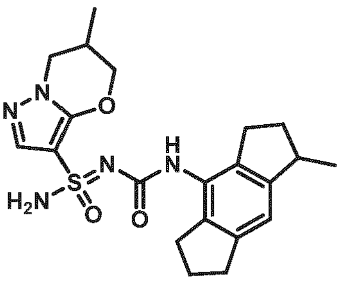 | 660 | 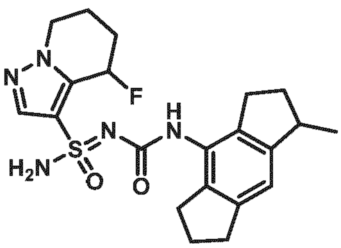 |
| 544 | 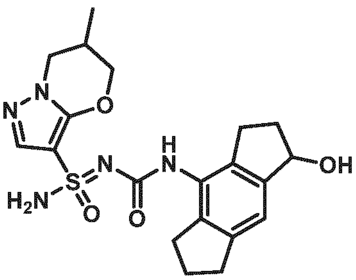 | 661 | 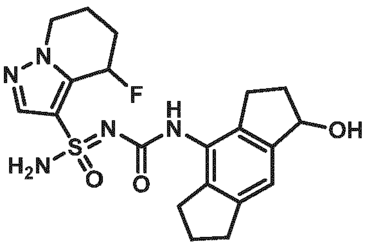 |
| 545 | 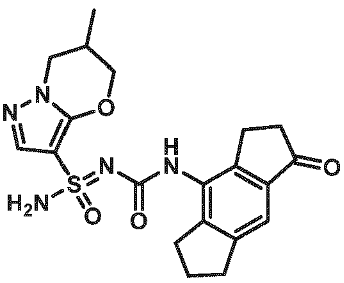 | 662 | 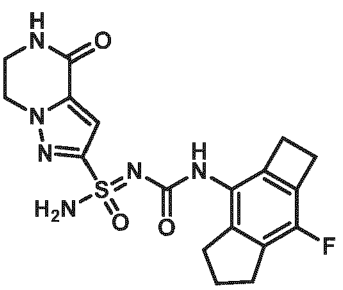 |

(continued)

| | | | |
|---|---|---|---|
| 546 | 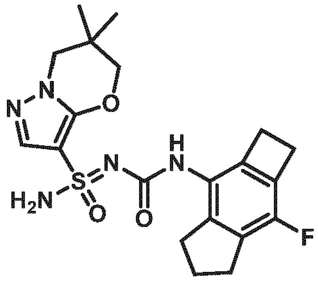 | 663 | 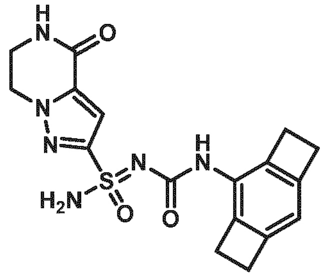 |
| 547 | 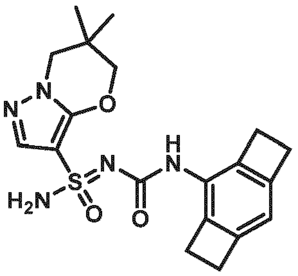 | 664 | 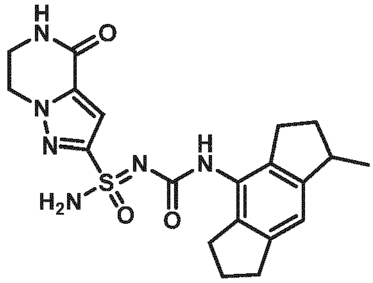 |
| 548 | 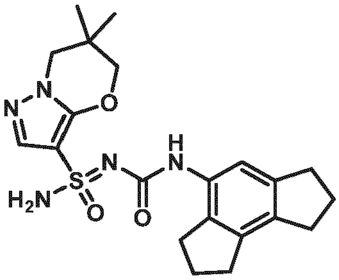 | 665 | 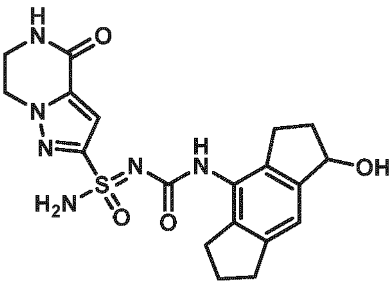 |
| 549 | 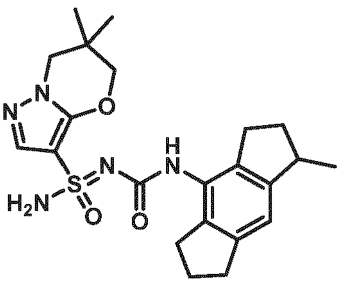 | 666 | 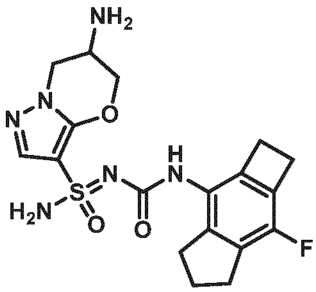 |
| 550 | 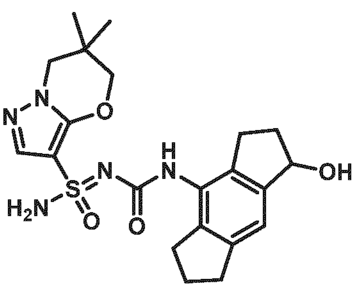 | 667 | 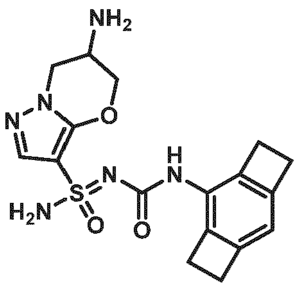 |

(continued)

| | | | |
|---|---|---|---|
| 551 | 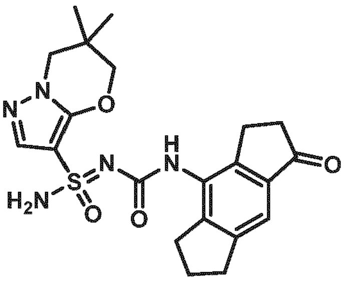 | 668 | 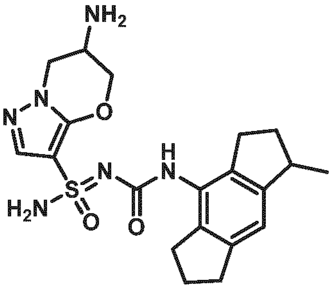 |
| 552 | 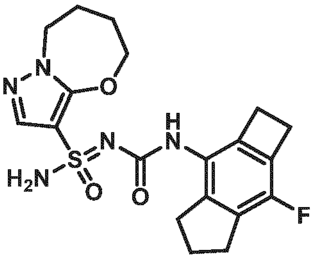 | 669 | 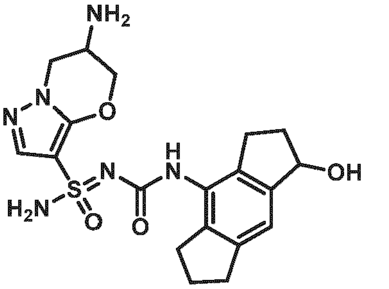 |
| 553 | 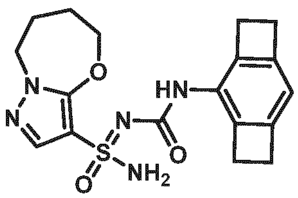 | 670 | 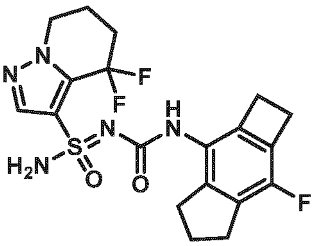 |
| 554 | 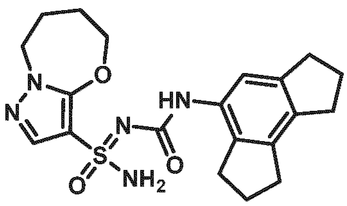 | 671 | 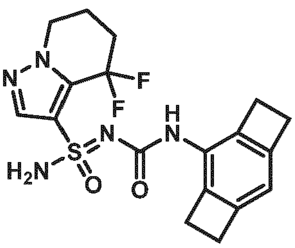 |
| 555 | 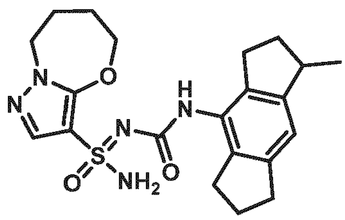 | 672 | 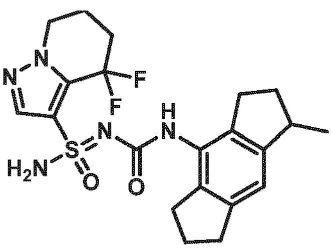 |
| 556 | 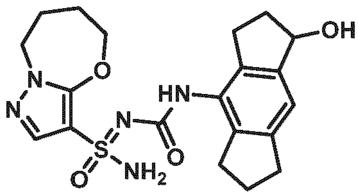 | 673 | 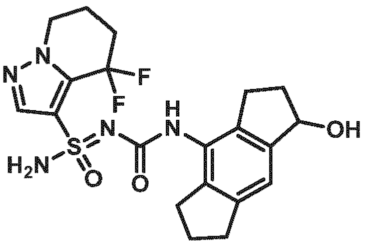 |

(continued)

| | | | |
|---|---|---|---|
| 557 | 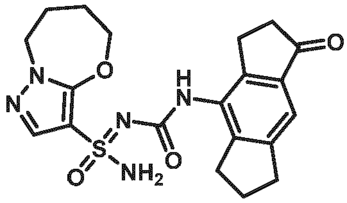 | 674 | 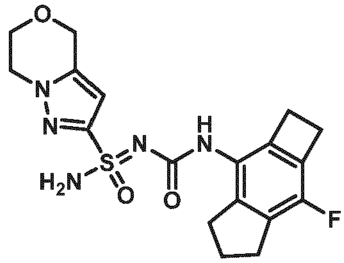 |
| 558 | 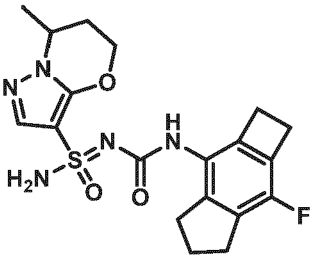 | 675 | 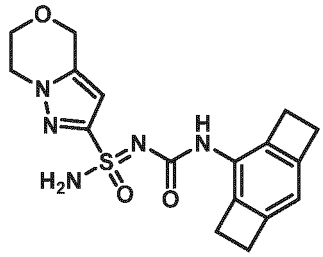 |
| 559 | 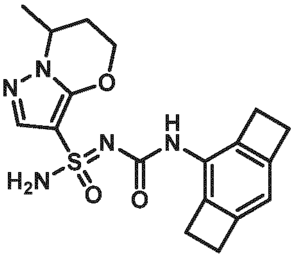 | 676 | 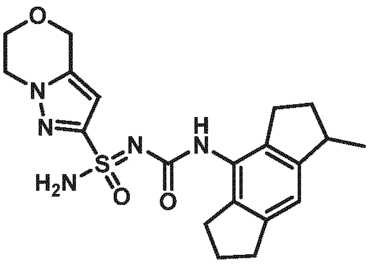 |
| 560 | 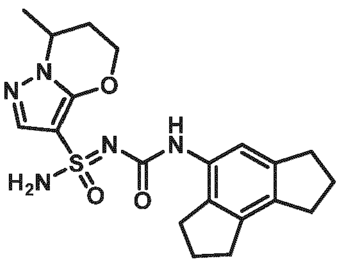 | 677 | 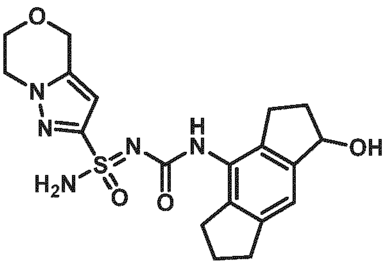 |
| 561 | 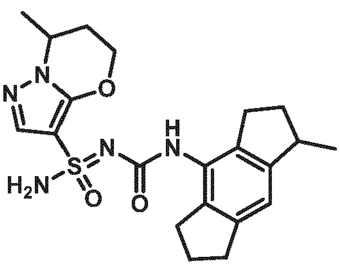 | 678 | 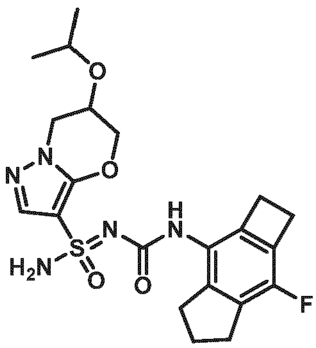 |

(continued)

| | | | |
|---|---|---|---|
| 562 | 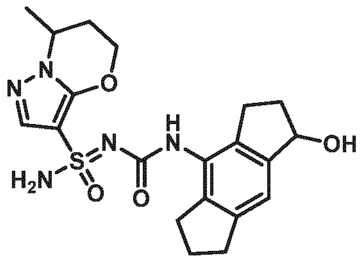 | 679 | 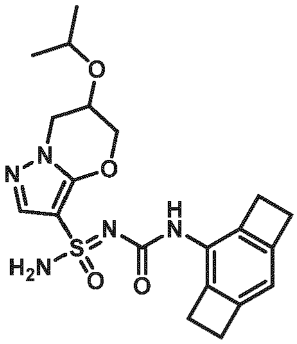 |
| 563 | 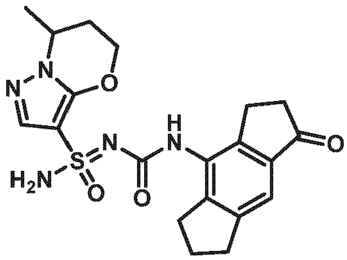 | 680 | 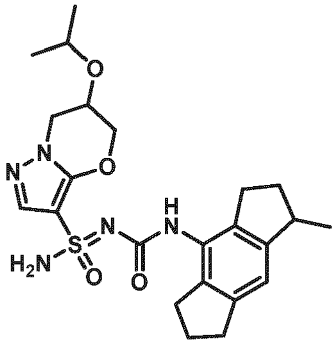 |
| 564 | 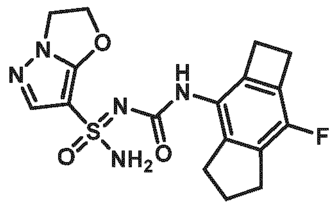 | 681 | 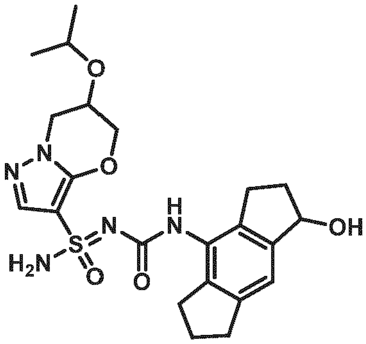 |
| 565 | 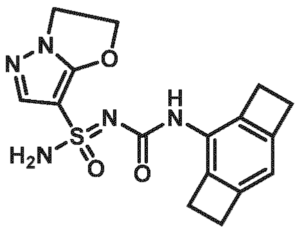 | 682 | 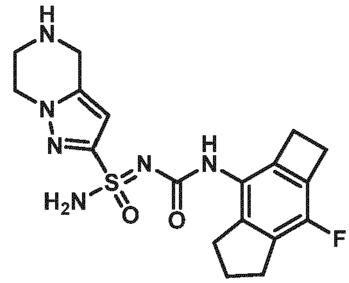 |
| 566 | 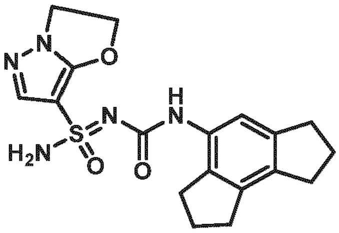 | 683 | 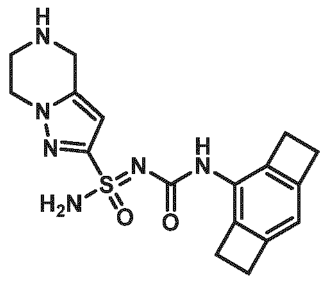 |

(continued)

| | | | |
|---|---|---|---|
| 567 | 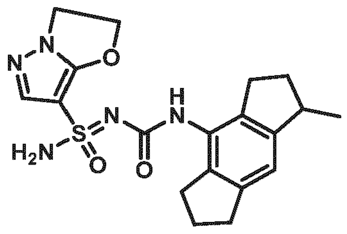 | 684 | 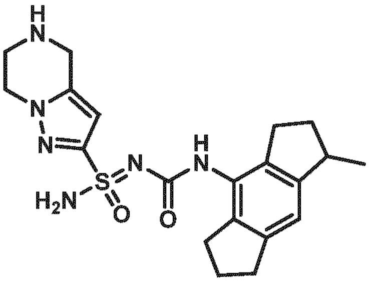 |
| 568 | 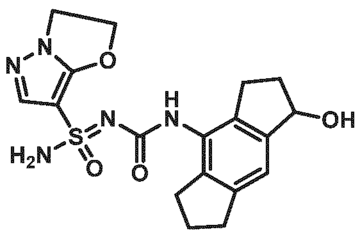 | 685 | 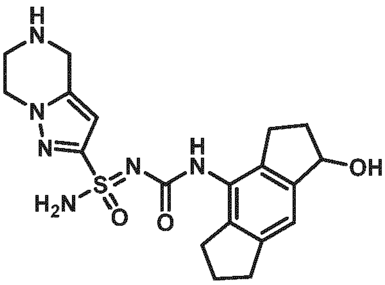 |
| 569 | 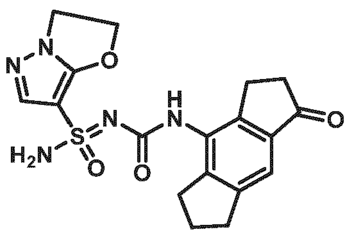 | 686 | 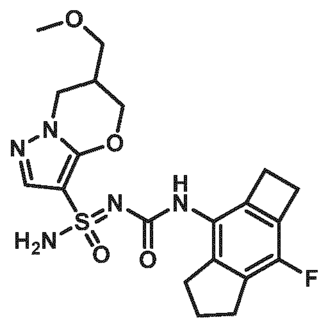 |
| 570 | 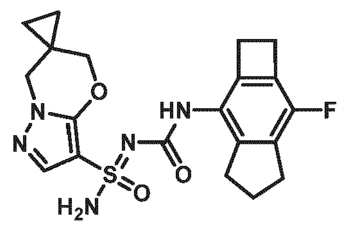 | 687 | 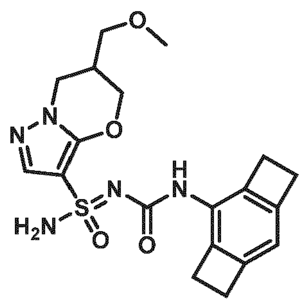 |
| 571 | 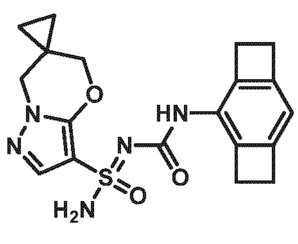 | 688 | 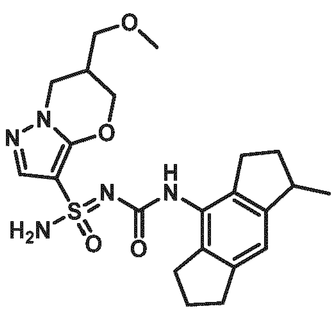 |

(continued)

| | | | |
|---|---|---|---|
| 572 | 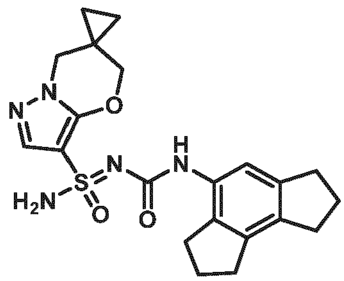 | 689 | 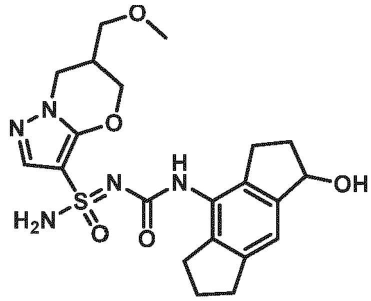 |
| 573 | 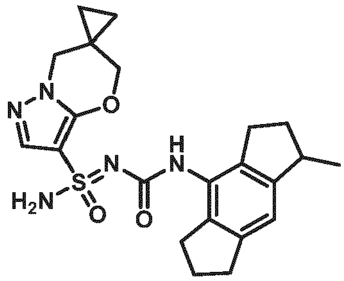 | 690 | 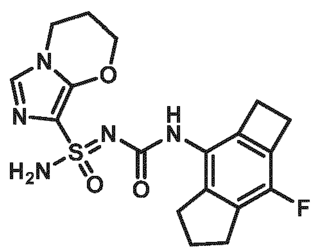 |
| 574 | 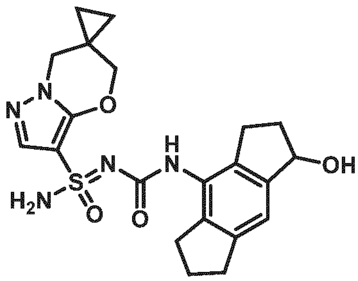 | 691 | 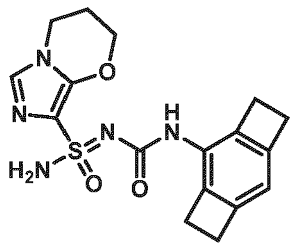 |
| 575 | 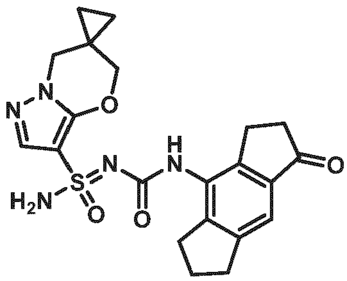 | 692 | 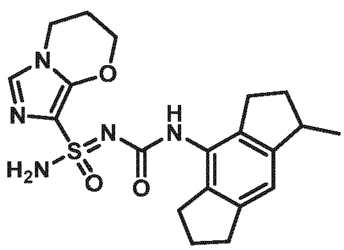 |
| 576 | 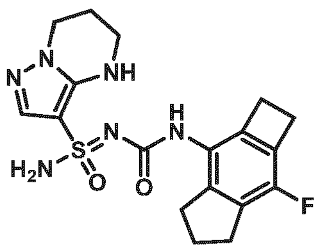 | 693 | 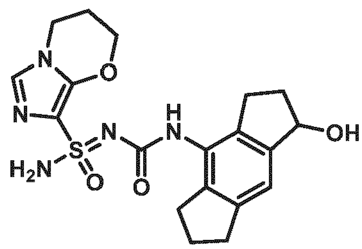 |

(continued)

| | | | |
|---|---|---|---|
| 577 | 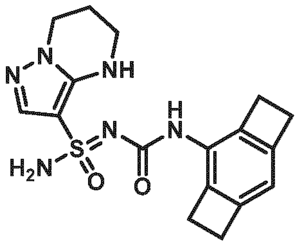 | 694 | 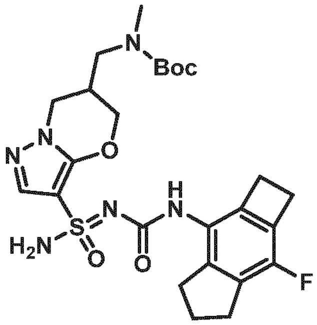 |
| 578 | 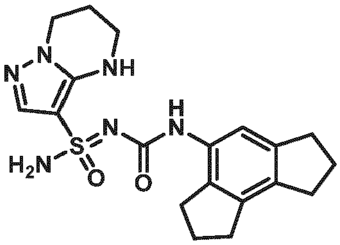 | 695 | 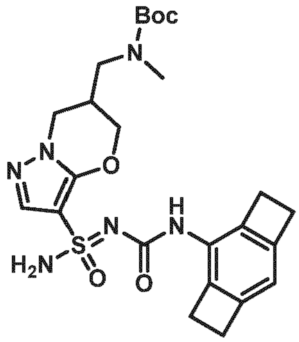 |
| 579 | 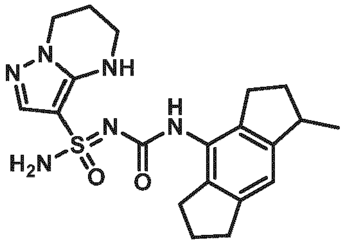 | 696 | 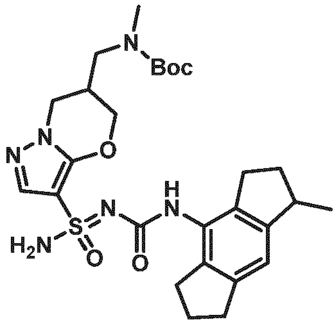 |
| 580 | 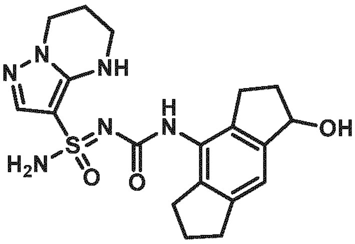 | 697 | 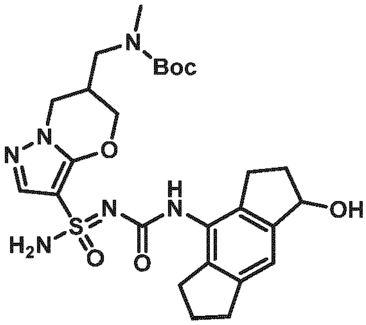 |
| 581 | 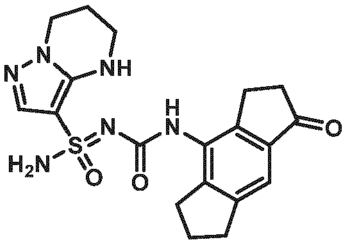 | 698 | 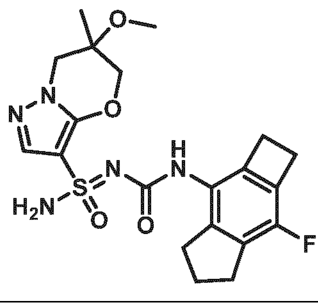 |

(continued)

| | | | |
|---|---|---|---|
| 582 | 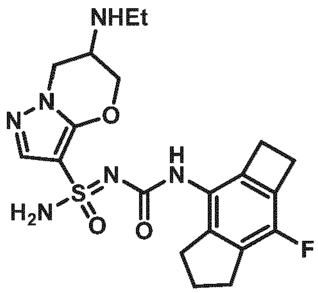 | 699 | 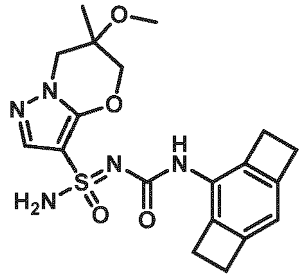 |
| 583 | 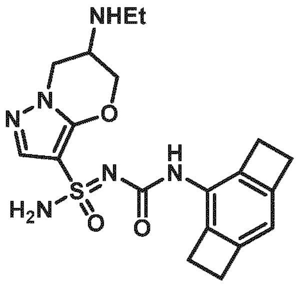 | 700 | 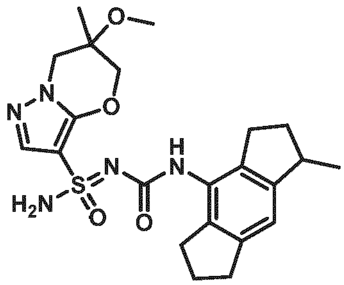 |
| 584 | 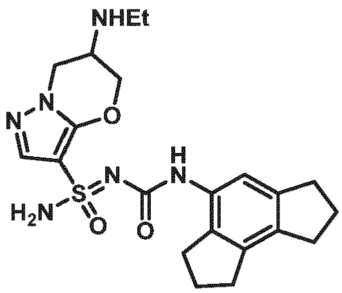 | 701 | 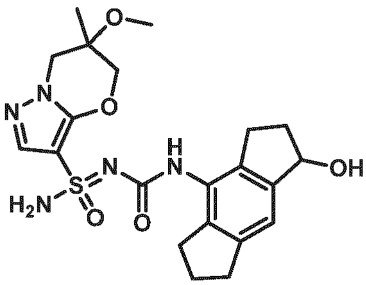 |
| 585 | 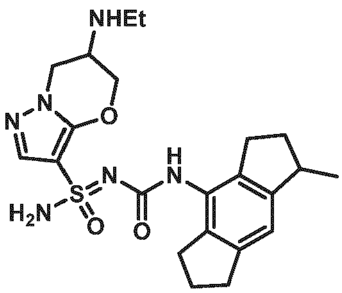 | 702 | 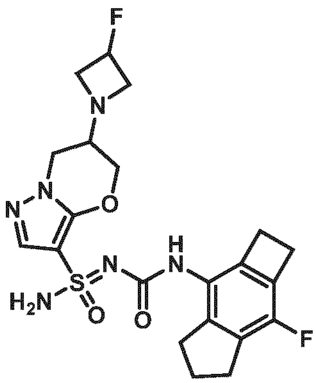 |
| 586 | 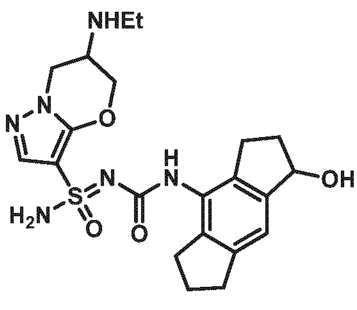 | 703 | 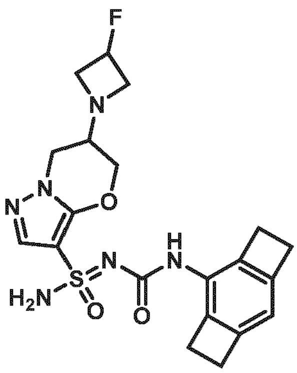 |

(continued)

| | | | |
|---|---|---|---|
| 587 | 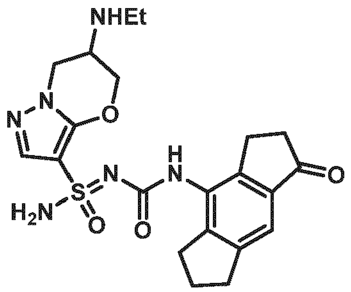 | 704 | 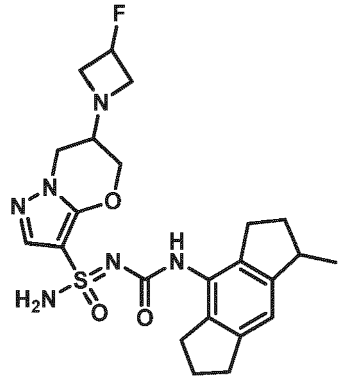 |
| 588 | 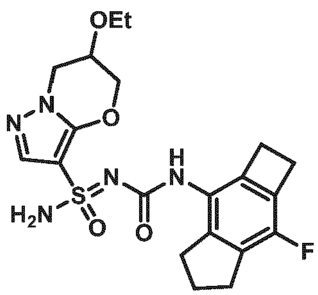 | 705 | 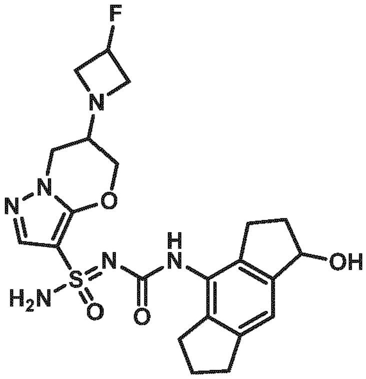 |
| 589 | 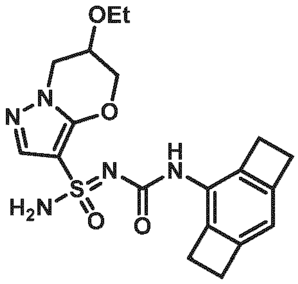 | 706 | 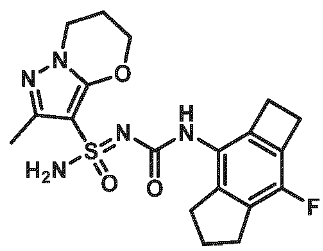 |
| 590 | 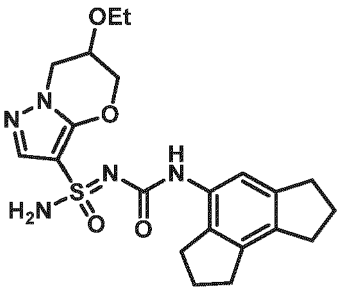 | 707 | 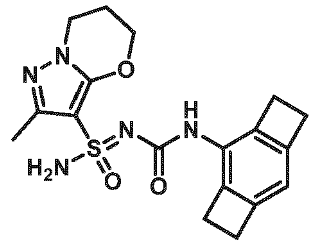 |
| 591 | 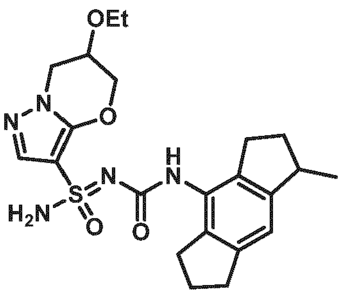 | 708 | 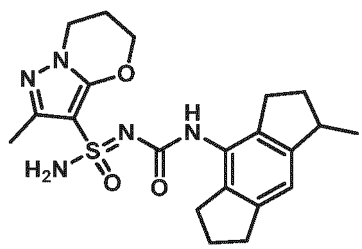 |

(continued)

| | | | |
|---|---|---|---|
| 592 | 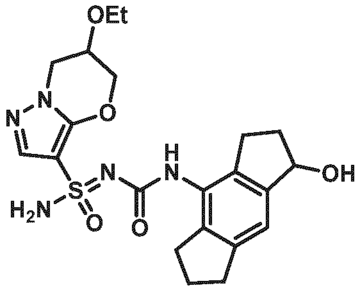 | 709 | 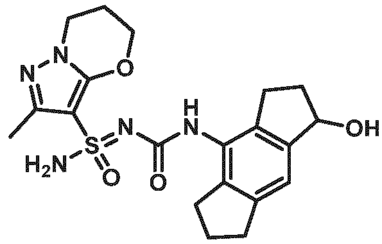 |
| 593 | 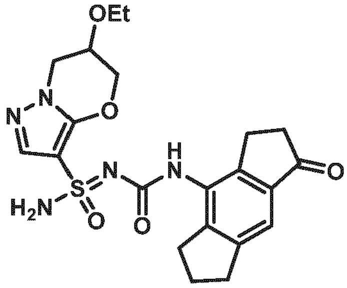 | 710 | 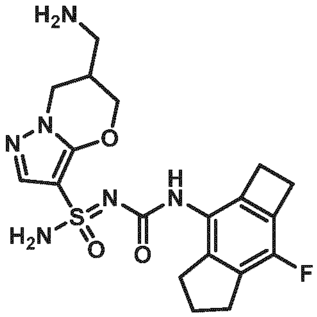 |
| 594 | 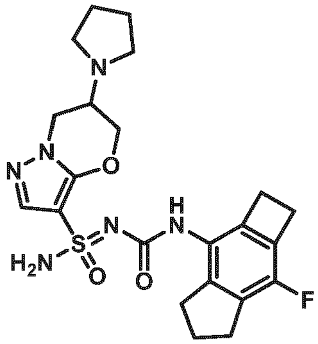 | 711 | 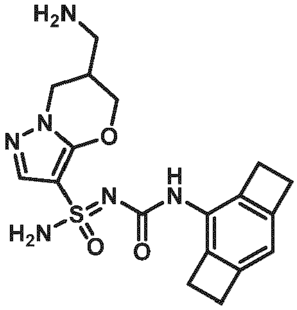 |
| 595 | 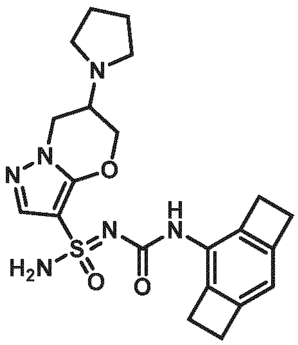 | 712 | 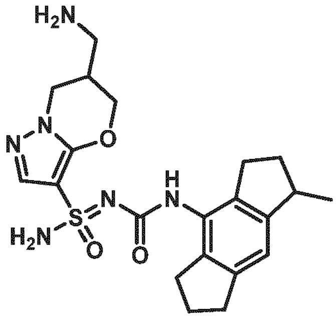 |
| 596 | 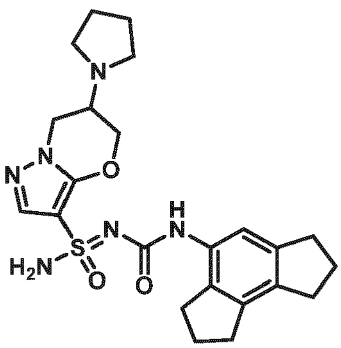 | 713 | 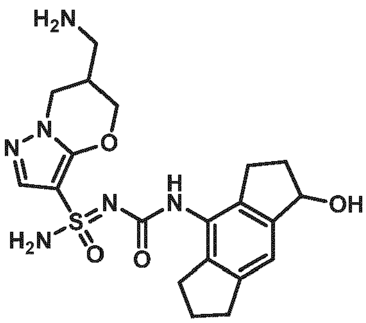 |

(continued)

| | | | |
|---|---|---|---|
| 597 | 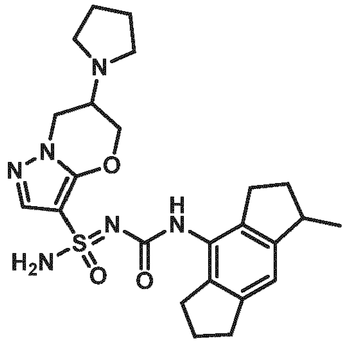 | 714 | 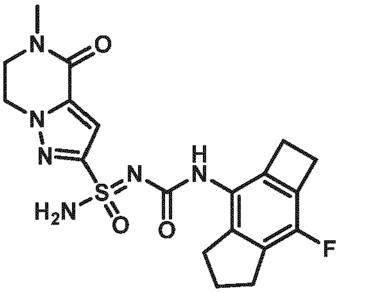 |
| 598 | 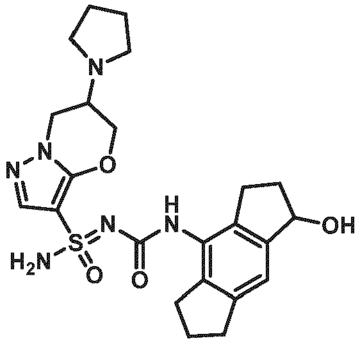 | 715 | 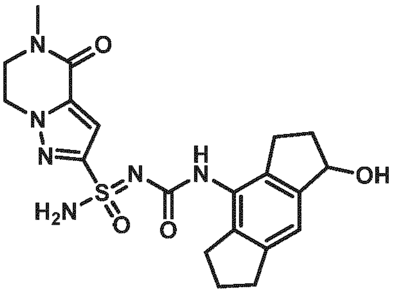 |
| 599 | 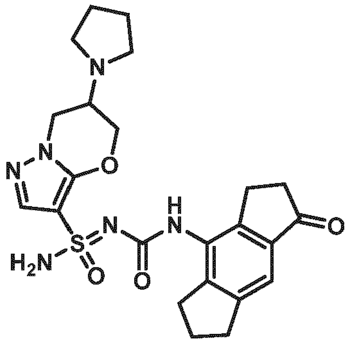 | 716 | 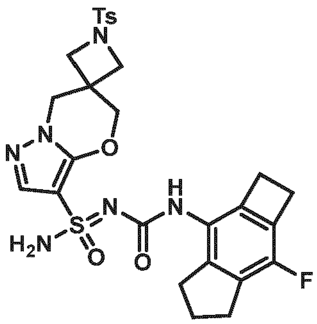 |
| 600 | 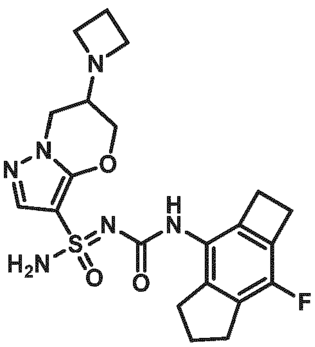 | 717 | 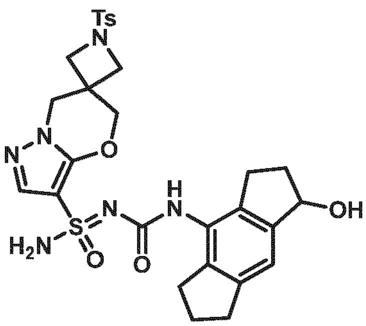 |

(continued)

| | | | |
|---|---|---|---|
| 601 | 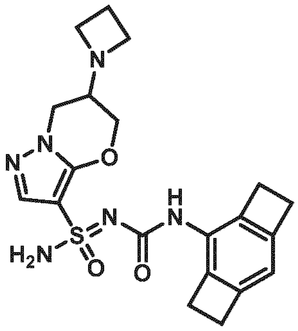 | 718 | 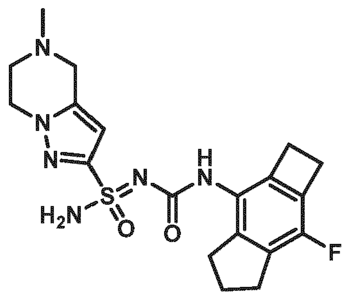 |
| 602 | 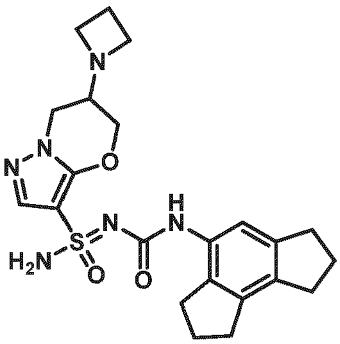 | 719 | 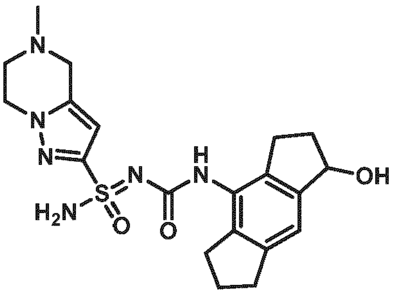 |
| 603 | 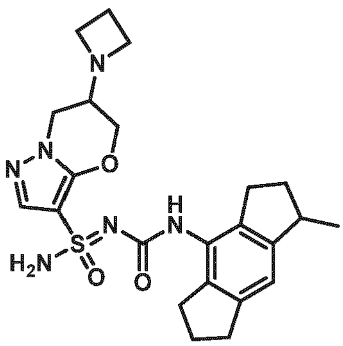 | 720 | 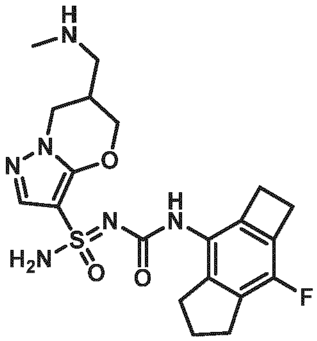 |
| 604 | 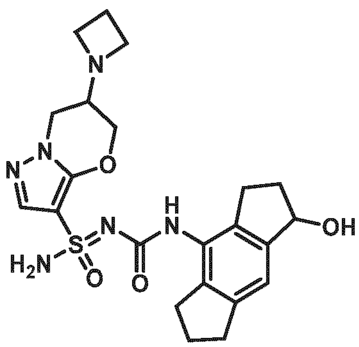 | 721 | 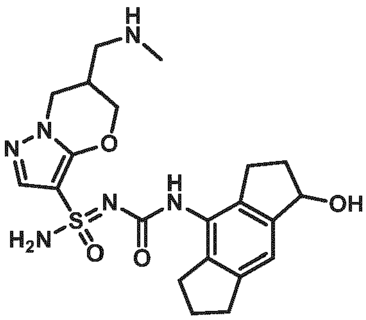 |

(continued)

| | | | |
|---|---|---|---|
| 605 | 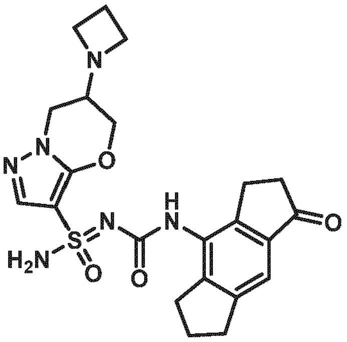 | 722 | 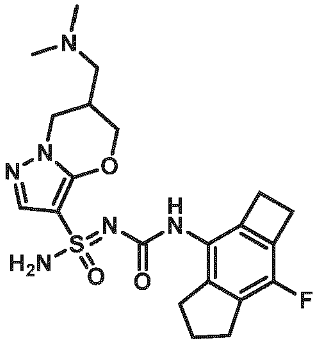 |
| 606 | 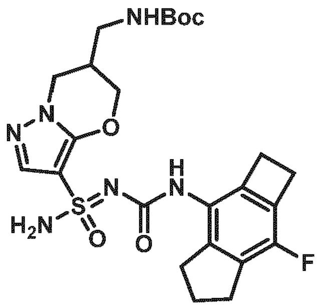 | 723 | 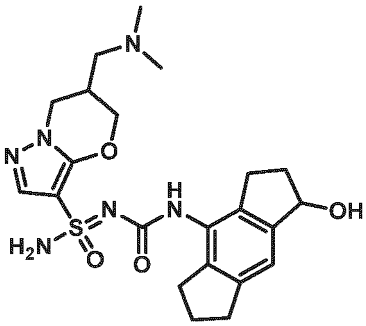 |
| 607 | 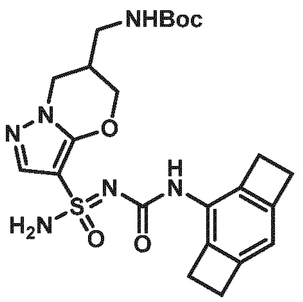 | 724 | 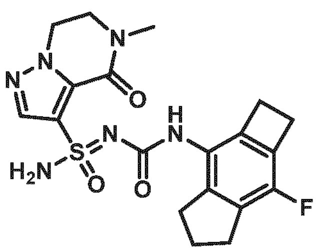 |
| 608 | 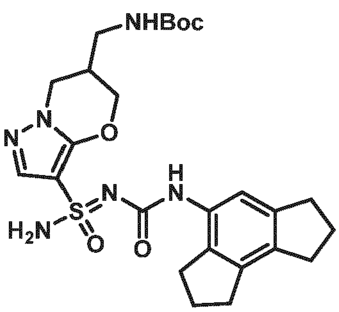 | 725 | 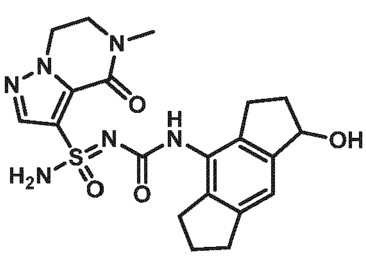 |
| 609 | 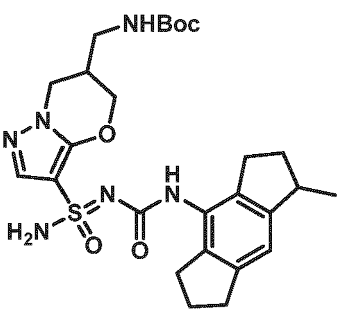 | 726 | 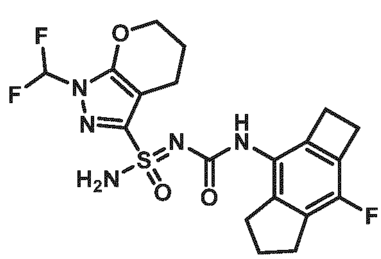 |

(continued)

| | | | |
|---|---|---|---|
| 610 | 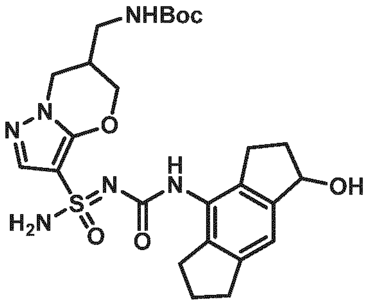 | 727 | 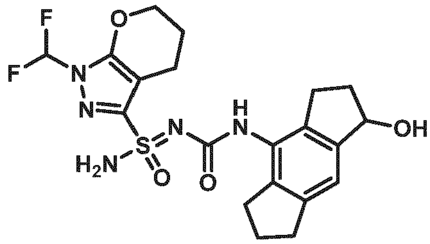 |
| 611 | 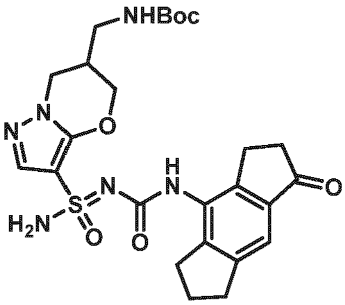 | 728 | 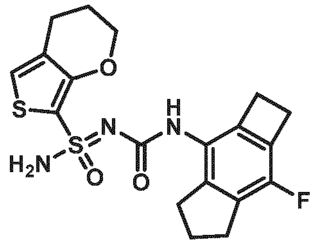 |
| 612 | 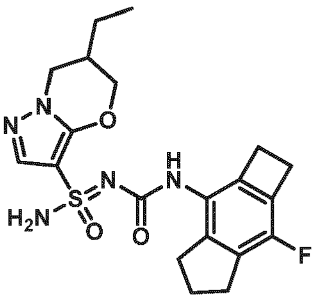 | 729 | 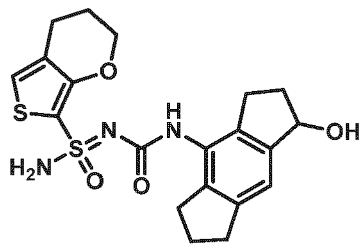 |
| 613 | 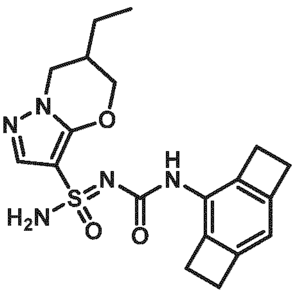 | 730 | 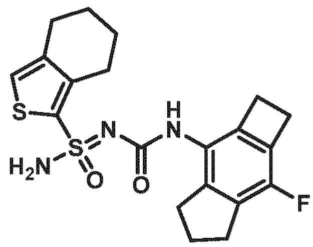 |
| 614 | 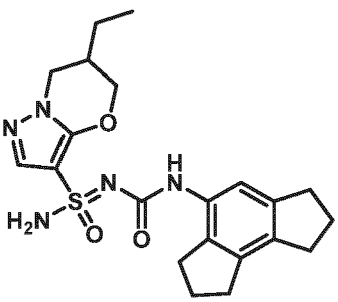 | 731 | 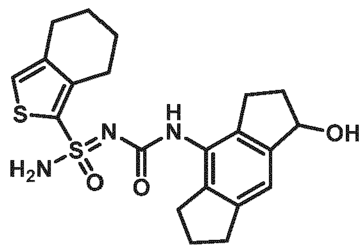 |

(continued)

| | | | |
|---|---|---|---|
| 615 | 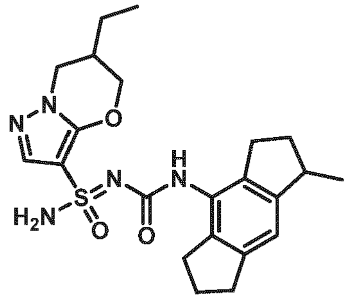 | 732 | 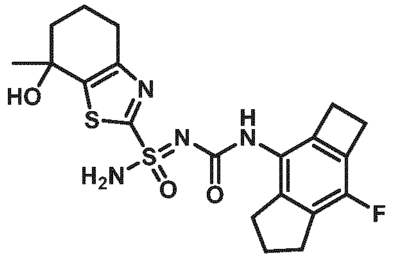 |
| 616 | 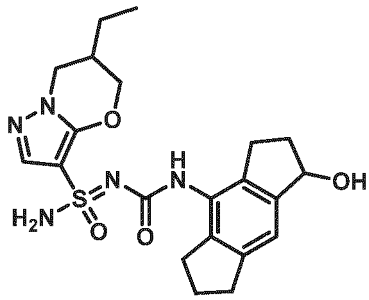 | 733 | 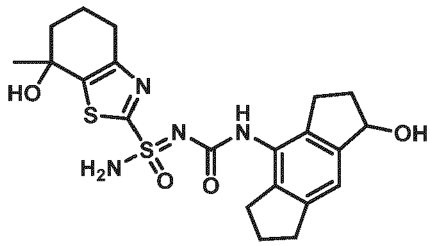 |
| 617 | 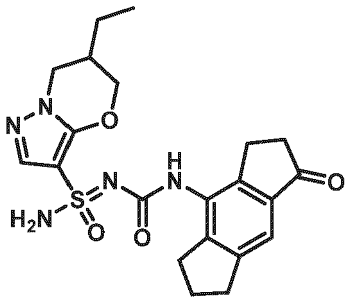 | 734 | 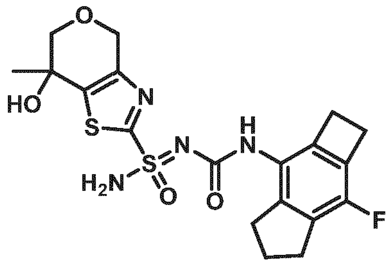 |
| | | 735 | 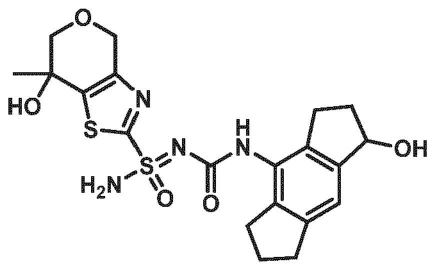 |

or a pharmaceutically acceptable salt thereof.

2. The compound of claim 1, wherein the sulfur in the moiety S(=O)(NHR$^3$)=N- has (S) stereochemistry.

3. The compound of any one of claims 1, wherein the sulfur in the moiety S(=O)(NHR$^3$)=N- has (R) stereochemistry.

4. A pharmaceutical composition comprising a compound or salt as claimed in any one of claims 1-3 and one or more pharmaceutically acceptable excipients.

## Patentansprüche

1. Verbindung, ausgewählt aus der Gruppe bestehend aus den nachstehenden Verbindungen:

| | | | |
|---|---|---|---|
| 501 | 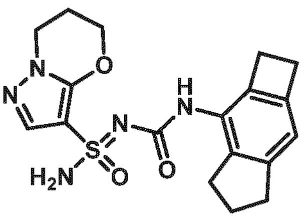 | 618 | 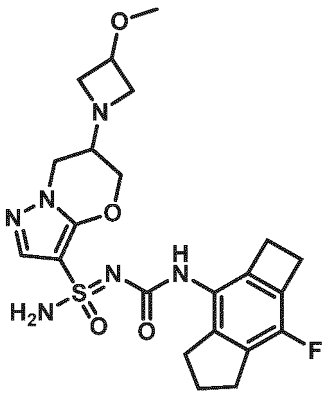 |
| 502 | 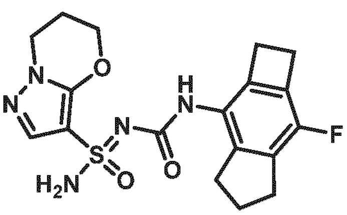 | 619 | 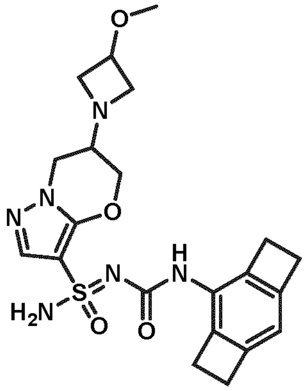 |
| 503 | 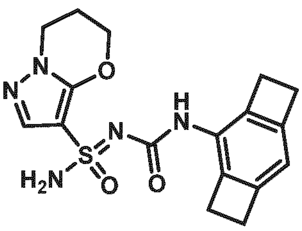 | 620 | 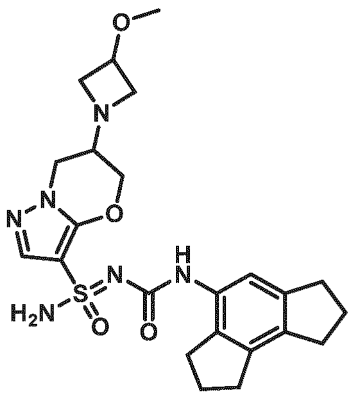 |
| 504 | 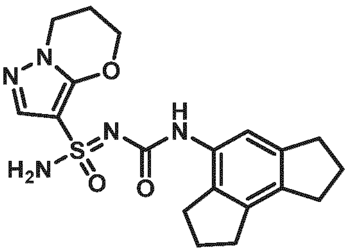 | 621 | 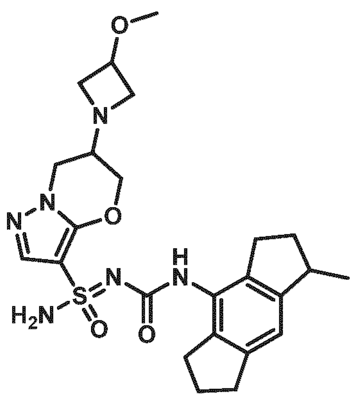 |

(fortgesetzt)

| | | | |
|---|---|---|---|
| 505 | 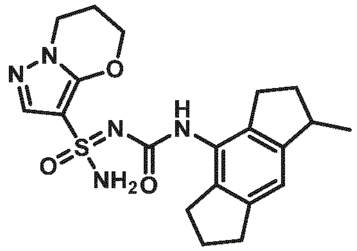 | 622 | 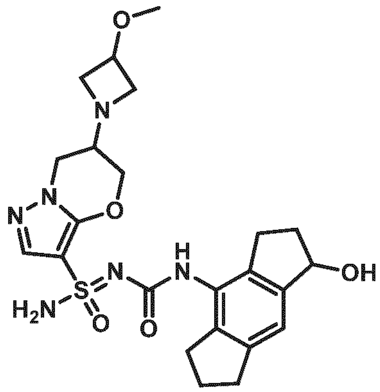 |
| 506 | 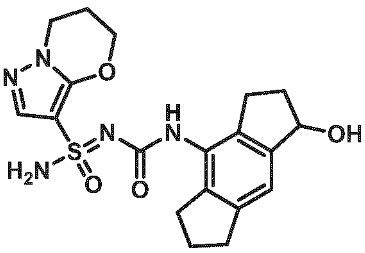 | 623 | 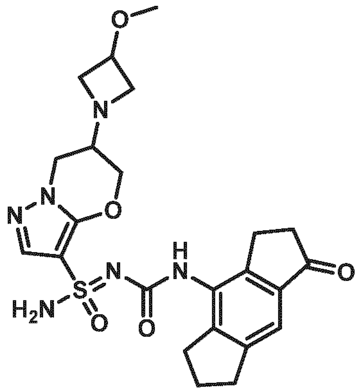 |
| 507 | 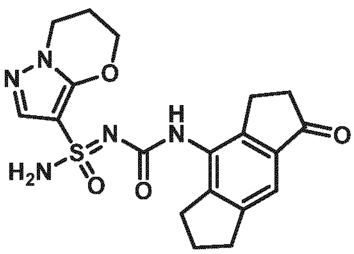 | 624 | 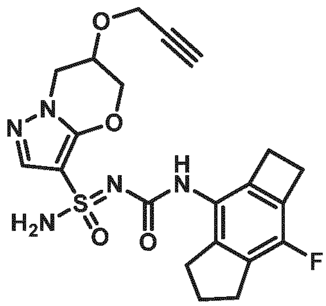 |
| 508 | 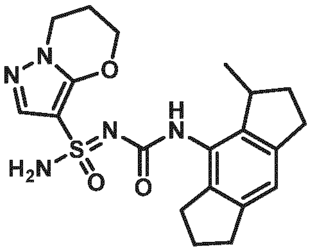 | 625 | 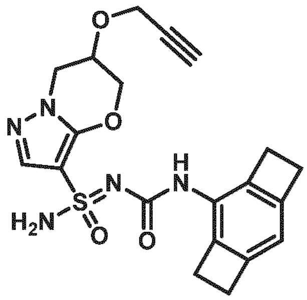 |

(fortgesetzt)

| | | | |
|---|---|---|---|
| 509 | 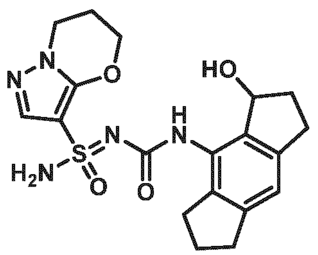 | 626 | 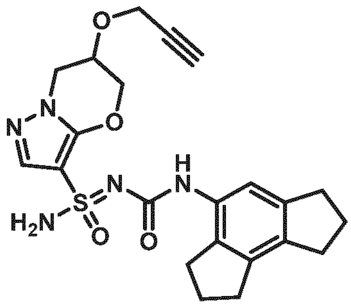 |
| 510 | 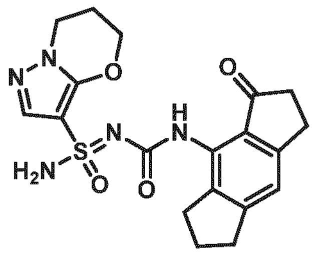 | 627 | 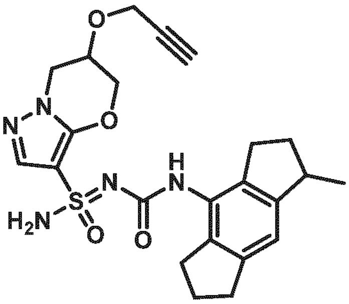 |
| 511 | 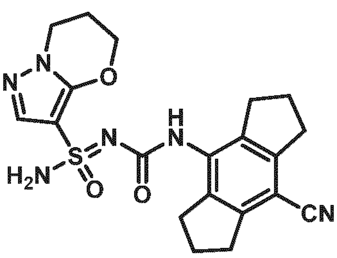 | 628 | 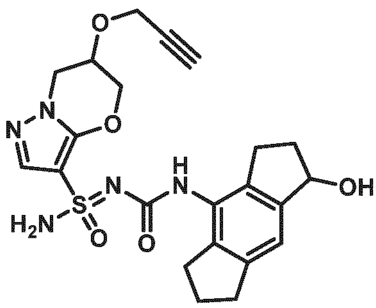 |
| 512 | 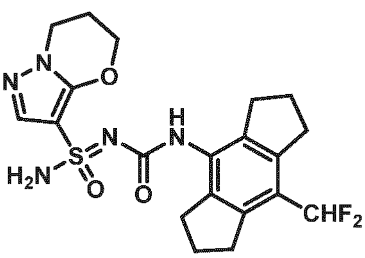 | 629 | 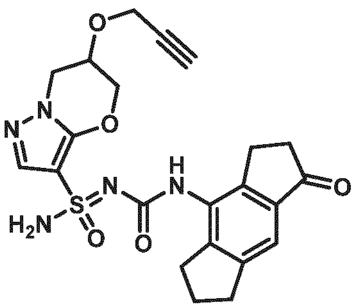 |
| 513 | 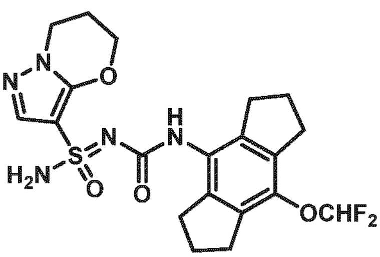 | 630 | 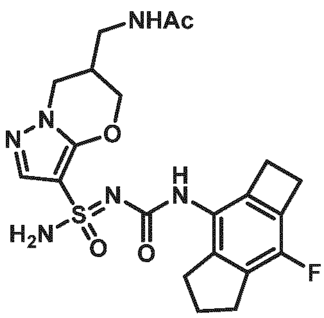 |

(fortgesetzt)

| | | | |
|---|---|---|---|
| 514 | 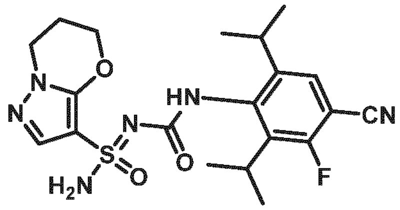 | 631 | 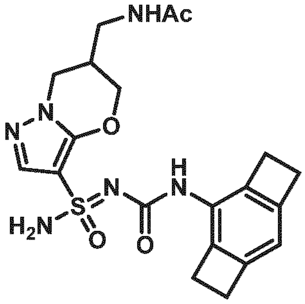 |
| 515 | 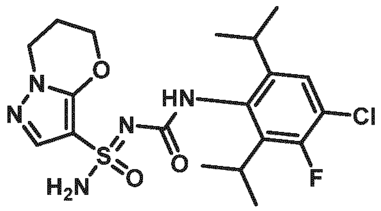 | 632 | 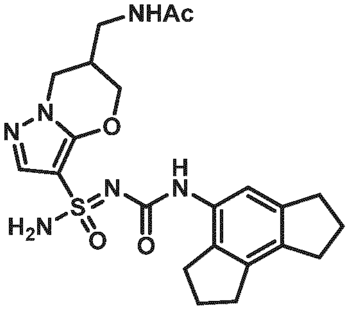 |
| 516 | 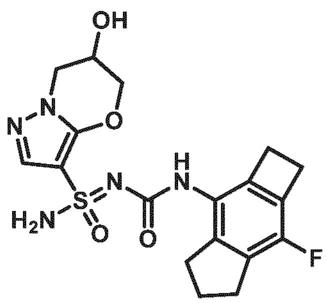 | 633 | 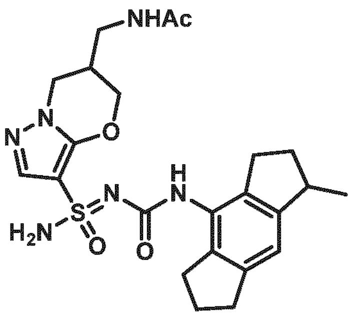 |
| 517 | 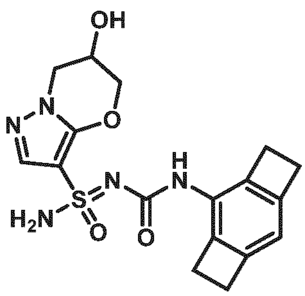 | 634 | 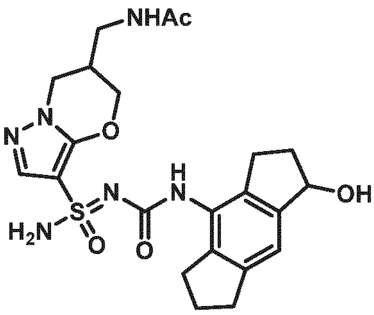 |
| 518 | 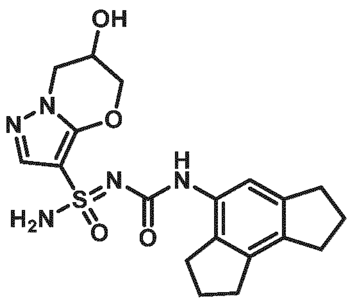 | 635 | 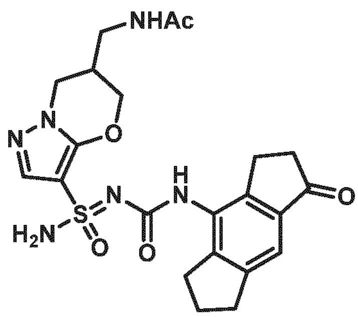 |

(fortgesetzt)

| | | | |
|---|---|---|---|
| 519 | 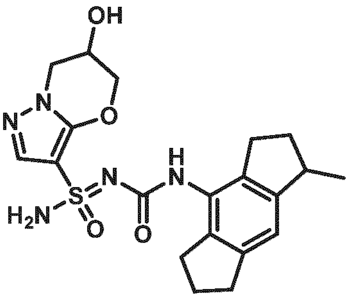 | 636 | 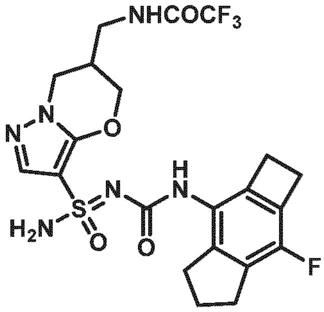 |
| 520 | 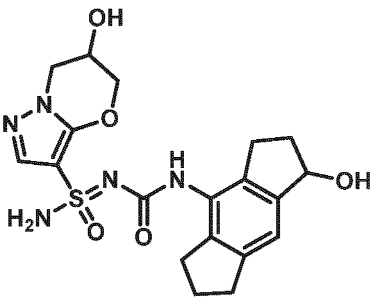 | 637 | 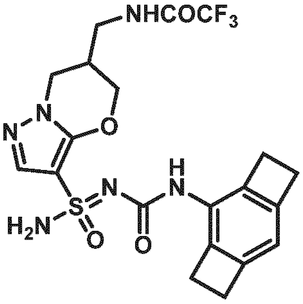 |
| 521 | 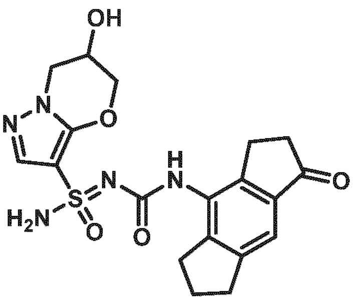 | 638 | 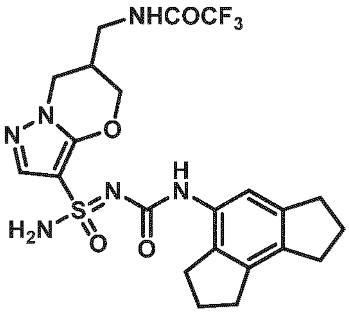 |
| 522 | 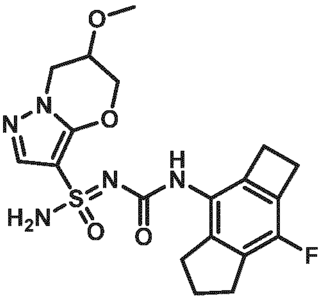 | 639 | 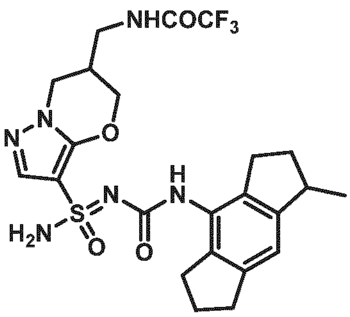 |
| 523 | 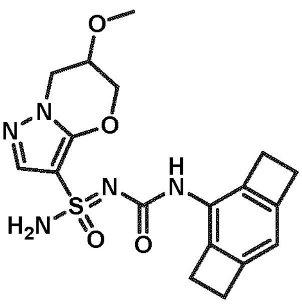 | 640 | 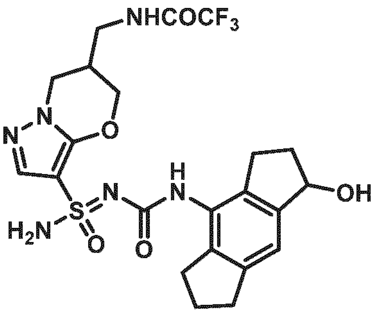 |

(fortgesetzt)

| | | | |
|---|---|---|---|
| 524 | 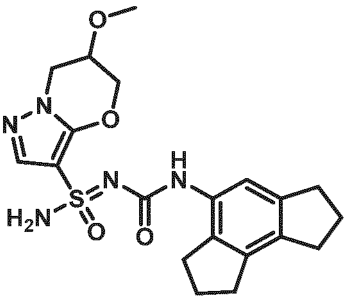 | 641 | 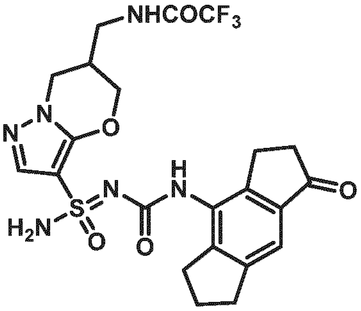 |
| 525 | 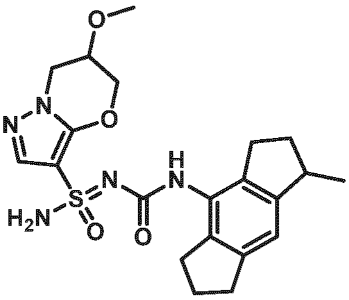 | 642 | 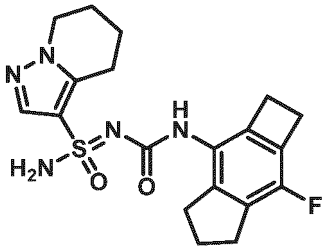 |
| 526 | 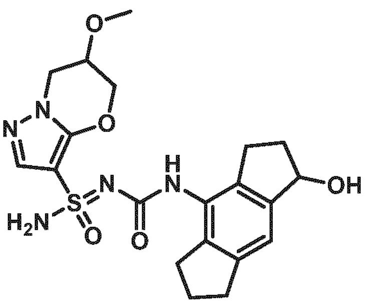 | 643 | 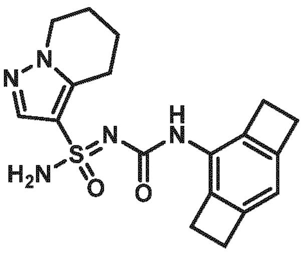 |
| 527 | 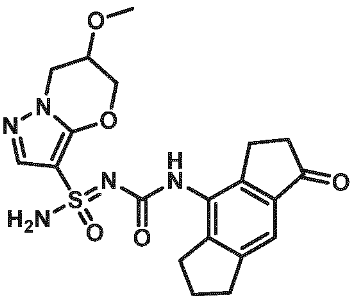 | 644 | 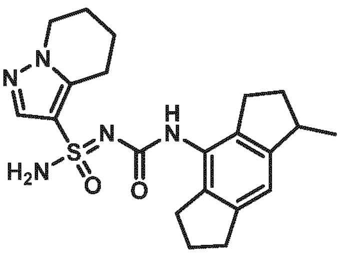 |
| 528 | 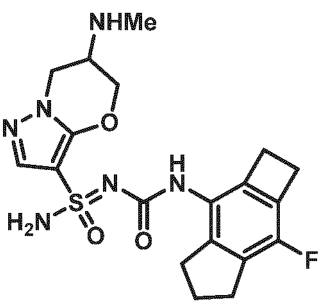 | 645 | 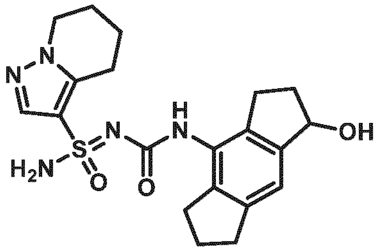 |

(fortgesetzt)

| | | | |
|---|---|---|---|
| 529 | 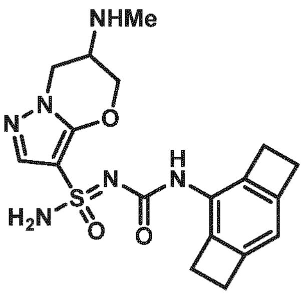 | 646 | 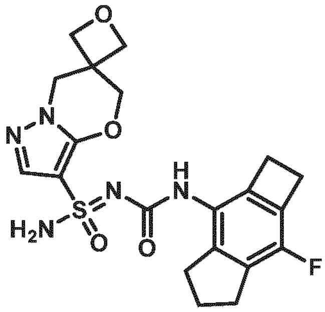 |
| 530 | 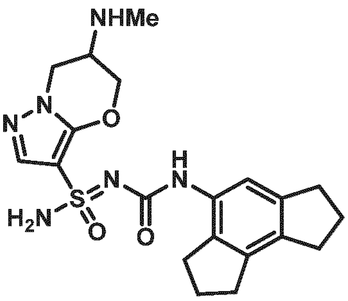 | 647 | 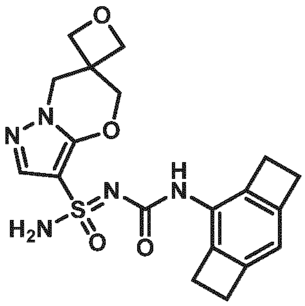 |
| 531 | 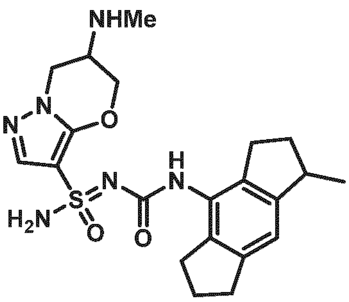 | 648 | 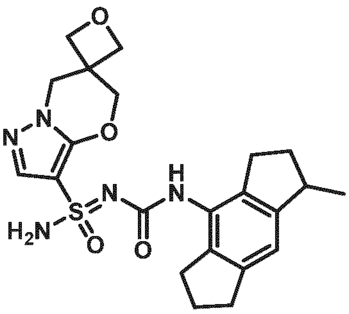 |
| 532 | 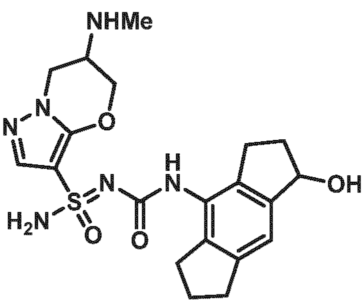 | 649 | 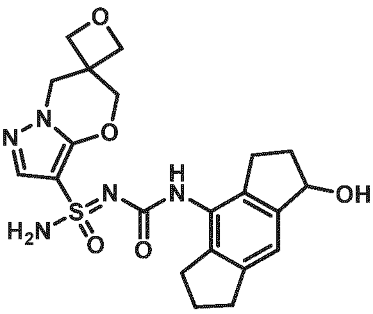 |
| 533 | 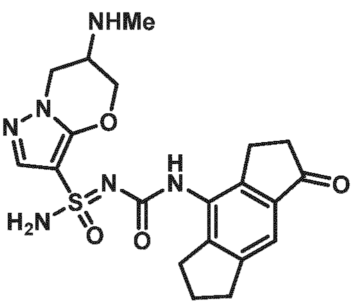 | 650 | 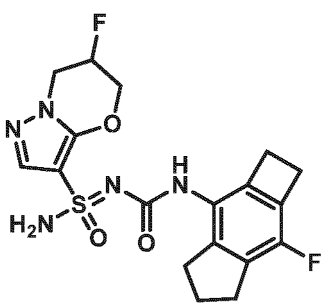 |

(fortgesetzt)

| | | | |
|---|---|---|---|
| 534 | 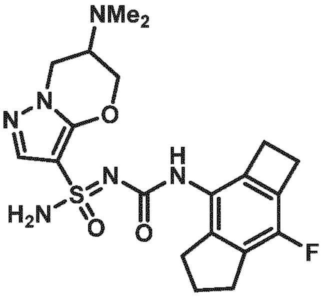 | 651 | 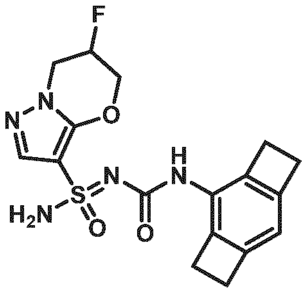 |
| 535 | 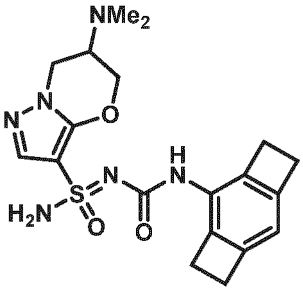 | 652 | 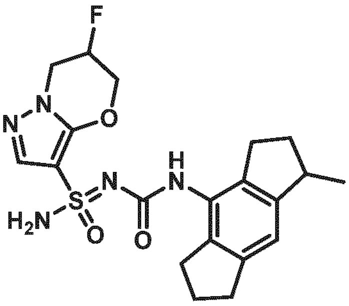 |
| 536 | 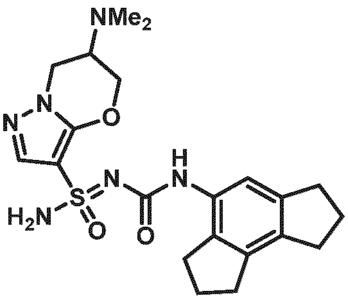 | 653 | 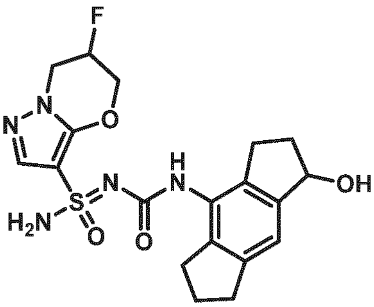 |
| 537 | 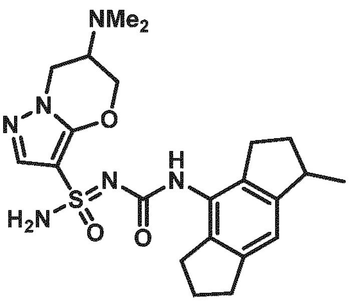 | 654 | 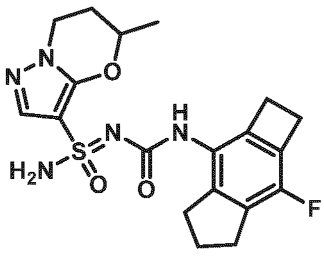 |
| 538 | 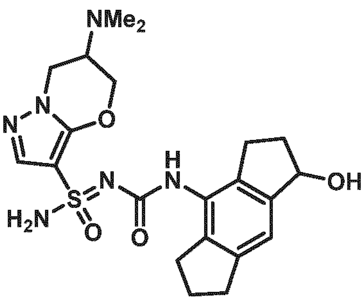 | 655 | 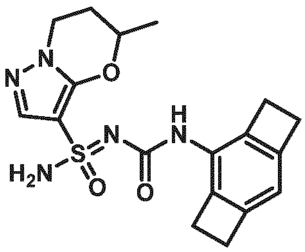 |

(fortgesetzt)

| | | | |
|---|---|---|---|
| 539 | 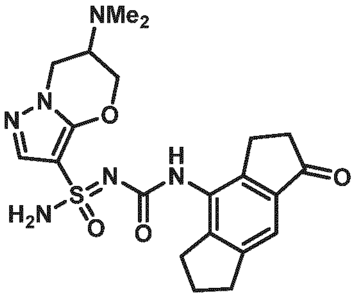 | 656 | 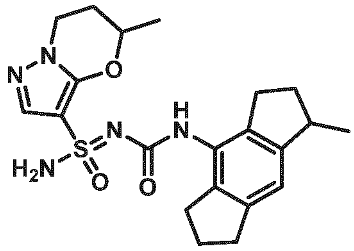 |
| 540 | 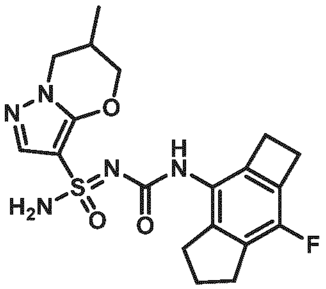 | 657 | 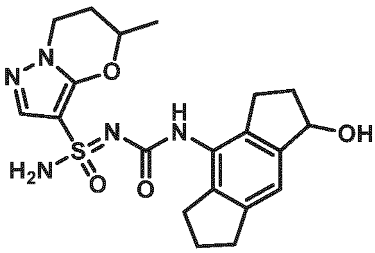 |
| 541 | 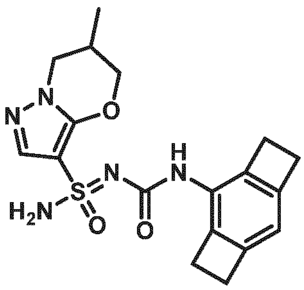 | 658 | 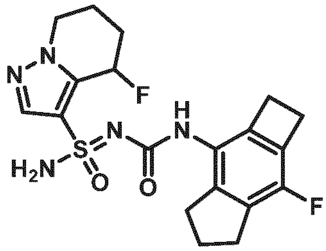 |
| 542 | 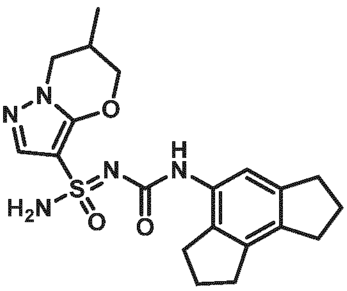 | 659 | 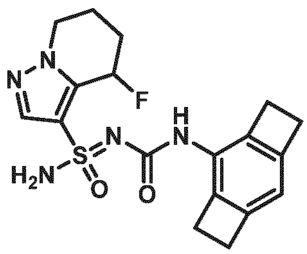 |
| 543 | 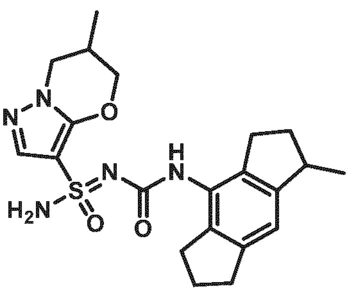 | 660 | 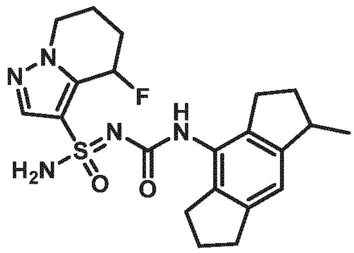 |

(fortgesetzt)

| | | | |
|---|---|---|---|
| 544 | 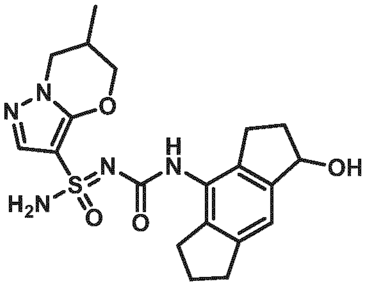 | 661 | 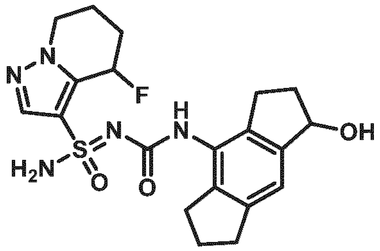 |
| 545 | 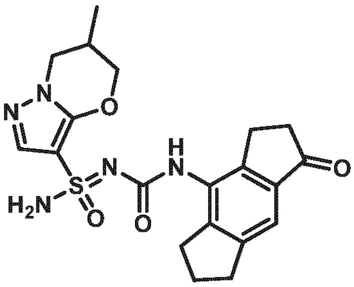 | 662 | 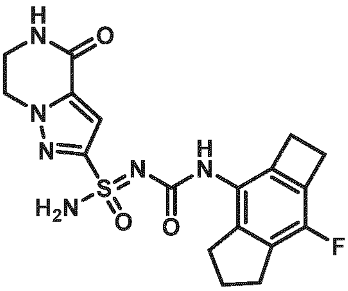 |
| 546 | 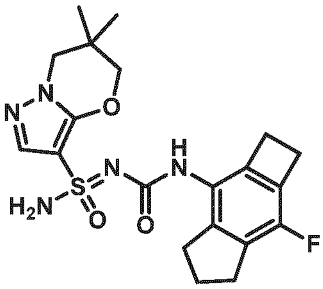 | 663 | 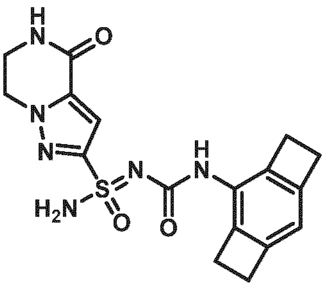 |
| 547 | 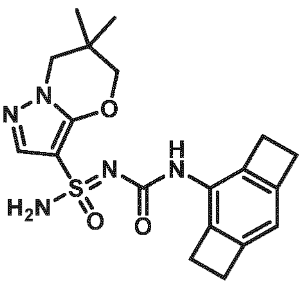 | 664 | 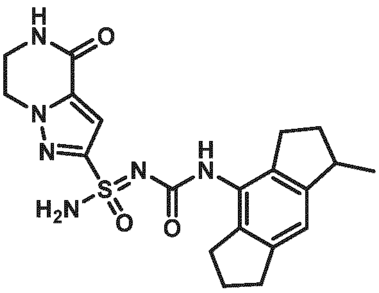 |
| 548 | 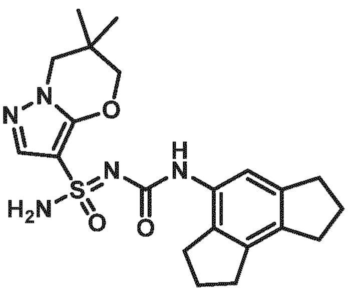 | 665 | 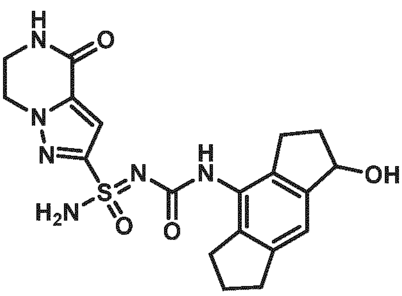 |

(fortgesetzt)

| | | | |
|---|---|---|---|
| 549 | 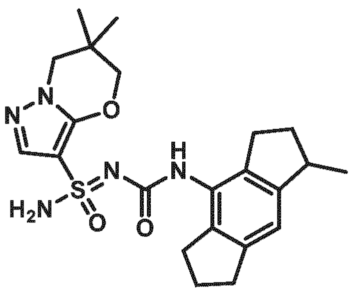 | 666 | 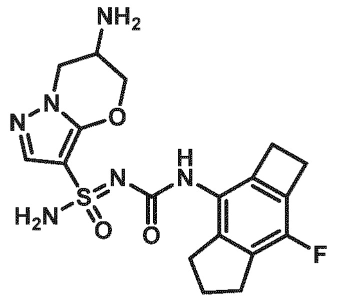 |
| 550 | 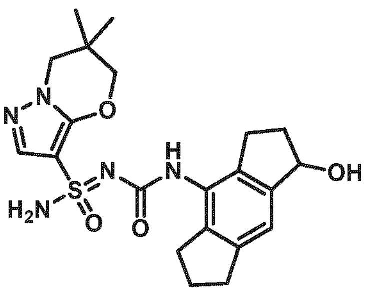 | 667 | 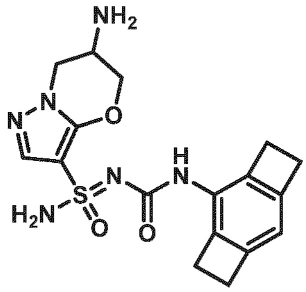 |
| 551 | 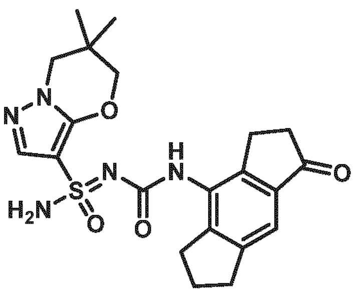 | 668 | 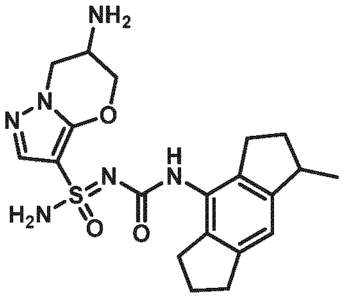 |
| 552 | 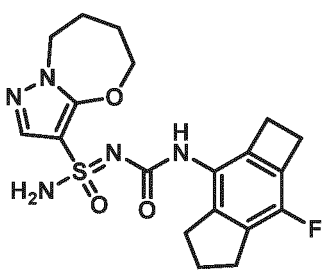 | 669 | 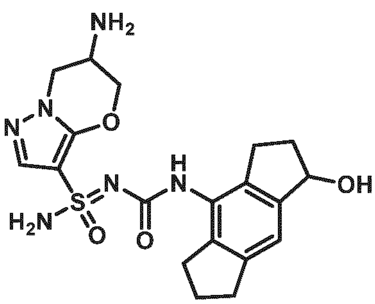 |
| 553 | 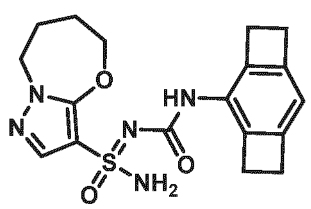 | 670 | 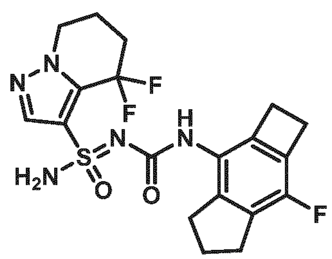 |

(fortgesetzt)

| | | | |
|---|---|---|---|
| 554 | 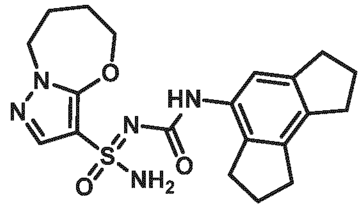 | 671 | 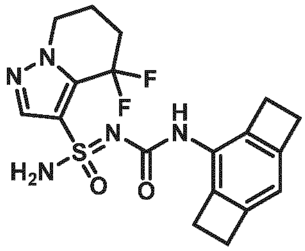 |
| 555 | 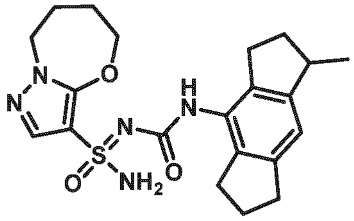 | 672 | 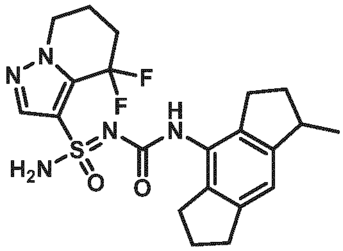 |
| 556 | 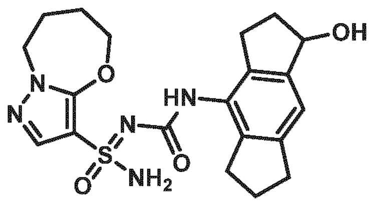 | 673 | 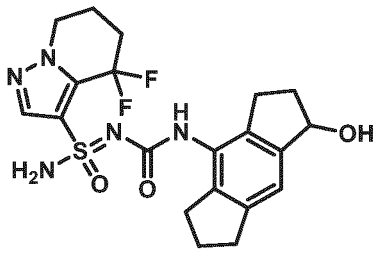 |
| 557 | 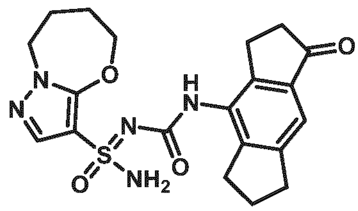 | 674 | 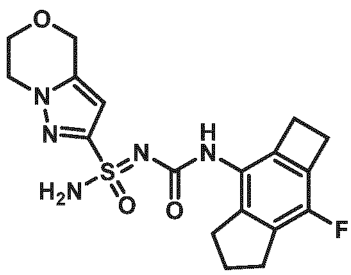 |
| 558 | 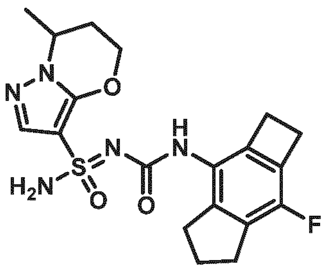 | 675 | 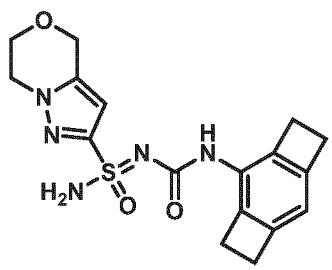 |

(fortgesetzt)

| | | | |
|---|---|---|---|
| 559 | 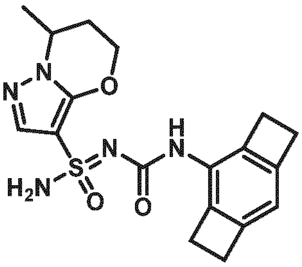 | 676 | 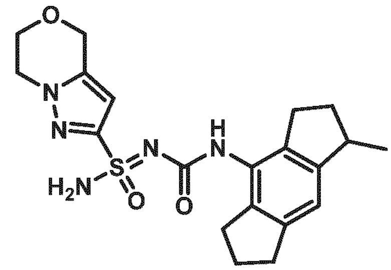 |
| 560 | 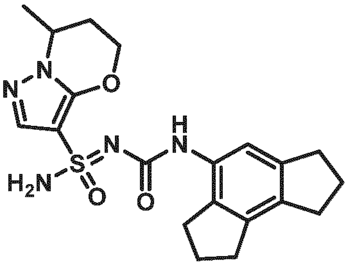 | 677 | 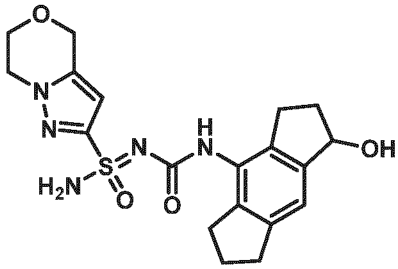 |
| 561 | 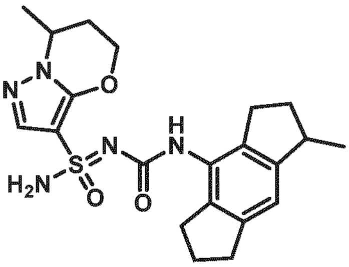 | 678 | 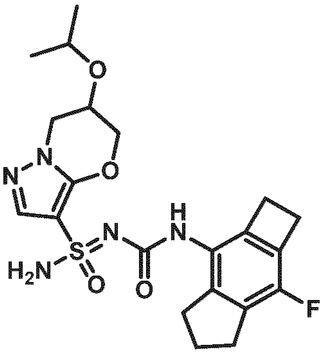 |
| 562 | 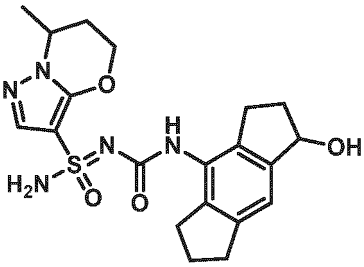 | 679 | 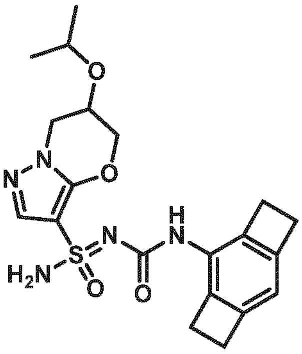 |

(fortgesetzt)

| | | | |
|---|---|---|---|
| 563 | 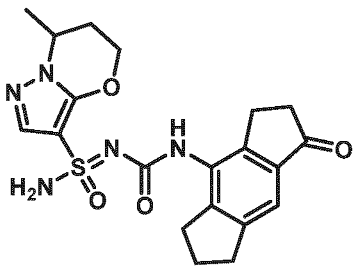 | 680 | 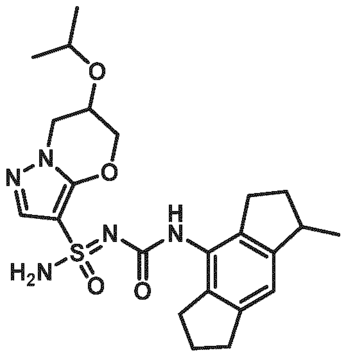 |
| 564 | 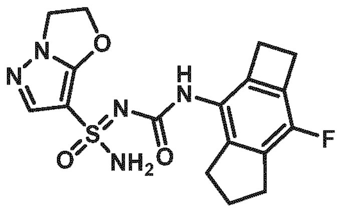 | 681 | 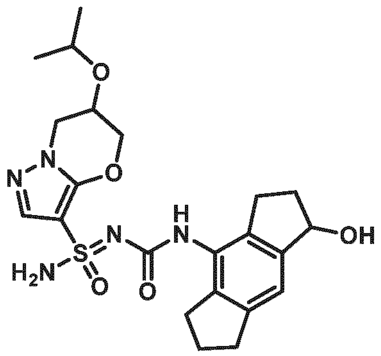 |
| 565 | 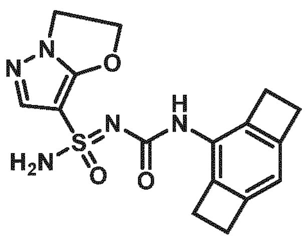 | 682 | 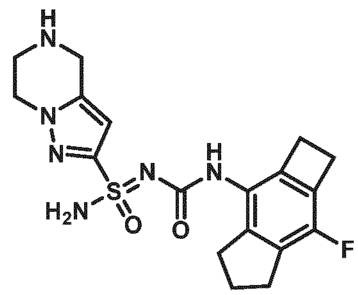 |
| 566 | 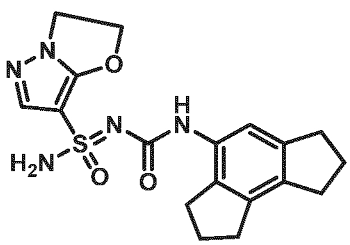 | 683 | 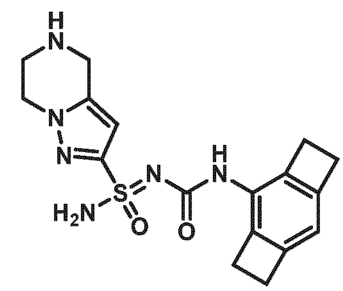 |
| 567 | 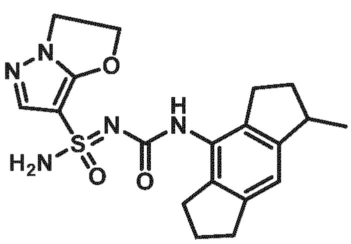 | 684 | 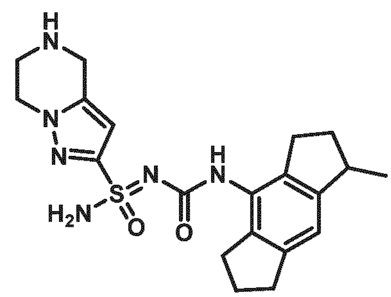 |

(fortgesetzt)

| | | | |
|---|---|---|---|
| 568 | 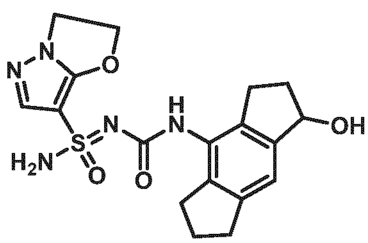 | 685 | 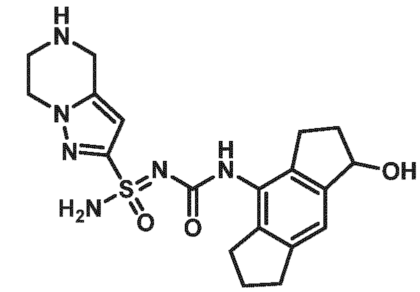 |
| 569 | 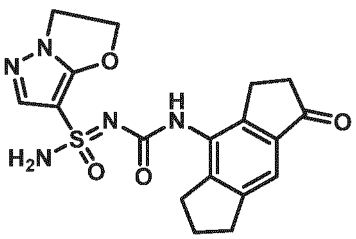 | 686 | 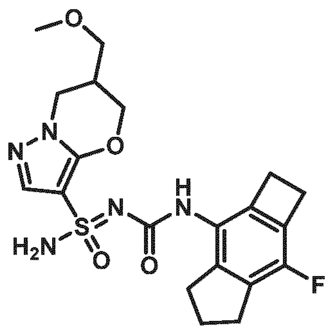 |
| 570 | 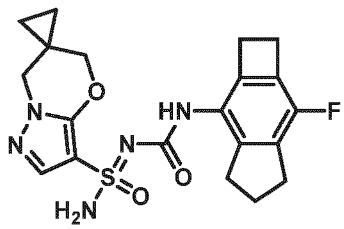 | 687 | 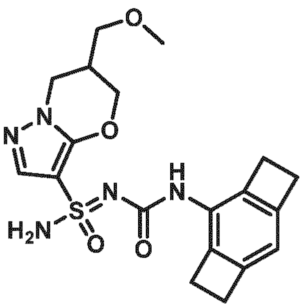 |
| 571 | 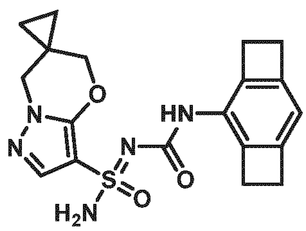 | 688 | 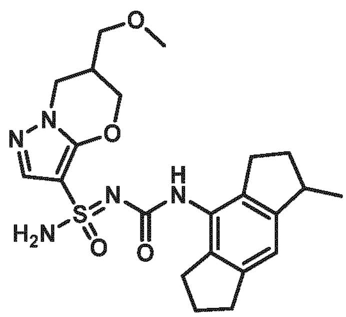 |
| 572 | 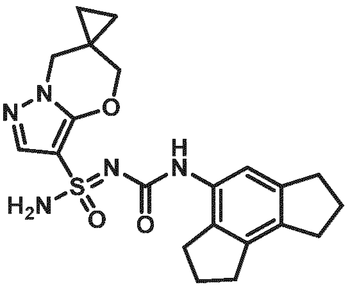 | 689 | 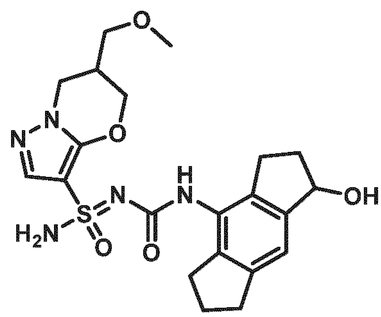 |

(fortgesetzt)

| | | | |
|---|---|---|---|
| 573 | 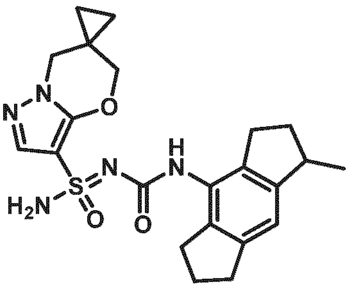 | 690 | 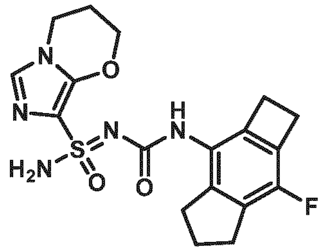 |
| 574 | 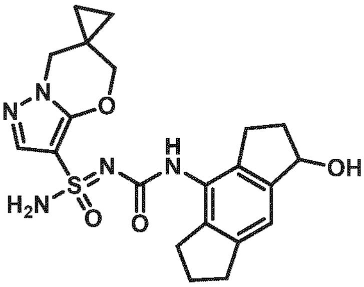 | 691 | 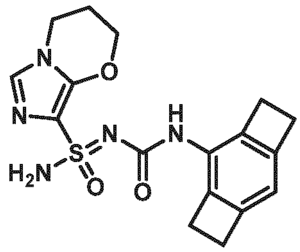 |
| 575 | 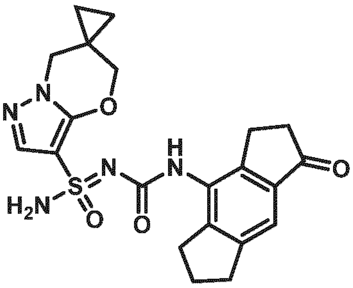 | 692 | 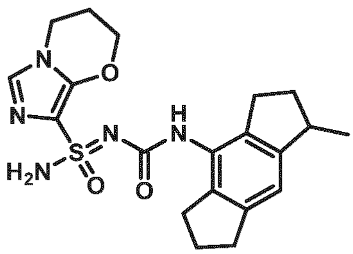 |
| 576 | 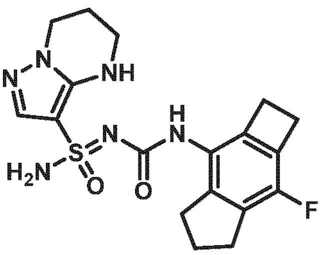 | 693 | 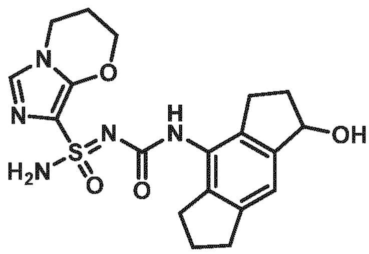 |
| 577 | 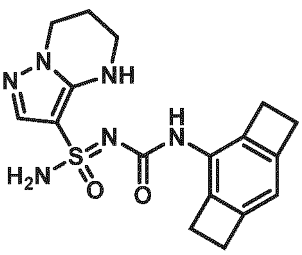 | 694 | 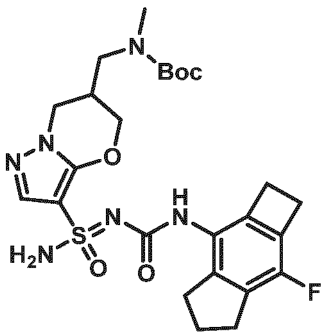 |

(fortgesetzt)

| | | | |
|---|---|---|---|
| 578 | 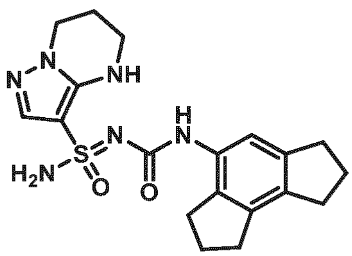 | 695 | 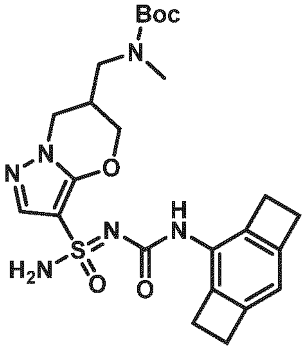 |
| 579 | 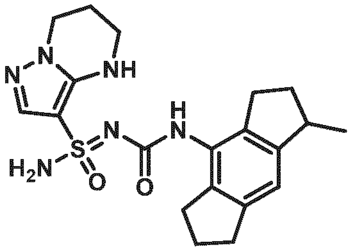 | 696 | 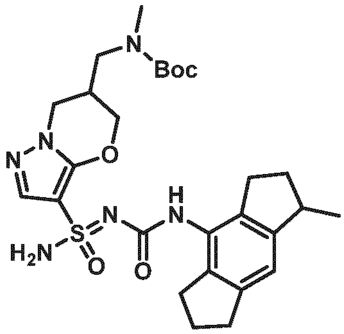 |
| 580 | 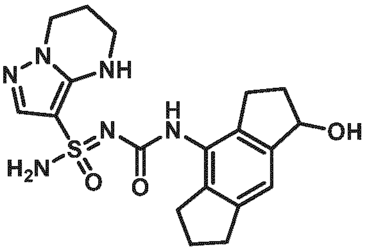 | 697 | 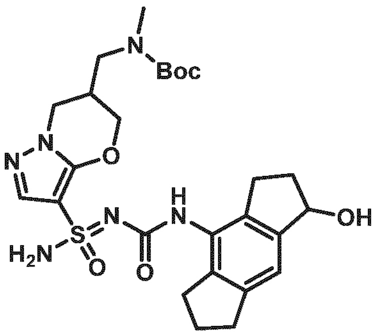 |
| 581 | 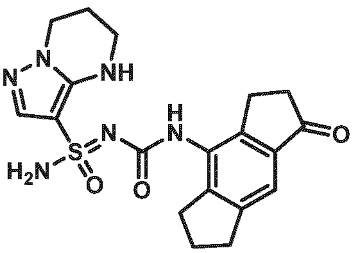 | 698 | 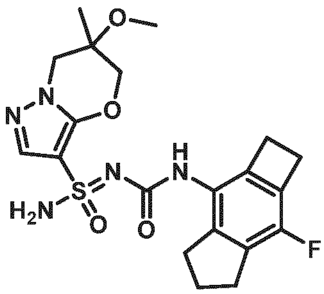 |
| 582 | 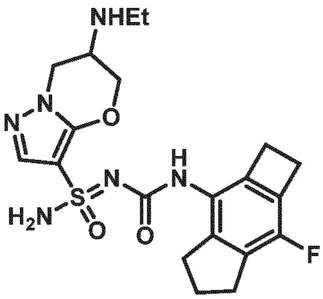 | 699 | 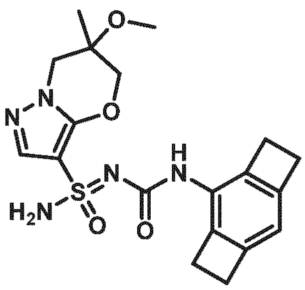 |

(fortgesetzt)

| | | | |
|---|---|---|---|
| 583 | 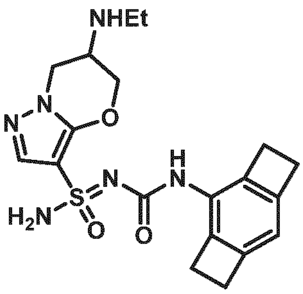 | 700 | 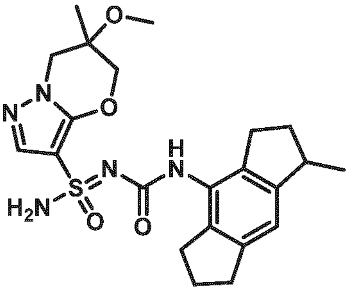 |
| 584 | 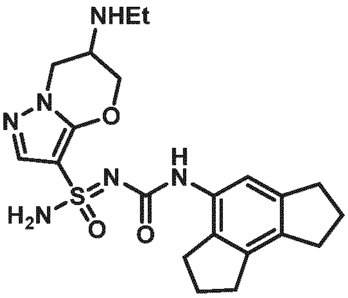 | 701 | 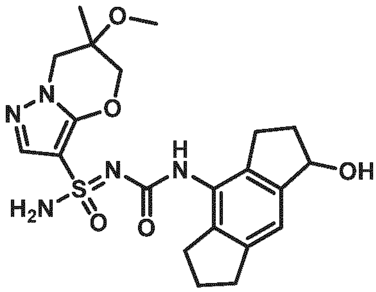 |
| 585 | 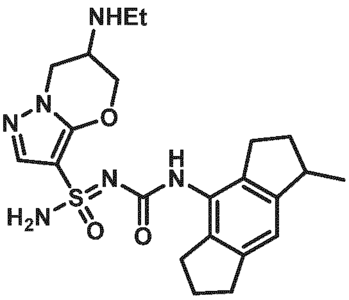 | 702 | 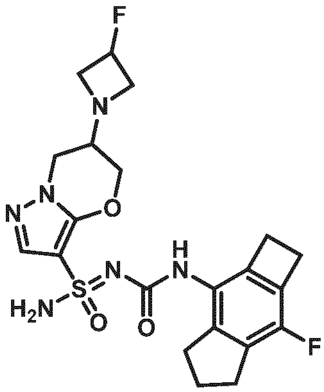 |
| 586 | 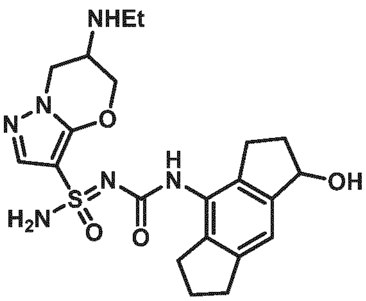 | 703 | 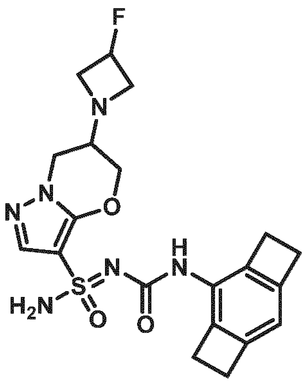 |

(fortgesetzt)

| | | | |
|---|---|---|---|
| 587 | 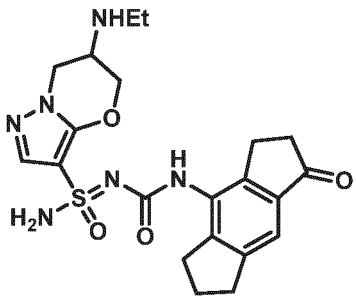 | 704 | 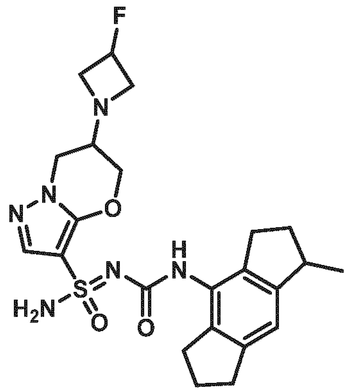 |
| 588 | 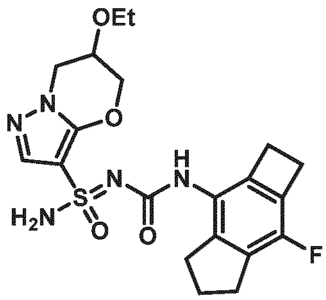 | 705 | 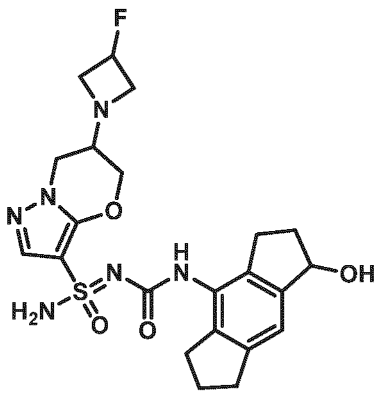 |
| 589 | 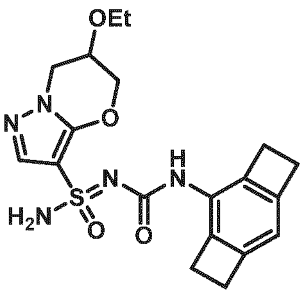 | 706 | 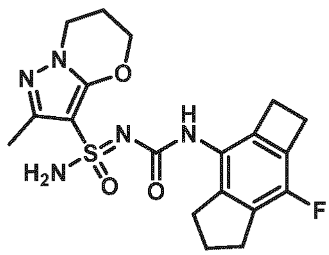 |
| 590 | 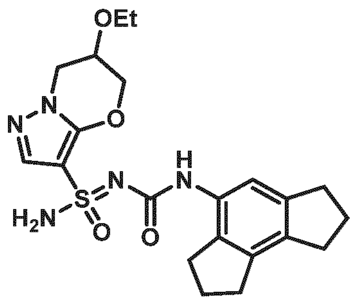 | 707 | 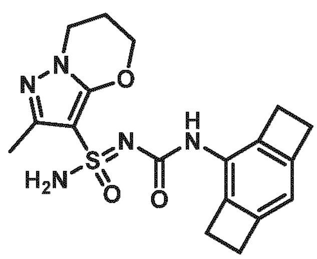 |

(fortgesetzt)

| | | | |
|---|---|---|---|
| 591 | 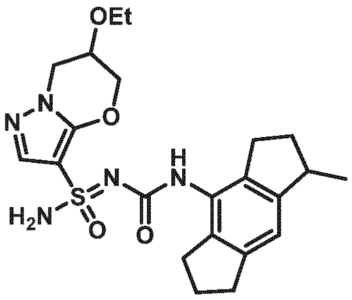 | 708 | 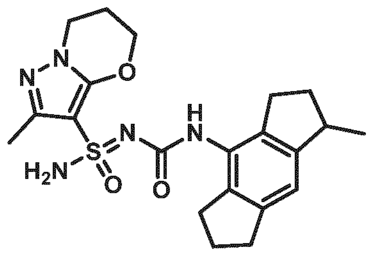 |
| 592 | 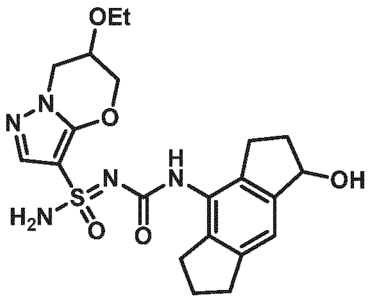 | 709 | 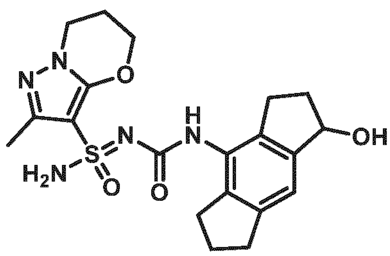 |
| 593 | 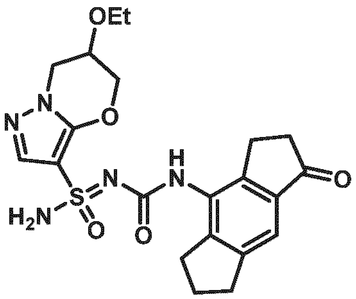 | 710 | 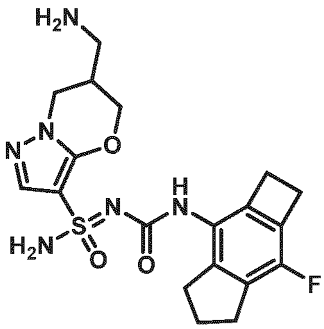 |
| 594 | 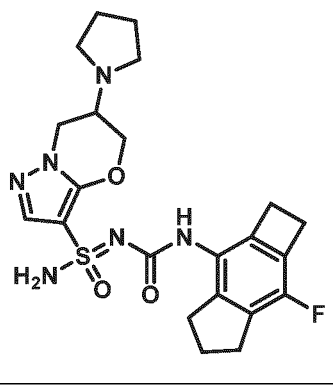 | 711 | 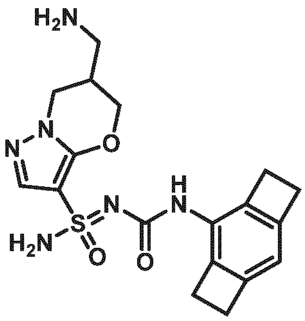 |

(fortgesetzt)

| | | | |
|---|---|---|---|
| 595 | 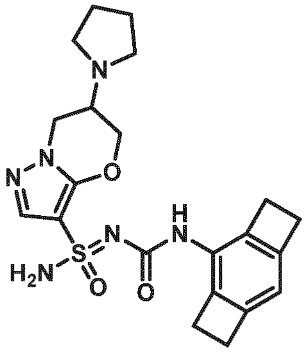 | 712 | 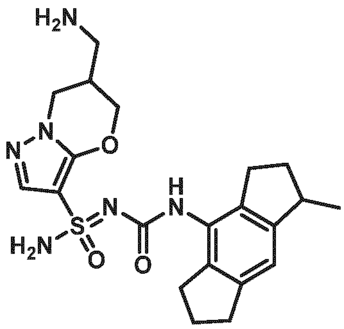 |
| 596 | 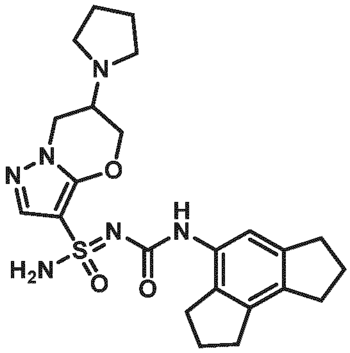 | 713 | 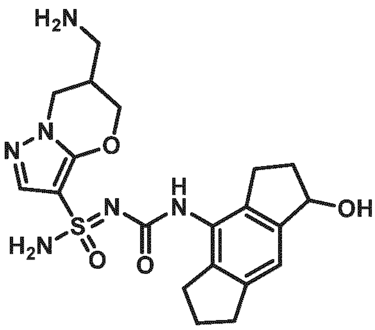 |
| 597 | 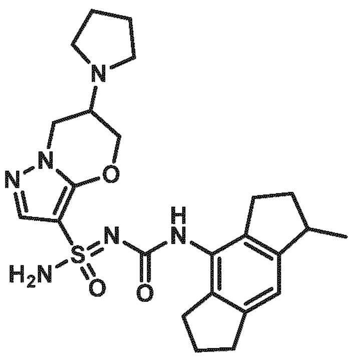 | 714 | 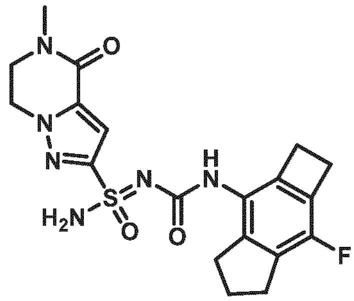 |
| 598 | 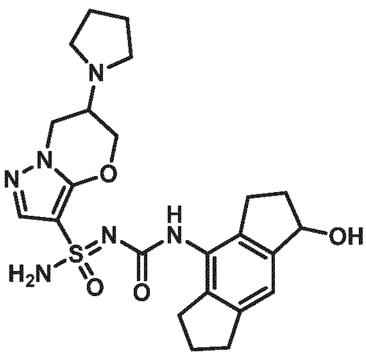 | 715 | 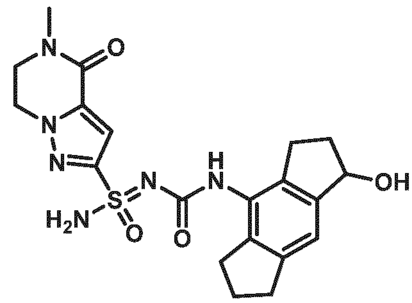 |

(fortgesetzt)

| | | | |
|---|---|---|---|
| 599 | 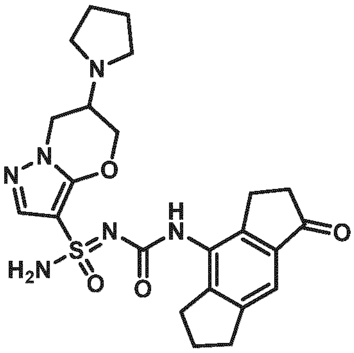 | 716 | 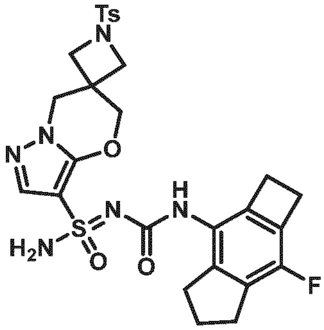 |
| 600 | 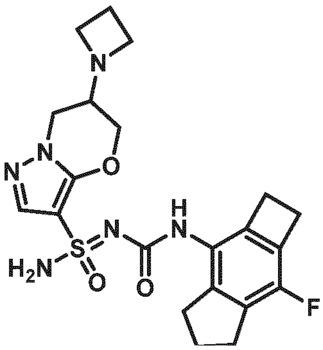 | 717 | 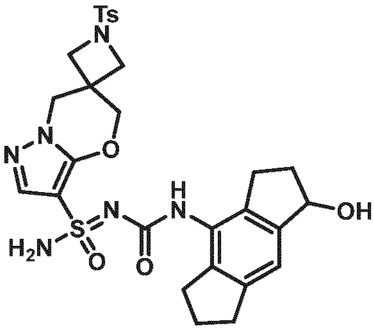 |
| 601 | 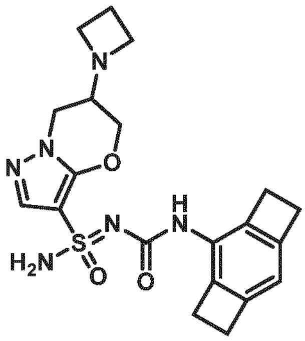 | 718 | 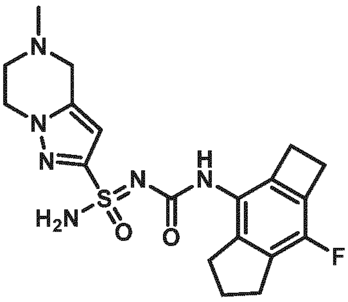 |
| 602 | 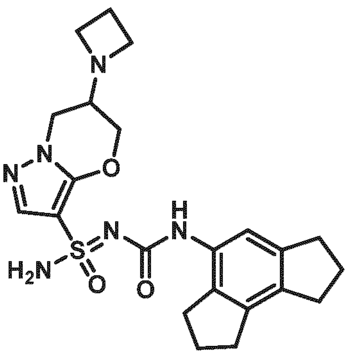 | 719 | 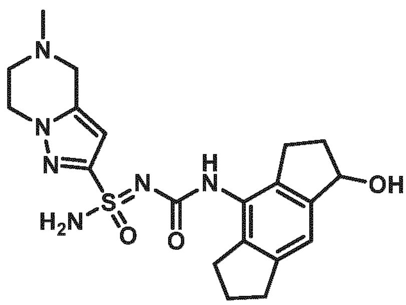 |

(fortgesetzt)

| | | | |
|---|---|---|---|
| 603 | 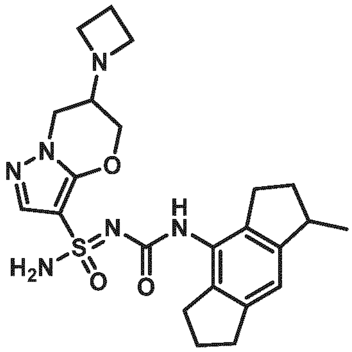 | 720 | 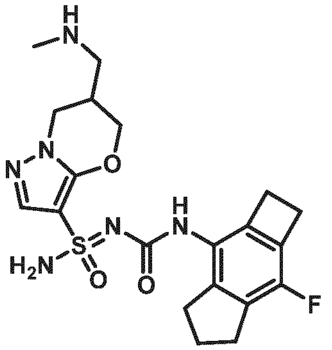 |
| 604 | 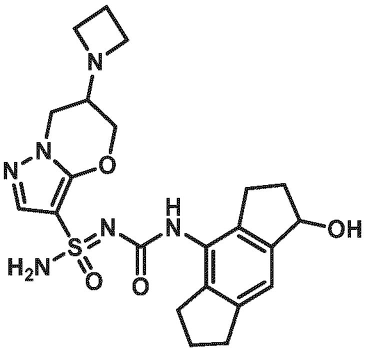 | 721 | 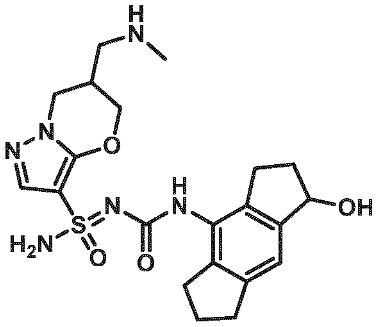 |
| 605 | 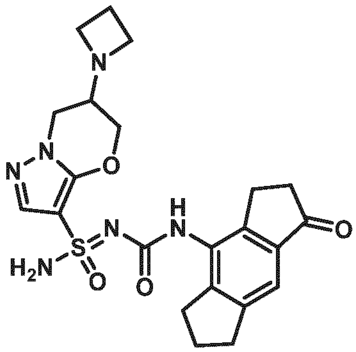 | 722 | 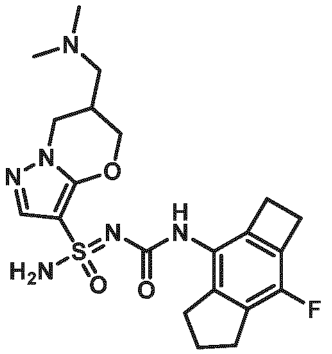 |
| 606 | 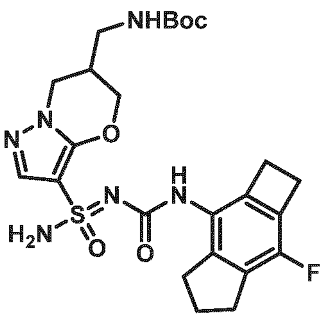 | 723 | 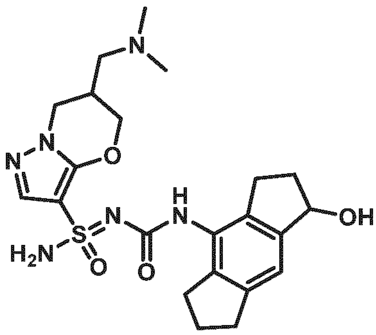 |

(fortgesetzt)

| | | | |
|---|---|---|---|
| 607 | 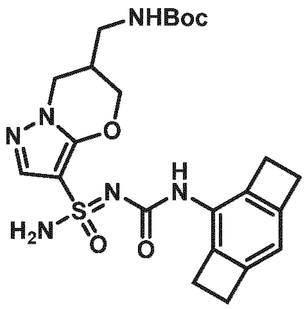 | 724 | 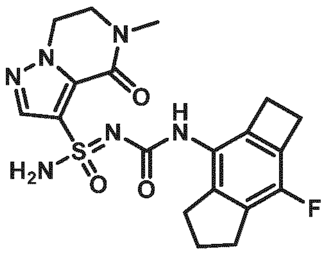 |
| 608 | 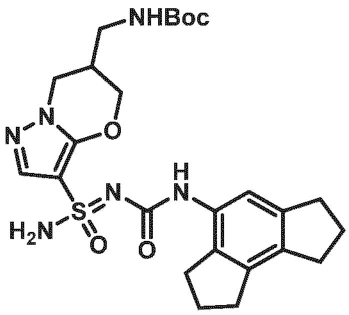 | 725 | 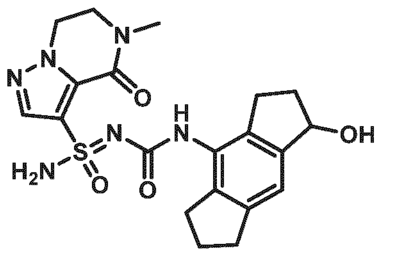 |
| 609 | 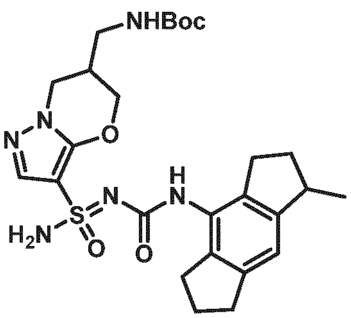 | 726 | 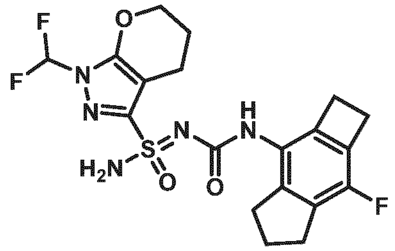 |
| 610 | 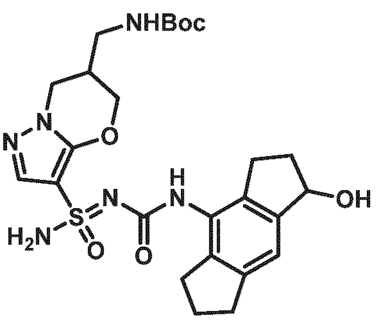 | 727 | 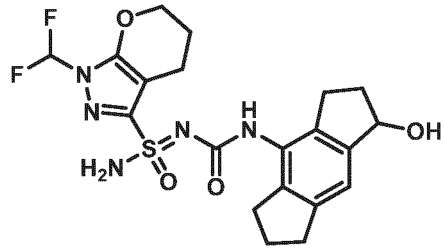 |
| 611 | 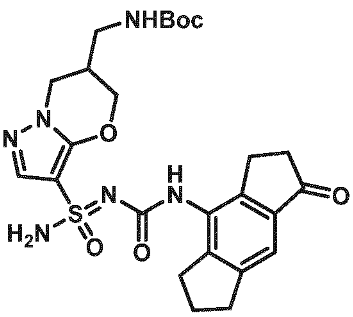 | 728 | 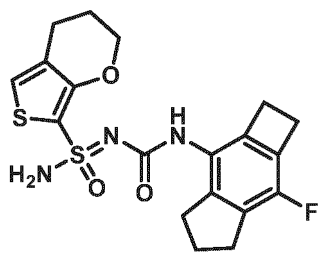 |

(fortgesetzt)

| | | | |
|---|---|---|---|
| 612 | 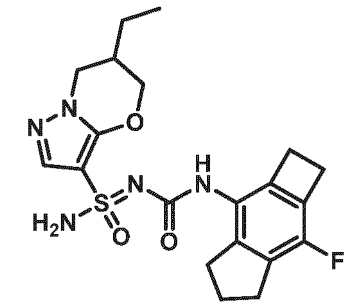 | 729 | 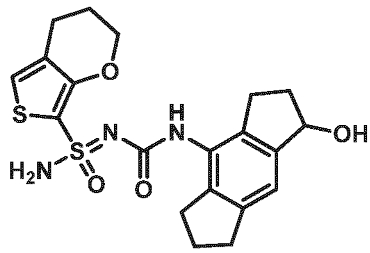 |
| 613 | 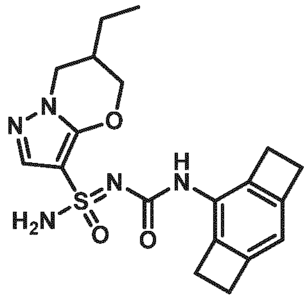 | 730 | 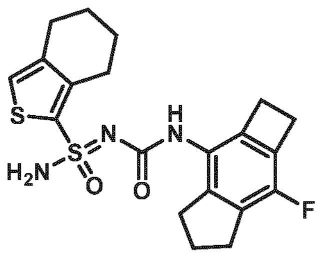 |
| 614 | 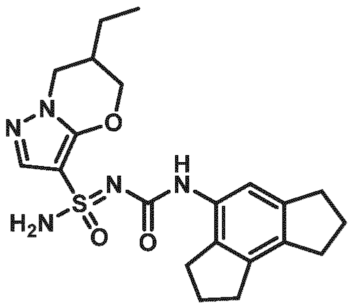 | 731 | 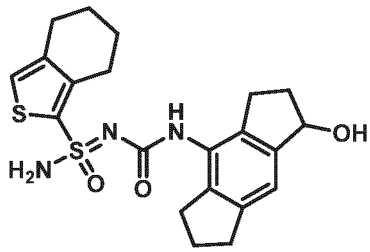 |
| 615 | 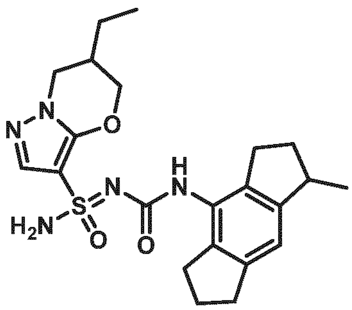 | 732 | 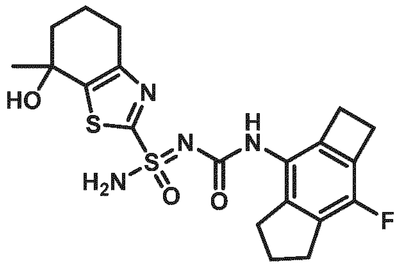 |
| 616 | 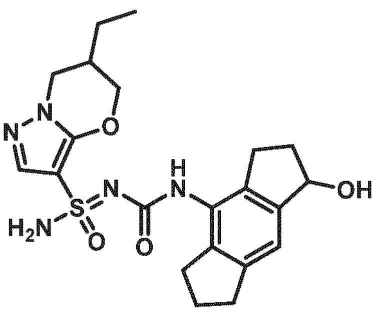 | 733 | 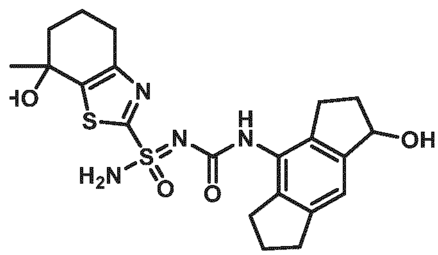 |

(fortgesetzt)

| | | | |
|---|---|---|---|
| 617 | 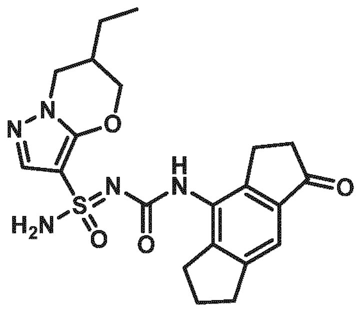 | 734 | 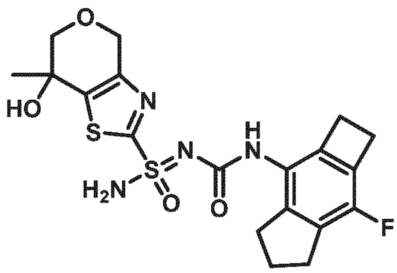 |
| | | 735 | 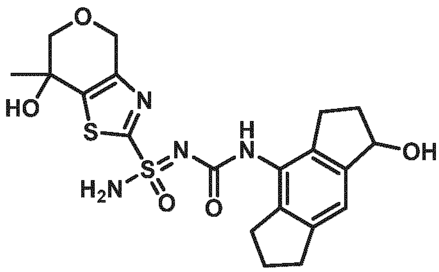 |

oder ein pharmazeutisch unbedenkliches Salz davon.

2. Verbindung nach Anspruch 1, wobei der Schwefel in der Gruppierung $S(=O)(NHR^3)=N-$ (S)-Stereochemie aufweist.

3. Verbindung nach Anspruch 1, wobei der Schwefel in der Gruppierung $S(=O)(NHR^3)=N-$ (R)-Stereochemie aufweist.

4. Pharmazeutische Zusammensetzung, umfassend eine Verbindung oder ein Salz gemäß einem der Ansprüche 1-3 und einen oder mehrere pharmazeutisch unbedenkliche Hilfsstoffe.

## Revendications

1. Composé choisi dans le groupe constitué par les composés ci-dessous :

| | | | |
|---|---|---|---|
| 501 | 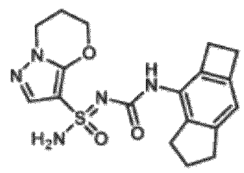 | 618 | 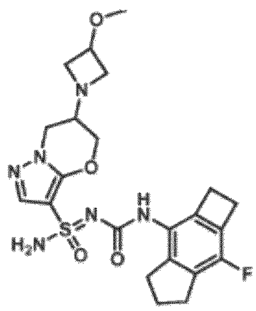 |

(suite)

| | | | |
|---|---|---|---|
| 502 | 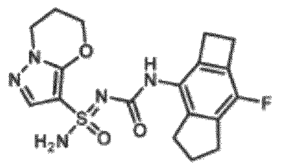 | 619 | 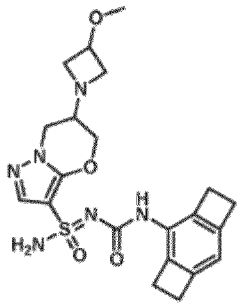 |
| 503 | 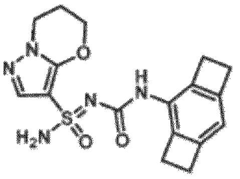 | 620 | 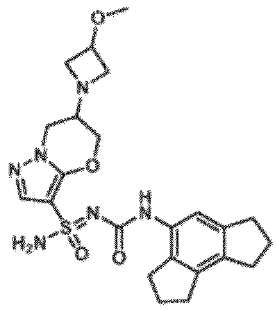 |
| 504 | 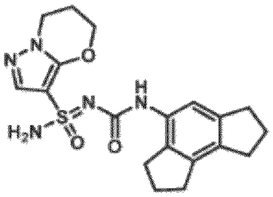 | 621 | 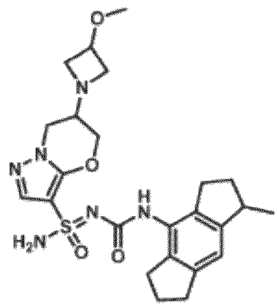 |
| 505 | 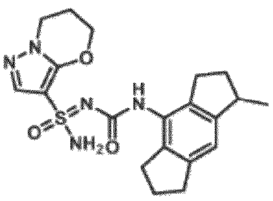 | 622 | 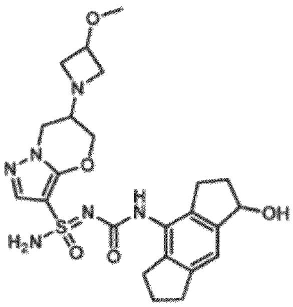 |
| 506 | 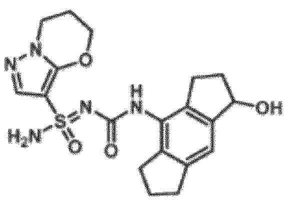 | 623 | 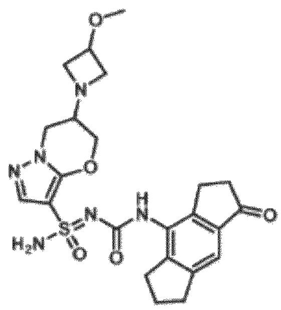 |

(suite)

| | | | |
|---|---|---|---|
| 507 | 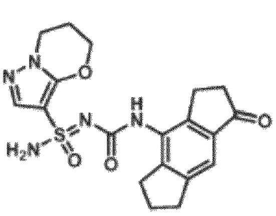 | 624 | 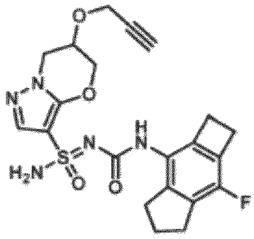 |
| 508 | 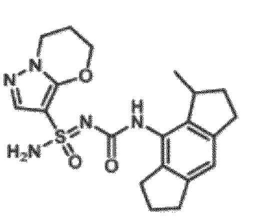 | 625 | 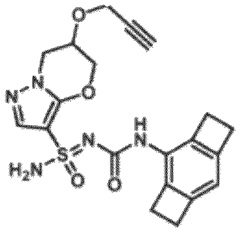 |
| 509 | 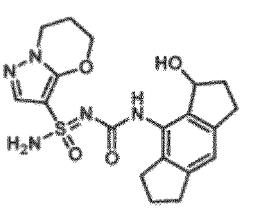 | 626 | 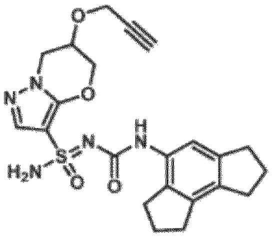 |
| 510 | 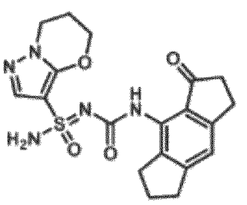 | 627 | 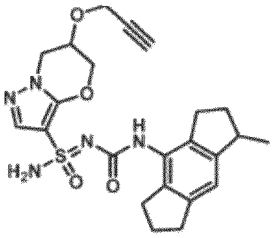 |
| 511 | 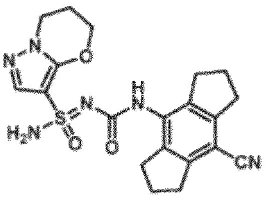 | 628 | 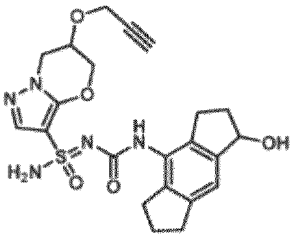 |
| 512 | 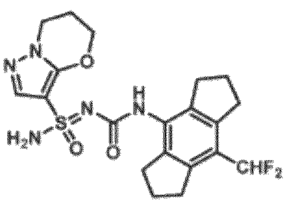 | 629 | 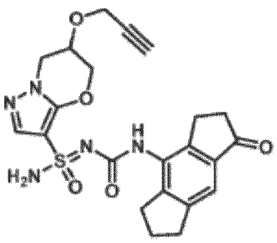 |

(suite)

| | | | |
|---|---|---|---|
| 513 | 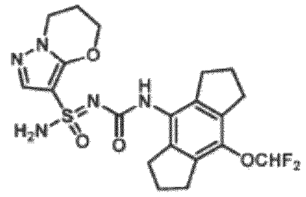 | 630 | 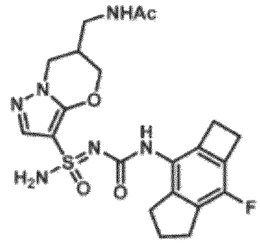 |
| 514 | 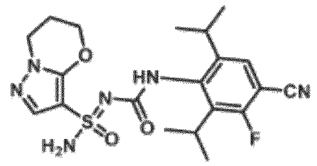 | 631 | 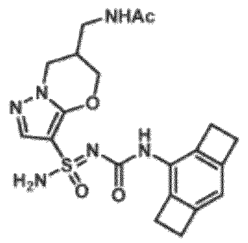 |
| 515 | 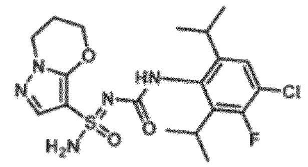 | 632 | 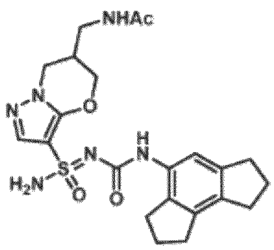 |
| 516 | 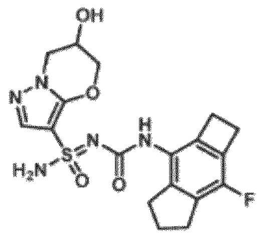 | 633 | 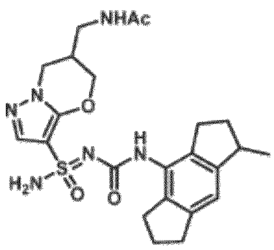 |
| 517 | 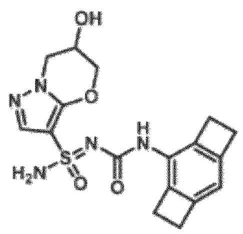 | 634 | 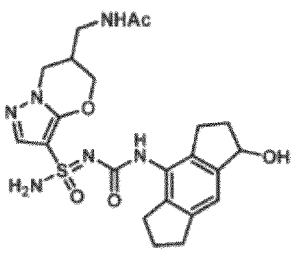 |
| 518 | 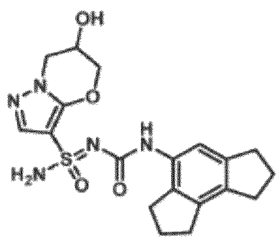 | 635 | 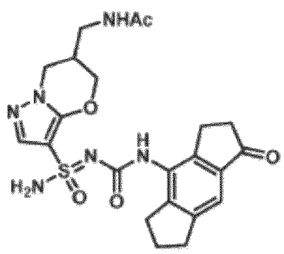 |

(suite)

| | | | |
|---|---|---|---|
| 519 | 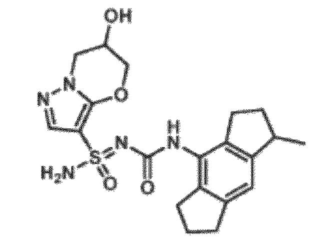 | 636 | 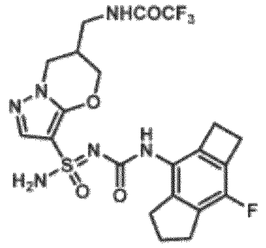 |
| 520 | 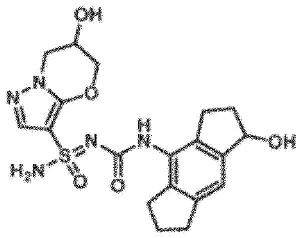 | 637 | 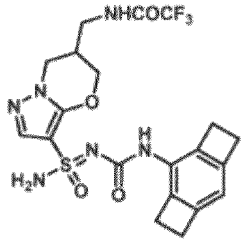 |
| 521 | 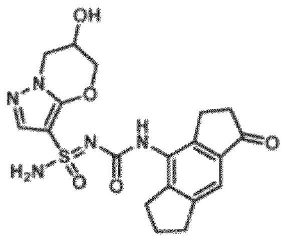 | 638 | 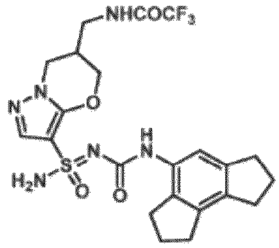 |
| 522 | 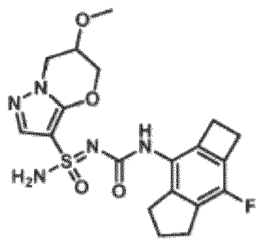 | 639 | 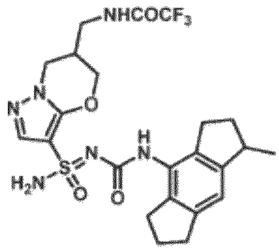 |
| 523 | 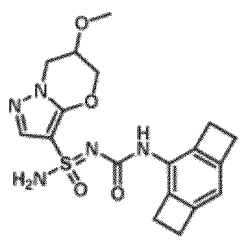 | 640 | 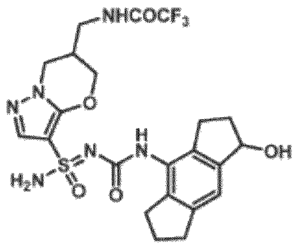 |
| 524 | 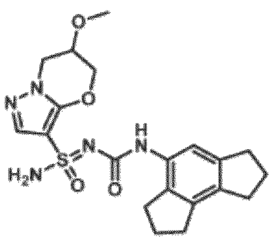 | 641 | 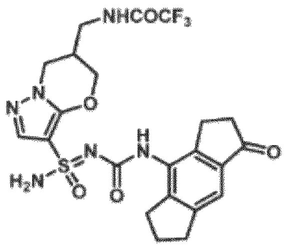 |

(suite)

| | | | |
|---|---|---|---|
| 525 | 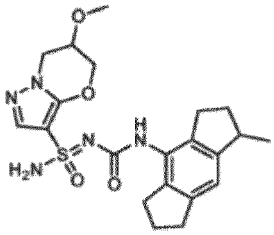 | 642 | 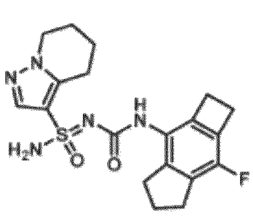 |
| 526 | 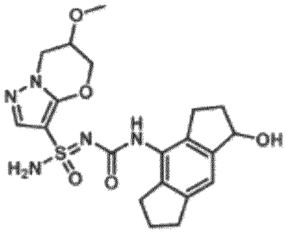 | 643 | 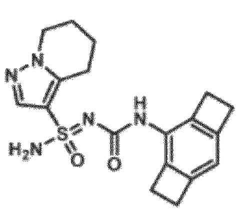 |
| 527 | 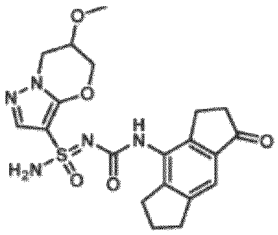 | 644 | 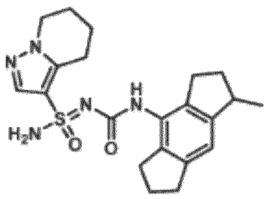 |
| 528 | 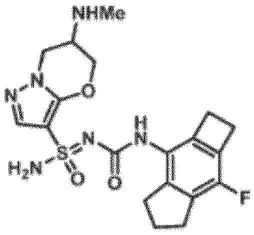 | 645 | 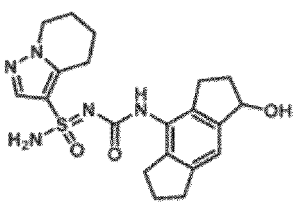 |
| 529 | 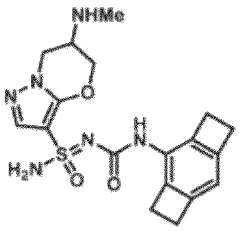 | 646 | 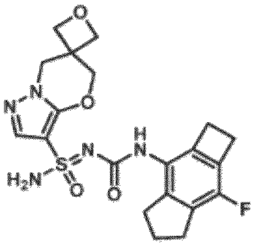 |
| 530 | 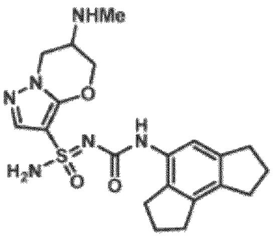 | 647 | 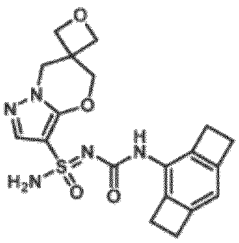 |

(suite)

| | | | |
|---|---|---|---|
| 531 | 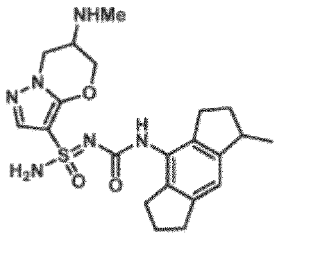 | 448 | 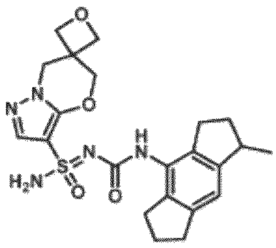 |
| 532 | 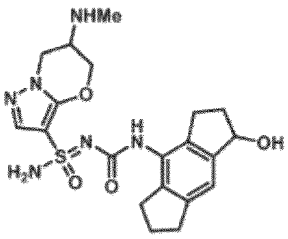 | 649 | 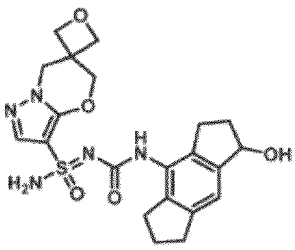 |
| 533 | 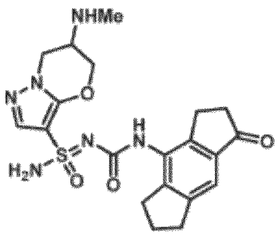 | 650 | 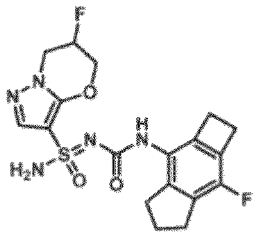 |
| 534 | 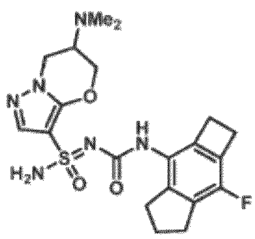 | 651 | 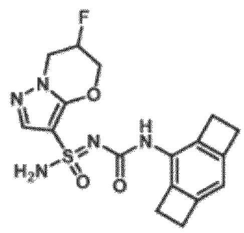 |
| 535 | 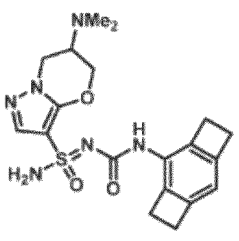 | 652 | 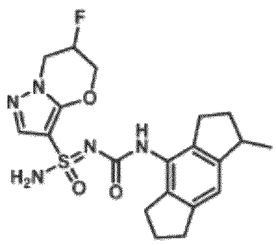 |
| 536 | 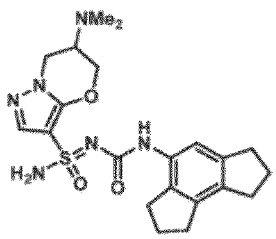 | 653 | 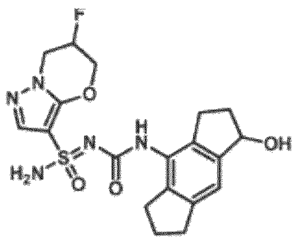 |

(suite)

| | | | |
|---|---|---|---|
| 537 | 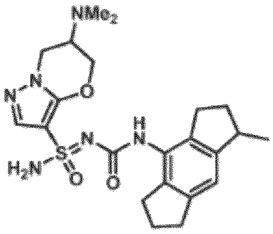 | 654 | 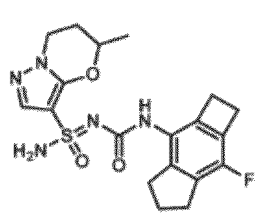 |
| 538 | 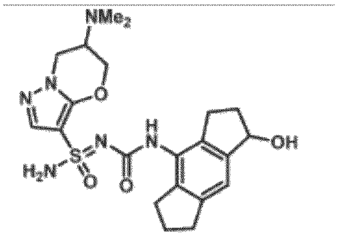 | 655 | 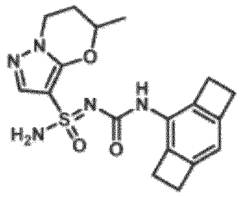 |
| 539 | 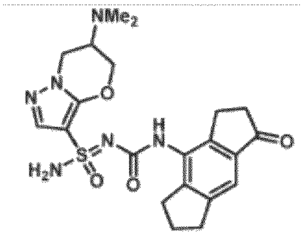 | 656 | 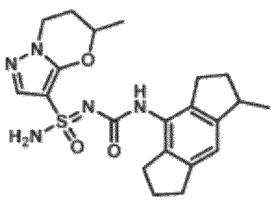 |
| 540 | 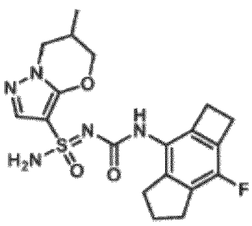 | 657 | 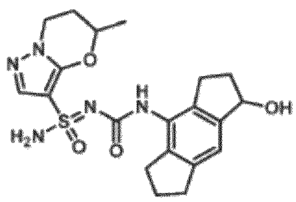 |
| 541 | 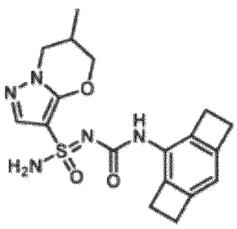 | 658 | 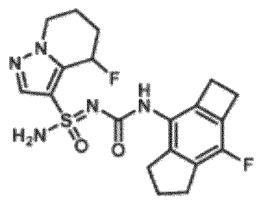 |
| 542 | 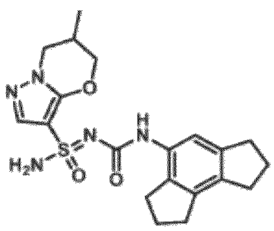 | 659 | 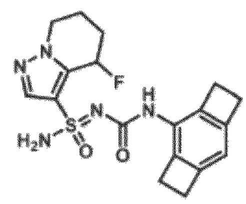 |

(suite)

| | | | |
|---|---|---|---|
| 543 | 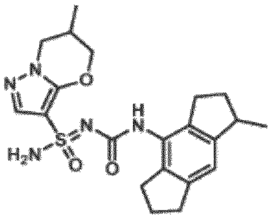 | 660 | 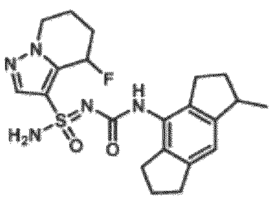 |
| 544 | 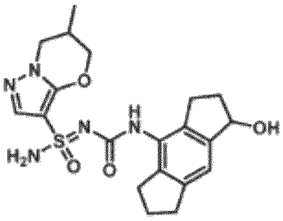 | 661 | 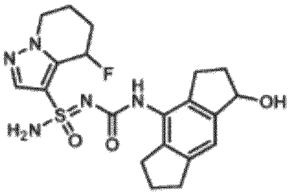 |
| 545 | 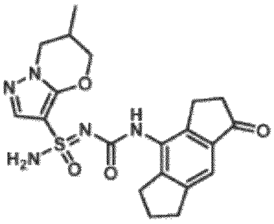 | 662 | 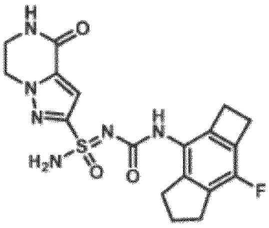 |
| 546 | 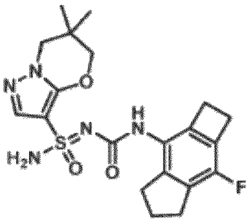 | 663 | 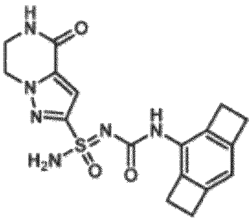 |
| 547 | 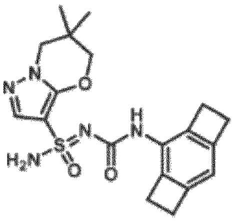 | 664 | 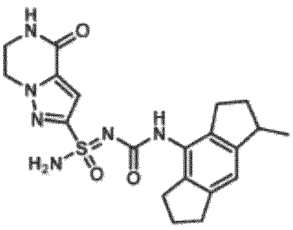 |
| 548 | 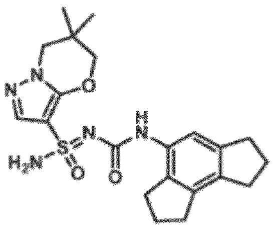 | 665 | 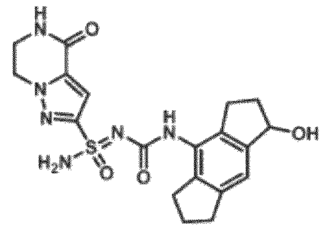 |

(suite)

| | | | |
|---|---|---|---|
| 549 | 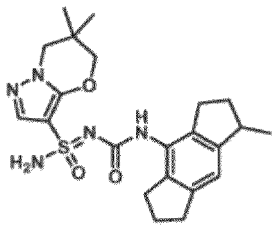 | 666 | 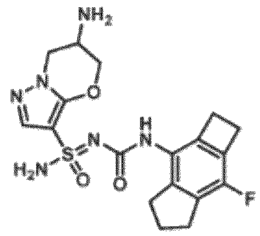 |
| 550 | 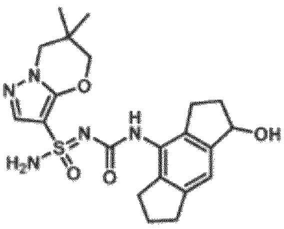 | 667 | 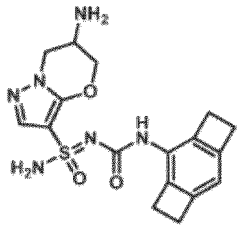 |
| 551 | 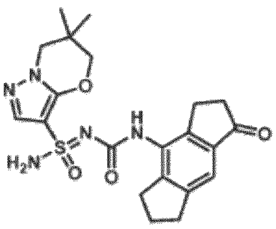 | 668 | 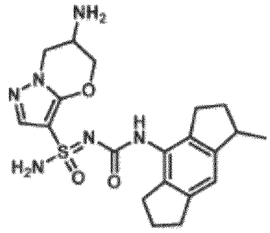 |
| 552 | 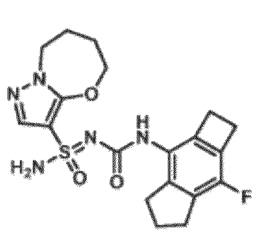 | 669 | 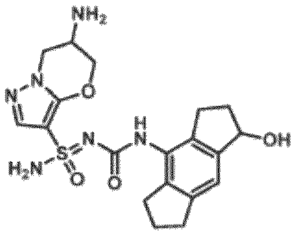 |
| 553 | 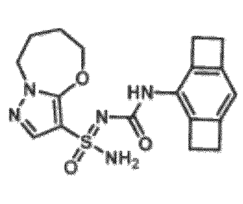 | 670 | 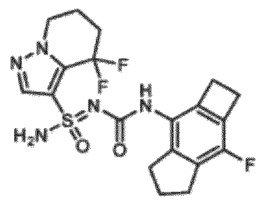 |
| 554 | 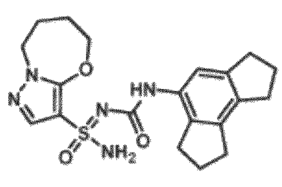 | 671 | 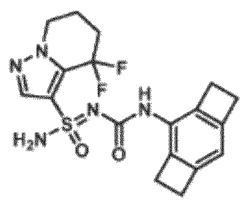 |
| 555 | 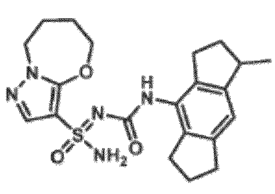 | 672 | 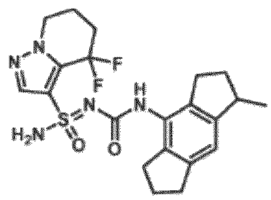 |

(suite)

| | | | |
|---|---|---|---|
| 556 | 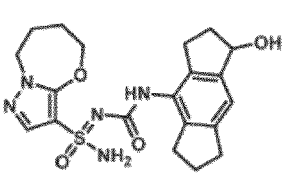 | 673 | 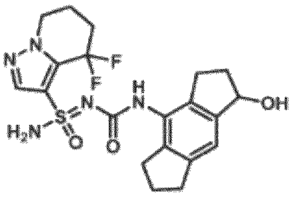 |
| 557 | 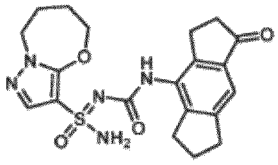 | 614 | 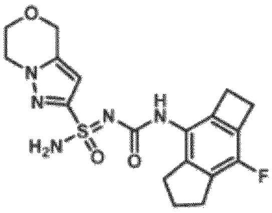 |
| 558 | 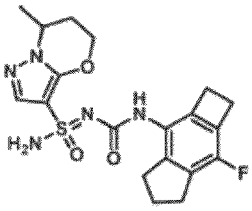 | 675 | 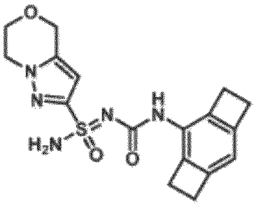 |
| 559 | 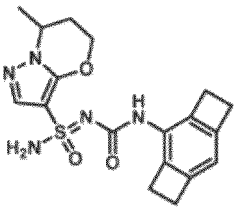 | 676 | 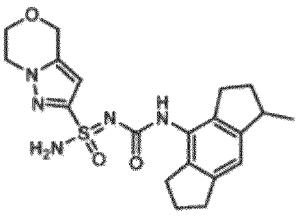 |
| 560 | 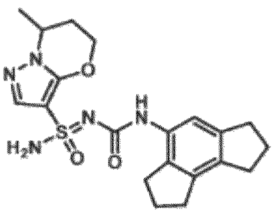 | 677 | 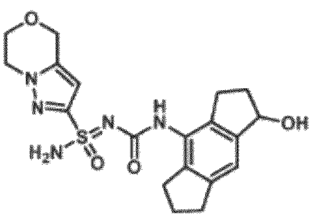 |
| 561 | 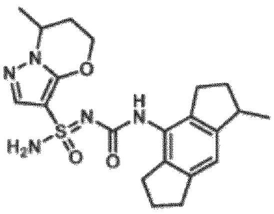 | 678 | 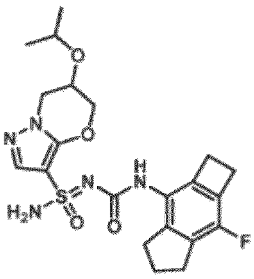 |

(suite)

| | | | |
|---|---|---|---|
| 562 | 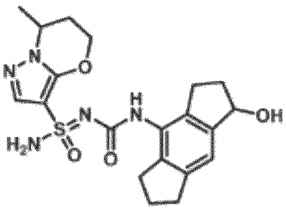 | 679 | 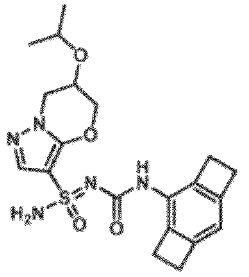 |
| 563 | 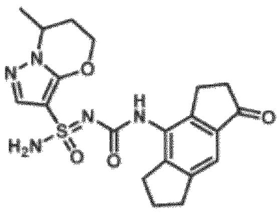 | 680 | 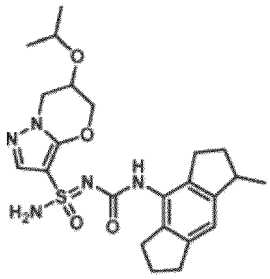 |
| 564 | 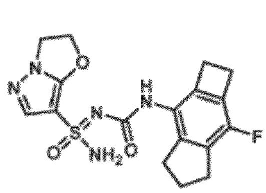 | 681 | 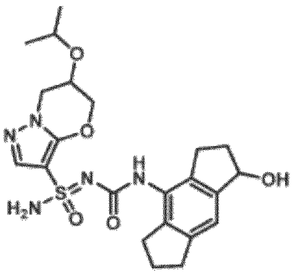 |
| 565 | 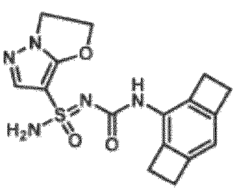 | 682 | 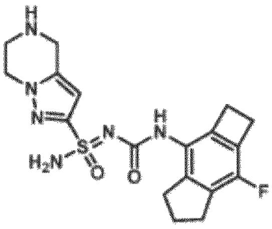 |
| 566 | 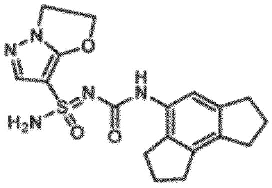 | 683 | 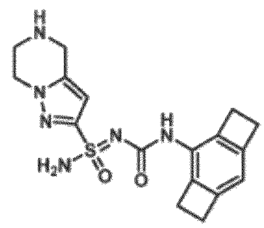 |
| 567 | 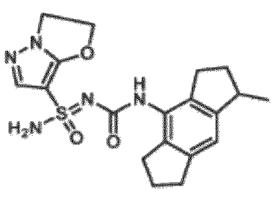 | 684 | 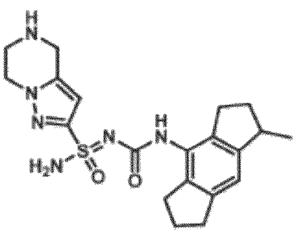 |

(suite)

| | | | |
|---|---|---|---|
| 568 | 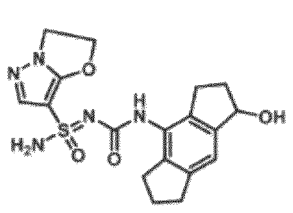 | 685 | 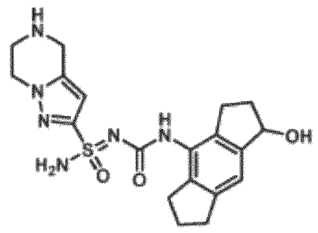 |
| 569 | 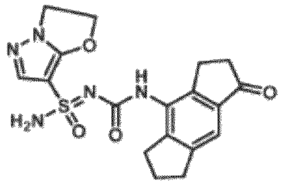 | 686 | 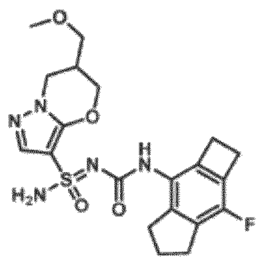 |
| 570 | 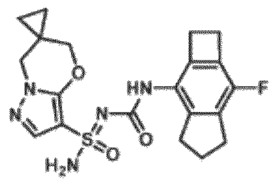 | 687 | 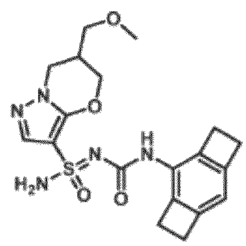 |
| 571 | 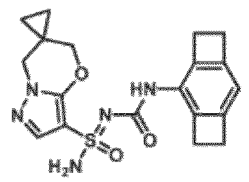 | 688 | 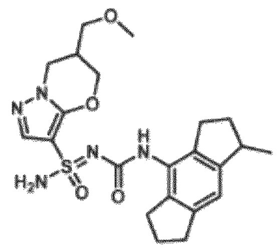 |
| 572 | 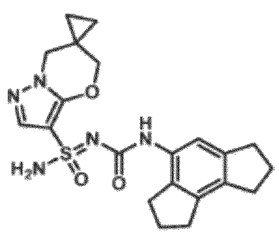 | 689 | 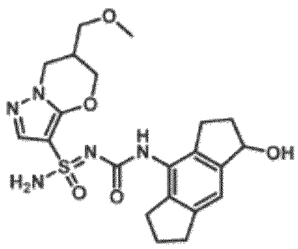 |
| 573 | 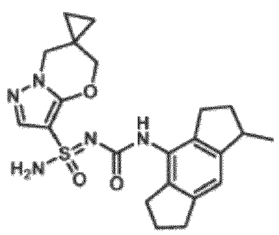 | 690 | 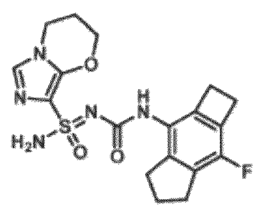 |

(suite)

| | | | |
|---|---|---|---|
| 574 | 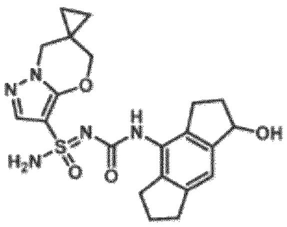 | 691 | 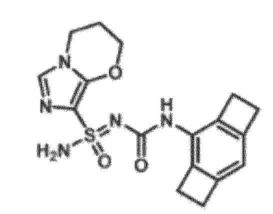 |
| 515 | 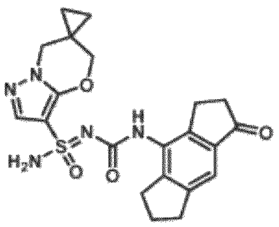 | 692 | 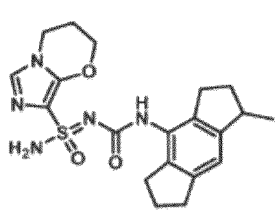 |
| 576 | 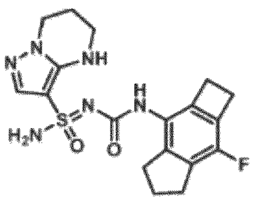 | 693 | 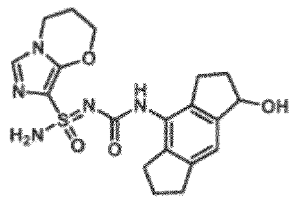 |
| 577 | 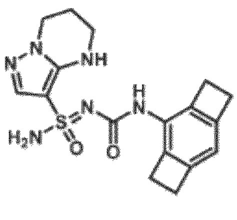 | 694 | 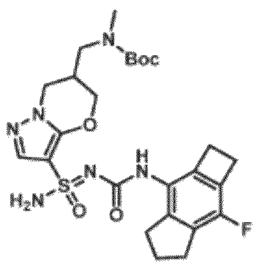 |
| 578 | 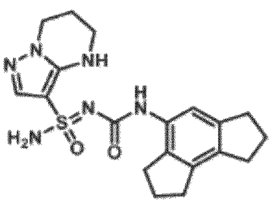 | 695 | 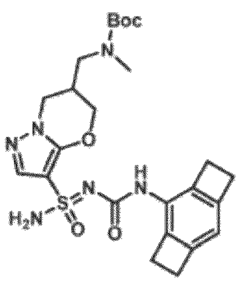 |
| 579 | 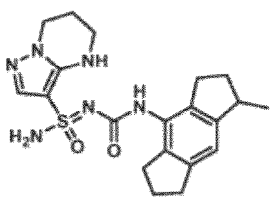 | 696 | 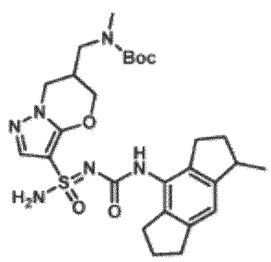 |

(suite)

| | | | |
|---|---|---|---|
| 580 | 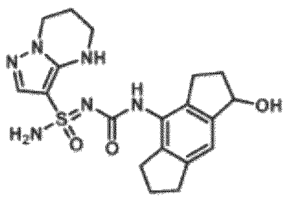 | 697 | 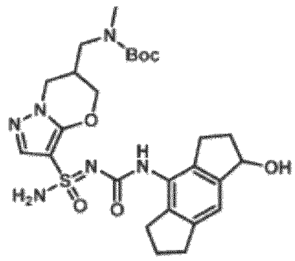 |
| 581 | 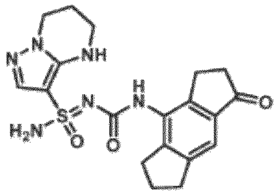 | 698 | 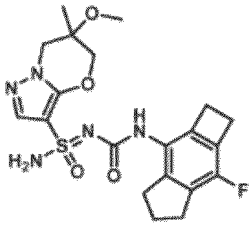 |
| 582 | 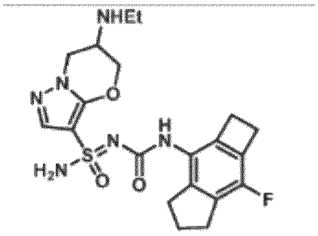 | 699 | 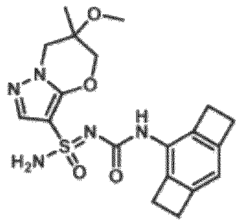 |
| 583 | 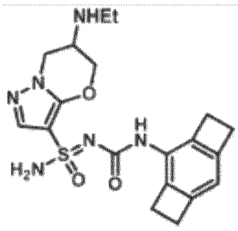 | 700 | 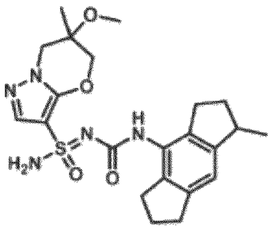 |
| 584 | 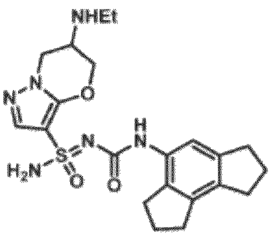 | 701 | 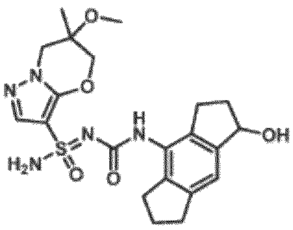 |
| 585 | 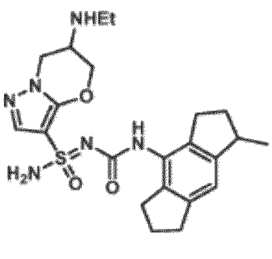 | 702 | 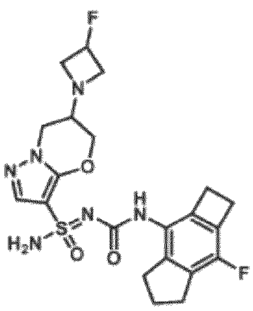 |

(suite)

| | | | |
|---|---|---|---|
| 586 | 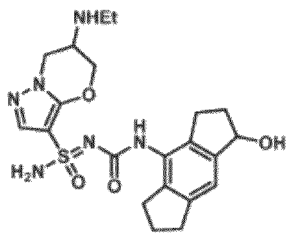 | 703 | 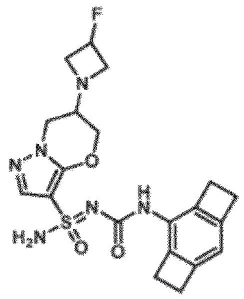 |
| 587 | 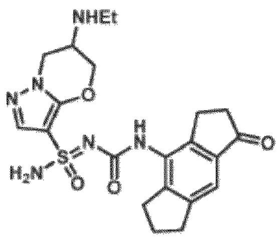 | 704 | 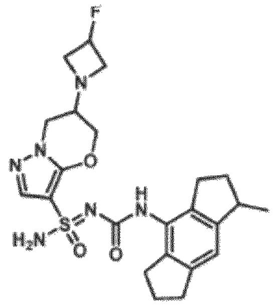 |
| 588 | 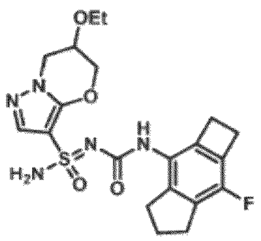 | 705 | 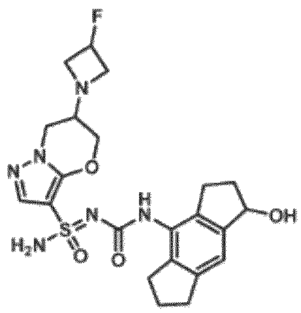 |
| 589 | 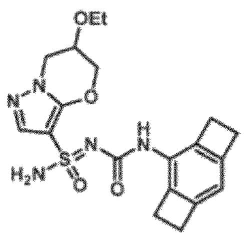 | 706 | 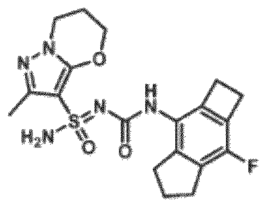 |
| 590 | 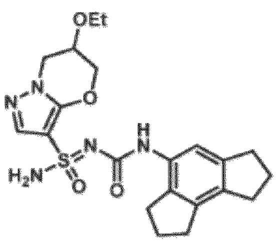 | 707 | 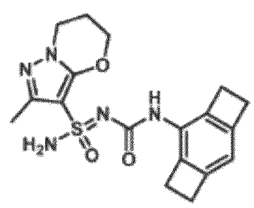 |

(suite)

| | | | |
|---|---|---|---|
| 591 | 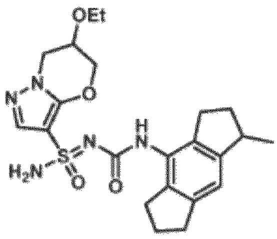 | 708 | 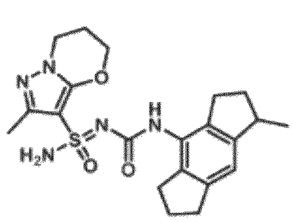 |
| 592 | 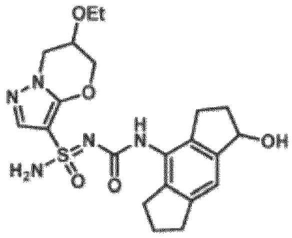 | 709 | 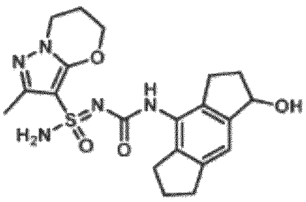 |
| 593 | 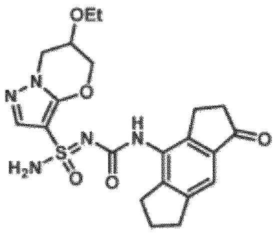 | 710 | 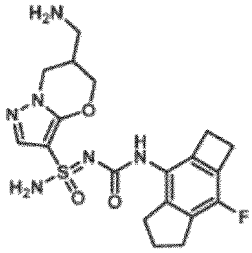 |
| 594 | 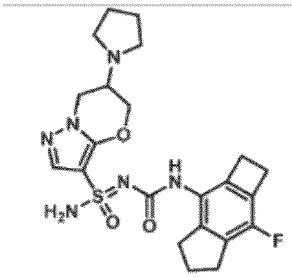 | 711 | 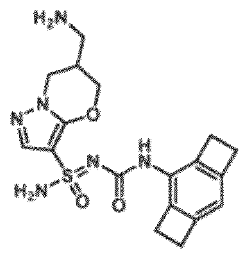 |
| 595 | 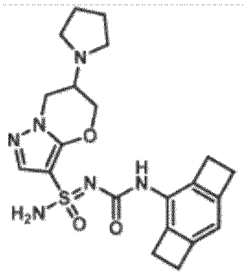 | 712 | 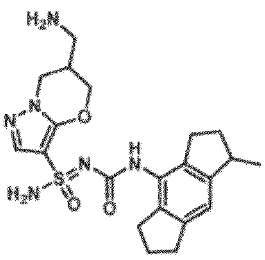 |
| 596 | 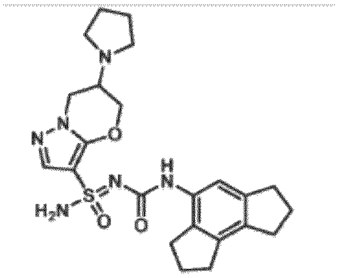 | 713 | 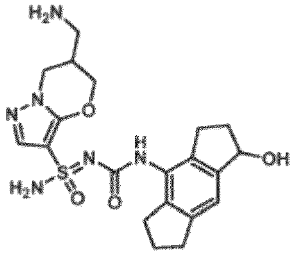 |

(suite)

| | | | |
|---|---|---|---|
| 597 | 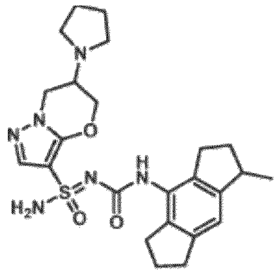 | 714 | 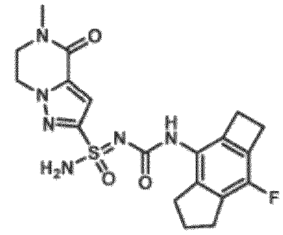 |
| 598 | 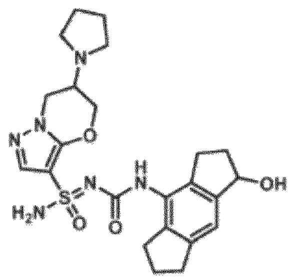 | 715 | 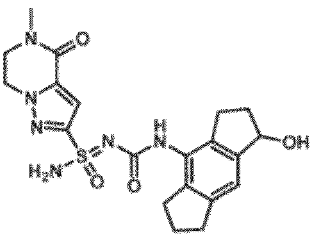 |
| 599 | 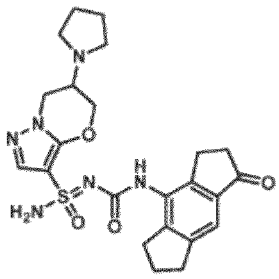 | 716 | 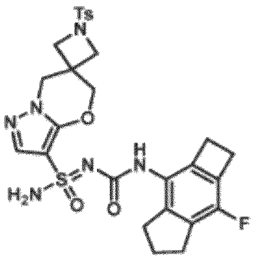 |
| 600 | 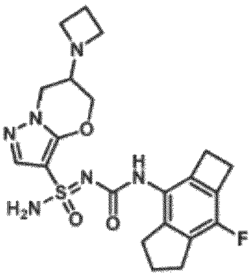 | 717 | 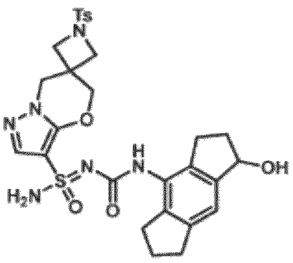 |
| 601 | 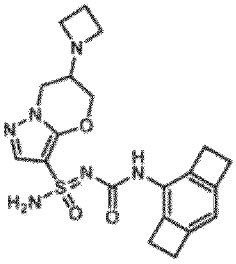 | 718 | 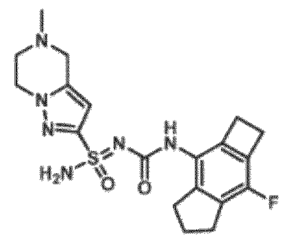 |

(suite)

| | | | |
|---|---|---|---|
| 602 | 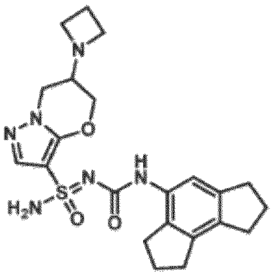 | 719 | 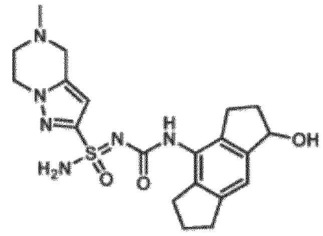 |
| 603 | 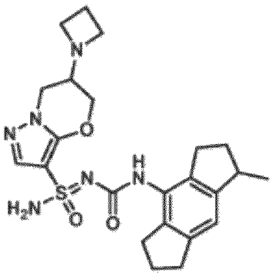 | 720 | 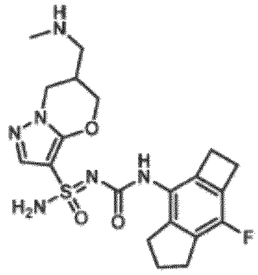 |
| 604 | 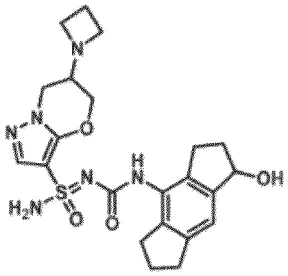 | 721 | 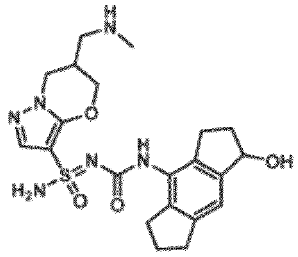 |
| 605 | 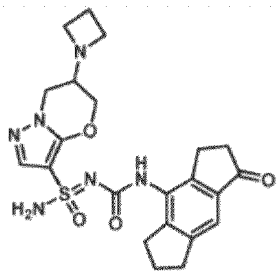 | 722 | 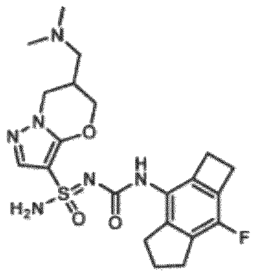 |
| 606 | 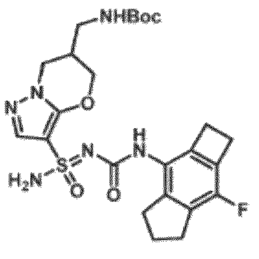 | 723 | 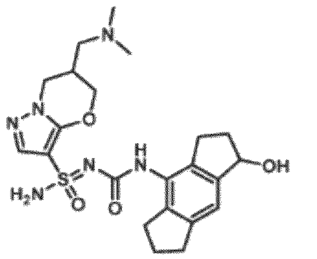 |

(suite)

| | | | |
|---|---|---|---|
| 607 | 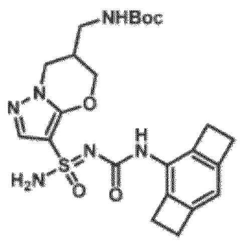 | 724 | 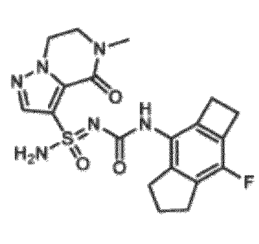 |
| 608 | 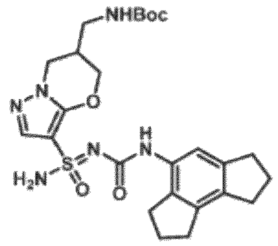 | 725 | 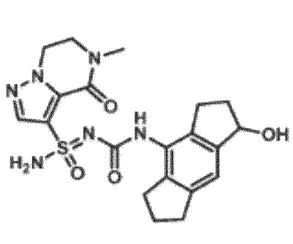 |
| 609 | 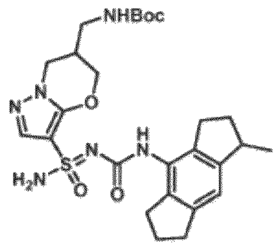 | 726 | 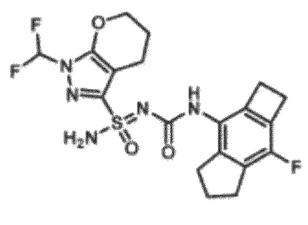 |
| 610 | 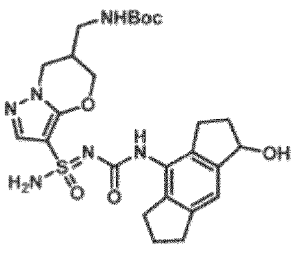 | 727 | 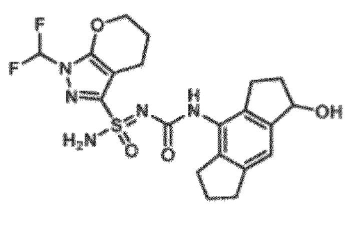 |
| 611 | 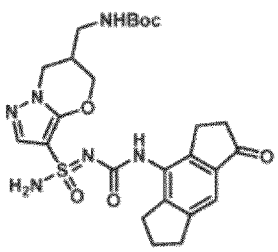 | 728 | 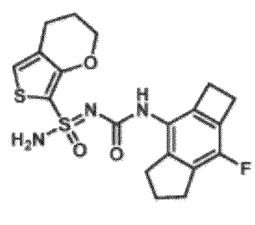 |
| 612 | 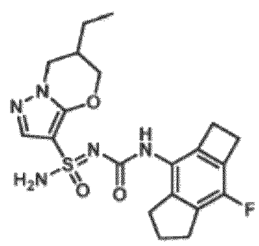 | 729 | 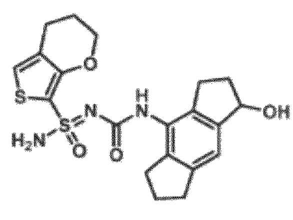 |

(suite)

| | | | |
|---|---|---|---|
| 613 | 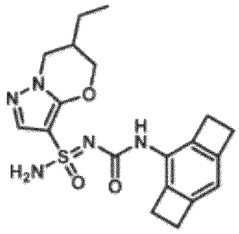 | 730 | 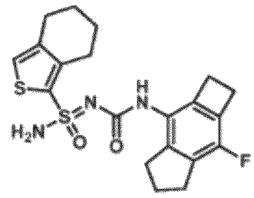 |
| 614 | 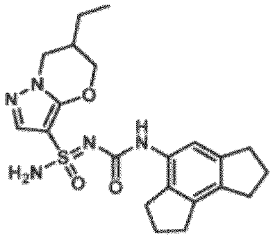 | 731 | 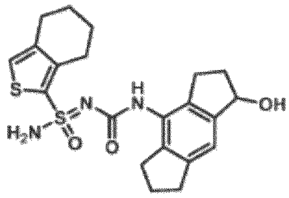 |
| 615 | 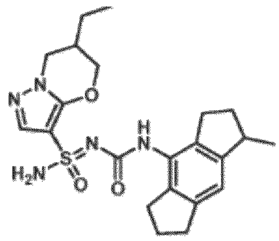 | 732 | 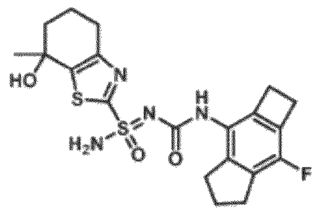 |
| 616 | 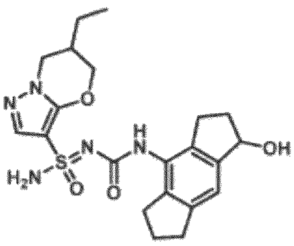 | 733 | 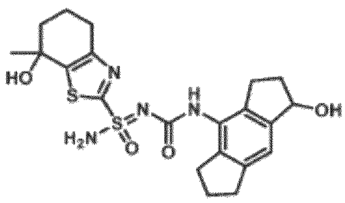 |
| 617 | 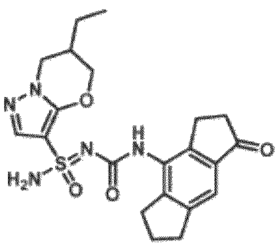 | 734 | 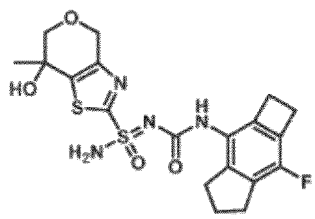 |
| | | 735 | 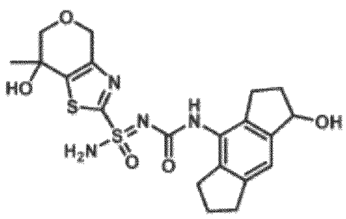 |

ou sel pharmaceutiquement acceptable correspondant.

**2.** Composé selon la revendication 1, le soufre dans le groupement S(=O)($NHR^3$)=N- ayant une stéréochimie (S).

**3.** Composé selon la revendication 1, le soufre dans le groupement S(=O)($NHR^3$)=N- ayant une stéréochimie (R).

**4.** Composition pharmaceutique comprenant un composé ou un sel selon l'une quelconque des revendications 1 à 3 et un ou plusieurs excipients pharmaceutiquement acceptables.

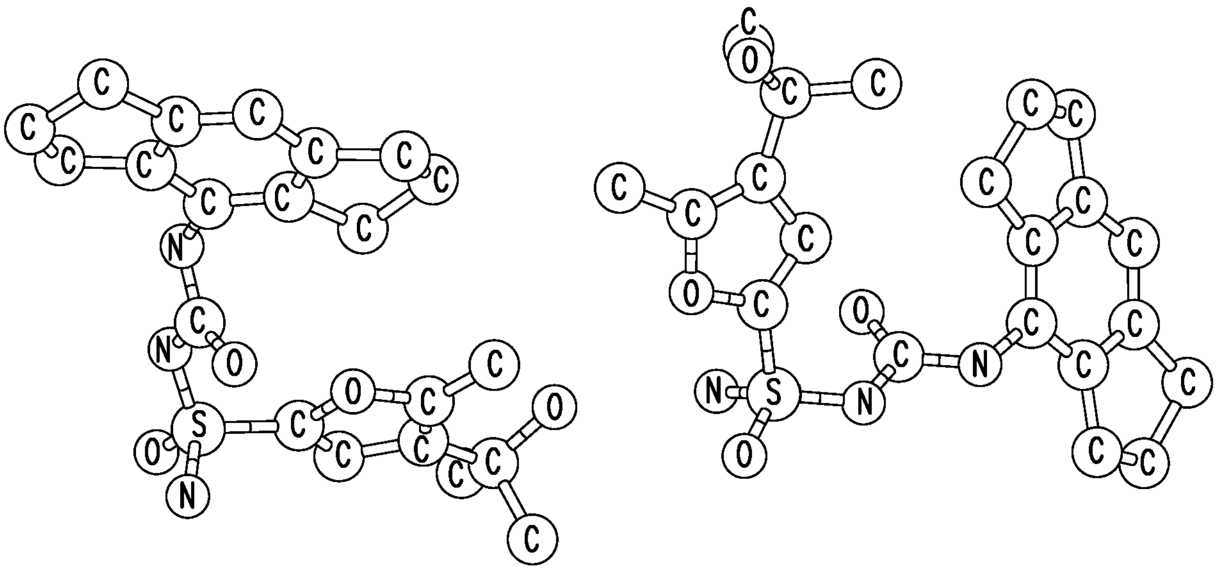

FIG.1

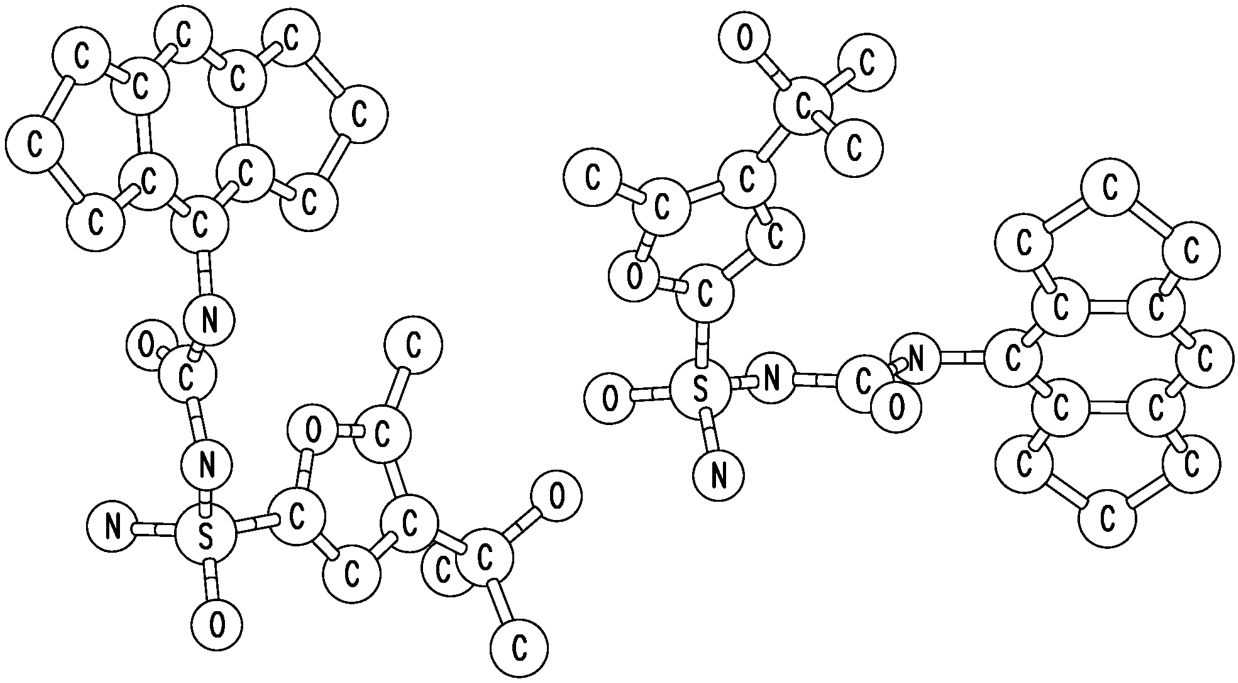

FIG.2

| | 1 | 2 | 3 | 4 | 5 | 6 | 7 | 8 | 9 | 10 | 10 | 10 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| A | | | | | | | | | | | Media | | | | | | | | | | | | | |
| B | | | Ref | Ref | Ref | Ref | Ref | Ref | Ref | Ref | Ref | Ref | Ref | Ref | Ref | Ref | Ref | Ref | Ref | Ref | Ref | Ref | | |
| C | | | cmpd1 | cmpd1 | cmpd1 | cmpd1 | cmpd1 | cmpd1 | cmpd1 | cmpd1 | cmpd1 | cmpd1 | cmpd1 | cmpd1 | cmpd1 | cmpd1 | cmpd1 | cmpd1 | cmpd1 | cmpd1 | cmpd1 | cmpd1 | | |
| D | | | cmpd2 | cmpd2 | cmpd2 | cmpd2 | cmpd2 | cmpd2 | cmpd2 | cmpd2 | cmpd2 | cmpd2 | cmpd2 | cmpd2 | cmpd2 | cmpd2 | cmpd2 | cmpd2 | cmpd2 | cmpd2 | cmpd2 | cmpd2 | | |
| E | | | cmpd3 | cmpd3 | cmpd3 | cmpd3 | cmpd3 | cmpd3 | cmpd3 | cmpd3 | cmpd3 | cmpd3 | cmpd3 | cmpd3 | cmpd3 | cmpd3 | cmpd3 | cmpd3 | cmpd3 | cmpd3 | cmpd3 | cmpd3 | | |
| F | | | cmpd4 | cmpd4 | cmpd4 | cmpd4 | cmpd4 | cmpd4 | cmpd4 | cmpd4 | cmpd4 | cmpd4 | cmpd4 | cmpd4 | cmpd4 | cmpd4 | cmpd4 | cmpd4 | cmpd4 | cmpd4 | cmpd4 | cmpd4 | | |
| G | | | cmpd5 | cmpd5 | cmpd5 | cmpd5 | cmpd5 | cmpd5 | cmpd5 | cmpd5 | cmpd5 | cmpd5 | cmpd5 | cmpd5 | cmpd5 | cmpd5 | cmpd5 | cmpd5 | cmpd5 | cmpd5 | cmpd5 | cmpd5 | | |
| H | Media | HC | cmpd6 | cmpd6 | cmpd6 | cmpd6 | cmpd6 | cmpd6 | cmpd6 | cmpd6 | cmpd6 | cmpd6 | cmpd6 | cmpd6 | cmpd6 | cmpd6 | cmpd6 | cmpd6 | cmpd6 | cmpd6 | cmpd6 | cmpd6 | LC | Media |
| I | | | cmpd7 | cmpd7 | cmpd7 | cmpd7 | cmpd7 | cmpd7 | cmpd7 | cmpd7 | cmpd7 | cmpd7 | cmpd7 | cmpd7 | cmpd7 | cmpd7 | cmpd7 | cmpd7 | cmpd7 | cmpd7 | cmpd7 | cmpd7 | | |
| J | | | cmpd8 | cmpd8 | cmpd8 | cmpd8 | cmpd8 | cmpd8 | cmpd8 | cmpd8 | cmpd8 | cmpd8 | cmpd8 | cmpd8 | cmpd8 | cmpd8 | cmpd8 | cmpd8 | cmpd8 | cmpd8 | cmpd8 | cmpd8 | | |
| K | | | cmpd9 | cmpd9 | cmpd9 | cmpd9 | cmpd9 | cmpd9 | cmpd9 | cmpd9 | cmpd9 | cmpd9 | cmpd9 | cmpd9 | cmpd9 | cmpd9 | cmpd9 | cmpd9 | cmpd9 | cmpd9 | cmpd9 | cmpd9 | | |
| L | | | cmpd10 | cmpd10 | cmpd10 | cmpd10 | cmpd10 | cmpd10 | cmpd10 | cmpd10 | cmpd10 | cmpd10 | cmpd10 | cmpd10 | cmpd10 | cmpd10 | cmpd10 | cmpd10 | cmpd10 | cmpd10 | cmpd10 | cmpd10 | | |
| M | | | cmpd11 | cmpd11 | cmpd11 | cmpd11 | cmpd11 | cmpd11 | cmpd11 | cmpd11 | cmpd11 | cmpd11 | cmpd11 | cmpd11 | cmpd11 | cmpd11 | cmpd11 | cmpd11 | cmpd11 | cmpd11 | cmpd11 | cmpd11 | | |
| N | | | cmpd12 | cmpd12 | cmpd12 | cmpd12 | cmpd12 | cmpd12 | cmpd12 | cmpd12 | cmpd12 | cmpd12 | cmpd12 | cmpd12 | cmpd12 | cmpd12 | cmpd12 | cmpd12 | cmpd12 | cmpd12 | cmpd12 | cmpd12 | | |
| O | | | cmpd13 | cmpd13 | cmpd13 | cmpd13 | cmpd13 | cmpd13 | cmpd13 | cmpd13 | cmpd13 | cmpd13 | cmpd13 | cmpd13 | cmpd13 | cmpd13 | cmpd13 | cmpd13 | cmpd13 | cmpd13 | cmpd13 | cmpd13 | | |
| P | | | | | | | | | | | Media | | | | | | | | | | | | | |

FIG.3

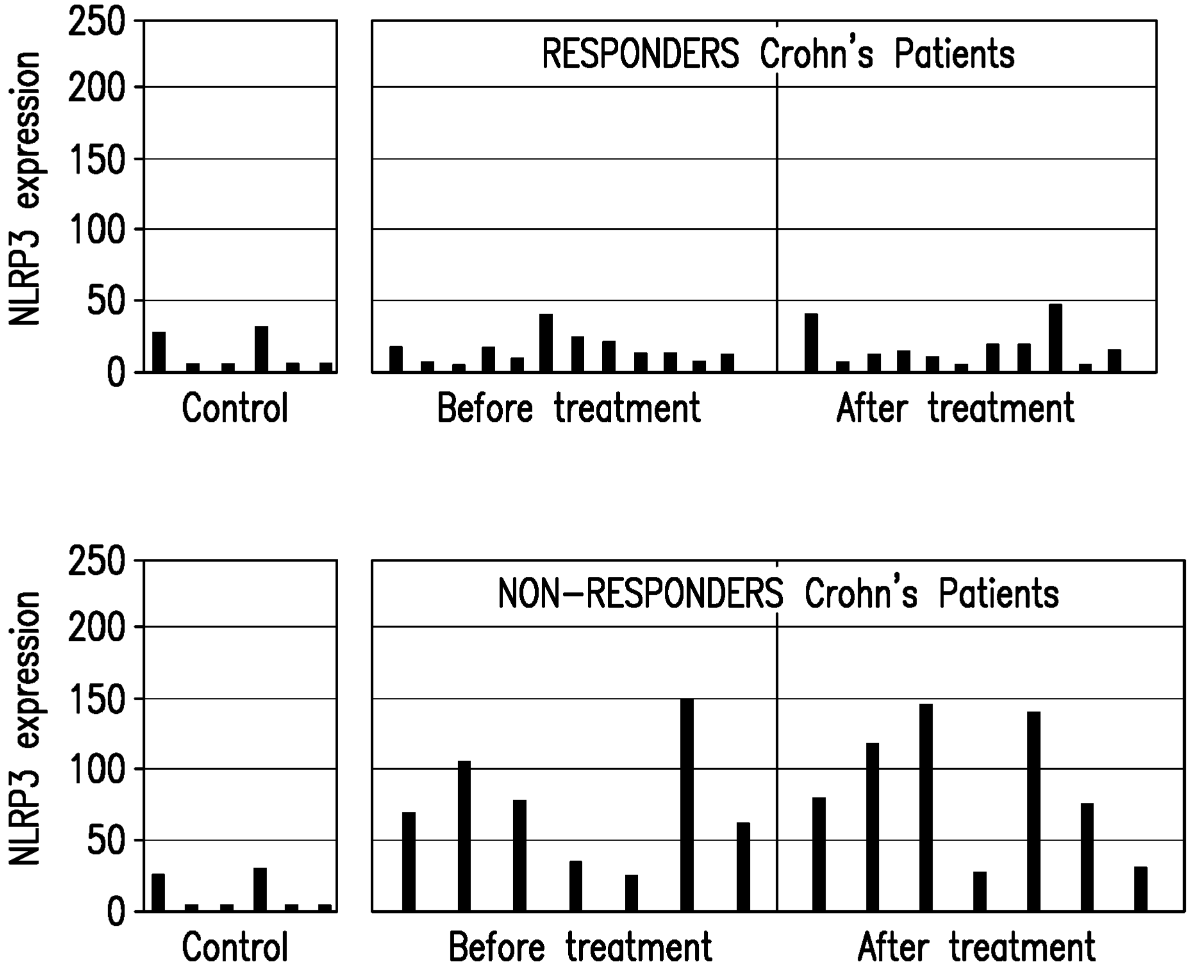

NLRP3 expression
250
200
150
100
50
0
Control
RESPONDERS Crohn's Patients
Before treatment
After treatment
NLRP3 expression
250
200
150
100
50
0
Control
NON-RESPONDERS Crohn's Patients
Before treatment
After treatment


FIG.4

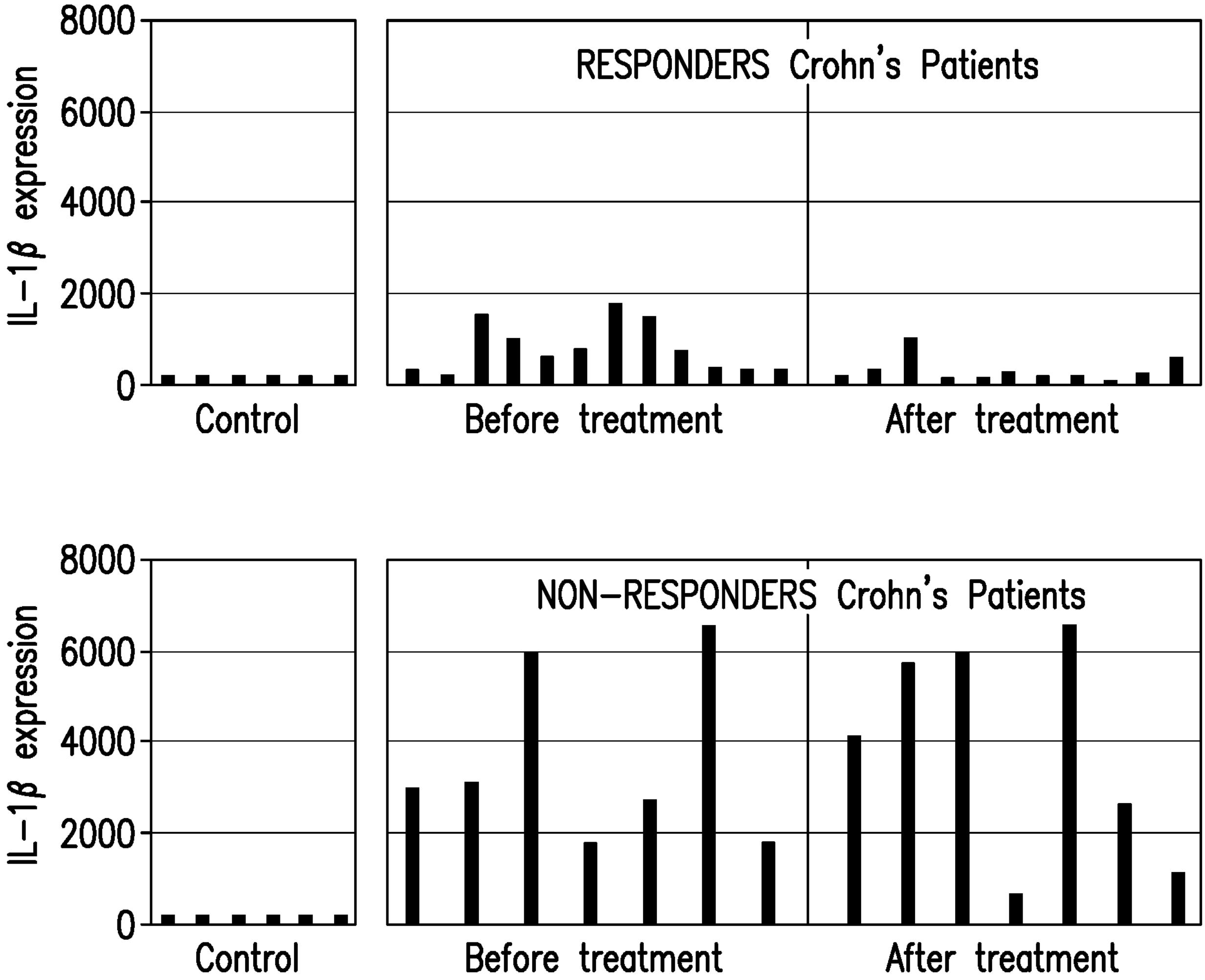
8000
6000
4000
2000
0
IL-1β expression
RESPONDERS Crohn's Patients
Control
Before treatment
After treatment
8000
6000
4000
2000
0
IL-1β expression
NON-RESPONDERS Crohn's Patients
Control
Before treatment
After treatment

FIG.5

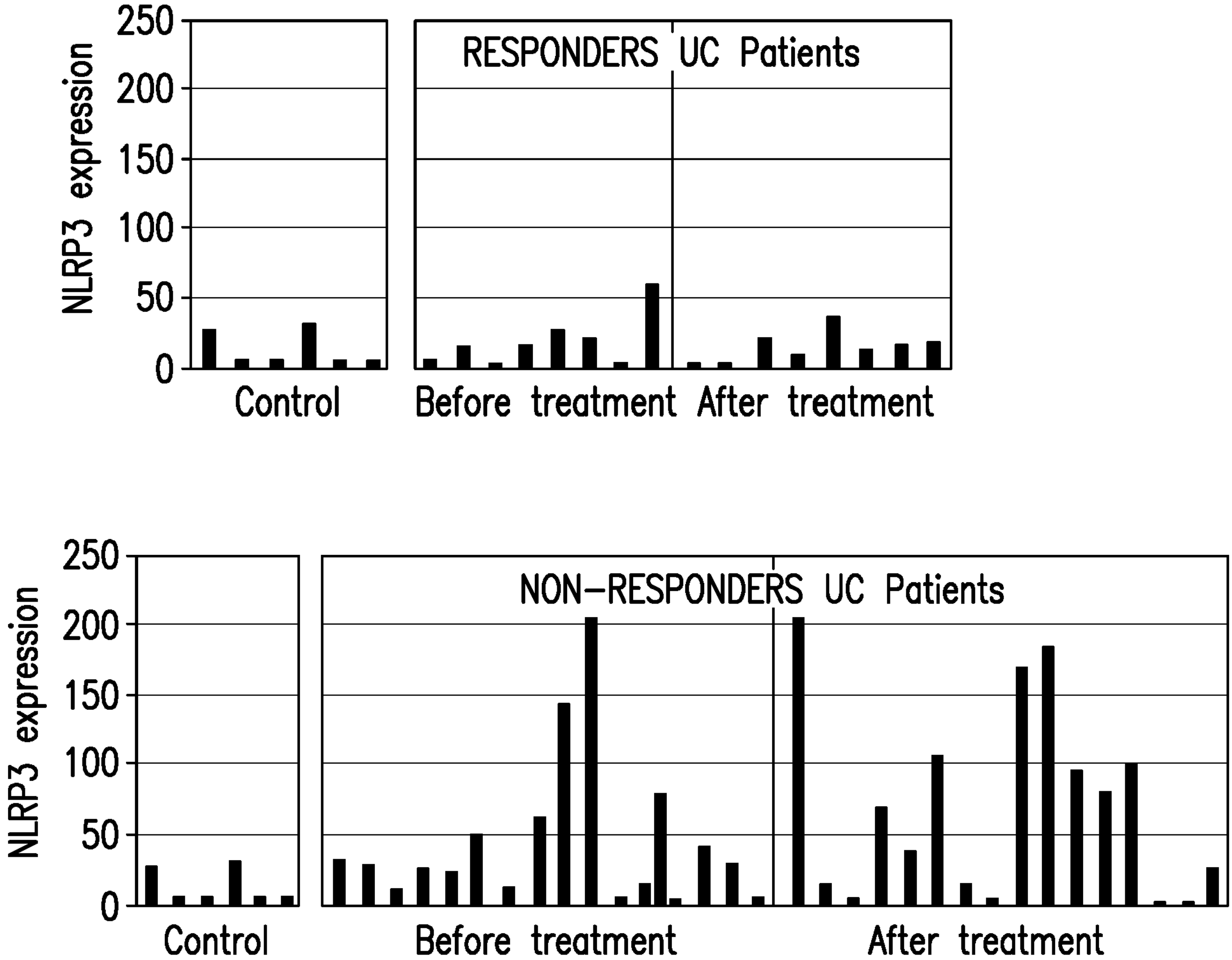
NLRP3 expression
250
200
150
100
50
0
Control
RESPONDERS UC Patients
Before treatment
After treatment
NLRP3 expression
250
200
150
100
50
0
Control
NON-RESPONDERS UC Patients
Before treatment
After treatment

FIG.6

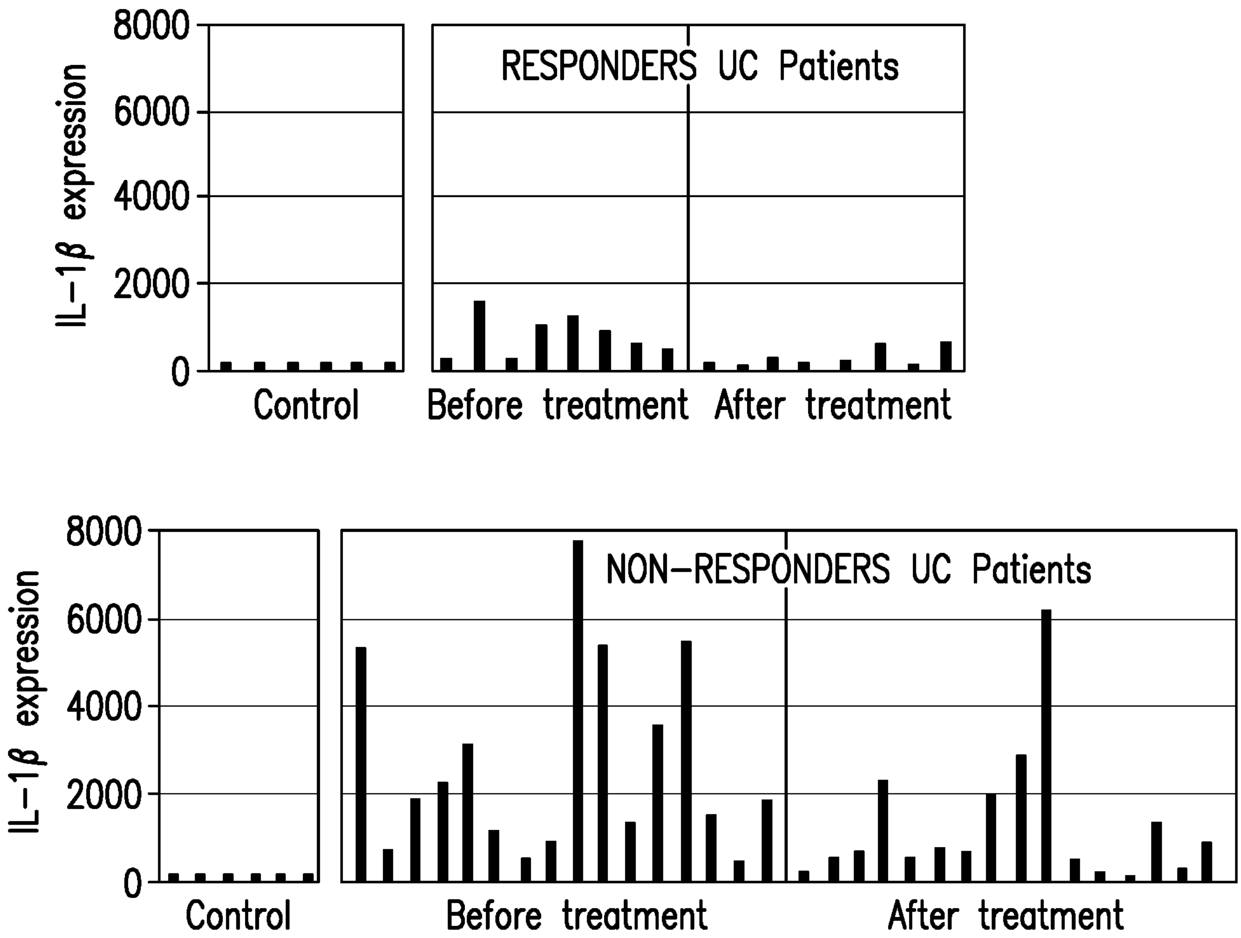

8000
6000
4000
2000
0
IL−1β expression
RESPONDERS UC Patients
Control
Before treatment
After treatment
8000
6000
4000
2000
0
IL−1β expression
NON−RESPONDERS UC Patients
Control
Before treatment
After treatment


FIG.7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016131098 A
- WO 2017129897 A
- US 6090382 A
- US 6258562 B
- US 6509015 B
- WO 9103553 A
- WO 09406476 A
- US 4987071 A
- US 5116742 A
- US 9278956 B
- US 5705398 A
- US 20160090598 A


### Non-patent literature cited in the description


- **RUTGEERTS P et al.** *N Engl J Med,* 2005, vol. 353, 2462-76
- **NEURATH MF.** *Nat Rev Immunol,* 2014, vol. 14, 329-42
- **MAO L et al.** *J Clin Invest,* 2018, vol. 238, 1793-1806
- **SHOUVAL DS et al.** *Gastroenterology,* 2016, vol. 151, 1100-1104
- **KANAI T et al.** *Curr Drug Targets,* 2013, vol. 14, 1392-9
- **PERERA AP et al.** *Sci Rep,* 2018, vol. 8, 8618
- **SAITOH T et al.** *Nature,* 2008, vol. 456, 264
- **SPALINGER MR.** *Cell Rep,* 2018, vol. 22, 1835
- **BEN-HORIN S et al.** *Autoimmun Rev,* 2014, vol. 13, 24-30
- **STEENHOLDT C et al.** *Gut,* 2014, vol. 63, 919-27
- **VAN DEN BRANDE et al.** *Gut,* 2007, vol. 56, 509-17
- **LEAL RF et al.** *Gut,* 2015, vol. 64, 233-42
- **SCHMITT H et al.** *Gut,* 2018, vol. 0, 1-15
- **GENOVESE MC et al.** *Arthritis Rheum,* 2004, vol. 50, 1412
- Remington: The Science and Practice of Pharmacy. Lippincott Williams & Wilkins, 2005
- Handbook of Pharmaceutical Excipients
- The Pharmaceutical Press and the American Pharmaceutical Association. 2009
- Handbook of Pharmaceutical Additives. Gower Publishing Company, 2007
- Pharmaceutical Preformulation and Formulation. CRC Press LLC, 2009
- Remington: The Science and Practice of Pharmacy. Pharmaceutical Press, 2012
- **LAMMERS et al.** Effect of Intratumoral Injection on the Biodistribution and the Therapeutic Potential of HPMA Copolymer-Based Drug Delivery Systems. *Neoplasia,* 2006, vol. 10, 788-795
- **FILIPSKI, K.J. et al.** *Current Topics in Medicinal Chemistry,* 2013, vol. 13, 776-802
- Pharmaceutical excipients. Marcel Dekker, 1999
- Fiedler Encyclopedia of Excipients for Pharmaceuticals, Cosmetics and Related Areas. 2002
- Lexikon der Hilfsstoffe für Pharmazie, Kosmetik and angrenzende Gebiete. 1989
- **WAJANT et al.** *Cell Death Differentiation,* 2003, vol. 10, 45-65
- **BEN-HORIN et al.** *Autoimmunity Rev,* 2014, vol. 13 (1), 24-30
- **BONGARTZ et al.** *JAMA,* 2006, vol. 295 (19), 2275-2285
- **BUTLER et al.** *Eur. Cytokine Network,* 1994, vol. 6 (4), 225-230
- **COHEN et al.** *Canadian J. Gastroenterol. Hepatol.,* 2001, vol. 15 (6), 376-384
- **ELLIOTT et al.** *Lancet,* 1994, vol. 344, 1125-1127
- **FELDMANN et al.** *Ann. Rev. Immunol.,* 2001, vol. 19 (1), 163-196
- **RANKIN et al.** *Br. J. Rheumatol.,* 1995, vol. 2, 334-342
- **KNIGHT et al.** *Molecular Immunol.,* 1993, vol. 30 (16), 1443-1453
- **LORENZ et al.** *J. Immunol.,* 1996, vol. 156 (4), 1646-1653
- **HINSHAW et al.** *Circulatory Shock,* 1990, vol. 30 (3), 279-292
- **ORDAS et al.** *Clin. Pharmacol. Therapeutics,* 2012, vol. 91 (4), 635-646
- **FELDMAN.** *Nature Reviews Immunol.,* 2002, vol. 2 (5), 364-371
- **TAYLOR et al.** *Nature Reviews Rheumatol.,* 2009, vol. 5 (10), 578-582
- **GARCES et al.** *Annals Rheumatic Dis.,* 2013, vol. 72 (12), 1947-1955

- **PALLADINO et al.** *Nature Rev. Drug Discovery,* 2003, vol. 2 (9), 736-746
- **SANDBORN et al.** *Inflammatory Bowel Diseases,* 1999, vol. 5 (2), 119-133
- **ATZENI et al.** *Autoimmunity Reviews,* 2013, vol. 12 (7), 703-708
- **MAINI et al.** *Immunol. Rev.,* 1995, vol. 144 (1), 195-223
- **WANNER et al.** *Shock,* 1999, vol. 11 (6), 391-395
- **DEEG et al.** *Leukemia,* 2002, vol. 16 (2), 162
- **PEPPEL et al.** *J. Exp. Med,* 1991, vol. 174 (6), 1483-1489
- **BJORNBERG et al.** *Lymphokine Cytokine Res,* 1994, vol. 13 (3), 203-211
- **KOZAK et al.** *Am. J. Physiol. Reg. Integrative Comparative Physiol,* 1995, vol. 269 (1), R23-R29
- **TSAO et al.** *Eur Respir J,* 1999, vol. 14 (3), 490-495
- **WATT et al.** *J Leukoc Biol.,* 1999, vol. 66 (6), 1005-1013
- **MOHLER et al.** *J. Immunol.,* 1993, vol. 151 (3), 1548-1561
- **NOPHAR et al.** *EMBO J.,* 1990, vol. 9 (10), 3269
- **PIGUET et al.** *Eur. Respiratory J,* 1994, vol. 7 (3), 515-518
- **GRAY et al.** *Proc. Natl. Acad. Sci. U.S.A.,* 1990, vol. 87 (19), 7380-7384
- **GRADSTEIN et al.** *J. Acquir. Immune Defic. Syndr.,* 2001, vol. 26 (2), 111-117
- **GAULTIER et al.** *Nucleic Acids Res.,* 1987, vol. 15, 6625-6641
- **INOUE et al.** *FEBS Lett,* 1987, vol. 215, 327-330
- **INOUE et al.** *Nucleic Acids Res.,* 1987, vol. 15, 6131-6148
- **HASELHOFF ; GERLACH.** *Nature,* 1988, vol. 334, 585-591
- **BARTEL et al.** *Science,* 1993, vol. 261, 1411-1418
- **MAHER.** *Bioassays,* 1992, vol. 14 (12), 807-15
- **HELENE.** *Anticancer Drug Des,* 1991, vol. 6 (6), 569-84
- **HELENE.** *Ann. N.Y. Acad. Sci.,* 1992, vol. 660, 27-36
- **HYRUP et al.** *Bioorganic Medicinal Chem,* 1996, vol. 4 (1), 5-23
- **PERRY-O'KEEFE et al.** *Proc. Natl. Acad. Sci. U.S.A.,* 1996, vol. 93, 14670-675
- **INOUE et al.** *FEBS Lett.,* 1987, vol. 215, 327-330
- **MOSS et al.** *Nature Clinical Practice Rheumatology,* 2008, vol. 4, 300-309
- **MA et al.** *J. Biol. Chem,* 2014, vol. 289 (18), 12457-66
- **HARAGUCHI et al.** *AIDS Res. Ther.,* 2006, vol. 3, 8
- **HE et al.** *Science,* 2005, vol. 310 (5750), 1022-1025
- **OLSON et al.** *Scientific Reports,* 2015, vol. 5, 14246
- **BAKER et al.** *Nature,* 2013, vol. 497, 577-578
- **AKINLEYE et al.** *J. Hematol. Oncol,* 2013, vol. 6 (27), 38
- **FACCIORUSSO et al.** *Expert Review Gastroentrol. Hepatol.,* 2015, vol. 9, 993-1003
- **MANDAL et al.** *Oncogene,* 2016, vol. 35, 2547-2561
- **MORTIER et al.** *Bioorg. Med. Chem. Lett.,* 2010, vol. 20, 4515-4520
- **REILLY et al.** *Nature Med,* 2013, vol. 19, 313-321
- **SUZUKI et al.** *Expert. Opin. Invest. Drugs,* 2011, vol. 20, 395-405
- **GUPTA et al.** *Biochim. Biophys. Acta,* 2010, vol. 1799 (10-12), 775-787
- **CHAUDHARY et al.** *J. Med. Chem.,* 2015, vol. 58 (1), 96-110
- **R. LAROCK.** Comprehensive Organic Transformations. VCH Publishers, 1989
- **T. W. GREENE ; RGM. WUTS.** Protective Groups in Organic Synthesis. John Wiley and Sons, 1991
- **L. FIESER ; M. FIESER.** Fieser and Fieser's Reagents for Organic Synthesis. John Wiley and Sons, 1994
- Encyclopedia of Reagents for Organic Synthesis. John Wiley and Sons, 1995
- *Org. Synth,* 2006, vol. 83, 131
- *PLoS One,* 24 November 2009, vol. 4 (11), e7984